(19) Europäisches Patentamt
European Patent Office
Office européen des brevets

(11) EP 4 729 561 A1

(12) **EUROPEAN PATENT APPLICATION**
published in accordance with Art. 153(4) EPC

(43) Date of publication:
**22.04.2026 Bulletin 2026/17**

(21) Application number: **24822802.5**

(22) Date of filing: **14.06.2024**

(51) International Patent Classification (IPC):
***C08G 69/40*** (2006.01) ***C08G 69/08*** (2006.01)
***C08G 65/333*** (2006.01) ***A61K 47/60*** (2017.01)
***A61K 47/64*** (2017.01) ***A61P 35/00*** (2006.01)

(52) Cooperative Patent Classification (CPC):
**A61K 47/60; A61K 47/64; A61P 35/00; C08G 65/333; C08G 69/08; C08G 69/40**

(86) International application number:
**PCT/CN2024/099265**

(87) International publication number:
**WO 2024/255854 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.06.2023 CN 202310714197**

(71) Applicant: **Chongqing Upgra Biotechnology Co., Ltd.**
**Chongqing 401120 (CN)**

(72) Inventors:
- **LI, Gaoquan**
  **Chongqing 401120 (CN)**
- **LIU, Nian**
  **Chongqing 401120 (CN)**
- **MEI, Gang**
  **Chongqing 401120 (CN)**
- **PENG, Yongchen**
  **Chongqing 401120 (CN)**
- **GAO, Yang**
  **Chongqing 401120 (CN)**
- **PENG, Yuanyuan**
  **Chongqing 401120 (CN)**
- **CHEN, Huiyu**
  **Chongqing 401120 (CN)**
- **LOU, Jie**
  **Chongqing 401120 (CN)**
- **ZENG, Xiafan**
  **Chongqing 401120 (CN)**
- **LI, Miao**
  **Chongqing 401120 (CN)**
- **LIU, Jing**
  **Chongqing 401120 (CN)**
- **HU, Qian**
  **Chongqing 401120 (CN)**
- **YIN, Yifeng**
  **Chongqing 401120 (CN)**
- **YANG, Shuai**
  **Chongqing 401120 (CN)**
- **WU, Yongqin**
  **Chongqing 401120 (CN)**
- **GUAN, Sheng**
  **Chongqing 401120 (CN)**
- **CHEN, Longwu**
  **Chongqing 401120 (CN)**
- **XU, Xia**
  **Chongqing 401120 (CN)**
- **ZHANG, Xinli**
  **Chongqing 401120 (CN)**
- **ZHANG, Qingmeng**
  **Chongqing 401120 (CN)**
- **SHI, Xichen**
  **Chongqing 401120 (CN)**

(74) Representative: **Lorenz Seidler Gossel Part. mbB**
**Widenmayerstr. 23**
**80538 München (DE)**

# (54) POLYETHYLENE GLYCOL DRUG, AND PREPARATION METHOD THEREFOR AND USE THEREOF

(57) The present application relates to a polyethylene glycol drug, and a preparation method therefor and the use thereof. Specifically, the present application relates to a compound as represented by formula I or a pharmaceutically acceptable salt thereof, intermediate compounds thereof as represented by formula II to for-

mula XX, a method for preparing the compound, and the use thereof in fighting tumors. Biological tests show that the compound of the present application has a significant inhibitory effect on tumors such as breast cancer, non-small cell lung cancer, melanoma, oral squamous cell carcinoma, osteosarcoma, etc.

## Description

The present application is based on and claims priority to the Chinese application with application number 202310714197.4 and application date June 15, 2023, the content of which is hereby incorporated by reference in its entirety.

## Technical Field

The present application relates to the field of medicinal chemistry, and specifically to a polyethylene glycol drug, and preparation method therefor and use thereof.

## Background Art

Over the past 30 years, the U.S. FDA has approved 30 drugs in the polyethylene glycol (PEG) drug category for marketing. More than 40 new drug candidates in Phase I, II, and III clinical trials or the NDA process, with half being PEGylated small-molecule drugs. PEG is undisputedly the most successful synthetic polymer carrier in clinical practice, known as the "gold standard" carrier. PEGylated drugs are shifting from large-molecules to small-molecules, with small-molecule drugs accounting for 80% to 90% of the drug market. With the successful research and development of PEGylated small-molecule dual drugs, as well as those incorporating active targeting moieties or enhancers, by Chongqing Upgra Biotechnology Co., Ltd., additionally, PEGylation technology is applicable not only in anti-tumor drugs but also in areas such as anti-infectives, gout, arthritis, pain, and other inflammatory conditions, as well as in diabetes, it potentially enables the development of hundreds of product pipelines, which could form a multibillion-dollar industry.

Recently, the polyethylene glycol-lysine dendritic polypeptide-chemotherapeutic single drugs (including DEP®cabazitaxel, docetaxel, and irinotecan) developed by Australia's STARPHARMA have demonstrated excellent efficacy in Phase II clinical trials, with significantly reduced toxicity and side effects. Pharmaceutical giants such as ASTRAZENECA, MERCK, and ROCHE/GENENTECH have begun participating in the research and development of this field. Chongqing Upgra Biotechnology Co., Ltd. has also synthesized PEG-polypeptide (e.g., glutamic acid, aspartic acid, lysine)-small molecule drugs, including small-molecule single drugs, dual drugs, multiple drugs, enhancers, and active targeting moieties, and these numerous pipeline products have shown significantly improved efficacy and significantly reduced side effects, and are currently advancing toward clinical trials. Recently, scientists from WINDOW Tx/Massachusetts Institute of Technology (MIT) and Harvard University published preclinical animal results of bottlebrush-like PEG-triple drugs synthesized via ring-opening metastasis polymerization (ROMP) for the treatment of Multiple myeloma. To facilitate the synthesis of PEG-dual drugs or multiple drugs in various ratios or PEG conjugates with active targeting moieties, the present application designs methods for synthesizing various compounds, including PEG-small molecule drugs, active targeting moieties, boron reagents, etc., based on certain aliphatic polyamine or polyhydroxyl core structures, and these compounds are synthesized. Biological assays demonstrate that these compounds have significant therapeutic efficacy.

## Contents of the present invention

### Compounds

In one aspect, the present application provides a compound represented by formula I or a pharmaceutically acceptable salt thereof:

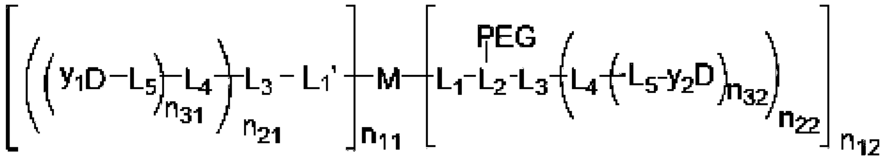

Formula I

wherein,

M is a hydrocarbonyl containing two or more (e.g., 2, 3, 4, 5, or 6) identical or different heteroatoms (e.g., N, O, or S), and M is connected to $L_1$ and $L_1$' via the heteroatoms;

each $L_1$ is independently selected from the group consisting of

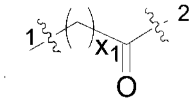

and

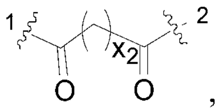
,

wherein the terminus 1 is connected to M, and the terminus 2 is connected to $L_2$;

each $L_1$' is independently selected from the group consisting of

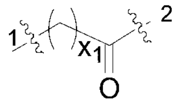

and

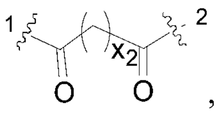
,

wherein the terminus 1 is connected to M, and the terminus 2 is connected to $L_3$; and $L_1$ and $L_1$' are different (e.g., $L_1$ is

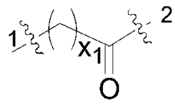

and $L_1$' is

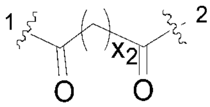
;

or $L_1$ is

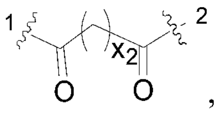
,

and $L_1$' is

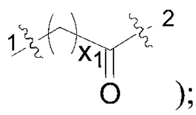
);

$x_1$ and $x_2$, at each occurrence, are independently selected from the group consisting of 0, 1, 2, 3, 4, 5, 6, 7, 8, 9, and 10;

each $L_2$ is independently selected from the group consisting of residues of Lys, Cys, Thr, Ser, Asp, or Glu;

each PEG is independently selected from

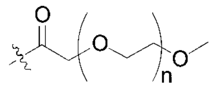

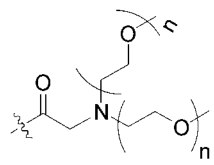

,

and

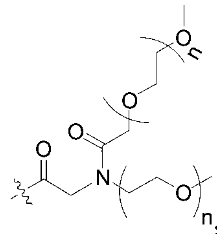

, each of which has a number-average molecular weight of 5k, 10k, or 10k, 40k, for example, 5k or 10k;

each $L_3$ is independently a bond or

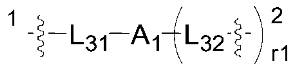

,

wherein the terminus 1 is connected to $L_2$ or $L_1$', and the terminus 2 is connected to $L_4$, wherein each $L_{31}$ and $L_{32}$ is independently selected from the group consisting of a bond and $-NH(CH_2)_{x3}C(O)-$, $x_3$ is selected from the group consisting of 0, 1, 2, 3, 4, 5, 6, 7, 8, 9, and 10, $A_1$ is selected from the group consisting of a bond, an amino acid residue or a derivative thereof, or a polypeptide fragment consisting of two or more amino acids or a derivative thereof, and r1 is selected from the group consisting of 1, 2, 3, 4, 5, and 6;

each $L_4$ is independently a bond or

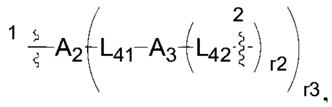

wherein the terminus 1 is connected to $L_3$ and the terminus 2 is connected to $L_5$, wherein each $L_{41}$ and $L_{42}$ is independently selected from the group consisting of a bond, $-NH(CH_2)_{x4}C(O)-$, $-NH(CH_2)_{x4}NH-$, $-NH((CH_2)_2O)_{x4}CH_2CH_2NH-$, and $-C(O)(CH_2)_{x4}C(O)-$; $A_2$ and $A_3$ are independently selected from the group consisting of a bond, an amino acid residue or a derivative thereof, or a polypeptide fragment consisting of two or more amino acids or a derivative thereof; r2 and r3 are each independently selected from the group consisting of 1, 2, 3 4, 5, and 6;

each $L_5$ is independently a bond or selected from the group consisting of

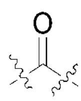

,

-NH-, hydrazino (i.e., -NH-N=), an amino acid residue or a derivative thereof, a polypeptide fragment consisting of two or more amino acids or a derivative thereof, $-NH(CH_2)_{x5}C(O)-$, $-NH(CH_2)_{x5}NH-$, $-NH((CH_2)_2O)_{x5}CH_2CH_2NH-$, $-NH((CH_2)_2O)_{x5}CO-$, $-C(O)(CH_2)_{x5}C(O)-$, and any combination thereof; or

$L_4$ and $L_5$ are connected to form

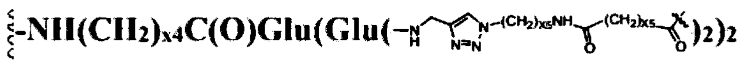

,

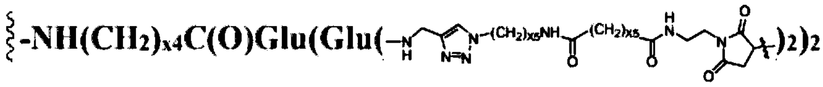

,

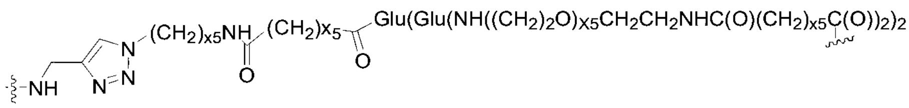

or

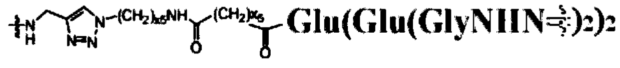

;

preferably, $L_4$ and $L_5$ are connected to form

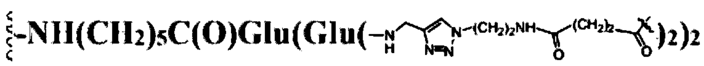

,

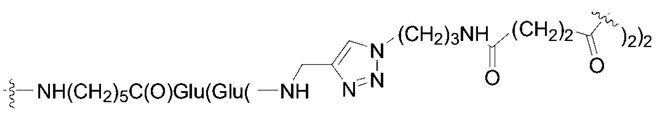

,

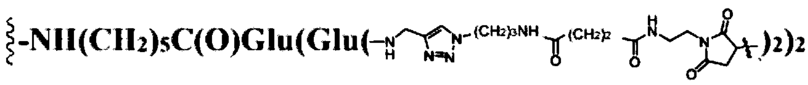

,

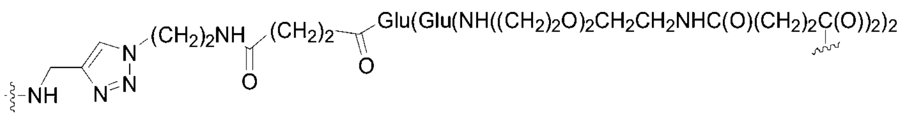

or

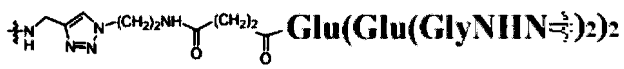

;

$x_4$ and $x_5$, at each occurrence, are independently selected from the group consisting of 0, 1, 2, 3, 4, 5, 6, 7, 8, 9, and 10;

each D is independently selected from the group consisting of a cytotoxic drug moiety, preferably, the cytotoxic drug is selected from the group consisting of tubulin inhibitors, DNA intercalators, DNA topoisomerase inhibitors, and RNA polymerase inhibitors; preferably, the cytotoxic drug is selected from the group consisting of PTX (paclitaxel), PCB (Palbociclib), SN38 (7-ethyl-10-hydroxy-camptothecin), NPB (Niraparib, MK-4827), AXT (Axitinib), LPT (Lapatinib), DOX (Doxorubicin), Ac-C-PLGLAG-iRGD, folic acid, SB7 (SB-743921), IRN (Irinotecan), sodium dodecahydrododecaborate, PPT-iRGD, dodecaborate(2-),1,2,3,4,5,6,7,8,9,10,11-undecahydro-12-mercapto-, sodium (1:2) ($^{10}$B-BSH, Sodium Mercaptododecaborate ($^{10}$B)),

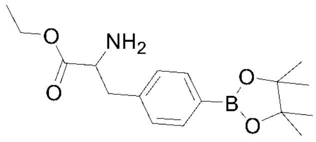, 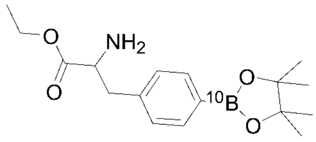

and

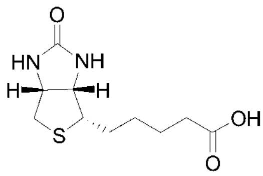;

each $n_{11}$ and $n_{12}$ is independently selected from the group consisting of 1, 2, 3, 4, 5, 6, 7, 8, 9, and 10;

each $n_{21}$ and $n_{22}$ is independently selected from the group consisting of 1, 2, 3, 4, 5, 6, 7, 8, 9, and 10;

each $n_{31}$ and $n_{32}$ is independently selected from the group consisting of 1, 2, 3, 4, 5, 6, 7, 8, 9, and 10;

each $y_1$ and $y_2$ is independently selected from the group consisting of 1, 2, 3, 4, 5, 6, 7, 8, 9, and 10.

In some embodiments, M is a hydrocarbonyl containing two or more (e.g., 2, 3, 4, 5, or 6) identical or different heteroatoms (e.g., N, O, or S), and M is connected to $L_1$ through the heteroatoms;

each $L_1$ is independently selected from the group consisting of

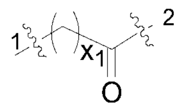

and

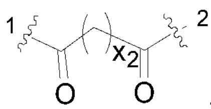,

wherein the terminus 1 is connected to M, and the terminus 2 is connected to $L_2$, wherein $x_1$ and $x_2$ are each independently selected from the group consisting of 0, 1, 2, 3, 4, 5, 6, 7, 8, 9, and 10;

each $L_1$' is independently selected from the group consisting of

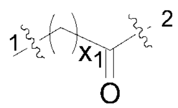

and

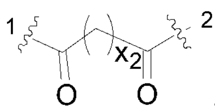,

wherein the terminus 1 is connected to M, and the terminus 2 is connected to $L_3$, wherein $x_1$ and $x_2$ are each independently selected from the group consisting of 0, 1, 2, 3, 4, 5, 6, 7, 8, 9, and 10; furthermore, $L_1$ and $L_1$' are different;

each $L_2$ is independently selected from the group consisting of residues of Lys, Cys, Thr, Ser, Asp, or Glu;

each PEG is independently selected from the group consisting of

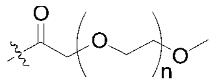,

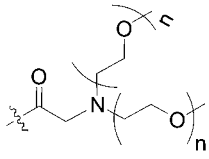 and 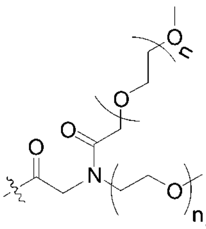,

, and has a number-average molecular weight of 5k, 10k, or 10k, 40k, for example, 5k or 10k;

each $L_3$ is indenendentlv a bond or

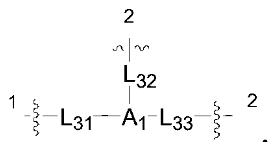

wherein the terminus 1 is connected to $L_2$, and the terminus 2 is connected to $L_4$, wherein each $L_{31}$, $L_{32}$ and L33 is independently selected from the group consisting of a bond and $-NH(CH_2)_{x3}C(O)-$, $x_3$ is selected from the group consisting of 0, 1, 2, 3, 4, 5, 6, 7, 8, 9, and 10; $A_1$ is selected from the group consisting of a bond, an amino acid residue or a derivative thereof, or a polypeptide fragment consisting of two or more amino acids or a derivative thereof, and when $A_1$ is a polypeptide fragment or a derivative thereof, $A_1$ is connected to one or more $L_{32}$;

each $L_4$ is independently a bond or

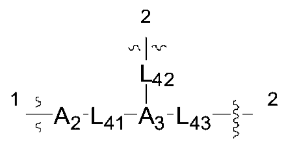,

wherein the terminus 1 is connected to $L_3$, and the terminus 2 is connected to $L_5$, wherein each $L_{41}$, $L_{42}$ and $L_{43}$ is independently selected from the group consisting of a bond, $-NH(CH_2)_{x4}C(O)-$, $-NH(CH_2)_{x6}NH-$, $-NH(CH_2O)_{x4}CH_2CH_2NH-$, or $-C(O)(CH_2)_{x4}C(O)-$; $x_4$ is selected from the group consisting of 0, 1, 2, 3, 4, 5, 6, 7, 8, 9, and 10; $A_2$ and $A_3$ are independently selected from the group consisting of a bond, an amino acid residue or a derivative thereof, or a polypeptide fragment consisting of two or more amino acids or a derivative thereof; when $A_3$ is a polypeptide fragment or a derivative thereof, $A_3$ is connected to one or more $L_{42}$;

each $L_5$ is independently a bond or selected from the group consisting of

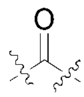,

-NH-, hydrazino (i.e., -NH-N=), an amino acid residue or a derivative thereof, a polypeptide fragment consisting of two or more amino acids or a derivative thereof, $-NH(CH_2)_{x5}C(O)-$, $-NH(CH_2)_{x5}NH-$, $-NH(CH_2O)_{x5}CH_2CH_2NH-$, $-NH(CH_2O)_{x5}CO-$, $-C(O)(CH_2)_{x5}C(O)-$,

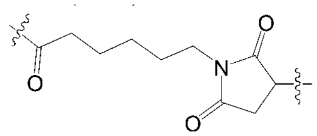

and any combination thereof, wherein each $x_5$ is independently selected from the group consisting of 0, 1, 2, 3, 4, 5, 6, 7, 8, 9, and 10; or,

$L_4$ and $L_5$ are connected to form

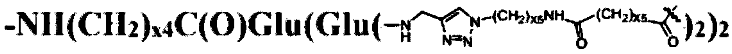,

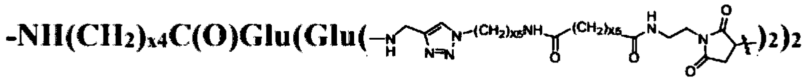,

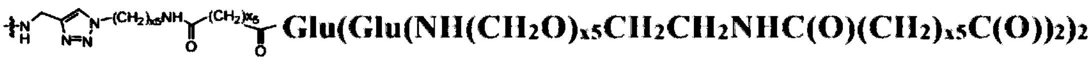

or

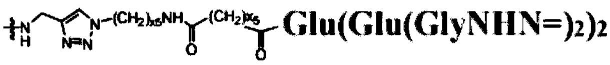;

preferably, $L_4$ and $L_5$ are connected to form

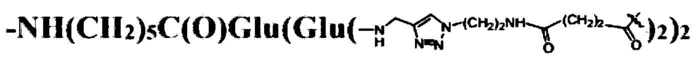,

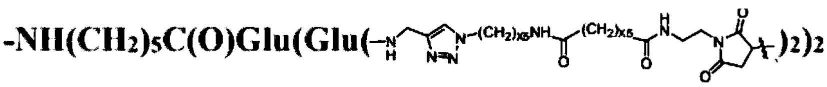

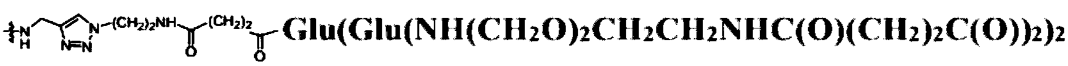

or

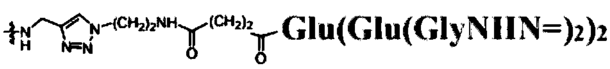;

each D is independently selected from the group consisting of a cytotoxic drug moiety, preferably, the cytotoxic drug is selected from the group consisting of tubulin inhibitors, DNA intercalators, DNA topoisomerase inhibitors, and RNA polymerase inhibitors; preferably, the cytotoxic drug is selected from the group consisting of PTX (paclitaxel), PCB (Palbociclib), SN38 (7-ethyl-10-hydroxy-camptothecin), NPB (Niraparib, MK-4827), AXT (Axitinib), LPT (Lapatinib), DOX (Doxorubicin), MI-AH-PLGLAG-iRGD, folic acid, SB7 (SB-743921), IRN (Irinotecan), sodium dodecahydro-12-thiobenzoate, PPT-iRGD, dodecaborate(2-),1,2,3,4,5,6,7,8,9,10,11-undecahydro-12-mercapto-, sodium (1:2) (BSH, Sodium Mercaptododecaborate ($^{10}B$)),

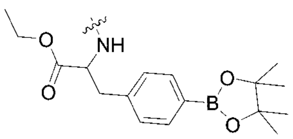, 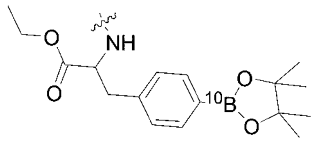

and

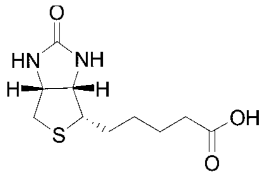
;

each $n_{11}$ and $n_{12}$ is independently selected from the group consisting of 1, 2, 3, 4, 5, 6, 7, 8, 9, and 10;

each $n_{21}$ and $n_{22}$ is independently selected from the group consisting of 1, 2, 3, 4, 5, 6, 7, 8, 9, and 10;

each $n_{31}$ and $n_{32}$ is independently selected from the group consisting of 1, 2, 3, 4, 5, 6, 7, 8, 9, and 10;

each $y_1$ and $y_2$ is independently selected from the group consisting of 1, 2, 3, 4, 5, 6, 7, 8, 9, and 10.

In some embodiments, M is a $C_{2-10}$ hydrocarbonyl containing 2 to 4 identical or different heteroatoms.

In some embodiments, M is a saturated $C_{2-6}$ hydrocarbonyl containing 2 to 4 heteroatoms independently selected from the group consisting of nitrogen and oxygen.

In some embodiments, M is selected from the group consisting of the following structures:

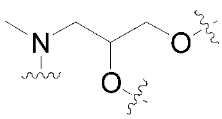
,
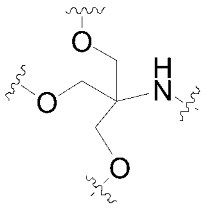
,
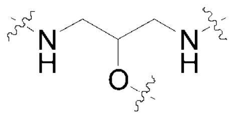
,
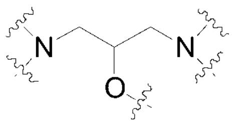
,
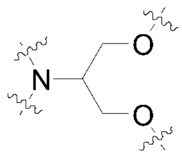
,
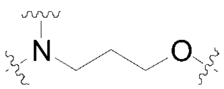
,
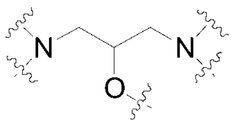
,
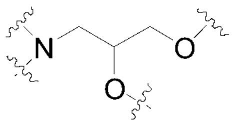
,
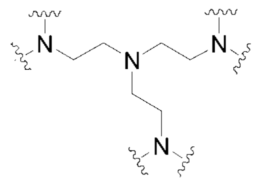

and

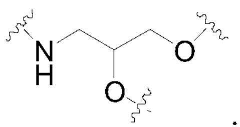
.

In some embodiments, each $L_1$ and $L_1$' is independently selected from

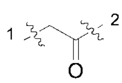
,

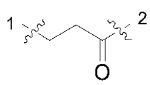
and
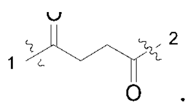
.

In some embodiments, $L_1$ is

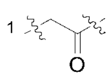 or 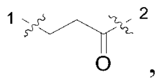,

and $L_1$' is

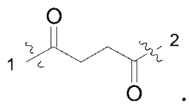.

In some embodiments, $L_1$ is

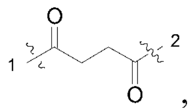,

and $L_1$' is

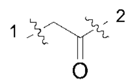 or 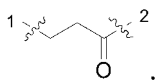.

In some embodiments, M is connected to $L_1$' via a nitrogen atom, or M is connected to $L_1$' via an oxygen atom; preferably, M is connected to $L_1$' via a nitrogen atom.
In some embodiments, $n_{11}$ = 1, 2, or 3, and $n_{11} \leq n_{12}$; preferably, $n_{11}$ = 1, $n_{12}$ = 2, 3, or 4; $n_{11}$ = 2, $n_{12}$ = 3; or $n_{11}$ = 3, $n_{12}$ = 3.
In some embodiments, each $L_2$ is independently a Lys residue.
In some embodiments, when $L_3$ is

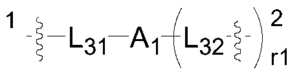,

each $L_{31}$ and $L_{32}$ is independently selected from the group consisting of a bond and $-NH(CH_2)_{x3}C(O)-$, $x_3$ is selected from the group consisting of 0, 1, 2, 3, 4, 5, and 6; $A_1$ is selected from the group consisting of a bond, an amino acid residue or a derivative thereof, or a polypeptide fragment consisting of two or more amino acids or a derivative thereof, and r1 is selected from the group consisting of 1, 2, 3, 4, 5, and 6. In some embodiments, $A_1$ is selected from the group consisting of Glu, Asp, and GluGlu.
In some embodiments, each $L_3$ is independently a bond or selected from the group consisting of the following structures:

$-NH(CH_2)_2C(O)-$,

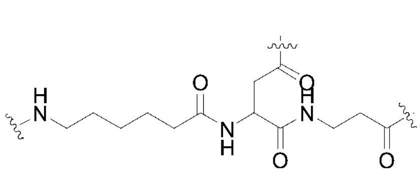, 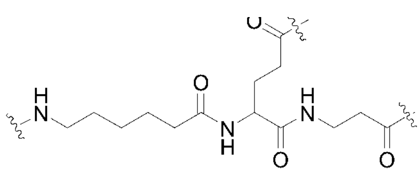,

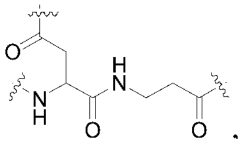, 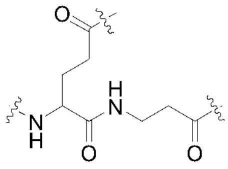 and 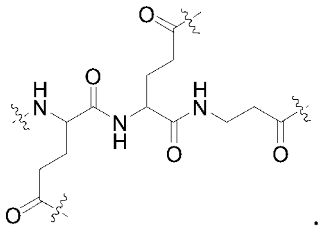.

In some embodiments, when $L_4$ is

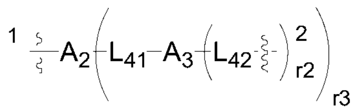,

each $L_{41}$ and $L_{42}$ is independently selected from the group consisting of a bond, $-NH(CH_2)_{x4}C(O)-$, $-NH(CH_2)_{x4}NH-$ and $-NH((CH_2)_2O)_{x4}CH_2CH_2NH-$ and $-C(O)(CH_2)_{x4}C(O)-$, each $x_4$ is independently selected from the group consisting of 0, 1, 2, 3, 4, 5 and 6; $A_2$ and $A_3$ are independently selected from the group consisting of a bond, an amino acid residue or a derivative thereof, or a polypeptide fragment consisting of more than two amino acids or a derivative thereof, and r1 is selected from the group consisting of 1, 2, 3, 4, 5 and 6. In some embodiments, each $A_2$ and $A_3$ is independently selected from the group consisting of Lys, Glu, Asp, GluGlu and $Glu(Glu)_2$.

In some embodiments, each $L_4$ is independently a bond or selected from the group consisting of the following structures:
$LysNH(CH_2)_5COGlu(Glu)_2$, $Lys(COC_2H_4CO)(NH(CH_2CH_2O)_2CH_2CH_2NH)$, $-NH(CH_2)_5COGlu$, $-NH(CH_2)_5COGlu(Glu)_2$, $-NH(CH_2)_5COGlu(Glu(NHCH_2CH_2NH)_2)_2$, $-NH(CH_2)_5COAsp$, and $-NH(CH_2)_5CO-Glu(Glu(NH(CH_2CH_2O)_2CH_2CH_2NH)_2)_2$.

In some embodiments, each $L_4$ is independently a bond or selected from the group consisting of the following structures:
$LysNH(CH_2)_5COGlu(Glu)_2$, $Lys(COC_2H_4CO)(NH(CH_2CH_2O)_2CH_2CH_2NH)$, $-NH(CH_2)_5COGlu$, $-NH(CH_2)_5COGlu(Glu)_2$, $-NH(CH_2)_5COGlu(Glu(NHCH_2CH_2NH)_2)_2$, $-NH(CH_2)_5COAsp$, and $-NH(CH_2)_5CO-Glu(Glu(NH(CH_2CH_2O)_2CH_2CH_2NH)_2)_2$.

In some embodiments, the amino acid in $L_5$ is selected from the group consisting of Glu, Gly, Phe, Leu, and Cys; preferably, the polypeptide consisting of two or more amino acids is selected from the group consisting of GlyPheLeuGly, and $Glu(Glu(Gly)_2)_2$; preferably, the derivative is selected from the group consisting of acylation (e.g., acetylation) or alkylation (e.g., methylation) derivatives.

In some embodiments, $L_5$ is selected from the group consisting of a bond,

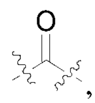,

-NH-N=, GlyPheLeuGly, $-NH(CH_2)_{x5}C(O)-$, $-NH((CH_2)_2O)_{x5}CH_2CH_2NH-$, $-NH((CH_2)_2O)_{x5}CH_2CH_2NHGlu$, $-NH(CH_2)_{x5}C(O)Glu(Glu(GlyNHN=)_2)_2$, $-NH((CH_2)_2O)_{x5}CO-$, $-C(O)(CH_2)_{x5}C(O)-$, $-C(O)(CH_2)_{x5}C(O)-GlyPheLeuGly-$, and

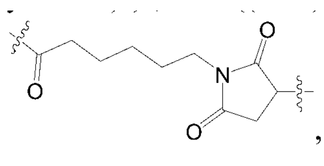,

and each $x_5$ is independently selected from the group consisting of 1, 2, 3, 4, 5, and 6.

In some embodiments, $L_5$ is selected from the group consisting of a bond,

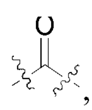, .

-NH-N=, GlyPheLeuGly, $-NH(CH_2)_{x5}C(O)-$, $-NH(CH_2O)_{x5}CH_2CH_2NH-$, $-NH(CH_2O)_{x5}CH_2CH_2NHGlu$, $-NH(CH_2)_{x5}C(O)Glu(Glu(GlyNHN=)_2)_2$, $-NH(CH_2O)_{x5}CO-$, $-C(O)(CH_2)_{x5}C(O)-$, $-C(O)(CH_2)_{x5}C(O)$-GlyPheLeuGly-, and

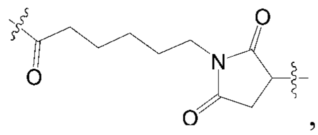

,

and each $x_5$ is independently selected from the group consisting of 1, 2, 3, 4, 5, and 6.

In some embodiments, $L_5$ is selected from the group consisting of a bond

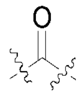

,

GlyPheLeuGly, $-NH(CH_2)_5C(O)Glu(Glu(GlyNHN=)_2)_2$, $-NH(CH_2)_5C(O)-$, $-NH((CH_2)_2O)_2CH_2CH_2NH-$, $-NH((CH_2)_2O)_2CH_2CH_2NHGlu$, $-NH((CH_2)_2O)_2CO-$, $-C(O)(CH_2)_2C(O)-$, $-C(O)(CH_2)_2C(O)$-GlyPheLeuGly-, and

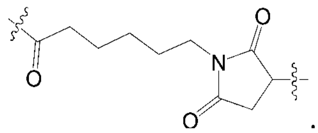

.

In some embodiments, $L_5$ is selected from the group consisting of a bond, GlyPheLeuGly, $-NH(CH_2)_5C(O)Glu(Glu(GlyNHN=)_2)_2$, $-NH(CH_2)_5C(O)-$, $-NH(CH_2O)_2CH_2CH_2NH-$, $-NH(CH_2O)_2CH_2CH_2NHGlu$, $-NH(CH_2O)_2CO-$, $-C(O)(CH_2)_2C(O)-$, $-C(O)(CH_2)_2C(O)$-GlyPheLeuGly-, and

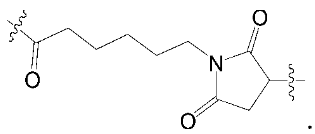

.

In some embodiments, $L_4$ and $L_5$ are connected to form

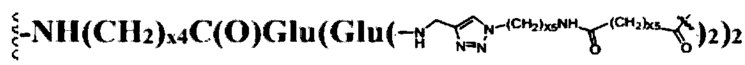

,

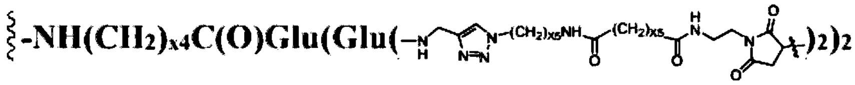

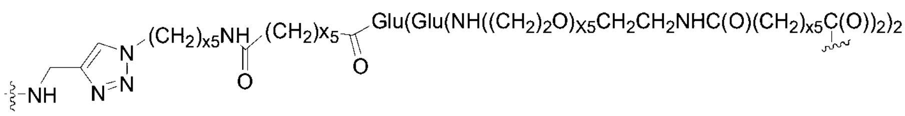

,

or

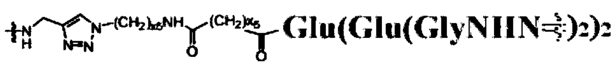

;

$x_4$ and $x_5$, at each occurrence, are each independently selected from the group consisting of 0, 1, 2, 3, 4, 5, 6, 7, 8, 9, and 10.

In some embodiments, $L_4$ and $L_5$ are connected to form

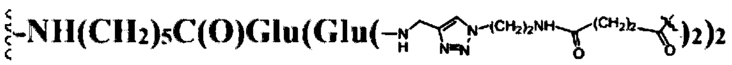

,

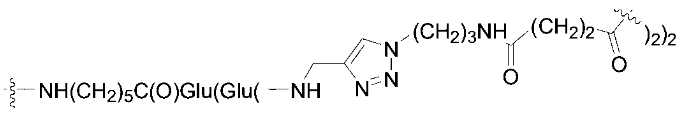

,

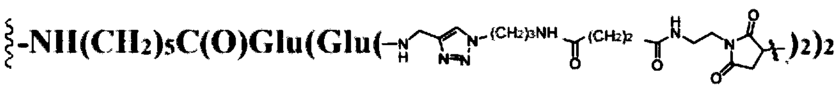

,

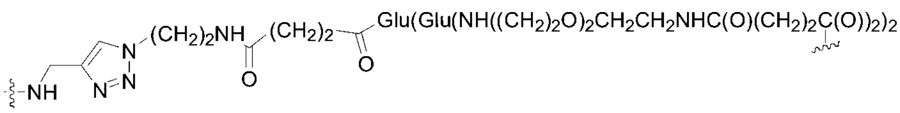

or

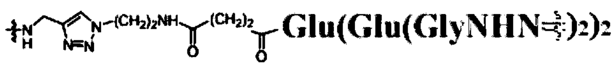

.

It should be understood that formula I has the following structure:

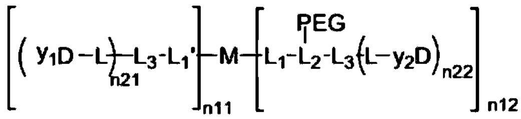

wherein, L represents the structure formed by the linkage of $L_4$ and $L_5$, and the remaining groups are as defined in any of the preceding items.

Herein, D represents a group formed by a linkage of the cytotoxic drug with the remaining moiety in the formula via a reactive group thereon. In some embodiments, the reactive group is an amino group ($-NH_2$), a secondary amine, a hydroxyl (-OH), a sulfhydryl (-SH), a carboxyl (-COOH), or the like.

In some embodiments, the D is selected from the group consisting of:

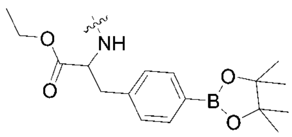

,

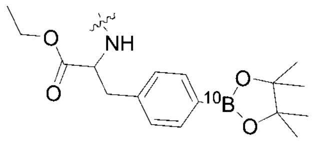

,

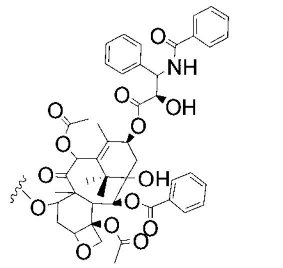

,

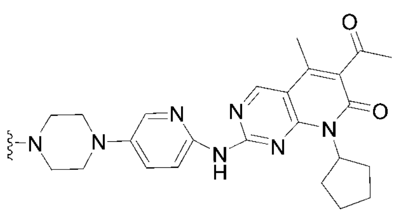

,

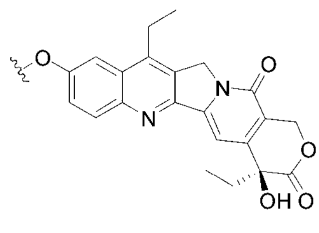

,

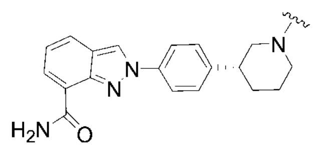

,

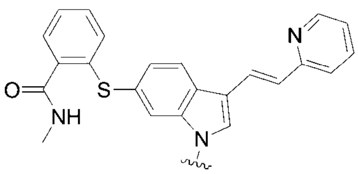, 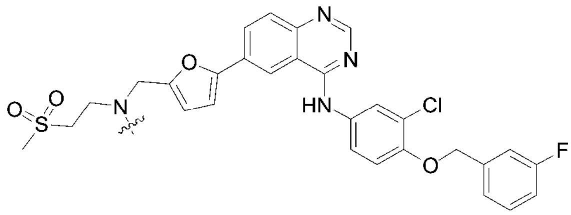, 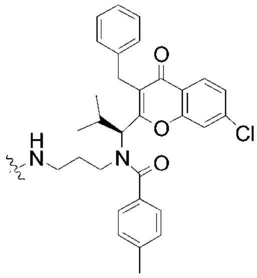,

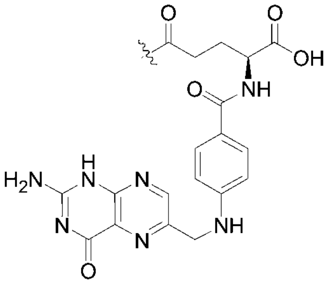, 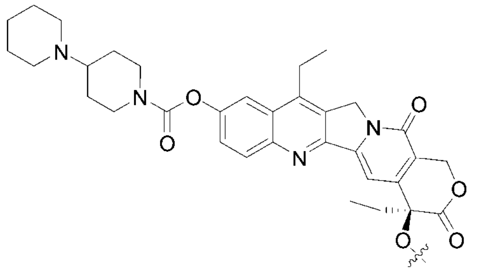, 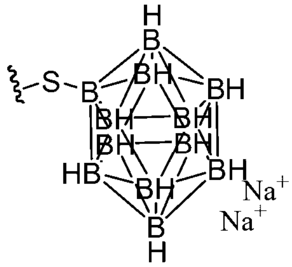,

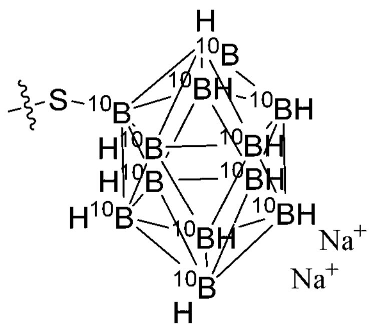, 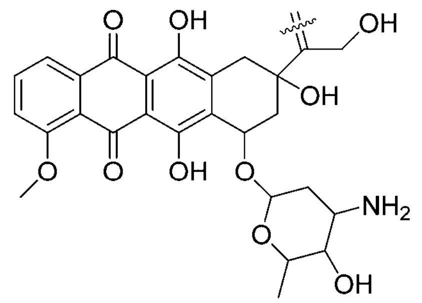,

and

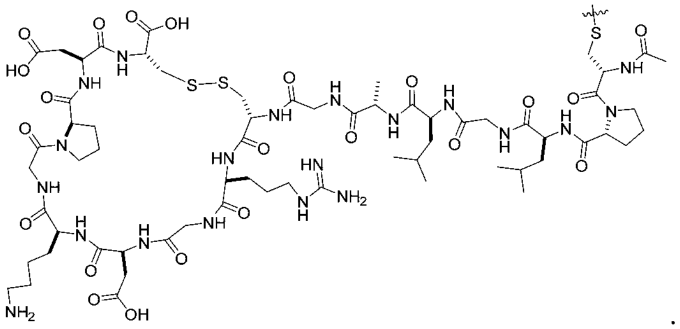.

In some embodiments,

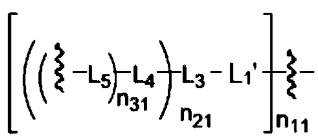

in formula I has one or more of the following characteristics:

1) $L_1$' is

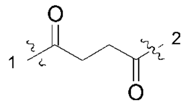 or 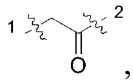,

and when the terminus 1 of $L_1$' is a carbonyl, the terminus 1 of $L_1$ is an alkylene group; when the terminus 1 of $L_1$' is an alkylene group, the terminus 1 of $L_1$ is a carbonyl;

2) $L_3$ is a bond or selected from the group consisting of the following structures:

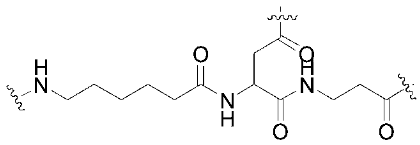, 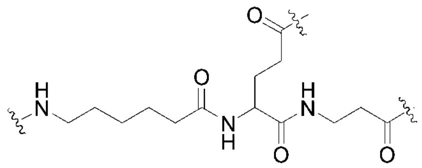 and 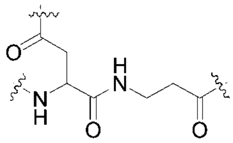;

3) $L_4$ is independently a bond or selected from the group consisting of the following structures: Lys($COC_2H_4CO$)($NH(CH_2CH_2O)_2CH_2CH_2NH$), $-NH(CH_2)_5COGlu$, $-NH(CH_2)_5COGlu(Glu)_2$ and $-NH(CH_2)_5CO$-Glu(Glu($NH(CH_2CH_2O)_2CH_2CH_2NH)_2)_2$;

4) $L_5$ is selected from the group consisting of a bond,

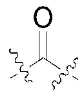,

GlyPheLeuGly, $-NH(CH_2)_5C(O)Glu(Glu(GlyNHN=)_2)_2$, $-NH(CH_2)_5C(O)-$, $-NH((CH_2)_2O)_2CH_2CH_2NH-$, and $-NH((CH_2)_2O)_2CO-$.

5) $n_{11}$ = 1, 2, or 3;

6) $n_{21}$ = 1 or 2;

7) $n_{31}$ = 1, 2, 3, or 4.

In some embodiments,

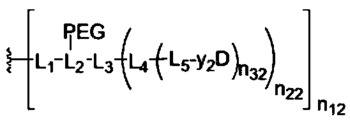

in formula I has one or more of the following characteristics:

1) $L_1$ is ,

acteristics:

, 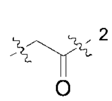 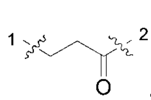, or 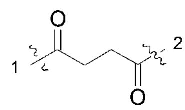,

and when the terminus 1 of $L_1$ is an alkylene group, the terminus 2 of $L_1$' is a carbonyl; when the terminus 1 of $L_1$ is a carbonyl, the terminus 2 of $L_1$' is an alkylene group;

2) $L_2$ is a Lys residue;

3) $L_3$ is a bond or selected from the group consisting of the following structures:

$-NH(CH_2)_2C(O)-$,

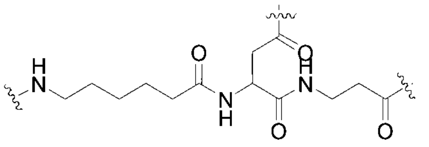,
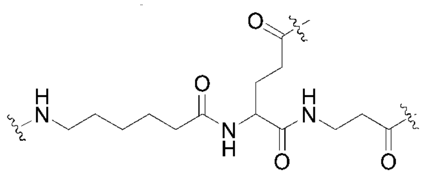,

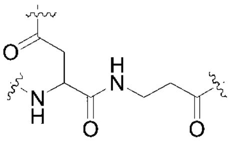,
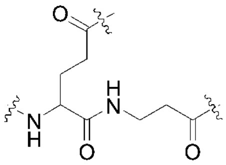
and
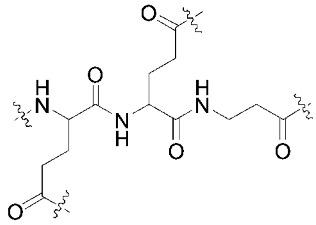;

4) $L_4$ is independently a bond or selected from the group consisting of the following structures: $LysNH(CH_2)_5COGlu(Glu)_2$, $-NH(CH_2)_5COGlu(Glu(NHCH_2CH_2NH)_2)_2$ and $-NH(CH_2)_5CO-Glu(Glu(NH(CH_2CH_2O)_2CH_2CH_2NH)_2)_2$;
5) $L_5$ is independently selected from the group consisting of the following structures: GlyPheLeuGly, $-NH(CH_2)_5C(O)Glu(Glu(GlyNHN=)_2)_2$, $-C(O)(CH_2)_2C(O)-$, $-C(O)(CH_2)_2C(O)$-GlyPheLeuGly- and

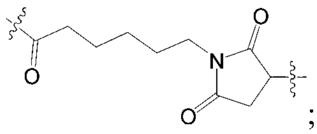;

6) $n_{12}$ = 2, 3, or 4;
7) $n_{21}$ = 1, 2, or 3;
8) $n_{32}$ = 1, 4, or 5.

Herein, when the specific selection of a divalent or higher-valent group in the general formula does not indicate its connection direction to other linked groups, the left end is connected to the group near M, and the right end is connected to the group near D.
Herein, $-y_1D$ or $-y_2D$ in each general formula indicates that the number of D groups connected to $L_5$ is $y_1$ or $y_2$, respectively.
Herein, when $L_5$ is-NH-N= or a group containing-NH-N=, -NH-N= represents the structural fragment formed after the connection of $L_5$ to D. Before its connection to D (i.e., when $L_5$ is present in an intermediate of the compounds described herein), $L_5$ may be-NH-NH-.
Herein, unless otherwise disclosed, the amino acids used in the preparation of the compounds and the amino acid residues in the structural formulas of the compounds are in their natural configuration (L-form, except for glycine Gly).
In some embodiments, the compound is selected from the group consisting of:

| Compound No. | Compound structure |
|---|---|
| **81-214** | 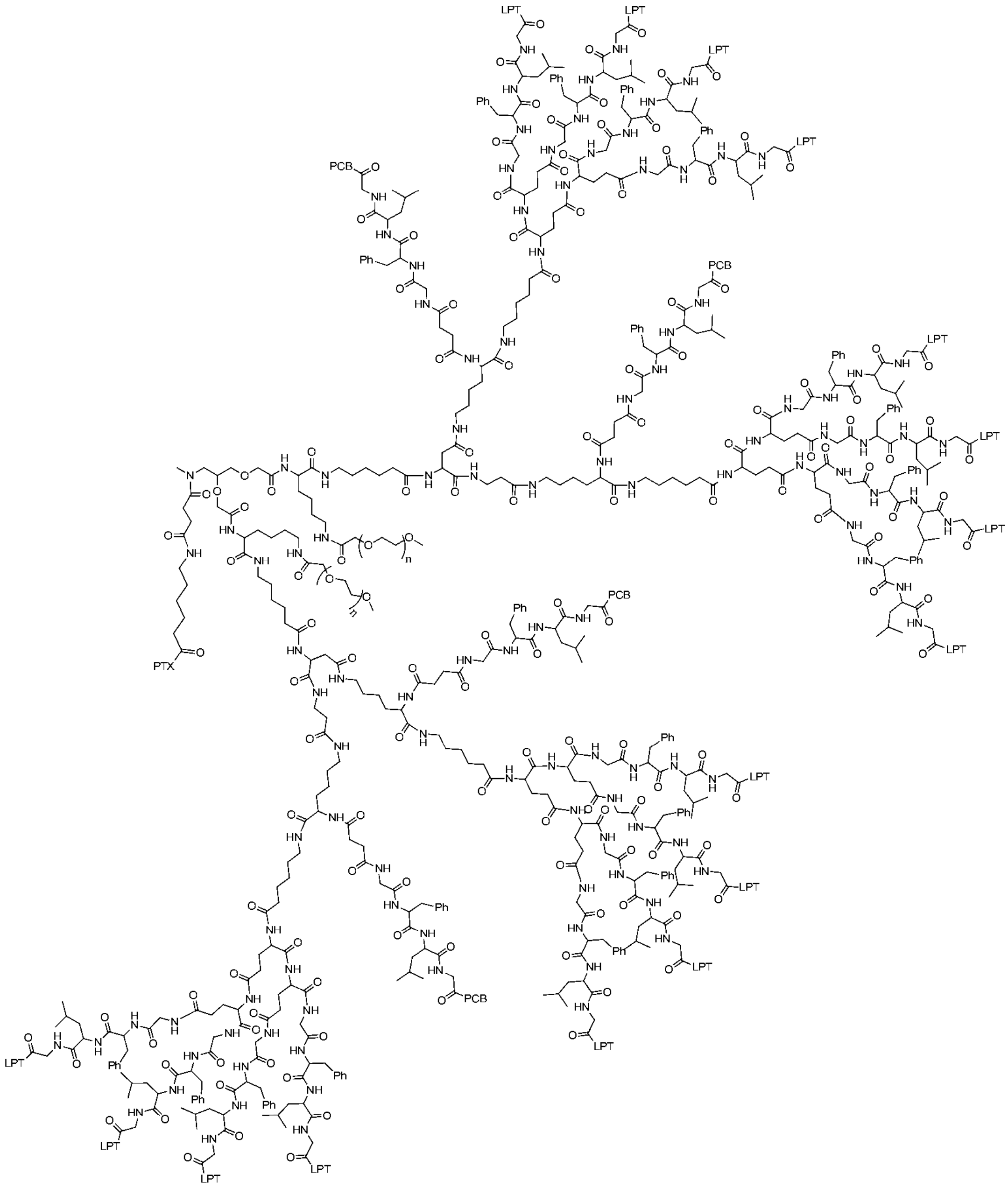 wherein, the number-average molecular weight of each polyethylene glycol fragment is 10k |

(continued)

| Compound No. | Compound structure |
|---|---|
| **76-258** | 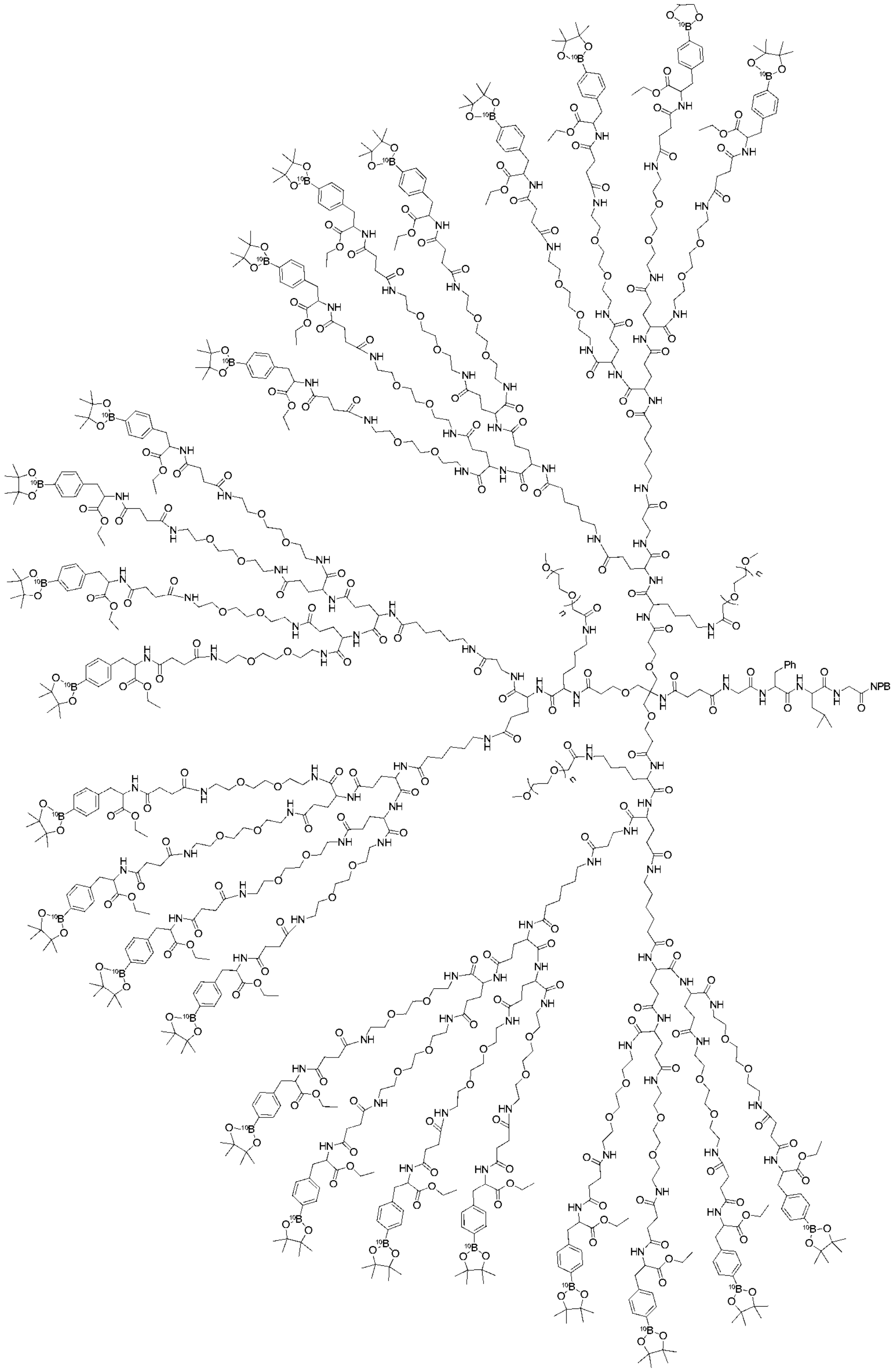<br>wherein, the number-average molecular weight of each polyethylene glycol fragment is 10k |

(continued)

| Compound No. | Compound structure |
|---|---|
| **88-206** | 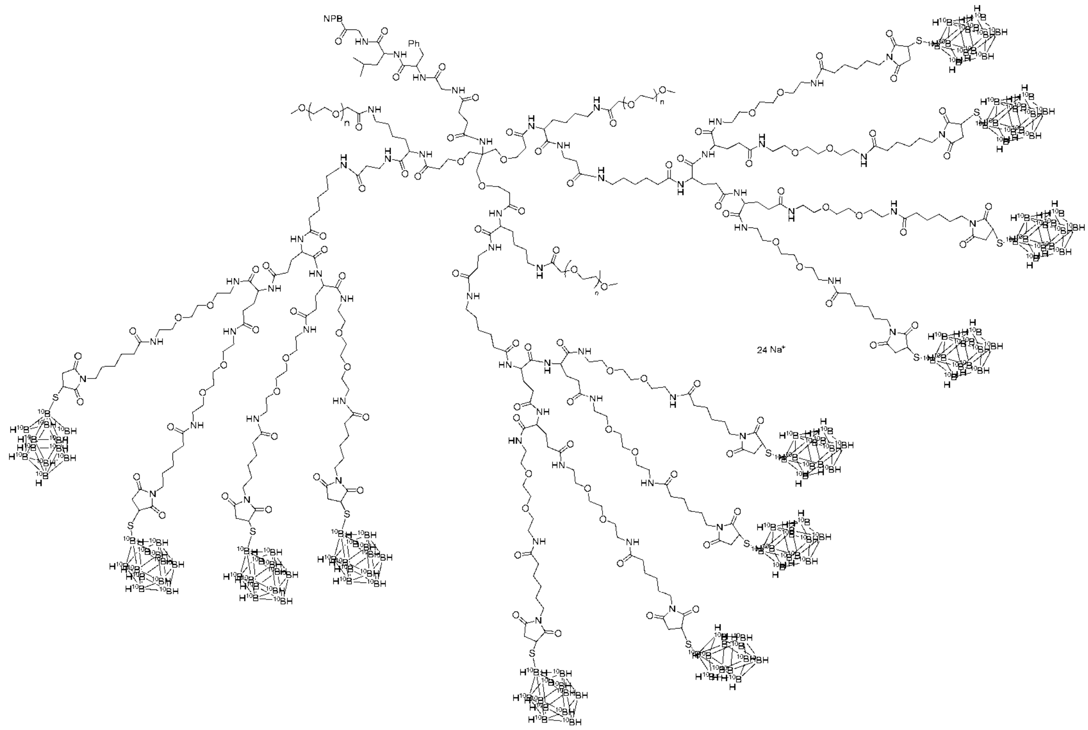 wherein, the number-average molecular weight of each polyethylene glycol fragment is 10k |

(continued)

| Compound No. | Compound structure |
| --- | --- |
| **78-131** | 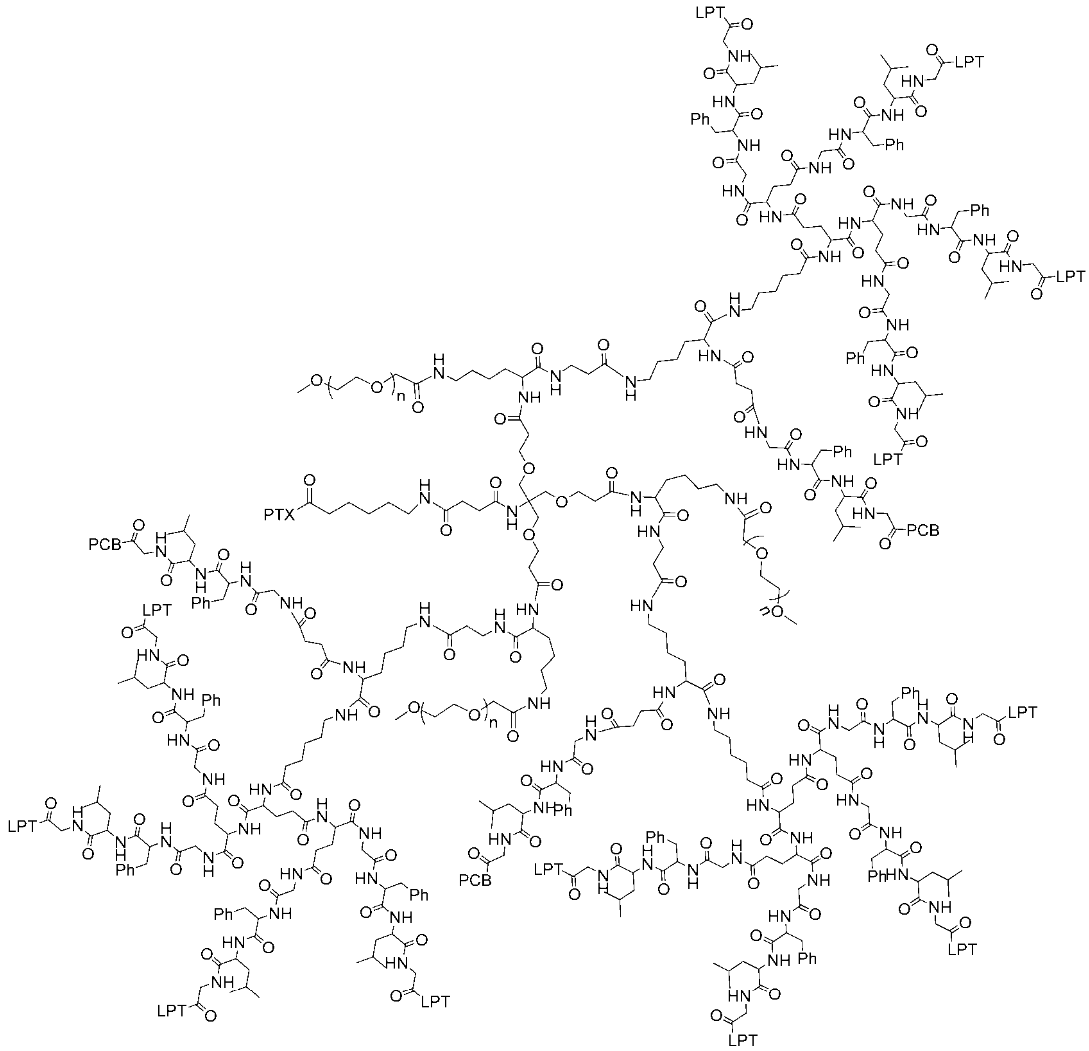 wherein, the number-average molecular weight of each polyethylene glycol fragment is 10k |

(continued)

| Compound No. | Compound structure |
|---|---|
| **72-188** | 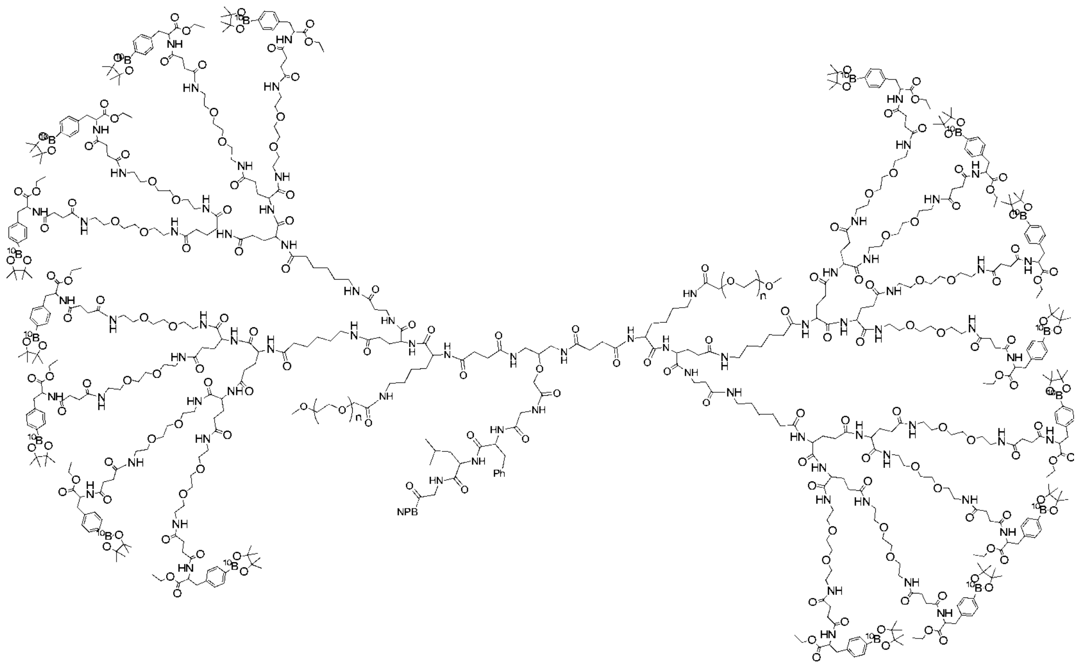 wherein, the number-average molecular weight of each polyethylene glycol fragment is 10k |

(continued)

| Compound No. | Compound structure |
|---|---|
| **70-261** | 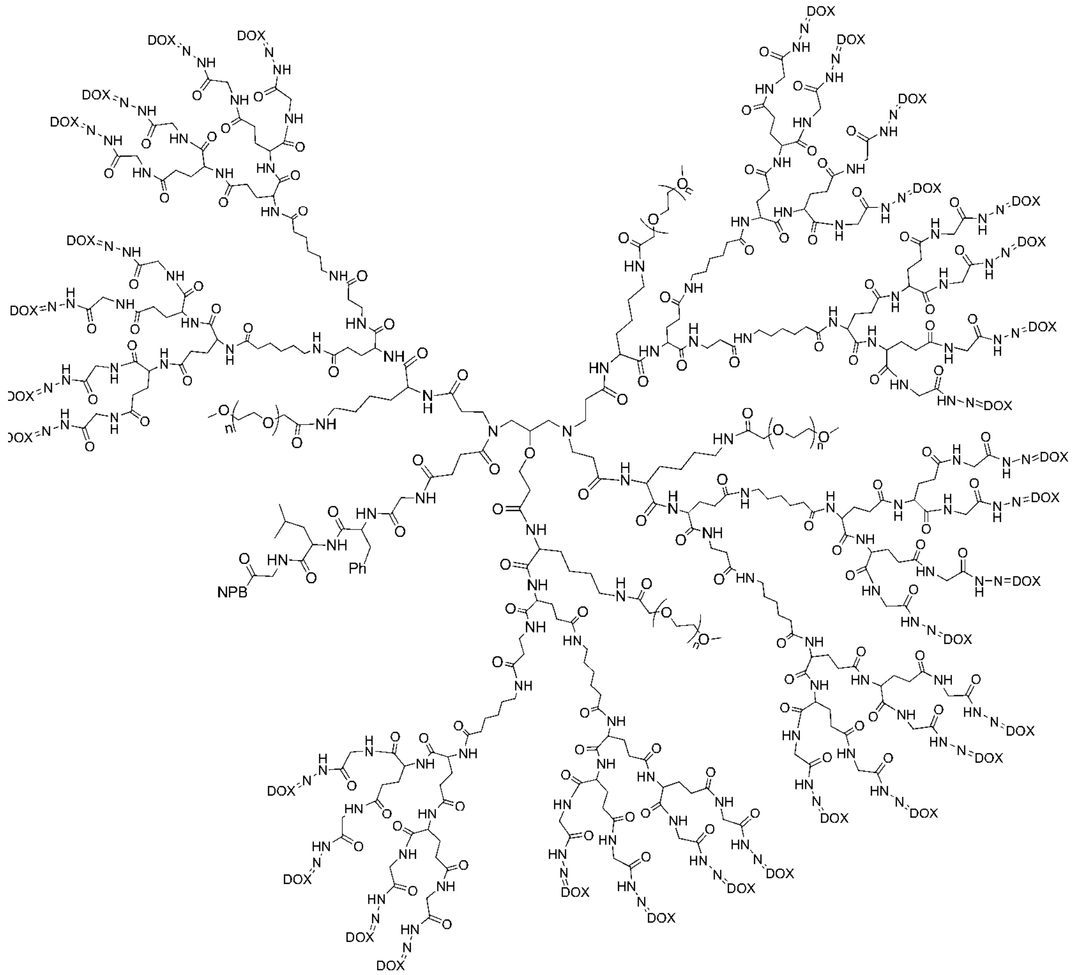 wherein, the number-average molecular weight of each polyethylene glycol fragment is 10k |
| **86-43** | 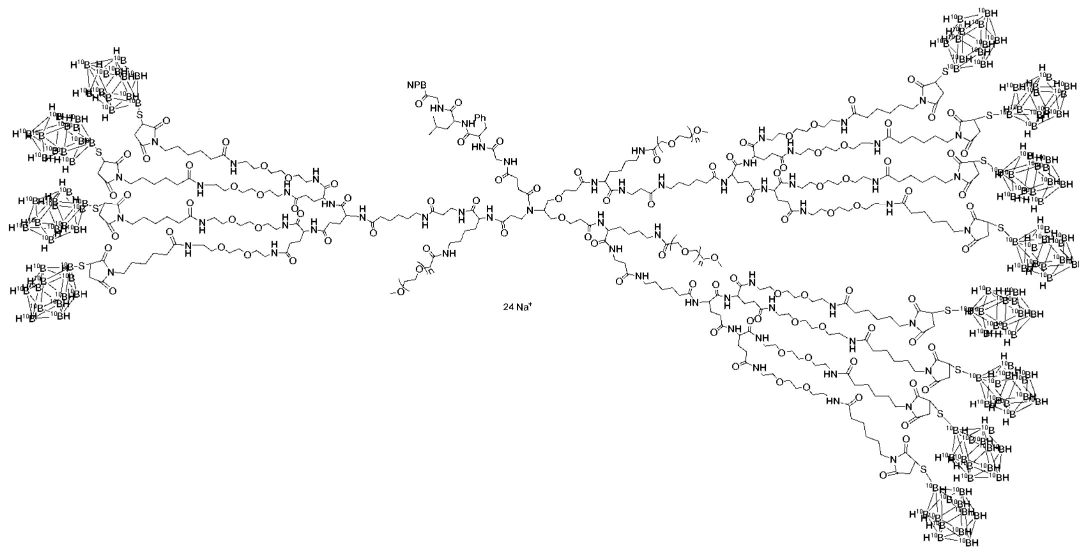 wherein, the number-average molecular weight of each polyethylene glycol fragment is 10k |

(continued)

| Compound No. | Compound structure |
|---|---|
| **87-35** | 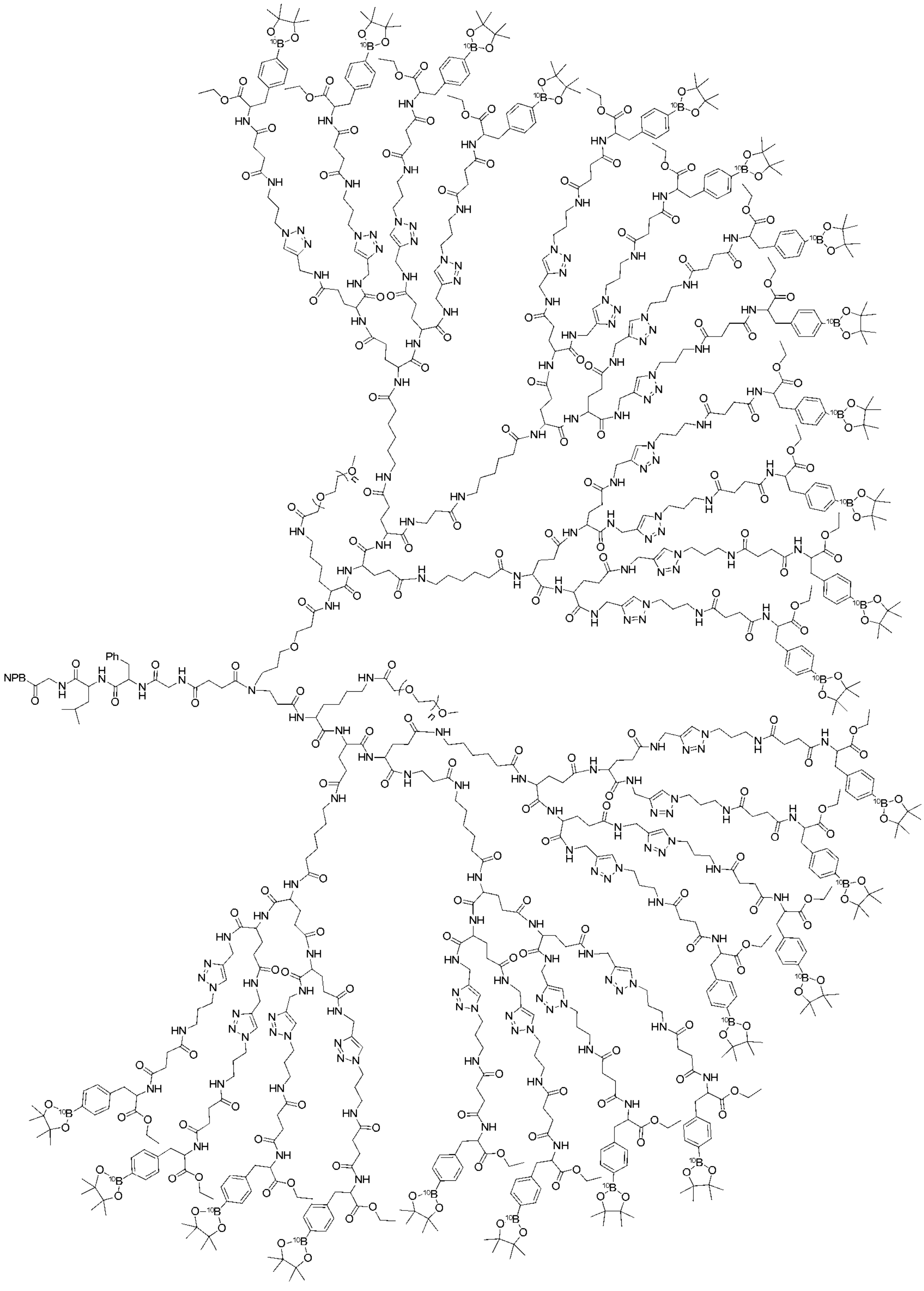 wherein, the number-average molecular weight of each polyethylene glycol fragment is 10k |

(continued)

| Compound No. | Compound structure |
|---|---|
| **75-236** | 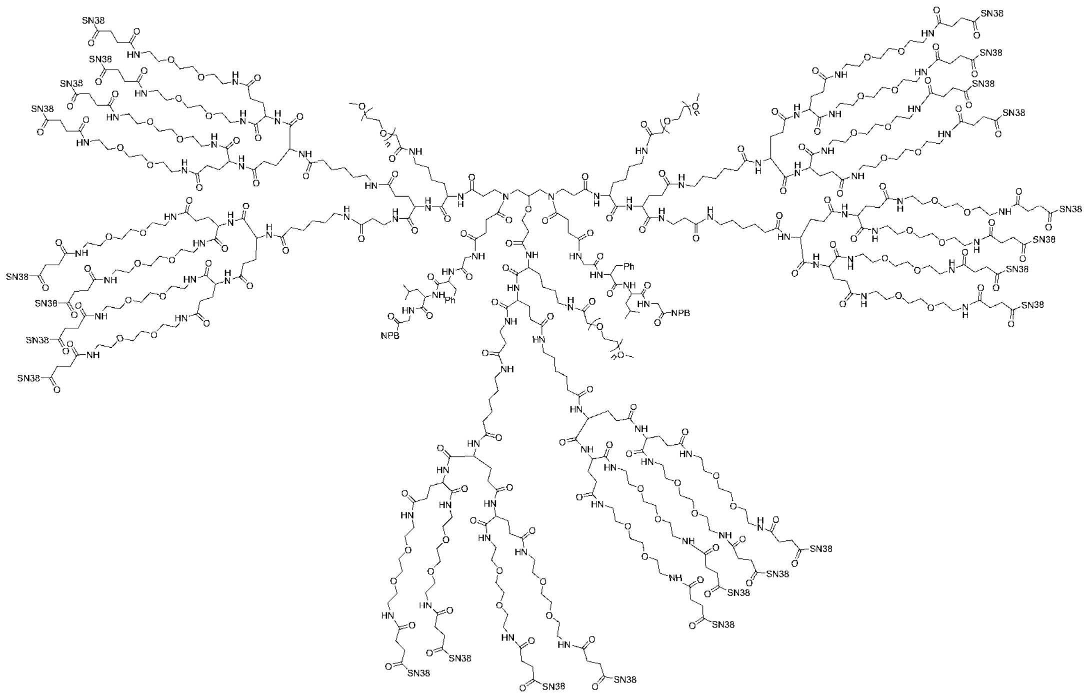 wherein, the number-average molecular weight of each polyethylene glycol fragment is 10k |
| **74-232** | 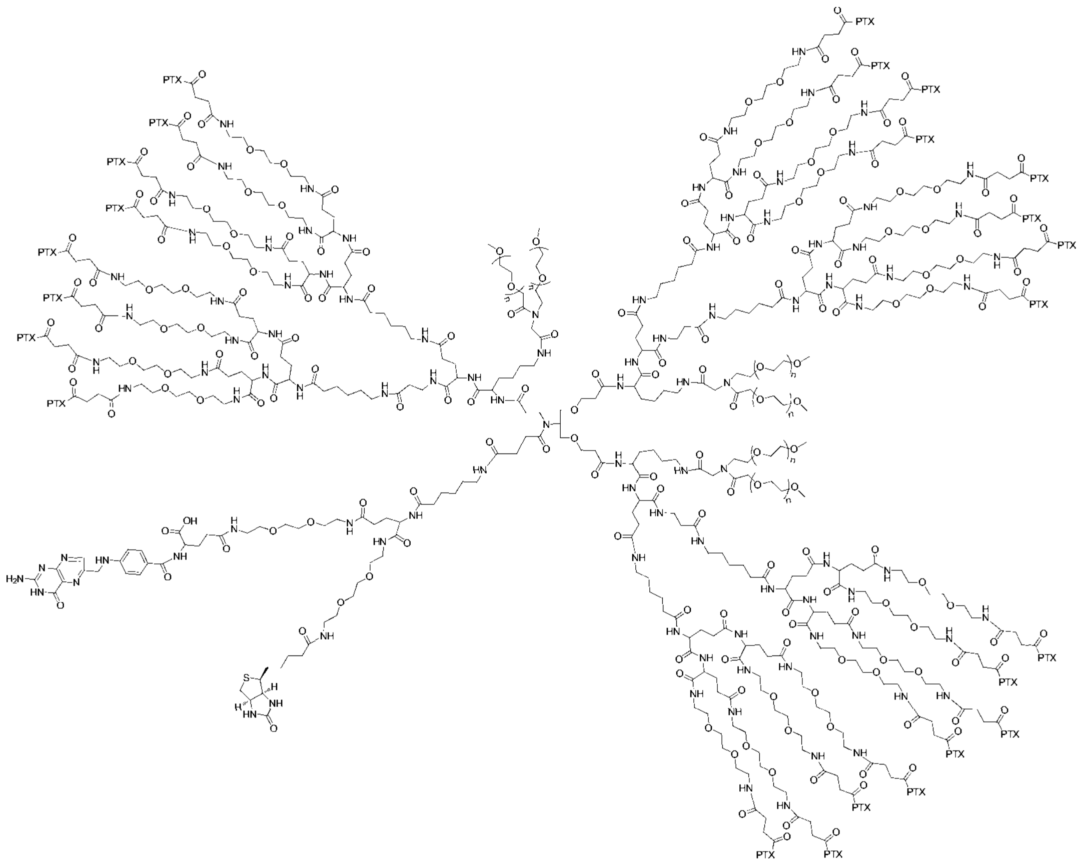 wherein, the number-average molecular weight of each polyethylene glycol fragment is 10k |

(continued)

| Compound No. | Compound structure |
|---|---|
| **85-97** | 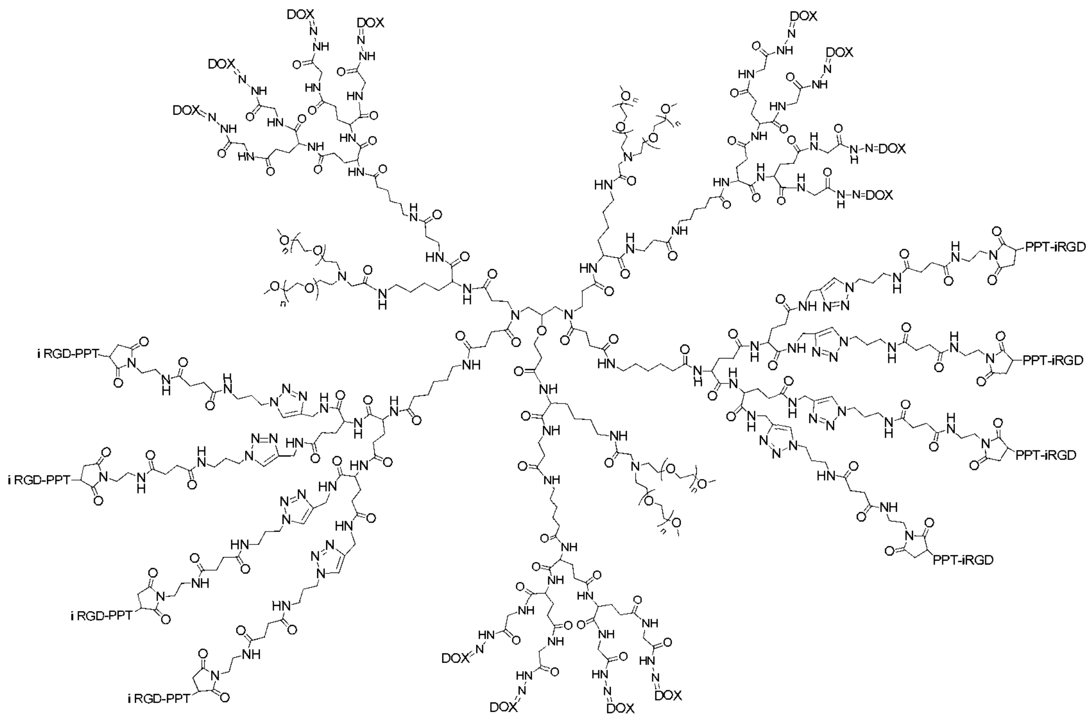 wherein, the number-average molecular weight of each polyethylene glycol fragment is 10k |

(continued)

| Compound No. | Compound structure |
|---|---|
| **85-26** | 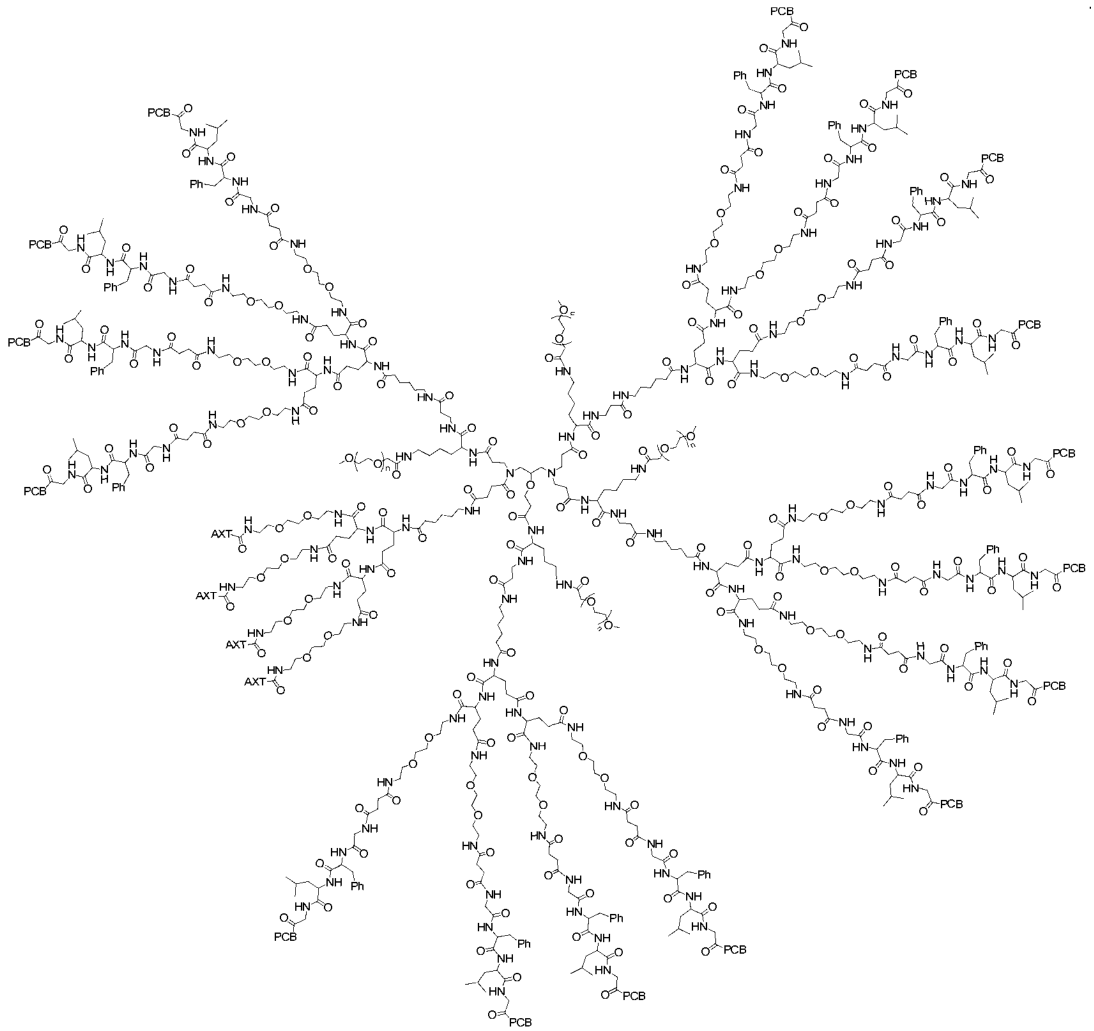 wherein, the number-average molecular weight of each polyethylene glycol fragment is 10k |

(continued)

| Compound No. | Compound structure |
|---|---|
| **75-509** | 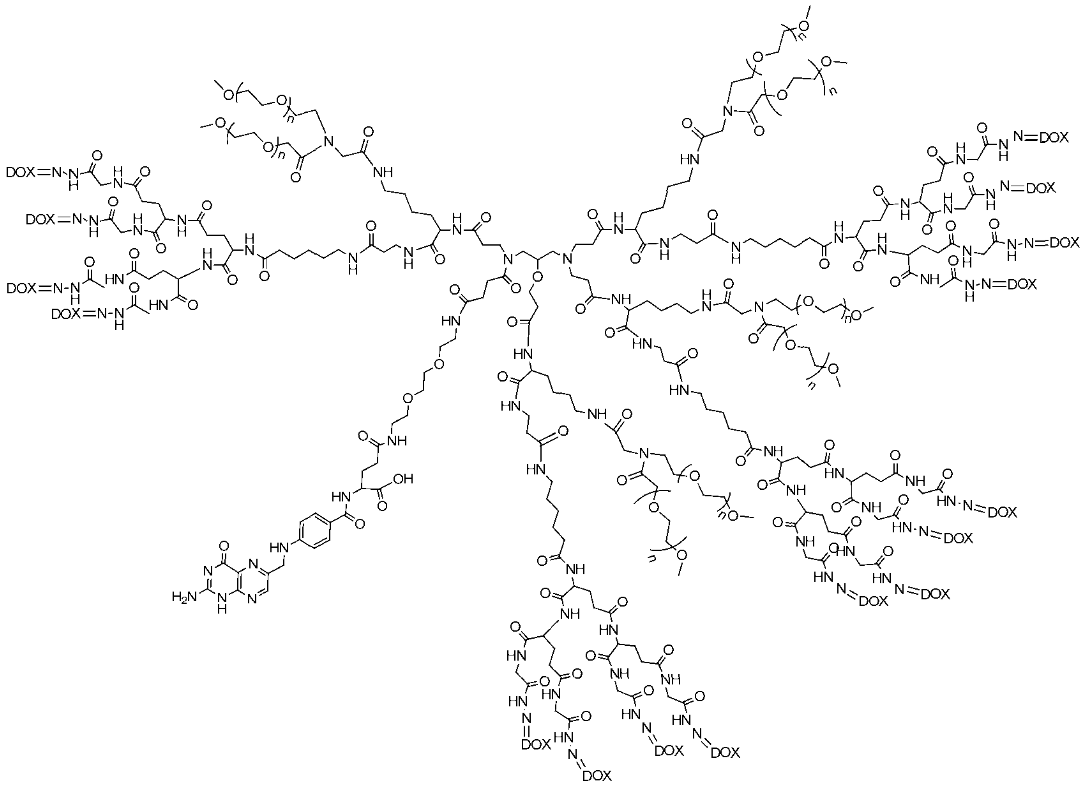<br>wherein, the number-average molecular weight of each polyethylene glycol fragment is 10k |
| **77-162** | 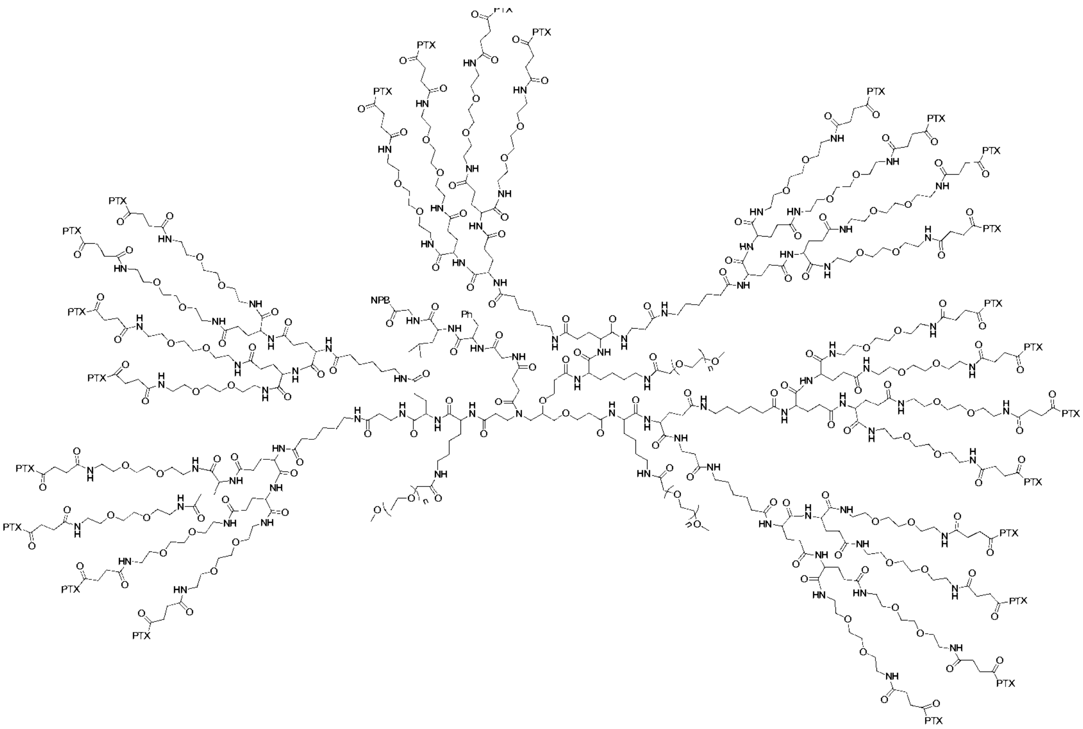<br>wherein, the number-average molecular weight of each polyethylene glycol fragment is 10k |

(continued)

| Compound No. | Compound structure |
| --- | --- |
| **81-231** | 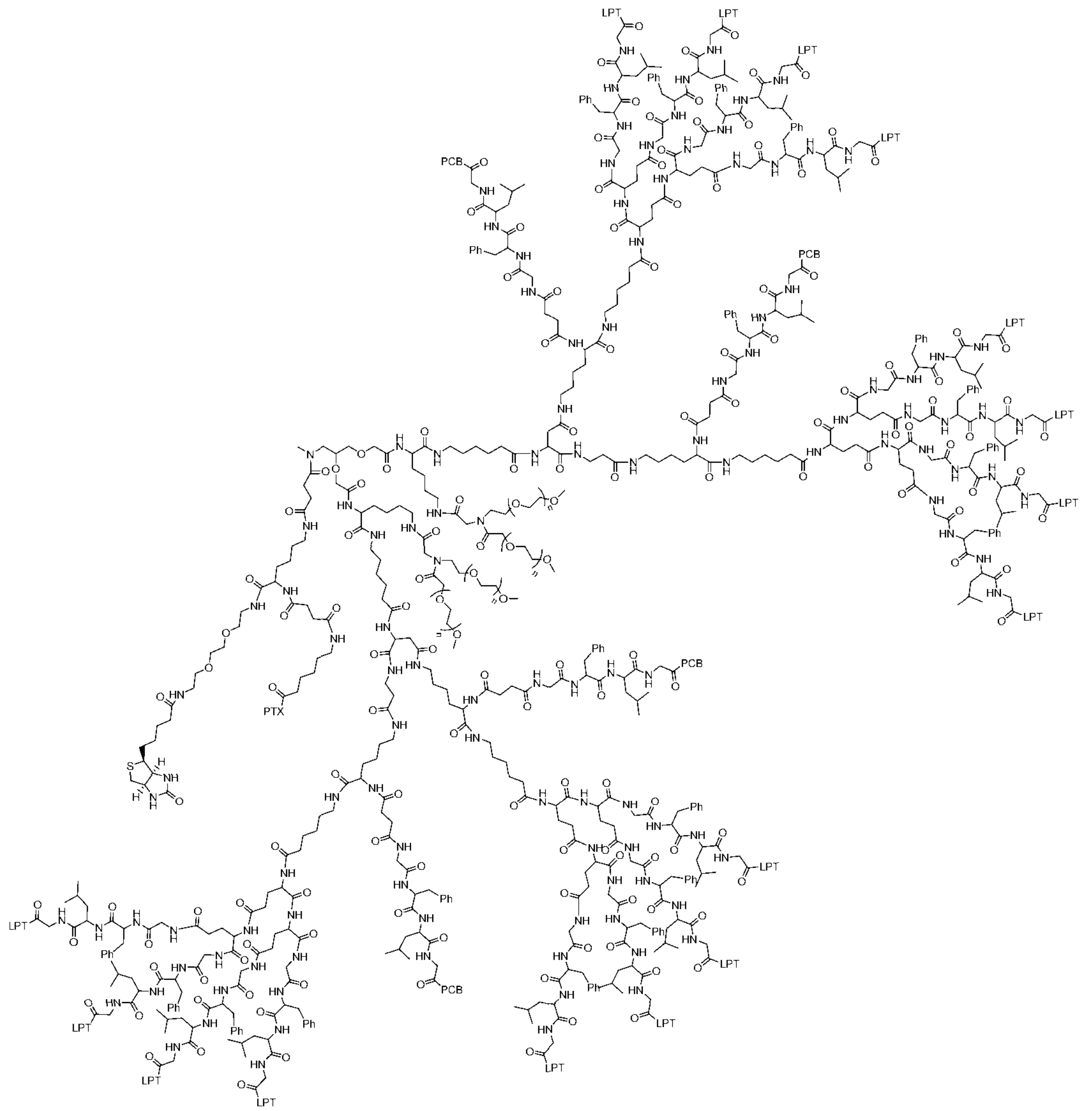 wherein, the number-average molecular weight of each polyethylene glycol fragment is 10k |

(continued)

| Compound No. | Compound structure |
|---|---|
| **84-102** | 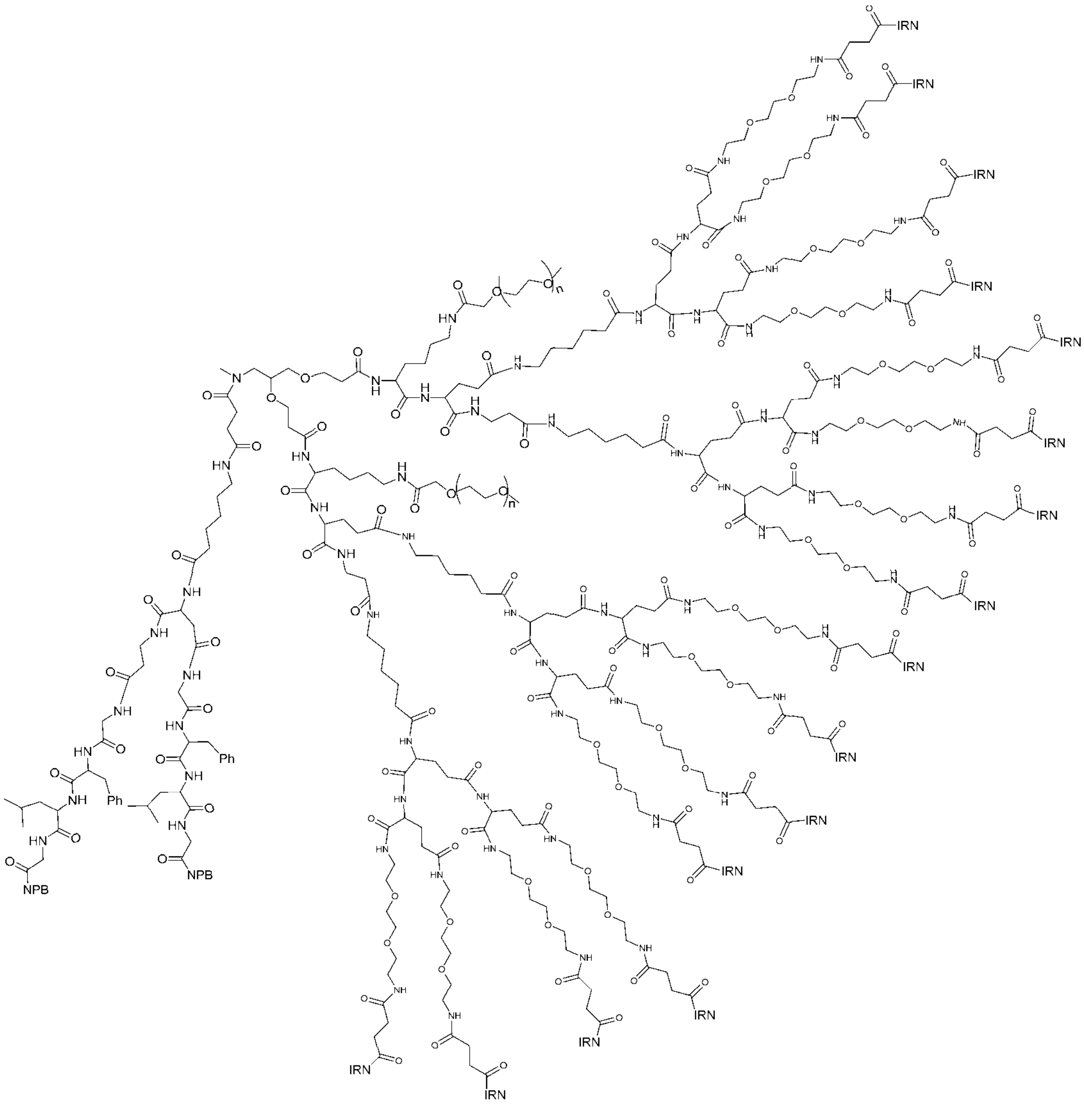 wherein, the number-average molecular weight of each polyethylene glycol fragment is 10k |

(continued)

| Compound No. | Compound structure |
|---|---|
| **88-228** | 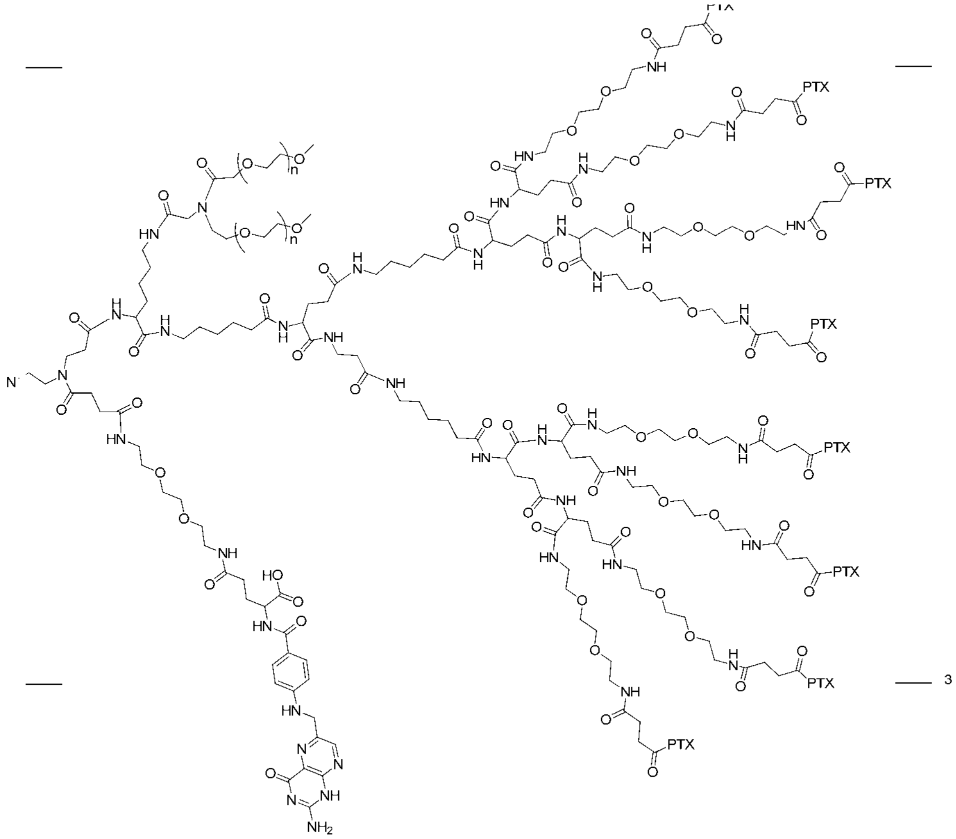 wherein, the number-average molecular weight of each polyethylene glycol fragment is 10k |

(continued)

| Compound No. | Compound structure |
|---|---|
| **76-179** | 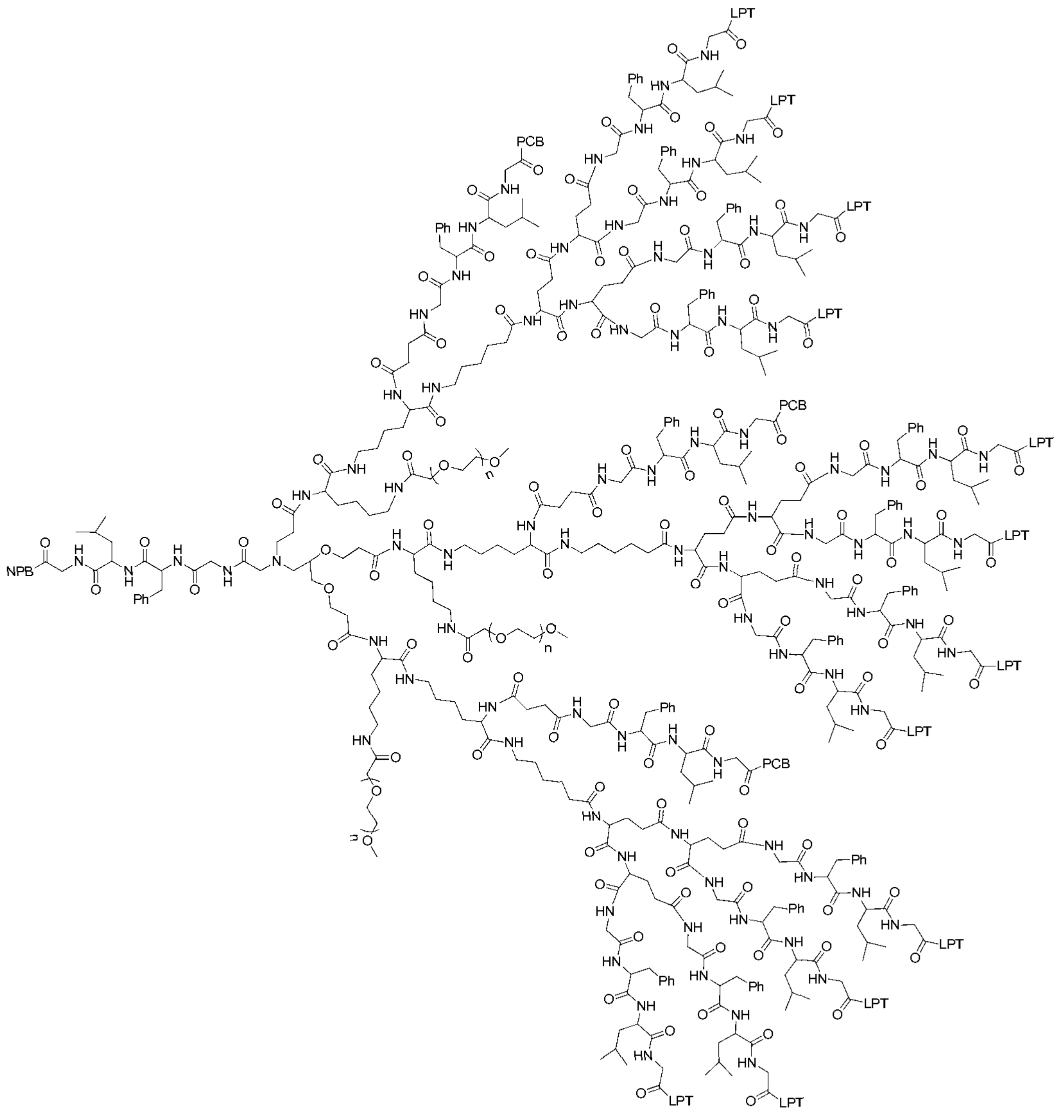<br>wherein, the number-average molecular weight of each polyethylene glycol fragment is 10k |

(continued)

| Compound No. | Compound structure |
|---|---|
| **87-47** | 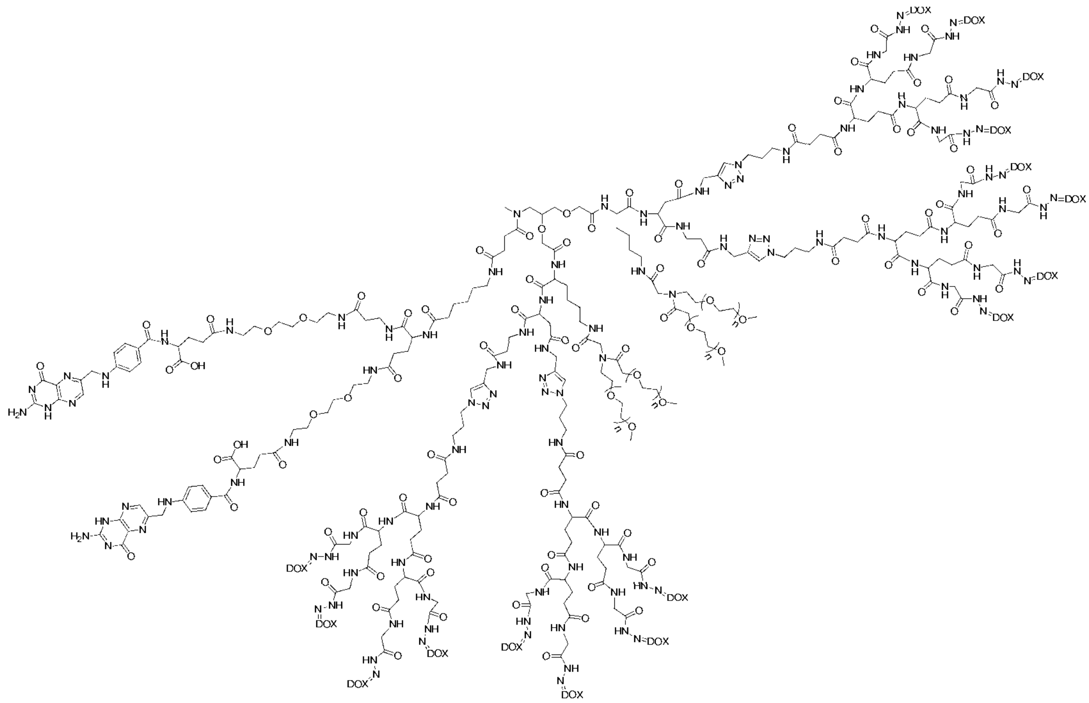 wherein, the number-average molecular weight of each polyethylene glycol fragment is 10k |

(continued)

| Compound No. | Compound structure |
|---|---|
| **72-248** | 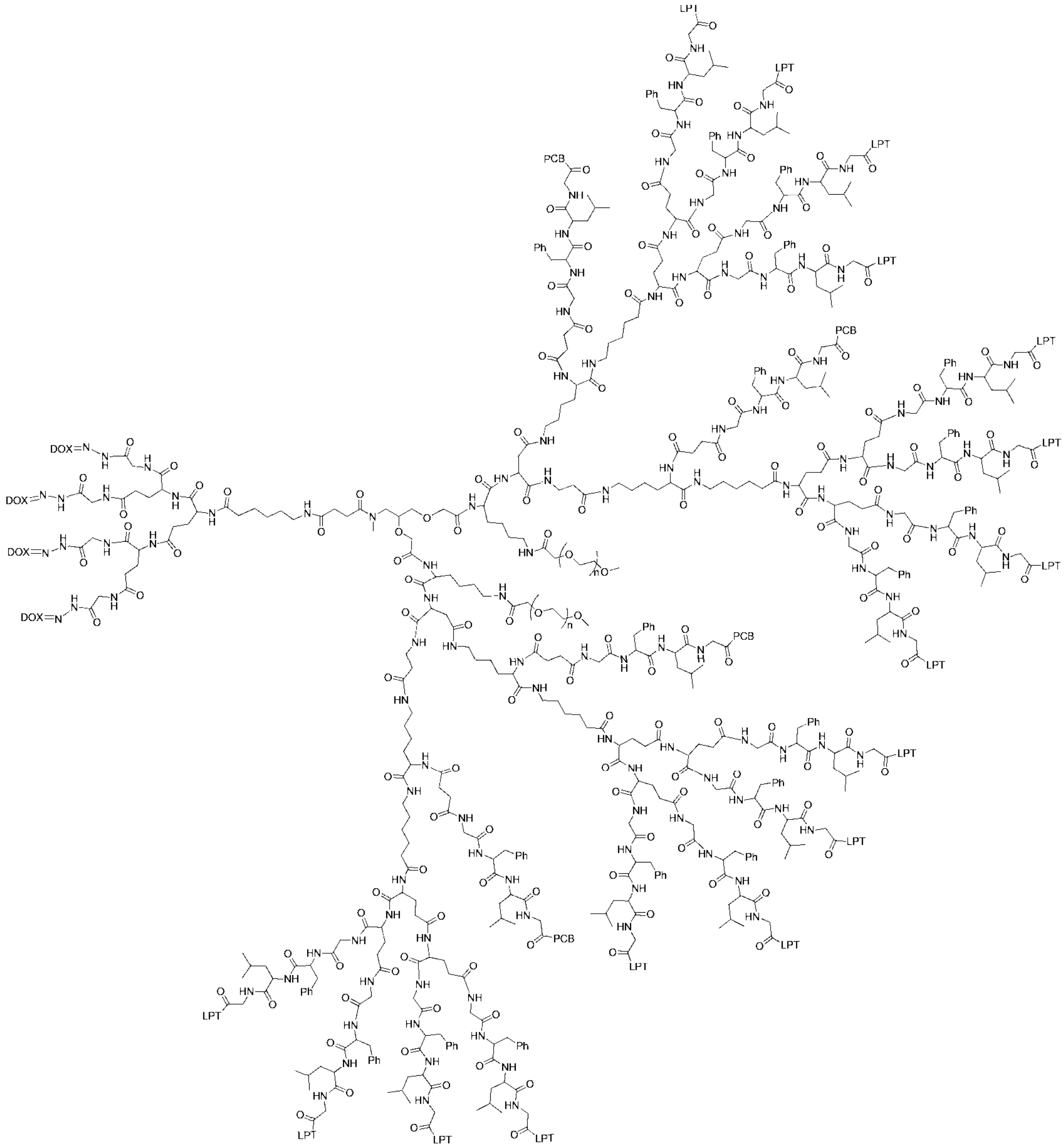 wherein, the number-average molecular weight of each polyethylene glycol fragment is 10k |

(continued)

| Compound No. | Compound structure |
| --- | --- |
| **72-279** | 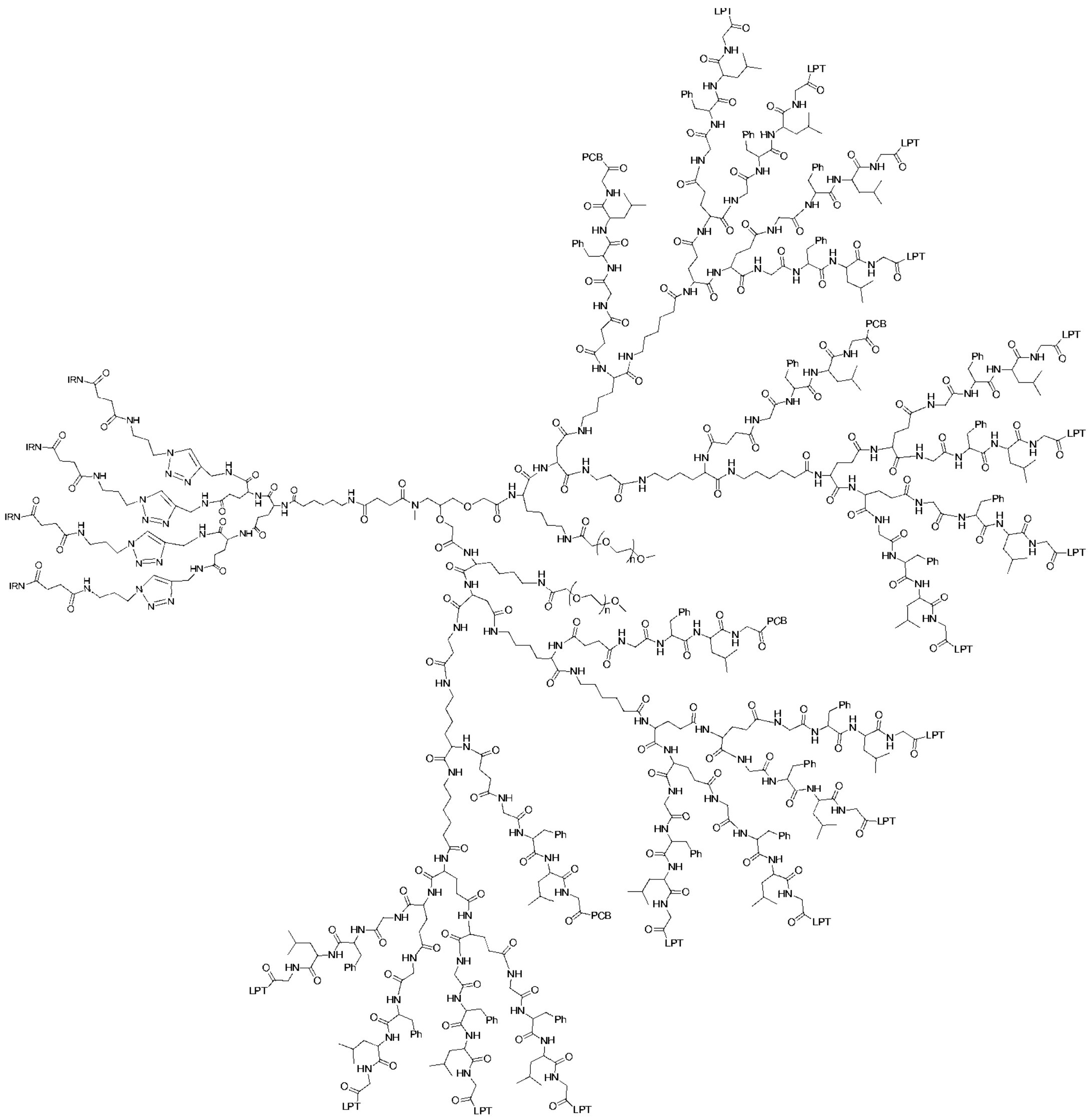 wherein, the number-average molecular weight of each polyethylene glycol fragment is 10k |

(continued)

| Compound No. | Compound structure |
|---|---|
| **87-40** | 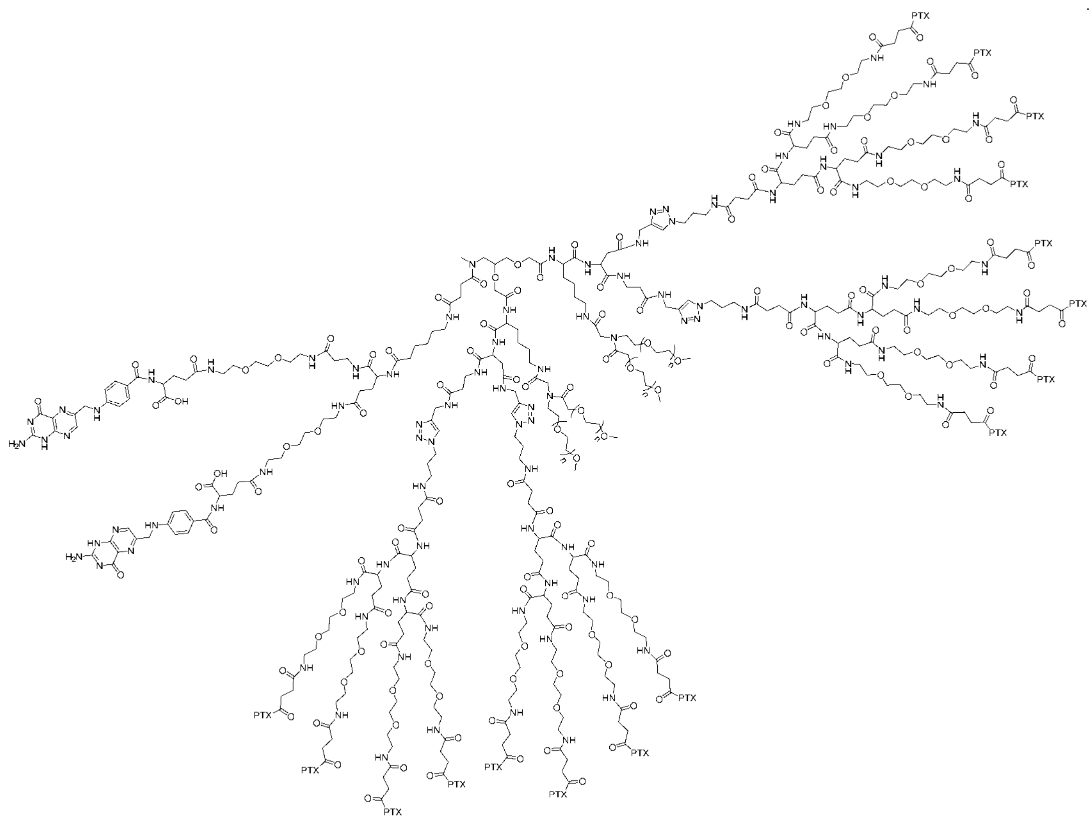<br>wherein, the number-average molecular weight of each polyethylene glycol fragment is 10k |
| **87-42** | 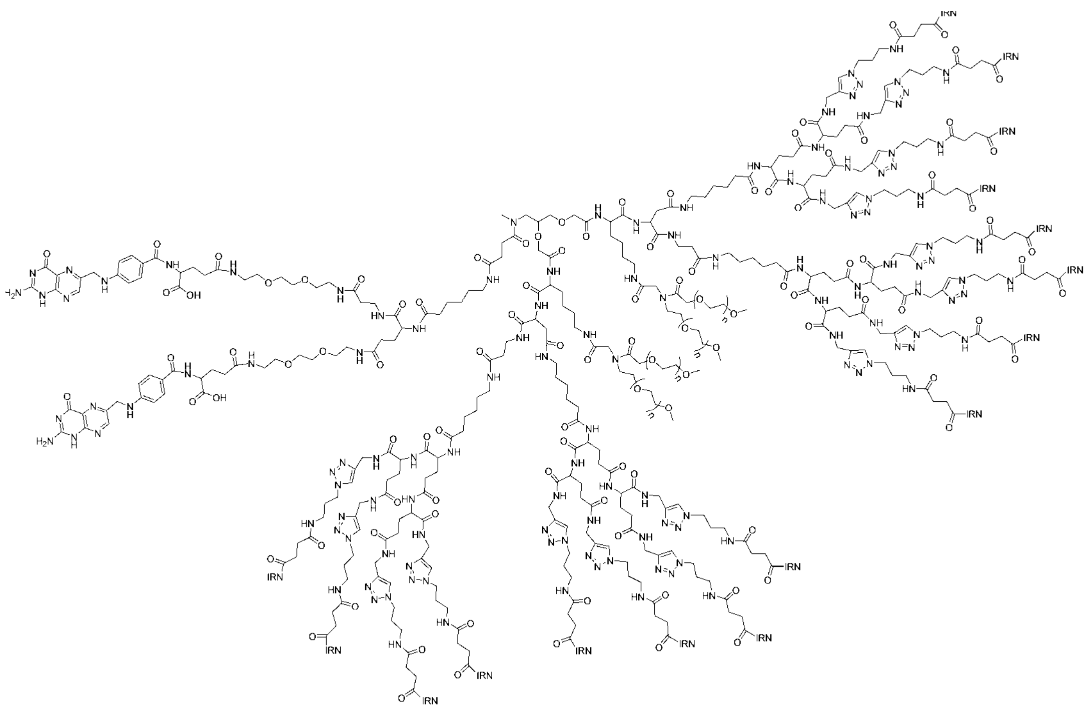<br>wherein, the number-average molecular weight of each polyethylene glycol fragment is 10k |

(continued)

| Compound No. | Compound structure |
|---|---|
| **85-63** | 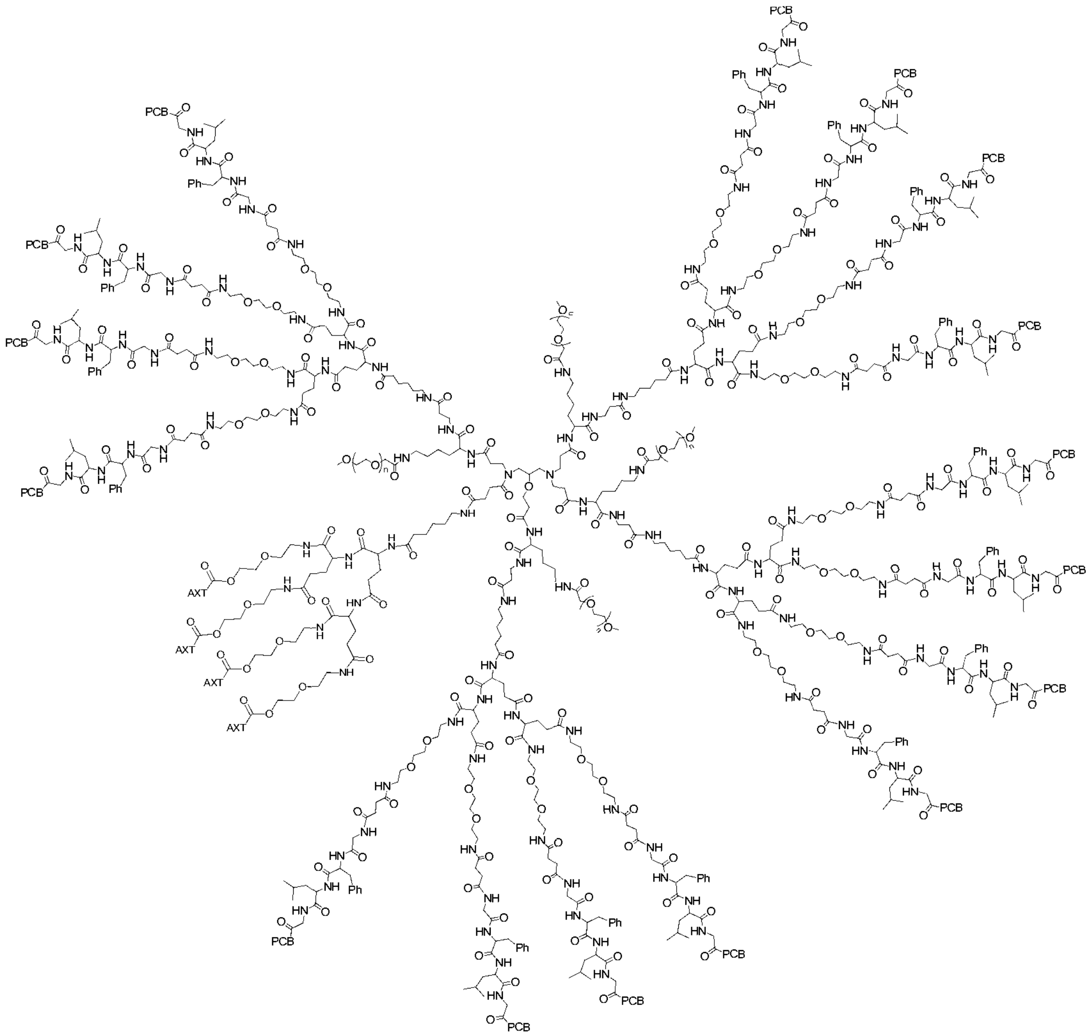 wherein, the number-average molecular weight of each polyethylene glycol fragment is 10k |

(continued)

| Compound No. | Compound structure |
|---|---|
| **88-233** | 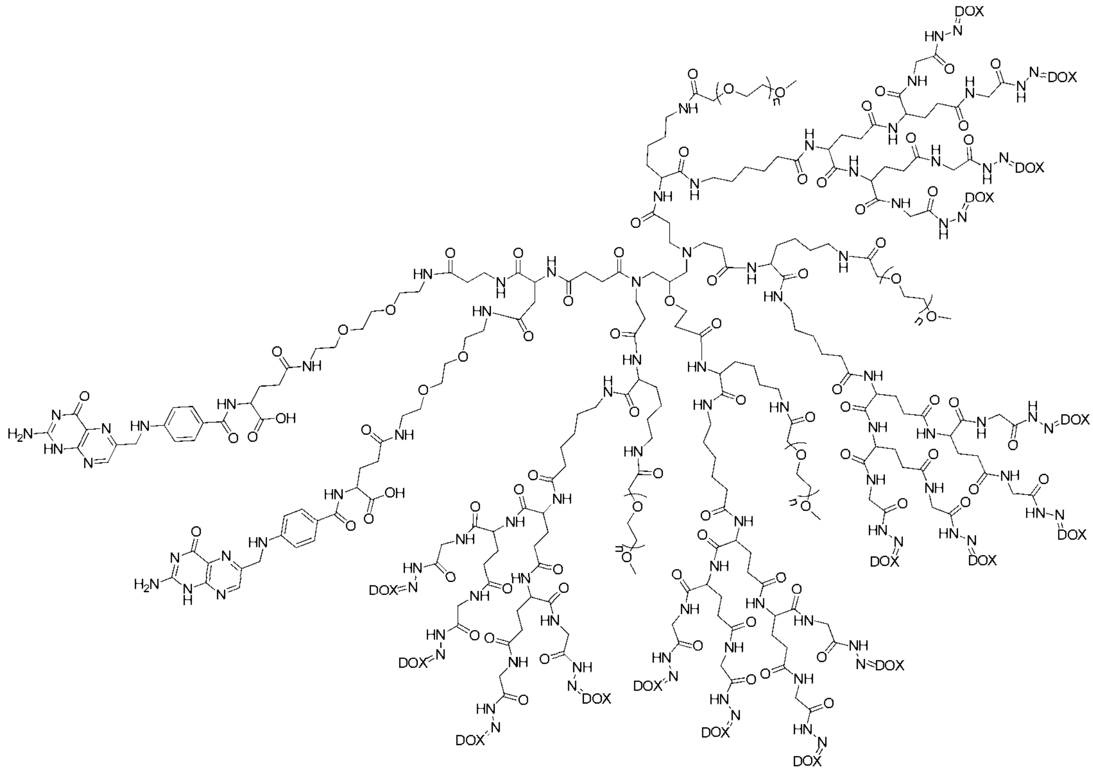 wherein, the number-average molecular weight of each polyethylene glycol fragment is 5k |

(continued)

| Compound No. | Compound structure |
|---|---|
| **82-220** | 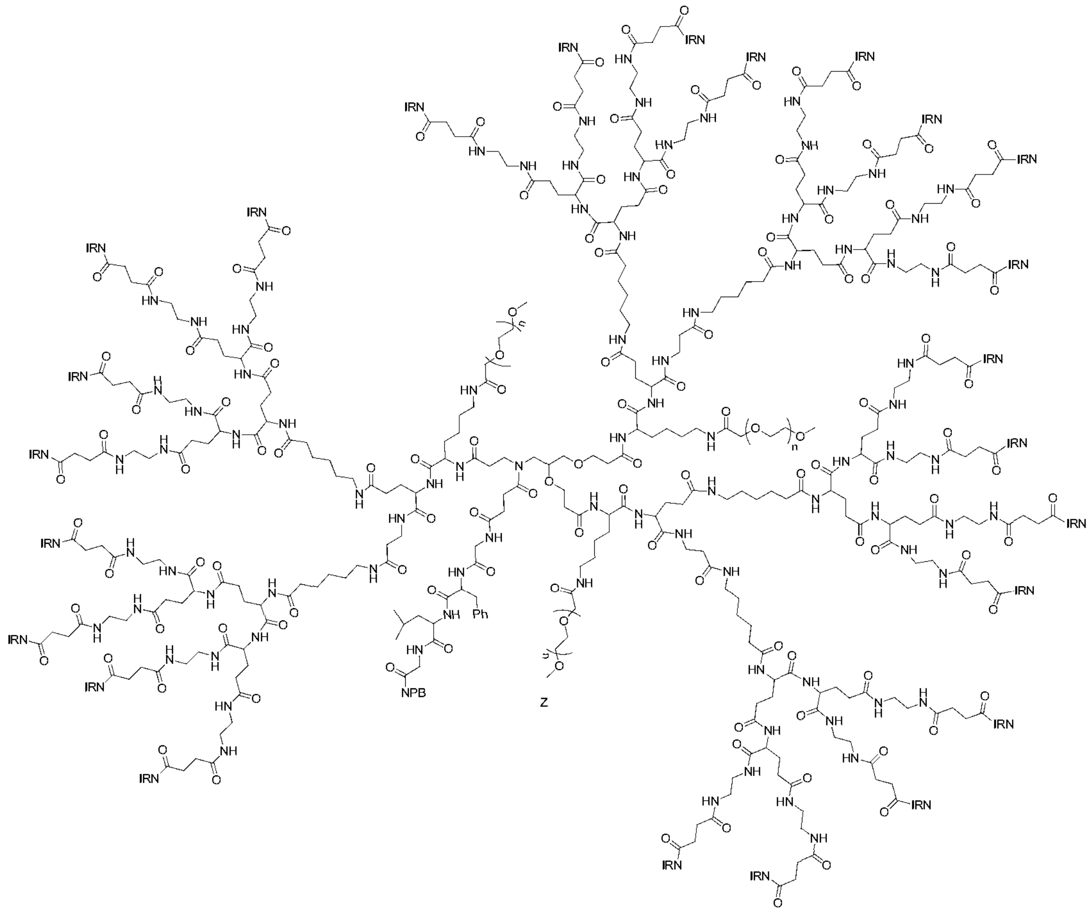 wherein, the number-average molecular weight of each polyethylene glycol fragment is 10k |

Intermediates

In the present application, dual drug (or triple drug) polyethylene glycol small-molecules are prepared through unique molecular design and protecting group chemistry. Therefore, the present application also provides intermediate compounds of formula II to formula XX for synthesizing the compounds described herein.

In one aspect, the present application provides a compound represented by formula II or a pharmaceutically acceptable salt thereof:

$$\left(Pg_1'\right)_{n_{11}} M \left(Pg_1\right)_{n_{12}}$$

Formula II

wherein, each of $Pg_1$ and $Pg_1$' is independently hydrogen or an amino protecting group, and $Pg_1$ and $Pg_1$' are different; preferably, the amino protecting group is selected from the group consisting of an alkyl-based protecting group (e.g., Bn, Trt, DMB, or PMB) and an alkoxycarbonyl-based protecting group (e.g., Boc, Fmoc, Cbz, or Teoc); preferably, $Pg_1$ is hydrogen and $Pg_1$' is an amino protecting group; alternatively, $Pg_1$ is an amino protecting group and $Pg_1$' is hydrogen;

the remaining groups are as defined in any of the preceding items.

In some embodiments, the compound is selected from the group consisting of:

| | | |
|---|---|---|
| **59-223** 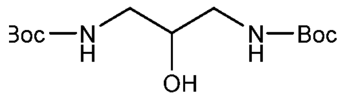 | **75-211** 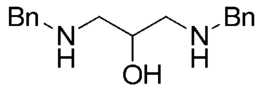 | **84-29** 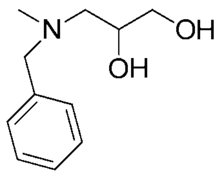 |
| **88-88** 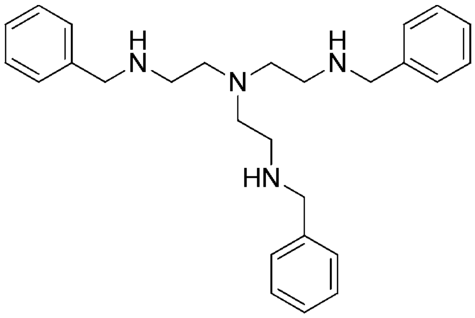 | **88-115** 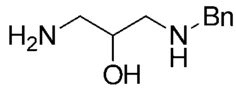 | |

In another aspect, the present application provides a compound represented by formula 111 or a pharmaceutically acceptable salt thereof:

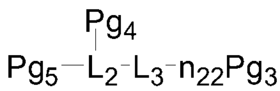

Formula III

wherein, each $Pg_3$ is independently hydrogen or selected from the group consisting of an amino protecting group and a carboxyl protecting group, the amino protecting group is selected from the group consisting of an alkoxycarbonyl-based protecting group, such as Boc, Fmoc, Cbz, or Teoc; the carboxyl protecting group is selected from the group consisting of an ester-based protecting group, such as methyl ester, ethyl ester, tert-butyl ester, allyl ester, or benzyl ester; preferably, each $Pg_3$ is independently hydrogen or a carboxyl protecting group, such as an ester-based protecting group, such as methyl ester, ethyl ester, tert-butyl ester, allyl ester, or benzyl ester, preferably tert-butyl ester or benzyl ester; preferably, each $Pg_3$ is the same, preferably all are tert-butyl ester or benzyl ester;

$Pg_4$ is hydrogen or a protecting group of the side chain of $L_2$, preferably, the protecting group is selected from the group consisting of an amino protecting group and a carboxyl protecting group, preferably, the amino protecting group is selected from the group consisting of an alkoxycarbonyl-based protecting group, such as Boc, Fmoc, Cbz, or Teoc, preferably, the carboxyl protecting group is selected from the group consisting of an ester-based protecting group, such as methyl ester, ethyl ester, tert-butyl ester, allyl ester, or benzyl ester, preferably, $Pg_4$ is an amino protecting group, preferably, $Pg_4$ is Boc or Cbz;

$Pg_5$ is hydrogen or an amino protecting group, the amino protecting group is selected from the group consisting of an alkoxycarbonyl-based protecting group, such as Boc, Fmoc, Cbz, or Teoc; preferably, $Pg_5$ is hydrogen or Fmoc; the remaining groups are as defined in any of the preceding items.

In some embodiments, the compound is selected from the group consisting of:

| | |
|---|---|
| **74-102**<br>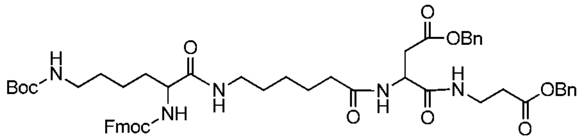 | **74-103**<br>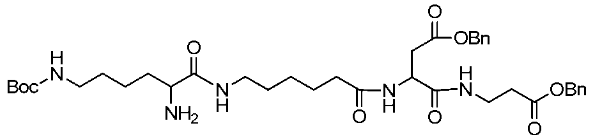 |
| **76-56**<br>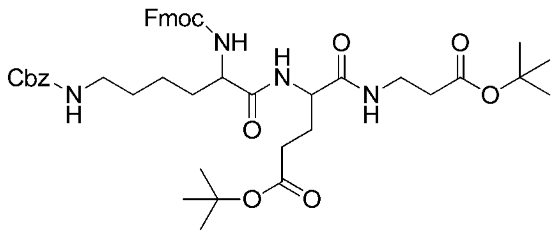 | **76-58**<br>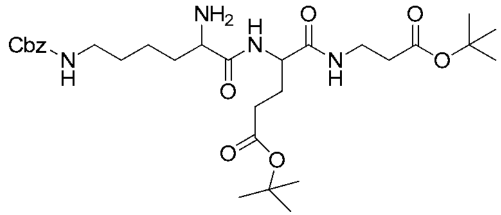 |
| **88-85**<br>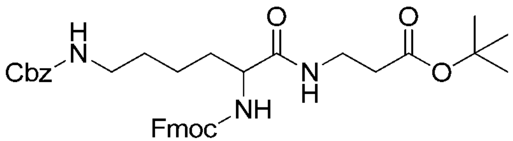 | **88-86**<br>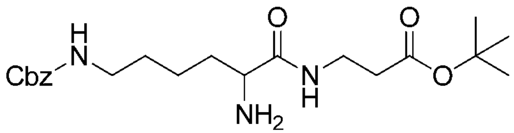 |
| **80-180**<br>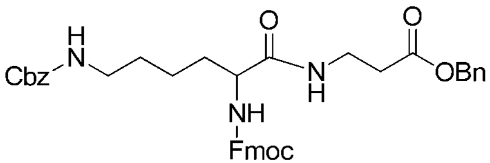 | **80-185**<br>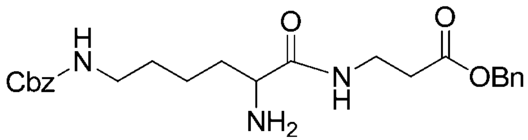 |
| **76-115**<br>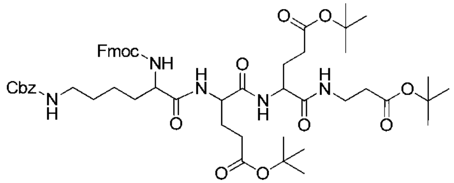 | **76-116**<br>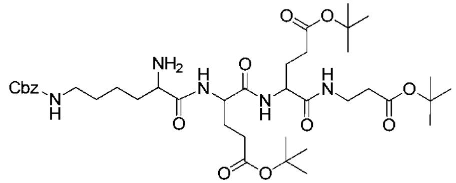 |
| **88-99** | **88-100** |

(continued)

| | |
|---|---|
| 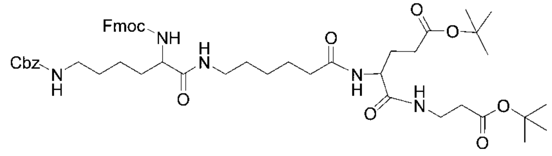 | 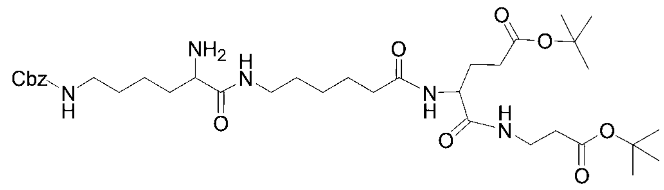 |
| 72-197 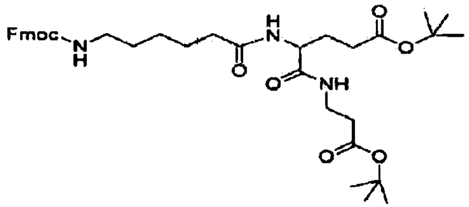 | 72-216 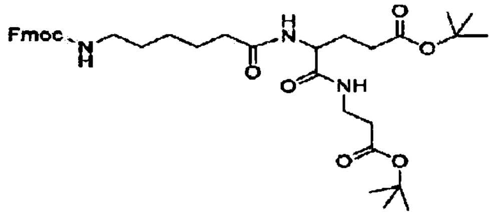 |
| 82-189 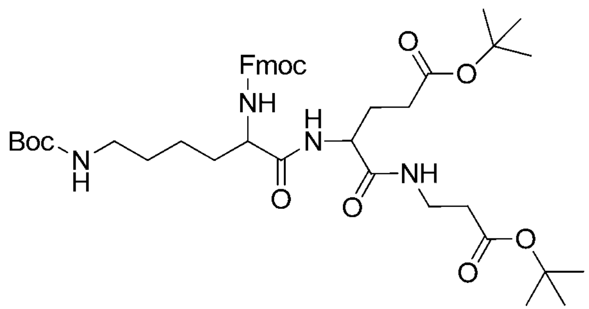 | 82-193 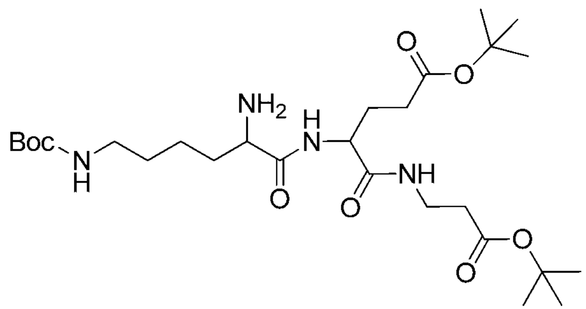 |

In another aspect, the present application provides a compound represented by formula IV or a pharmaceutically acceptable salt thereof,

$$Pg_6-L_4\left(L_5-y_2Pg_7\right)_{n_{32}}$$

formula IV

wherein, each of $Pg_6$ and $Pg_7$ is independently hydrogen or an amino protecting group, preferably, the amino protecting group is selected from the group consisting of an alkyl-based protecting group (e.g., Bn, Trt, DMB, or PMB) and alkoxycarbonyl-based protecting group (e.g., Boc, Fmoc, Cbz, or Teoc);

the remaining groups are as defined in any of the preceding items.

In another aspect, the present application provides a compound represented by formula V or a pharmaceutically acceptable salt thereof,

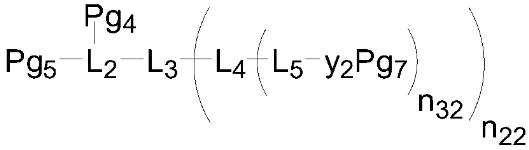

formula V

wherein, each group is as defined in any of the preceding items.

In some embodiments, the compound is selected from the group consisting of:

| | |
|---|---|
| **88-162** | 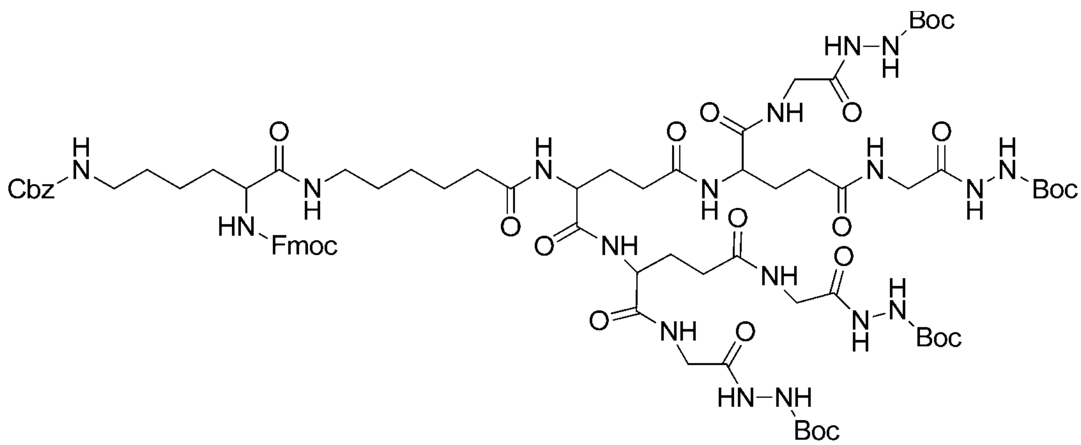 |
| **88-164** | 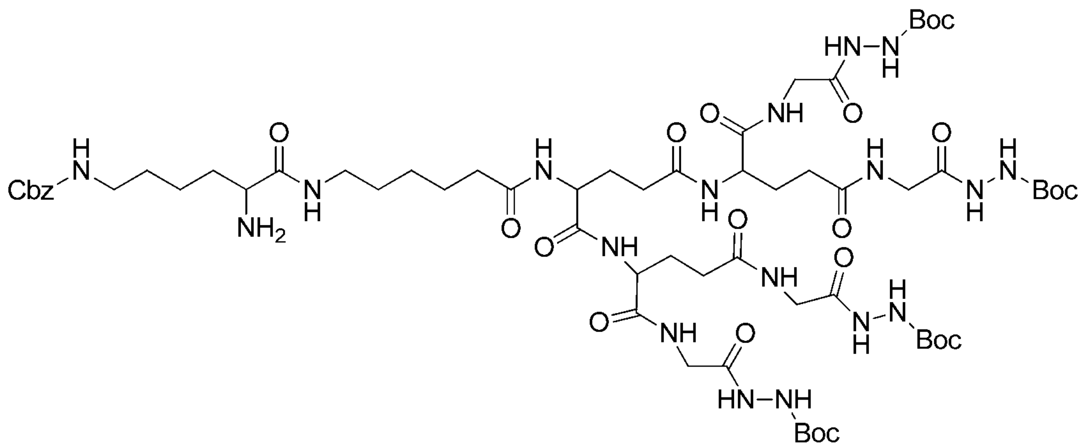 |

In another aspect, the present application provides a compound represented by formula VI or a pharmaceutically acceptable salt thereof:

$$Pg_6-L_4\left(L_5-y_2D\right)_{n_{32}}$$

VI

wherein, each group is as defined in any of the preceding items.

In another aspect, the present application provides a compound represented by formula VII or a pharmaceutically acceptable salt thereof,

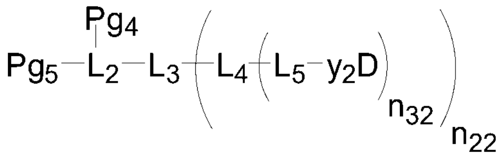

VII

wherein, each group is as defined in any of the preceding items.

In some embodiments, the compound is selected from the group consisting of:

| | |
|---|---|
| **76-168** | 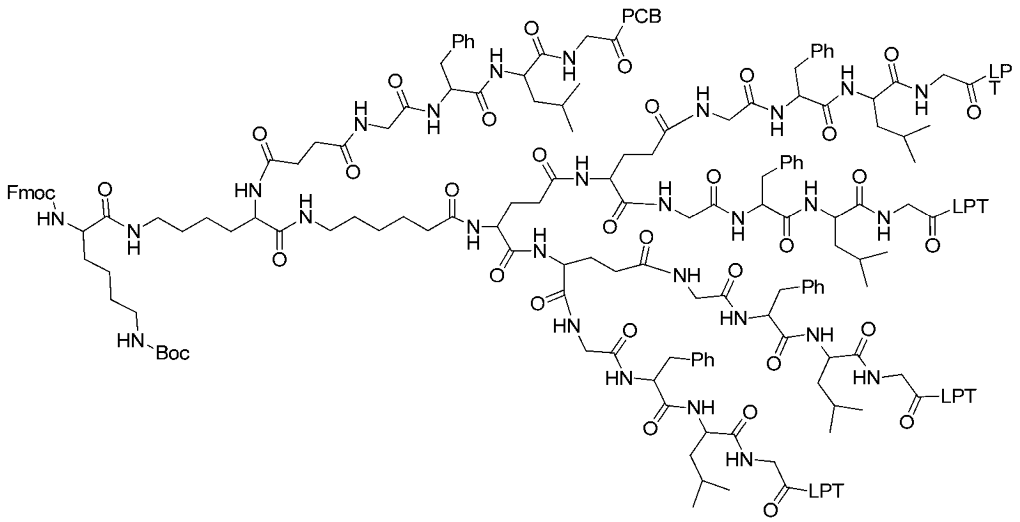 |

(continued)

| | |
|---|---|
| **76-169** | 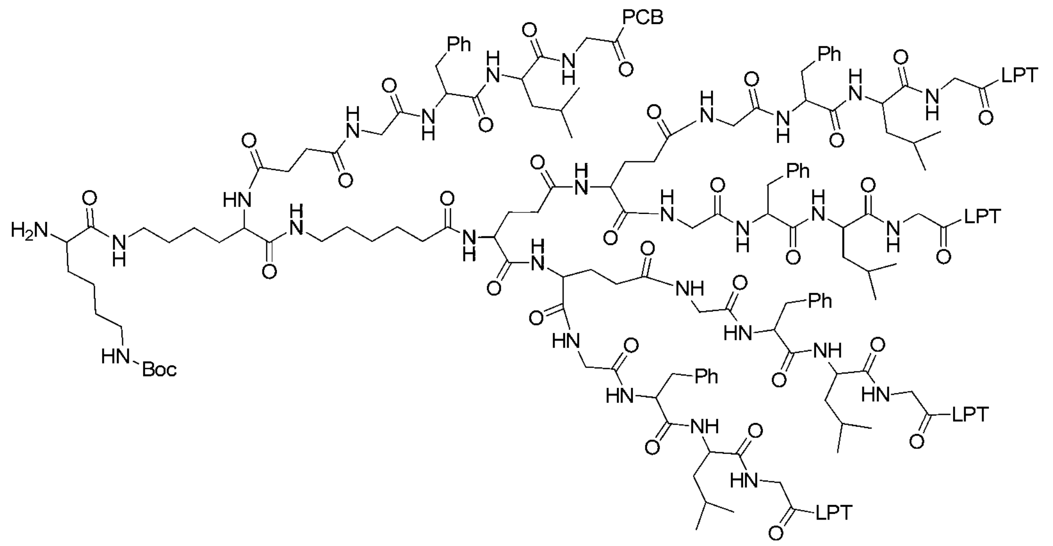 |

In another aspect, the present application provides a compound represented by formula VIII or a pharmaceutically acceptable salt thereof:

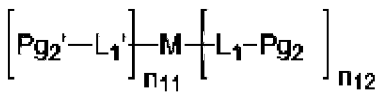

VIII

wherein, each of $Pg_2$ and $Pg_2$' is independently hydrogen or a carboxyl protecting group; preferably, the carboxyl protecting group is selected from the group consisting of an ester-based protecting group, such as methyl ester, ethyl ester, tert-butyl ester, allyl ester, or benzyl ester;

preferably, $Pg_2$ is hydrogen and $Pg_2$' is a carboxyl protecting group; $Pg_2$ is a carboxyl protecting group and $Pg_2$' is hydrogen; or, both $Pg_2$ and $Pg_2$' are carboxyl protecting groups (e.g., tert-butyl ester or benzyl ester), and $Pg_2$ and $Pg_2$' are different;

the remaining groups are as defined in any of the preceding items.

In some embodiments, the compound is selected from the group consisting of:

| | |
|---|---|
| **81-65** 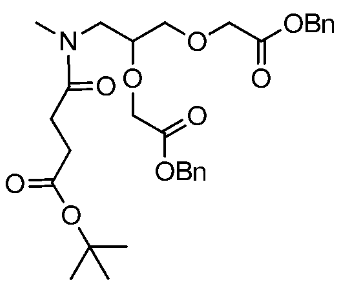 | **81-82** 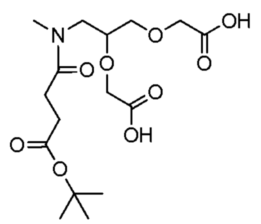 |
| **76-88** | **76-89** |

(continued)

| | |
|---|---|
| 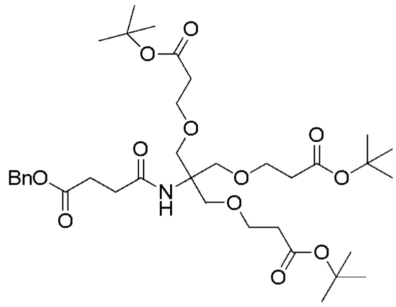 | 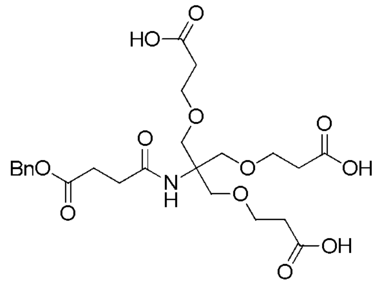 |
| **80-183** 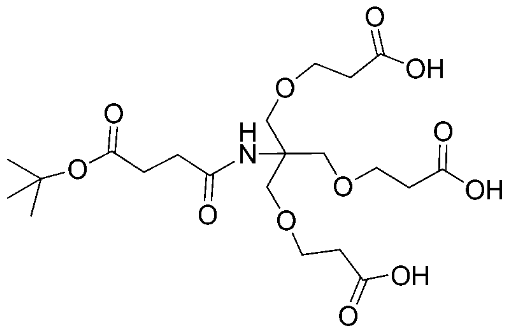 | **59-231** 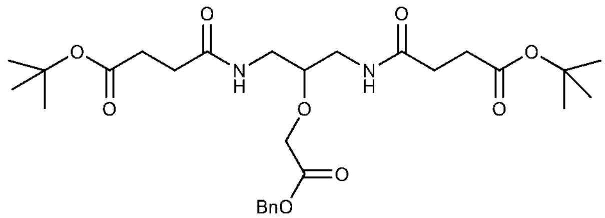 |
| **72-129** 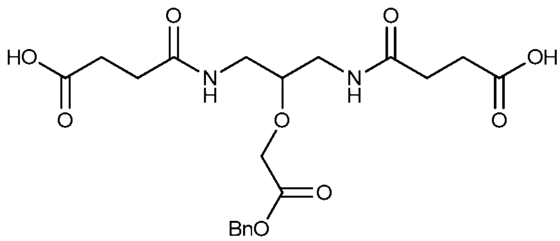 | **70-185** 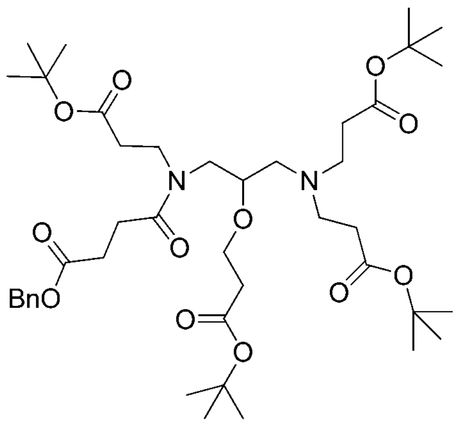 |
| **70-186** | **69-243** |
| 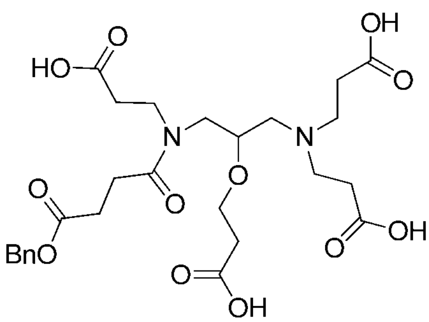 | 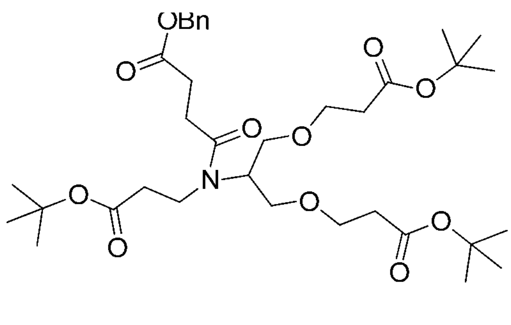 |
| **69-246** | **70-177** |

(continued)

| | |
|---|---|
| 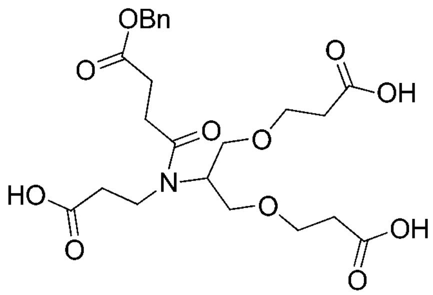 | 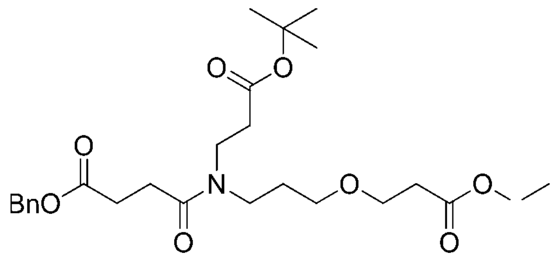 |
| **76-117**<br>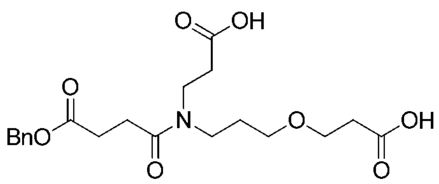 | **75-215**<br>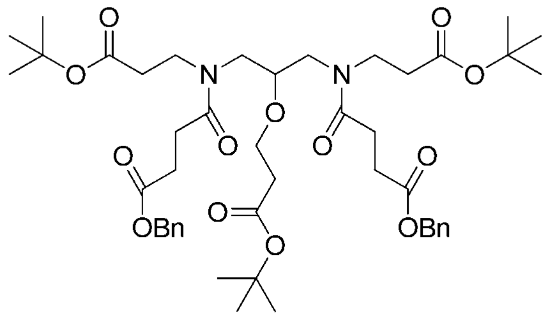 |
| **75-217**<br>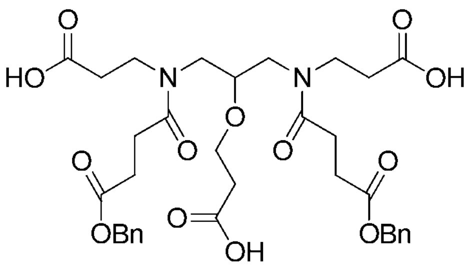 | **77-82**<br>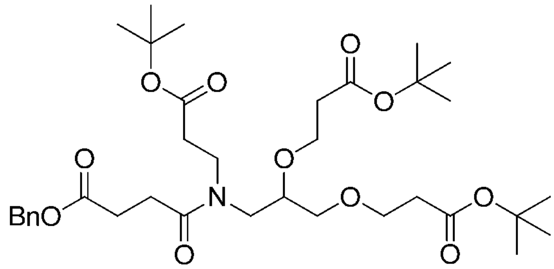 |
| **77-83**<br>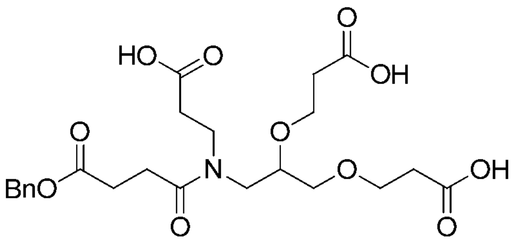 | **84-35**<br>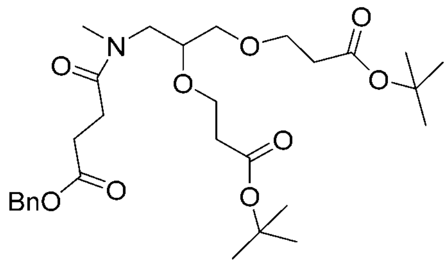 |
| **84-36** | **88-101** |
| 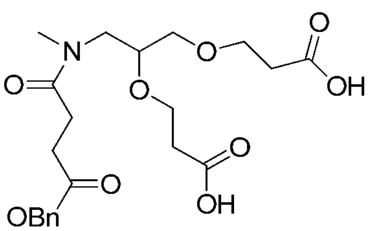 | 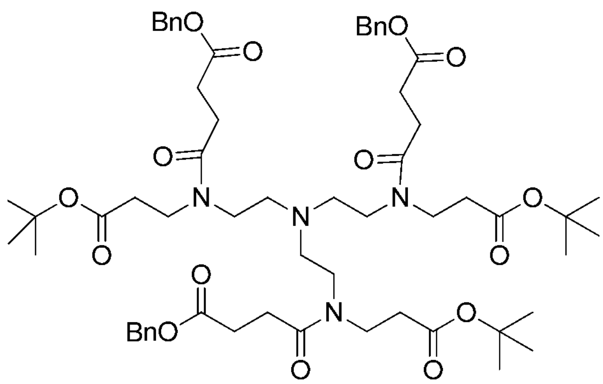 |

(continued)

| | |
|---|---|
| **88-148** 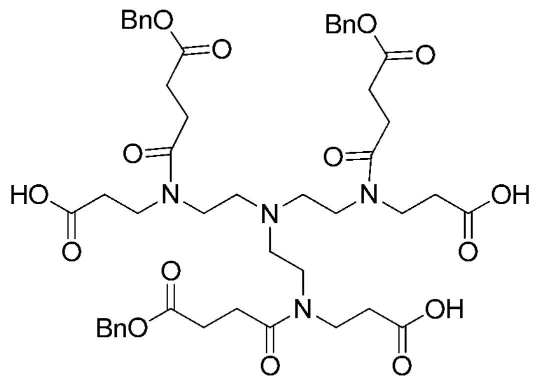 | **76-149** 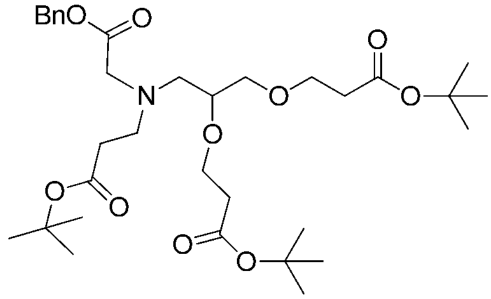 |
| **76-155** 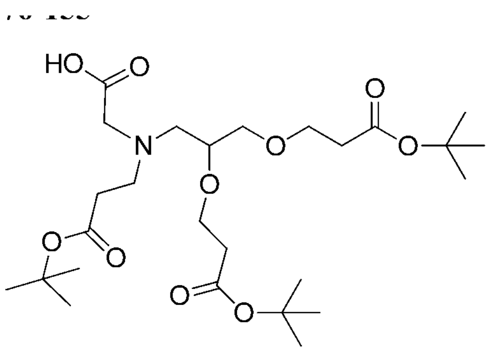 | **76-199** 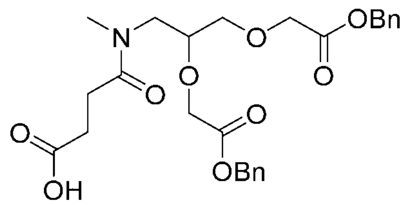 |
| **82-184** 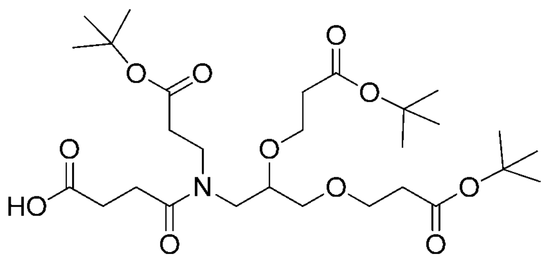 | |

In another aspect, the present application provides a compound represented by formula IX, or a pharmaceutically acceptable salt thereof,

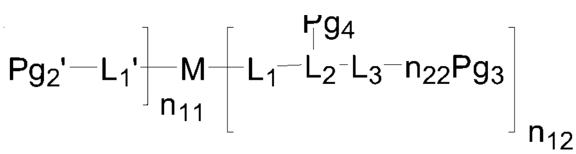

IX

wherein each group is as defined in any of the preceding items.

In some embodiments, the compound is selected from the group consisting of:

| | |
|---|---|
| 81-85<br>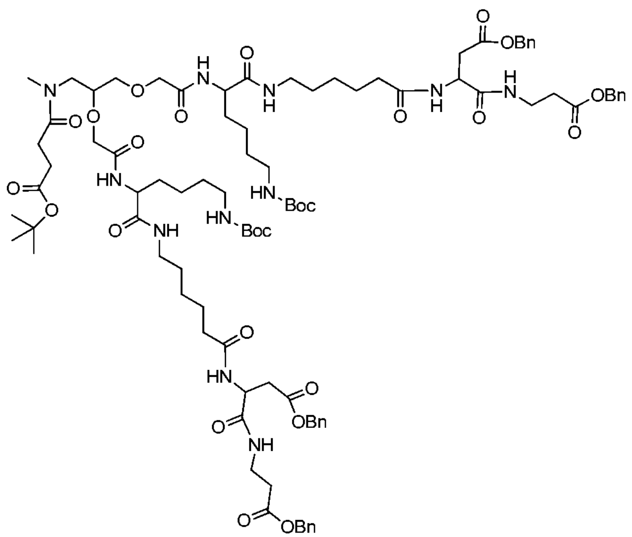 | 81-197<br>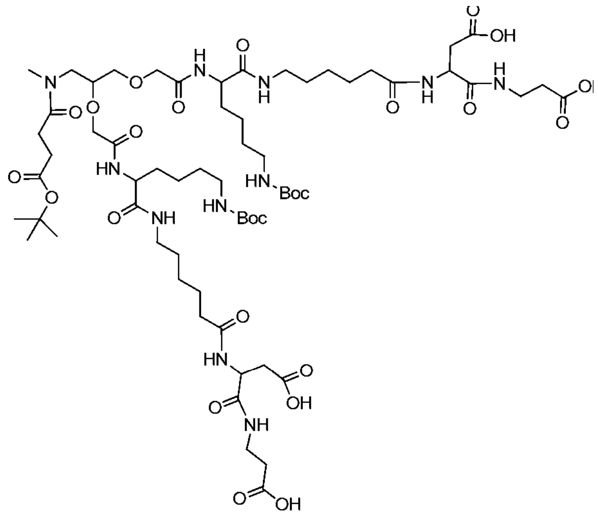 |
| 76-99 | 76-103 |

(continued)

| | |
|---|---|
| 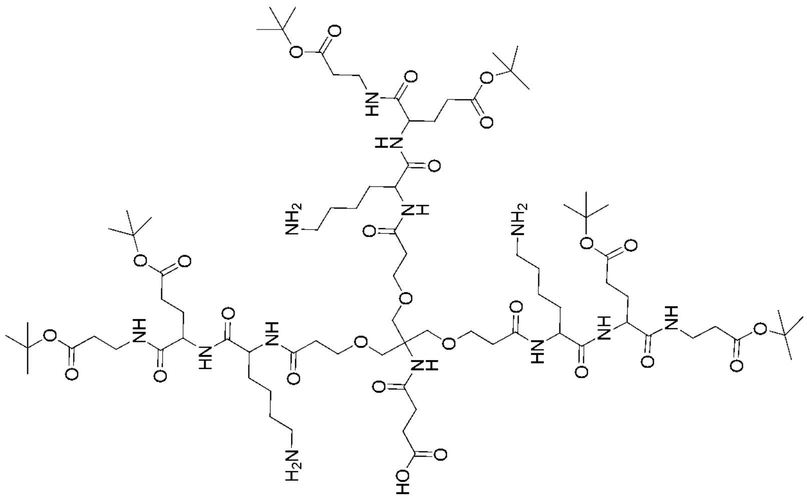 | 88-90 |
| 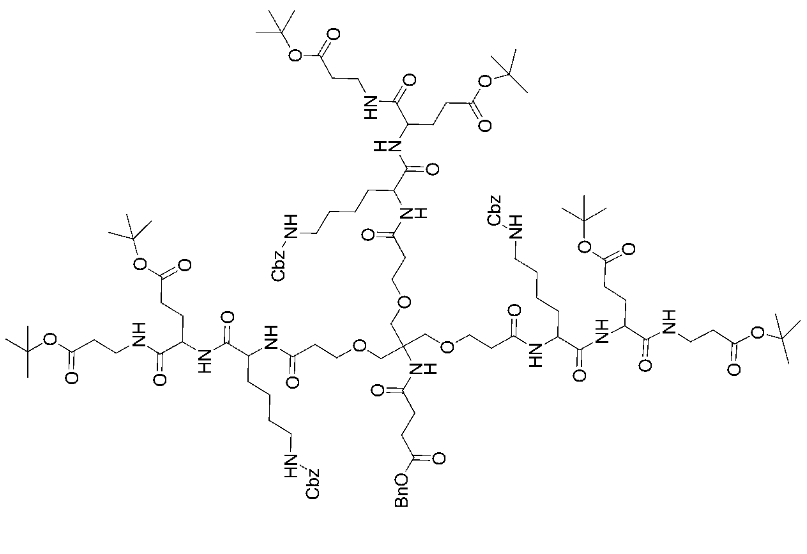 | 88-89 |

(continued)

| | |
|---|---|
| 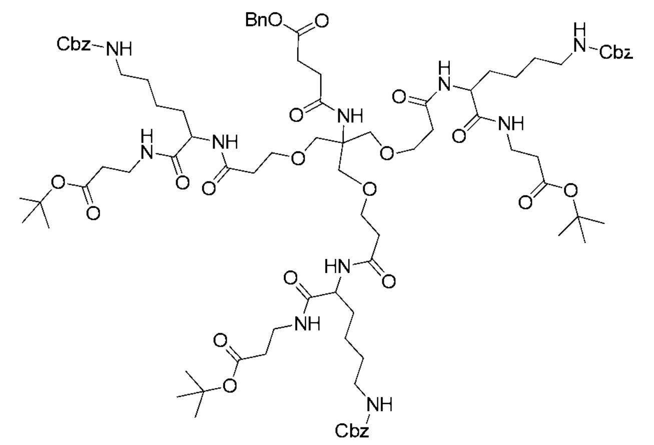 | 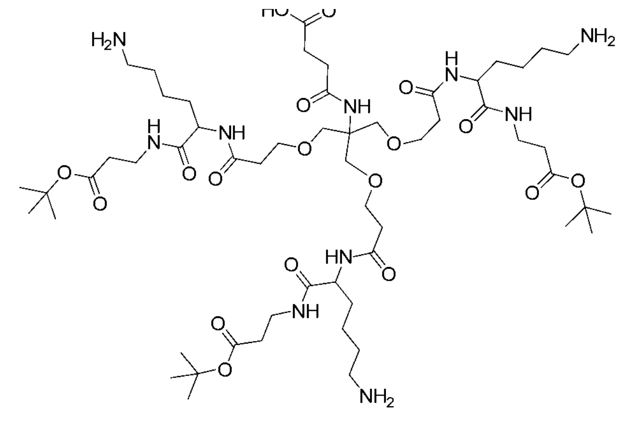 |
| **80-186** | **78-108** |
| 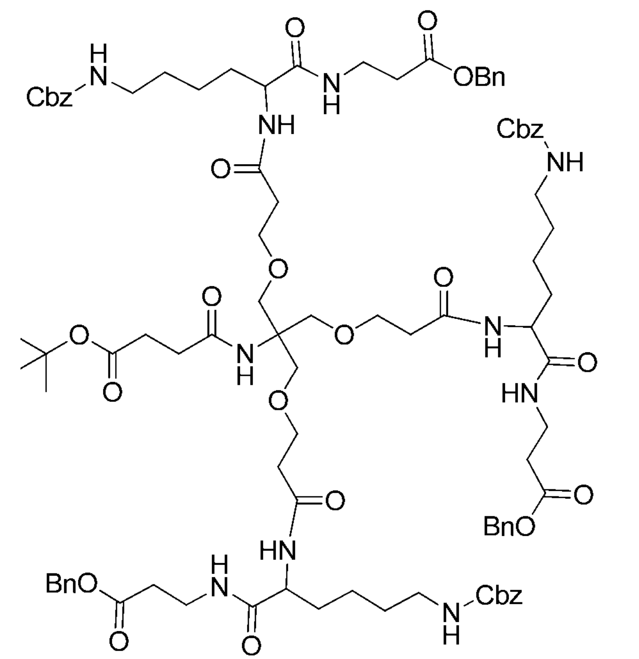 | 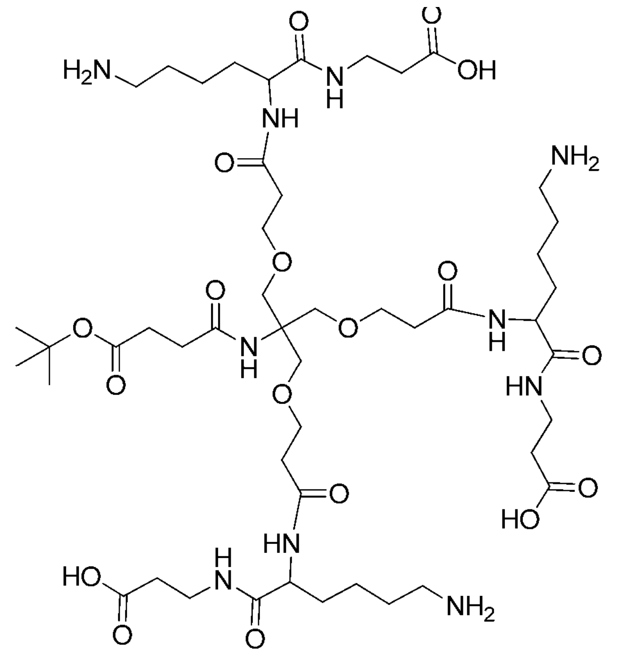 |
| **72-139** | **72-142** |

(continued)

| | |
|---|---|
| 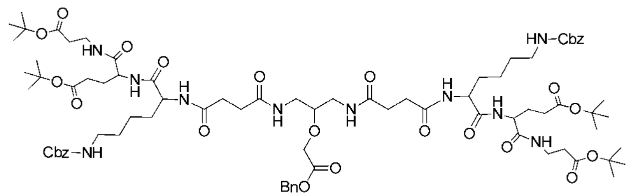 | 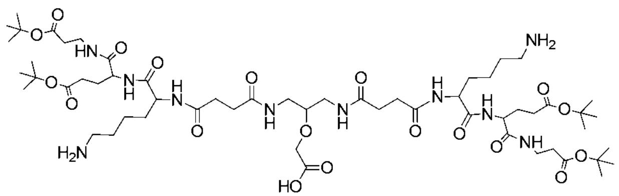 |
| **70-204** 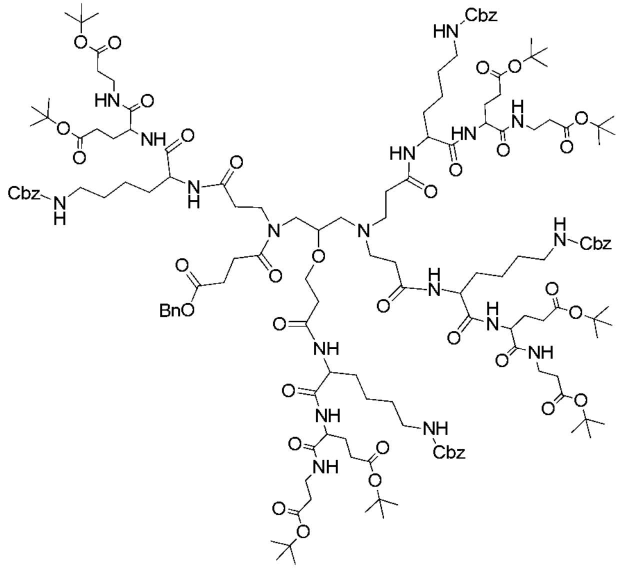 | **70-208** 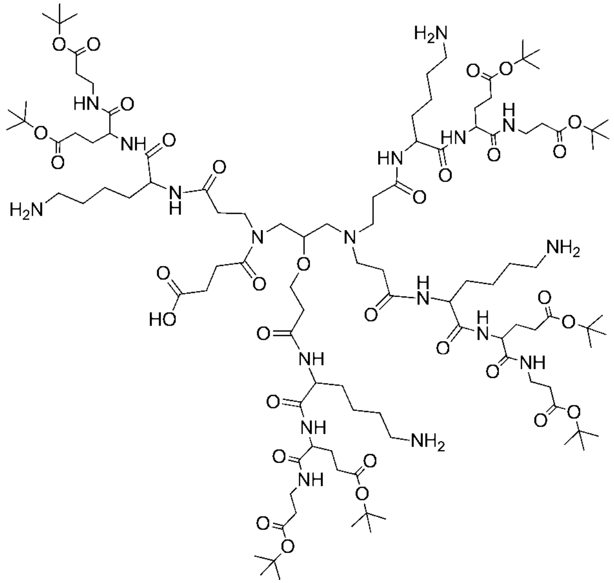 |
| **69-251** | **69-257** |

(continued)

| | |
|---|---|
| 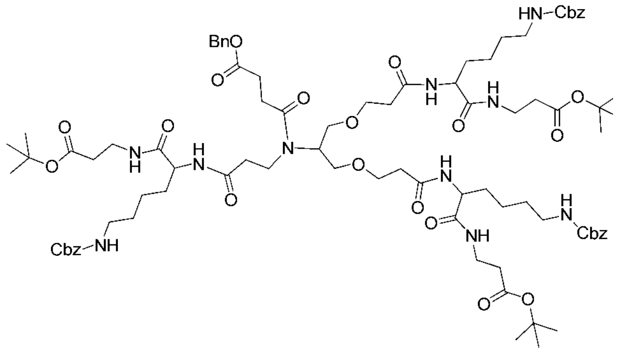 | 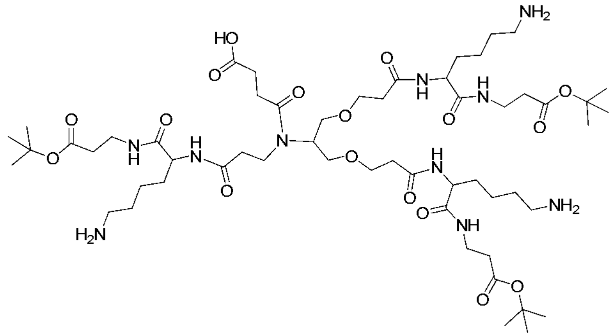 |
| 76-118 | 76-119 |
| 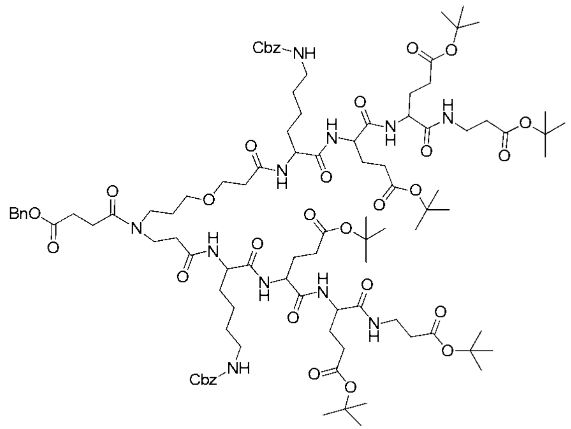 | 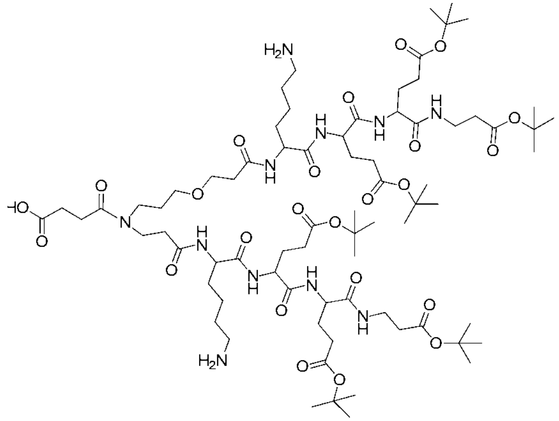 |
| 75-220 | 75-222 |

(continued)

| | |
|---|---|
| 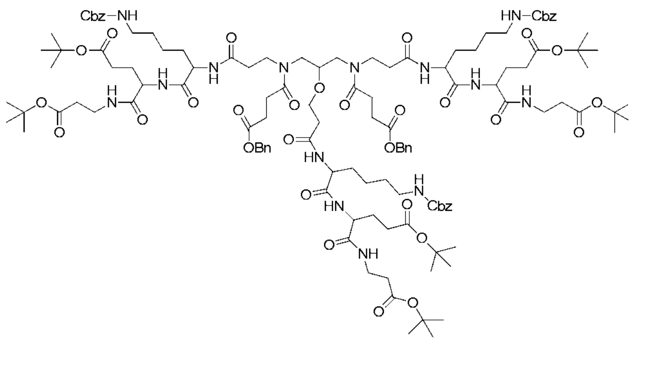 | 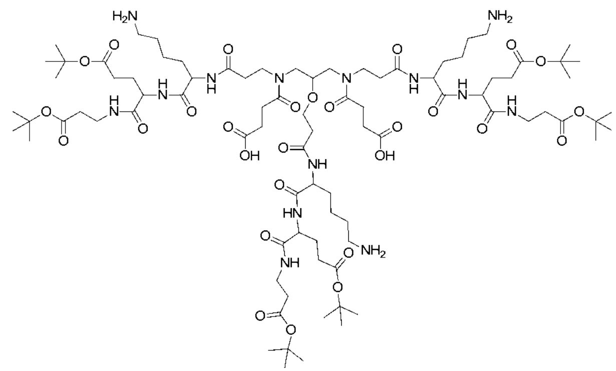 |
| **74-130** | **74-214** |
| 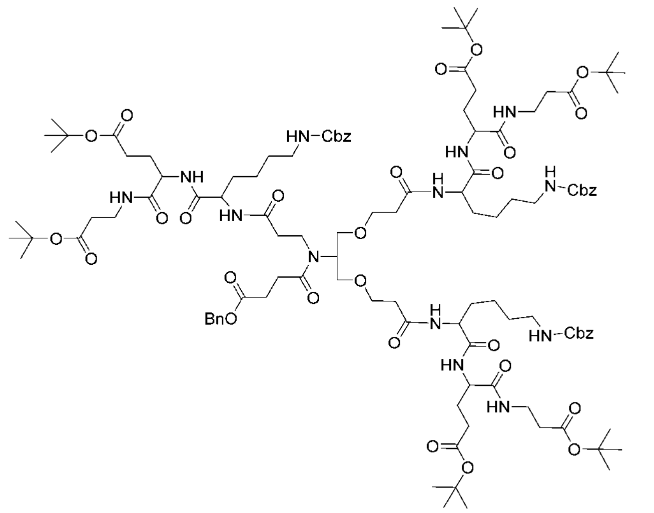 | 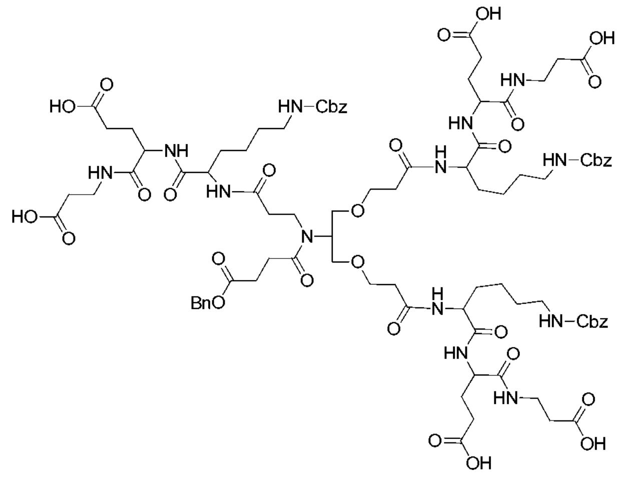 |
| **85-55** | **85-61** |

(continued)

| | |
|---|---|
| 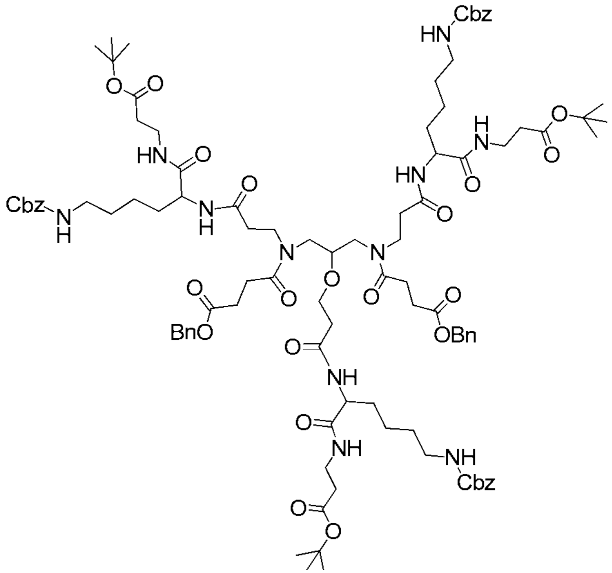 | 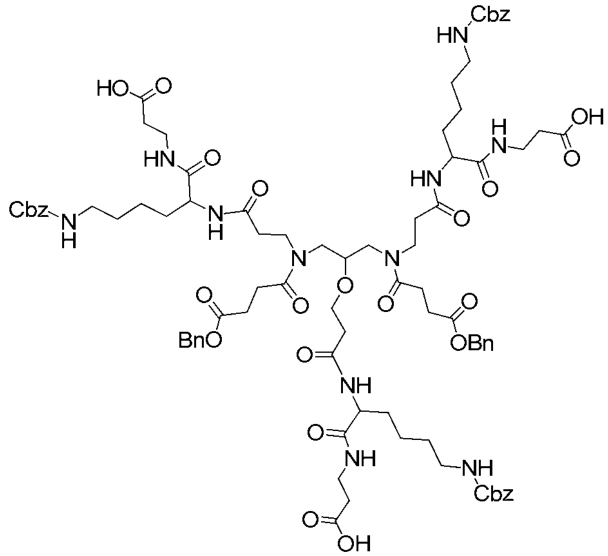 |
| **71-264** | **71-274** |

(continued)

| | |
|---|---|
| 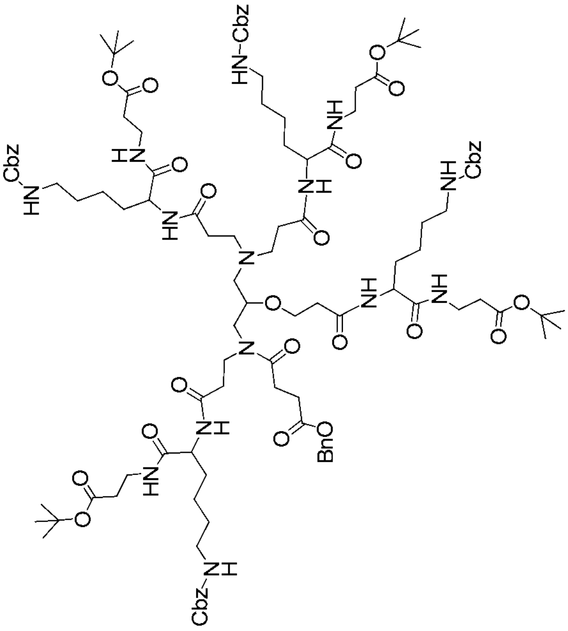 | 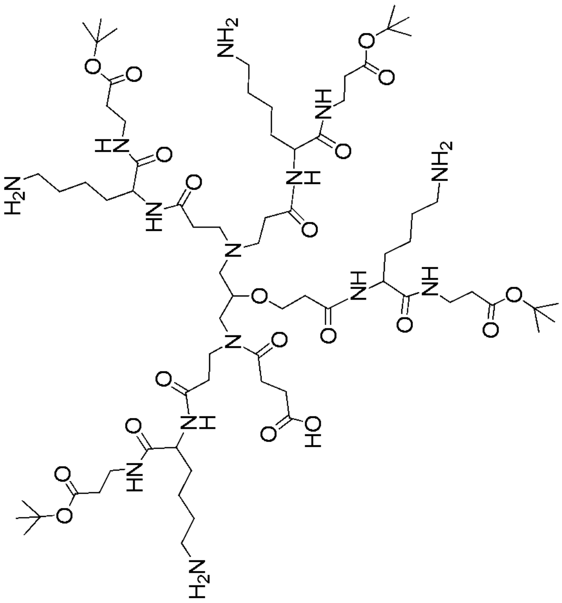 |
| 77-135 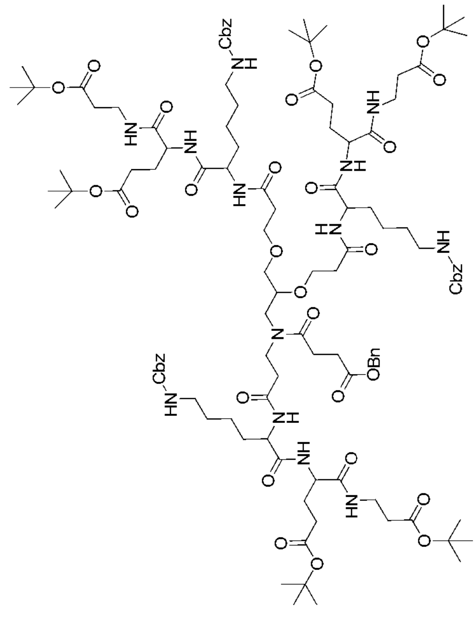 | 77-144 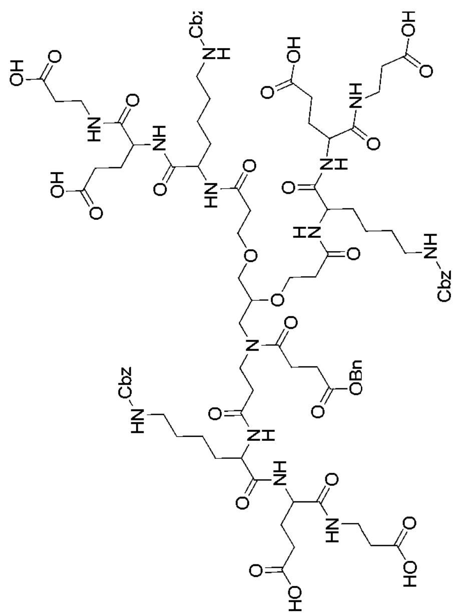 |
| 84-38 | 84-64 |

(continued)

| | |
|---|---|
| 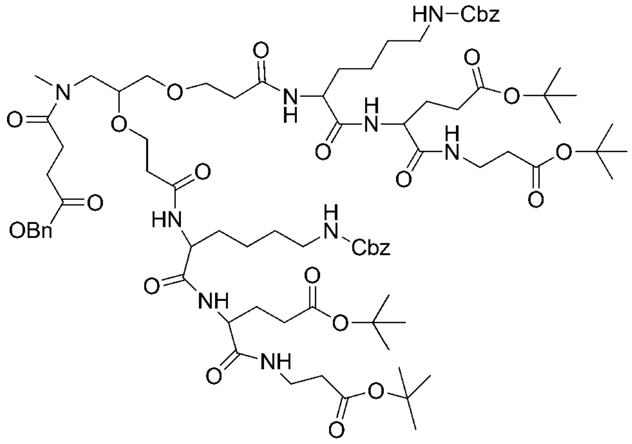 | 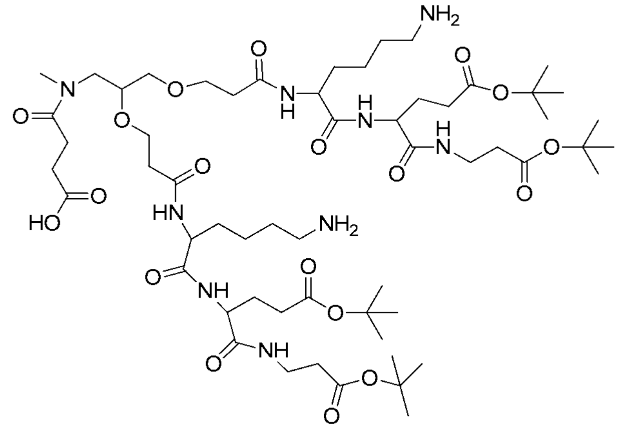 |
| **88-149** | **88-165** |
| 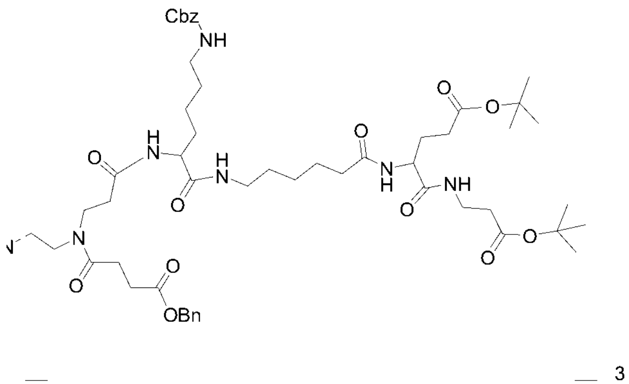 | 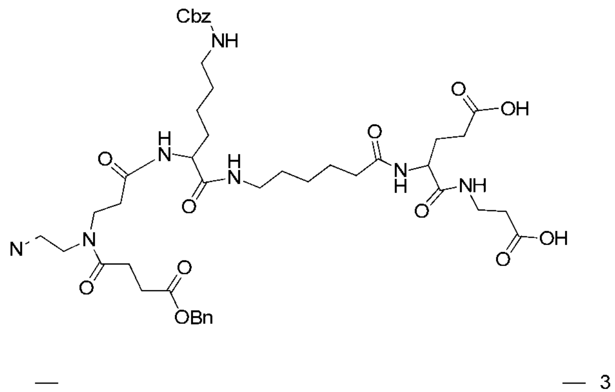 |
| **76-203** | **76-231** |

(continued)

| | |
|---|---|
| 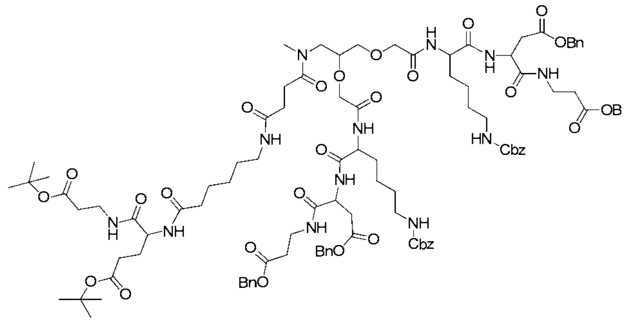 | 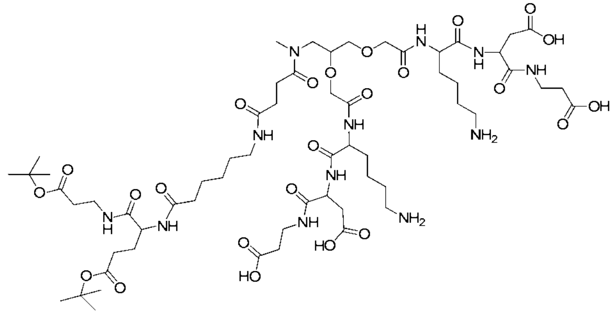 |
| 72-217 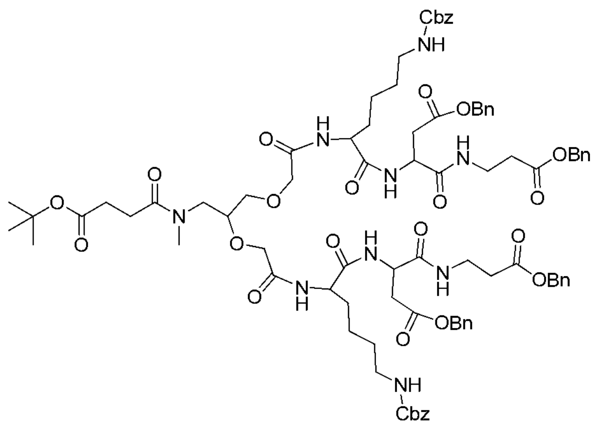 | 72-218 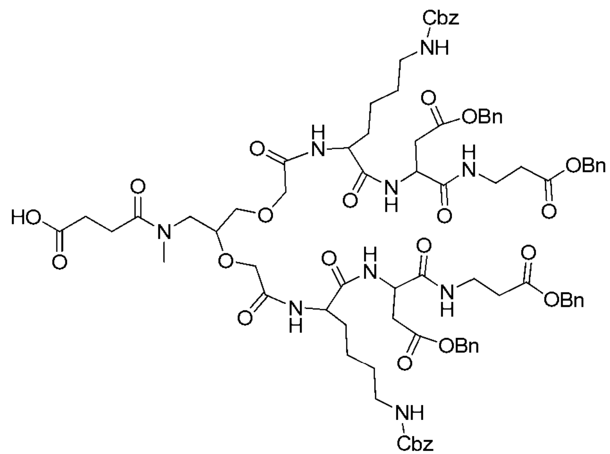 |
| 72-226 | |

(continued)

| | |
|---|---|
| | 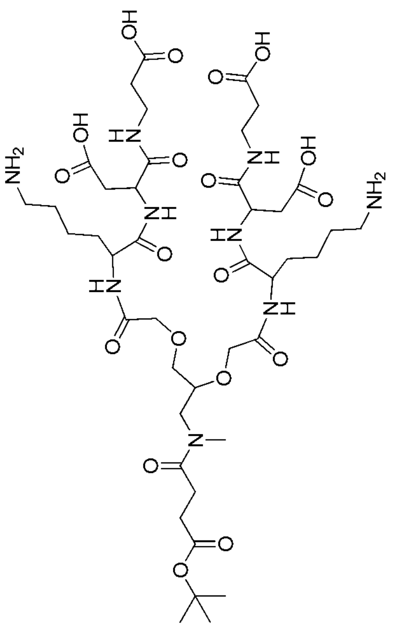 |

In another aspect, the present application provides a compound represented by formula X, or a pharmaceutically acceptable salt thereof,

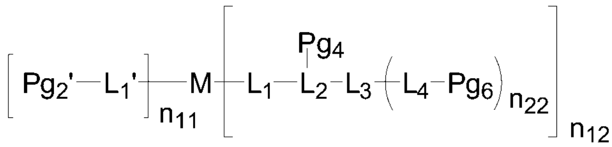

X

wherein each group is as defined in any of the preceding items.

In some embodiments, the compound is selected from the group consisting of:

| | |
|---|---|
| **74-215** | 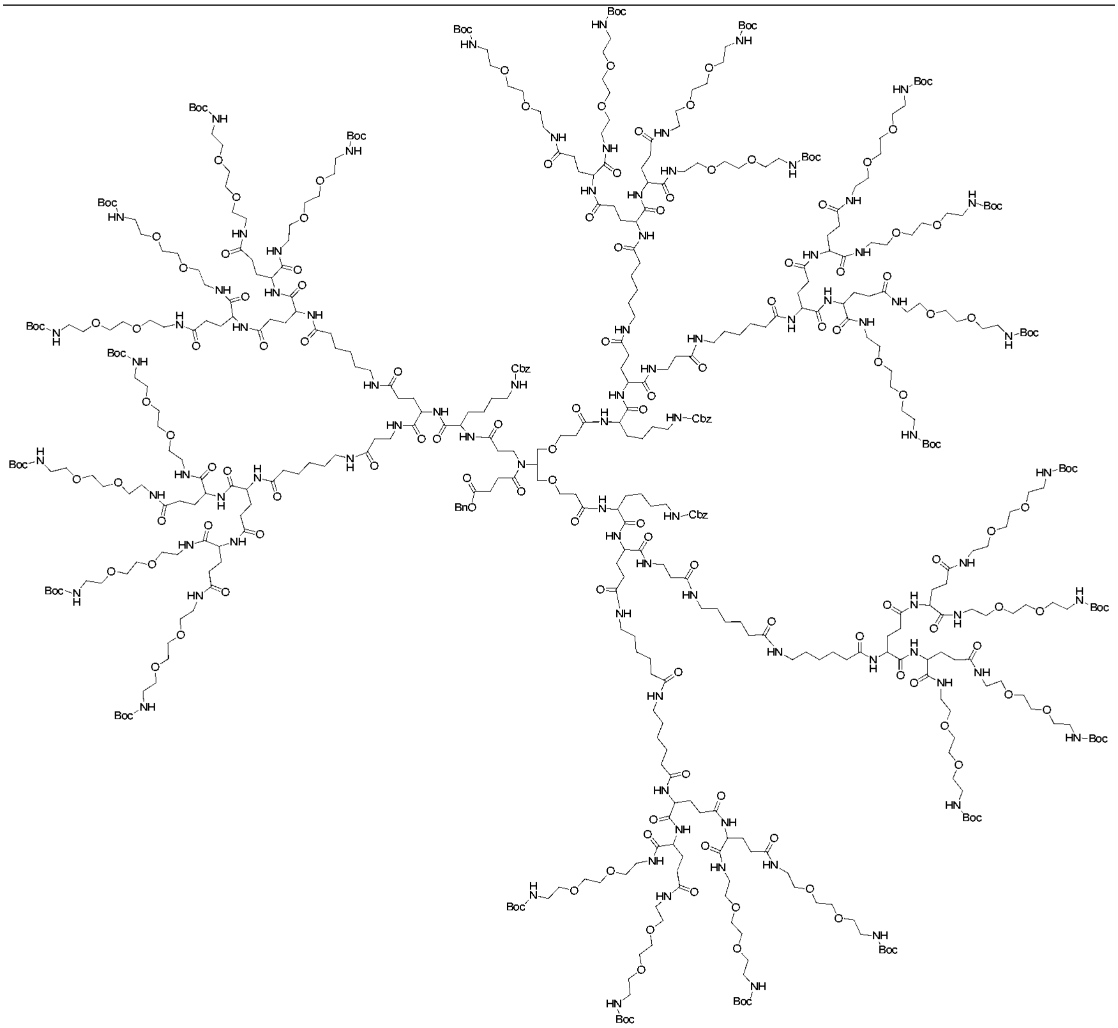 |

(continued)

| | |
|---|---|
| **74-216** | 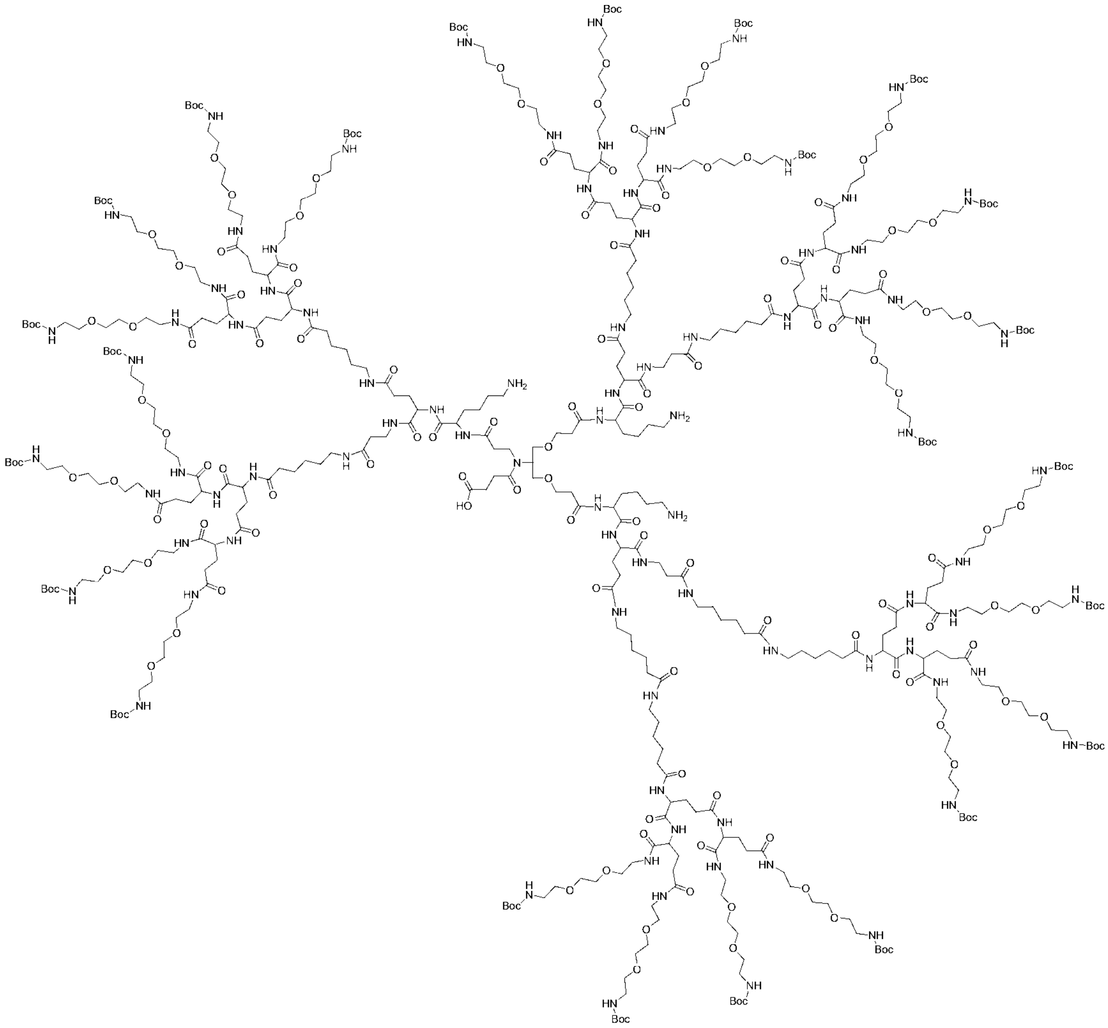 |
| **77-146** | 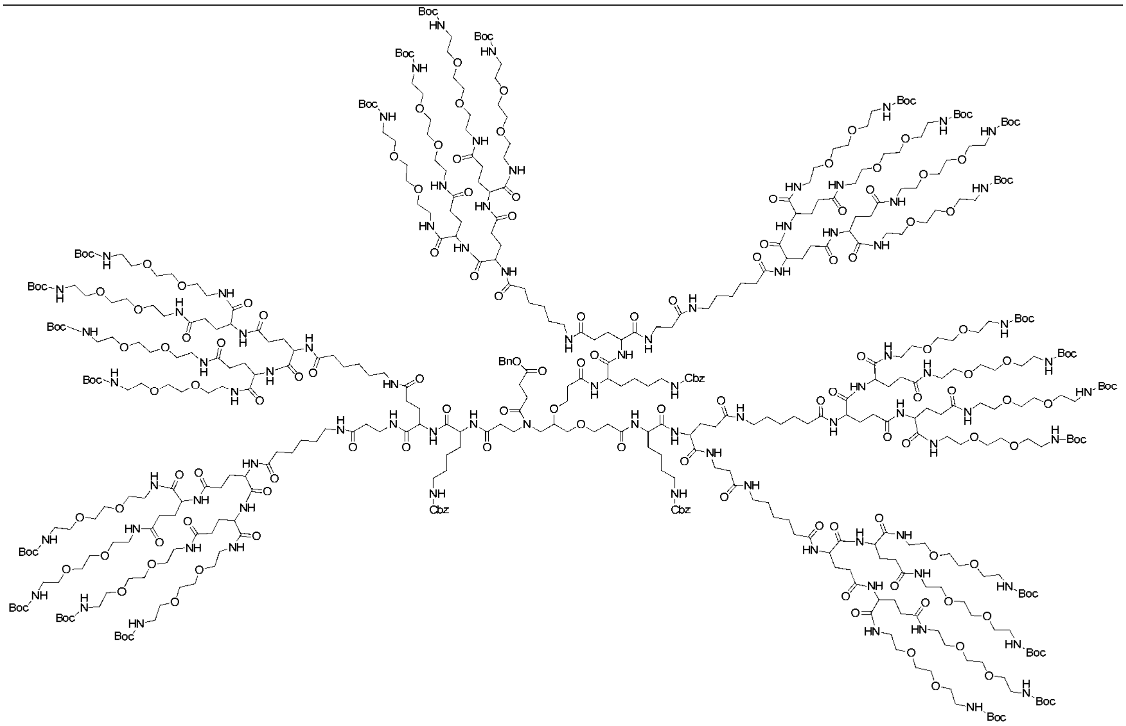 |

(continued)

| | |
|---|---|
| **77-149** | 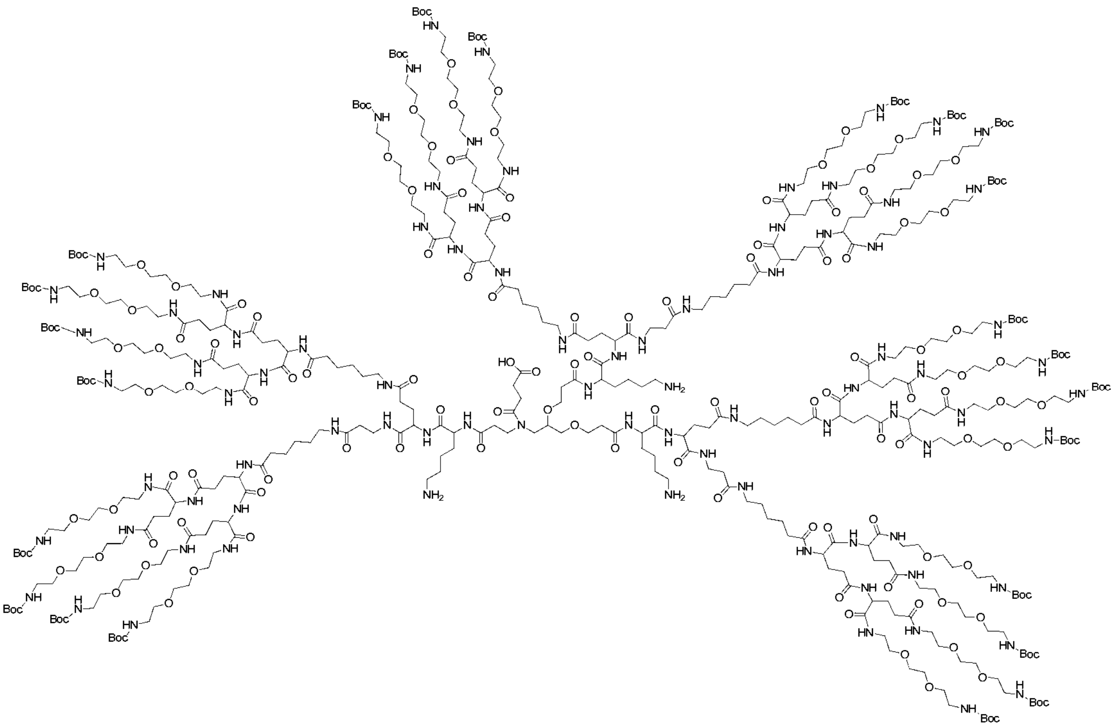 |
| **88-168** | 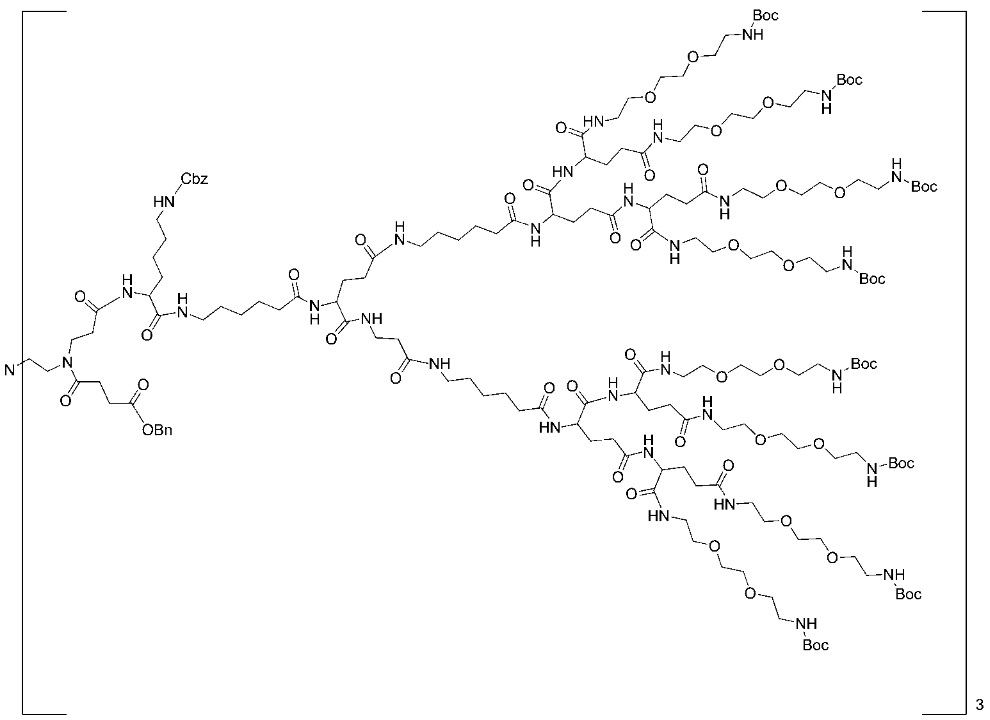 |

(continued)

| | |
|---|---|
| **88-169** | 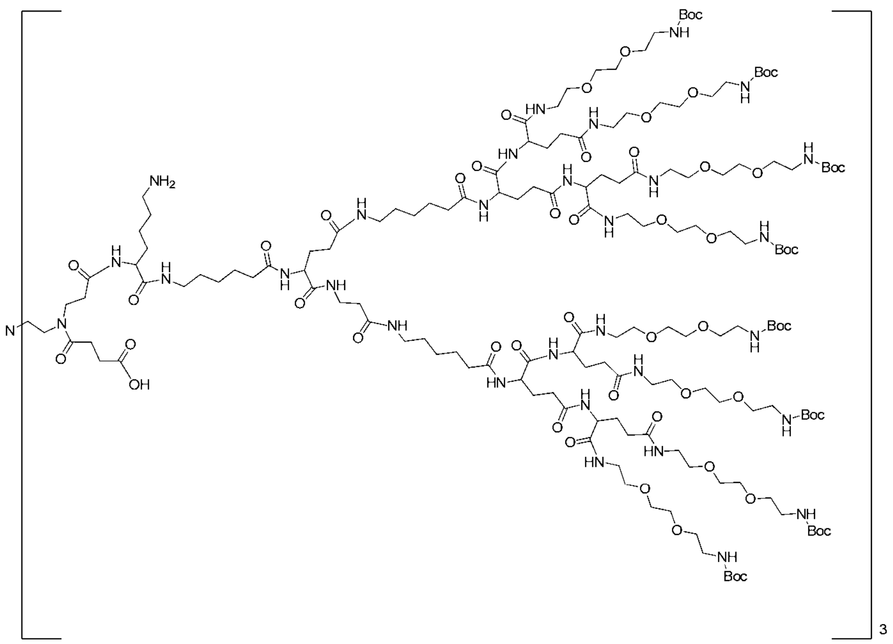 |

In another aspect, the present application provides a compound represented by formula XI, or a pharmaceutically acceptable salt thereof,

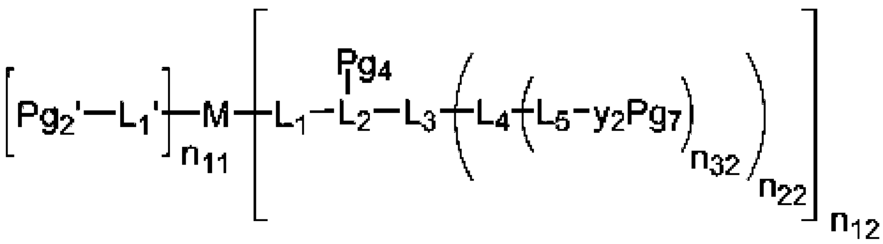

XI

wherein each group is as defined in any of the preceding items.

In some embodiments, the compound is selected from the group consisting of:

| | |
|---|---|
| **85-80** | 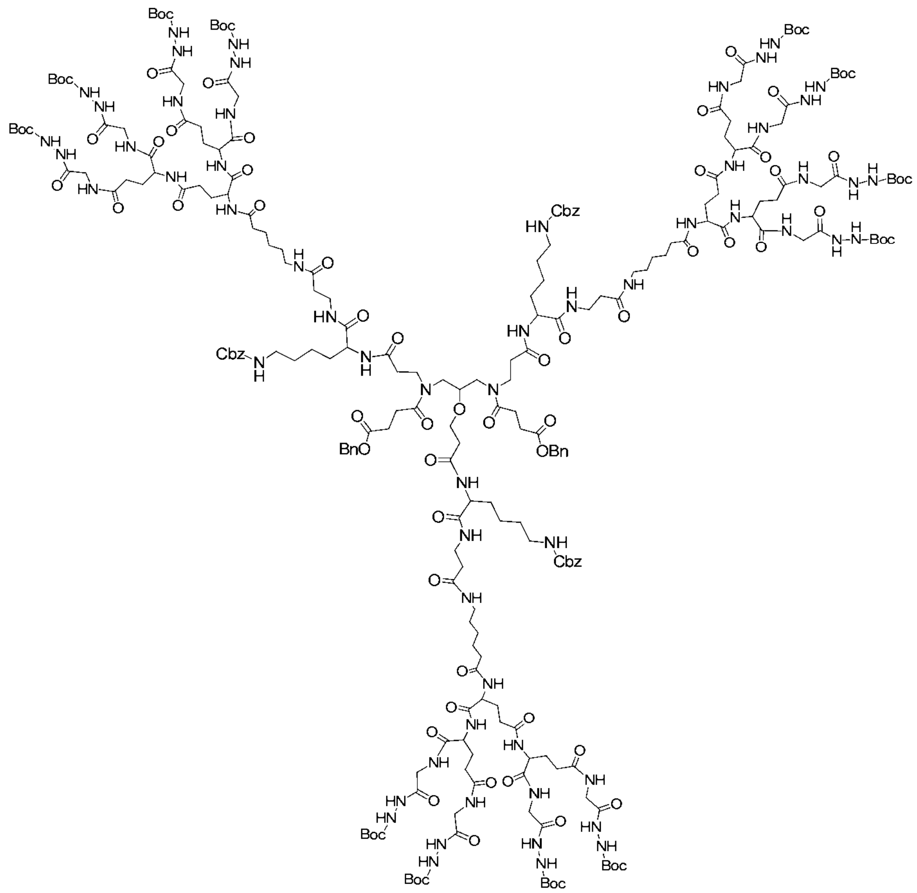 |

(continued)

| | |
|---|---|
| **85-82** | 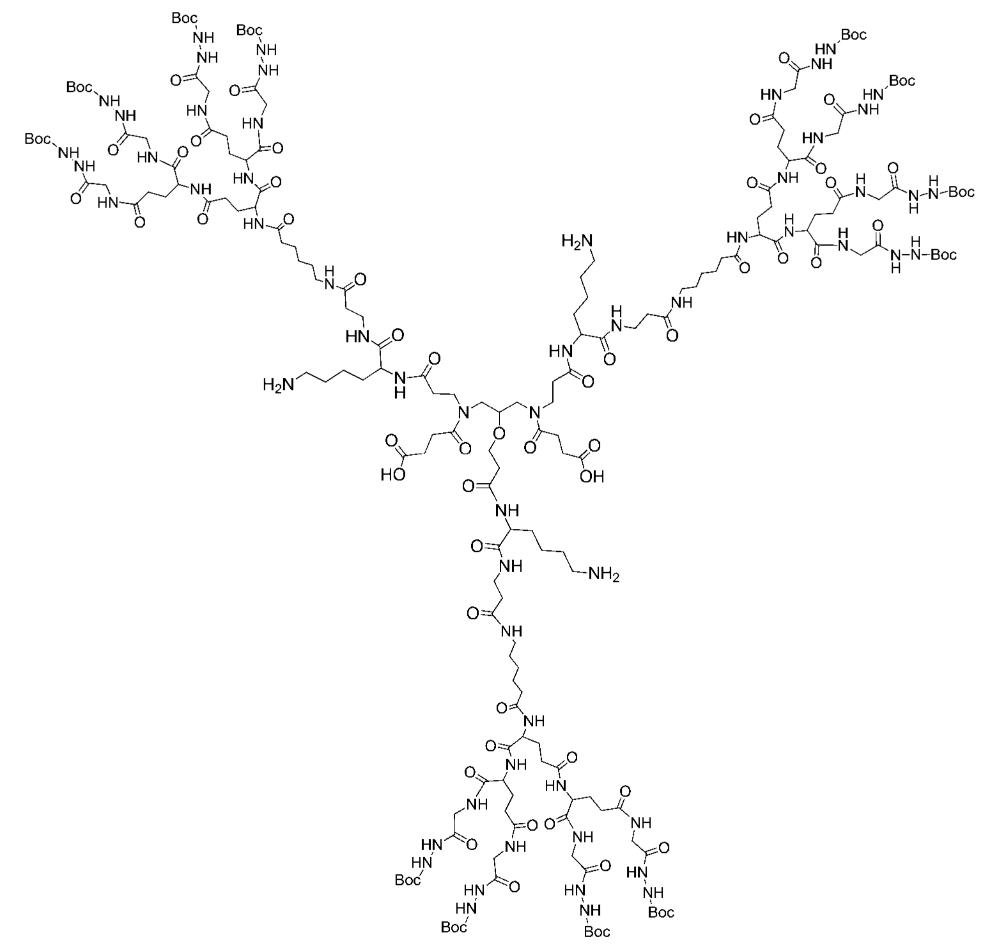 |

In another aspect, the present application provides a compound represented by formula XII, or a pharmaceutically acceptable salt thereof,

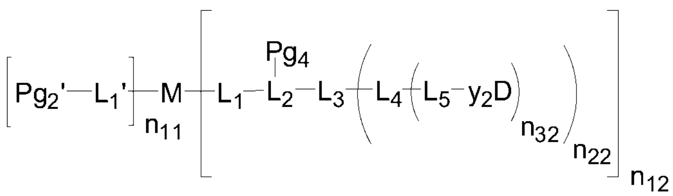

XII

wherein each group is as defined in any of the preceding items.

In some embodiments, the compound is selected from the group consisting of:

| | |
|---|---|
| **81-204** | 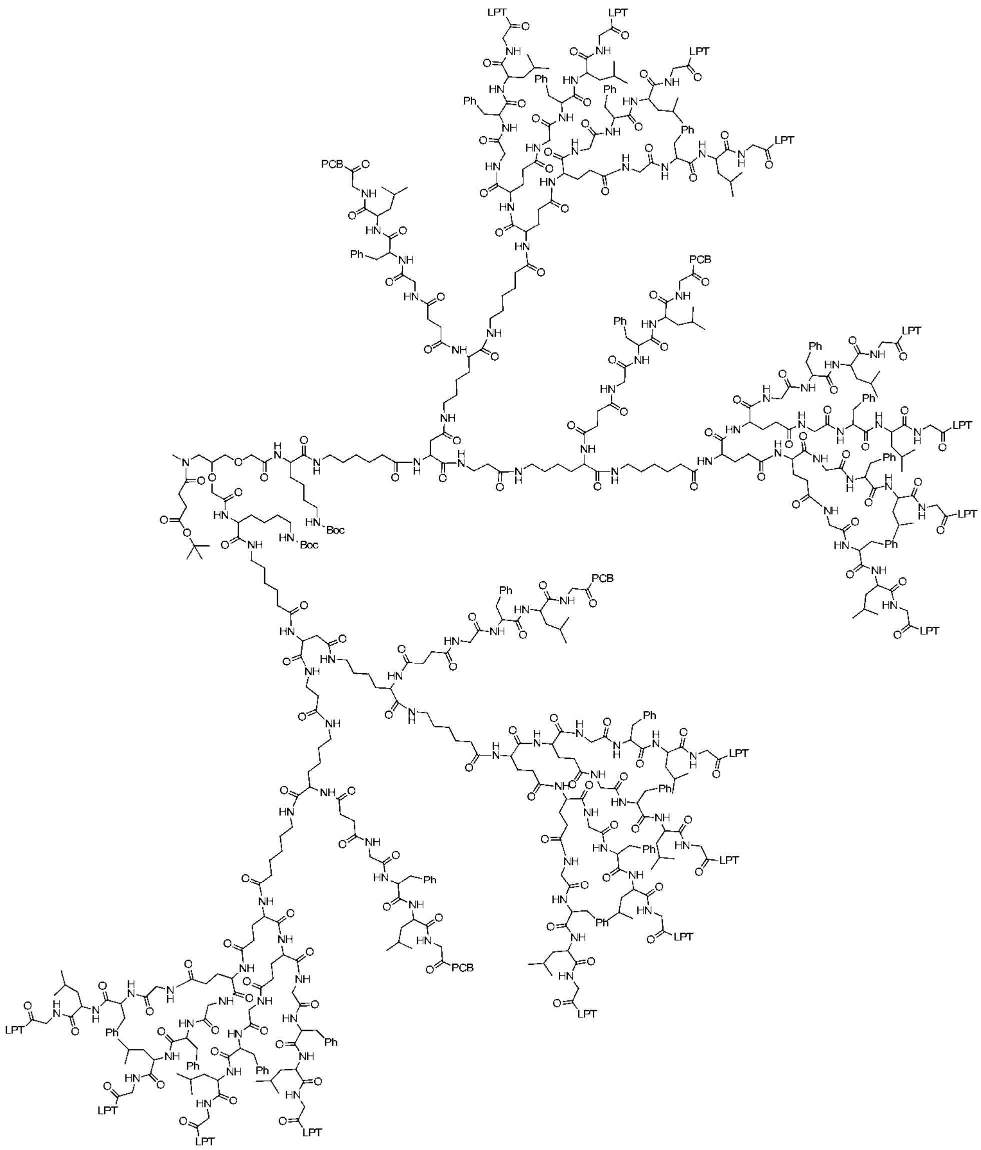 |

(continued)

| | |
|---|---|
| **81-205** | 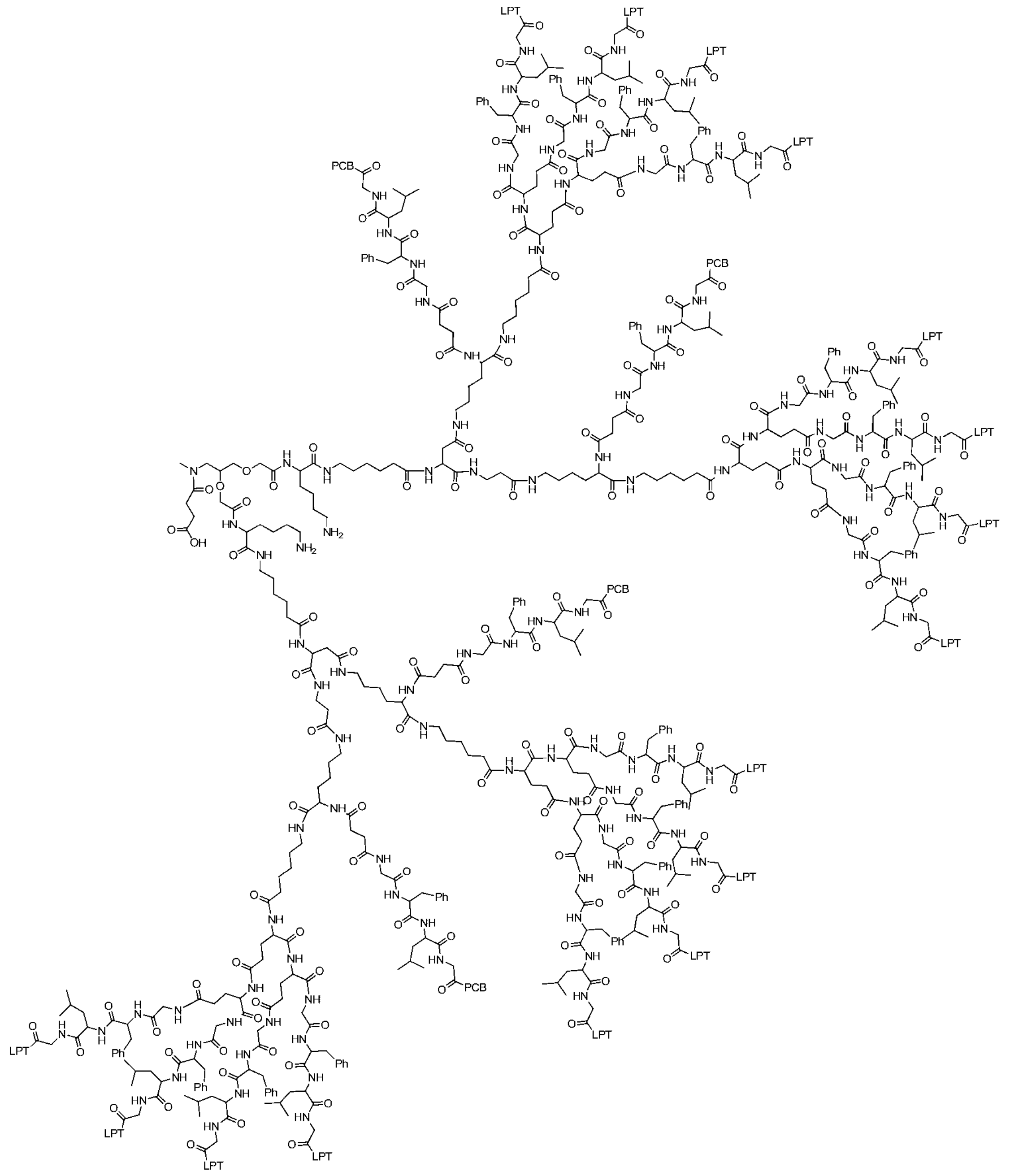 |

In another aspect, the present application provides a compound represented by formula XIII or a pharmaceutically acceptable salt thereof,

$$\left[{}_{n_{21}}Pg_3'\text{-}L_3\text{-}L_1'\right]_{n_{11}} M \left[L_1\text{-}Pg_2\right]_{n_{12}}$$

XIII

wherein each group is as defined in any of the preceding items.

In some embodiments, the compound is selected from the group consisting of:

| | |
|---|---|
| 76-201 | 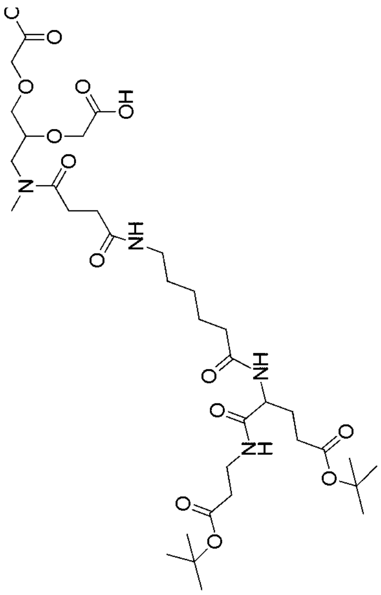 |
| 88-163 | 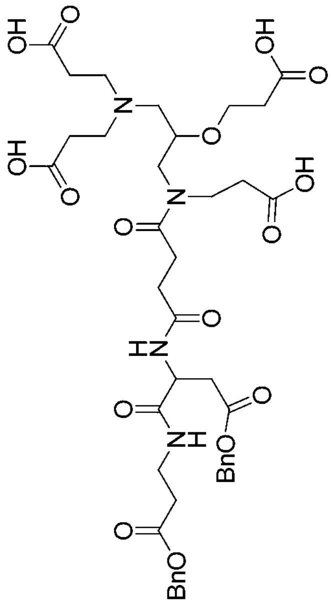 |
| 76-200 | 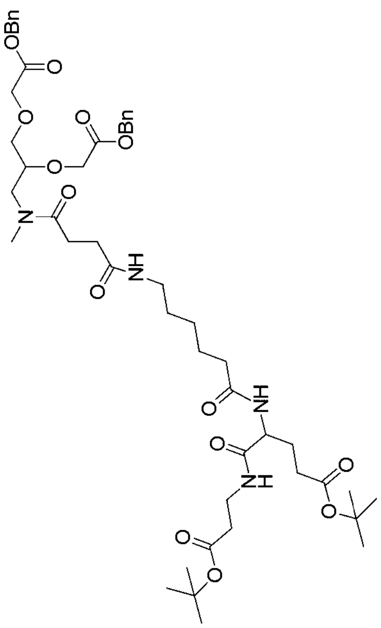 |
| 88-138 | 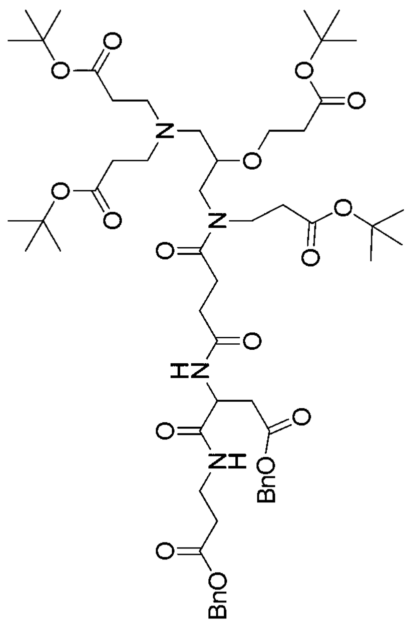 |

In another aspect, the present application provides a compound represented by formula XIV or a pharmaceutically acceptable salt thereof,

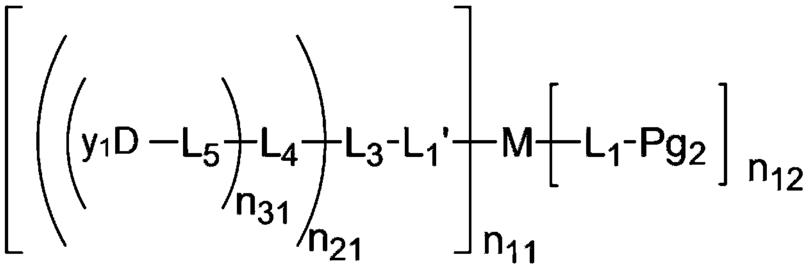

XIV

wherein each group is as defined in any of the preceding items.

In some embodiments, the compound is selected from the group consisting of:

| | |
|---|---|
| **76-156** | 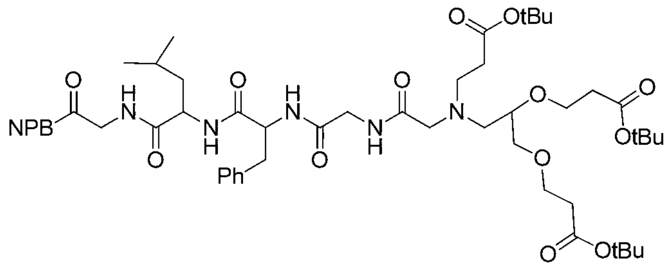 |
| **76-170** | 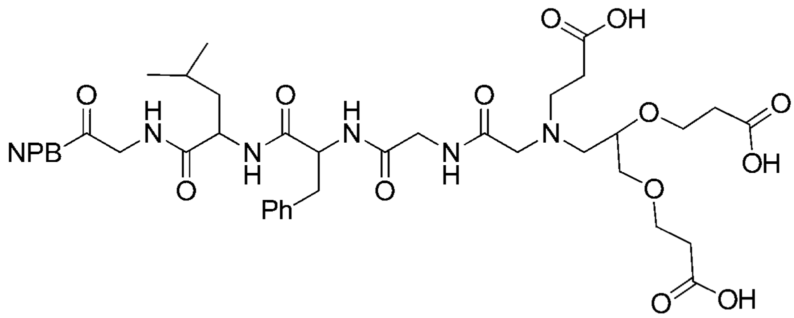 |
| **82-185** | 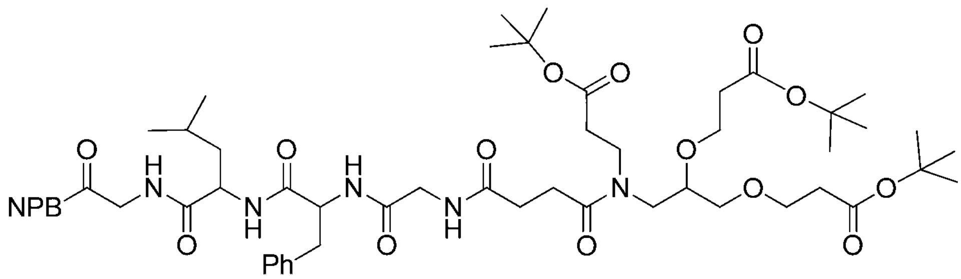 |
| **82-196** | 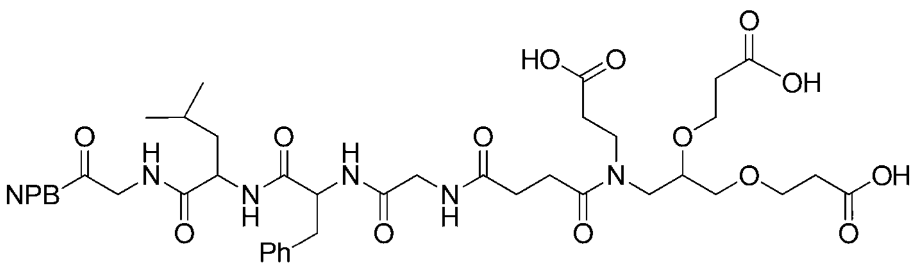 |

In another aspect, the present application provides a compound represented by formula XV or a pharmaceutically acceptable salt thereof:

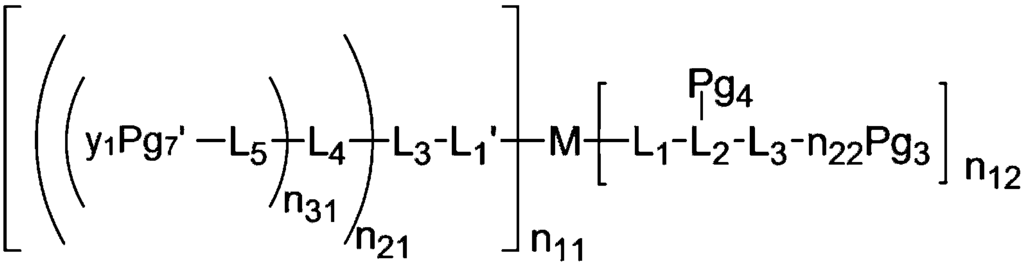

XV

wherein, $Pg_7$' is hydrogen or selected from the group consisting of an amino protecting group and a carboxyl protecting group; preferably, the amino protecting group is selected from the group consisting of an alkyl-based protecting group (e.g., Bn, Trt, DMB, or PMB) and an alkoxycarbonyl-based protecting group (e.g., Boc, Fmoc, Cbz, or Teoc), and the carboxyl protecting group is selected from the group consisting of an ester-based protecting group (e.g., methyl ester, ethyl ester, tert-butyl ester, allyl ester, or benzyl ester);
the remaining groups are as defined in any of the preceding items.

In some embodiments, the compound is selected from the group consisting of:

| | |
|---|---|
| **72-219** | 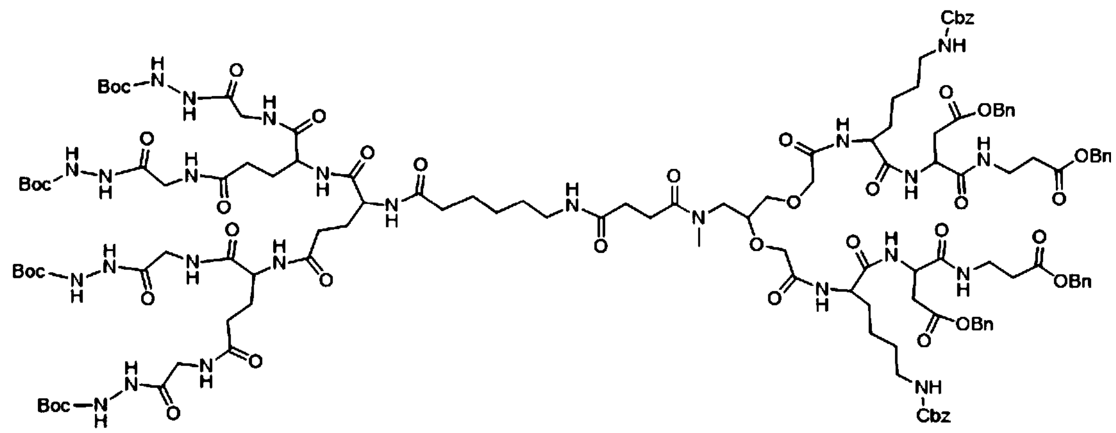 |
| **72-220** | 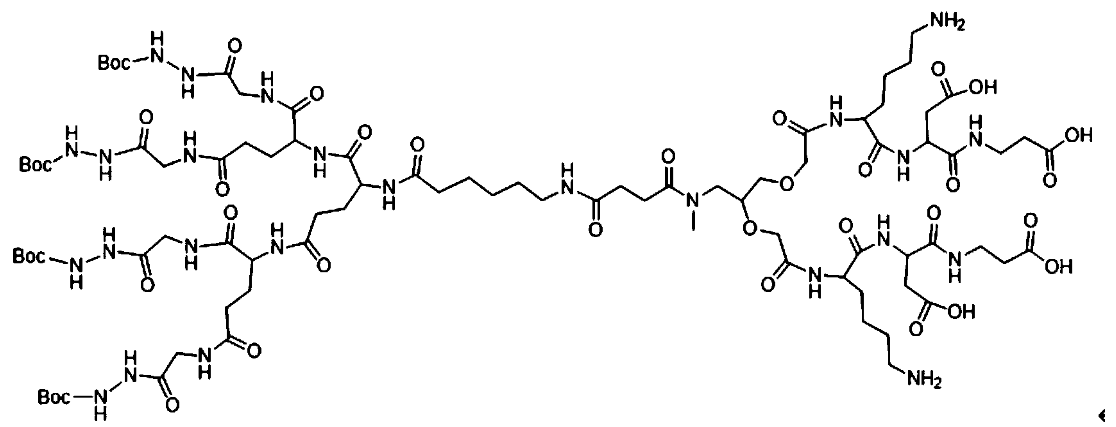 |

In another aspect, the present application provides a compound represented by formula XVI or a pharmaceutically acceptable salt thereof:

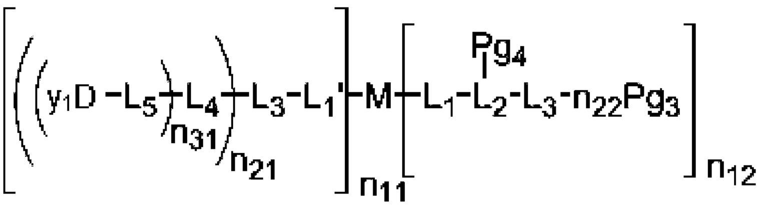

XVI

wherein each group is as defined in any of the preceding items.

In some embodiments, the compound is selected from the group consisting of:

| | |
|---|---|
| **82-197** | 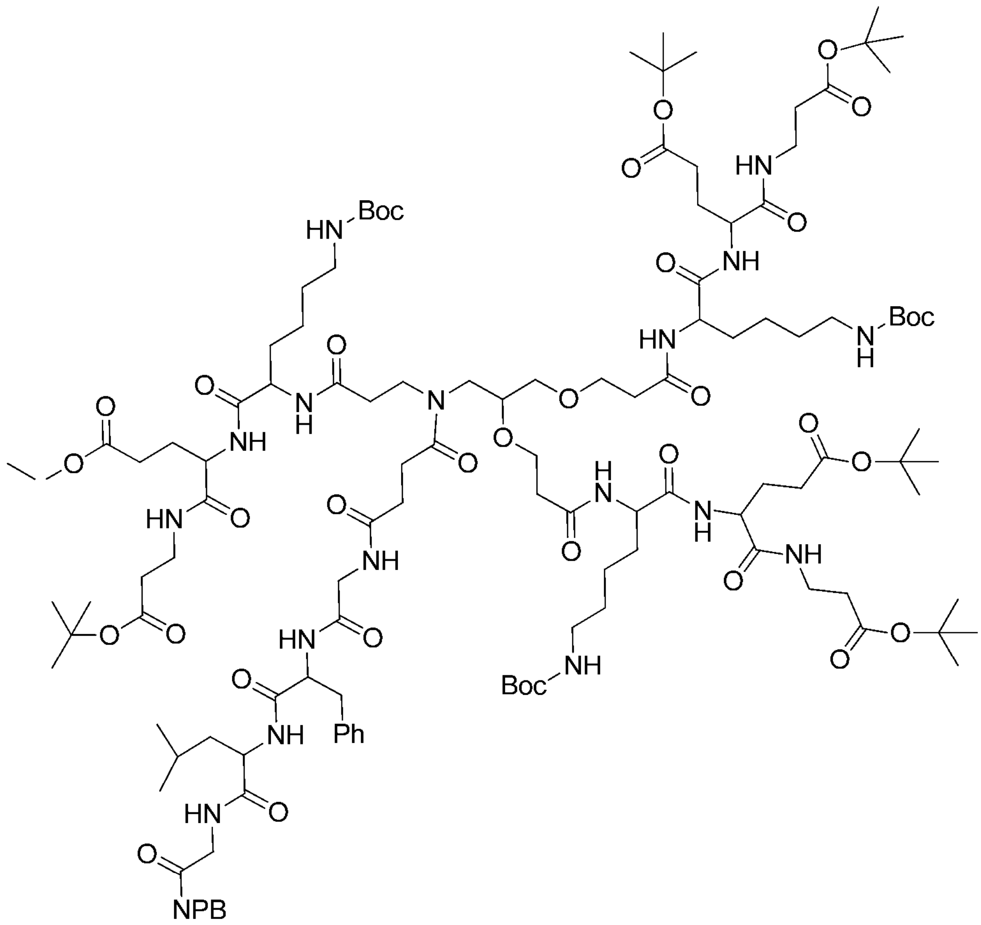 |

(continued)

| | |
|---|---|
| **82-200** | 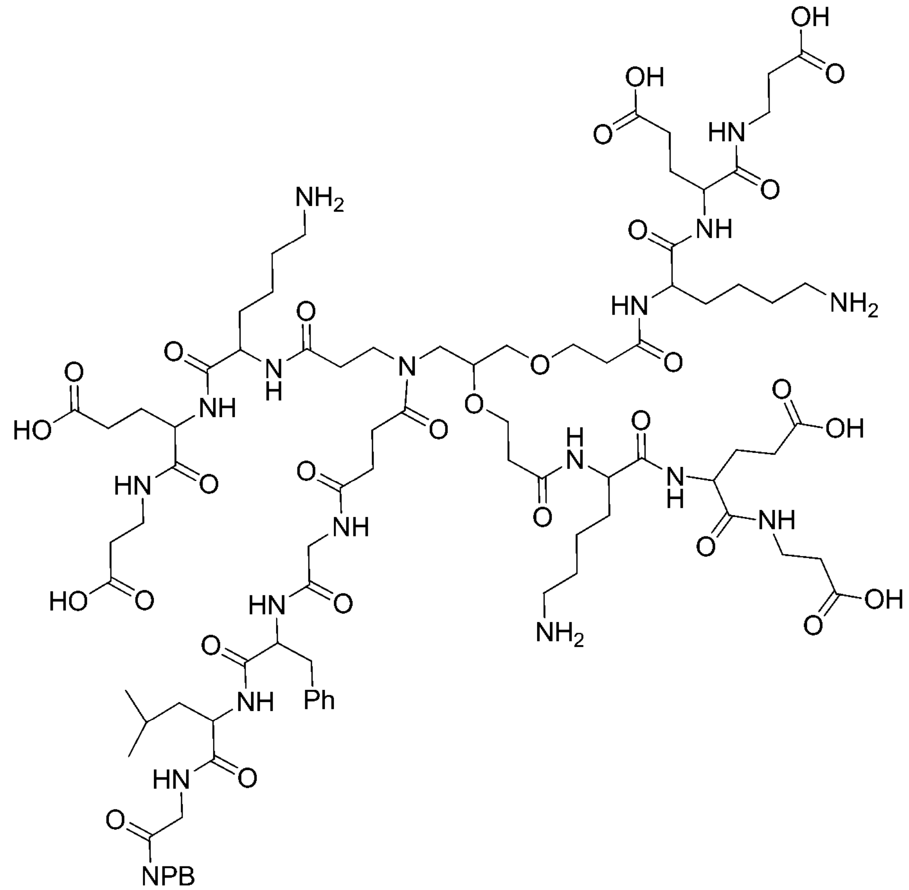 |

In another aspect, the present application provides a compound represented by formula XVII or a pharmaceutically acceptable salt thereof:

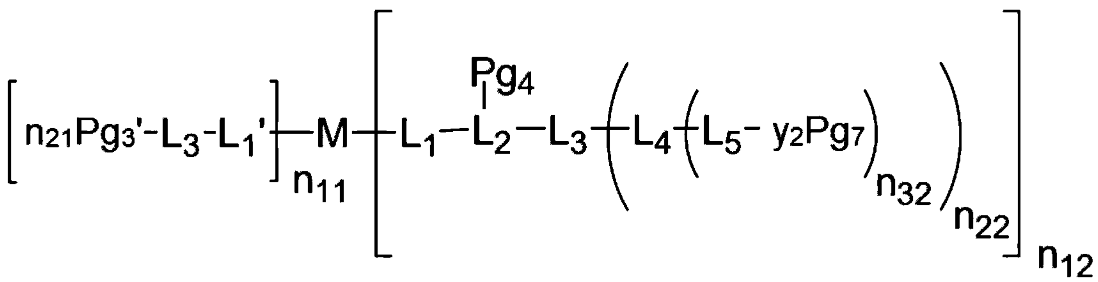

XVII

Wherein, each $Pg_3$' is independently hydrogen or selected from the group consisting of an amino protecting group and a carboxyl protecting group, the amino protecting group is selected from the group consisting of an alkoxycarbonyl-based protecting group, such as Boc, Fmoc, Cbz, or Teoc; the carboxyl protecting group is selected from the group consisting of an ester-based protecting group, such as methyl ester, ethyl ester, tert-butyl ester, allyl ester, or benzyl ester; preferably, each $Pg_3$ is independently hydrogen or a carboxyl protecting group, such as an ester-based protecting group, such as methyl ester, ethyl ester, tert-butyl ester, allyl ester, or benzyl ester, preferably tert-butyl ester or benzyl ester; preferably, each $Pg_3$ is the same, preferably all are tert-butyl ester or benzyl ester;
the remaining groups are as defined in any of the preceding items.

In some embodiments, the compound is selected from the group consisting of:

| | |
|---|---|
| **88-166** | 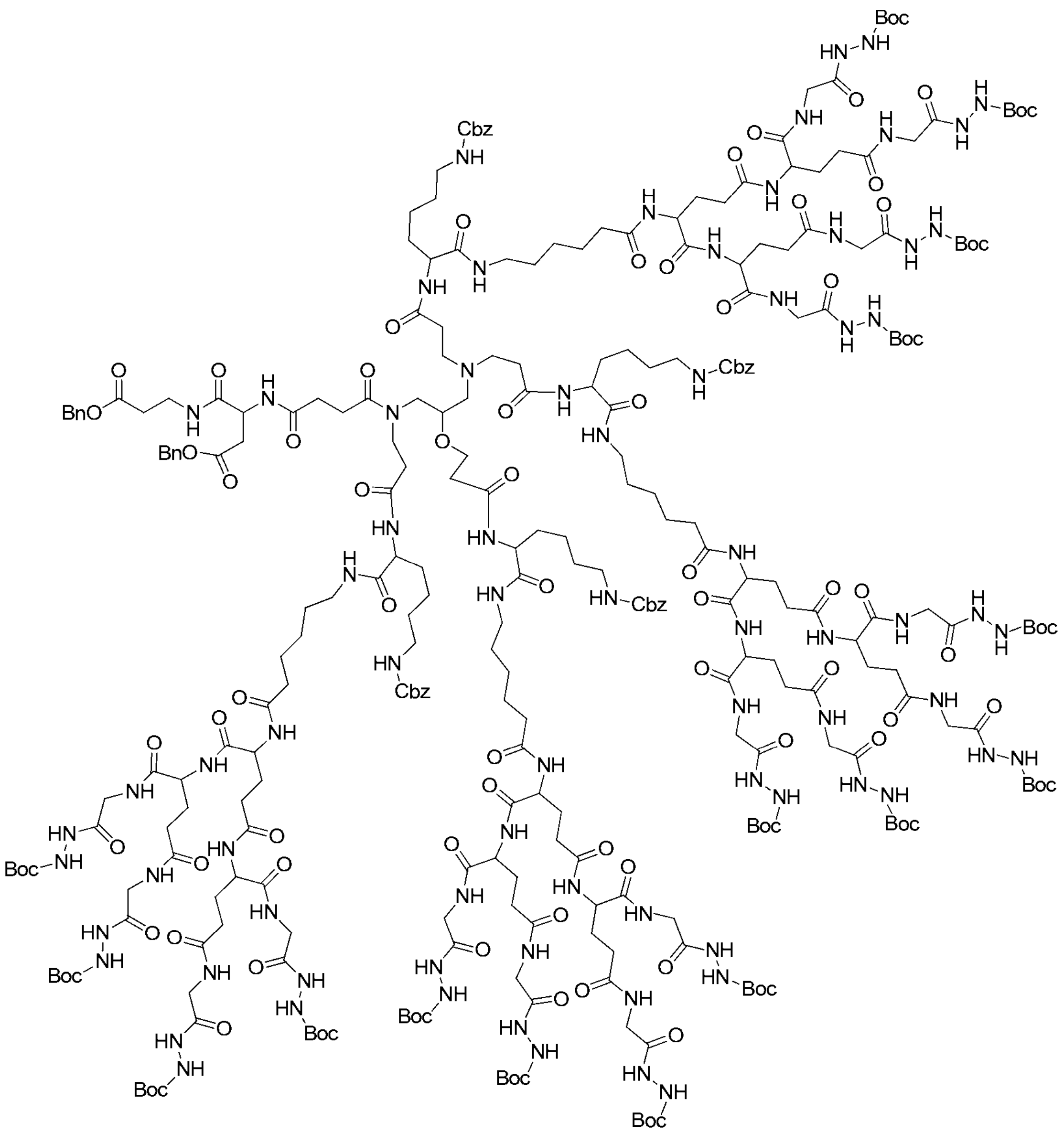 |

| | |
|---|---|
| **88-211** | 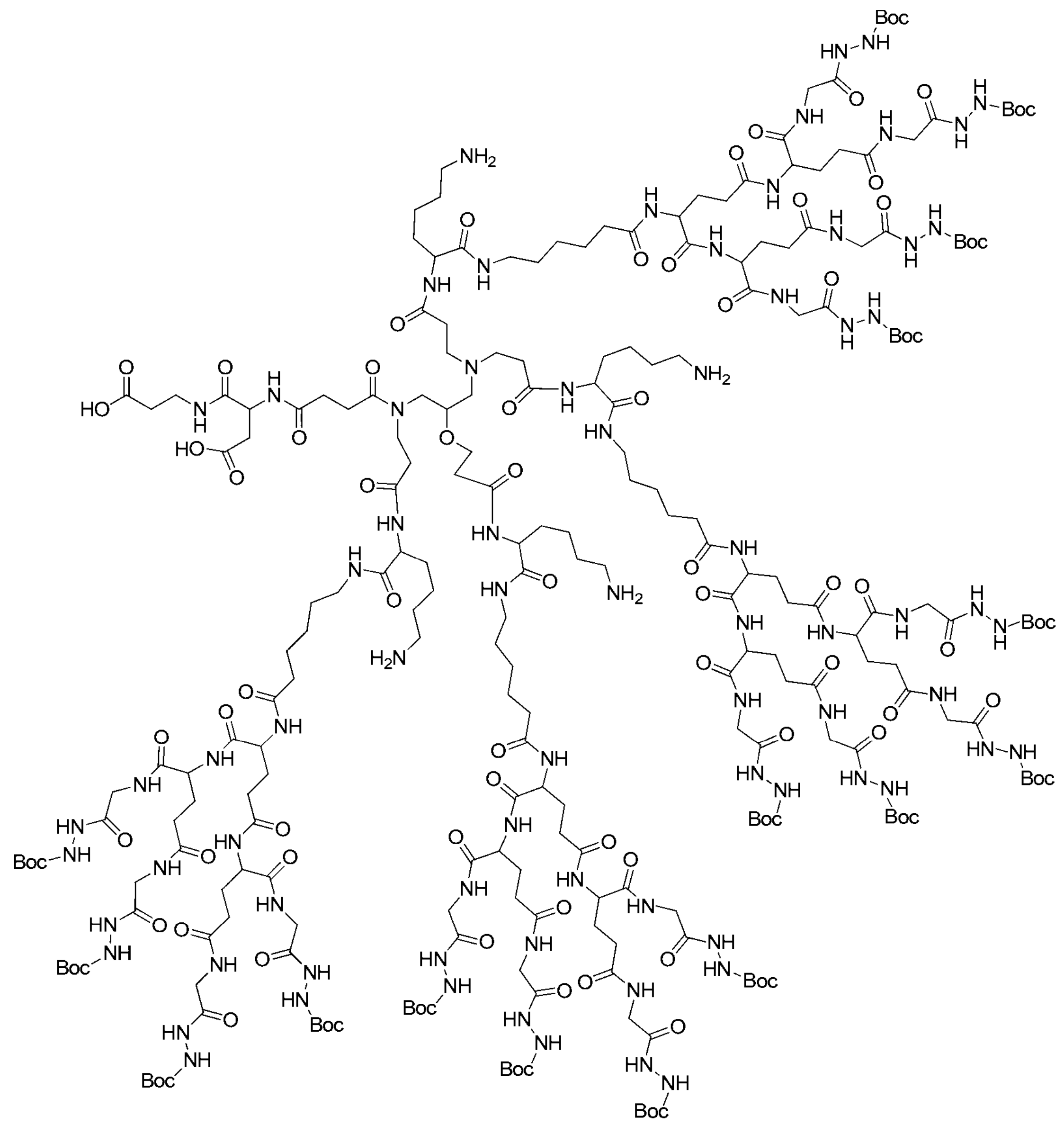 |

In another aspect, the present application provides a compound represented by formula XVIII or a pharmaceutically acceptable salt thereof,

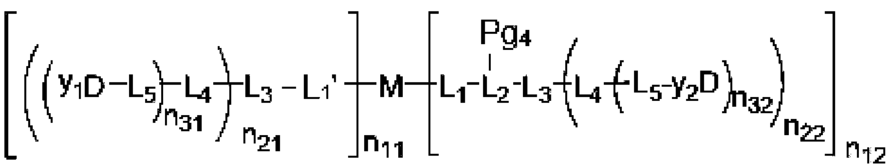

XVIII

wherein, each group is as defined in any of the preceding items.

In some embodiments, the compound is selected from the group consisting of:

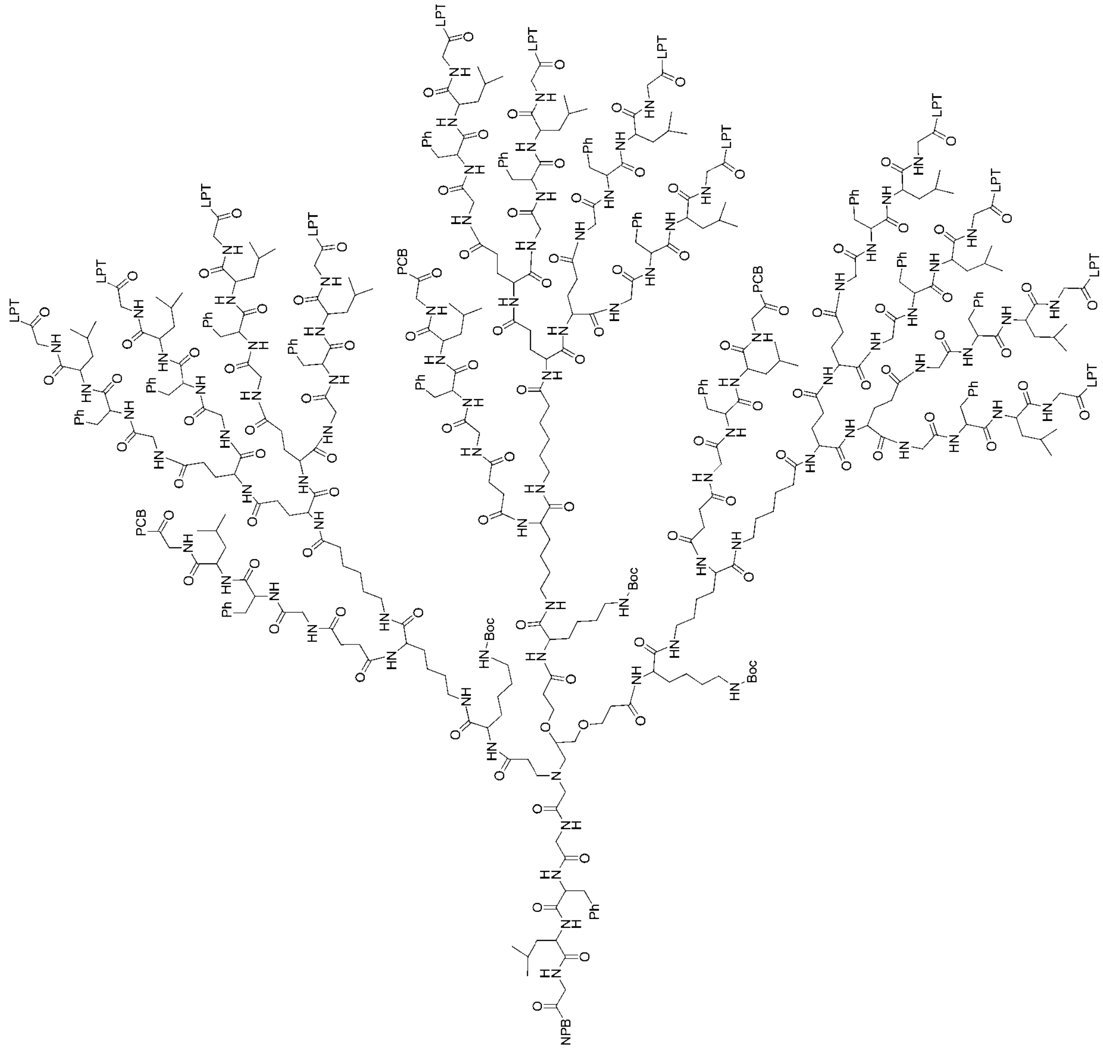

**76-171**

| | |
|---|---|
| 76-177 | 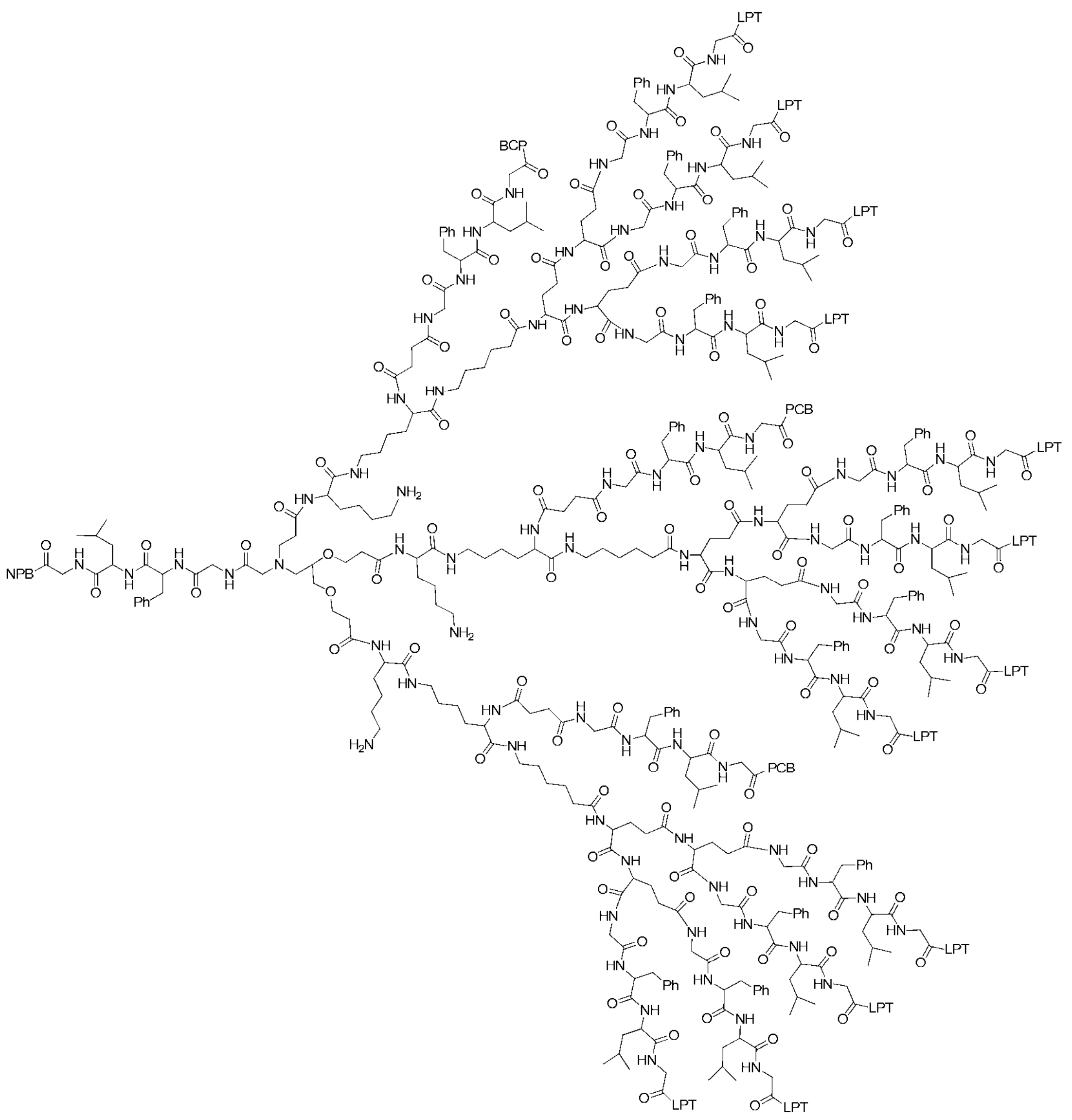 |

In another aspect, the present application provides a compound represented by formula XIX or a pharmaceutically acceptable salt thereof,

$$\left(Pg_1'\right)_{n_{11}} M \left[L_1 - Pg_2\right]_{n_{12}}$$

XIX

wherein, each group is as defined in any of the preceding items.

In some embodiments, the compound is selected from the group consisting of:

| | |
|---|---|
| **76-84** | **78-50** 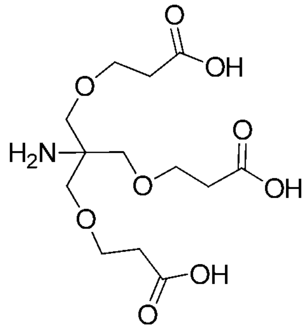 |
| 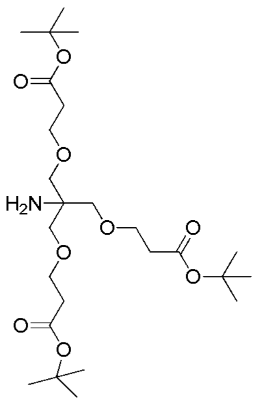 | |
| **71-222** 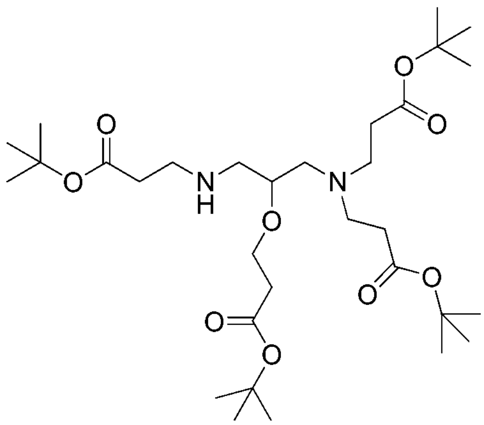 | **69-237** 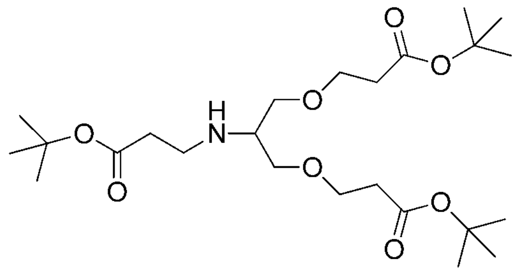 |
| **70-173** 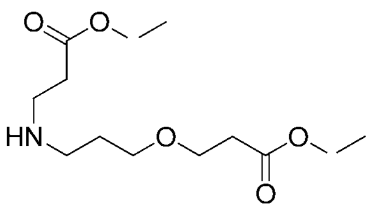 | **75-213** 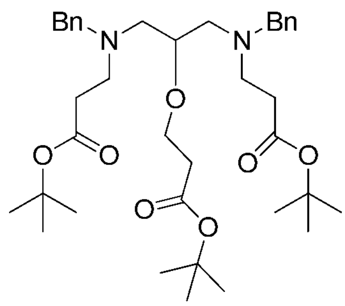 |
| **75-214** | **82-49** |

(continued)

| | |
|---|---|
| 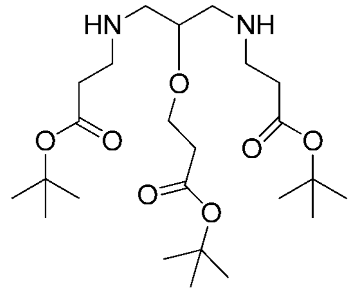 | 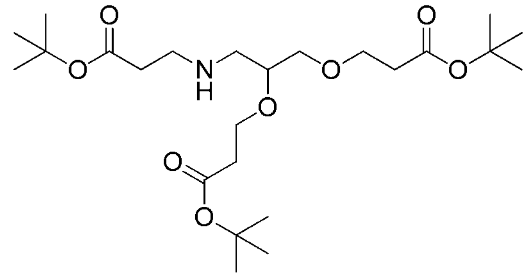 |
| **84-30** 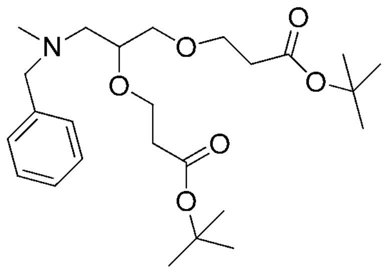 | **84-31** |
| | 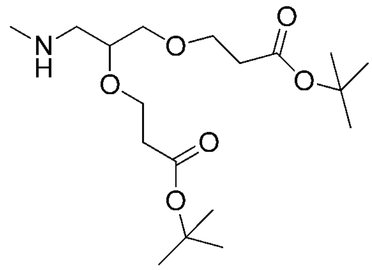 |
| **88-91** 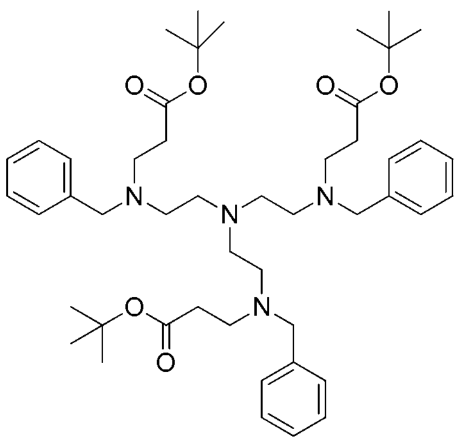 | **88-92** 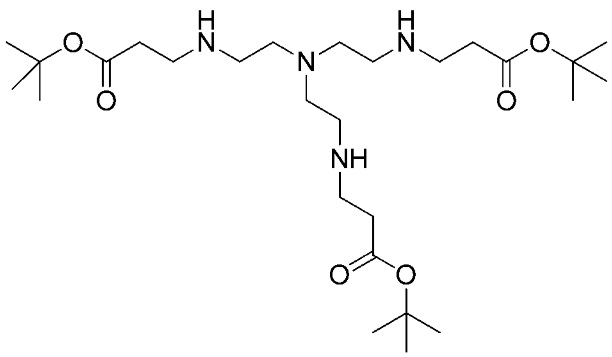 |
| **82-50** 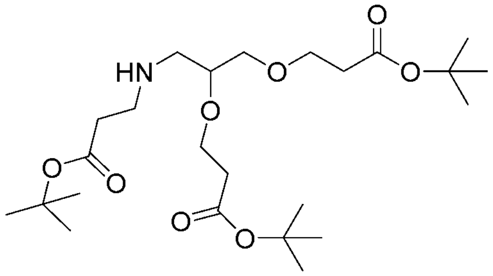 | **88-127** 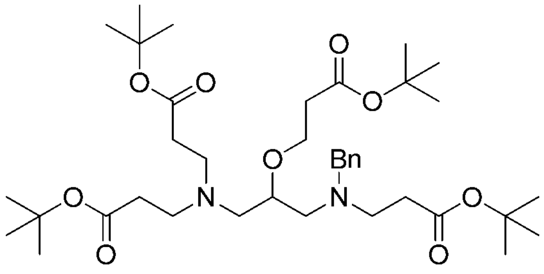 |
| **88-137** | |

(continued)

| | |
|---|---|
| 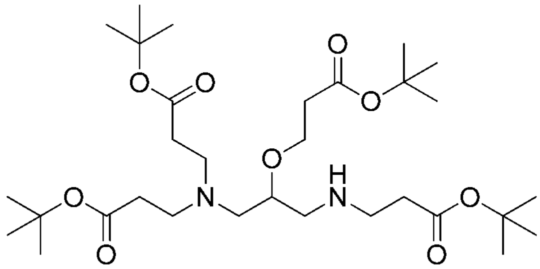 | |

In another aspect, the present application provides a compound represented by formula XX or a pharmaceutically acceptable salt thereof,

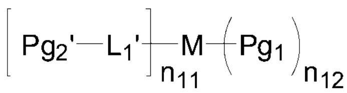

XX

wherein, each group is as defined in any of the preceding items.

In some embodiments, the compound is selected from the group consisting of:

| 81-60 | 59-224 |
|---|---|
| 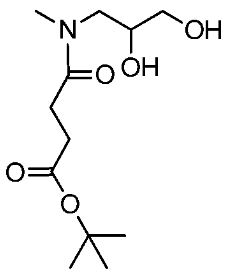 | 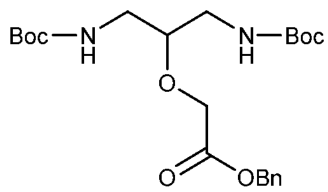 |
| **59-230** | |
| 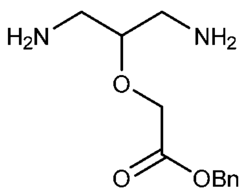 | |

## Pharmaceutical composition

In another aspect, the present application provides a pharmaceutical composition, which comprises the compound or pharmaceutically acceptable salt thereof described in any of the items of the present application in an amount effective for treating and/or preventing a disease.

In some embodiments, the composition further comprises one or more pharmaceutically acceptable excipients.

In some embodiments, the pharmaceutical composition is formulated as an injectable formulation.

The present application further provides an injectable solution, which comprises the compound or pharmaceutically acceptable salt thereof, or the pharmaceutical composition described in any of the items of the present application.

In some embodiments, the injectable solution uses physiological saline as a carrier.

## Medical use and therapeutic method

In another aspect, the present application provides a use of the compound or pharmaceutically acceptable salt

thereof described herein in the manufacture of a medicament for treating and/or preventing a disease (e.g., a cancer).

In another aspect, the present application provides the compound or pharmaceutically acceptable salt thereof described herein, which is used in treating and/or preventing a disease (e.g., a cancer).

In another aspect, the present application provides a method for treating and/or preventing a disease (e.g., a cancer), which comprises administering a therapeutically and/or prophylactically effective amount of the compound or pharmaceutically acceptable salt thereof described herein to a subject in need thereof.

Herein, the cancer is selected from the group consisting of colon cancer, leukemia, lymphoma, bladder cancer, bone cancer, brain tumor, medulloblastoma, glioma, breast cancer, adenoma/carcinoid, adrenocortical carcinoma, islet cell carcinoma, cervical cancer, endometrial cancer, ovarian cancer, colorectal cancer, skin cancer, esophageal cancer, eye cancer, gallbladder cancer, gastric cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid gland cancer, penile cancer, prostate cancer, urethral cancer, vaginal cancer, vulvar cancer, anal cancer, sarcoma, and metastases of the cancers.

## Preparation method

In another aspect, the present application also relates to a use of the intermediate described herein (including any one of the compounds represented by formula II to formula XX, or pharmaceutically acceptable salts thereof) in the manufacture of a medicament. In some embodiments, the medicament is selected from the group consisting of the compound represented by formula I or pharmaceutically acceptable salt thereof described herein.

Furthermore, the present application provides a method for preparing the compound represented by formula I and intermediates thereof.

In some embodiments, the compound represented by formula II is used as a raw material, and the Li or $L_1$' fragment is selectively connected to obtain a compound represented by formula XIX or XX. Further, the compound represented by formula XIX or XX is connected to the $L_1$' or Li fragment to obtain a compound represented by formula VIII.

In some embodiments, the $L_2$ fragment and the $L_3$ fragment are connected to obtain a compound represented by formula III.

In some embodiments, the $L_4$ fragment and the $L_5$ fragment are connected to obtain a compound represented by formula IV.

In some embodiments, the compound represented by formula V is obtained by the following method:

(1) reacting the compound represented by formula III with the compound represented by formula IV;
(2) sequentially connecting the compound represented by formula III with the $L_4$ fragment and the $L_5$ fragment;
(3) sequentially connecting the compound represented by formula IV with the $L_3$ fragment and the $L_2$ fragment.

In some embodiments, the compound represented by formula VIII is reacted with the compound represented by formula III, or sequentially reacted with the $L_2$ fragment and the $L_3$ fragment to obtain the compound represented by formula IX.

In some embodiments, the compound represented by formula IX is connected to the $L_4$ fragment to obtain the compound represented by formula X.

In some embodiments, the compound represented by formula IX is reacted with the compound represented by formula IV to obtain the compound represented by formula XI.

In some embodiments, the compound represented by formula VIII is connected to the $L_3$ fragment, or the compound represented by formula XIX is connected to the $L_1$'-L3 fragment to obtain the compound represented by formula XIII.

In some embodiments, the compound represented by formula VIII is connected to the $L_5$-$y_1$D fragment to obtain the compound represented by formula XIV, wherein $L_3$ and $L_4$ are both a bonds.

In some embodiments, the compound represented by formula XV is obtained by the following method:

(1) the compound represented by formula IX is connected to the $L_3$ fragment, the $L_4$ fragment and the $L_5$ fragment in sequence;
or, (2) the compound represented by formula IX is reacted with the

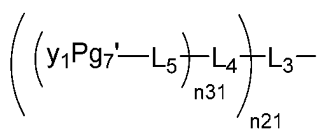

fragment.

In some embodiments, the compound represented by formula XIV is reacted with the compound represented by formula III to obtain the compound represented by formula XVI.

In some embodiments, the compound represented by formula XIII is reacted with the compound represented by formula V to obtain the compound represented by formula XVII.

In some embodiments, the compound represented by formula XIV is reacted with the compound represented by formula VII to obtain the compound represented by formula XVIII.

In each of the above embodiments, each fragment comprises its protected form or deprotected form. The choice of protecting group can be determined based on common knowledge in the art, or as defined above.

In the above embodiments, a step of amino or carboxyl group protection, or a step of amino or carboxyl group deprotection is optionally included before or after each reaction.

In some embodiments, the method for preparing the compound represented by formula I is selected from the group consisting of the following routes:

Route 1:

(1) reacting the compound represented by formula XII with PEG connected with an activating group to obtain Intermediate 1-1;

(2) reacting Intermediate 1-1 obtained in step (1) with

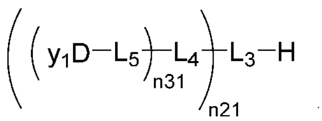

to obtain the compound represented by formula I;

Route 2:

(1) reacting the compound represented by formula IX with PEG connected with an activating group to obtain Intermediate 2-1;

(2) reacting Intermediate 2-1 obtained in step (1) directly with

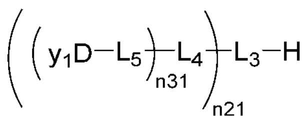

,

or connecting step-by-step the fragments of

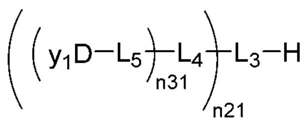

(e.g., connecting step-by-step $L_3$, $L_4$, $L_5$ and D, or $L_3$-$(L_4)_{n21}$, $L_5$ and D, or $L_3$-$(L_4)_{n21}$, $L_5$-$y_1$D, or $L_3$-$(L_4$-$(L_5)_{n31})_{n21}$ and D) to obtain Intermediate 2-2;

(3) reacting Intermediate 2-2 obtained in step (2) directly with the compound represented by formula VI, or reacting step-by-step with the compound represented by formula IV and the cytotoxic drug, or connecting step-by-step $L_4$, $L_5$-$y_2$D, or connecting step-by-step $L_4$, $L_5$ and D to obtain the compound represented by formula I;

or, the order of steps (2) and (3) is exchanged;

Route 3:

(1) reacting Intermediate 2-1 obtained in step (1) of Route 2 with the compound represented by formula VI to obtain Intermediate 3-1:

(2) reacting Intermediate 3-1 obtained in step (1) with

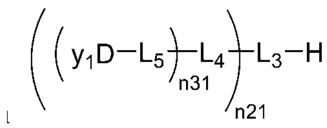

to obtain the compound represented by formula I;

Route 4:

(1) reacting the compound represented by formula X with PEG connected with an activating group to obtain Intermediate 4-1;
(2) reacting Intermediate 4-1 obtained in step (1) with

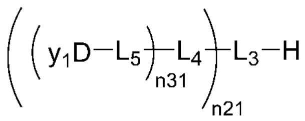

to obtain Intermediate 4-2, wherein $n_{31}'+n_{31}''=n_{31}$;
(3) reacting Intermediate 4-2 obtained in step (2) with H-$L_5$-$y_2$D to obtain Intermediate 4-3;
(4) reacting Intermediate 4-3 obtained in step (3) with H-$L_5$-$y_1$D to obtain the compound represented by formula I;

or, replacing

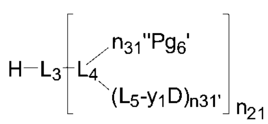

in step (2) with

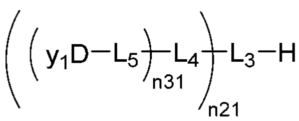

,

wherein case step (4) is not performed;
or, using the compound represented by formula XII as a raw material, performing steps (1), (2) and (4) to obtain the compound represented by formula I;

Route 5:

(1) reacting the compound represented by formula XI with PEG connected with an activated group to obtain Intermediate 5-1;
(2) reacting Intermediate 5-1 obtained in step (1) directly with

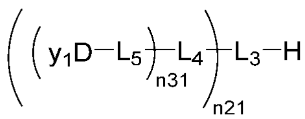

,

or connecting step-by-step the fragments of

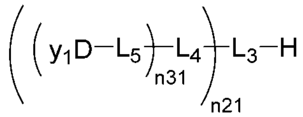

(e.g., connecting step-by-step $L_3$, $L_4$, $L_5$ and D, or $L_3$-$(L_4)_{n21}$, $L_5$ and D, or $L_3$-$(L_4)_{n21}$ and $L_5$-$y_1$D, or $L_3$-$(L_4$-$(L_5)_{n31})_{n21}$ and D), to obtain Intermediate 5-2;
(3) reacting Intermediate 5-2 obtained in step (2) with the cytotoxic drug to obtain the compound represented by formula I;

Route 6:

(1) reacting the compound represented by formula XIV with the compound represented by formula VII to obtain Intermediate 6-1;
(2) reacting Intermediate 6-1 obtained in step (1) with PEG connected with an activating group to obtain the compound represented by formula I;

Route 7:

(1) reacting the compound represented by formula XIII with PEG connected with an activating group to obtain Intermediate 7-1;
(2) reacting Intermediate 7-1 obtained in step (1) with H-$L_4$ to obtain Intermediate 7-2;
(3) reacting Intermediate 7-2 obtained in step (2) with

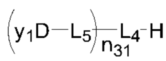

to obtain Intermediate 7-3;
(4) reacting Intermediate 7-3 obtained in step (3) with $L_5$-$y_2$$Pg_7$ or $L_5$'-$y_2$$Pg_7$ to obtain Intermediate 7-4;
(5) reacting Intermediate 7-4 obtained in step (4) with the cytotoxic drug or $L_5$"-$y_2$D to obtain the compound represented by formula I; wherein $L_5$' and $L_5$" are connected to form the $L_5$;

Route 8:

(1) reacting the compound represented by formula XV with PEG connected with an activating group to obtain Intermediate 8-1;
(2) reacting Intermediate 8-1 obtained in step (1) with the compound represented by formula VI to obtain Intermediate 8-2;
(3) reacting Intermediate 8-2 obtained in step (3) with the cytotoxic drug to obtain the compound represented by formula I;

Route 9:

(1) reacting the compound represented by formula XVII with PEG connected with an activating group to obtain Intermediate 9-1;
(2) reacting Intermediate 9-1 obtained in step (1) with H-$L_5$-$y_1$D to obtain Intermediate 9-2;
(3) reacting Intermediate 9-2 obtained in step (2) with the cytotoxic drug to obtain the compound represented by formula I;

Route 10:

(1) reacting the compound represented by formula XIV with the compound represented by formula III to obtain Intermediate 10-1;
(2) reacting Intermediate 10-1 obtained in step (1) with PEG connected with an activating group to obtain Intermediate 10-2;
(3) reacting Intermediate 10-2 obtained in step (2) with H-$L_4$-$n_{32}$$Pg_6$' to obtain Intermediate 10-3;
(4) reacting Intermediate 10-3 obtained in step (3) with H-$L_5$-$y_2$D to obtain the compound represented by formula I;

optionally, before or after the reaction of any step of Routes 1 to 10, a step of removal of the protecting group and/or activation (e.g., carbonyl activation) is further included;

preferably, the PEG activating group is

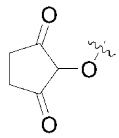

;

the remaining compounds and groups are as defined in any of the preceding items.

Definition of terms

Unless otherwise defined below, all technical and scientific terms used herein are intended to have the same meaning as commonly understood by those skilled in the art. References to technology used herein are intended to refer to technology commonly understood in the art, including those variations or equivalent technical substitutions that are obvious to those skilled in the art. Although the following terms are believed to be well understood by those skilled in the art, the following definitions are set forth to better explain the present invention.

The terms "comprise", "include", "have", "contain", or "involve" and variations thereof herein are inclusive or open-ended and do not exclude other unrecited elements or method steps.

As used herein, the term "pharmaceutically acceptable carrier" refers to a carrier that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995) and includes, but is not limited to, pH regulator, surfactant, adjuvant, ionic strength enhancer, diluent, agent for maintaining osmotic pressure, agent for delaying absorption, and preservative. For example, the pH regulator includes, but is not limited to, phosphate buffer. The surfactant includes, but is not limited to, cationic, anionic, or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol, and polyol (e.g., glycerol). The preservative includes, but is not limited to, various antibacterial and antifungal agents, such as thimerosal, 2-phenoxyethanol, paraben, chlorobutanol, phenol, and sorbic acid. Stabilizers have the meaning generally understood by those skilled in the art and are capable of stabilizing the desired activity of the active ingredient in a pharmaceutical product, including, but not limited to, sodium glutamate, gelatin, SPGA, sugar (e.g., sorbitol, mannitol, starch, sucrose, lactose, dextran, or glucose), amino acid (e.g. glutamic acid and glycine), protein (e.g., dried whey, albumin, or casein) or degradation product thereof (e.g., lactalbumin hydrolysate), etc.

As used herein, the term "prevention" refers to a method performed to prevent or delay the onset of a disease, condition, or symptom (e.g., a tumor) in a subject.

As used herein, the term "treatment" refers to a method performed to achieve a beneficial or desired clinical outcome. For purposes of the present invention, beneficial or desired clinical results include, but are not limited to, alleviation of symptoms, reduction in the extent of disease, stabilization (i.e., no longer worsening) of the disease state, delaying or slowing the progression of disease, improvement or alleviation of the disease state, and alleviation of symptoms (whether partial or complete), whether detectable or undetectable. Furthermore, "treatment" may also refer to prolonging survival compared to expected survival if not receiving treatment.

As used herein, the term "subject" refers to a mammal, such as a primate, such as a human. In certain embodiments, the subject (e.g., a human) has a tumor, or is at risk for such a disease.

As used herein, the term "effective amount" refers to an amount sufficient to achieve, or at least partially achieve, a desired effect. For example, an effective amount for preventing a disease (e.g., a tumor) means an amount sufficient to prevent, arrest, or delay the onset of a disease (e.g., a tumor); an effective amount for treating a disease means an amount sufficient to cure or at least partially arrest the disease and its complications in a patient already suffering from the disease. Determination of such an effective amount is well within the capabilities of those skilled in the art. For example, the effective amount for therapeutic use will depend on the severity of the disease being treated, the overall state of the patient's own immune system, the patient's general condition such as age, weight, and sex, the method of drug administration, and any other concurrently administered therapies.

The terms "cancer" and "tumor" are used interchangeably to refer to a broad class of diseases characterized by the uncontrolled growth of abnormal cells in the body. Unregulated cell division may lead to the formation of malignant

tumors, or cells that invade adjacent tissues and may metastasize to distant sites in the body via the lymphatic system or bloodstream. Cancer includes both benign and malignant cancers, as well as dormant tumors or micrometastases. Cancers also include hematologic tumors, particularly hematologic malignancies.

The term "hydrocarbonyl" refers to a group obtained by losing a hydrogen atom from a corresponding hydrocarbon, including monovalent, divalent, and trivalent hydrocarbonyl groups. For example, $C_{2-10}$ trivalent hydrocarbonyl and $C_{2-6}$ trivalent saturated hydrocarbonyl.

The term "alkyl" is defined as a straight-chain or branched saturated aliphatic hydrocarbonyl. In some embodiments, the alkyl has 1 to 12, for example, 1 to 6, carbon atoms. For example, as used herein, the term "$C_{1-6}$ alkyl" refers to a linear or branched group of 1 to 6 carbon atoms (e.g., methyl, ethyl, n-propyl, isopropyl, n-butyl, isobutyl, sec-butyl, tert-butyl, n-pentyl, and n-hexyl).

The term "alkylamino" refers to a group having the structure "alkyl-NH-," where alkyl is as defined above. Examples include $C_{1-6}$ alkylamino, $C_{1-4}$ alkylamino, $C_{1-3}$ alkylamino, and $C_{1-2}$ alkylamino. Common alkylamino groups include (but are not limited to) methylamino, ethylamino, n-propylamino, isopropylamino, n-butylamino, isobutylamino, tert-butylamino, pentylamino, and hexylamino.

The structural formula of PTX is:

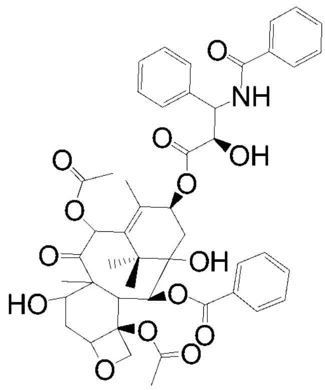

The structural formula of PCB is:

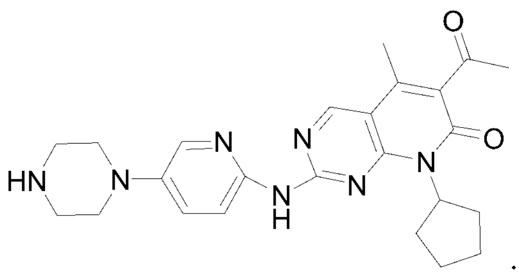

The structural formula of SN38 is:

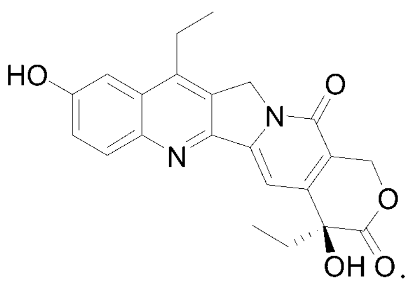

The structural formula of NPB is:

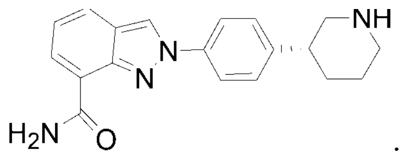

.

The structural formula of AXT is:

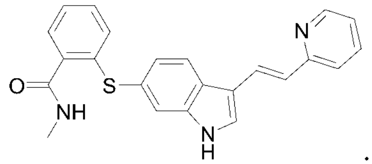

.

The structural formula of LPT is:

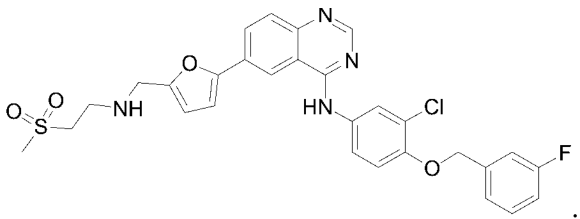

.

The structural formula of DOX is:

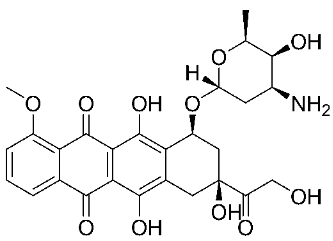

.

The structural formula of Ac-C-PLGLAG-iRGD is:

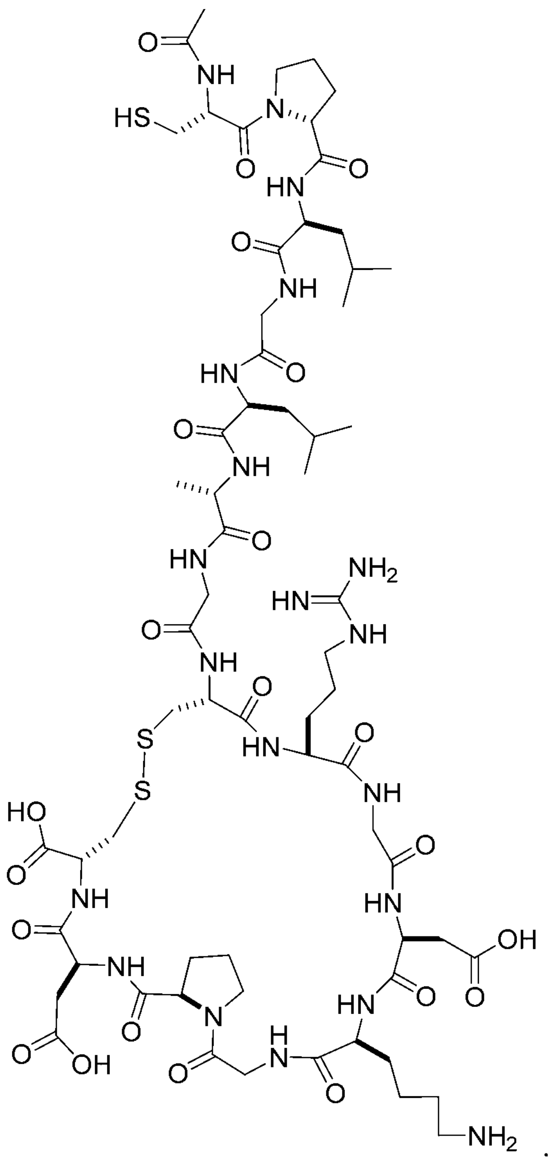

The structural formula of SB7 is:

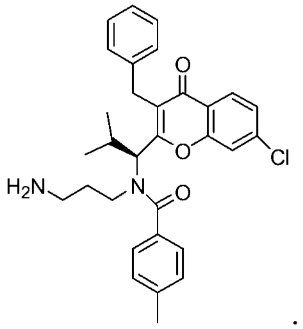

The structural formula of folic acid is:

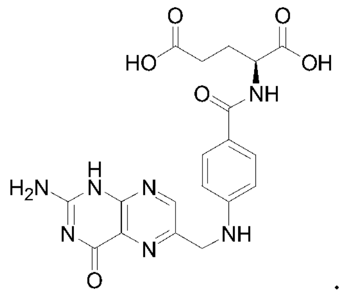

.

The structural formula of sodium dodecahydrododecaborate is:

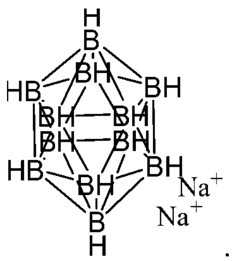

.

The structural formula of IRN is:

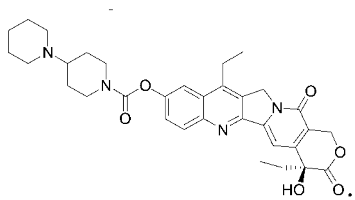

.

As used herein, the term "amino acid" primarily includes the following 20 common amino acids: alanine (Ala), arginine (Arg), aspartic acid (Asn), asparagine (Asp), cysteine (Cys), glutamine (Gln), glutamic acid (Glu), glycine (Gly), histidine (His), leucine (Leu), isoleucine (Ile), lysine (Lys), methionine (Met), phenylalanine (Phe), proline (Pro), serine (Ser), threonine (Thr), tryptophan (Trp), tyrosine (Tyr), and valine (Val). With the exception of glycine, all other amino acids have the L-configuration. Amino acids are known to contain both amino and carboxyl functional groups. The term "amino acid residue" herein refers to a residue remaining after removing a hydrogen from the amino group and/or a hydroxyl from the carboxyl group.

## Specific Models for Carrying Out the present invention

The following examples describe the embodiments of the present disclosure in detail. However, those skilled in the art should understand that the following examples are merely illustrative of the embodiments of the present disclosure and should not be construed as limiting the scope of the present disclosure. For examples where specific conditions are not specified, standard conditions or manufacturer-recommended conditions are followed. Reagents and instruments used without manufacturer identification are commercially available conventional products.

Example 1: Synthesis of Compound **81-214**

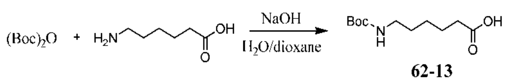


**62-13**

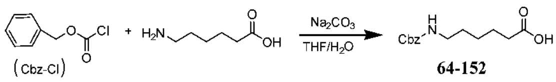


**64-152**

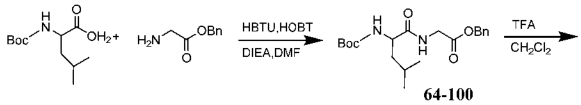


64-100

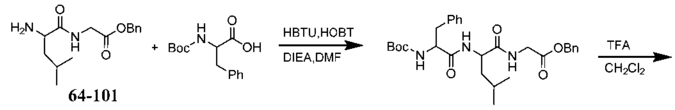


64-101

64-102

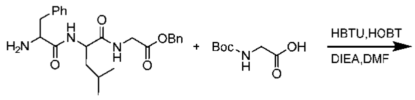


64-105

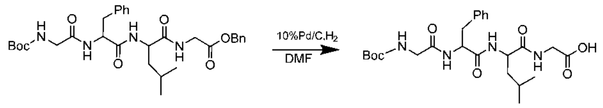


64-106

71-85

71-85 +

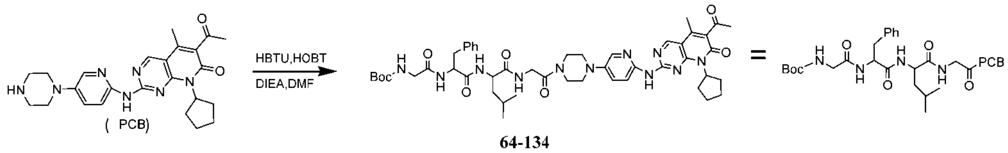


64-134

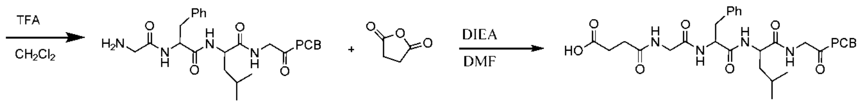


64-135

81-189

71-85 +

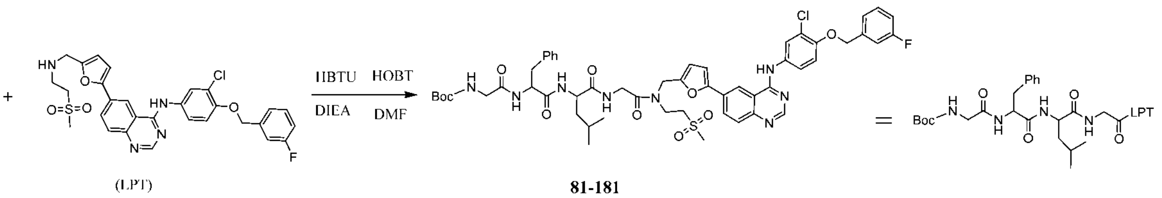


81-181

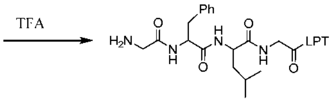


81-128

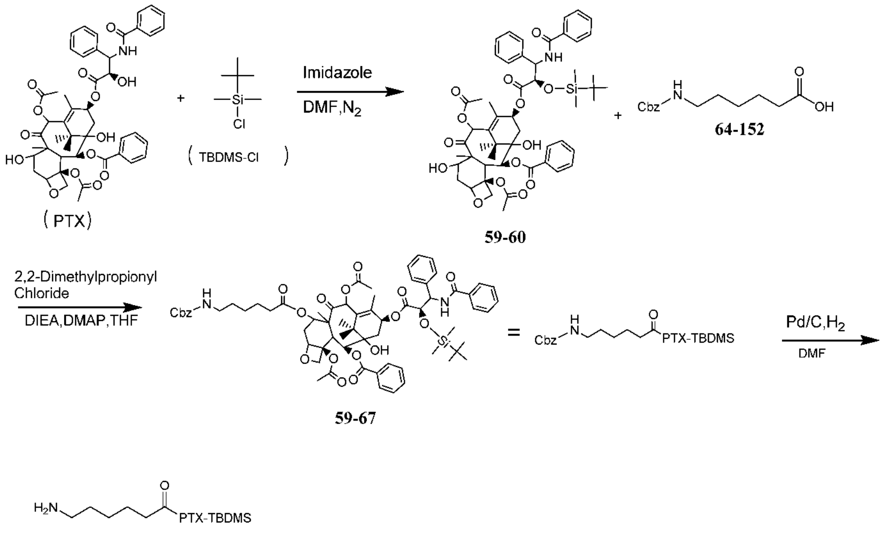


(PTX)

(TBDMS-Cl)

59-60

64-152

59-67

59-69

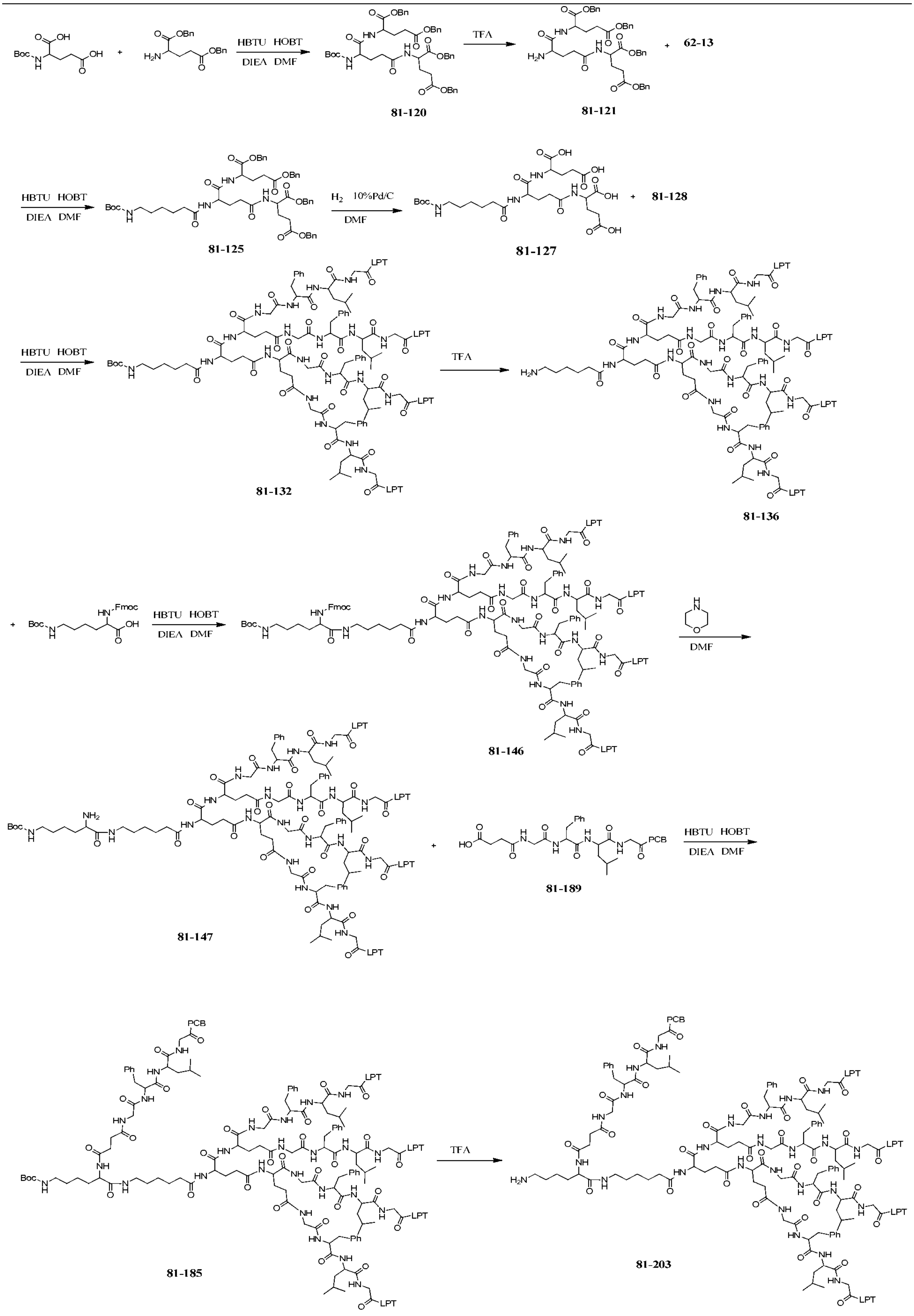


81-120

81-121

81-125

81-127

81-132

81-136

81-146

81-147

81-189

81-185

81-203

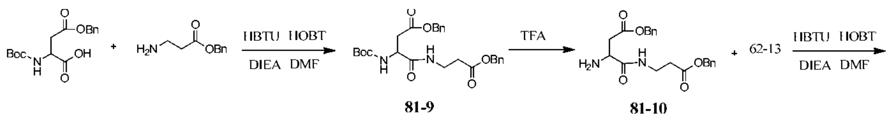


81-9

81-10

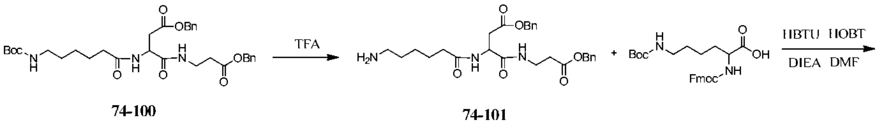


74-100

74-101

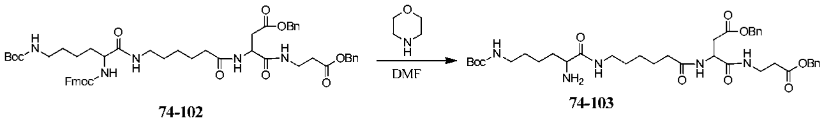


74-102

74-103

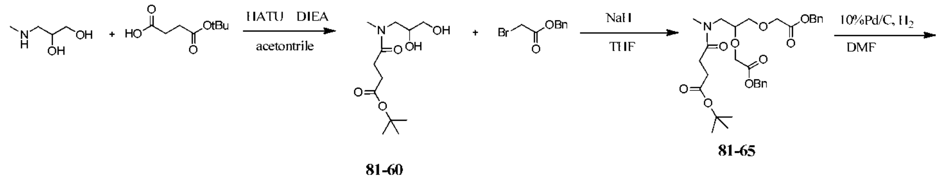


81-60

81-65

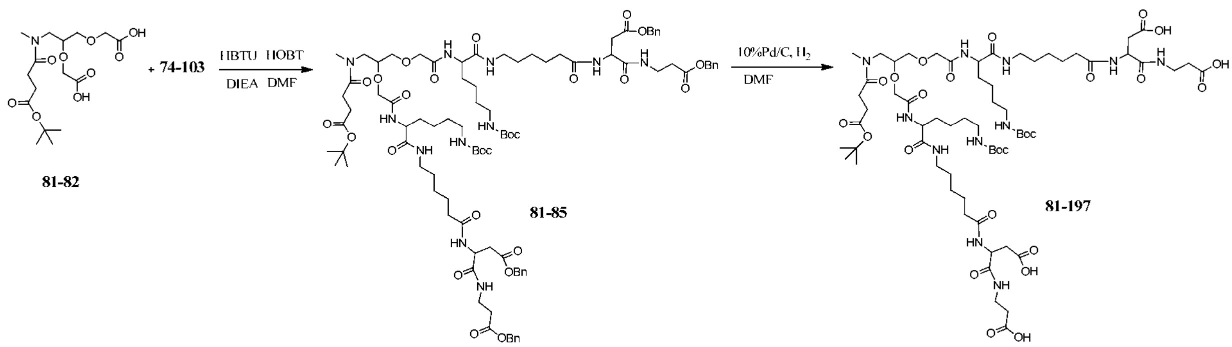


81-82

81-85

81-197

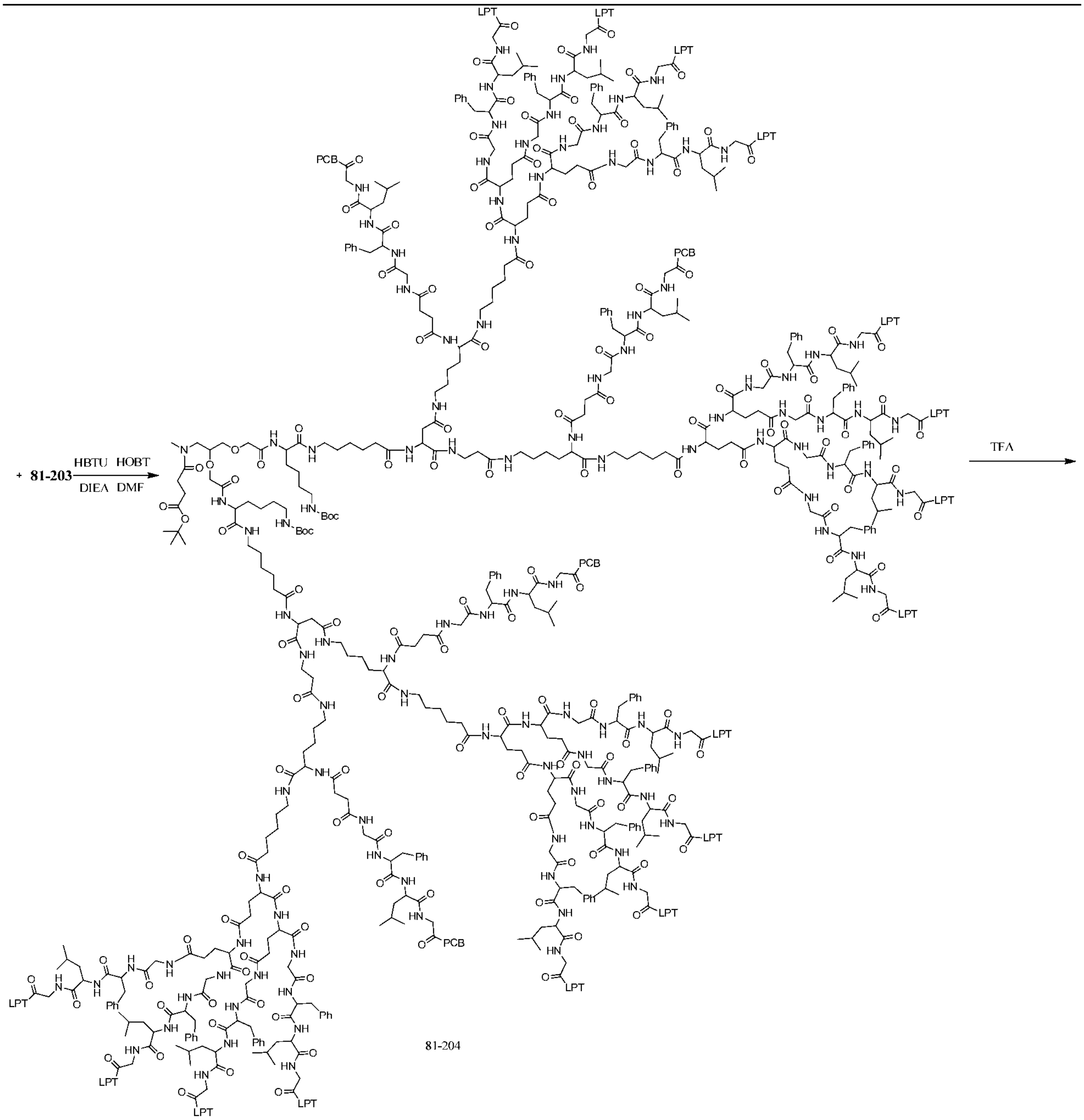

+ 81-203
HBTU HOBT
DIEA DMF
TFA
81-204

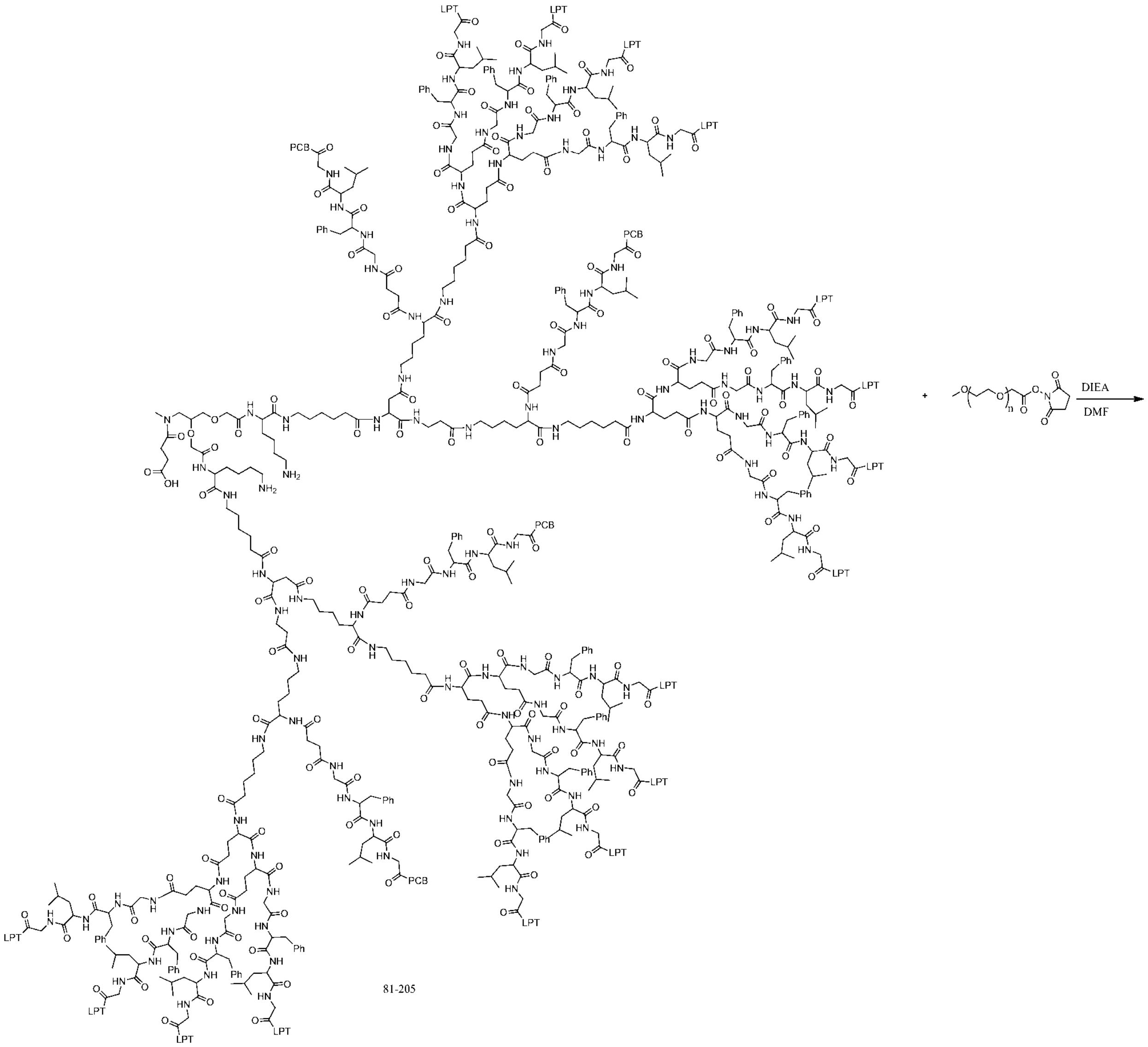


81-205

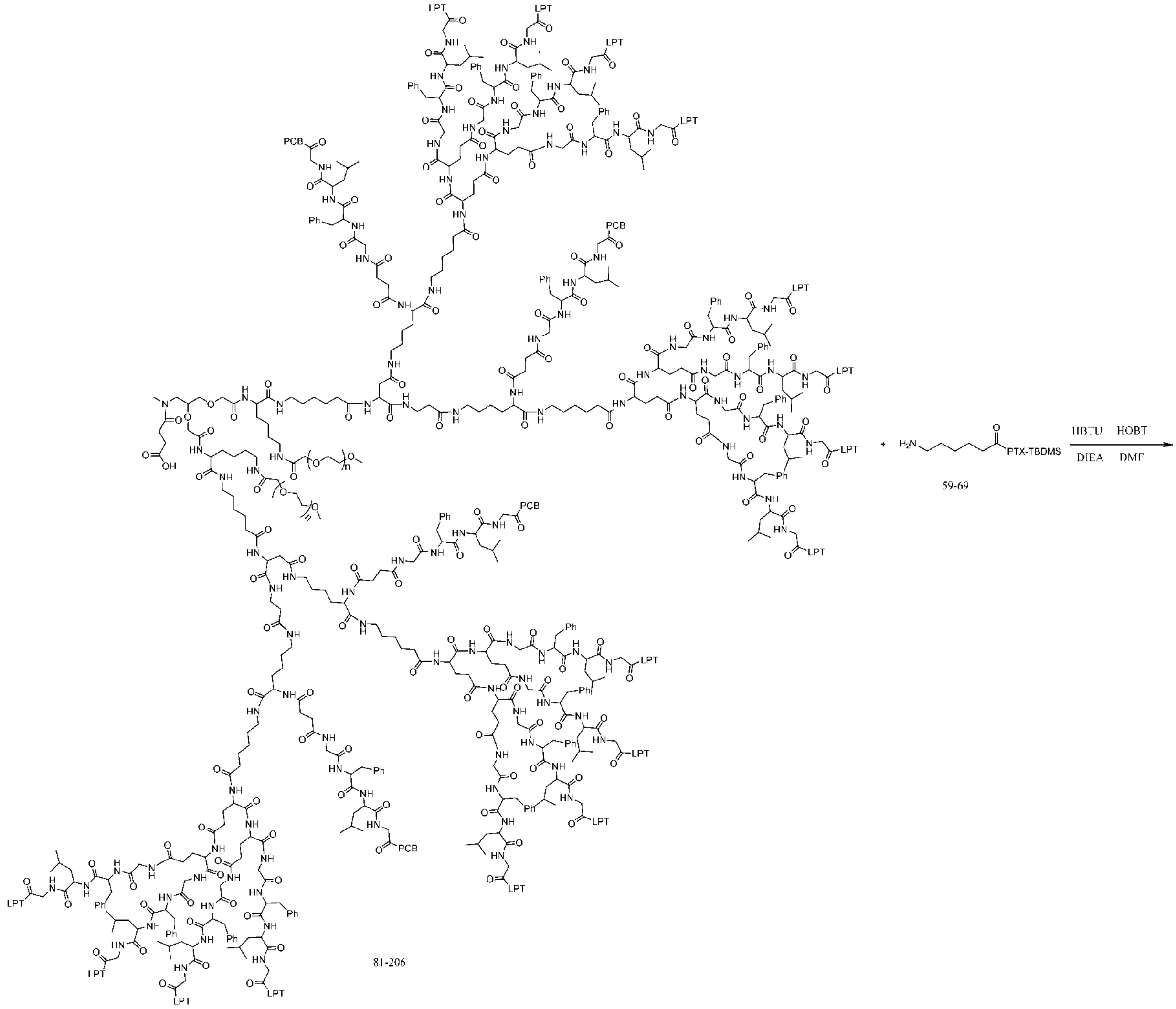


81-206

59-69

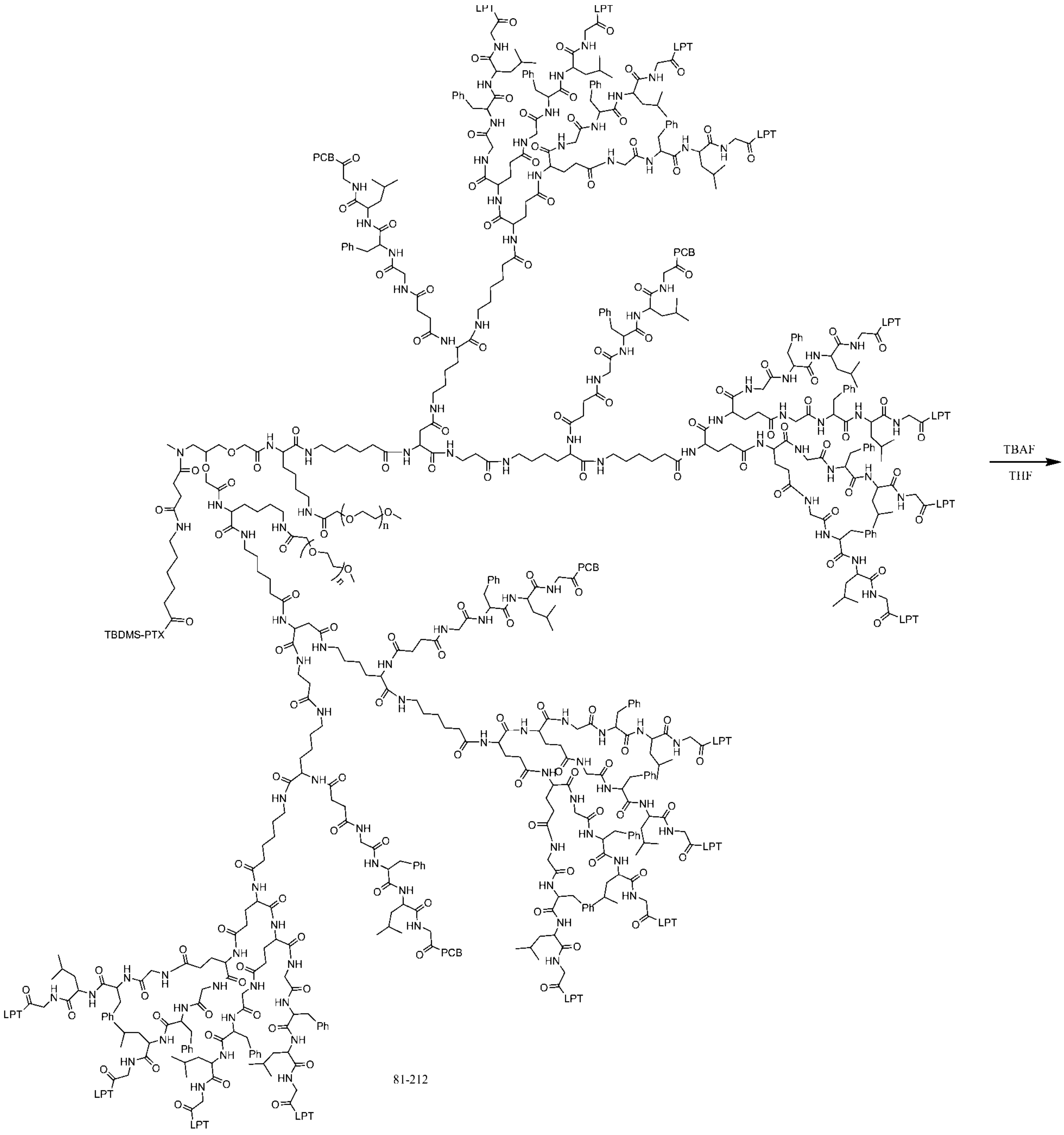


81-212

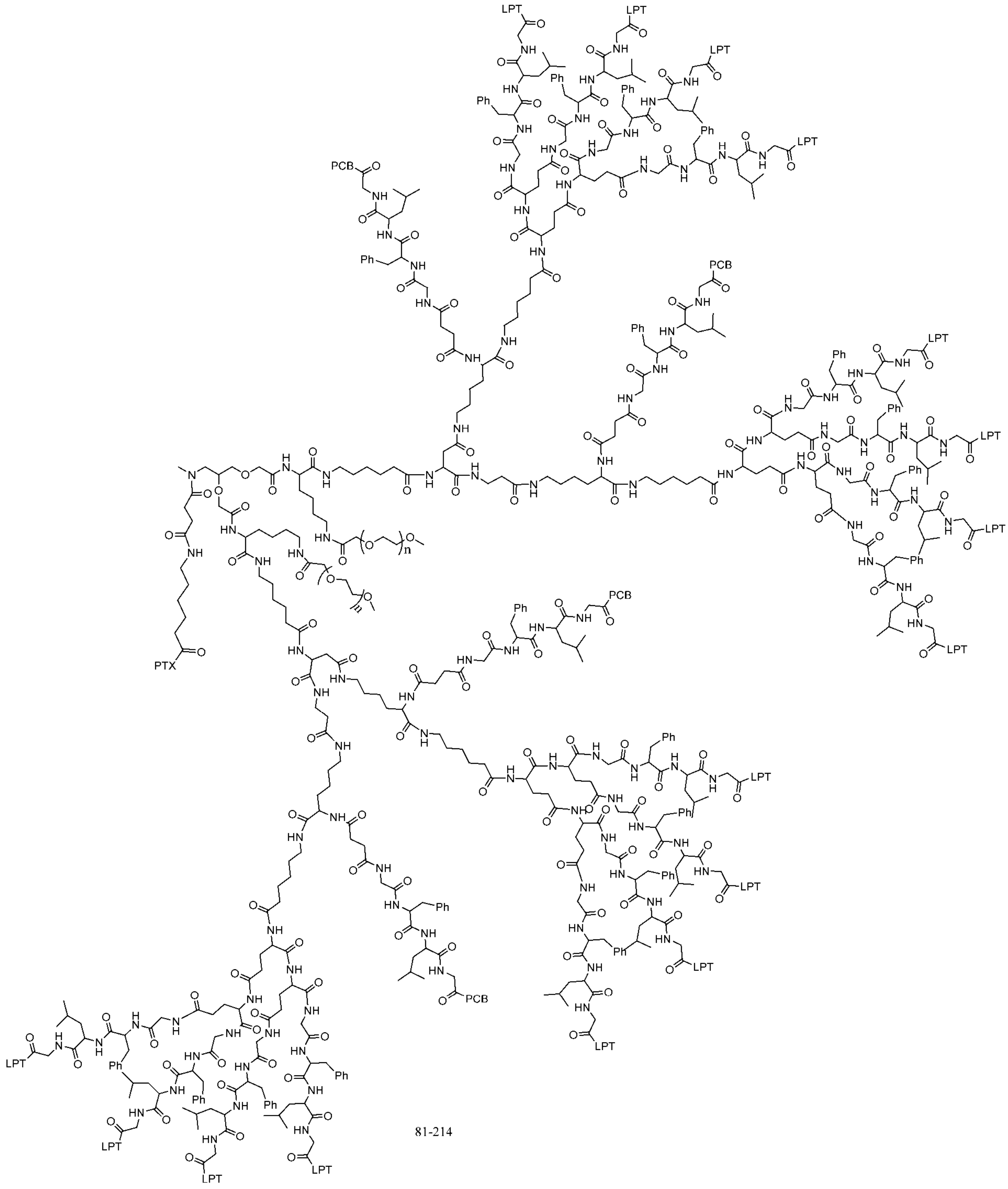

81-214

1.1 Preparation of Compound **62-13**

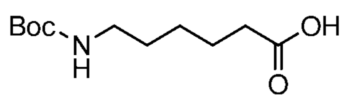

6-Aminohexanoic acid (15 g, 114.3554 mmol), 1,4-dioxane (200 mL), and pure water (100 mL) were added to a 1 L round-bottom flask, and dissolution was assisted by ultrasonication. The mixture was stirred at -5°C for 30 minutes. An aqueous sodium hydroxide solution (5 g NaOH + 20 mL pure water) was added, and the mixture was stirred at -5°C for 30

minutes. Di-tert-butyl dicarbonate (34.9 g, 160.09 mmol) was then added, and the reaction was allowed to stir at room temperature overnight. After the reaction was completed, the reaction mixture was transferred to a 2 L separatory funnel, and extracted by adding pure water (300 mL) and ethyl acetate (350 mL). Dilute hydrochloric acid was added dropwise to adjust the pH until acidic. The organic phase was separated, and the aqueous phase was extracted with ethyl acetate (200 mL × 3). The combined organic phases were concentrated and evaporated to dryness to provide 24 g of the product.

1.2 Preparation of Compound **64-152**

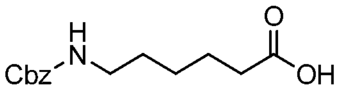

6-Aminohexanoic acid (9.19 g, 70.06 mmol) was placed in a 1 L round-bottom flask, THF/$H_2O$=1:1 solution (150 mL) was added for dissolution, and then the mixture was stirred in a 0°C reaction bath for 1 hour. Anhydrous sodium carbonate (14.85 g, 140.12 mmol) was then added, and dissolution was assisted by ultrasonication. The reaction flask was then placed in a 0°C reaction bath, and stirring was performed for another 30 minutes. Cbz-Cl (12.5494 g, 73.56 mmol) was then dissolved in THF (30 mL) and slowly added dropwise to the reaction flask. After the addition was completed, the reaction flask was removed from the 0°C reaction bath and stirring was performed at room temperature for 2.5 hours. After the reaction was completed, the mixture was extracted by adding ethyl acetate (200 mL) and purified water (100 mL). The pH was adjusted to a slightly acidic state with dilute hydrochloric acid, and the organic phase was collected. The aqueous phase was then extracted again with ethyl acetate (200 mL). The organic phases were combined, concentrated, and dehydrated over anhydrous magnesium sulfate, then 200-300 mesh silica gel powder (25 g) was added, and the mixture was evaporated to dryness. The loading was performed under dry state, and column chromatography was carried out using 30-70% ethyl acetate/petroleum ether as the eluent. The desired product was collected, concentrated, and dried to provide 11.6 g of the product, with a yield of 62.41%.

1.3 Preparation of **64-100**

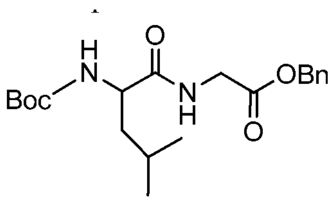

Boc-L-Leucine (30 g, 129.70 mmol), benzyl glycine hydrochloride (28.77 g, 142.67 mmol), HBTU (73.78 g, 194.56 mmol) and HOBT (26.28 g, 194.56 mmol) were added to a 1000 mL flask, DMF (50 mL) was added for dissolution, and the mixture was stirred at -5°C for approximately 20 minutes. DIEA (96.5 mL, 583.68 mmol) was then slowly added dropwise. After the addition was completed, stirring was continued at -5°C for 1 hour. The mixture was then brought to room temperature and stirred for reaction. After the reaction was completed, the reaction mixture was transferred to a 2 L separatory funnel and extracted by adding saturated sodium bicarbonate solution (250 mL) and ethyl acetate (300 mL). The aqueous phase was then extracted with ethyl acetate (200 mL × 3). The organic phases were combined, washed with saturated brine (200 mL × 2), dehydrated over anhydrous magnesium sulfate, concentrated, and evaporated to dryness to provide 50.9873 g of the product.

1.4 Preparation of **64-101**

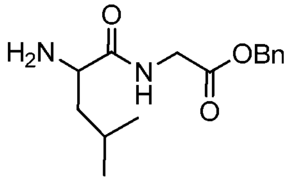

**64-100** (49.08 g, 129.70 mmol) was placed in a 1000 mL flask, and dichloromethane (60 mL) was added for dissolution. Then TFA (96.3 mL, 1297.0 mmol) was added under stirring state, and the reaction mixture was stirred at room temperature overnight. Upon completion of the reaction, the reaction mixture was concentrated and poured into a 2 L separatory funnel filled with saturated sodium bicarbonate solution (350 mL) and ethyl acetate (300 mL) for extraction. The organic phase was collected, and the aqueous phase was extracted again with ethyl acetate (150 mL × 2). The organic phases were combined, concentrated, dehydrated over anhydrous magnesium sulfate, and evaporated to dryness to provide 29.3 g of the product.

1.5 Preparation of **64-102**

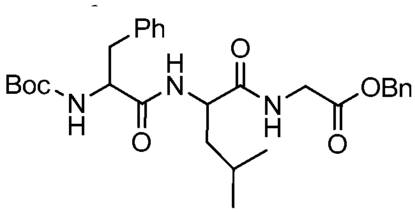

**64-101** (29.3 g, 105.26 mmol), Boc-L-phenylalanine (33.51 g, 126.31 mmol), HBTU (59.88 g, 157.89 mmol) and HOBT (21.33 g, 157.89 mmol) were added to a 1000 mL flask, DMF (200 mL) was added for dissolution, and the mixture was stirred at -5°C for 20 minutes. Then DIEA (104.4 mL, 631.57 mmol) was slowly added dropwise. After the addition was completed, stirring was continued at -5°C for 1 hour, and then the mixture was brought to room temperature and stirred overnight. After the reaction was completed, the reaction mixture was transferred to a 2 L separatory funnel and extracted by adding saturated sodium bicarbonate solution (350 mL) and ethyl acetate (300 mL). The organic phase was collected, and the aqueous phase was extracted with ethyl acetate (200 mL × 3). The organic phases were combined, washed with saturated sodium chloride solution (250 mL × 2), concentrated, dehydrated over anhydrous magnesium sulfate, and evaporated to dryness to provide 49.4 g of the product.

1.6 Preparation of **64-105**

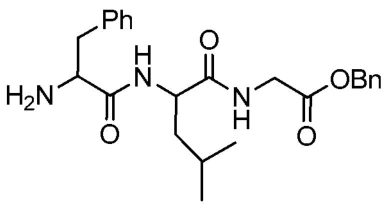

**64-102** (49.4 g, 93.98 mmol) was placed in a 1000 mL flask, and dichloromethane (60 mL) was added for dissolution. Then TFA (71.0 mL, 939.8 mmol) was added under stirring state, and the mixture was reacted under stirring at room temperature overnight. After the reaction was completed, the reaction mixture was concentrated and poured into a 2 L separatory funnel containing saturated sodium bicarbonate solution (350 mL) and ethyl acetate (300 mL) for extraction. The organic phase was collected, and the aqueous phase was further extracted with ethyl acetate (150 mL × 2). The organic phases were combined, concentrated, dehydrated over anhydrous magnesium sulfate, and evaporated to dryness to provide 32.7 g of the product.

1.7 Preparation of **64-106**

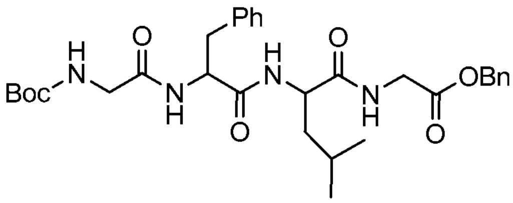

**64-105** (32.7 g, 76.84 mmol), Boc-Gly-OH (16.15 g, 92.21 mmol), HBTU (45.71 g, 115.27 mmol) and HOBT (15.57 g, 115.27 mmol) were added to a 1000 mL flask, and DMF (200 mL) was added to dissolve the mixture. The reaction mixture was stirred at -5°C for approximately 20 minutes, then DIEA (76.2 mL, 461.0832 mmol) was slowly added dropwise. After the addition was completed, stirring was continued at -5°C for 1 hour, and then the mixture was brought to room temperature and stirred overnight. After the reaction was completed, the reaction mixture was transferred to a 2 L separatory funnel and extracted by adding saturated sodium bicarbonate solution (300 mL) and ethyl acetate (350 mL). The organic phase was collected, and the aqueous phase was extracted again with ethyl acetate (200 mL × 3). The organic phases were combined, washed with saturated sodium chloride solution (250 mL × 2) and allowed to stand at room temperature for 1.5 hours. The precipitated solid was filtered, and the filter cake was washed with a 3/7 ethyl acetate/petroleum ether mixture (150 mL × 5) to provide 34.6 g of the product.

1.8 Preparation of**71-85**

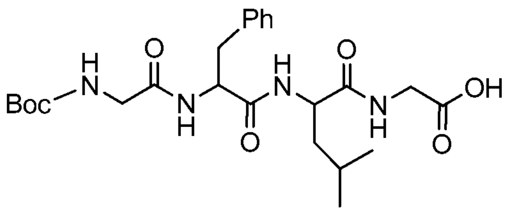

**64-106** (10 g, 17.16 mmol) was added to a hydrogenation reactor. 10% Pd/C catalyst (0.1 g) and DMF (40 mL) were added. Hydrogen gas was introduced at a pressure of 300 psi and the reaction was carried out under stirring at room temperature overnight. After the reaction was completed, the reaction mixture was filtered through a Büchner funnel containing qualitative filter paper. The filter cake was washed with DMF (10 mL × 3). The filtrate was collected and used as the starting material for the next step.

1.9 Preparation of **64-134**

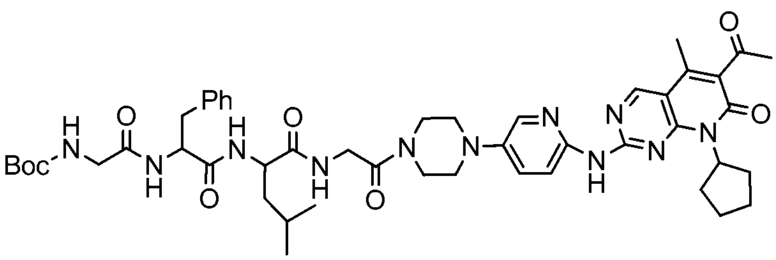

**71-85** (8.45 g, 17.16 mmol), PCB (6.40 g, 14.30 mmol, purchased from Tianjin Famoxi), HBTU (8.13 g, 21.45 mmol), and HOBT (2.89 g, 21.45 mmol) were added to a 500 mL round-bottom flask, and DMF (80 mL) was added to dissolve the mixture. The reaction was carried out under stirring at -5°C for approximately 20 minutes. DIEA (11.8 mL, 71.5076 mmol) was then slowly added dropwise. The reaction was continued at -5°C with stirring for 1 hour. The mixture

was brought to at room temperature and reacted under stirring. After the reaction was completed, methyl tert-butyl ether (450 mL) was added to allow the solid to precipitate. The solid was filtered, and the filter cake was redissolved in an appropriate amount of DMF. Methyl tert-butyl ether (450 mL) was then added to allow the solid to precipitate. This process was repeated three times. The filter cake was collected and dried to provide 13.7 g of the product.

1.10 Preparation of **64-135**

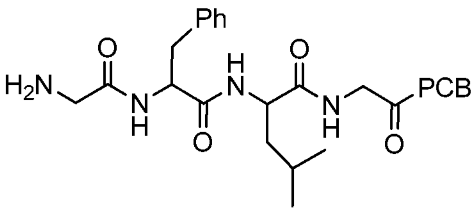

**64-134** (13.18 g, 14.30 mmol) was added to a 250 mL round-bottom flask, and dichloromethane (30 mL) was added for dissolution with the assistance of ultrasonication. TFA (10.6 mL, 143.0 mmol) was then added, and the reaction was carried out under stirring at room temperature overnight. After the reaction was completed, the mother liquor was concentrated and evaporated to dryness to remove the dichloromethane. Methyl tert-butyl ether (300 mL) was then added to allow the solid to precipitate. The solid was filtered, and the filter cake was washed three times with methyl tert-butyl ether (40 mL). The filter cake was collected and dried under vacuum to provide 16.6 g of the product.

1.11 Preparation of **81-189**

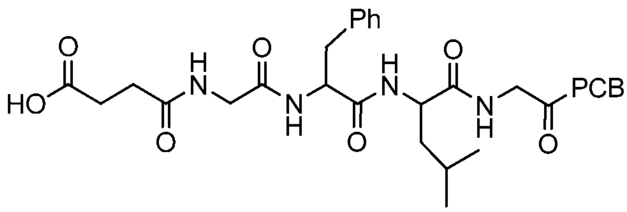

**64-138** (5.6 g, 6.81 mmol) was placed in a 500 mL round-bottom flask, and then DMF (10 mL) was added to dissolve the mixture with the assistance of ultrasonication. The mixture was stirred at 0°C for 5 minutes. DIEA (5.62 mL, 34.05 mmol) was then slowly added dropwise. The reaction was continued under stirring for 3 minutes, and then succinic anhydride (2.04 g, 20.42 mmol) was added. After the reaction was completed, methyl tert-butyl ether (450 mL) was added for precipitation. The mixture was then subjected to ultrasonication and allowed to stand. The supernatant was discarded and the oily substance in the lower layer was dissolved in an appropriate amount of methanol/dichloromethane (1/9) mixture. Methyl tert-butyl ether (200 mL) was then added for precipitation. The solid precipitated and was filtered. The filter cake was collected and dried to provide 5.5 g of the product.

1.12 Preparation of **81-181**

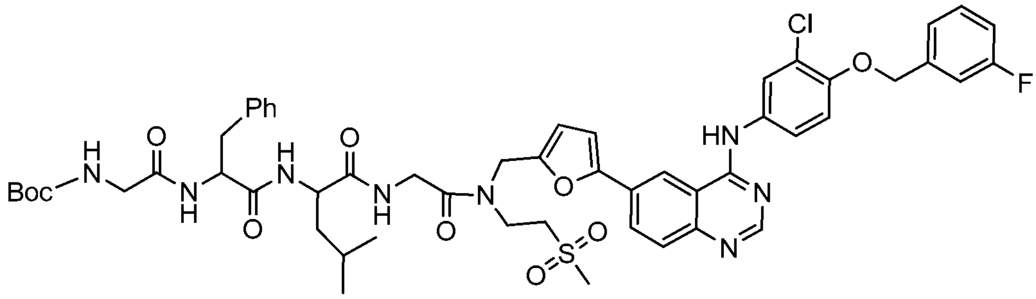

Reactants **71-85** (5.08 g, 10.32 mmol), lapatinib (5 g, 8.60 mmol, purchased from Shanghai Hengxin), HOBT (1.39 g, 10.32 mmol) and HBTU (3.91 g, 10.32 mmol) were placed in a reaction flask, and DMF (20 mL) was added for dissolution with the assistance of ultrasonication. The mixture was stirred at a low temperature of 0°C. DIEA (7.11 mmol, 43.02 mmol) was added dropwise. After the addition was completed, the mixture was brought to room temperature and stirred for 3 hours. After the reaction was completed, the mixture was extracted by adding deionized water (400 mL) and ethyl acetate (400 mL). The organic phase was collected, and the aqueous phase was extracted again with ethyl acetate (200 mL). The organic phases were combined and washed with saturated sodium bicarbonate solution (100 mL × 2). The organic phases were collected, dehydrated over anhydrous magnesium sulfate, concentrated, and evaporated to dryness to provide 8.48 g of the product.

1.13 Preparation of **81-128**

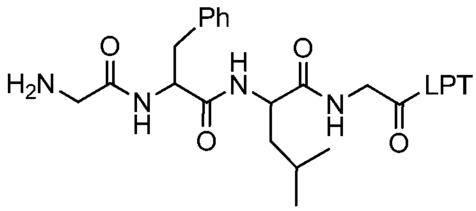

Reactant **81-181** (4.4 g, 4.168 mmol) was added to dichloromethane (10 mL), then TFA (10 mL) was added, and the mixture was reacted under stirring at room temperature. After the reaction was completed, the dichloromethane was removed by rotary evaporation under reduced pressure, and methyl tert-butyl ether (200 mL) was then added to allow the solid to precipitate under standing. The supernatant was discarded, and the solid was dissolved in an appropriate amount of methanol/dichloromethane (1/4) mixture. 200-300 mesh silica gel powder was added, then the mixture was evaporated to dryness. The resultant product was loaded by dry method, and subjected to column chromatography using 1% aqueous ammonia/2% methanol/ dichloromethane as the eluent. The desired product was collected, concentrated, and evaporated to dryness to provide 3.97 g of the product. $^1$H-NMR (600 MHz, DMSO-$d_6$) δ 9.85 (s, 1H), 8.79-8.67 (m, 1H), 8.56 (s, 1H), 8.26-7.96 (m, 5H), 7.80 (dd, $J$ = 4.9, 8.8 Hz, 1H), 7.74 (ddd, $J$ = 2.6, 8.8, 13.7 Hz, 1H), 7.47 (td, $J$ = 6.1, 7.9 Hz, 1H), 7.36-7.27 (m, 3H), 7.26-7.03 (m, 7H), 5.26 (d, $J$ = 2.0 Hz, 2H), 4.74 (d, $J$ = 29.3 Hz, 3.86 (t, $J$ = 7.4 Hz, 1H), 3.78 (t, $J$ = 7.5 Hz, 1H), 3.63 (t, $J$ = 7.6 Hz, 1H), 3.41-3.37 (m, 3H), 3.05-3.03 (m, 4H), 2.84 (ddd, $J$ = 5.8, 8.7, 14.4 Hz, 1H), 1.90 (s, 2H), 1.62 (dd, $J$ = 6.6, 13.3 Hz, 1H), 1.50 (dd, $J$ = 4.8, 10.0 Hz, 2H), 0.94-0.76 (m, 6H); FT MS ESI m/z [M+H$^+$] 955.33

1.14 Preparation of **59-60**

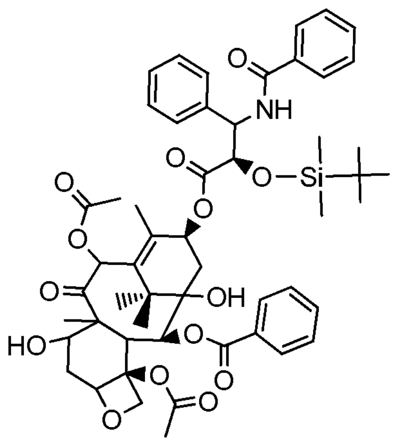

Paclitaxel (20 g, 23.42 mmol, purchased from Wuhan Yingyuanbei, referred to as PTX) and imidazole (7.98 g, 117.12 mmol) were added to a 250 mL round-bottom flask, and DMF was added to dissolve the mixture with the assistance of ultrasonication. $N_2$ was introduced to evacuate the air, and then TBDMS-Cl (21.2 g, 142.54 mmol) was added. The nitrogen flow was stopped, and the reaction was continued under stirring at room temperature. After the reaction was

completed, the reaction mixture was transferred to a 1 L separatory funnel and extracted by adding saturated sodium bicarbonate solution (200 mL) and ethyl acetate (200 mL). The aqueous phase was extracted again with ethyl acetate (150 mL × 2). The organic phases were combined, washed with saturated sodium chloride solution (100 mL × 2), collected, concentrated, and evaporated to dryness after the addition of 200-300 mesh silica gel powder. The resultant product was loaded by dry method, and subjected to column chromatography using 0-100% ethyl acetate/petroleum ether as the eluent. The desired product was collected, and concentrated to dryness to provide 22.7 g of the product.

1.15 Preparation of **59-67**

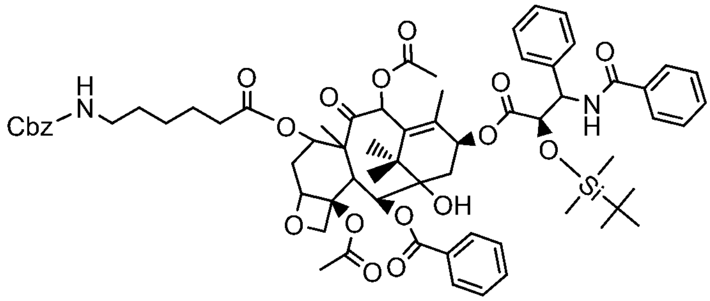

**59-60** (2 g, 7.83 mmol) was dissolved in tetrahydrofuran (20 mL), and stirred at 0°C. DIEA (1.5 mL, 9.04 mmol) was slowly added dropwise, and then pivaloyl chloride (1.1 mL, 9.04 mmol) was added dropwise. After the additions were completed, the reaction mixture was allowed to react at room temperature for 3 h. After confirming the reaction was completed, the reaction mixture was stirred at 0°C. **64-152** (3.65 g, 3.76 mmol) was dissolved in THF (5 mL) and slowly added dropwise to the reaction mixture. DIEA (0.69 mL, 4.1459 mmol) was then added dropwise. After the addition was completed, the reaction mixture was allowed to react under stirring at room temperature. After the reaction was completed, the reaction mixture was poured into a 2L separatory funnel filled with pure water (300 mL) and ethyl acetate (300 mL). The organic phase was separated, and the aqueous phase was extracted again with ethyl acetate (100 mL × 2). The organic phases were combined, concentrated to 150 mL, and washed with dilute hydrochloric acid (200 mL × 2) and then with saturated sodium bicarbonate solution (200 mL × 2). The organic phase was collected and dried to provide 4.58 g of the product.

1.16 Preparation of **59-69**

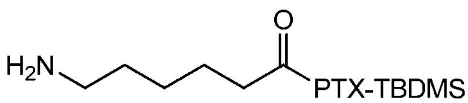

**59-67** (4.58 g, 3.769 mmol) was dissolved in DMA (30 mL) pretreated with activated carbon, and transferred to a hydrogenation reactor. 10% Pd/C catalyst (250 mg) was then added. A water pump was connected to the reactor, the air was removed and hydrogen gas was filled, and this process was repeated three times. The hydrogen pressure was adjusted to 1.8 MPa. The reaction was carried out under high-speed stirring at room temperature. After the reaction was completed, the reaction mixture was filtered through a Celite-containing filter funnel. The filtrate was poured into a 1 L separatory funnel. Saturated sodium chloride solution (300 mL) and ethyl acetate (300 mL) were added to the separatory funnel for extraction. The organic phase was separated, and the aqueous phase was then extracted with ethyl acetate (100 mL × 2). The organic phases were combined and washed twice with saturated sodium chloride solution (200 mL). The organic phases were collected, concentrated, and dissolved in a mixture of dichloromethane and methanol. 200-300 mesh silica gel powder was added, and the mixture was evaporated to dryness. The resultant product was loaded by dry method, and subjected to column chromatography using 30% ethyl acetate/petroleum ether and then 3-10% methanol/dichloromethane as eluents. The desired product was collected and dried to provide 4.08 g of the product.

1.17 Preparation of **81-120**

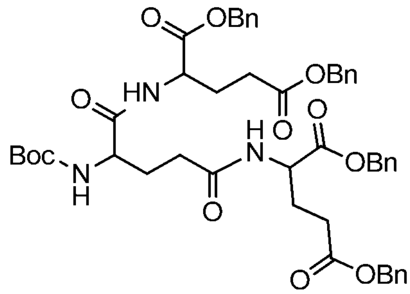

Boc-Glu-OH (3 g, 12.13 mmol), L-glutamic acid dibenzyl ester p-toluenesulfonate (12.72 g, 25.48 mmol), HBTU (9.66 g, 25.48 mmol) and HOBT (3.44 g, 25.48 mmol) were added to a 500 mL round-bottom flask. DMF (30 mL) was then added to dissolve the mixture. After stirring in an ice-water bath for 3 min, DIEA (16.03 mL, 97.04 mmol) was added dropwise. The mixture was stirred at room temperature. Upon completion of the reaction, the reaction mixture was transferred to a 2 L separatory funnel filled with purified water (200 mL) and ethyl acetate (200 mL). The organic phase was collected, and the aqueous phase was then extracted with ethyl acetate (50 mL × 2). The organic phases were combined, washed with saturated sodium bicarbonate solution (50 mL × 2), dried over anhydrous magnesium sulfate, concentrated under reduced pressure, and evaporated to dryness to provide 10.5 g of the product.

1.18 Preparation of **81-121**

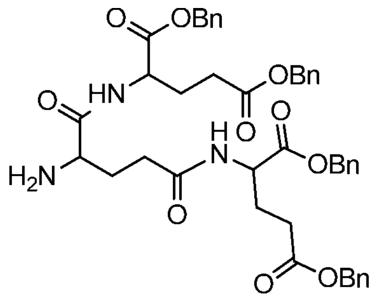

**81-120** (10.5 g, 12.13 mmol) was placed in a 500 mL round-bottom flask, and dichloromethane (14 mL) was added for dissolution with the assistance of ultrasonication. TFA (21 mL) was then added and the reaction was carried out under stirring at room temperature. After the reaction was completed, TFA and dichloromethane were removed by rotary evaporation under reduced pressure. The reaction mixture was transferred to a 1 L separatory funnel and extracted by adding purified water (300 mL) and ethyl acetate (350 mL). The aqueous phase was further extracted with ethyl acetate (100 mL × 3). The organic phases were combined, neutralized with saturated sodium bicarbonate solution, dehydrated over anhydrous magnesium sulfate, concentrated under reduced pressure, and evaporated to dryness to provide 9.28 g of the product.

1.19 Preparation of **81-125**

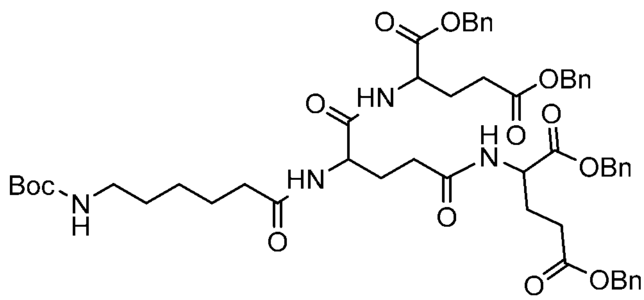

**62-13** (2.8 g, 12.13 mmol), **81-121** (9.28 g, 12.13 mmol), HBTU (5.05 g, 13.34 mmol) and HOBT (1.8 g, 13.34

mmol) were added to a 500 mL round-bottom flask, and then DMF (20 mL) was added to dissolve the mixture. After stirring in an ice-water bath for 2 min, DIEA (8.01 mL, 48.52 mmol) was added and the reaction was carried out under stirring at room temperature. Upon completion of the reaction, the reaction mixture was transferred to a 2 L separatory funnel and extracted by adding purified water (200 mL) and ethyl acetate (100 mL). The organic phase was collected, and the aqueous phase was then extracted with ethyl acetate (100 mL × 3). The organic phases were combined and washed with saturated sodium bicarbonate solution (100 mL × 2). The organic phases were collected and concentrated under reduced pressure, then 200-300 mesh silica gel powder was added, and the mixture was evaporated to dryness. The resultant product was loaded by dry method, and subjected to column chromatography using 1-2.5% methanol/dichloromethane as the eluent. The desired product was collected, concentrated and evaporated to dryness to provide 10.8 g of the product in a yield of 91%. FT-MS ESI m/z [M+Na$^+$] 1002.34

1.20 Preparation of **81-127**

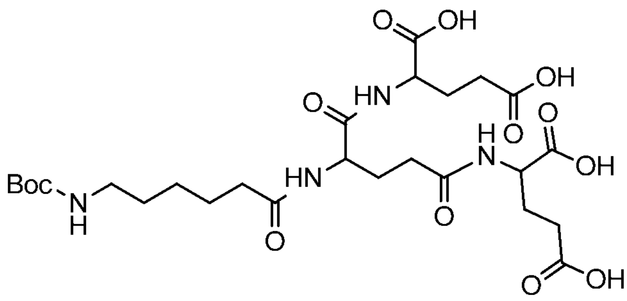

**81-125** (2 g, 2.04 mmol) was added to a hydrogenation reactor. 10% Pd/C catalyst (0.2 g) and methanol (30 mL) were added. Hydrogen gas was introduced at a pressure of 2 MPa, and the mixture was stirred at 30°C overnight. Upon completion of the reaction, the reaction mixture was filtered through a Büchner funnel containing filter paper, concentrated under reduced pressure, and evaporated to dryness to provide the product.

1.21 Preparation of **81-132**

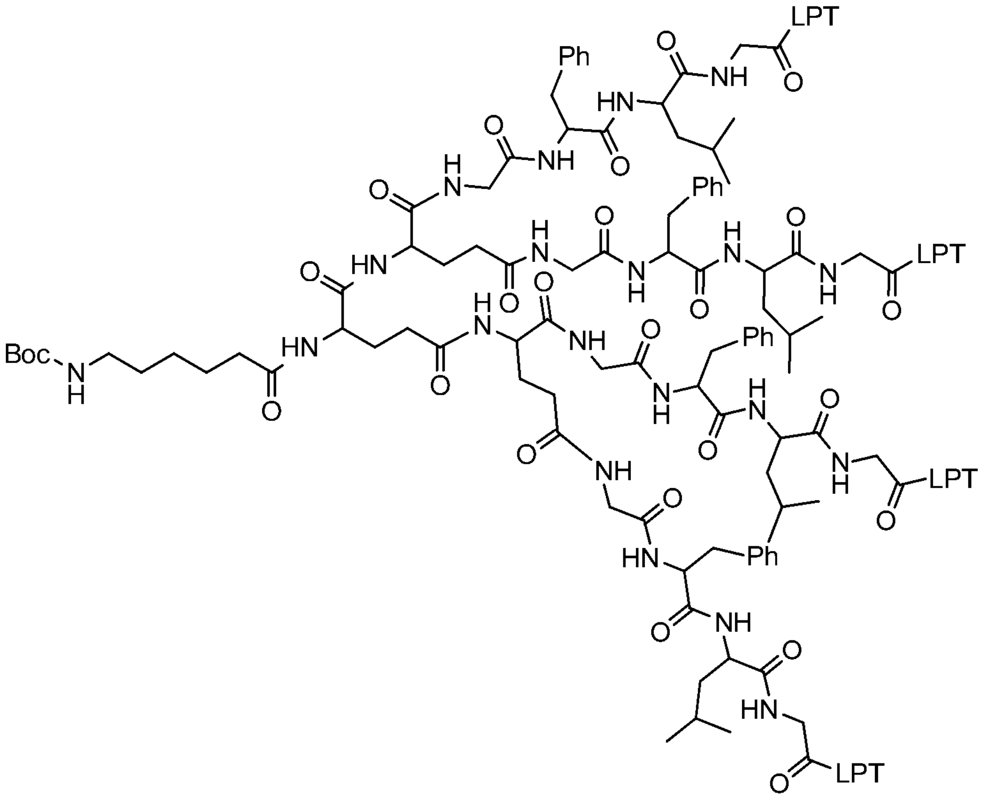

**81-127** (1.16 g, 1.875 mmol), **81-128** (8.95 g, 9.375 mmol), HBTU (3.55 g, 9.375 mmol), and HOBT (1.26 g, 9.375 mmol) were placed in a 500 mL round-bottom flask, and then DMF (15 mL) was added to dissolve the mixture. The mixture was stirred at 0°C for 1 minute. DIEA (5.57 mL, 33.75 mmol) was then slowly added dropwise, and the reaction was continued. After the reaction was completed, methyl tert-butyl ether (450 mL) was added to allow the solid to precipitate, and then the solid was filtered. The filter cake was redissolved in an appropriate amount of ethyl acetate, stirred at 37°C for 5 minutes, and filtered to collect the filter cake. This process was repeated three times. The filter cake was then dissolved in an appropriate amount of DMA, and methyl tert-butyl ether was added dropwise under stirring until fine solid particles formed. An appropriate amount of methyl tert-butyl ether was then added, the mixture was filtered, and the filter cake was dried to provide 5.1 g of the product with a yield of 62.27%.

1.22 Preparation of **81-136**

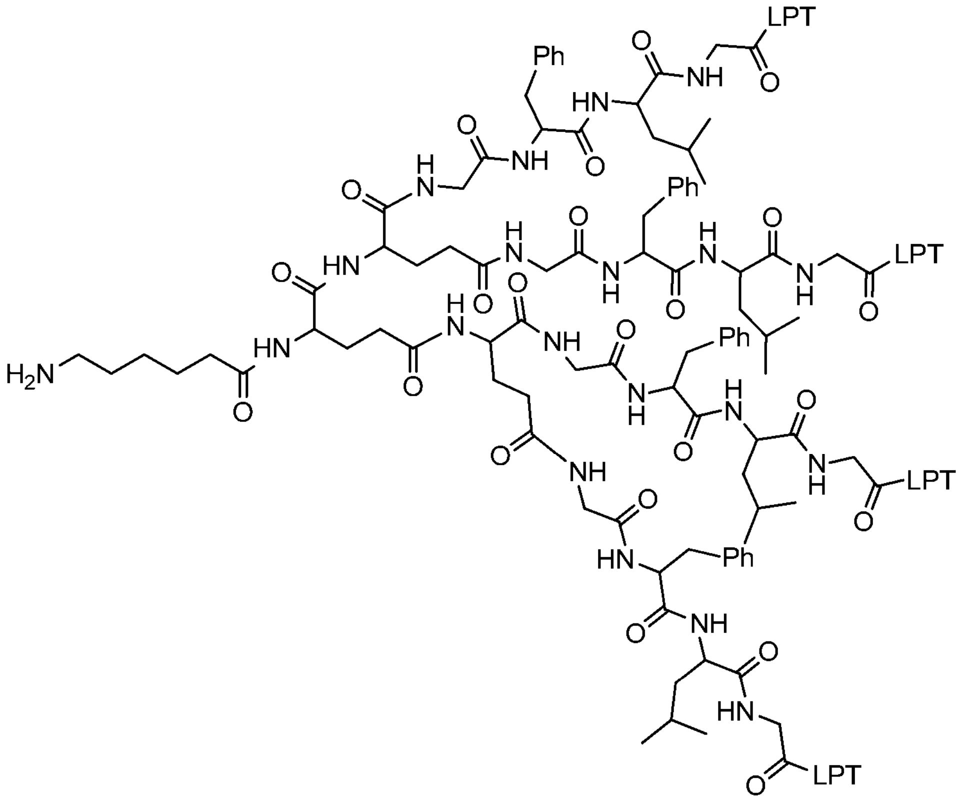

**81-132** (5.1 g, 1.16 mmol) was added to a 500 mL round-bottom flask, then TFA (15 mL) was added, and the mixture was reacted under stirring at room temperature. After the reaction was completed, dichloromethane and TFA were removed by rotary evaporation under reduced pressure. An appropriate amount of dichloromethane was then added, and the mixture was evaporated under reduced pressure until the product became a viscous oil. An appropriate amount of methyl tert-butyl ether was then added, resulting in the precipitation of solids. The solids were filtered, and the filter cake was dried to provide 4.95 g of the product. MALDI-TOF MS m/z [M+H+] 4269.65

1.23 Preparation of **81-146**

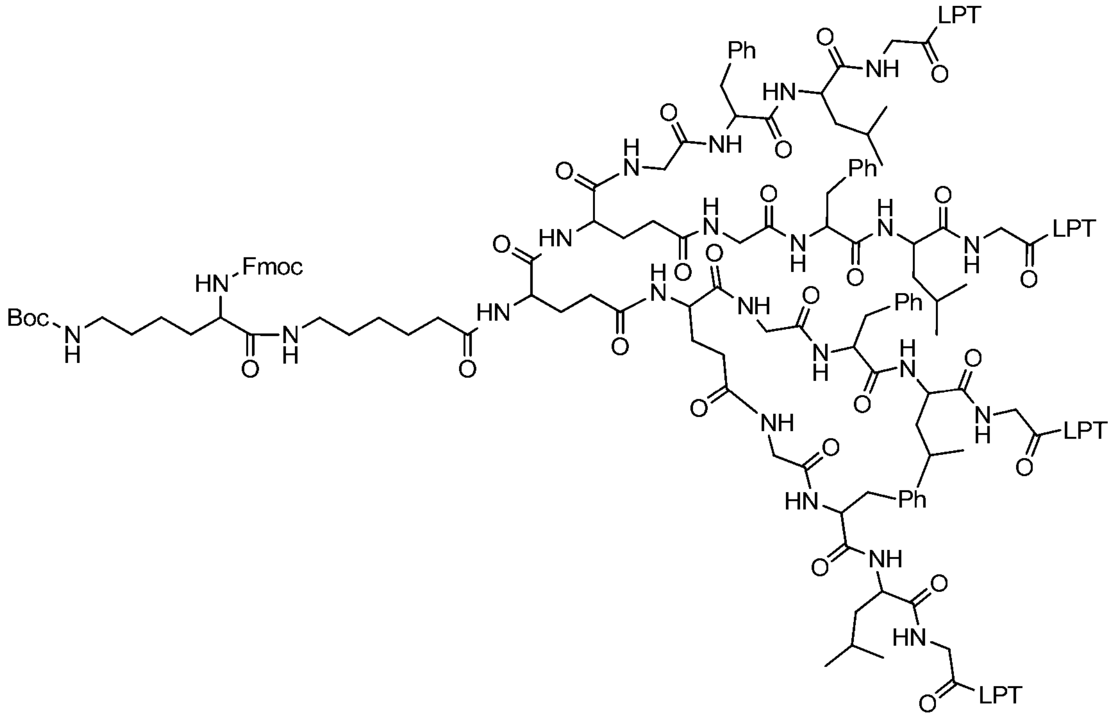

Reactant **81-136** (3.7 g, 0.866 mmol), Fmoc-Lys(Boc)-OH (0.6 g, 1.3 mmol), HOBT (0.175 g, 1.3 mmol) and HBTU (0.49 g, 1.3 mmol) were placed in a reaction flask, DMF (20 mL) was added to dissolve the mixture with the assistance of ultrasonication. The mixture was stirred at a low temperature of 0°C for 2 minutes. DIEA (0.5 mL, 3.03 mmol) was then added dropwise. Upon completion of the addition, the reaction mixture was stirred at room temperature for 3 hours. After the reaction was completed, methyl tert-butyl ether (450 mL) was added, the mixture was ultrasonicated, and allowed to stand. The supernatant was discarded, and methyl tert-butyl ether (200 mL) was added for precipitation. A solid precipitated and was filtered. The filter cake was redissolved in an appropriate amount of DMF, and methyl tert-butyl ether (450 mL) was added in batches with small amounts for precipitation. The mixture was filtered, and the filter case was dried to provide 4.08 g of the product.

1.24 Preparation of **81-147**

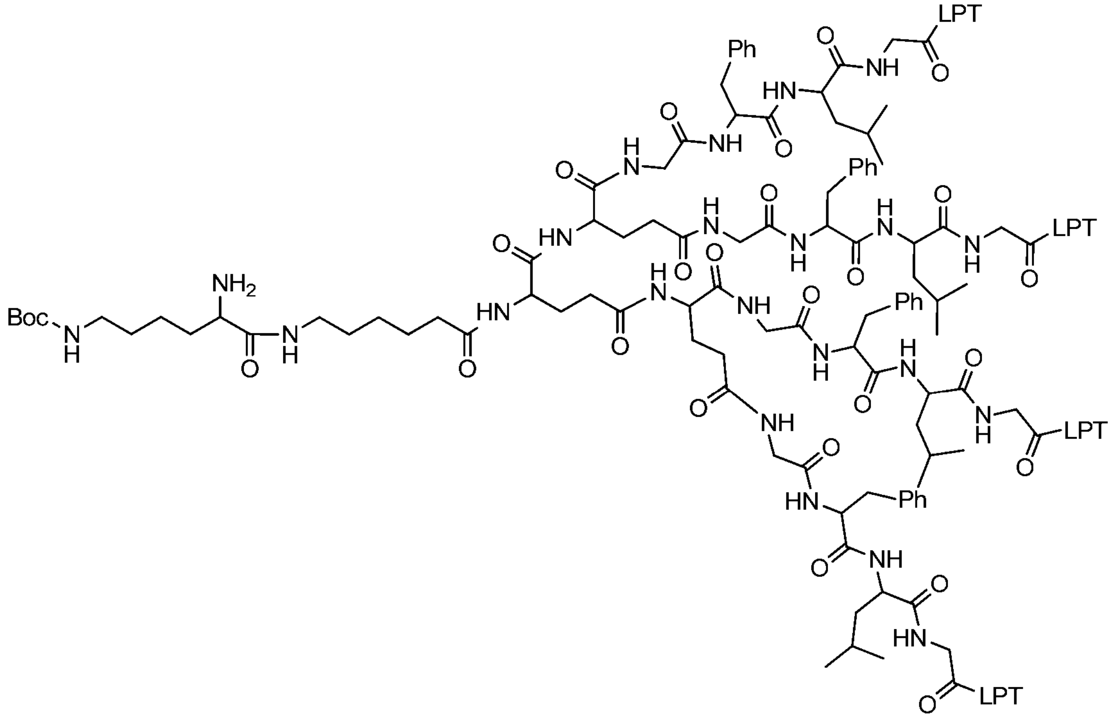

Reactant **81-146** (4 g, 0.84 mmol) was dissolved in DMF (30 mL), then diethylamine (30 mL, 6.3574 mmol) was added, and the mixture was reacted under stirring at room temperature. After the reaction was completed, n-hexane (50 mL) and methyl tert-butyl ether (450 mL) were added for precipitation. A solid precipitated and the supernatant was discarded. The solid was redissolved in an appropriate amount of DMF, and methyl tert-butyl ether (450 mL) was added in batches with small amounts until a large amount of solid precipitated. The solid was filtered out, and the filter cake was redissolved in an appropriate amount of DMF. MTBE (450 mL) was then added to allow precipitation. This process was repeated three times. The filter cake was dried to provide 3.1 g of the product.

1.25 Preparation of **81-185**

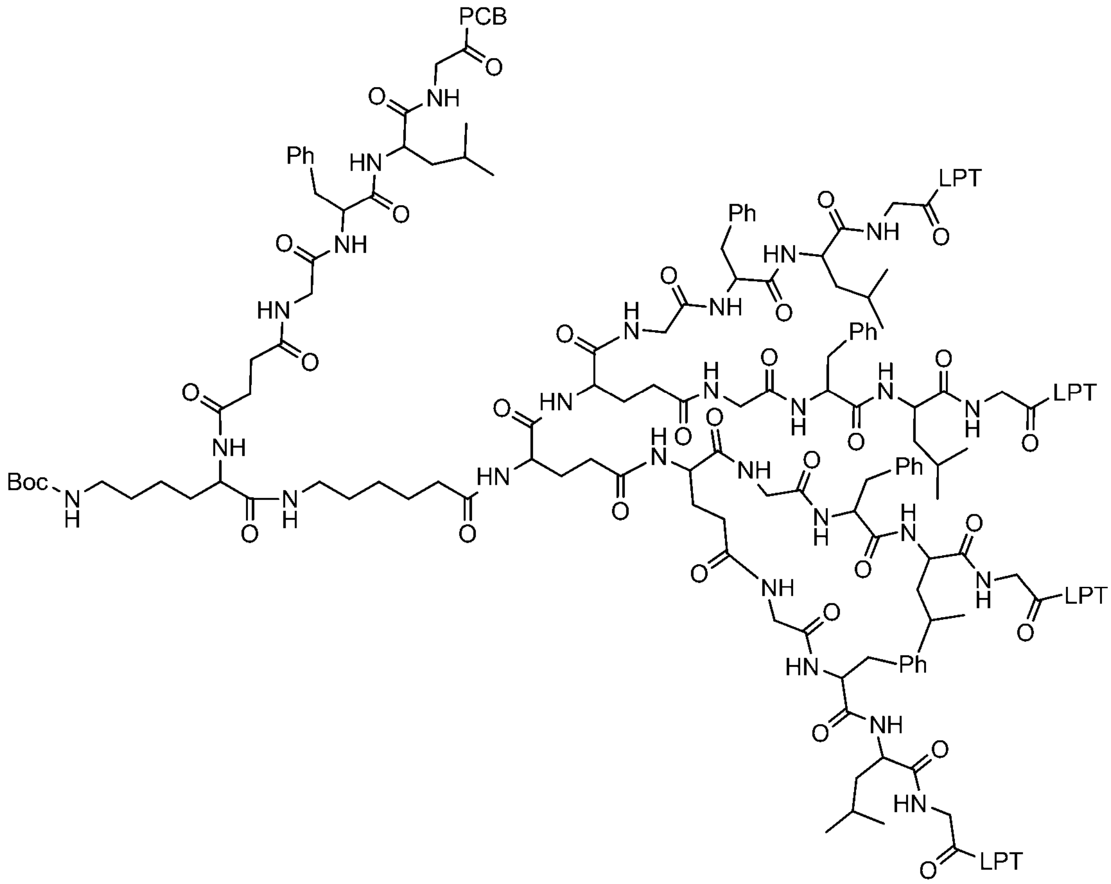

**81-189** (2.31 g, 2.508 mmol), HBTU (0.9511 g, 2.508 mmol) and HOBT (0.06 g, 0.495 mmol) were placed in a 50 mL round-bottom flask, and then DMF (5 mL) was added to dissolve the mixture. The mixture was stirred at 0°C for 1 minute, and DIEA (1.72 mL, 10.45 mmol) was then slowly added dropwise. The reaction was carried out under stirring at room temperature for 5 minutes, and then a DMF solution of **81-147** (9.4 g, 2.09 mmol) was added. The reaction was continued. After the reaction was completed, methyl tert-butyl ether (450 mL) was added for precipitation. A solid precipitated and was filtered. The filter cake was redissolved in an appropriate amount of DMF and then methyl tert-butyl ether (450 mL) was added for precipitation. This process was repeated three times. The filter cake was dried to provide the product.

1.26 Preparation of **81-203**

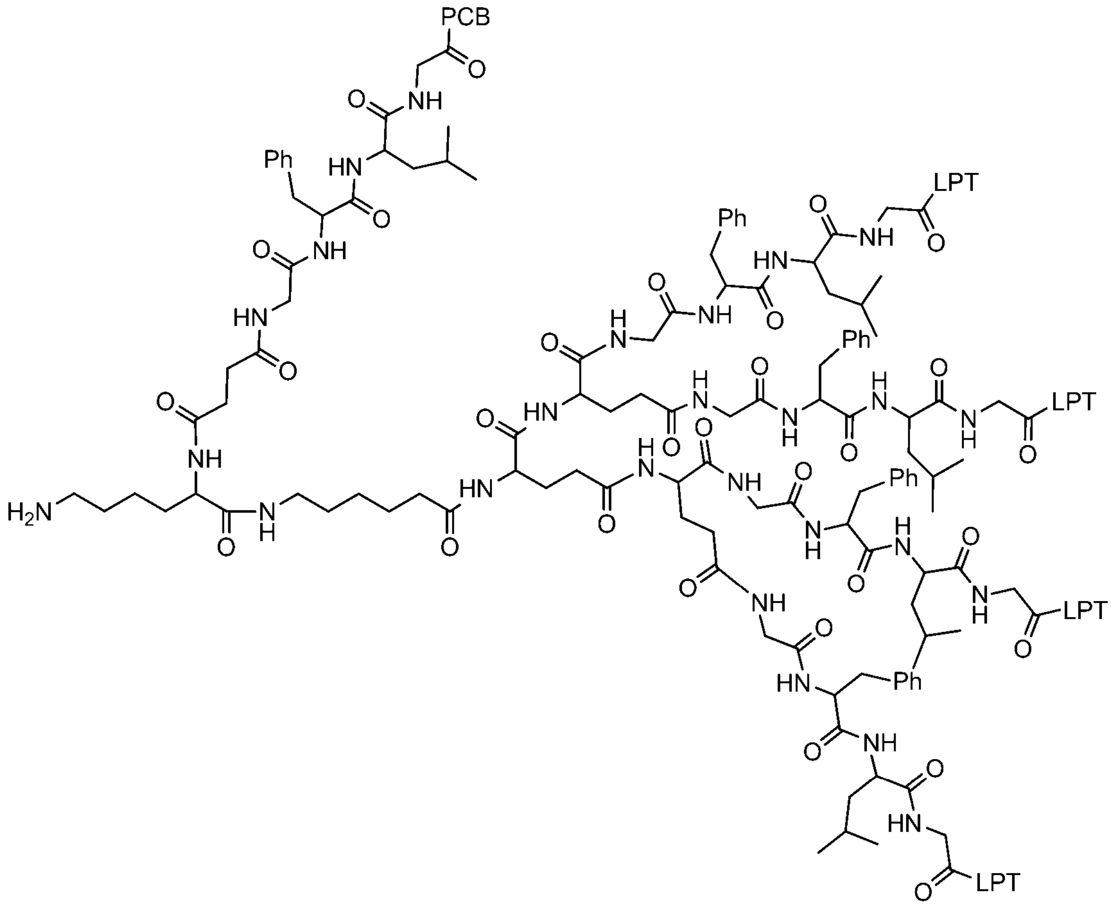

**81-185** (5.5 g, 1.018 mmol) was added to a 500 mL round-bottom flask, and dichloromethane (3 mL) was added. TFA (20 mL) was then added, and the mixture was reacted under stirring at room temperature. After the reaction was completed, the dichloromethane and TFA were removed by rotary evaporation. An appropriate amount of dichloromethane was then added for dissolution with the assistance of ultrasonication. Methyl tert-butyl ether (450 mL) was added for precipitation. A solid precipitated and was filtered. The filter cake was redissolved in an appropriate amount of DMF and then MTBE (450 mL) was added for precipitation. This process was repeated three times. The filter cake was dried to provide the product.

1.27 Preparation of Compound **81-9**

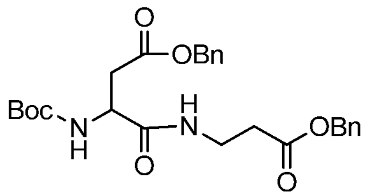

Boc-Asp(Obzl)-OH (9.19 g, 28.45 mmol), H-β-Ala-Obzl.TsOH (10 g, 28.45 mmol), HBTU (15.1 g, 39.83 mmol) and HOBT (5.38 g, 39.83 mmol) were added to a 500 mL round-bottom flask, and an appropriate amount of DMF was added to dissolve the mixture. The reaction was carried out under stirring at 0°C for approximately 3 minutes. DIEA (21.16 L, 128.025 mmol) was then slowly added dropwise, and the reaction was carried out under stirring at room temperature overnight. After the reaction was completed, the reaction mixture was transferred to a 1 L separatory funnel and extracted by adding purified water (300 mL) and ethyl acetate (200 mL). The organic phase was collected, and the aqueous phase was then extracted with ethyl acetate (200 mL × 2). The organic phases were combined and washed with saturated sodium bicarbonate solution (100 mL × 2), concentrated, dehydrated over anhydrous magnesium sulfate, and evaporated to dryness to provide 13.78 g of the product.

1.28 Preparation of**81-10**

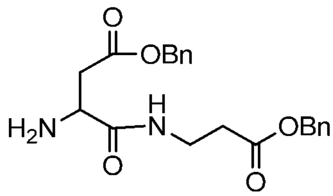

**81-9** (13.78 g, 28.45 mmol) was placed in a 250 mL round-bottom flask, dichloromethane (20 mL) was added for dissolution, then TFA (48.63 g, 426.59 mmol) was added, and the mixture was reacted under stirring at room temperature. After the reaction was completed, the dichloromethane was removed by concentration under reduced pressure. The reaction mixture was then transferred to a 1 L separatory funnel and extracted by adding purified water (100 mL) and ethyl acetate (200 mL). Sodium bicarbonate powder was added in batches with small amounts to neutralize the TFA. The organic phase was collected, and the aqueous phase was further extracted with ethyl acetate (200 mL × 2). The organic phases were combined, dehydrated over anhydrous magnesium sulfate, and evaporated to dryness to provide 10.92 g of the product.

1.29 Preparation of **74-100**

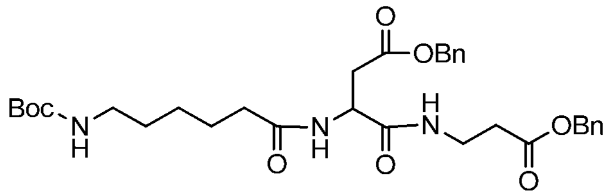

**62-13** (2.63 g, 11.38 mmol), 81-10 (4.37 g, 11.38 mmol), HBTU (1.84 g, 13.65 mmol) and HOBT (5.17 g, 13.65 mmol) were placed in a 500 mL round-bottom flask, and then DMF (15 mL) was added to dissolve the mixture. The mixture was stirred at 0°C for 2 minutes. DIEA (8.46 mL, 51.21 mmol) was slowly added dropwise to continue the reaction. After the reaction was completed, the reaction mixture was poured into a 2 L separatory funnel. Pure water (300 mL) and ethyl acetate (300 mL) were added to the separatory funnel for extraction. The organic phase was separated, and the aqueous phase was further extracted with ethyl acetate (100 mL × 2). The organic phases were combined, and washed with saturated sodium bicarbonate solution (200 mL × 2). The organic phases were collected, concentrated, dehydrated over anhydrous magnesium sulfate, 200-300 mesh silica gel powder was added, and the mixture was evaporated to dryness. The resultant product was loaded by dry method, and subjected to column chromatography using 0.5-2% methanol/dichloromethane as the eluent. The desired product was collected and evaporated to dryness to provide 3.4 g of the product.

1.30 Preparation of **74-101**

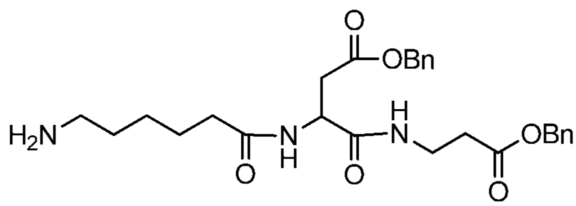

**74-100** (3.4 g, 5.68 mmol) was added to a 500 mL round-bottom flask, TFA (12.98 g) was added for dissolution, and the mixture was reacted under stirring at room temperature. After the reaction was completed, the TFA was removed by rotary evaporation. The product was dissolved in an appropriate amount of ethyl acetate and poured into a 2-liter separatory funnel. Pure water (300 mL) and ethyl acetate (300 mL) were added to the separatory funnel for extraction.

Sodium bicarbonate powder was added in bathes with small amounts to neutralize the TFA. The organic phase was collected, and the aqueous phase was further extracted again with ethyl acetate (100 mL × 2). The organic phases were combined, concentrated, dehydrated over anhydrous magnesium sulfate, and evaporated to dryness to provide 2.83 g of the product.

1.31 Preparation of **74-102**

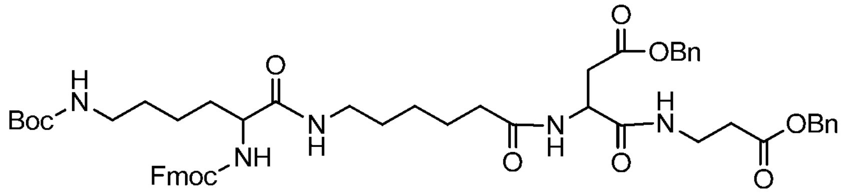

**74-101** (2.83 g, 5.68 mmol), Fmoc-Lys(Boc)-OH (2.66 g, 5.68 mmol), HBTU (2.58 g, 6.82 mmol) and HOBT (0.92 g, 6.82 mmol) were placed in a 500-ml round-bottom flask. DMF (15 mL) was added to dissolve the mixture. The mixture was stirred at 0°C for 3 minutes. DIEA (4.23 mL, 25.59 mmol) was then slowly added dropwise to continue the reaction. After the reaction was completed, the reaction mixture was poured into a 2 L separatory funnel. Pure water (300 mL) and ethyl acetate (300 mL) were added to the separatory funnel for extraction. The organic phase was separated, and the aqueous phase was further extracted with ethyl acetate (100 mL × 2). The organic phases were combined, and washed with saturated sodium bicarbonate solution (200 mL × 2). The organic phases were collected, concentrated, and dehydrated over anhydrous magnesium sulfate, then 200-300 mesh silica gel powder was added, and the mixture was evaporated to dryness. The resultant product was loaded by dry method, and subjected to column chromatography using 0.5-1.5% methanol/dichloromethane as the eluent. The product was collected, concentrated, and evaporated to dryness to provide 5.39 g of the product.

1.32 Preparation of **74-103**

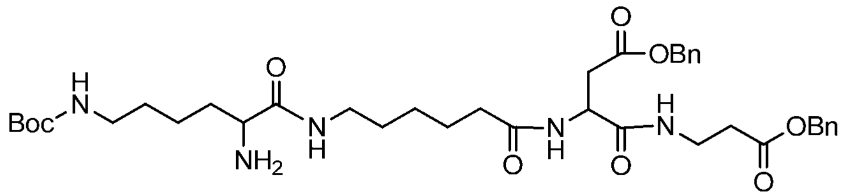

**74-102** (5.39 g, 5.68 mmol) was placed in a 500 mL round-bottom flask. DMF (10 mL) was added for dissolution, then morpholine (7.43 mL, 85.32 mmol) was added, and the mixture was reacted under stirring at room temperature. After the reaction was completed, the reaction mixture was poured into a 2 L separatory funnel. Pure water (300 mL) and ethyl acetate (300 mL) were added to the separatory funnel for extraction. The organic phase was separated, and the aqueous phase was further extracted with ethyl acetate (100 mL × 2). The organic phases were combined, and washed with saturated sodium bicarbonate solution (200 mL × 2). The organic phases were collected, concentrated, and dehydrated over anhydrous magnesium sulfate, then 200-300 mesh silica gel powder was added, and the mixture was evaporated to dryness. The resultant product was loaded by dry method, and subjected to column chromatography using 5-7% methanol/dichloromethane as the eluent. The product was collected, concentrated, and evaporated to dryness to provide 1.4 g of the product.

1.33 Preparation of**81-60**

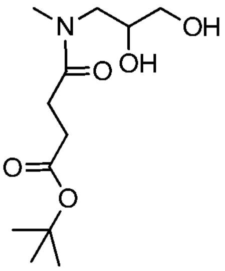

3-(Methylamine)propane-1,2-diol (3 g, 28.53 mmol), mono-tert-butyl succinate (4.96 g, 28.53 mmol) and HATU (11.39 g, 29.96 mmol) were placed in a 500 mL round-bottom flask, acetonitrile (20 mL) was added to dissolve the mixture with the assistance of ultrasonication. The mixture was stirred at -5°C for 2 minutes. DIEA (24.75 mL, 149.8 mmol) was then slowly added dropwise, and the reaction was continued. After the reaction was completed, the acetonitrile was removed by rotary evaporation under reduced pressure. Methyl tert-butyl ether (200 mL) was added again for precipitation, and the supernatant was discarded. Methyl tert-butyl ether (200 mL) was then added for precipitation, and the supernatant was discarded. This process was repeated several times until the product became a viscous oil. An appropriate amount of dichloromethane was added for dissolution with the assistance of ultrasonication. 200-300 mesh silica gel powder was added, and the mixture was evaporated to dryness. The resultant product was loaded by dry method, and subjected to column chromatography using 0-1.5% methanol/dichloromethane as the eluent. The desired product was collected, concentrated, and evaporated to dryness to provide 5.45 g of the product with a yield of 3.15%. $^1$H-NMR (600 MHz, DMSO-$d_6$) δ 3.75-3.53 (m, 3H), 3.43-3.14 (m, 4H), 2.90 (s, 3H), 2.57-2.45 (m, 2H), 2.43-2.31 (m, 2H), 1.35 (s, 9H); FT MS ESI m/z [M+H$^+$] 262.16

1.34 Preparation of **81-65**

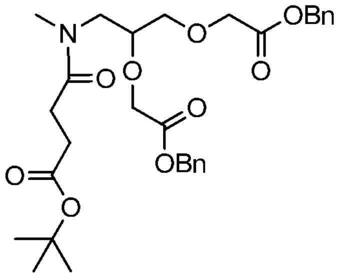

Ultra-dry THF (15 mL) was placed into a 500 mL round-bottom flask. 60% NaH (0.22 g, 5.739 mmol) was added, and the mixture was stirred until no bubbles formed, then **81-60** (0.5 g, 1.913 mmol) and benzyl bromoacetate (1.75 g, 7.653 mmol) were added, and the mixture was reacted under stirring under a nitrogen atmosphere. After the reaction was completed, the reaction mixture was poured into a 1 L separatory funnel. Dilute hydrochloric acid solution (100ml) and ethyl acetate (200ml) were added to the separatory funnel for extraction. The organic phase was separated, and the aqueous phase was further extracted with ethyl acetate (100ml × 2). The organic phases were combined, and washed with saturated sodium chloride solution (50ml × 2). The organic phases were collected, concentrated, then dehydrated over anhydrous magnesium sulfate, then 200-300 mesh silica gel powder was added, and the mixture was evaporated to dryness. The resultant product was loaded by dry method, and subjected to column chromatography using 0.2-5% methanol/dichloromethane as the eluent. The desired product was collected, concentrated, and evaporated to dryness to provide 0.23g of the product with a yield of 21.69%. $^1$H-NMR (600 MHz, DMSO-$d_6$) δ 7.44-7.27 (m, 10H), 5.18-5.12 (m, 4H), 4.22-4.17 (m, 4H), 3.76-3.71 (m, 1H), 3.43-3.14 (m, 4H), 3.01 (s, 3H), 2.49-2.43 (m, 2H), 2.42-2.30 (m, 2H), 1.37 (s, 9H); FT MS ESI m/z [M+H$^+$] 558.26

1.35 Preparation of**81-82**

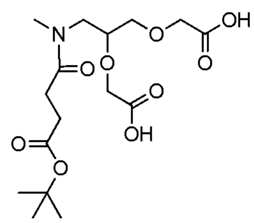

Raw material **81-65** (0.5 g, 0.896 mmol) and 10% Pd/C catalyst (20 mg) was added to a hydrogenation reaction apparatus, then DMF (8 mL) was added for dissolution. Hydrogen was introduced to 2 MPa, and the reaction was allowed to proceed overnight at room temperature. After the reaction was completed, the reaction mixture containing palladium-on-carbon was collected.

1.36 Preparation of**81-85**

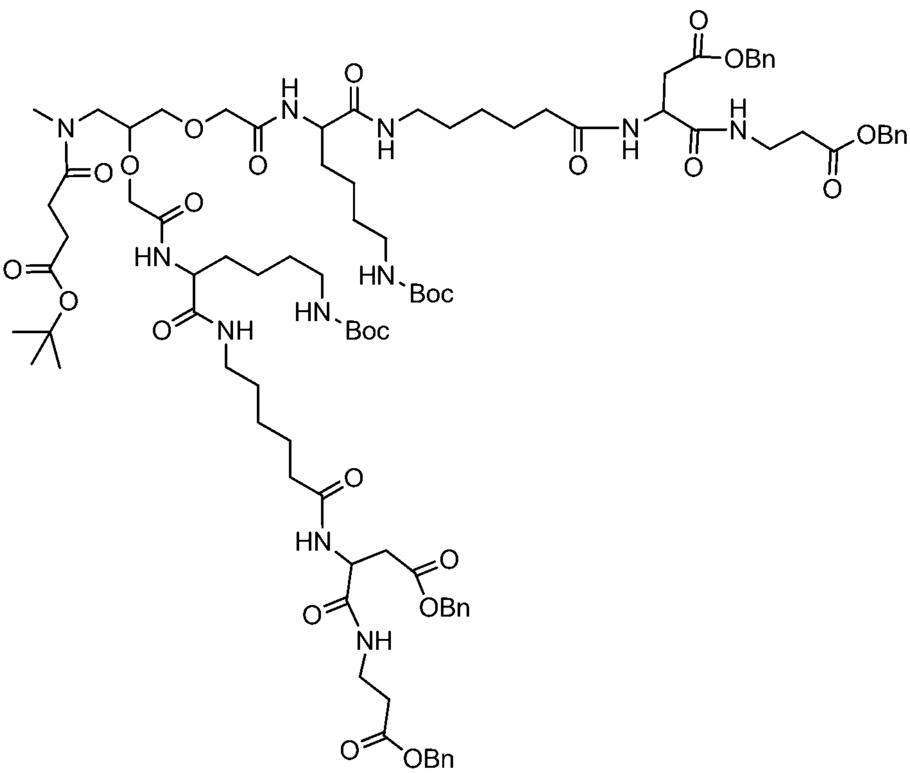

**81-82** (0.33 g, 0.896 mmol), **74-103** (1.42 g, 1.97 mmol), HBTU (0.74 g, 1.97 mmol) and HOBT (0.26 g, 1.97 mmol) were placed in a 50 mL round-bottom flask, and then DMF (5 mL) was added to dissolve the mixture. The mixture was stirred at 0°C for 1 minute. DIEA (1.03 mL, 6.27 mmol) was then slowly added dropwise to continue the reaction. After the reaction was completed, the palladium-on-carbon was removed by filtration, n-hexane (50 mL) and methyl tert-butyl ether (450 mL) were added for precipitation, followed by ultrasonic treatment. The mixture was allowed to stand, and the supernatant was discarded. The process was repeated several times until the product became a viscous oil. Dichloromethane was then added for dissolution with the assistance of ultrasonication. 200-300 mesh silica gel powder was added, and the mixture was evaporated to dryness. The resultant product was loaded by dry method, and subjected to column chromatography using 2-3% methanol/dichloromethane as the eluent. The desired product was collected, concentrated, and evaporated to dryness to provide 0.96 g of the product with a yield of 60%. FT MS ESI m/z [M+Na$^+$] 1815.93

1.37 Preparation of **81-197**

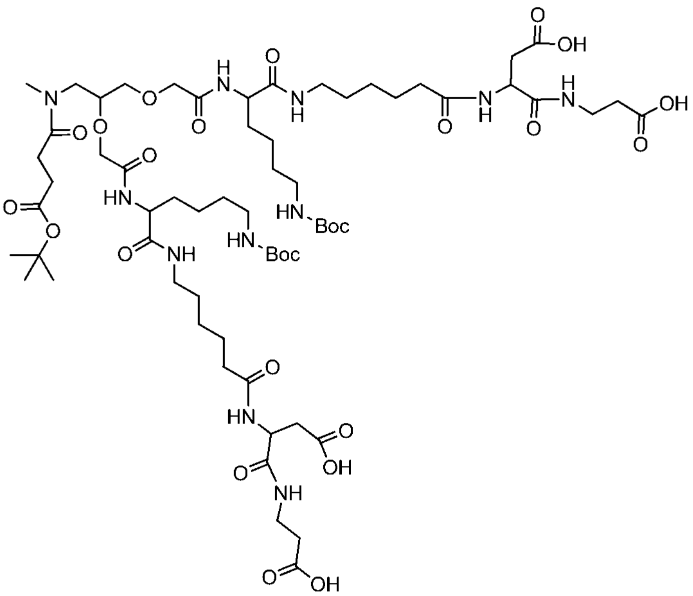

Raw material **81-85** (0.4 g, 0.223 mmol) and 10% Pd/C catalyst (50 mg) were added to a hydrogenation reaction apparatus, and then methanol (10 mL) was added for dissolution. The hydrogenation apparatus was sealed and evacuated with a water pump. Hydrogen gas was then introduced to approximately 2 MPa. This process was repeated three times, and the hydrogen pressure was finally adjusted to 2.2 MPa. The reaction was carried out at room temperature overnight. After the reaction was completed, the mixture was filtered through a Büchner funnel containing filter paper, and the reaction apparatus was rinsed with methanol (3 mL × 3). The mixture was evaporated to dryness to provide 0.31 g of the reaction product. $^1$H-NMR (600 MHz, DMSO-$d_6$) δ 12.10-12.01 (m,4H), 8.13-8.05 (m,4H), 8.02-7.90(m, 4H), 7.44-7.37 (m, 2H), 4.22-4.12 (m, 8H), 3.76-3.71 (m, 3H), 3.65-3.52 (m, 4H), 3.43-3.14 (m, 12H), 3.08-3.00 (m, 5H), 2.52-2.43 (m, 10H), 2.42-2.30 (m, 2H), 1.67-1.57 (m, 2H), 1.57-1.49 (m, 2H), 1.49-1.41 (m, 4H), 1.41-1.29 (m, 35H), 1.28-1.10 (m, 8H)

1.38 Preparation of **81-204**

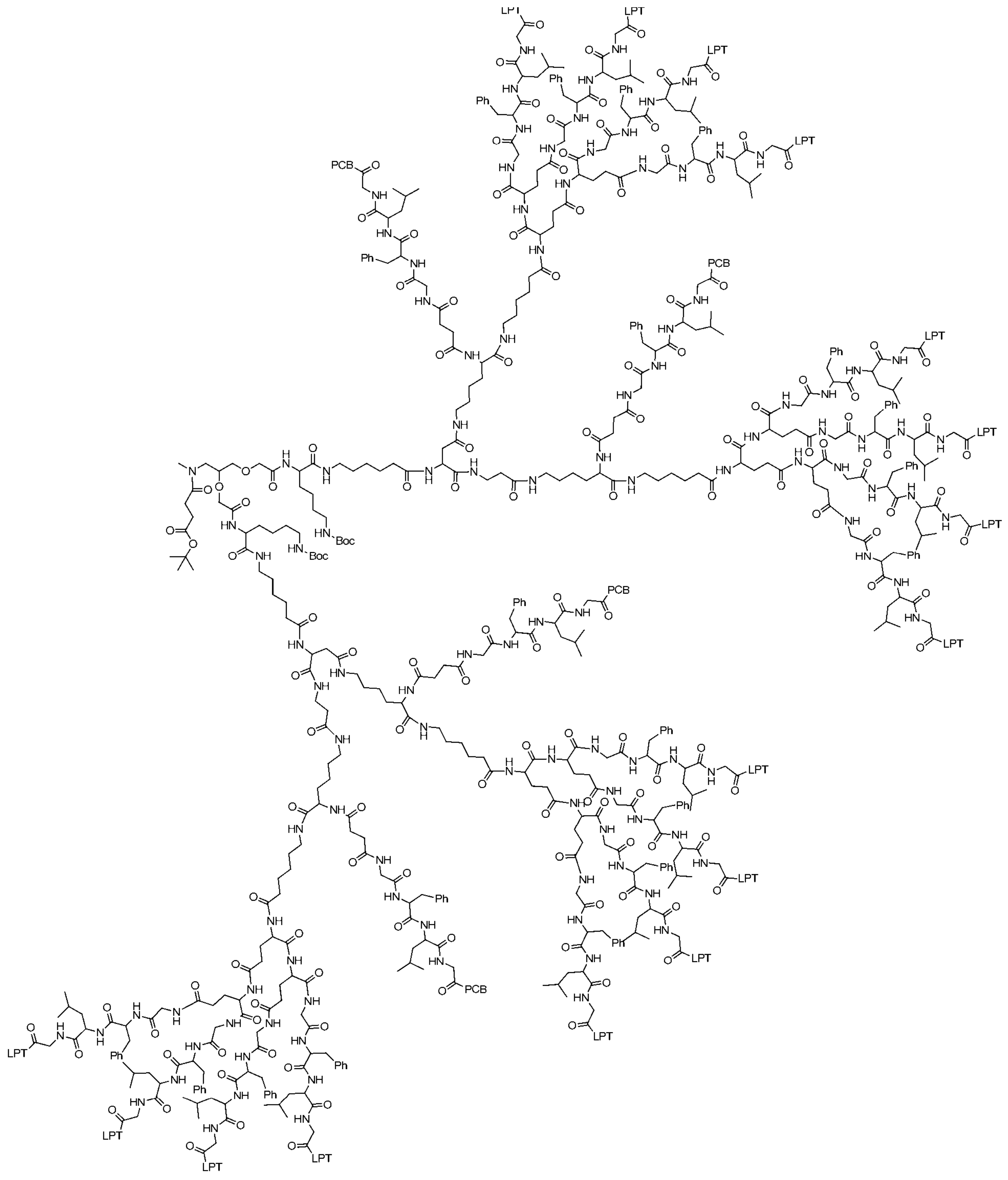

**81-197** (0.31 g, 0.217 mmol), **81-203** (5.3 g, 0.999 mmol), HBTU (0.378 g, 0.999 mmol) and HOBT (0.134 g, 0.999 mmol) were placed in a 50 mL round-bottom flask, and then DMF (20 mL) and NMP (10 mL) were added to dissolve the mixture. The mixture was stirred at 0°C for 1 minute. DIEA (0.57 mL, 3.472 mmol) was then slowly added dropwise to continue the reaction. After the reaction was completed, methanol (450 mL) was added to precipitate a solid, which was then filtered. The filter cake was redissolved in appropriate amounts of DMF and NMP, and methanol (450 mL) was then added to allow precipitation. This process was repeated three times. The filter cake was dried to provide 4.89 g of product.

1.39 Preparation of **81-205**

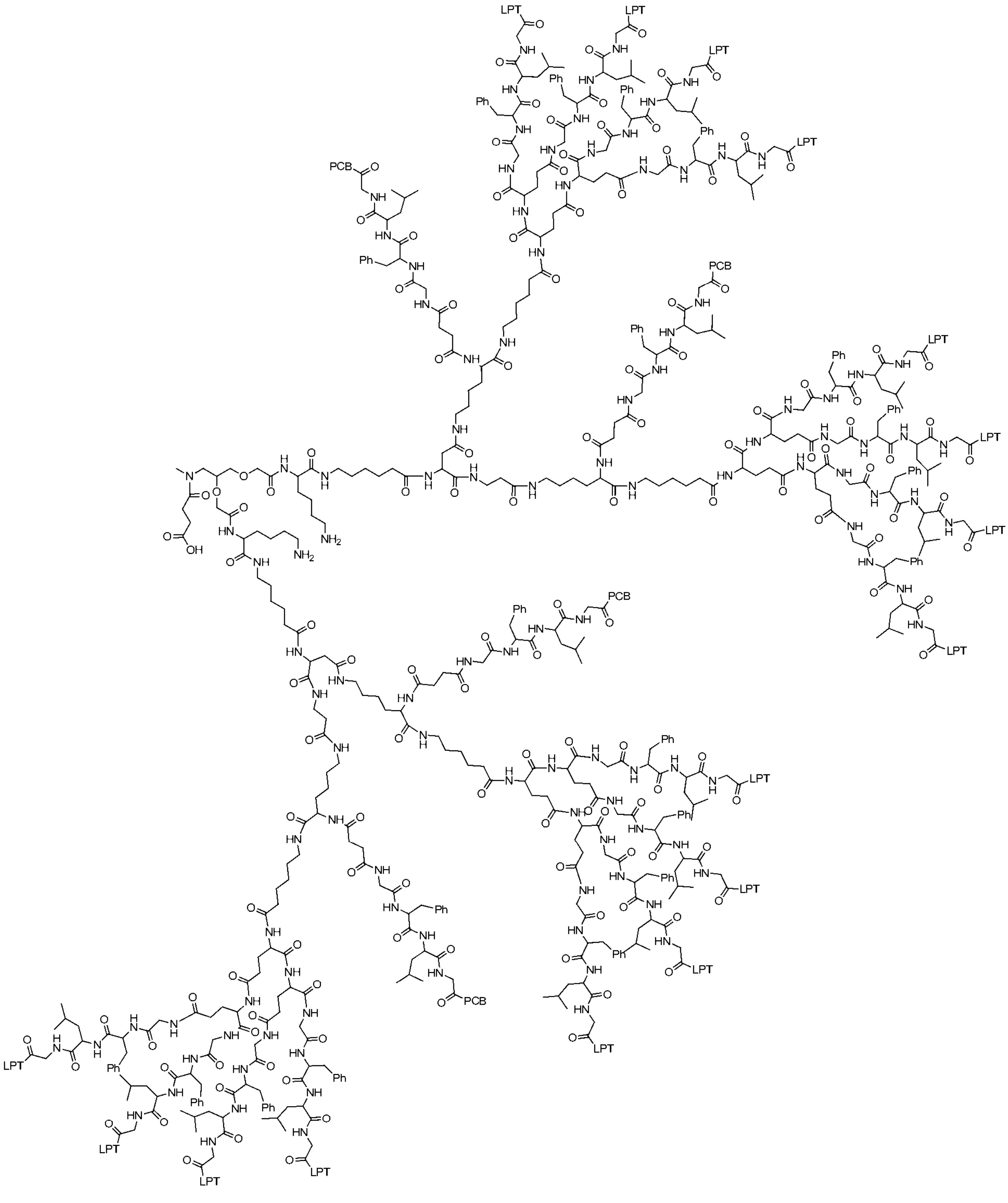

**81-204** (4.94 g, 0.218 mmol) was added to a 50 mL round-bottom flask, then TFA (35 mL) was added, and the mixture was reacted under stirring at room temperature for 20 hours. After the reaction was completed, an appropriate amount of dichloromethane was added for dissolution. The dichloromethane was removed by rotary evaporation, and methyl tert-butyl ether (30 mL) was added to precipitate a solid. The mixture was allowed to stand, the supernatant was discarded, and the remaining mixture was evaporated to dryness. The resultant solid was then dissolved by adding an appropriate amount of DMF, then DIEA was added dropwise, and the mixture was shaken. Methyl tert-butyl ether (30 mL) was then added, and a solid precipitated and was filtered with suction. The filter cake was dried to provide 4.5 g of product.

1.40 Preparation of **81-206**

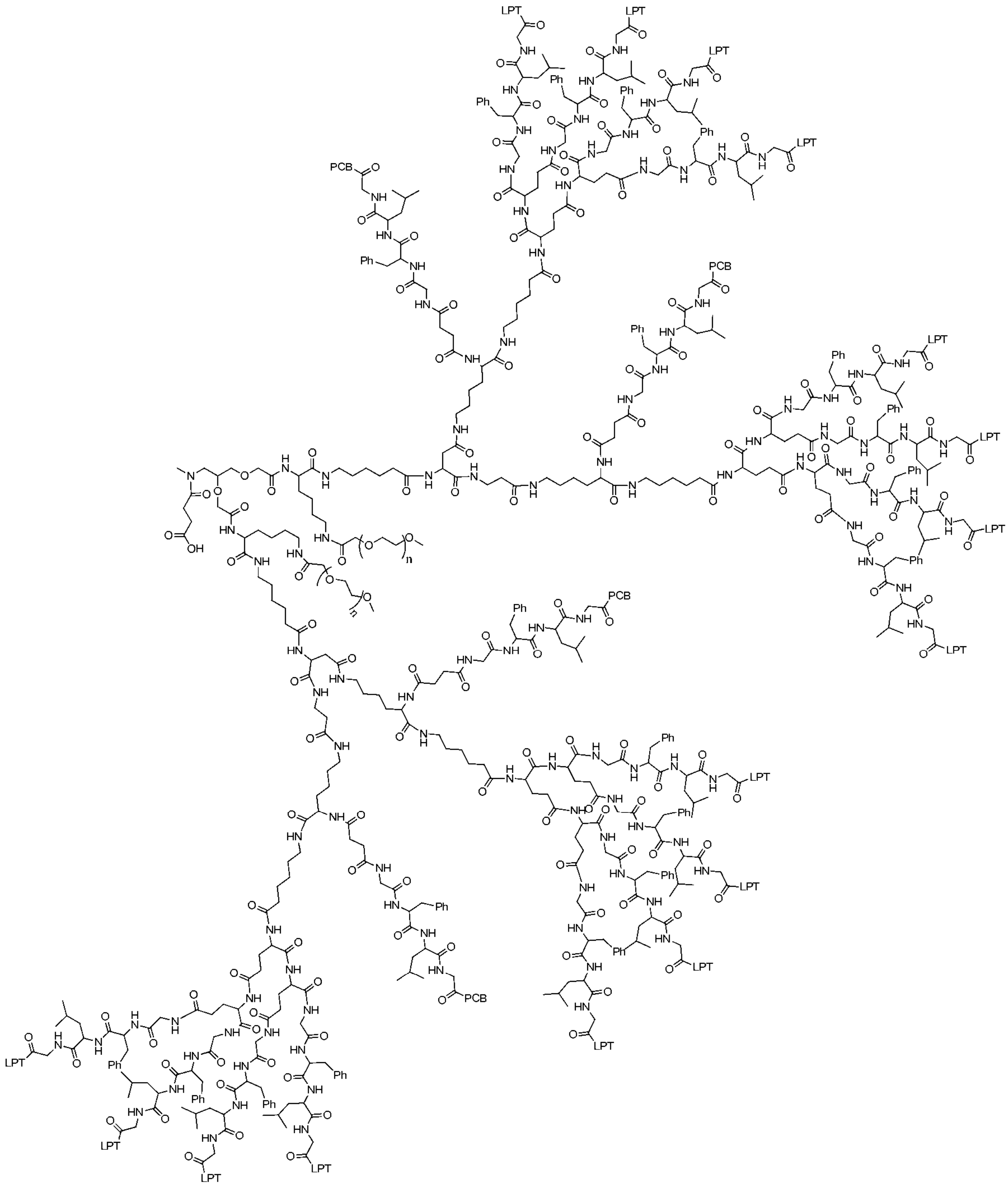

**81-205** (2 g, 0.896 mmol) was placed in a 50 mL round-bottom flask, and then ultra-dry DMF (10 mL) was added for dissolution with the assistance of ultrasonication. The mixture was stirred at 0°C for 3 minutes. DIEA (0.148 mL, 0.896 mmol) was then slowly added dropwise. The reaction was carried out under stirring for 5 minutes, and then M-SCM-10K (2 g, 0.188 mmol, purchased from Jenkem, batch number: A3016-N230101) was added. After the reaction was completed, methyl tert-butyl ether (450 mL) was added to precipitate a solid. The supernatant was discarded. MTBE was then added and ultrasonication was performed. The mixture was allowed to stand, and the supernatant was discarded. This process was repeated five times, and then the mixture was evaporated to dryness to provide a solid powder. The solid powder was then dissolved in an appropriate amount of a methanol/dichloromethane (4:6 by volume) mixture, then 100-200 mesh silica gel powder was added, and the mixture was evaporated to dryness. The resultant product was loaded by dry method, and subjected to column chromatography using 1% aqueous ammonia/6-9% methanol/dichloromethane as the eluent.

The desired product was collected, concentrated, and evaporated to dryness to provide 2.2 g of the product.

1.41 Preparation of **81-212**

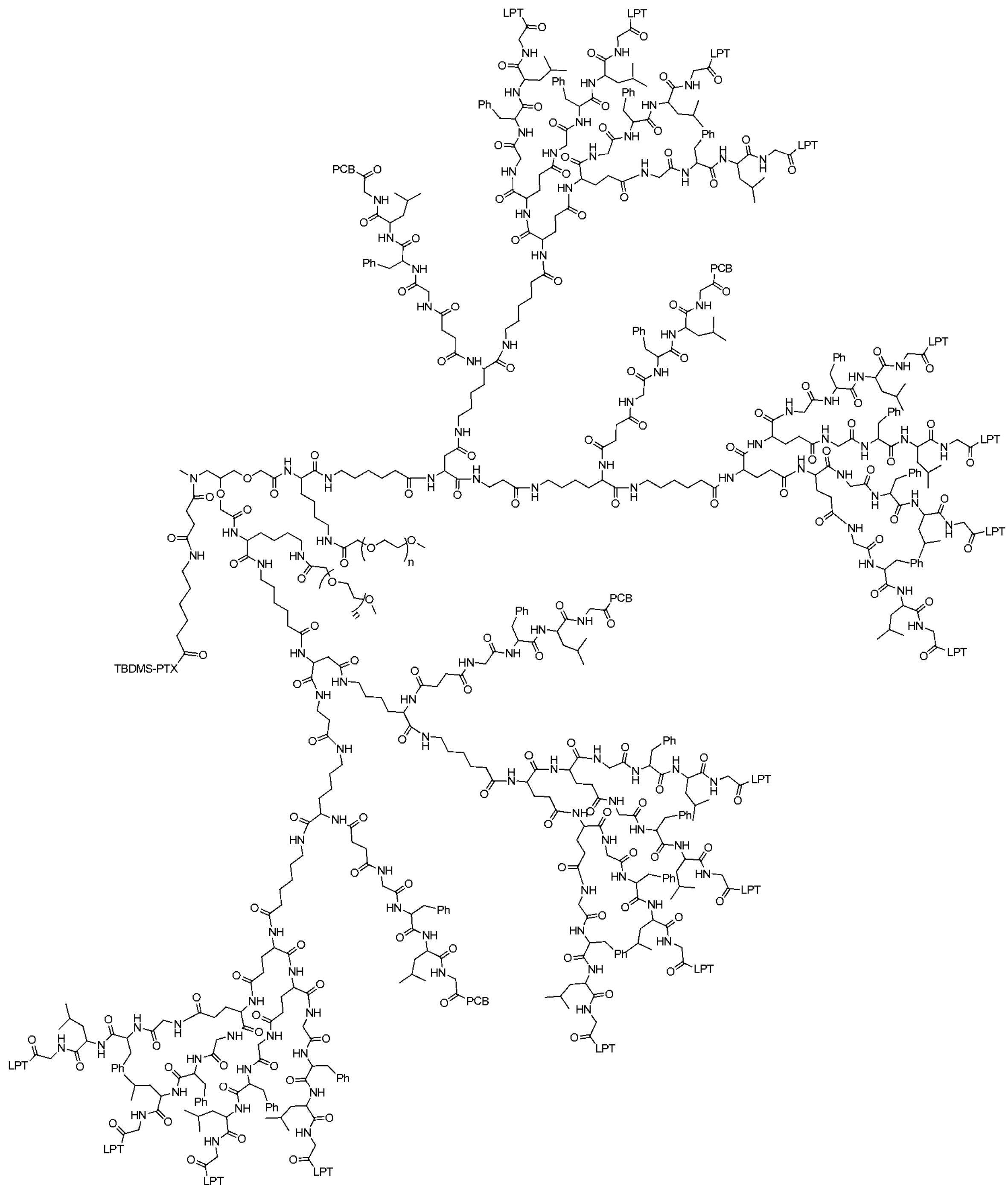

**81-206** (1.9 g, 0.0437 mmol), 59-69 (0.141 g, 0.131 mmol), HBTU (0.049 g, 0.1313 mmol) and HOBT (0.0177 g, 0.1313 mmol) were placed in a 50 mL round-bottom flask, then DMF (10 mL) was added for dissolution, and triethylamine (0.1 mL) was added dropwise to allow the reaction to proceed. After the reaction was completed, methyl tert-butyl ether (45 mL) was added to allow the precipitation of a solid. The mixture was then treated by ultrasonication, allowed to stand, and the supernatant was discarded. This process was repeated three times. The mixture was then evaporated to dryness to obtain a solid. The solid was dissolved in an appropriate amount of a methanol/dichloromethane (4:6 by volume) mixture,

and then a large amount of methyl tert-butyl ether was added, resulting in the precipitation of a solid. The mixture was allowed to stand, and the supernatant was discarded. The remaining solid was evaporated to dryness to provide 1.68 g of the product.

1.42 Preparation of **81-214**

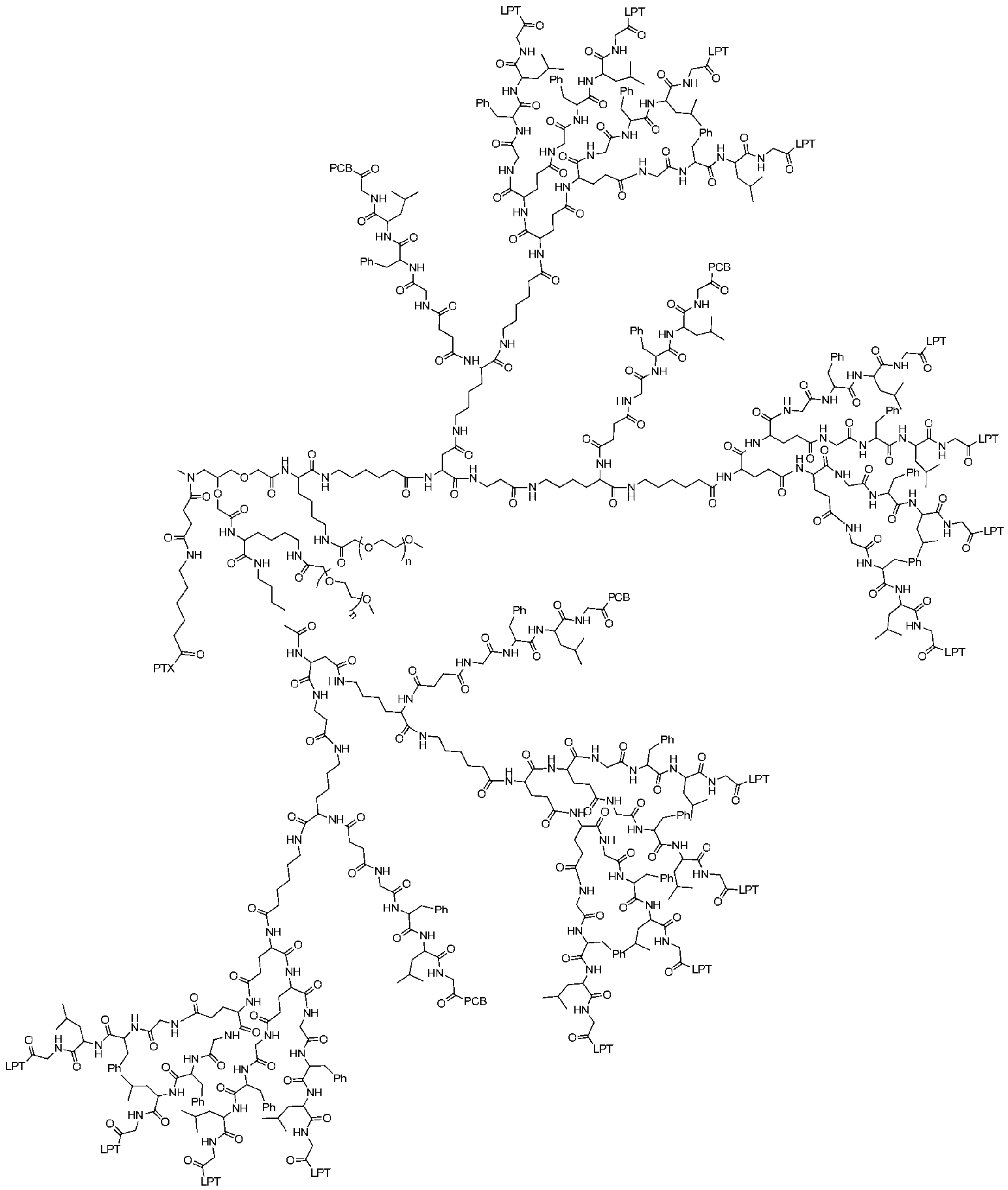

**81-212** (1.68 g, 0.0378 mmol) was weighed and dissolved in an appropriate amount of THF. TBAF (0.477 g, 1.512 mmol) was then added, and the reaction was carried out under stirring at room temperature. After the reaction was completed, methyl tert-butyl ether (300 mL) was added to precipitate a solid. The mixture was treated by ultrasonication, allowed to stand, and the supernatant was discarded. An appropriate amount of methyl tert-butyl ether was then added, subjected to ultrasonication treatment, and allowed to stand, the supernatant was poured out, and the remaining solid was evaporated to dryness. The solid was dissolved in an appropriate amount of methanol/dichloromethane (4/6) mixture, then 100-200 mesh silica gel powder was added, and the mixture was evaporated to dryness. The resultant product was loaded by dry method, and subjected to column chromatography using 1% triethylamine/10% methanol/dichloromethane as the

eluent. The desired product was collected, evaporated to dryness, and an appropriate amount of ethanol/dichloromethane (volume ratio of 3:7) mixture was added for dissolution. Methyl tert-butyl ether (300 mL) was then added to precipitate a solid, and the solid was filtered. This dissolution/precipitation process was repeated for three times. The filter cake was dried to provide 1.2 g of the product. $^1$H-NMR (600 MHz, DMSO-$d_6$) δ 10.16-10.04 (m, 5H), 9.93-9.77 (m, 16H), 8.96-8.80 (m, 5H), 8.80-8.66 (m, 16H), 8.60-8.47 (m, 15H), 8.29-7.89 (m, 169H), 7.89-7.58 (m, 19H), 7.58-7.36 (m, 22H), 7.36-6.96 (m, 183H), 6.72-6.59 (m, 10H), 6.57-6.47 (m,10H), 6.05--5.72 (m, 6H), 5.57-5.36 (m, 7H), 5.29-4.98 (m, 36H), 4.85-4.65 (m, 30H), 4.63-4.43 (m, 28H), 4.43-4.29 (m, 50H), 4.29-3.91 (m, 44H), 3.87-3.26 (m, 2053H), 3.20-2.67 (m, 173H), 2.65-2.24 (m, 65H), 2.24-2.01 (m, 37H), 1.95-1.78 (m, 17H), 1.78-1.07 (m, 195H), 0.94-0.66 (m, 120H)

Example 2: Synthesis of Compound **76-258**

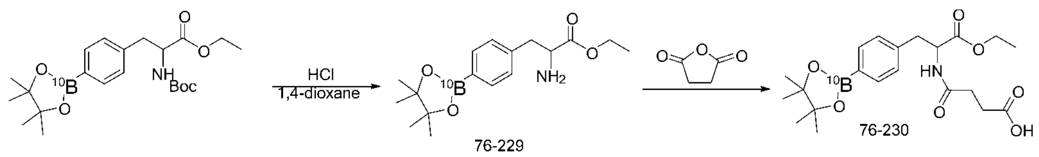

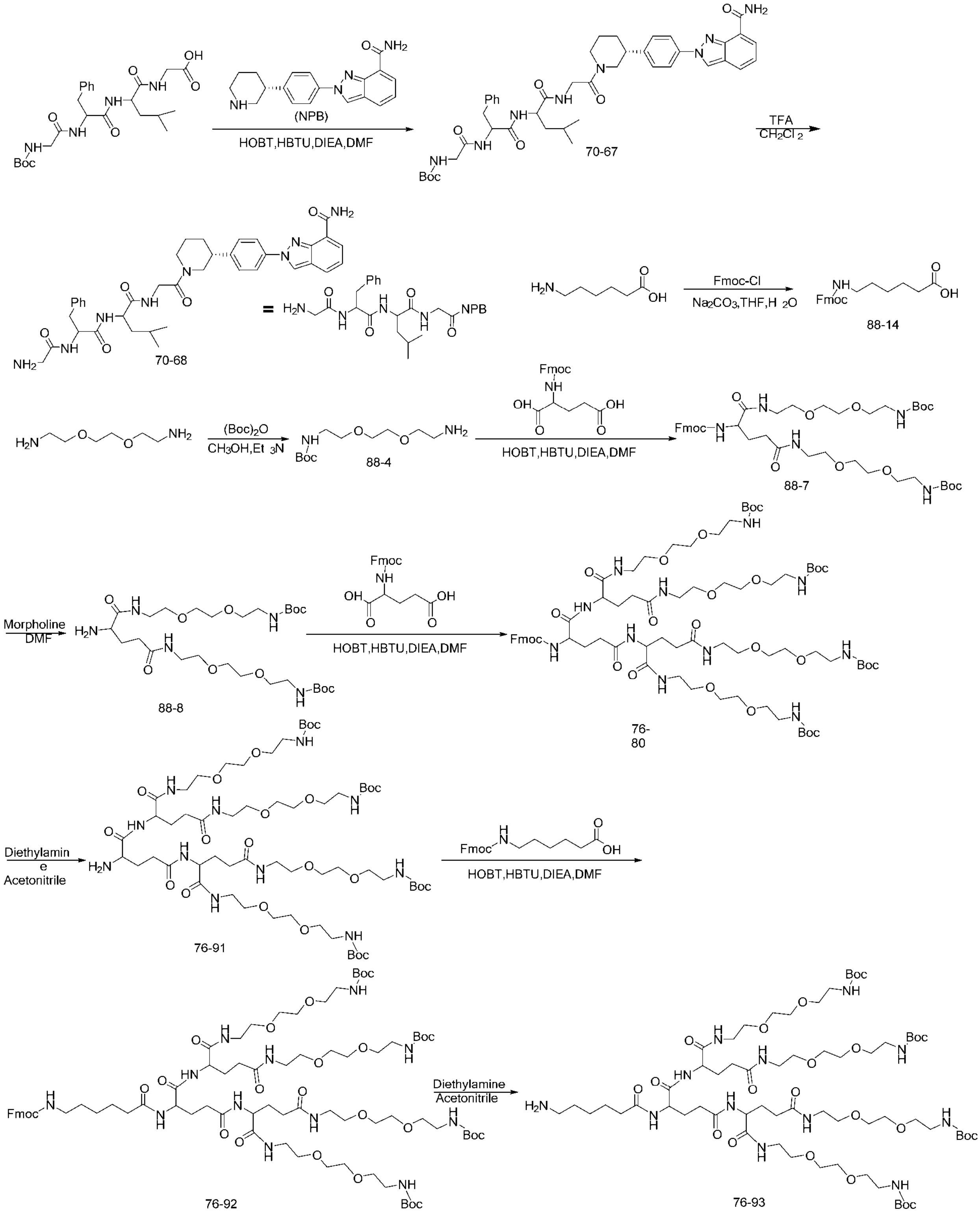


70-67

70-68

88-14

88-4

88-7

88-8

76-80

76-91

76-92

76-93

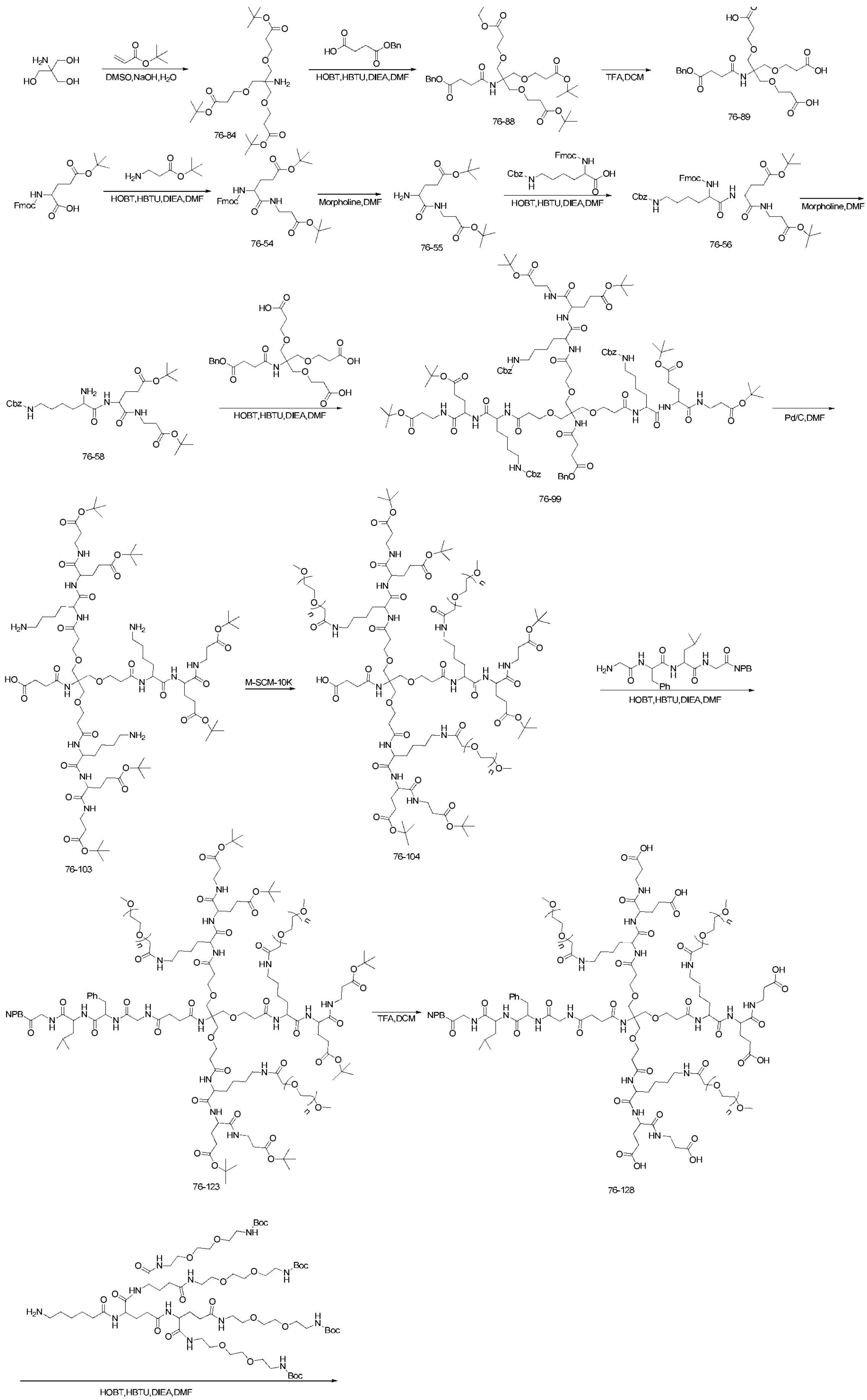


76-84

76-88

76-89

76-54

76-55

76-56

76-58

76-99

76-103

76-104

76-123

76-128

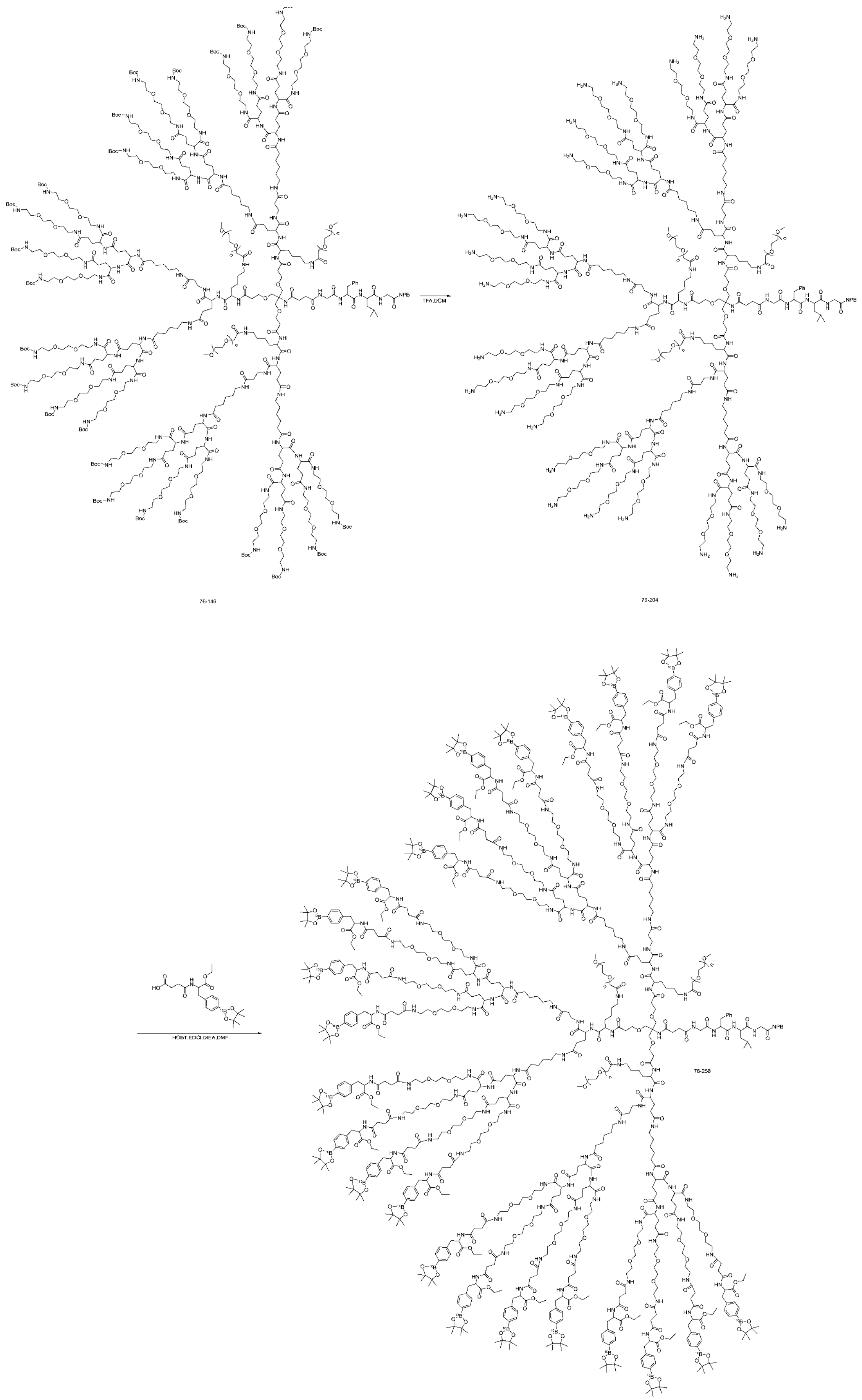


76-146

76-204

76-258

2.1 Preparation of Compound **76-84**

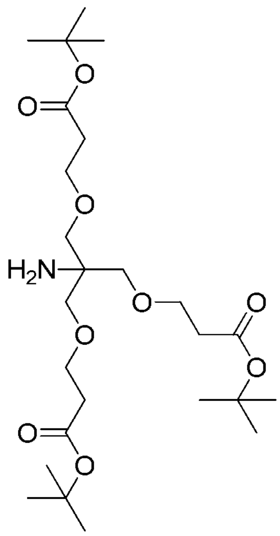

Trometamol (2.42 g, 20.00 mmol, purchased from LeYan) was placed in a 250 mL flask, DMSO (4 mL) was added, and the mixture was purged with $N_2$ and cooled to 15°C. While stirring, 5 M aqueous sodium hydroxide solution (0.4 mL) was added, followed by dropwise addition of tert-butyl acrylate (10.00 mL, 68.00 mmol). After the reaction was completed, saturated NaCl solution (200 mL) and ethyl acetate (200 mL) were added for extraction. The organic phase was collected after standing to separate the layers, and the aqueous phase was further extracted with ethyl acetate (200 mL × 3). The organic phases were combined and evaporated to dryness to provide a solid, then the solid was dissolved in methanol/dichloromethane (1/4) mixture. Then silica gel powder (100 mL) was added, and the mixture was evaporated to dryness to provide a powdery solid. The resultant product was loaded by dry method, and subjected to column chromatography using a mixed solvent of 0.2% ammonia water/2-4% methanol/dichloromethane as the eluent. The desired product was collected, concentrated, and dried in a vacuum oven to provide 3.85 g of the product with a yield of 38%. $^1$H-NMR (600 MHz, DMSO-$d_6$) δ 4.43-4.36 (m, 1H), 4.13-4.06 (m, 1H), 3.59-3.53 (m, 6H), 3.20-3.17 (m, 6H), 2.45-2.35 (m, 6H), 1.40 (s, 27H); ESI [M+H$^+$] 506.33

2.2 Preparation of **76-88**

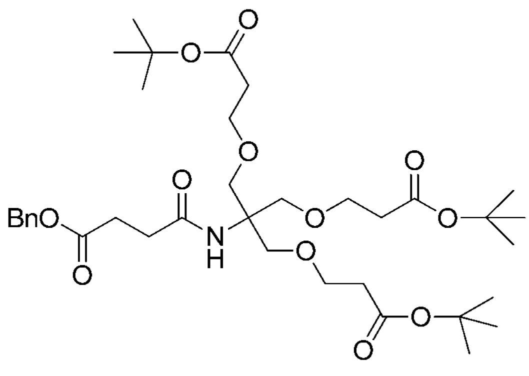

Monobenzyl succinate (0.46 g, 2.18 mmol), HBTU (0.90 g, 2.37 mmol) and HOBT (0.32 g, 2.37 mmol) were weighed and added into a flask containing **76-84** (1.0 g, 1.98 mmol), then an appropriate amount of DMF was added to dissolve the mixture, and the mixture was allowed to stand at -5°C. DIEA (1.18 mL, 7.12 mmol) was slowly added dropwise. After the reaction was carried out for half an hour, the flask was taken out and the mixture was stirred at room temperature overnight. After the reaction was completed, saturated NaCl solution (200 mL) and ethyl acetate (200 mL) were added for extraction. After standing and separation of layers, the organic phase was collected, and the aqueous phase was further

extracted with ethyl acetate (200 mL × 3). The organic phases were combined, and evaporated to dryness to provide a solid, then methanol/dichloromethane (1/4) mixture was added for dissolution. Silica gel powder (100 mL) was added, and the mixture was evaporated to dryness to provide a powdery solid. The resultant product was loaded by dry method, and subjected to column chromatography using a 20% ethyl acetate/petroleum ether mixture as the eluent. The desired product was collected, concentrated, and dried in a vacuum oven to provide 0.87 g of the product. $^1$H-NMR (600 MHz, DMSO-$d_6$) δ 7.38-7.32 (m, 5H), 7.12 (s, 1H), 5.07 (s, 2H), 3.54-3.51 (m, 12H), 2.40-2.36 (m, 8H), 1.40-1.38 (m, 27H), 1.32-1.22 (m, 2H); ESI [M+H$^+$] 696.39, [M+Na$^+$] 718.37, [M+K$^+$] 734.35

2.3 Preparation of **76-89**

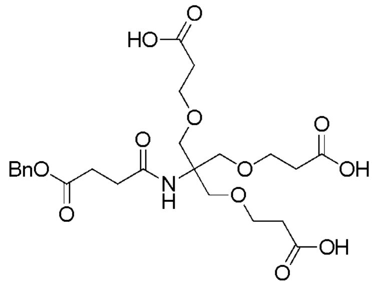

To a flask containing **76-88** (0.87 g, 1.25 mmol) was added dichloromethane. Ultrasonication treatment was performed until complete dissolution, and then TFA (0.2 mL) was added and reacted under stirring at room temperature overnight. After the reaction was completed, the reaction mixture was evaporated to obtain an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide the product. $^1$H-NMR (600 MHz, DMSO-$d_6$) δ 12.58-11.75 (s, 3H), 7.38-7.32 (m, 5H), 7.12 (s, 1H), 5.07 (s, 2H), 3.54-3.51 (m, 12H), 2.40-2.36 (m, 8H), 1.32-1.22 (m, 2H)

2.4 Preparation of **76-54**

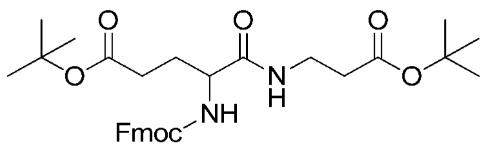

Fmoc-Glu(OtBu)-OH (10.00 g, 23.50 mmol), HBTU (10.69 g, 28.20 mmol) and HOBT (3.81 g, 28.20 mmol) were added into a flask containing β-alanine tert-butyl ester hydrochloride (4.48 g, 24.68 mmol). An appropriate amount of DMF was then added to dissolve the mixture. DIEA (13.98 mL, 84.60 mmol) was slowly added dropwise at -5°C. After the reaction was carried out for half an hour, the flask was taken out and the mixture was stirred at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a crude solid product.

2.5 Preparation of **76-55**

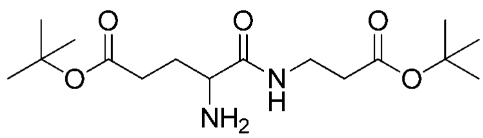

To a flask containing **76-54** (23.50 mmol), DMF was added for dissolution with the assistance of ultrasonication, and then morpholine (40 mL, 470 mmol) was added and reacted under stirring at room temperature for 2 h. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid, and then methanol/dichloromethane (1/4) mixture was added for dissolution. Silica gel powder (100 mL) was added, and the mixture was evaporated to dryness to form a powdery solid. The resultant product was loaded by dry method, and subjected to column chromatography using a 20% ethyl acetate/petroleum ether mixture as the eluent. The desired product was collected, concentrated, and dried in a vacuum oven to provide 6.89 g of the product in an 88.7% yield.

2.6 Preparation of **76-56**

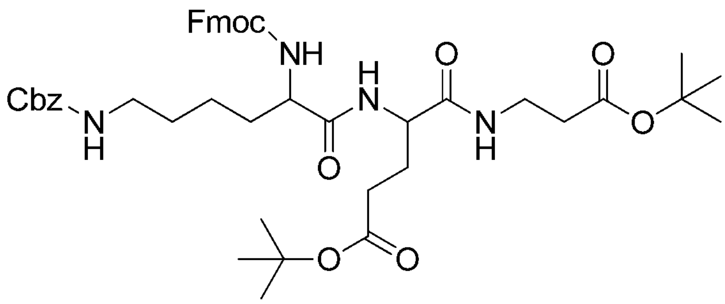

N'-Fmoc-N-benzyloxycarbonyl-L-lysine (2.90 g, 5.76 mmol), HBTU (2.62 g, 6.91 mmol) and HOBT (0.93 g, 6.91 mmol) were weighed and added into a flask containing **76-55** (2.0 g, 6.05 mmol), and then an appropriate amount of DMF was added to dissolve the mixture. The mixture was allowed to stand at -5°C, and DIEA (3.43 mL, 20.74 mmol) was slowly added dropwise. After the reaction was carried out for half an hour, the flask was taken out and the mixture was stirred at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a crude solid product.

2.7 Preparation of **76-58**

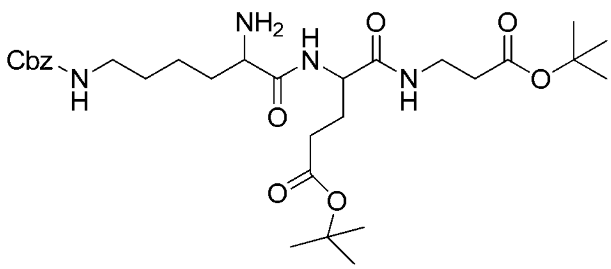

To a flask containing **76-56** (5.76 mmol), DMF was added for dissolution with the assistance of ultrasonication, then morpholine (10 mL, 115.21 mmol) was added, and the mixture was reacted under stirring at room temperature for 2 h. After the reaction was completed, saturated NaCl solution (200 mL) and ethyl acetate (200 mL) were added for extraction. After standing for layer separation, the organic phase was collected. The aqueous phase was further extracted with ethyl acetate (200 mL × 3). The organic phases were combined and evaporated to dryness to provide a solid. The solid was then dissolved in methanol/dichloromethane (1/4) mixture, then silica gel powder (100 mL) was added, and the mixture was evaporated to dryness to form a powdery solid. The resultant product was loaded by dry method, and subjected to column chromatography using a 20% ethyl acetate/petroleum ether mixture as the eluent. The desired product was collected, concentrated, and dried in a vacuum oven to provide 2.26 g of the product in a yield of 66.2%. $^1$H-NMR (400 MHz, DMSO-$d_6$) δ 8.14-7.97 (m, 2H), 7.38-7.29 (m, 5H), 7.26-7.21 (s, 1H), 5.04-4.98(m, 2H), 4.26-4.18 (s, 1H), 3.33-3.25(m, 2H), 3.20-3.10 (m,2H), 2.98-2.94 (m, 2H), 2.38-2.31 (m, 2H), 2.19-2.11 (m, 2H), 1.91-1.79 (m, 2H), 1.72-1.66 (m, 1H), 1.59-1.47

(m, 2H), 1.40-1.38 (m, 9H), 1.38-1.37 (m, 9H), 1.35-1.22 (m, 4H); ESI [M+H$^+$] 593.35, [M+Na$^+$] 615.33, [M+K$^+$] 631.30

2.8 Preparation of **76-99**

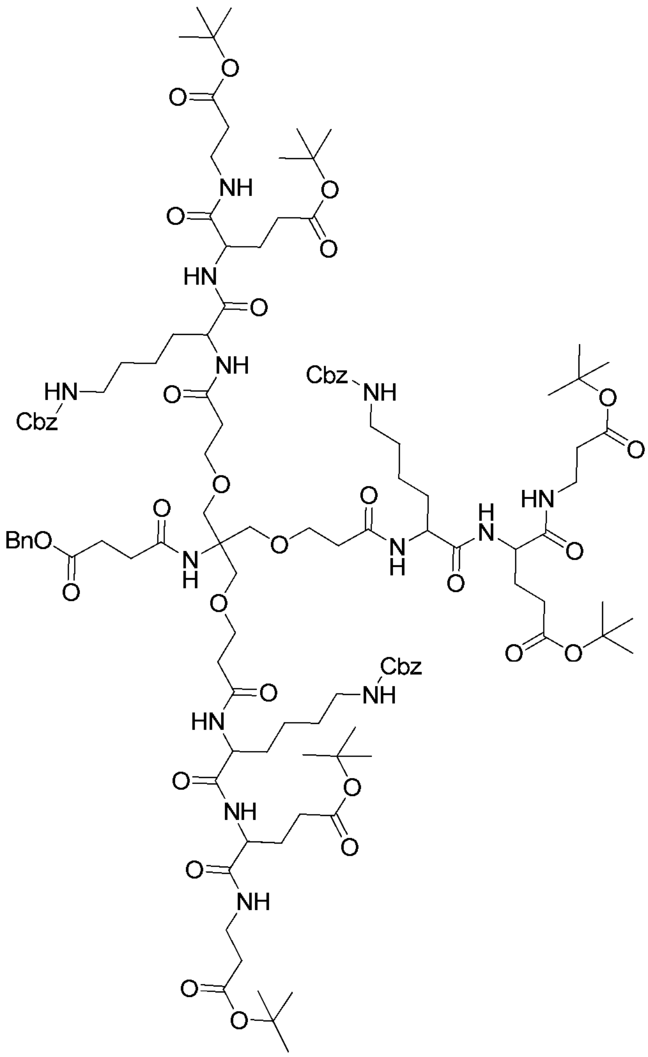

**76-89** (0.28 g, 0.55 mmol), HBTU (0.75 g, 1.98 mmol) and HOBT (0.27 g, 1.98 mmol) were weighed and added into a flask containing **76-58** (1.07 g, 1.81 mmol), and an appropriate amount of DMF was added to dissolve the mixture. DIEA (1.0 mL, 5.94 mmol) was slowly added dropwise at -5°C. After the dropwise addition was completed, the reaction was carried out for half an hour, the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid, and the solid was then dissolved in methanol/dichloromethane (1/4) mixture. Silica gel powder (100 mL) was added, and the mixture was evaporated to dryness to form a powdery solid. The resultant product was loaded by dry method, and subjected to column chromatography using a mixed solvent of dichloromethane and methanol as the eluent. The desired product was collected, concentrated, and dried in a vacuum oven to provide 0.82 g of the product. $^1$H-NMR (400 MHz, DMSO-$d_6$) δ 8.02-7.97 (m, 3H), 7.97-7.93 (m, 3H), 7.93-7.88 (m, 3H), 7.37-7.29(m, 20H),7.24-7.17 (m,3H), 7.15-7.13(m, 1H), 5.09-5.05 (m, 2H), 5.02-4.96 (m, 6H), 4.25-4.20 (m, 3H), 4.20-4.15 (m, 3H), 3.58-3.50 (m, 12H), 3.32-3.26 (m, 6H), 3.18-3.12 (m, 3H), 2.98-2.92 (m, 6H), 2.44-2.30(m, 15H), 2.19-2.11 (m, 6H), 1.85-1.79 (m, 3H), 1.51-1.43 (m, 6H), 1.38-1.36(m, 54H), 1.34-1.32(m, 6 H), 1.25-1.23 (m, 6H), 1.23-1.21 (m, 6H)

2.9 Preparation of **70-67**

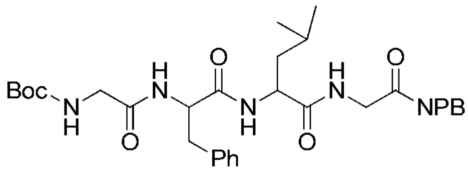

**71-85** (34.3 mmol), NPB (11.0 g, 34.3 mmol, purchased from Anhui Nuoquan), HBTU (15.6 g, 41.2 mmol) and HOBT (5.6 g, 41.2 mmol) were placed into a 500 mL round-bottom flask, and DMF (80 mL) was added to dissolve the mixture. The mixture was carried out under stirring at -5°C for approximately 20 minutes. DIEA (12.5 mL, 75.5 mmol) was then slowly added dropwise. The reaction was carried out under stirring at -5°C for 1 hour. The mixture was taken out and reacted under stirring at room temperature. After the reaction was completed, methyl tert-butyl ether (450 mL) was added for precipitation. The solid precipitated and was filtered. The filter cake was redissolved in an appropriate amount of DMF, and then methyl tert-butyl ether (450 mL) was added for precipitation. This process was repeated three times. The filter cake was collected and dried to provide 28.8 g of the product.

2.10 Preparation of **70-68**

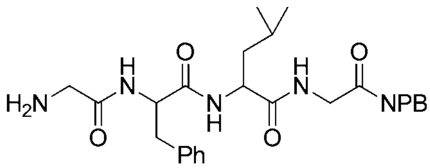

**70-67** (34.3 mmol) was placed into a 250 mL round-bottom flask. Dichloromethane (20 mL) was added for dissolution with the assistance of ultrasonication. Then TFA (51.8 mL, 686 mmol) was added, and the reaction was stirred overnight at room temperature. After the reaction was completed, the mother liquor was concentrated and evaporated to dryness to remove the dichloromethane. Methyl tert-butyl ether (300 mL) was then added to allow precipitation. The precipitated solid was filtered, and the filter cake was washed three times with methyl tert-butyl ether (40 mL). The filter cake was collected and vacuum dried to provide 13.0 g of the product.

2.11 Preparation of **76-103**

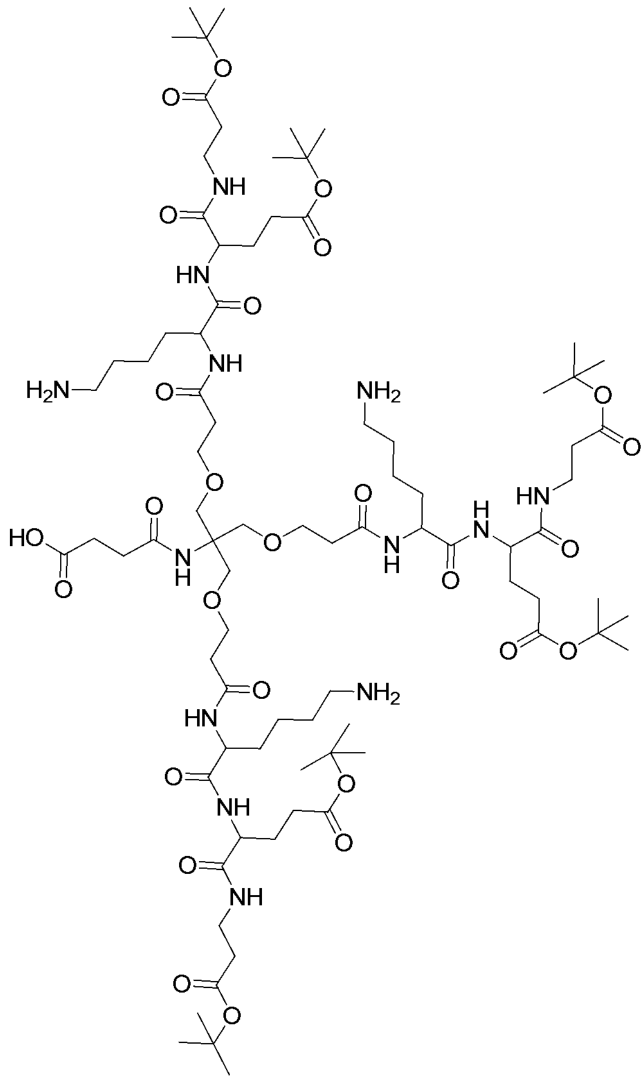

**76-99** (0.15 g, 0.07 mmol) and 10% Pd/C catalyst (0.05 g) were placed in a hydrogenation reactor, and then DMF (20 mL) was added for dissolution. The hydrogenation reactor was sealed and evacuated with a water pump. Hydrogen gas was then introduced to approximately 2 MPa. This process was repeated three times. The pressure of the hydrogenation reactor was finally adjusted to 1.8 MPa. The reaction was then allowed to proceed overnight at room temperature. After the reaction was completed, the reaction mixture was filtered through Celite, and the filter cake was washed with DMF (20 mL × 3) to provide a DMF solution of the product, which served as the raw material for the next reaction. $^1$H-NMR (400 MHz, DMSO-$d_6$) δ 12.50-11.71 (s,1H),8.02-7.97 (m, 3H), 7.97-7.93 (m, 3H), 7.93-7.88 (m, 3H) ,7.24-7.17 (m, 3H), 7.15-7.13(m, 1H), 4.25-4.20 (m, 3H), 4.20-4.15 (m, 3H), 3.58-3.50 (m, 12H), 3.32-3.26 (m, 6H),3.18-3.12 (m, 3H), 2.98-2.92(m, 6H), 2.44-2.30(m,15 H), 2.19-2.11 (m, 6H), 1.85-1.79 (m, 3H), 1.51-1.43 (m, 6H), 1.38-1.36 (m, 54H), 1.34-1.32 (m, 6H), 1.25-1.23 (m, 6H), 1.23-1.21 (m, 6H)

2.12 Preparation of **76-104**

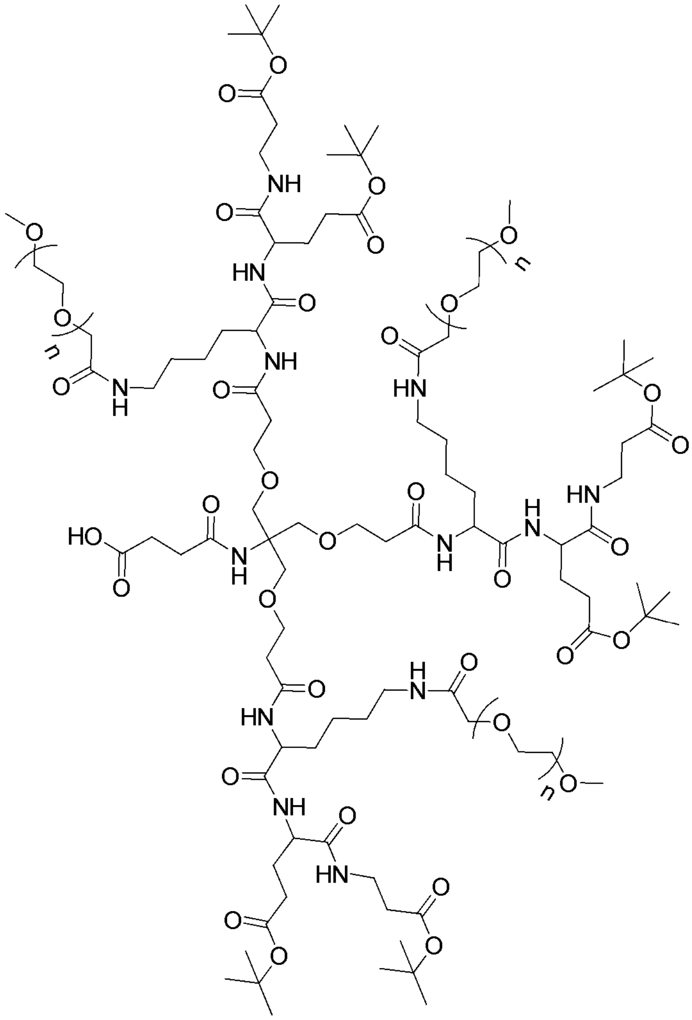

**76-103** (0.07 mmol) was placed in a 250 mL flask, dissolved in DMF (20 mL), and then slowly added dropwise to a DMF solution containing DIEA (0.44 mL, 2.66 mmol) and M-SCM-10K (2.29 g, 0.22 mmol, purchased from Jenkem). The reaction mixture was allowed to react under stirring in the dark at low speed at room temperature for one week. After the reaction was completed, n-hexane (25 mL) and methyl tert-butyl ether (200 mL) were added for precipitation. The supernatant was then discarded, and n-hexane and methyl tert-butyl ether were added again for precipitation. This process was repeated three times. The solid product was filtered, collected, and dried in a vacuum oven to provide the product.

2.13 Preparation of **76-123**

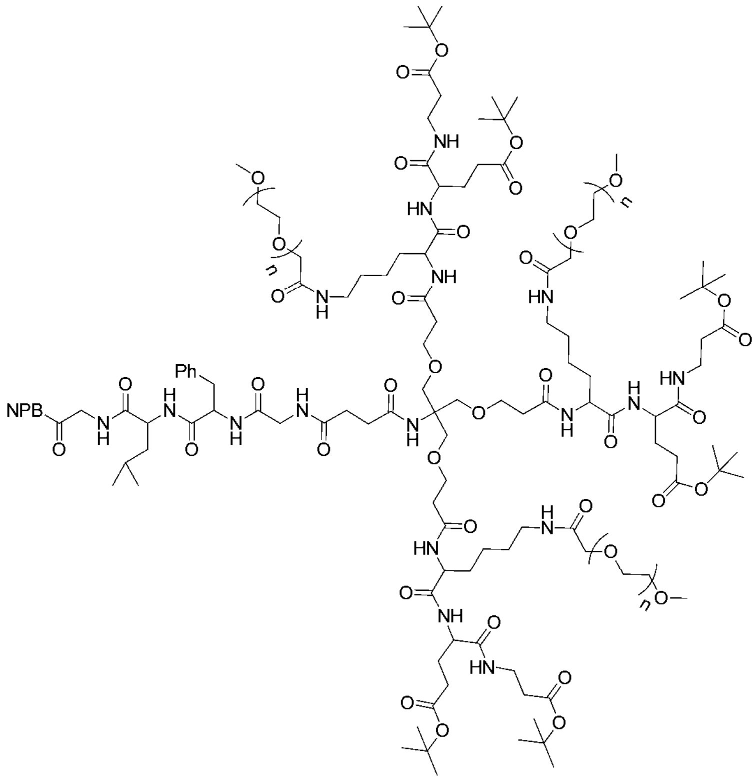

**76-104** (0.46 g, 0.01 mmol), HBTU (0.01 g, 0.02 mmol) and HOBT (0.003 g, 0.02 mmol) were weighed and added into a flask containing **70-68** (0.01 g, 0.02 mmol), and an appropriate amount of DMF was then added to dissolve the mixture. The mixture was then placed at -5°C and DIEA (0.02 mL, 0.07 mmol) was slowly added dropwise. After the dropwise addition was completed, the mixture was brought to room temperature and reacted overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The solid product was filtered with suction, collected and dried to provide the product.

2.14 Preparation of **76-128**

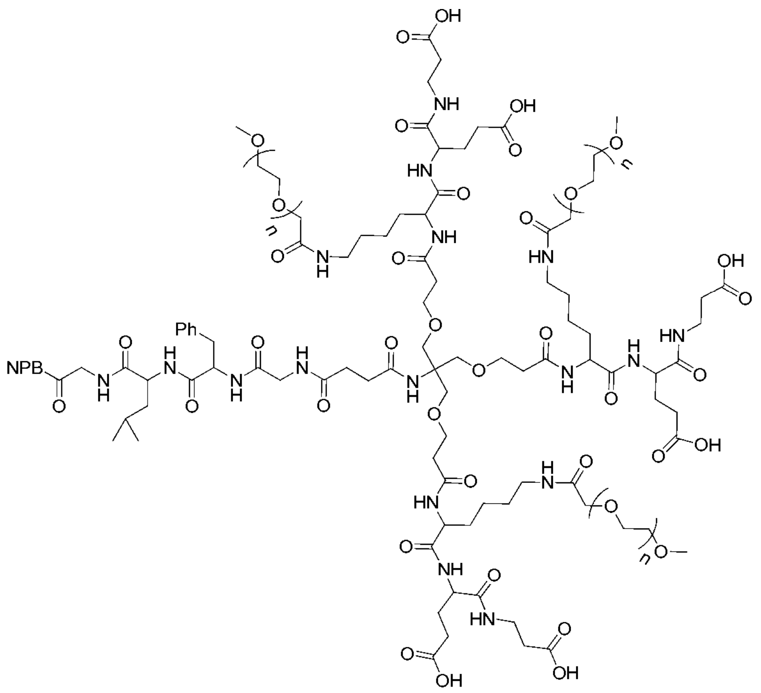

Dichloromethane was added into a flask containing **76-123** (0.01 mmol) for dissolution with the assistance of ultrasonication, then TFA (10.0 mL) was added, and the mixture was reacted under stirring at room temperature overnight. After the reaction was completed, the reaction mixture was evaporated to obtain an oil using a rotary evaporator. MTBE (60 mL) was then added, and a powdery solid precipitated from the reaction mixture and filtered. The filter cake was washed with MTBE (40 mL × 3). The product had poor solubility. The filter cake was collected and dried in a vacuum oven to provide the product.

2.15 Preparation of**88-14**

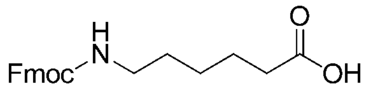

6-Aminohexanoic acid (4.6094 g, 35.1407 mmol) was placed in a 1 L round-bottom flask, THF/$H_2O$ solution (150 mL) was added for dissolution, and then the mixture was reacted under stirring in a 0°C reaction bath for 1 hour. Anhydrous sodium carbonate (7.4491 g, 70.2814 mmol) was then added, and dissolved with the assistance of ultrasonication. The reaction flask was kept in a 0°C reaction bath, and stirring was continued for 30 minutes. 9-Fluorenylmethyl chloroformate (Fmoc-Cl, 10.0 g, 38.6548 mmol) was then dissolved in THF (30 mL) and slowly added dropwise to the reaction flask. After the addition was completed, the reaction flask was taken out from the 0°C reaction bath, and stirring was performed at room temperature for 2.5 hours. Finally, 1.0 mol/L aqueous hydrochloric acid (115 mL) was added dropwise to adjust the pH of the reaction mixture to 3.0. After the reaction was completed, the reaction mixture was transferred to a 1 L separatory funnel and extracted with ethyl acetate (150 mL × 3). The aqueous and organic phases were separated. The organic phases were combined and washed twice with 1.0 N hydrochloric acid (250 mL × 2). The aqueous and organic phases were separated. The organic phases were concentrated under reduced pressure and evaporated to dryness. Dichloromethane (100 mL) was then added for dissolution. Silica gel powder (60 mL) was then added, and the mixture was evaporated to dryness. The resultant product was loaded by dry method, and subjected to column chromatography using 20-50% ethyl acetate/petroleum ether as the eluent. The desired product was collected, concentrated, evaporated, and dried to provide 12.0 g of the product in a yield of 96.63%.

2.16 Preparation of **88-4**

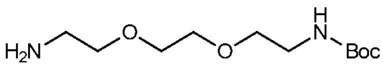

In a 1 L round-bottom flask, 1,2-bis(2-aminoethoxy)ethane (5.96 g, 40.270 mmol) was dissolved in 1,4-dioxane (40 mL), and the flask was placed in a constant temperature 0°C reaction bath. NaOH (1.932 g) was dissolved in pure water (20 mL), and $Boc_2O$ (10.54 g, 48.324 mmol) was added. The reaction was carried out under stirring at room temperature for 2 hours. The reaction progress was monitored by TLC. After the reaction was completed, water (100 mL) was added, and methyl tert-butyl ether was used for extraction. The organic phase was collected. The aqueous phase was adjusted to pH 1-2 with hydrochloric acid (1.0 mol/L), and further extracted with ethyl acetate. The organic phases were combined, and dried by rotation to dryness to provide 9.88 g of the product with a yield of 98.8%.

2.17 Preparation of **88-7**

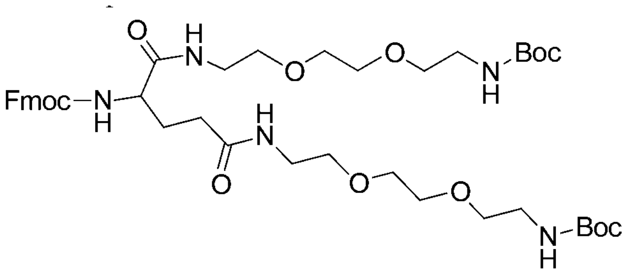

Fmoc-Glu-OH (3.45 g, 9.34 mmol), HBTU (7.79 g, 20.54 mmol) and HOBT (2.78 g, 20.54 mmol) were weighed and added into a flask containing **88-4** (5.10 g, 20.54 mmol), and an appropriate amount of DMF was added to dissolve the mixture. DIEA (6.80 mL, 41.07 mmol) was then slowly added dropwise at -5°C. After the dropwise addition was completed, the reaction was carried out for half an hour, the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid.

2.18 Preparation of **88-8**

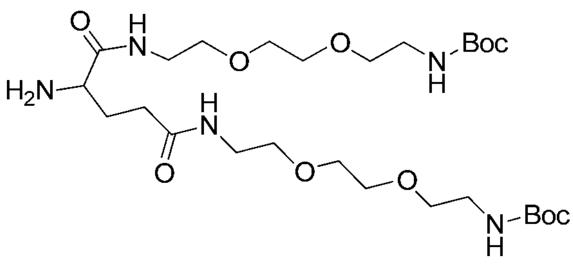

Acetonitrile was added into a flask containing **88-7** (9.34 mmol). After complete dissolution with the assistance of ultrasonication, piperidine (13.90 mL, 140.03 mmol) was added and reacted under stirring at room temperature for 2 h. After the reaction was completed, the mixture was evaporated to dryness to provide a solid. The solid was then dissolved in dichloromethane/methanol (4/1) mixture, then 200-300 mesh silica gel powder (30 g) was added, and the mixture was evaporated to dryness. The resultant product was loaded by dry method, and subjected to column chromatography using 1% methanol/dichloromethane as the eluent. The desired product was collected, concentrated and evaporated to dryness to provide 7.0 g of the product.

2.19 Preparation of **76-80**

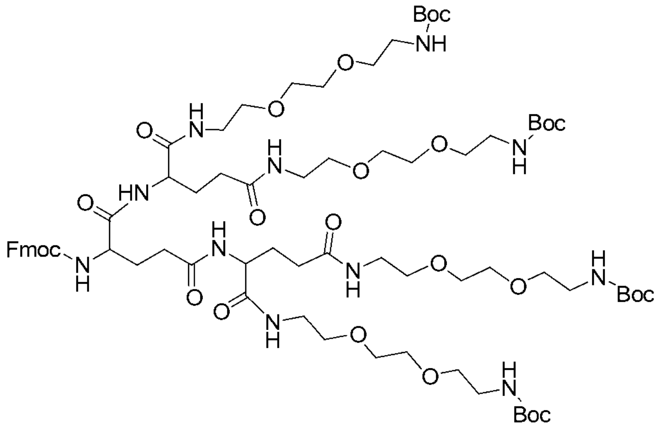

Fmoc-Glu-OH (0.72 g, 1.94 mmol), HBTU (1.77 g, 4.66 mmol) and HOBT (0.63 g, 4.66 mmol) were weighed and added into a flask containing **88-8** (2.60 g, 4.28 mmol), and then an appropriate amount of DMF was added to dissolve the mixture. DIEA (2.31 mL, 13.97 mmol) was slowly added dropwise at -5°C. After the dropwise addition was completed, the reaction was carried out for half an hour, the flask was then taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid.

2.20 Preparation of **76-91**

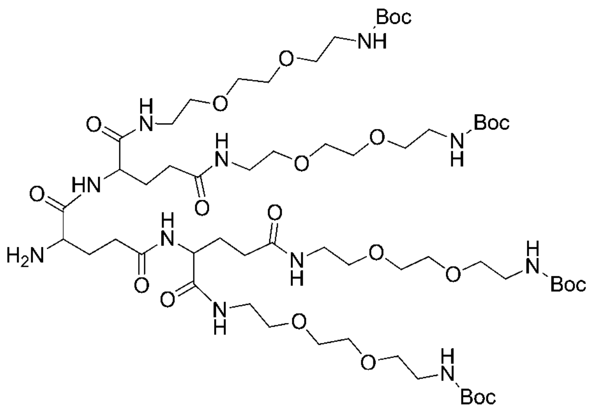

Acetonitrile was added into a flask containing **76-80** (1.5 g, 0.97 mmol). After dissolution with the assistance of ultrasonication, diethylamine (20 mL) was added, and reacted under stirring at room temperature for 2 h. After the reaction was completed, the mixture was evaporated to dryness to provide a solid.

2.21 Preparation of **76-92**

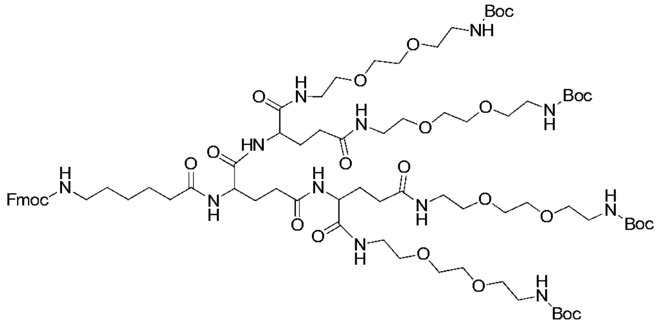

**88-14** (0.36 g, 1.02 mmol), HBTU (0.44 g, 1.16 mmol) and HOBT (0.16 g, 1.16 mmol) were weighed and added into a flask containing **76-91** (0.97 mmol), and then an appropriate amount of DMF was added to dissolve the mixture. DIEA (0.58 mL, 3.49 mmol) was slowly added dropwise at -5°C. After the dropwise addition was completed, the reaction was carried out for half an hour, and then the flask was taken out and the reaction was carried out under stirring at room temperature overnight. After the reaction was completed, n-hexane (30 mL) and methyl tert-butyl ether (200 mL) were added for precipitation. The supernatant was discarded, and methyl tert-butyl ether (100 mL) was added for precipitation. The mixture was filtered to provide a solid product, and the filter cake was collected and dried under vacuum to provide the product.

2.22 Preparation of **76-93**

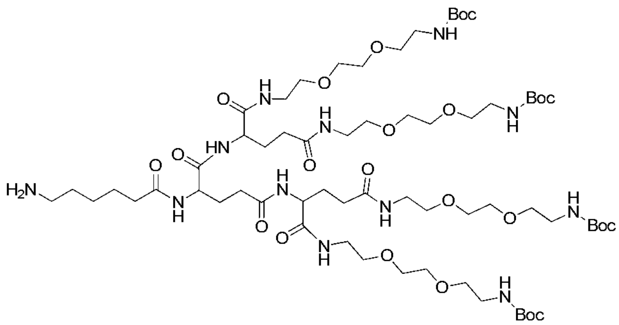

Acetonitrile was added into a flask containing **76-92** (0.97 mmol). After dissolution with the assistance of ultrasonication, diethylamine (20 mL) was added and reacted under stirring at room temperature for 2 h. After the reaction was completed, the mixture was evaporated to dryness to provide a solid. The solid was then dissolved in dichloromethane/methanol (4/1) mixture, then 200-300 mesh silica gel powder (30 g) was added, and the mixture was evaporated to dryness. The resultant product was loaded by dry method, and subjected to column chromatography using 1% methanol/dichloromethane as the eluent. The desired product was collected, concentrated, and evaporated to dryness to provide 0.75 g of the product in a yield of 54%. $^1$H-NMR (400 MHz, DMSO-$d_6$) δ 8.25-8.16 (m, 2H), 8.12-8.06 (m, 2H), 8.03-7.99 (m, 1H), 7.89-7.85 (m, 1H), 7.85-7.82 (m, 1H), 6.80-6.74(m, 4H), 4.23-4.17 (m, 2H), 4.12-4.06 (m, 1H), 3.50-3.47 (m, 16H), 3.42-3.36 (m, 22H), 3.29-3.23 (m, 4H), 3.07-3.04 (m, 8H), 2.73-2.69 (m, 2H), 2.16-2.08 (m, 8H), 1.53-1.43 (m, 6H), 1.38-1.36 (m, 36H), 1.31-1.23 (m, 4H); ESI [M+H$^+$] 1439.88

2.23 Preparation of **76-146**

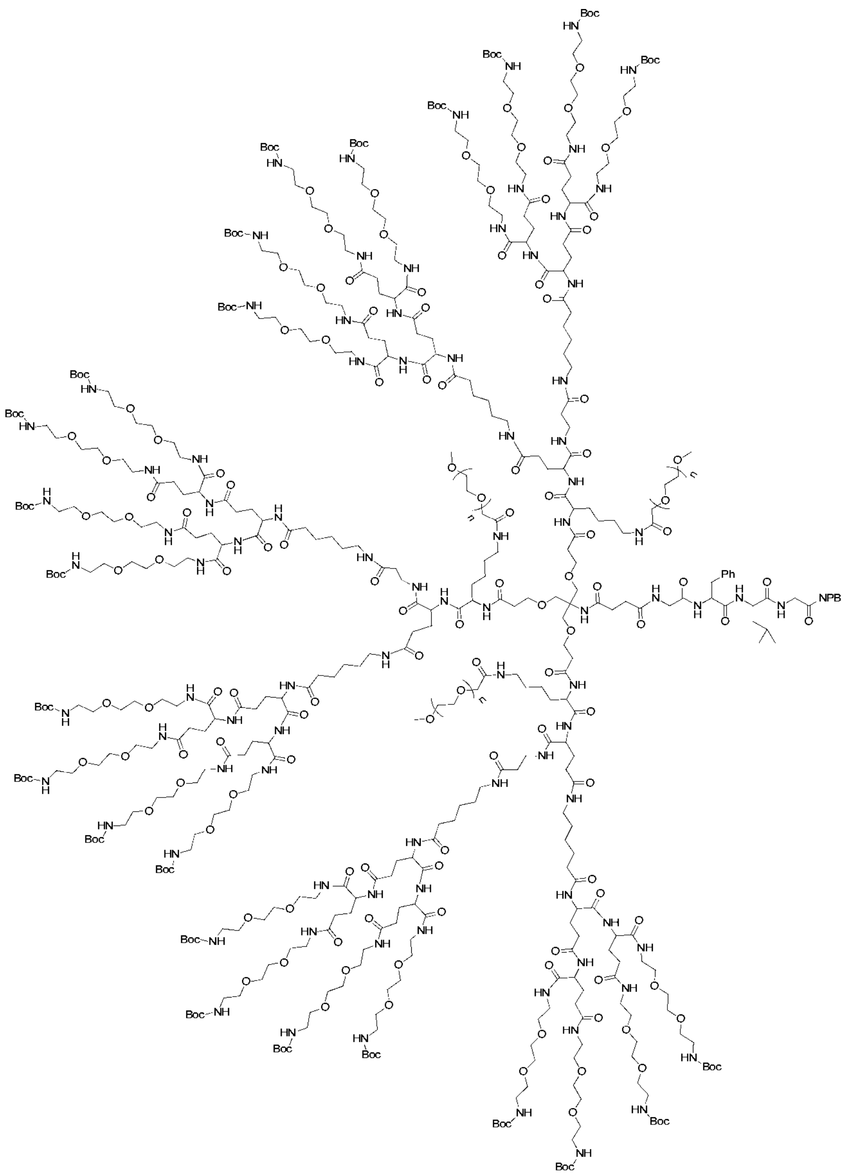

**76-128** (0.47 g, 0.01 mmol), HBTU (0.04 g, 0.10 mmol) and HOBT (0.01 g, 0.10 mmol) were weighed and added into a flask containing **76-93** (0.14 g, 0.09 mmol), and then an appropriate amount of DMF was added to dissolve the mixture. DIEA (0.05 mL, 0.10 mmol) was slowly added dropwise at -5°C. After the dropwise addition was completed, the reaction was carried out for half an hour, the flask was then taken out and the reaction was carried out under stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered with suction to obtain a solid product, which was collected and dried to provide the product.

2.24 Preparation of **76-204**

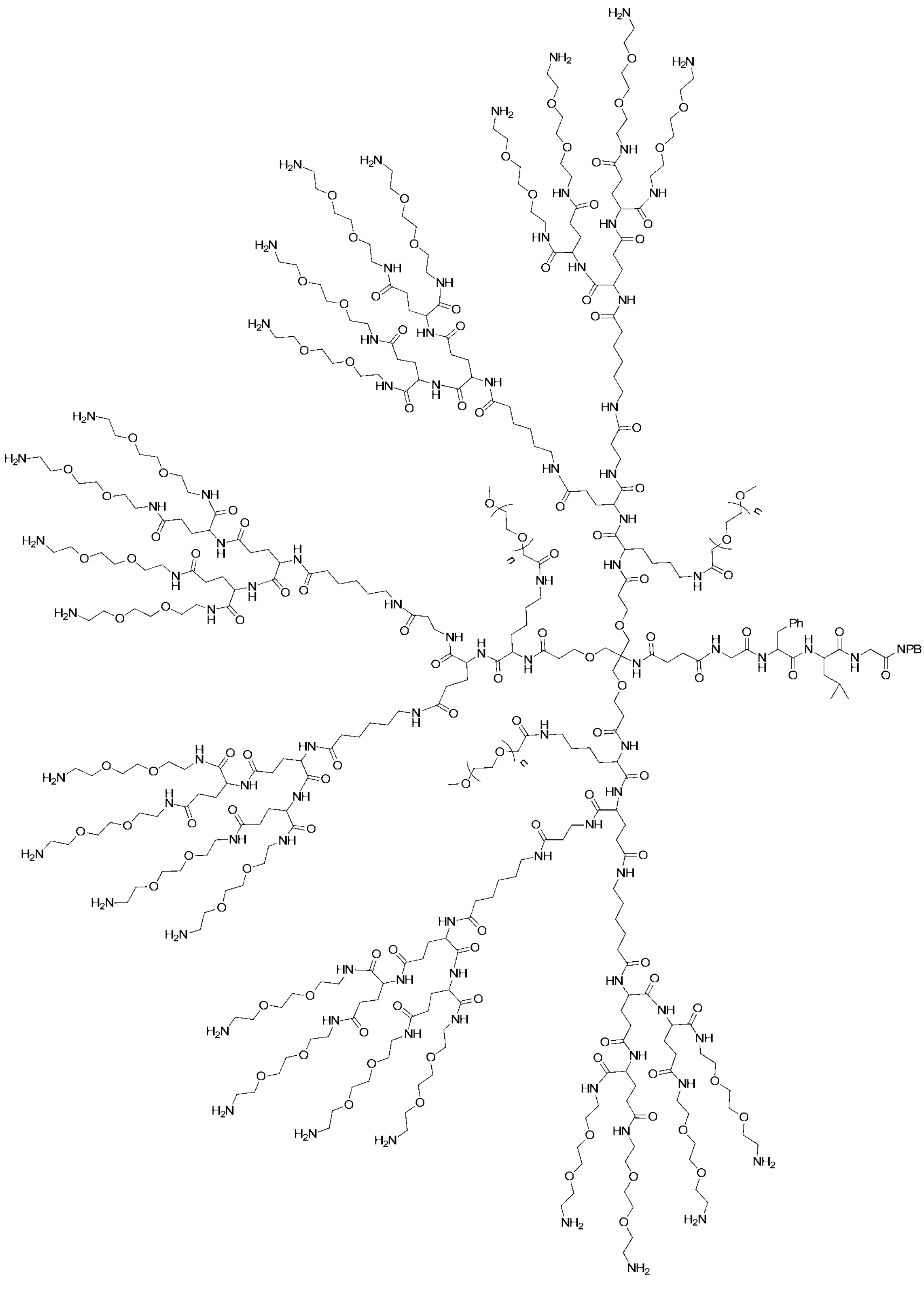

Dichloromethane was added into a flask containing **76-146** (0.0068 mmol). After dissolution with the assistance of ultrasonication, TFA (0.2 mL) was added, and the reaction was stirred at room temperature overnight. After the reaction was completed, the reaction mixture was evaporated to provide an oil using a rotary evaporator. MTBE (60 mL) was then added. A powdery solid precipitated from the reaction mixture and filtered. The filter cake was washed with MTBE (40 mL × 3). The product had poor solubility. The filter cake was collected and dried in a vacuum oven to provide the product.

2.25 Preparation of **76-229**

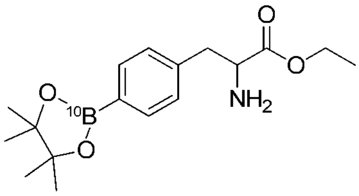

Ethyl 2-((tert-butoxycarbonyl)amino)-3-(4-(4,4,5,5-tetramethyl-1,3,2-dioxa-borolan-2-yl-2-$^{10}$B)phenyl)propanoate (2.0 g, 4.78 mmol, purchased from Lakeside) was added to a 250 mL flask, and 4.0 M hydrochloride in 1,4-dioxane (12 mL) was added to dissolve the mixture. The reaction flask was allowed to stay at room temperature and the reaction was carried out under stirring for 2 hours. After the reaction was completed, the reaction mixture was evaporated to dryness using a vacuum rotary evaporator. The resulting mixture was then dissolved in dichloromethane (10 mL) and evaporated again using a vacuum rotary evaporator. This step was repeated multiple times to provide a solid, which was then dried to provide the product.

2.26 Preparation of **76-230**

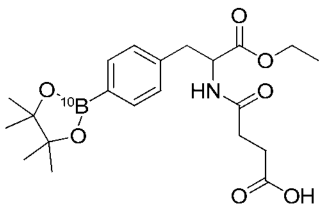

**76-229** (4.78 mmol) was added to a 500 mL flask and dissolved in dichloromethane/acetonitrile (1/1) mixture (20 mL) with the assistance of ultrasonication. DIEA (3.16 mL, 19.11 mmol) was then slowly added dropwise. After the addition was completed, the mixture was stirred for 5 minutes, and succinic anhydride (1.44 g, 14.34 mmol, purchased from Innochem) was then added and reacted under stirring for 3 hours. The reaction was monitored by TLC. After the reaction was completed, the dichloromethane and acetonitrile were evaporated to dryness by vacuum rotary evaporation. The resulting mixture was dissolved in a dichloromethane/methanol mixture, then silica gel powder was added, and the mixture was evaporated to dryness. The resultant product was loaded by dry method, and subjected to column chromatography using a 0-5% methanol/dichloromethane mixture as the eluent. The desired product was collected, concentrated, and dried in a vacuum oven to provide 1.28 g of the product. $^{1}$H-NMR (600 MHz, DMSO-$d_6$) δ 12.10 (s, 1H), 8.44-8.23 (m, 1H), 7.66-7.49 (m, 2H), 7.30-7.14 (m, 2H), 4.46-4.35 (m, 1H), 4.08-3.99 (m, 2H), 3.02-2.97 (m, 1H), 2.95-2.87 (m, 1H), 2.38-2.29 (m, 4H), 1.28 (s, 12H), 1.13-1.08 (m, 3H)

2.7 Preparation of **76-258**

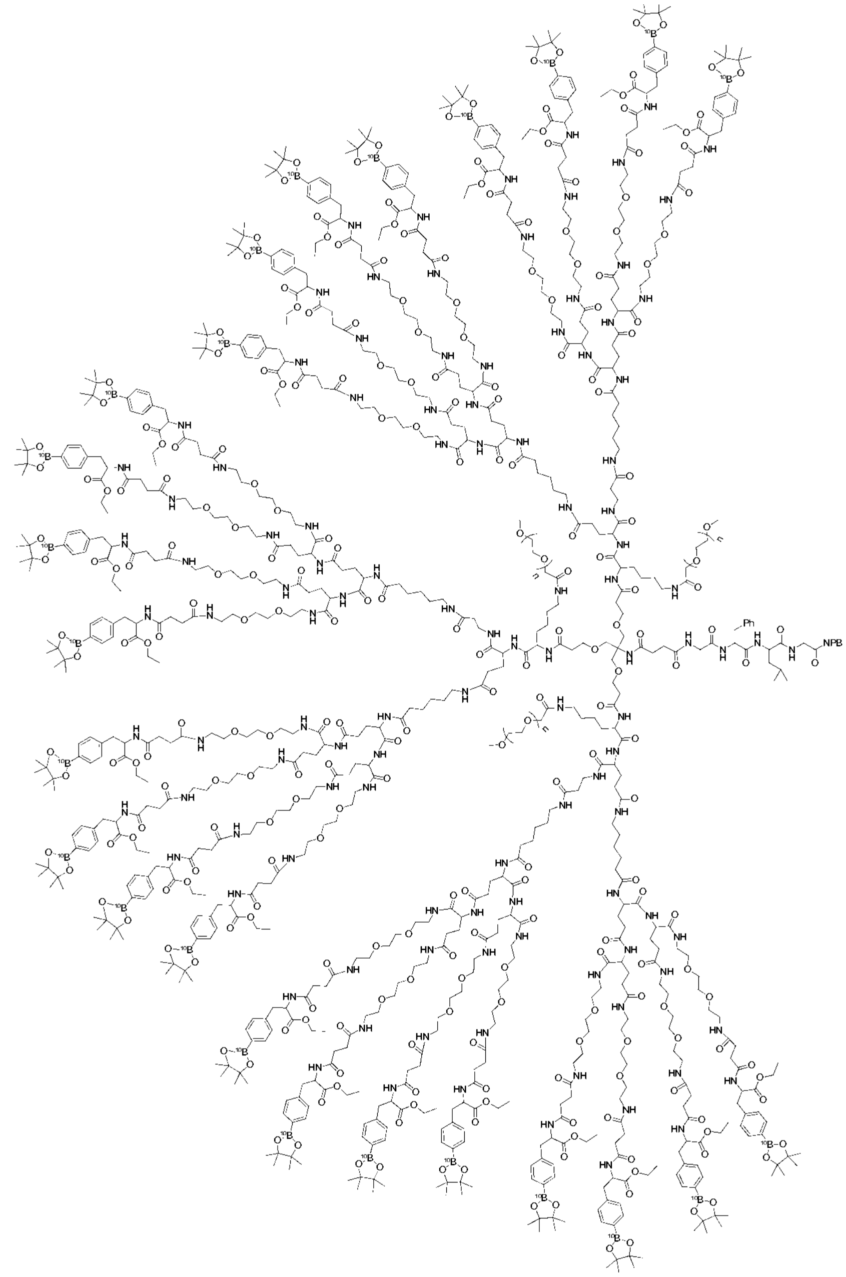

**76-204** (0.78 g, 0.0068 mmol), **76-230** (0.2 g, 0.2448 mmol), EDCI (0.04 g, 0.10 mmol) and HOBT (0.01 g, 0.10 mmol) were added to a 250 mL round-bottom flask, and dissolved in DMF (20 mL). The reaction flask was then allowed to stay at 0°C, and the mixture was stirred for approximately 20 minutes. DIEA (0.1 mL, 0.61 mmol) was then slowly added dropwise. After the dropwise addition was completed, the reaction flask was allowed to stay at room temperature, and the reaction was carried out under stirring. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, and the obtained solid product was dissolved in dichloromethane/methanol (1/1) mixture. The resultant product was loaded by dry method, and subjected to column chromatography using a 1% ammonia water/6-12% methanol/dichloromethane mixture as the eluent. The desired product was collected, concentrated, and dried in a vacuum oven to provide 0.50 g of the product. $^1$H-NMR (600 MHz, DMSO-$d_6$) δ10.10-9.63 (m, 24H), 8.97-8.74 (m, 4H), 8.61-7.47 (m, 150H), 7.34-6.92 (m, 42H), 6.72-6.49 (m, 4H), 6.20-5.90 (m, 52H), 4.57-4.30 (m, 24H), 4.27-4.17 (m, 15H), 4.05-3.98 (m, 40H), 3.72-3.38 (m, 2901H), 3.21-3.10 (m, 94H), 3.07-2.93 (m, 182H), 2.93-2.83 (m, 30H), 2.81-2.67 (m, 162H), 2.36-2.21 (m, 81H), 2.18-2.01 (m, 49H), 1.79-1.65 (m, 95H), 1.57-1.03 (m, 277H), 1.03-0.93 (m, 72H)

Example 3: Synthesis of Compound **88-206**

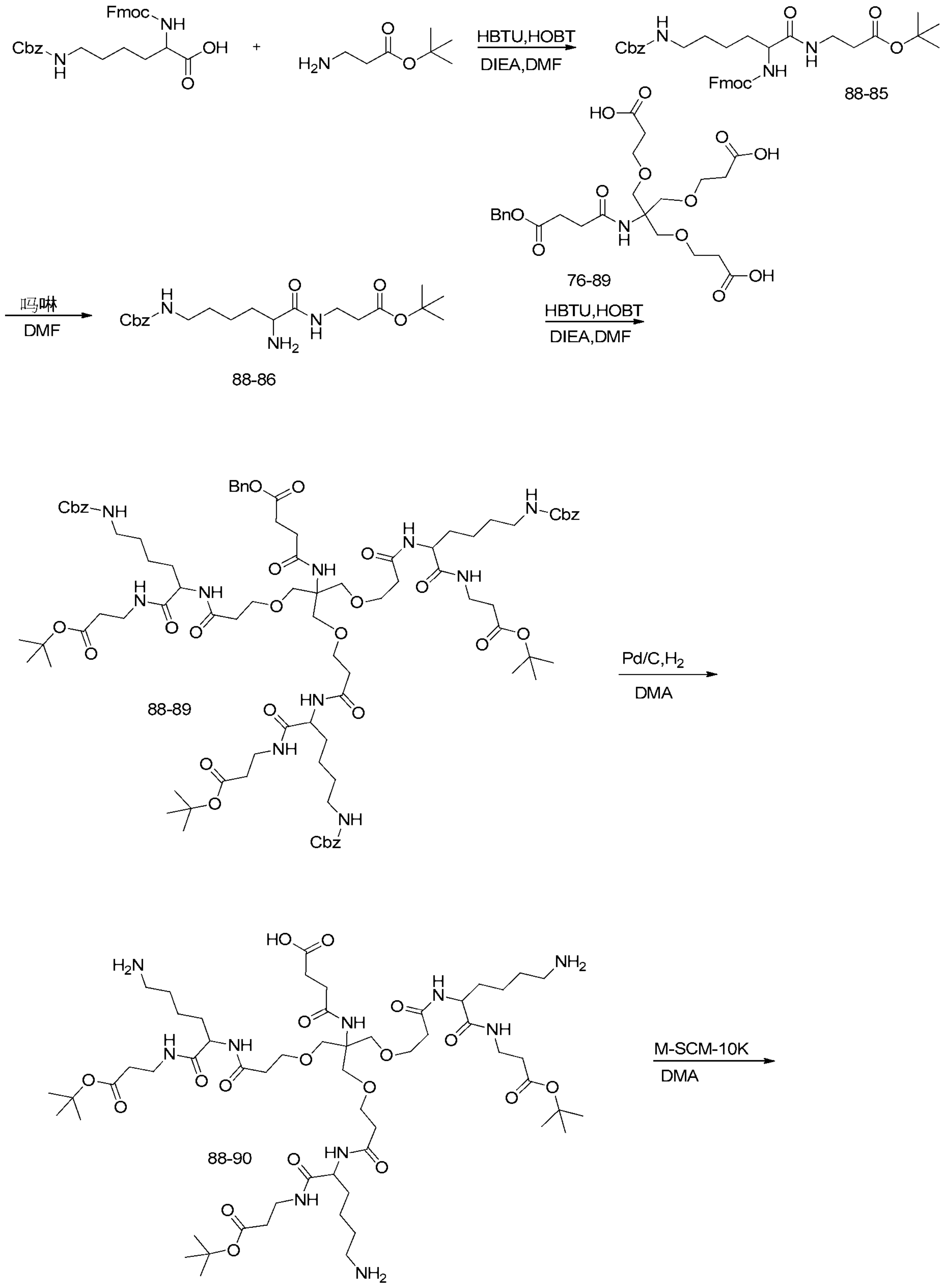

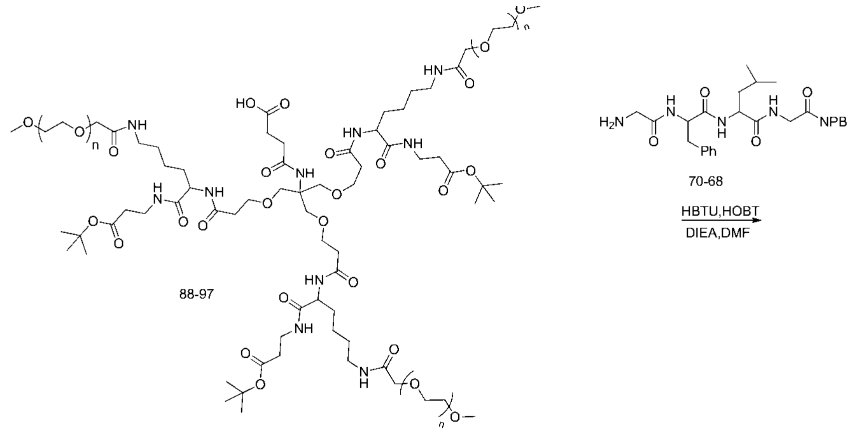


88-97

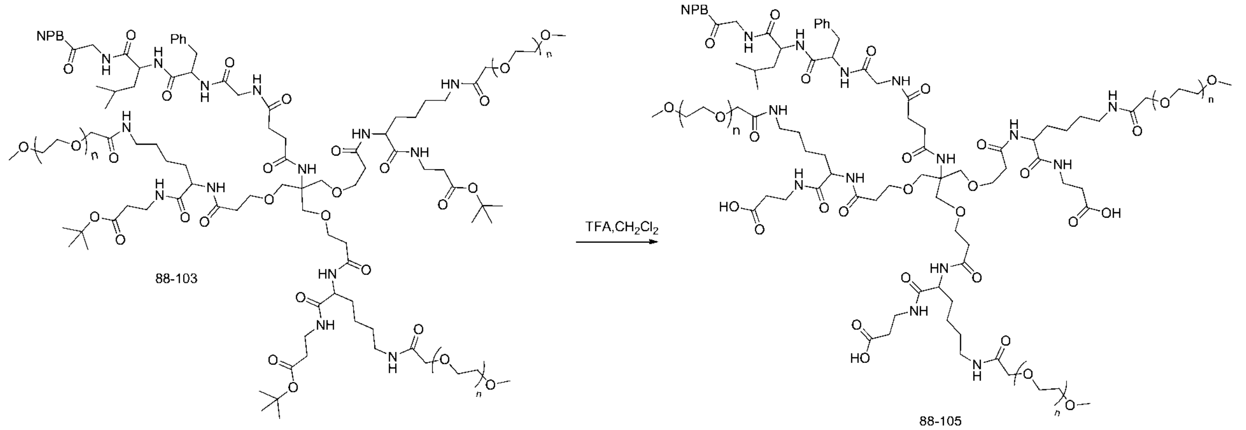


88-103

88-105

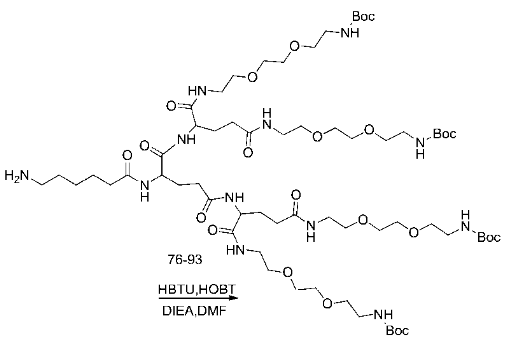


76-93

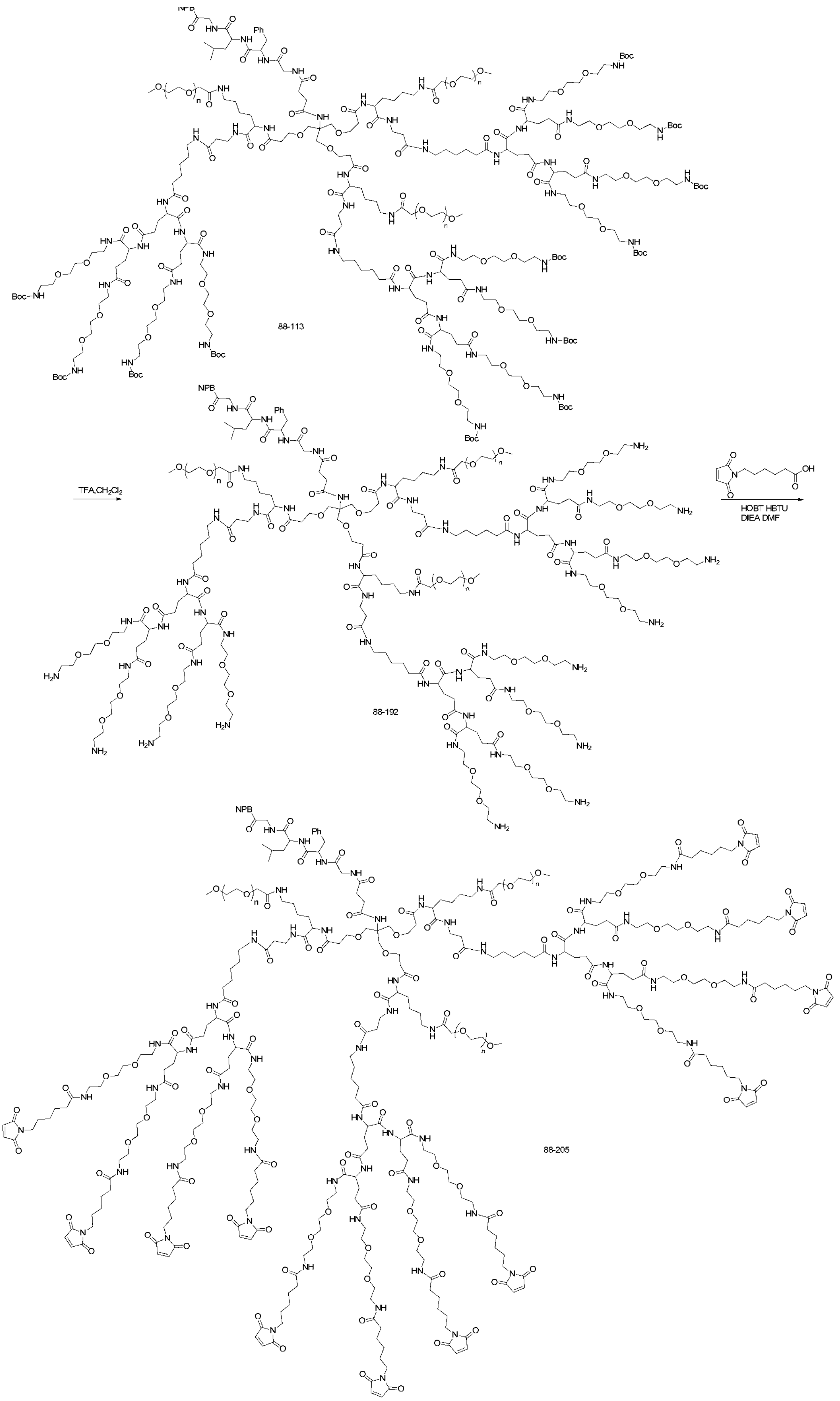


88-113

88-192

88-205

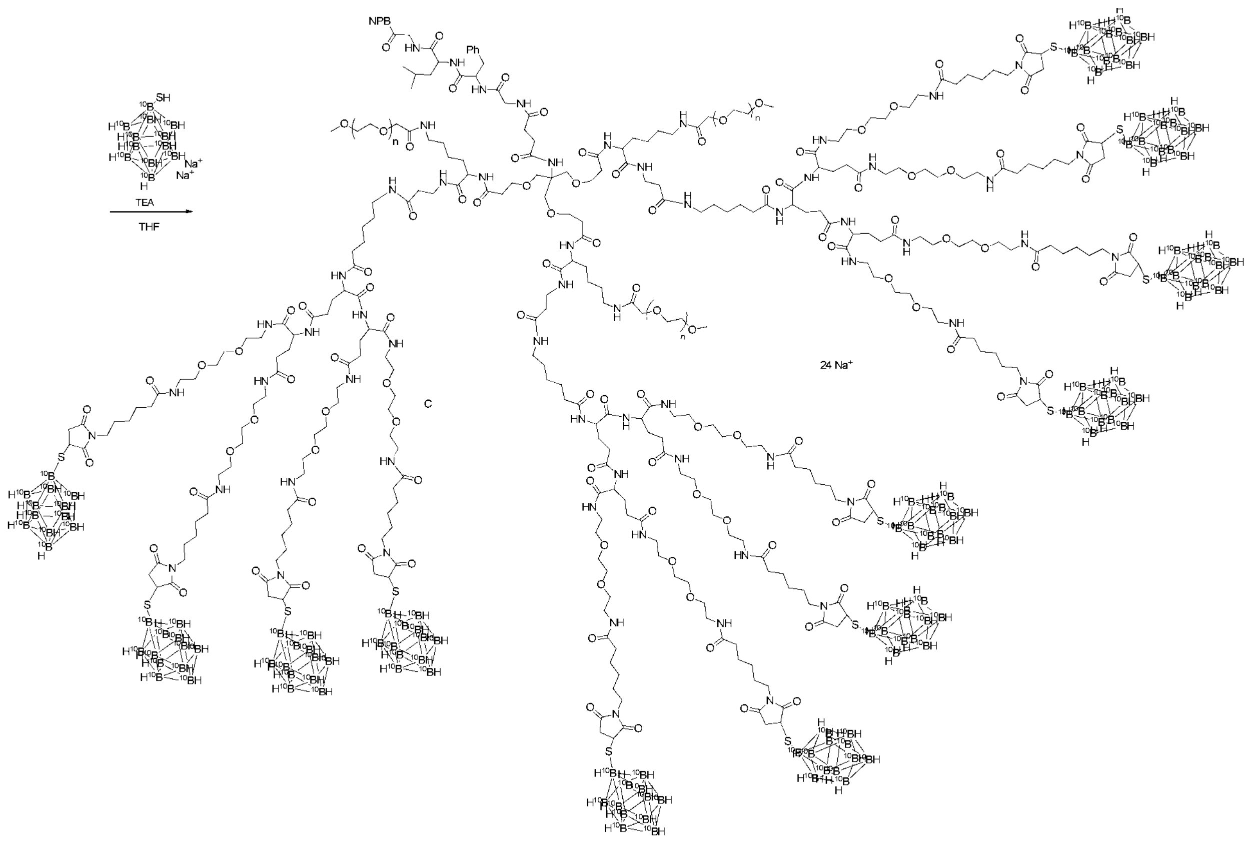


88-206

3.1 Preparation of Compound **88-85**

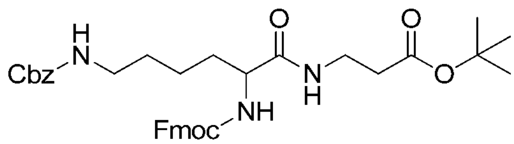

N'-Fmoc-N-benzyloxycarbonyl-L-lysine (4.0 g, 7.95 mmol), HBTU (3.32 g, 8.75 mmol) and HOBT (1.18 g, 8.75 mmol) were added into a flask containing tert-butyl β-alaninate (1.59 g, 8.75 mmol), an appropriate amount of DMF was added to dissolve the mixture, and the mixture was allowed to stir at -5°C. DIEA (2.29 mL, 17.51 mmol) was then slowly added dropwise. After the dropwise addition, the reaction was carried out for half an hour, and then the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide 5 g of crude product.

3.2 Preparation of **88-86**

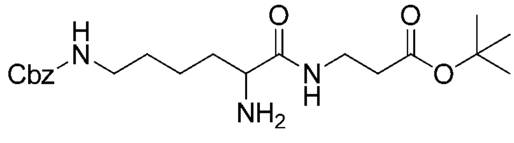

To a flask containing **88-85** (5 g, 7.95 mmol), DMF was added. After complete dissolution with the assistance of

ultrasonication, morpholine (17 mL, 119 mmol) was added, and the reaction was stirred at room temperature for 2 h. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to obtain a solid. The solid was then dissolved in methanol/dichloromethane (1/4) mixture, then silica gel powder (100 mL) was added, and the mixture was evaporated to dryness to provide a powdery solid. The resultant product was loaded by dry method, and subjected to column chromatography using a methanol/dichloromethane mixture as the eluent. The desired product was collected, concentrated, and dried in a vacuum oven to provide 3.3 g of the product.

3.3 Preparation of **88-89**

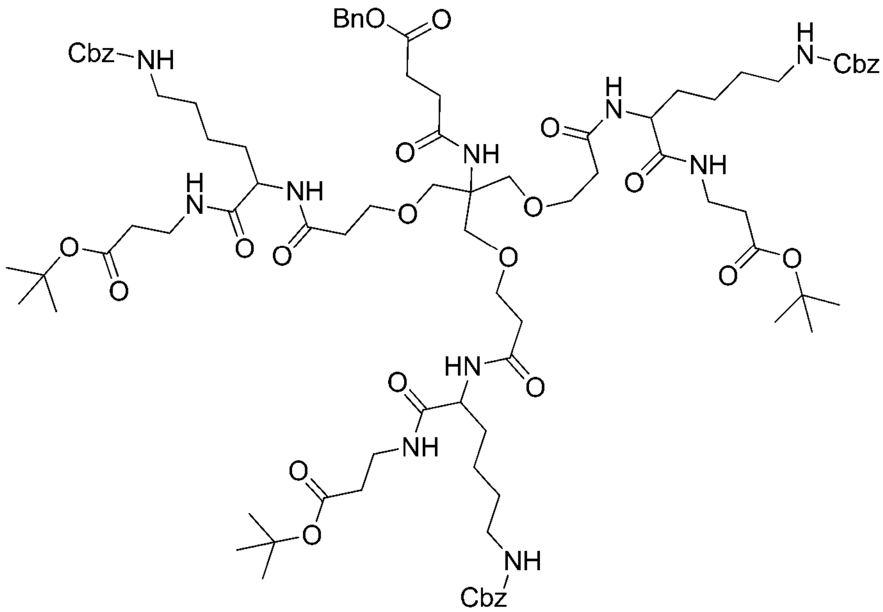

**76-89** (1.02 g, 1.93 mmol), HBTU (2.42 g, 6.38 mmol) and HOBT (0.86 g, 6.38 mmol) were weighed and added into a flask containing **88-86** (2.60 g, 6.38 mmol), an appropriate amount of DMF was added to dissolve the mixture, and allowed to stay at -5°C. DIEA (1.52 mL, 9.21 mmol) was then slowly added dropwise. After the dropwise addition was completed, the reaction was carried out for half an hour, the flask was then taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to obtain a solid. The solid was then dissolved in a methanol/dichloromethane (volume ratio of 1:4) mixed solvent, then silica gel powder (100 mL) was added, and the mixture was evaporated to dryness to provide a powdery solid. The resultant product was loaded by dry method, and subjected to column chromatography using a dichloromethane/-methanol mixed solvent as the eluent. The desired product was collected, concentrated, and dried in a vacuum oven to provide 1.5 g of the product. $^1$H-NMR (600 MHz, DMSO-$d_6$) δ 7.98-7.89 (m, 6H), 7.39-7.28 (m, 20H), 7.22-7.16 (m, 3H), 7.15-7.10 (m, 1H), 5.08-5.05 (m, 2H), 5.04-4.98 (m, 6H), 4.20-4.14 (m, 3H), 3.56-3.49 (m, 12H), 3.30-3.25 (m, 4H), 3.20-3.13 (m, 3H), 2.97-2.92 (m, 6H), 2.44-2.40 (m, 2H), 2.37-2.31 (m, 12H), 1.60-1.53 (m, 3H), 1.50-1.40 (m, 5H), 1.39-1.37 (m, 27H), 1.27-1.14 (m, 8H), 1.12-1.03 (m, 2H)

3.4 Preparation of **88-90**

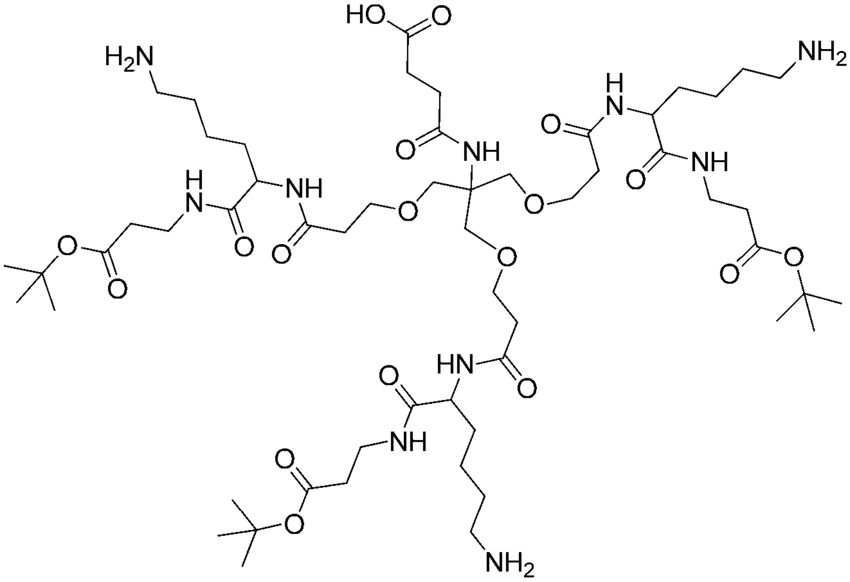

**88-89** (0.13 g, 0.077 mmol) and 10% Pd/C catalyst (0.08 g) were placed in a hydrogenation reactor, and then dissolved in DMF (30 mL). The hydrogenation reactor was sealed, evacuated with a water pump, and then filled with hydrogen gas at approximately 2 MPa. This process was repeated three times. Finally, the pressure of the hydrogenation reactor was adjusted to 1.8 MPa. The reaction was carried out at room temperature overnight. After the reaction was completed, the reaction mixture was filtered through Celite, and the filter cake was washed with DMF (20 mL × 3) to provide a DMF solution of the product, which was used as the raw material for the next step. FT-MS ESI m/z [M+H$^+$] 1203.74316, [M+Na$^+$] 1225.72241

3.5 Preparation of **88-97**

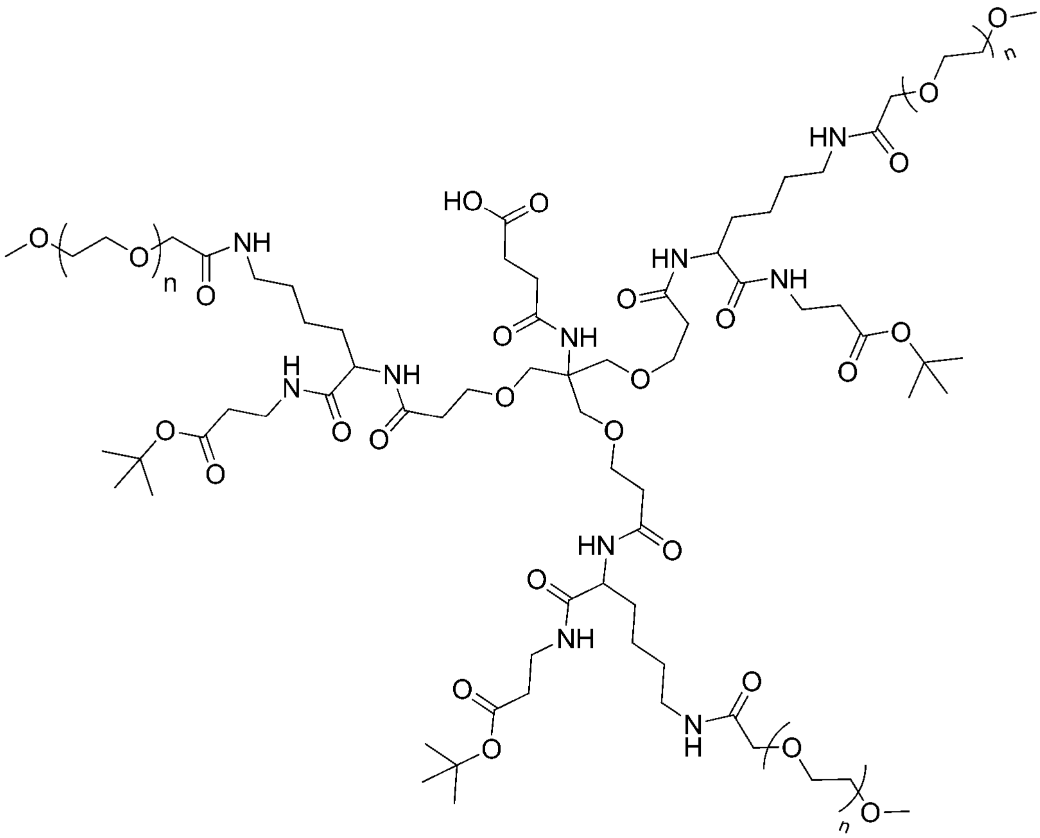

**88-90** (0.092 g, 0.077 mmol) was placed in a 250 mL flask, and dissolved in DMF (20 mL). The resultant solution was then slowly added dropwise to a DMF solution containing DIEA (0.2 mL, 1.23 mmol) and M-SCM-10K (2.02 g, 0.24 mmol, purchased from Jenkem). The reaction was carried out under stirring at low speed at room temperature in the dark for one week. After the reaction was completed, n-hexane (25 mL) and methyl tert-butyl ether (200 mL) were added for precipitation. The supernatant was then discarded, and n-hexane and methyl tert-butyl ether were added again for precipitation. This process was repeated three times. The mixture was filtered to provide a solid product, which was collected and dried in a vacuum oven to provide the product.

3.6 Preparation of **88-103**

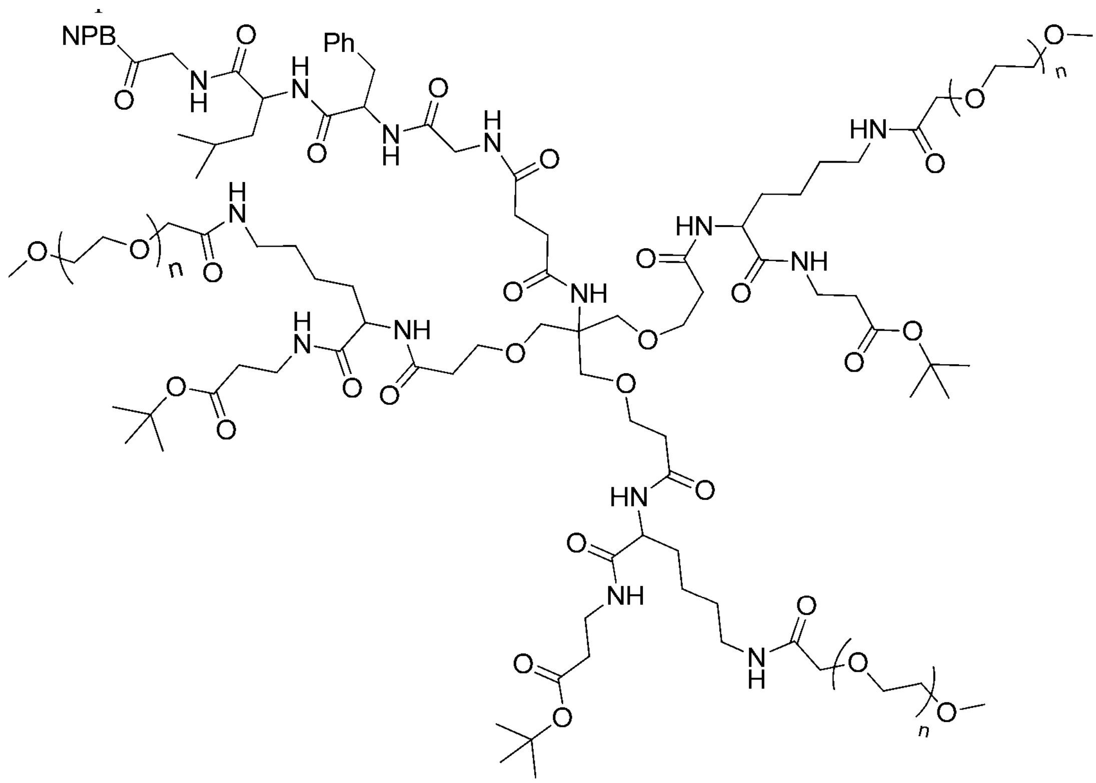

**88-97** (2.53 g, 0.077 mmol), HBTU (0.032 g, 0.0847 mmol) and HOBT (0.012 g, 0.0847 mmol) were weighed and added into a flask containing **70-68** (0.059 g, 0.0847 mmol), then an appropriate amount of DMF was added to dissolve the mixture, and the reaction was allowed to stay at -5°C. DIEA (0.10 mL, 0.3390 mmol) was then slowly added dropwise. After the dropwise addition was completed, the reaction was carried out for half an hour, and then the flask was taken out and the reaction was carried out under stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected and dried to provide 1.1 g of the product with a yield of 44.00%.

3.7 Preparation of **88-105**

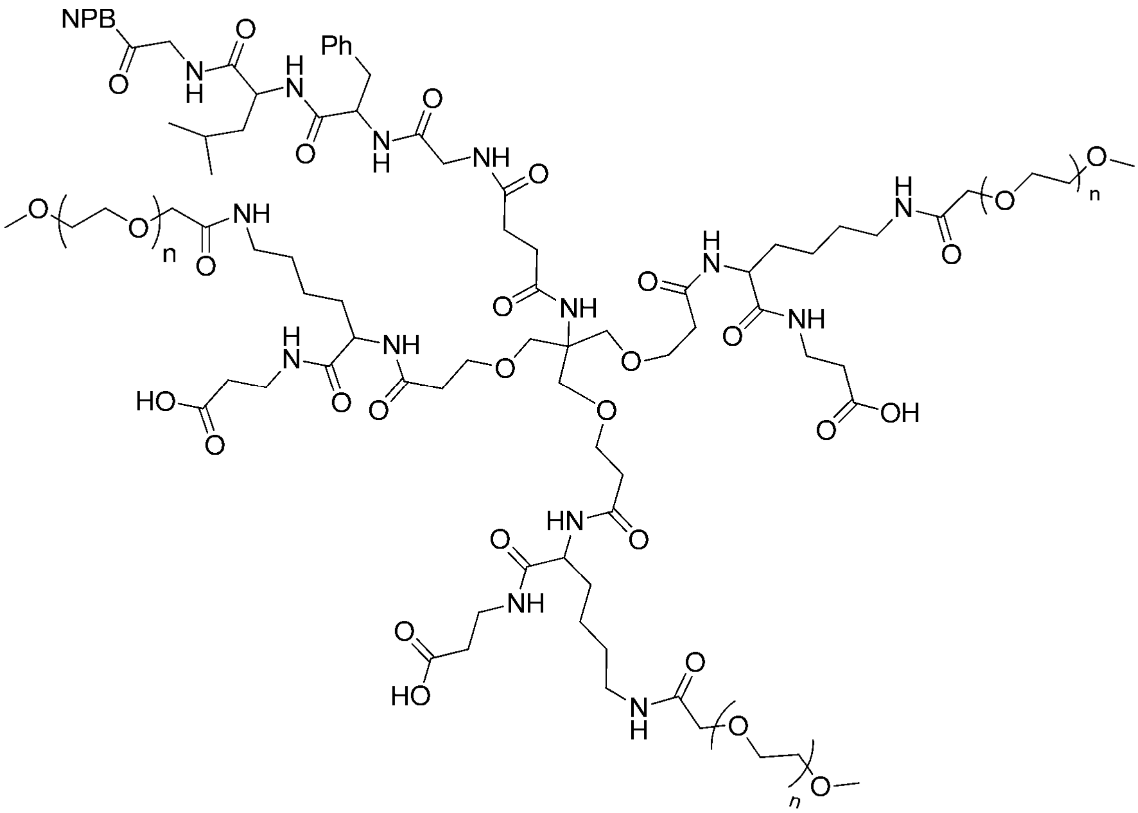

Dichloromethane was added into a flask containing **88-103** (1.1 g, 0.0.0328 mmol). After complete dissolution with the assistance of ultrasonication, TFA (0.2 mL) was added, and the reaction was carried out under stirring at room temperature overnight. After the reaction was completed, the reaction mixture was evaporated to provide an oil using a rotary evaporator. MTBE (60 mL) was then added, and a powdery solid precipitated from the reaction mixture. The filter cake was then washed with MTBE (40 mL × 3). The product had poor solubility. The filter cake was collected and dried in a vacuum oven to provide 0.9 g of the product.

3.8 Preparation of **88-113**

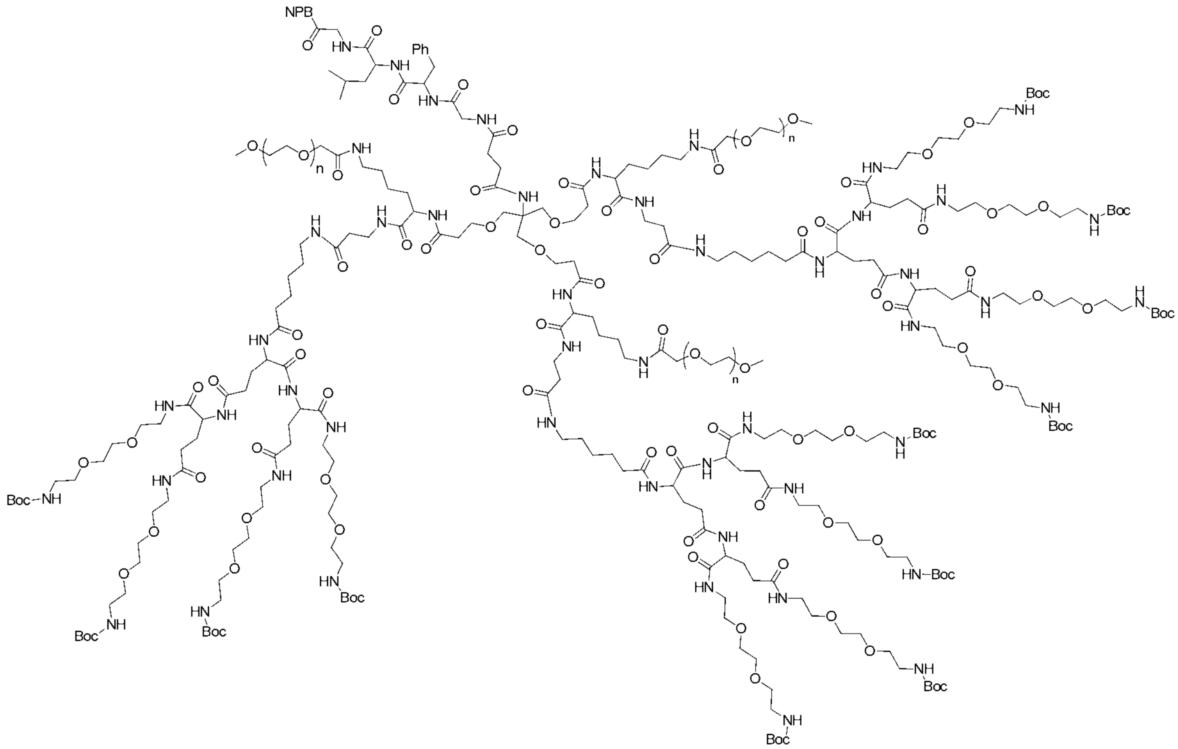

**88-105** (0.9 g, 0.027 mmol), HBTU (0.06 g, 0.16 mmol) and HOBT (0.02 g, 0.16 mmol) were weighed and added into a flask containing **76-93** (0.23 g, 0.16 mmol), an appropriate amount of DMF was added to dissolve the mixture, and the mixture was allowed to stay at -5°C. DIEA (0.2 mL, 0.8 mmol) was slowly added dropwise. After the dropwise addition was completed, the reaction was carried for half an hour, and then the flask was taken out and the reaction was carried out under stirring at room temperature overnight. After the reaction was completed, n-hexane (25 mL) and methyl tert-butyl ether (200 mL) were added for precipitation. The supernatant was then discarded, and n-hexane and methyl tert-butyl ether were added for precipitation. This process was repeated three times. The mixture was filtered by suction to provide a solid product, which was collected and dried to provide the product.

3.9 Preparation of **88-192**

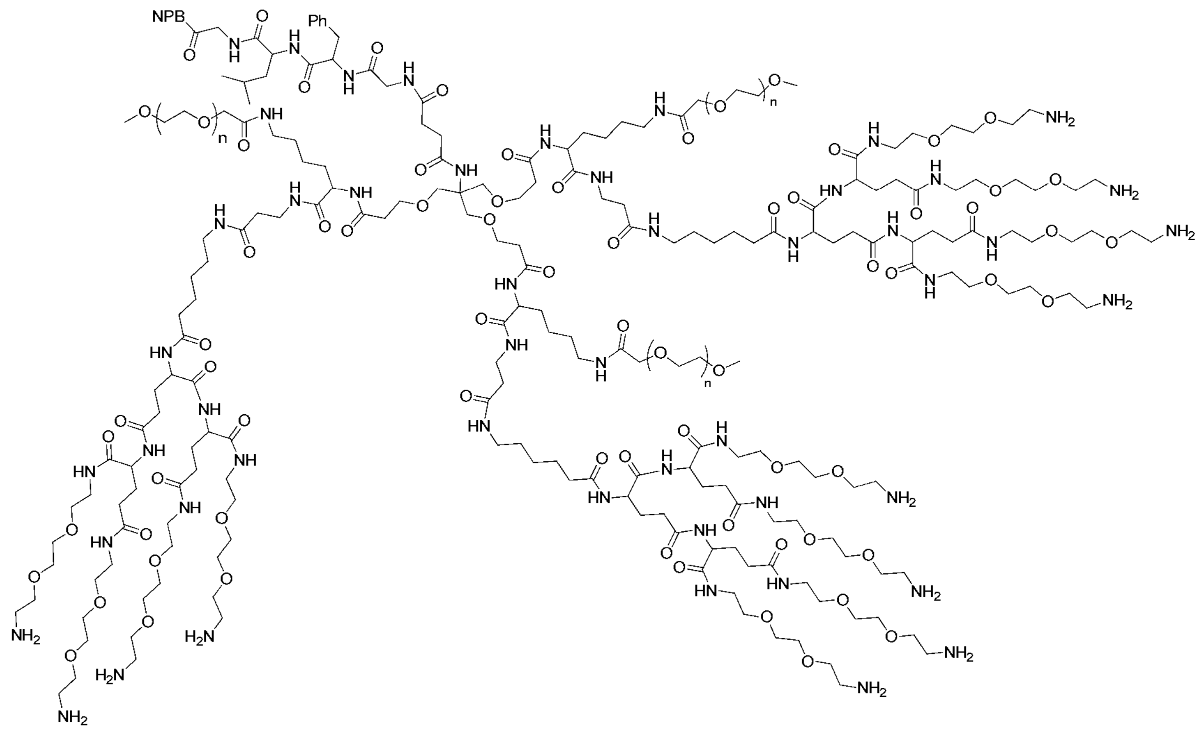

To a flask containing **88-113** (0.8 g, 0.023 mmol) was added dichloromethane. After complete dissolution with ultrasonication, TFA (0.2 mL) was added and reacted under stirring at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide 0.78 g of the product.

3.10 Preparation of **88-205**

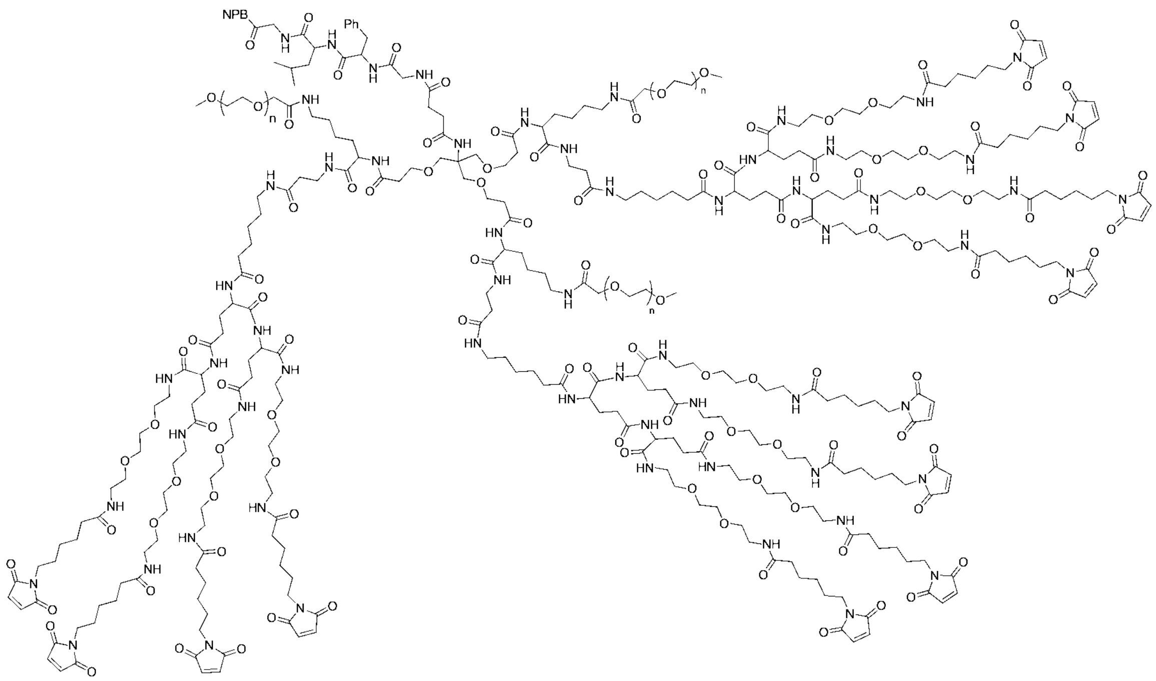

**88-203** (0.78 g, 0.02 mmol), HBTU (0.22 g, 0.6013 mmol) and HOBT (0.08 g, 0.0613 mmol) were weighed and added into a flask containing 6-maleimidocaproic acid (0.78 g, 0.4410 mmol), an appropriate amount of DMF was added to dissolve the mixture, and the mixture was allowed to stay at -5°C. DIEA (0.30 mL, 1.8040 mmol) was slowly added dropwise. After the dropwise addition, the reaction was carried out for half an hour, the flask was taken out and the reaction was carried out under stirring at room temperature overnight. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered to provide a solid product, which was collected and dried to provide the product.

3.11 Preparation of **88-206**

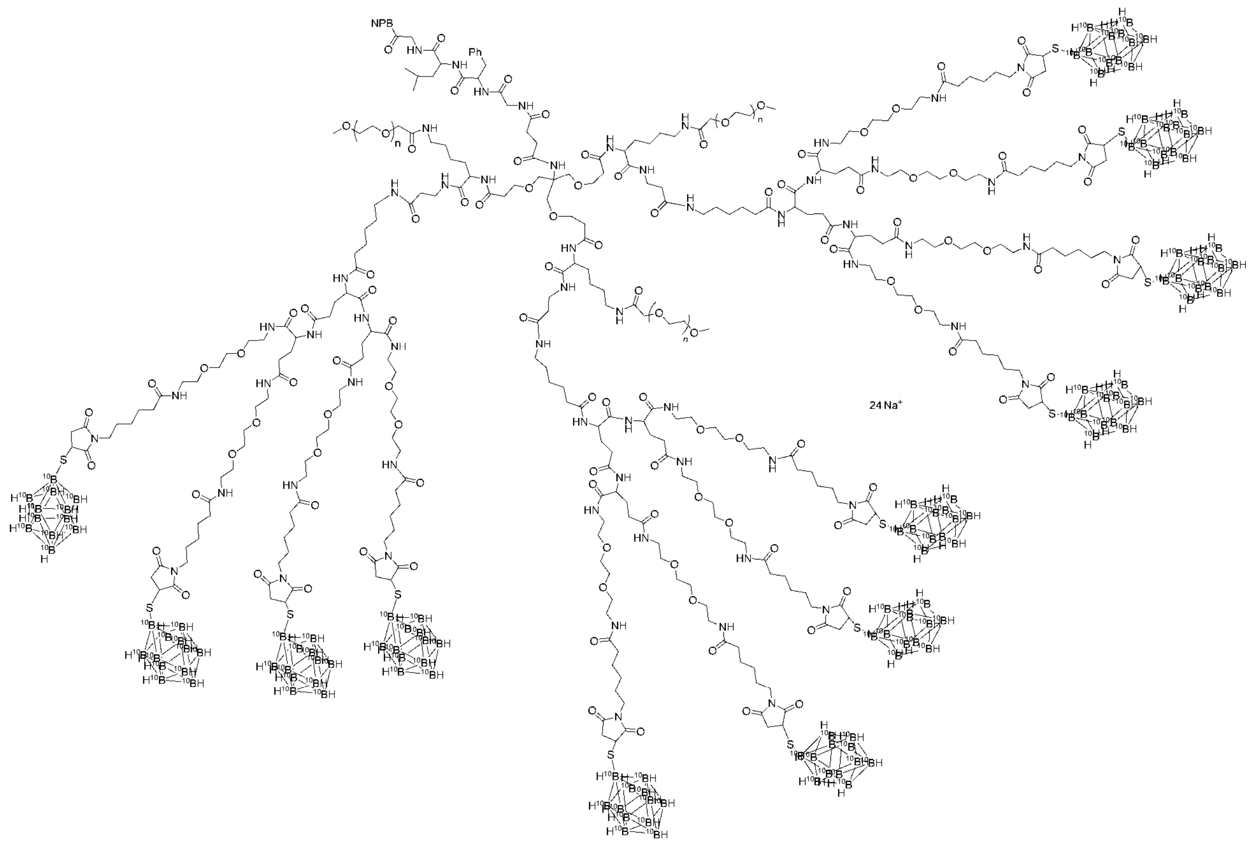

**88-205** (0.68 g, 0.0176 mmol) and sodium mercaptododecaborate ($^{10}B$, BSH) (0.0886 g, 0.4216 mmol, purchased from CATCHEM, Czech Republic) were placed in a 250 mL flask, dissolved in 10 mL of DMF, and the reaction was stirred overnight. After the reaction was completed, methyl tert-butyl ether and n-hexane were added. The obtained mixture was treated with ultrasonication, and allowed to stand for 20 minutes to precipitate a solid. The supernatant was discarded, and methyl tert-butyl ether and n-hexane were added again, treated with ultrasonication, and allowed to stand for 20 minutes. The supernatant was discarded, and the residue was dried to provide 0.6 g of the product with a yield of 83.33%. $^{1}H$-NMR (600 MHz, DMSO-$d_6$) $\delta$ 8.26-7.63 (m, 65H), 4.24-4.15 (m, 12H), 4.13-4.$\delta$ 10 (m, 18H), 3.90-3.83 (m, 33H), 3.54-3.50 (m, 3256H), 3.20-3.16 (m, 73H), 2.92-2.84 (m, 64H), 2.26-2.19 (m, 32H), 2.15-2.07 (m, 60H), 1.49-1.43 (m, 72H), 1.35-1.25 (m, 162H), 1.03-0.94 (m, 176H)

Example 4: Synthesis of Compound **78-131**

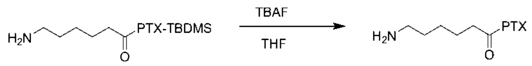


59-69 78-129

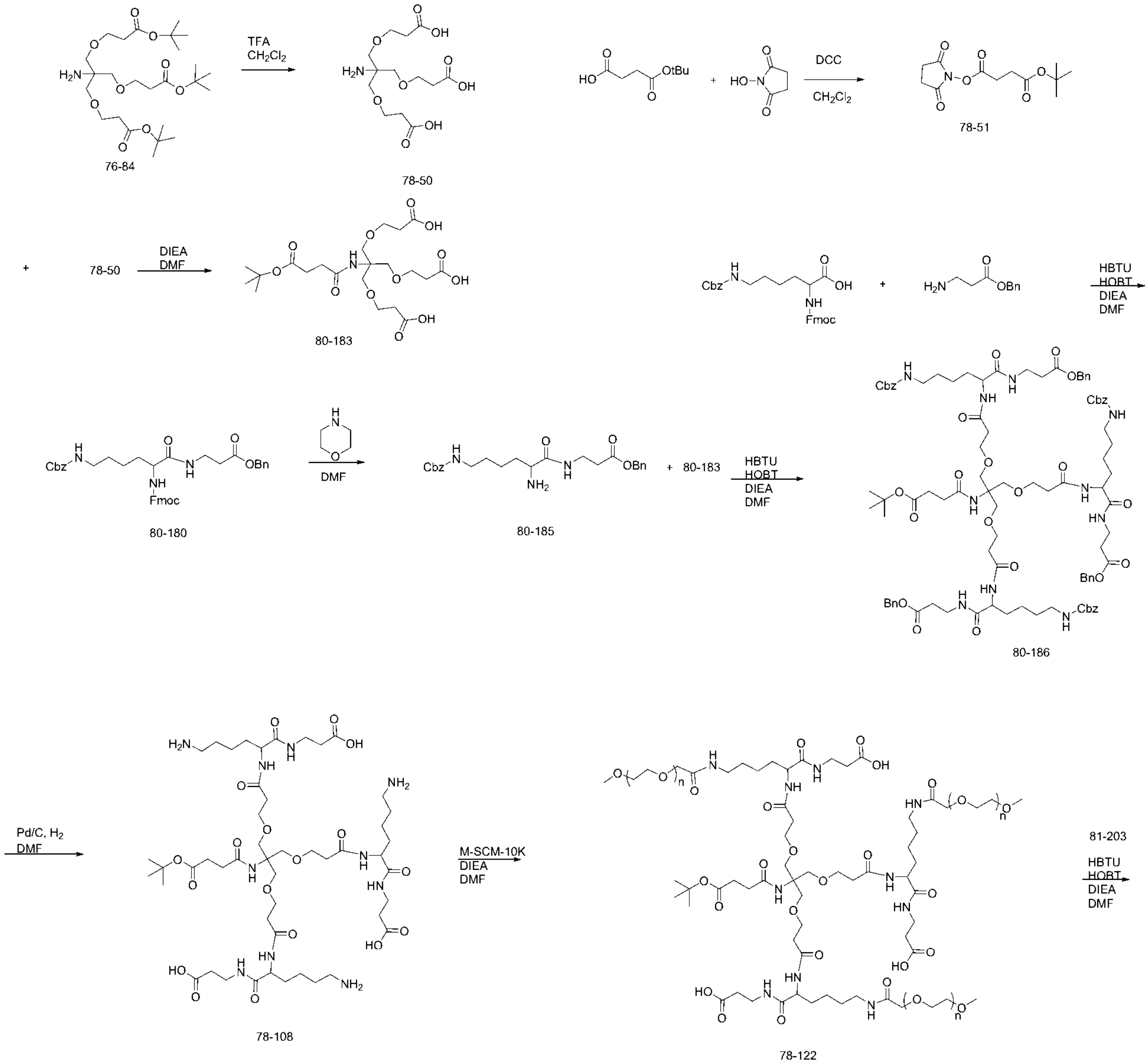


76-84 78-50 78-51 78-50 80-183 80-180 80-185 80-183 80-186 78-108 78-122

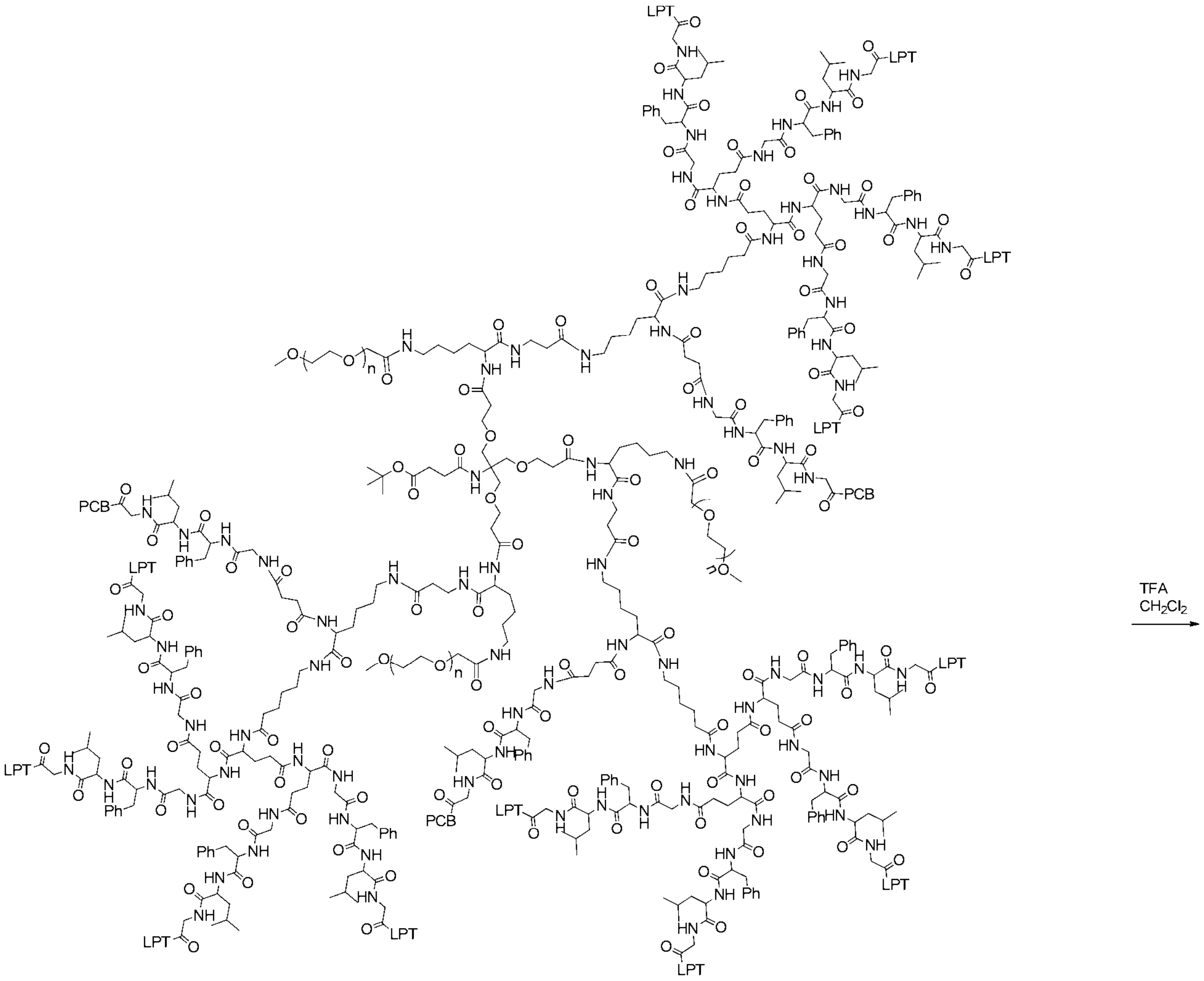


78-125

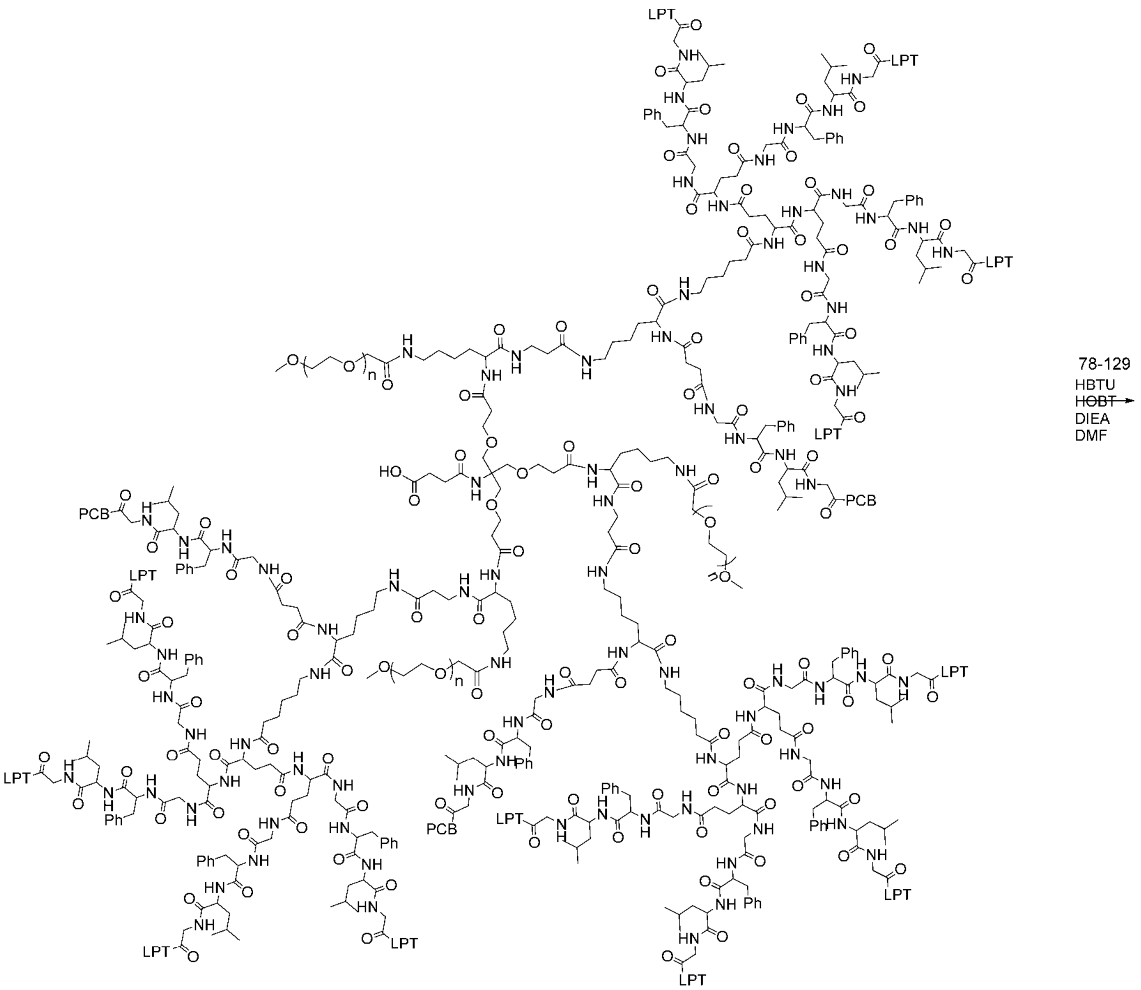


78-128

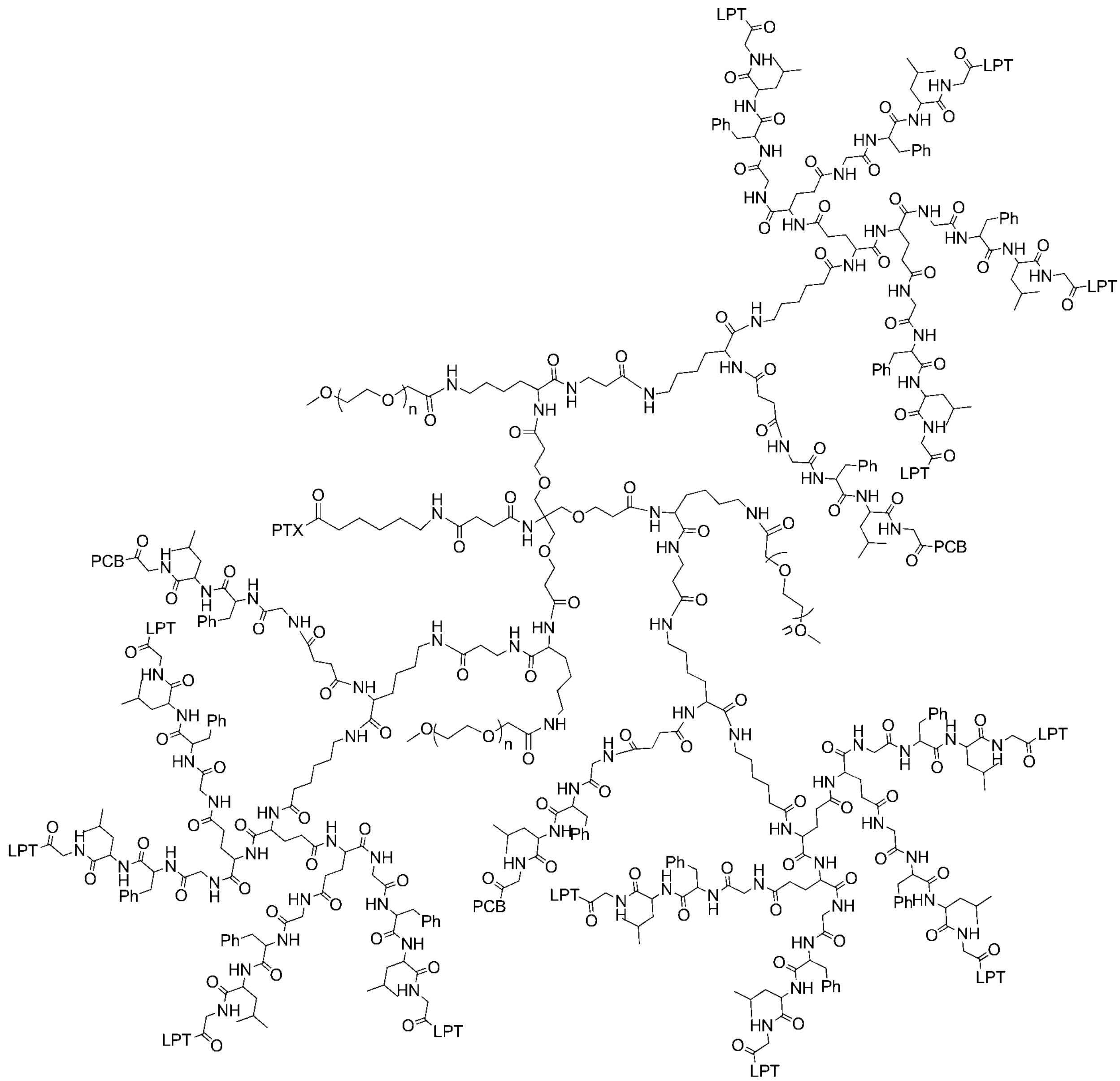

78-131

4.1 Preparation of **78-129**

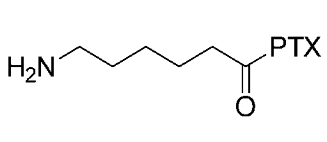

TBAF (2.9 g, 9.248 mmol) was added into a flask containing **59-69** (0.5 g, 0.4624 mmol), and THF (15 mL) was added. After complete dissolution with the assistance of ultrasonication, the reaction was carried out under stirring at room temperature overnight. After the reaction was completed, THF was removed by rotary evaporation under reduced pressure. Ethyl acetate and purified water were added for extraction. The organic phase was collected, and the aqueous phase was further extracted twice with ethyl acetate. The organic phase was collected, dehydrated over anhydrous sodium sulfate, and evaporated to dryness to provide 0.3 g of the product.

4.2 Preparation of **78-50**

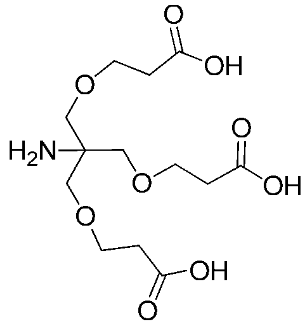

Dichloromethane was added into a flask containing **76-84** (1 g, 1.98 mmol). After complete dissolution with the assistance of ultrasonication, TFA (2.2 mL) was added, and the reaction was stirred at room temperature overnight. After the reaction was completed, the reaction mixture was evaporated to provide an oil using a rotary evaporator. Methyl tert-butyl ether (60 mL) and n-hexane (20 mL) were then added. The product remained as an oil at the bottom of the flask. The supernatant was discarded, and methyl tert-butyl ether (60 mL) and n-hexane (20 mL) were added again. This process was repeated four times. The mixture was then dried in a vacuum oven to provide 0.67 g of product **78-50.** ESI [M+H$^+$] 338.14, [M+Na$^+$] 360.13

4.3 Preparation of **78-51**

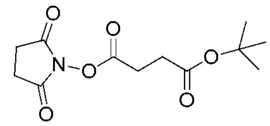

Mono-tert-butyl succinate (3 g, 17.2 mmol) and N-hydroxysuccinimide (2.6 g, 22.4 mmol) were weighed and placed in a 500 mL round-bottom flask. Dichloromethane (20 mL) was added for dissolution with the assistance of ultrasonication, and then DCC (16.71 g, 81.015 mmol) was added and the reaction was stirred overnight. After the reaction was completed, the filtrate was collected and concentrated, then n-hexane/petroleum ether (4/1) mixture was added for precipitation. The mixture was filtered to provide a product, which was then washed three times with n-hexane/petroleum ether mixture. After the product was dried, it was dissolved in the mixed solvent, then silica gel powder (40 mL) was added, and the mixture was evaporated to dryness. The resultant product was loaded by dry method, and subjected to column chromatography using 20-40% ethyl acetate/petroleum ether as the eluent to provide 3.5 g of product **78-51** with a yield of 75%. $^1$H-NMR (600 MHz, DMSO-$d_6$) $\delta$ 2.88-2.84 (m, 2H), 2.80-2.77 (s, 4H), 2.58-2.53 (m, 2H), 1.41-1.37 (m, 9H); ESI [M+Na$^+$] 294.09

4.4 Preparation of **80-183**

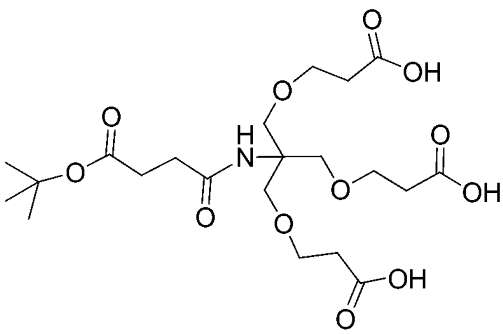

**78-50** (0.67 g, 1.98 mmol) and **78-51** (0.65 g, 2.41 mmol) were weighed into a 500 mL flask, and an appropriate amount of DMF was added to dissolve the mixture. DIEA (3.7 mL, 21.9 mmol) was slowly added dropwise. After the dropwise addition was completed, the reaction was carried out under stirring at room temperature overnight. After the

reaction was completed, methyl tert-butyl ether (60 mL) and n-hexane (20 mL) were added for precipitation. The product, an oily substance, was adhered to the bottom of the flask. After the supernatant was discarded, methyl tert-butyl ether (60 mL) and n-hexane (20 mL) were added again for precipitation. This process was repeated four times. The mixture was dried in a vacuum oven to provide 0.84 g of product **80-183.**

4.5 Preparation of **80-180**

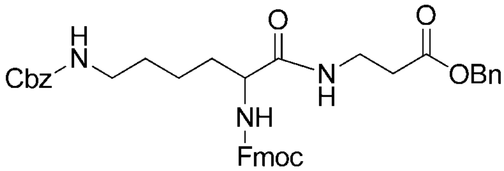

N'-Fmoc-N-benzyloxycarbonyl-L-lysine (3.5 g, 7 mmol), β-alanine benzyl ester (2.46 g, 7 mmol), HBTU (2.9 g, 7.7 mmol) and HOBT (1.0 g, 7.7 mmol) were weighed and added to a 500 mL flask, then an appropriate amount of DMF was added to dissolve the mixture, and the mixture was allowed to stay at -5°C. DIEA (2.3 mL, 14 mmol) was slowly added dropwise. After the dropwise addition was completed, the reaction was carried out for half an hour, the flask was then taken out, and the reaction was carried out under stirring at room temperature overnight. After the reaction was completed, saturated sodium chloride solution and ethyl acetate (200 mL) were added for extraction. The organic phase was collected, concentrated, evaporated to dryness, and dried to provide 4.6 g of product **80-180.**

4.6 Preparation of **80-185**

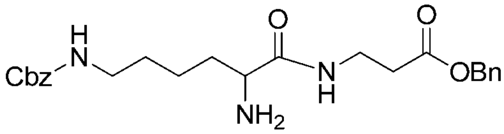

The reactant **80-180** (4.6 g, 7 mmol) was taken and dissolved in DMF (30 mL), then morpholine (9 mL, 105 mmol) was added, and the mixture was stirred at room temperature overnight. After the reaction was completed, saturated sodium bicarbonate solution and ethyl acetate (200 mL) were added for extraction. The organic phase was collected and concentrated, then 200-300 mesh silica gel powder was added, and the mixture was evaporated to dryness. The resultant product was loaded by dry method, and subjected to column chromatography using 1.5% methanol/dichloromethane as the eluent. The desired product was collected, concentrated, and evaporated to dryness to provide 3.09 g of product **80-185.**

4.7 Preparation of **80-186**

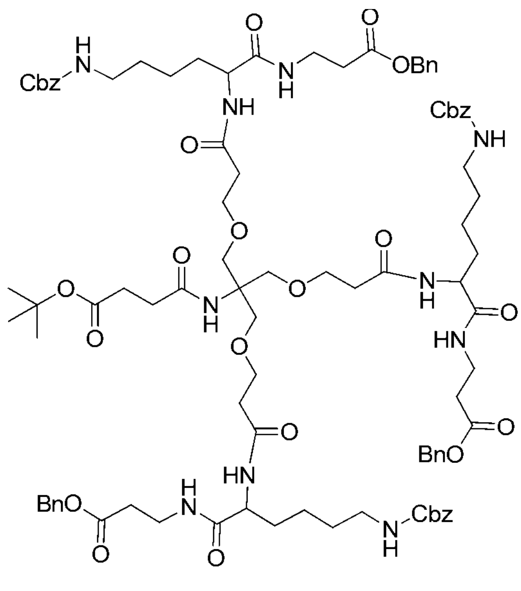

**80-183** (0.84 g, 1.7 mmol), **80-185** (2.5 g, 5.6 mmol), HBTU (2.12 g, 5.6 mmol) and HOBT (0.76 g, 5.6 mmol) were weighed and added to a 500 mL flask, and an appropriate amount of DMF was added to dissolve the mixture. DIEA (1.7 mL, 10.2 mmol) was slowly added dropwise, and then the reaction was stirred at room temperature overnight. After the reaction was completed, methyl tert-butyl ether (60 mL) and n-hexane (20 mL) were added. A powdery solid precipitated from the reaction mixture and was filtered. The filter cake was washed with methyl tert-butyl ether (40 mL × 3), and dissolved in the mixed solvent, then silica gel powder was added, and the mixture was evaporated to dryness. The resultant product was loaded by dry method, and subjected to column chromatography using 3% methanol/dichloromethane as the eluent. The desired product was collected, concentrated and evaporated to dryness to provide 0.8 g of product **80-186.** $^1$H-NMR (600 MHz, DMSO-$d_6$) δ 8.08-7.85 (m, 6H), 7.46-7.24 (m, 30H), 7.24-7.13 (m, 3H), 5.08 (s, 6H), 5.01 (s, 6H), 4.17 (s, 3H), 3.76-3.37 (m, 18H), 3.22 (s, 6H), 3.02-2.70 (m, 8H), 2.60 (m, 2H), 2.39-2.29 (m, 6H), 1.50 (s, 6H), 1.35 (s, 9H), 1.29-1.25 (m, 6H), 1.22 (s, 6H); ESI [M+Na$^+$] 1786.85

4.8 Preparation of **78-108**

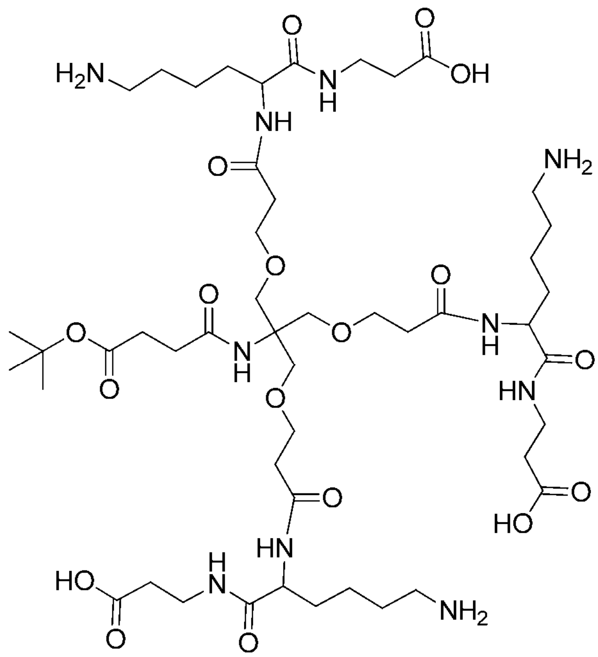

**80-186** (1.0 g, 0.567 mmol) and 10% Pd/C catalyst (0.3 g) were placed in a hydrogenation reactor, and then DMF

(30 mL) was added to dissolve the mixture. The hydrogenation reactor was sealed and evacuated with a water pump. Hydrogen gas was then introduced at approximately 2 MPa. This process was repeated three times. The pressure of the hydrogenation reactor was adjusted to 1.8 MPa. The reaction was then carried out overnight at room temperature. After the reaction was completed, the reaction mixture was filtered through Celite, and the filter cake was washed with DMF (20 mL × 3) to provide a DMF solution containing 0.49 g of product **78-108,** which served as the raw material for the next reaction. $^1$H-NMR (600 MHz, DMSO-$d_6$) δ 8.21-8.13 (m, 7H), 4.16 (s, 3H), 3.63 (s, 18H), 2.74 (s, 8H), 2.31 (s, 6H), 2.17 (s, 8H), 1.51 (s, 12H), 1.38 (s, 9H), 1.23 (s, 6H)

4.9 Preparation of **78-122**

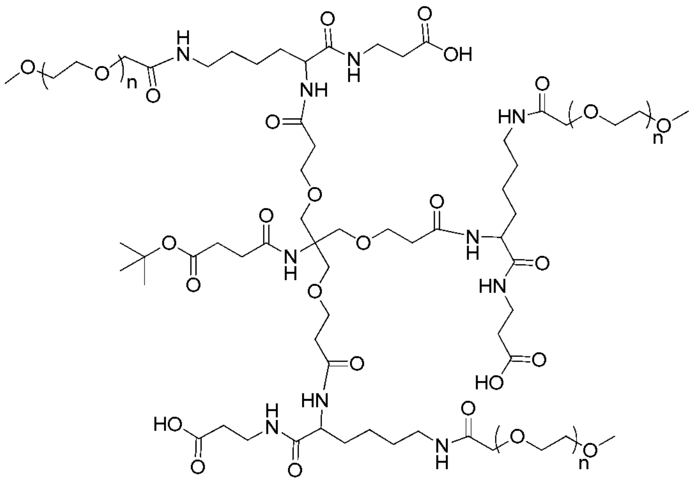

**78-108** (0.1 g, 0.09 mmol) was placed in a 50 mL round-bottom flask, and ultra-dry DMF (3 mL) was added for dissolution with the assistance of ultrasonication. The mixture was stirred at 0°C for 3 minutes. DIEA (0.2 mL, 1.23 mmol) was then slowly added dropwise, and the reaction was stirred for 5 minutes. M-SCM-10K (3.0 g, 0.28 mmol) was then added. After the reaction was completed, methyl tert-butyl ether (450 mL) was added for precipitation, and a solid precipitated and was filtered. The filter cake was redissolved in an appropriate amount of dichloromethane, then 200-300 mesh silica gel powder was added, and the mixture was evaporated to dryness. The resultant product was loaded by dry method, and subjected to column chromatography using 1% ammonia/9% methanol/dichloromethane as the eluent. The desired product was collected, concentrated, and evaporated to dryness to provide 3.0 g of product **78-122.**

4.10 Preparation of **78-125**

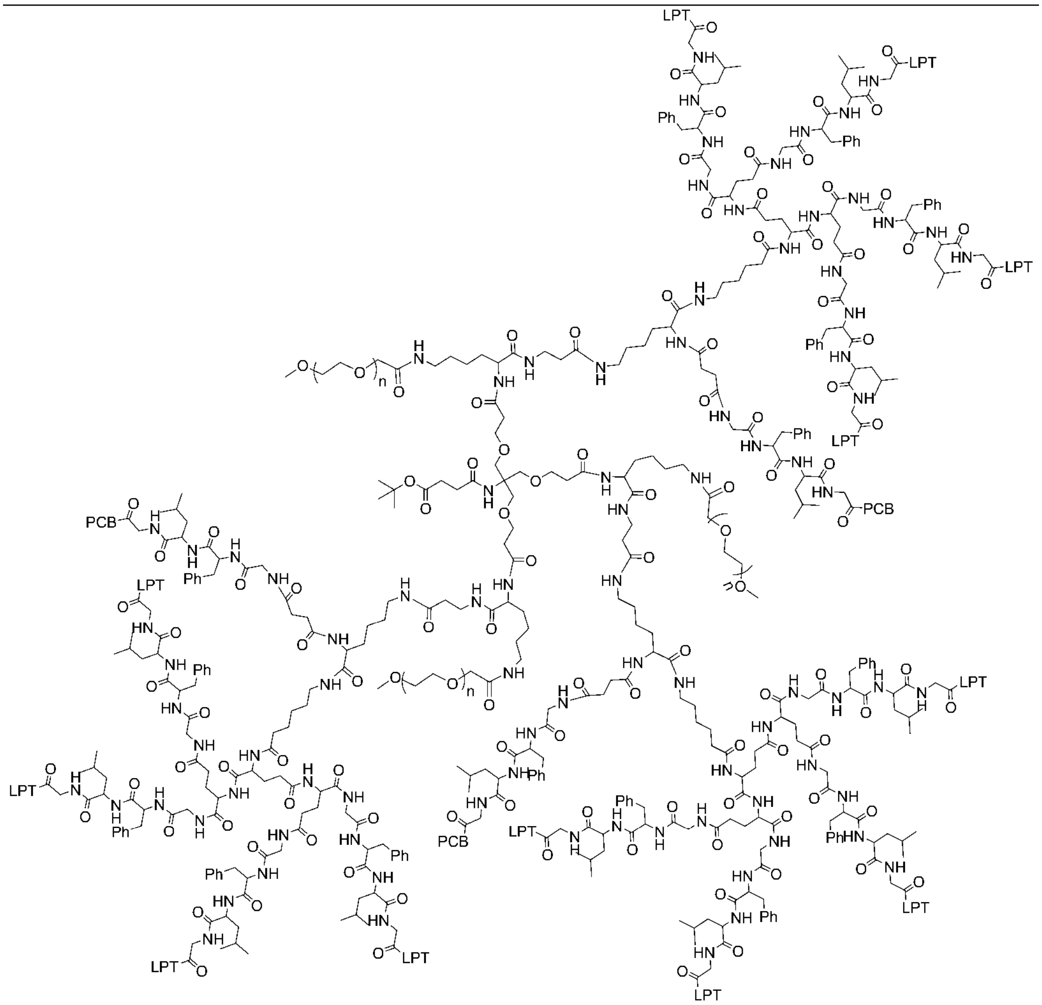

**78-122** (1.8 g, 0.056 mmol), **81-203** (1.22 g, 0.23 mmol), HBTU (0.1 g, 0.26 mmol) and HOBT (0.037 g, 0.26 mmol) were weighed and added to a 500 mL flask, then an appropriate amount of DMF was added to dissolve the mixture. DIEA (0.12 mL, 0.76 mmol) was slowly added dropwise under stirring. After the dropwise addition was completed, the reaction was carried out under stirring overnight at room temperature. After the reaction was completed, methyl tert-butyl ether (60 mL) and n-hexane (20 mL) were added. A powdery solid precipitated from the reaction mixture and was filtered. The filter cake was washed with methyl tert-butyl ether (40 mL × 3), and dissolved in the mixed solvent, then silica gel powder was added, and the mixture was evaporated to dryness. The resultant product was loaded by dry method, and subjected to column chromatography using 7% methanol/dichloromethane as the eluent. The desired product was collected, concentrated and evaporated to dryness to provide 2.65 g of product **78-125.**

4.11 Preparation of compound **78-128**

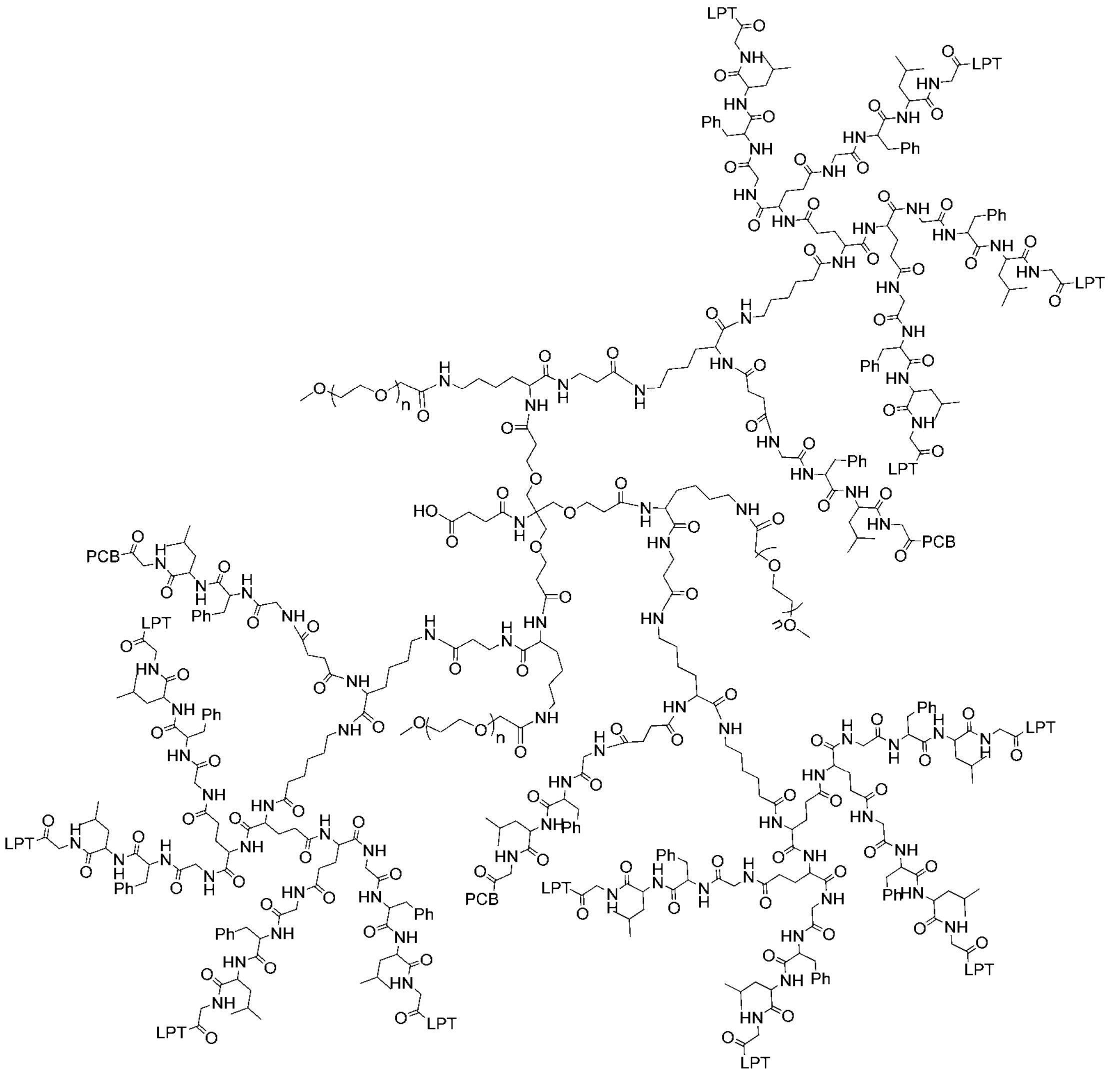

To a flask containing **78-125** (2.65 g, 0.055 mmol), dichloromethane was added. After complete dissolution with the assistance of ultrasonication, TFA (13.5 mL) was added, and the reaction was stirred overnight at room temperature. After the reaction was completed, methyl tert-butyl ether (60 mL) and n-hexane (20 mL) were added for precipitation. A powdery solid precipitated from the reaction mixture and was filtered. The filter cake was washed with methyl tert-butyl ether (40 mL × 3) and dried in a vacuum oven to provide 2.5 g of product **78-128.**

4.12 Preparation of **78-131**

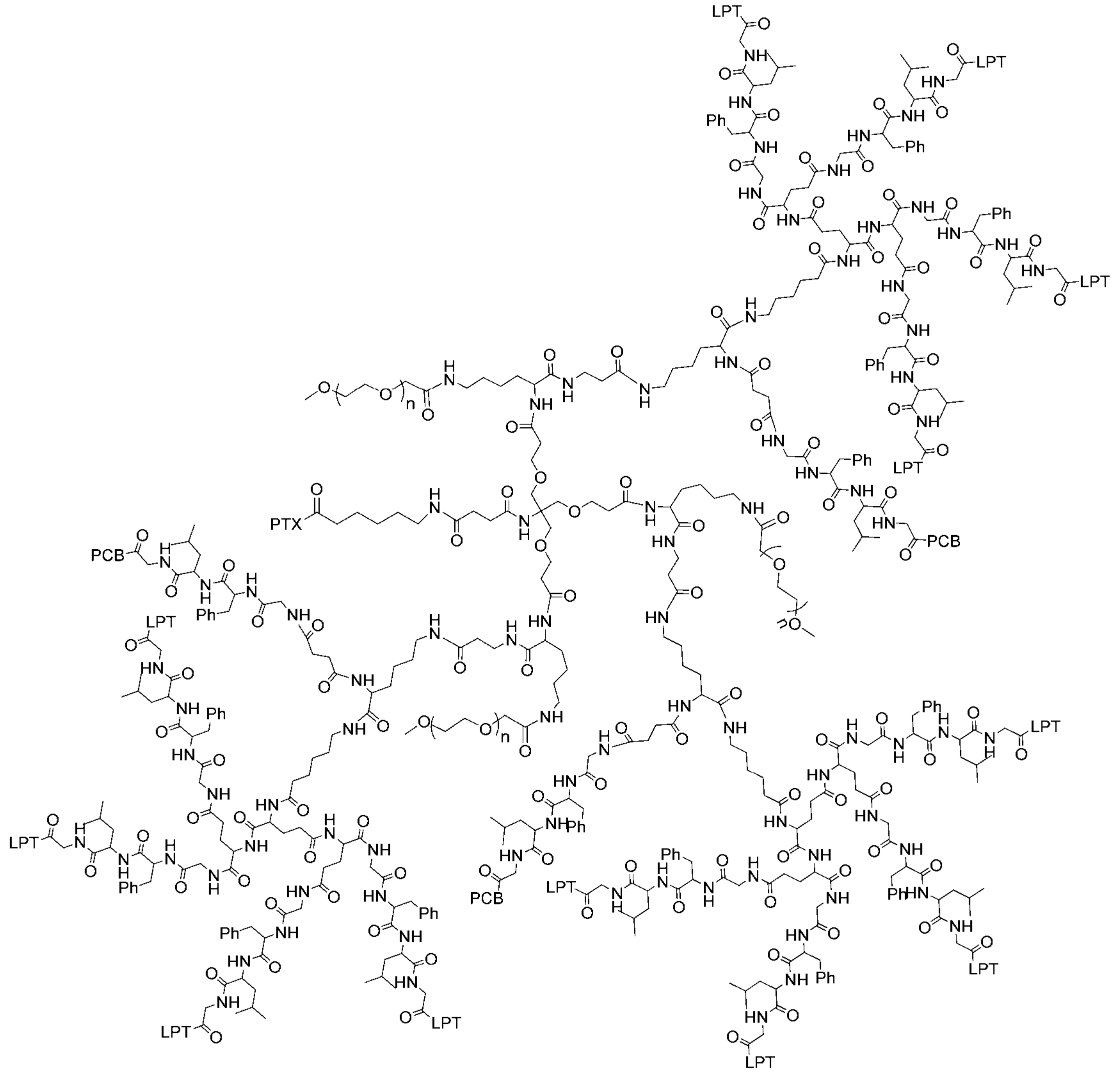

**78-128** (2.5 g, 0.052 mmol), **78-129** (0.1 g, 0.1 mmol), HBTU (0.041 g, 0.11 mmol) and HOBT (0.015 g, 0.11 mmol) were weighed and added to a 500 mL flask, and then an appropriate amount of DMF was added to dissolve the mixture. DIEA (0.04 mL, 0.234 mmol) was slowly added dropwise under stirring. After the dropwise addition was completed, the reaction was carried out under stirring overnight at room temperature. After the reaction was completed, methyl tert-butyl ether (60 mL) and n-hexane (20 mL) were added for precipitation. A powdery solid precipitated in the reaction mixture and was filtered. The filter cake was washed with methyl tert-butyl ether (40 mL × 3), and dissolved in the mixed solvent, then silica gel powder was added, and the mixture was evaporated to dryness. The resultant product was loaded by dry method, and subjected to column chromatography using 1% triethylamine/8% methanol/dichloromethane as the eluent. The desired product was collected, concentrated, and evaporated to dryness to provide 0.2 g of product **78-131.** $^1$H-NMR (600 MHz, DMSO-$d_6$) δ 8.77-8.69 (m, 10H), 8.58-8.49 (m, 10H), 8.26-8.15 (m, 28H), 8.14-7.93 (m, 72H), 7.91-7.82 (m, 14H), 7.81-7.77 (m, 11H), 7.76-7.63 (m, 15H), 7.52-7.43 (m, 16H), 7.40-7.25 (m, 42H), 7.25-7.16 (m, 78H), 7.15-7.11 (m, 21H), 7.11-7.08 (m, 12H), 7.06-7.03 (m, 3H), 7.03-6.96 (m, 16H), 6.91-6.88 (m, 5H), 5.30-5.22 (m, 21H), 4.80-4.67 (m, 18H), 4.62-4.52 (m, 15H), 4.42-4.32 (m, 15H), 4.31-4.09 (m, 42H), 3.88-3.71 (m, 42H), 3.69-3.58 (m, 62H), 3.56-3.44 (m, 2876H), 3.28-3.10 (m, 15H), 3.09-2.97 (m, 60H), 2.86-2.70 (m, 27H), 2.65-2.58 (m, 15H), 2.44-2.28 (m, 35H), 2.28-2.03 (m, 36H), 1.94-1.67 (m, 30H), 1.66-1.55 (m, 33H), 1.54-1.41 (m, 63H), 1.36-1.35 (m, 32H), 1.34-1.34 (m, 36H), 1.23-1.22 (m, 90H)

Example 5: Synthesis of Compound **72-188**

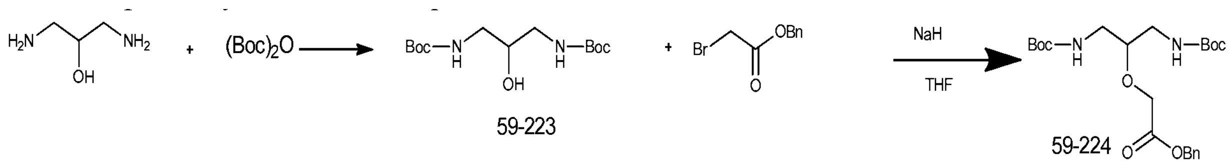


59-223

59-224

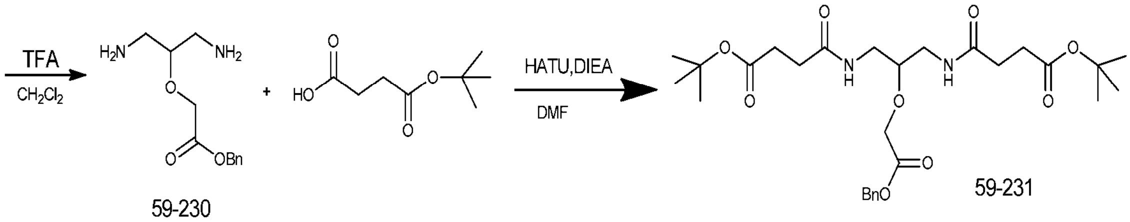


59-230

59-231

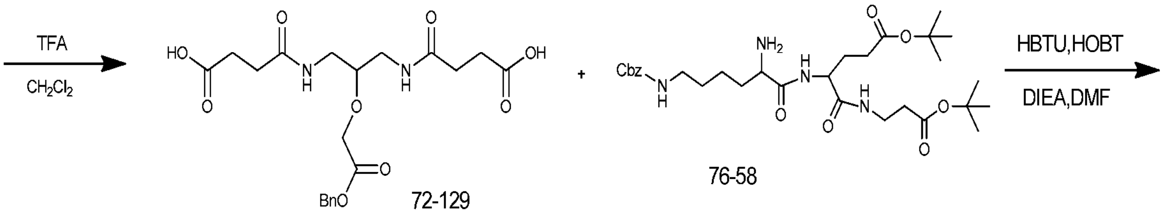


72-129

76-58

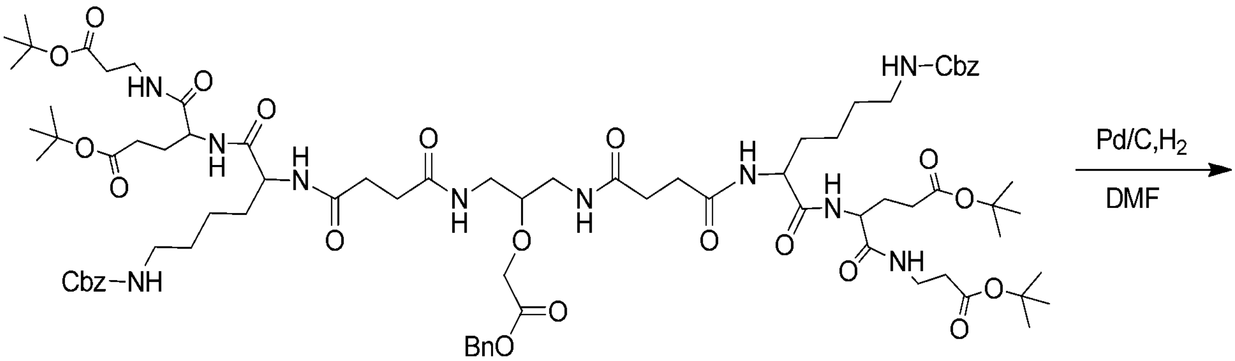


72-139

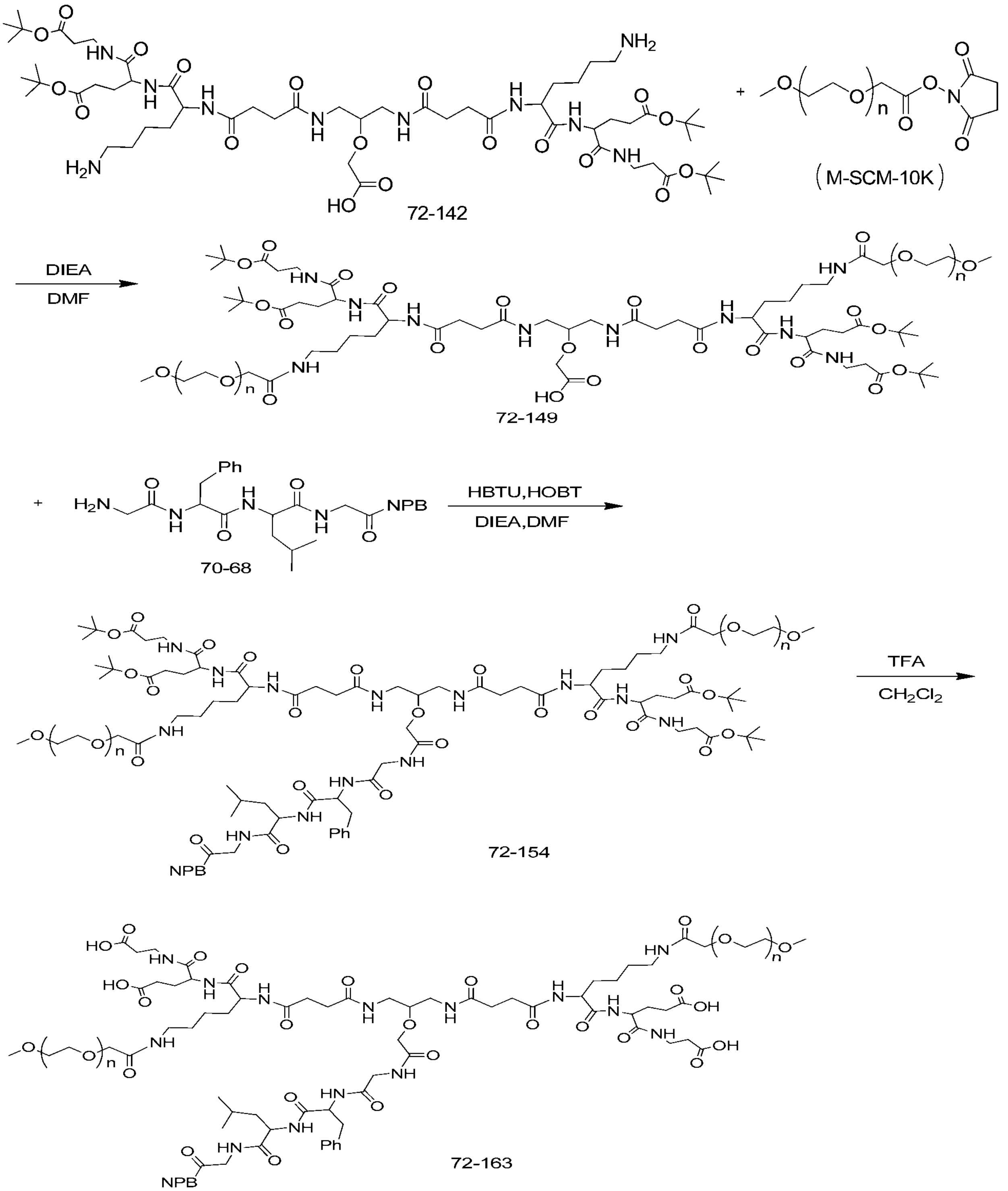


72-142

(M-SCM-10K)

72-149

70-68

72-154

72-163

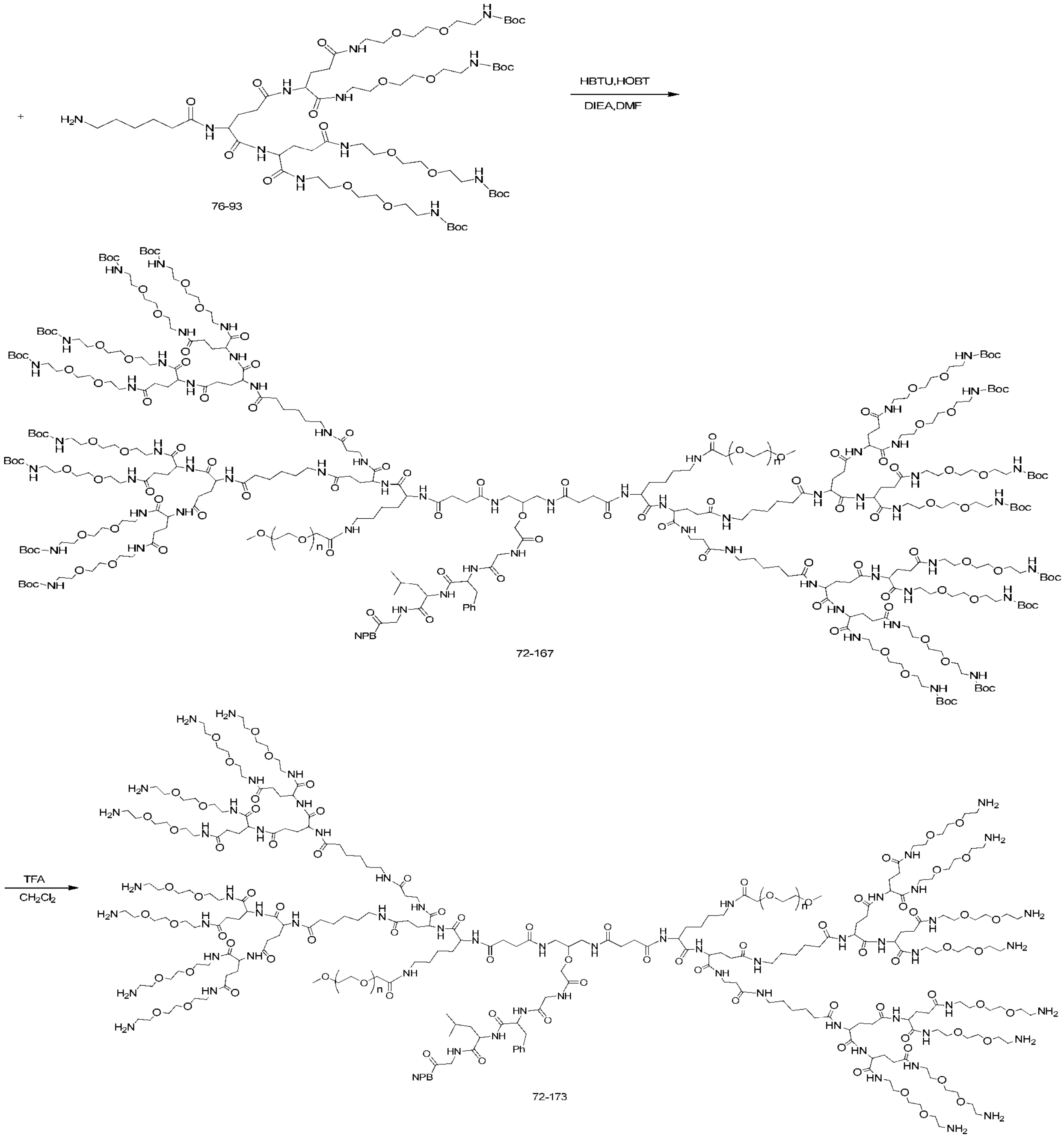


76-93

72-167

72-173

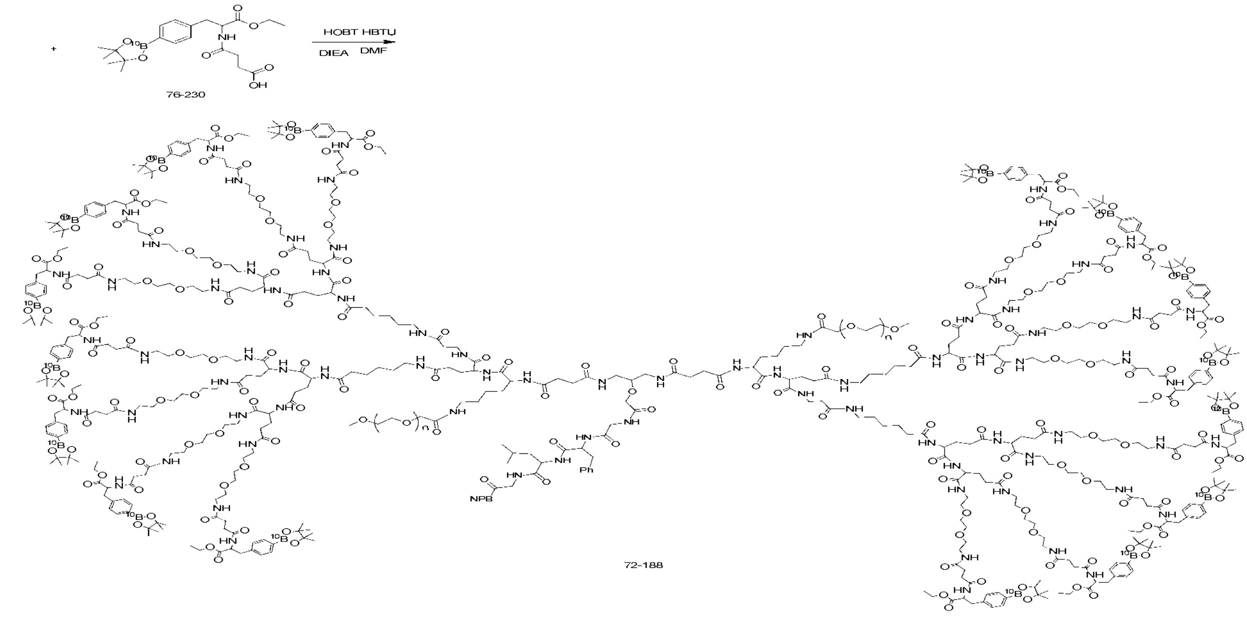


76-230

72-188

5.1 Preparation of **59-223**

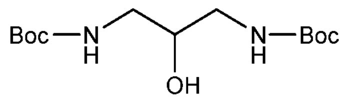

1,3-Diamino-2-hydroxypropane (4.5 g, 52.74 mmol) was added to a 250 mL flask, methanol (40 mL) was added for dissolution. The flask was allowed to stand at room temperature, and the reaction was carried out under stirring. Triethylamine (1 mL) was added, and the mixture was reacted under stirring at room temperature for 10 minutes. Boc anhydride (35 g, 146.52 mmol) was slowly added, the flask was placed in a 45°C water bath, and the reaction was carried out under stirring for 20 minutes. The flask was taken out and allowed to stand at room temperature, and the reaction was carried out under stirring for 3 hours. Upon completion of the reaction, the reaction mixture was concentrated under reduced pressure, and extracted by adding ethyl acetate (100 mL) and purified water (200 mL). The organic phases were combined, dried over anhydrous sodium sulfate, filtered, and the filtrate was collected and evaporated to dryness. The mixture was then dissolved in n-hexane and refluxed in a 65°C water bath for 20 minutes. The mixture was cooled to room temperature. A solid precipitated and was filtered to provide a solid product. The filter cake was collected and dried in a vacuum oven to provide 10 g of the product. $^1$H-NMR (600 MHz, DMSO-$d_6$) δ 6.61 (s, 2H), 4.76 (s, 1H), 3.44 (dd, $J$ = 10.8, 5.4 Hz, 1H), 2.89 (d, $J$ = 50.2 Hz, 4H), 1.38 (s, 18H)

5.2 Preparation of **59-224**

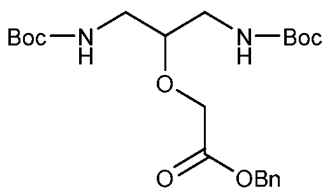

Sodium hydride (1.2 g, 46.35 mmol) was added to a 250 mL two-necked flask, and ultra-dry THF (20 mL) was added for dissolution. The flask was placed in an ice-water bath, and the reaction was carried out under stirring. **59-223** (9.38 g, 32.305 mmol) and benzyl bromoacetate (7.7 mL, 48.46 mmol) were added sequentially, and then the reaction was stirred at room temperature. The entire process was carried out under nitrogen protection. After the reaction was completed, the reaction mixture was transferred to a 1 L separatory funnel containing 5% dilute formic acid. Ethyl acetate

(200 mL) was added for extraction to provide an organic phase. The aqueous phase was further extracted with ethyl acetate (200 mL × 3). The organic phases were combined, and washed with saturated brine (200 mL × 2) and evaporated to dryness. The resulting solid product was dissolved in a dichloromethane/methanol mixture. The resultant product was loaded by dry method, and subjected to column chromatography using an 8-20% ethyl acetate/petroleum ether mixture as the eluent. The desired product was collected, concentrated, and dried in a vacuum oven to provide 6.8 g of the product with a yield of 39.8%. $^1$H-NMR (600 MHz, DMSO-$d_6$) δ 7.37 (t, *J* = 11.2 Hz, 5H), 6.66 (s, 2H), 5.16 (s, 2H), 4.24 (s, 2H), 3.08-2.95 (m, 5H), 1.37 (s, 18H)

5.3 Preparation of **59-230**

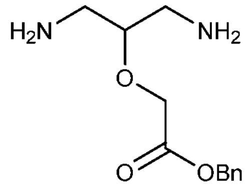

**59-224** (6.8 g, 15.507 mmol) was added to a reaction flask, and 4 M hydrochloric acid in 1,4-dioxane (155.07 mL) was added. After complete dissolution with the assistance of ultrasonication, the reaction was carried out under stirring at room temperature for 5 hours. After the reaction was completed, the reaction mixture was concentrated and evaporated to dryness. Dichloromethane (30 mL) was then added. The mixture was treated by ultrasonication, and rotary evaporated to dryness. This process was repeated five times. The mixture was dried to provide 3.7 g of the product.

5.4 Preparation of 59-231

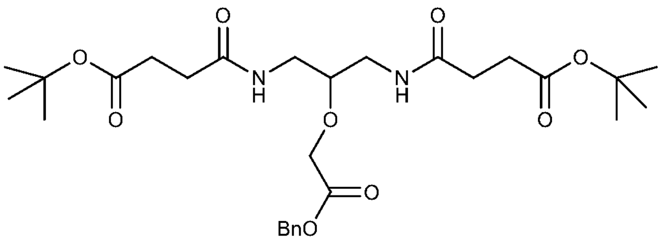

**59-230** (3.7 g, 15.507 mmol), tert-butyl succinate (5.94 g, 34.1154 mmol) and HATU (12.97 g, 34.1154 mmol) were added to a 250 mL flask, DMF (50 mL) was added to dissolve the mixture, and the mixture was reacted under stirring at 0°C for approximately 20 minutes. DIEA (23 mL, 139.563 mmol) was then slowly added dropwise. After the dropwise addition was completed, the reaction was carried out under stirring at 0°C for 5 hours. After the reaction was completed, ethyl acetate (200 mL) and pure water (200 mL) were added for extraction. The aqueous phase was extracted with ethyl acetate (100 mL × 3). The organic phases were combined, concentrated to 150 mL, and washed three times with pure water. The ethyl acetate phase was then collected, rotary evaporated to dryness, and dissolved in a dichloromethane/methanol mixed solvent. The resultant product was loaded by dry method, and subjected to column chromatography using a 0.5-1.5% methanol/dichloromethane mixture as the eluent. The desired product was collected, concentrated, and dried in a vacuum oven to provide 4.8 g of the product with a yield of 56.47%. $^1$H-NMR (600 MHz, DMSO-$d_6$) δ 7.83 (s, 2H), 7.38 (d, *J* = 4.5 Hz, 5H), 5.16 (s, 2H), 4.28 (s, 2H), 3.21-3.10 (m, 5H), 2.39 (t, *J* = 6.8 Hz, 4H), 2.30 (t, *J* = 7.0 Hz, 4H), 1.37 (d, *J* = 3.5 Hz, 18H)

5.5 Preparation of **72-129**

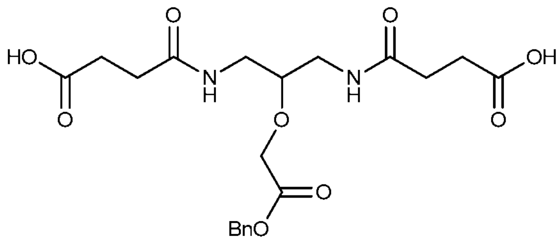

**59-231** (2.5 g, 4.54 mmol) was added to a reaction flask, and then dichloromethane (5 mL) was added. After complete dissolution with the assistance of ultrasonication, TFA (15 mL, 181.6 mmol) was added, and the reaction was stirred at room temperature overnight. After the reaction was completed, the reaction mixture was concentrated and evaporated to dryness, then dichloromethane was added, and the mixture was rotary evaporated to dryness. This process was repeated three times. Methyl tert-butyl ether was then added for precipitation. The mixture was treated by ultrasonication for 10 minutes, allowed to stand, and filtered. The filter cake was collected. This process was repeated three times. The mixture was dried in vacuum to provide 1.52 g of the product with a yield of 76.38%.

5.6 Preparation of **72-139**

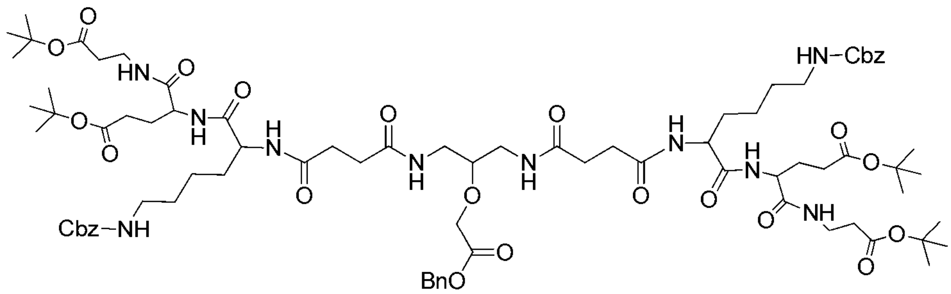

**72-129** (0.71 g, 1.634 mmol), **76-58** (2.13 g, 3.593 mmol), HBTU (1.8531 g, 4.901 mmol) and HOBT (0.66 g, 4.901 mmol) were added to a 250 mL round-bottom flask, and dissolved in DMF (20 mL). The reaction flask was then allowed to stand at room temperature, and the mixture was stirred for approximately 20 minutes. DIEA (8.8 mL, 68.426 mmol) was then slowly added dropwise. After the dropwise addition was completed, the reaction flask was allowed to stand at room temperature, and the mixture was stirred. After the reaction was completed, n-hexane (30 mL) and methyl tert-butyl ether (200 mL) were added for precipitation, the supernatant was discarded, and n-hexane and methyl tert-butyl ether were added for precipitation. This process was repeated four times. The mixture was filtered by suction to provide a solid product. The obtained solid product was dissolved in a dichloromethane/methanol mixed solvent. The resultant product was loaded by dry method, and subjected to column chromatography using a 3-4% methanol/dichloromethane mixture as the eluent. The desired product was collected, concentrated, and dried in a vacuum oven to provide 2.247 g of the product with a yield of 86.4%. $^1$H-NMR (600 MHz, DMSO-*d*$_6$) δ 8.12 (d, *J* = 7.2 Hz, 2H), 7.93-7.87 (m, 4H), 7.73 (s, 2H), 7.35 (dt, *J* = 12.1, 5.6 Hz, 15H), 7.23 (s, 2H), 5.15 (s, 2H), 5.00 (s, 4H), 4.25 (s, 2H), 4.15 (d, *J* = 5.2 Hz, 5H), 3.27 (d, *J* = 6.9 Hz, 2H), 3.15 (dd, *J* = 12.9, 6.1 Hz, 6H), 2.96 (d, *J* = 6.7 Hz, 4H), 2.40-2.26 (m, 14H), 2.20-2.13 (m, 4H), 1.89 (td, *J* = 13.8, 5.3 Hz, 2H), 1.76-1.69 (m, 2H), 1.63 (dd, *J* = 14.0, 8.6 Hz, 2H), 1.52-1.46 (m, 2H), 1.38 (s, 18H), 1.37 (s, 18H), 1.25 (dd, *J* = 12.2, 6.2 Hz, 6H)

5.7 Preparation of **72-142**

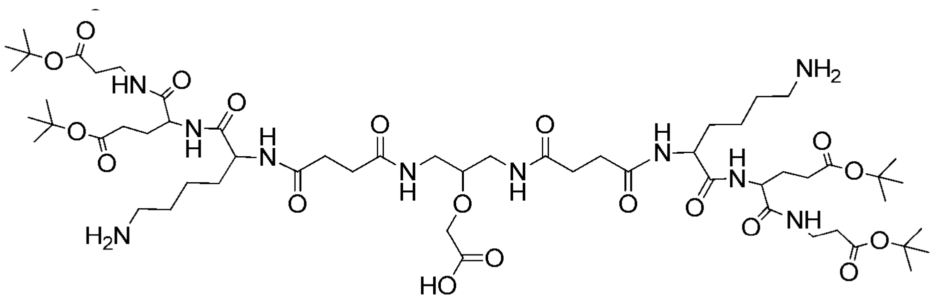

**72-139** (0.5 g, 0.315 mmol) was added to a hydrogenation reactor, then 10% Pd/C catalyst (0.03 g) and methanol (20 mmol) were added. Hydrogen gas was introduced, and the pressure of hydrogen gas was adjusted to 300 Psi. The reaction was carried out under stirring at room temperature for 3 days. After the reaction was completed, the reaction mixture was filtered through Celite, and the filter cake was washed with methanol (15 mL × 3). The filtrate was placed in a 500 mL round-bottom flask and rotary evaporated. Dichloromethane was added to dissolve the mixture, and then 0.05 mL of toluene was added and rotary evaporated to dryness. This process was repeated five times. The residue was dried to provide 0.387 g of product **72-142.** $^1$H-NMR (600 MHz, DMSO-$d_6$) δ 9.16-9.09 (m, 1H), 8.18 (dd, *J* = 13.8, 7.4 Hz, 2H), 8.01 (dd, *J* = 23.7, 7.9 Hz, 2H), 7.89 (dd, *J*= 6.1, 3.0 Hz, 4H), 5.12-5.10 (m, 4H), 4.16 (d, *J* = 4.7 Hz, 5H), 3.75 (s, 2H), 3.27 (dd, *J* = 11.9, 6.1 Hz, 4H), 3.20-3.13 (m, 4H), 2.79-2.72 (m, 4H), 2.43-2.28 (m, 14H), 2.18 (dd, *J* = 15.9, 9.5 Hz, 4H), 1.89 (d, *J* = 14.5 Hz, 2H), 1.76-1.62 (m, 6H), 1.50 (dd, *J* = 15.5, 6.0 Hz, 6H), 1.38 (d, *J*= 5.1 Hz, 36H)

5.8 Preparation of **72-149**

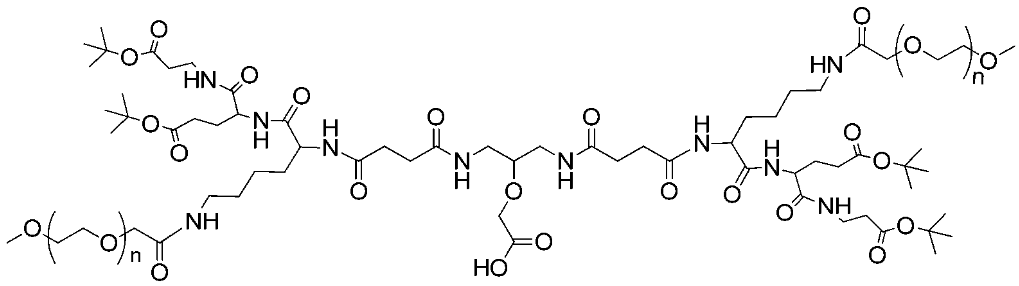

**72-142** (0.115 g, 0.0939 mmol) was placed in a 250 mL flask, and dissolved with anhydrous DMF (30 mL). The flask was allowed to stand at -5°C and the reaction was carried out for 10 minutes. DIEA (0.3 mL, 1.877 mmol) was then slowly added dropwise. After the stirring was continued at low temperature for 30 minutes, M-SCM-10K (2 g, 0.1877 mmol, purchased from Jenkem, Lot Number: A3016-N230101) was added, and the reaction was stirred in the dark at low speed for one week at room temperature. After the reaction was completed, methyl tert-butyl ether (200 mL) and n-hexane (70 mL) were added to precipitate a solid, which was filtered and washed with methyl tert-butyl ether (40 mL × 3). The resulting solid was dissolved in a dichloromethane/methanol mixture, loaded by dry method, and subjected to column chromatography using a mixture of 1% aqueous ammonia and 5-10% methanol/dichloromethane as the eluent. The desired product was collected, concentrated, and dried in a vacuum oven to provide 2.01 g of the product with a yield of 76.8%. $^1$H-NMR (600 MHz, DMSO-$d_6$) δ9.16-9.13 (m, 1H), 9.06-8.56 (m, 2H), 8.26-8.202 (m, 2H), 8.018-7.96 (m, 2H), 7.729-7.661 (m, 4H), 4.181-4.114 (m, 3H), 4.089-4.056 (m, 2H), 4.02-3.96 (m, 2H), 3.52-3.50 (m, 1914H), 3.28-3.25 (m, 4H), 3.202-3.134 (m, 4H), 3.10-3.06 (m, 4H), 2.42-2.31 (m, 14H), 2.21-2.15 (m, 4H), 1.95-1.87 (m, 2H), 1.77-1.69 (m, 2H), 1.68-1.605 (m, 2H), 1.56-1.48 (m, 2H), 1.406-1.394 (m, 2H), 1.388-1.369 (m, 36H), 1.351-1.318 (m, 4H)

5.9 Preparation of **72-154**

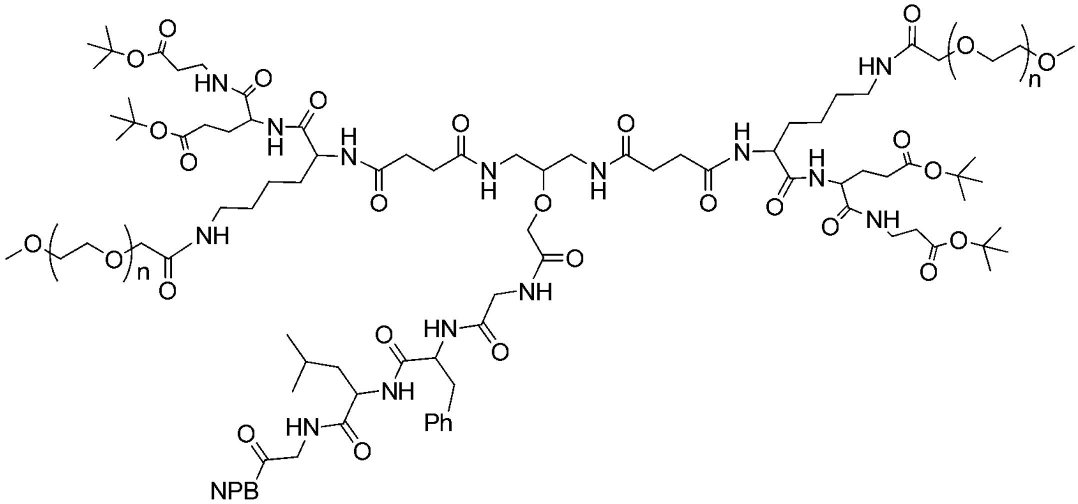

**72-149** (2.01 g, 0.0901 mmol), **70-68** (0.13 g, 0.189 mmol), HBTU (0.0513 g, 0.135 mmol) and HOBT (0.0183 g, 0.135 mmol) were added to a 250 mL round-bottom flask, and DMF (20 mL) was added for dissolution. The reaction flask was placed at room temperature and the mixture was stirred for approximately 20 minutes. DIEA (0.1 mL, 0.6051 mmol) was then slowly added dropwise. After the dropwise addition was completed, the reaction flask was kept at room temperature, and the reaction was carried out under stirring. After the reaction was completed, n-hexane (30 mL) and methyl tert-butyl ether (200 mL) were added for precipitation. The supernatant was discarded, and n-hexane and methyl tert-butyl ether were added again for precipitation. This process was repeated four times. The mixture was filtered to provide a solid product. The obtained solid product was dissolved in a dichloromethane/methanol mixture, loaded by dry method, and subjected to column chromatography using a mixture of 1% aqueous ammonia and 5-7% methanol/dichloromethane as the eluent. The desired product was collected, concentrated, and dried in a vacuum oven to provide 0.9 g of the product in a yield of 45%. $^1$H-NMR (600 MHz, DMSO-$d_6$) δ 8.61-8.54 (m, 1H), 8.25-8.21 (m, 1H), 8.20-8.08 (m, 6H), 8.075-8.008 (m, 4H), 7.97-7.85 (m, 6H), 7.75-7.71 (m, 2H), 7.69-7.66 (m, 2H), 7.61-7.59 (m, 1H), 7.54-7.51 (m, 1H), 7.29-7.23 (m, 5H), 4.6-4.56 (m, 1H), 4.48-4.42 (m, 1H), 4.39-4.33 (m, 1H), 4.19-4.03 (m, 7H), 3.98-3.93 (m, 3H), 3.52-3.50 (m, 1914H), 3.27-3.26 (m, 1H), 3.18-3.14 (m, 4H), 3.08-3.03 (m, 8H), 2.92-2.89 (m, 2H), 2.81-2.72 (m, 4H), 2.40-2.32 (m, 14H), 2.2-2.14 (m, 4H), 2.02-1.96 (m, 2H), 1.93-1.86 (m, 3H), 1.82-1.77 (m, 2H), 1.74-1.7 (m, 2H), 1.66-1.6 (m, 4H), 1.54-1.46 (m, 6H), 1.42-1.39 (m, 2H), 1.38-1.36 (m, 36H), 0.91-0.88 (m, 3H), 0.86-0.84 (m, 3H)

5.10 Preparation of **72-163**

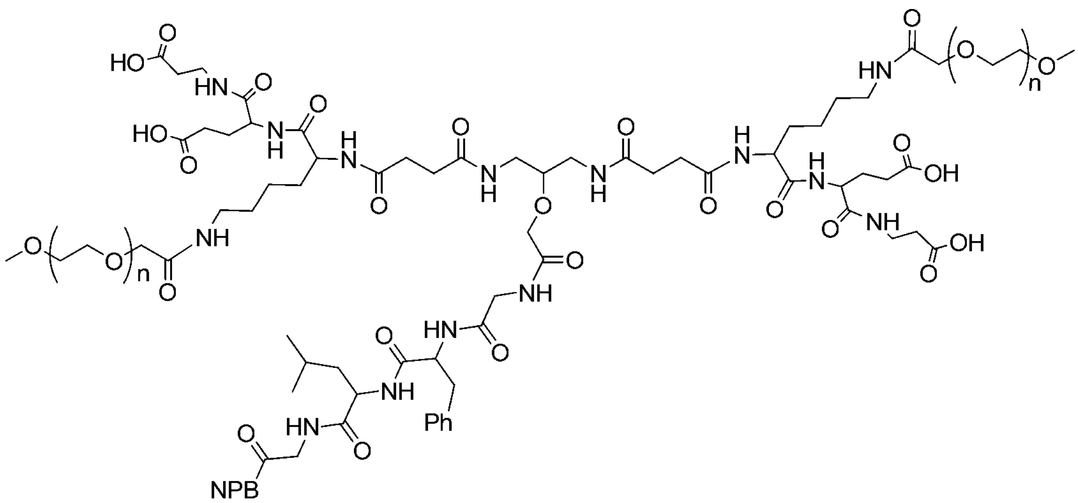

**72-154** (0.9 g, 0.03916 mmol) was placed in a 250 mL round-bottom flask, dichloromethane (2 mL) was added for dissolution with the assistance of ultrasonication. TFA (10 mL, 3.13 mmol) was then added, and the reaction was stirred at room temperature. After the reaction was completed, the reaction mixture was evaporated to provide an oil using a rotary

evaporator, and methyl tert-butyl ether (60 mL) was added. A powdery solid precipitated from the reaction mixture and was filtered. The filter cake was washed with methyl tert-butyl ether (40 mL × 3), collected, and dried in a vacuum oven to provide 0.83 g of the product. $^{1}$H-NMR (600 MHz, DMSO-$d_6$) δ 9.33-9.29 (m, 4H), 8.59-8.56 (m, 1H), 8.28-8.24 (m, 1H), 8.23-8.15 (m, 4H), 8.13-8.08 (m, 3H), 8.07-8.05 (m, 2H), 8.04-8.01 (m, 1H), 8.0-7.85 (m, 6H), 7.72-7.66 (m, 4H), 7.62-7.57 (m, 1H), 7.55-7.50 (m, 1H), 7.29-7.27 (m, 1H), 7.26-7.22 (m, 4H), 4.58-4.55 (m, 1H), 4.45-4.43 (m, 1H), 4.36-4.34 (m, 1H), 4.11-4.05(m, 7H), 3.96-3.94 (m, 3H), 3.52-3.50 (m, 1914H), 3.21-3.02 (m, 13H), 2.8-2.72 (m, 2H), 2.65-2.60 (m, 4H), 2.40-2.33 (m, 14H), 2.22-2.15 (m, 4H), 2.0-1.88 (m, 3H), 1.82-1.72 (m, 4H), 1.68-1.58 (m, 4H), 1.54-1.48 (m, 4H), 1.46-1.33 (m, 6H), 0.91-0.88 (m, 3H), 0.85-0.83 (m, 3H)

5.11 Preparation of **72-167**

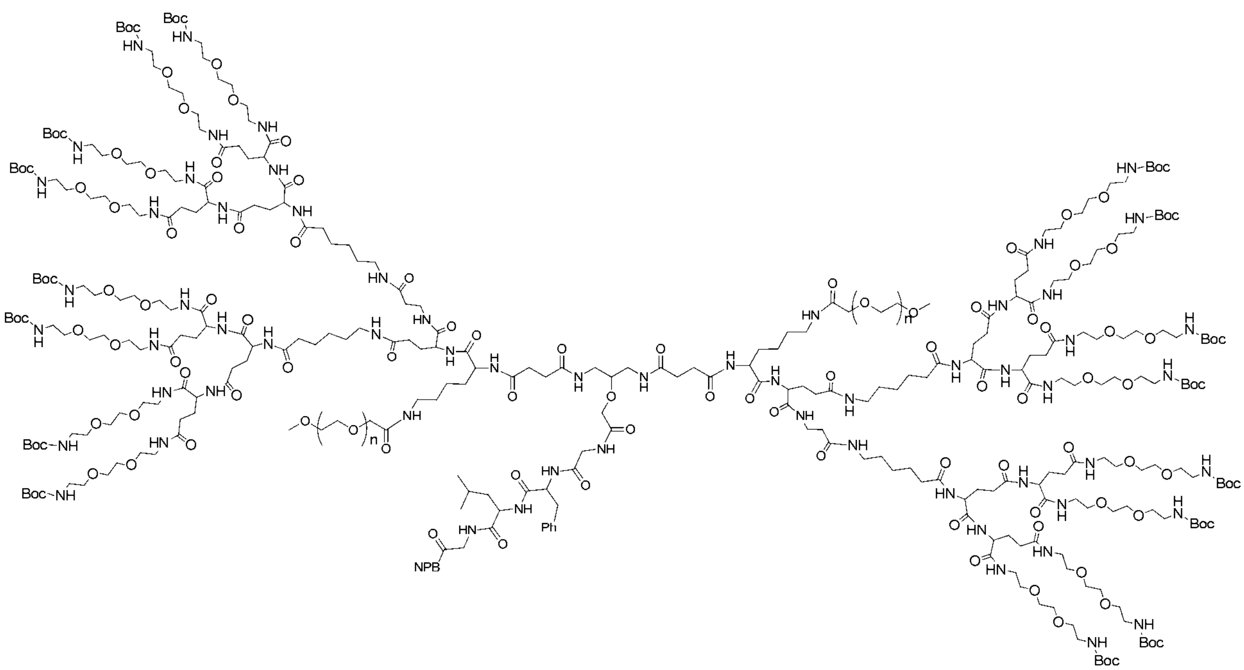

**72-163** (0.56 g, 0.0246 mmol), **76-93** (0.28 g, 0.1968 mmol), HBTU (0.075 g, 0.1476 mmol) and HOBT (0.027 g, 0.1476 mmol) were added to a 250 mL round-bottom flask, and dissolved with DMF (40 mL). The reaction flask was allowed to stand at room temperature and the mixture was stirred for approximately 20 minutes. DIEA (0.07 mL, 0.4428 mmol) was then slowly added dropwise. After the dropwise addition was completed, the reaction flask was allowed to stand at room temperature, and the reaction was carried out under stirring. After the reaction was completed, n-hexane (30 mL) and methyl tert-butyl ether (200 mL) were added for precipitation, the supernatant was discarded, and n-hexane and methyl tert-butyl ether were added for precipitation, and the process was repeated three times. The mixture was filtered to provide a solid product, and the obtained solid product was dissolved in a dichloromethane/methanol mixed solvent. The resultant product was loaded by dry method, and subjected to column chromatography using a 1% ammonia water/6-10% methanol/dichloromethane mixed solution as the eluent. The desired product was collected, concentrated, and dried in a vacuum oven to provide 0.793 g of the product with a yield of 76.47%. $^{1}$H-NMR (600 MHz, DMSO-$d_6$) δ 8.58-8.56 (m, 1H), 8.28-8.17 (m, 17H), 8.11-8.05 (m, 21H), 8.03-8.00 (m, 5H), 7.99-7.97 (m, 6H), 7.87-7.85 (m, 6H), 7.83-7.82 (m, 6H), 7.77-7.74 (m, 4H), 7.71-7.68 (m, 4H), 7.60-7.58 (m, 1H), 7.53-7.51 (m, 1H), 7.24-7.22 (m, 5H), 4.58-4.55 (m, 1H), 4.45-4.43 (m, 1H), 4.36-4.34 (m, 1H), 4.21-4.17 (m, 12H), 4.09-4.06 (m, 7H), 3.96-3.95 (m, 3H), 3.52-3.50 (m, 1914H), 3.43-3.38 (m, 128H), 3.19-3.15 (m, 45H), 3.06-3.04 (m, 32H), 2.76-2.74 (m, 2H), 2.65-2.60 (m, 12H), 2.37-2.31 (m, 14H), 2.13-2.10 (m, 28H), 1.87-1.78 (m, 32H), 1.75-1.71 (m, 15H), 1.48-1.43 (m, 30H), 1.37-1.36 (m, 144H), 0.91-0.88 (m, 3H), 0.85-0.83 (m, 3H)

5.12 Preparation of **72-173**

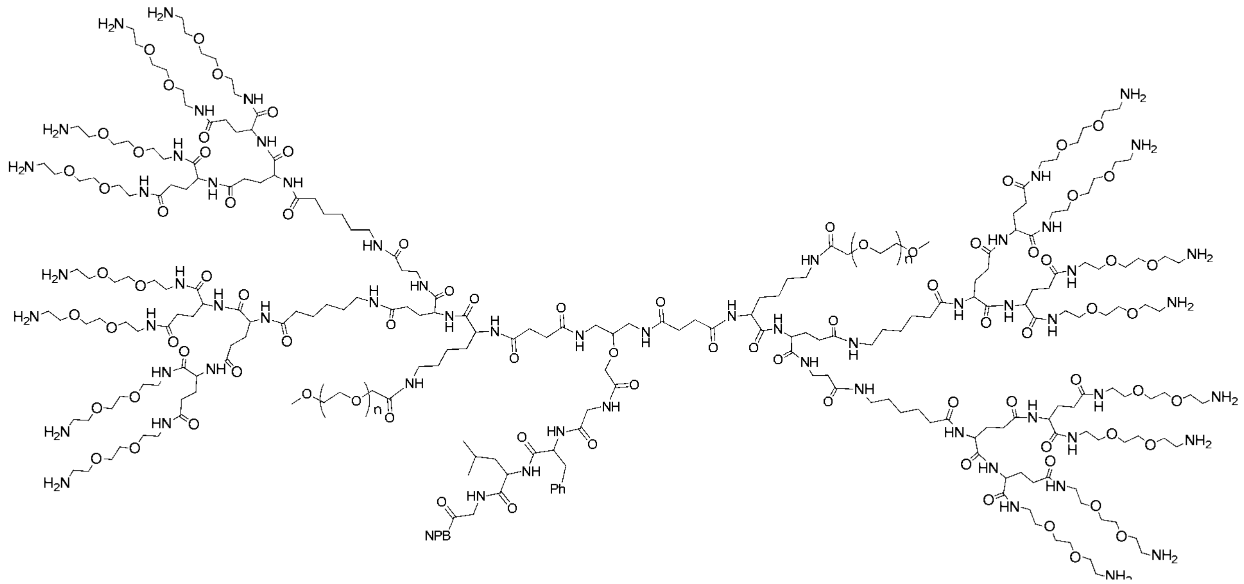

**72-167** (0.793 g, 0.0279 mmol) was placed in a 250 mL round-bottom flask, dichloromethane (5 mL) was added for dissolution with the assistance of ultrasonication. TFA (0.7 mL, 8.8 mmol) was then added and the reaction was carried out under stirring at room temperature. After the reaction was completed, the reaction mixture was evaporated to obtain an oil using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide 0.649 g of the product.

5.13 Preparation of **72-188**

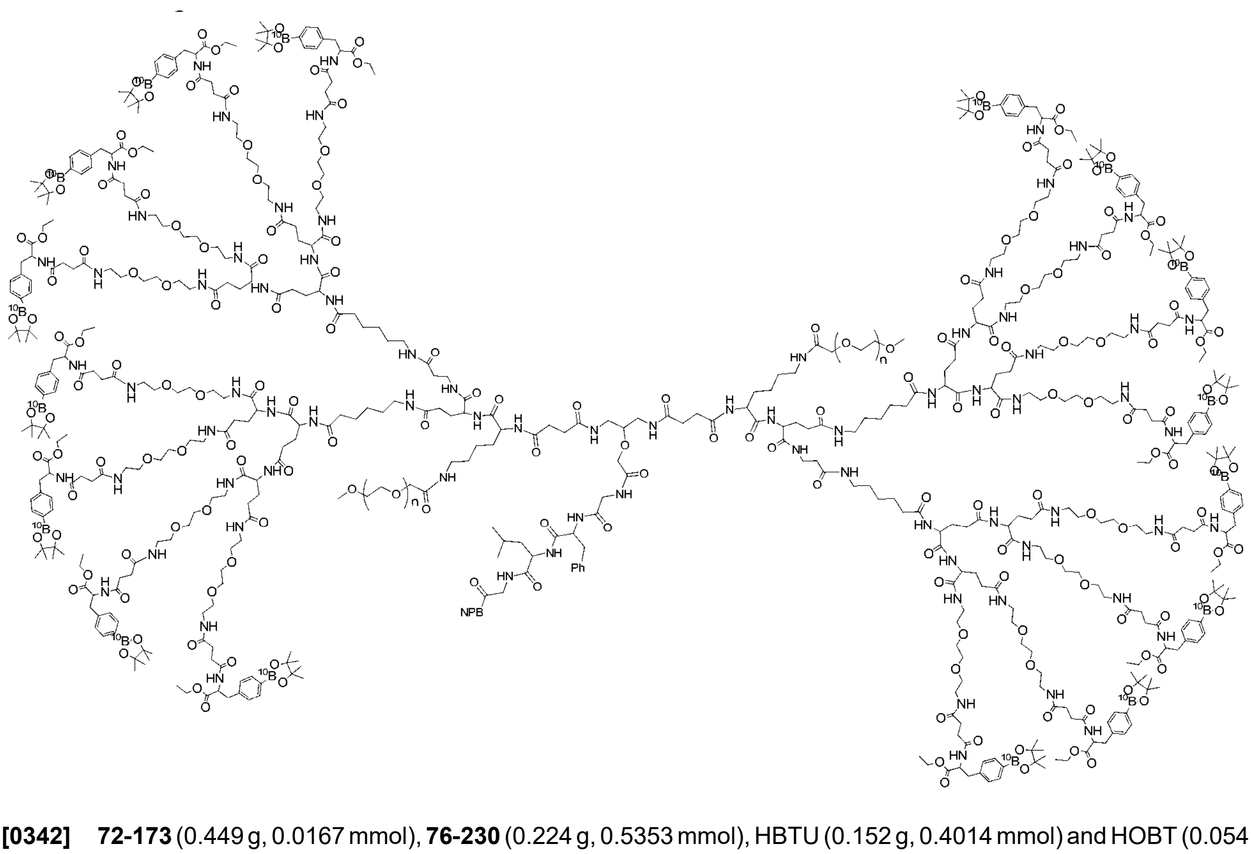

**72-173** (0.449 g, 0.0167 mmol), **76-230** (0.224 g, 0.5353 mmol), HBTU (0.152 g, 0.4014 mmol) and HOBT (0.054

g, 0.4014 mmol) were added to a 250 mL round-bottom flask, and dissolved with DMF (30 mL). The reaction flask was then allowed to stand at room temperature, and the mixture was stirred for approximately 20 minutes. DIEA (0.2 mL, 1.204 mmol) was then slowly added dropwise. After the dropwise addition was completed, the reaction flask was allowed to continuously stand at room temperature, and the reaction was carried out under stirring. After the reaction was completed, n-hexane (30 mL) and methyl tert-butyl ether (200 mL) were added for precipitation, the supernatant was discarded, and n-hexane and methyl tert-butyl ether were added for precipitation. This process was repeated three times. The mixture was filtered to provide a solid product. The obtained solid product was dissolved in a mixed solvent of dichloromethane and methanol, loaded by dry method, and subjected to column chromatography using a mixed solution of 1% ammonia water/5-10% methanol/dichloromethane as the eluent. The desired product was collected, concentrated, and dried in a vacuum oven to provide 0.053 g of the product with a yield of 8.55%. $^1$H-NMR (600 MHz, DMSO-$d_6$) δ 8.57 (s, 1H), 8.35-8.28 (m, 16H), 8.27-8.18 (m, 17H), 8.11-8.05 (m, 26H), 8.00-7.99 (m, 12H), 7.90-7.86 (m, 22H), 7.75-7.74 (m, 4H), 7.70-7.68 (m, 36H), 7.59-7.58 (m, 2H), 7.18-7.16 (m, 36H), 4.60-4.59 (m, 1H), 4.43-4.38 (m, 18H), 4.26-4.22 (m, 12H), 4.04-4.00 (m, 42H), 3.52-3.50 (m, 1914H), 3.44-3.40 (m, 128H), 3.22-3.11 (m, 45H), 3.00-2.98 (m, 32H), 2.92-2.91 (m, 32H), 2.76-2.75 (m, 2H), 2.62-2.60 (m, 12H), 2.40-2.19 (m, 78H), 2.18-2.05 (m, 28H), 1.66-1.62 (m, 47H), 1.45-1.43 (m, 30H), 1.36-1.34 (m,177H), 1.11-1.08 (m, 48H), 0.91-0.90 (m, 6H)

Example 6: Synthesis of Compound **70-261**

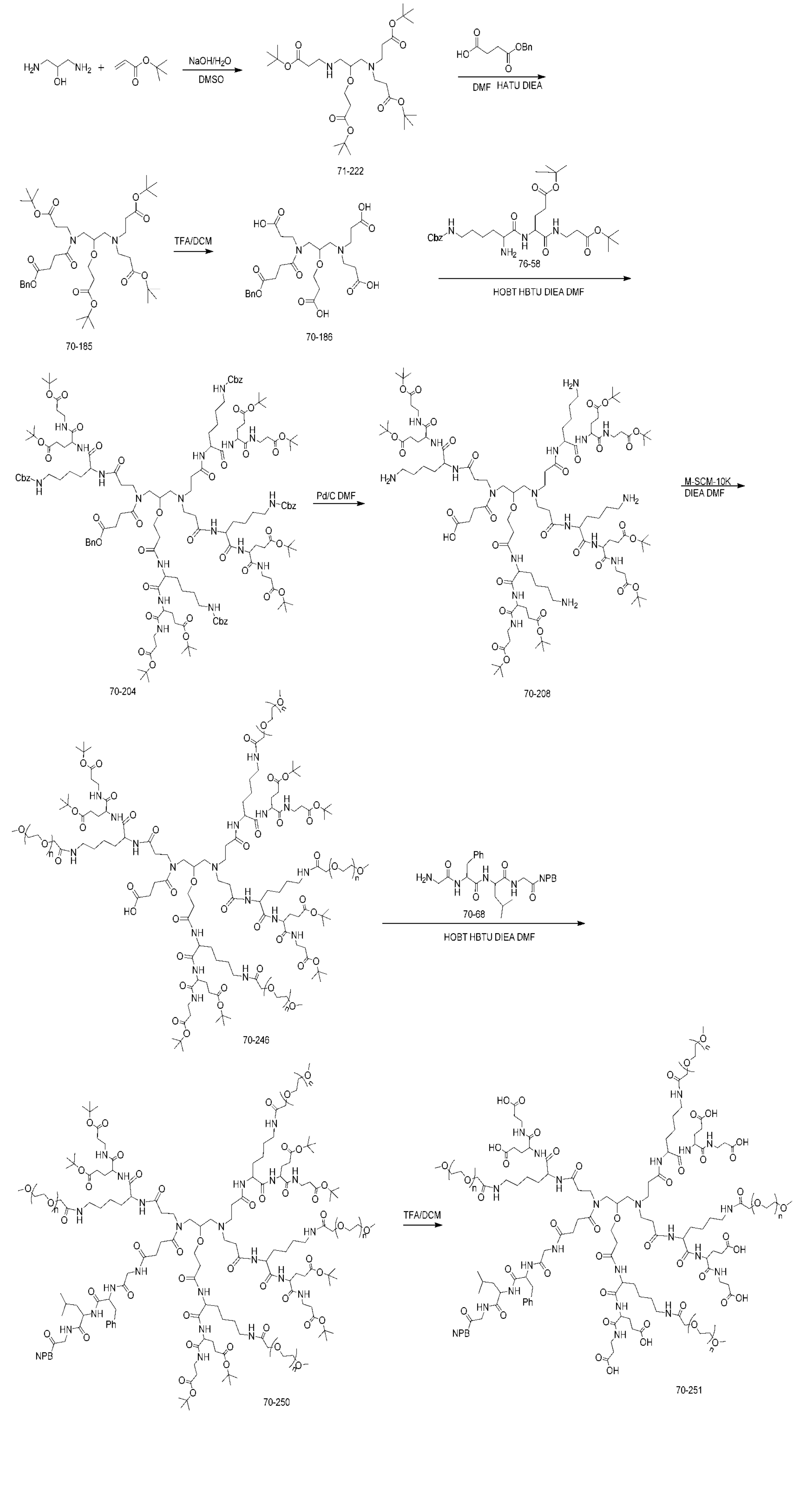


71-222

70-185

70-186

76-58

70-204

70-208

70-246

70-68

70-250

70-251

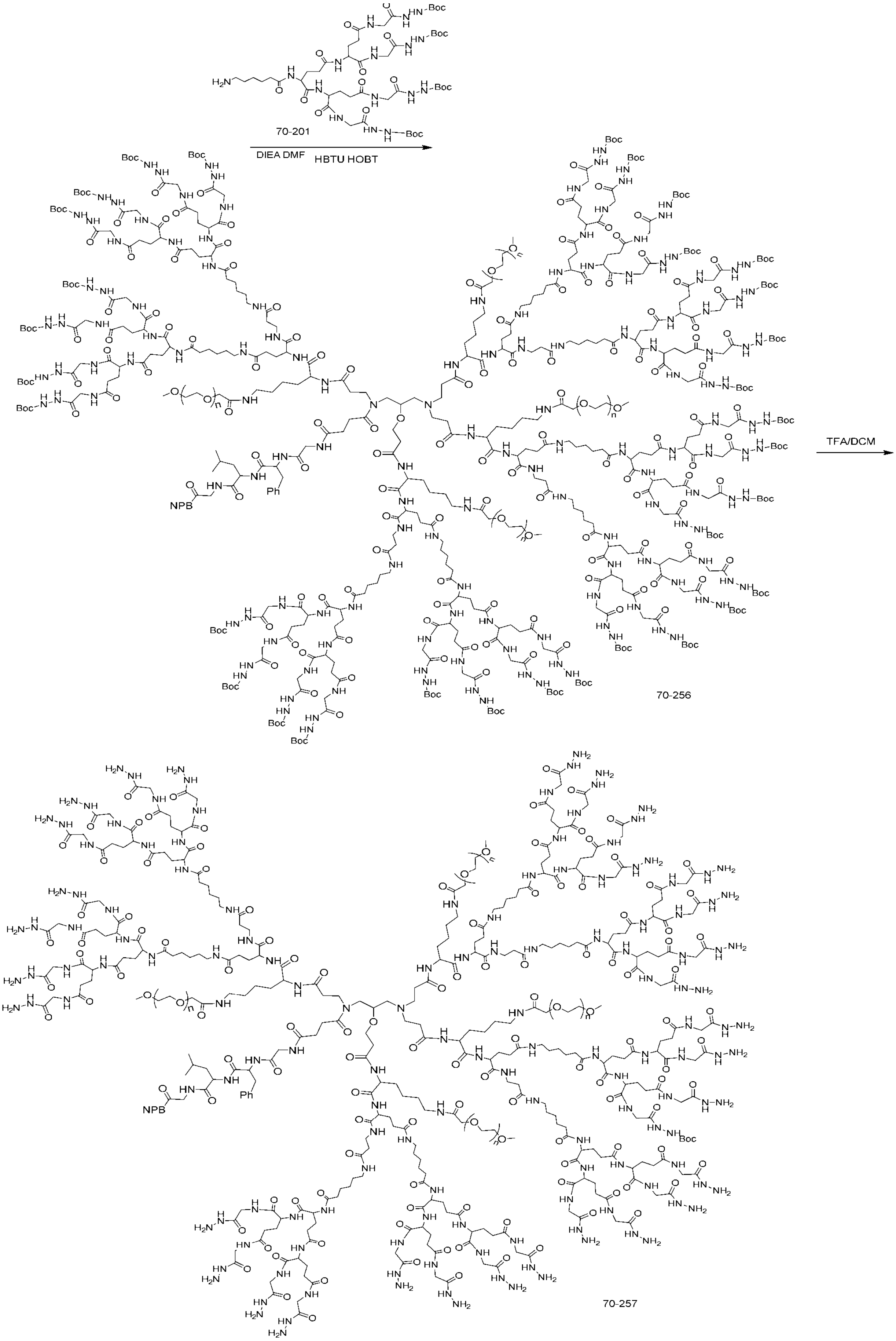


70-201

70-256

70-257

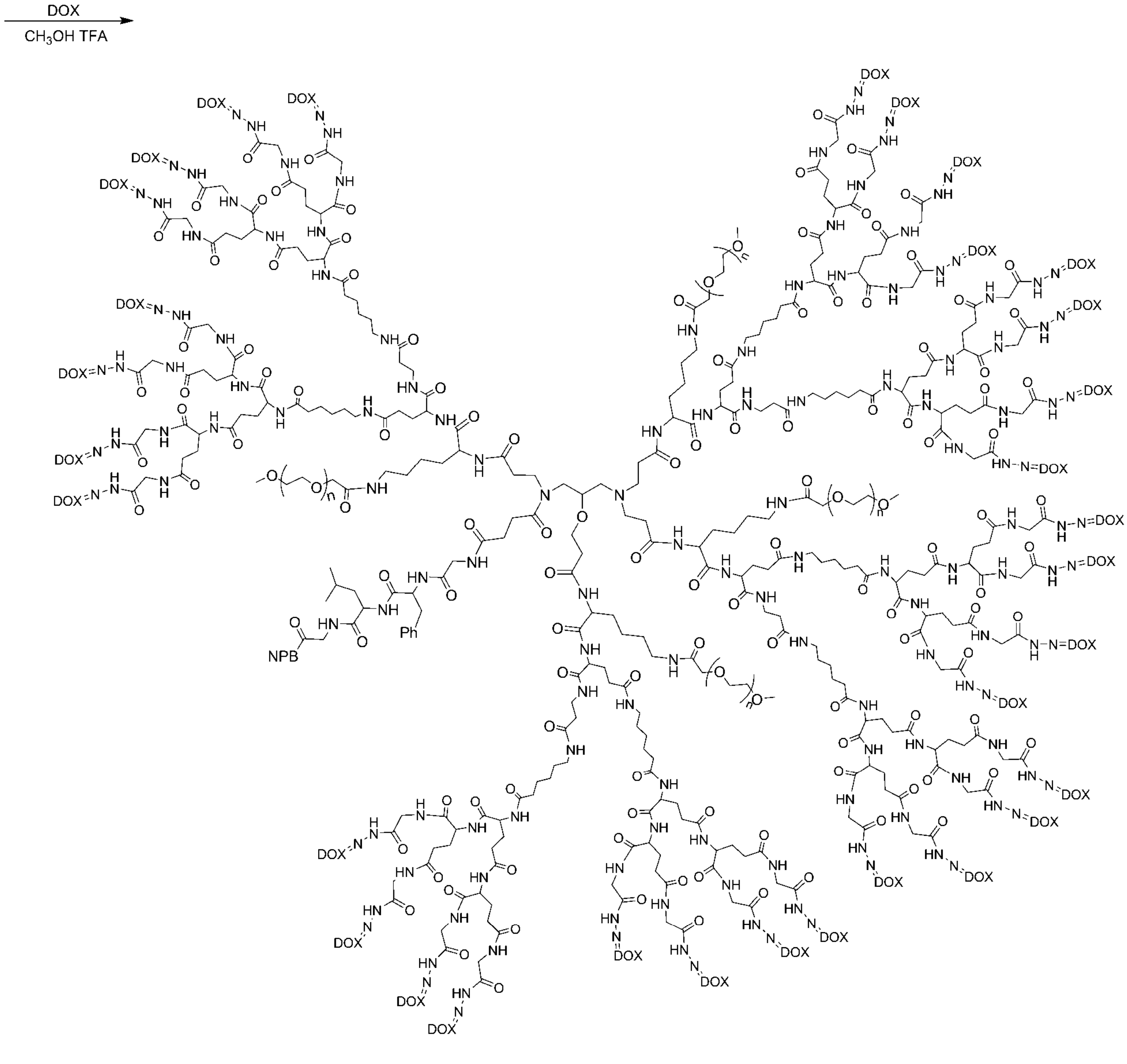


70-261

6.1 Preparation of Compound **70-187**

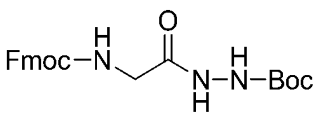

Fmoc-glycine (10 g, 33.64 mmol, purchased from InnoChem), tert-butyloxycarbamoyl-hydrazide (5.78 g, 43.72 mmol) and DCC (6.94 g, 33.64 mmol) were weighed and placed in a flask, then ethyl acetate (30 mL) was added to completely dissolve the mixture, and the resultant mixture was reacted at 0°C for 2 hours. The flask was then taken out and the reaction was carried out under stirring at room temperature. After 4 hours, the reaction was monitored by TLC. After the reaction was completed, saturated NaCl solution (200 mL) and ethyl acetate (200 mL) were added for extraction. After standing for layer separation, the organic phase was collected, and the aqueous phase was extracted with ethyl acetate (3 × 200 mL). The organic phases were combined, concentrated, evaporated to dryness, and dried in a vacuum oven to provide the product.

6.2 Preparation of **70-193**

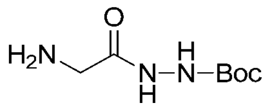

To a flask containing **70-187** (33.64 mmol), THF (40 mL) was added. After dissolution with the assistance of ultrasonication, diethylamine (68 mL, 672.8 mmol) was added, and the reaction was stirred at room temperature for 2 h. After the reaction was completed, the reaction was monitored by TLC. After the reaction was completed, the reaction mixture was evaporated to dryness using a rotavapor, then dissolved by adding dichloromethane (if not completely dissolved) and evaporated again to dryness. This process was repeated five times. Finally, a methanol/dichloromethane mixture was added to the reaction mixture for dissolution, then silica gel powder was added, and the mixture was evaporated to dryness. The resultant product was loaded by dry method, and subjected to column chromatography using 0.8% ammonia/8% methanol/dichloromethane as the eluent. The desired product was collected, and dried to provide 6.3 g of the product with a yield of 86.82%.

6.3 Preparation of **70-196**

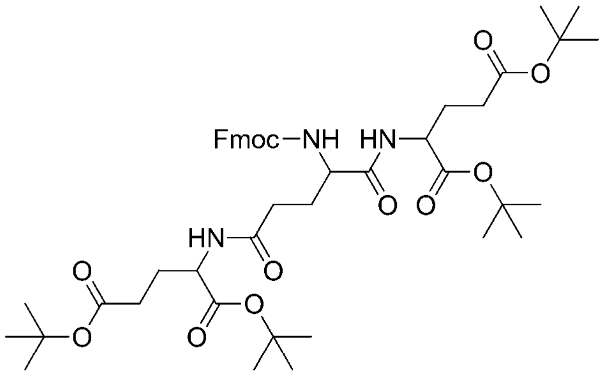

Fmoc-Glu-OH (2.0 g, 5.4 mmol), H-Glu(OtBu)-OtBu.HCl (3.5 g, 11.9 mmol), HOBT (1.8 g, 13.0 mmol) and HBTU (4.9 g, 13.0 mmol) were added to a 500 mL flask, and dissolved by adding DMF (60 mL). The reaction was carried out under stirring at 0°C for approximately 20 minutes. DIEA (4.0 mL, 23.8 mmol) was slowly added dropwise. After the dropwise addition was completed, the reaction mixture was taken out and stirred at room temperature. The reaction was monitored by TLC. After the reaction was completed, the reaction mixture was transferred to a 1 L separatory funnel, and saturated sodium bicarbonate solution (200 mL) and ethyl acetate (300 mL) were added for extraction. The organic phase was separated, and the aqueous phase was further extracted with ethyl acetate (100 mL). The organic phases were combined, and washed with pure water (150 mL), concentrated, and evaporated to dryness to provide 5.9 g of the product (the yield exceeded 100%).

6.4 Preparation of **70-197**

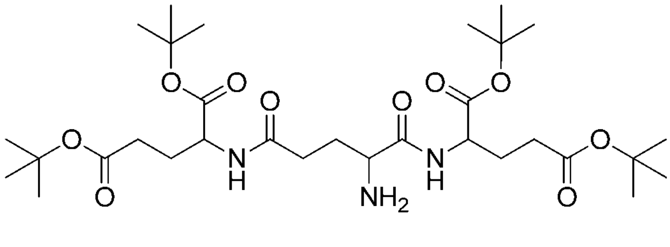

To a flask containing **70-196** (11.9 g, 5.4 mmol), DMF (80 mL) was added. After dissolution with the assistance of ultrasonication, morpholine (9.4 mL, 108 mmol) was added and the reaction was stirred at room temperature for 2 h. After the reaction was completed, the mixture was filtered and saturated sodium bicarbonate solution (200 mL) and ethyl acetate (200 mL) were added for extraction. After standing for layer separation, the organic phase was collected, and the aqueous phase was further extracted with ethyl acetate (200 mL × 3). The organic phases were combined, and evaporated to dryness to provide 4.5 g of the solid product (the yield exceeded 100%).

6.5 Preparation of **70-198**

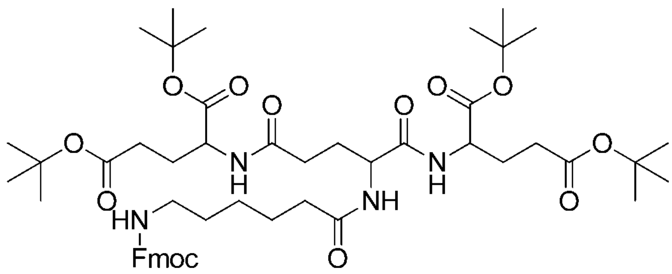

**70-197** (4.5 g, 5.4 mmol), **88-14** (1.9 g, 5.4 mmol), HOBT (0.88 g, 6.5 mmol) and HBTU (2.5 g, 6.5 mmol) were weighed and added to a reaction flask, and DMF (40 mL) was added to dissolve the mixture. The reaction was carried out under stirring at 0°C for approximately 20 minutes. DIEA (2.0 mL, 11.9 mmol) was then slowly added dropwise. After the dropwise addition was completed, the reaction was continued under stirring at 0°C. After the reaction was completed, the reaction mixture was transferred to a 1 L separatory funnel and saturated sodium chloride solution (200 mL) and ethyl acetate (150 mL) were added for extraction. The organic phase was obtained, and the aqueous phase was further extracted with ethyl acetate (200 mL × 2). The organic phases were combined, washed with deionized water (250 mL × 2), concentrated, and evaporated to dryness to provide a solid. The solid was then dissolved in a methanol/dichloromethane (1/4) mixture (100 mL), then silica gel powder (100 mL) was added, and the mixture was evaporated to dryness to provide a powdery solid. The resultant product was loaded by dry method, and subjected to column chromatography using a 0.5-5% methanol/dichloromethane mixture as the eluent. The desired product was collected, concentrated, and dried in a vacuum oven to provide 4.2 g of the product with a yield of 80.75%.

6.6 Preparation of **70-199**

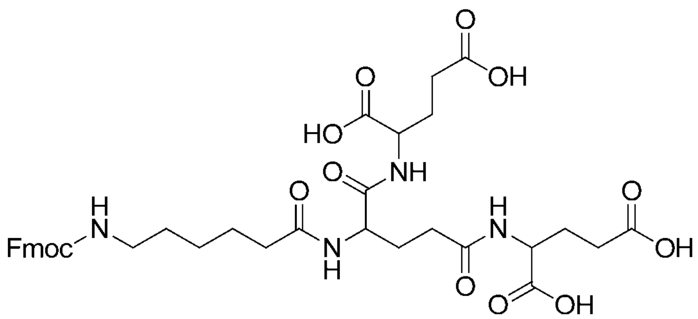

To a flask containing **70-198** (4.2 g, 4.35 mmol) was added dichloromethane (15 mL). After dissolution with the assistance of ultrasonication, TFA (25.9 mL, 348.12 mmol) was added, and the reaction was stirred at room temperature overnight. The reaction progress was monitored by TLC. After the reaction was completed, the reaction mixture was concentrated under reduced pressure to remove dichloromethane and most of the TFA. Then, n-hexane (100 mL) and methyl tert-butyl ether (100 mL) were added for precipitation, resulting in an oily solid. The supernatant was discarded, and ethyl acetate (15 mL) was added, treated by ultrasonication to provide a white turbid liquid, and then n-hexane (100 mL) was added for precipitation, resulting in a powdery solid. The product was filtered, and the filter cake was washed three times with methyl tert-butyl ether (40 mL). The filter cake was collected and dried under vacuum to provide 3.0 g of the product with a yield of 93.8%.

6.7 Preparation of **70-200**

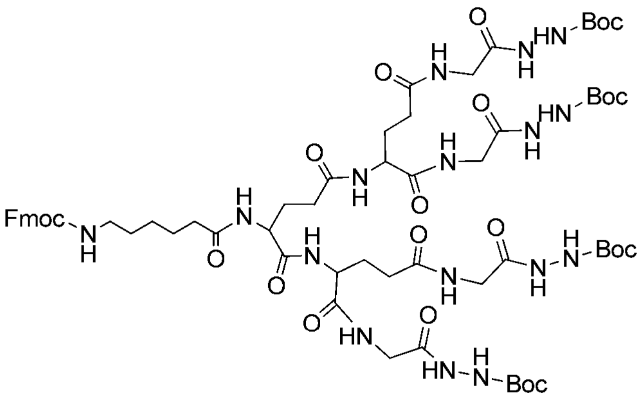

**70-193** (1.2 g, 5.94 mmol), **70-199** (1 g, 1.35 mmol), HBTU (2.5 g, 6.48 mmol) and HOBT (0.9 g, 6.48 mmol) were weighed and added to a 250 mL flask, DMF (30 mL) was added to dissolve the mixture, and the mixture was allowed to stand at -5°C. DIEA (2.0 mL, 11.88 mmol) was slowly added dropwise. After the dropwise addition was completed, the reaction was carried out for half an hour, the flask was then taken out, and the reaction was carried out under stirring at room temperature overnight. The reaction progress was monitored by TLC. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered and dried to provide 2.5 g of solid product.

6.8 Preparation of **70-201**

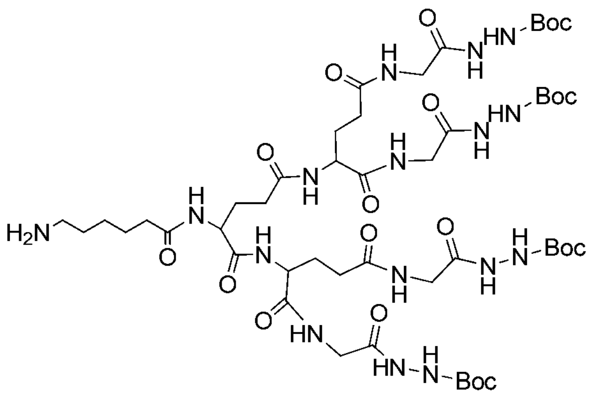

In a flask containing **70-200** (2.5 g, 1.35 mmol), THF (15 mL) was added. After dissolution with the assistance of ultrasonication, diethylamine (2.0 mL, 27 mmol) was added, and the reaction was stirred at room temperature for 2 hours. After the reaction was completed, the reaction mixture was evaporated to dryness. The resulting solid product was dissolved in dichloromethane and methanol, loaded by dry method, and subjected to column chromatography using 5-12% methanol/dichloromethane as the eluent. The desired product was collected, concentrated, and dried to provide 0.9 g of the product in a yield of 56.25%. $^1$H-NMR (600 MHz, DMSO-$d_6$) δ 8.29 (s, 3H), 8.14-8.06 (m, 5H), 8.05-7.99 (m, 2H), 7.95 (s, 5H), 5.13 (s, 2H), 4.28-4.23 (m, 2H), 4.19-4.15 (m, 1H), 3.76-3.67 (m, 8H), 2.65-2.60 (m, 2H), 2.20-2.16 (m, 6H), 1.94-1.83 (m, 4H), 1.80-1.68 (m, 4H), 1.43-1.53 (m, 4H), 1.39 (s, 36H), 1.29-1.22 (m, 2H); ESI [M+H$^+$] 1203.628

6.9 Preparation of **71-222**

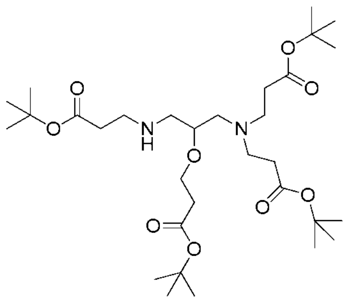

1,3-Diamino-2-hydroxypropane (6 g, 66.5778 mmol) was weighed and added into a 250 mL flask, then DMSO (7 mL) was added for dissolution, and the solution was stirred at 15°C under nitrogen protection. 5 M aqueous sodium hydroxide solution (0.7 mL) was then added dropwise. After the dropwise addition was completed, the mixture was stirred for 10 minutes. Tert-butyl acrylate (38.6 mL, 266.3112 mmol) was then added dropwise. After the dropwise addition was completed, the mixture was stirred for 20 minutes. The reaction flask was taken out, and the reaction was carried out under stirring at room temperature for 24 hours. After the reaction was completed, the reaction mixture was transferred to a 1 L separatory funnel, and saturated sodium chloride solution (200 mL) and ethyl acetate (150 mL) were added for extraction. The organic phase was obtained, and the aqueous phase was further extracted with ethyl acetate (200 mL × 2). The organic phases were combined, and washed with deionized water (250 mL × 2), concentrated, evaporated to dryness, then dissolved in methanol/dichloromethane (1/4) mixture (50 mL). Silica gel powder (100 mL) was added, and the mixture was evaporated to dryness to provide a powdery solid. The resultant product was loaded by dry method, and subjected to column chromatography using a 0-2% methanol/dichloromethane mixture as the eluent. The desired product was collected, concentrated, and dried in a vacuum oven to provide 1.0 g of the product. $^1$H-NMR (600 MHz, DMSO-*d*$_6$) δ 3.68-3.58 (m, 2H), 3.37-3.34 (m, 1H), 2.73-2.63 (m, 4H), 2.62-2.56 (m, 3H), 2.47-2.34 (m, 6H), 2.29 (d, 6.6 Hz, 6H), 1.39 (d 36H)

6.10 Preparation of **70-185**

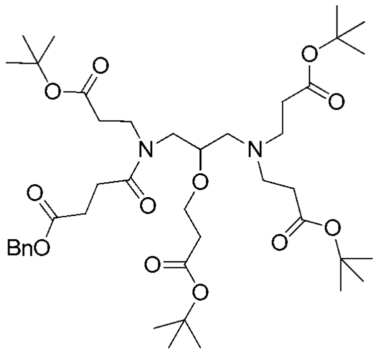

**71-222** (1.2 g, 2.0 mmol), monobenzyl succinate (0.46 g, 2.2 mmol) and HATU (0.92 g, 2.4 mmol) were placed in a 250 mL flask, DMF (20 mL) was added to dissolve the mixture. The flask was allowed to stand at -5°C, and the mixture was stirred for approximately 20 minutes. DIEA (1.0 mL, 4.4 mmol) was then slowly added dropwise. After the addition was completed, the mixture was continuously stirred at -5°C for 20 minutes, and then allowed to react under stirring at room temperature. After the reaction was completed, saturated NaCl solution (100 mL) and ethyl acetate (100 mL) were added for extraction. After standing for layer separation, the organic phase was collected, the aqueous phase was further extracted with ethyl acetate (200 mL × 3). The organic phases were combined, evaporated to dryness, and then dissolved in the mixed solvent. Silica gel powder was added, and the mixture was evaporated to dryness. The resultant product was loaded by dry method, and subjected to column chromatography using 30% ethyl acetate/petroleum ether as the eluent. The product was collected, concentrated, and dried to provide 1.3 g of the product with a yield of 85.44%. $^1$H-NMR (600 MHz, DMSO-*d*$_6$) δ 7.38-7.31 (m, 5H), 5.06 (s, 2H), 3.74-3.44 (m, 5H), 3.38-3.35 (m, 2H), 3.17 (s, 1H), 2.70-2.56 (m, 8H), 2.41-2.26 (m, 9H), 1.40-1.37 (m, 36H)

6.11 Preparation of **70-186**

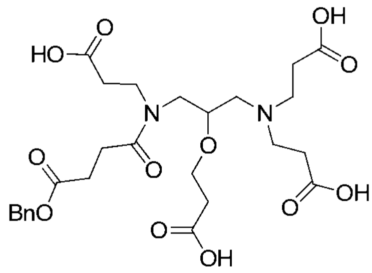

To a flask containing **70-185** (0.3 g, 0.38 mmol) was added dichloromethane (5 mL). After dissolution with the assistance of ultrasonication, TFA (4.9 mL, 66.356 mmol) was added, and the reaction was stirred at room temperature. After the reaction was completed, the reaction mixture was concentrated to remove dichloromethane and most of the TFA. Then, n-hexane (200 mL) and methyl tert-butyl ether (200 mL) were added for precipitation. The supernatant was discarded to obtain an oily solid. Ethyl acetate (15 mL) was added to the solid, and the mixture was treated by ultrasonication to obtain a white turbid solution. Then, n-hexane (200 mL) was added for precipitation to obtain a powdery solid, and filtered. The filter cake was washed three times with methyl tert-butyl ether (40 mL). The filter cake was collected and dried under vacuum to provide 0.16 g of the product.

6.12 Preparation of **70-204**

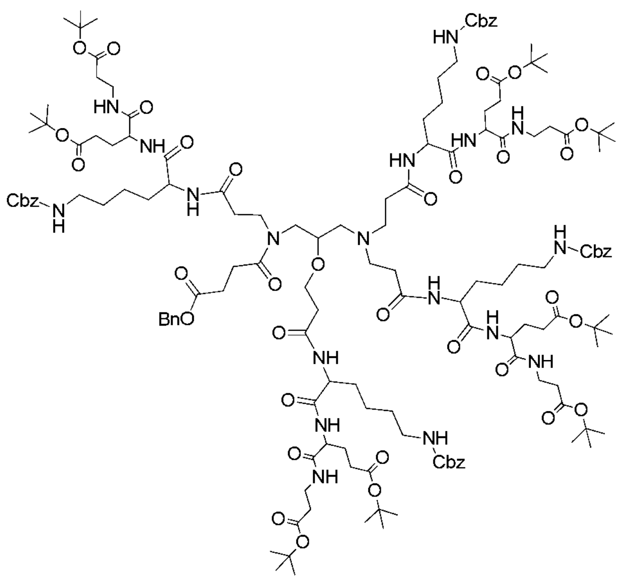

**70-186** (0.16 g, 0.28 mmol), **76-58** (0.73 g, 1.24 mmol), HBTU (0.64 g, 1.69 mmol) and HOBT (0.23 g, 1.69 mmol) were added to a 500 mL flask, dissolved with DMF (60 mL), and then the reaction was stirred at 0°C for approximately 20 minutes. DIEA (20 mL, 121.8290 mmol) was slowly added dropwise. After the dropwise addition was completed, the reaction was continued under stirring at 0°C. After the reaction was completed, the reaction mixture was transferred to a 1 L separatory funnel, and saturated sodium chloride solution (100 mL) and ethyl acetate (150 mL) were added for extraction to obtain the organic phase. The aqueous phase was further extracted with ethyl acetate (100 mL × 2). The organic phases were combined, washed with deionized water (150 mL × 2), concentrated, evaporated to dryness, and then dissolved in methanol/dichloromethane (1/4) mixture (50 mL). Silica gel powder (10 mL) was added, and the mixture was evaporated to dryness to provide a powdery solid. The resultant product was loaded by dry method, and subjected to column chromatography using a 0.3% ammonia solution/0-4% methanol/dichloromethane mixture as the eluent. The desired product was collected, concentrated, and dried in a vacuum oven to provide 0.5 g of the product. $^1$H-NMR (600 MHz, DMSO-$d_6$) δ 8.03-7.86 (m, 12H), 7.40-7.30 (m, *J* = 6.6, 25H), 7.22-7.15 (m, 5H), 5.08-5.04 (m, 2H), 5.01-4.96 (m, 8H),

4.34-4.09 (m, 10H), 4.06-3.95 (m, 2H), 3.62-3.46 (m, 6H), 3.22-3.10 (m, 6H), 3.05-2.90 (m, 12H), 2.86-2.80 (m, 2H), 2.73-2.63 (m, 6H), 2.62-2.61 (m, 2H), 2.39-2.38 (m, 2H), 2.36-2.30 (m, 14H), 2.21-2.11 (m, 10H), 1.88-1.79 (m, 6H), 1.75-1.66 (m, 6H), 1.64-1.55 (m, 6H), 1.50-1.44 (m, *J* = 20.1, 6H), 1.38-1.37 (m, 32H), 1.37-1.36 (m, 32H), 1.26-1.23 (m, 8H); ESI [M+H$^+$] 2867.570

6.13 Preparation of **70-208**

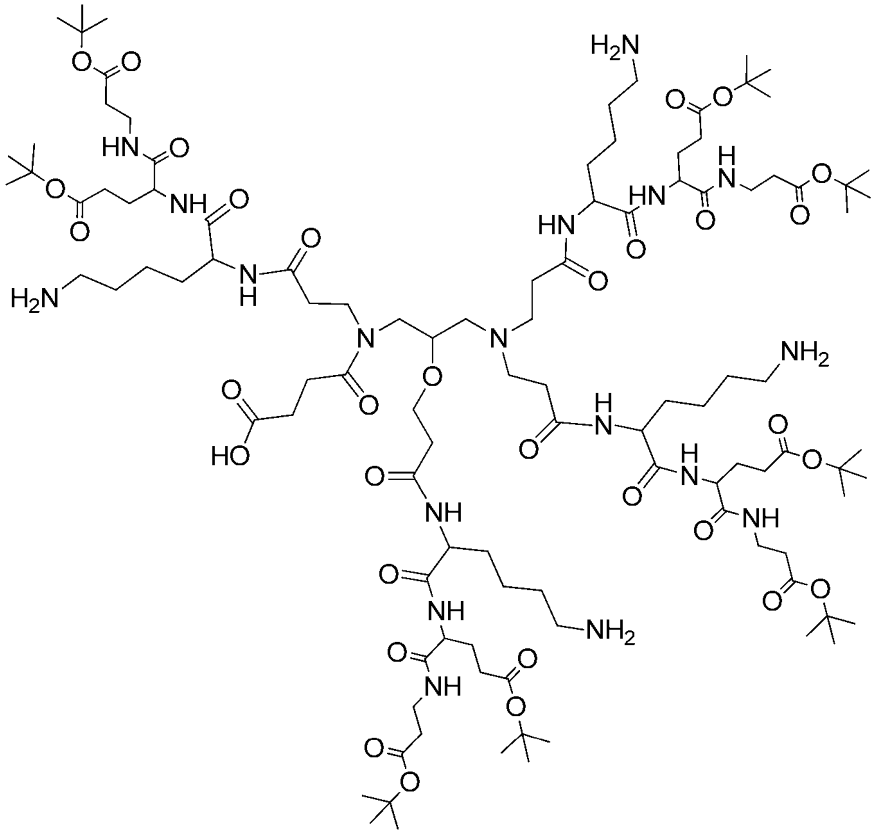

**70-204** (0.3 g, 0.105 mmol) was weighed and added to a microhydrogenation reactor, DMF (20 mL) was added for dissolution, and then 10% Pd/C catalyst (0.1 mg) was added. The raw material on the reactor wall were flushed down using a rubber-tipped pipette, and a stirring magnet was added. The reactor was then sealed and evacuated with a water pump. $H_2$ was introduced, and then evacuation was performed. This process was repeated three times. Finally, $H_2$ was introduced to stabilize the hydrogenation reactor pressure at 2 MPa. The reaction was carried out under stirring overnight and monitored by TLC. After the reaction was completed, the reaction mixture was filtered through Celite, and the filtrate was collected for later use. A portion of the filtrate was dried and then characterized. $^1$H-NMR (600 MHz, DMSO-*d*$_6$) $\delta$ 8.09-7.83 (m, 12H), 7.38-7.24 (m, 4H), 5.02-4.89 (m, 2H), 4.22-4.11 (m, 8H), 3.55-3.50 (m, 12H), 3.19-3.13 (m, 12H), 3.07-3.01 (m, 6H), 2.91-2.86 (m, 4H), 2.74-2.73 (m, 2H), 2.71-2.69 (m, 2H), 2.63-2.60 (m, 2H), 2.39-2.38 (m, 2H), 2.38-2.32 (m, 12H), 2.21-2.10 (m, 14H), 1.87-1.79 (m, 6H), 1.75-1.67 (m, 6H), 1.64-1.55 (m, 6H), 1.51-1.45 (m, 6H), 1.40-1.34 (m, 72H); ESI [M+Na$^+$] 2269.371

6.14 Preparation of **70-246**

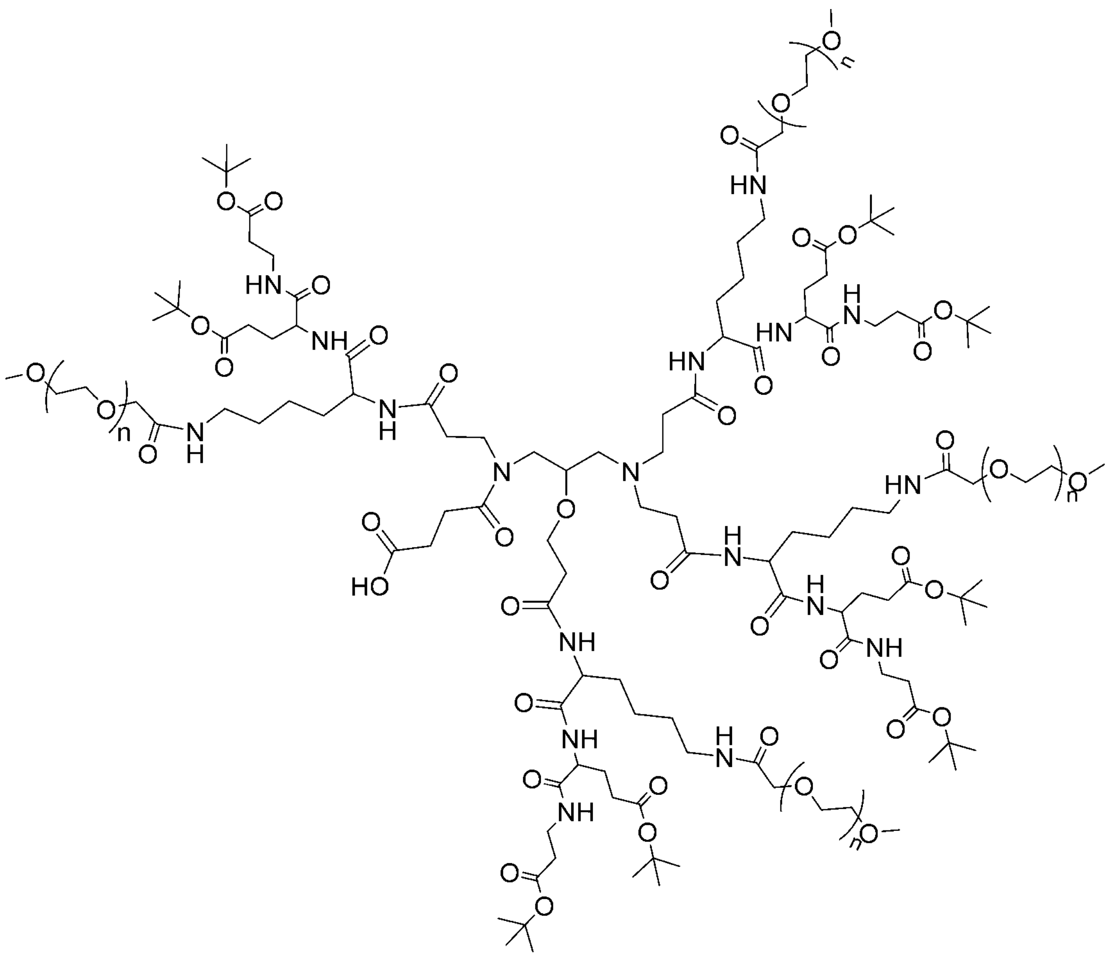

The filtrate **70-208** (0.066 mmol) was taken, then DMF (15 mL) and DIEA (0.5 mL, 2.64 mmol) were added. The mixture was stirred for 30 minutes, then M-SCM-10K (2.90 g, 0.27 mmol, purchased from Jenkem) was added. The reaction was carried out under stirring in the dark at a low speed at room temperature. After two days, the reaction progress was monitored by TLC. After the reaction was completed, methyl tert-butyl ether/n-hexane were added for precipitation, the mixture was filtered using a filtrate funnel to collect a filter cake, and the filter cake was rinsed twice with methyl tert-butyl ether and transferred to a flask, and dried to provide 2.8 g of the product with a yield of 94.11%.

6.15 Preparation of **70-250**

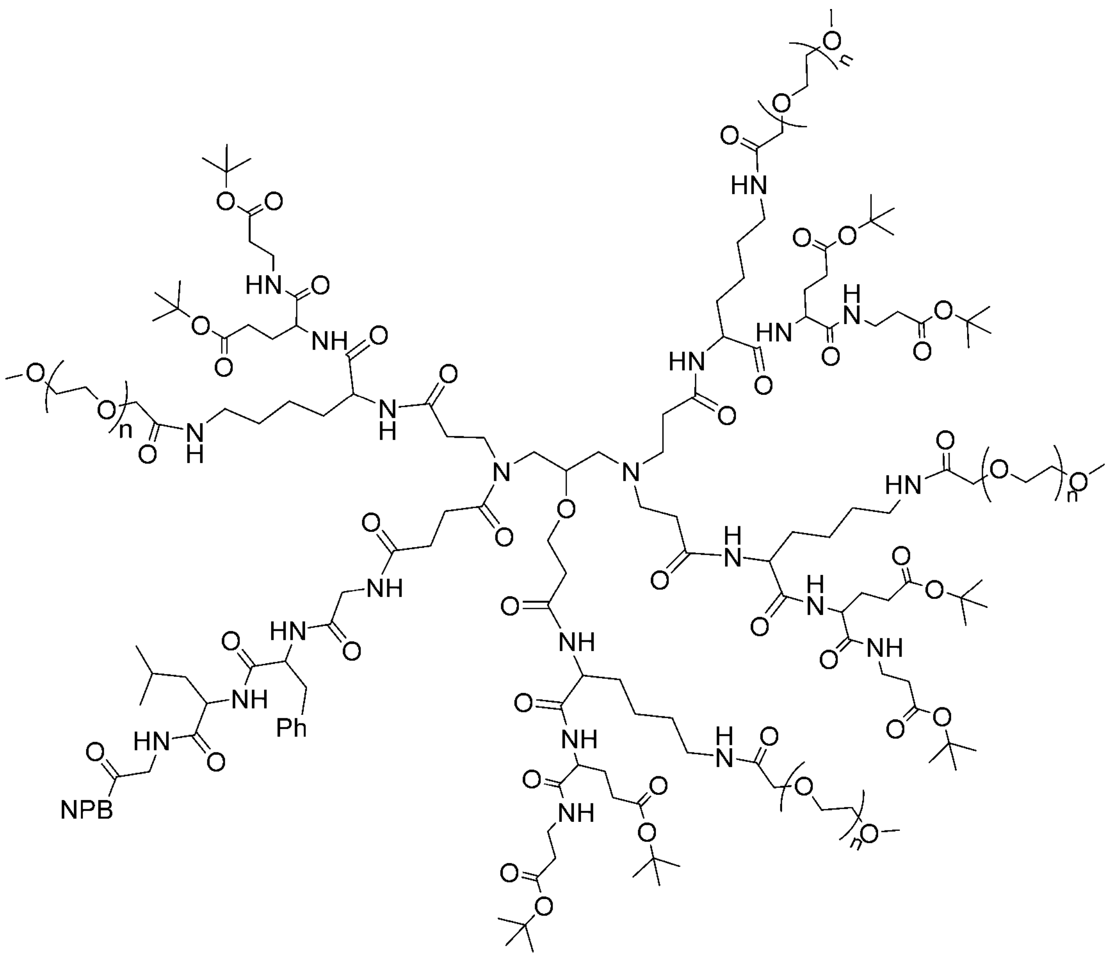

Reactants **70-246** (7.82.8 g, 0.64 mmol), **70-68** (70.06 g, 0.080 mmol), HOBT (12 g, 0.9 mmol) and HBTU (0.34 g, 0.9 mmol) were placed in a reaction flask, an appropriate amount of DMF was added to dissolve with the assistance of ultrasonication. The mixture was stirred at 0°C and DIEA (0.7 mmol, 4.4 mmol) was added dropwise. After the dropwise addition was completed, the mixture was brought to room temperature and the reaction was stirred for 1 hour. The reaction progress was monitored by TLC. After the reaction was completed, methyl tert-butyl ether/n-hexane were added for precipitation, the mixture was filtered with a fritted funnel, and the filter cake was collected. The previous step was repeated twice. The filter cake was transferred to a flask, dissolved by adding the mixed solvent. Silica gel powder was added, and the mixture was evaporated to dryness. The resultant product was loaded by dry method, and subjected to column chromatography using 1% ammonia water/8% methanol/dichloromethane as the eluent. The desired product was collected and dried to provide 2.1 g of the product with a yield of 70.71%. $^1$H NMR (600 MHz, DMSO-$d_6$) δ 8.20-7.81 (m, 12H), 7.71-7.63 (m, 2H), 7.41-7.21 (m, 1H), 6.62-6.54 (m, 1H), 4.25-4.15 (m, 8H), 3.63-3.63 (m, 8H), 3.51-3.50 (m, 3828H), 3.24-3.24 (m, 12H), 3.20-3.19 (m, 2H), 3.18-3.16 (m, 3H), 3.16-3.15 (m, 2H), 3.15-3.14 (m, 2H), 3.12-3.11 (m, 1H), 3.09-3.04 (m, 7H), 2.63-2.61 (m, 6H), 2.60-2.58 (m, 4H), 2.41-2.37 (m, 6H), 2.37-2.32 (m, 12H), 1.88-1.78 (m, 6H), 1.74-1.65 (m, 6H), 1.64-1.54 (m, 6H), 1.53-1.45 (m, 6H), 1.40-1.36 (m, 72H), 1.24-1.21 (m, 39H), 0.91-0.88 (m, 3H), 0.86-0.83 (m, 3H)

6.16 Preparation of **70-251**

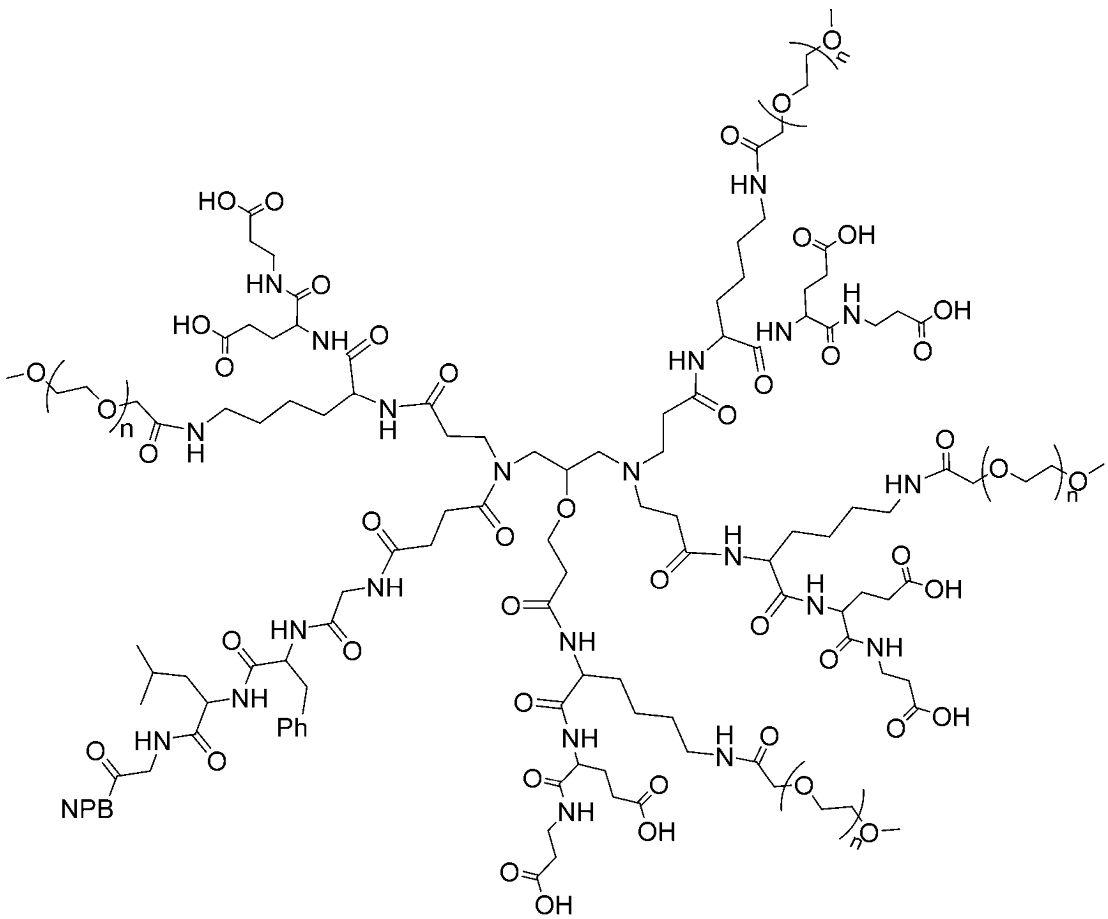

Reactant **70-250** (0.044 mmol) was taken and dissolved in dichloromethane (20 mL), then TFA (35 mL, 472 mmol) was added, and the mixture was reacted under stirring at room temperature. The reaction progress was monitored by TLC. After the reaction was completed, the reaction mixture was concentrated using a rotary evaporator, tert-butyl ether/n-hexane were added for precipitation, the mixture was filtered using a fritted funnel to collect a filter cake, which was washed twice with methyl tert-butyl ether and dried to provide 1.68 g of the product with a yield of 85.81%.

6.17 Preparation of **70-256**

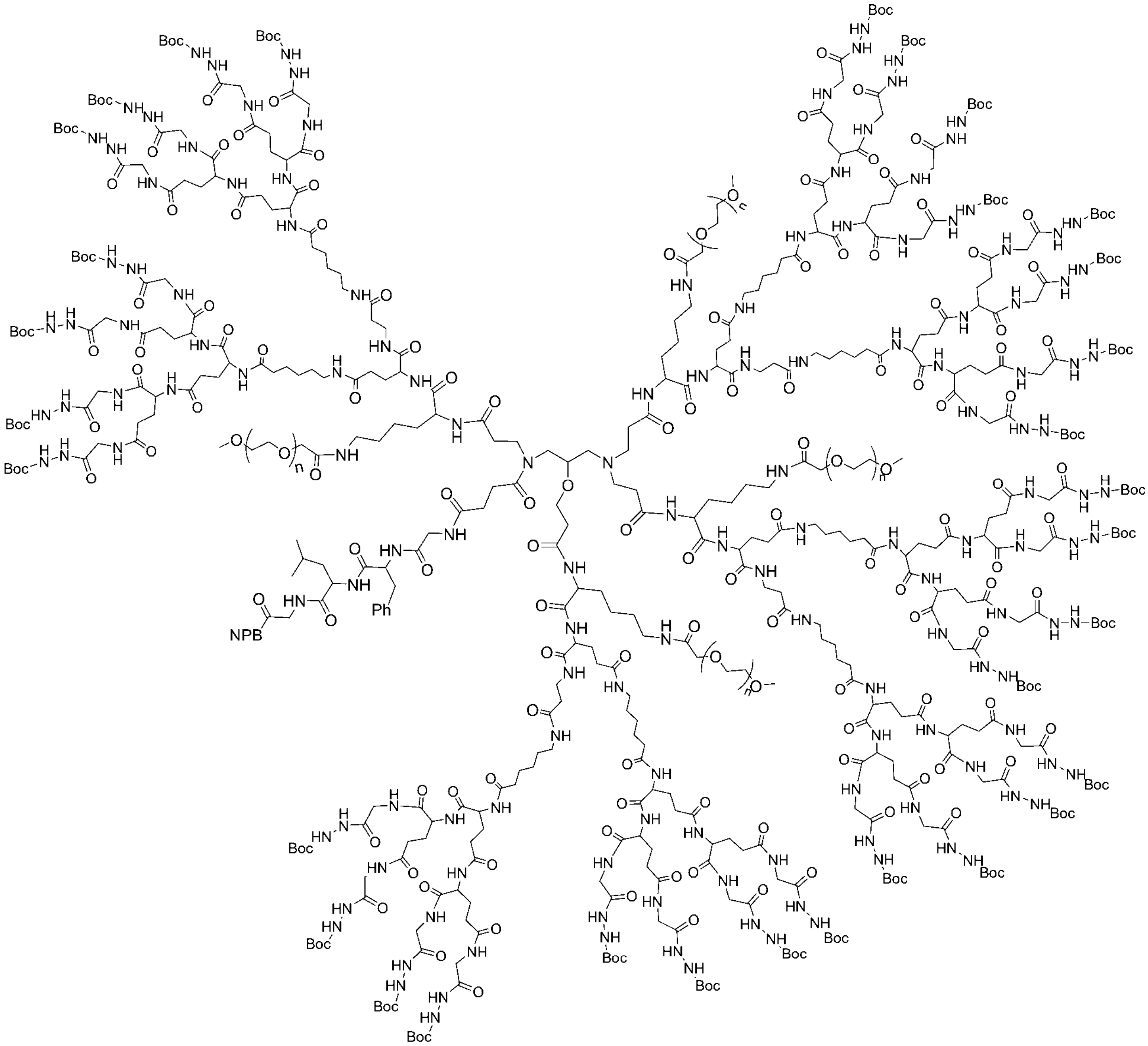

Reactants **70-251** (1.68 g, 0.0378 mmol), **70-201** (2.25 g, 1.87 mmol), HOBT (30 mg, 0.21 mmol) and HBTU (80 mg, 0.21 mmol) were placed in a reaction flask, an appropriate amount of DMF was added to dissolve with the assistance of ultrasonication, and the mixture was stirred at 0°C. DIEA (0.1 mL, 0.4 mmol) was added dropwise. After the dropwise addition was completed, the mixture was brought to room temperature and reacted under stirring for 1 hour. The reaction progress was monitored by TLC. After the reaction was completed, methyl tert-butyl ether/n-hexane were added for precipitation, and the mixture was filtered using a fritted funnel to collect a filter cake, which was then rinsed twice with methyl tert-butyl ether, and dried to provide 1.69 g of product.

6.18 Preparation of **70-257**

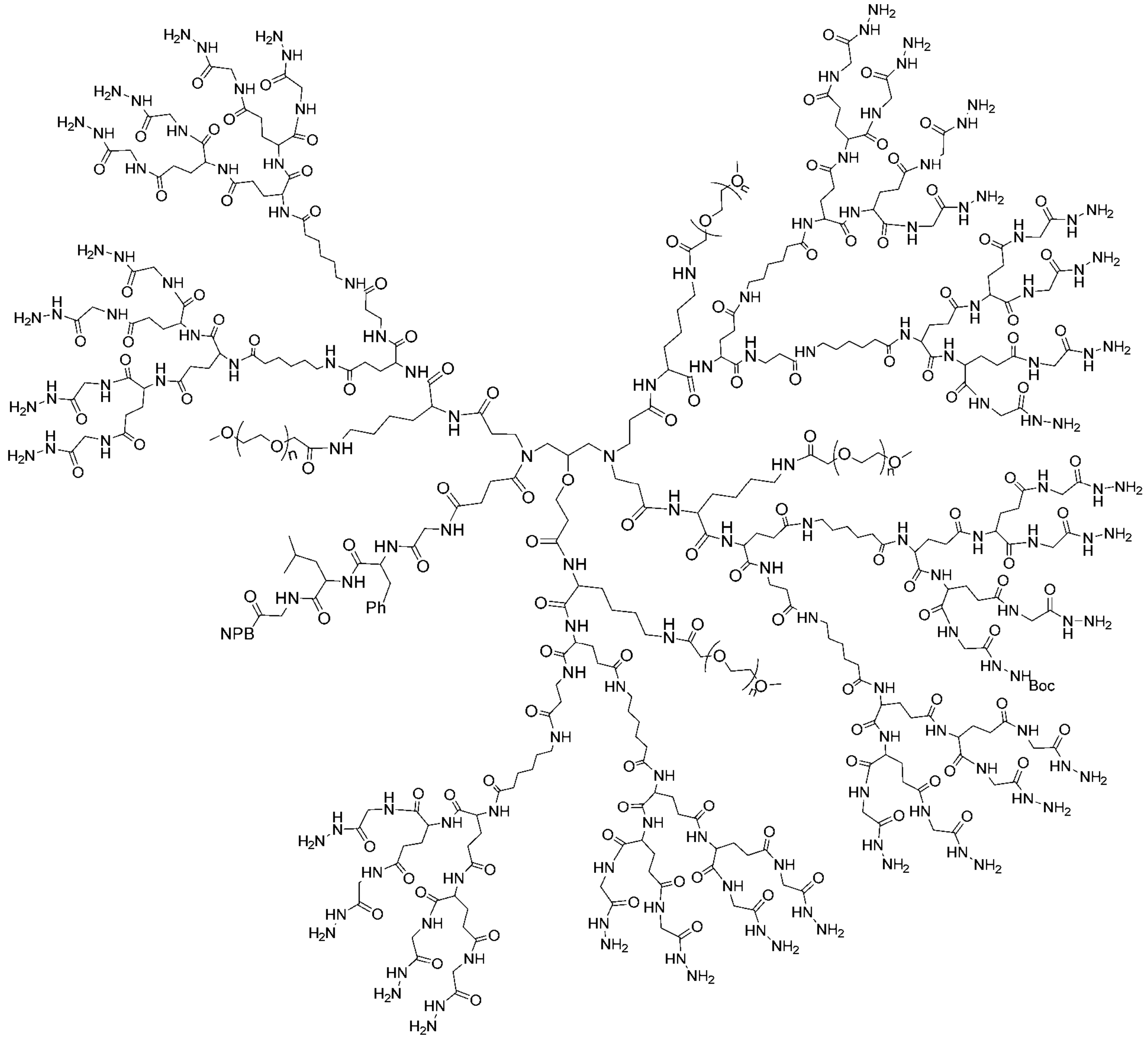

Reactant **70-256** (1.69 g, 0.31 mmol) was taken and dissolved by adding TFA (20 mL), and the reaction was stirred at room temperature. The reaction progress was monitored by TLC. After the reaction was completed, the mixture was evaporated to dryness using a rotary evaporator, methyl tert-butyl ether/n-hexane were added for precipitation, and the mixture was filtered using a fritted funnel to collect a filter cake, which was washed twice with methyl tert-butyl ether and dried in an oven to provide 1.49 g of the product.

6.19 Preparation of **70-261**

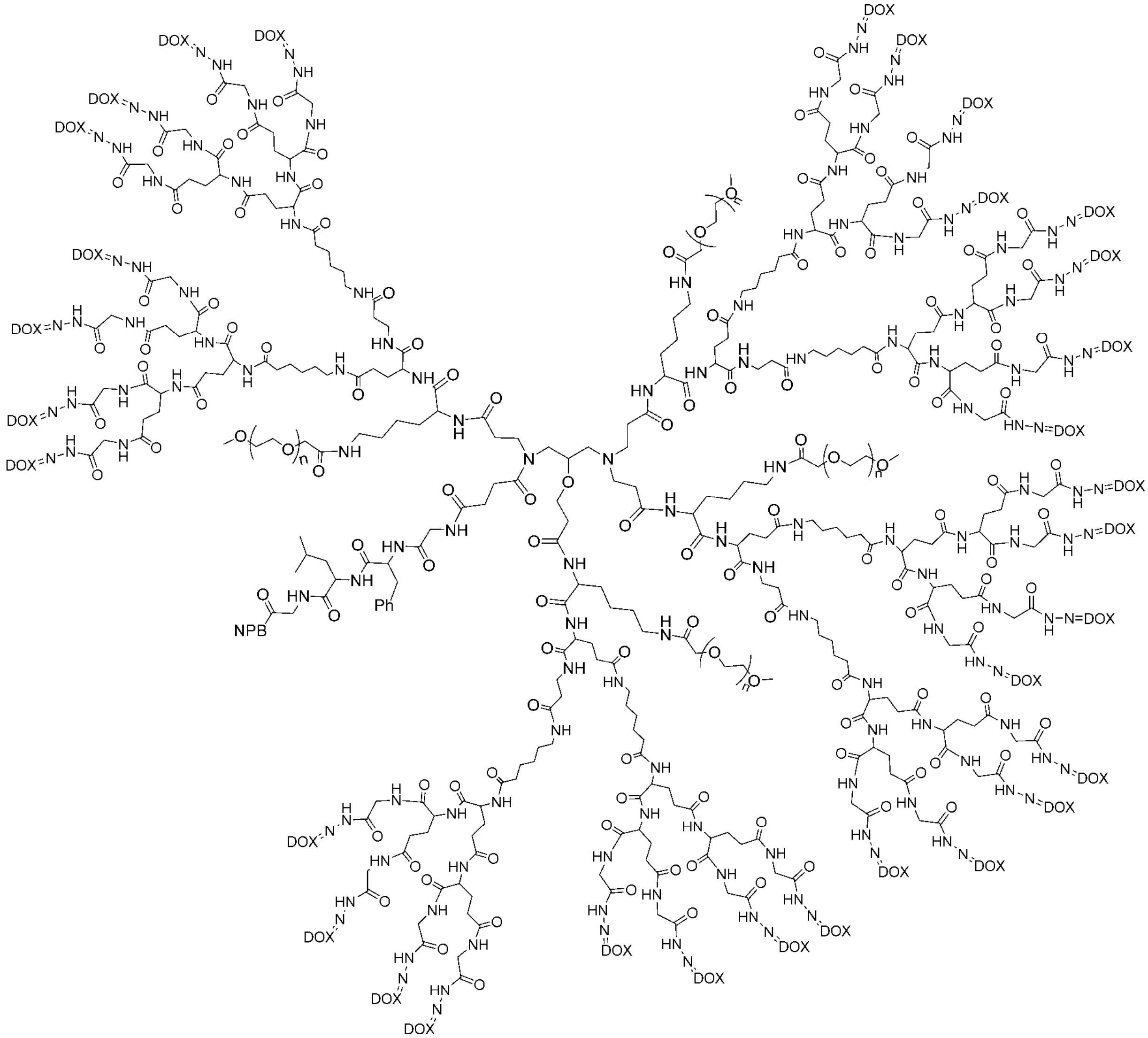

Reactant **70-257** (1.49 g, 0.029 mmol) was taken and dissolved by adding methanol (20 mL), then TFA (0.1 mL, 1.173 mmol) and doxorubicin hydrochloride (0.68 g, 1.173 mmol) were added, and the mixture was reacted under stirring at low speed at room temperature in the dark. The reaction progress was monitored by TLC. After the reaction was completed, the mixture was evaporated to dryness using a rotary evaporator, methyl tert-butyl ether/n-hexane were added for precipitation, and the mixture was filtered using a fritted funnel to collect a filter cake, which was washed twice with methyl tert-butyl ether, and the resultant filter cake was collected and dried to provide 0.26 g of the product. $^1$H-NMR (600 MHz, DMSO-$d_6$) δ 9.32-9.29 (m, 1H), 8.58-8.56 (m, 1H), 8.53-8.50 (m, 1H), 8.43-8.40 (m, 1H), 8.23 - 8.19 (m, 15H), 8.18-8.09 (m, 25H), 8.08-7.97 (m, 45H), 7.97-7.90 (m, 96H), 7.90-7.84 (m, 16H), 7.84-7.72 (m, 40H), 7.72-7.65 (m, 64H), 7.62-7.50 (m, 34H), 7.29-7.27 (m, 5H), 7.26-7.26 (m, 1H), 5.71-5.32 (m, 64H), 5.33-5.27 (m, 32H), 5.01-4.93 (m, 64H), 4.61-4.56 (m, 64H), 4.51-4.32 (m, 18H), 4.25-4.14 (m, 64H), 4.13-4.09 (m, 8H), 4.02-3.98 (m, 99H), 3.91-3.61 (m, 70H), 3.60-3.57 (m, 65H), 3.52- 3.50 (m, 3828H), 3.24-3.24 (m, 6H), 3.10-3.08 (m, 2H), 3.03-3.01 (m, 12H), 3.01-2.97 (m, 32H), 2.93-2.87 (m, 32H), 2.80-2.75 (m, 8H), 2.72-2.68 (m, 3H), 2.65-2.60 (m, 16H), 2.44-2.35 (m, 16H), 2.27-2.15 (m, 48H), 2.15-2.12 (m, 42H), 2.12-2.08 (m, 32H), 2.05-1.94 (m, 32H), 1.93-1.85 (m, 34H), 1.84-1.74 (m, 24H), 1.71-1.66 (m, 43H), 1.62-1.58 (m, 16H), 1.56-1.47 (m, 32H), 1.24-1.21 (m, 20H), 1.17-1.13 (m, 96H), 1.12-1.10 (m, 1H), 0.85-0.83 (m, 6H)

Example 7: Synthesis of Compound **86-43**

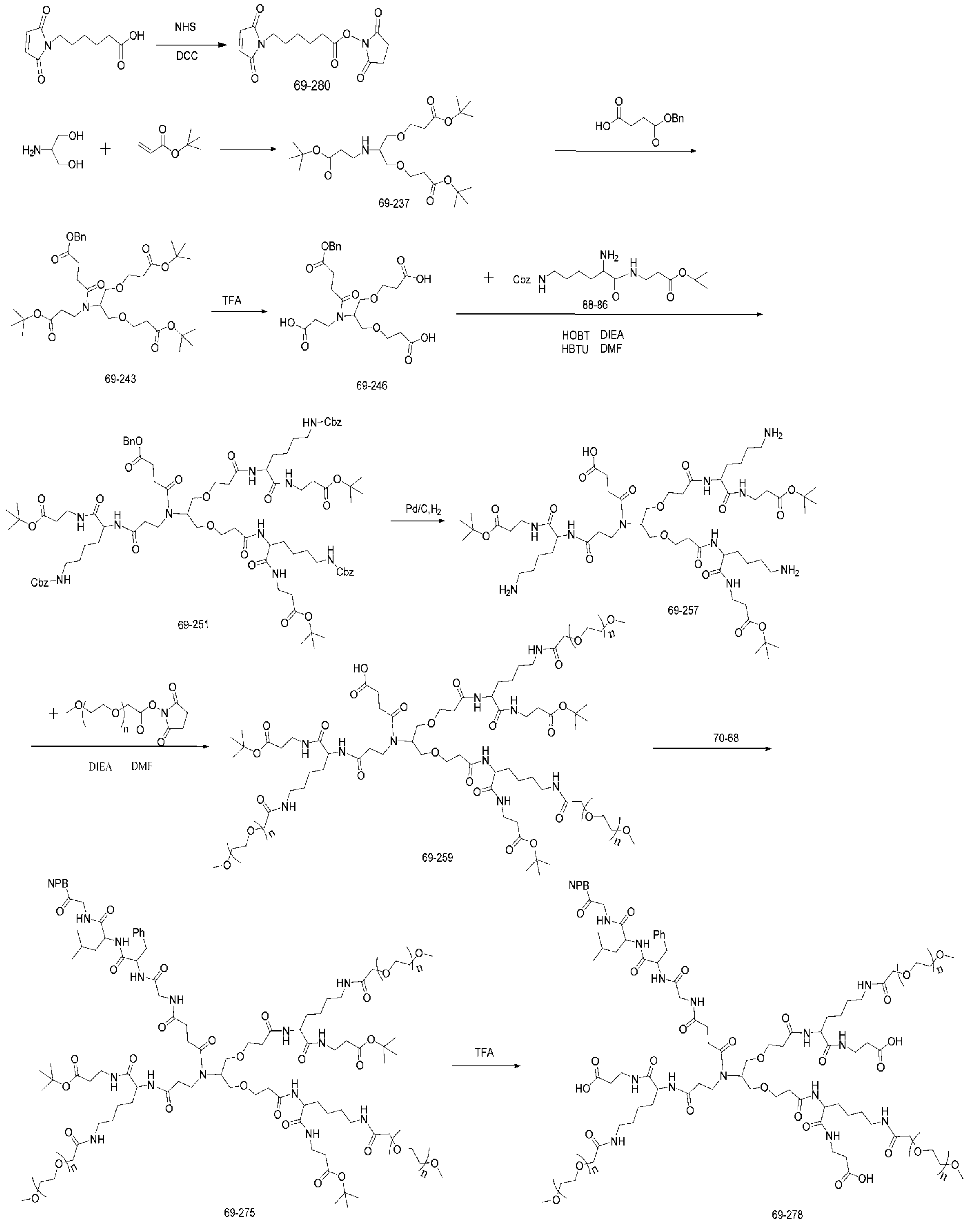
NHS
DCC
69-280
69-237
TFA
69-243
69-246
88-86
HOBT DIEA
HBTU DMF
Pd/C,$H_2$
69-251
69-257
DIEA DMF
69-259
70-68
TFA
69-275
69-278

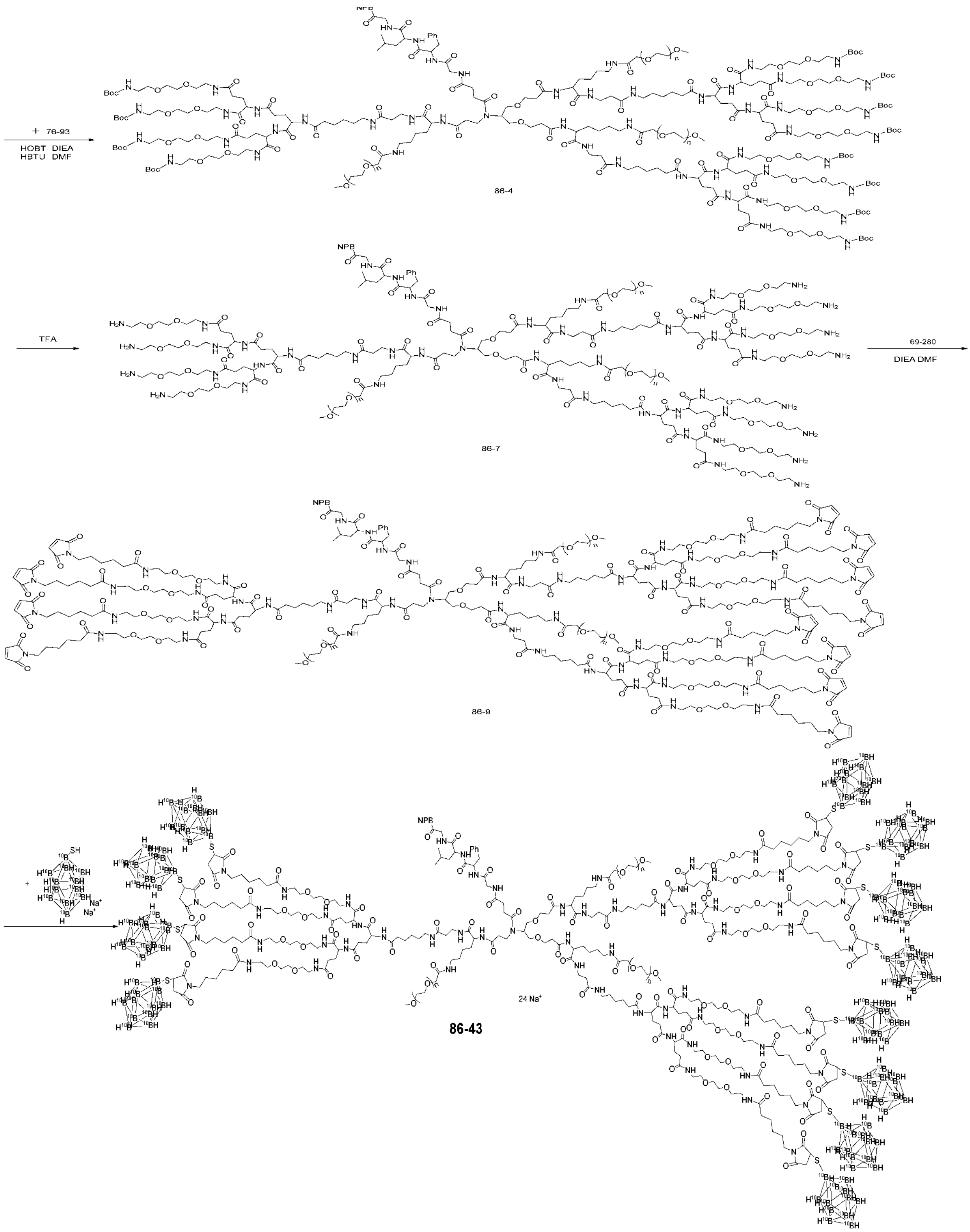


86-4

86-7

86-9

86-43

7.1 Preparation of **69-280**

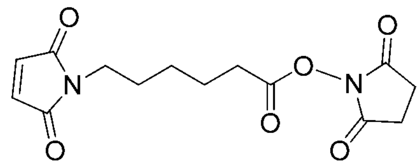

5-Maleimidocaproic acid (5 g, 23.67 mmol) was weighed and placed in a 500 mL reaction flask, then NHS (3.59 g, 31.24 mmol) was added, and the mixture was dissolved in DMF (30 mL). Then DCC (6.25 g, 30.30 mmol) was added, and the mixture was reacted under stirring at room temperature overnight. After the reaction was completed, the white solid was removed by filtration. N-hexane (200 mL) was added to the resultant crude product. The mixture was treated by ultrasonication, allowed to stand for 1 hour, and the supernatant was discarded. The process of adding n-hexane (200 mL), treating by ultrasonication, allowing to stand for 1 hour, and discarding the supernatant was repeated several times until the product become a viscous oil, which was dried to provide 4.86 g of the product. $^1$H-NMR (600 MHz, DMSO-$d_6$) δ 7.01 (s, 2H), 3.40-3.36 (m, 2H), 2.87-2.73 (m, 4H), 2.65 (t, *J* = 7.3 Hz, 2H), 1.66-1.58 (m, 2H), 1.50 (tt, *J* = 14.7, 7.4 Hz, 2H), 1.35-1.26 (m, 2H); FT MS ESI m/z [M+H$^+$] 309.10

7.2 Preparation of **69-237**

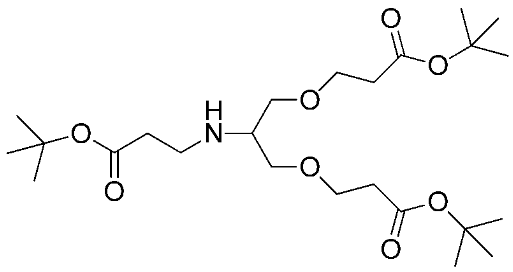

2-Amino-1,3-propanediol (3.644 g, 40.00 mmol) was weighed and placed into a reaction flask, DMSO (3 mL) was added for dissolution, purged with nitrogen gas for protection, and stirred at 15°C. 5 mol/L NaOH solution (0.4 mL) and tert-butyl acrylate (13.16 mL, 90.6668 mmol) were added dropwise, and the reaction was carried out at room temperature for 24 hours. After the reaction was completed, deionized water (300 mL) and ethyl acetate (300 mL) were added for extraction. The organic phase was collected, and the aqueous phase was further extracted with ethyl acetate (300 mL × 2). The organic phases were combined, evaporated to dryness, then dissolved in an appropriate amount of a methanol/dichloromethane mixture, loaded by dry method, and subjected to column chromatography using a 20% ethyl acetate/n-hexane mixture as the eluent. The desired product was collected, concentrated, and dried to provide 2.85 g of the product. $^1$H-NMR (600 MHz, DMSO-$d_6$) δ 3.55 (t, *J* = 6.1 Hz, 4H), 3.29 (dd, *J* = 10.2, 5.7 Hz, 4H), 3.27(m, 1H), 2.74 (dd, *J* = 10.9, 4.2 Hz, 2H), 2.40 (t, *J* = 6.1 Hz, 4H), 2.28 (t, *J* = 6.6 Hz, 2H), 1.91 (s, 1H), 1.43-1.34 (m, 27H); FT MS ESI m/z [M+H$^+$] 476.32

7.3 Preparation of **69-243**

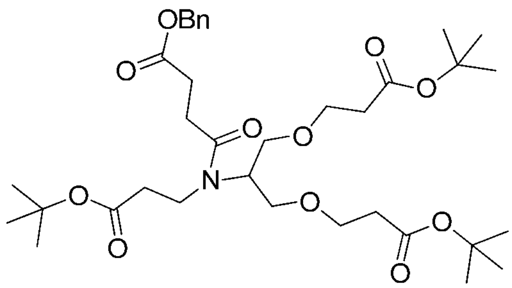

**69-237** (0.7 g, 1.471 mmol), benzyl succinate (0.337 g, 1.618 mmol), HOBT (0.298 g, 2.2065 mmol) and HBTU (0.836 g, 2.2065 mmol) were weighed and placed into a 250 mL reaction flask, DMF (10 mL) was added for dissolution. DIEA (1.094 mL, 6.6195 mmol) was added dropwise, and the reaction was stirred at room temperature. After the reaction

was completed, deionized water (300 mL) and ethyl acetate (300 mL) were added for extraction. The organic phase was collected, and the aqueous phase was further extracted with ethyl acetate (300 mL × 2). The organic phases were combined and concentrated to obtain a crude product, which was dissolved, then 200-300 mesh silica gel powder was added, and the mixture was evaporated to dryness. The resultant product was loaded by dry method, and subjected to column chromatography using a 20% ethyl acetate/n-hexane mixture as the eluent. The desired product was collected, concentrated and dried in a vacuum oven to provide 0.83 g of the product. $^1$H-NMR (600 MHz, DMSO-$d_6$) δ 7.41-7.25 (m, 5H), 5.08 (s, 2H), 4.14-4.02 (m, 1H), 3.63-3.42 (m, 10H), 2.60 (t, $J$ = 6.5 Hz, 2H), 2.57 (dd, $J$ = 6.6, 4.8 Hz, 2H), 2.54-2.47 (m, 2H), 2.39 (t, $J$ = 6.1 Hz, 4H), 1.43-1.34 (m, 27H); FT MS ESI m/z [M+H$^+$] 666.38

7.4 Preparation of **69-246**

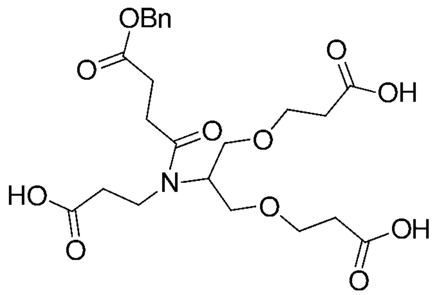

To a flask containing **69-243** (13.1871 g, 9.6699 mmol) was added dichloromethane (20 mL). After dissolution with the assistance of ultrasonication, TFA (7.2 mL, 96.699 mmol) was added, and the reaction was stirred overnight at room temperature. After the reaction was completed, the reaction mixture was concentrated, methyl tert-butyl ether (300 mL) was added, a solid precipitated and was filtered, and the filter cake was washed with methyl tert-butyl ether (40 mL). The filter cake was collected, and dried to provide 0.56 g of the product. FT MS ESI m/z [M+H$^+$] 498.523

7.5 Preparation of **69-251**

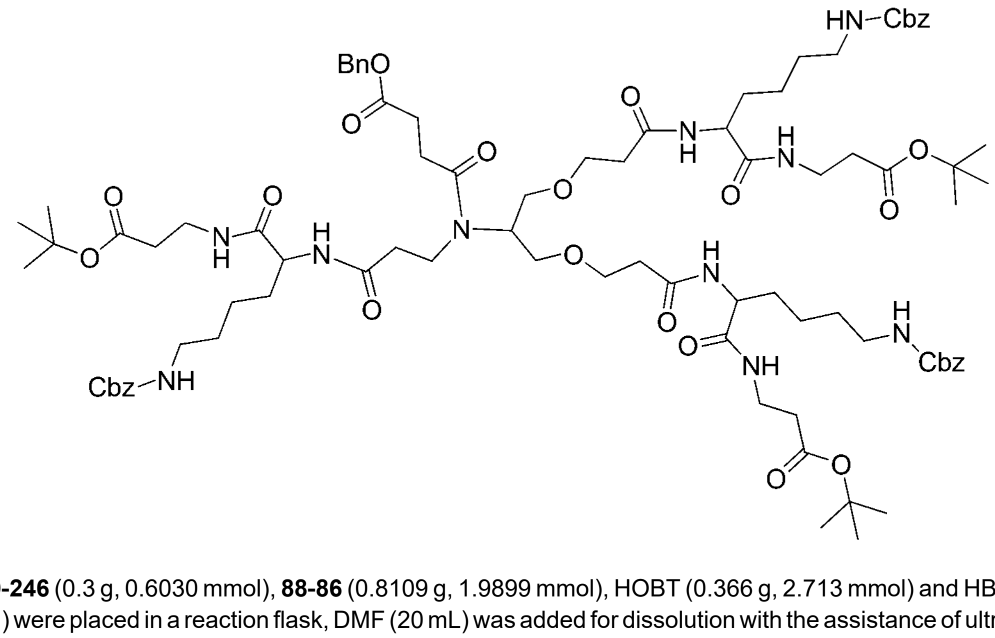

**69-246** (0.3 g, 0.6030 mmol), **88-86** (0.8109 g, 1.9899 mmol), HOBT (0.366 g, 2.713 mmol) and HBTU (1.028 g, 2.713 mmol) were placed in a reaction flask, DMF (20 mL) was added for dissolution with the assistance of ultrasonication, and the contents were stirred at 0°C. DIEA (1.345 mL, 8.1405 mmol) was added dropwise. After the dropwise addition was completed, the mixture was brought to room temperature and reacted under stirring. After the reaction was completed, methyl tert-butyl ether (400 mL) was added to the reaction mixture, a solid precipitated and was filtered to collect a filter cake. The filter cake was dissolved in an appropriate amount of dichloromethane/methanol (1/4) mixture, then 200-300

mesh silica gel powder was added, and the mixture was evaporated to dryness. The resultant product was loaded by dry method, and subjected to column chromatography using 3% methanol/dichloromethane as the eluent. The desired product was collected, concentrated and dried to provide 0.45 g of the product. $^1$H-NMR (600 MHz, DMSO-$d_6$) δ 8.08-7.86.(m, 6H), 7.51-7.24 (m, 20H), 7.20-7.15 (m, 3H), 5.07 (s, 2H), 5.05-4.98 (m, 6H), 4.10 (q, *J* = 5.2 Hz, 3H), 4.03 (dd, *J* = 14.2, 7.1 Hz, 1H), 3.49-3.41 (m, 6H), 3.23-3.14 (m, 16H), 2.94 (d, *J* = 5.8 Hz, 4H), 2.40-2.29 (m, 12H), 1.58-1.53 (m, 3H), 1.40-1.36 (m, 36H), 1.28-1.19 (m, 6H); FT MS ESI m/z [M+Na$^+$] 1688.989

7.6 Preparation of **69-257**

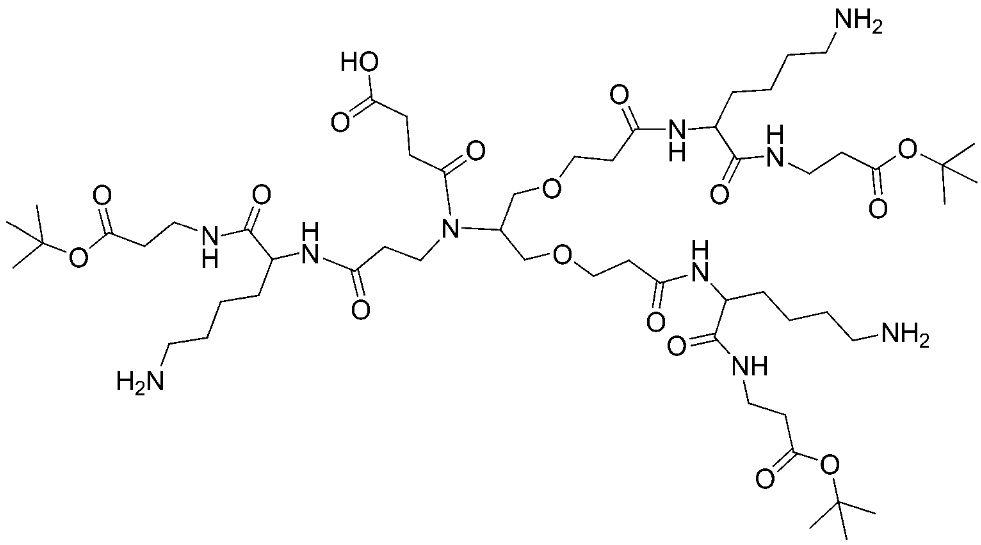

**69-251** (1.0 g, 3.0640 mmol) was added to a hydrogenation reactor, then 10% Pd/C catalyst (0.8 g) and DMF (20 mL) were added. The reactor was sealed, evacuated, and then introduced with hydrogen gas. This process was repeated five times. Then, hydrogen gas was refilled to 300 psi. The reaction was carried out under stirring at room temperature overnight. After the reaction was completed, the reaction mixture was filtered through Celite, the filter cake was washed with DMF (15 mL × 3), and the filtrate was transferred to a 500 mL round-bottom flask and used as the raw material in the next step. FT MS ESI m/z [M+H$^+$] 1173.732

7.7 Preparation of **69-259**

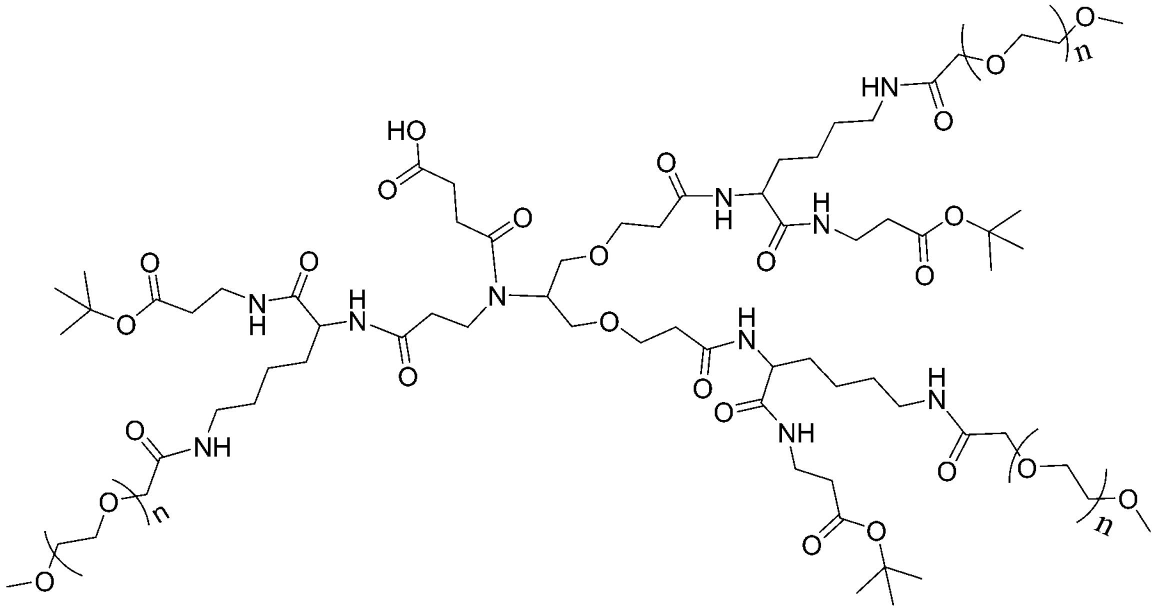

**69-257** (0.1 mmol) was added to a 50 mL round-bottom flask. The mixture was stirred at -5°C for 30 minutes. DIEA (0.146 mL, 0.887 mol) was then slowly added dropwise. The mixture was stirred continuously for 30 minutes, taken out, then M-SCM-10K (2.10 g, 0.21 mmol, purchased from Jenkem, Lot Number: ZZ363P139) was added, and the mixture was reacted in the dark under stirring at low speed for 72 hours at room temperature. After the reaction was completed, methyl tert-butyl ether (500 mL) was added to the reaction mixture, a solid precipitated and was filtered. The filter cake was dried to provide the product. $^1$H-NMR (600 MHz, DMSO-$d_6$) $\delta$ 12.01(s, 1H), 8.08-7.86.(m, 6H), 7.20-7.15 (m, 3H),4.19-4.15 (m, 4H), 3.53-3.48 (m, 2979H), 3.23-3.14 (m, 16H), 2.65-2.57 (m, 6H), 2.41-2.29 (m, 16H), 1.58-1.53 (m, 3H), 1.40-1.36 (m, 36H), 1.28-1.19 (m, 6H)

7.8 Preparation of **69-275**

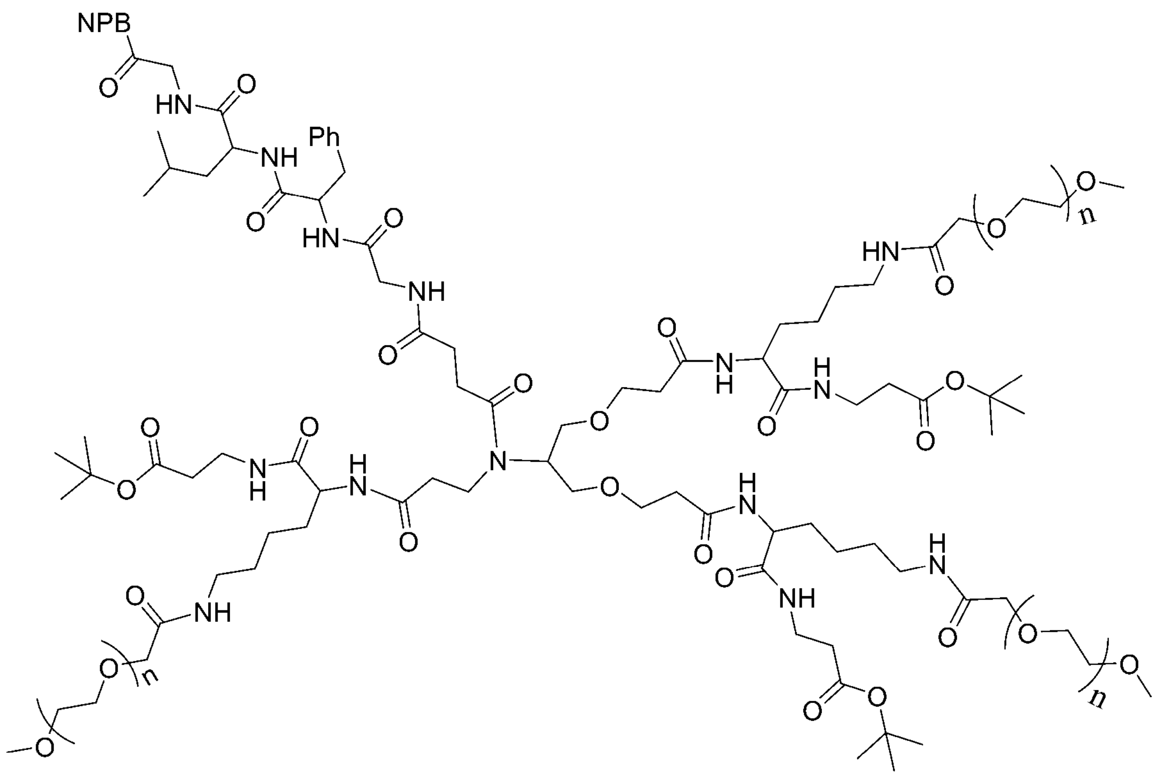

Reactants **69-259** (0.1 mmol), **70-68** (0.56 g, 0.48 mmol), HOBT (0.081 g, 0.6 mmol) and HBTU (0.227 g, 0.6 mmol) were placed in a reaction flask, an appropriate amount of DMF was added for dissolution with the assistance of ultrasonication. The mixture was stirred at 0°C for 3 minutes. DIEA (0.297 mmol, 1.8 mmol) was then added dropwise. The mixture was taken out, and the reaction was carried out under stirring at room temperature overnight. After the reaction was completed, methyl tert-butyl ether (300 mL) was added. A solid precipitated and was filtered. The filter cake was collected and dried to provide 1.25 g of the product.

7.9 Preparation of **69-278**

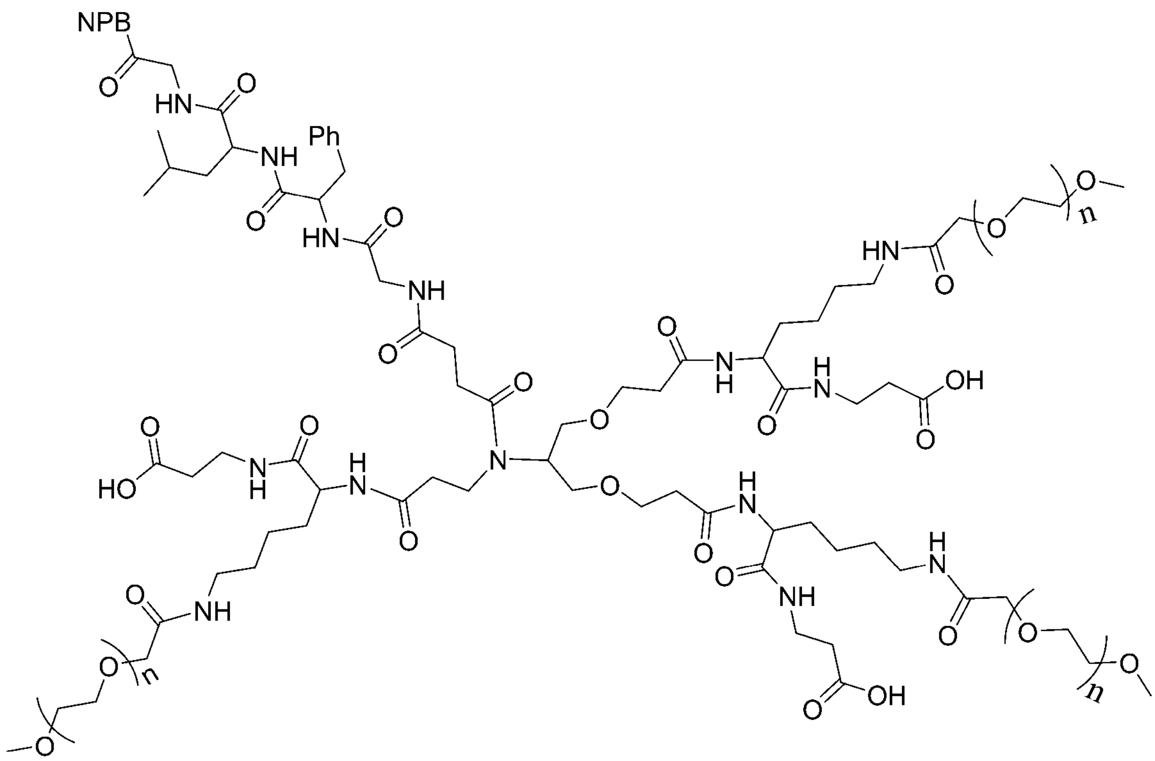

**69-275** (0.1 mmol) was weighed and added to a 50 mL round-bottom flask, then TFA (10 mL) was added, and the mixture was reacted under stirring at room temperature. After the reaction was completed, TFA was removed by rotary evaporation under reduced pressure, and methyl tert-butyl ether (500 mL) was added. A solid precipitated and was filtered. The filter cake was dried to provide the product.

7.10 Preparation of **86-4**

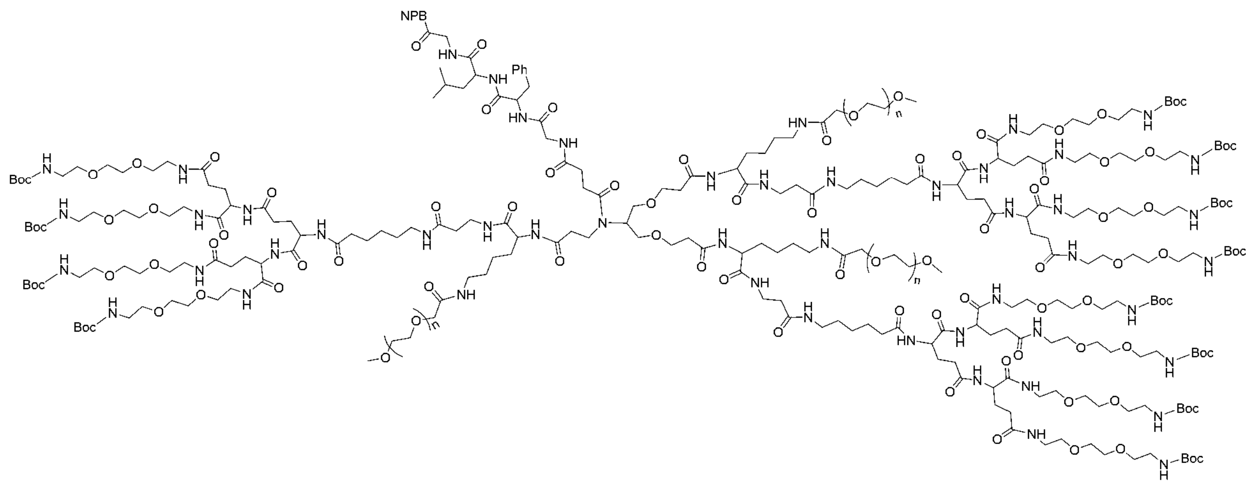

Reactants **69-278** (0.1 mmol), **76-93** (0.56 g, 0.48 mmol), HOBT (0.081 g, 0.6 mmol) and HBTU (0.227 g, 0.6 mmol) were placed in a reaction flask, DMF (50 mL) was added for dissolution with the assistance of ultrasonication. The mixture was stirred at 0°C. DIEA (0.297 mL, 1.8 mmol) was added dropwise. The mixture was taken out and reacted under stirring at room temperature overnight. After the reaction was completed, methyl tert-butyl ether (300 mL) was added, a solid precipitate and was filtered. The filter cake was collected and dried to provide 1.25 g of the product. $^1$H-NMR (600 MHz, DMSO-$d_6$) δ 9.33-9.24 (m, 1H), 9.10-8.98 (m, 1H), 8.62-8.51 (m, 1H), 8.30-7.76(m, 34H), 7.72-7.61 (m, 4H), 7.61-7.55 (m, 1H), 7.54-7.46 (m, 2H), 7.37-6.92 (m, 20H), 4.57-4.26 (m, 14H), 4.25-3.82 (m, 39H), 3.81-3.28 (m, 3025H), 3.28-3.21 (m, 15H), 3.21-3.11 (m, 16H), 3.10-2.96 (m, 29H), 2.82-2.66 (m, 7H), 2.65-2.45 (m, 42H), 2.42-2.26 (m, 6H), 2.25-2.17 (m, 4H), 2.17-2.03 (m, 20H), 1.89-1.63 (m, 22H), 1.63-1.30 (m, 110H), 1.30-1.11 (m, 37H), 0.92-0.87 (m, 3H), 0.87-0.77 (m,3H)

7.11 Preparation of **86-7**

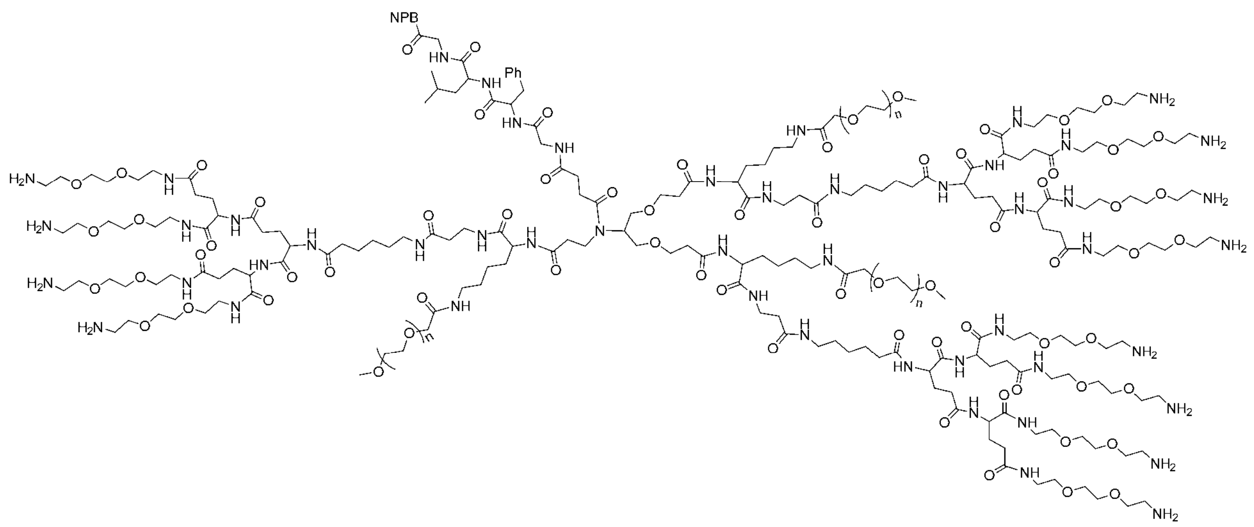

**86-4** (1.25 g, 0.0756 mmol) was weighed, dichloromethane (5 mL) and TFA (0.084 mL, 1.134 mmol) were added, and the reaction was stirred at room temperature. After the reaction was completed, the reaction mixture was concentrated under reduced pressure, and methyl tert-butyl ether (150 mL) and n-hexane (100 mL) were added. A solid precipitated and was filtered. The filter cake was dissolved in an appropriate amount of a dichloromethane/methanol (1/4) mixture, then 100-200 mesh silica gel powder was added, and the mixture was evaporated to dryness. The resultant product was loaded by dry method, and subjected to column chromatography using 1% aqueous ammonia/10-12% methanol/ dichloromethane as the eluent. The desired product was collected, concentrated, and dried in oven to provide 0.95 g of the product.

7.12 Preparation of **86-9**

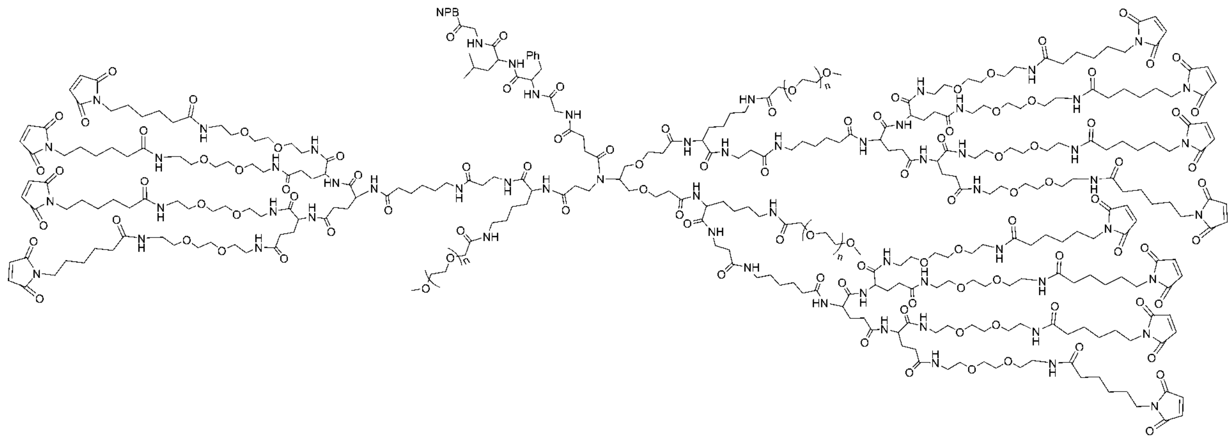

**86-7** (0.95 g, 0.0325 mmol) and **69-280** (0.18 g, 0.4528 mmol) were taken and dissolved in DMF (10 mL). DIEA (1.442 mL, 8.730 mmol) was then added dropwise, and the reaction was stirred at room temperature. After the reaction was completed, methyl tert-butyl ether (300 mL) and n-hexane (100 mL) were added to the reaction mixture. A solid precipitated and was dissolved in an appropriate amount of a methanol/dichloromethane (1/4) mixture, then 100-200 mesh silica gel powder was added, and the mixture was evaporated to dryness. The resultant product was loaded by dry method, and subjected to column chromatography using 1% aqueous ammonia/15-20% methanol/dichloromethane as the eluent. The desired product was collected and dried to provide 0.89 g of the product.

7.13 Preparation of **86-43**

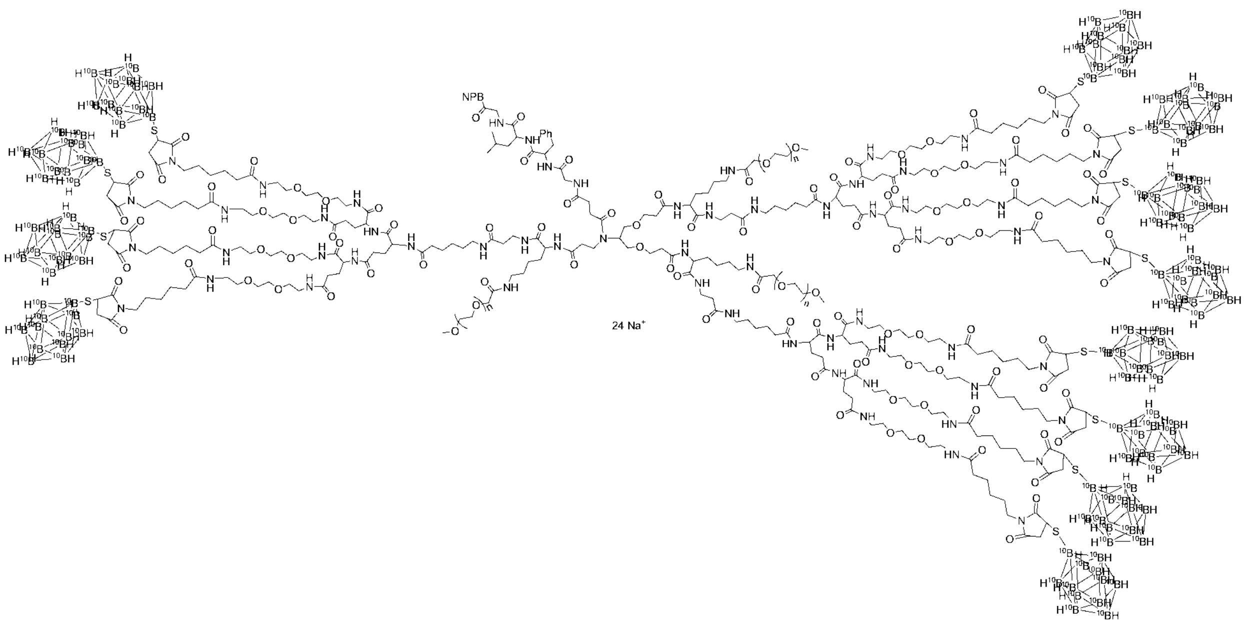

**86-9** (0.16 g, 0.004 mmol) was weighed and placed into a 100 mL reaction flask, then methanol (3 mL) and 1,2,3,4,5,6,7,8,9,10,11-undecahydro-12-mercapto-dodecaborate (2-), sodium (1:2) ($^{10}B$) (0.015 g, 0.073 mmol, purchased from CATCHEM, Czech Republic) were added, and the mixture was stirred at room temperature for 3 hours. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered by suction, and the filter cake was collected to obtain a solid product, which

was dried in a vacuum oven to provide 0.15 g of the product. $^{1}$H-NMR (600 MHz, DMSO-$d_6$) δ 9.33-9.24 (m, 1H), 9.10-8.98 (m, 1H), 8.62-8.51 (m, 1H), 8.30-7.76(m, 34H), 7.72-7.61 (m, 4H), 7.61-7.55 (m, 1H), 7.54-7.46 (m, 2H), 7.37-6.92 (m, 20H), 4.57-4.26 (m, 14H), 4.25-3.82 (m, 39H), 3.81-3.28 (m, 3049H), 3.28-3.21 (m, 27H), 3.21-3.11 (m, 16H), 3.10-2.96 (m, 29H), 2.82-2.66 (m, 31H), 2.65-2.45 (m, 42H), 2.42-2.26 (m, 30H), 2.25-2.17 (m, 4H), 2.17-2.03 (m, 20H), 1.89--0.77 (m, 271H)

Example 8: Synthesis of Compound **87-35**

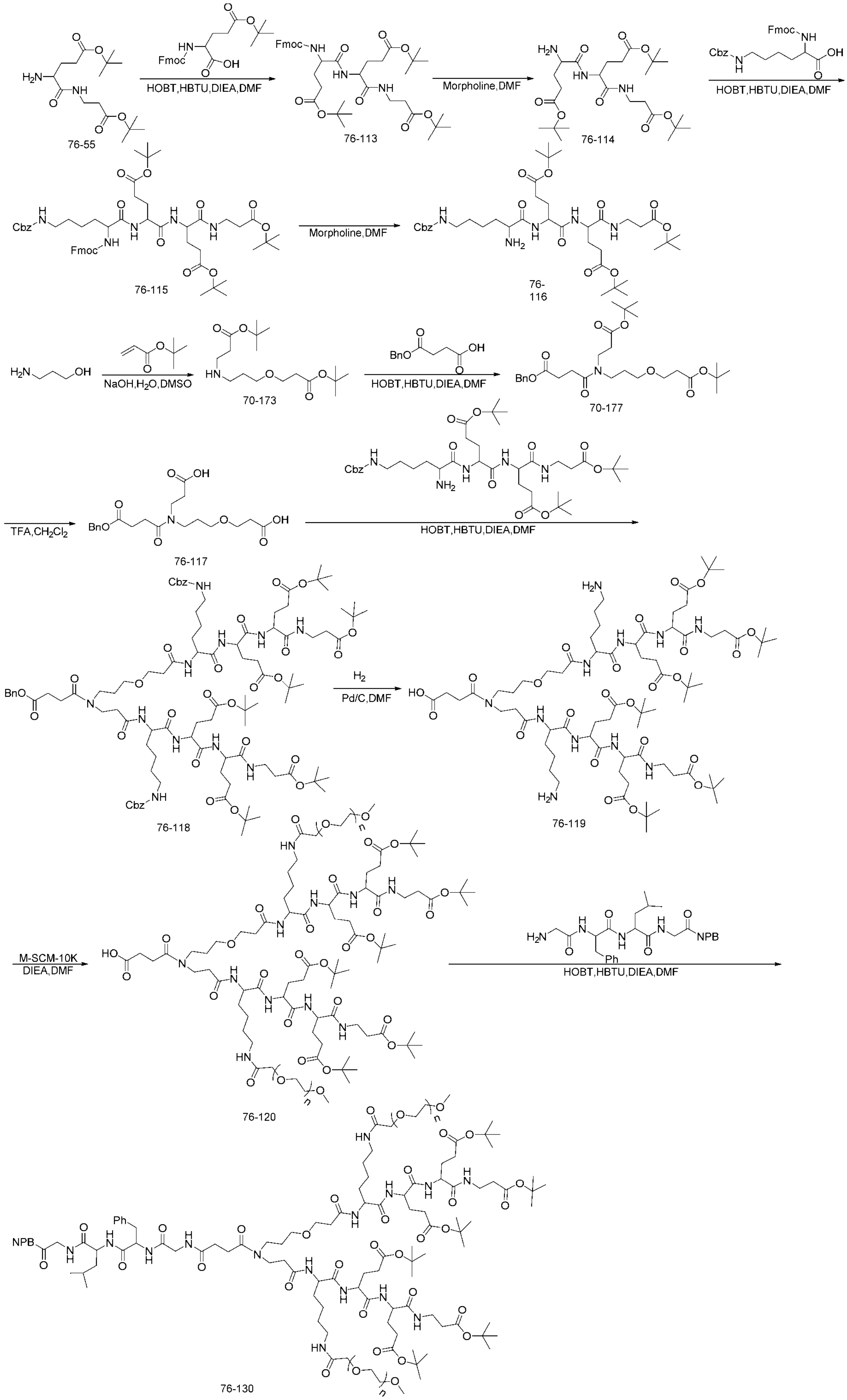


76-55, 76-113, 76-114, 76-115, 76-116, 70-173, 70-177, 76-117, 76-118, 76-119, 76-120, 76-130

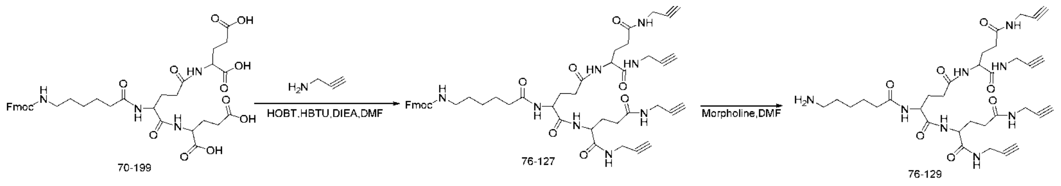


70-199

76-127

76-129

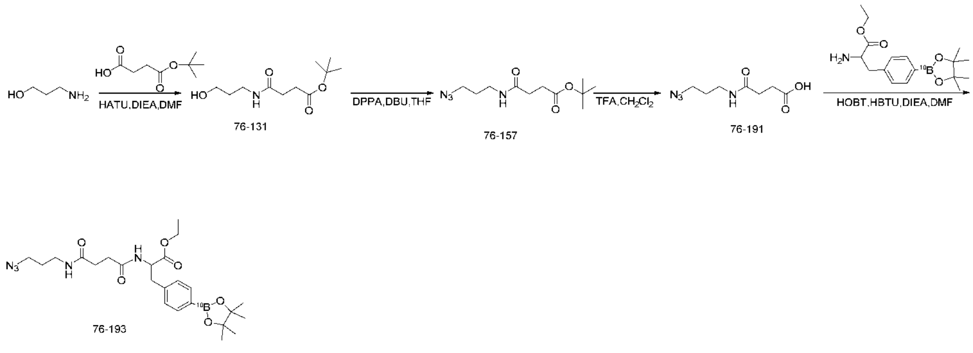


76-131

76-157

76-191

76-193

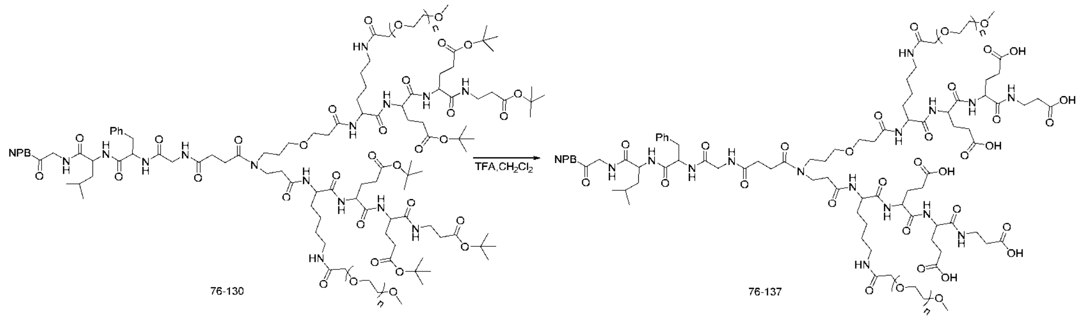


76-130

76-137

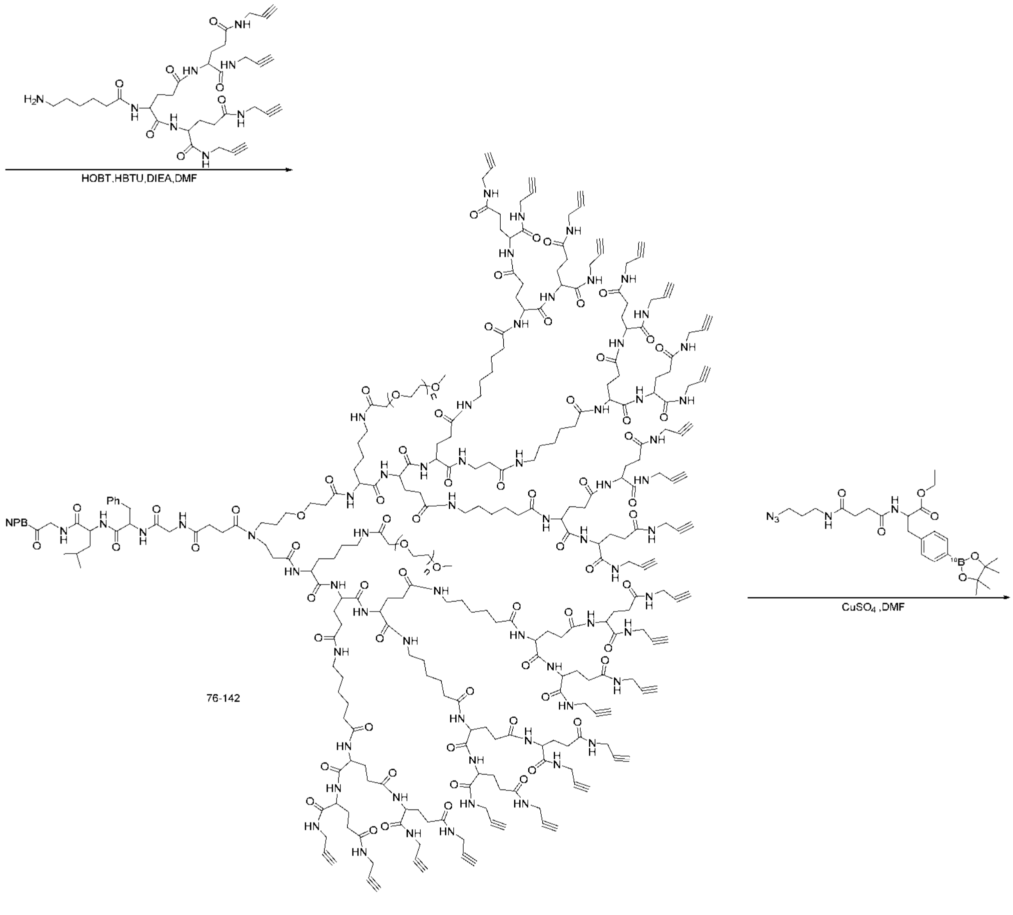


76-142

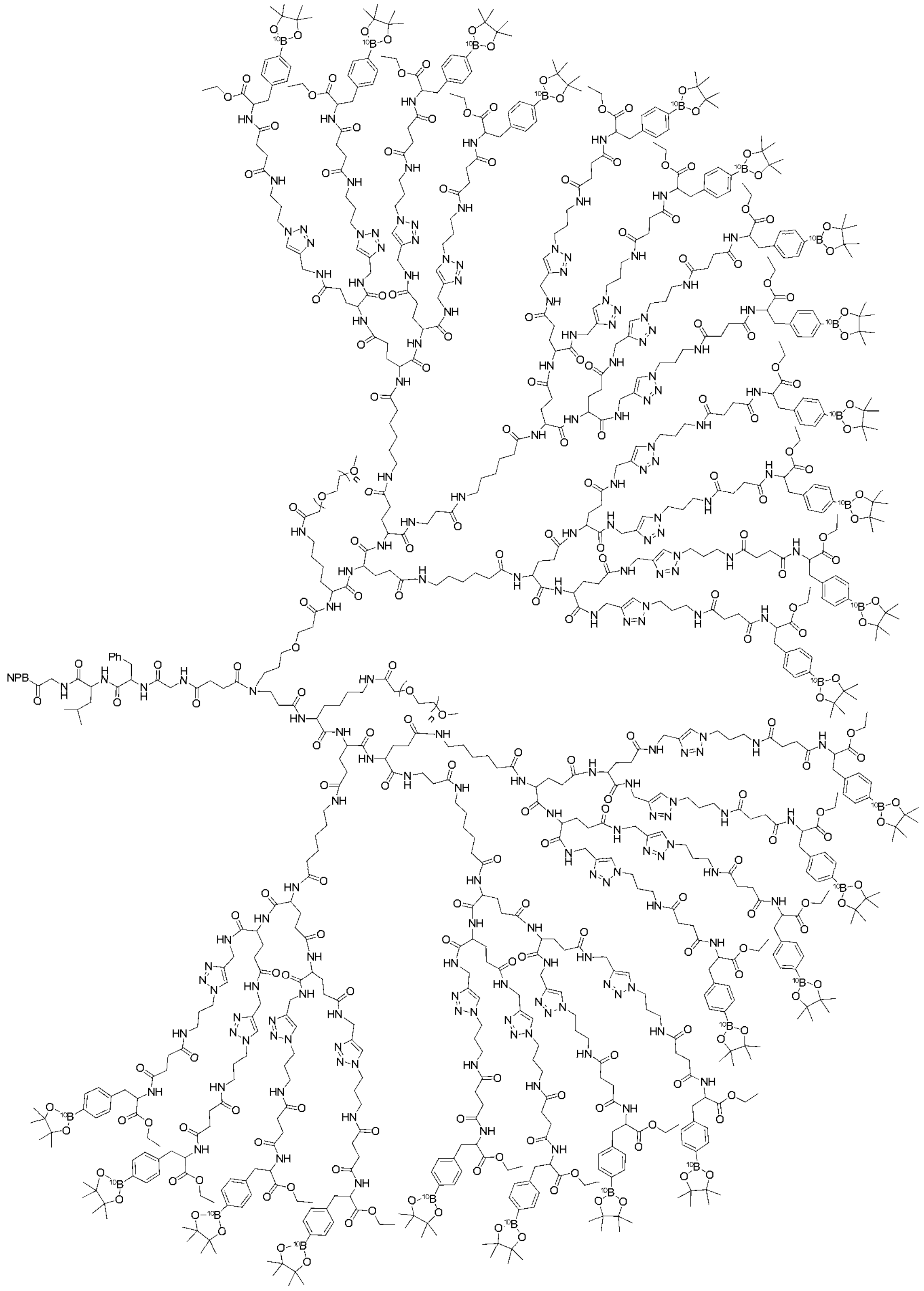

87-35

8.1 Preparation of Compound **76-113**

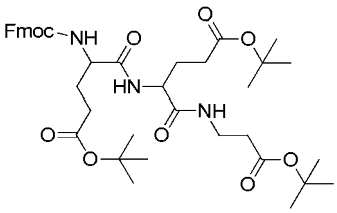

Fmoc-Glu(OtBu)-OH (2.35 g, 5.53 mmol), HBTU (2.52 g, 6.64 mmol) and HOBT (0.90 g, 6.64 mmol) were weighed, added into a flask containing **76-55** (1.92 g, 5.81 mmol), then an appropriate amount of DMF was added to dissolve the mixture, and the contents were allowed to stir at -5°C. DIEA (3.29 mL, 19.91 mmol) was slowly added dropwise. After the dropwise addition, the reaction was stirred for half an hour, the flask was taken out and the reaction was carried out under stirring at room temperature overnight. After the reaction was completed, saturated NaCl solution (200 mL) and ethyl acetate (200 mL) were added for extraction. After standing for lay separation, the organic phase was collected, and the aqueous phase was further extracted with ethyl acetate (200 mL × 3). The organic phases were combined, concentrated, evaporated to dryness, and dried in a vacuum oven to provide the product.

8.2 Preparation of **76-114**

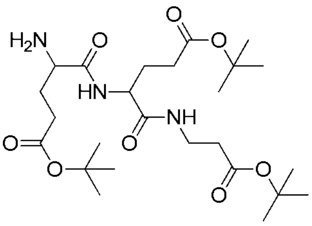

DMF was added into a flask containing **76-113** (5.53 mmol). After dissolution with the assistance of ultrasonication, morpholine (9.64 mL, 116.0 mmol) was added, and the reaction was stirred at room temperature for 2 h. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, evaporated to dryness, and dried in a vacuum oven to provide the product.

8.3 Preparation of **76-115**

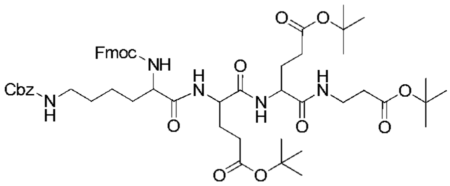

N'-Fmoc-N-benzyloxycarbonyl-L-lysine (2.65 g, 5.27 mmol), HBTU (2.40 g, 6.32 mmol) and HOBT (0.85 g, 6.32 mmol) were weighed and added into a flask containing **76-114** (5.53 mmol), then an appropriate amount of DMF was added to dissolve the mixture, and the contents were allowed to stir at -5°C. DIEA (3.13 mL, 18.96 mmol) was slowly added dropwise. After the dropwise addition was completed, the reaction was carried out for half an hour, the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid.

8.4 Preparation of **76-116**

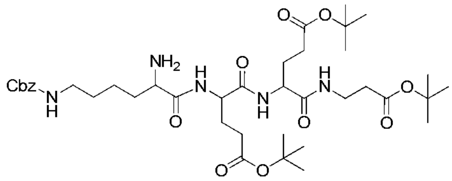

To a flask containing **76-115** (5.27 mmol) was added DMF. After dissolution with the assistance of ultrasonication, morpholine (9.18 mL, 105.4 mmol) was added, and the reaction was stirred at room temperature for 2 h. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid, which was purified by column chromatography using 1% ammonia/5% methanol/dichloromethane as the eluent. The desired product was collected, concentrated, and dried in a vacuum oven to provide 2.85 g of the product with a yield of 70%. $^1$H-NMR (600 MHz, DMSO-*d*$_6$) δ 8.17-7.98 (m, 2H), 7.97-7.93 (m, 1H), 7.39-7.28 (m, 5H), 7.25-7.16 (m, 1H), 5.06-4.96 (m, 2H), 4.27 (s, 1H), 4.22-4.13 (m, 1H), 3.31-3.27 (m, 1H), 3.22-3.10 (m, 2H), 3.02-2.93 (m, 2H), 2.64-2.54 (m, 1H), 2.40-2.30 (m, 2H), 2.24-2.13 (m, 4H), 1.91-1.64 (m, 5H), 1.63-1.46 (m, 2H), 1.40-1.36 (m, 27H), 1.32-1.23 (m, 2H); ESI [M+H$^+$] 778.46

8.5 Preparation of **70-173**

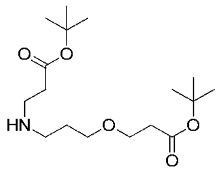

In a 250ml flask, 3-amino-1-propanol (2ml, 27.10mmol) was dissolved in DMSO (18ml). After stirring in a water bath at 30°C for 10 minutes, 5.0M NaOH solution (0.4ml) was added. Stirring was continued for 10 minutes, followed by the addition of tert-butyl acrylate (8.3ml, 56.90mmol), and the reaction was allowed to proceed to completion. The reaction mixture was transferred to a 1 L separatory funnel, and saturated brine (200 mL) and ethyl acetate (200 mL) were added for extraction. The organic phase was collected, and the aqueous phase was further extracted with ethyl acetate (200 mL × 3). The organic phases were combined, washed with saturated brine (200 mL × 2) and evaporated to dryness. The resulting product was dissolved in a mixture of dichloromethane and methanol, loaded by dry method, and subjected to column chromatography using a 10% ethyl acetate/petroleum ether mixture as the eluent. The desired product was collected, concentrated, and dried in a vacuum oven to provide the product.

8.6 Preparation of **70-177**

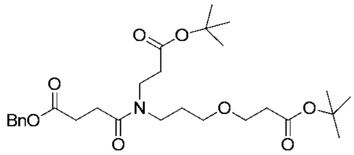

Mono-benzyl succinate (0.80 g, 3.70 mmol), HBTU (1.50 g, 4.00 mmol) and HOBT (0.50 g, 4.00 mmol) were weighed and added into a flask containing **70-173** (1.07 g, 3.40 mmol), then an appropriate amount of DMF was added to dissolve the mixture, and allowed to stir at -5°C. DIEA (1.20 mL, 7.40 mmol) was slowly added dropwise. After the dropwise addition was completed, the reaction was carried out for half an hour, the flask was taken out and the reaction was carried out under stirring at room temperature overnight. After the reaction was completed, saturated NaCl solution (200 mL) and

ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid.

8.7 Preparation of **76-117**

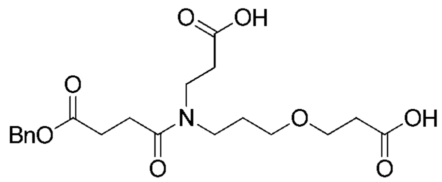

An appropriate amount of dichloromethane was added into a flask containing **70-177** (1.50 g, 2.88 mmol). After dissolution with the assistance of ultrasonication, TFA (4.27 mL, 57.51 mmol) was added and reacted under stirring at room temperature overnight. After the reaction was completed, the reaction mixture was evaporated to provide an oil using a rotary evaporator. Methyl tert-butyl ether (60 mL) was then added for precipitation. The product remained as an oil at the bottom of the flask. The methyl tert-butyl ether was discarded, and the product was dried in a vacuum oven to provide 1.26 g of the product with a yield of 100%. ESI [M+H$^+$] 410.18, [M+Na$^+$] 432.16

8.8 Preparation of **76-118**

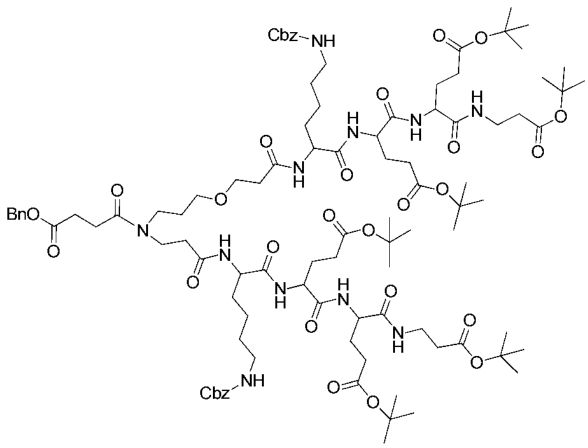

**76-117** (0.71 g, 1.74 mmol), HBTU (1.58 g, 4.18 mmol) and HOBT (0.57 g, 4.18 mmol) were weighed and added into a flask containing **76-116** (2.85 g, 3.66 mmol), then an appropriate amount of DMF was added to dissolve the mixture, and the contents were allowed to stir at -5°C. DIEA (2.07 mL, 12.53 mmol) was slowly added dropwise. After the dropwise addition was completed, the reaction was carried out for half an hour, the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, concentrated, evaporated to dryness, and dried in a vacuum oven to provide 2.89 g of the product with a yield of 86%. $^1$H-NMR (600 MHz, DMSO-$d_6$) δ 8.17-8.07 (m, 1H), 8.06-7.97 (m, 3H), 7.97-7.89 (m, 2H), 7.87-7.78 (m, 2H), 7.39-7.27 (m, 15H), 7.27-7.17 (m, 2H), 5.07-4.97 (m, 6H), 4.29-4.15 (m, 6H), 4.06-4.00 (m, 1H), 3.59-3.35 (m, 6H), 3.31-3.25 (m, 4H), 3.24-3.11 (m, 3H), 3.01-2.93 (m, 5H), 2.70-2.60 (m, 2H), 2.59-2.54 (m, 4H), 2.43-2.28 (m, 8H), 2.23-2.13 (m, 8H), 1.76-1.65 (m, 6H), 1.65-1.55 (m, 3H), 1.53-1.45 (m, 3H), 1.39-1.36 (m, 54H), 1.32-1.19 (m, 5H); ESI [M+Na$^+$] 1951.04

8.9 Preparation of **76-119**

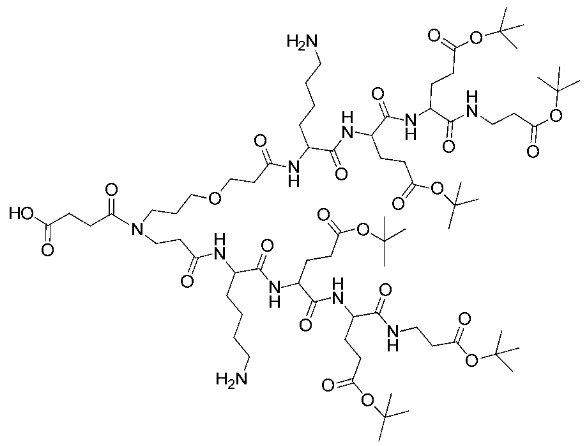

**76-118** (0.19 g, 0.10 mmol) and 10% Pd/C catalyst (0.01 g) were added into a hydrogenation reactor, then DMF (30 mL) was added for dissolution. The hydrogenation reactor was sealed, evacuated with a water pump, and introduced with hydrogen at about 2 MPa. The evacuation/hydrogenation operation was repeated three times. Finally, the pressure reading of the hydrogenation reactor was adjusted to 1.8 MPa, and the reaction was allowed to proceed overnight at room temperature. After the reaction was completed, the reaction mixture was filtered through Celite, and the filter cake was washed with DMF (20 mL × 3) to provide a DMF solution of the product, which served as the raw material for the next step.

8.10 Preparation of **76-120**

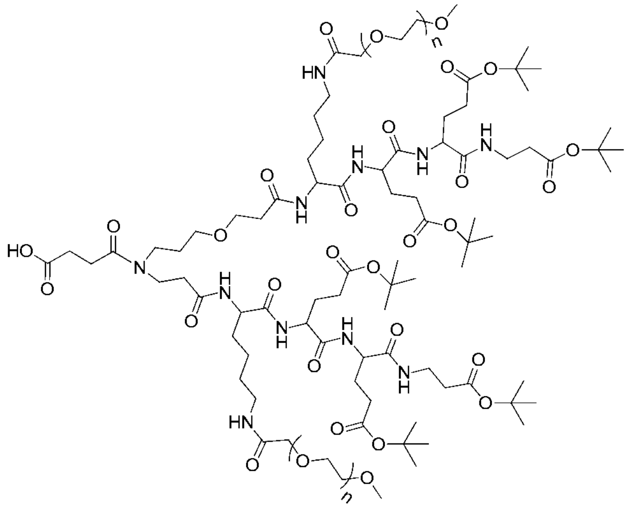

**76-119** (0.10 mmol) was placed in a 250 mL flask, followed by the addition of DMF (20 mL). This mixture was then slowly added dropwise to a DMF solution containing DIEA (0.95 mL, 5.75 mmol) and M-SCM-10K (2.0 g, 0.19 mmol, purchased from Jenkem). The reaction was carried out under stirring in the dark at low speed at room temperature for one week. After the reaction was completed, n-hexane (25 mL) and methyl tert-butyl ether (200 mL) were added for precipitation. The supernatant was discarded, then n-hexane and methyl tert-butyl ether were added again for precipitation. This process was repeated three times. The mixture was filtered to provide a solid product, which was collected, and dried in a vacuum oven to provide the product.

8.11 Preparation of **76-130**

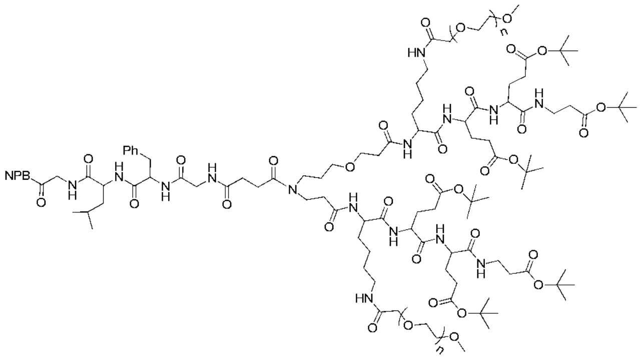

**76-120** (0.81 g, 0.04 mmol), HBTU (0.02 g, 0.13 mmol) and HOBT (0.01 g, 0.04 mmol) were weighed and added into a flask containing **70-68** (0.03 g, 0.04 mmol), then an appropriate amount of DMF was added to dissolve the mixture, and the contents were allowed to stir at -5°C. DIEA (0.02 mL, 0.13 mmol) was slowly added dropwise. After the dropwise addition was completed, the reaction was carried out for half an hour, the flask was then taken out and the reaction was carried out under stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, and dried to provide the product.

8.12 Preparation of **76-127**

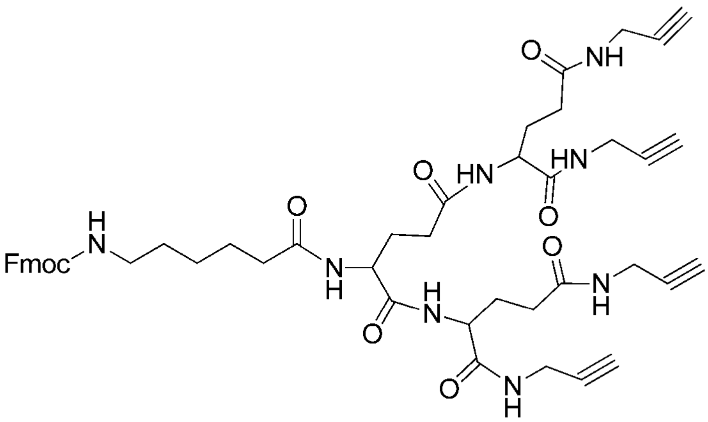

**70-199** (0.71 g, 0.96 mmol), HBTU (1.75 g, 4.61 mmol) and HOBT (0.62 g, 4.61 mmol) were added into a flask containing propargylamine (0.23 g, 4.22 mmol), then an appropriate amount of DMF was added to dissolve the mixture, and the contents were allowed to stir at -5°C. DIEA (2.29 mL, 13.82 mmol) was slowly added dropwise. After the dropwise addition was completed, the reaction was carried out for half an hour, and then the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, concentrated, evaporated to dryness, and dried in a vacuum oven to provide 0.66 g of the product. ESI [M+H$^+$] 889.42, [M+Na$^+$] 911.40

8.13 Preparation of **76-129**

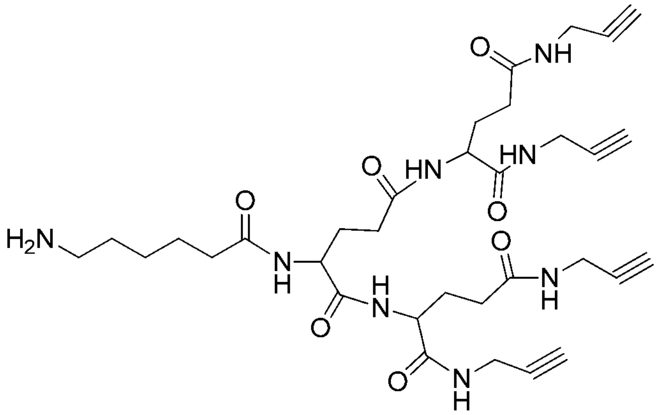

To a flask containing **76-127** (0.96 mmol) was added DMF. After dissolution with the assistance of ultrasonication, morpholine (2 mL) was added, and the reaction was stirred at room temperature for 2 h. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dried in a vacuum oven to provide 0.42 g of the product. ESI [M+H$^+$] 667.35, [M+Na$^+$] 689.34

8.14 Preparation of **76-131**

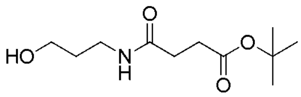

Mono-tert-butyl succinate (3.0 g, 17.22 mmol) and HATU (7.86 g, 20.67 mmol) were weighed and added into a flask containing 3-amino-1-propanol (1.55 g, 20.67 mmol), then an appropriate amount of DMF was added to dissolve the mixture, and the contents were allowed to stir at -5°C. DIEA (10.25 mL, 62.01 mmol) was slowly added dropwise. After the dropwise addition was completed, the reaction was carried out for half an hour, and then the flask was taken out and the reaction was carried out under stirring at room temperature overnight. After the reaction was completed, n-hexane (25 mL) and methyl tert-butyl ether (200 mL) were added for precipitation. The supernatant was discarded, and then n-hexane and methyl tert-butyl ether were added again for precipitation. This process was repeated three times. The mixture was filtered to obtain a solid product, which was collected, and dried to provide the product.

8.15 Preparation of **76-157**

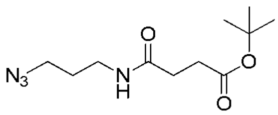

**76-131** (1.02 g, 4.41 mmol) was weighed and dissolved in dry THF, then the contents were allowed to stir at 0°C, and DPPA (1.46 g, 5.29 mmol) and DBU (0.81 g, 5.29 mmol, purchased from Innochem) were slowly added dropwise. After the dropwise addition was completed, the mixture was brought to room temperature and reacted for 2 hours. A THF solution of TBAA (1.50 g, 5.29 mmol) was then added dropwise, and the reaction was stirred at 50°C for 12 hours. After the reaction was completed, saturated $NH_4Cl$ solution (200 mL) and ethyl acetate (200 mL) were added for extraction. The organic phase was collected, dried over anhydrous $Na_2SO_4$, concentrated, and dried to provide the product. $^1$H-NMR (600 MHz, DMSO-$d_6$) δ 7.93-7.85 (m, 1H), 3.38-3.33 (m, 2H), 3.12-3.06 (m, 2H), 2.42-2.38 (m, 2H), 2.30-2.25 (m, 2H), 1.66-1.60 (m, 2H), 1.38 (s, 9H); ESI [M+Na$^+$] 279.14, [2M+Na$^+$] 535.30

8.16 Preparation of **76-191**

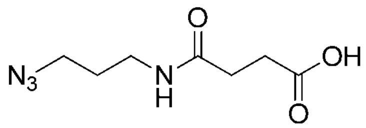

To a flask containing **76-157** (0.0068 mmol) was added dichloromethane. After dissolution with assistance of ultrasonication, TFA (0.2 mL) was added, and the reaction was stirred at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide the product.

8.17 Preparation of **76-193**

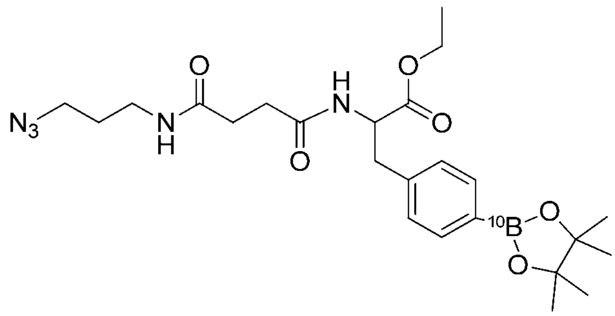

**76-191** (3.32 g, 9.4014 mmol), HBTU (3 g, 7.9277 mmol) and HOBT (1.07 g, 7.9277 mmol) were weighed and added into a flask containing **76-192** (9.4014 mmol), then an appropriate amount of DMF was added to dissolve the mixture, and allowed to stir at -5°C. DIEA (3.93 mL, 22.7830 mmol) was slowly added dropwise. After the dropwise addition was completed, the reaction was carried out for half an hour, and then the flask was taken out. The reaction was carried out under stirring at room temperature overnight. After the reaction was completed, methyl tert-butyl ether and n-hexane were added for precipitation. The mixture was filtered, and dried to provide 3.45 g of the product with a yield of 100%.

8.18 Preparation of **76-137**

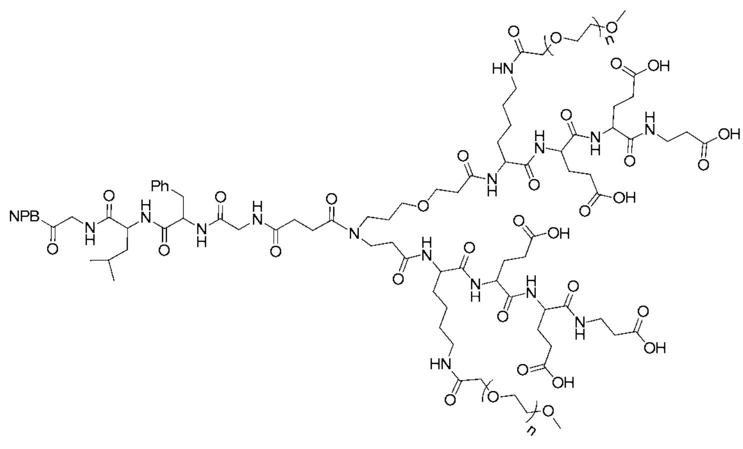

Dichloromethane was added into a flask containing **76-130** (0.33 g). After dissolution with the assistance of

ultrasonication, TFA (0.2 mL) was added, and the reaction was stirred at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide the product.

8.19 Preparation of **76-142**

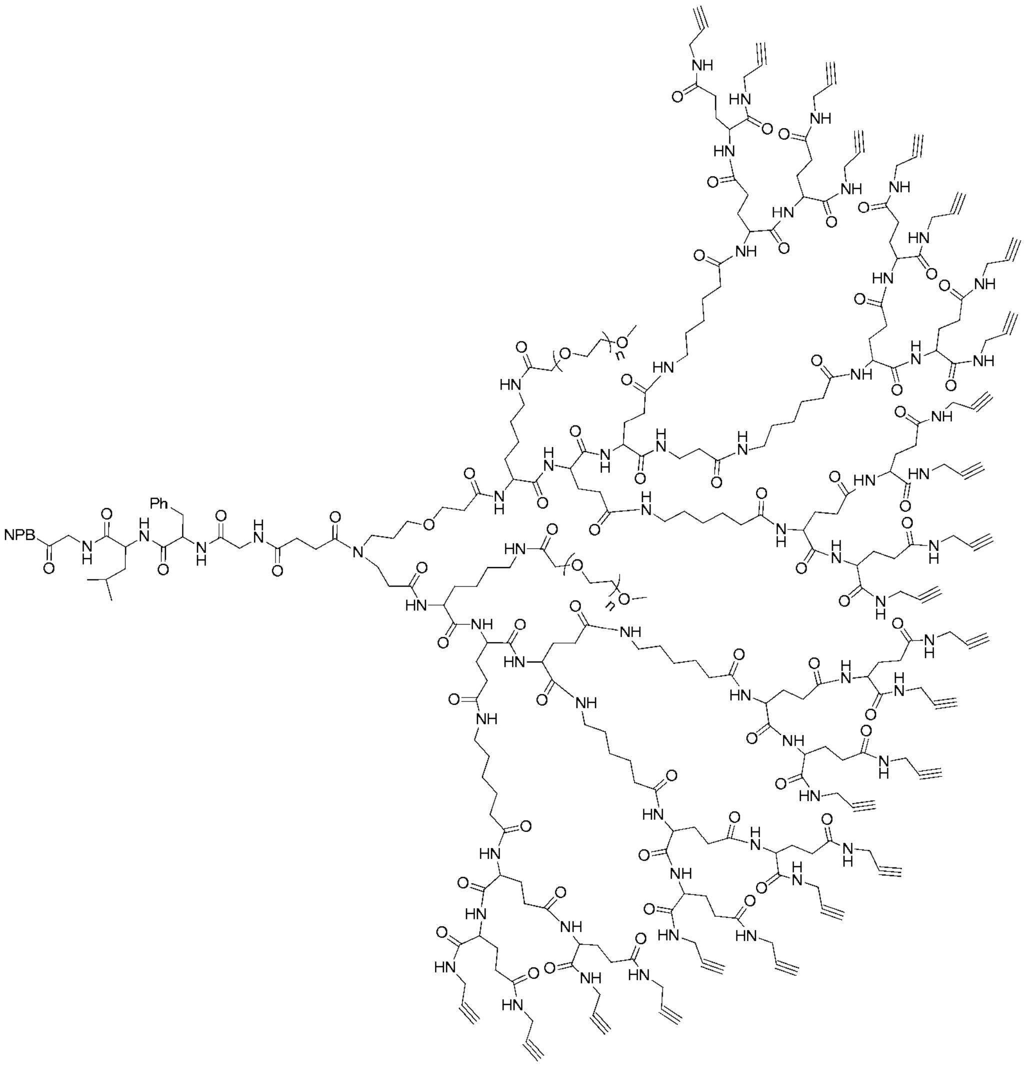

**76-137** (0.42 g, 0.02 mmol), HBTU (0.06 g, 0.13 mmol) and HOBT (0.02 g, 0.13 mmol) were weighed and added into a flask containing **76-129** (0.08 g, 0.12 mmol), then an appropriate amount of DMF was added to dissolve the mixture, and the contents were allowed to stir at -5°C. DIEA (0.07 mL, 0.40 mmol) was slowly added dropwise. After the dropwise addition was completed, the reaction was carried out for half an hour, and then the flask was taken out and the reaction was carried out under stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected and dried to provide 0.1 g of the product.

8.20 Preparation of **87-35**

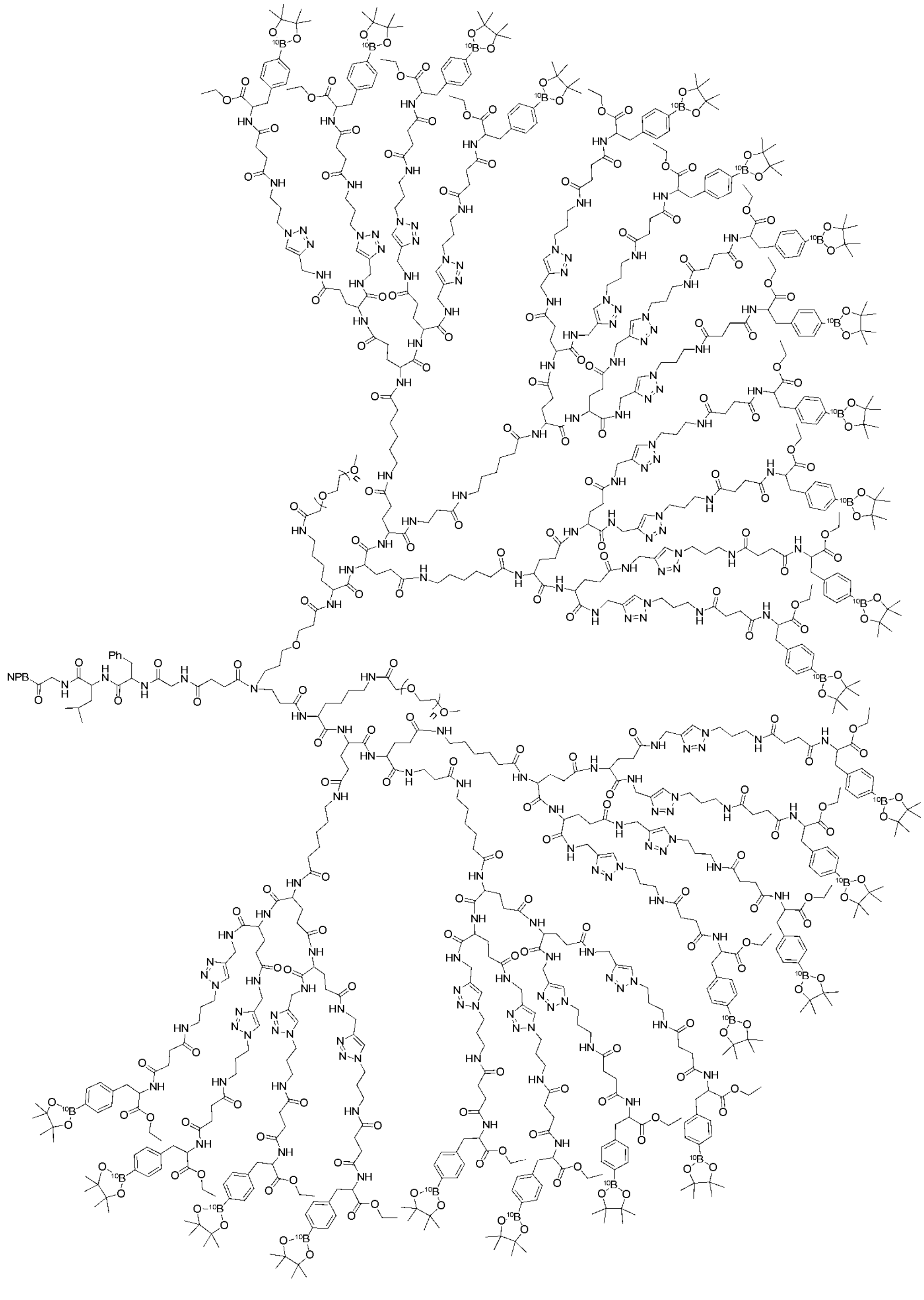

**76-142** (0.0532 mmol) and anhydrous $CuSO_4$ (0.41 g, 2.55 mmol) were weighed and added into a flask containing anhydrous DMF (10 mL), then **76-193** (0.96 g, 1.92 mmol) and sodium ascorbate (1.01 g, 5.11 mmol) were added. After dissolution with the assistance of ultrasonication, the reaction was carried under stirring at room temperature under nitrogen protection overnight. After the reaction was completed, n-hexane (25 mL) and methyl tert-butyl ether (200 mL) were added for precipitation. The mixture was filtered to provide a solid, which was dissolved with dichloromethane, and then n-hexane (25 mL) and methyl tert-butyl ether (200 mL) were added for precipitation. This process was repeated three times. The mixture was filtered to provide a solid product. The solid product was dissolved with a methanol/dichloromethane (1/4) mixture, then silica gel powder was added, and the mixture was evaporated to dryness. The resulting product was loaded by dry method, and subjected to column chromatography using a 1% ammonia water/5-7% methanol/dichloromethane mixed solvent as the eluent. The desired product was collected, evaporated to dryness, and dried in a vacuum oven to provide 0.44 g of the product with a yield of 21.56%. $^1$H-NMR (600 MHz, DMSO-*d*$_6$) δ 8.68-7.67 (m, 130H), 7.41-7.16 (m, 12H), 7.10-6.92 (m, 10H), 6.75-6.59 (m, 6H), 4.60-4.23 (m, 118H), 4.24-4.13 (m, 72H),

4.04-3.90 (m, 103H), 3.57-3.48 (m, 2755H), 3.15-3.00 (m, 120H), 2.96-2.82 (m, 64H), 2.42-2.30 (m, 14H), 2.28-2.02 (m, 68H), 1.93-1.63 (m, 51H), 1.52-1.43 (m, 15H), 1.41-1.34 (m, 18H), 1.29-1.20 (m, 72H), 1.15-0.99 (m, 288H)

Example 9: Synthesis of Compound **75-236**

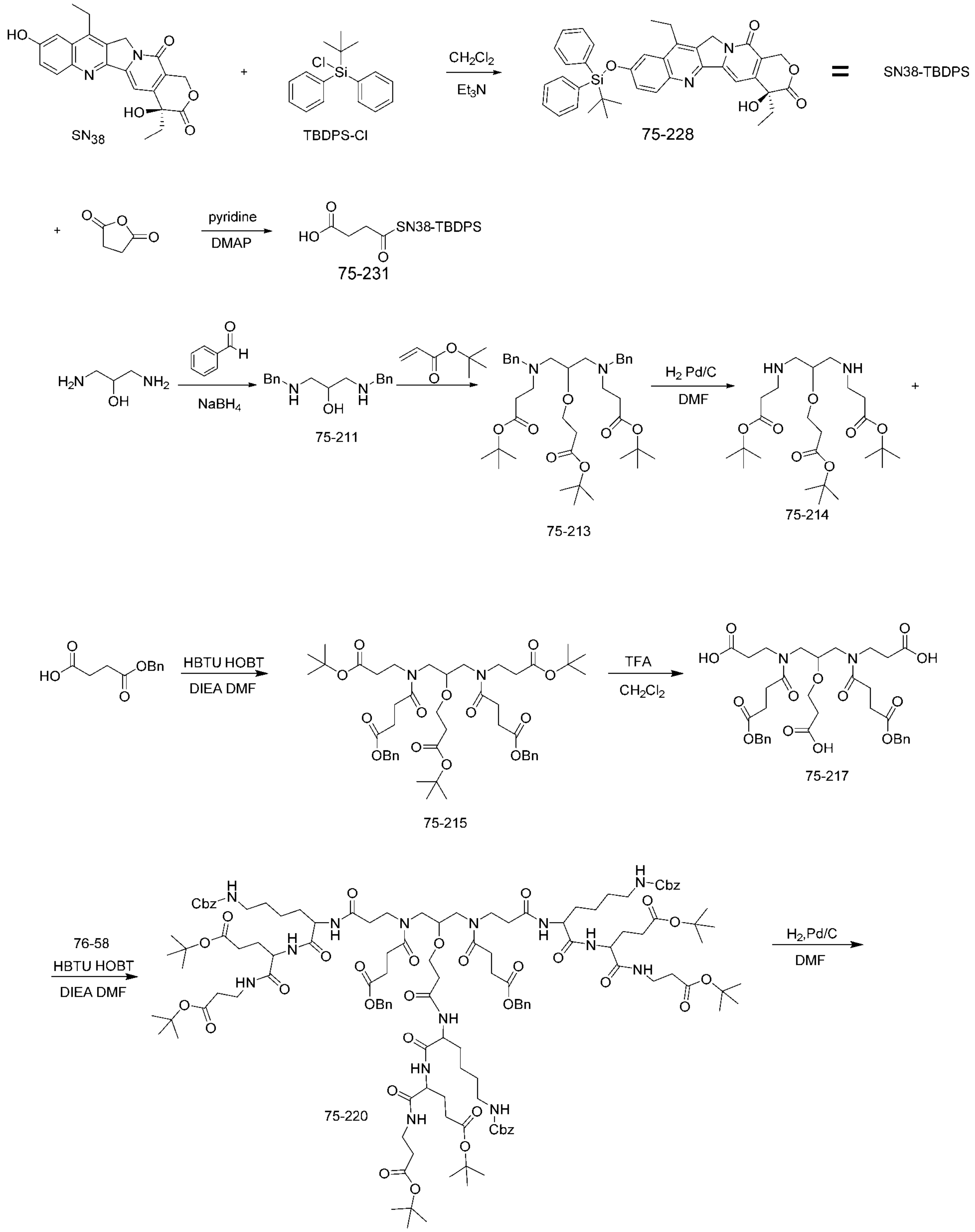

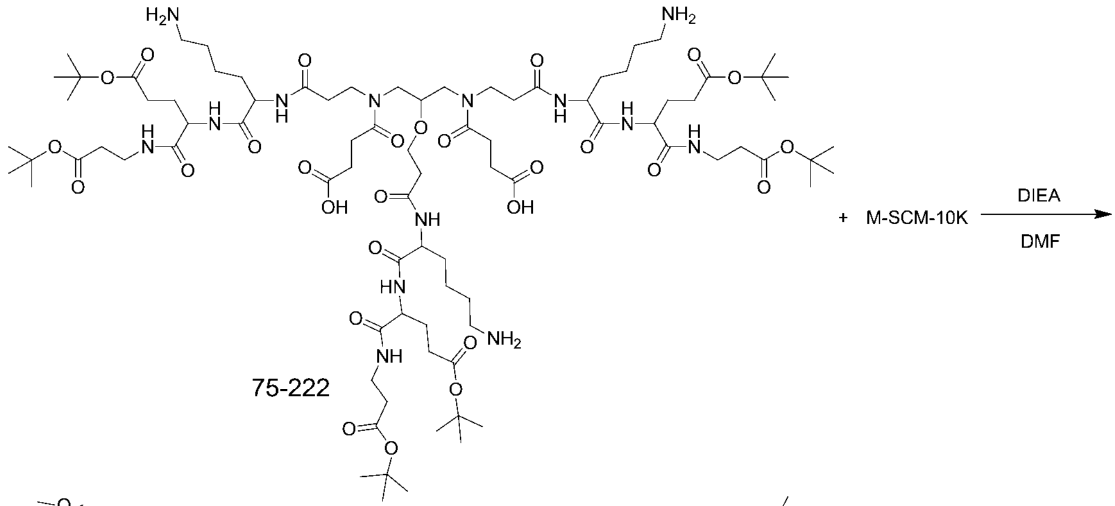


75-222

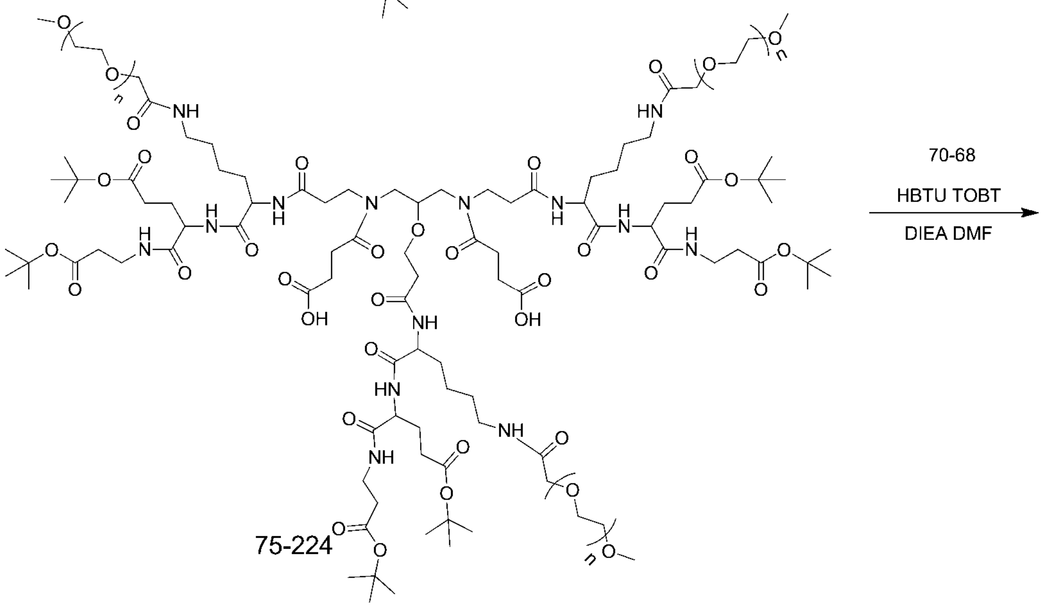


75-224

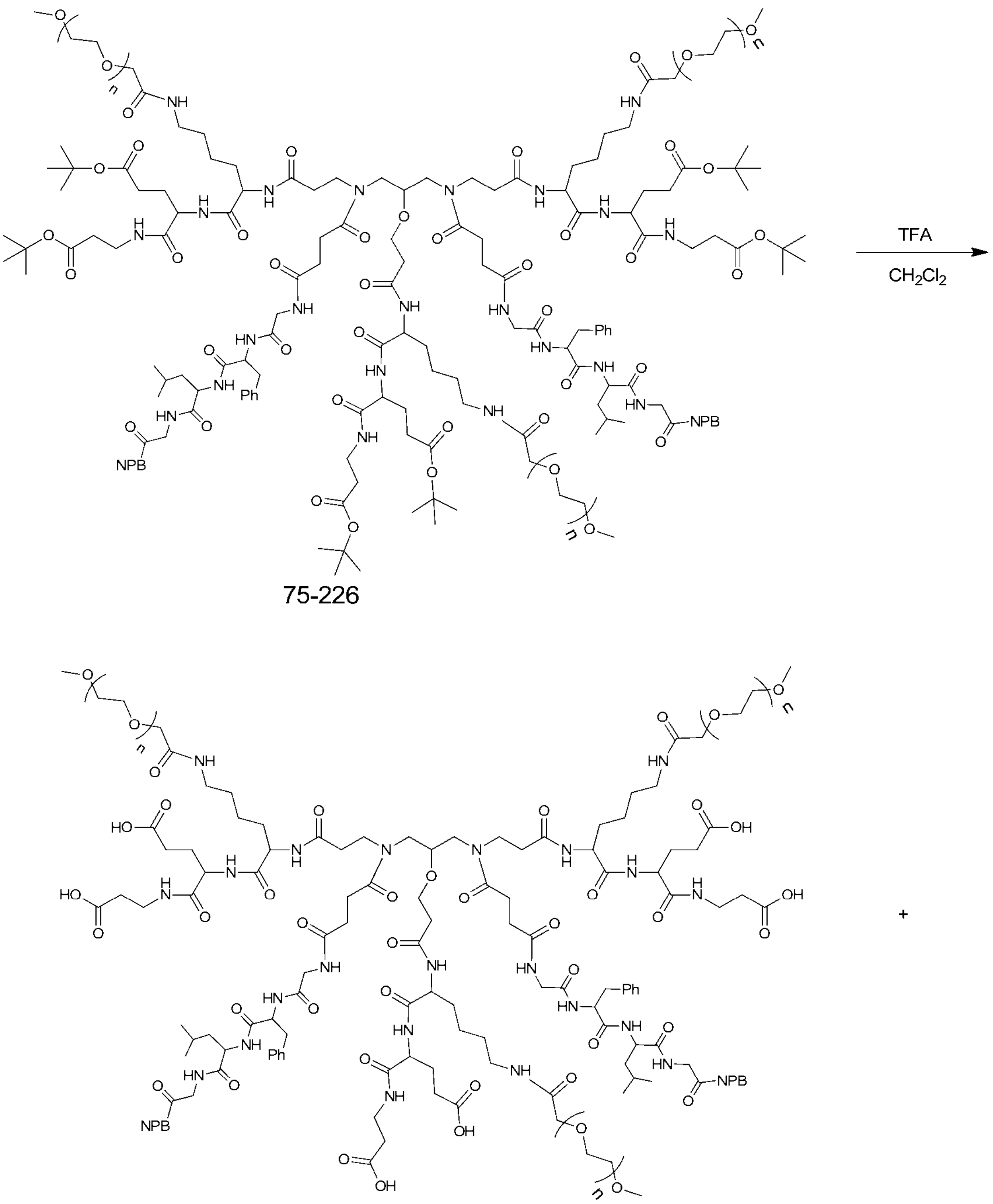


75-226

75-227

+

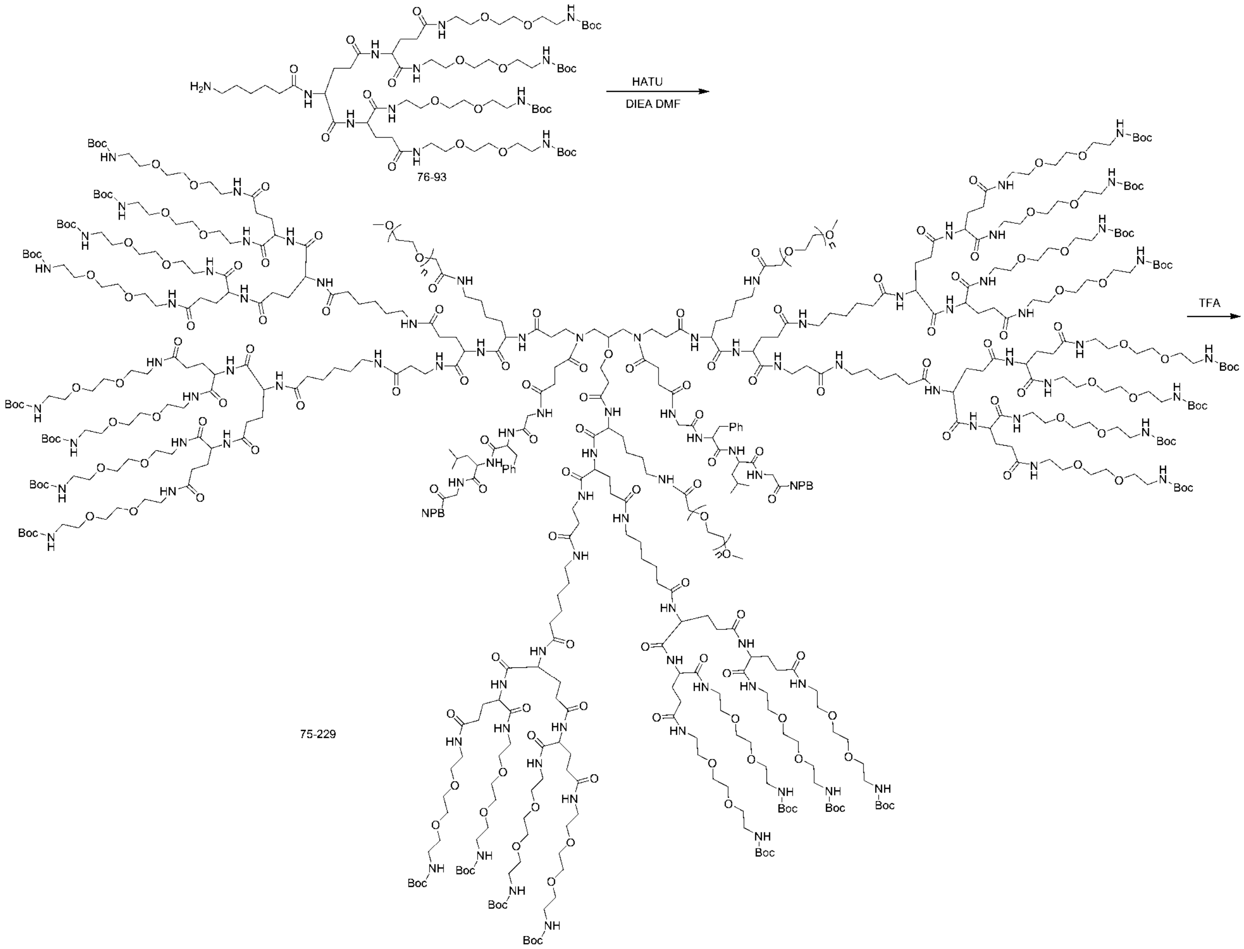


76-93

75-229

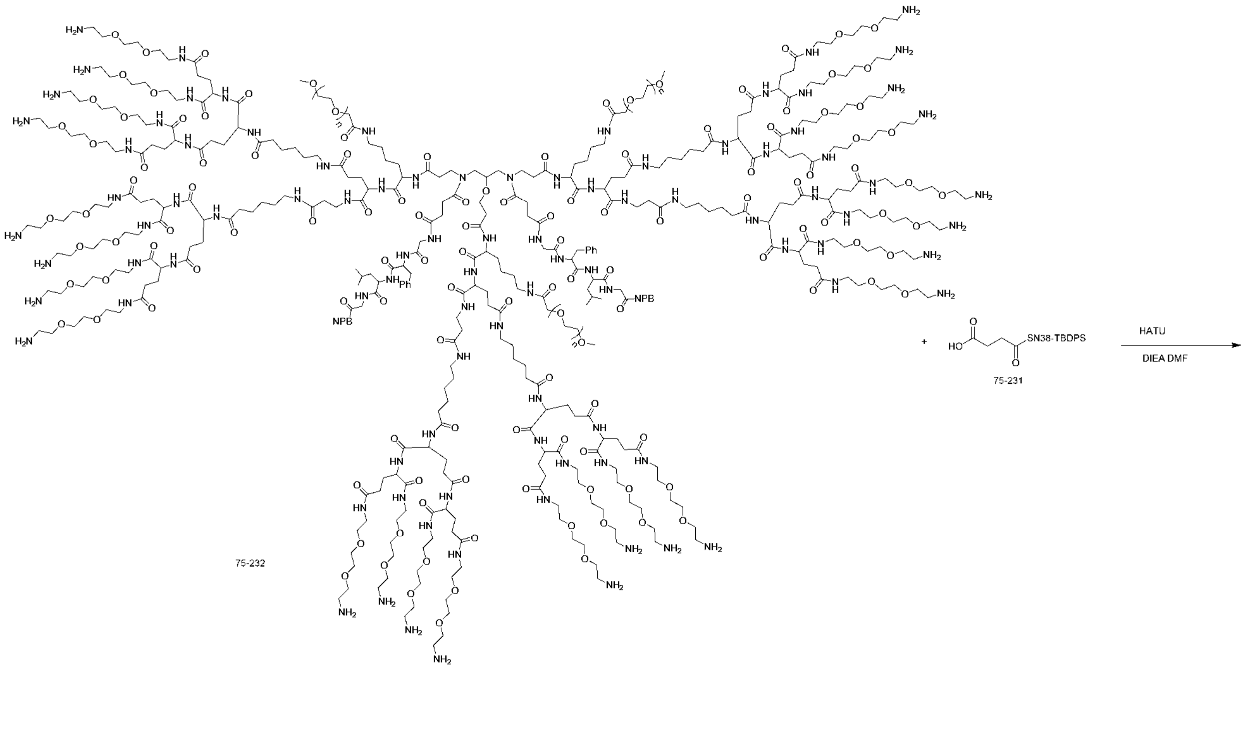


75-232

75-231

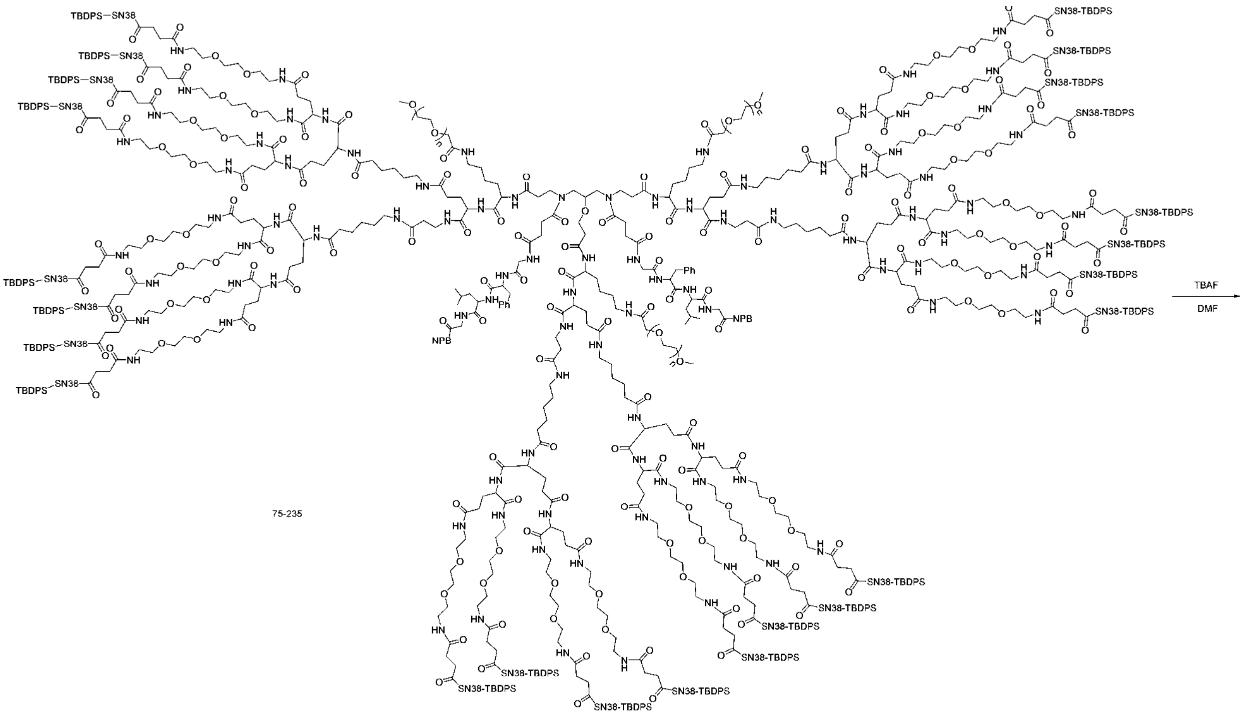


75-235

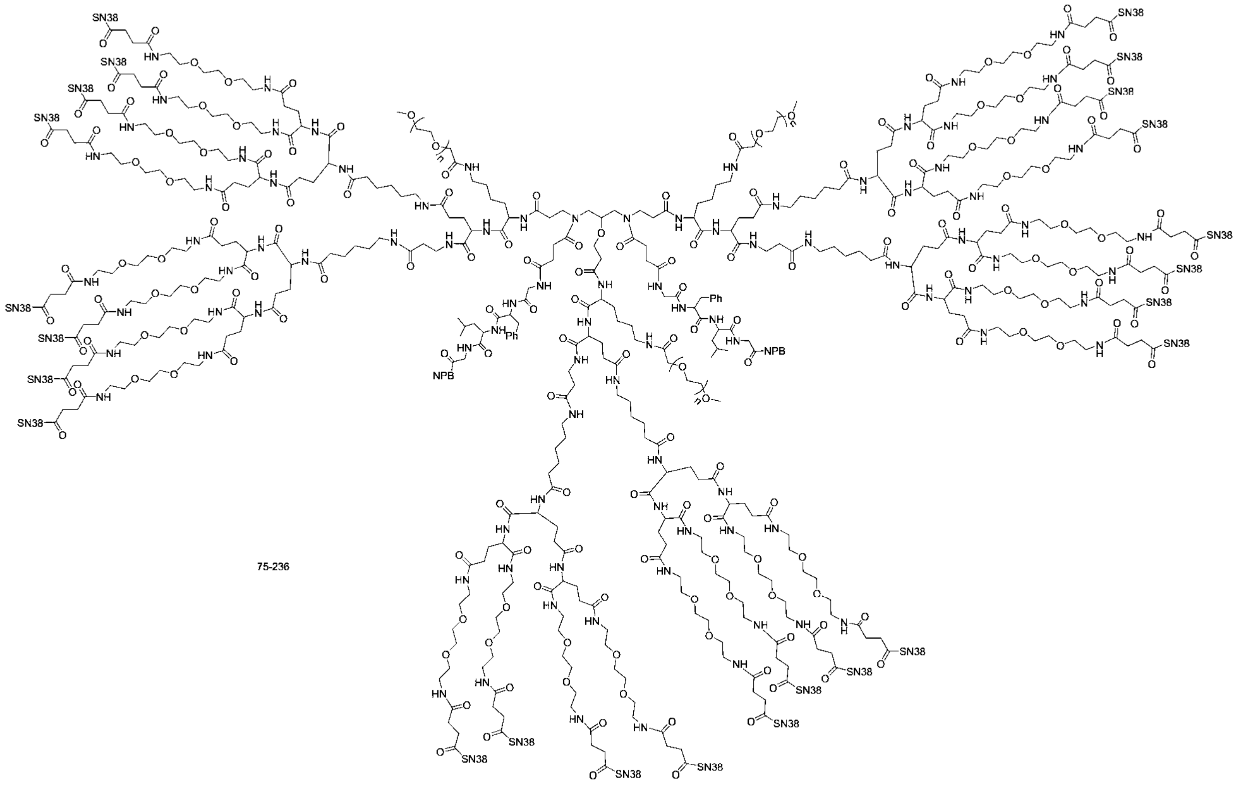

75-236

9.1 Preparation of Compound **75-228**

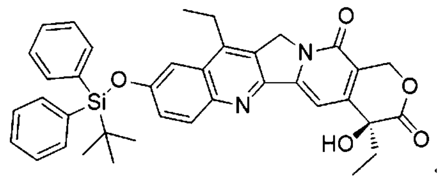

SN38 (2.0 g, 5.096 mmol) was weighed and added into a 250 mL flask, dichloromethane (80 mL) was added for dissolution with the assistance of ultrasonication, then TBDPS-Cl (5.3 mL, 20.387 mmol) and triethylamine (3.1 mL, 22.935 mmol) were added, and the mixture was reacted under stirring in an oil bath at 30°C under reflux for 12 h. After the reaction was completed, the reaction mixture was transferred to a 1 L separatory funnel, and 1N hydrochloric acid (150 mL) was added for extraction. The dichloromethane phase was collected and washed with sodium bicarbonate solution (150 mL × 2), and then with saturated sodium chloride solution. The dichloromethane phase was evaporated to dryness and dried in a vacuum oven to provide 2.17 g of the product.

9.2 Preparation of **75-231**

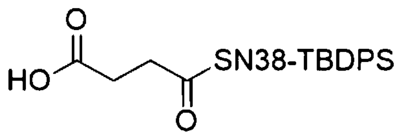

To a reaction flask containing **75-228** (2.17 g, 3.44 mmol) were added succinic anhydride (2.06 g, 20.640 mmol) and pyridine (20 mL). After dissolution with the assistance of ultrasonication, DMAP (0.84 g, 6.880 mmol) was added, and the mixture was stirred overnight in a 45°C oil bath. After the reaction was completed, as determined by TLC, a large amount of water was added to the reaction mixture, and the pH was adjusted to 2 with hydrochloric acid. The precipitated solid was filtered, and the filter cake was dried in a vacuum oven for 4 hours to provide 3.76 g of the product.

9.3 Preparation of **75-211**

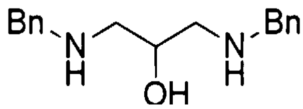

1,3-Diamino-2-propanol (2.0 g, 22.191 mmol) was dissolved in dichloromethane (30 mL) and stirred rapidly with a stirrer. Solid sodium sulfate (6.30 g, 44.383 mmol) was then added, followed by benzaldehyde (4.71 g, 44.383 mmol). The mixture was stirred at room temperature for 18 hours, and then filtered. The filtrate was concentrated. The residue was dissolved in anhydrous ethanol (20 mL) and cooled to 0°C. Solid $NaBH_4$ (5.95 g, 56.082 mmol) was then added in batches, over 10 minutes each. Stirring was performed for 1 hour. Water (10 mL) was added to the mixture. Then the mixture was concentrated, dissolved in ethyl acetate (50 mL), washed with hydrochloric acid (20 mL × 2), and adjusted with NaOH solution until the pH was to alkaline. The mixture was extracted with dichloromethane (30 mL × 3), the dichloromethane phases were combined, dehydrated over anhydrous magnesium sulfate, filtered, concentrated and dried to provide 4.6 g of the product with a yield of 57.14%. $^1$H-NMR (600 MHz, DMSO-$d_6$) δ 7.38-7.16 (m, 10H), 3.74-3.42 (m, 8H), 2.57-2.54 (m, 2H), 2.50-2.47 (m, 2H); ESI [M+H$^+$] 271.1796

9.4 Preparation of **75-213**

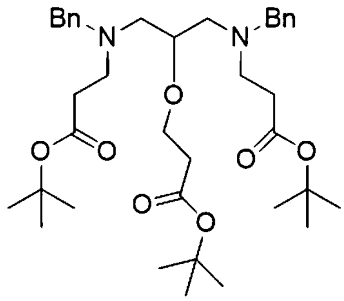

**75-211** (4.6 g, 17.013 mmol) was dissolved in DMSO (10 mL), and stirred in a water bath at 25°C. 5 mol/L NaOH solution was added dropwise. After stirring for 10 minutes, tert-butyl acrylate (14.75 mL, 102.082 mmol) was added dropwise. The reaction was allowed to proceed overnight and monitored by TLC. The mixture was extracted three times with ethyl acetate/saturated NaCl solution. The combined organic phases were evaporated to dryness, then dissolved in a mixed solvent. Silica gel powder was added, and the mixture was evaporated to dryness. The resulting product was loaded by dry method, and purified on a silica gel column using ethyl acetate/petroleum ether as the eluent. The desired product was collected and dried to provide 2.22 g of the product. $^1$H-NMR (600 MHz, DMSO-$d_6$) δ 7.32-7.17 (m, 10H), 3.70-3.62 (m, 2H), 3.53-3.50 (m, 4H), 3.48-3.42 (m, 1H), 2.70-2.56 (m, 4H), 2.47-2.42 (m, 2H), 2.40-2.33 (m, 4H), 2.33-2.27 (m, 4H), 1.36 (s, 27H); ESI [M+H$^+$] 655.42950

9.5 Preparation of **75-214**

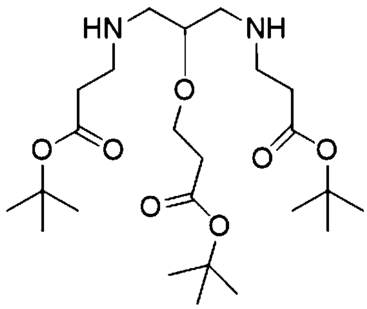

**75-213** (2.22 g, 3.402 mmol) was placed in a hydrogenation reactor, then 10% Pd/C catalyst (0.4 g) was added. Methanol (30 mL) was added for dissolution, then hydrogen gas was introduced at a pressure of 200 psi, and the reaction was carried out under stirring at room temperature overnight. After the reaction was completed, the reaction mixture was filtered through Celite, which was then washed with methanol (5 mL × 3). The methanol phases were combined, then dichloromethane was added, and the mixture was evaporated to dryness using a rotary evaporator. Dichloromethane and toluene (0.2 mL) were added, then the mixture was treated by ultrasonication and evaporated to dryness. This process was repeated five times. Dichloromethane was added for dissolution with the assistance of ultrasonication and evaporated to dryness. This process was repeated five times. Finally, the resulting product was concentrated and dried to provide 1.6 g of the product.

9.6 Preparation of **75-215**

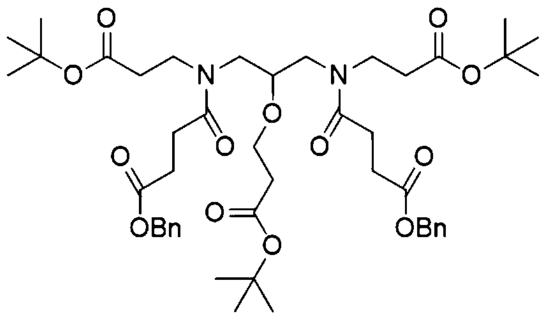

**75-214** (0.6 g, 1.264 mmol), mono-benzyl succinate (0.55 g, 2.654 mmol) and HATU (1.15 g, 3.033 mmol) were added to a 250 mL flask, then DMF (20 mL) was added to dissolve the mixture, and the contents were stirred at -5°C for approximately 20 minutes. DIEA (0.9 mL, 5.561 mmol) was then slowly added dropwise. After the dropwise addition was completed, the mixture was stirred at -5°C for 20 minutes, and then the mixture was brought to room temperature and reacted under stirring. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated 5 times. The mixture was filtered to obtain a solid product. The filter cake was collected and dried in a vacuum oven to provide 1.08 g of the product. $^1$H-NMR (600 MHz, DMSO-$d_6$) δ 7.39-7.30 (m, 10H) 5.11-5.06 (m, 4H), 4.03 (s, 1H), 3.79-3.36 (m, 8H), 3.30-3.25 (m, 2H), 2.66-2.53 (m, 8H), 2.42-2.32 (m, 6H), 1.41-1.35 (m, 27H); ESI [M+Na$^+$] 877.442

9.7 Preparation of **75-217**

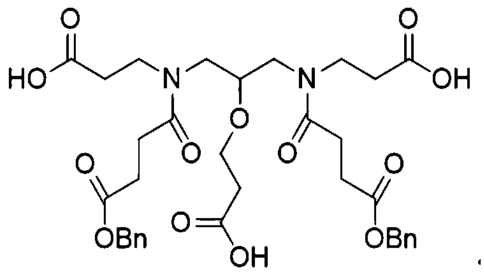

To a flask containing **75-215** (1.08 g, 1.264 mmol) was added dichloromethane (5 mL). After dissolution with the assistance of ultrasonication, TFA (5.6 mL, 75.84 mmol) was added, and the reaction was stirred at room temperature. After the reaction was completed, the reaction mixture was concentrated and evaporated to dryness to remove the dichloromethane and most of the TFA. Then, n-hexane (200 mL) and methyl tert-butyl ether (200 mL) were added for precipitation to provide an oily solid. The supernatant was discarded, and ethyl acetate (15 mL) was added. Ultrasonication was performed to obtain a white, turbid liquid, to which was then added n-hexane (200 mL) for precipitation to provide a powdery solid. The mixture was filtered by suction, and the filter cake was washed three times with methyl tert-butyl ether (40 mL). The filter cake was collected and dried under vacuum to provide 0.86 g of the product. ESI [M+Na$^+$] 709.254

9.8 Preparation of **75-220**

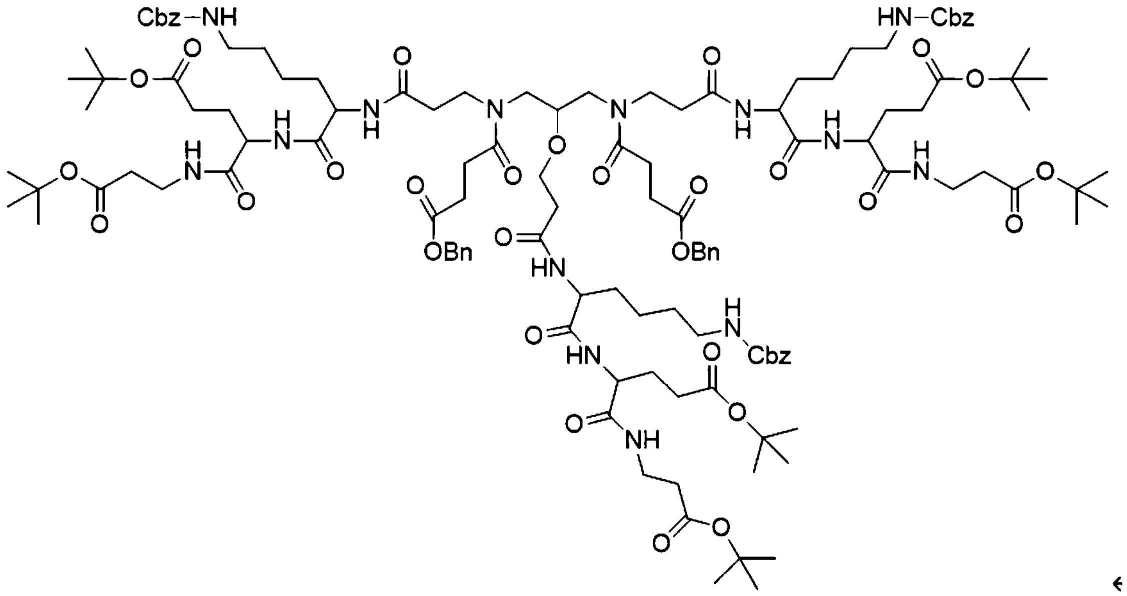

**75-217** (0.86 g, 1.264 mmol), **76-58** (2.42 g, 4.171 mmol), HBTU (2.15 g, 5.688 mmol) and HOBT (0.77 g, 5.688 mmol) were added into a 500 mL round-bottom flask, then DMF (15 mL) was added to dissolve the mixture, and the contents were stirred at -5°C for approximately 20 minutes. DIEA (2.8 mL, 17.064 mmol) was then slowly added dropwise, and the reaction was stirred at -5°C for 1 hour. The mixture was taken out and reacted under stirring at room temperature. After the reaction was completed, the reaction mixture was transferred to a 1 L separatory funnel, and deionized water (250 mL) and ethyl acetate (200 mL) were added for extraction. The organic phase (with solid product precipitated) was obtained, and the aqueous phase was further extracted with ethyl acetate (200 mL × 2). The organic phases were combined and concentrated by rotary evaporation under reduced pressure. The obtained solid product was dissolved in a dichloromethane/methanol (4/1) mixture, then silica gel powder was added, and the mixture was evaporated to dryness. The resulting product was loaded by dry method, and subjected to column chromatography using a 2-6% methanol/dichloromethane mixture as the eluent. The organic solvent containing the product component was collected, evaporated to dryness, and dried in a vacuum oven to provide 1.1 g of the product. $^{1}$H-NMR (600 MHz, DMSO-$d_6$) δ 8.05-7.86 (m, 9H), 7.37-7.32 (m, 25H), 7.24-7.17 (m, 3H), 5.07-5.06 (m, 4H), 4.99 (s, 6H), 4.27-4.13 (m,6H), 3.57-3.46(m, 13H), 3.30-3.28 (m, 4H), 3.20-3.11 (m, 6H), 2.63-2.56 (m, 10H), 2.55-2.52 (m,8H), 2.36-2.31 (m,8H), 2.19-2.13 (m,6H), 1.87-1.77 (m, 6H), 1.52-1.43 (m, 6H), 1.39 (s, 54H), 1.24-1.23 (m, 6H)

9.9 Preparation of **75-222**

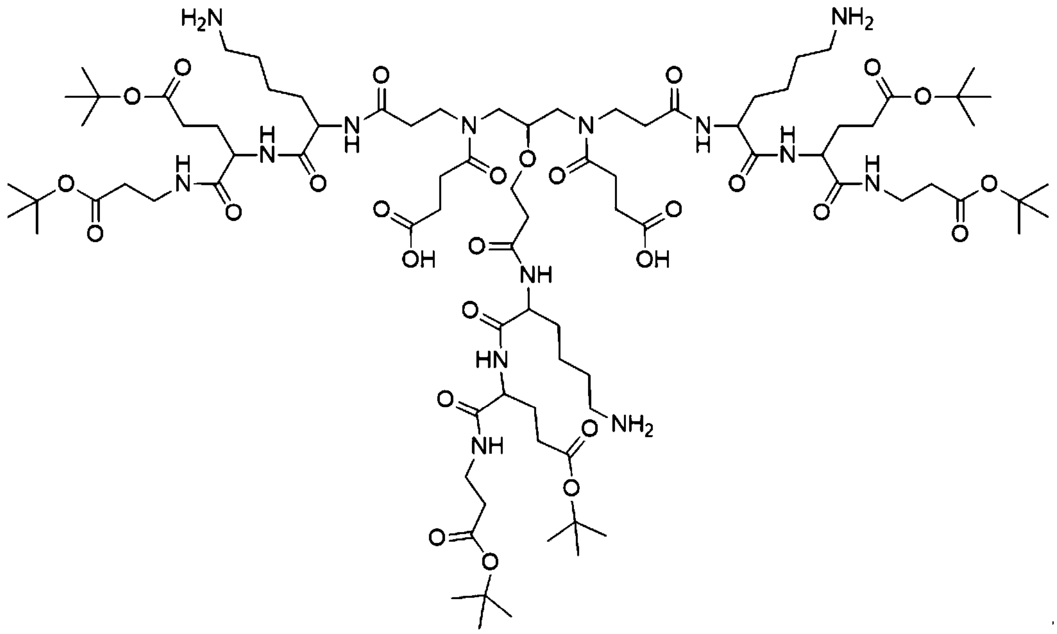

**75-220** (0.42 g, 1.3688 mmol) was placed in a hydrogenation reactor, then 10% Pd/C catalyst (0.1 g) was added. DMF (30 mL) was added to dissolve the mixture. Hydrogen gas was introduced at a pressure of 300 psi, and the reaction was stirred at room temperature overnight. After the reaction was completed, the reaction mixture was filtered through Celite, the filter cake was washed with DMF (5 mL × 3), and the filtrate was transferred to a 500 mL round-bottom flask. Methyl tert-butyl ether (150 mL) and n-hexane (80 mL) were then added to the filtrate for precipitation, then the supernatant was discarded, and methyl tert-butyl ether (150 mL) and n-hexane (80 mL) were added again for precipitation. This process was repeated five times. Finally, the mixture was filtered and dried to provide 0.24 g of the product.

9.10 Preparation of **75-224**

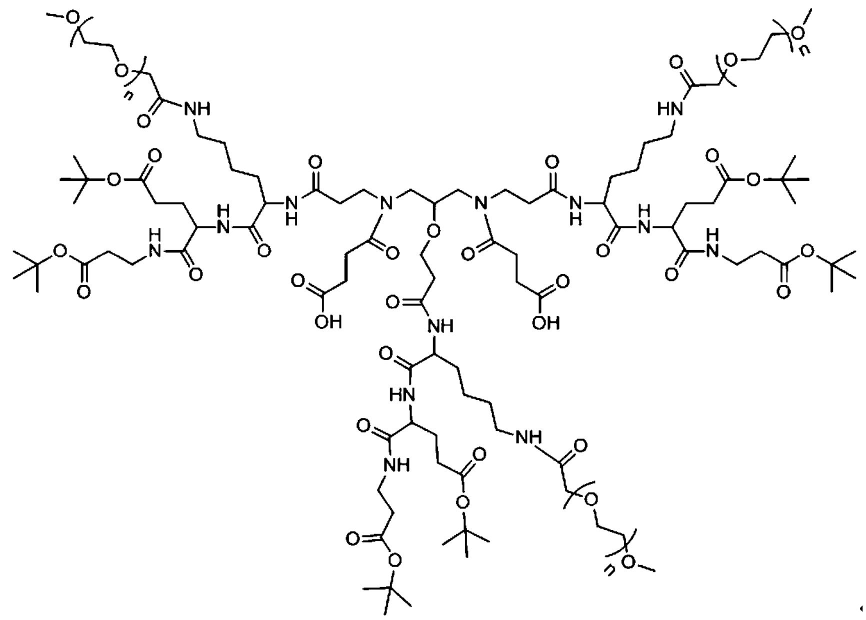

**75-222** (0.088 g, 0.0482 mmol) was dissolved in anhydrous DMF (5 mL) in a 250 mL round-bottom flask, followed by a dropwise addition of DIEA (0.95 mL, 5.787 mmol). The mixture was stirred for 30 minutes. Polymer M-SCM-10K (1.5 g, 0.1519 mmol) was then added. After dissolution with the assistance of ultrasonication, the reaction was carried out under stirring in the dark at a low speed for four days. After the reaction was completed, methyl tert-butyl ether (150 mL) and n-hexane (70 mL) were added, and the supernatant was discarded. This process was repeated three times. The obtained solid was dissolved in a mixed solvent, then silica gel powder was added, and the mixture was evaporated to dryness. The resulting product was loaded by dry method, and subjected to column chromatography using 1% ammonia water/5-10% methanol/dichloromethane as the eluent. The resulting product was collected and dried to provide 1.46 g of the product. $^{1}$H-NMR (600 MHz, DMSO-$d_6$) δ 9.72-9.69 (m, 2H), 8.09-8.04 (m, 12H), 4.19-4.16 (m,6H), 3.86-3.83(m, 13H), 3.53-3.50 (m, 2871H), 3.24-3.23 (m, 4H), 2.62-2.61 (m, 16H), 2.39-2.38 (m, 16H), 2.19-2.14 (m,6H), 1.88-1.79 (m,6H), 1.53-1.45 (m,6H), 1.39-137 (m, 54H),1.25-1.21 (m, 6H)

9.11 Preparation of **75-226**

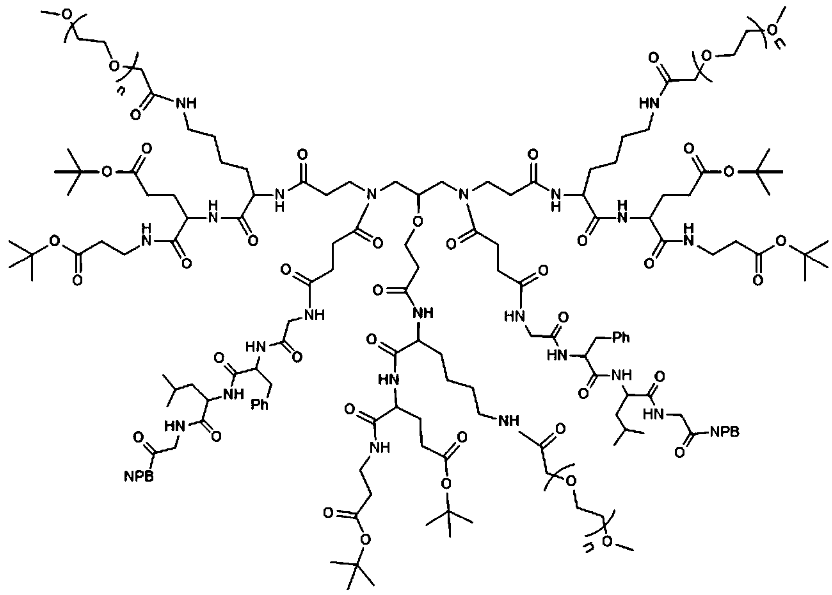

**75-224** (1.46 g, 0.0437 mmol), **70-68** (0.066 g, 0.0961 mmol), HBTU (0.049 g, 0.1311 mmol) and HOBT (0.017 g, 0.1311 mmol) were added into a 250 mL flask, then DMF (7 mL) was added to dissolve the mixture, and the contents were stirred at room temperature for approximately 20 minutes. DIEA (0.1 mL, 0.3935 mmol) was then slowly added dropwise. After the dropwise addition was completed, the mixture was stirred at room temperature overnight. TLC was then used to monitor the reaction. After the reaction was completed, methyl tert-butyl ether (150 mL) and n-hexane (70 mL) were added for precipitation. The supernatant was discarded, and methyl tert-butyl ether (150 mL) and n-hexane (70 mL) were added again for precipitation. This process was repeated three times. The mixture was filtered and dried to provide 1.52 g of the product. $^{1}$H-NMR (600 MHz, DMSO-$d_6$) δ 8.58-8.55 (m, 2H), 8.22-8.19 (m, 2H), 8.13-8.01 (m, 16H), 7.91-7.86 (m, 6H), 7.70-7.64 (m,2H), 7.54-7.51 (m,2H), 7.29-7.21 (m, 16H), 7.20-7.14 (m, 4H), 4.37-4.34 (m, 10H), 4.06-4.04 (m, 9H), 3.63-3.62 (m, 24H), 3.53-350 (m, 2871H), 3.44-3.43 (m, 10H), 2.62-2.61 (m, 20H), 2.39-2.38 (m, 8H), 2.36-2.34 (m, 6H), 1.82-1.74 (m, 10H), 1.53-1.47 (m, 16H), 1.38-136 (m, 54H), 1.24-1.22 (m, 6H), 0.90-0.88 (m, 12H)

9.12 Preparation of **75-227**

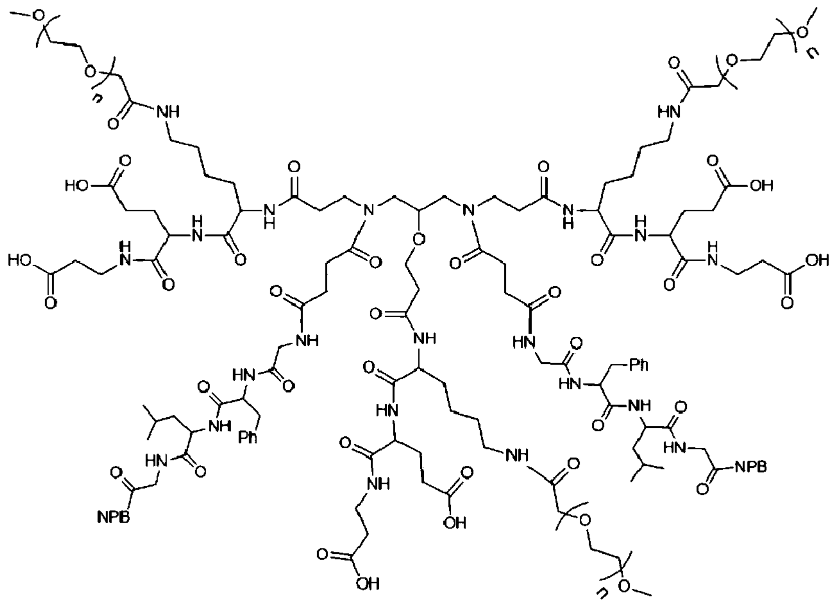

**75-226** (1.52 g, 0.0437 mmol) was placed in a 250 mL flask, and dissolved by adding dichloromethane (5 mL). TFA (0.4 mL, 5.249 mmol) was added while stirring. The reaction flask was allowed to stir at room temperature and the reaction was carried out under stirring overnight. After the reaction was completed, the reaction mixture was concentrated under reduced pressure to remove dichloromethane and most of the TFA, methyl tert-butyl ether (150 mL) and n-hexane (70 mL) were added for precipitation, the supernatant was discarded, and methyl tert-butyl ether (150 mL) and n-hexane (70 mL) were added again for precipitation. This process was repeated three times. The mixture was filtered and dried to provide 1.34 g of the product.

9.13 Preparation of **75-229**

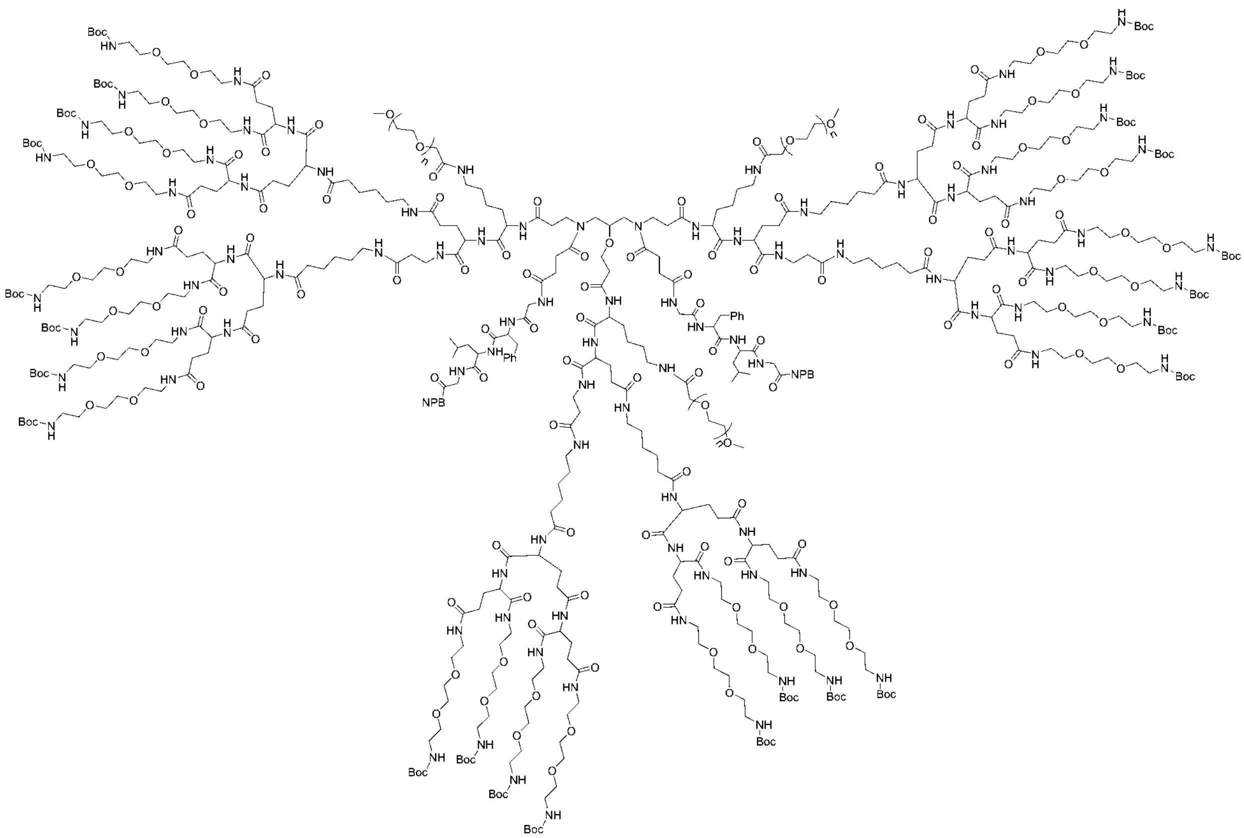

**75-227** (1.34 g, 0.0889 mmol), **76-93** (0.504 g, 0.3501 mmol) and HATU (0.106 g, 0.2801 mmol) were mixed and added into a 250 mL flask, then DMF (5 mL) was added to dissolve the mixture, and the contents were stirred at room temperature for approximately 20 minutes. DIEA (0.2 mL, 1.0503 mmol) was then slowly added dropwise. After the dropwise addition was completed, the mixture was stirred at room temperature overnight, and the reaction was monitored by TLC. After the reaction was completed, methyl tert-butyl ether (150 mL) and n-hexane (70 mL) were added for precipitation, the supernatant was discarded, and methyl tert-butyl ether (150 mL) and n-hexane (70 mL) were added again for precipitation. This process was repeated three times. The mixture was filtered and dried to provide 1.67 g of the product.

9.14 Preparation of **75-232**

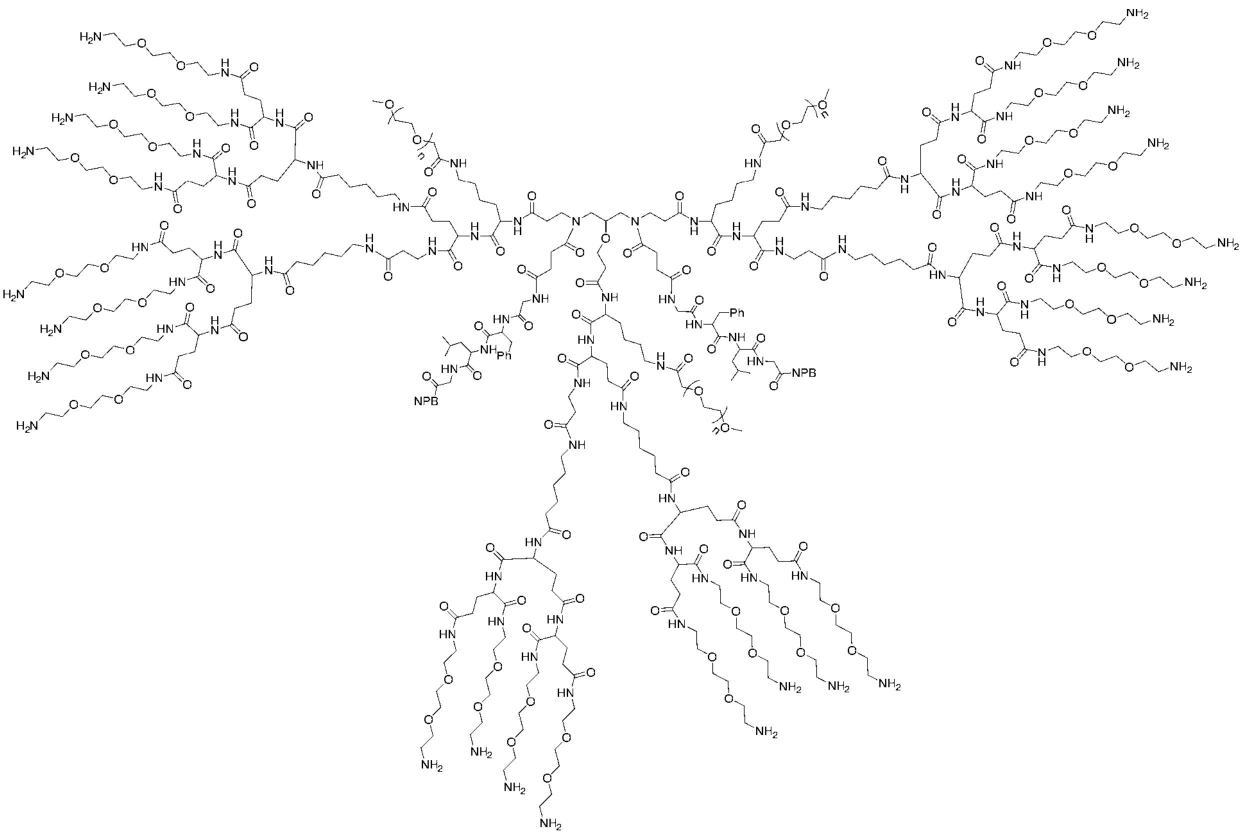

**75-229** (0.1 g, 0.00232 mmol) was placed in a 250 mL flask, and dissolved by adding dichloromethane (2 mL). TFA (0.08 mL, 1.1169 mmol) was added while stirring. The reaction was carried out under stirring at room temperature overnight. After the reaction was completed, the reaction mixture was concentrated under reduced pressure to remove dichloromethane and most of the TFA, methyl tert-butyl ether (150 mL) and n-hexane (70 mL) were added for precipitation, the supernatant was discarded, and methyl tert-butyl ether (150 mL) and n-hexane (70 mL) were added again for precipitation. This process was repeated three times. The mixture was filtered and dried to provide 0.06 g of the product. $^{1}$H-NMR (600 MHz, DMSO-$d_6$) δ 8.67-8.62 (m, 2H), 8.60-8.54 (m, 2H), 8.18-7.98 (m, 70H), 7.65-7.49 (m, 10H), 7.28-7.17 (m, 14H), 5.34-5.17 (m, 48H), 4.61-4.50 (m, 10H), 4.45-4.31 (m, 18H), 4.05-4.01 (m, 9H), 3.61-3.61 (m, 108H), 3.59-3.57 (m, 108H), 3.53-350 (m, 2871H), 3.20-3.17 (m, 58H), 2.99-2.94 (m, 54H), 2.82-2.73 (m, 20H), 2.62-2.61 (m, 12H), 2.40-2.36 (m, 8H), 2.16-2.07 (m, 80H), 1.76-1.73 (m, 14H), 1.54-1.47 (m, 28H), 1.29-1.18 (m, 30H), 0.93-0.80 (m, 12H)

9.15 Preparation of **75-235**

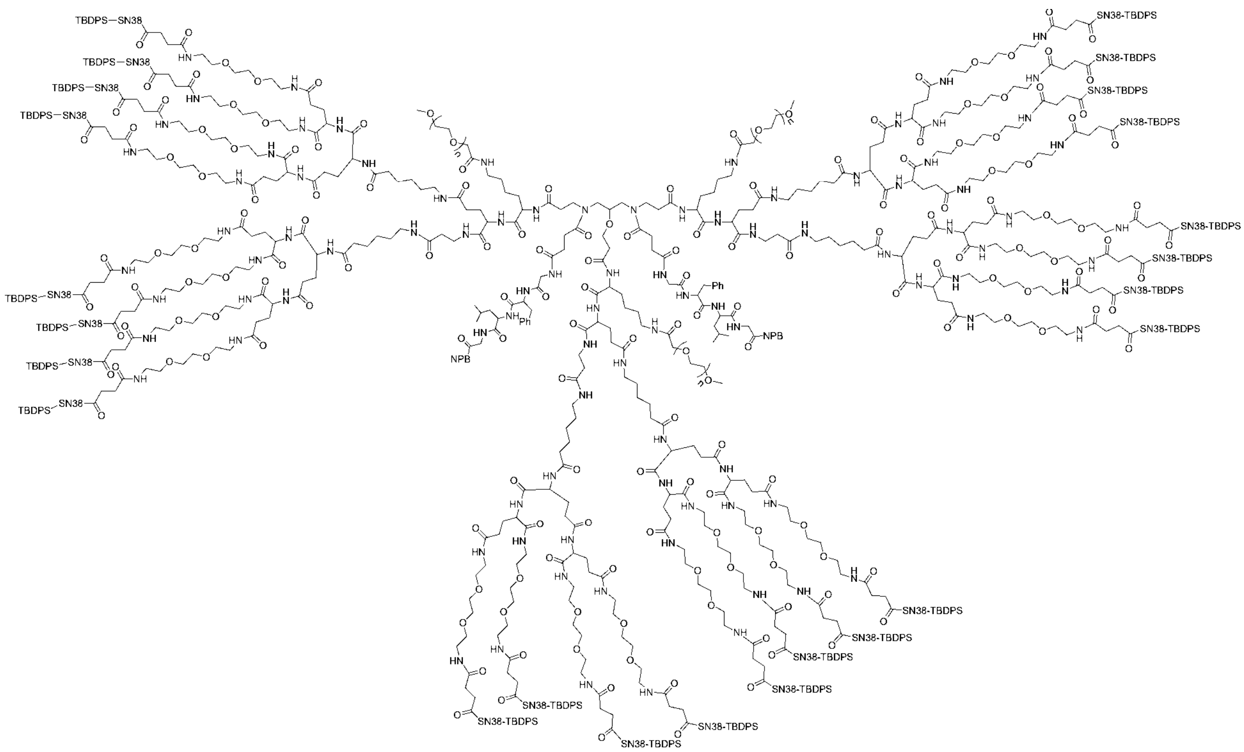

**75-232** (0.06 g, 0.0014 mmol), **75-231** (0.037 g, 0.0517 mmol) and HATU (0.015 g, 0.0413 mmol) were mixed and added to a 50 mL flask, and DMF (1 mL) was added to dissolve the mixture. The reaction was carried out under stirring at room temperature for approximately 20 minutes. DIEA (0.025 mL, 0.1551 mmol) was then slowly added dropwise. After the dropwise addition was completed, the mixture was stirred at room temperature overnight. TLC was used to monitor the reaction. After the reaction was completed, methyl tert-butyl ether (150 mL) and n-hexane (70 mL) were added for precipitation, the supernatant was discarded, and methyl tert-butyl ether (150 mL) and n-hexane (70 mL) were added again for precipitation. This process was repeated three times. The mixture was filtered and dried to provide 0.04 g of the product.

9.16 Preparation of **75-236**

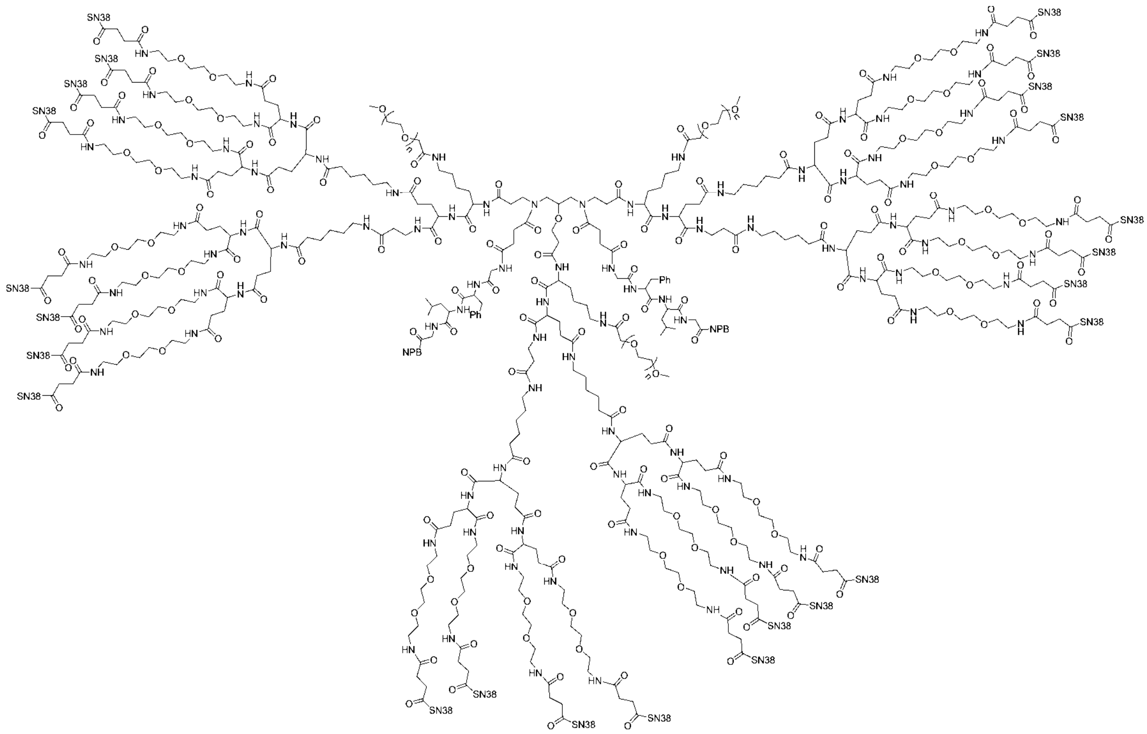

**75-235** (0.04 g, 0.000679 mmol) was dissolved in THF (1 mL), and TBAF (0.1 g, 0.326 mmol) was added and reacted for 2 h. The mixture was then concentrated under reduced pressure, methyl tert-butyl ether (150 mL) and n-hexane (70 mL) were added for precipitation, the supernatant was discarded, and methyl tert-butyl ether (150 mL) and n-hexane (70 mL) were added again for precipitation. This process was repeated three times. The mixture was filtered and dried to provide 0.04 g of the product. $^1$H-NMR (600 MHz, DMSO-*d$_6$*) δ 8.59-8.56 (m, 2H), 8.24-8.22 (m, 2H), 8.10-7.99 (m, 118H), 7.85-7.81 (m, 24H), 7.57-7.56 (m,10H), 7.41-7.39 (m,62H), 6.82-6.80 (m, 24H), 5.36-5.32 (m, 24H), 5.17-5.02 (m, 48H), 4.94-4.93 (m, 2H), 4.26-4.16 (m, 48H), 4.12-4.06 (m, 26H), 4.05-4.03 (m, 4H), 3.72-3.65 (m, 124H), 3.64-3.61 (m, 108H), 3.53-350 (m, 2871H), 3.25-3.23 (m, 64H), 3.09-3.07 (m, 54H), 2.85-2.75 (m, 168H),2.46-2.45 (m,16H), 2.14-2.12 (m, 90H), 1.90-1.75 (m, 62H), 1.48-1.45 (m, 24H), 1.29-1.27 (m, 79H), 0.91-0.90 (m, 60H)

Example 10: Synthesis of Compound **74-232**

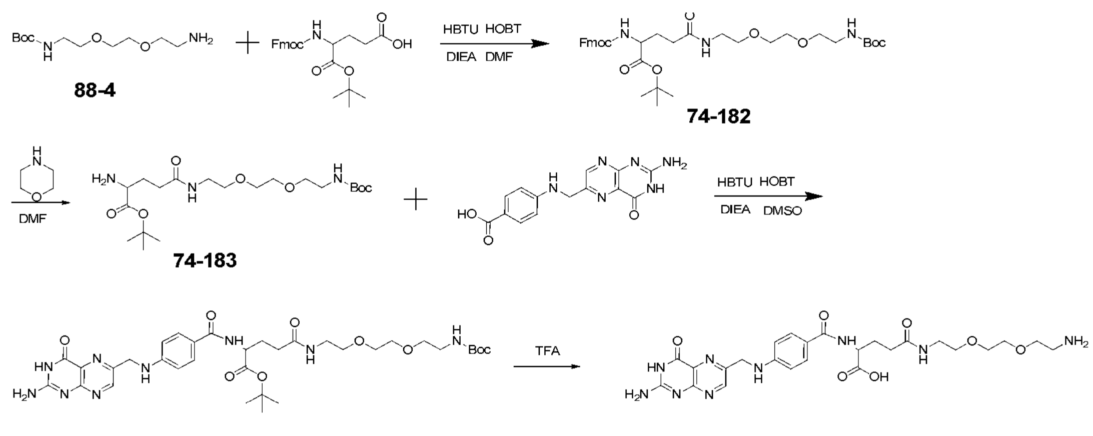


**88-4** **74-182** **74-183** **74-184** **74-185**

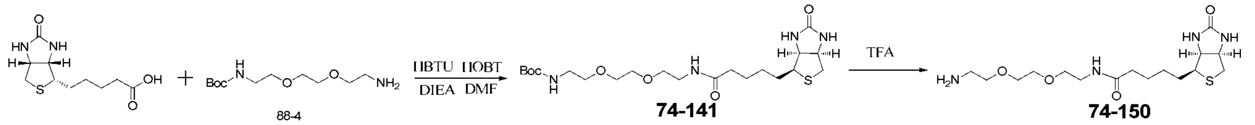


88-4

74-141

74-150

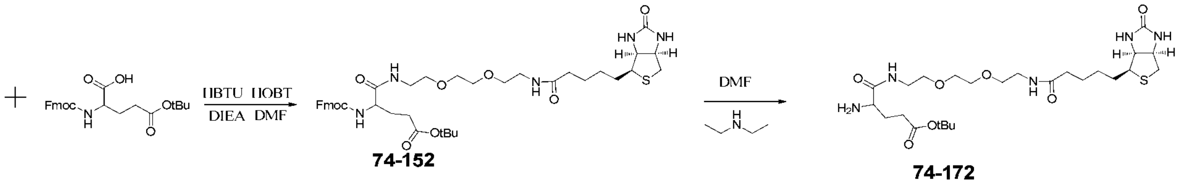


74-152

74-172

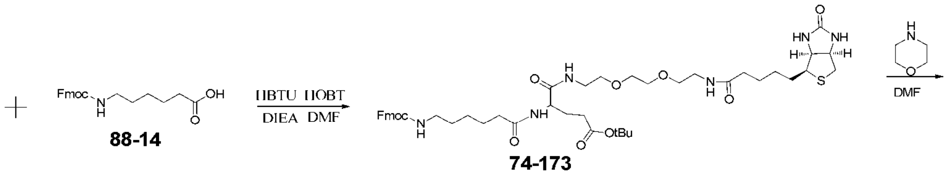


88-14

74-173

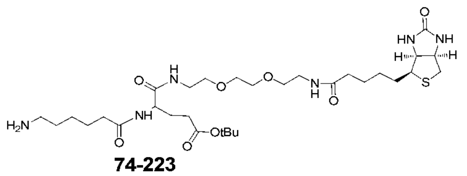

74-223

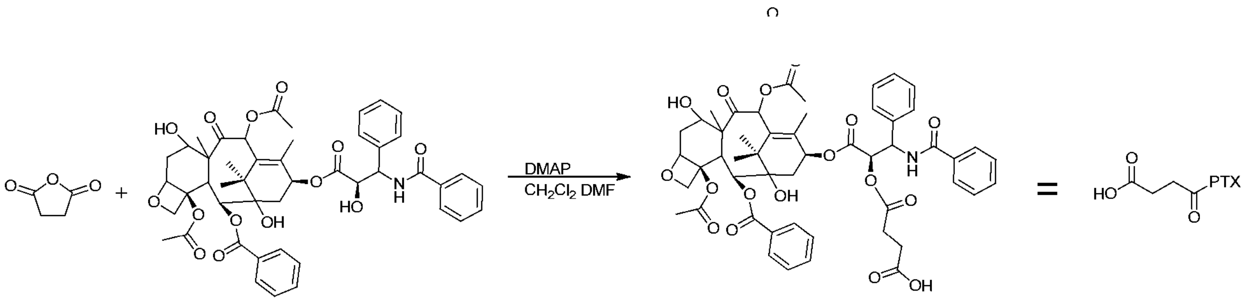


77-163

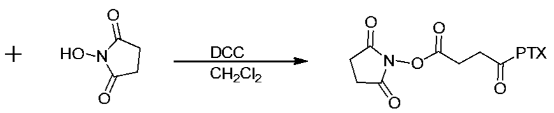


77-164

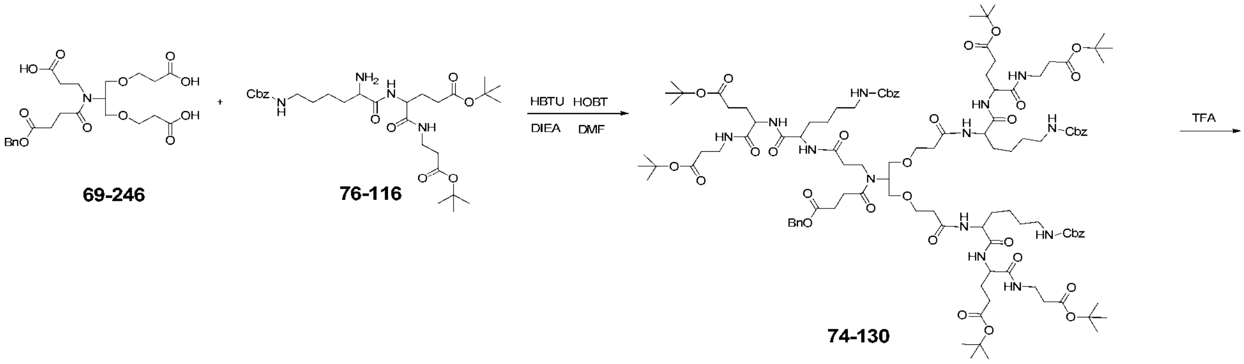


69-246 76-116 74-130

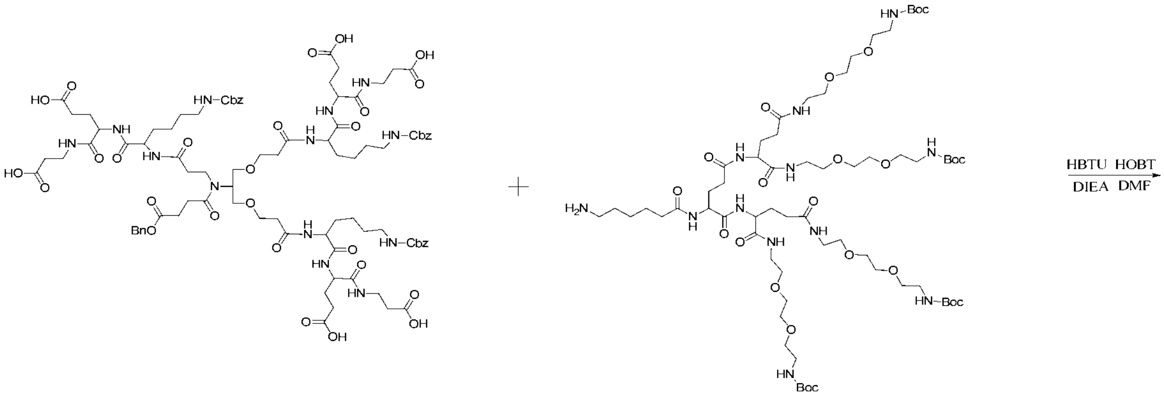


74-214

76-93

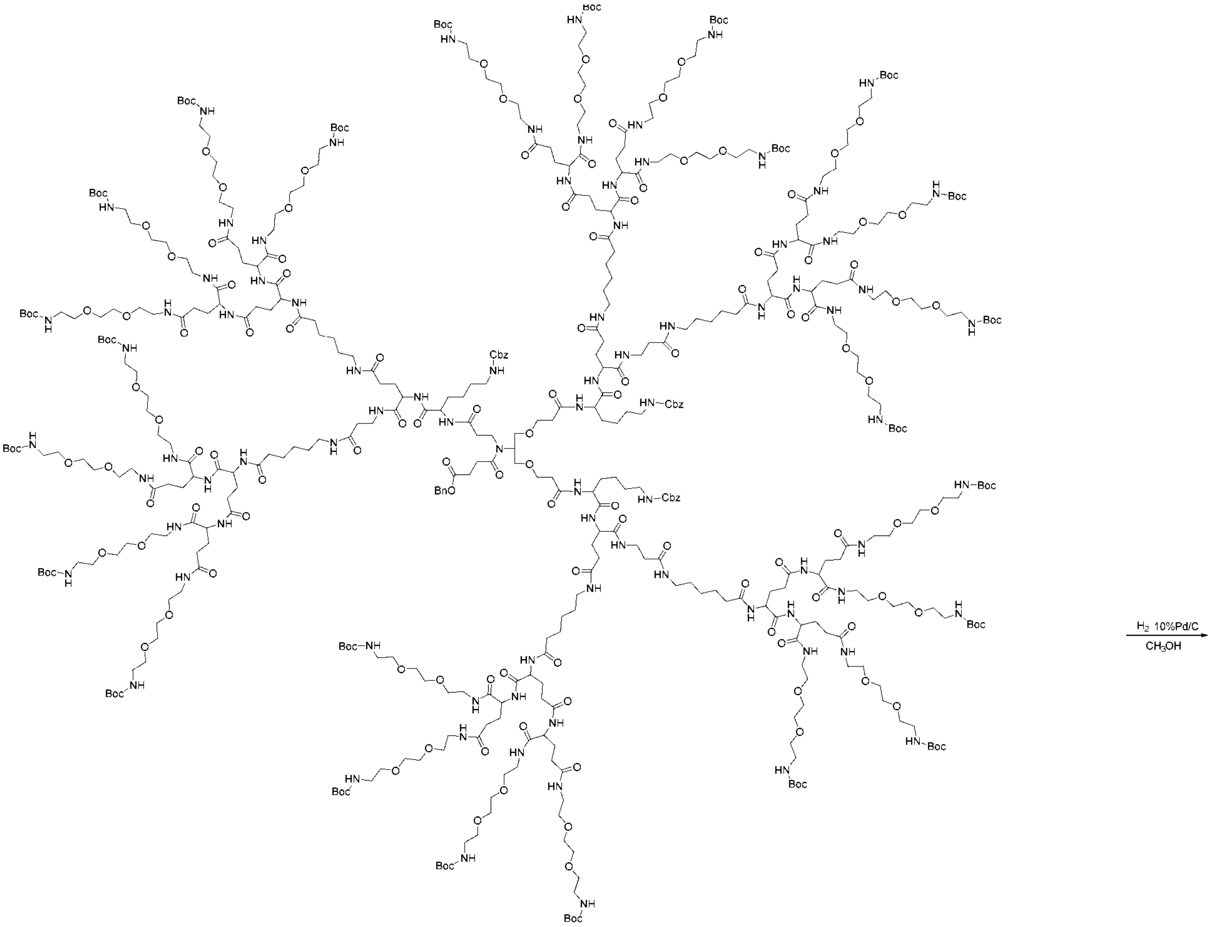


74-215

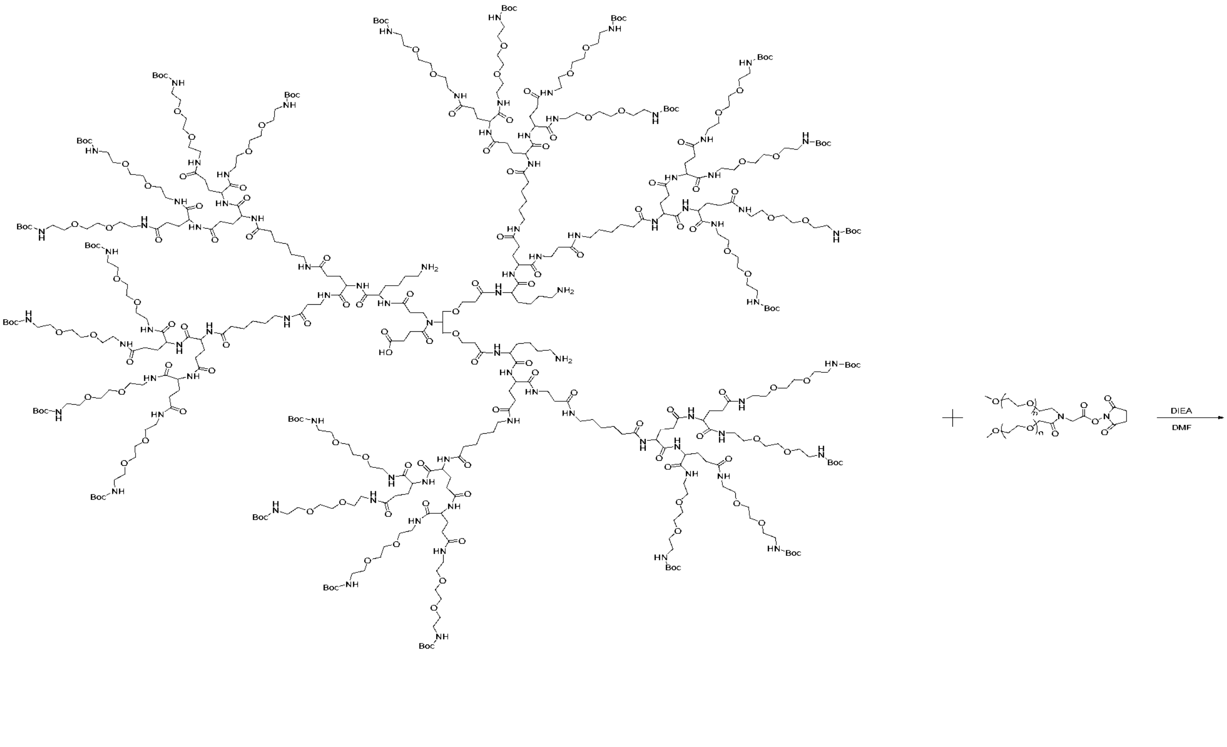


74-216

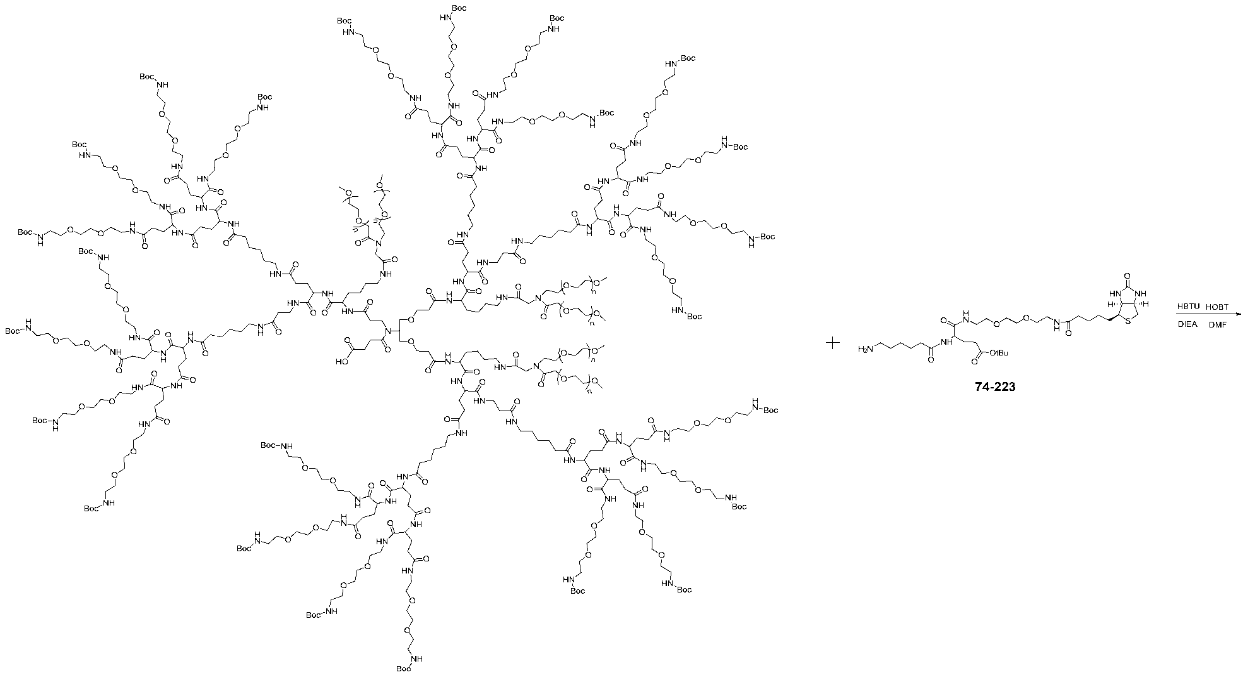


74-223

74-217

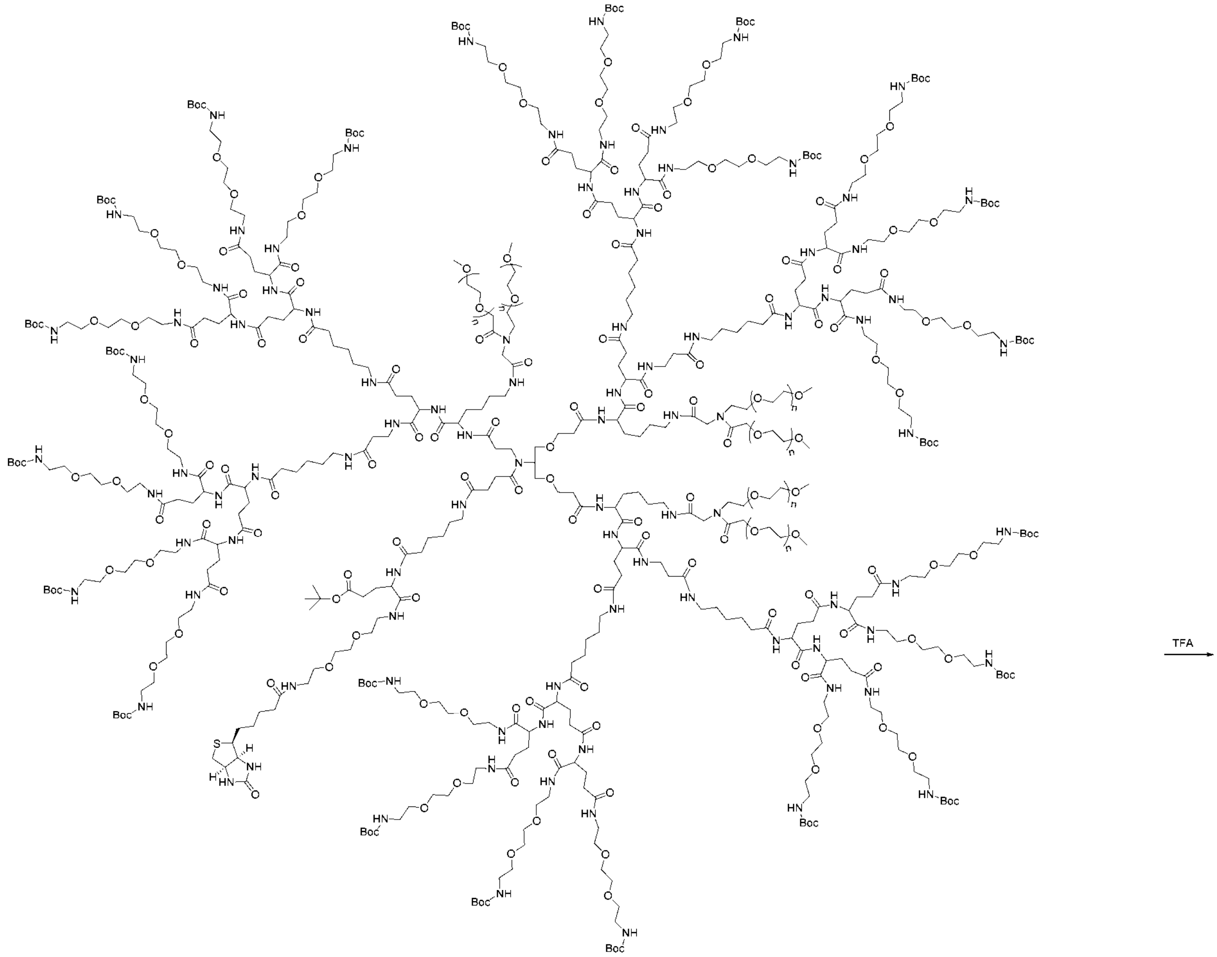


74-225

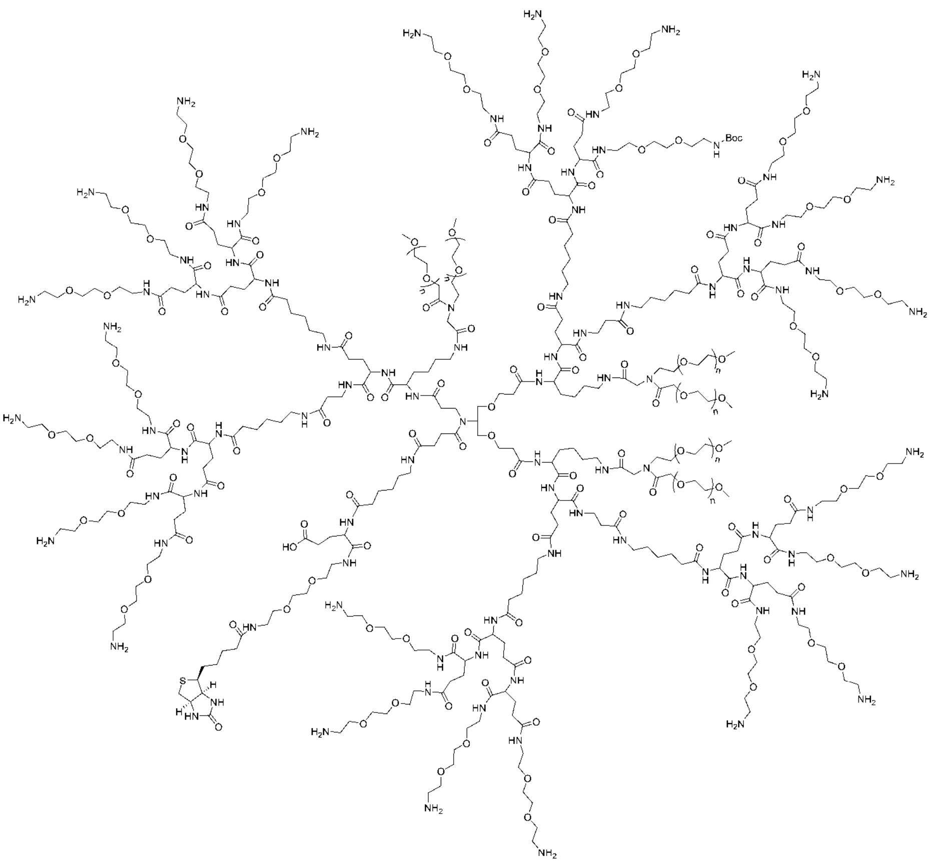

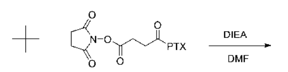


77-164

74-228

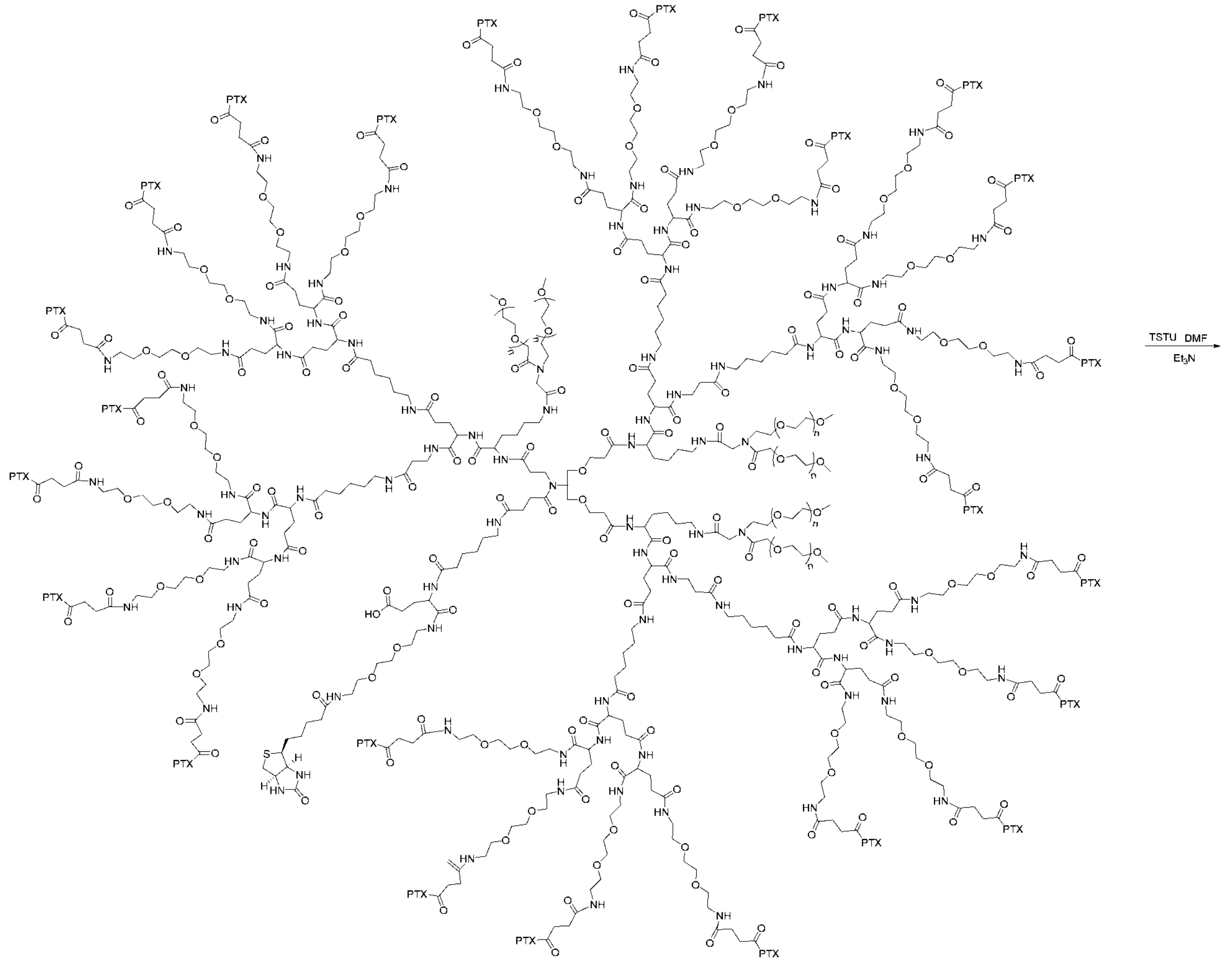


74-229

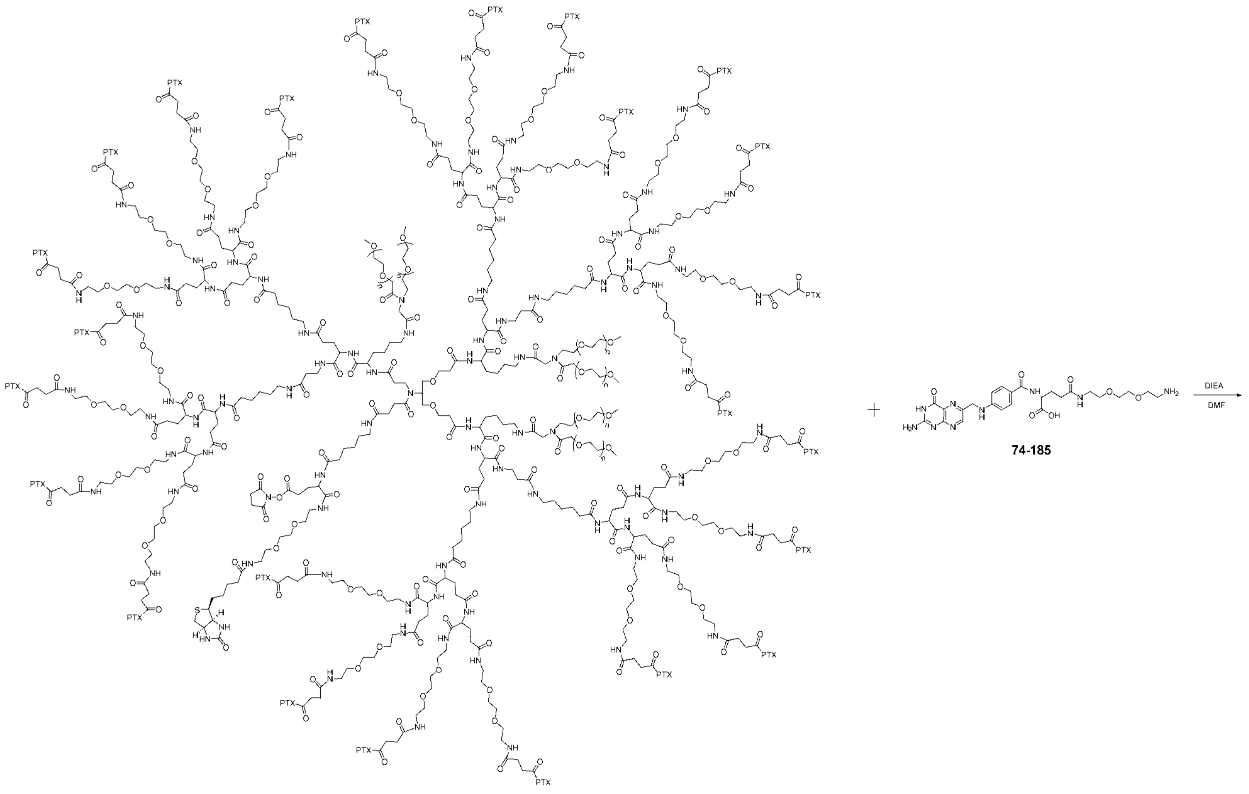


74-185

74-230

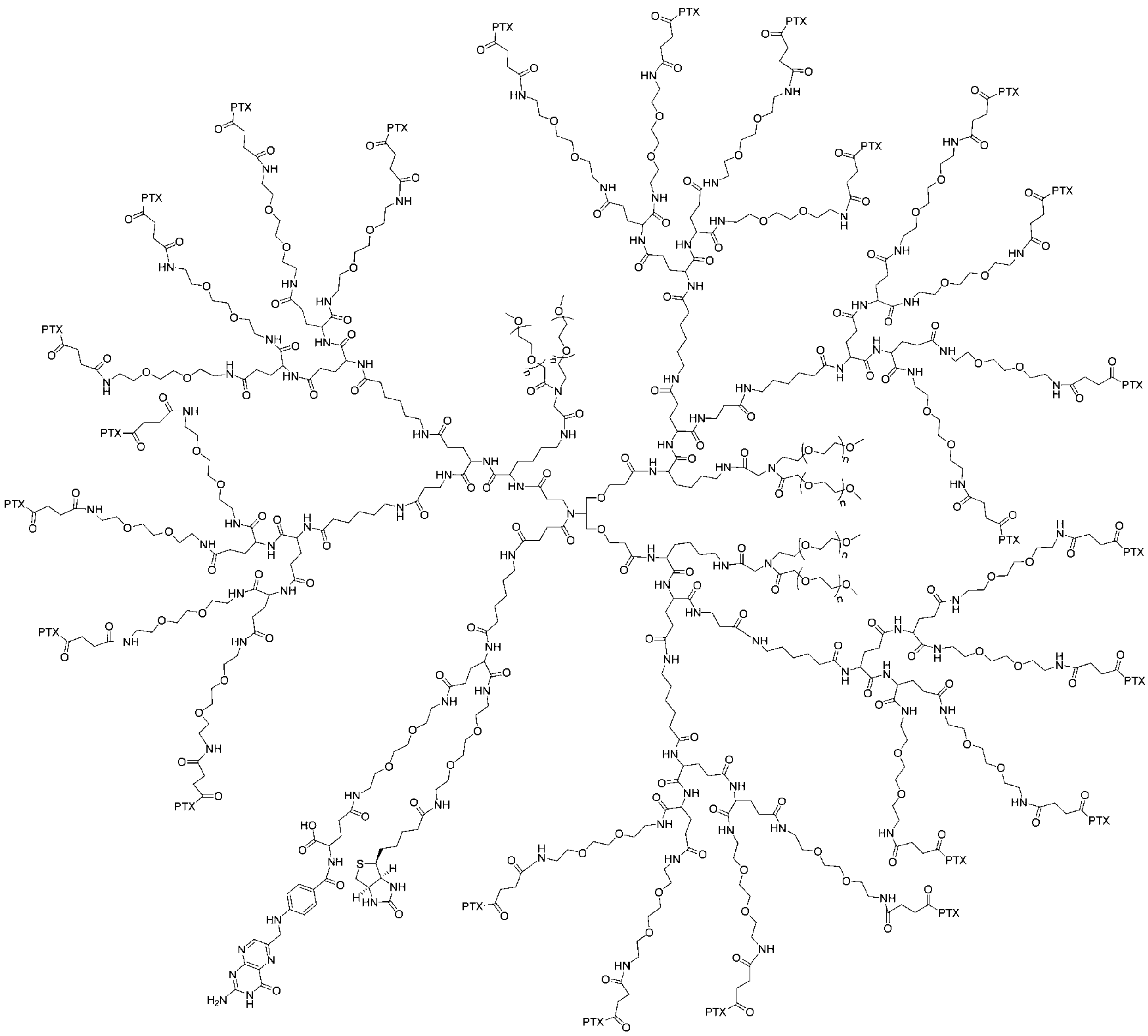

**74-232**

10.1 Preparation of Compound 74-182

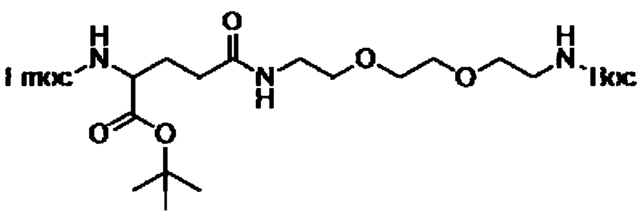

**88-4** (4 g, 16.108 mmol), Fmoc-Glu-OtBu (6.853 g, 16.108 mmol), HBTU (7.330 g, 19.329 mmol) and HOBT (2.611 g, 19.329 mmol) were placed in a 500 mL round-bottom flask, and DMF (50 mL) was added to dissolve the mixture with the assistance of ultrasonication. After dissolution, DIEA (7.987 mL, 48.324 mmol) was added dropwise in an ice-water bath, and finally, the reaction was carried out under stirring at room temperature. After the reaction was completed,

pure water (200 mL) and ethyl acetate (200 mL) were added into a 2-liter separatory funnel, and the reaction mixture was poured into it for extraction. The organic phase was collected, and the aqueous phase was further extracted with ethyl acetate (100 mL × 2). The organic phases were combined and washed once with saturated sodium bicarbonate solution (200 mL). Finally, the combined organic phases were dehydrated over anhydrous magnesium sulfate and subjected to rotary evaporation, then an appropriate amount of dichloromethane and 200-300 mesh silica gel powder were added, and the mixture was evaporated to dryness. The resulting product was loaded by dry method, and subjected to column chromatography using dichloromethane and then 0.5-1% methanol/dichloromethane as eluents. The desired product was collected, concentrated and evaporated to dryness to provide the product.

10.2 Preparation of **74-183**

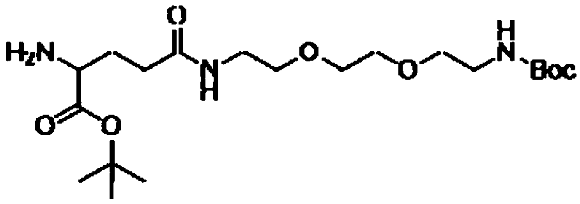

**74-182** (10.563 g, 16.108 mmol) was placed in a 500 mL round-bottom flask, and acetonitrile (50 mL) was added for dissolution with the assistance of ultrasonication. Diethylamine (23.562 mL, 322.16 mmol) was then added and the reaction was stirred at room temperature for 2 h. After the reaction was completed, the acetonitrile was removed by rotary evaporation. An appropriate amount of mixed solvent and 200-300 mesh silica gel powder were added, and the mixture was evaporated to dryness. The resulting product was loaded by dry method, and subjected to column chromatography using dichloromethane and then 2-5% methanol/dichloromethane as eluents. The desired product was collected, concentrated and evaporated to dryness [the yield exceeded 100%].

10.3 Preparation of **74-184**

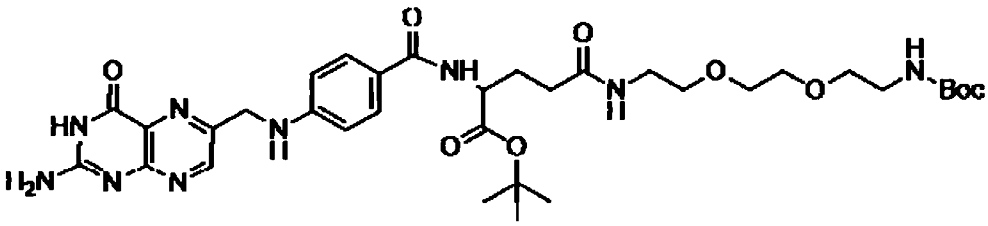

**74-183** (1.5 g, 3.459 mmol), pteroic acid (0.981 g, 3.144 mmol, Shanghai Hengxin), HBTU (1.788 g, 4.716 mmol) and HOBT (0.637 g, 4.716 mmol) were placed in a 250 mL round-bottom flask, and DMSO (30 mL) was added for dissolution. DIEA (1.588 mL, 9.432 mmol) was slowly added dropwise at room temperature. After the reaction was completed, methyl tert-butyl ether (450 mL) was added for precipitation. A solid precipitated and was filtered by suction. After filtration, the solid was dissolved by adding an appropriate amount of mixed solvent, then 200-300 mesh silica gel powder was added, and the mixture was evaporated to dryness. The resulting product was loaded by dry method, and subjected to column chromatography using 7-9% methanol/dichloromethane as the eluent. The desired product was collected, concentrated and evaporated to dryness to provide the product.

10.4 Preparation of **74-185**

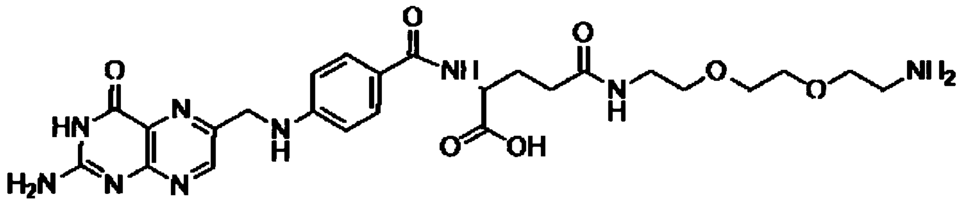

**74-184** (5 g, 4.25 mmol) was placed in a 500 mL round-bottom flask, then dichloromethane (4 mL) and TFA (2 mL) were added, and the mixture was reacted under stirring at room temperature. After the reaction was completed, dichloromethane and most of the TFA were removed by rotary evaporation. Methyl tert-butyl ether (150 mL) was then added for precipitation, and a solid precipitated. The mixture was treated by ultrasonication and allowed to stand, and the supernatant was discarded. The resulting product was filtered and dried to provide 3.8 g of the product.

10.5 Preparation of 74-141

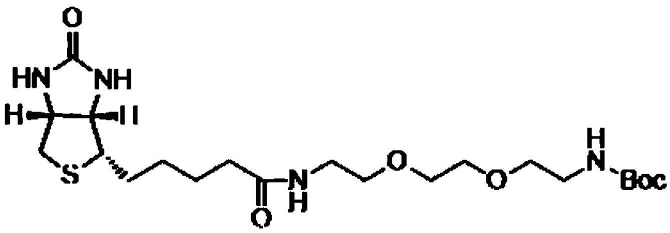

A sufficient amount of D-Biotin (5 g, 20.465 mmol) was weighed and placed in a 500 mL round-bottom flask, then DMF (30 mL) was added, and the mixture was stirred in an oil bath at 70°C until it became clear. Then the mixture was cooled to room temperature. **88-4** (1.016 g, 4.093 mmol), HBTU (1.862 g, 4.911 mmol) and HOBT (0.663 g, 4.911 mmol) were added, then DIEA (10.147 mL, 61.395 mmol) was added dropwise, and the reaction was stirred at 25°C for 2 h. After the reaction was completed, pure water (200 mL) and ethyl acetate (200 mL) were added to a 2-liter separatory funnel, and the reaction mixture was then poured into the funnel for extraction. The organic phase was collected, and the aqueous phase was further extracted with ethyl acetate (100 mL × 2). The organic phases were combined, washed with saturated sodium bicarbonate solution (200 mL), then dehydrated over anhydrous magnesium sulfate, and rotary evaporated. An appropriate amount of methanol/dichloromethane mixed solvent and 200-300 mesh silica gel powder were added, and the mixture was evaporated to dryness. The resulting product was loaded by dry method, and subjected to column chromatography using 1-6% methanol/dichloromethane as the eluent. The desired product was collected, concentrated and evaporated to dryness to provide 1.5 g of the product with a yield of 15.46%. $^1$H-NMR (600 MHz, DMSO-$d_6$) δ 7.83 (t, $J$ = 5.6, 1H), 6.76 (s, 1H), 6.41 (s, 1H), 6.35 (s, 1H), 4.30 (dd, $J$ = 7.5, 5.3, 1H), 4.15-4.10 (m, 1H), 3.49 (s, 4H), 3.40-3.35 (m, 4H), 3.18 (q, $J$ = 5.9, 2H), 3.12-3.03 (m, 3H), 2.82 (dd, $J$ = 12.4, 5.1, 1H), 2.58 (d, $J$ = 12.4, 1H), 2.06 (t, $J$ = 7.4, 2H), 1.60 (dd, $J$ = 8.7, 5.1, 1H), 1.55-1.42 (m, 3H), 1.37 (s, 9H), 1.33-1.26 (m, 2H)

10.6 Preparation of **74-150**

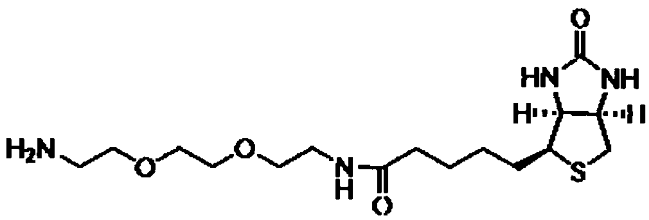

**74-141** (1.5 g, 3.160 mmol) was weighed and added into a 100 mL round-bottom flask, then TFA (4.693 mL) was added, and the reaction was stirred at room temperature for 1 hour. After the reaction was completed, the TFA was partially removed by rotary evaporation, methyl tert-butyl ether was then added for precipitation, the mixture was treated by ultrasonication, and the supernatant was discarded. This process was repeated four times until the product became a

viscous oil. The oil was dissolved by adding an appropriate amount of methanol/ dichloromethane mixed solvent, then 200-300 mesh silica gel powder was added, and the mixture was evaporated to dryness. The resulting product was loaded by dry method, and subjected to column chromatography using 1% ammonia water/6-10% methanol/ dichloromethane as the eluent. The desired product was collected, concentrated and evaporated to dryness to provide 1.046 g of the product with a yield of 87.23%. $^1$H-NMR (600MHz, DMSO-$d_6$) δ 7.87(t, *J* = 5.5, 1H), 6.41 (s, 1H), 6.35 (s, 1H), 4.32-4.29 (m, 1H), 4.14-4.11 (m, 1H), 3.54-3.47(m,4H),3.42-3.35 (m,4H),3.27(s,2H), 3.20-3.17 (m, 2H), 3.12-3.06 (m, 2H), 2.82 (dd, *J* = 5.1, 12.4 Hz, 1H), 2.67 (t, *J* = 5.7 Hz, 2H), 2.57 (d, *J* = 12.4 Hz, 1H), 2.06 (t, *J* = 7.4 Hz, 2H), 1.64-1.57 (m, 1H), 1.53-1.42 (m, 3H), 1.35-1.23 (m, 2H); FT MS ESI m/z [M+H$^+$] 375.20

10.7 Preparation of **74-152**

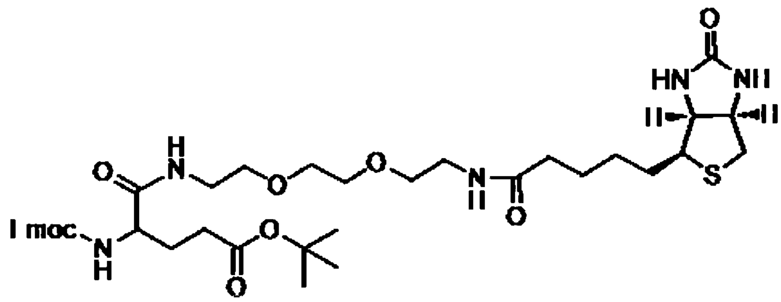

**74-150** (1.026 g, 2.739 mmol), Fmoc-Glu(OtBu)-OH (1.165 g, 2.739 mmol), HBTU (1.246 g, 3.287 mmol) and HOBT (0.444 g, 3.287 mmol) were weighed and added into a 100 mL round-bottom flask, then DMF (15 mL) was added for dissolution with the assistance of ultrasonication. DIEA (1.584 mL, 9.588 mmol) was added dropwise in an ice-water bath. After the dropwise addition was completed, the flask was placed in a 25°C water bath, and the reaction was carried out under stirring for 2 h. After the reaction was completed, methyl tert-butyl ether/petroleum ether were added, treated by ultrasonication, and allowed to stand, and the supernatant was discarded. This process was repeated four times until the product became a viscous oil. The oil was dissolved by adding an appropriate amount of methanol/ dichloromethane mixture, then 200-300 mesh silica gel powder was added, and the mixture was evaporated to dryness. The resulting product was loaded by dry method, and subjected to column chromatography using 2-4% methanol/dichloromethane as the eluent. The desired product was collected, concentrated, and evaporated to dryness to provide 1.847 g of the product with a yield of 86.26%. $^1$H-NMR (600 MHz, DMSO-$d_6$) δ 7.95-7.90 (m, 1H), 7.89 (d, *J* = 7.5,2H), 7.82 (t, *J* = 5.5, 1H), 7.73 (t, *J* = 7.7, 2H), 7.51 (d, *J* = 8.3,1H), 7.42 (t, *J* = 7.4,2H), 7.33 (t, *J* = 7.4,2H), 6.41 (s, 1H), 6.35 (s, 1H), 4.29 (dd, *J* = 10.9, 4.8,2H), 4.23 (dd, *J* = 16.2, 6.8, 2H), 4.15-4.09 (m, 1H), 3.98 (d, *J* = 5.4,1H), 3.51-3.46 (m, 4H), 3.43-3.36(m, 4H), 3.28-3.22(m, 1H), 3.18 (dd, *J* = 11.8, 5.2, 3H), 3.08 (dd, *J* = 4.4, 2.7, 1H), 2.81 (dd, *J* = 5.1, 12.4 Hz, 1H), 2.57 (d, *J* = 12.4 Hz, 1H), 2.26-2.18 (m, 2H), 2.06 (t, *J* = 7.4, 2H), 1.90-1.82 (m, 1H), 1.76-1.68 (m, 1H), 1.64-1.57 (m, 1H), 1.53-1.43 (m, 3H), 1.39 (s, 9H), 1.34-1.20 (m, 2H); FT MS ESI m/z [M+H$^+$] 782.37, [M+Na$^+$] 804.35

10.8 Preparation of **74-172**

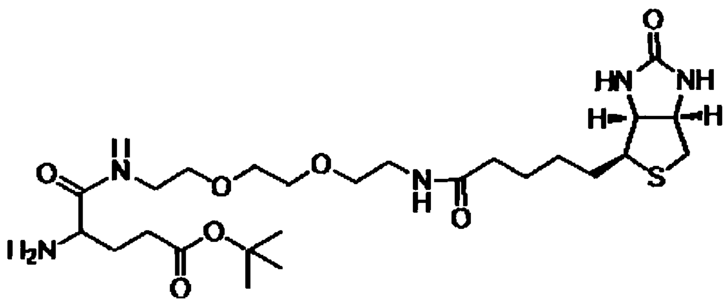

**74-152** (1.847 g, 2.362 mmol) was placed in a 100 mL round-bottom flask, DMF (5 mL) was added for dissolution, then diethylamine (3.455 mL, 47.24 mmol) was added, and the mixture was reacted under stirring at room temperature. After the reaction was completed, methyl tert-butyl ether was added for precipitation. The mixture was treated by

ultrasonication, allowed to stand, filtered, and dried to provide 0.978 g of the product with a yield of 73.97%.

10.9 Preparation of **74-173**

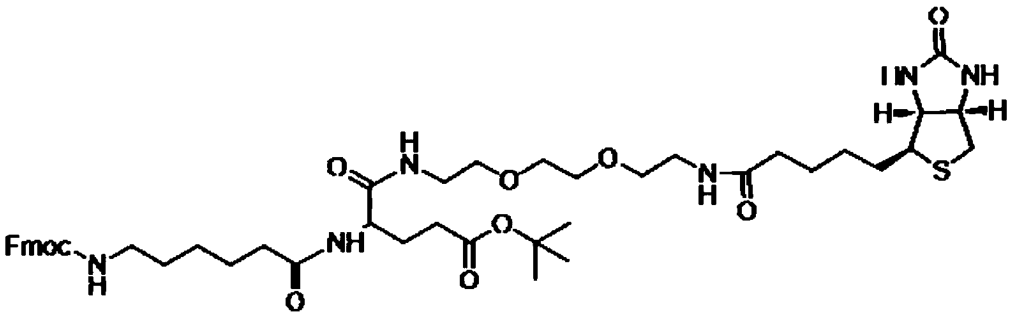

**74-172** (0.978 g, 1.747 mmol), **88-14** (0.617 g, 1.747 mmol), HBTU (0.861 g, 2.271 mmol) and HOBT (0.306 g, 2.271 mmol) were placed in a 100 mL round-bottom flask, and then DMF (15 mL) was added to dissolve the mixture with the assistance of ultrasonication. DIEA (0.866 mL, 5.241 mmol) was added dropwise in an ice-water bath. The mixture was allowed to react under stirring at room temperature for 2 h. After the reaction was completed, methyl tert-butyl ether (200 mL) was added for precipitation. The mixture was treated by ultrasonication, and allowed to stand, and the supernatant was discarded. This process was repeated four times until the product became a viscous oil. The oil was dissolved by adding an appropriate amount of methanol/dichloromethane mixed solvent, then 200-300 mesh silica gel powder was added, and the mixture was evaporated to dryness. The resulting product was loaded by dry method, and subjected to column chromatography using 2-4% methanol/dichloromethane as the eluent. The desired product was collected, concentrated and evaporated to dryness to provide 1.23 g of the product with a yield of 78.69%. $^1$H-NMR (600 MHz, DMSO-$d_6$) δ 7.96-7.91 (m, 2H), 7.89 (d, *J* = 7.5, 2H), 7.86-7.82 (m, 1H), 7.68 (d, *J* = 7.5, 2H), 7.41 (t, *J* = 7.4, 2H), 7.35-7.31 (m, 2H), 7.29-7.24 (m, 1H) 6.44 (s, 1H), 6.37 (s, 1H), 4.31-4.27 (m, 3H), 4.21 (d, *J* = 6.7, 2H), 4.14-4.11 (m, 1H), 3.52-3.47 (m, 4H), 3.43-3.36 (m, 4H), 3.27-3.21 (m, 1H), 3.20-3.13 (m, 3H), 3.10-3.07 (m, 1H), 2.96 (d, *J* = 6.3, 2H), 2.81 (dd, *J* = 12.4, 5.1, 1H), 2.57 (d, *J* = 12.4, 1H), 2.21-2.16 (m, 2H), 2.11 (d, *J* = 6.2, 2H), 2.06 (t, *J* = 7.4, 2H), 1.88-1.79 (m, 1H), 1.73-1.64 (m, 1H), 1.64-1.56 (m, 1H), 1.55-1.42 (m, 6H), 1.38 (s, 9H), 1.32-1.18 (m, 5H); FT MS ESI m/z [M+H$^+$] 895.45, [M+Na$^+$] 917.44

10.10 Preparation of **74-223**

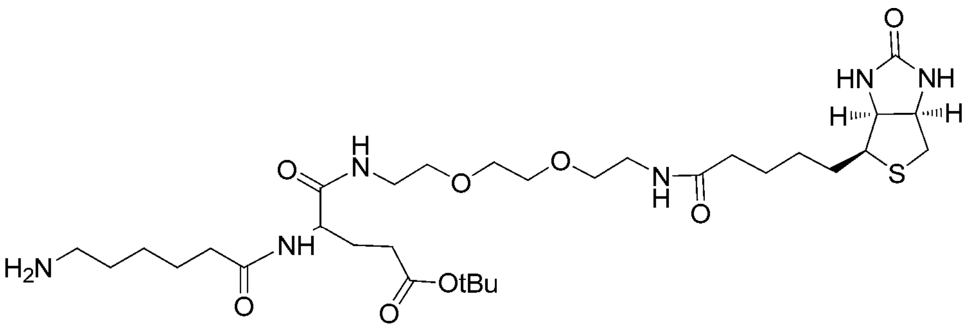

**74-173** (3.54 g, 3.961 mmol) was placed in a 100 mL round-bottom flask, DMF (10 mL) was added for dissolution, then diethylamine (5.794 mL, 79.22 mmol) was added, and the reaction was stirred at room temperature. After the reaction was completed, methyl tert-butyl ether (200 mL) was added for precipitation. The mixture was treated by ultrasonication, allowed to stand, filtered, and dried to provide 1.7 g of the product with a yield of 63.83%.

10.11 Preparation of **77-163**

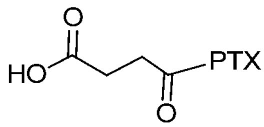

Paclitaxel (5 g, 5.86 mmol, abbreviated as PTX), succinic anhydride (2.1 g, 21.096 mmol), and DMAP (0.024 g, 0.3876 mmol) were placed in a 250 mL flask. A mixture of anhydrous dichloromethane and DMA (11/1, 24 mL) was added for dissolution with the assistance of ultrasonication. The reaction was stirred at room temperature overnight. After the reaction was completed, the mixture was rotary evaporated to dryness and dissolved in ethyl acetate (30 mL). The reaction mixture was transferred to a 500 mL separatory funnel, and dilute hydrochloric acid (1%, w/v) (35 mL × 3) was added for extraction. The ethyl acetate phase was collected and washed with purified water (40 mL × 3). The organic phase was collected and dried over anhydrous sodium sulfate, filtered, concentrated, and dried to provide 4.9 g of the product. $^1$H-NMR (600 MHz, DMSO-$d_6$) δ 12.26 (s, 1H), 8.00-7.98 (m, 2H), 7.86-7.84 (m, 2H), 7.74 (t, *J* = 7.4 Hz, 1H), 7.67 (t, *J* = 7.6 Hz, 2H), 7.56 (d, *J* = 7.4 Hz, 1H), 7.52-7.44 (m, 7H), 7.20 (td, *J* = 5.9, 3.0 Hz, 1H), 6.30 (s, 1H), 5.82 (t, *J* = 8.8 Hz, 1H), 5.55 (t, *J* = 8.8 Hz, 1H), 4.93-4.89 (m, 2H), 4.64 (s, 1H), 4.12 (dd, *J* = 6.6, 4.1 Hz, 1H), 4.06-3.98 (m, 3H), 3.59 (d, *J* = 7.2 Hz, 1H), 2.63 (t, *J* = 6.5 Hz, 3H), 2.33 (d, *J* = 1.3 Hz, 2H), 2.25 (s, 3H), 2.11 (s, 3H), 1.76 (s, 3H), 1.51 (d, *J* = 11.3 Hz, 4H), 1.01 (t, *J* = 13.8 Hz, 9H)

10.12 Preparation of **77-164**

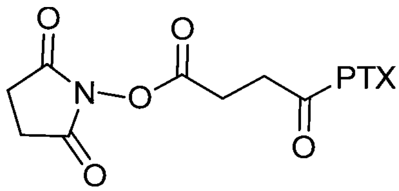

**77-163** (2.24 g, 2.34 mmol), NHS (0.355 g, 3.0888 mmol) and DCC (0.618 g, 2.9952 mmol) were placed in a 100 mL flask, then dichloromethane (10 mL) was added for dissolution. The reaction was stirred overnight at room temperature. After the reaction was completed, the mixture was filtered, and silica gel powder was added to the filtered, then the mixture was concentrated and dried. The resulting product was loaded by dry method, and subjected to column chromatography using a 1-2% methanol/dichloromethane mixture as the eluent. The desired product was collected, concentrated and dried to provide 2.47 g of the product.

10.13 Preparation of **74-130**

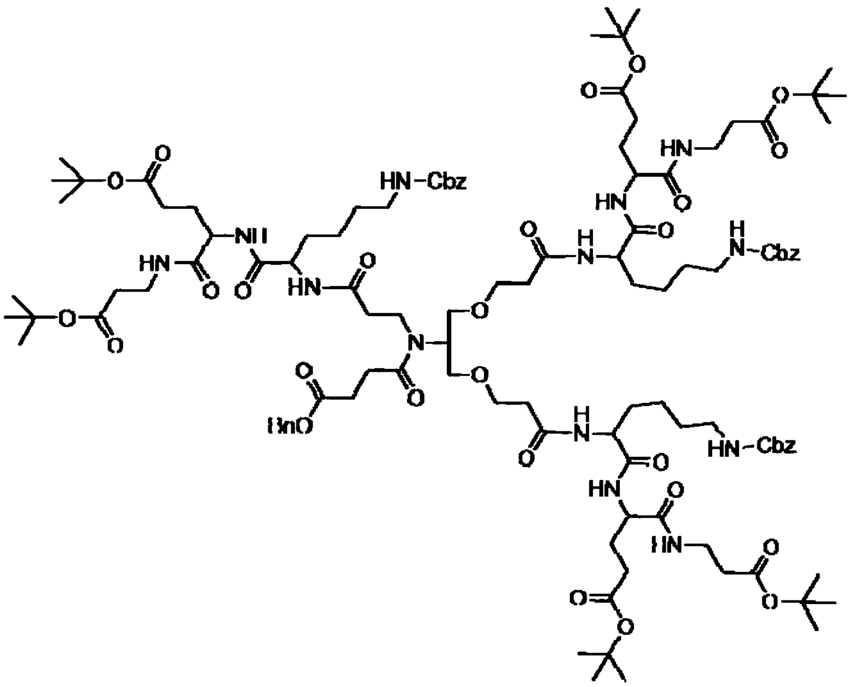

**69-246** (0.963 g, 1.937 mmol) was weighed and placed in a 250 mL round-bottom flask, and DMF (25 mL) was added for dissolution, followed by a dropwise addition of DIEA (8.003 mL, 48.425 mmol) in an ice-water bath. **76-116** (4.592 g, 7.748 mmol), HBTU (2.938 g, 7.748 mmol) and HOBT (1.046 g, 7.748 mmol) were weighed using beakers, and then poured into the flask, and the mixture was reacted under stirring at 30°C. After the reaction was completed, pure water (200 mL) and ethyl acetate (200 mL) were added to a 2-liter separatory funnel, and then the reaction mixture was poured into the funnel for extraction. The organic phase was collected, and the aqueous phase was further extracted with ethyl acetate (100 mL × 2). The organic phases were combined and washed with saturated sodium bicarbonate solution (200 mL). Finally, the obtained organic phase was dehydrated over anhydrous magnesium sulfate, rotary evaporated, then dissolved by adding an appropriate amount of methanol/dichloromethane mixture. 200-300 mesh silica gel powder was added, and the mixture was evaporated to dryness. The resulting product was loaded by dry method, and subjected to column chromatography using 1-5% methanol/dichloromethane as the eluent. The desired product was collected, concentrated and evaporated to dryness to provide 3.203 g of the product with a yield of 74.436%. $^1$H-NMR (600 MHz, DMSO-$d_6$) δ 8.12-7.86 (m, 4H), 7.80-7.71 (m, 5H), 7.69 (d, $J$ = 8.2 Hz, 1H), 7.67-7.56 (m, 2H), 7.52-7.26 (m, 20H), 5.14-4.94 (m, 8H), 4.35-4.29 (m, 3H), 4.27-4.19 (m, 3H), 3.74-3.64 (m, 5H), 3.60-3.49 (m, 4H), 3.43-3.36 (m, 6H), 3.21-3.03 (m, 8H), 2.69-2.57 (m, 4H), 2.55-2.45 (m, 12H), 2.26-2.15 (m, 6H), 1.93-1.80 (m, 4H), 1.79-1.67 (m, 3H), 1.67-1.56 (m, 3H), 1.56-1.43 (m, 6H), 1.43-1.34 (m, 2H), 1.34-1.19 (m, 60H)

10.14 Preparation of **74-214**

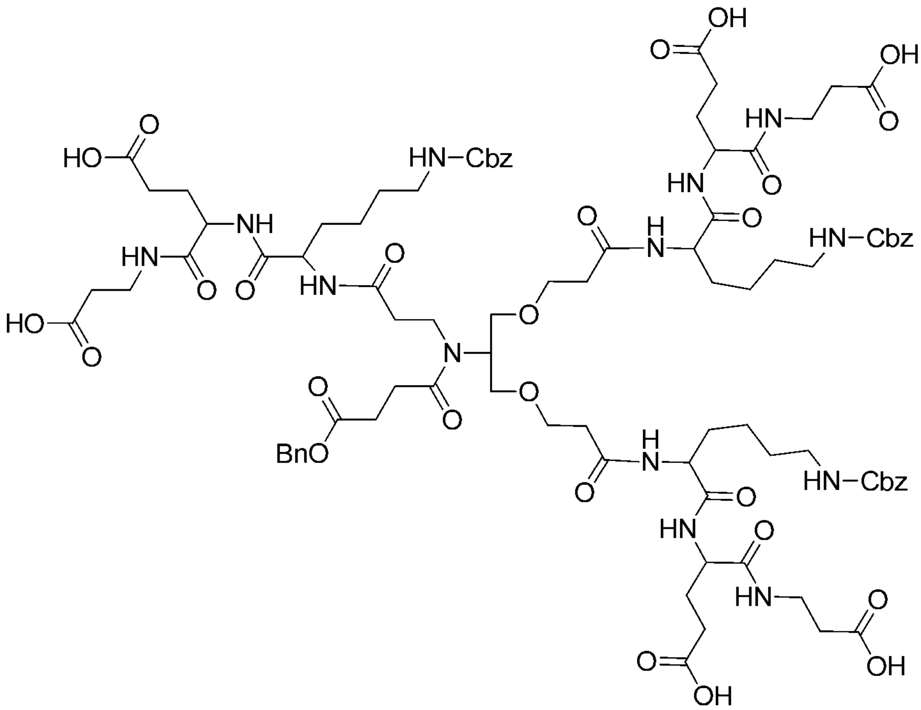

**74-206** (1 g, 0.45 mmol) was placed in a 100 mL round-bottom flask, then TFA (5 mL) was added, and the reaction was stirred at room temperature for 1 hour. After the reaction was completed, the TFA was partially removed by rotary evaporation under reduced pressure. Methyl tert-butyl ether (50 mL) was added, treated by ultrasonication, and rotary evaporated under reduced pressure. The residue was dissolved in dichloromethane, then n-hexane/methyl tert-butyl ether were added for precipitation, and the supernatant was discarded. This process was repeated several times until the product became a viscous oil. Finally, the oil was rotary evaporated and dried to provide 0.84 g of the product. $^1$H-NMR (600 MHz, DMSO-$d_6$) δ 13.05-11.38 (m, 6H), 8.12-7.86 (m, 4H), 7.80-7.71 (m, 5H), 7.69 (d, $J$ = 8.2 Hz, 1H), 7.67-7.56 (m, 2H), 7.52-7.26 (m, 20H), 5.14-4.94 (m, 8H), 4.35-4.29 (m, 3H), 4.27-4.19 (m, 3H), 3.74-3.64 (m, 5H), 3.60-3.49 (m, 4H), 3.43-3.36 (m, 6H), 3.21-3.03 (m, 8H), 2.69-2.57 (m, 4H), 2.55-2.45 (m, 12H), 2.26-2.15 (m, 6H), 1.93-1.80(m, 4H),1.79-1.67(m, 3H),1.67-1.56(m, 3H), 1.56-1.43(m, 6H),1.43-1.34(m, 2H), 1.34-1.19 (m, 6H)

10.15 Preparation of **74-215**

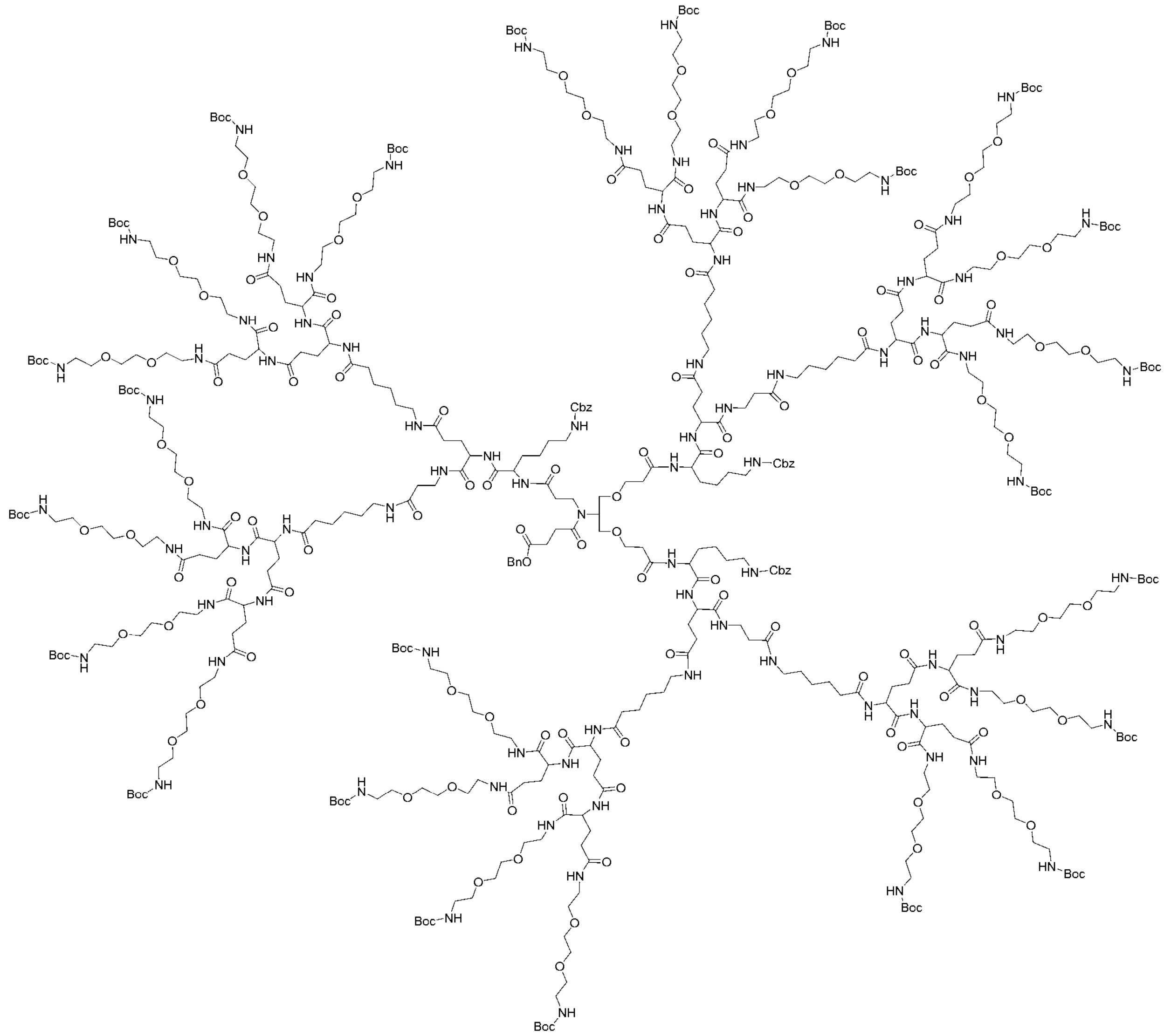

**74-214** (0.58 g, 0.31 mmol), **76-93** (2.68 g, 1.86 mmol), HBTU (0.962 g, 2.538 mmol) and HOBT (0.342 g, 2.538 mmol) were placed in a 100 mL round-bottom flask, then DMF (8 mL) and NMP (8 mL) were added for dissolution with the assistance of ultrasonication. The mixture was stirred at 0°C for 3 minutes. DIEA (0.83 g, 5.07 mmol) was then added dropwise. After 3 minutes, the mixture was taken out and reacted under stirring at room temperature. After the reaction was completed, methyl tert-butyl ether (150 mL) was added to the reaction mixture, a solid precipitated, which was filtered by suction. The filter cake was dissolved in an appropriate amount of methanol/dichloromethane (1/4) mixture, then 100-200 mesh silica gel powder was added, and the mixture was evaporated to dryness. The resulting product was loaded by dry method, and subjected to column chromatography using 1% ammonia water/5-9% methanol/dichloromethane as the eluent. The desired product was collected, concentrated, and evaporated to dryness to provide 1.7 g of the product. $^1H$-NMR (600 MHz, DMSO-$d_6$) $\delta$ 8.29-8.14 (m, 14H), 8.14-8.02 (m, 14H), 8.02-7.92 (m, 10H), 7.91-7.76 (m, 15H), 7.40-7.25 (m, 20H), 6.82-6.68 (m,11H), 5.17-4.90 (m, 8H),4.30-4.00 (m, 24H), 3.65-3.55 (m,17H), 3.55-3.44 (m, 97H), 3.44-3.35 (m, 79H), 3.31-3.21 (m, 46H), 3.21-3.11 (m, 36H), 3.11-2.93 (m, 60H), 2.92-2.84 (m, 18H), 2.67-2.52 (m, 4H), 2.47-2.37 (m, 2H), 2.37-2.19 (m, 8H), 2.19-1.95 (m, 52H), 1.94-1.58 (m, 37H), 1.56-1.40 (m, 20H), 1.40-1.30 (m, 225H), 1.30-1.15 (m, 24H)

10.16 Preparation of **74-216**

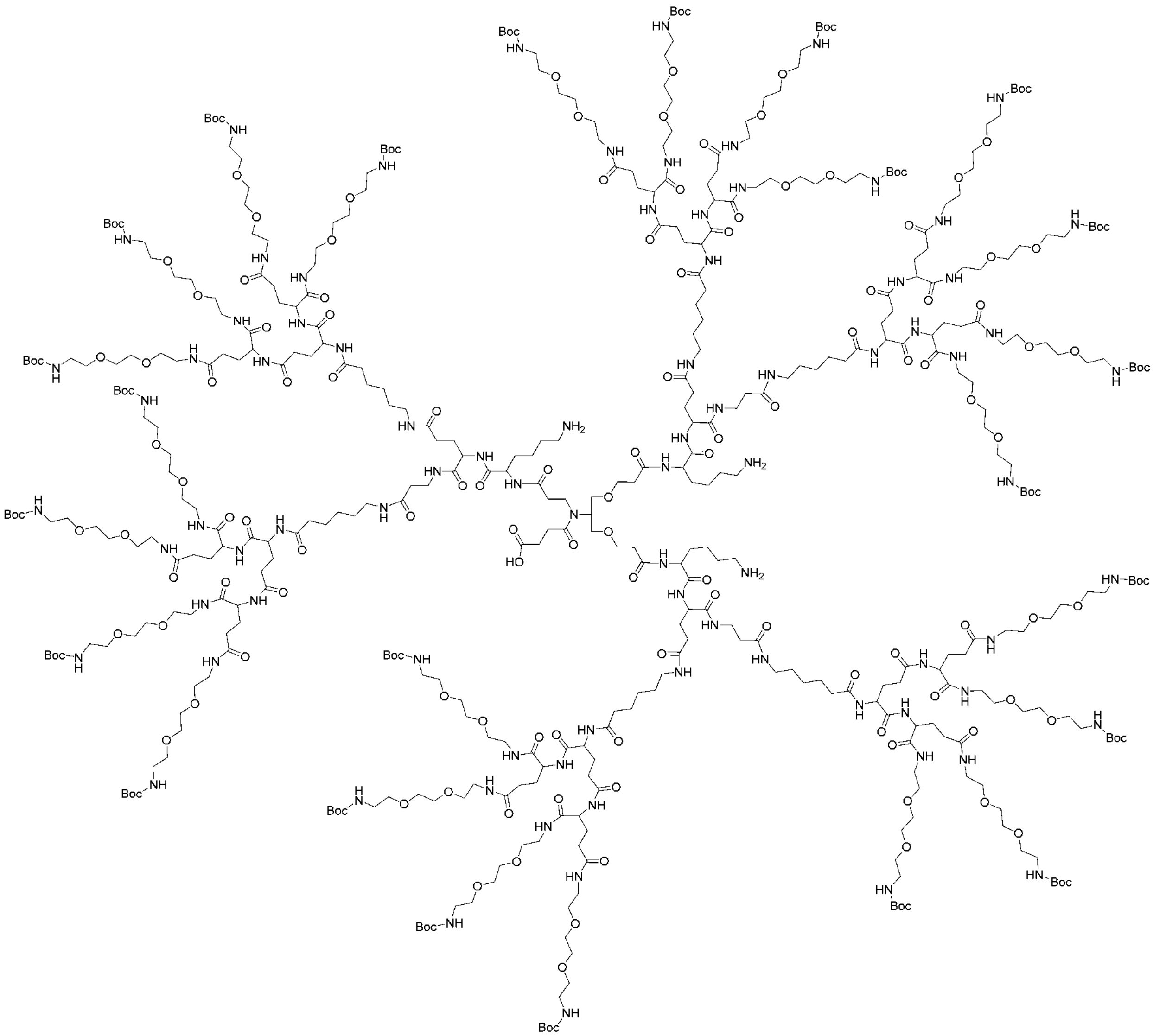

Raw material **74-215** (1.3 g, 0.124 mmol) and 10% Pd/C catalyst (100 mg) were added to a hydrogenation reactor, and then methanol (30 mL) was added for dissolution. The hydrogenation reactor was sealed and filled with hydrogen gas, then hydrogen gas was evacuated. This process was repeated five times. Hydrogen gas was then refilled to 2 MPa, and the reaction was carried out under stirring overnight in a 50°C oil bath. After the reaction was completed, the mother liquor was filtered through a Büchner funnel containing filter paper, rotary evaporated under reduced pressure, and dried to provide 1.23 g of the product. $^1$H-NMR (600 MHz, DMSO-$d_6$) δ 12.01(s, 1H), 8.29-8.14 (m, 14H), 8.14-8.02 (m, 14H), 8.02-7.92 (m, 10H), 7.91-7.76 (m, 12H), 6.82-6.68 (m,11H),4.30-4.00 (m, 24H), 3.65-3.55 (m,17H), 3.55-3.44 (m, 97H), 3.44-3.35 (m, 79H), 3.31-3.21 (m, 46H), 3.21-3.11 (m, 36H), 3.11-2.93 (m, 60H), 2.92-2.84 (m, 24H), 2.67-2.52 (m, 4H), 2.47-2.37 (m, 2H), 2.37-2.19 (m, 8H), 2.19-1.95 (m, 52H), 1.94-1.58 (m, 37H), 1.56-1.40 (m, 20H), 1.40-1.30 (m, 225H), 1.30-1.15 (m, 24H)

10.17 Preparation of **74-217**

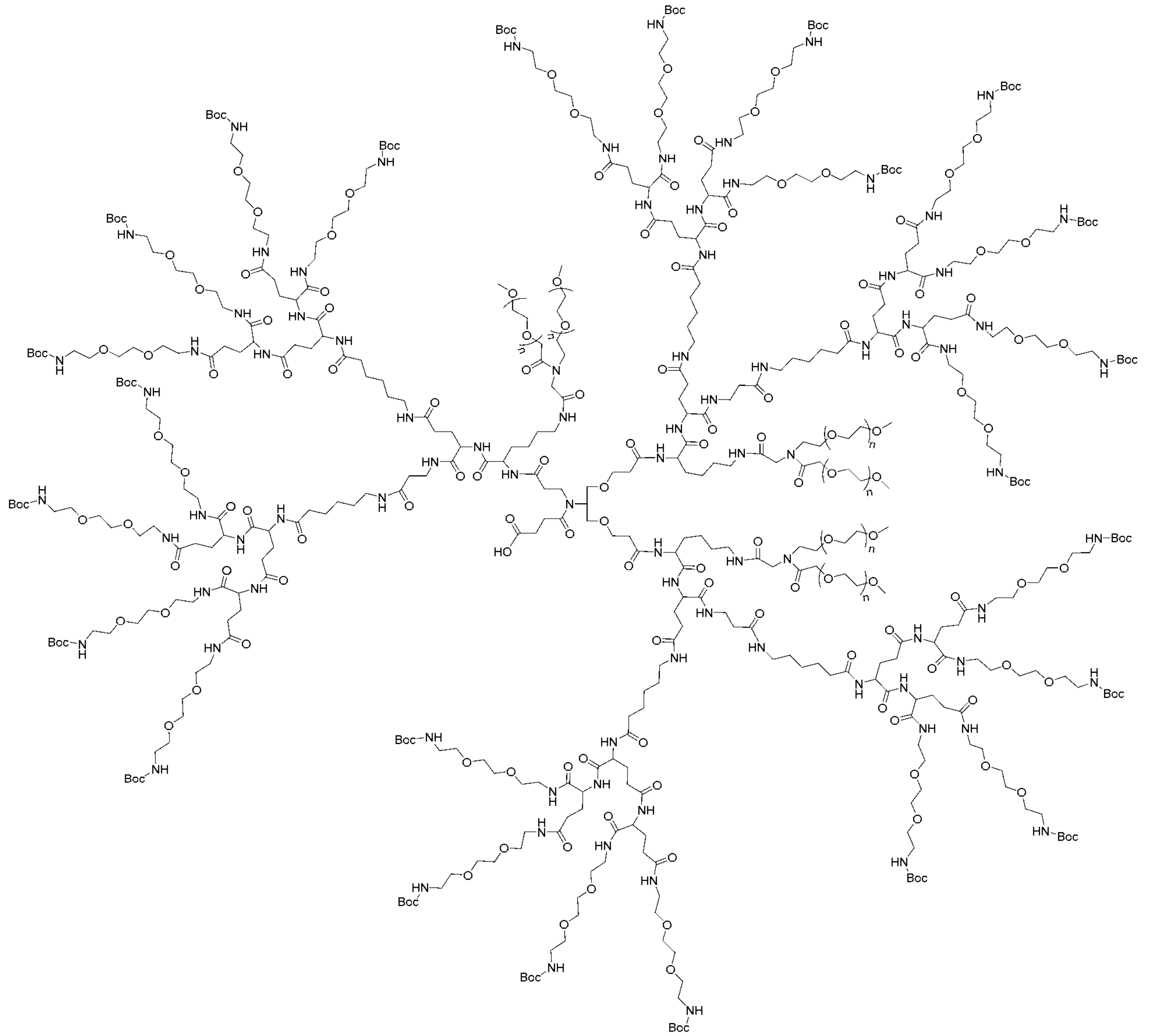

**74-216** (1.23 g, 0.124 mmol) was placed in a 100 mL round-bottom flask, then ultra-dry DMF (15 mL) was added for dissolution with the assistance of ultrasonication, and the mixture was stirred at 0°C for 3 minutes. DIEA (0.614 g, 3.72 mmol) was then slowly added dropwise. After stirring for 3 minutes, Y-NHS-10K (3.86 g, 0.375 mmol, purchased from Jenkem, lot number: ZZ403P059) was added, and reacted under stirring in the dark at low speed at room temperature. After the reaction was completed, methyl tert-butyl ether (450 mL) was added to the reaction mixture. A solid precipitated and was filtered. The filter cake was dried to provide 4.5 g of product.

10.18 Preparation of **74-225**

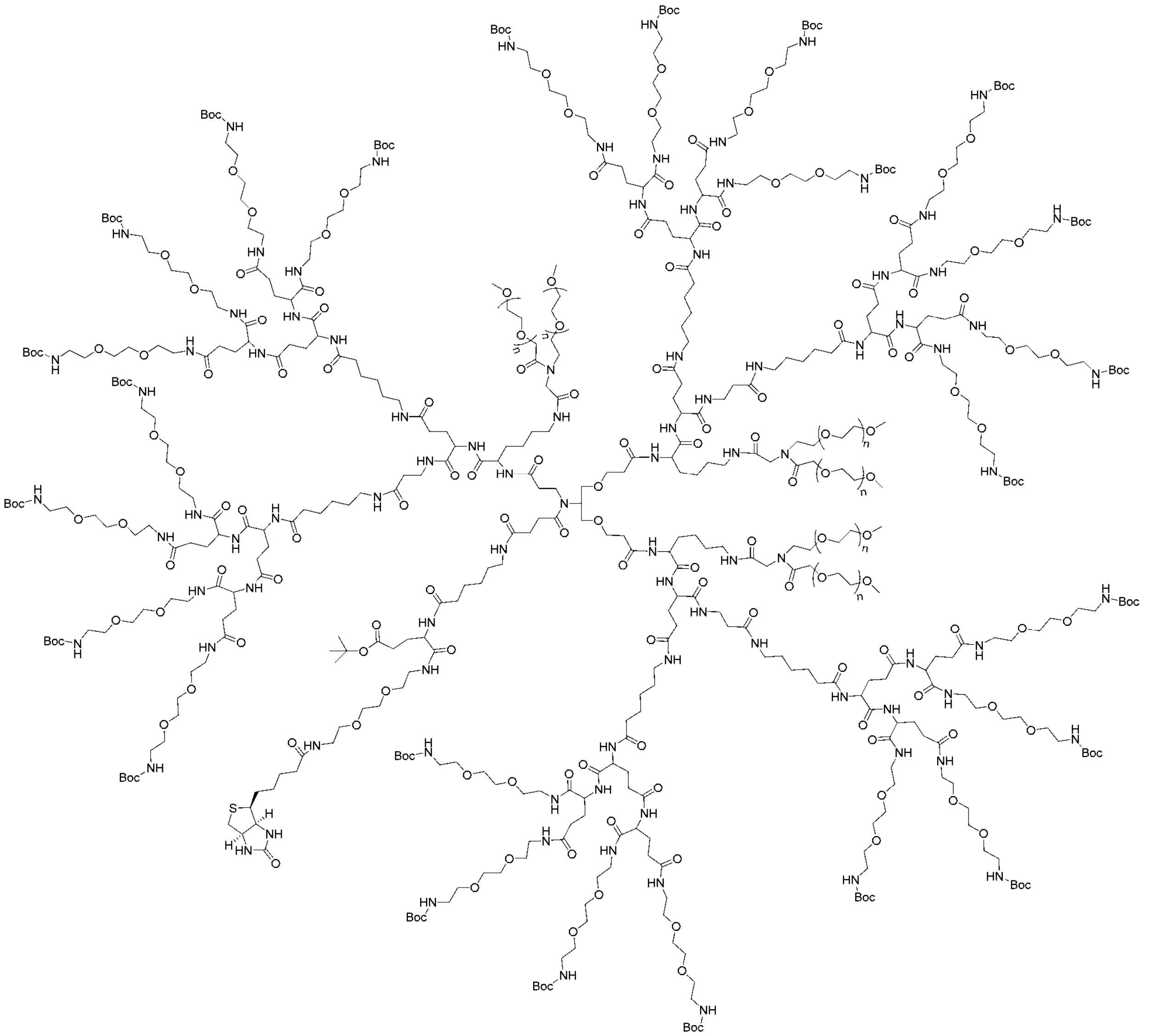

**74-217** (4 g, 0.098 mmol), **74-223** (0.263 g, 0.392 mmol), HBTU (0.148 g, 0.392 mmol) and HOBT (0.052 g, 0.392 mmol) were placed in a 100 mL round-bottom flask, then DMF (10 mL) was added for dissolution with the assistance of ultrasonication, and stirred at 0°C for 3 minutes. DIEA (0.194 g, 1.176 mmol) was then added dropwise. After 5 minutes, the mixture was taken out and reacted under stirring at room temperature. After the reaction was completed, methyl tert-butyl ether (450 mL) was added to the reaction mixture, and a solid precipitated and was filtered. The filter cake was redissolved in a methanol/dichloromethane (1/4) mixture, then 100-200 mesh silica gel powder was added, and the mixture was evaporated to dryness. The resulting product was loaded by dry method, and subjected to column chromatography using 1% ammonia water/3-8% methanol/dichloromethane as the eluent. The desired product was collected, concentrated, and dried to provide 1.74 g of the product.

10.19 Preparation of **74-228**

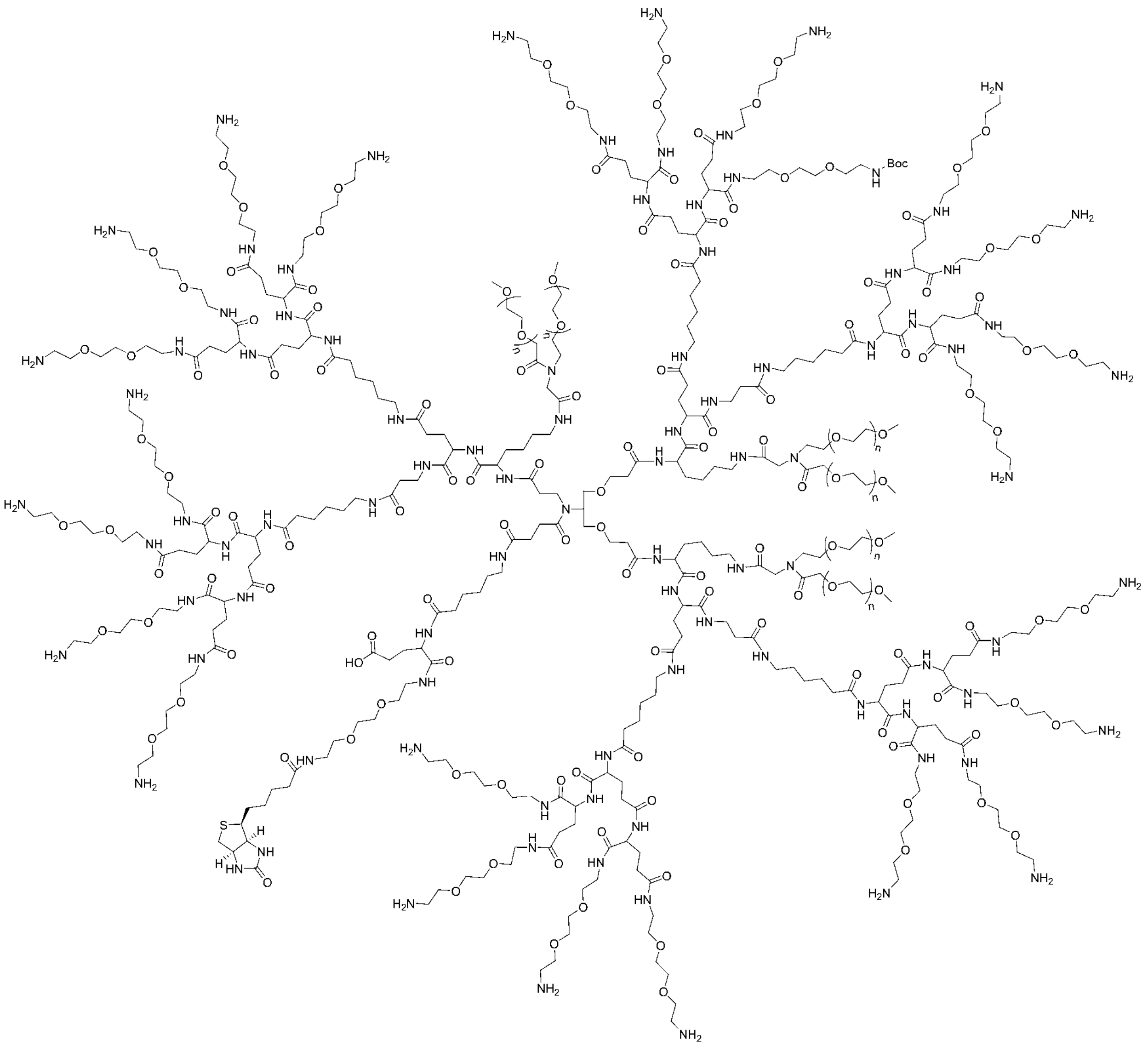

**74-225** (1.74 g, 0.042 mmol) was placed in a 250 mL round-bottom flask, then TFA (10 mL) was added, and the reaction mixture was stirred at room temperature. After the reaction was completed, dichloromethane was added, and then dichloromethane and most of the TFA were removed by rotary evaporation. This process was repeated three times to obtain an oily viscous solution. Methyl tert-butyl ether (100 mL) was then added, and a solid precipitated and was filtered. The filter cake was filtered and dried to provide 0.92 g of the product.

10.20 Preparation of **74-229**

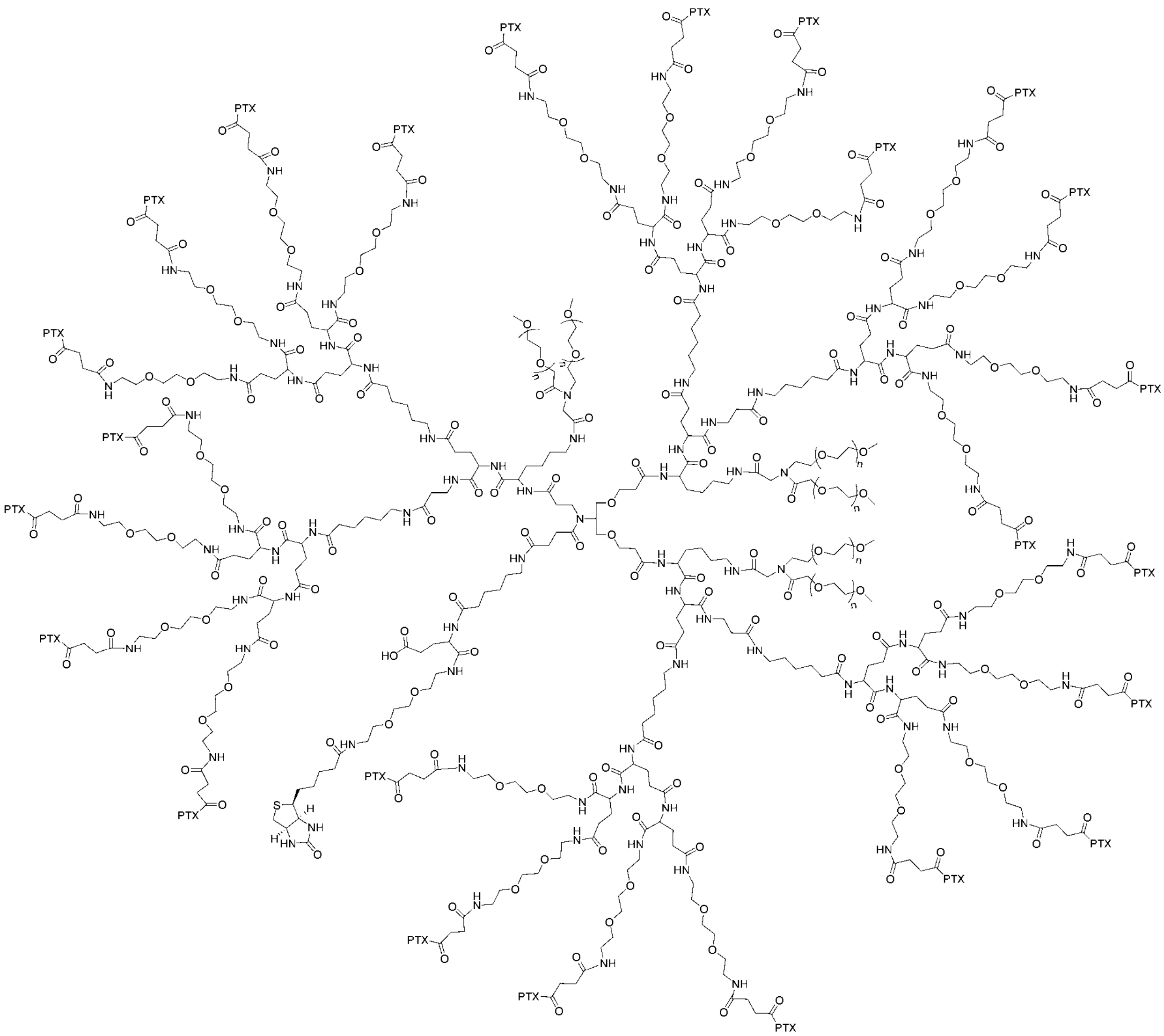

**74-228** (0.92 g, 0.024 mmol) was placed in a 100 mL round-bottom flask, then DMF (10 mL) was added for dissolution with the assistance of ultrasonication, and the contents were stirred at 0°C for 3 minutes. DIEA (0.119 g, 0.72 mmol) and **77-164** (0.75 g, 0.72 mmol) were slowly added dropwise. After 3 minutes, the mixture was taken out, and the reaction was carried out under stirring at room temperature overnight. After the reaction was completed, methyl tert-butyl ether (150 mL) was added to the reaction mixture, and a solid precipitated. The mixture was allowed to stand at room temperature for 4 hours, and the supernatant was discarded. An appropriate amount of dichloromethane was added to the resultant solid to dissolve the product. Methyl tert-butyl ether (100 mL) was then added, and a solid precipitated and was filtered with suction. The filter cake was dried to provide 1.46 g of the product.

10.21 Preparation of **74-230**

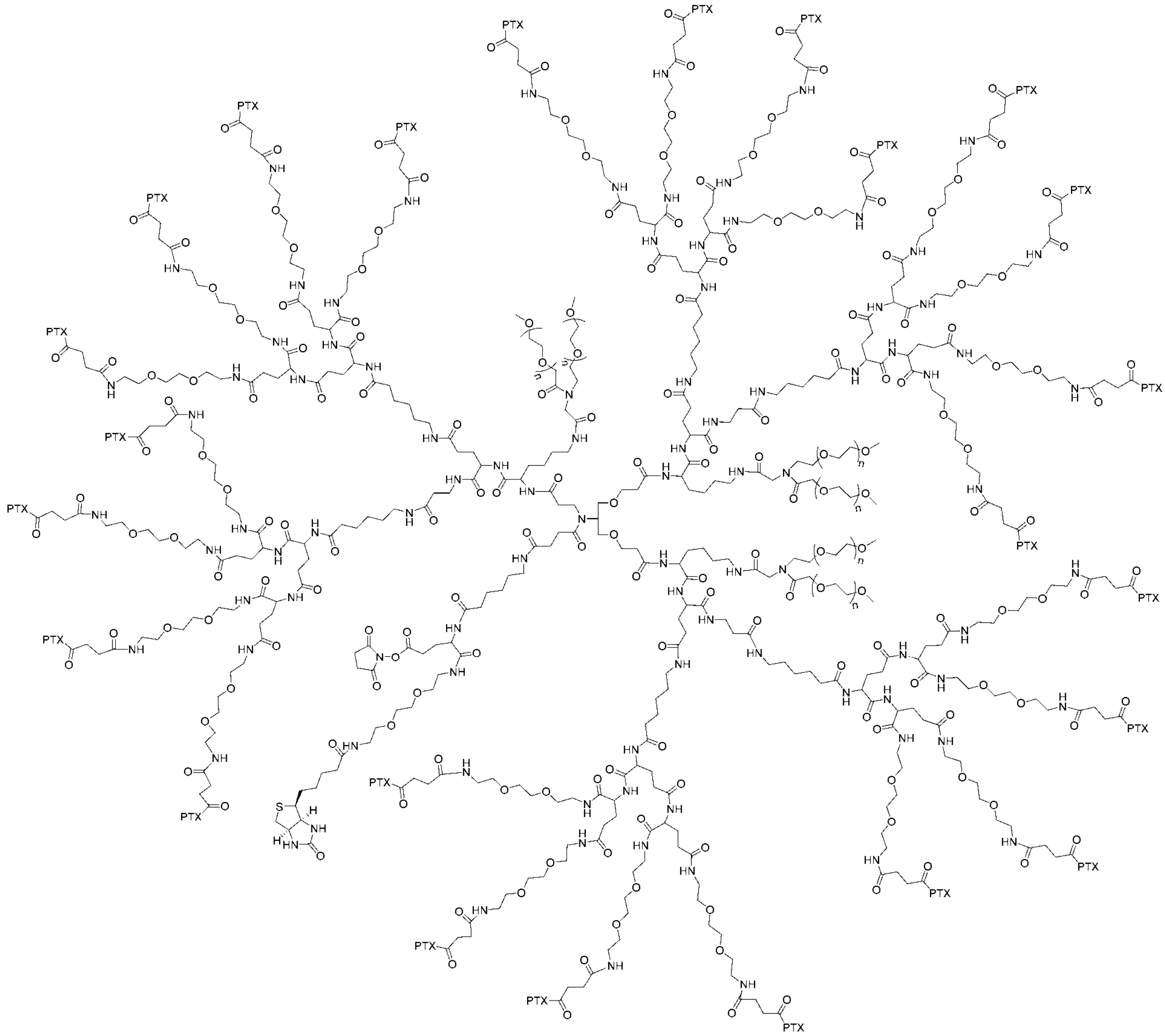

**74-229** (1.46 g, 0.024 mmol) and TSTU (0.014 g, 0.048 mmol) were placed in a 100 mL round-bottom flask, DMF (10 mL) was added for dissolution, then triethylamine (0.1 mL) was added, and the reaction mixture was stirred at room temperature. After the reaction was completed, n-hexane (10 mL) and methyl tert-butyl ether (50 mL) were added, and a solid precipitated. The mixture was allowed to stand for 1 hour, and the supernatant was discarded. This process was repeated four times. The mixture was filtered with suction, and the filter cake was dried to provide 1.26 g of the product.

10.22 Preparation of **74-232**

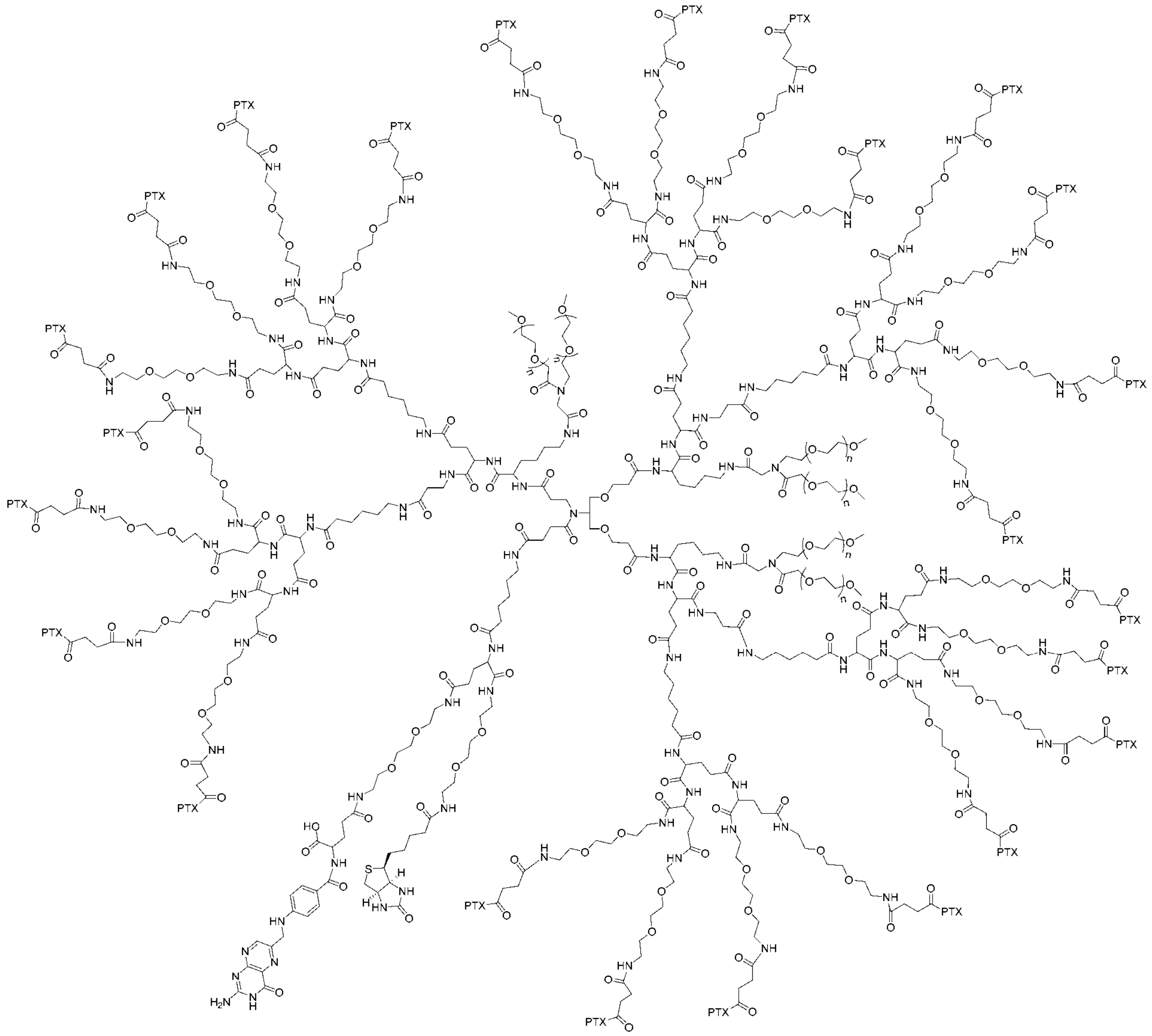

**74-230** (1 g, 0.016 mmol) was placed in a 100 mL round-bottom flask, then DMF (10 mL) was added for dissolution with the assistance of ultrasonication, and the mixture was stirred at 0°C for 3 minutes. DIEA (0.0074 mL, 0.0576 mmol) was then added dropwise, followed by **74-185** (0.01 g, 0.0176 mmol), and the reaction was carried out under stirring at room temperature. After the reaction was completed, methyl tert-butyl ether (450 mL) and n-hexane (50 mL) were added to the reaction mixture, and a solid precipitated. After standing for 4 hours, the supernatant was discarded, and the solid was dissolved in an appropriate amount of dichloromethane, then silica gel powder (100-200 mesh) was added, and the mixture was evaporated to dryness. The resulting product was loaded by dry method, and subjected to column chromatography using 1% triethylamine/5% methanol/dichloromethane as the eluent. The desired product was collected and concentrated and evaporated to dryness to provide 0.62 g of the product. $^1$H-NMR (600MHz, DMSO-$d_6$) δ 9.31-9.11 (m, 24H), 8.33-8.06 (m, 40H), 8.04-7.79 (m, 185H), 7.79-7.61 (m, 84H), 7.61-7.38 (m, 170H), 7.25-7.11 (m, 22H), 6.40-6.24 (m, 24H), 5.91-5.74 (m, 24H), 5.55-5.24 (m, 83H), 5.02-4.79 (m, 44H), 4.75-4.58 (m, 22H), 4.54-3.83 (m, 134H), 3.82-2.85 (m, 2850H), 2.70-2.00 (m, 454H), 1.91-0.90 (m, 534H)

Example 11: Synthesis of Compound **85-97**

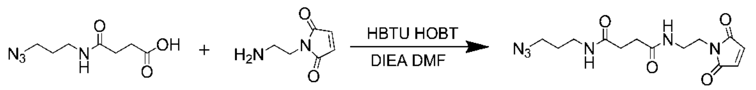


76-191

85-83

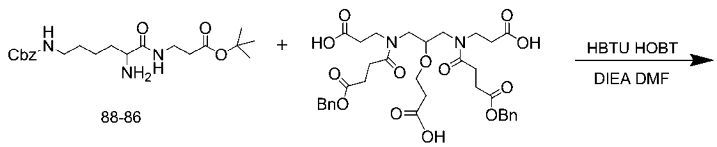


88-86

75-217

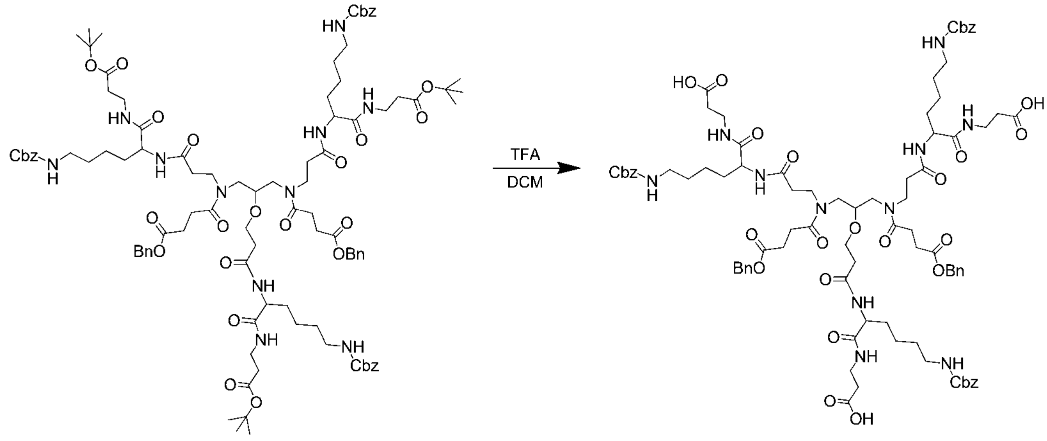


85-55

85-78

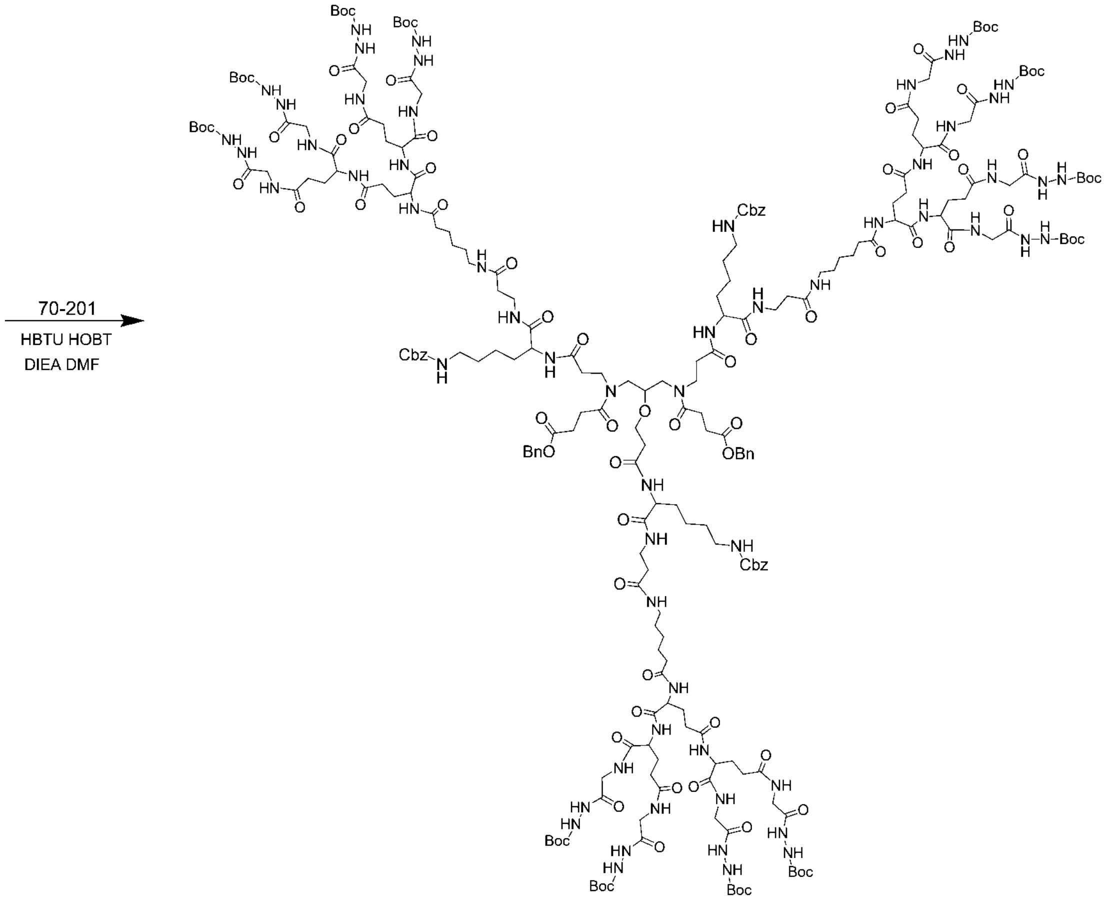


85-80

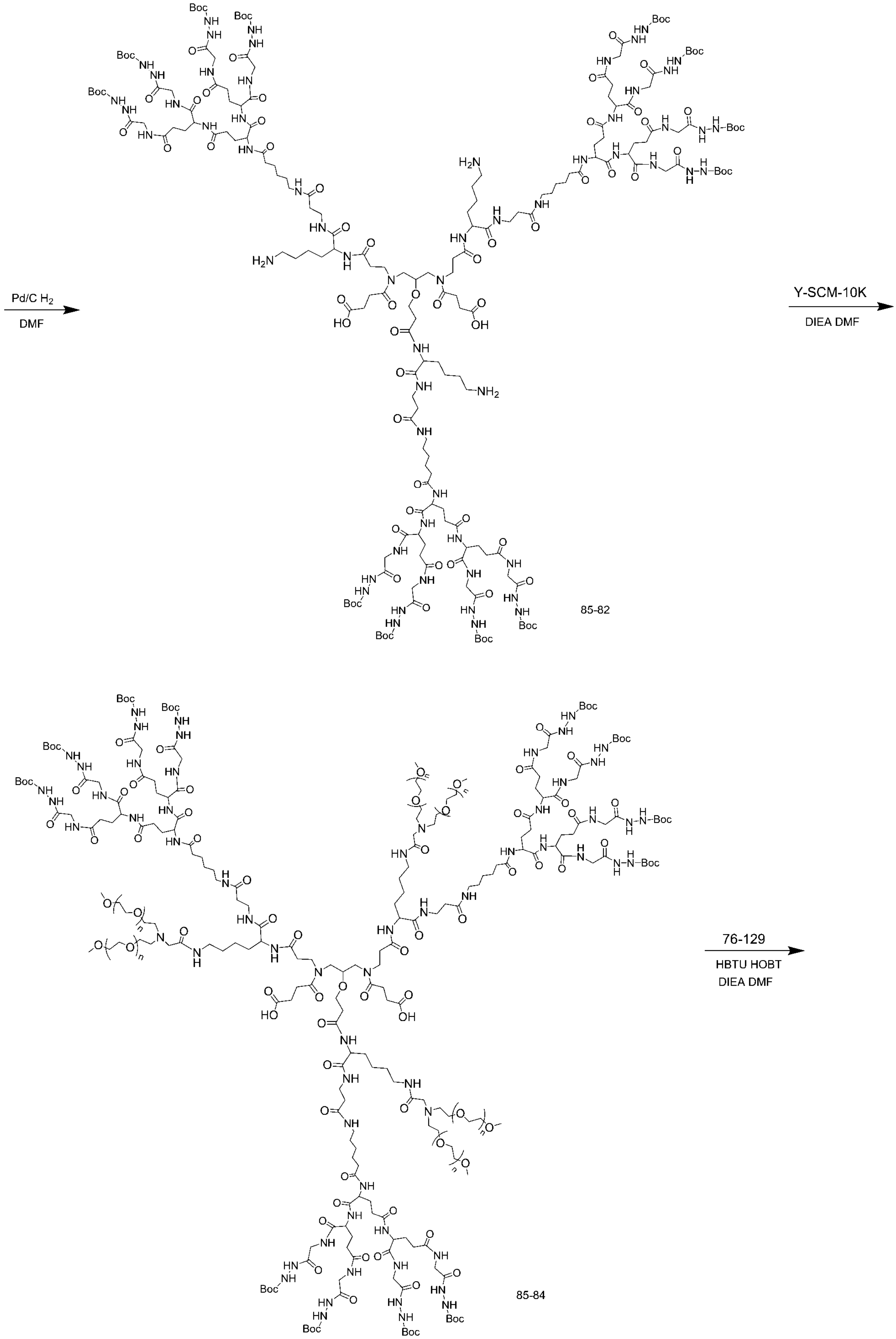


85-82

85-84

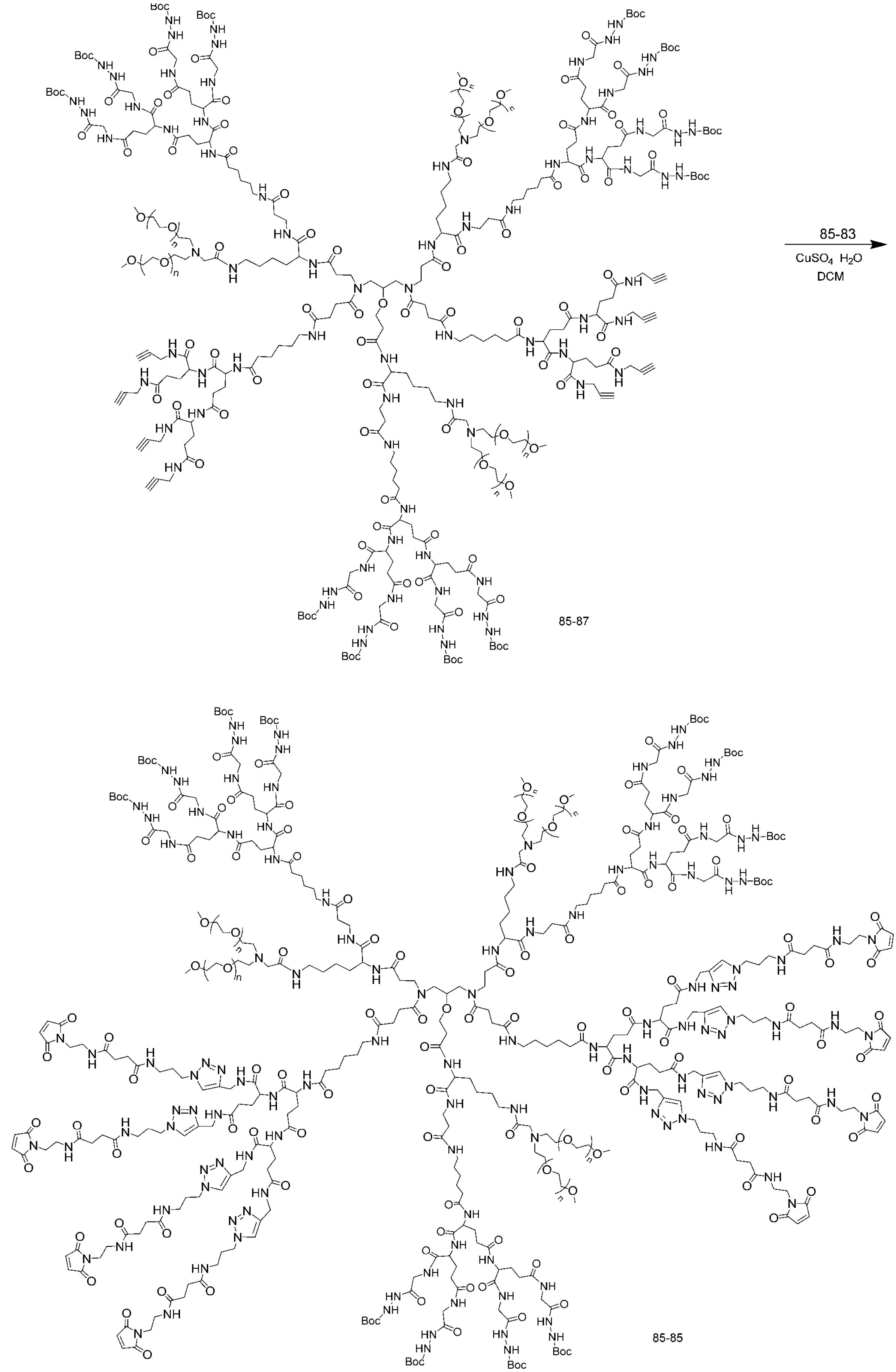


85-87

85-85

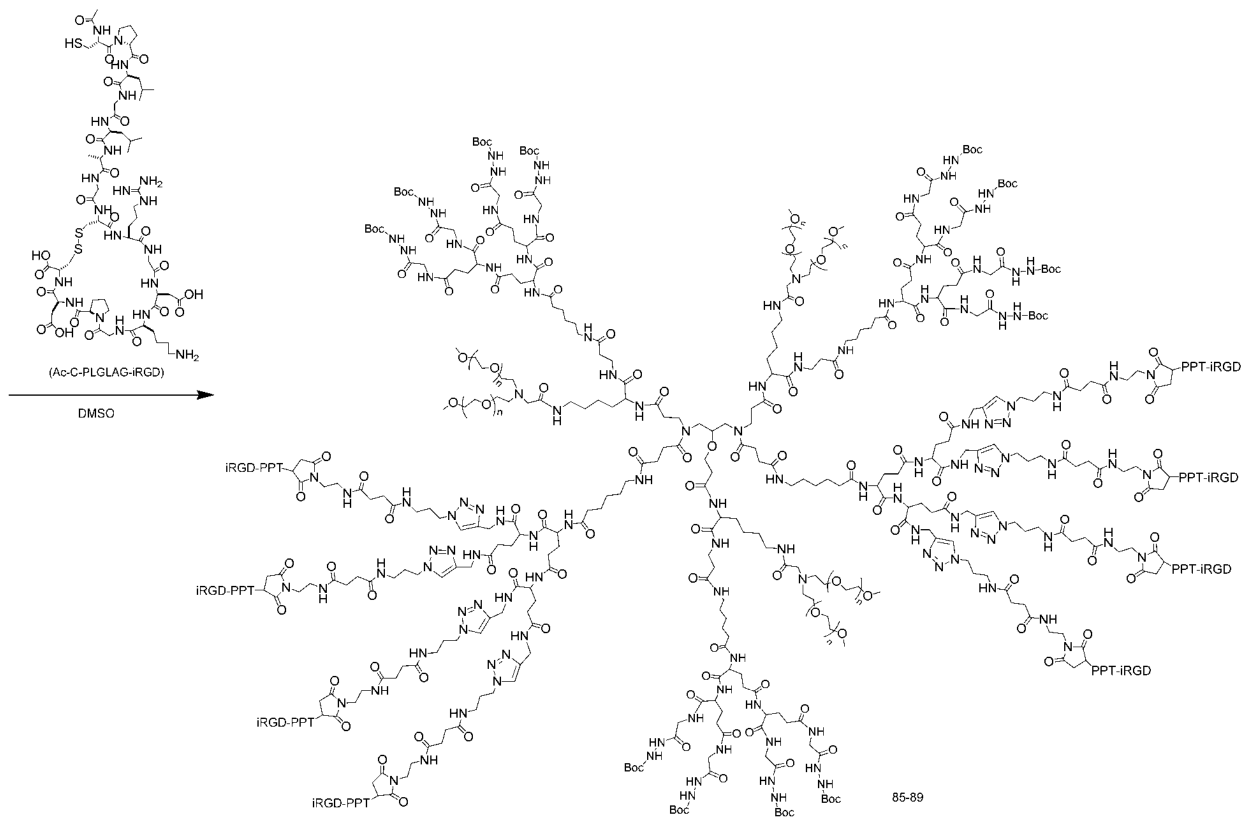


85-89

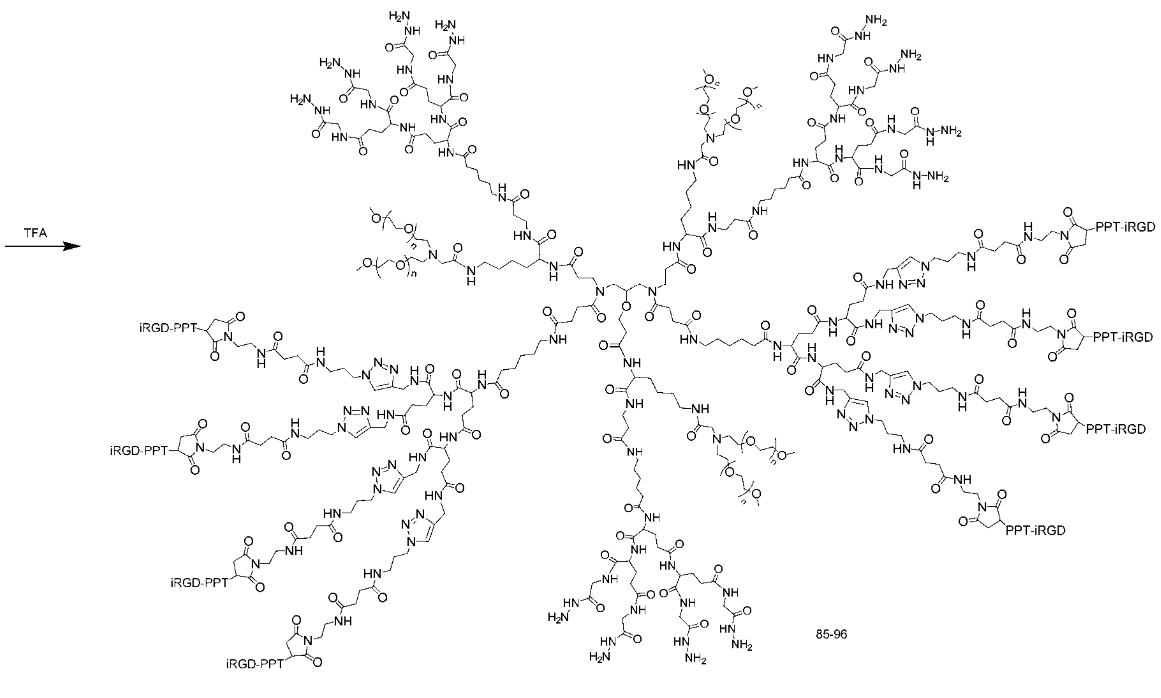


85-96

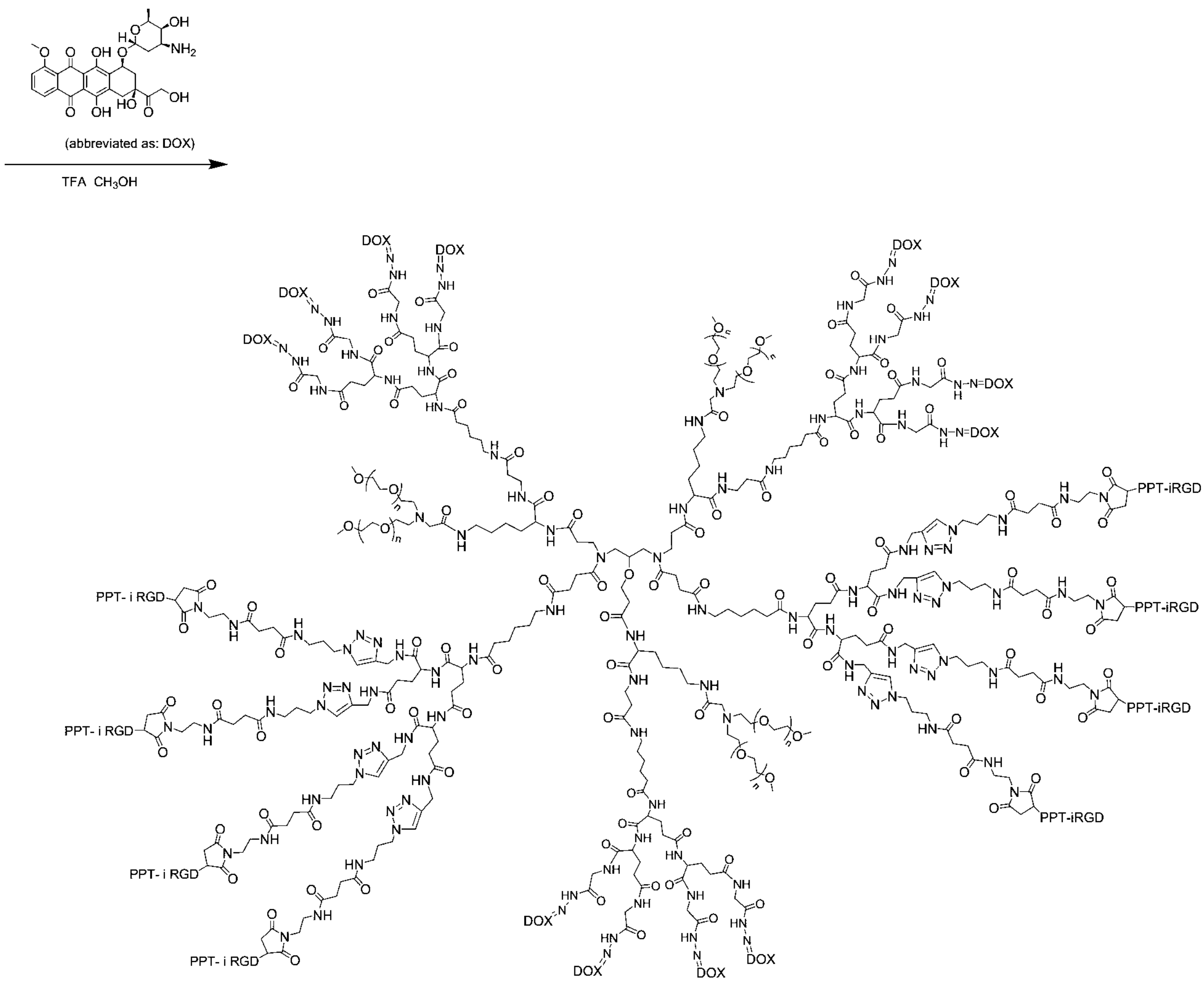


85-97

11.1 Preparation of **85-83**

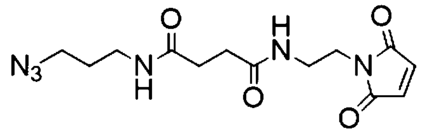

**76-191** (0.70 g, 3.5115 mmol), N-(2-aminoethyl)maleimide hydrochloride (0.62 g, 3.5115 mmol), HOBT (0.71 g, 5.2672 mmol) and HBTU (1.9 g, 5.2672 mmol) were taken and placed in a 100 mL flask, DMF (15 mL) was added for dissolution, and the contents were stirred at -10°C for approximately 10 minutes. DIEA (2.3 mL, 14.046 mmol) was then slowly added dropwise, and the reaction was continued at -10°C. TLC was used to monitor the reaction progress. After the reaction was completed, the reaction mixture was transferred to a 1 L separatory funnel, and saturated brine (150 mL) and ethyl acetate (150 mL) were added for extraction. The organic phase was collected, and the aqueous phase was further extracted with ethyl acetate (150 mL). The organic phases were combined and washed with deionized water (100 mL × 2), concentrated, and evaporated to dryness. The product was dissolved in a dichloromethane/methanol (4/1) mixture, then silica gel powder was added, and the mixture was evaporated to dryness. The resulting product was loaded by dry method, and subjected to column chromatography using a 60-80% ethyl acetate/petroleum ether mixture as the eluent. The organic solvent containing the product component was collected, concentrated, and dried in a vacuum oven to provide 0.17 g of the product.

11.2 Preparation of **85-55**

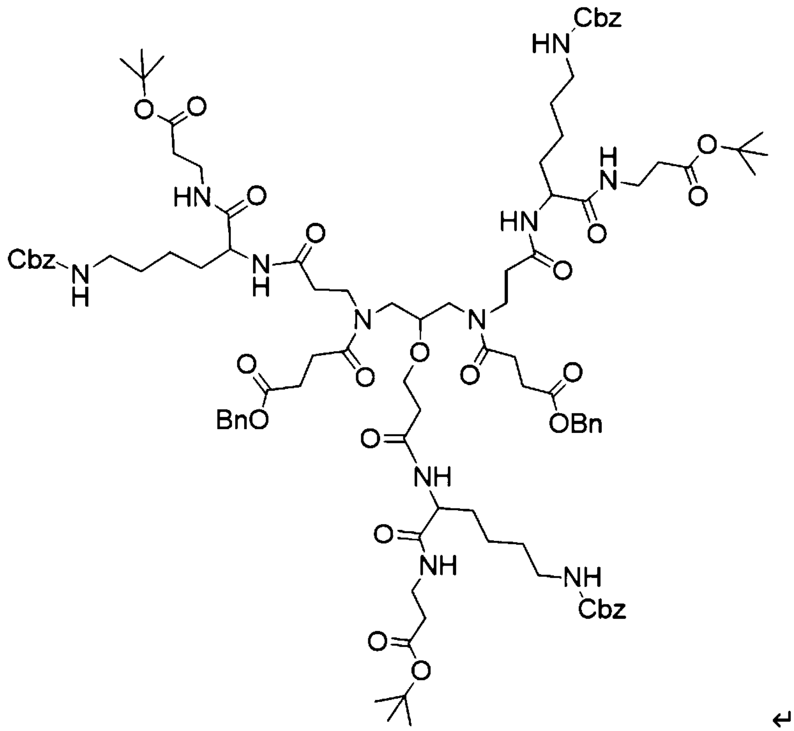

↵

**75-217** (1.6 g, 2.4093 mmol), **88-86** (3.24 g, 7.9508 mmol), HBTU (4.11 g, 10.8418 mmol) and HOBT (1.46 g, 10.8418 mmol) were placed in a 250 mL reaction flask, DMF (30 mL) was added for dissolution, and the contents were stirred at room temperature for approximately 20 minutes. DIEA (5.9 mL, 36.1395 mmol) was then slowly added dropwise, and the reaction mixture was stirred continuously at room temperature. After the reaction was completed, n-hexane (250 mL) and methyl tert-butyl ether (70 mL) were added for precipitation. The supernatant was discarded, and n-hexane and methyl tert-butyl ether were added again for precipitation. This process was repeated five times. The mixture was filtered to provide a solid product, which was dissolved in a dichloromethane/methanol mixed solvent, then silica gel powder was added, and the mixture was evaporated to dryness. The resulting product was loaded by dry method, and subjected to column chromatography using a 2-6% methanol/ dichloromethane mixture as the eluent. The desired product was collected, concentrated and dried in a vacuum oven to provide 2.64 g of the product with a yield of 60.17%. $^1$H-NMR (600 MHz, DMSO-$d_6$) $\delta$ 8.07-7.87 (m, 6H), 7.36-7.28 (m, 25H), 7.24-7.19 (m, 3H), 5.08-5.05 (m, 4H), 4.99 (s, 6H), 4.22-4.09 (m,5H), 3.69-3.61(m, 2H), 3.61-3.54 (m, 2H), 3.54-3.47 (m, 2H), 3.44-3.37 (m, 2H), 3.29-3.23 (m, 4H), 3.17-3.15 (m,4H), 2.99-2.90 (m,7H), 2.73-2.51 (m, 8H), 2.49-2.44 (m, 2H), 2.44-2.36 (m, 2H), 2.36-3.28 (m, 10H), 1.61-1.50 (m, 4H), 1.49-1.41 (m, 4H), 1.37 (s, 27H), 1.26-1.21 (m, 6H); ESI [M+Na$^+$] 1877.680

11.3 Preparation of **85-78**

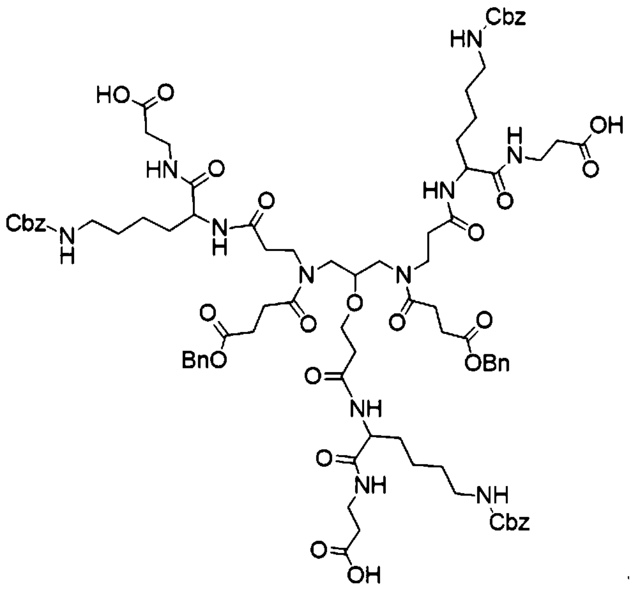

To a flask containing **85-55** (2.64 g, 1.4230 mmol) was added dichloromethane (5 mL). After dissolution with the assistance of ultrasonication, TFA (6.2 mL, 42.691 mmol) was added, and the reaction was stirred at room temperature. After the reaction was completed, the reaction mixture was concentrated and evaporated to dryness to remove dichloromethane and most of the TFA, and methyl tert-butyl ether (300 mL) was added for precipitation. A solid product precipitated and was filtered, and the filter cake was washed three times with methyl tert-butyl ether (40 mL). The filter cake was collected and dried in vacuum to provide 2.4 g of the product. $^1$H-NMR (600 MHz, DMSO-$d_6$) δ 11.0 (s, 3H), 8.08-7.90 (m, 6H), 7.36-7.29 (m, 25H), 7.24-7.17 (m, 3H), 5.08-5.04 (m, 4H), 4.99 (s, 6H), 4.23-4.11 (m, 4H), 3.70-3.44 (m, 12H), 3.22-3.16 (m, 4H), 2.98-2.91 (m, 6H), 2.70-2.61 (m, 4H), 2.59-2.53 (m, 4H), 2.40-2.28 (m,12H), 1.60-1.50 (m, 4H), 1.50-1.40 (m, 4H), 1.28-1.13 (m, 10H); ESI [M+H$^+$] 1684.76807

11.4 Preparation of **85-80**

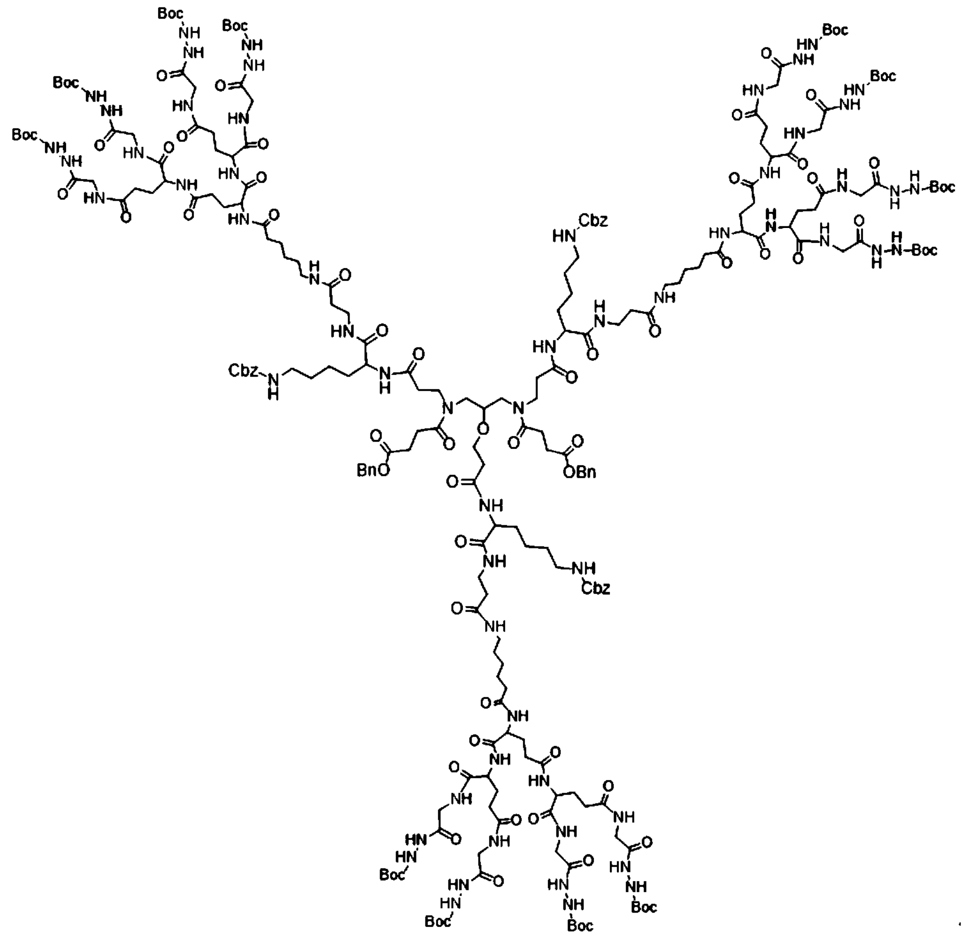

**85-78** (0.25 g, 0.1482 mmol), **70-201** (0.6 g, 0.48901 mmol), HBTU (0.25 g, 0.6669 mmol) and HOBT (0.09 g, 0.6669 mmol) were added to a 250 mL round-bottom flask, and dissolved in DMF (16 mL). The reaction flask was allowed to stand at -5°C, and the mixture was stirred for approximately 20 minutes. DIEA (0.4 mL, 2.223 mmol) was then slowly added dropwise. After the dropwise addition was completed, the reaction flask was placed at -5°C, and the reaction was carried out under stirring for 1 hour, then the reaction flask was brought to room temperature and stirring was continued. After the reaction was completed, n-hexane (30 mL) and methyl tert-butyl ether (100 mL) were added for precipitation, and the supernatant was decanted. This process was repeated three times. The mixture was filtered to provide a solid product. The solid product was dissolved in a 20% methanol/dichloromethane mixture, then silica gel powder was added, and the mixture was evaporated to dryness. The resulting product was loaded by dry method, and subjected to column chromatography using a 1% ammonia water/7-15% methanol/dichloromethane mixture as the eluent. The organic solvent containing the product component was collected, evaporated to dryness, and dried in a vacuum oven to provide 0.2 g of the product with a yield of 23.33%. $^1$H-NMR (600 MHz, DMSO-$d_6$) δ 8.36-8.22 (m, 9H), 8.18-7.98 (m, 24H), 7.37-7.29 (m, 25H), 7.21-7.01 (m, 24H), 5.09-5.04 (m, 4H), 4.98 (s, 6H), 4.33-4.19 (m, 12H), 4.15-4.05 (m, 25H), 3.72-3.68 (m, 12H), 3.29-3.27 (m, 4H), 3.02-2.97 (m, 6H), 2.91-2.88 (m, 4H), 2.56-2.52 (m,6H), 2.25-2.06 (m, 42H), 1.98-1.88 (m, 16H), 1.73-1.71 (m, 6H), 1.52-1.46 (m, 12H), 1.39 (s, 108H), 1.25-1.23 (m, 18H)

11.5 Preparation of **85-82**

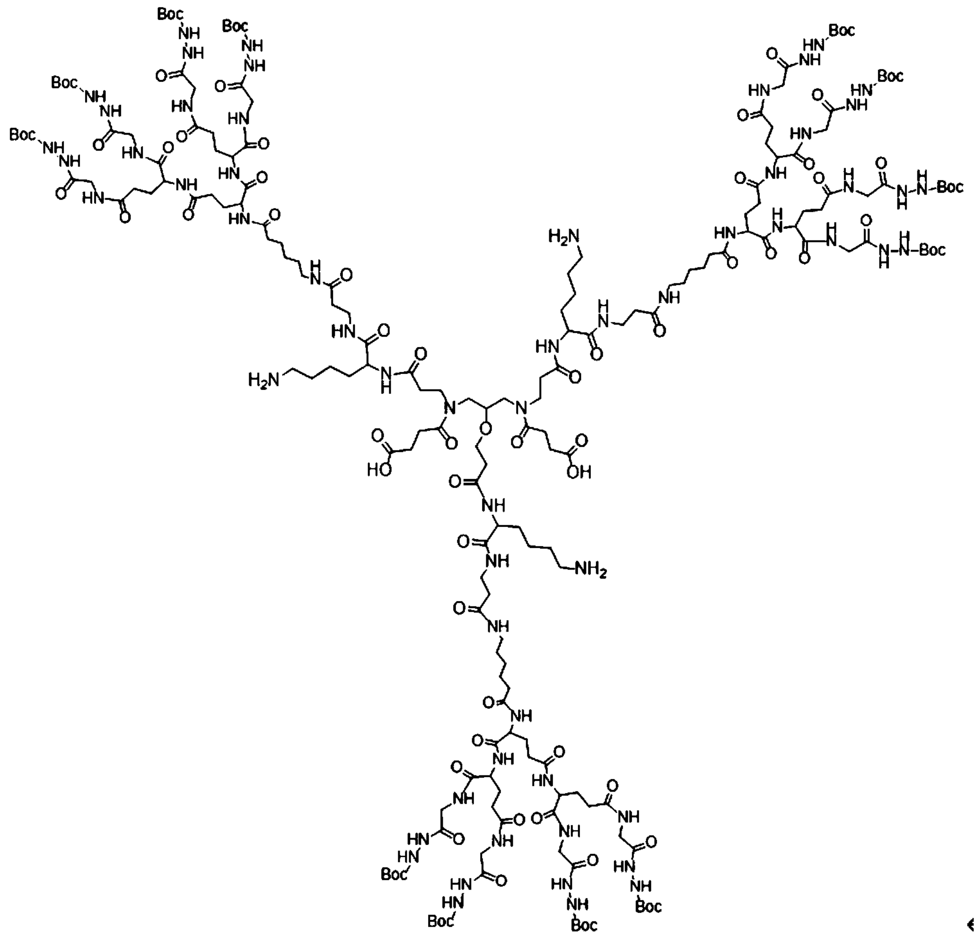

**85-80** (0.2 g, 0.0381 mmol) was dissolved in anhydrous DMF by heating, and then added to a hydrogenation reactor. 10% Pd/C catalyst (0.1 g) was then added. The hydrogenation reactor was sealed, filled with hydrogen, and then evacuated with a water pump. This process was repeated three times. Finally, the pressure reading of the hydrogenation reactor was adjusted to 2.0 MPa, and the mixture was stirred overnight in an oil bath at 43°C. After the reaction was completed, the reaction mixture was filtered through Celite, the Celite was washed with DMF (5 mL × 3), and a liquid product was obtained. Methyl tert-butyl ether and n-hexane were added for precipitation, the supernatant was discarded, and methyl tert-butyl ether and n-hexane were added again. This process was repeated three times. Finally, the mixture was filtered, the filter cake was dried to provide 0.17 g of the product. $^1$H-NMR (600 MHz, DMSO-$d_6$) δ 9.70-9.56 (m, 2H), 8.20-7.89 (m, 54H), 5.11 (s, 6H), 4.31-4.11 (m,12H), 3.81-3.64 (m, 27H), 3.12-3.05 (m, 20H), 3.03-2.97 (m, 10H), 2.64-2.60 (m, 6H), 2.42-2.34 (m, 10H), 2.30-2.03 (m,42H), 1.75-1.69 (m,6H), 1.44-1.42 (m, 12H), 1.40-1.37 (m, 108H) , 1.28-1.21 (m, 18H)

11.6 Preparation of **85-84**

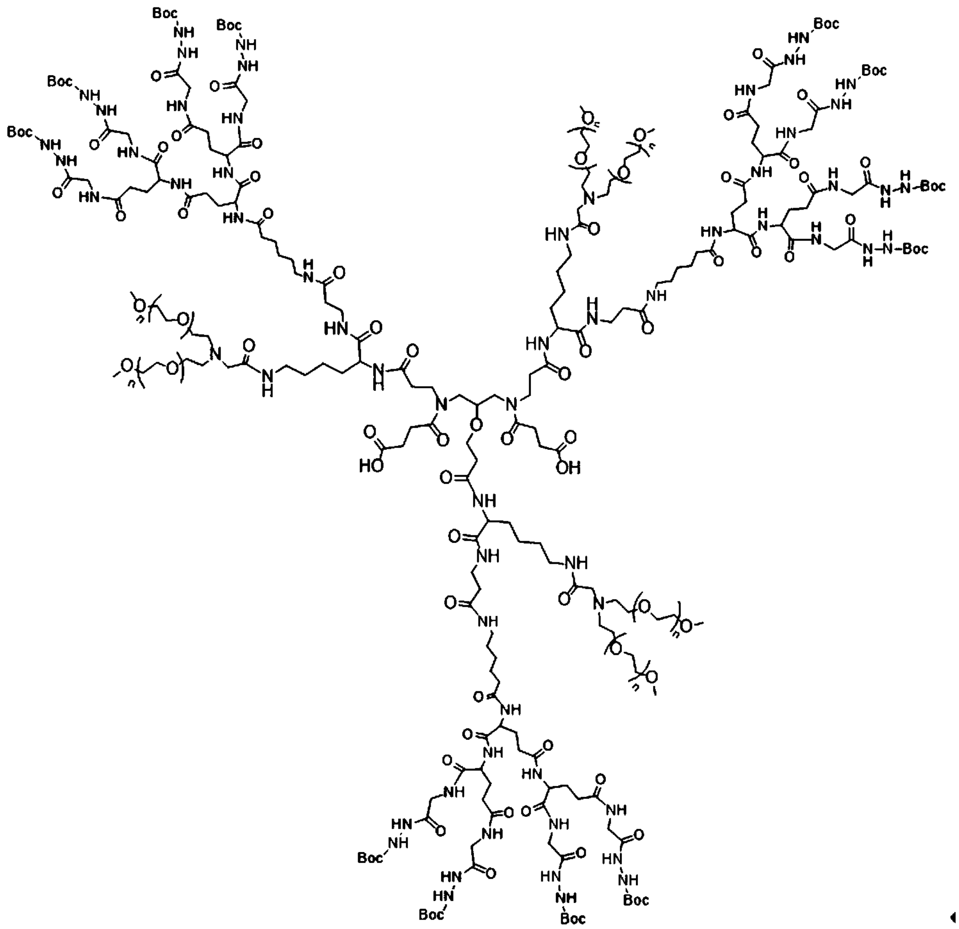

**85-82** (0.17 g, 0.0381 mmol) was placed in a 250 mL flask, dissolved in anhydrous DMF (10 mL), and the contents were stirred at 0°C for 30 minutes. DIEA (0.2 mL, 1.4287 mmol) was then slowly added dropwise. After stirring at low temperature for 10 minutes, Y-NHS-10K (1.17 g, 0.1143 mmol, purchased from Jenkem) was added. The reaction was carried out under stirring at low speed at room temperature in the dark for one week. After the reaction was completed, methyl tert-butyl ether (100 mL) and n-hexane (30 mL) were added. A solid precipitated and was filtered. The filter cake was washed with methyl tert-butyl ether (40 mL × 3). The filter cake was collected and dried in a vacuum oven to provide 1.07 g of the product. $^1$H-NMR (600 MHz, DMSO-$d_6$) δ 9.70-9.56 (m, 2H), 8.22-7.79 (m, 57H), 4.19-4.12 (m,12H), 3.71-3.69 (m, 27H), 3.54-3.49 (m, 2727H), 3.06-2.96 (m, 30H), 2.41-2.36 (m, 16H), 2.21-2.15 (m, 42H), 1.74-1.70 (m,6H), 1.46-1.45 (m, 12H), 1.41-1.38 (m, 108H), 1.21-1.16 (m, 18H)

11.7 Preparation of **85-87**

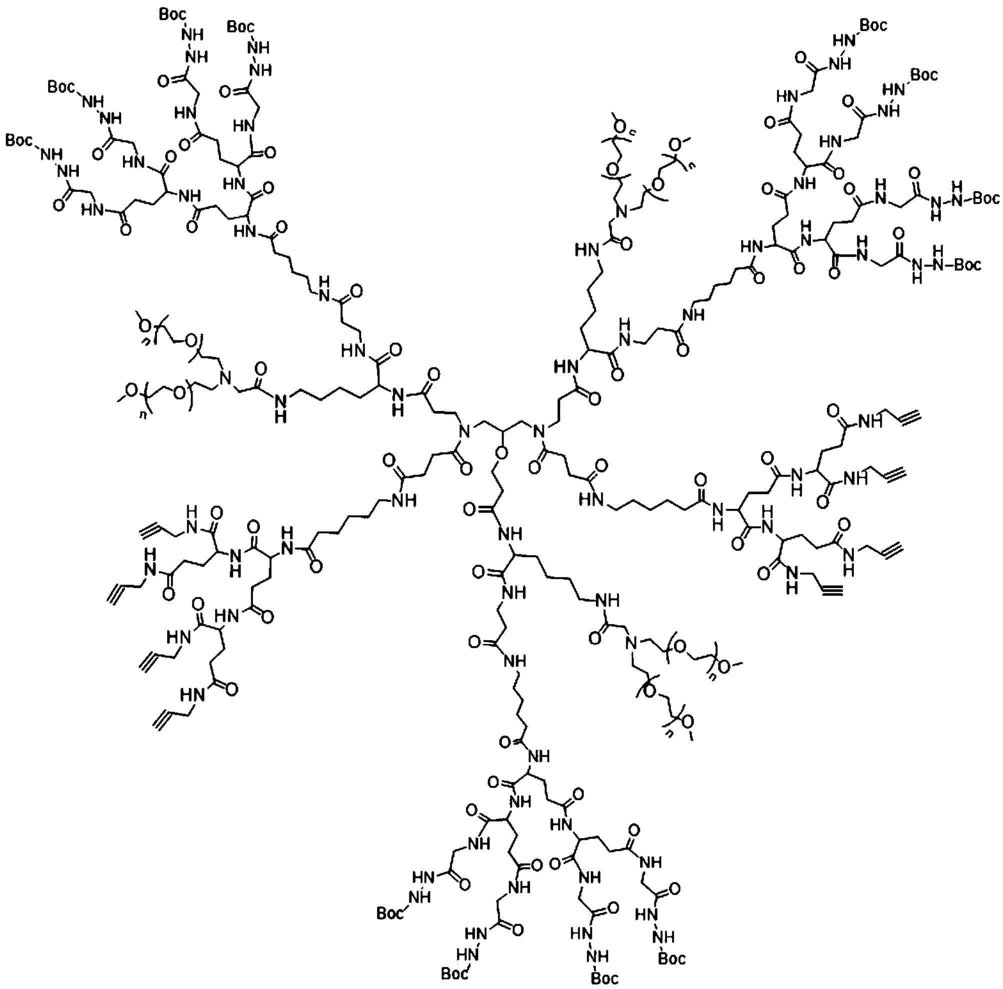

**85-84** (0.5 g, 0.0142 mmol), **76-129** (0.028 g, 0.0426 mmol), HBTU (0.016 g, 0.0426 mmol) and HOBT (0.0057 g, 0.0426 mmol) were placed in a 250 mL flask, DMF (15 mL) was added to dissolve the mixture, and the contents were stirred at room temperature for approximately 20 minutes. DIEA (0.02 mL, 0.1279 mmol) was then slowly added dropwise, and the reaction was stirred in the dark continuously at room temperature. After the reaction was completed, n-hexane (250 mL) and methyl tert-butyl ether (70 mL) were added for precipitation. The supernatant was discarded, and n-hexane and methyl tert-butyl ether were added again for precipitation. This process was repeated five times. The mixture was filtered to obtain a solid product, which was dried in a vacuum oven to provide 0.51 g of the product. $^1$H-NMR (600 MHz, DMSO-$d_6$) δ 8.32-7.95 (m, 73H), 4.25-4.11 (m, 24H), 4.01-3.95 (m, 8H), 3.75-3.70 (m, 27H), 3.54-3.49 (m, 2727H), 3.16-3.05 (m, 42H), 2.81-2.76 (m, 16H), 2.17-2.09 (m, 72H), 1.54-1.49 (m, 22H), 1.41-1.38 (m, 108H), 1.27-1.25 (m, 26H)

11.8 Preparation of **85-85**

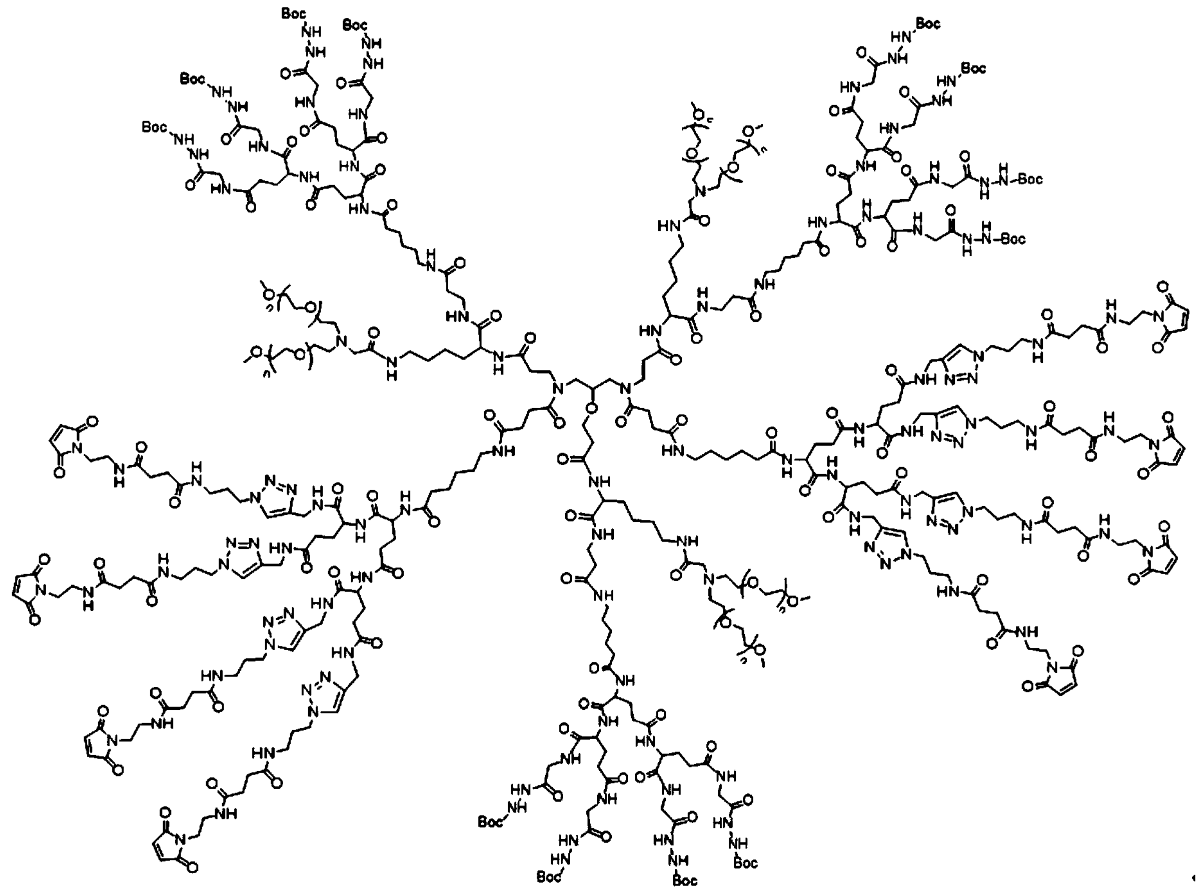

**85-87** (0.51 g, 0.0142 mmol) and **85-83** (0.054 g, 0.1704 mmol) were placed in a reaction flask, and anhydrous DMF (16 mL) was added to dissolve the mixture. The reaction flask was then filled with nitrogen. $CuSO_4$ (0.036 g, 0.2272 mmol) and sodium ascorbate (0.09 g, 0.4544 mmol) were then added. The reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried to provide 0.44 g of the product. $^1$H-NMR (600 MHz, DMSO-$d_6$) δ 8.54-8.21 (m,33H), 8.18-7.89 (m, 32H), 7.76-7.67 (m, 8H), 7.21-6.92 (m, 24H), 6.85-6.62 (m, 16H), 4.52-4.41 (m,24H), 4.38-4.30 (m,18H), 4.21-4.11 (m, 33H), 3.87-3.81 (m, 44H), 3.54-3.49 (m, 2727H), 3.25-3.22 (m, 20H), 3.09-3.06 (m, 6H), 2.90-2.88 (m, 4H), 2.64-2.60 (m, 10H), 2.46-2.35 (m, 48H), 2.25-2.05 (m, 56H), 1.94-1.80 (m, 16H), 1.79-1.63 (m, 20H), 1.55-1.47 (m, 16H), 1.41-1.38 (m, 108H), 1.26-1.25 (m, 26H)

11.9 Preparation of **85-89**

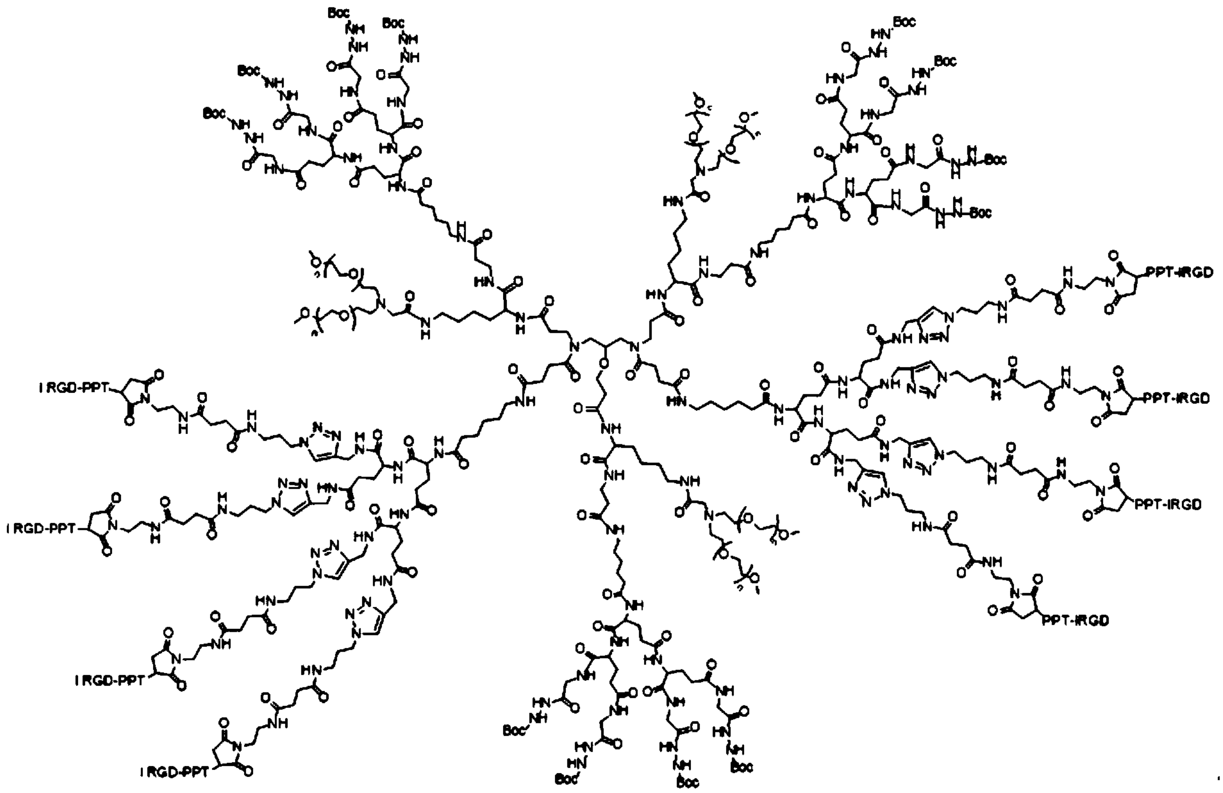

**85-85** (0.1 g, 0.0025 mmol) and Ac-C-PLGLAG-iRGD (0.032 g, 0.0204 mmol) were added to a 100 mL flask, then DMSO (6 mL) was added to dissolve the mixture, and the reaction was stirred at room temperature overnight. After the reaction was completed, n-hexane (20 mL) and methyl tert-butyl ether (200 mL) were added for precipitation, the supernatant was discarded, and n-hexane and methyl tert-butyl ether were added again for precipitation. This process was repeated six times. The mixture was filtered to provide a solid product, which was dried in a vacuum oven to provide 0.129 g of the product.

11.10 Preparation of **85-96**

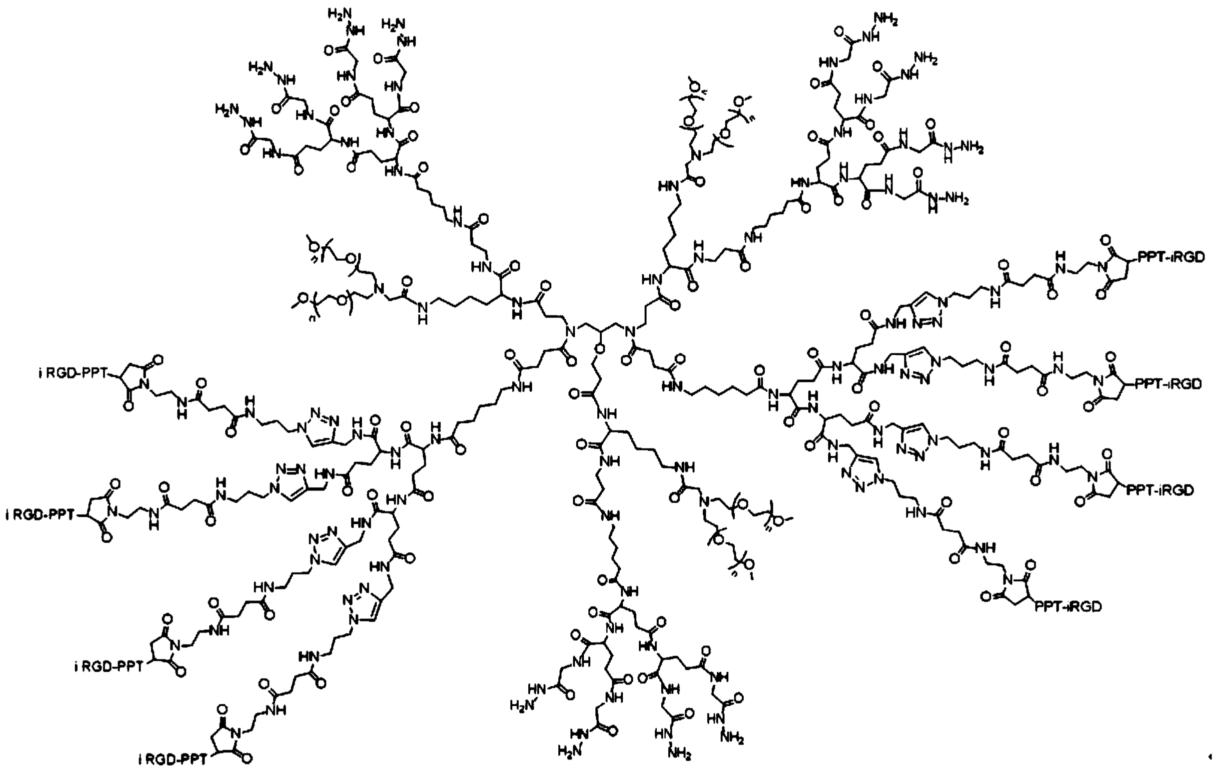

TFA (0.1 mL, 1.34 mmol) was added into a flask containing **85-89** (0.129 g, 0.0025 mmol) for dissolution with the assistance of ultrasonication, and the reaction was stirred at room temperature overnight. After the reaction was completed, the reaction mixture was concentrated and evaporated to dryness, dissolved in DMF (5 mL), neutralized with excess DIEA, and then precipitated with methyl tert-butyl ether (200 mL) and n-hexane (100 mL). The supernatant was discarded, and methyl tert-butyl ether (200 mL) and n-hexane (100 mL) were added again for precipitation. A solid product precipitated and was filtered by suction. The filter cake was washed three times with methyl tert-butyl ether (40 mL). The filter cake was collected and dried under vacuum to provide 0.126 g of the product.

11.11 Preparation of **85-97**

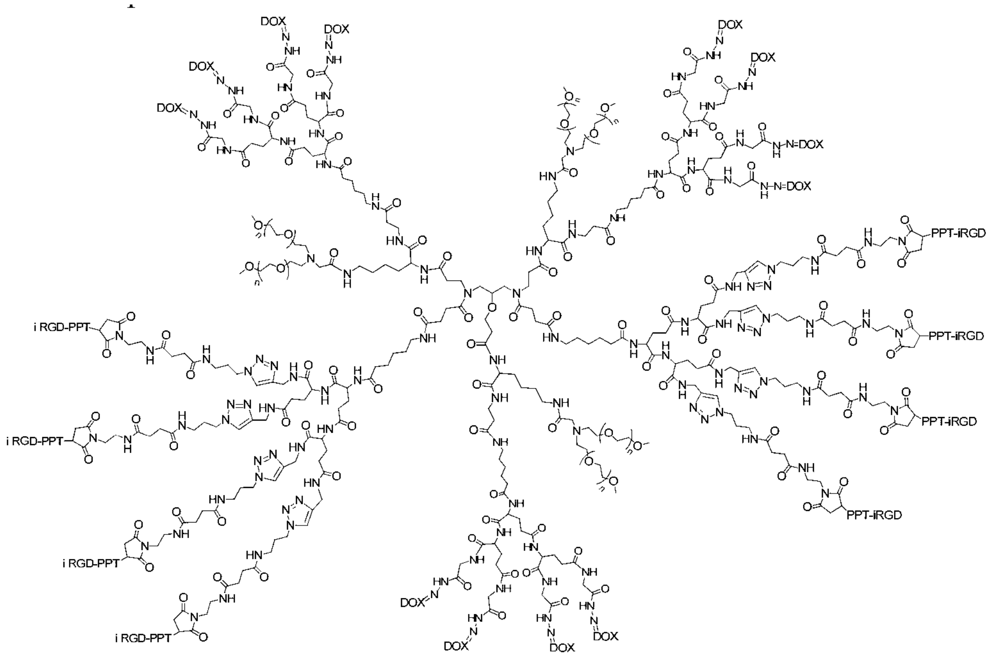

Reactants **85-96** (0.126 g, 0.0025 mmol) and DOX (0.034 g, 0.06 mmol) were placed in a reaction flask, methanol (20 mL) was added for dissolution, then TFA (0.003 mL, 0.0375 mmol) was added, and the reaction mixture was stirred at room temperature. The reaction progress was monitored by TLC. After the reaction was completed, the reaction mixture was filtered to remove solid, the filtrate was evaporated to dryness using a rotary evaporator, and methyl tert-butyl ether was added for precipitation to provide a powdery solid, which was filtered, and the filter cake was collected. The resulting solid product was washed three times with a 2% methanol/dichloromethane mixture. The filtrate was collected, concentrated, and dried to provide 0.061 g of the product. $^1$H-NMR (600 MHz, DMSO-$d_6$) δ9.10-9.04 (m, 24H), 8.44-8.39 (m, 49H), 8.30-8.27 (m, 32H), 8.08-7.99 (m, 112H), 7.85-7.82 (m, 56H), 7.30-7.18 (m, 48H), 4.97-4.95 (m, 64H), 4.91-4.86 (m, 60H), 4.35-4.31 (m, 50H), 4.26-4.24 (m, 64H), 4.20-4.17 (m, 109H), 3.87-3.82(m, 100H), 3.64-3.63 (m, 80H), 3.54-3.49 (m, 2727H), 3.24-3.23 (m, 90H), 3.06-3.01 (m, 36H), 2.93-2.91 (m, 24H), 2.76-2.72 (m, 14H), 2.63-2.60 (m,28H), 2.41-2.38 (m, 24H), 2.17-2.14 (m, 172H), 1.88-1.87 (m, 40H), 1.70-1.67 (m,84H), 1.52-1.47 (m, 88H), 1.28-1.26 (m, 42H), 1.19-1.18 (m, 36H), 0.89-0.86 (m, 96H)

Example 12 Synthesis of Compound **85-26**

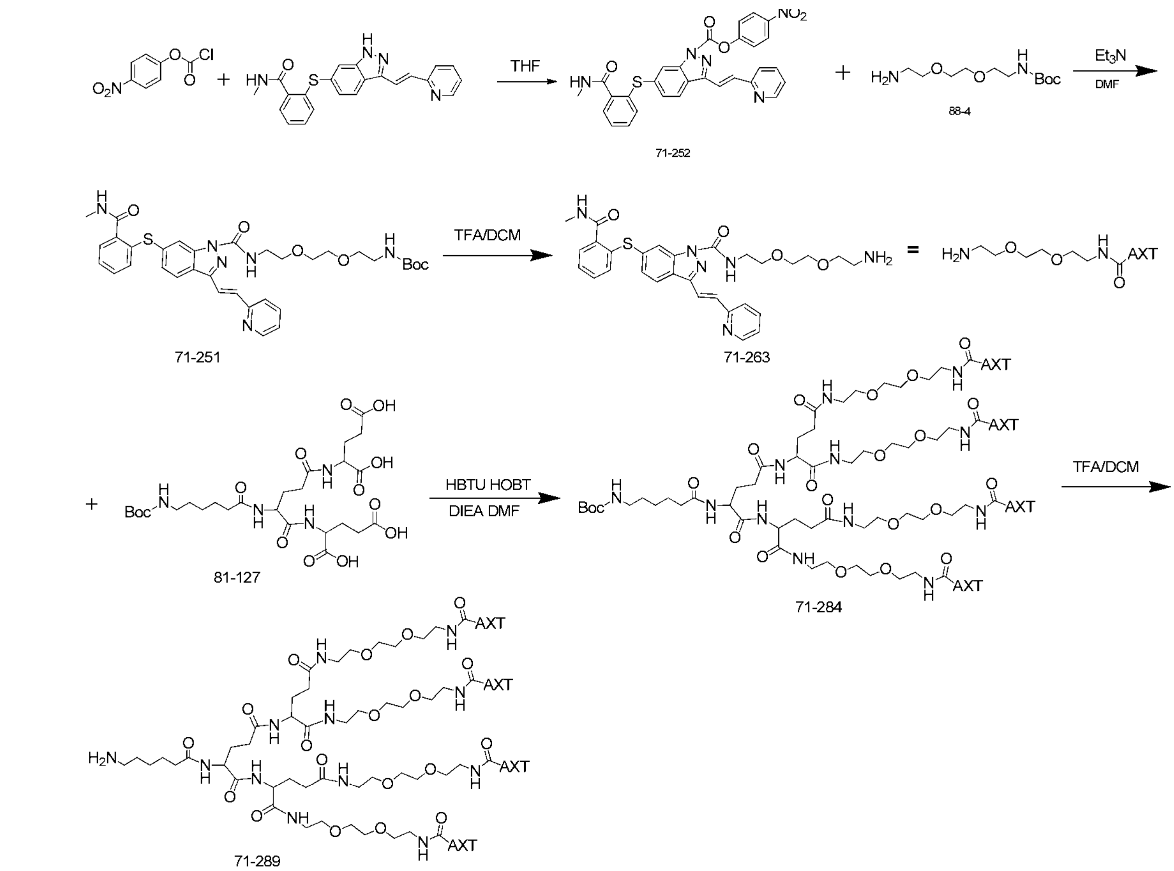


88-4

71-252

71-251

71-263

81-127

71-284

71-289

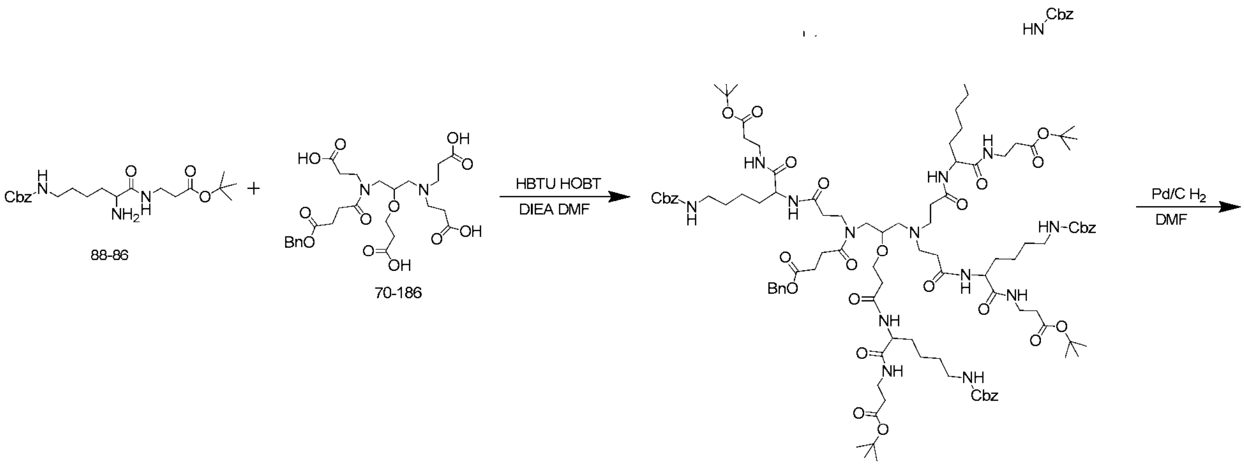


88-86

70-186

71-264

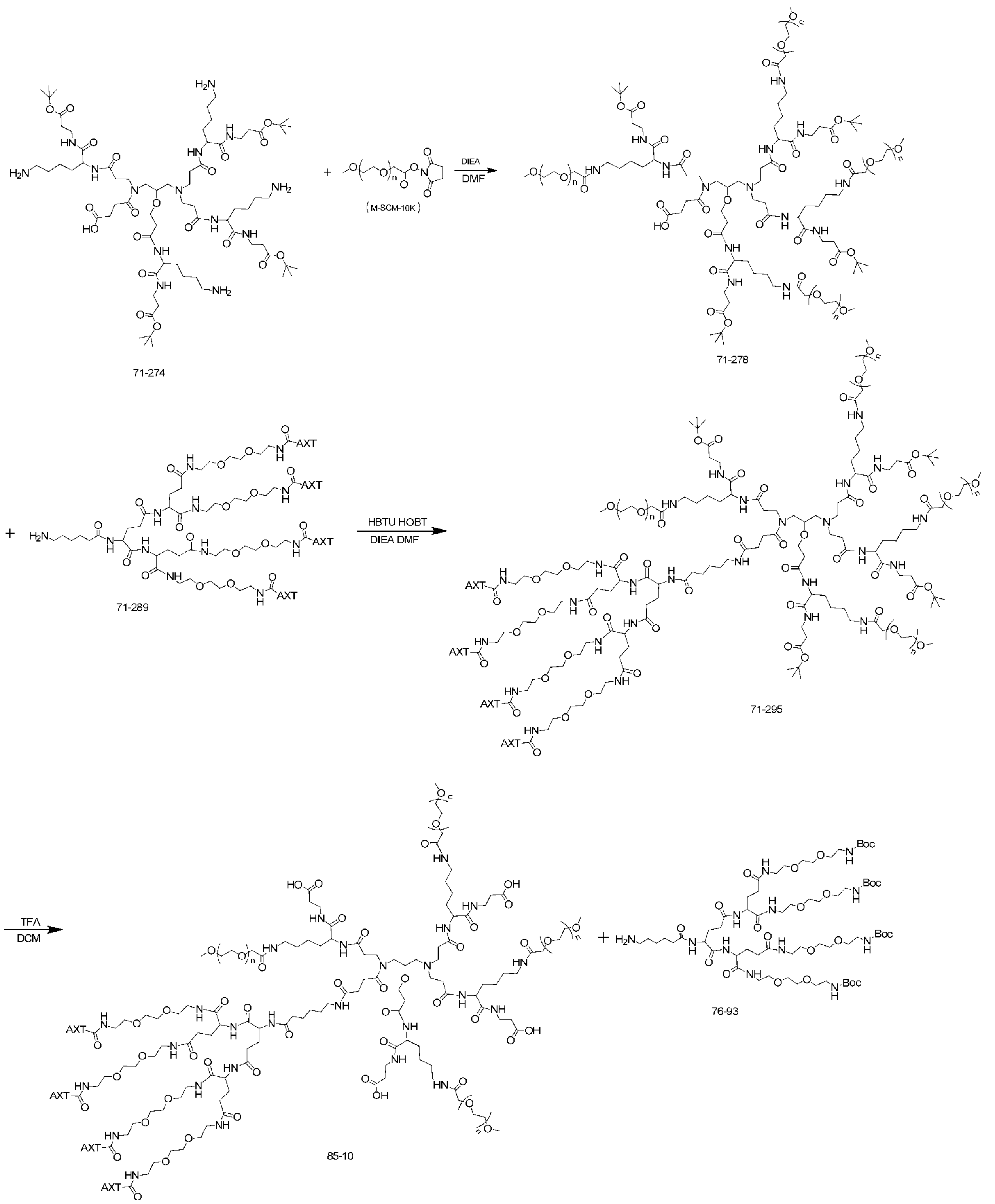


71-274

71-278

71-289

71-295

85-10

76-93

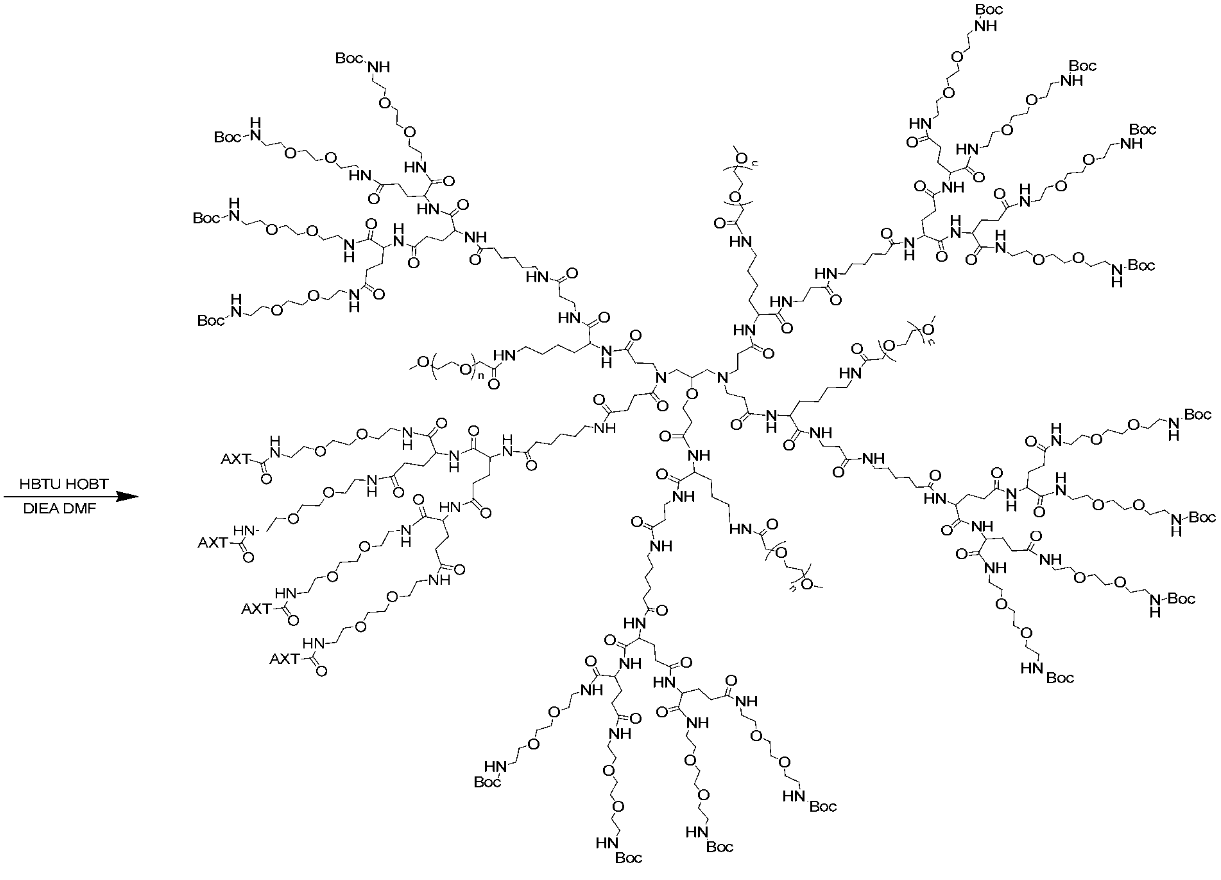


85-16

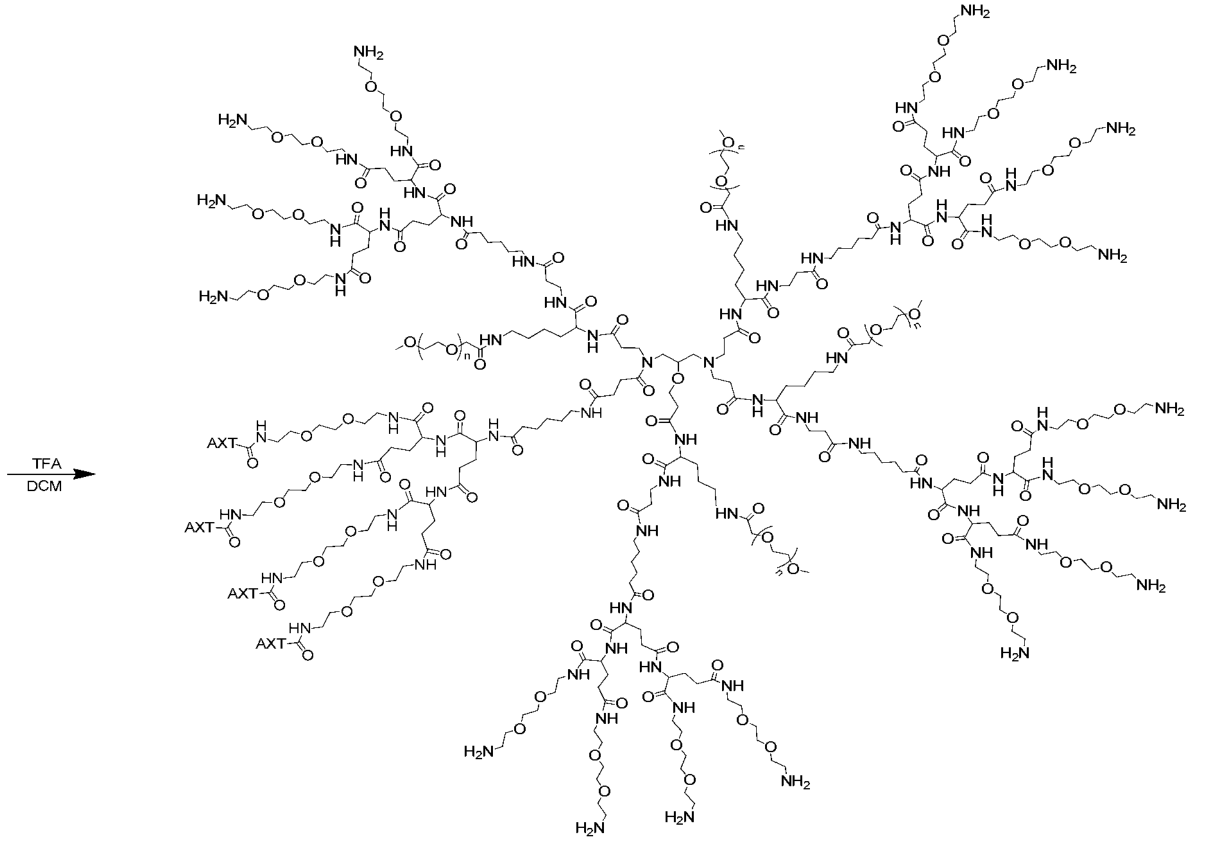


85-23

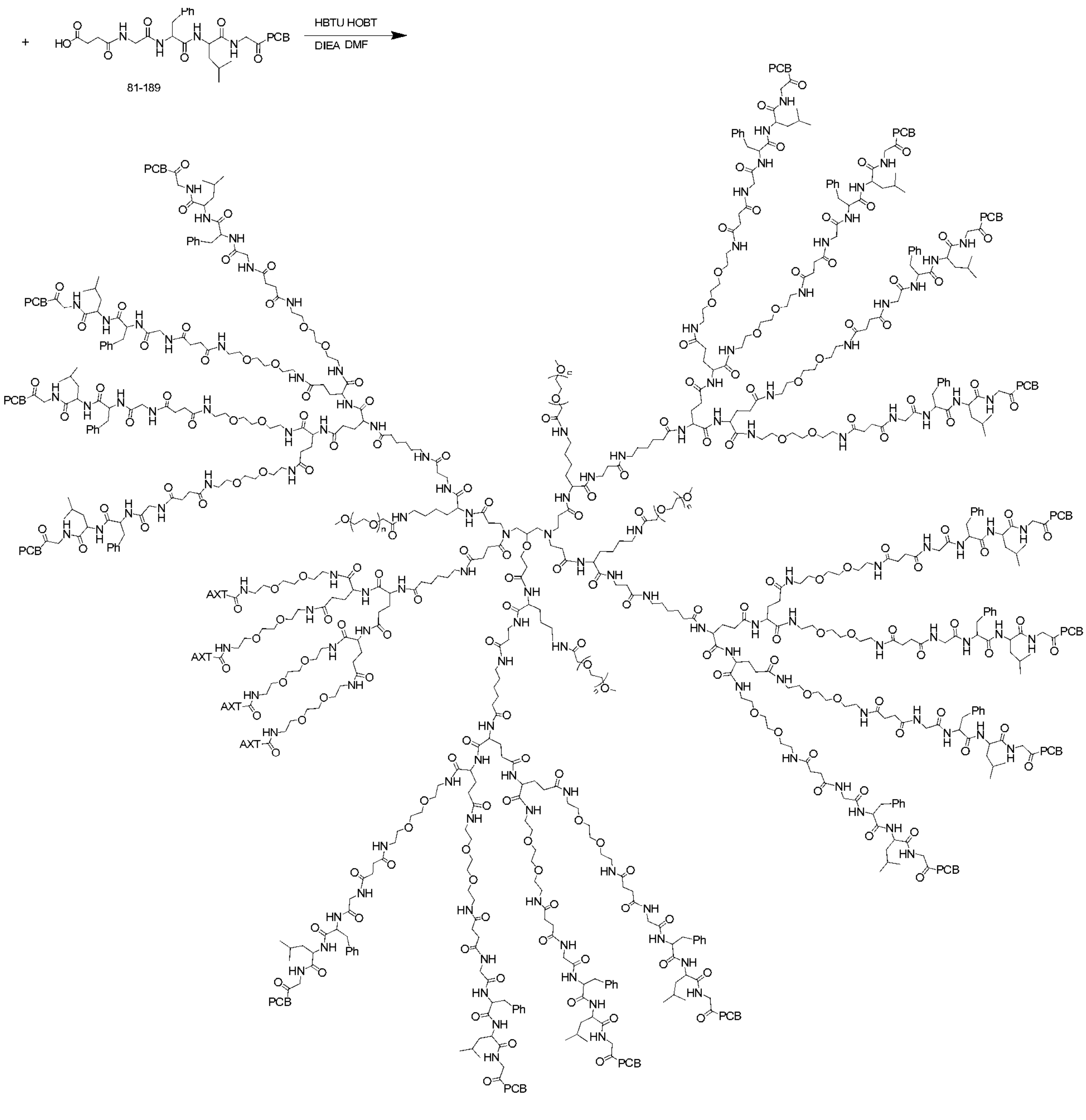


81-189

85-26

12.1 Preparation of **71-252**

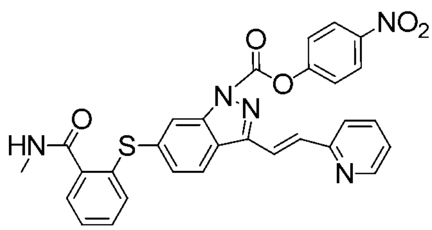

Axitinib (5 g, 12.938 mmol, referred to as AXT) and 4-nitrobenzene chloroformate (5.215 g, 25.875 mmol) were weighed, and added to THF (approximately 500 mL) for dissolution with the assistance of ultrasonication, resulting a homogeneous phase. The reaction was carried out under stirring in an oil bath at 75°C under reflux for 4 hours. After the

reaction was completed, methyl tert-butyl ether (150 mL) and n-hexane (100 mL) were added to the reaction mixture to precipitate a solid product, which was filtered and dried under vacuum to provide 7.4 g of the product. ESI [M+H$^+$] 552.132

12.2 Preparation of **71-251**

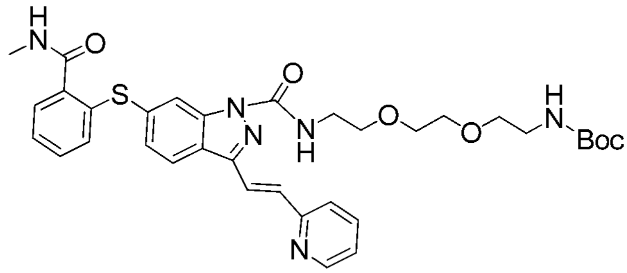

**71-252** (3.0 g, 5.4389 mmol) and **88-4** (1.35 g, 5.4389 mmol) were weighed and added to a reaction flask, then triethylamine (2.3 mL, 16.3169 mmol) was added, dissolved with DMF (26 mL), and the reaction was stirred at room temperature. After the reaction was completed, n-hexane and methyl tert-butyl ether were added to the reaction mixture to precipitate a powdery solid, the supernatant was discarded, and the solid was dissolved in a 20% methanol/ dichloromethane mixture, then silica gel powder (20 mL) was added, and the mixture was evaporated to dryness. The resulting product was loaded by dry method, and subjected to column chromatography using a 0-2% methanol/dichloromethane mixture as the eluent. The desired product was collected, concentrated, and dried in a vacuum oven to provide 2.57 g of the product with a yield of 69.45%. $^1$H-NMR (600 MHz, DMSO-$d_6$) δ 8.65 (d, *J* = 4.5 Hz, 1H), 8.47-8.37 (m, 2H), 8.35 (s, 1H), 8.30 (d, *J* = 8.4 Hz, 1H), 7.98-7.93 (m, 1H), 7.86 (dd, *J* = 7.6, 1.6 Hz, 1H), 7.82 (d, *J* = 16.3 Hz, 1H), 7.72 (d, *J* = 7.8 Hz, 1H), 7.51 (dd, *J* = 7.3, 1.6 Hz, 1H), 7.40-7.28 (m, 4H), 7.12 (d, *J* = 7.6 Hz, 1H), 6.76 (s, 1H), 3.64-3.54 (m, 4H), 3.49 (ddd, *J* = 17.7, 8.4, 4.4 Hz, 4H), 3.38 (d, *J* = 12.1 Hz, 2H), 3.10-3.00 (m, 2H), 2.76 (d, *J* = 4.6 Hz, 3H), 1.34 (s, 9H); ESI [M+Na$^+$] 683.26147

12.3 Preparation of **71-263**

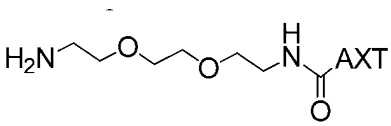

**71-251** (2.57 g, 3.8923 mmol) was weighed and dissolved in dichloromethane (5 mL), then TFA (2.9 mL, 38.923 mmol) was added, and the reaction mixture was stirred at room temperature. After the reaction was completed, the reaction mixture was concentrated, and methyl tert-butyl ether (150 mL) and n-hexane (100 mL) were added for precipitation. A solid product precipitated and was filtered. The solid was dissolved in a 20% methanol/ dichloromethane mixed solvent, then silica gel powder was added. The mixture was mixed evenly and evaporated to dryness. The resulting product was loaded by dry method, and subjected to column chromatography using 1% ammonia water/5% methanol/dichloromethane as the eluent. The solution fraction of the desired product was collected, concentrated, and evaporated to dryness to provide 2.18 g of the product.

12.4 Preparation of **71-284**

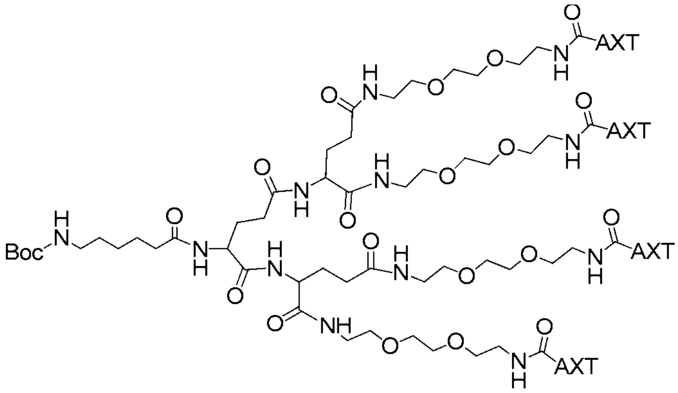

**71-263** (2.18 g, 3.8922 mmol), **81-127** (0.54 g, 0.8846 mmol), HBTU (2.01 g, 5.3076 mmol) and HOBT (0.717 g, 5.3076 mmol) were placed in a 500 mL reaction flask, DMF (50 mL) was added for dissolution with the assistance of ultrasonication, and the contents were stirred at 0°C for approximately 20 minutes. DIEA (2.6 mL, 17.692 mmol) was then slowly added dropwise. The reaction was stirred continuously at 0°C for 1 hour, and then at room temperature. After the reaction was completed, n-hexane (250 mL) and methyl tert-butyl ether (70 mL) were added for precipitation, the supernatant was discarded, and n-hexane and methyl tert-butyl ether were added again for precipitation. This process was repeated four times. The mixture was filtered to obtain a solid product. The obtained solid product was dissolved in a 20% methanol/ dichloromethane mixture, then silica gel powder was added, and the mixture was evaporated to dryness. The resulting product was loaded by dry method, and subjected to column chromatography using a 2-5% methanol/dichloromethane mixture as the eluent. The desired product was collected, concentrated and dried in a vacuum oven to provide 2.12 g of the product with a yield of 86.17%. $^1$H-NMR (600 MHz, DMSO-$d_6$) δ 8.64-8.61 (m, 4H), 8.43-8.40 (m, 4H), 8.38-8.35 (m,4H), 8.34-8.31 (m, 4H), 8.27-8.26 (m, 4H), 8.22-8.16 (m, 2H), 8.09-8.03 (m, 2H), 7.99-7.89 (m, 6H), 7.85-7.76 (m, 10H), 7.72-7.66 (m, 4H), 7.53-7.47 (m, 4H), 7.35-7.30 (m, 16H), 7.13-7.06 (m, 4H) 4.25-4.13 (m, 3H), 3.60-3.42 (m, 40H), 3.26-3.21 (m, 2H), 3.17-3.12 (m, 8H), 2.78-2.74 (m, 12H), 2.21-1.97 (m, 10H), 1.91-1.77 (m, 4H), 1.46-1.40 (m, 2H), 1.33 (s, 9H), 1.25-1.12 (m, 4H); ESI [M+Na$^+$] 2810.351

12.5 Preparation of **71-289**

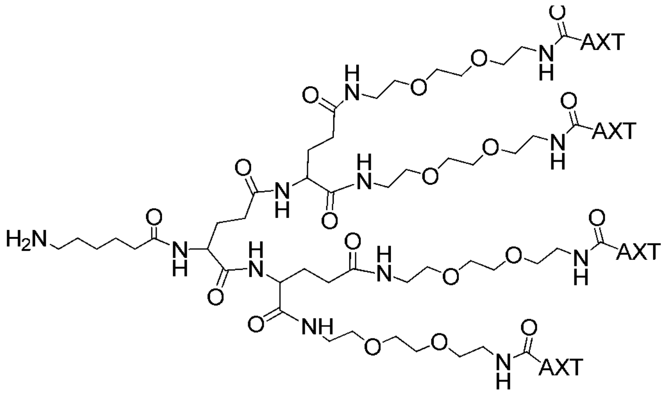

To a flask containing **71-284** (2.12 g, 0.7612 mmol), dichloromethane (5% HCl) was added for dissolution with the assistance of ultrasonication, then TFA (1.2 mL, 15.224 mmol) was added, and the reaction mixture was stirred at room temperature. After the reaction was completed, the reaction mixture was concentrated and evaporated to dryness to remove dichloromethane and most of the TFA. Methyl tert-butyl ether (300 mL) was added for precipitation. A solid product precipitated and was filtered. The filter cake was washed three times with methyl tert-butyl ether (40 mL). The filter cake was collected and dried under vacuum to provide 2.04 g of the product. ESI [M+H$^+$] 2688.391

12.6 Preparation of **71-264**

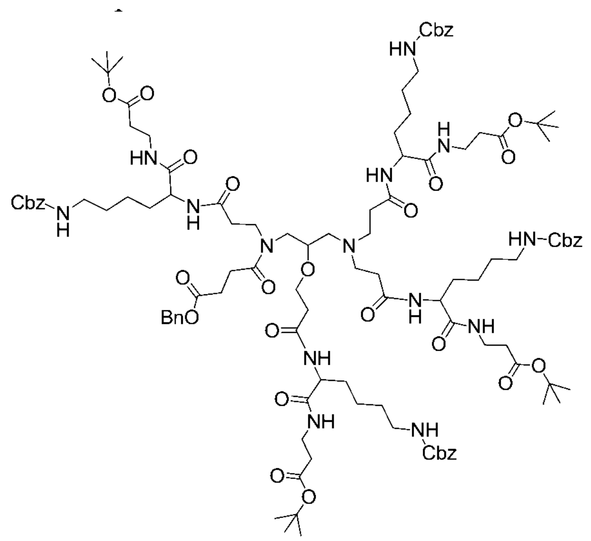

**70-186** (0.27 g, 0.4748 mmol), **88-86** (0.92 g, 2.2794 mmol), HBTU (1.08 g, 2.8492 mmol) and HOBT (0.38 g, 2.8492 mmol) were placed in a 250 mL flask, DMF (20 mL) was added for dissolution, and the contents were stirred at room temperature for approximately 20 minutes. DIEA (1.6 mL, 9.4975 mmol) was then slowly added dropwise, and the reaction was stirred continuously at room temperature. After the reaction was completed, the reaction mixture was transferred to a 1 L separatory funnel, and saturated sodium chloride solution (200 mL) and ethyl acetate (150 mL) were added for extraction. The organic phase was collected, and the aqueous phase was further extracted with ethyl acetate (200 mL × 2). The organic phases were combined and washed with deionized water (250 mL × 2). The obtained organic phase was concentrated, evaporated to dryness, and dried in a vacuum oven to provide 0.68 g of the product with a yield of 68.0%. $^1H$-NMR (600 MHz, DMSO-$d_6$) δ 8.03-7.92 (m, 8H), 7.34-7.28 (m, 25H), 7.24-7.15 (m, 4H), 5.07 (s, 2H), 4.99 (s, 8H), 4.21-4.14 (m,4H), 3.73-3.44 (m, 7H), 3.28-3.26 (m, 2H), 3.17-3.16 (m, 6H), 2.97-2.92 (m, 8H), 2.74-2.55 (m, 10H), 2.35-2.24 (m, 16H), 1.58-1.52 (m,4H), 1.50-1.41 (m, 6H), 1.37 (s, 36H), 1.26-1.13 (m, 14H); ESI [M+H$^+$] 2127.15796

12.7 Preparation of **71-274**

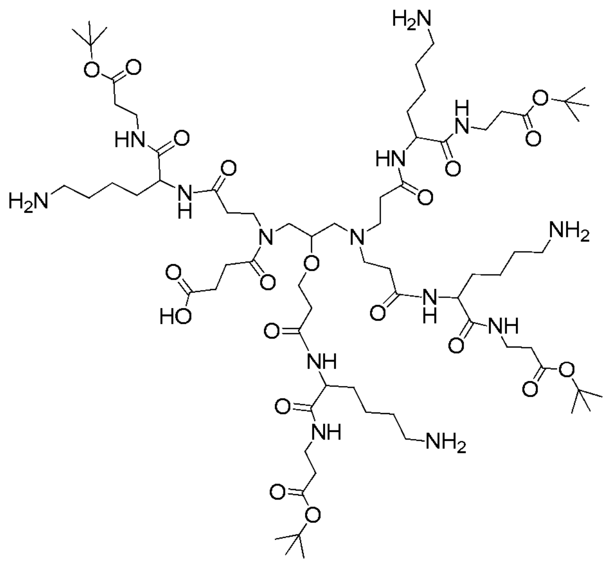

**71-264** (0.48 g, 0.2257 mmol) was placed in a hydrogenation reactor, and then 10% Pd/C catalyst (0.1 g) was added, followed by the addition of methanol (20 mL) for dissolution. Hydrogen was introduced at a pressure of 1.8 MPa, and the reaction was carried out under stirring at room temperature overnight. After the reaction was completed, the reaction mixture was filtered through Celite, and the Celite was then washed with methanol (5 mL × 3). The methanol mixtures were combined, then dichloromethane was added, and the mixture was evaporated to dryness using a vacuum

rotary evaporator. Dichloromethane and toluene (0.2 mL) were then added and treated by ultrasonication, and the mixture was evaporated to dryness. This process was repeated five times. The obtained product was dissolved in dichloromethane with the assistance of ultrasonication, and the mixture was evaporated to dryness. This process was repeated five times. The resulting product was dried in a vacuum oven to provide 0.33 g of the product. $^1$H-NMR (600 MHz, DMSO-$d_6$) δ11.0 (s, 1H), 8.68-7.73 (m, 8H), 5.11 (s, 8H), 4.17-4.14 (m,4H), 4.06-3.85 (m, 10H), 3.77-3.43 (m, 11H), 3.30-3.22 (m, 4H), 3.21-3.14 (m, 4H), 2.80-2.57 (m, 10H), 2.37-2.32 (m, 10H), 2.30-2.12 (m,8H), 1.61-1.52 (m, 4H), 1.52-1.45 (m, 4H), 1.39 (s, 36H), 1.35-1.22 (m, 8H); ESI [M+H$^+$] 1499.96240

12.8 Preparation of **71-278**

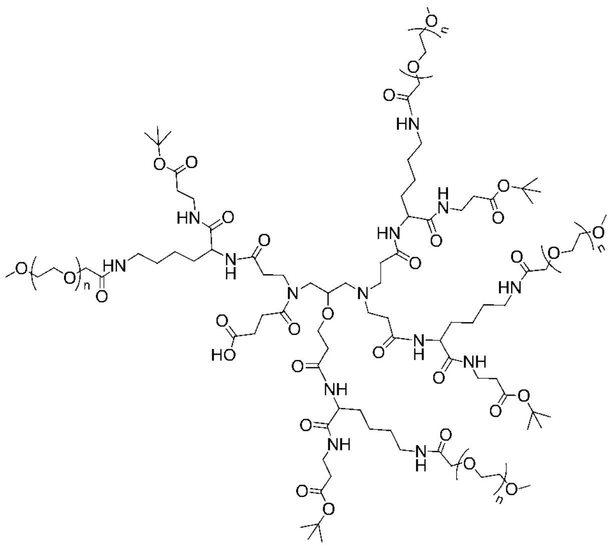

**71-274** (0.05 g, 0.0333 mmol) was placed in a 250 mL flask, dissolved with anhydrous DMF (15 mL), and the contents were stirred at 0°C for 30 minutes. DIEA (0.3 mL, 1.6668 mmol) was then slowly added dropwise. Stirring was continued at low temperature for 10 minutes. M-SCM-10K (1.56 g, 0.1466 mmol, purchased from Jenkem, Lot Number: A3016-N230101) was then added. The reaction was carried out under stirring in the dark at low speed at room temperature for one week. After the reaction was completed, methyl tert-butyl ether (200 mL) and n-hexane (70 mL) were added for precipitation. A solid precipitated and was filtered. The filter cake was washed with methyl tert-butyl ether (40 mL × 3). The filter cake was collected and dried in a vacuum oven to provide 1.06 g of the product. $^1$H-NMR (600 MHz, DMSO-$d_6$) δ9.05-9.04 (m, 1H), 8.11-7.85 (m, 12H), 4.17-4.13 (m, 4H), 3.88-3.82 (m, 15H), 3.51-3.50 (m, 3828H), 2.65-2.59 (m, 24H), 2.40-2.37 (m, 10H), 1.59-1.52 (m, 8H), 1.50-1.43 (m, 8H), 1.39-1.37 (m, 36H), 1.30-1.25 (m, 8H)

12.9 Preparation of **71-295**

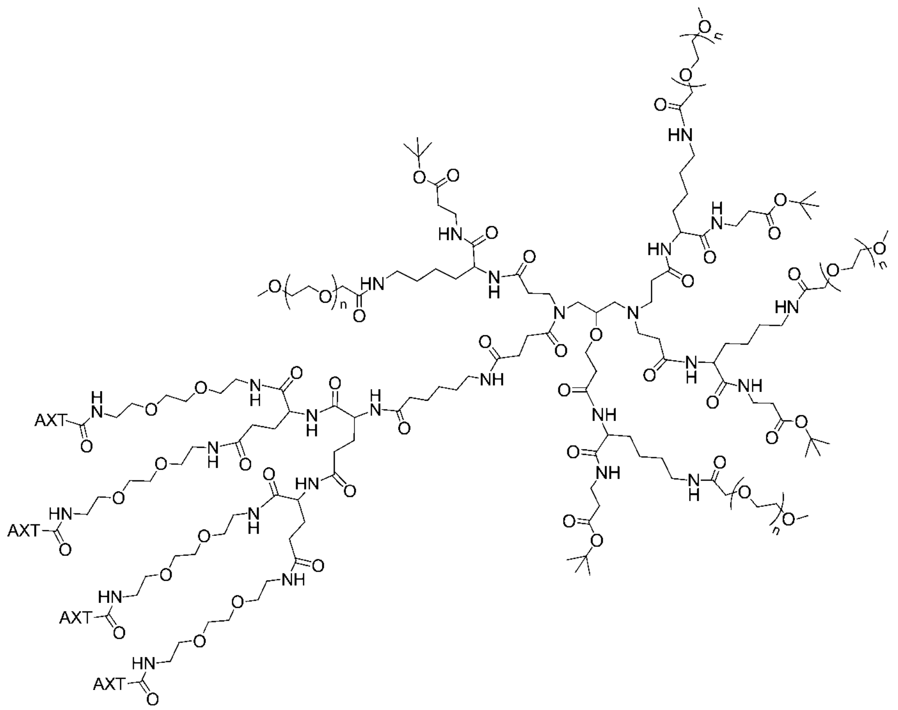

**71-278** (1.0 g, 0.0233 mmol), **71-289** (0.094 g, 0.035 mmol), HBTU (0.013 g, 0.035 mmol) and HOBT (0.0046 g, 0.0047 mmol) were placed in a 250 mL flask, DMF (30 mL) was added for dissolution, and the contents were stirred at room temperature for approximately 20 minutes. DIEA (0.1 mL, 0.607 mmol) was then added slowly dropwise, and the reaction was stirred continuously at room temperature. After the reaction was completed, n-hexane (250 mL) and methyl tert-butyl ether (70 mL) were added for precipitation, the supernatant was discarded, and n-hexane and methyl tert-butyl ether were added again for precipitation. This process was repeated five times. The mixture was filtered to provide a solid product, which was dissolved in a mixture of dichloromethane and methanol. The resulting product was loaded by dry method, and subjected to column chromatography using a 10% methanol/dichloromethane mixture as the eluent. The desired product was collected, concentrated, and dried in a vacuum oven to provide 0.53 g of the product. $^1$H-NMR (600 MHz, DMSO-$d_6$) δ8.65-8.60 (m, 4H), 8.41-8.32 (m, 12H), 8.27-8.24 (m, 4H), 8.10-8.07 (m, 2H), 8.01-7.90 (m, 12H), 7.86-7.76 (m, 12H), 7.71-7.62 (m, 10H), 7.55-7.48 (m, 4H), 7.35-7.29 (m, 16H), 7.13-7.12 (m, 4H), 4.20-4.17 (m,7H), 3.63-3.61 (m,43H), 3.51-3.50 (m, 3828H), 3.18-3.12 (m, 22H), 3.07-3.02 (m, 12H), 2.77-2.73 (m, 24H), 2.36-2.32 (m, 10H), 2.15-2.04 (m, 14H), 1.51-1.43 (m, 18H), 1.39-1.37 (m, 36H), 1.28-1.26 (m, 12H)

12.10 Preparation of**85-10**

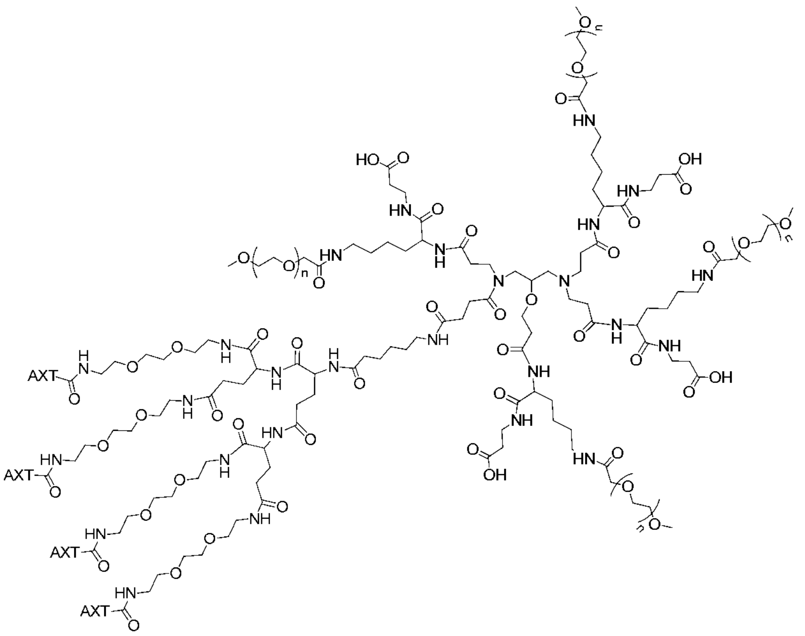

To a flask containing **71-295** (0.53 g, 0.0114 mmol), TFA (0.5 mL, 0.9153 mmol) was added for dissolution with the assistance of ultrasonication, and the reaction was stirred at room temperature overnight. After the reaction was completed, the reaction mixture was concentrated and evaporated to remove most of the TFA, and then precipitated with methyl tert-butyl ether (300 mL) to precipitate a solid product. The solid product was filtered, and the filter cake was washed with methyl tert-butyl ether (40 mL × 3). The filter cake was collected and dried in vacuum to provide 0.5 g of the product. $^{1}$H-NMR (600 MHz, DMSO-$d_6$) δ10.04-9.89 (m, 4H), 8.65-8.57 (m, 4H), 8.45-8.30 (m, 12H), 8.29-8.24 (m, 6H), 8.14-8.09 (m, 18H), 7.86-7.74 (m, 10H), 7.75-7.60 (m, 10H), 7.53-7.47 (m, 4H), 7.35-7.26 (m, 12H), 7.19-7.16 (m, 4H), 4.21-4.15 (m,7H), 3.64-3.62 (m,43H), 3.51-3.50 (m, 3828H), 3.25-3.22 (m, 12H), 3.20-3.10 (m, 12H), 3.09-3.0 (m, 10H), 2.85-2.66 (m, 24H), 2.40-2.33 (m, 10H), 2.15-2.04 (m, 8H), 1.90-1.77 (m, 6H), 1.60-1.53 (m, 4H), 1.51-1.43 (m, 6H), 1.40-1.35 (m, 8H), 1.28-1.14 (m, 12H)

12.11 Preparation of**85-16**

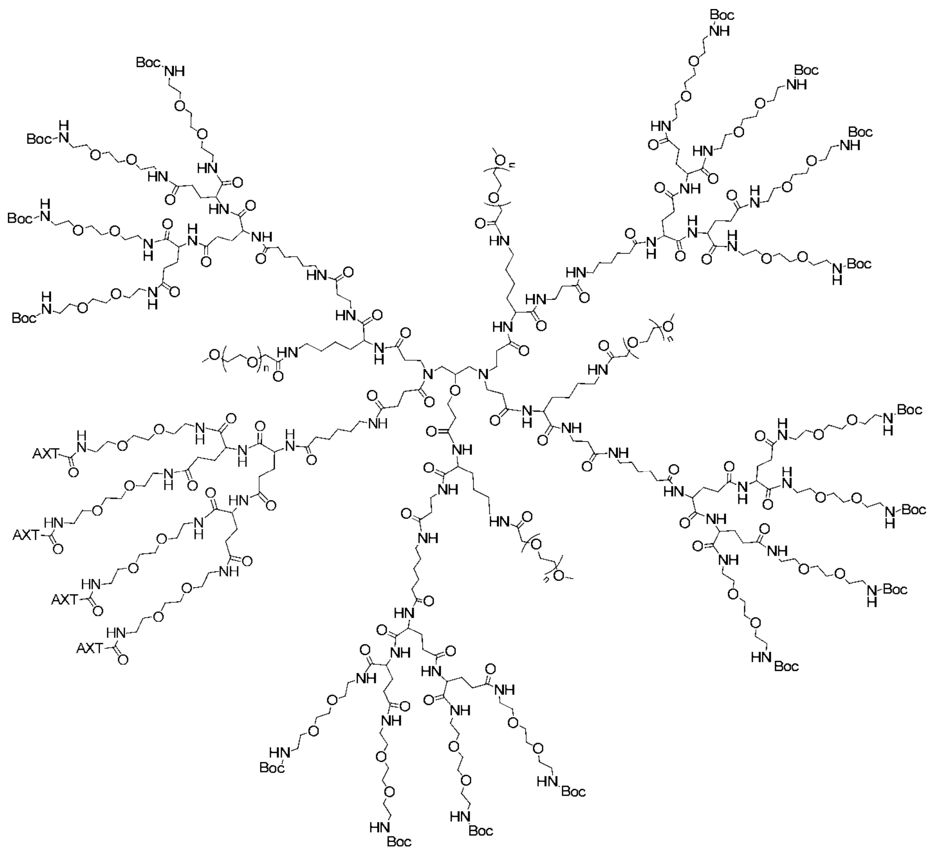

**85-10** (0.5 g, 0.0108 mmol), **76-93** (0.093 g, 0.0651 mmol), HBTU (0.024 g, 0.0651 mmol) and HOBT (0.0087 g, 0.0651 mmol) were added to a round-bottom flask, then DMF (20 mL) was added for dissolution, and the contents were stirred at room temperature for about 20 minutes. DIEA (0.041 mL, 0.2172 mmol) was slowly added dropwise. After the dropwise addition was completed, the reaction was continued under stirring at room temperature. After the reaction was completed, n-hexane (150 mL) and methyl tert-butyl ether (100 mL) were added for precipitation, the supernatant was discarded, and then n-hexane and methyl tert-butyl ether were added again for precipitation. This process was repeated three times. The mixture was filtered by suction to provide a solid product, and the obtained solid product was dissolved in a dichloromethane/ methanol mixture. The resulting product was loaded by dry method, and subjected to column chromatography using a mixture of 1% ammonia water/5-8% methanol/dichloromethane as the eluent. The desired product was collected, concentrated, and dried in a vacuum oven to provide 0.31 g of the product. $^1$H-NMR (600 MHz, DMSO-$d_6$) δ8.65-8.57 (m, 4H), 8.43-8.30 (m, 12H), 8.28-8.18 (m, 16H), 8.13-8.06 (m, 16H), 8.02-7.93 (m, 12H), 7.90-7.78 (m, 18H), 7.75-7.63 (m, 10H), 7.53-7.45 (m, 8H), 7.42-7.38 (m, 4H), 7.36-7.23 (m, 12H), 7.14-7.13 (m, 4H), 7.02-6.95 (m,12H), 4.26-4.23 (m,20H), 4.11-4.07 (m, 40H), 3.98-3.94 (m, 18H), 3.70-3.59 (m, 112H), 3.51-3.50 (m, 3828H), 3.17-3.15 (m, 70H), 2.75-2.73 (m, 12H), 2.70-2.66 (m, 32H), 2.13-2.10 (m, 80H), 1.48-1.43 (m, 16H), 1.47-1.46 (m, 26H), 1.37-1.36 (m, 144H), 1.26-1.25 (m, 28H)

12.12 Preparation of **85-23**

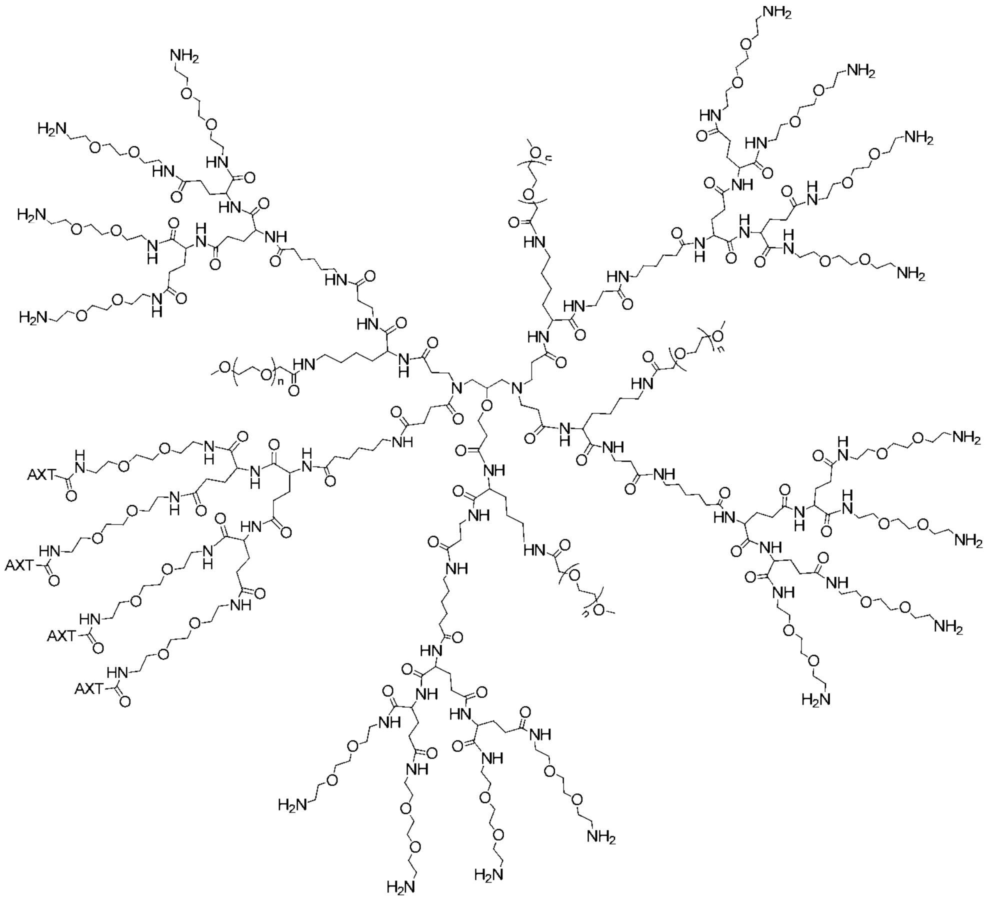

To a flask containing **85-23** (0.31 g, 0.0059 mmol), TFA (0.1 mL, 0.9577 mmol) was added for dissolution with the assistance of ultrasonication, and the reaction mixture was stirred at room temperature overnight. After the reaction was completed, the reaction mixture was concentrated to remove most of the TFA. Methyl tert-butyl ether (200 mL) and n-hexane (100 mL) were then added for precipitation, the supernatant was discarded, and then methyl tert-butyl ether (200 mL) and n-hexane (100 mL) were added again for precipitation. A solid product precipitated and was filtered. The filter cake was washed six times with methyl tert-butyl ether (40 mL). The filter cake was collected and dried under vacuum to provide 0.27 g of the product.

12.13 Preparation of **85-26**

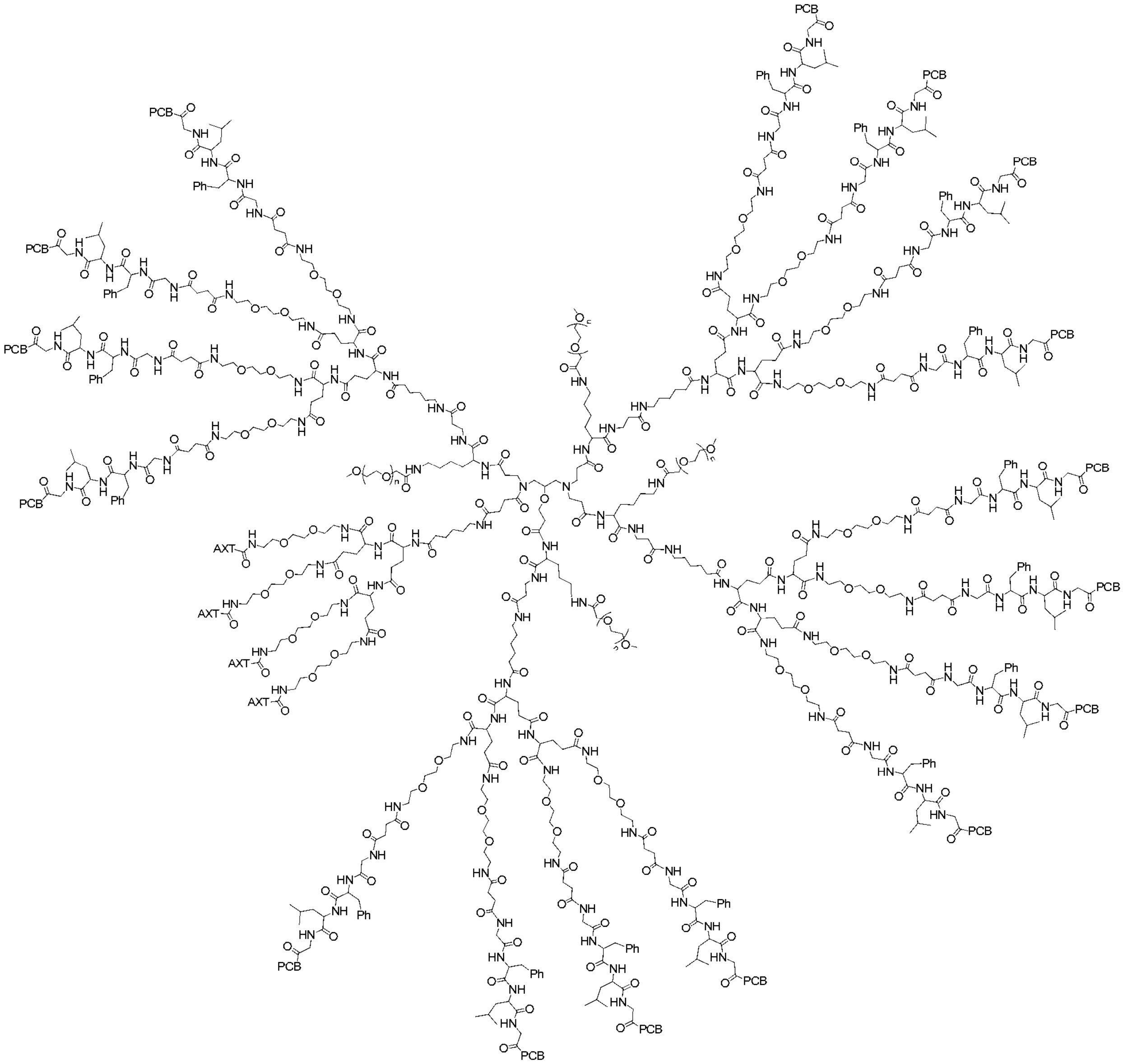

**85-23** (0.27 g, 0.0053 mmol), **81-189** (0.12 g, 0.1291 mmol), HBTU (0.048 g, 0.1291 mmol) and HOBT (0.017 g, 0.1291 mmol) were added to a 250 mL round-bottom flask, DMF (15 mL) was added for dissolution, and the contents were stirred at room temperature for approximately 20 minutes. DIEA (0.07 mL, 0.4304 mmol) was then slowly added dropwise. After the dropwise addition was completed, the reaction flask was allowed to stand at room temperature, and the mixture was continuously stirred. After the reaction was completed, n-hexane (150 mL) and methyl tert-butyl ether (100 mL) were added for precipitation, the supernatant was discarded, and n-hexane and methyl tert-butyl ether were added again for precipitation. This process was repeated three times. The mixture was filtered to provide a solid product, and the obtained solid product was dissolved in a mixed solvent of dichloromethane and methanol. The resulting product was loaded by dry method, and subjected to column chromatography using a mixture of 1% ammonia water/5-10% methanol/dichloromethane as the eluent. The target product was collected, concentrated, and dried in a vacuum oven to provide 0.2 g of the product. $^1$H-NMR (600 MHz, DMSO-$d_6$) δ8.39-8.35 (m, 16H), 8.18-8.15 (m, 18H), 8.11-8.05 (m, 80H), 7.93-7.86 (m, 96H), 7.27-7.20 (m,130H), 4.56-4.48 (m,15H), 4.37-4.32 (m, 36H), 4.26-4.16 (m, 60H), 4.04-3.98 (m, 58H), 3.96-3.92 (m, 36H), 3.66-3.60 (m, 156H), 3.51-3.50 (m, 3828H), 3.25-3.22 (m, 50H), 3.15-2.12 (m, 66H), 3.07-3.02 (m, 50H), 2.81-2.76 (m, 50H), 2.75-2.72 (m, 26H), 2.28-2.20 (m, 76H), 2.19-2.05 (m, 132H), 1.79-1.76 (m, 65H), 1.61-1.56 (m, 68H), 1.54-1.48 (m, 96H), 1.25-1.20 (m, 34H), 0.92-0.83 (m, 96H)

Example 13: Synthesis of Compound **75-209**

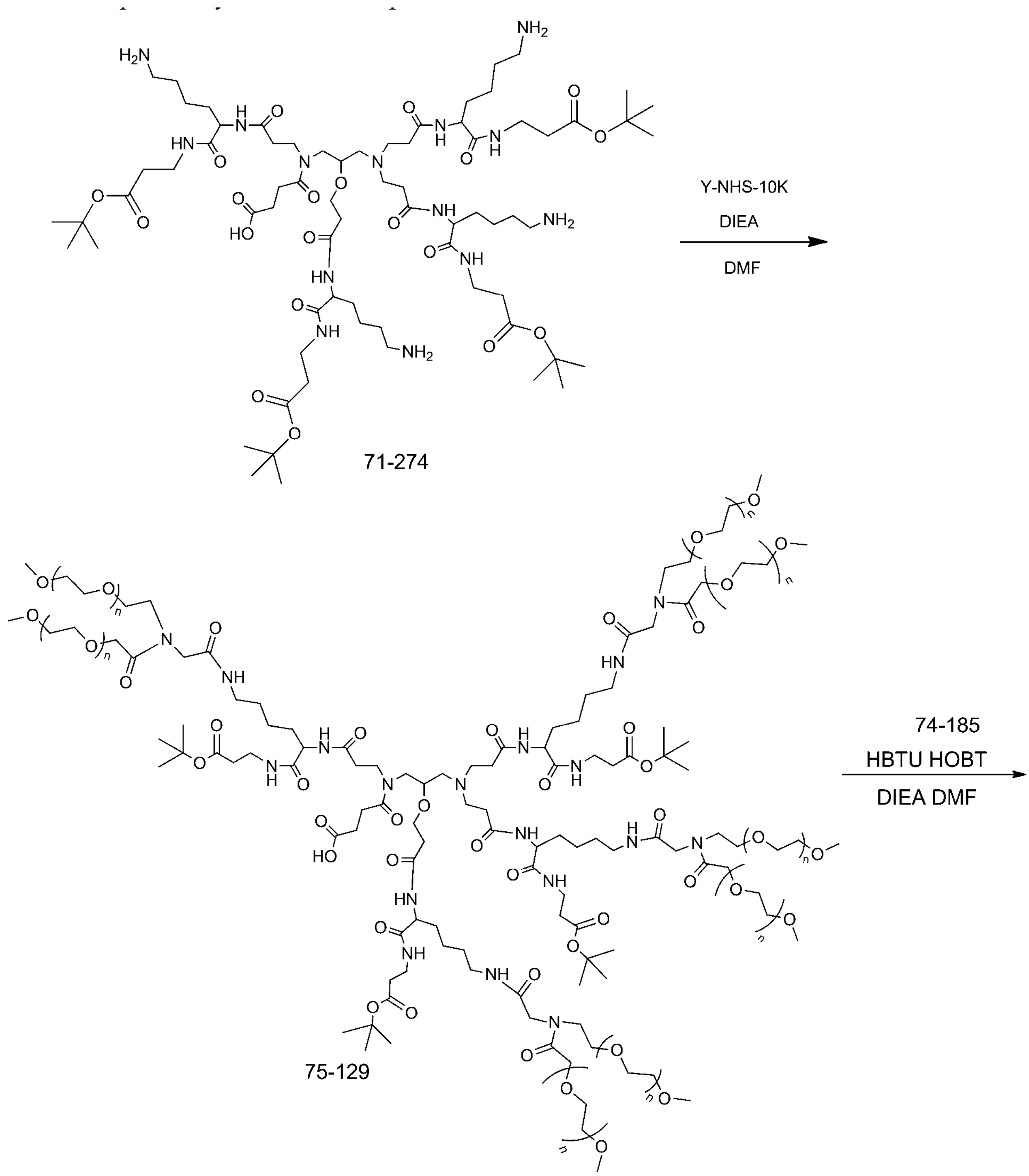


71-274

75-129

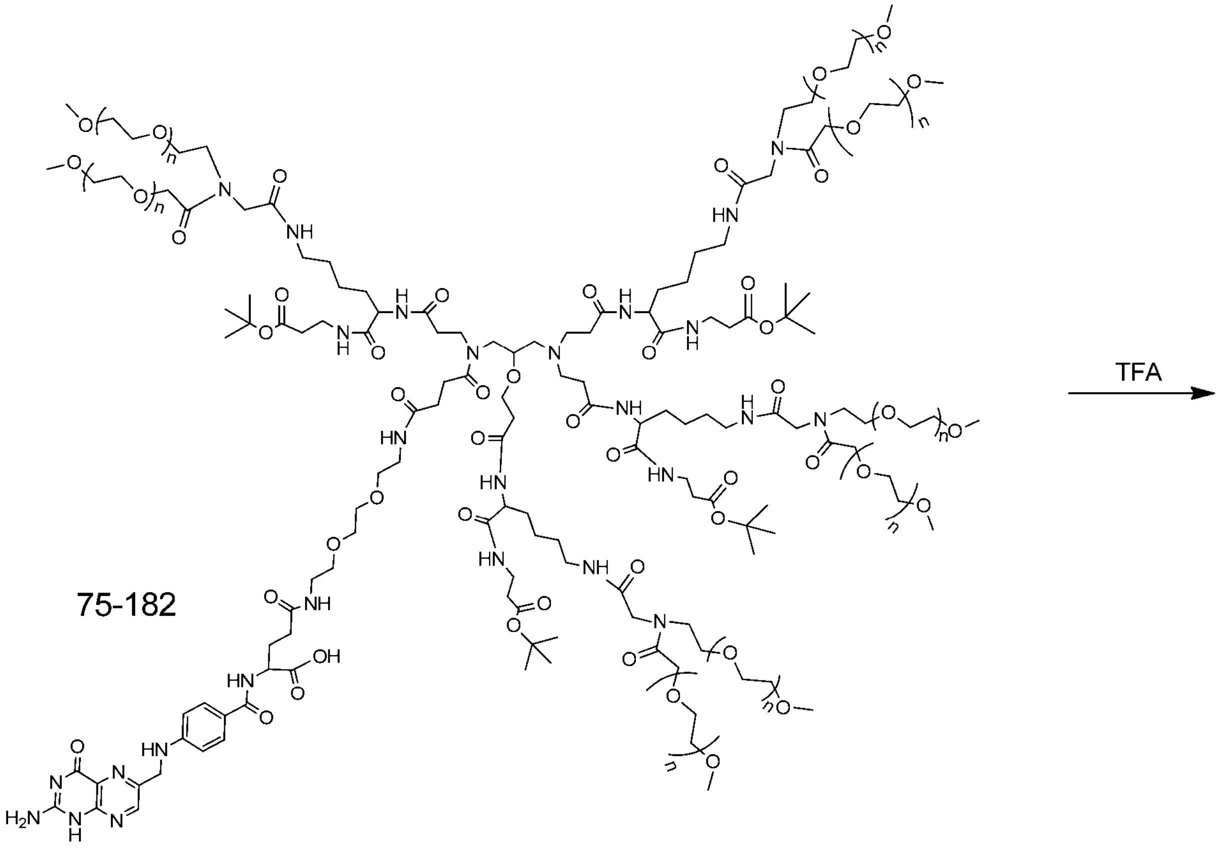


75-182

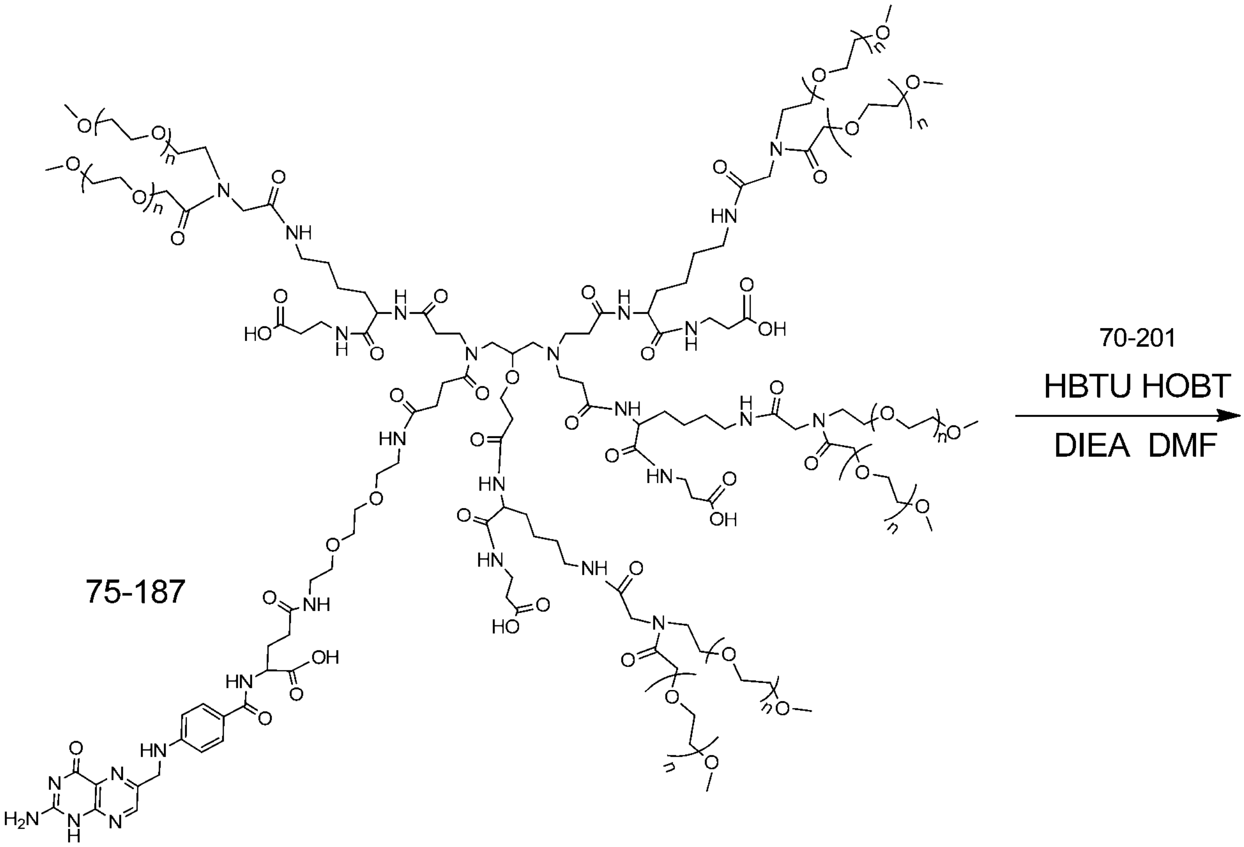


75-187

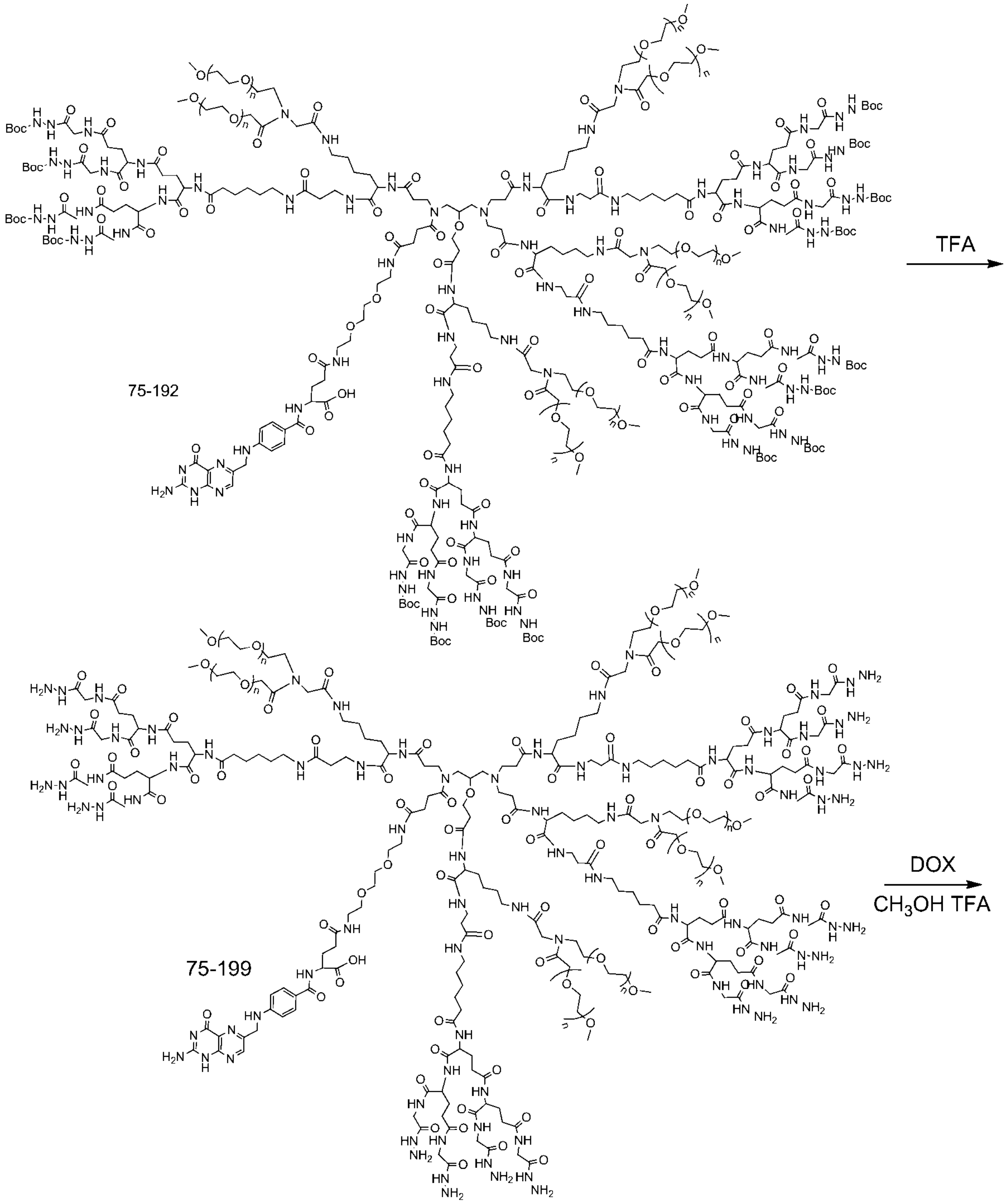


75-192

75-199

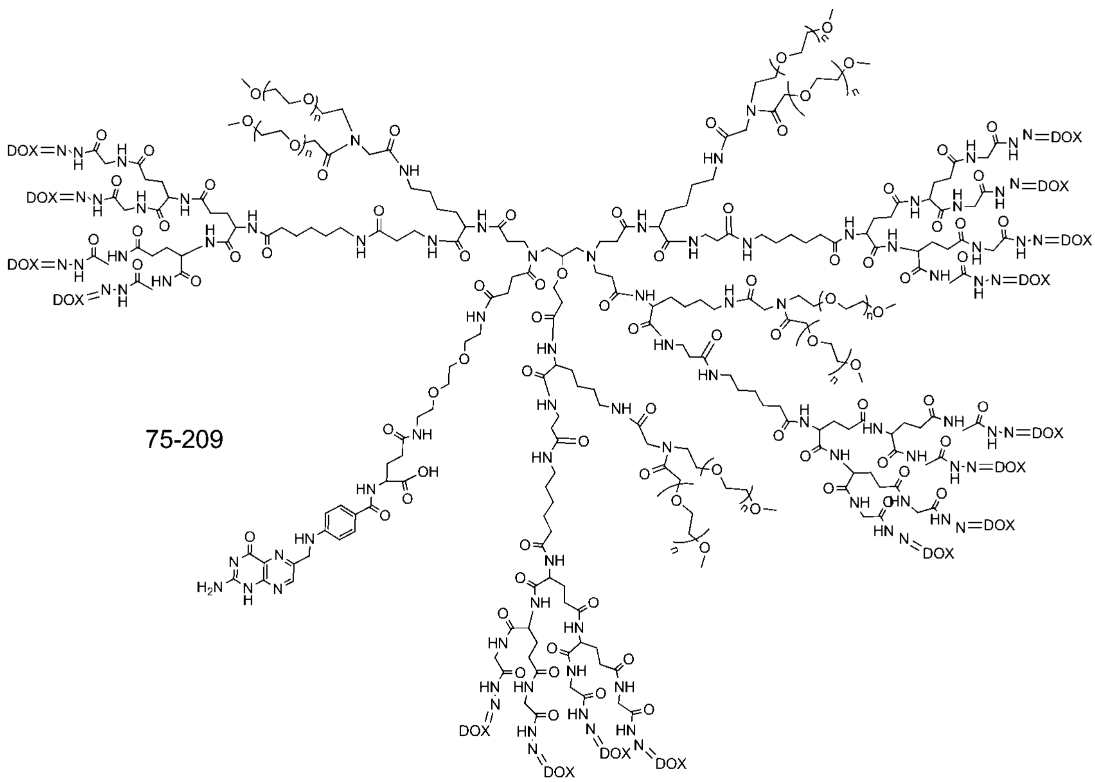

75-209

13.1 Preparation of **75-129**

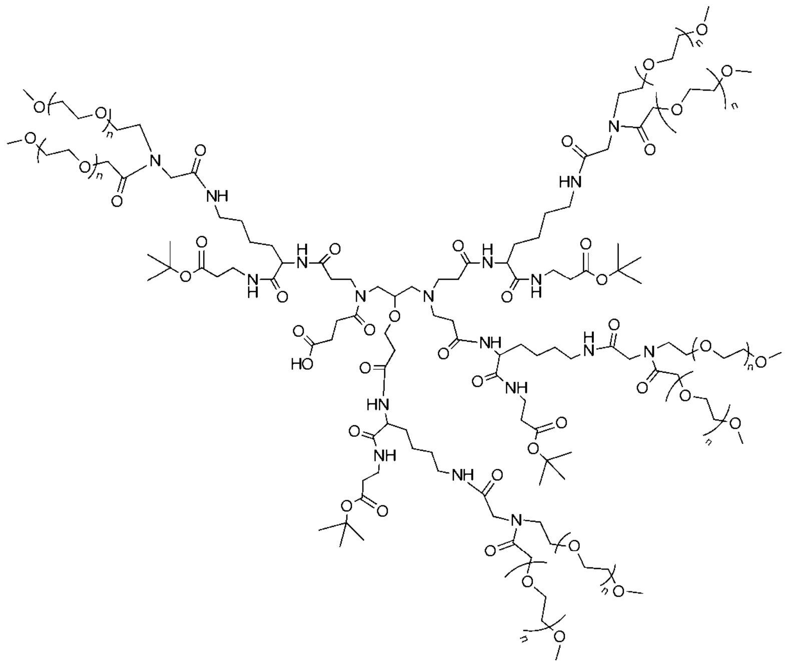

**71-274** (0.05 g, 0.0333 mmol) was added to a 250 mL round-bottom flask, then ultra-dry DMF (10 mL) was added for dissolution with the assistance of ultrasonication. The mixture was stirred at -5°C for 10 minutes. DIEA (0.44 mL, 2.666 mmol) was then slowly added dropwise, and the contents were stirred continuously for 10 minutes. Y-NHS-10K (1.49 g, 0.1466 mmol) was then added. The reaction was carried out under stirring in the dark at low speed at room temperature. After the reaction was completed, methyl tert-butyl ether (450 mL) was added for precipitation. A solid precipitated and was filtered. The filter cake was redissolved in DMF, and then methyl tert-butyl ether (450 mL) was added for precipitation. This

process was repeated three times. The filter cake was dried to provide 1.49 g of the product. $^1$H-NMR (600 MHz, DMSO-$d_6$) δ 10.31-10.29 (m, 1H), 8.14-8.08 (m, 12H), 4.56-4.54 (m, 4H), 3.59-3.55 (m, 13H), 3.53-3.50 (m, 3840H), 2.72-2.64 (m, 14H), 2.49-2.28 (m, 12H), 2.40-2.38 (m, 2H), 2.14-2.11 (m, 8H), 1.81-1.77 (m, 8H), 1.53-1.49 (m, 8H), 1.39-1.37 (m, 36H), 1.25-1.23 (m, 8H)

13.2 Preparation of **75-182**

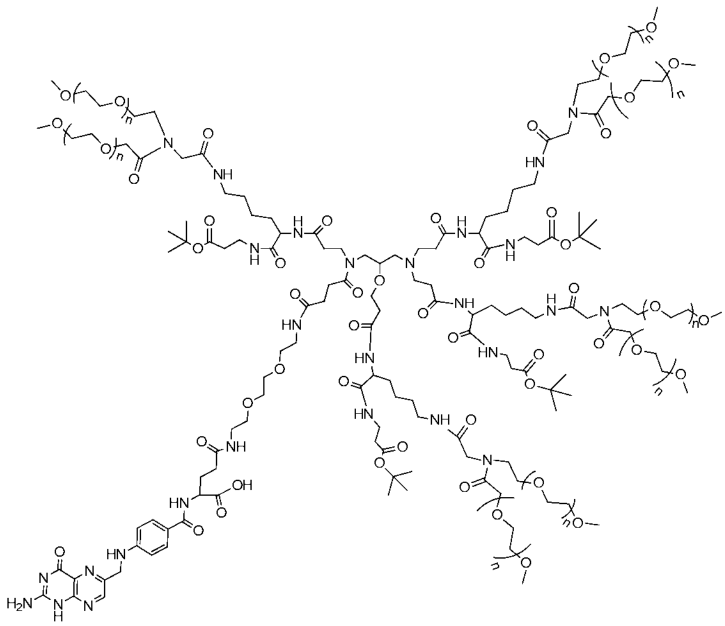

**75-129** (0.75 g, 0.0178 mmol), **74-185** (0.01 g, 0.0178 mmol), HBTU (0.01 g, 0.0267 mmol) and HOBT (0.003 g, 0.0267 mmol) were mixed in a flask, DMF (5 mL) was added for dissolution with the assistance of ultrasonication, and reacted at -5°C in a constant temperature reaction bath for 30 minutes. DIEA (0.1 mL, 0.08 mmol) was then added dropwise, and stirred at room temperature for 2 hours. The reaction progress was monitored by TLC. After the reaction was completed, methyl tert-butyl ether (300 mL) and n-hexane (100 mL) were added for precipitation, and the supernatant was discarded. This process was repeated three times. Finally, the mixture was filtered to provide a solid, which was dissolved in a methanol/dichloromethane mixed solvent, then silica gel powder was added, and the mixture was evaporated to dryness and placed in an oven. The resulting product was loaded by dry method, and subjected to column chromatography using dichloromethane and 2-4% methanol/dichloromethane as the eluents in sequence. The desired product was collected and dried to provide 0.39 g of the product. $^1$H-NMR (600 MHz, DMSO-$d_6$) δ 10.31-10.29 (m, 1H), 9.05-9.04 (m, 1H), 8.56-8.45 (m, 3H), 8.03-7.98 (m, 14H), 7.56 (m, 2H), 6.68 (m, 2H), 4.28-4.21 (m, 5H), 4.08-4.06 (m, 2H), 3.88-3.82 (m, 4H), 3.65-3.60 (m, 21H), 3.53-3.50 (m, 3840H), 3.29-3.07 (m, 4H), 2.76-2.72 (m, 2H), 2.63-2.61 (m, 16H), 2.56-2.52 (m, 16H), 2.10-2.05 (m, 4H), 1.79-1.76 (m, 8H), 1.60-1.53 (m, 8H), 1.39-1.37 (m, 36H), 1.25-1.22 (m, 8H)

13.3 Preparation of **75-187**

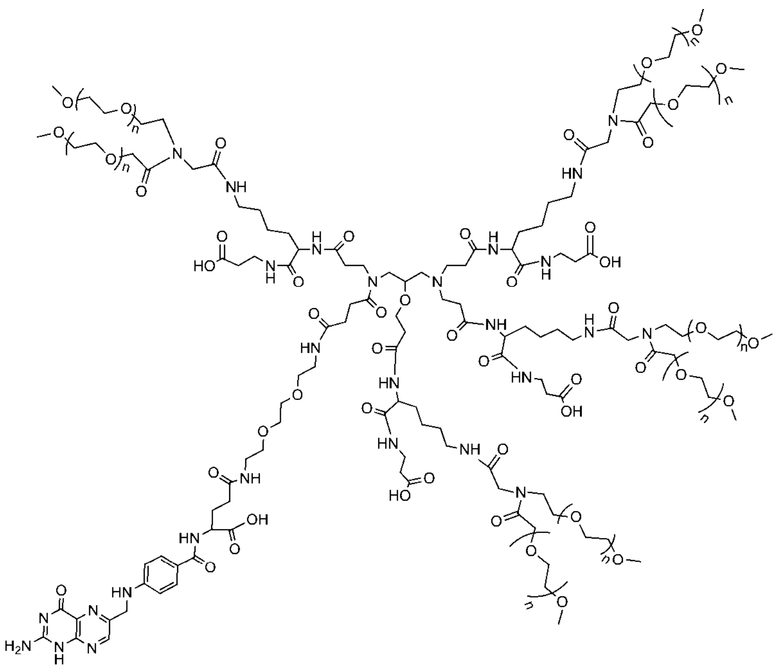

To a 250 mL flask containing raw material **75-182** (0.39g, 0.00912mmol) was added TFA (0.2ml, 0.7299 mmol), and the reaction was stirred for 2 hours. The reaction was monitored by TLC. After the reaction was completed, the mixture was concentrated under reduced pressure, methyl tert-butyl ether and n-hexane were added for precipitation, and the supernatant was discarded. This process was repeated three times. The mixture was filtered and dried to provide 0.34g of the product. $^1$H-NMR (600 MHz, DMSO-$d_6$) δ 10.57-10.29 (m,5H), 9.05-9.04 (m, 1H), 8.56-8.45 (m, 3H), 8.15-8.03 (m, 14H), 7.57-7.55 (m, 2H), 6.68-6.64 (m, 2H), 4.55-4.53 (m, 5H), 4.39-4.37 (m, 2H), 3.88-3.82 (m, 4H), 3.65-3.54 (m, 21H), 3.53-3.50 (m, 3840H), 3.14-3.07 (m, 4H), 2.76-2.72 (m, 2H), 2.63-2.61 (m, 16H), 2.56-2.52 (m, 16H), 2.10-2.05 (m, 4H), 1.79-1.76 (m, 8H), 1.64-1.56 (m, 8H), 1.27-1.22 (m, 8H)

13.4 Preparation of **75-192**

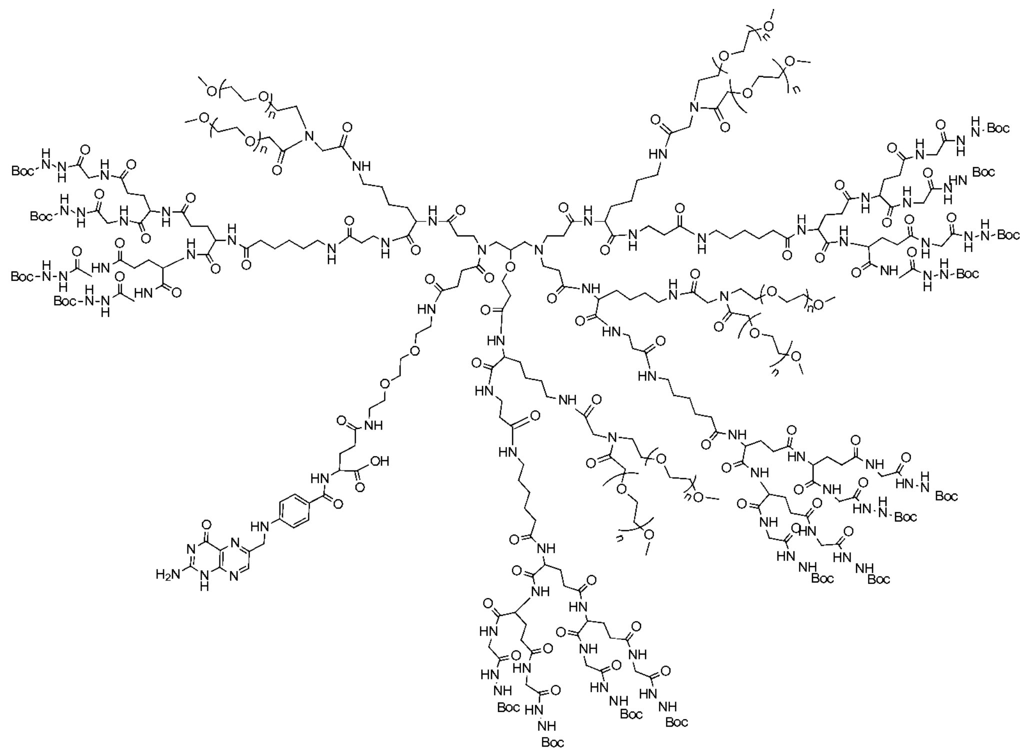

**75-187** (0.26 g, 0.0071 mmol), **70-201** (0.03 g, 0.0284 mmol), HBTU (0.016 g, 0.0423 mmol), and HOBT (0.005 g, 0.0423 mmol) were mixed in a 250 mL flask, DMF (5 mL) was added for dissolution with the assistance of ultrasonication, and the contents were stirred at -5°C in a constant temperature reaction bath for 30 min. DIEA (0.2 mL, 0.1269 mmol) was then added dropwise, and the reaction mixture was stirred at room temperature for 2 hours. The reaction progress was monitored by TLC. After the reaction was completed, methyl tert-butyl ether (100 mL) and n-hexane (50 mL) were added for precipitation, the supernatant was discarded, and then methyl tert-butyl ether (100 mL) and n-hexane (50 mL) were added again for precipitation. This process was repeated three times. The obtained solid was dissolved in a mixed solvent, then 0.7 g of silica gel powder was added. The mixture was evaporated to dryness and placed in an oven. The resulting product was loaded by dry method, and subjected to column chromatography using 2-6% methanol/dichloromethane as the eluent. The desired product was collected and dried to provide 0.25 g of the product. $^1$H-NMR (600 MHz, DMSO-$d_6$) δ10.30-10.29 (m, 1H), 9.09-9.08 (m, 1H), 8.83-8.73 (m, 16H), 8.35-7.65 (m, 64H), 7.55-7.54 (m, 2H), 7.29-7.11 (m, 4H), 6.55-6.53 (m, 2H), 4.29-4.15 (m, 21H), 3.83-3.66 (m, 50H), 3.53-3.50 (m, 3840H), 3.10-3.08 (m, 4H), 2.84-2.60 (m, 26H), 2.41-2.37 (m, 8H), 2.25-2.05 (m, 5H), 1.52-1.47 (m, 16H), 1.40-1.37 (m, 144H), 1.27-1.22 (m, 24H)

13.5 Preparation of **75-199**

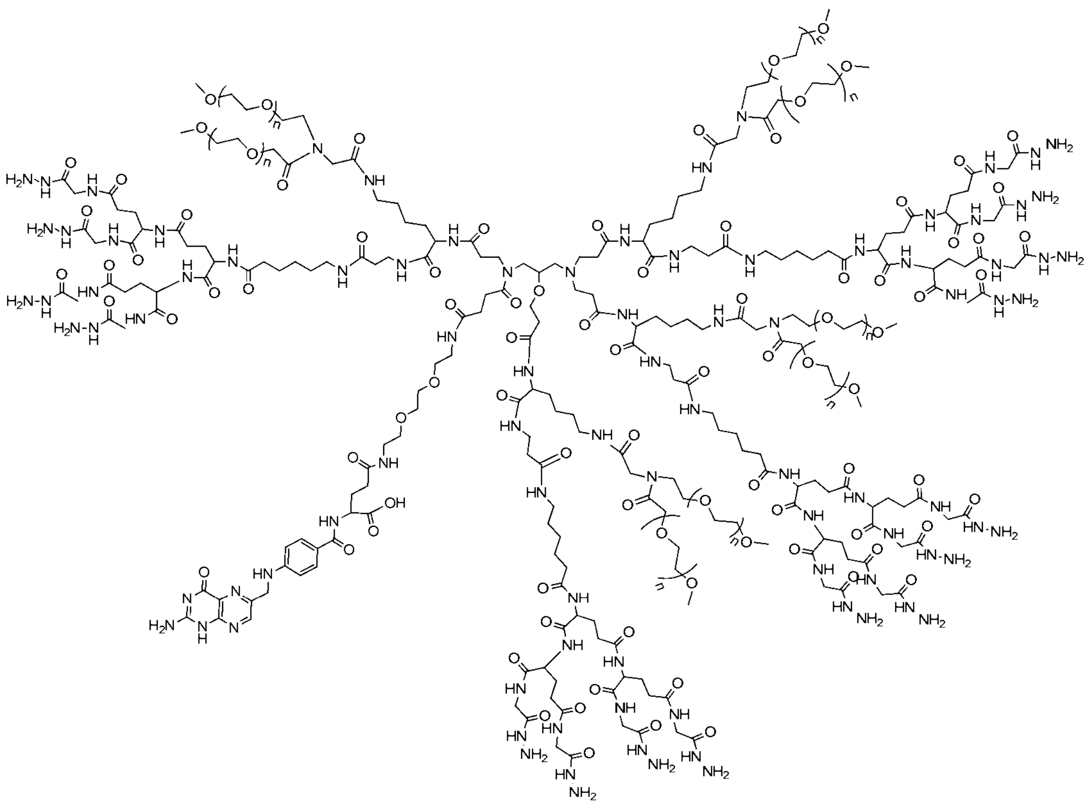

To a 250 mL flask containing **75-192** (0.25 g, 0.00406 mmol), TFA (0.1 mL, 1.301 mmol) was added, and the reaction was stirred under stirring for 2 hours. The reaction progress was monitored by TLC. After the reaction was completed, methyl tert-butyl ether (80 mL) and n-hexane (30 mL) were added for precipitation, the supernatant was discarded, and methyl tert-butyl ether (100 mL) and n-hexane (50 mL) were added again for precipitation. This process was repeated three times. The mixture was filtered to provide 0.24 g of the product. $^1$H-NMR (600 MHz, DMSO-$d_6$) δ10.30-10.29 (m, 1H), 9.09-9.08 (m, 1H), 8.56-8.50 (m, 16H), 8.31-7.76 (m, 46H), 7.52-7.51 (m, 2H), 7.21-7.14 (m, 4H), 6.50-6.49 (m, 2H), 4.28-4.20 (m, 21H), 3.87-3.63(m, 50H), 3.53-3.50 (m, 3840H), 3.12-3.08 (m, 4H), 2.82-2.63 (m, 26H), 2.42-2.36 (m, 8H), 2.21-2.01 (m, 92H), 1.55-1.48 (m, 16H), 1.25-1.22 (m, 24H)

13.6 Preparation of **75-209**

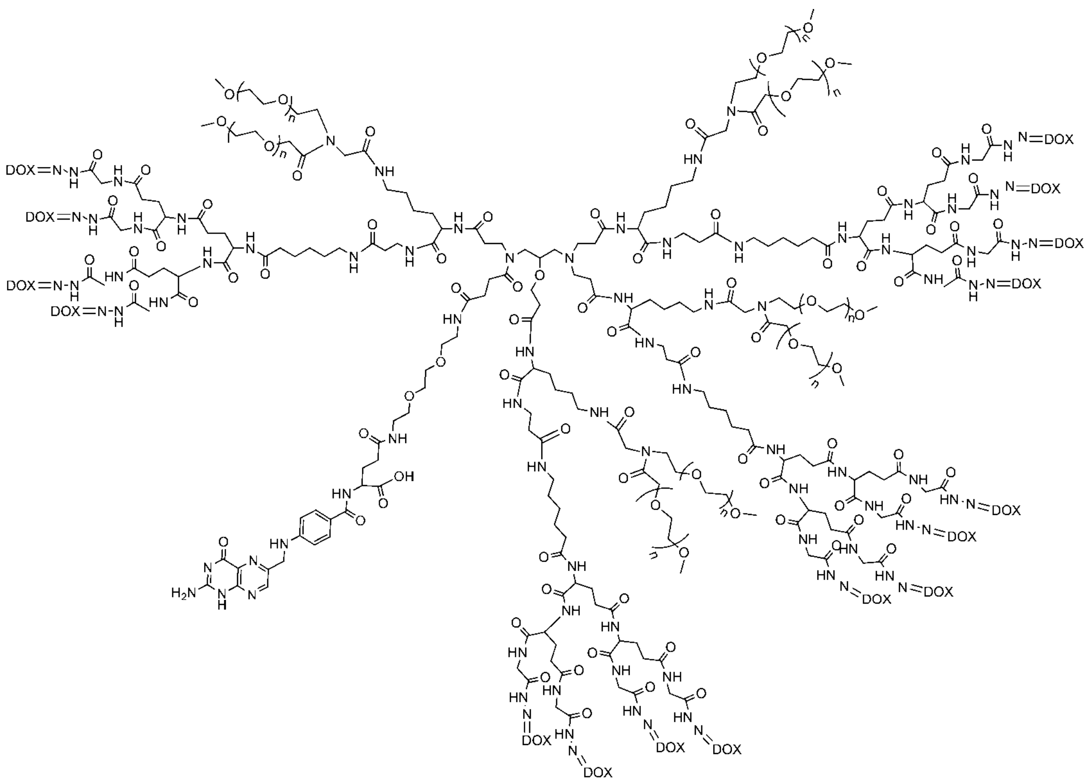

**75-199** (0.24 g, 0.00400 mmol), DOX-HCl (0.0409 g, 0.070 mmol) and methanol (3 mL) were mixed, and the contents were dissolved with the assistance of ultrasonication. TFA (0.05 mL) was then added and allowed to react for 48 hours. After the reaction was completed, the methanol and TFA were removed by concentration and evaporation. Methyl tert-butyl ether and n-hexane were added for precipitation, the supernatant was discarded, and methyl tert-butyl ether (100 mL) and n-hexane (50 mL) were added again for precipitation. This process was repeated three times. The resulting product was dissolved in ethyl acetate (10 mL), and precipitated in petroleum ether (30 mL). This process was repeated three times. The mixture was filtered to obtain a solid, which was dried to provide 0.22 g of the product. $^1$H-NMR (600 MHz, DMSO-$d_6$) δ10.30-10.29 (m, 1H), 9.04-9.03 (m, 1H), 8.42-8.27 (m, 16H), 8.11-7.75 (m, 96H), 7.55-7.54 (m, 2H), 7.19-7.08 (m, 22H), 5.49-5.27 (m, 64H), 4.63-4.40 (m, 16H), 4.30-4.19 (m, 39H), 4.01-3.98 (m, 16H), 3.85-3.73 (m, 50H), 3.63-3.56 (m, 65H), 3.53-3.50 (m, 3840H), 3.44-3.41 (m, 64H), 3.25-3.23 (m, 36H), 2.74-2.72 (m, 8H), 2.63-2.60 (m, 26H), 2.25-2.10 (m, 124H), 1.52-1.49 (m, 23H), 1.35-1.32 (m, 48H), 1.20-1.14 (m, 48H)

Example 14: Synthesis of Compound **77-162**

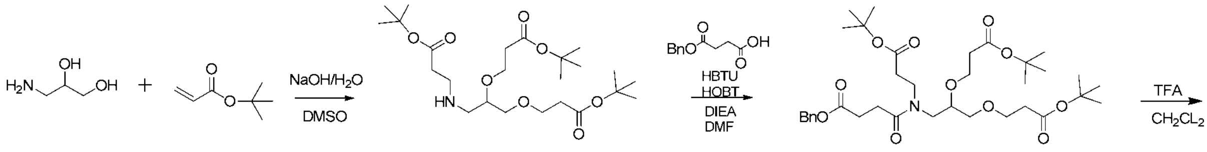


82-49

77-82

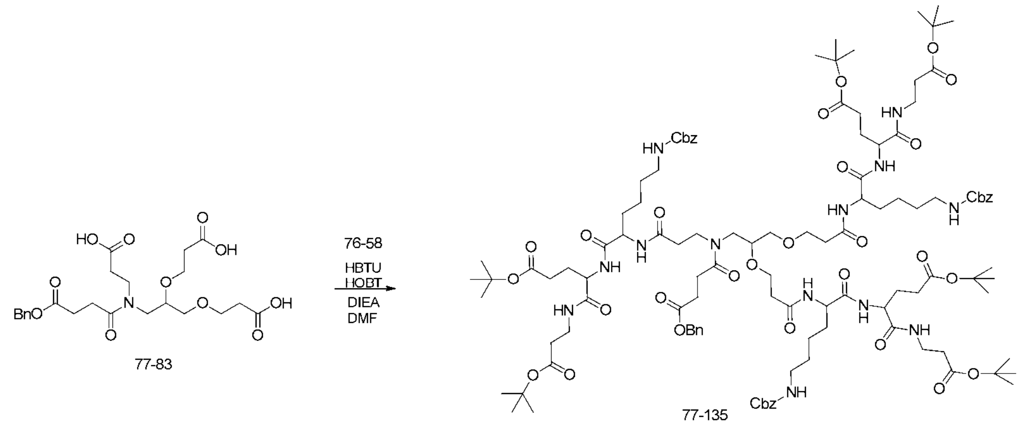


77-83

77-135

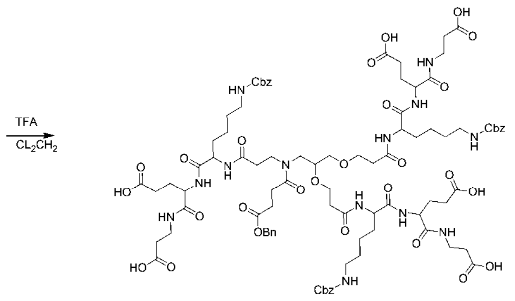


77-144

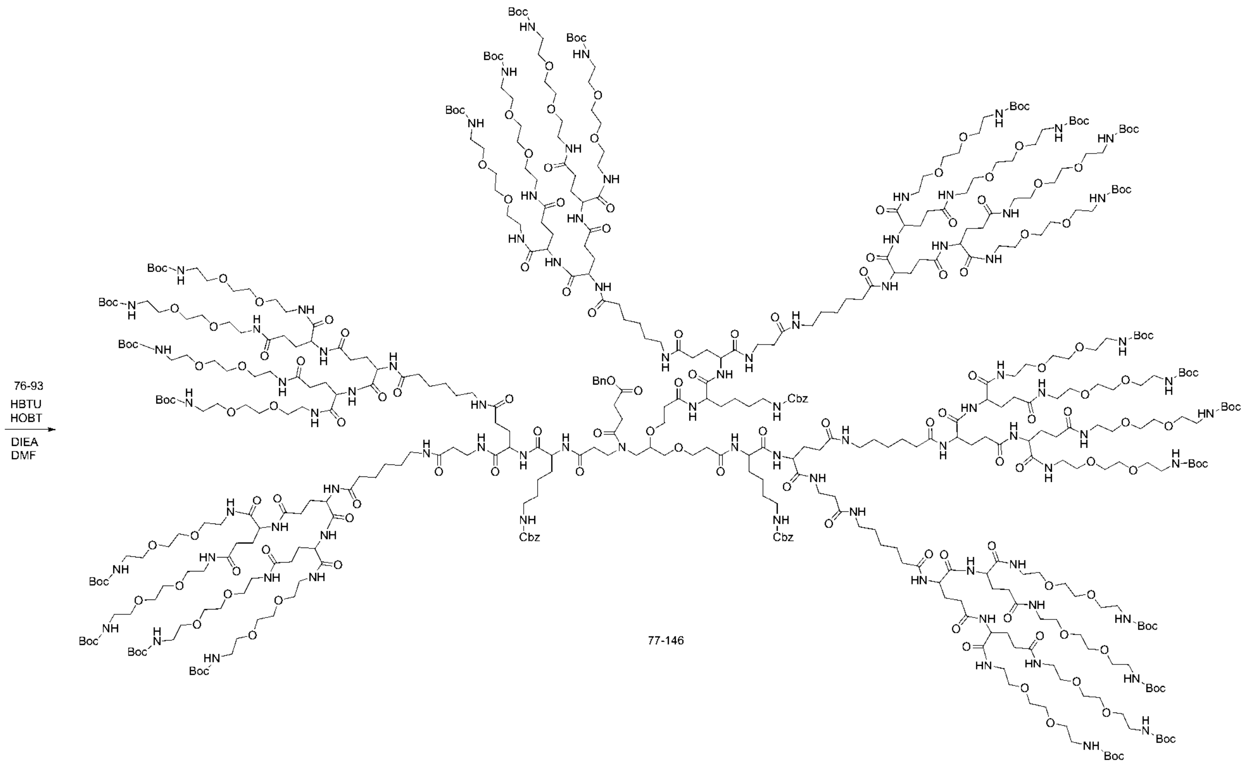


77-146

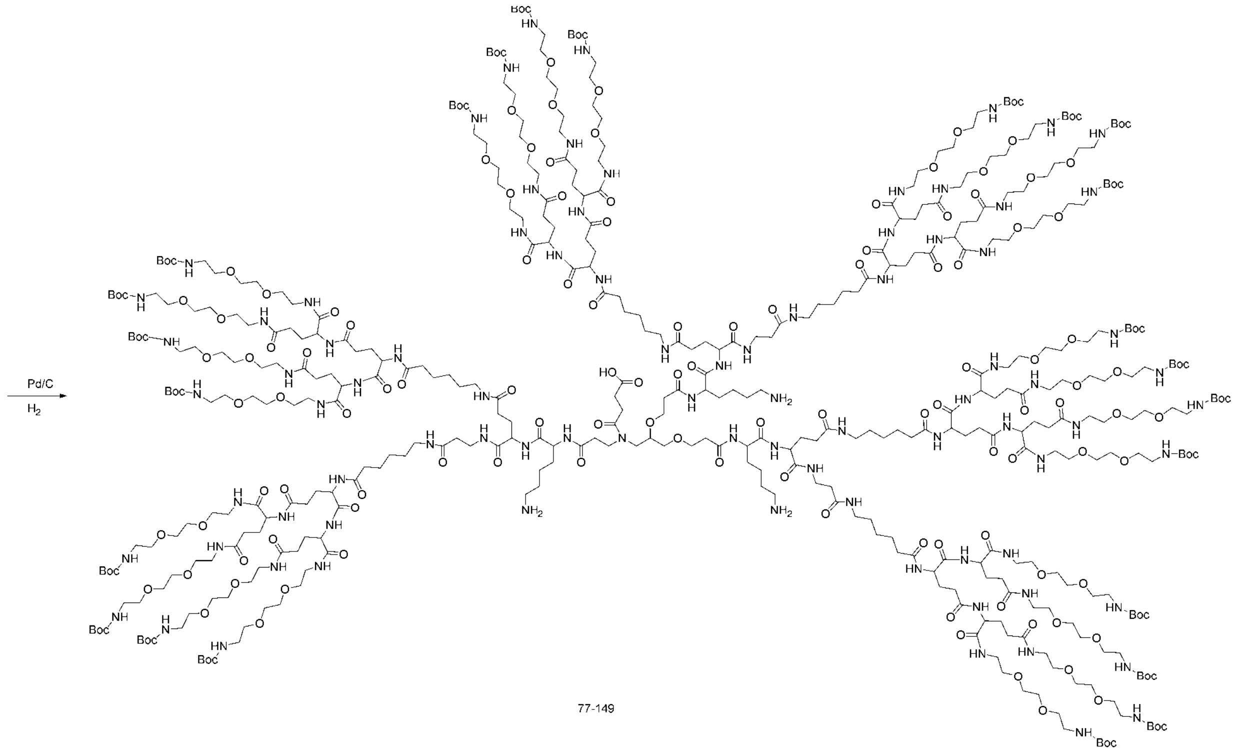


77-149

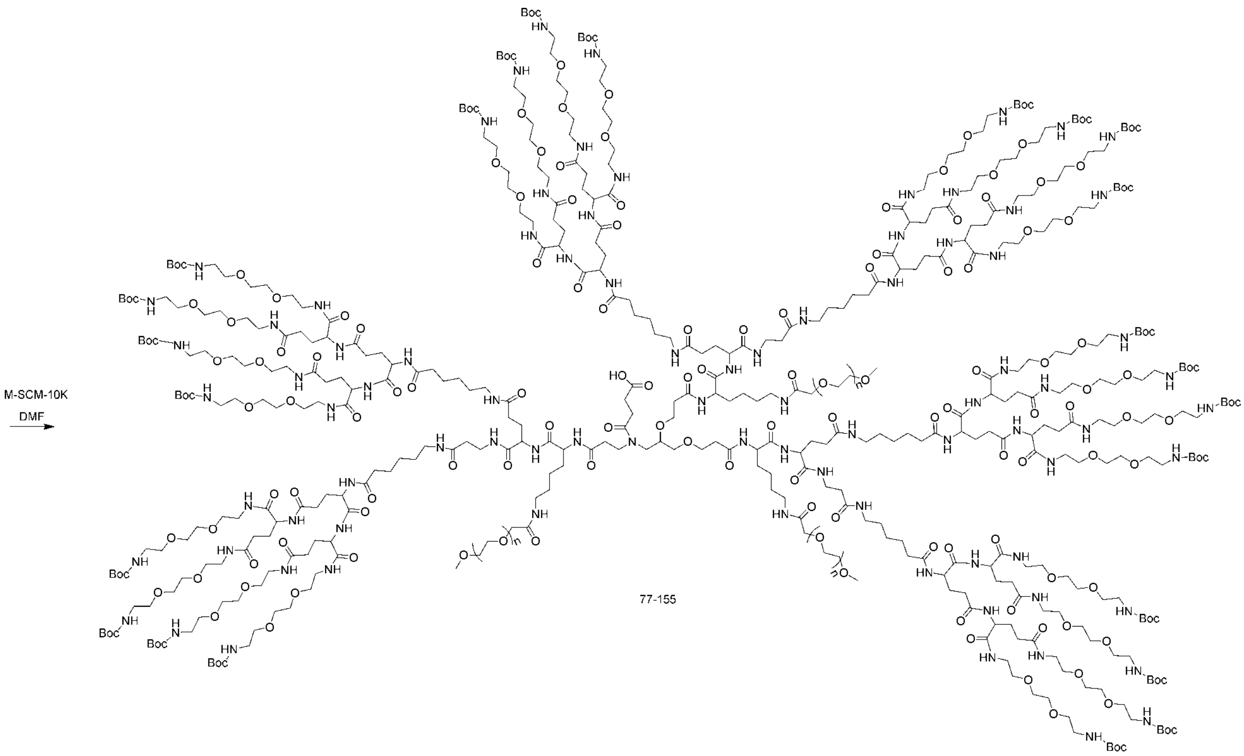


77-155

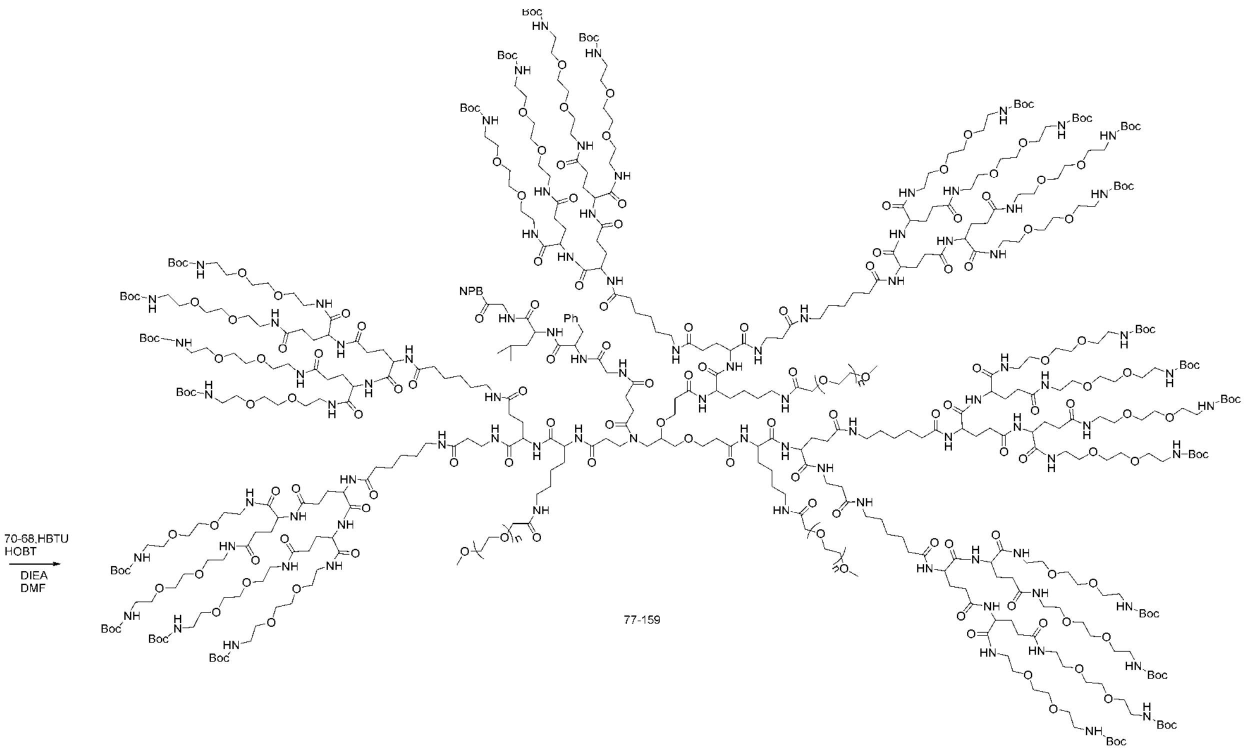


77-159

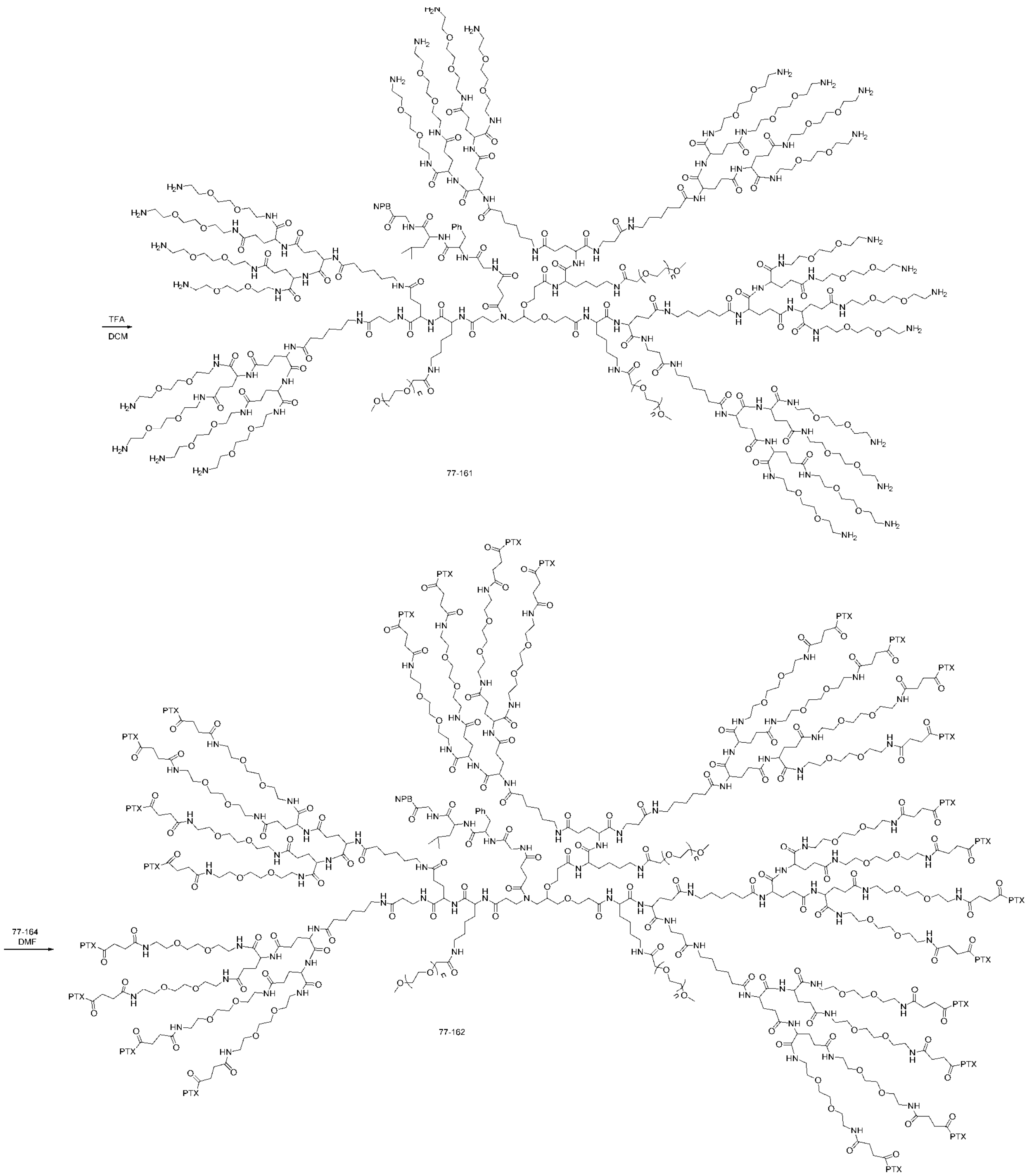


77-161

77-162

14.1 Preparation of **82-49**

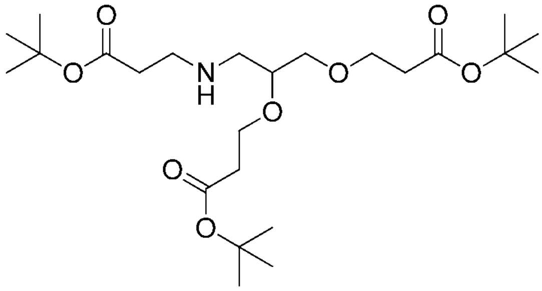

3-Amino-1,2-propanediol (5.0 g, 54.879 mmol) was weighed and placed in a 500 mL flask, and DMSO (20 mL) was added to completely dissolve the mixture. Under nitrogen protection at 15°C, 5.0 mol/L NaOH solution (2.0 mL) was added, and the contents were stirred for 5 minutes. Tert-butyl acrylate (23.9 mL, 164.637 mmol) was then slowly added dropwise. After the dropwise addition was completed, the reaction mixture was taken out and stirred at room temperature for 24 hours. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid. The solid was then dissolved in a methanol/dichloromethane (1/4) mixture, and silica gel powder (100 mL) was added. Then the mixture was evaporated to dryness, resulting in a powdery solid. The solid was loaded by dry method, and subjected to column chromatography using a 20% ethyl acetate/petroleum ether mixture as the eluent. The desired product was collected, concentrated, and dried in a vacuum oven to provide 2.22 g of the product with a yield of 8.51%.

14.2 Preparation of **77-82**

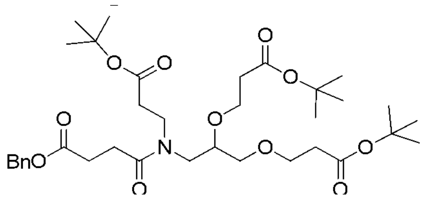

**82-49** (1 g, 2.1025 mmol), benzyl succinate (0.48 g, 2.3128 mmol), HBTU (0.96 g, 2.523 mmol) and HOBT (0.34 g, 2.523 mmol) were weighed and placed in a 500 mL flask, then an appropriate amount of DMF was added to dissolve the mixture, and the contents were allowed to stand at -5°C. DIEA (0.83 mL, 5.046 mmol) was then slowly added dropwise. After the dropwise addition, the reaction was carried out for half an hour, and then the flask was taken out and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid, which was then dissolved in a methanol/ dichloromethane (1/4) mixture, then silica gel powder (100 mL) was added. The mixture was evaporated to dryness to obtain a powdery solid. The powdery solid was loaded by dry method, and subjected to column chromatography using ethyl acetate/petroleum ether as the eluent. The desired product was collected, concentrated, and dried in a vacuum oven to provide 1.1 g of the product. $^1$H-NMR (600 MHz, DMSO-$d_6$) δ 7.44-7.26 (m, 5H), 5.12-5.05 (m, 2H), 3.75-3.66 (m, 1H), 3.63-3.47 (m, 5H), 3.44-3.33 (m, 3H), 2.68-2.52 (m, 4H), 2.45-2.34 (m, 5H), 1.44-1.34 (m, 27H); ESI [M+H$^+$] 666.38

14.3 Preparation of **77-83**

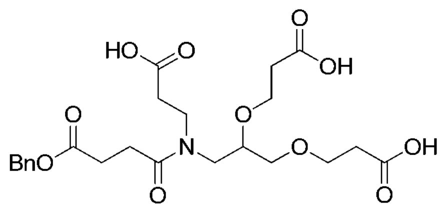

**77-82** (0.476 g, 0.7157 mmol), TFA (1.59 mL, 21.471 mmol) and dichloromethane (5 mL) were placed in a 250 mL flask and dissolved, and the reaction mixture was stirred at room temperature until the reaction was completed. After the reaction was completed, the reaction mixture was concentrated under reduced pressure, and then n-hexane (20 mL) and methyl tert-butyl ether (50 mL) were added for precipitation, the supernatant was discarded, and n-hexane and methyl tert-butyl ether were added again for precipitation. This process was repeated three times. The mixture was filtered to provide a solid product, which was dried in a vacuum oven to provide 0.4175 g of the product.

14.4 Preparation of **77-135**

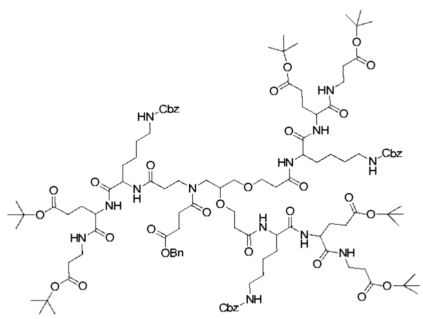

**76-58** (3.28 g, 5.53 mmol), **77-83** (0.8346 g, 1.67 mmol), HBTU (2.216 g, 5.845 mmol) and HOBT (0.789 g, 5.845 mmol) were weighed and placed in a 500 mL flask, and then an appropriate amount of DMF was added to dissolve the mixture. DIEA (1.932 mL, 11.69 mmol) was then slowly added dropwise at -5°C. After the dropwise addition was completed, the reaction was carried out for half an hour, the flask was taken out, and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, evaporated to dryness to obtain a solid, which was then dissolve in methanol/dichloromethane (1/4) mixture, then silica gel powder (100 mL) was added. The mixture was evaporated to dryness to obtain a powdery solid. The solid was loaded by dry method, and subjected to column chromatography using 3-5% $CH_3OH/CH_2Cl$: as the eluent. The desired product was collected, concentrated, and dried in a vacuum oven to provide 2.5 g of the product with a yield of 87.38%. ESI [M+Na$^+$] 2244.59

14.5 Preparation of **77-144**

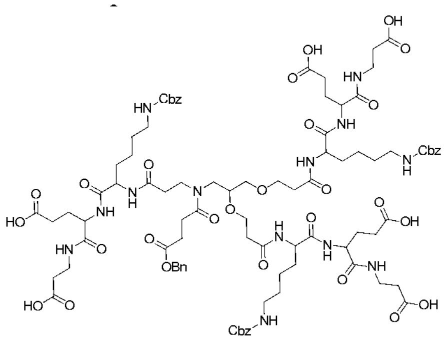

**77-135** (0.8 g, 0.3601 mmol), TFA (0.268 mL, 3.601 mmol) and dichloromethane (5 mL) were placed and dissolved in a 250 mL flask. The mixture was stirred at room temperature until the reaction was completed. After the reaction was completed, the reaction mixture was concentrated under reduced pressure, and then n-hexane (20 mL) and methyl tert-butyl ether (50 mL) were added for precipitation, the supernatant was discarded, and n-hexane and methyl tert-butyl ether were added again for precipitation. This process was repeated three times. Finally, the mixture was filtered to obtain a solid product, which was dried in a vacuum oven to provide 0.9605 g of the product.

14.6 Preparation of **77-146**

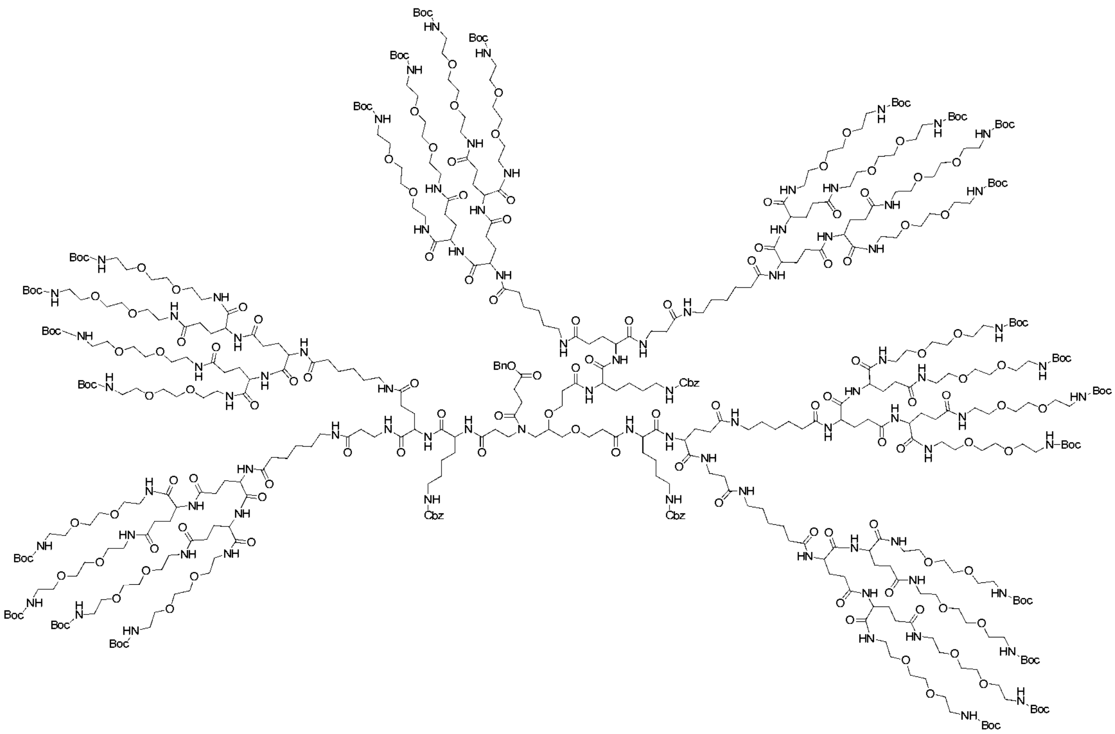

**77-144** (0.678 mmol, 0.3601 mmol), HBTU (1.229 g, 3.24 mmol) and HOBT (0.437 g, 3.24 mmol) were weighed and added into a flask containing **76-93** (3.3 g, 2.376 mmol), then an appropriate amount of DMF was added to dissolve the mixture. DIEA (1.07 mL, 6.481 mmol) was then slowly added dropwise at -5°C. After the dropwise addition, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, and dried to provide 2.5 g of the product with a yield of 60%. $^1$H-NMR (600 MHz, DMSO-$d_6$) δ 8.22 (s, 11H), 8.03 (m, 23H), 7.81 m, 19H), 7.33 (s, 21H), 7.20 (s, 3H), 6.76 (s, 19H), 6.41 (s, 2H), 5.02 (m, 8H), 4.14 (m, 23H), 3.48 (s, 92H), 3.44-3.33 (m, 117H), 3.24 (s, 25H), 3.17 (s, 37H), 3.05 (m, 46H), 2.99-2.91 (m, 16H), 2.60 (m, 6H), 2.39 (s, 8H), 2.21 (s, 7H), 2.10 (s, 48H), 1.79 (m, 44H), 1.47 (s, 19H), 1.35 (m, 216H), 1.30-1.20 (m, 27H), 1.05 (s, 5H), 0.97 (m, 5H)

14.7 Preparation of **77-149**

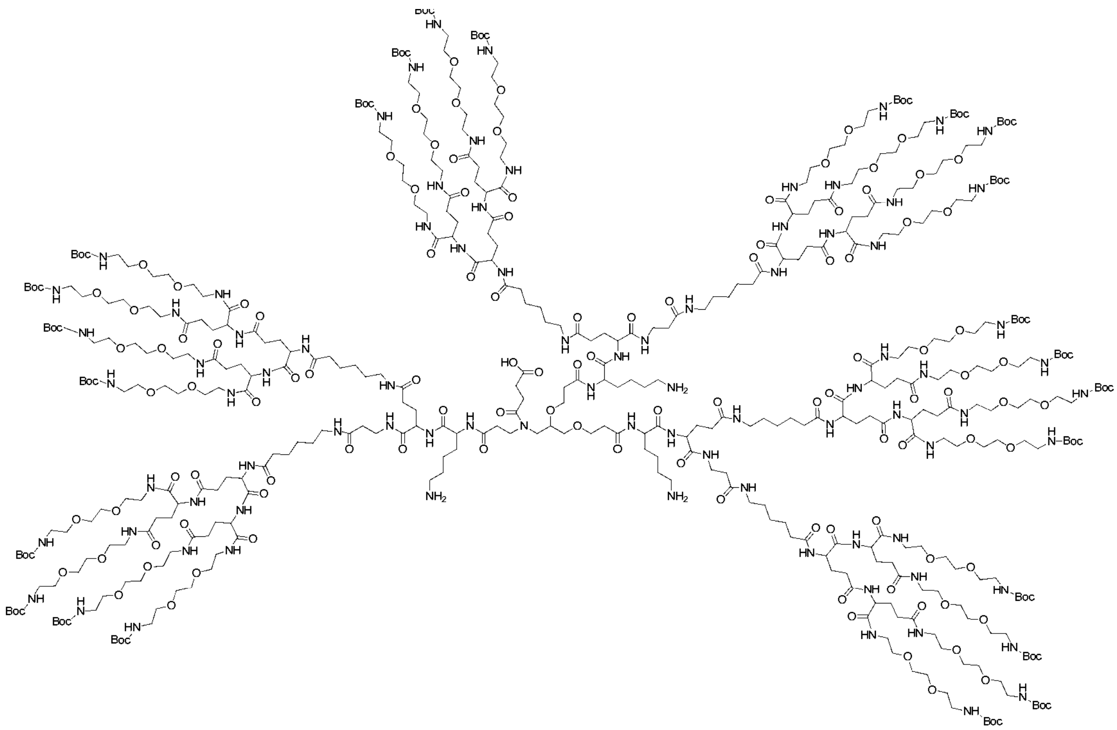

**77-146** (1.5 g, 0.14402 mmol) and 10% Pd/C catalyst (0.01 g) were placed in a hydrogenation reactor, then $CH_3OH$ (30 mL) was added for dissolution. The hydrogenation reactor was sealed, evacuated with a water pump, and filled with hydrogen at approximately 2 MPa. This process was repeated three times. Finally, the pressure reading of the hydrogenation reactor was adjusted to 1.8 MPa. The reaction was then allowed to proceed at 30°C overnight. After the reaction was completed, the reaction mixture was filtered through a Büchner funnel with Celite. The filter cake was washed with methanol (3 mL × 3). The filtrate was placed in a 500 mL round-bottom flask, rotary evaporated under reduced pressure, and subjected to benzene-methanol azeotropic distillation to remove methanol to provide 1.2 g of the product.

14.8 Preparation of **77-155**

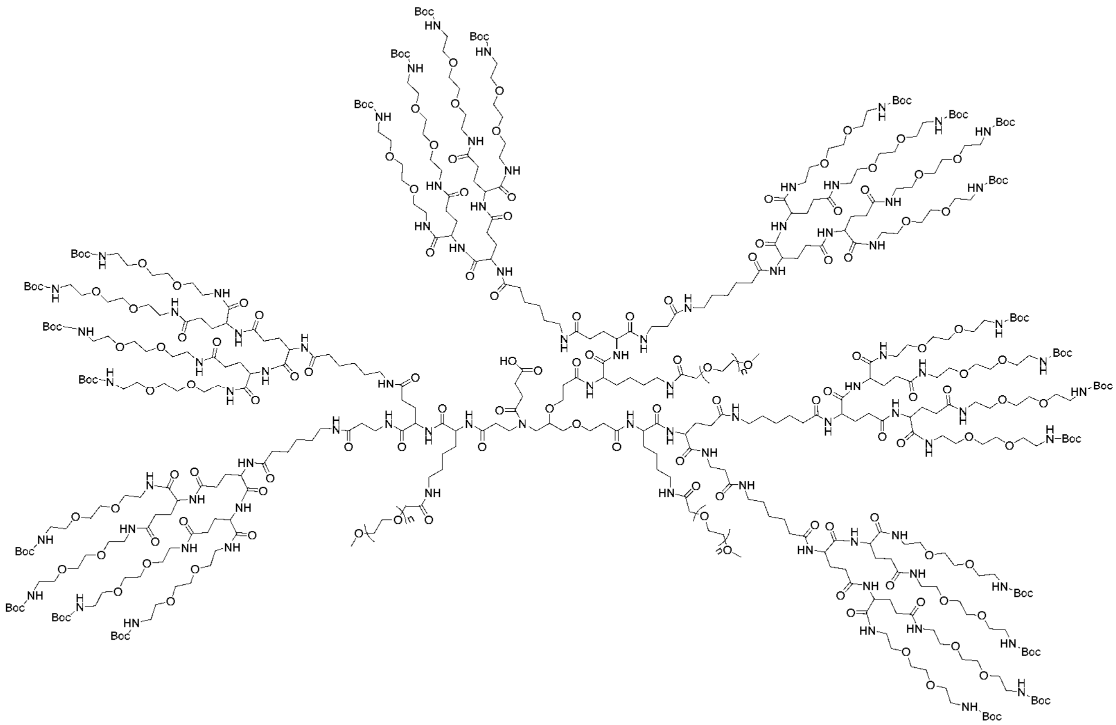

**77-149** (0.85 g, 0.0853 mmol) was placed in a 250 mL flask, DMF (15 mL) was added for dissolution, and the contents were stirred at -5°C for 10 minutes. DIEA (2.2 mL, 13.216 mmol) was then slowly added dropwise, and the reaction was stirred continuously at low temperature for 30 minutes. M-SCM-10K (3 g, 0.2816 mmol, purchased from Jenkem, Lot Number: ZZ390P163) was then added, and reacted under stirring in the dark at low speed for one week at room temperature. After the reaction was completed, methyl tert-butyl ether (200 mL) and n-hexane (70 mL) were added for precipitation. A solid precipitated and was filtered. The filter cake was washed with methyl tert-butyl ether (40 mL × 3). The filter cake was collected and dried in a vacuum oven to provide 3.5 g of product with a yield of 98%.

14.9 Preparation of **77-159**

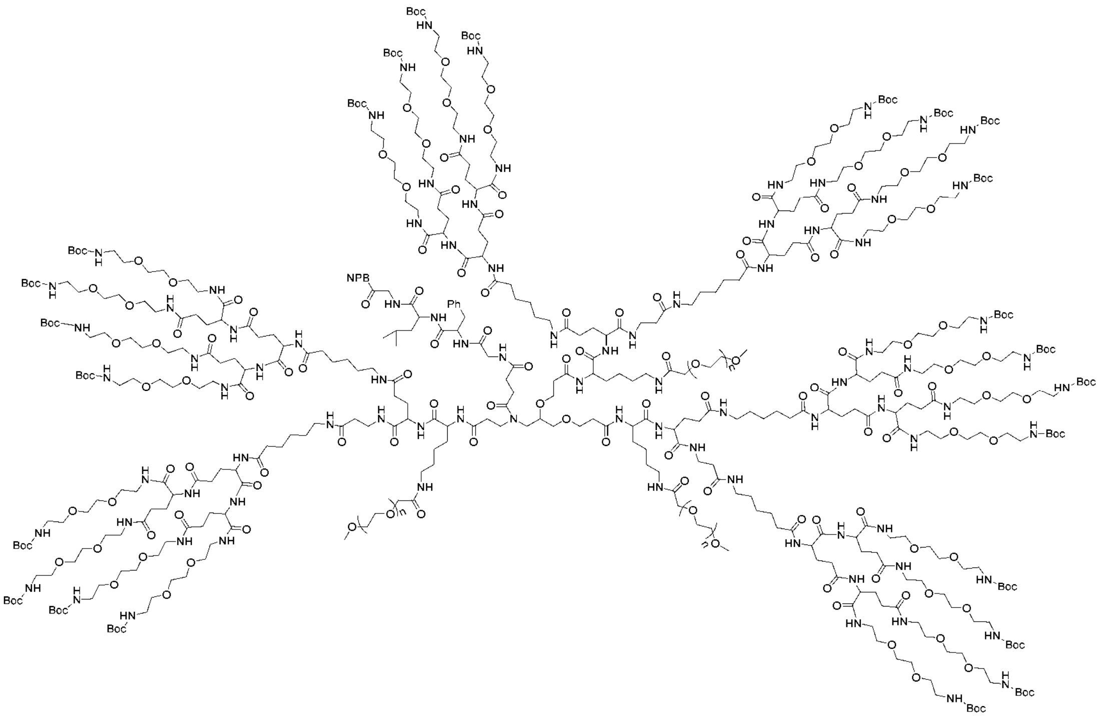

**77-155** (3.5 g, 0.08534 mmol), HBTU (0.161 g, 0.426 mmol) and HOBT (0.057 g, 0.426 mmol) were weighed and added into a flask containing **70-68** (0.292 g, 0.426 mmol), then an appropriate amount of DMF was added to dissolve the mixture. DIEA (0.061 mL, 0.8534 mmol) was then slowly added dropwise at -5°C. After the dropwise addition was completed, the reaction was carried out for half an hour, the flask was then taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was then dissolved in a methanol/dichloromethane (1/4) mixture, then silica gel powder (100 mL) was added. The mixture was evaporated to dryness to provide a powdery solid. The resulting product was loaded by dry method, and subjected to column chromatography using 3-5% $CH_3OH/CH_2Cl_2$ as the eluent. The desired product was collected, concentrated, and dried in a vacuum oven to provide 3.8 g of the product.

14.10 Preparation of **77-161**

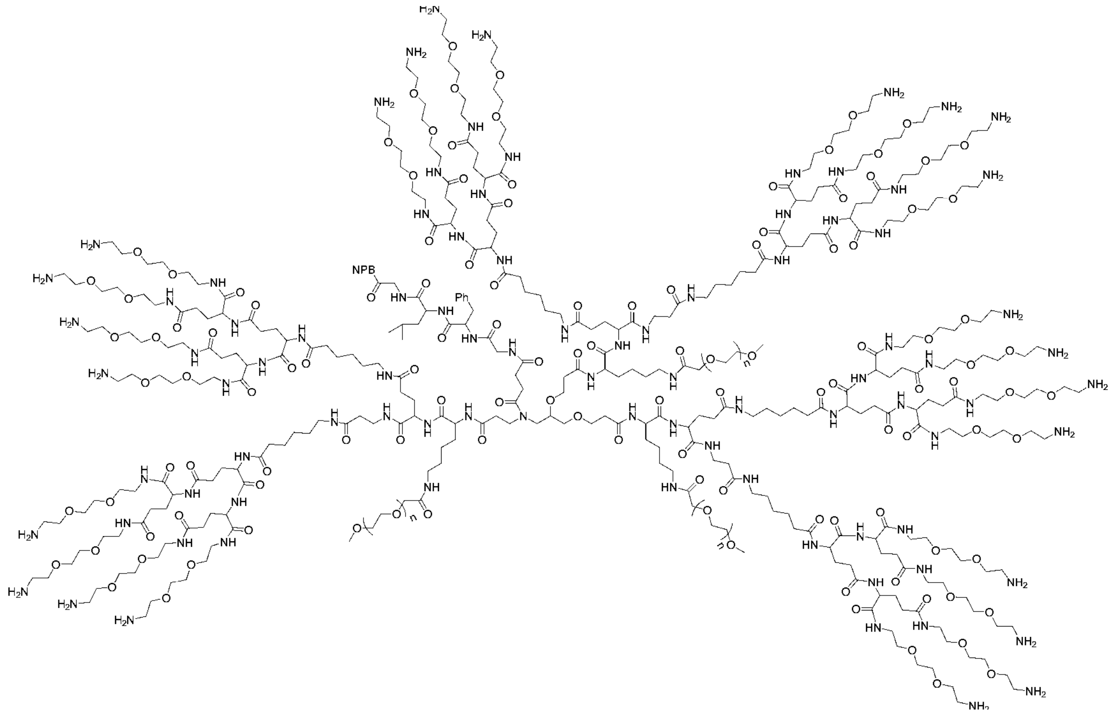

To a flask containing **77-159** (3.8 g, 0.08534 mmol), dichloromethane (5 mL) was added for dissolution with the assistance of ultrasonication, then TFA (1.5 mL, 14 mmol) was added, and the reaction mixture was stirred at room temperature overnight. After the reaction was completed, most of the TFA in the reaction mixture was removed using a rotary evaporator, n-hexane and methyl tert-butyl ether were added to the reaction mixture for precipitation, the supernatant was discarded, and n-hexane and methyl tert-butyl ether were added again for precipitation. This process was repeated three times. Finally, the mixture was evaporated to dryness to provide a solid, which was dried in a vacuum oven to provide 3.39 g of the product.

14.11 Preparation of **77-162**

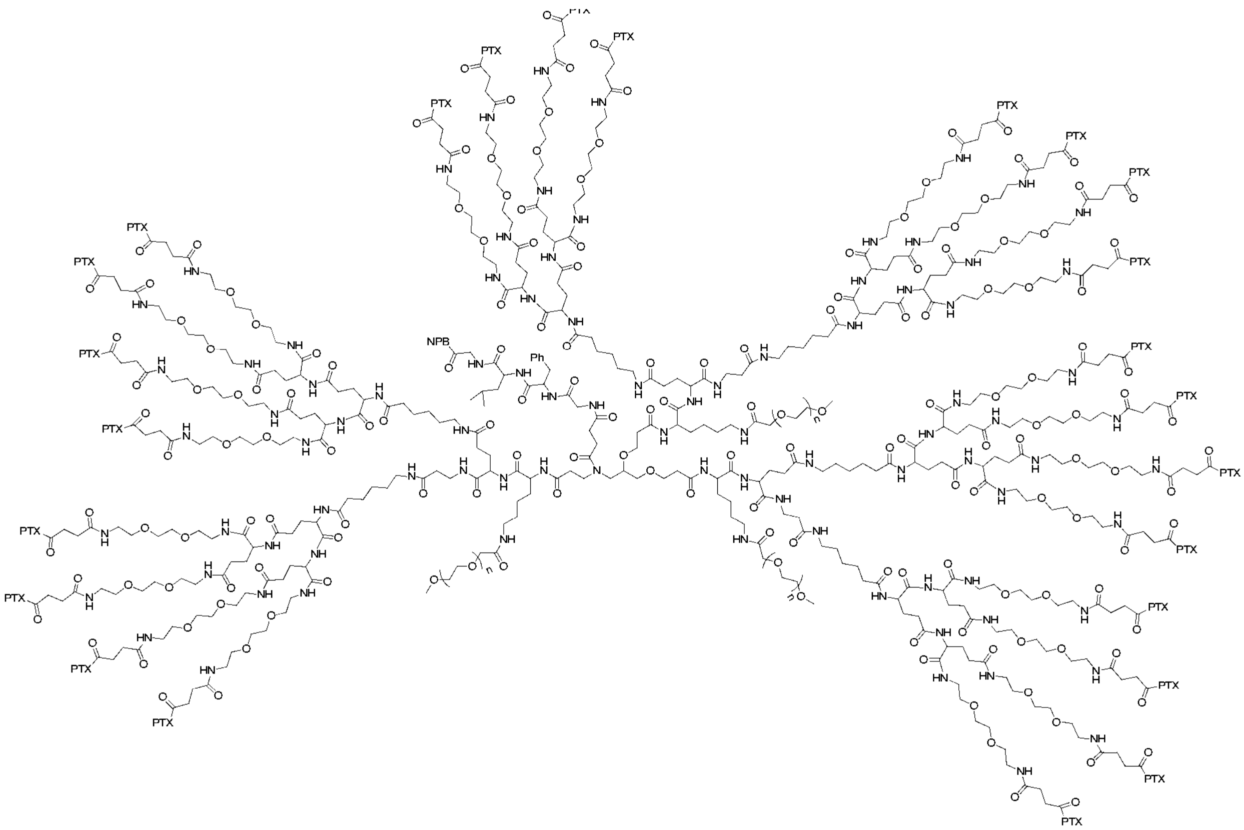

To **76-164** (2.09 g, 1.99 mmol), DIEA (1.345 mL, 8.14 mmol) and DMF (15 mL) were added for dissolution, followed by a dropwise addition of a DMF solution of **77-161** (3 g, 0.07538 mmol). The reaction mixture was stirred overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was then dissolved in a methanol/dichloromethane (1/4) mixture, then silica gel powder (100 mL) was added. The mixture was evaporated to dryness to provide a powdery solid. The powdery solid was loaded by dry method, and subjected to column chromatography using 3-8% $CH_3OH/CH_2Cl_2$ and then 0.5% ammonia water/8% $CH_3OH/CH_2Cl_2$ as the eluents. The desired product was collected, concentrated, and dried in a vacuum oven to provide 0.9 g of the product with a yield of 19%. $^1$H-NMR (600 MHz, DMSO-$d_6$) $\delta$ 9.30-9.18 (m, 24H), 8.29-8.19 (m, 12H), 8.12-8.04 (m, 22H), 8.01-7.92 (m, 80H), 7.90-7.80 (m, 71H), 7.78-7.69 (m, 31H), 7.68-7.61 (m, 48H), 7.58-7.52 (m, 28H), 7.51-7.38 (m, 142H), 7.27-7.21 (m, 8H), 7.20-7.14 (m, 24H), 6.32-6.25 (m, 24H), 5.85-5.78 (m, 24H), 5.56-5.49 (m, 24H), 5.45-5.37 (m, 24H), 5.36-5.30 (m, 24H), 4.96-4.86 (m, 48H), 4.67-4.61 (m, 24H), 4.23-4.16 (m, 24H), 4.15-4.05 (m, 148H), 4.05-3.95 (m, 54H), 3.58-3.43 (m, 2394H), 3.24-3.23 (m, 12H), 3.19-3.15 (m, 307H), 2.63-2.56 (m, 60H), 2.40-2.35 (m, 48H), 2.26-2.19 (m, 74H), 2.13-2.04 (m, 105H), 1.81-1.73 (m, 104H), 1.66-1.60 (m, 30H), 1.51-1.48 (m, 66H), 1.36-1.32 (m, 20H), 1.24-1.21 (m, 18H), 1.05-0.96 (m, 135H)

Example 15: Synthesis of Compound **81-231**

74-150

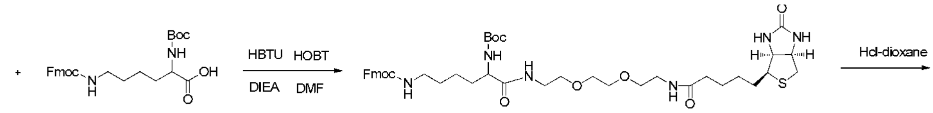


74-171

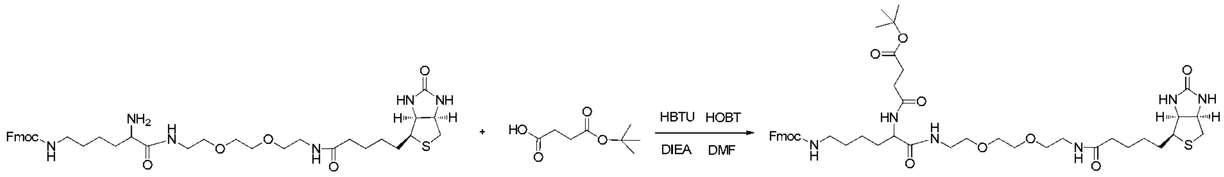


81-207

81-209

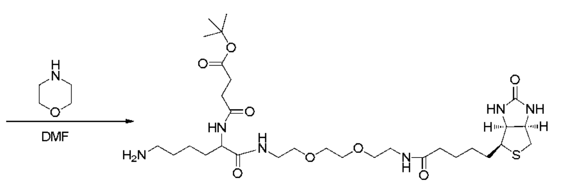


81-213

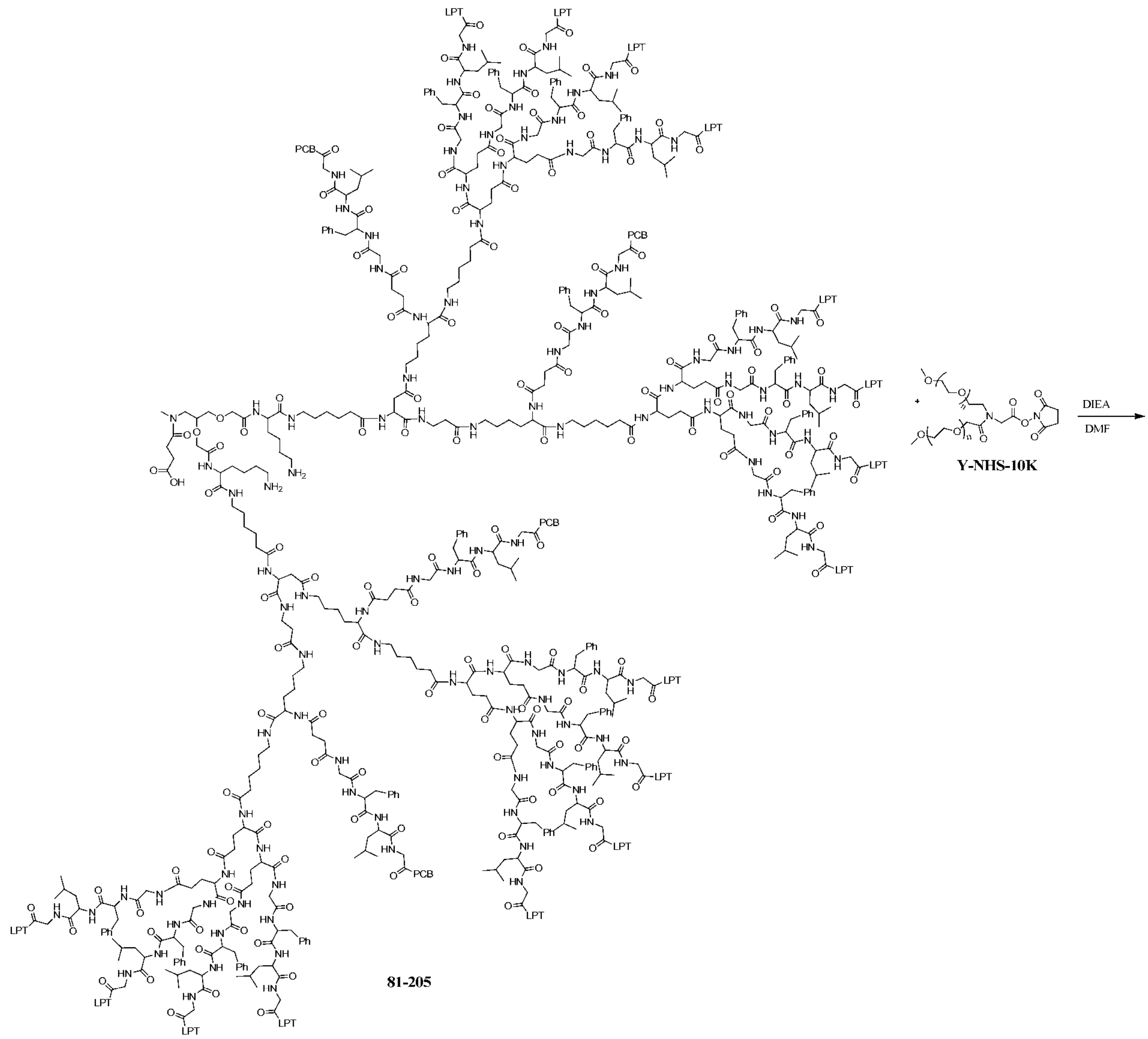


81-205

Y-NHS-10K

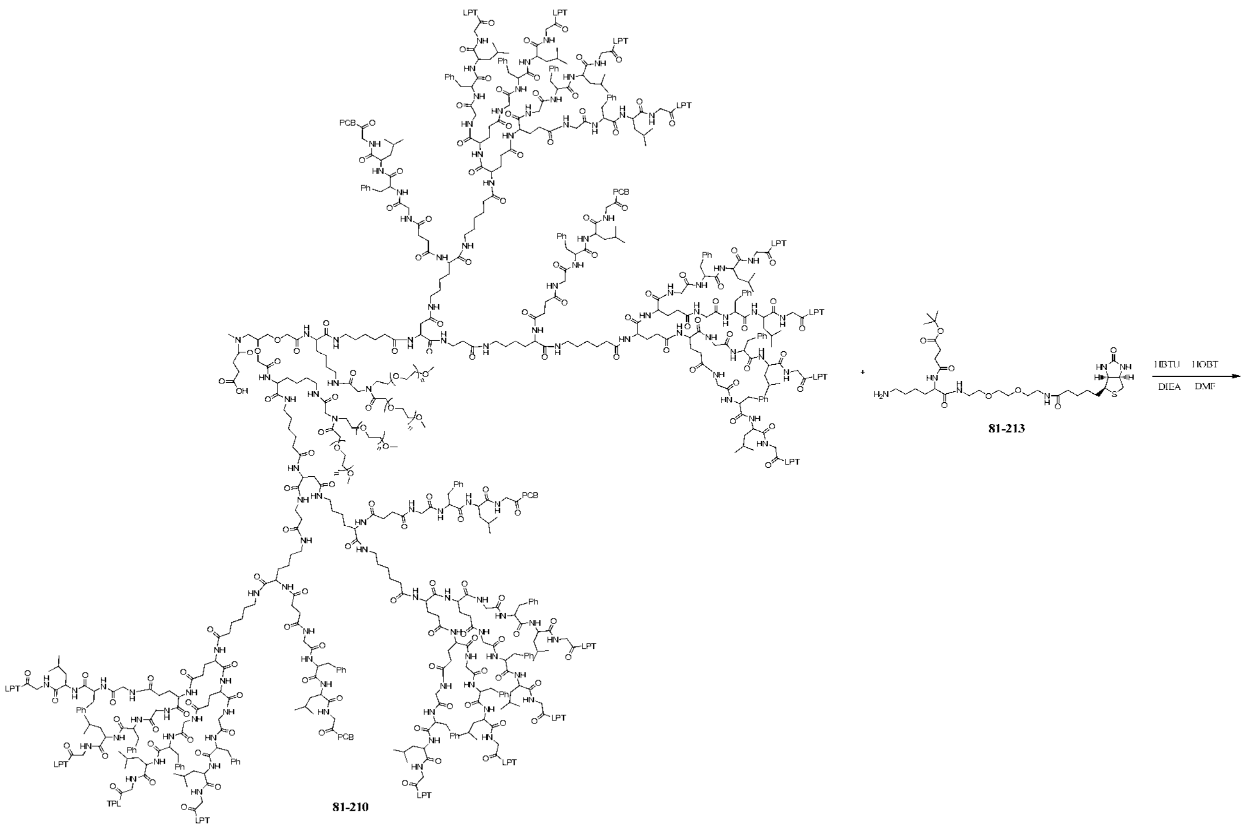


81-210

81-213

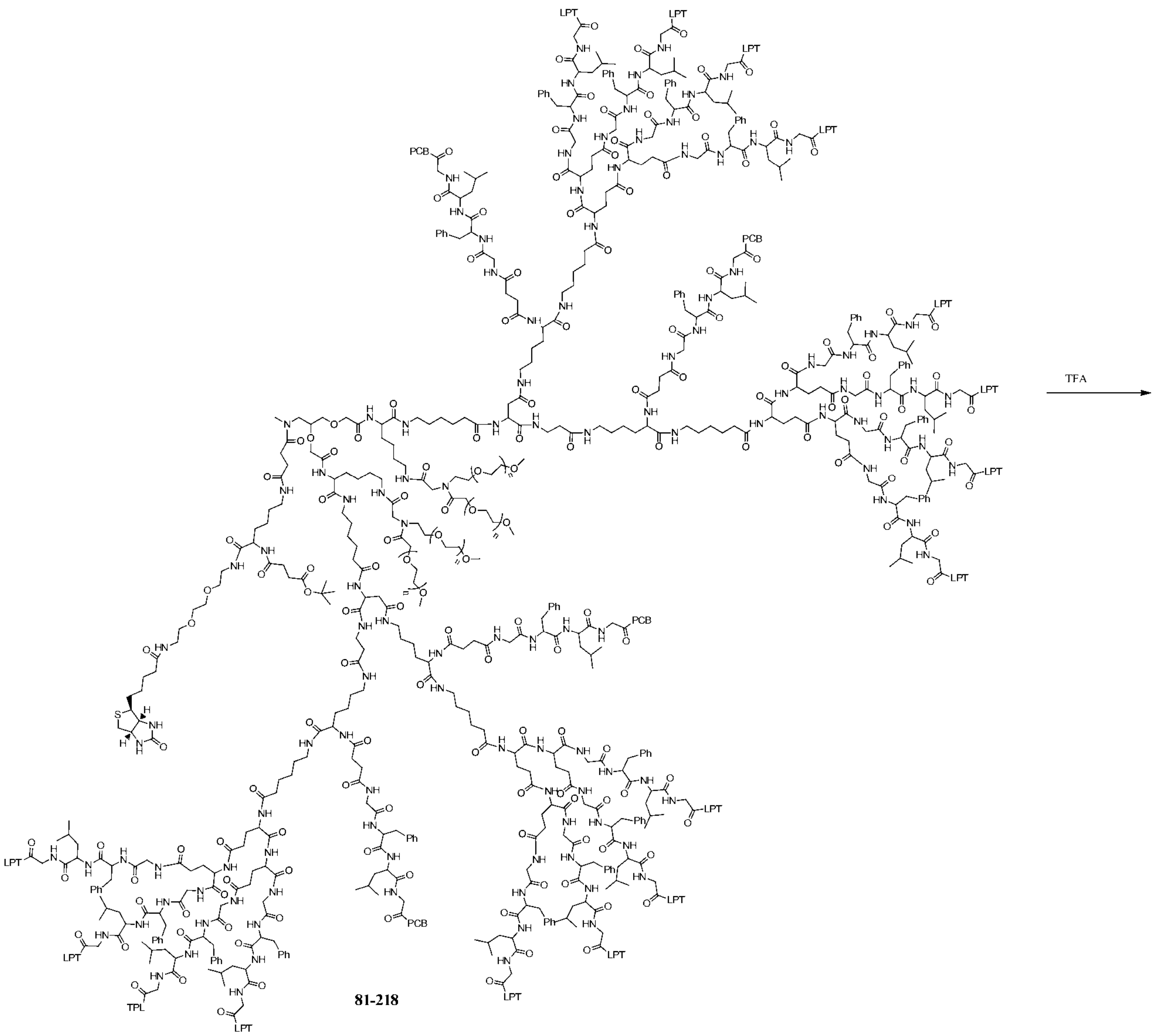


81-218

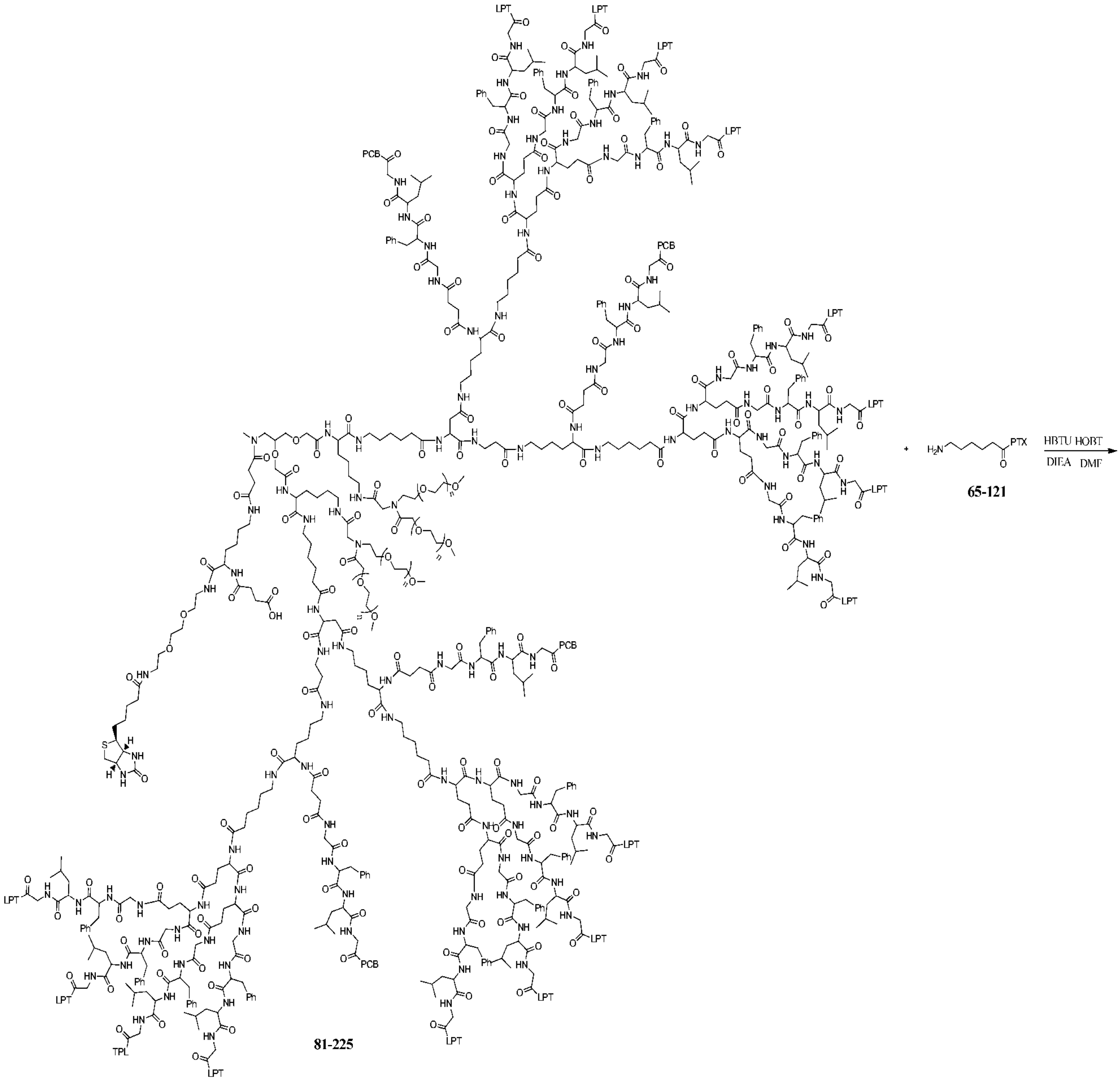


81-225

65-121

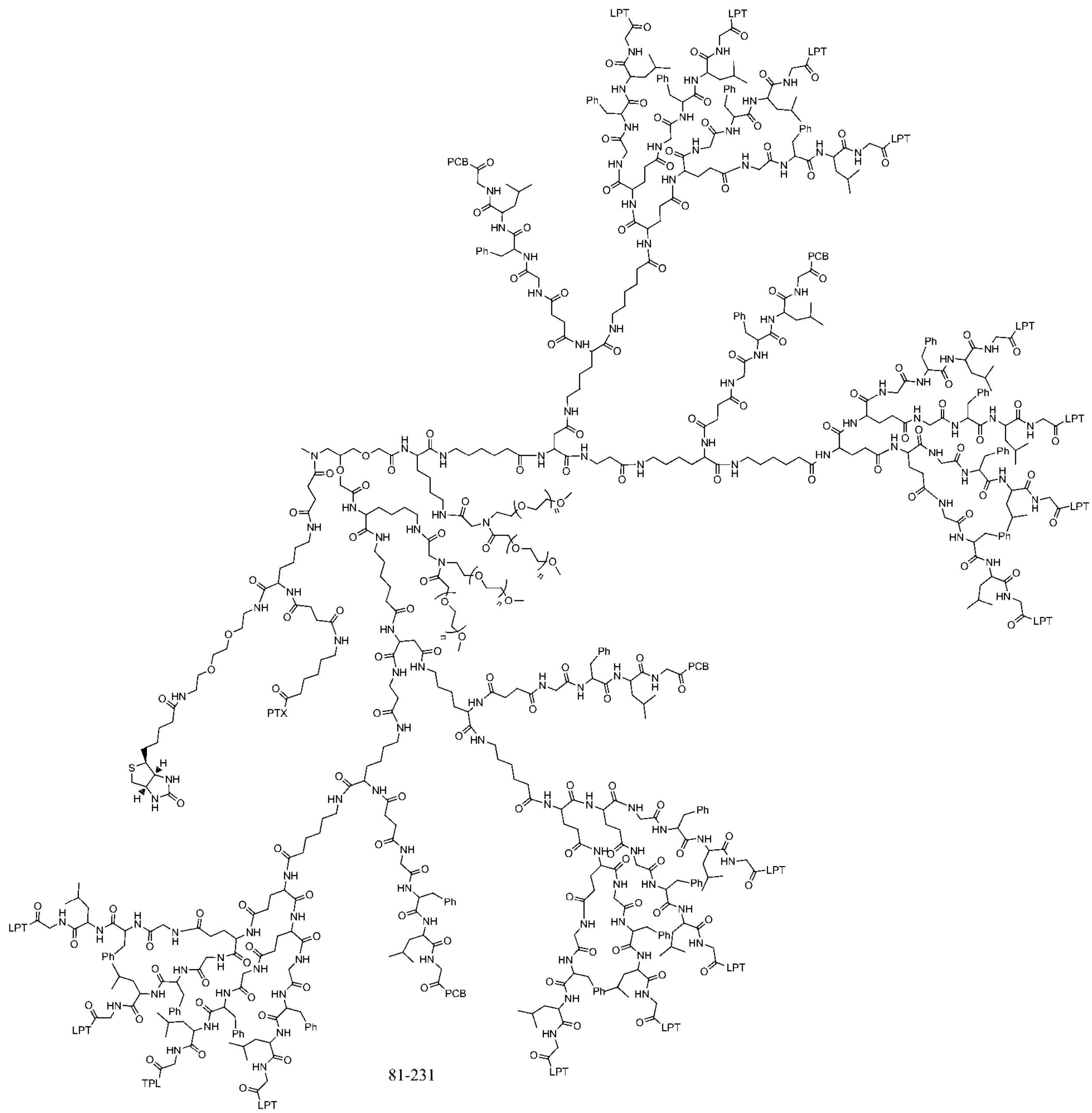

81-231

15.1 Preparation of **74-171**

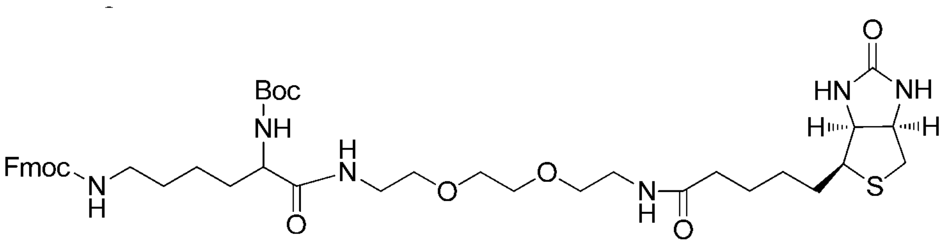

**74-150** (3.9 g, 10.413 mmol), Boc-Lys(Fmoc)-OH (4.878 g, 10.413 mmol), HBTU (4.738 g, 12.495 mmol) and HOBT (1.688 g, 12.495 mmol) were placed in a 250 mL round-bottom flask, then DMF (30 mL) was added for dissolution with the assistance of ultrasonication. The mixture was stirred at 0°C for 3 minutes. DIEA (5.163 mL, 31.239 mmol) was then slowly added dropwise. After 5 minutes, the flask was taken out, and the reaction was carried out under stirring at room

temperature. After the reaction was completed, petroleum ether and methyl tert-butyl ether were added for precipitation. After standing, the supernatant was discarded. This process was repeated several times until the product became a viscous oil. The resulting product was then dissolved in a methanol/dichloromethane (2/8) mixture, then 200-300 mesh silica gel powder was added. The mixture was evaporated to dryness. The resulting product was loaded by dry method, and subjected to column chromatography using 2-4% methanol/dichloromethane as the eluent. The desired product was collected, concentrated, and evaporated to dryness to provide 7.7 g of the product.

15.2 Preparation of **81-207**

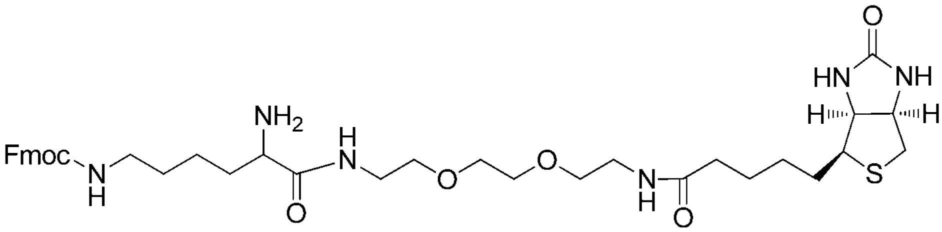

**74-171** (1.5 g, 1.818 mmol) was placed in a 500 mL round-bottom flask, 4 M HCl-dioxane (10 mL) was added for dissolution with the assistance of ultrasonication, and the mixture was reacted under stirring at room temperature. After the reaction was completed, the mixture was concentrated and evaporated to dryness. Methyl tert-butyl ether (50 mL) was then added, treated by ultrasonication, and then rotary evaporated. This process was repeated several times until the product became a viscous oil, which was then dried to provide 1.31 g of the product.

15.3 Preparation of **81-209**

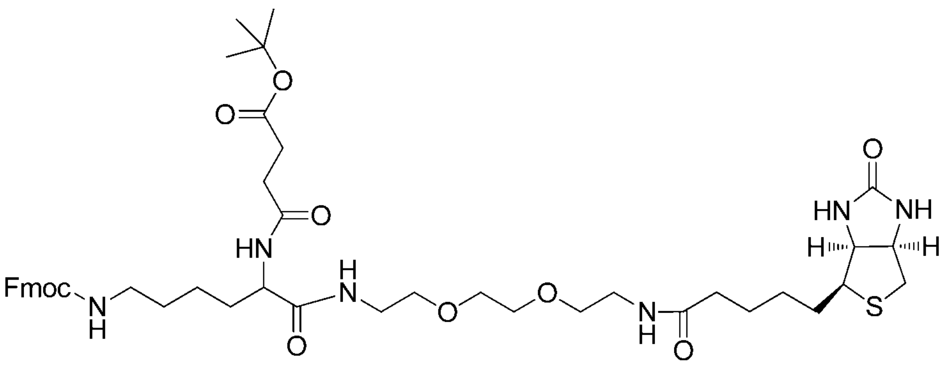

**81-207** (1.317 g, 1.818 mmol), mono-tert-butyl succinate (0.316 g, 1.818 mmol), HBTU (0.827 g, 2.18 mmol) and HOBT (0.294 g, 2.18 mmol) were placed in a 250 mL round-bottom flask, and then DMF (10 mL) was added for dissolution with the assistance of ultrasonication. The mixture was stirred at 0°C for 1 minute. DIEA (1.05 mL, 6.363 mmol) was then slowly added dropwise, and reacted under stirring at room temperature. After the reaction was completed, n-hexane and methyl tert-butyl ether were added, treated by ultrasonication, and allowed to stand, and the supernatant was discarded. This process was repeated several times until the product became a viscous oil, an appropriate amount of dichloromethane was added thereto for dissolution with the assistance of ultrasonication, and the mixture was washed twice with saturated sodium bicarbonate. The organic phase was collected, dehydrated over anhydrous magnesium sulfate, and then 200-300 mesh silica gel powder was added. The mixture was evaporated to dryness. The resulting product was loaded by dry method, and subjected to column chromatography using 6% methanol/dichloromethane as the eluent. The desired product was collected, concentrated, and dried to provide 0.6 g of the product.

15.4 Preparation of **81-213**

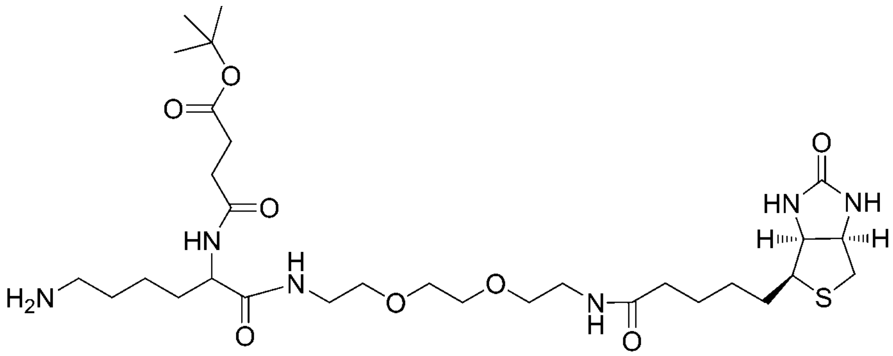

Raw material **81-209** (0.6 g, 0.68 mmol) was placed in a 500 mL round-bottom flask. Acetonitrile (10 mL) and then diethylamine (25 mL) were added, and the mixture was reacted under stirring at room temperature. After the reaction was completed, the diethylamine and acetonitrile were removed by rotary evaporation under reduced pressure. To the mixture was added an appropriate amount of dichloromethane for dissolution. Then 200-300 mesh silica gel powder was added. The mixture was evaporated to dryness. The resulting product was loaded by dry method, and subjected to column chromatography using 1% ammonia/15% methanol/ dichloromethane as the eluent. The organic phase containing the product was collected, concentrated, and evaporated to dryness to provide 0.3 g of the product.

15.5 Preparation of **81-210**

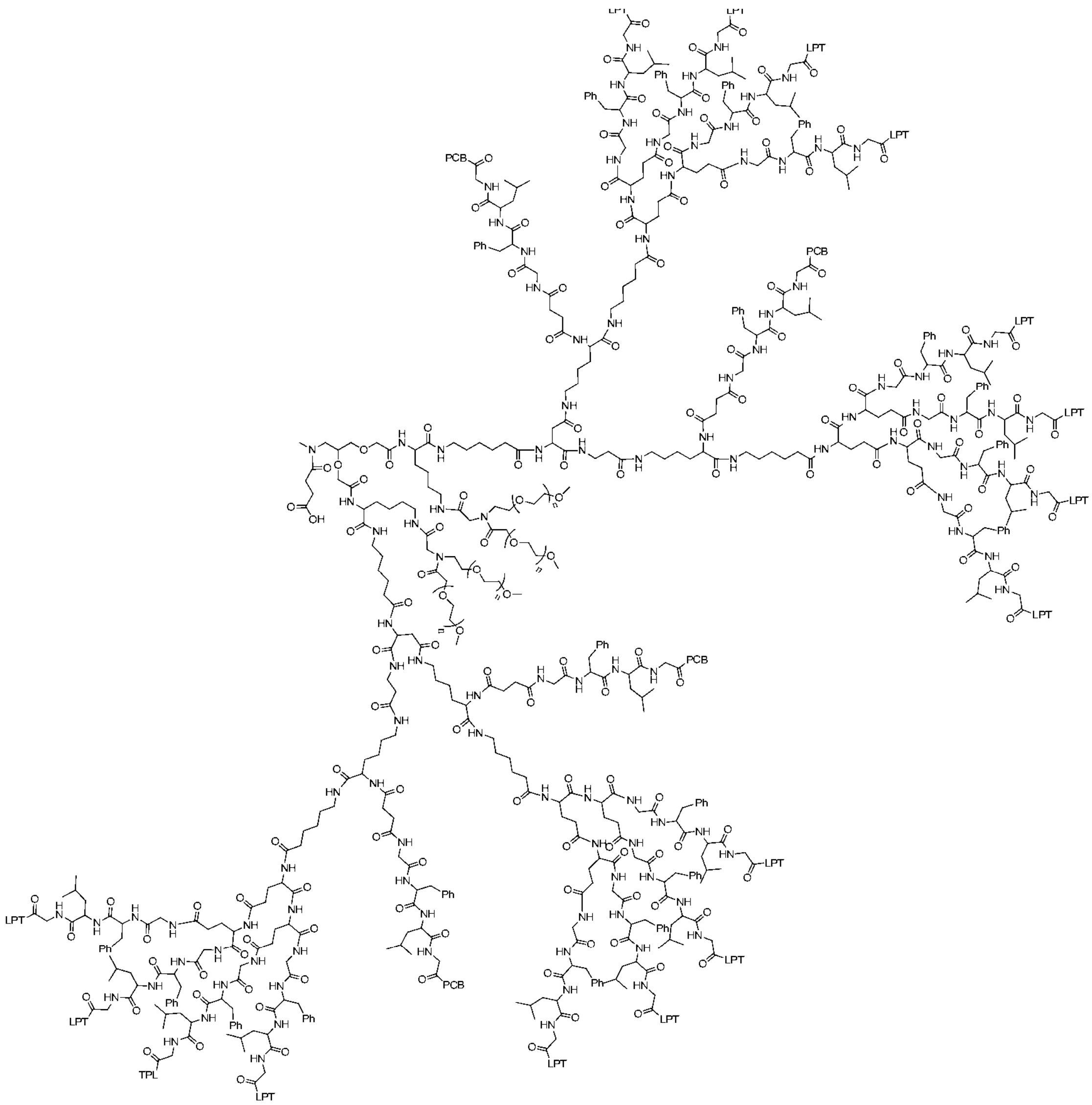

**81-205** (9.6 g, 0.72 mmol) was placed in a 500 mL round-bottom flask, and then ultra-dry DMF (15 mL) was added for dissolution with the assistance of ultrasonication. The mixture was stirred at 0°C for 3 minutes. DIEA (0.185 mL, 1.12 mmol) was then slowly added dropwise, followed by Y-NHS-10K (16.47 g, 1.58 mmol, purchased from JenKem, lot number: ZZ403P059). The reaction was carried out under stirring in the dark at low speed at room temperature under nitrogen. After the reaction was completed, methyl tert-butyl ether (200 mL) was added for precipitation. A solid precipitated, and it was then treated by ultrasonication. The mixture was allowed to stand, and the supernatant was discarded. This process was repeated five times. The mixture was rotary evaporated under reduced pressure to provide a solid, which was then dissolved in 100 mL of a methanol/dichloromethane (1/4) mixture. Then 100-200 mesh silica gel powder was added. The mixture was evaporated to dryness. The resulting product was loaded by dry method, and subjected to column chromatography using 10% methanol/ dichloromethane as the eluent. The organic phase containing the product was collected, concentrated, and evaporated to dryness to provide 2.57 g of the product. $^1$H-NMR (600 MHz, DMSO-$d_6$) $\delta$ 10.18-10.00 (m, 5H), 9.97-9.73 (m, 16H), 8.96-8.84 (m, 5H), 8.82-8.66 (m, 16H), 8.64-8.46 (m, 15H), 8.37-7.61 (m, 169H), 7.55-7.41 (m, 19H), 7.39-6.94 (m, 173H), 6.77-6.62 (m, 12H), 6.62-6.48 (m, 6H), 5.88-5.74 (m, 4H), 5.35-5.13 (m, 32H), 4.89-4.65 (m, 30H), 4.65-4.44 (m, 23H), 4.44-3.93 (m, 54H), 3.91-3.24 (m,2003H), 3.20-3.08 (m, 20H), 3.08-2.86 (m, 114H), 2.86-2.66 (m,26H), 2.65-2.58 (m,28H), 2.43-2.25 (m,25H), 2.25-1.98 (m,31H), 1.92-1.78 (m, 17H), 1.78-1.65 (m,21H), 1.65-1.53 (m,34H), 1.53-1.38 (m,53H), 1.38-1.06 (m,66H), 0.94-0.66 (m, 120H)

15.6 Preparation of **81-218**

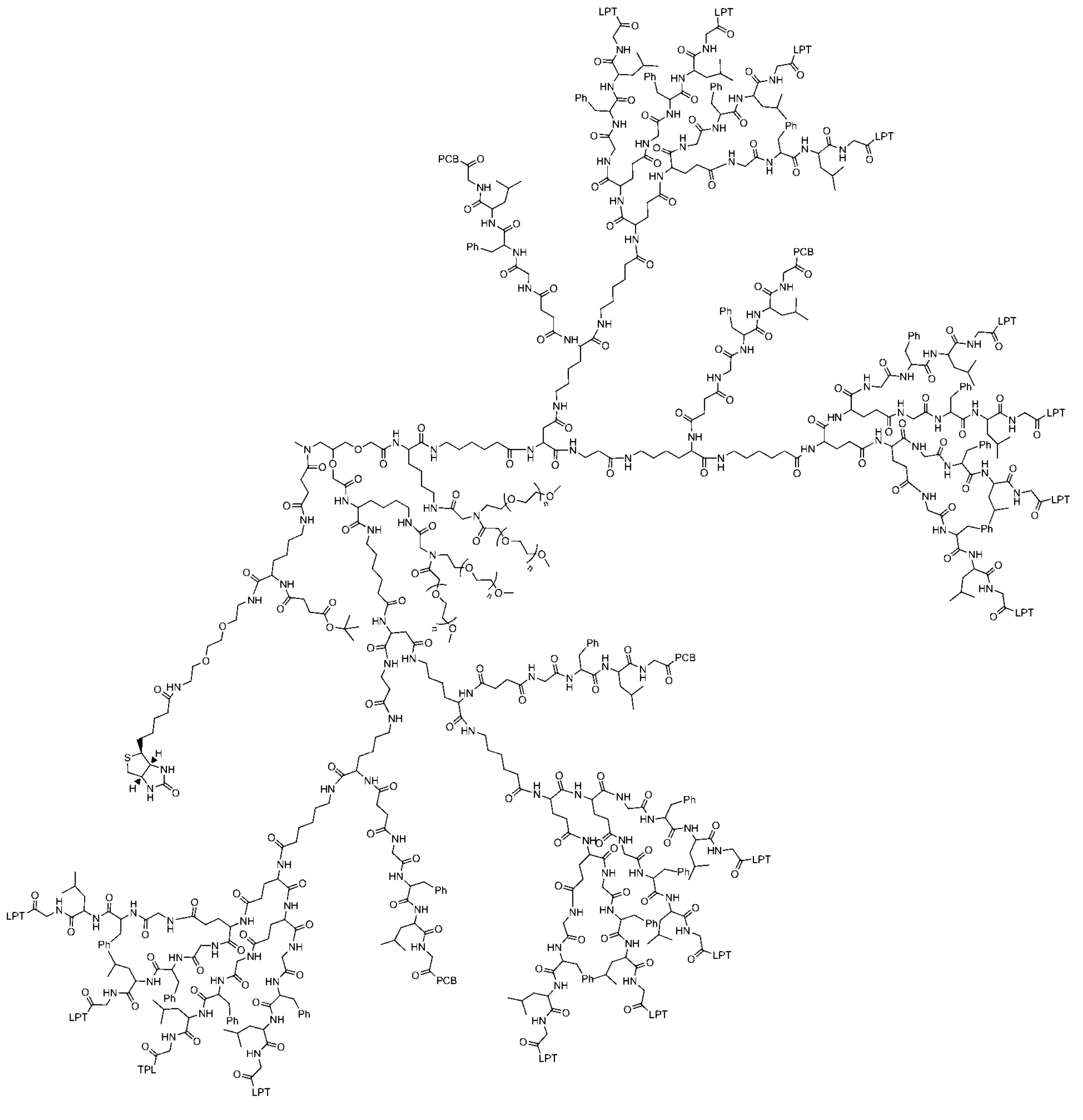

**81-210** (2.57 g, 0.06 mmol), **81-213** (0.23 g, 0.36 mmol), HBTU (0.136 g, 0.36 mmol) and HOBT (0.0486 g, 0.36 mmol) were placed in a 50 mL round-bottom flask, and then DMF (20 mL) was added for dissolution with the assistance of ultrasonication. The mixture was stirred at 0°C for 1 minute. DIEA (0.56 mL, 3.4 mmol) was then slowly added dropwise. After 10 minutes, the flask was taken out, and the reaction was carried out under stirring at room temperature. After the reaction was completed, methyl tert-butyl ether (200 mL) was added for precipitation. A solid precipitated, and the mixture was treated by ultrasonication. The mixture was allowed to stand, and the supernatant was discarded. This process was repeated five times. The mixture was rotary evaporated under reduced pressure and dried to provide a solid crude product, which was then dissolved in an appropriate amount of methanol/dichloromethane (1/4) mixture (100 mL), then 100-200 mesh silica gel powder was added, and the mixture was evaporated to dryness. The resulting product was loaded by dry method, and subjected to column chromatography using 1% ammonia water/10% methanol/dichloromethane as the eluent. The desired product was collected, concentrated, and evaporated to dryness to provide 1.4 g of the product. $^1$H-NMR (600 MHz, DMSO-$d_6$) δ 10.18-10.00 (m, 5H), 9.97-9.73 (m, 16H), 8.96-8.84 (m, 5H), 8.82-8.66 (m, 16H), 8.64-8.46 (m, 15H), 8.37-7.61 (m, 172H), 7.55-7.41 (m, 19H), 7.39-6.94 (m, 173H), 7.16-7.08 (m, 1H), 6.77-6.62 (m, 12H), 6.62-6.48 (m, 7H), 6.44-6.40 (m, 1H), 6.29 (s, 1H), 6.18 (s, 1H), 5.88-5.74 (m, 4H), 5.35-5.13 (m, 32H), 4.89-4.65 (m, 30H), 4.65-4.44 (m, 23H), 4.44-3.93 (m, 60H), 3.91-3.26 (m, 2001H), 3.20-3.08 (m, 28H), 3.08-2.86 (m, 114H), 2.86-2.66 (m, 29H), 2.65-2.58 (m, 29H), 2.43-2.25 (m, 29H), 2.25-1.98 (m, 33H), 1.92-1.78 (m, 17H), 1.78-1.65 (m, 21H), 1.65-1.53 (m, 39H), 1.53-1.38 (m, 58H), 1.38-1.06 (m, 80H), 0.94-0.66 (m, 120H)

15.7 Preparation of **81-225**

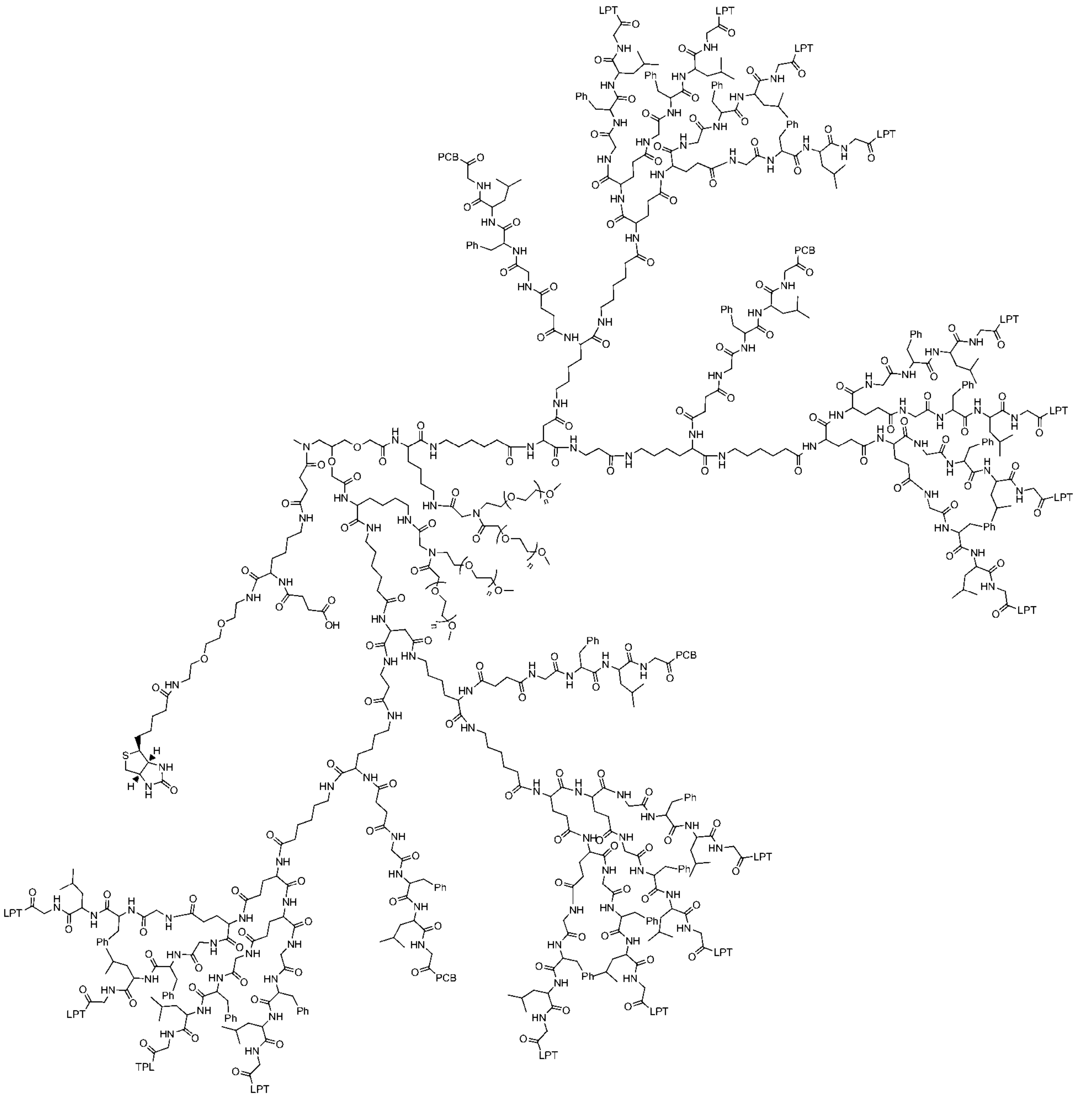

**81-218** (1.4 g, 0.0328 mmol) was placed in a 500 mL round-bottom flask, dichloromethane (10 mL) was added for dissolution, then TFA (20 mL) was added, and the reaction mixture was stirred at room temperature. After the reaction was completed, dichloromethane and most of the TFA were removed by rotary evaporation under reduced pressure. Methyl tert-butyl ether (300 mL) was added for precipitation. A solid precipitated, and the mixture was treated by ultrasonication and allowed to stand. The supernatant was discarded. This process was repeated once. The mixture was evaporated to dryness under reduced pressure to provide a solid crude product, an appropriate amount of DMF was added thereto for dissolution with the assistance of ultrasonication, and the pH was adjusted to a weakly alkaline state by dropwise addition of a small amount of DIEA. Methyl tert-butyl ether (200 mL) was added again for precipitation. A solid precipitated, was treated by ultrasonication. After standing, the supernatant was discarded. This process was repeated three times. The mixture was evaporated to dryness under reduced pressure and then dried to provide 1.3 g of the product. $^1$H-NMR (600 MHz, DMSO-$d_6$) δ 12.01 (s, 1H), 10.18-9.73 (m, 21H), 8.96-8.84 (m, 5H), 8.82-8.66 (m, 16H), 8.64-8.46 (m, 15H), 8.37-7.61 (m, 172H), 7.55-7.41 (m, 19H), 7.39-6.94 (m, 173H), 7.16-7.08 (m, 1H), 6.77-6.62 (m, 12H), 6.62-6.48 (m, 7H), 6.44-6.40 (m, 1H), 6.29 (s, 1H), 6.18 (s, 1H), 5.88-5.74 (m, 4H), 5.35-5.13 (m, 32H), 4.89-4.65 (m, 30H), 4.65-4.44 (m,

23H), 4.44-3.93 (m, 60H), 3.91-3.26 (m, 2001H), 3.20-2.86 (m, 142H), 2.86-2.66 (m, 29H), 2.65-2.58 (m, 29H), 2.43-2.25 (m, 29H), 2.25-1.98 (m, 33H), 1.92-1.78 (m, 17H), 1.78-1.53 (m, 60H), 1.53-1.06 (m, 129H), 0.94-0.66 (m, 120H)

15.8 Preparation of **81-231**

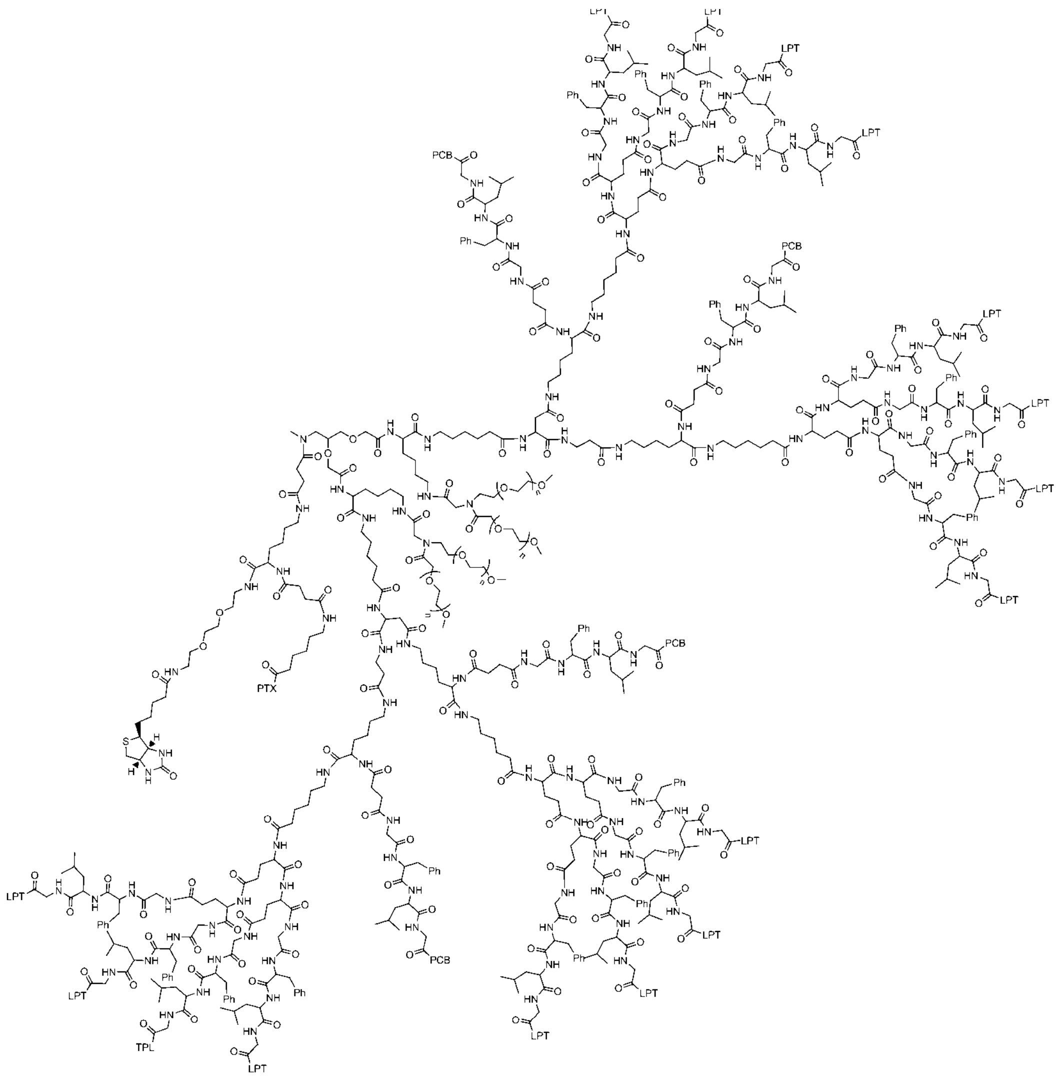

**81-225** (1.3 g, 0.0305 mmol), **65-121** (0.094 g, 0.0976 mmol), HBTU (0.037 g, 0.0976 mmol) and HOBT (0.013 g, 0.0976 mmol) were placed in a 100 mL round-bottom flask, and then DMF (10 mL) was added for dissolution with the assistance of ultrasonication. The mixture was stirred at 0°C for 2 minutes. DIEA (0.1 mL, 0.605 mmol) was then slowly added dropwise. After 2 minutes, the flask was taken out, and the reaction was carried out under stirring at room temperature. After the reaction was completed, methyl tert-butyl ether (450 mL) was added for precipitation. The mixture was allowed to stand at 2-8°C for 15 hours, and then the supernatant was discarded. The solid was then dissolved in 50 mL of an anhydrous ethanol/dichloromethane (1/4) mixture, and 600 mL of methyl tert-butyl ether was added to precipitate a solid, which was then filtered by suction. This operation was repeated three times. Finally, the filter cake was dried to provide 1.2 g of the product. $^1$H-NMR (600 MHz, DMSO-$d_6$) δ 10.18-10.00 (m, 5H), 9.97-9.73 (m, 16H), 8.96-8.84 (m, 6H), 8.82-8.66 (m, 16H), 8.64-8.46 (m, 15H), 8.37-7.61 (m, 180H), 7.55-7.41 (m, 22H), 7.39-6.94 (m, 179H), 6.77-6.62 (m, 12H), 6.62-6.40 (m,8H), 6.29 (s, 1H), 6.18 (s, 1H), 5.89-5.74 (m, 5H), 5.47-5.13 (m, 34H), 4.92-4.65 (m, 33H), 4.66-3.94 (m, 110H), 3.91-3.26 (m, 2018H), 3.20-3.08 (m, 28H), 3.08-2.86 (m, 106H), 2.86-2.58 (m, 58H), 2.43-2.25 (m, 30H), 2.25-1.65 (m, 83H), 1.65-1.38 (m, 92H), 1.38-1.02 (m, 77H), 0.94-0.66 (m, 120H)

Example 16: Synthesis of Compound **84-102**

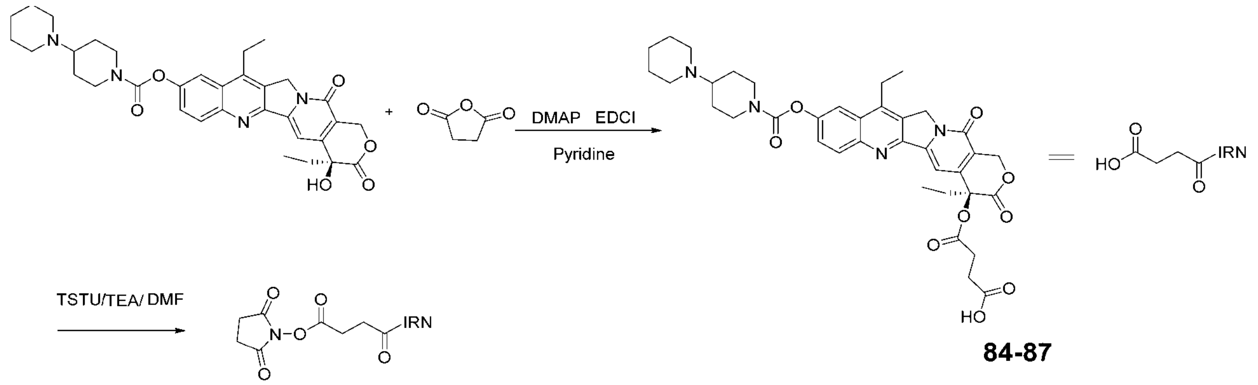


**84-87**

**84-97**

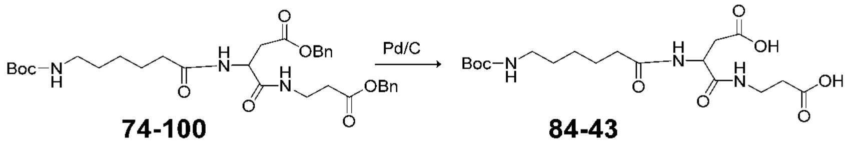


**74-100** **84-43**

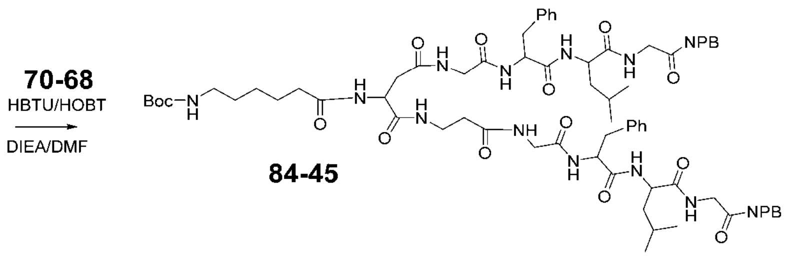


**84-45**

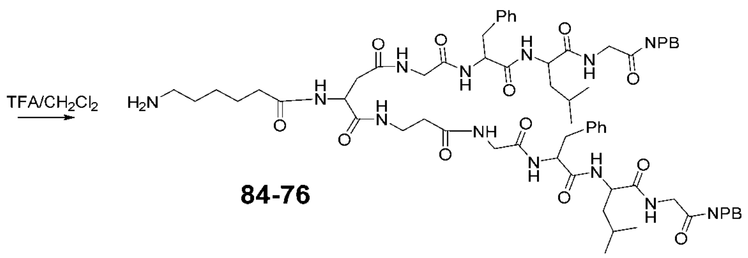


**84-76**

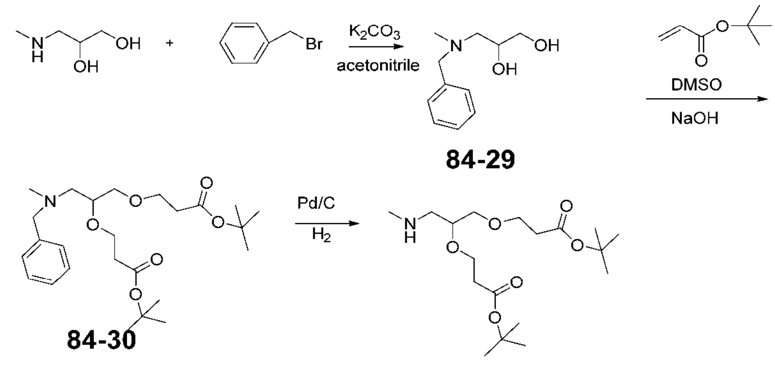


**84-29**

**84-30**

**84-31**

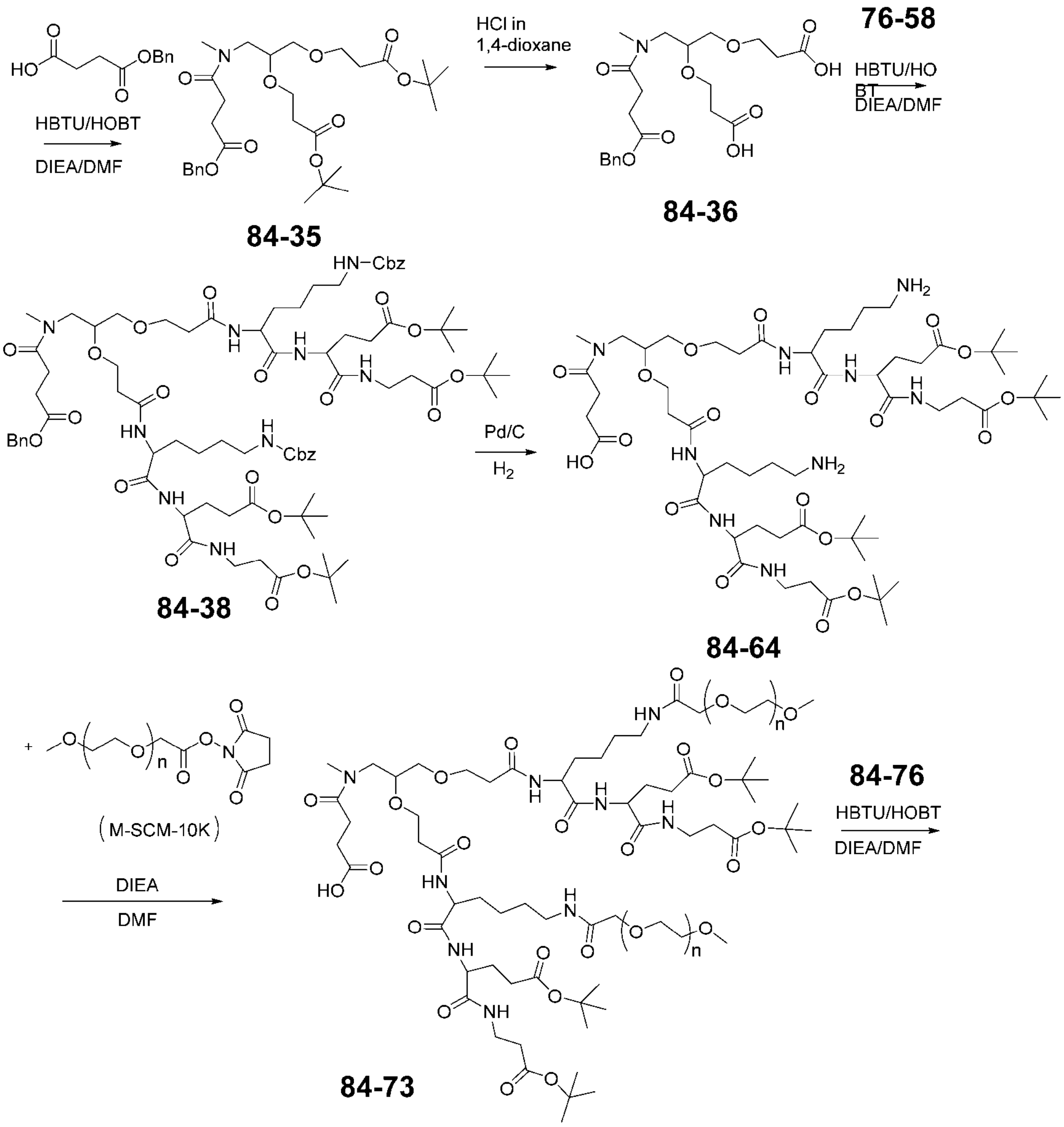


84-35

76-58

84-36

84-38

84-64

84-76

84-73

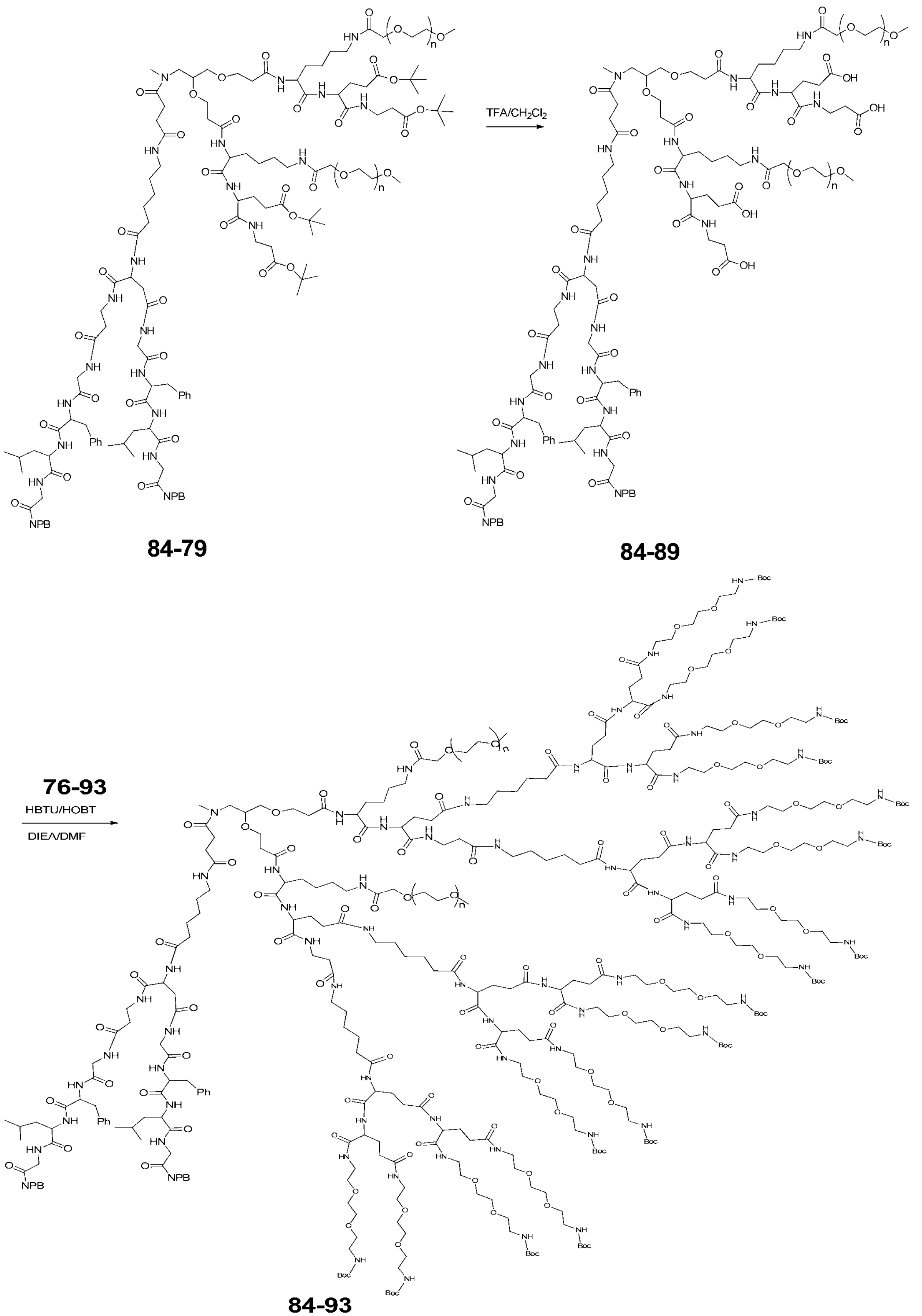


84-79

84-89

84-93

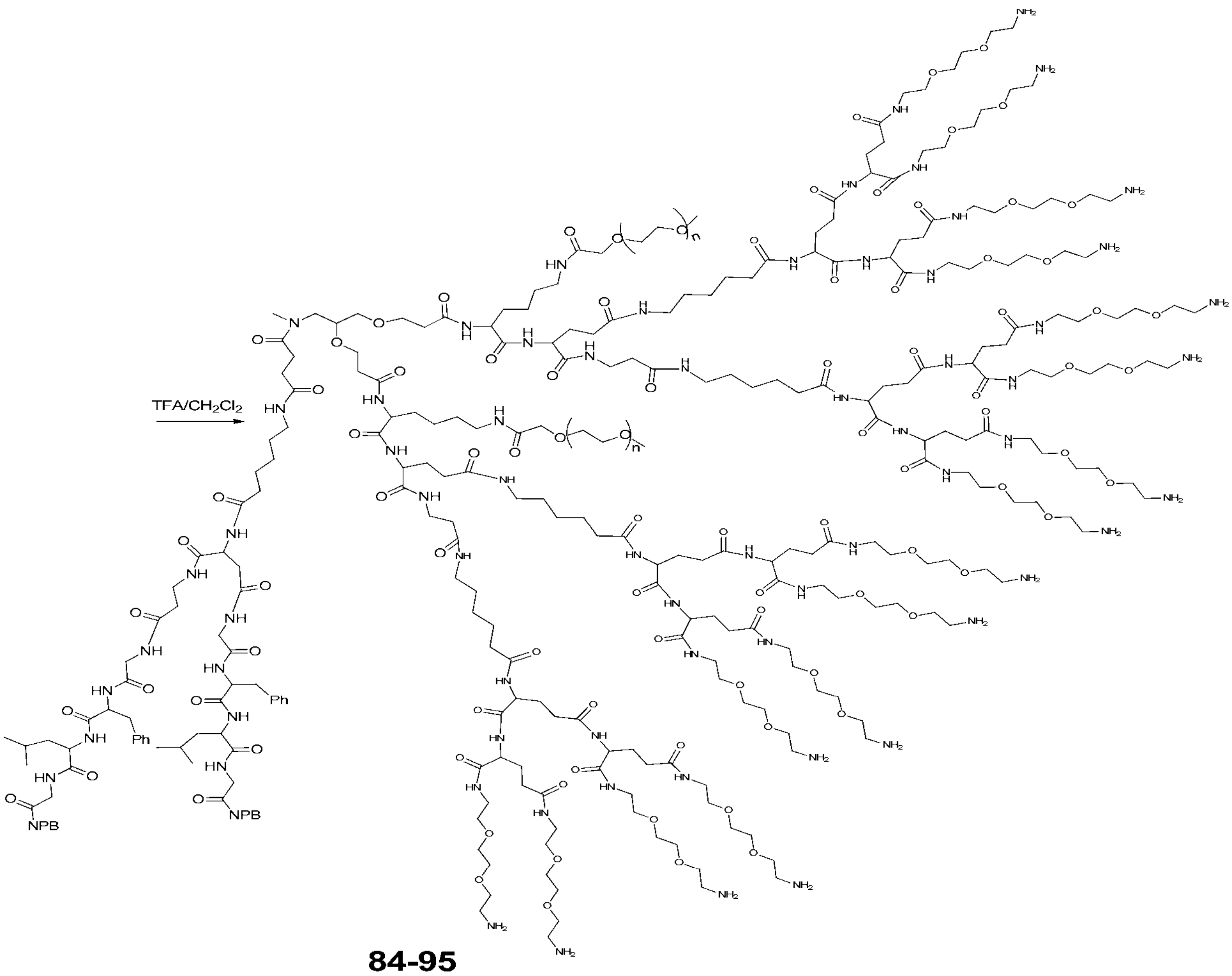


84-95

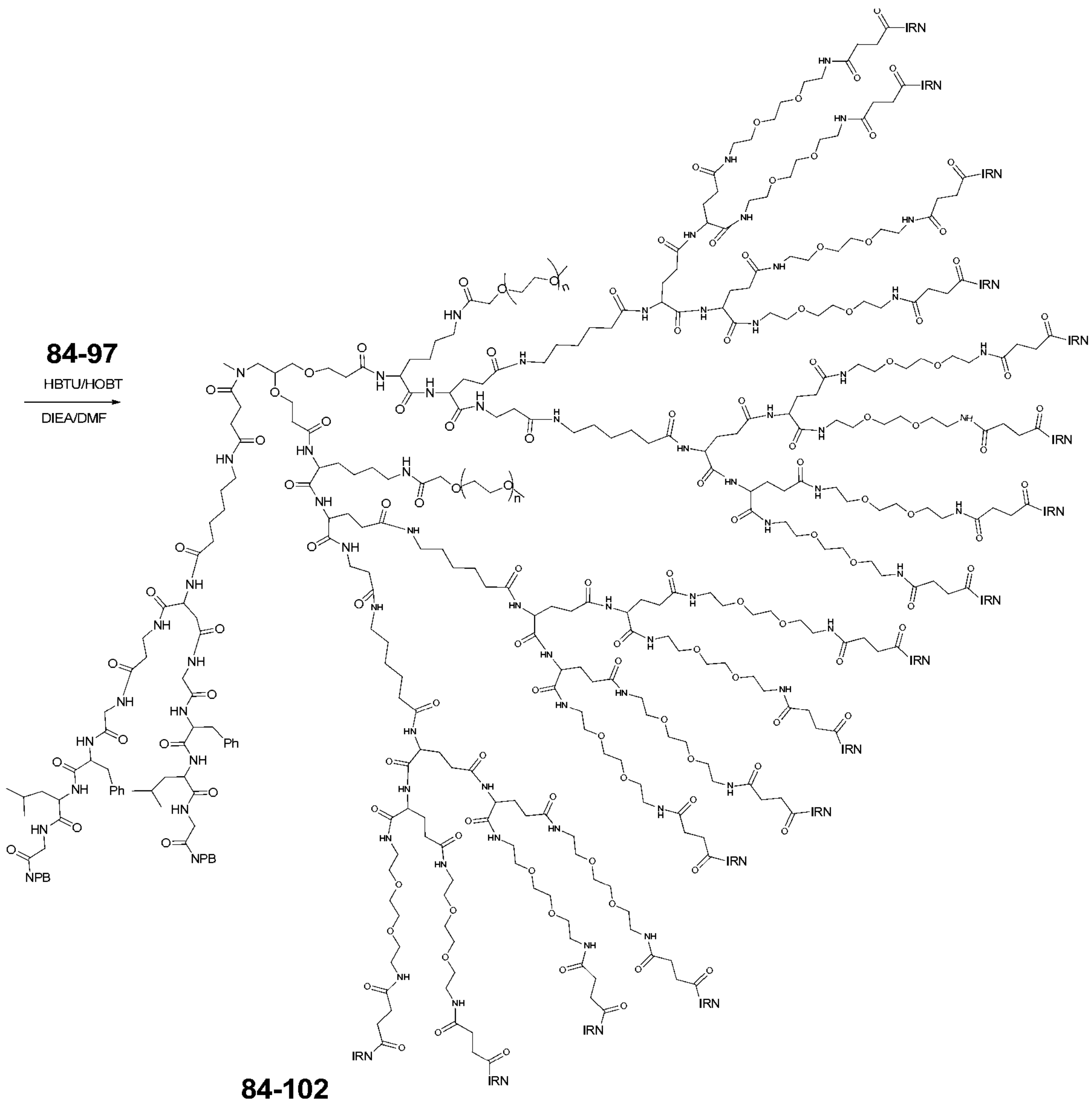


**84-102**

16.1 Preparation of **84-87**

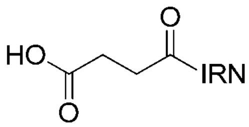

Irinotecan (5 g, 8.52 mmol), succinic anhydride (1.7 g, 17.04 mmol) and pyridine (15 mL) were dissolved in a 250 mL flask, then DMAP (1.04 g, 8.52 mmol) was added, and the mixture was reacted under stirring in an oil bath at 45°C for 24 h. After the reaction was completed, an appropriate amount of toluene was added, and the mixture was rotary evaporated under reduced pressure. An appropriate amount of dichloromethane was then added. The organic phase was washed with 0.1 N hydrochloric acid and then saturated sodium chloride solution, dried over anhydrous magnesium sulfate, concentrated, then silica gel powder was added, and the mixture was evaporated to dryness. The resulting product was loaded by dry method, and subjected to column chromatography using 4% methanol/dichloromethane as the eluent. The desired product was collected and dried to provide 2.5 g of the product.

16.2 Preparation of **84-97**

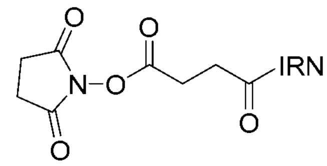

**84-87** (2.5 g, 3.644 mmol), TSTU (1.2 g, 4.0084 mmol) and DMF (10 mL) were dissolved in a 50 mL flask, then triethylamine (1.2 mL, 9.15 mmol) was added, and the mixture was reacted under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The resulting product was dissolved in a mixture of dichloromethane and methanol, loaded by dry method, and subjected to column chromatography using 3% methanol/dichloromethane as the eluent. The desired product was collected, concentrated, and dried in a vacuum oven to provide 2 g of the product.

16.3 Preparation of **84-43**

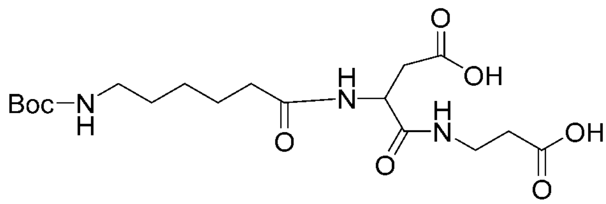

**74-100** (1.5 g, 2.5096 mmol) was added to a hydrogenation reactor. To this were added 10% Pd/C catalyst (0.08 g) and then DMF (15 mL). The reactor was charged with hydrogen gas to a pressure of 300 psi, and the mixture was reacted under stirring at room temperature overnight. After the reaction was completed, the reaction mixture was filtered through a Büchner funnel with filter paper. The filter cake was washed with DMF (15 mL × 3). The filtrate was then transferred to a 250 mL round-bottom flask and used as the raw material for the next step.

16.4 Preparation of **84-45**

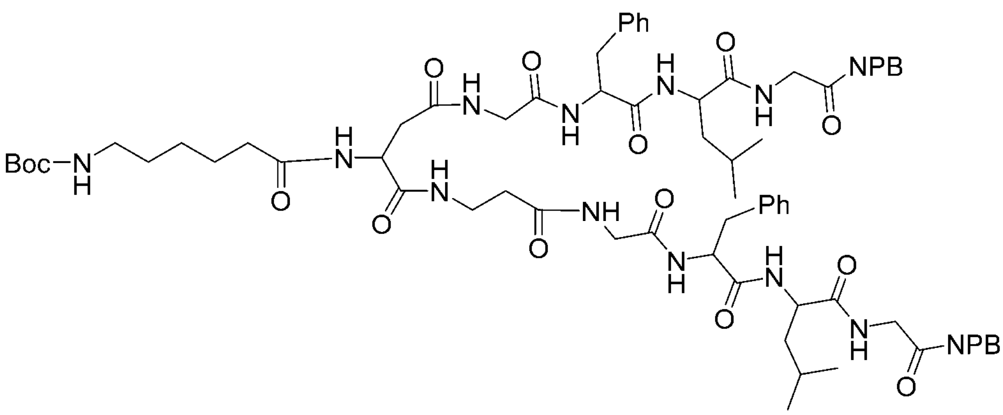

**84-43** (0.45 g, 1.0938 mmol), **70-68** (1.75 g, 2.5159 mmol), HBTU (1.08 g, 2.8439 mmol), HOBT (0.4 g, 2.8439 mmol) and DMF (2 mL) were dissolved in a 250 mL flask under stirring. The reaction flask was then placed at -5°C, and the mixture was stirred for approximately 20 minutes. DIEA (1.5 mL, 8.7504 mmol) was then slowly added dropwise. After the dropwise addition was completed, the reaction was carried out under stirring at -5°C for 1 hour, and then the reaction flask

was brought to room temperature, and the mixture was stirred. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to provide a solid product. The obtained solid product was dissolved in a mixture of dichloromethane and methanol, loaded by dry method, and subjected to column chromatography using 3% methanol/dichloromethane as the eluent. The desired product was collected, concentrated, and dried in a vacuum oven to provide 0.86 g of the product.

16.5 Preparation of **84-76**

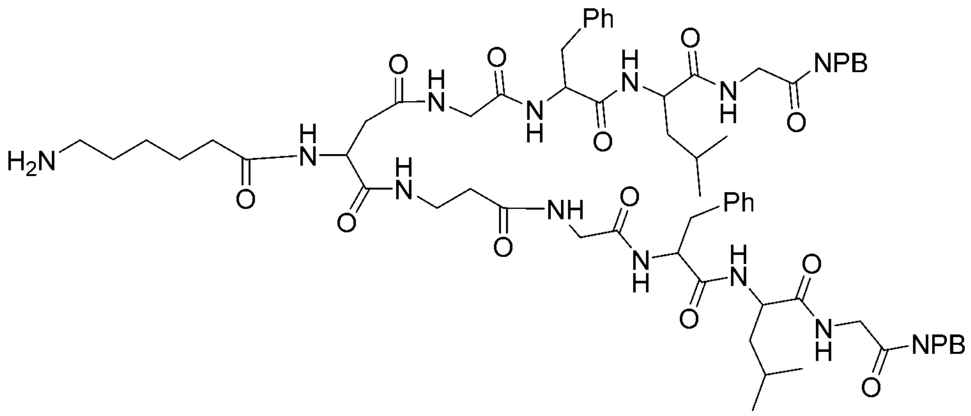

**84-45** (0.86 g, 0.4856 mmol), TFA (0.72 mL, 9.7116 mmol) and dichloromethane (0.5 mL) were dissolved in a 50 mL flask, and reacted under stirring at room temperature until the reaction was completed. After the reaction was completed, n-hexane (30 mL) and methyl tert-butyl ether (20 mL) were added for precipitation, the supernatant was discarded, and n-hexane and methyl tert-butyl ether were added again for precipitation. This process was repeated four times. The mixture was filtered to provide a solid product, which was dried in a vacuum oven to provide 0.8 g of the product.

16.6 Preparation of **84-29**

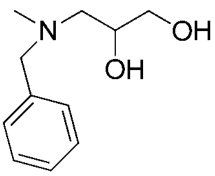

3-Methylamino-1,2-propanediol (8 g, 76.089 mmol), benzyl bromide (15.6 g, 91.3068 mmol), $K_2CO_3$ (31.54 g, 228.267 mmol) and acetonitrile (35 mL) were placed in a 500 mL flask, and reacted under stirring at 30°C in a water bath. After the reaction was completed, the white solid in the reaction mixture was filtered off, dichloromethane was added to the filtrate, and then silica gel powder was added and evaporated to dryness. The resultant product was loaded by dry method, and subjected to column chromatography using 0.5% ammonia water/1% methanol/dichloromethane as the eluent. The desired product was collected, concentrated and dried to provide 9.2 g of the product. $^1$H-NMR (600 MHz, DMSO-$d_6$) δ 7.34-7.19 (m, 5H), 4.55 (s, 1H), 3.69 (q, *J* = 5.7 Hz, 1H), 3.60 (d, *J* = 14.2 Hz, 1H), 3.55-3.45 (m, 2H), 2.14 (s, 3H); FT MS ESI m/z [M+H$^+$] 196.13

16.7 Preparation of **84-30**

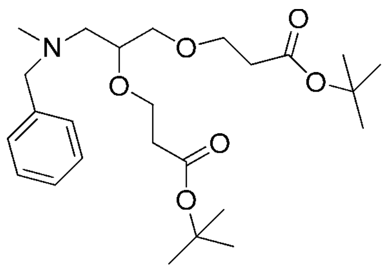

In a 250 mL flask, **84-29** (5 g, 25.6082 mmol) was dissolved in DMSO (18 mL), stirred in a water bath at 30°C for 10 minutes, then 5.0 M NaOH solution (1 mL) was added, and the mixture was stirred continuously for 10 minutes, followed by the addition of tert-butyl acrylate (7.88 g, 61.4597 mmol), and reacted until the reaction was completed. The reaction mixture was transferred to a 1 L separatory funnel, and saturated brine (200 mL) and ethyl acetate (200 mL) were added for extraction. The organic phase was collected, and the aqueous phase was further extracted with ethyl acetate (200 mL × 3). The organic phases were combined and washed with saturated brine (200 mL × 2) and evaporated to dryness. The obtained product was dissolved in a mixed solvent of dichloromethane and methanol, loaded by dry method, and subjected to column chromatography using 10% ethyl acetate/petroleum ether as the eluent. The product fraction was collected, concentrated, and dried in a vacuum oven to provide 4.4 g of the product. $^1$H-NMR (600 MHz, DMSO-$d_6$) δ 7.33-7.19 (m, 5H), 3.74-3.66 (m, 2H), 3.59-3.55 (m, 2H), 3.54-3.51 (m, 1H), 3.49-3.45 (m, 3H), 3.38-3.34 (m, 1H), 2.45-2.33 (m, 6H), 2.13 (s, 3H), 1.40-1.37 (m, 18H)

16.8 Preparation of **84-31**

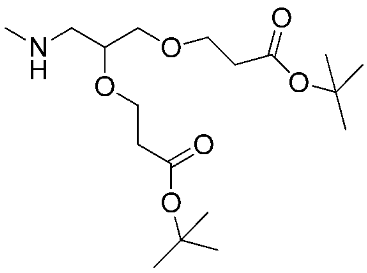

**84-30** (4.4 g, 9.7434 mmol) was added to a hydrogenation reactor. To this were added 10% Pd/C catalyst (0.3 g) and methanol (30 mL). The reactor was charged with hydrogen gas to a pressure of 300 psi, and the mixture was reacted under stirring at room temperature overnight. After the reaction was completed, the reaction mixture was filtered through a Büchner funnel with filter paper and washed with methanol (15 mL × 3). The filtrate was collected, evaporated under reduced pressure, and dried to provide 3 g of the product, which was used as the raw material for the next step. $^1$H-NMR (600 MHz, DMSO-$d_6$) δ 3.71 (dt, $J$ = 9.6, 5.9 Hz, 1H), 3.62 (dt, $J$ = 9.6, 6.3 Hz, 1H), 3.59-3.54 (m, 2H), 3.46-3.42 (m, 1H), 3.41-3.36 (m, 1H),3.23(s, 1H), 2.53-2.47 (m, 2H), 2.45 (dd, $J$ = 12.2, 6.6 Hz, 1H), 2.42-2.36 (m, 4H), 2.26 (s, 3H), 1.40-1.33 (m, 18H); FT MS ESI m/z [M+H$^+$] 362.25

16.9 Preparation of **84-35**

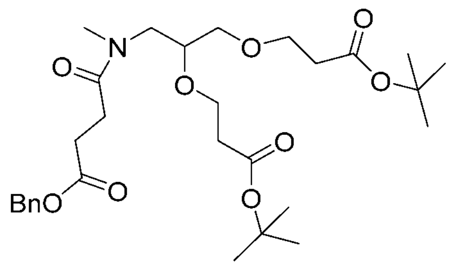

In a 250ml flask, mono-benzyl succinate (1.84g, 8.8251mmol) was dissolved in DMF (10ml) under stirring. The reaction flask was then placed at -5°C, and DIEA (5.3ml, 32.0912mmol) was slowly added dropwise, and continuously stirred for 5 minutes. HATU (3.66g, 9.6274mmol) was then added, and the contents were stirred continuously for 10 minutes. Then **84-31** (2.9g, 8.0228mmol) was added. The reaction flask was placed at -5°C and stirred for 1 hour. The flask was taken out and brought to room temperature, and the mixture was stirred. After the reaction was completed, the reaction mixture was transferred to a 1L separatory funnel, and saturated brine (200ml) and ethyl acetate (200ml) were added for extraction. The organic phase was collected, and the aqueous phase was further extracted with ethyl acetate (200 mL × 3). The organic phases were combined, washed with saturated brine (200 mL × 2), and evaporated to dryness. The obtained product was dissolved in a mixed solvent of dichloromethane and methanol, loaded by dry method, and subjected to column chromatography using 20% ethyl acetate/petroleum ether as the eluent. The desired product was collected, concentrated, and dried in a vacuum oven to provide 3.06 g of the product. $^1$H-NMR (600 MHz, DMSO-$d_6$) δ 7.38-7.34 (m, 5H), 5.08-5.06 (m, 2H), 3.74-3.67 (m, 1H), 3.61-3.49 (m, 4H), 3.44-3.34 (m, 2H), 3.33-3.14 (m, 2H),2.90(s, 3H) 2.66-2.51 (m, 4H), 2.45-2.39 (m, 2H), 2.37-2.31 (m, 2H), 1.46-1.33 (m, 18H); FT MS ESI m/z [M+H$^+$] 552.31, [M+Na$^+$] 574.29

16.10 Preparation of **84-36**

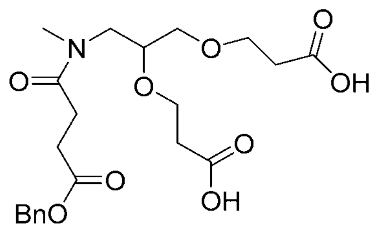

To a 250ml flask, **84-35** (3.06g, 5.5468mmol) and dichloromethane (10ml) were added, then TFA (16.5ml, 221.872mmol) was added for dissolution under stirring. The reaction flask was then placed at room temperature, and the reaction was carried out for 2 hours. After the reaction was completed, the liquid in the reaction mixture was evaporated to dryness using a vacuum rotary evaporator. The resulting mixture was then dissolved in dichloromethane (10ml) and evaporated again using a rotary evaporator. This step was repeated several times. The mixture was then dissolved in dichloromethane, neutralized with DIEA, and evaporated using a rotary evaporator. Finally, the mixture was dried in an oven to provide 2.4g of product.

16.11 Preparation of **84-38**

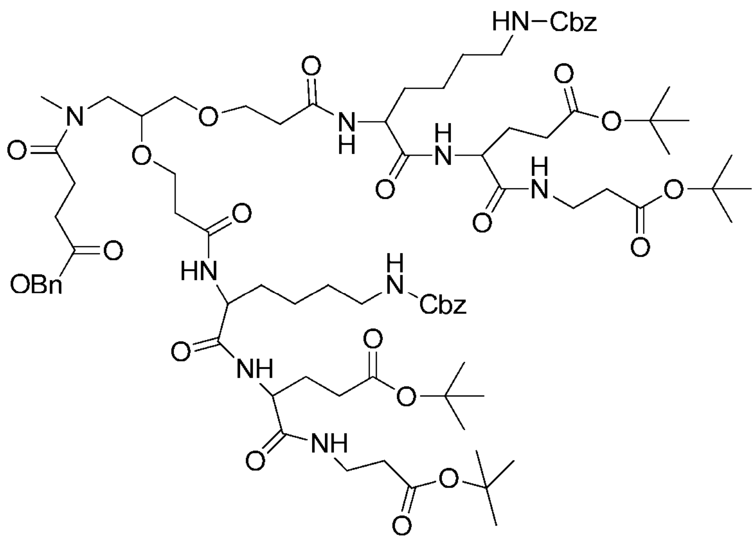

**84-36** (2.4 g, 3.0675 mmol), **76-58** (4 g, 6.7485 mmol), HBTU (3.5 g, 9.2025 mmol), HOBT (1.2 g, 9.2025 mmol) and DMF (25 mL) were dissolved in a 250 mL flask, then stirred at -5°C for approximately 20 minutes. DIEA (4 mL, 24.54

mmol) was then slowly added dropwise. After the dropwise addition was completed, the reaction flask was placed at -5°C, the reaction was carried out under stirring for 1 hour. The flask was then brought to room temperature, and the mixture was stirred. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated 4 times. The mixture was filtered to provide a solid product. The obtained solid product was dissolved in a mixed solvent of dichloromethane/methanol, loaded by dry method, and subjected to column chromatography using 3% methanol/dichloromethane as the eluent. The desired product component was collected, concentrated, and dried in a vacuum oven to provide 4.5 g of the product. $^1$H-NMR (600 MHz, DMSO-$d_6$) δ 8.07-7.81 (m, 7H), 7.47-7.24 (m, 15H), 7.23-7.13 (m, 1H), 5.15-4.93 (m, 6H), 4.27-4.14 (m, 4H), 3.77-3.47 (m, 6H), 3.47-3.24 (m, 5H), 3.21-3.08(m, 4H), 3.06-2.84(m, 3H), 2.84-2.62 (m, 3H), 2.61-2.45 (m, 3H), 2.44-2.25 (m, 8H), 2.22-2.08 (m, 4H), 1.87-1.78 (m, 2H), 1.73-1.64 (m, 2H), 1.63-1.52 (m, 2H), 1.51-1.31 (m, 42H), 1.31-1.14 (m, 4H); FT MS ESI m/z [M+Na$^+$] 1610.84

16.12 Preparation of **84-64**

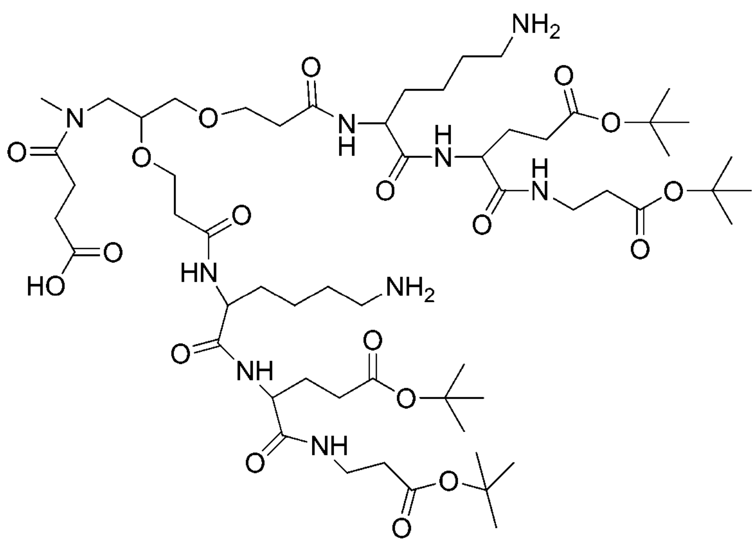

**84-38** (1 g, 0.6293 mmol) was added to a hydrogenation reactor. 10% Pd/C catalyst (0.10 g) was added, then methanol (30 mL) was added for dissolution. The reactor was charged with hydrogen gas to a pressure of 300 psi, and the mixture was reacted under stirring at room temperature overnight. After the reaction was completed, the reaction mixture was filtered through a Büchner funnel with Celite, and the filter cake was washed with methanol (3 mL × 3). The filtrate was placed in a 500 mL round-bottom flask, and rotary evaporated under reduced pressure. The filtrate was collected, then toluene was added to remove methanol by azeotropic distillation, and the mixture was dried to provide 0.68 g of the product. $^1$H-NMR (600 MHz, DMSO-$d_6$) δ 12.02(s, 1H),8.07-7.81 (m, 6H), 4.27-4.14 (m,4H), 3.77-3.47 (m, 6H), 3.47-3.24 (m, 5H), 3.21-3.08(m, 8H),3.06-2.84(m, 3H),2.84-2.62 (m, 3H), 2.61-2.45 (m, 3H), 2.44-2.25 (m, 8H), 2.22-2.08 (m, 4H),1.87-1.78 (m,2H), 1.73-1.64 (m,2H), 1.63-1.52 (m, 2H), 1.51-1.31 (m, 42H), 1.31-1.14 (m, 4H)

16.13 Preparation of **84-73**

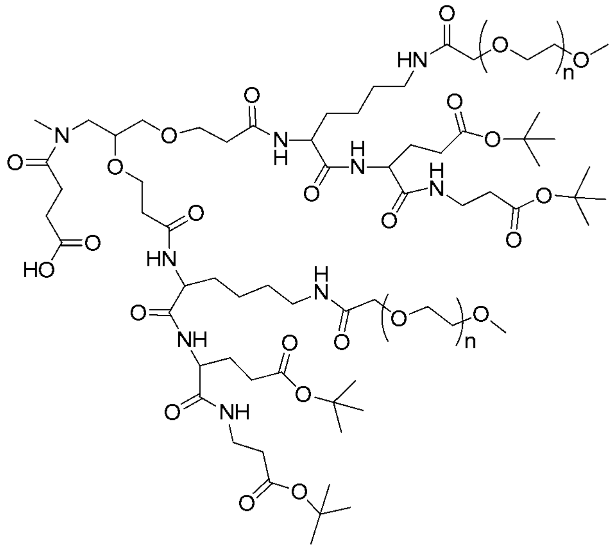

**84-64** (0.17 g, 0.1408 mmol) was dissolved in DMF (15 mL) in a 250 mL flask, and the mixture was allowed to stir at -5°C for 10 minutes. DIEA (2.2 mL, 13.216 mmol) was then slowly added dropwise. Stirring was continued at low temperature for 30 minutes. M-SCM-10K (3 g, 0.2816 mmol, purchased from Jenkem, Lot Number: ZZ390P163) was then added. The reaction was carried out under stirring in the dark at low speed at room temperature for one week. After the reaction was completed, methyl tert-butyl ether (200 mL) and n-hexane (70 mL) were added for precipitation. A solid precipitated and was filtered. The filter cake was washed with methyl tert-butyl ether (40 mL × 3). The filter cake was collected, and the obtained solid product was dissolved in a mixed solvent of dichloromethane/methanol. The resulting product was loaded by dry method, and subjected to column chromatography using 0-1% ammonia water/5-8% methanol/dichloromethane as the eluent. The product component was collected, concentrated, and dried in a vacuum oven to provide 2.8 g of the product. $^1$H-NMR (600 MHz, DMSO-$d_6$) δ 12.02(s, 1H),8.07-7.81 (m, 6H),7.68-7.61(m, 2H), 4.27-4.14 (m,4H), 3.77-3.41 (m, 1867H), 3.41-3.24 (m, 2H), 3.21-3.08(m, 8H),3.06-2.84(m, 3H),2.84-2.62 (m, 3H), 2.61-2.45 (m, 3H), 2.44-2.25 (m, 8H), 2.22-2.08 (m, 4H),1.87-1.78 (m,2H), 1.73-1.64 (m, 2H), 1.63-1.52 (m, 2H), 1.51-1.31 (m, 42H), 1.31-1.14 (m, 4H)

16.14 Preparation of **84-79**

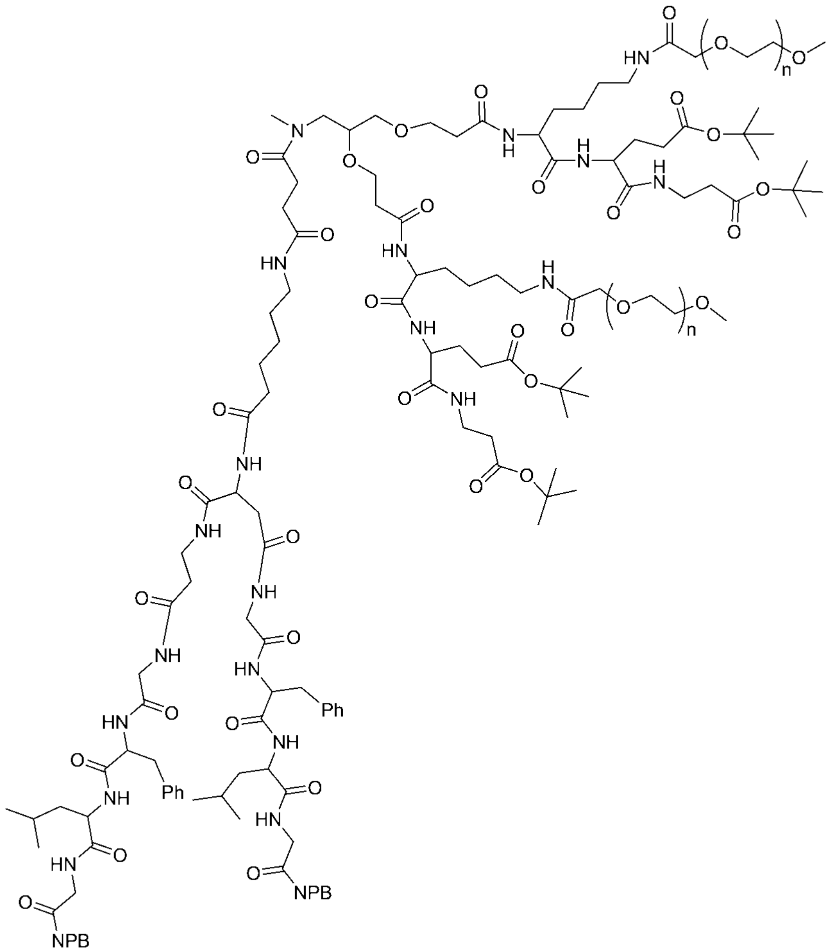

**84-73** (2.8 g, 0.1259 mmol), **84-76** (0.15 g, 0.1474 mmol), HBTU (0.06 g, 0.1701 mmol), HOBT (0.02 g, 0.1701 mmol) and DMF (7 mL) were dissolved in a 50 mL flask under stirring. The reaction flask was placed at -5°C, and the mixture was stirred for approximately 20 minutes. DIEA (8.8 mL, 68.426 mmol) was then slowly added dropwise. After the dropwise addition was completed, the reaction was carried out under stirring at -5°C for 1 hour. The flask was taken out and brought to room temperature, and the mixture was stirred. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered by suction to provide a solid product, the obtained solid product was dissolved in a mixture of dichloromethane and methanol, loaded by dry method, and subjected to column chromatography using 1% ammonia water/7% methanol/dichloromethane as the eluent. The desired product was collected, concentrated, and dried in a vacuum oven to provide 1.7 g of the product. $^1$H-NMR (600 MHz, DMSO-$d_6$) δ 9.31-9.22 (m, 2H), 8.64-8.49 (m, 2H), 8.42-8.31 (m, 2H), 8.25-8.14 (m, 2H),8.11-7.81 (m, 20H),7.68-7.61(m, 2H), 7.55-7.44 (m, 4H), 7.44-7.21 (m, 12H), 4.63-4.50 (m, 2H), 4.41-4.14 (m,6H), 3.99-3.79 (m, 5H), 3.77-3.41 (m, 1867H), 3.41-3.24 (m, 2H), 3.21-3.08(m, 8H), 3.06-2.84(m,22H), 2.84-2.62 (m, 9H), 2.61-2.45 (m, 5H), 2.44-2.25 (m, 10H), 2.22-2.08 (m, 6H), 1.98-1.84 (m, 2H),1.87-1.78 (m,2H), 1.73 -1.31 (m, 54H), 1.31-1.14 (m, 10H) ,0.89-0.74 (m, 12H)

16.15 Preparation of **84-89**

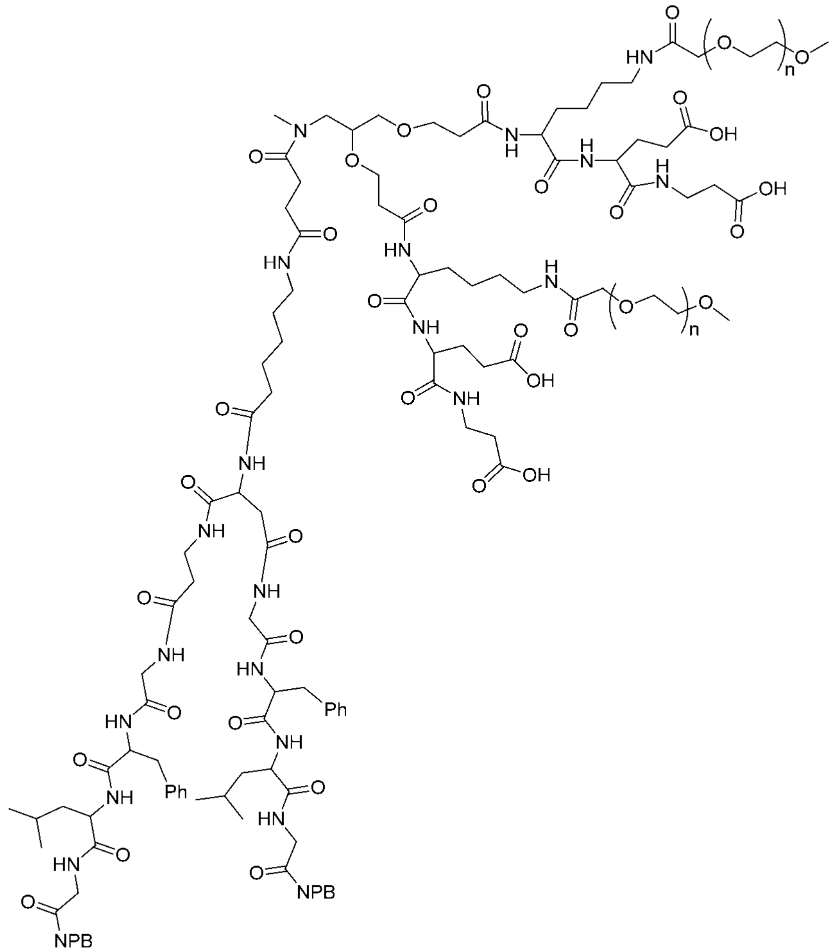

**84-79** (1.7 g, 0.0742 mmol), TFA (0.6 mL, 8.336 mmol) and dichloromethane (1 mL) were stirred and dissolved in a 50 mL flask. The reaction was allowed to proceed at room temperature until the reaction was completed. The reaction mixture was evaporated to provide a viscous oil using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide 1.09 g of the product. $^1$H-NMR (600 MHz, DMSO-$d_6$) δ12.11-12.01(m, 4H), 9.31-9.22 (m, 2H), 8.64-8.49 (m, 2H), 8.42-8.31 (m, 2H), 8.25-8.14 (m, 2H),8.11-7.81 (m, 20H),7.68-7.61(m, 2H), 7.55-7.44 (m, 4H), 7.44-7.21 (m, 12H), 4.63-4.50 (m, 2H), 4.41-4.14 (m,6H), 3.99-3.79 (m, 5H), 3.77-3.41 (m, 1867H), 3.41-3.24 (m, 2H), 3.21-3.08(m, 8H), 3.06-2.62 (m,31H), 2.61-2.45 (m, 5H), 2.44-2.25 (m, 10H), 2.22-2.08 (m, 6H), 1.98-1.84 (m, 2H), 1.87-1.78 (m, 2H), 1.73 -1.31 (m, 18H), 1.31-1.14 (m, 10H), 0.89-0.74 (m, 12H)

16.16 Preparation of **84-93**

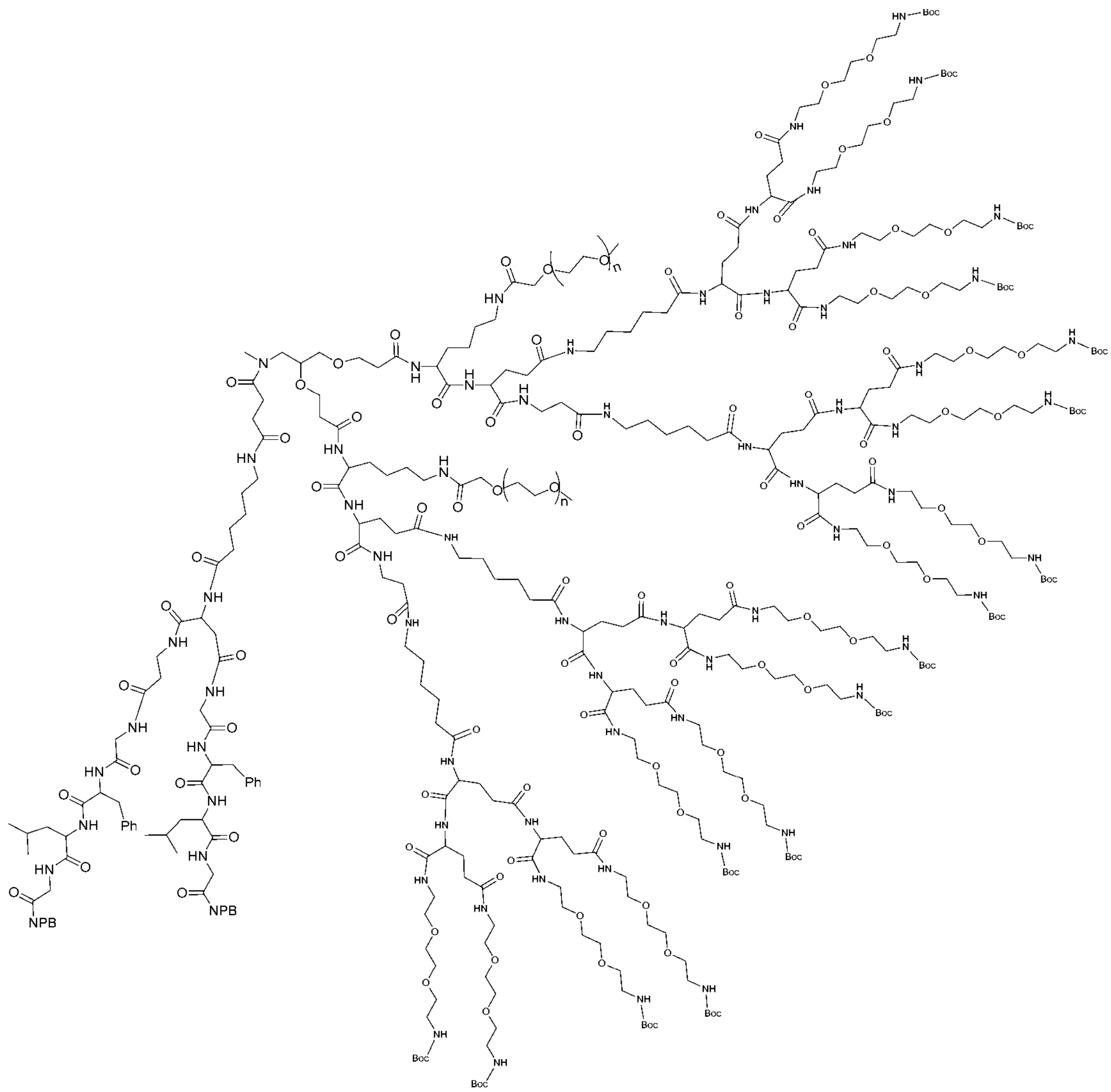

**84-89** (1.09 g, 0.0461 mmol), **76-93** (0.4 g, 0.2766 mmol), HBTU (0.1 g, 0.2766 mmol), HOBT (0.04 g, 0.2766 mmol) and DMF (10 mL) were stirred in a 100 mL flask at room temperature for approximately 20 minutes. DIEA (0.3 mL, 1.8144 mmol) was then slowly added dropwise. After the dropwise addition was completed, the reaction was continuously carried out under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product. The obtained solid product was dissolved in a mixed solvent of dichloromethane and methanol, loaded by dry method, and subjected to column chromatography using 1% ammonia water/9% methanol/dichloromethane as the eluent. The product component was collected, concentrated, and dried in a vacuum oven to provide 0.94 g of the product. $^1$H-NMR (600 MHz, DMSO-$d_6$) δ 9.31-9.22 (m, 2H), 8.64-8.49 (m, 2H), 8.42-8.31 (m, 2H), 8.25-8.14 (m, 26H),8.11-7.81 (m, 20H),7.68-7.61(m, 14H), 7.55-7.44 (m, 16H), 7.44-7.21 (m, 12H), 4.63-4.50 (m, 2H), 4.41-4.14(m,18H), 3.99-3.79(m, 5H), 3.77-3.41(m, δ (m/cm2, 1975H), 3.41-3.24 (m, 66H), 3.21-3.08 (m, 28H), 3.06-2.61 (m, 31H), 2.61-2.45 (m, 5H), 2.44-2.08 (m, 48H), 1.98-1.78 (m, 28H), 1.74-1.14 (m, 196H), 0.89-0.74 (m, 12H)

16.17 Preparation of **84-95**

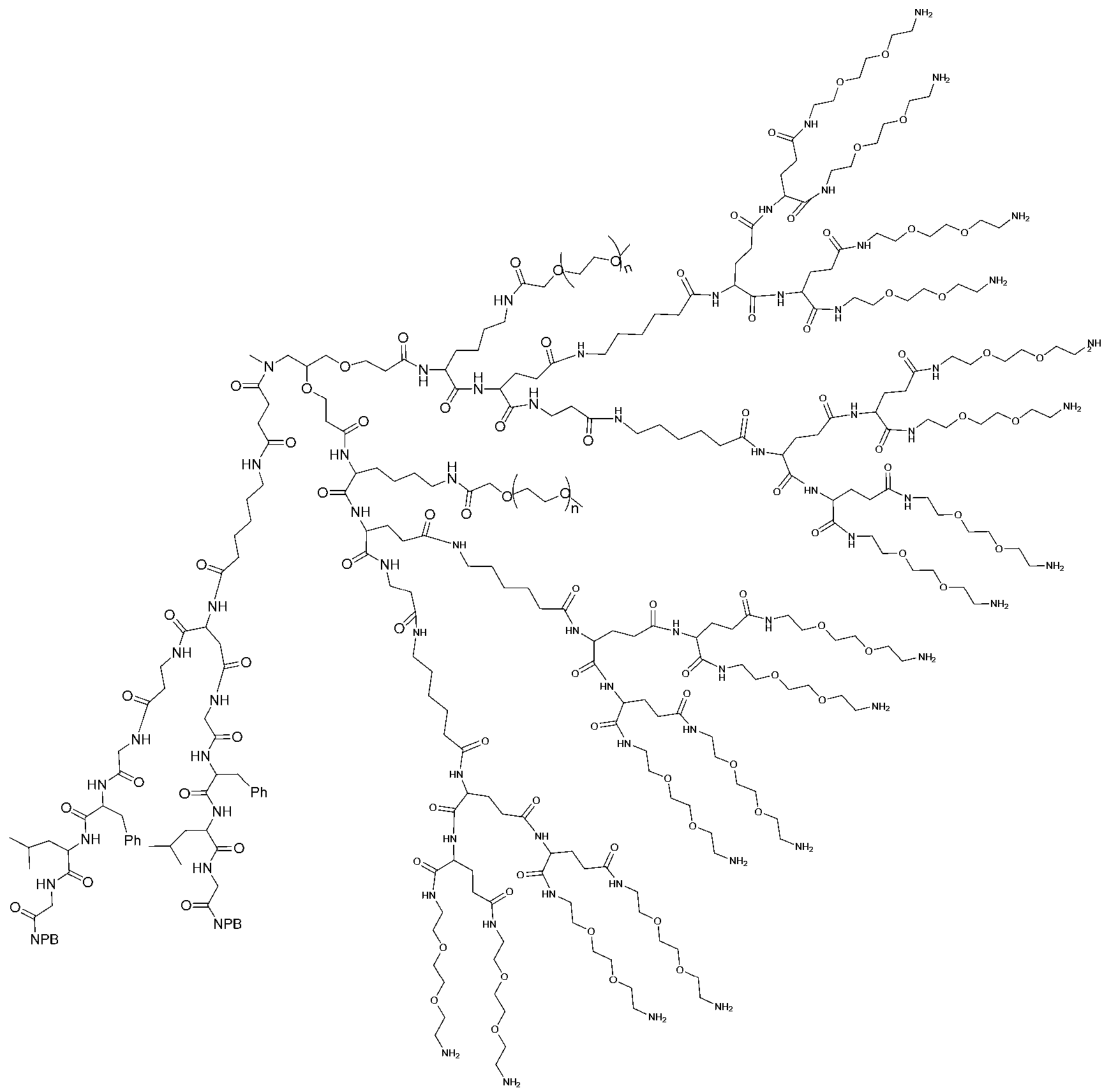

**84-93** (0.94 g, 0.032 mmol), TFA (2 mL, 28.064 mmol) and dichloromethane (2 mL) were stirred and dissolved in a 50 mL flask, and the mixture was reacted at room temperature until the reaction was completed. The reaction mixture was evaporated to provide an oil using a rotary evaporator, and methyl tert-butyl ether (60 mL) was added. A powdery solid precipitated from the reaction mixture and was filtered. The filter cake was washed with methyl tert-butyl ether (40 mL × 3), collected, and dried in a vacuum oven to provide 0.8 g of the product. $^1$H-NMR (600 MHz, DMSO-$d_6$) δ 9.31-9.22 (m, 2H), 8.64-8.49 (m, 2H), 8.42-8.31 (m, 2H), 8.25-8.14(m,26H),8.11-7.81 (m,20H),7.68-7.61(m, 14H),7.44-7.21(m, 12H),4.63-4.50(m,2H),4.41 -4.14(m,18H), 3.99-3.79(m, 5H), 3.77-3.41(m, 1975H),3.41-3.24(m,98H), 3.24-3.06(m, 48H), 3.06-2.61 (m, 31H), 2.61-2.45 (m, 5H), 2.44-2.08 (m, 48H), 1.98-1.78 (m, 28H), 1.74-1.14 (m, 52H), 0.89-0.74 (m, 12H)

16.18 Preparation of **84-102**

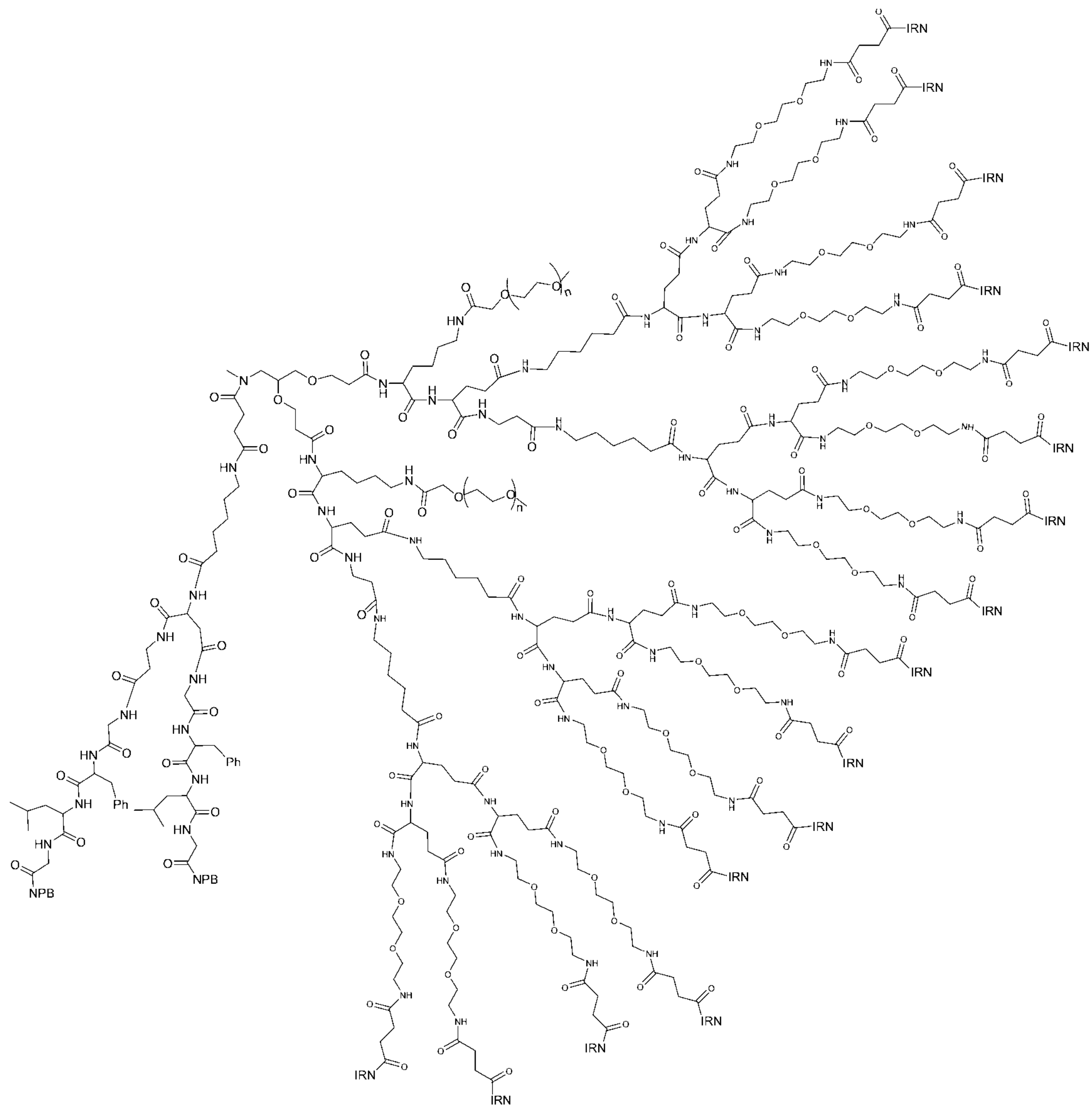

**84-95** (0.8 g, 0.0288 mmol) was dissolved in DMF (10 mL) in a 100 mL flask, and the mixture was stirred at room temperature for approximately 2 minutes. DIEA (0.6 mL, 3.4029 mmol) was then slowly added dropwise. After the dropwise addition was completed, **84-97** (1.17 g, 1.7014 mmol) was added and reacted under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product. The obtained solid product was dissolved in a mixture of dichloromethane and methanol, loaded by dry method, and subjected to column chromatography using 1% ammonia water/10% methanol/dichloromethane as the eluent. The desired product was collected, concentrated, and dried in a vacuum oven to provide 0.95 g of the product. $^1$H-NMR (600 MHz, DMSO-$d_6$) δ 9.31-9.22 (m, 2H), 8.64-8.49 (m, 2H), 8.42-8.31 (m, 2H), 8.25-8.11 (m, 42H), 8.11-7.81 (m, 36H), 7.72-7.61 (m, 30H), 7.44-7.21 (m, 28H), 6.56-6.47 (m, 16H), 5.50-5.38 (m, 16H), 5.36-5.27 (m, 32H), 4.63-4.50 (m, 2H), 4.41-3.99 (m, 50H), 3.99-3.79 (m, 5H), 3.77-3.41 (m, 1975H), 3.41-3.24 (m, 114H), 3.24-2.61 (m, 115H), 2.61-2.45 (m, 53H), 2.44-2.08 (m, 112H), 1.98-1.14 (m, 336H), 0.98-0.74 (m, 60H)

Example 17: Synthesis of Compound **88-228**

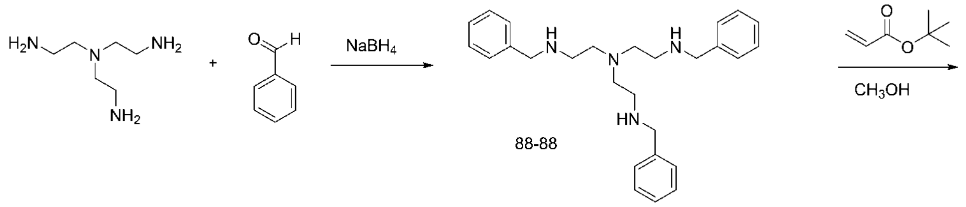


88-88

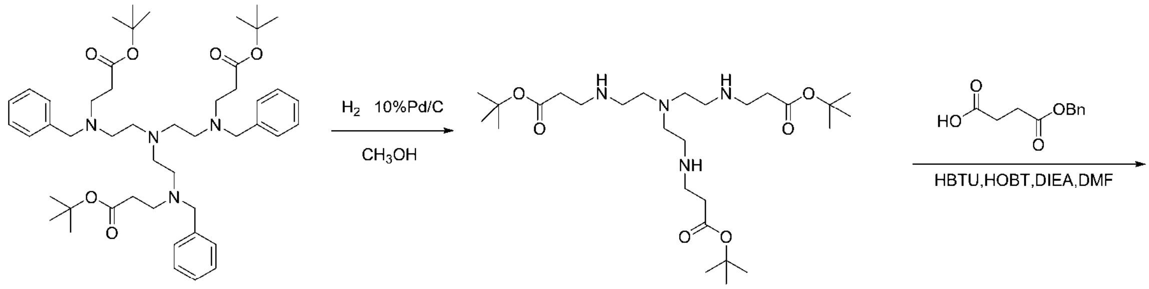


88-91

88-92

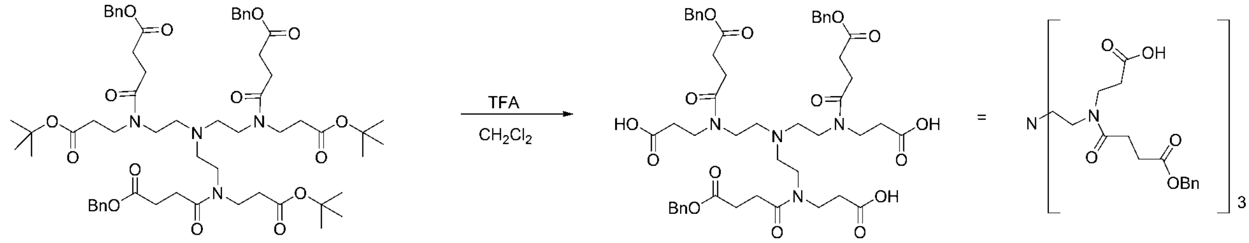


88-101

88-148

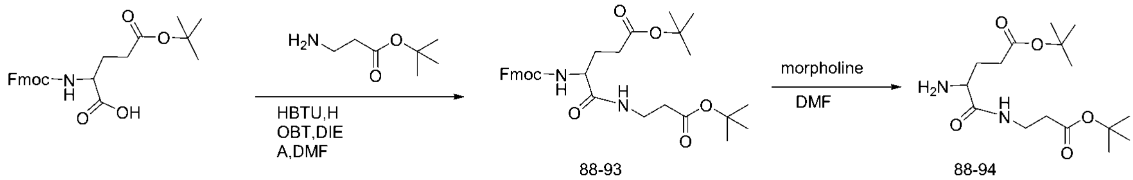


88-93

88-94

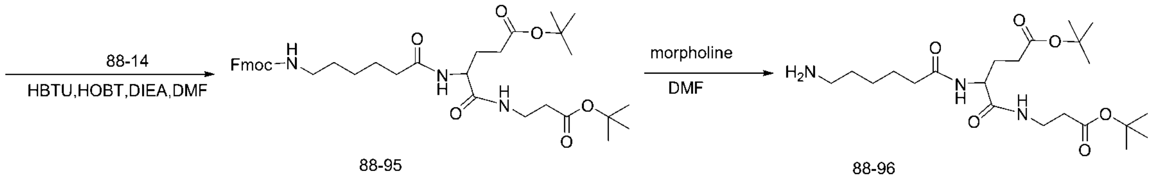


88-95

88-96

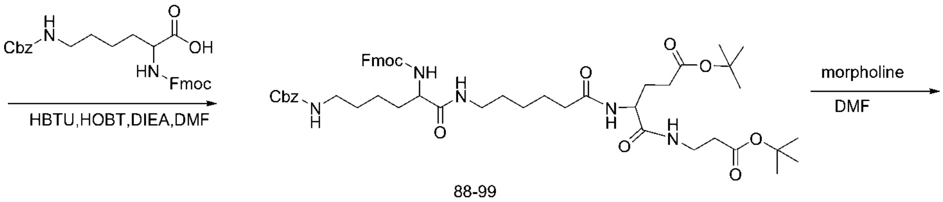


88-99

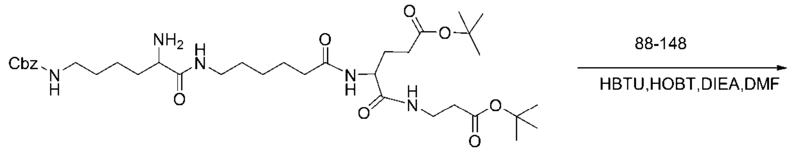


88-100

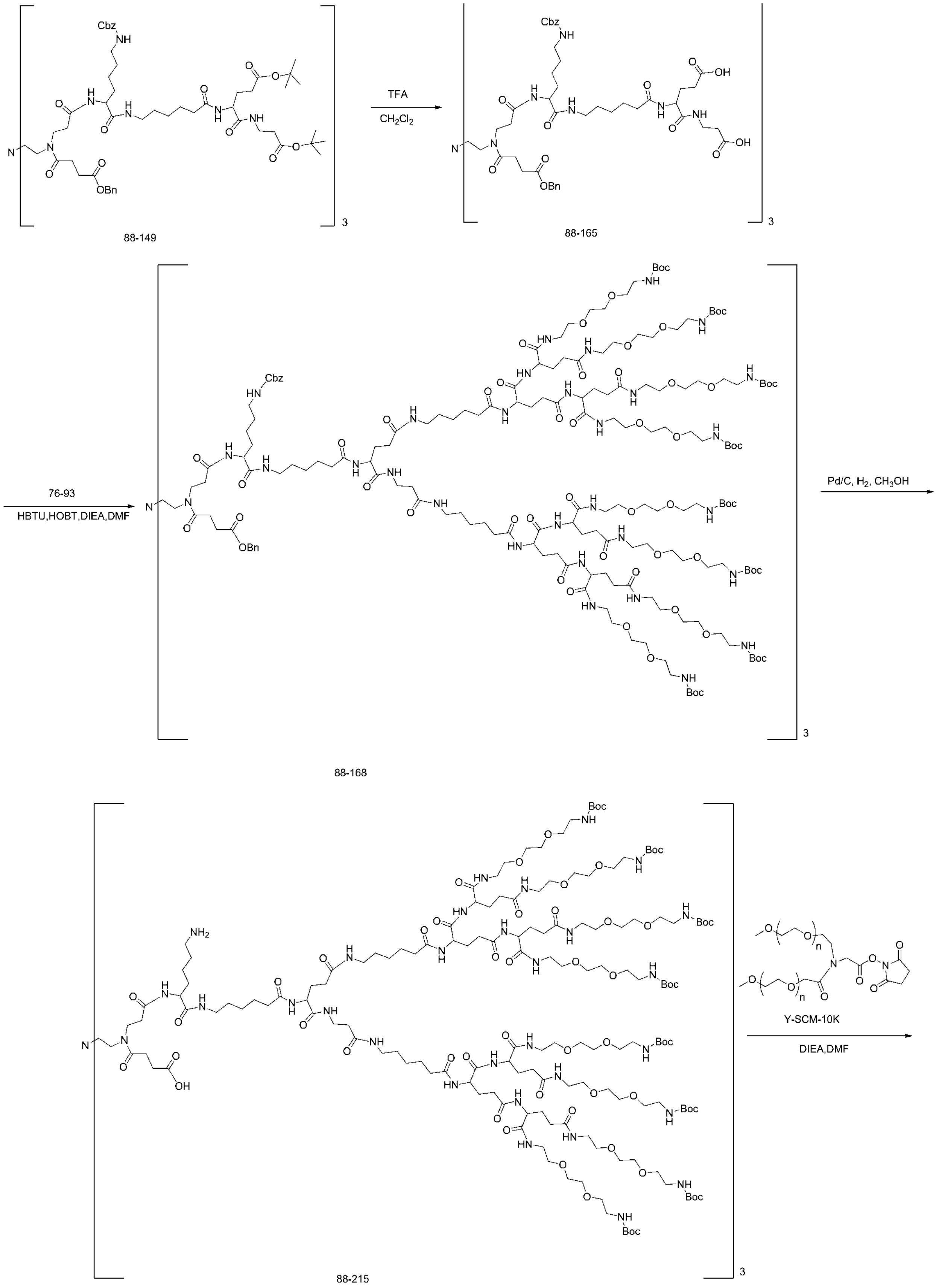

TFA
$CH_2Cl_2$
88-149
88-165
76-93
HBTU,HOBT,DIEA,DMF
Pd/C, $H_2$, $CH_3OH$
88-168
Y-SCM-10K
DIEA,DMF
88-215

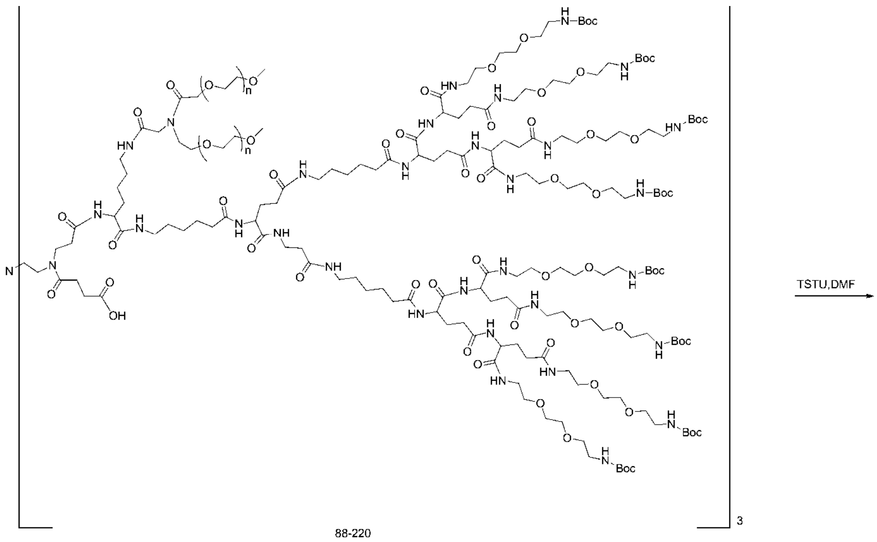


88-220

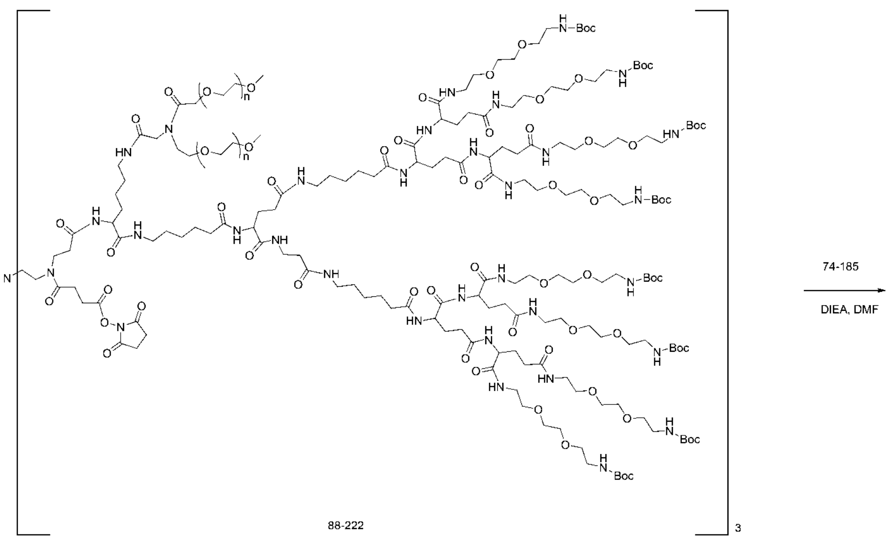


88-222

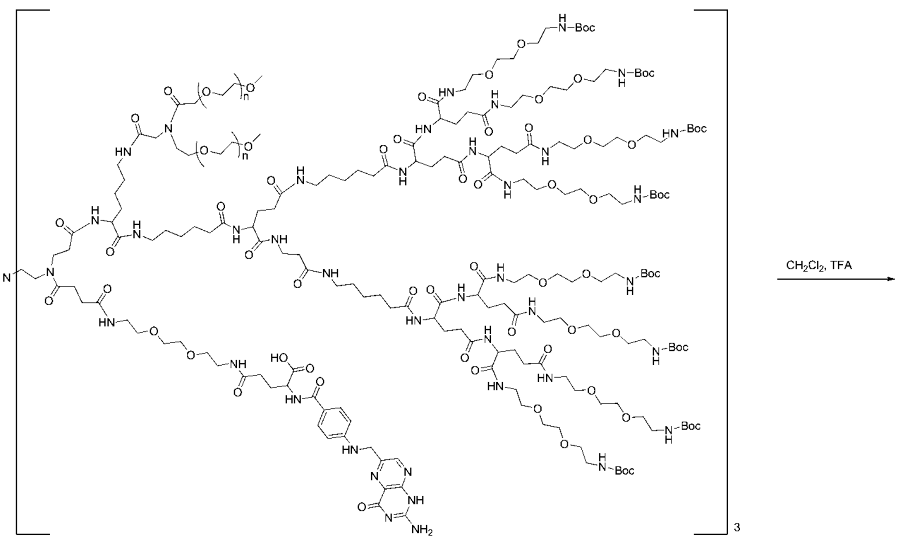


88-226

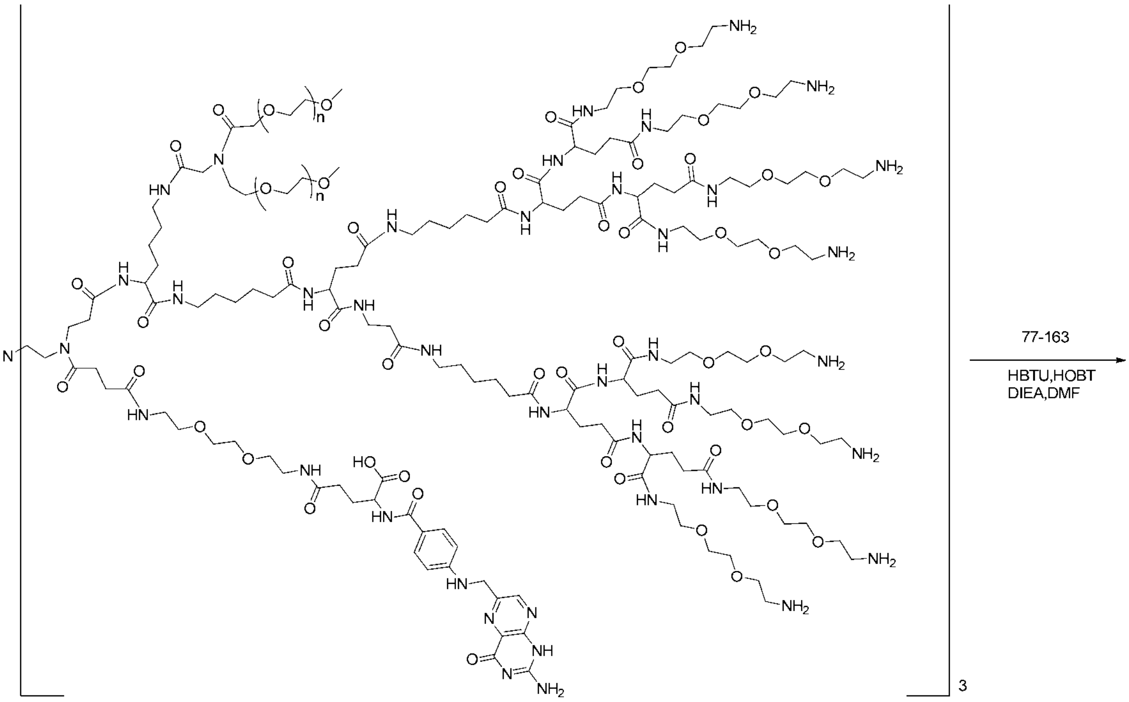


88-227

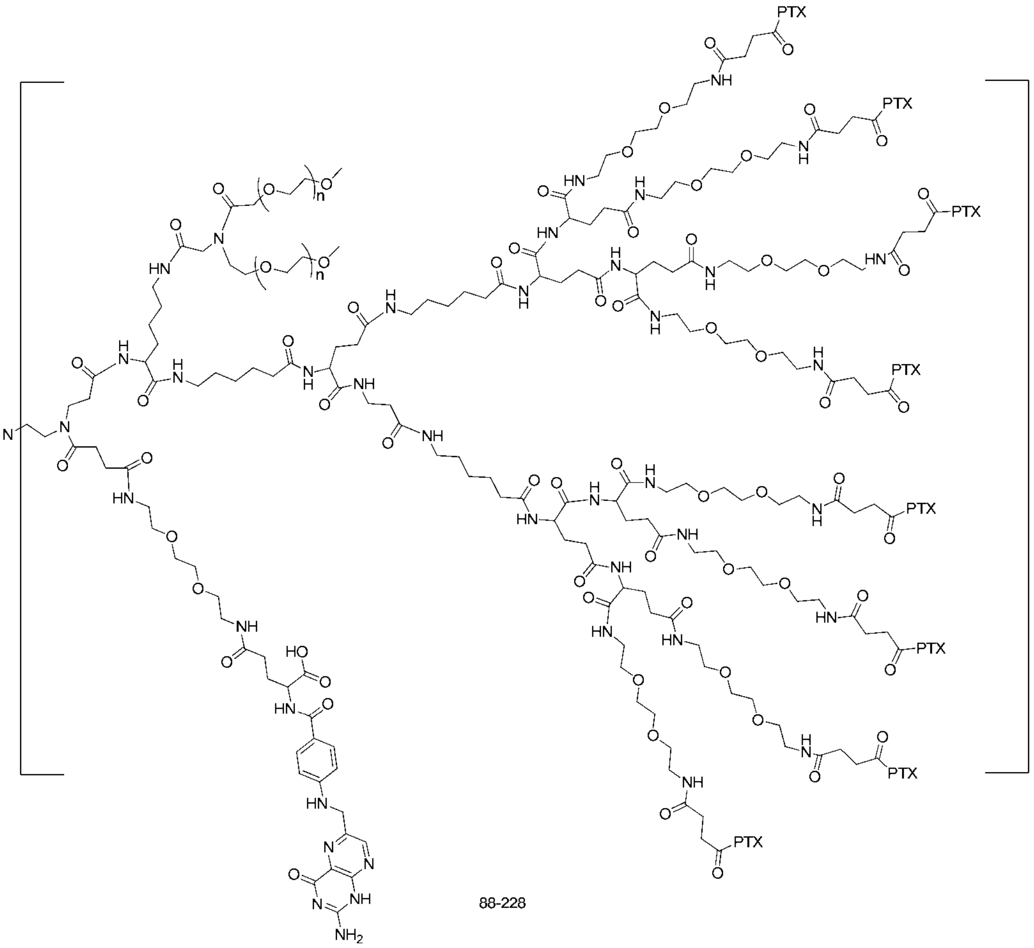

88-228

17.1 Preparation of **88-88**

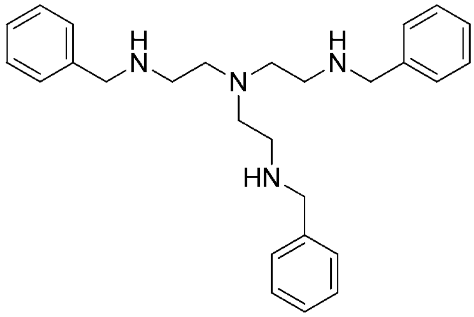

Tris(2-aminoethyl)amine (4.0 g, 27.35 mmol) was added to a 500 mL round-bottom flask, and then dichloromethane (40 mL) was added for dissolution with the assistance of ultrasonication. Anhydrous sodium sulfate (12.82 g, 90.25 mmol) and benzaldehyde (8.99 g, 84.79 mmol) were then added. The mixture was reacted under stirring at room temperature for 18 h. The filtrate was concentrated under reduced pressure. Methanol (35 mL) was added under nitrogen protection. The mixture was cooled and maintained at 0°C. Sodium borohydride (4.14 g, 109.4 mmol) was then added in batches with small amounts. After the reaction was carried out for 1 h, purified water (200 mL) was added, then extracted with ethyl acetate (300 mL). The organic phase was separated and washed with 1N HCl (20 mL × 2). The organic phase was collected, dehydrated over anhydrous magnesium sulfate, then 200-300 mesh silica gel powder was added, and the mixture was evaporated to dryness. The resulting product was loaded by dry method, and subjected to column chromatography using 3% methanol/dichloromethane as the eluent. The desired product was collected, concentrated, and evaporated to dryness to provide 4.7 g of the product with a yield of 41%. $^1$H-NMR (600 MHz, DMSO-$d_6$) δ 7.39-7.18 (m, 15H), 3.78 (m, 6H), 2.71 (m, 6H), 2.62 (m, 6H), 1.89 (m, 3H); ESI [M+H$^+$] 417.29

17.2 Preparation of **88-91**

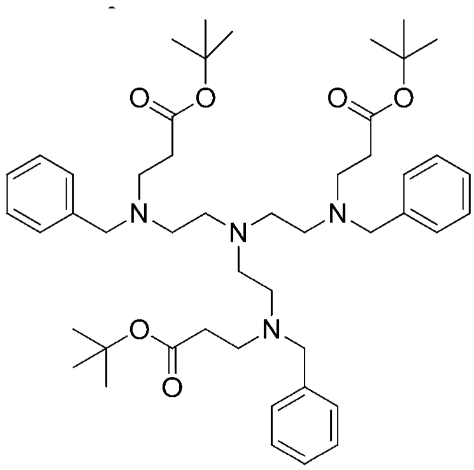

**88-88** (2.2 g, 5.28 mmol) and tert-butyl acrylate (3.8 g, 26.4 mmol) were added to a 100 mL round-bottom flask, then methanol (15 mL) was added for dissolution with the assistance of ultrasonication, and reacted under stirring at room temperature. After the reaction was completed, 200-300 mesh silica gel powder was added and evaporated to dryness. The resulting product was loaded by dry method, and subjected to column chromatography using 50% ethyl acetate/petroleum ether as the eluent. The desired product was collected, concentrated, and evaporated to dryness to provide 3.1 g of the product with a yield of 73.3%. $^1$H-NMR (600 MHz, DMSO-$d_6$) δ 7.38-7.14 (m, 15H), 3.53 (s, 6H), 2.74 (m, 6H), 2.43 (m, 12H), 2.36 (m, 6H), 1.46 (m, 27H); ESI [M+H$^+$] 801.55

17.3 Preparation of **88-92**

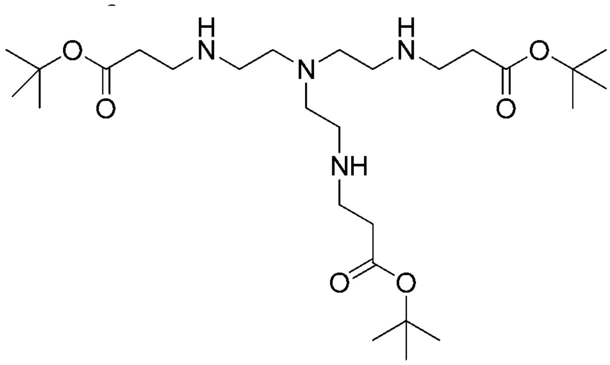

Raw material **88-91** (3.1 g, 3.87 mmol) and 10% Pd/C catalyst (0.11 g) were added to a hydrogenation reactor, then methanol (15 mL) was added for dissolution. The hydrogenation reactor was sealed, evacuated with a water pump, and then filled with hydrogen at approximately 2 MPa. This process was repeated three times. Finally, the hydrogen pressure was adjusted to 2 MPa. The reaction was carried out under stirring at room temperature for 24 hours. After the reaction was completed, the mixture was filtered through a Büchner funnel containing filter paper. The reaction apparatus was rinsed with DMF (5 mL × 3). The filtrate was collected and concentrated to provide 1.5 g of the product.

17.4 Preparation of **88-101**

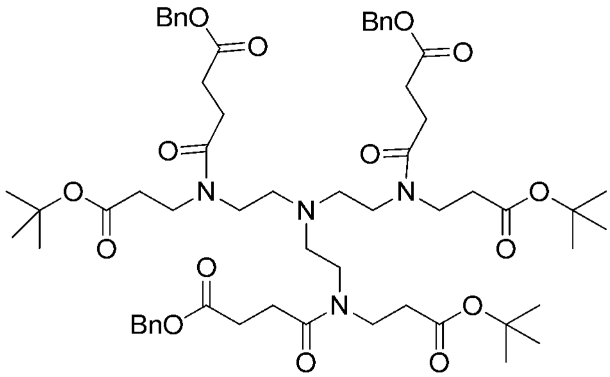

**88-92** (1.5 g, 2.83 mmol), mono-benzyl succinate (1.94 g, 9.33 mmol), HBTU (3.54 g, 9.33 mmol) and HOBT (1.26 g, 9.33 mmol) were added to a 100 mL round-bottom flask, then DMF (15 mL) was added for dissolution with the assistance of ultrasonication, and the mixture was stirred at 0°C for 1 minute. DIEA (3.1 mL, 18.66 mmol) was then added dropwise. The flask was taken out, and the reaction was carried out under stirring at room temperature. After the reaction was completed, the mixture was extracted with saturated sodium chloride solution (100 mL) and ethyl acetate (100 mL). The organic phase was separated, and the aqueous phase was further extracted with ethyl acetate (100 mL × 2). The organic phases were combined, concentrated, and evaporated to dryness, then 200-300 mesh silica gel powder was added, and the mixture was evaporated to dryness. The resulting product was loaded by dry method, and subjected to column chromatography using 35% ethyl acetate/petroleum ether as the eluent. The desired product was collected, concentrated, and evaporated to dryness to provide 2.8 g of the product. FT-MS ESI m/z [$M+H^+$] 1101.59961, [$M+Na^+$] 1123.57092

17.5 Preparation of **88-148**

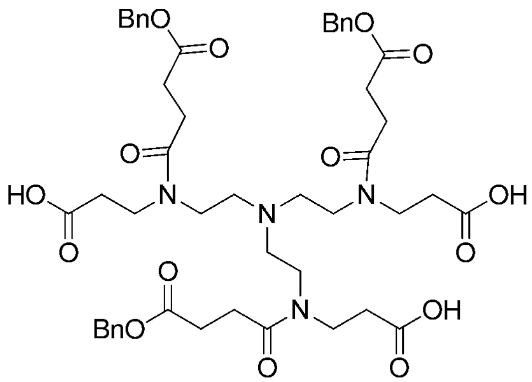

**88-101** (1.2 g, 1.08 mmol) was placed in a 500 mL round-bottom flask, then TFA (15 mL) was added, and the mixture was reacted under stirring at room temperature. After the reaction was completed, an appropriate amount of dichloromethane was added, and the dichloromethane and TFA were removed by rotary evaporation under reduced pressure. Dichloromethane was added again, and then rotary evaporated. This process was repeated several times until the product became a viscous oil. An appropriate amount of dichloromethane was added to the oil for dissolution, followed by the addition of DIEA to adjust the pH to alkaline, concentrated under reduced pressure and evaporated to dryness to provide 0.9 g of the product. FT MS ESI m/z [M+H$^+$] 933.40784, [M+Na$^+$] 955.38971

17.6 Preparation of **88-93**

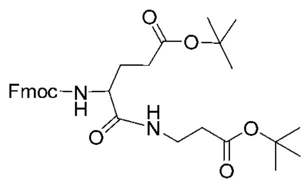

Fmoc-Glu(OtBu)-OH (6.0 g, 14.1 mmol), tert-butyl alanine (2.82 g, 15.51 mmol), HBTU (5.88 g, 15.51 mmol) and HOBT (2.09 g, 15.51 mmol) were placed in a 100 mL round-bottom flask, then DMF (30 mL) was added for dissolution with the assistance of ultrasonication, and the contents were stirred at 0°C for 1 minute. DIEA (4.7 mL, 28.2 mmol) was then added dropwise. The flask was taken out, and the reaction was carried out under stirring at room temperature. After the reaction was completed, purified water (100 mL) and ethyl acetate (100 mL) were added for extraction. The organic phase was separated, and the aqueous phase was further extracted twice with ethyl acetate (100 mL × 2). The organic phases were combined and washed with saturated sodium chloride solution (50 mL × 2), and concentrated. Then 200-300 mesh silica gel powder was added, and the mixture was evaporated to dryness. The resulting product was loaded by dry method, and subjected to column chromatography using 35% ethyl acetate/petroleum ether as the eluent. The desired product was collected, concentrated, and evaporated to dryness to provide 7.79 g of the product.

17.7 Preparation of **88-94**

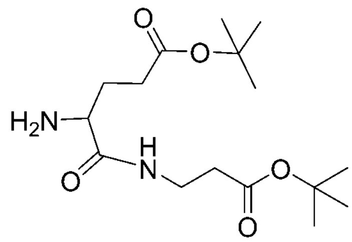

**88-93** (7.79 g, 314.1 mmol) was placed in a 500 mL round-bottom flask, DMF (100 mL) was added for dissolution with the assistance of ultrasonication, then morpholine (18.4 mL, 211.5 mmol) was added, and the mixture was stirred at

room temperature for 2 h. After the reaction was completed, saturated NaCl solution (200 mL) and ethyl acetate (200 mL) were added for extraction. After standing for layer separation, the organic phase was collected; the aqueous phase was further extracted with ethyl acetate (200 mL × 3). The organic phases were combined and evaporated to dryness to provide 4.05 g of a solid product.

17.8 Preparation of **88-95**

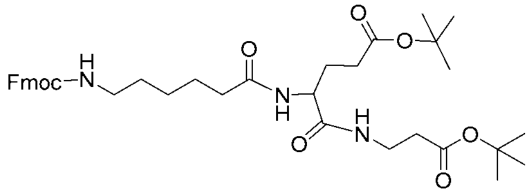

**88-14** (2.73 g, 7.72 mmol), **88-94** (2.8 g, 8.49 mmol), HBTU (3.22 g, 8.49 mmol) and HOBT (1.15 g, 8.49 mmol) were added to a 100 mL round-bottom flask, then DMF (30 mL) was added for dissolution with the assistance of ultrasonication. The mixture was stirred at 0°C for 1 minute, and then DIEA (2.6 mL, 15.44 mmol) was added dropwise. The flask was taken out, and the reaction was carried out under stirring at room temperature. After the reaction was completed, saturated NaCl solution (200 mL) was added, and the mixture was extracted with ethyl acetate (200 mL). After standing for layer separation, the organic phase was collected, and the aqueous phase was further extracted with ethyl acetate (200 mL × 3). The organic phases were combined and evaporated to dryness to provide 5.14 g of a solid product.

17.9 Preparation of **88-96**

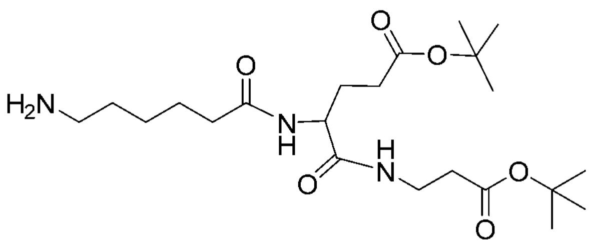

To a flask containing **88-95** (5.14 g, 7.72 mmol), DMF was added for dissolution with the assistance of ultrasonication, then morpholine (2.7 mL, 30 mmol) was added, and the mixture was reacted under stirring at room temperature for 2 h. After the reaction was completed, saturated NaCl solution (200 mL) and ethyl acetate (200 mL) were added for extraction. After standing for layer separation, the organic phase was collected. The aqueous phase was further extracted with ethyl acetate (200 mL × 3). The organic phases were combined and evaporated to dryness to provide 3.2 g of a solid product.

17.10 Preparation of **88-99**

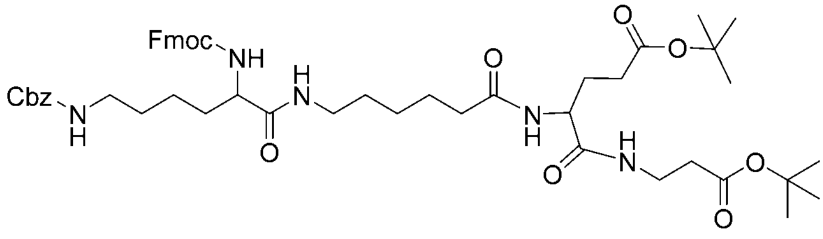

**88-96** (3.2 g, 7.21 mmol), N'-Fmoc-N-benzyloxycarbonyl-L-lysine (3.31 g, 6.55 mmol), HBTU (2.74 g, 7.21 mmol)

and HOBT (0.97 g, 7.21 mmol) were placed in a 100 mL round-bottom flask, then DMF (30 mL) was added for dissolution with the assistance of ultrasonication. The mixture was stirred at 0°C for 1 minute, and DIEA (1.2 mL, 13.16 mmol) was then added dropwise. The flask was taken out, and the reaction was carried out under stirring at room temperature. After the reaction was completed, methyl tert-butyl ether (200 mL) was added for precipitation. A solid precipitated and was filtered, the filter cake was dissolved, then 200-300 mesh silica gel powder was added, and the mixture was evaporated to dryness. The resulting product was loaded by dry method, and subjected to column chromatography using 9% methanol/dichloromethane as the eluent. The desired product was collected, concentrated, and evaporated to dryness to provide 4.65 g of the product with a yield of 76.5%.

17.11 Preparation of **88-100**

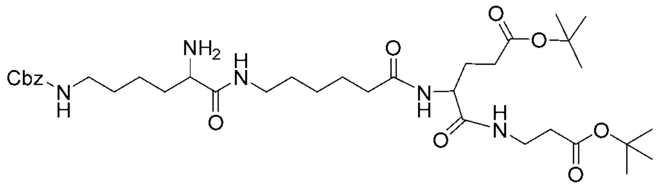

To a flask containing **88-99** (4.65 g, 5.01 mmol), DMF (100 mL) was added for dissolution with the assistance of ultrasonication, then morpholine (8.7 mL, 100 mmol) was added, and the mixture was reacted under stirring at room temperature for 2 h. After the reaction was completed, methyl tert-butyl ether (200 mL) was added for precipitation. A solid precipitated and was filtered. The filter cake was dissolved, then 200-300 mesh silica gel powder was added, and the mixture was evaporated to dryness. The resulting product was loaded by dry method, and subjected to column chromatography using 9% methanol/dichloromethane as the eluent. The product was collected, concentrated, and evaporated to dryness to provide 2.62 g. ESI [M+H$^+$] 706.43

17.12 Preparation of **88-149**

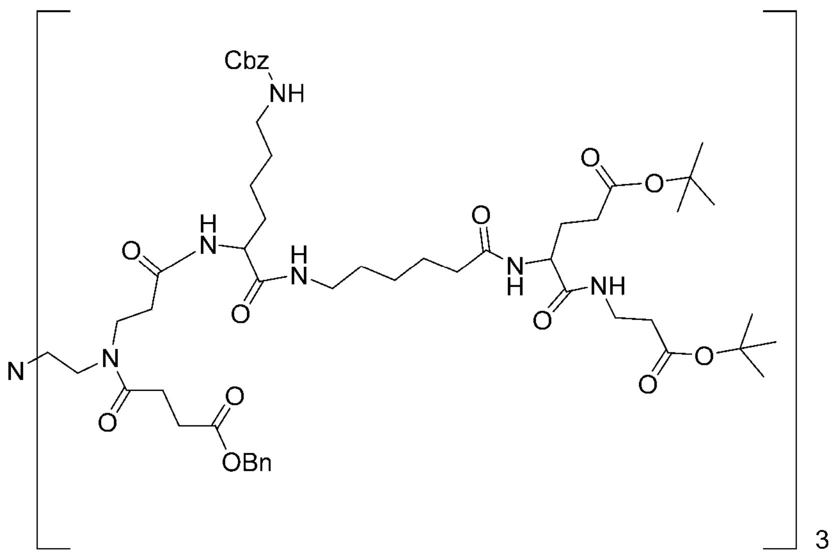

**88-148** (0.9 g, 0.96 mmol), **88-100** (2.24 g, 3.18 mmol), HBTU (1.21 g, 3.18 mmol) and HOBT (0.40 g, 3.18 mmol) were added to a 100 mL round-bottom flask, then DMF (15 mL) was added for dissolution with the assistance of ultrasonication, and stirred at 0°C for 1 minute, DIEA (1.9 mL, 11.57 mmol) was then added dropwise. The flask was taken out, and the reaction was carried out under stirring at room temperature. After the reaction was completed, methyl tert-butyl ether (200 mL) was added for precipitation, a solid precipitated and was filtered. The filter cake was dissolved in a methanol/dichloromethane mixed solvent, then 200-300 mesh silica gel powder was added, and the mixture was evaporated to dryness. The resulting product was loaded by dry method, and subjected to column chromatography

using 9% methanol/ dichloromethane as the eluent. The desired product was collected, concentrated, and evaporated to dryness to provide 1.1 g of the product. $^1$H-NMR (600 MHz, DMSO-$d_6$) δ 8.13-8.03 (m, 2H), 7.97-7.86 (m, 10H), 7.35-7.27 (m, 30H), 7.23-7.17 (m, 3H), 5.06-5.03 (m, 6H), 5.01-4.96 (m, 6H), 4.24-4.13 (m, 6H), 3.59-3.38 (m, 9H), 3.31-3.12 (m, 14H), 3.08-2.88 (m, 13H), 2.66-2.53 (m, 16H), 2.40-2.28 (m, 10H), 2.20-2.13 (m, 6H), 2.12-2.04 (m, 6H), 1.85-1.77 (m, 3H), 1.69-1.62 (m, 3H), 1.59-1.52 (m, 3H), 1.49-1.42 (m, 9H), 1.39-1.36 (m, 54H), 1.26-1.16 (m, 18H); ESI [M+H$^+$] 2995.72

17.13 Preparation of **88-165**

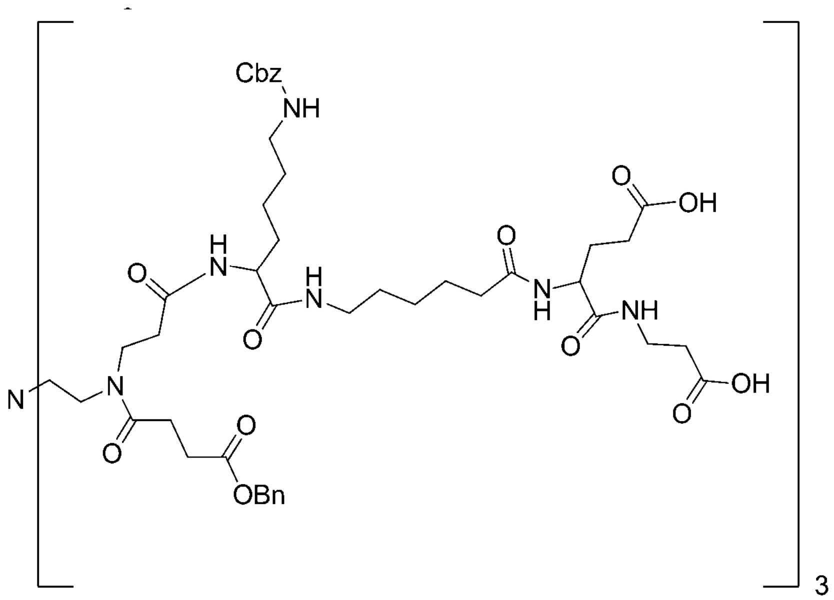

**88-149** (1.1 g, 0.36 mmol) was placed in a 500 mL round-bottom flask, then TFA (2.5 mL, 33.04 mmol) was added, and the mixture was reacted under stirring at room temperature. After the reaction was completed, an appropriate amount of dichloromethane was added, the dichloromethane and TFA were removed by rotary evaporation under reduced pressure, then dichloromethane was added and evaporated under reduced pressure. This process was repeated several times until the product became a viscous oil. An appropriate amount of dichloromethane was added for dissolution, and the pH was adjusted to alkaline by adding DIEA. The mixture was concentrated under reduced pressure, and evaporated to dryness to provide 0.97 g of the product. $^1$H-NMR (600 MHz, DMSO-$d_6$) δ 13.01-11.52 (m, 5H), 8.15-8.09 (m, 2H), 8.03-7.82 (m, 10H), 7.39-7.27 (m, 30H), 7.25-7.18 (m, 3H), 5.08-4.96 (m, 12H), 4.24-4.17 (m, 6H), 3.33-3.24 (m, 9H), 3.24-3.15 (m, 5H), 3.06-3.01 (m, 3H), 2.98-2.90 (m, 9H), 2.71-2.63 (m, 6H), 2.59-2.53 (m, 6H), 2.40-2.34 (m, 8H), 2.21-2.16 (m, 6H), 2.13-2.06 (m, 6H), 1.88-1.80 (m, 3H), 1.72-1.64 (m, 3H), 1.61-1.53 (m, 3H), 1.52-1.40 (m, 12H), 1.40-1.32 (m, 12H), 1.25-1.17 (m, 12H)

17.14 Preparation of **88-168**

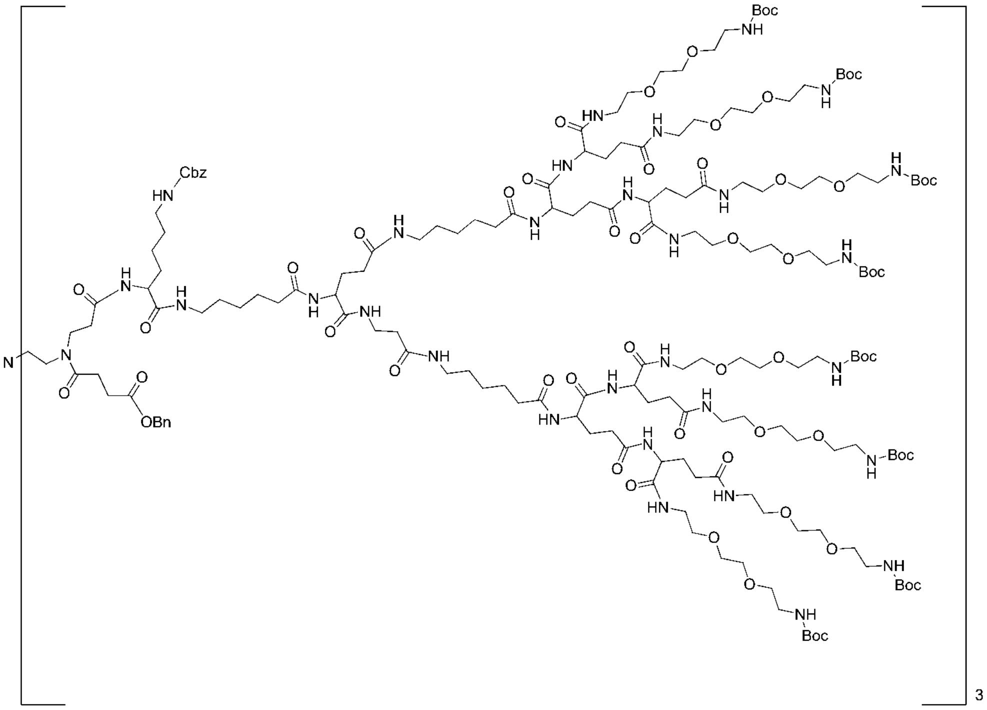

**88-165** (0.23 g, 0.089 mmol), **76-93** (2.62 g, 6.72 mmol), HBTU (0.24 g, 0.62 mmol) and HOBT (0.09 g, 0.62 mmol) were added to a 100 mL round-bottom flask, then DMF (15 mL) was added for dissolution with the assistance of ultrasonication. The mixture was stirred at 0°C for 1 minute, and DIEA (0.4 mL, 2.14 mmol) was then added dropwise. The flask was taken out, and the reaction was carried out under stirring at room temperature. After the reaction was completed, methyl tert-butyl ether (200 mL) was added for precipitation. A solid precipitated and was filtered. The filter cake was dissolved, then 200-300 mesh silica gel powder was added, and the mixture was evaporated to dryness. The resulting product was loaded by dry method, and subjected to column chromatography using 9% methanol/91% dichloromethane as the eluent. The product was collected, concentrated, and evaporated to dryness to provide 0.87 g of the product. $^1$H-NMR (600 MHz, DMSO-$d_6$) δ 8.34-7.74 (m, 67H), 7.39-7.26 (m, 30H), 7.23-7.12 (m, 3H), 5.10-4.93 (m, 12H), 4.25-4.05 (m, 25H), 3.54-3.45 (m, 107H), 3.22-3.13 (m, 46H), 3.10-2.99 (m, 65H), 2.19-2.04 (m, 67H), 1.36 (s, 216H)

17.15 Preparation of **88-215**

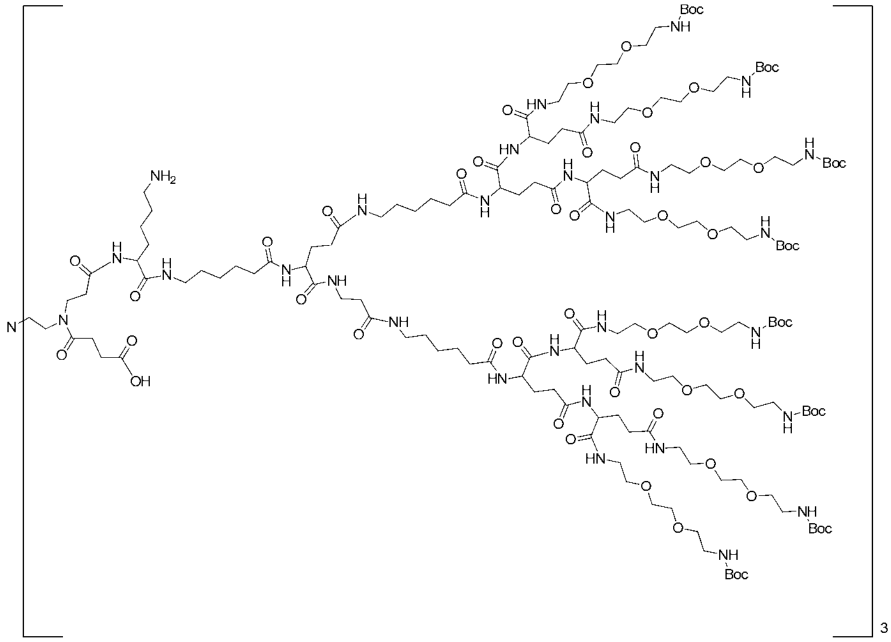

**88-214** (0.7 g, 0.42 mmol) was added to a hydrogenation apparatus, methanol (20 mL) was added for dissolution with the assistance of ultrasonication, and then 10% Pd/C catalyst (25 mg) was added. The air in the hydrogenation apparatus was replaced with hydrogen three times, the hydrogen pressure was then maintained at 1.6 MPa. The reaction was allowed to proceed under magnetic stirring overnight. The reaction was monitored by using TLC plate. After the reaction was completed, the mixture was filtered through Celite, and the filtrate was taken for later use.

17.16 Preparation of **88-220**

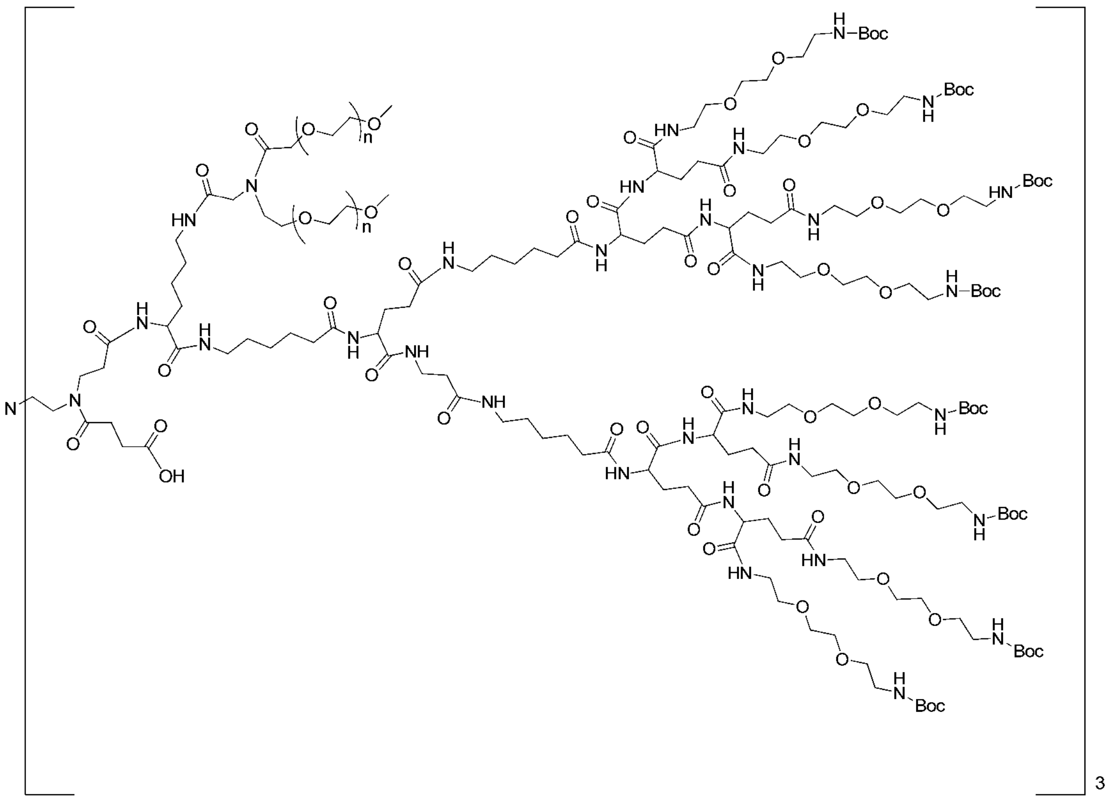

**88-215** (0.6 g, 0.057 mmol) was placed in a 500 mL round-bottom flask, then ultra-dry DMF (15 mL) was added for dissolution with the assistance of ultrasonication, and the mixture was stirred at 0°C for 5 minutes. DIEA (0.48 mL, 2.28 mmol) was added dropwise, followed by Y-SCM-10K (1.94 g, 0.188 mmol, purchased from Jenkem). The air in the flask was replaced with nitrogen, and the reaction was carried out under stirring in the dark at low speed at 35°C. After the reaction was completed, methyl tert-butyl ether (200 mL) was added for precipitation. A solid precipitated and was filtered. The filter cake was dissolved, then 200-300 mesh silica gel powder was added, and the mixture was evaporated to dryness. The resulting product was loaded by dry method, and subjected to column chromatography using 1% ammonia/7-10% methanol/ dichloromethane as the eluent. The product was collected, concentrated, and evaporated to dryness to provide 2.0 g of the product.

17.17 Preparation of **88-222**

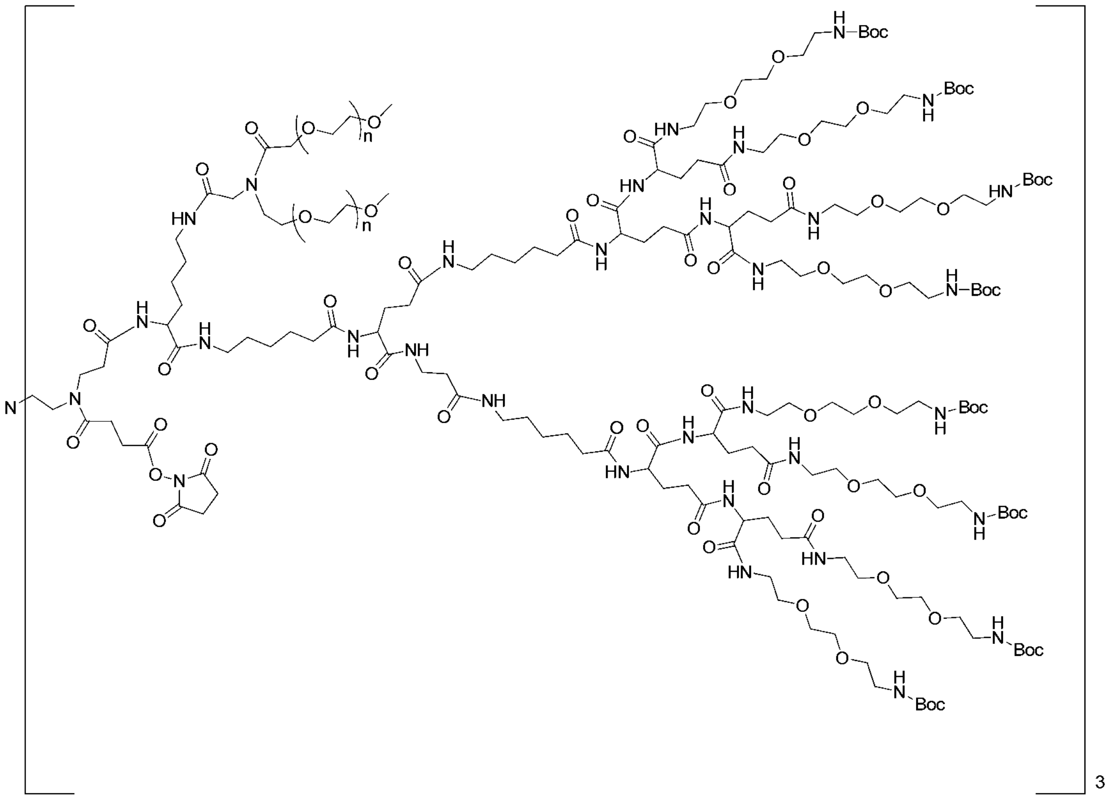

**88-220** (2.0 g, 0.073 mmol), TSTU (0.12 g, 0.16 mmol) and DMF (10 mL) were dissolved in a 50 mL flask, then triethylamine (0.12 mL, 0.95 mmol) was added, and the mixture was reacted under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The resulting product was dissolved in a dichloromethane/methanol mixture, loaded by dry method, and subjected to column chromatography using 9% methanol/ dichloromethane as the eluent. The desired product was collected, concentrated, and dried in a vacuum oven to provide the product.

17.18 Preparation of **88-226**

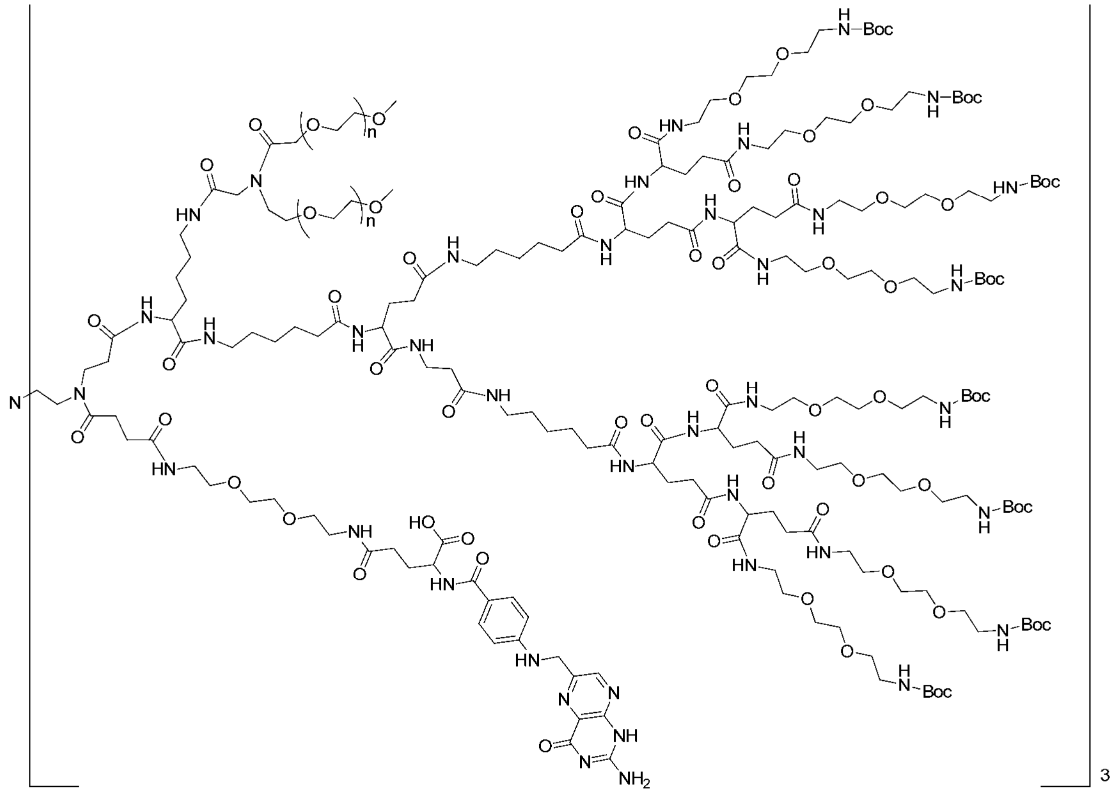

**88-222** (2.0 g, 0.073 mmol) and **74-185** (0.15 g, 0.7 mmol) were added to a 250 mL round-bottom flask, then DMF (15 mL) was added for dissolution with the assistance of ultrasonication. The mixture was stirred at 0°C for 10 minutes. DIEA (0.8 mL, 4.47 mmol) was then added dropwise. The flask was taken out, and the reaction was carried out under stirring at room temperature. After the reaction was completed, methyl tert-butyl ether (200 mL) was added for precipitation. A solid precipitated and was filtered. The filter cake was dissolved in a methanol/dichloromethane mixture, then 200-300 mesh silica gel powder was added, and the mixture was evaporated to dryness. The resulting product was loaded by dry method, and subjected to column chromatography using 9% methanol/dichloromethane as the eluent. The desired product was collected, concentrated, and evaporated to dryness to provide the product.

17.19 Preparation of **88-227**

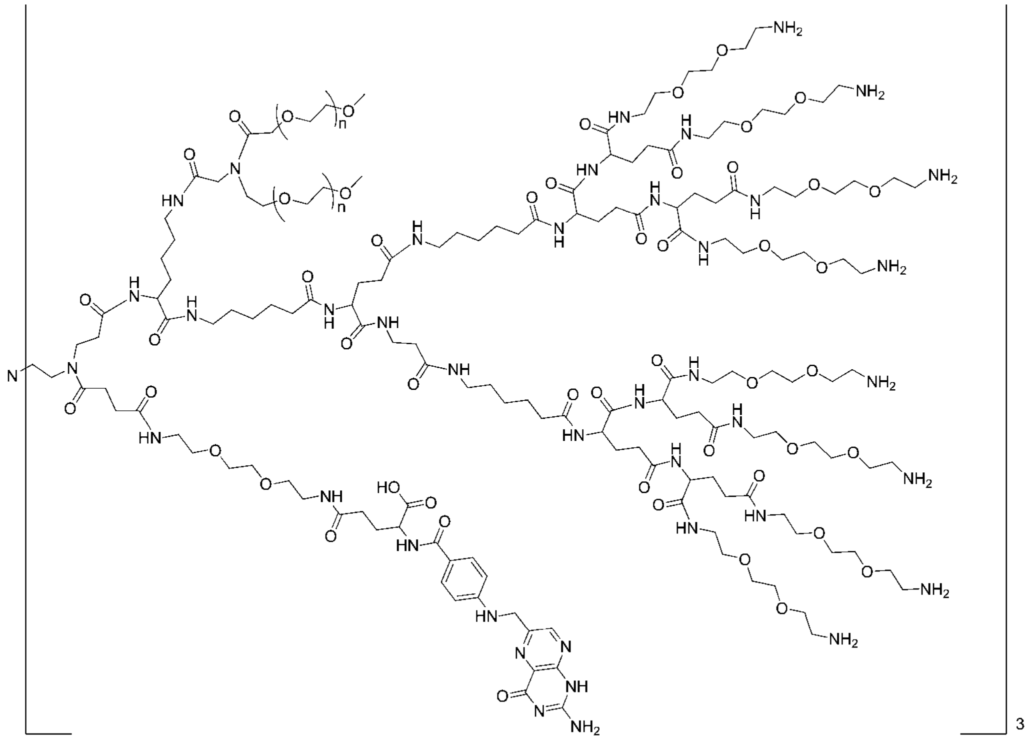

**88-226** (2.1 g, 0.0492 mmol) was placed in a 500 mL round-bottom flask, then TFA (15 mL) was added, and the mixture was reacted under stirring at room temperature. After the reaction was completed, an appropriate amount of dichloromethane was added. The dichloromethane and TFA were removed by rotary evaporation under reduced pressure. Dichloromethane was then added, and rotary evaporated under reduced pressure. This process was repeated several times until the product became a viscous oil. An appropriate amount of dichloromethane was added for dissolution, and DIEA was added to adjust the pH to alkaline. The resulting product was concentrated under reduced pressure and evaporated to dryness to provide the product.

17.20 Preparation of **88-228**

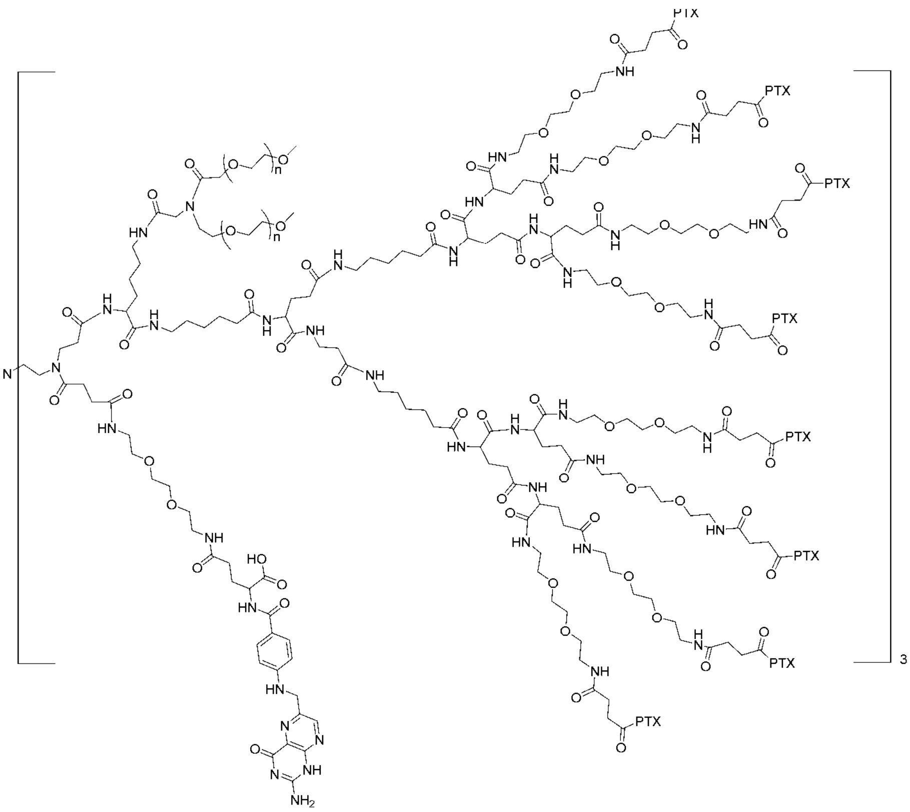

**88-227** (1.98 g, 0.0491 mmol) and **77-163** (1.85 g, 1.768 mmol) were added to a 250 mL round-bottom flask, and then DMF (30 mL) was added for dissolution with the assistance of ultrasonication. The mixture was stirred at 0°C for 1 minute, followed by the dropwise addition of DIEA (0.87 mL, 5.292 mmol). The flask was taken out, and the reaction was carried out under stirring at room temperature. After the reaction was completed, methyl tert-butyl ether (200 mL) was added for precipitation. A solid precipitated and was filtered. The filter cake was dissolved, then 200-300 mesh silica gel powder was added, and the mixture was evaporated to dryness. The resulting product was loaded by dry method, and subjected to column chromatography using 9% methanol/dichloromethane as the eluent. The desired product was collected, concentrated, and evaporated to dryness to provide 1.3 g of the final product with a yield of 42.21%. $^1$H-NMR (600 MHz, DMSO-$d_6$) δ 9.38-9.16 (m, 24H), 8.43-7.80 (m, 188H), 7.78-7.30 (m, 255H), 7.21-7.16 (m, 24H), 6.38-6.22 (m, 24H), 5.93-5.74 (m, 24H), 5.63-5.27 (m, 72H), 5.08-4.78 (m, 48H), 4.73-4.58 (m, 24H), 4.28-3.93 (m, 103H), 3.71-3.45 (m, 2168H), 3.25-3.10 (m, 127H), 3.04-2.92 (m, 38H), 2.84-2.76 (m, 43H), 2.67-2.56 (m, 54H), 2.44-2.30 (m, 76H), 2.28-2.01 (m, 218H), 1.86-1.74 (m, 106H), 1.70-1.60 (m, 43H), 1.57-1.41 (m, 115H), 1.38-1.30 (m, 20H), 1.13-0.81 (m, 179H)

Example 18: Synthesis of Compound **76-179**

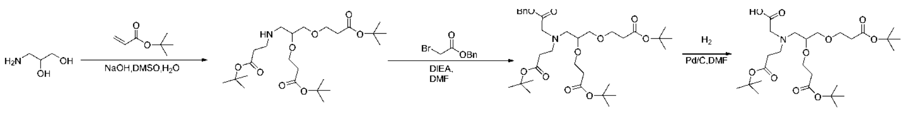


82-50 76-149 76-155

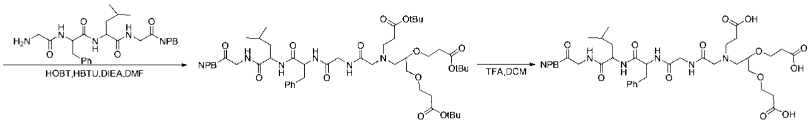


76-156 76-170

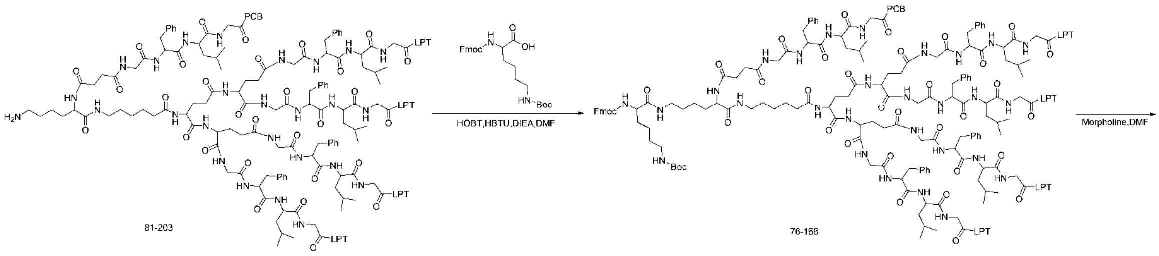


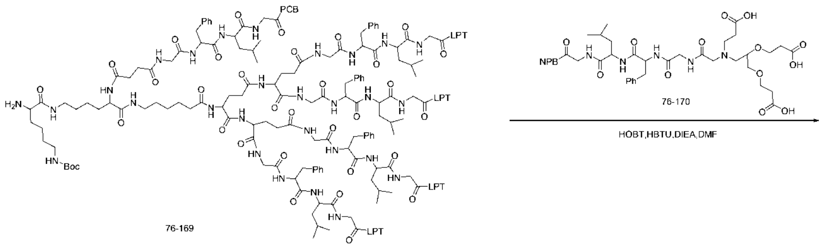

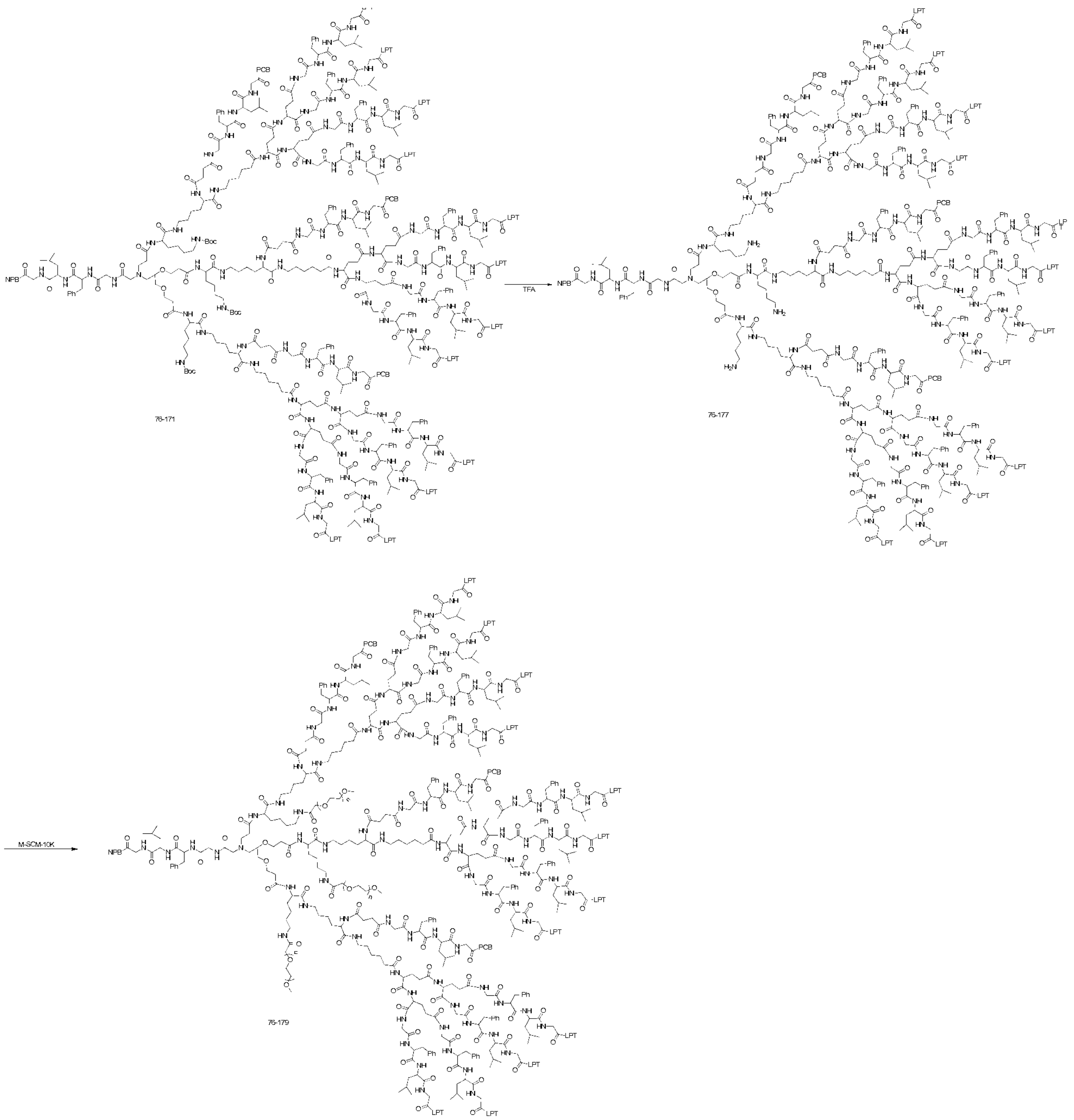


76-171

76-177

76-179

18.1 Preparation of **82-50**

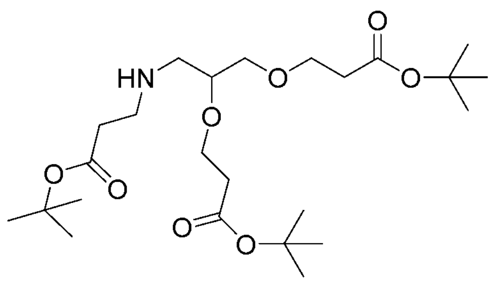

(*R*)-3-Amino-1,2-propanediol (1.0 g, 10.98 mmol) was placed in a 250 mL flask, followed by the addition of DMSO

(4 mL). The reaction flask was charged with nitrogen gas, and cooled to 15°C. While stirring, 5 M aqueous sodium hydroxide solution (0.4 mL) was added, followed by dropwise addition of tert-butyl acrylate (4.78 mL, 32.93 mmol). After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness, then dissolved in a methanol/dichloromethane (1/4) mixture, then silica gel powder (100 mL) was added, and the mixture was evaporated to dryness to provide a powdery solid, which was loaded by dry method, and subjected to column chromatography using 0.2% aqueous ammonia/2-4% methanol/dichloromethane as the eluent. The desired product was collected, concentrated, and dried in a vacuum oven to provide 3.85 g. $^1$H-NMR (600 MHz, DMSO-$d_6$) δ 3.74-3.66 (m, 1H), 3.64-3.54 (m, 3H), 3.44-3.37 (m, 3H), 2.71-2.65 (m, 2H), 2.57-2.52 (m, 1H), 2.49-2.45 (m, 1H), 2.43-2.35 (m, 4H), 2.32-2.26 (m, 2H), 1.45-1.37 (m, 27H)

18.2 Preparation of **76-149**

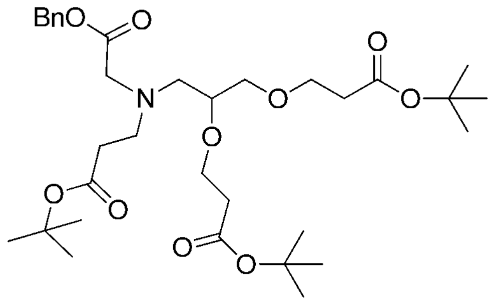

**82-50** (0.50 g, 1.05 mmol) was weighed, dissolved by adding an appropriate amount of DMF, and allowed to stir at 0°C. DIEA (0.52 mL, 2.15 mmol) was then slowly added dropwise. After the dropwise addition was completed, stirring was performed for half an hour, benzyl bromoacetate (0.24 mL, 1.05 mmol) was slowly added dropwise. After the dropwise addition was completed, the reaction was carried under stirring overnight at room temperature. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, and evaporated to dryness to obtain a solid, which was then dissolved in a methanol/dichloromethane (1/4) mixture, then silica gel powder (100 mL) was added, and the mixture was evaporated to dryness to provide a powdery solid, which was loaded by dry method, and subjected to column chromatography using a 20% ethyl acetate/petroleum ether mixture as the eluent. The product was collected, concentrated, and dried in a vacuum oven to provide 0.53 g of the product with a yield of 81%. $^1$H-NMR (600 MHz, DMSO-$d_6$) δ 7.39-7.31 (m, 5H), 5.11-5.08 (m, 2H), 3.68-3.65 (m, 1H), 3.61-3.53 (m, 4H), 3.48-3.47 (m, 2H), 2.85-2.81 (m, 2H), 2.63-2.60 (m, 3H), 2.41-2.37 (m, 1H), 2.35-2.33 (m, 2H), 2.31-2.28 (m, 2H), 1.39-1.37 (m, 27H); ESI [M+H$^+$] 624.37, [M+Na$^+$] 646.35

18.3 Preparation of **76-155**

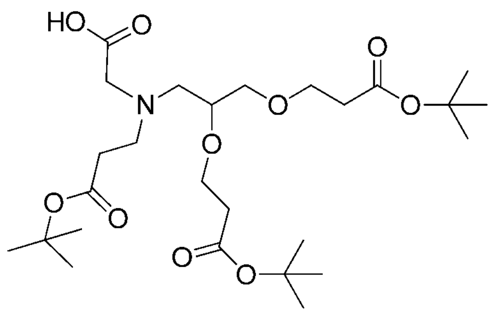

**76-149** (0.53 g, 0.85 mmol) and 10% Pd/C catalyst (0.1 g) were placed in a hydrogenation reactor, then DMF (10 mL) was added for dissolution. The hydrogenation reactor was sealed, evacuated with a water pump, and then refilled with hydrogen. This process was repeated three times. The pressure reading of the hydrogenation reactor was adjusted to 1.8

MPa. The reaction was carried out overnight at room temperature. When the reaction was completed, the reaction mixture was filtered through Celite, and the filter cake was washed with DMF (5 mL × 3) to provide a DMF solution of the product, which served as the raw material for the next reaction.

18.4 Preparation of **76-156**

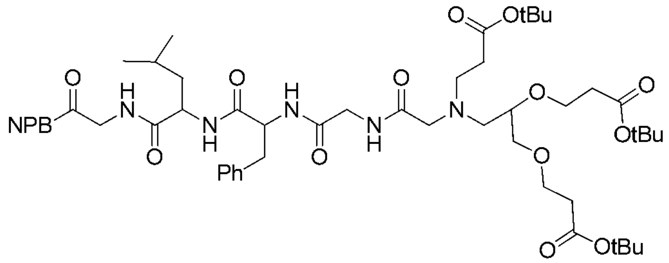

**70-68** (0.58 g, 0.83 mmol), HBTU (0.39 g, 1.02 mmol) and HOBT (0.14 g, 1.02 mmol) were weighed and added into a flask containing **76-155** (0.85 mmol), then an appropriate amount of DMF was added to dissolve the mixture. The mixture was allowed to stand at -5°C, and DIEA (0.51 mL, 3.06 mmol) was then slowly added dropwise. After the dropwise addition was completed, and the reaction was carried out under stirring for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added for precipitation. This process was repeated three times. The mixture was filtered to provide a solid product, which was collected and dried to provide 0.9 g of the product with a yield of 89.2%. $^1$H-NMR (600 MHz, DMSO-$d_6$) δ 9.30 (s, 1H), 8.57 (s, 1H), 8.23 - 7.98 (m, 6H), 7.93-7.77 (m, 3H), 7.61-7.47 (m, 2H), 7.31-7.11 (m, 6H), 4.62-4.30 (m, 3H), 4.10-3.81 (m, 3H), 3.76-3.50 (m, 6H), 3.47-3.36 (m, 3H), 3.14-3.02 (m, 3H), 2.81-2.60 (m, 5H), 2.41-2.28 (m, 7H), 2.05-1.93 (m, 1H), 1.86-1.75 (m, 2H), 1.66-1.47 (m, 4H), 1.41-1.35 (m, 27H), 1.28-1.20 (m, 2H), 0.87 (s, 6H)

18.5 Preparation of **76-170**

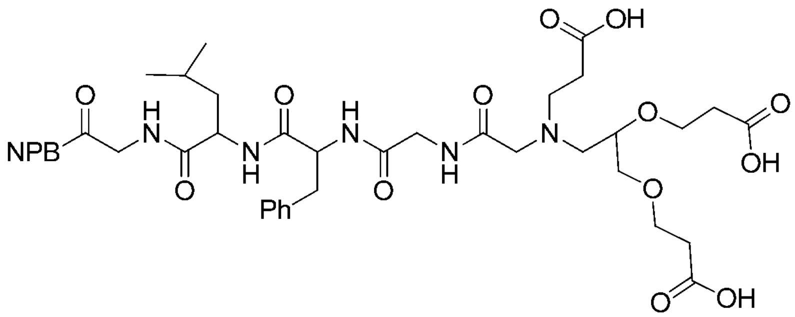

To a flask containing **76-156** (0.90 g, 0.74 mmol), dichloromethane (5 mL) was added for dissolution with the assistance of ultrasonication, then TFA (2 mL) was added, and the mixture was reacted under stirring at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with MTBE (40 mL × 3). The product had poor solubility. The filter cake was collected and dried in a vacuum oven to provide 0.89 g of the product.

18.6 Preparation of **76-168**

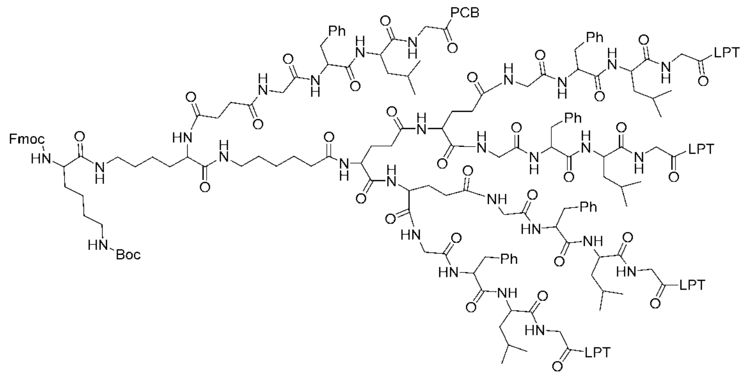

Fmoc-Lys(Boc)-OH (0.12 g, 0.26 mmol), HBTU (0.22 g, 0.60 mmol) and HOBT (0.08 g, 0.60 mmol) were weighed and added into a flask containing **81-203** (0.26 mmol), then an appropriate amount of DMF was added to dissolve the mixture, and allowed to stand at -5°C. DIEA (0.08 mL, 0.50 mmol) was then slowly added dropwise. After the dropwise addition was completed, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried to provide the product.

18.7 Preparation of **76-169**

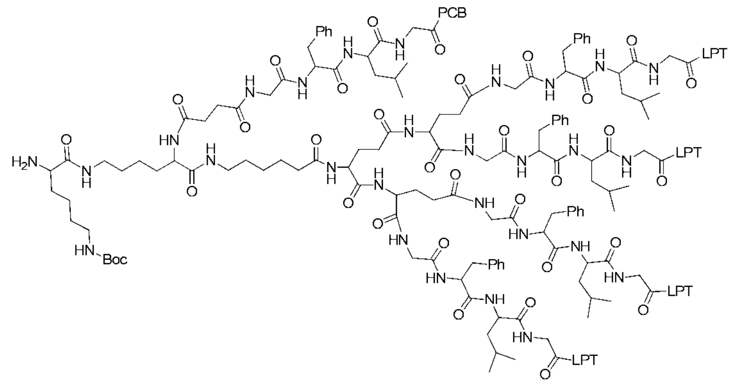

To a flask containing **76-168** (0.26 mmol), DMF (10 mL) was added for complete dissolution with the assistance of ultrasonication, then morpholine (0.3 mL) was added, and the mixture was reacted under stirring at room temperature overnight. After the reaction was completed, methyl tert-butyl ether (60 mL) was added. A powdery solid precipitated from the reaction mixture and was filtered. The filter cake was washed with methyl tert-butyl ether (40 mL × 3). The product has poor solubility. The filter cake was collected and dried in a vacuum oven to provide the product. FT MS ESI m/z [M+H$^+$] 5523.364

18.8 Preparation of **76-171**

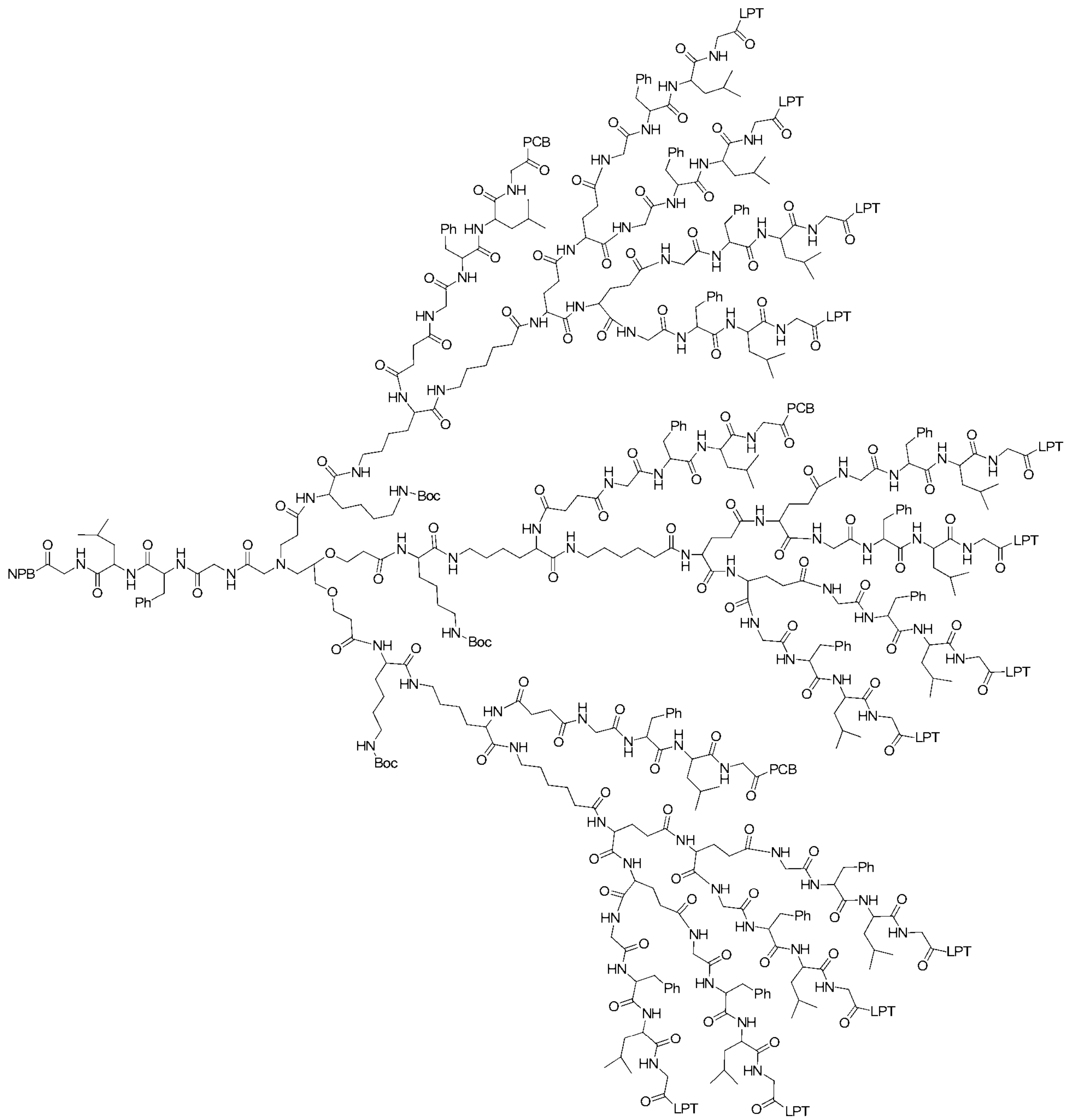

**76-170** (0.044 g, 0.0422 mmol), HBTU (0.06 g, 0.15 mmol) and HOBT (0.02 g, 0.15 mmol) were weighed and added into a flask containing **76-169** (0.77 g, 0.14 mmol), then an appropriate amount of DMF was added to dissolve the mixture. The mixture was stirred at -5°C, and DIEA (0.08 mL, 0.46 mmol) was slowly added dropwise. After the dropwise addition was completed, the reaction was carried out for half an hour. The flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product. The crude product was dissolved in a methanol/dichloromethane (1/4) mixture, then silica gel powder (30 mL) was added, and the mixture was evaporated to dryness to provide a powdery solid. The resulting product was loaded by dry method, and subjected to column chromatography using a 1% ammonia solution/6-12% methanol/dichloromethane mixture as the eluent. The desired product was collected, concentrated, and dried in a vacuum oven to provide the product.

18.9 Preparation of **76-177**

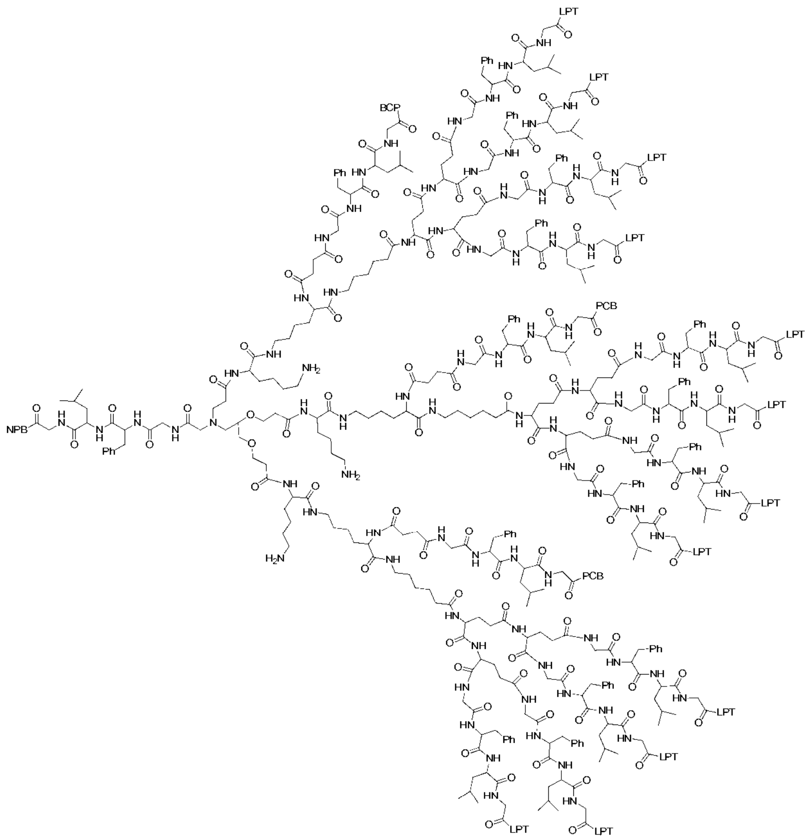

To a flask containing **76-171** (0.0422 mmol) was added dichlorohexane (20 mL). The reaction flask was subjected to ultrasonic agitation, and then trifluoroacetic acid (20 mL) was added. The mixture was reacted under stirring at room temperature for 2 h. After the reaction was completed, most of the trifluoroacetic acid was removed by rotary evaporation under reduced pressure, and then n-hexane (25 mL) and methyl tert-butyl ether (200 mL) were added for precipitation. The mixture was filtered to provide a solid, which was dissolved in a 20% methanol/dichloromethane mixture, and then n-hexane and methyl tert-butyl ether were added for precipitation. This process was repeated three times. The mixture was filtered to provide a solid product, which was then dried in a vacuum oven to provide 0.86 g of the product.

18.10 Preparation of **76-179**

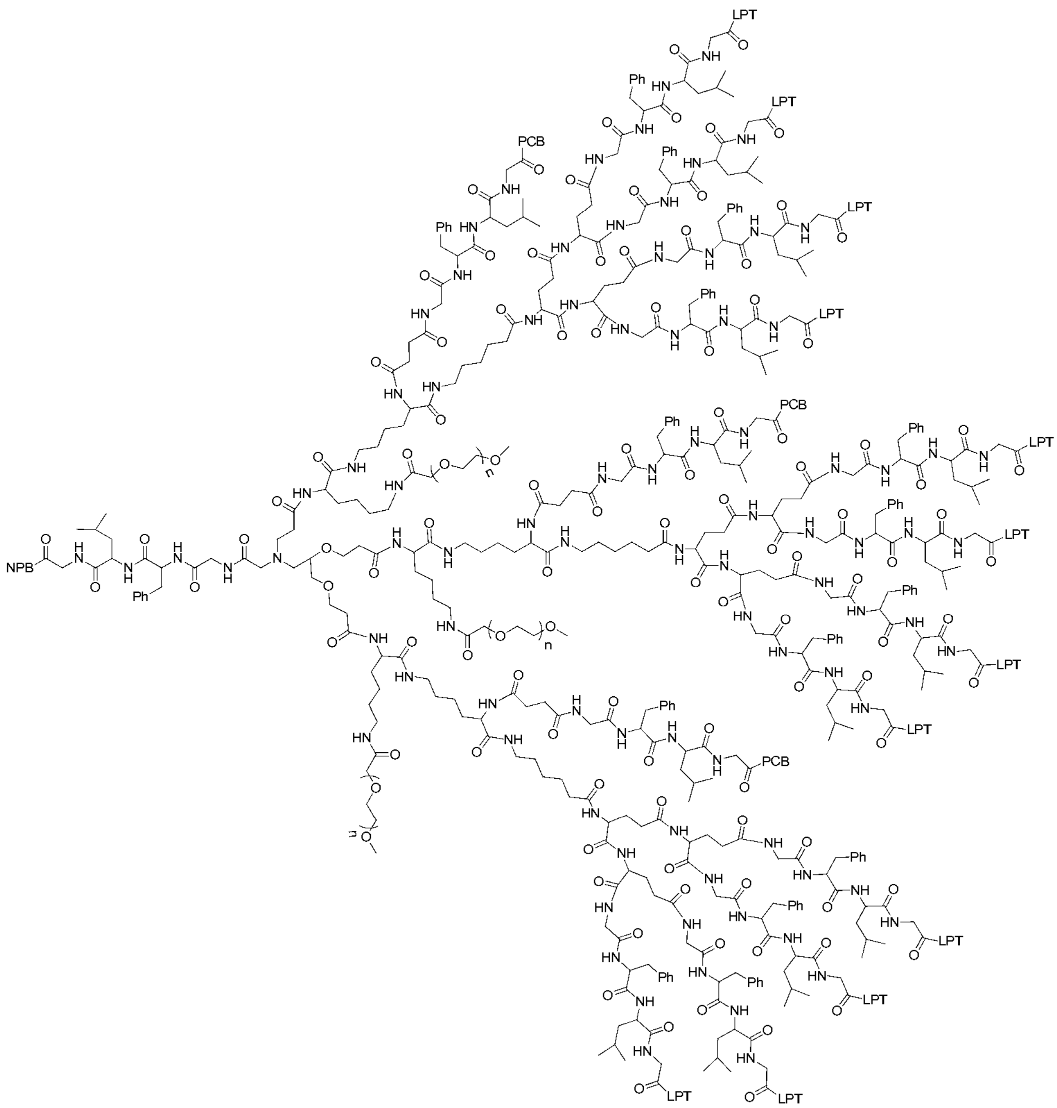

**76-177** (0.0422 mmol) was placed in a 250 mL flask, and DMF (20 mL) was added for dissolution. Then this solution was slowly added dropwise to a DMF solution containing DIEA (0.63 mL, 3.80 mmol) and M-SCM-10K (1.42 g, 0.13 mmol, purchased from Jenkem). The reaction was carried out under stirring in the dark at low speed at room temperature for one week. After the reaction was completed, n-hexane (25 mL) and methyl tert-butyl ether (200 mL) were added for precipitation, the supernatant was discarded, and n-hexane and methyl tert-butyl ether were added for precipitation. This process was repeated three times. The mixture was filtered to provide a solid product. The crude product was dissolved in a methanol/dichloromethane (1/4) mixture, then silica gel powder (30 mL) was added, and the mixture was evaporated to dryness to provide a powdery solid, which was loaded by dry method, and subjected to column chromatography using a 1% ammonia solution/6-12% methanol/dichloromethane mixed solvent as the eluent. The desired product was collected, concentrated, and dried in a vacuum oven to provide 1.61 g of the product. $^1$H-NMR (600 MHz, DMSO-$d_6$) $\delta$10.25-9.94 (m, 12H), 9.00-8.88 (m, 3H), 8.85-8.50 (m, 24H), 8.30-8.10 (m, 44H), 8.10-8.03 (m, 26H), 8.03-7.92 (m, 42H), 7.90-7.84 (m, 11H), 7.83-7.75 (m, 15H), 7.74-7.63 (m, 16H), 7.51-7.42 (m, 16H), 7.37-7.25 (m, 40H), 7.25-7.13 (m, 88H), 7.13-7.03 (m, 29H), 7.02-6.98 (m, 4H), 6.73-6.50 (m, 16H), 5.31-5.20 (m, 24H), 4.90-4.65 (m, 24H), 4.63-4.48 (m, 19H), 4.45-4.30 (m, 21H), 4.29-4.07 (m, 48H), 4.06-3.95 (m, 15H), 3.90-3.79 (m, 34H), 3.79-3.73 (m, 33H), 3.55-3.44 (m, 2863H), 3.08-2.98 (m, 80H), 2.83-2.71 (m, 36H), 2.63-2.60 (m, 19H), 2.32-2.26 (m, 15H), 2.17-1.98 (m, 35H), 1.65-1.53 (m, 37H), 1.53-1.41 (m, 50H), 1.28-1.11 (m, 32H), 0.89-0.77 (m, 96H)

Example 19: Synthesis of Compound **87-47**

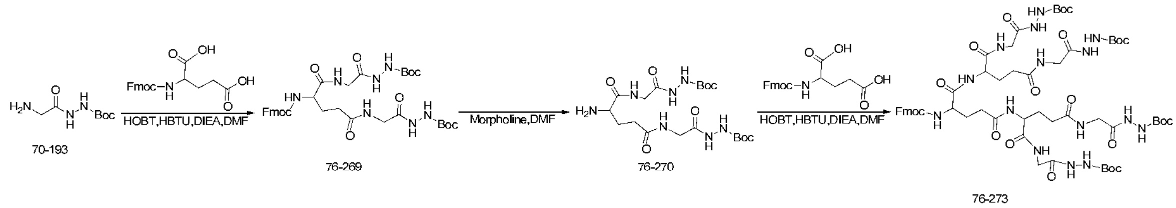

70-193 76-269 76-270 76-273

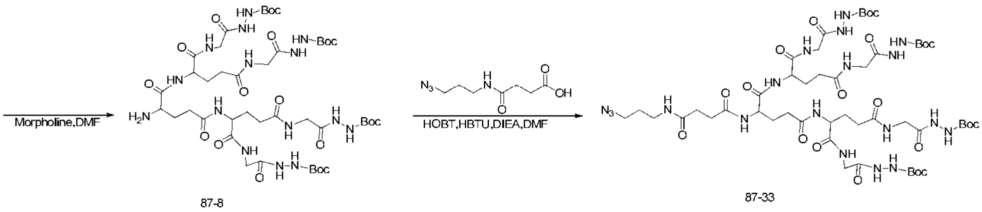

87-8 87-33

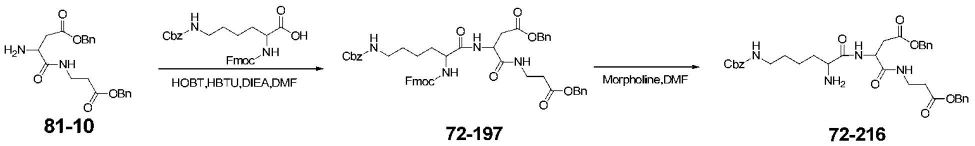

**81-10** **72-197** **72-216**

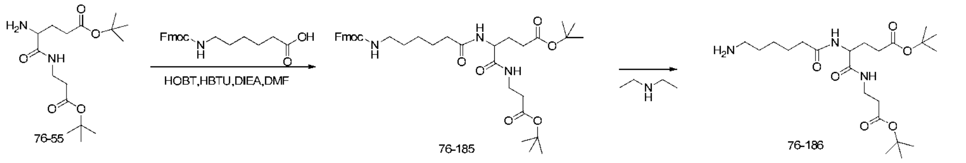

76-55 76-185 76-186

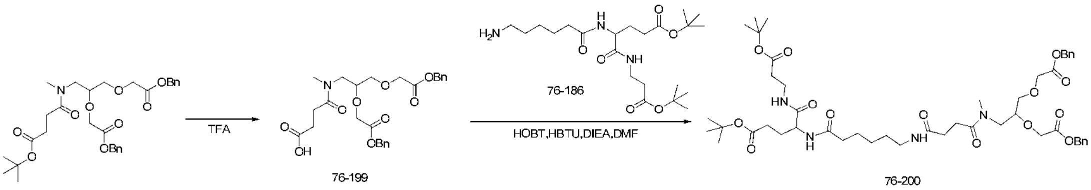

76-199 76-200

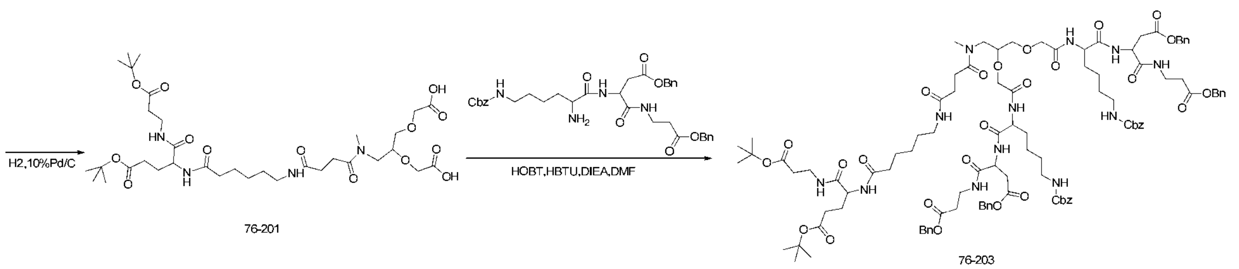

76-201 76-203

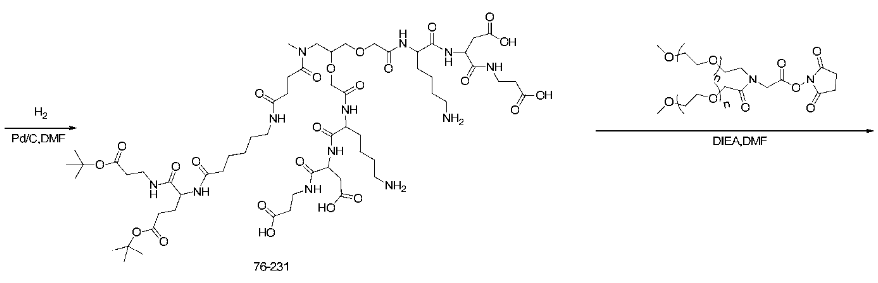

76-231

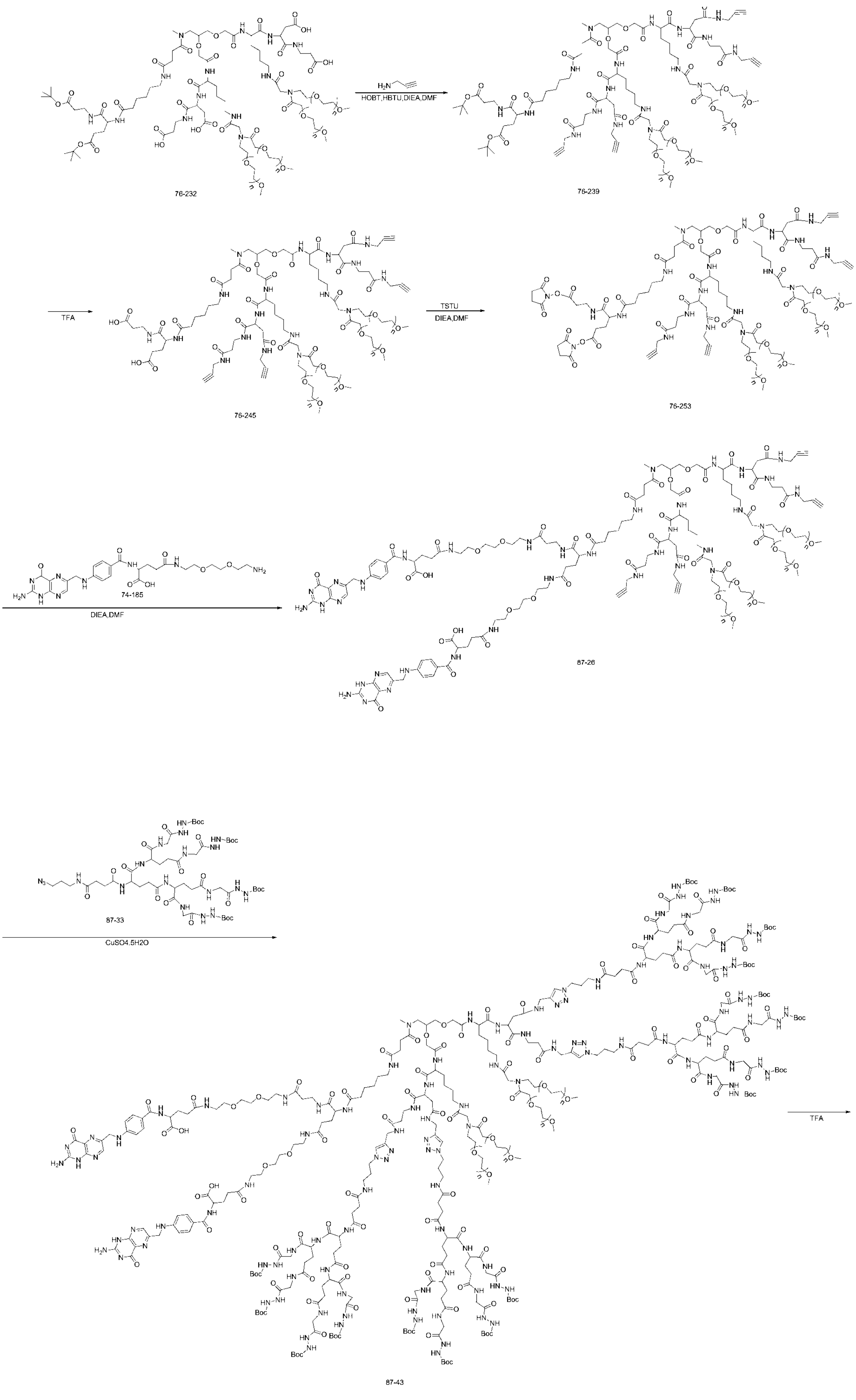


76-232

76-239

76-245

76-253

87-26

87-43

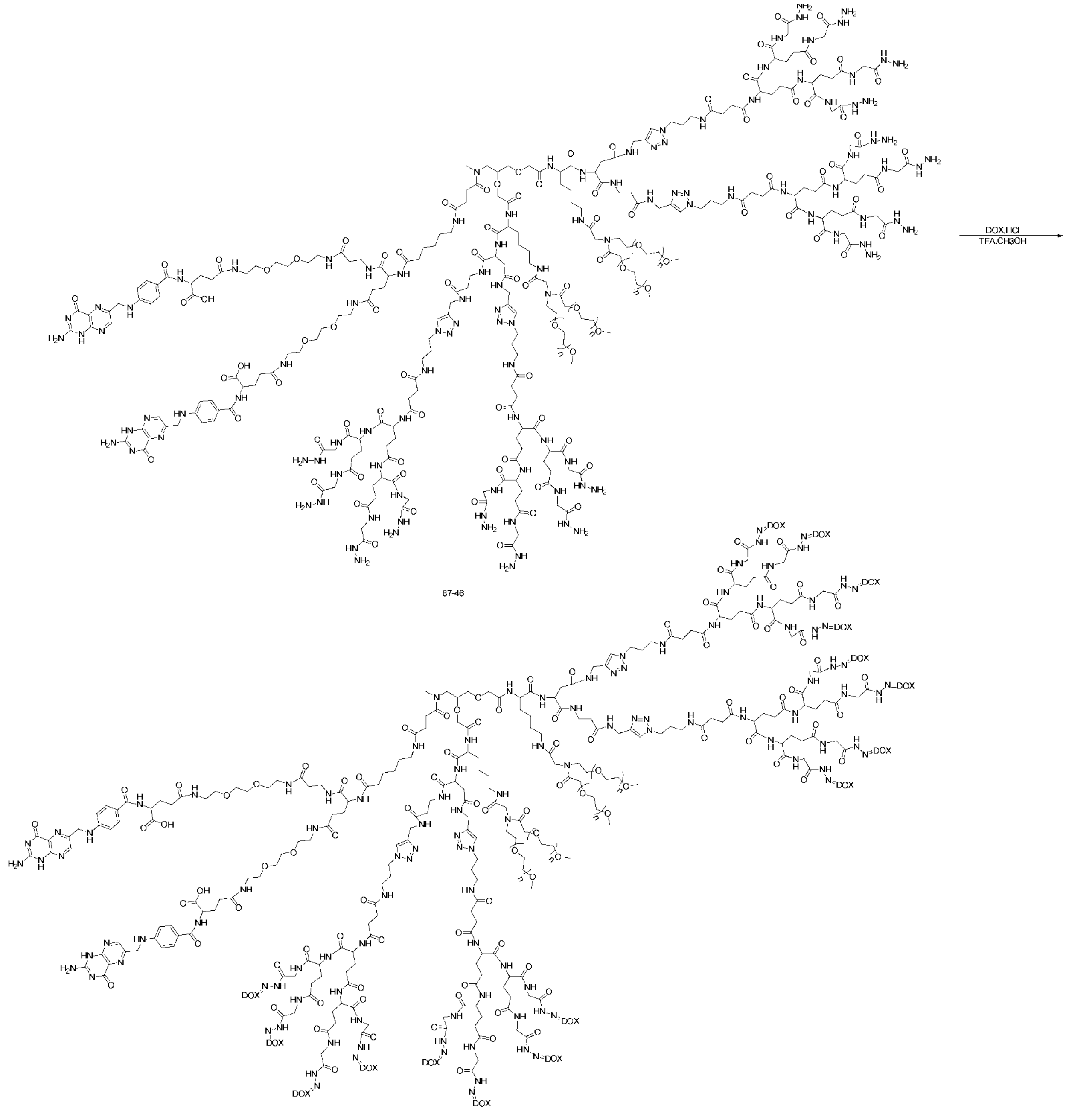


87-46

87-47

19.1 Preparation of Compound **76-269**

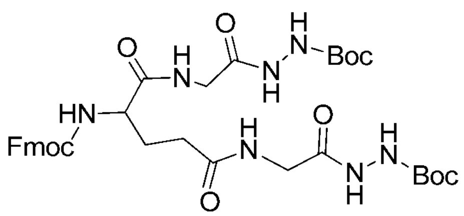

Fmoc-Glu-OH (4.46 g, 12.10 mmol), HBTU (10.99 g, 29.04 mmol) and HOBT (3.92 g, 29.04 mmol) were added into a flask containing **70-193** (4.80 g, 25.37 mmol), then an appropriate amount of DMF was added to dissolve the mixture,

and allowed to stand at -5°C. DIEA (14.40 mL, 87.12 mmol) was then slowly added dropwise. After the dropwise addition, the reaction was carried out for half an hour. Then the flask was taken out, and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, evaporated to dryness to obtain a solid, which was collected, and dried in a vacuum oven to provide 6.9 g of the product.

19.2 Preparation of **76-270**

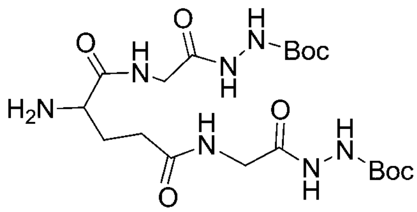

To a flask containing **76-269** (2.0 g) was added DMF (20 mL). The reaction mixture was subjected to ultrasonication until complete dissolution, then morpholine (2 mL) was added, and the mixture was reacted under stirring at room temperature for 2 h. After the reaction was completed, n-hexane (25 mL) and methyl tert-butyl ether (200 mL) were added for precipitation, the supernatant was discarded, and then n-hexane and methyl tert-butyl ether were added for precipitation. This process was repeated three times. The mixture was filtered to provide a solid product, which was then dried in a vacuum oven to provide the product.

19.3 Preparation of **76-273**

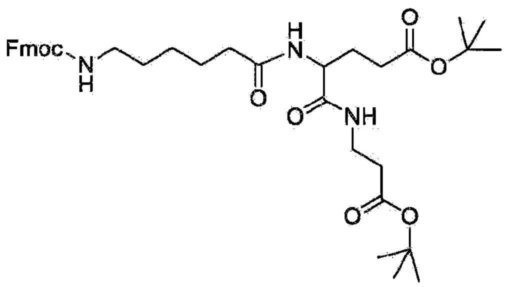

Fmoc-Glu-OH (0.49 g, 1.34 mmol), HBTU (1.22 g, 3.22 mmol) and HOBT (0.44 g, 3.22 mmol) were weighed and added into a flask containing **76-270** (2.81 mmol), then an appropriate amount of DMF was added to dissolve the mixture, and allowed to stir at -5°C. DIEA (1.60 mL, 9.65 mmol) was slowly added dropwise. After the dropwise addition, the reaction was carried out for half an hour. Then the flask was taken out, and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined and evaporated to dryness to provide a solid, which was collected, and dried in a vacuum oven to provide 1.07 g of the product.

19.4 Preparation of **87-8**

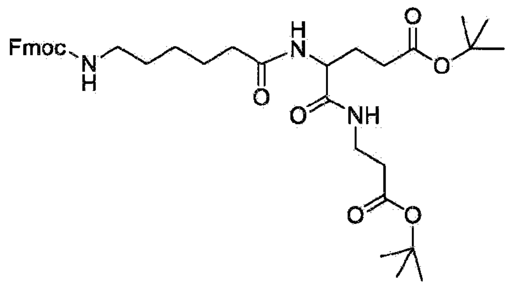

To a flask containing **76-273** (1.07 g) was added DMF (10 mL). The reaction mixture was subjected to ultrasonication until complete dissolution, then morpholine (2 mL) was added, and the mixture was reacted under stirring at room temperature for 2 h. After the reaction was completed, n-hexane (25 mL) and methyl tert-butyl ether (200 mL) were added for precipitation, the supernatant was discarded, and then n-hexane and methyl tert-butyl ether were added again for precipitation. This process was repeated three times. The mixture was filtered to provide a solid product, which was then dried in a vacuum oven to provide the product.

19.5 Preparation of **87-33**

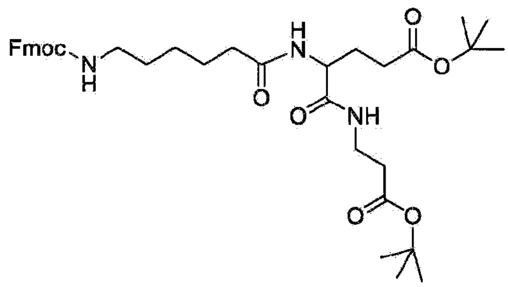

**87-8** (0.82 mmol), HBTU (0.38 g, 0.98 mmol) and HOBT (0.14 g, 0.98 mmol) were weighed and added into a flask containing **76-191** (0.82 mmol), then an appropriate amount of DMF was added to dissolve the mixture, and the contents were allowed to stand at -5°C. DIEA (0.50 mL, 2.95 mmol) was slowly added dropwise. After the dropwise addition was completed, the reaction was carried out for half an hour, the flask was taken out, and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, evaporated to dryness to provide a solid, which was collected, and dried in a vacuum oven to provide 1.0 g of the product. ESI [M+H$^+$] 1272.62, [M+Na$^+$] 1294.61

19.6 Preparation of **72-197**

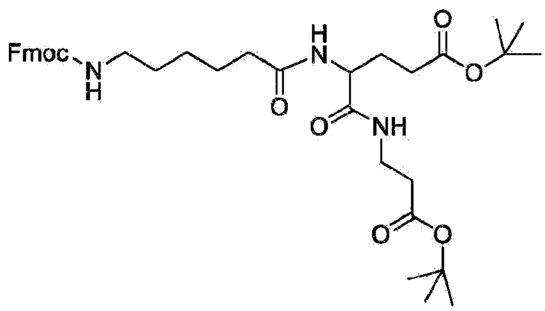

**81-10** (5.2 g, 13.55 mmol), N'-Fmoc-N-benzyloxycarbonyl-L-lysine (6.19 g, 12.32 mmol), HBTU (7.0 g, 18.48 mmol) and HOBT (2.49 g, 18.48 mmol) were added to a 500 mL round-bottom flask, then DMF (30 mL) was added for

dissolution with the assistance of ultrasonication. The mixture was stirred at 0°C for 3 minutes, and then DIEA (9.16 mL, 55.43 mmol) was added dropwise. The flask was taken out, and the reaction was carried out under stirring at room temperature. After the reaction was completed, the reaction mixture was poured into a 1 L separatory funnel. Pure water (200 mL) and ethyl acetate (200 mL) were added to the separatory funnel for extraction. The organic phase was separated, and the aqueous phase was further extracted twice with ethyl acetate (100 mL × 2). The organic phases were combined and washed with saturated sodium bicarbonate solution (200 mL), dried over anhydrous magnesium sulfate, concentrated, and evaporated to dryness to provide 12.0 g of the product.

19.7 Preparation of **72-216**

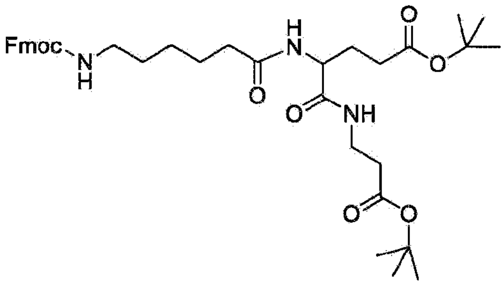

**72-197** (4.84 mmol) was placed in a 500 mL round-bottom flask, DMF (10 mL) was added for dissolution, then morpholine (15 mL) was added, and the mixture was reacted under stirring at room temperature for 2 hours. After the reaction was completed, the reaction mixture was poured into a 1 L separatory funnel, and purified water (200 mL) and ethyl acetate (100 mL) were added for extraction. The organic phase was separated, and the aqueous phase was further extracted twice with ethyl acetate (100 mL). The organic phases were collected, combined, concentrated and dried in a vacuum oven to provide 3.12 g of the product.

19.8 Preparation of **76-185**

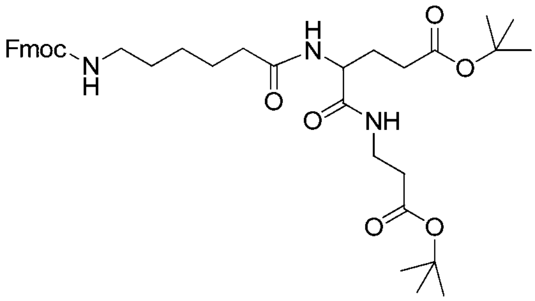

**76-55** (11.75 mmol), **88-14** (4.98 g, 14.1 mmol), HBTU (5.35 g, 14.1 mmol) and HOBT (1.91 g, 14.1 mmol) were added to a 250 mL round-bottom flask, then DMF (20 mL) was added for dissolution with the assistance of ultrasonication, and stirred at 0°C for 2 minutes. DIEA (6.99 mL, 42.3 mmol) was then added dropwise. The flask was taken out, and the reaction was carried out under stirring at room temperature. After the reaction was completed, the reaction mixture was poured into a 1 L separatory funnel, purified water (200 mL) and ethyl acetate (200 mL) were added for extraction, the organic phase was separated, and the aqueous phase was further extracted with ethyl acetate (100 mL × 2). The organic phases were combined, washed with saturated sodium bicarbonate solution (200 mL × 2), collected, dried over anhydrous magnesium sulfate, concentrated, and evaporated to dryness to provide the product.

19.9 Preparation of **76-186**

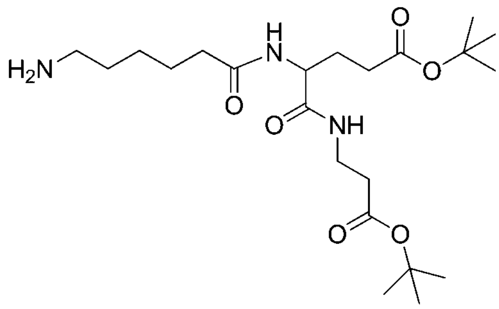

**76-185** (11.75 mmol) was placed in a 500 mL round-bottom flask, acetonitrile (20 mL) was added for dissolution with the assistance of ultrasonication, then diethylamine (20 mL) was added, and the mixture was reacted under stirring at room temperature. After the reaction was completed, the acetonitrile and diethylamine were removed by rotary evaporation under reduced pressure, an appropriate amount of dichloromethane was then added for dissolution, then 200-300 mesh silica gel powder was added, and the mixture was evaporated to dryness. The resulting product was loaded by dry method, and subjected to column chromatography using 1% ammonia/2% methanol/dichloromethane as the eluent. The desired product was collected, concentrated, and evaporated to dryness to provide 3.7 g of the product. ESI [M+H+] 444.31

19.10 Preparation of **76-199**

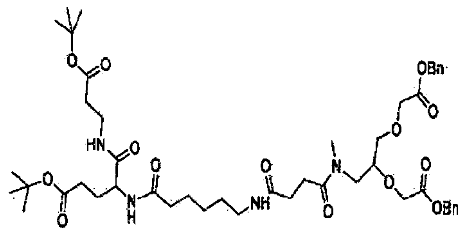

**81-65** (2.0 g, 3.59 mmol) was placed in a 500 mL round-bottom flask, dichloromethane (5 mL) was added for dissolution, then TFA (5 mL) was added, and the mixture was reacted under stirring at room temperature. After the reaction was completed, the TFA and dichloromethane were removed by rotary evaporation under reduced pressure. Methyl tert-butyl ether (50 mL) was then added and subjected to ultrasonication treatment. After standing, the methyl tert-butyl ether was removed by rotary evaporation under reduced pressure, and then an appropriate amount of dichloromethane was added for dissolution with the assistance of ultrasonication. An appropriate amount of DIEA was then added to adjust the pH to alkaline. The mixture was rotary evaporated under reduced pressure and dried to provide the product.

19.11 Preparation of **76-200**

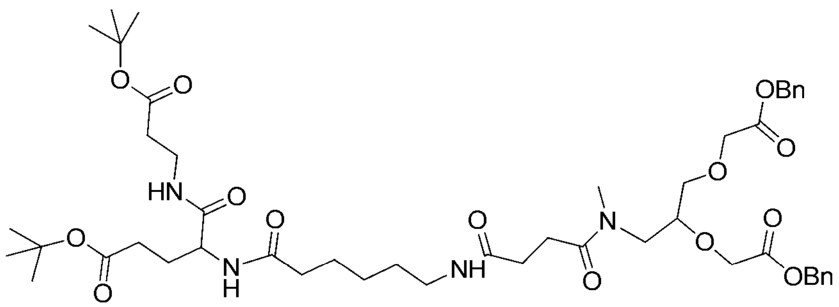

**76-199** (3.59 mmol), **76-186** (1.62 g, 3.66 mmol), HBTU (1.63 g, 4.31 mmol) and HOBT (0.58 g, 4.31 mmol) were added to a 100 mL round-bottom flask, and then DMF (15 mL) was added for dissolution with the assistance of ultrasonication. The mixture was stirred at 0°C for 1 minute. DIEA (2.14 mL, 12.92 mmol) was then added dropwise.

The flask was taken out and stirred at room temperature. After the reaction was completed, the reaction mixture was poured into a 1 L separatory funnel. Pure water (100 mL) and ethyl acetate (100 mL) were added to the separatory funnel for extraction. The organic phase was separated, and the aqueous phase was further extracted with ethyl acetate (100 mL × 2). The organic phases were combined, washed with saturated sodium bicarbonate solution (200 mL × 2), dried over anhydrous magnesium sulfate, concentrated, then 200-300 mesh silica gel powder was added, and the mixture was evaporated to dryness. The resulting product was loaded by dry method, and subjected to column chromatography using 40-70% ethyl acetate/petroleum ether as the eluent. The desired product was collected, concentrated, and evaporated to dryness to provide 2.92 g of the product. $^1$H-NMR (600 MHz, DMSO-$d_6$) δ 8.00-7.86 (m, 3H), 7.39-7.32 (m, 10H), 5.18-5.10 (m, 4H), 4.29-4.17 (m, 5H), 3.68-3.46 (m, 5H), 3.31-3.23 (m, 2H), 3.04-2.95 (m, 4H), 2.92-2.86 (m, 2H), 2.75-2.66 (m, 4H), 2.37-2.26 (m, 4H), 2.20-2.08 (m, 4H), 1.39 (s, 9H), 1.38 (s, 9H), 1.28-1.20 (m, 4H)

19.12 Preparation of **76-201**

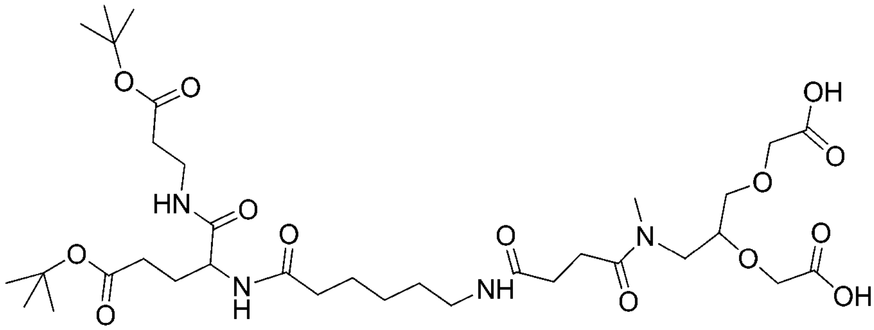

Raw material **76-200** (2.04 g, 2.20 mmol) and 10% Pd/C catalyst (200 mg) were added into a hydrogenation reactor, and then methanol (20 mL) was added to dissolve the mixture. The hydrogenation reactor was sealed, evacuated with a water pump, and then filled with hydrogen. This process was repeated three times. Finally, the hydrogen pressure was adjusted to 2 MPa, and the reaction was carried out under stirring at room temperature. After the reaction was completed, the mixture was filtered using a Büchner funnel containing filter paper. The reactor was rinsed with methanol (5 mL × 3). The filtrate was collected, concentrated, and evaporated to dryness to provide the product.

19.13 Preparation of **76-203**

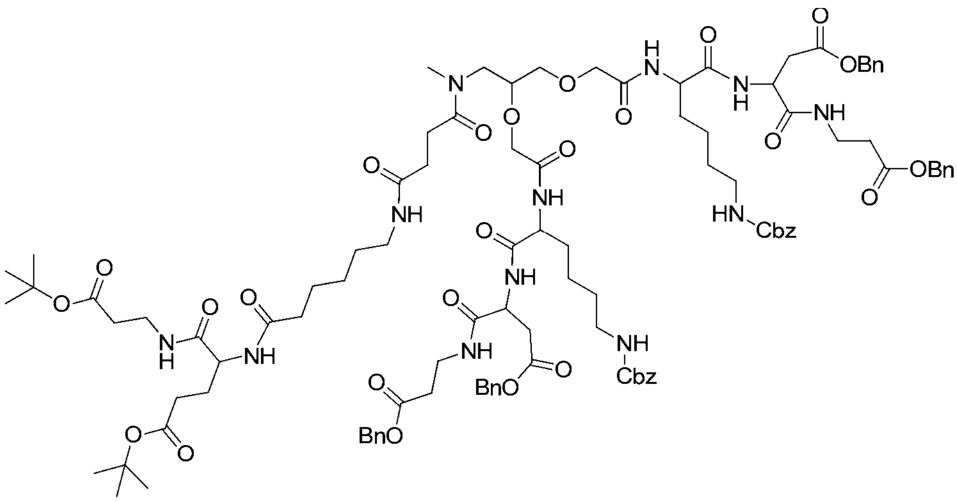

**76-201** (2.20 mmol), **72-216** (4.84 mmol), HBTU (2.00 g, 5.28 mmol) and HOBT (0.71 g, 5.28 mmol) were added to a 250 mL round-bottom flask, and then DMF (10 mL) was added for dissolution with the assistance of ultrasonication. The mixture was stirred at 0°C for 2 minutes. DIEA (2.62 mL, 15.84 mmol) was then added dropwise. The flask was taken

out, and the reaction was carried out under stirring at room temperature. After the reaction was completed, methyl tert-butyl ether (200 mL) was added for precipitation. A solid precipitated and was filtered, then 200-300 mesh silica gel powder was added, and the mixture was evaporated to dryness. The resulting product was loaded by dry method, and subjected to column chromatography using 3% methanol/dichloromethane as the eluent. The desired product was collected, concentrated, and evaporated to dryness to provide 10.76 g of the product. $^1$H-NMR (600 MHz, DMSO-$d_6$) δ 8.41-8.31 (m, 3H), 7.97-7.88 (m, 5H), 7.39-7.25 (m, 30H), 7.24-7.17 (m, 3H), 5.09-5.03 (m, 8H), 5.00-4.96 (m, 4H), 4.61-4.54 (m, 3H), 4.31-4.14 (m, 6H), 4.09-3.88 (m, 8H), 3.01-2.89 (m, 10H), 2.85-2.72 (m, 6H), 2.64-2.58 (m, 5H), 2.41-2.26 (m, 7H), 2.17-2.14 (m, 2H), 2.13-2.05 (m, 3H), 1.70-1.59 (m, 5H), 1.58-1.48 (m, 4H), 1.38 (s, 9H), 1.37 (s, 9H), 1.25-1.16 (m, 8H); ESI [M+H$^+$] 2004.97, [M+Na$^+$] 2026.95

19.14 Preparation of **76-231**

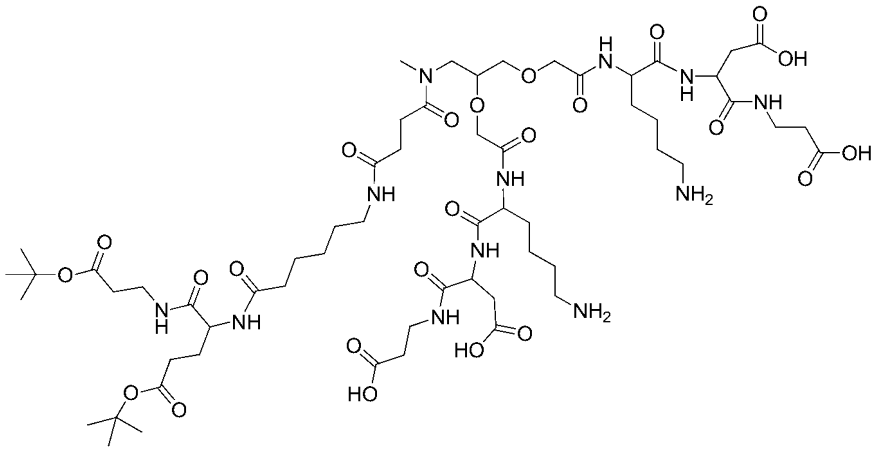

Raw material **76-203** (10.48 g, 3.26 mmol) and 10% Pd/C palladium-on-carbon catalyst (250 mg) were added to a hydrogenation reactor, and then DMF (30 mL) was added for dissolution. The hydrogenation reactor was sealed, evacuated with a water pump, and then filled with hydrogen. This process was repeated three times. Finally, the hydrogen pressure was adjusted to 2 MPa, and the reaction was carried out under stirring at room temperature. After the reaction was completed, the reaction mixture was filtered through a Büchner funnel containing filter paper. The reactor was rinsed with DMF (3 mL). The filtrate was collected, then methyl tert-butyl ether (200 mL) was added. A solid precipitated and was filtered. The filter cake was dried to provide 1.0 g of product.

19.15 Preparation of **76-232**

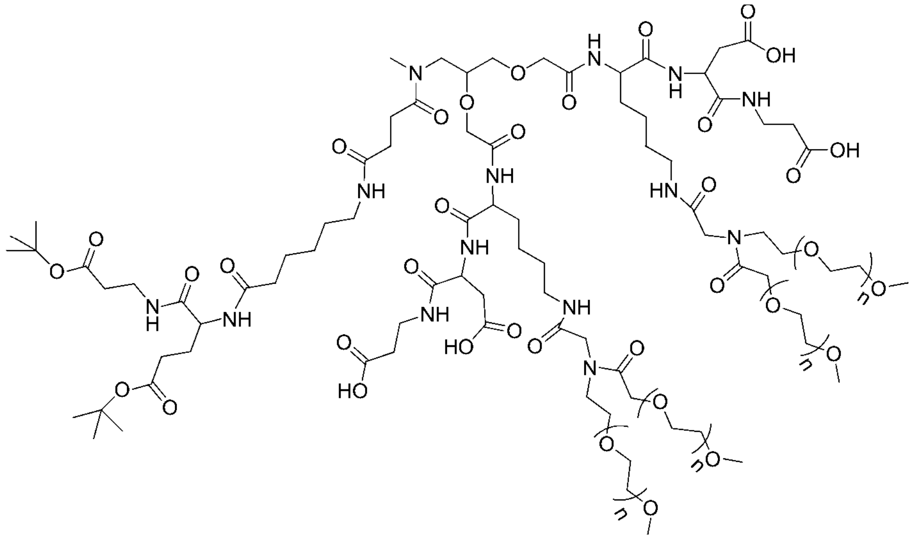

**76-231** (0.33 g, 0.24 mmol) was placed in a 500 mL round-bottom flask, ultra-dry DMF (10 mL) was added for dissolution with the assistance of ultrasonication. The mixture was stirred at 0°C for 5 minutes. DIEA (0.80 mL, 4.81 mmol) was added dropwise, followed by Y-NHS-10K (5.0 g, 0.49 mmol, purchased from JenKem, lot number: ZZ403P095). The air was replaced with nitrogen, and the reaction was carried out under stirring at 35°C in the dark. After the reaction was completed, methyl tert-butyl ether (200 mL) was added. A solid precipitated and was filtered. The filter cake was dried to provide 5.0 g of product.

19.16 Preparation of **76-239**

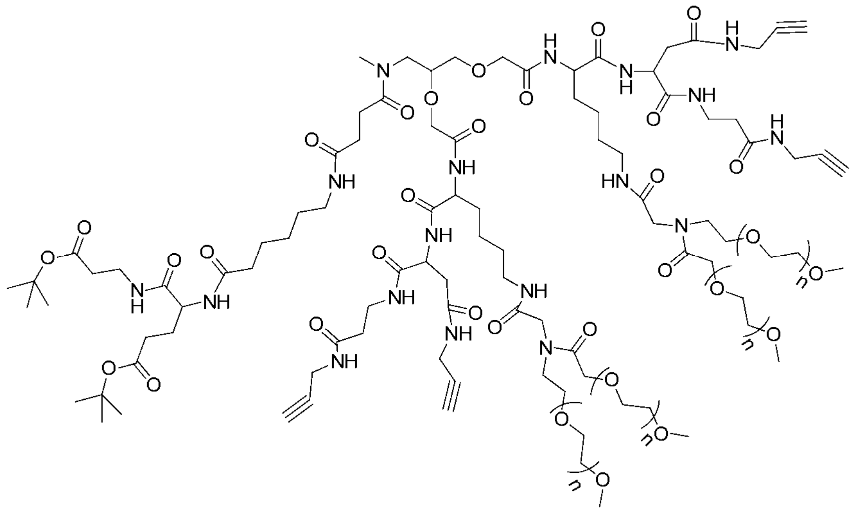

**76-232** (3.0 g, 0.14 mmol), HBTU (0.26 g, 0.69 mmol) and HOBT (0.1 g, 0.69 mmol) were weighed and added into a flask containing propargylamine (0.04 g, 0.64 mmol). An appropriate amount of DMF was added to dissolve the mixture. The mixture was allowed to stir at -5°C. DIEA (0.35 mL, 2.08 mmol) was slowly added dropwise. After the dropwise addition was completed, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added for precipitation. This process was repeated three times. The solid product was filtered, collected, and dried to provide the product.

19.17 Preparation of **76-245**

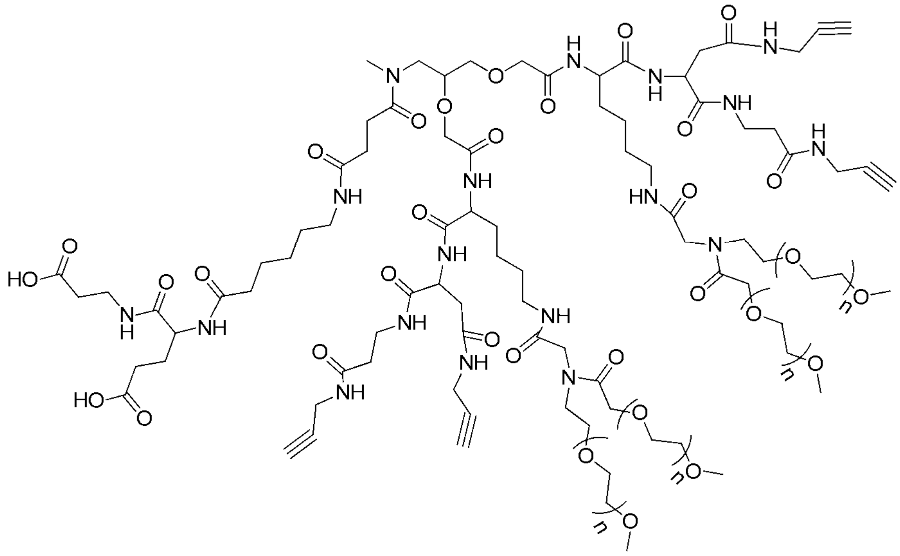

To a flask containing **76-239** (0.1444 mmol) was added dichloromethane. After dissolution with the assistance of ultrasonication, TFA (25 mL) was added, and the reaction was stirred at room temperature overnight. After the reaction was completed, the reaction mixture was evaporated to obtain an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide the product.

19.18 Preparation of **76-253**

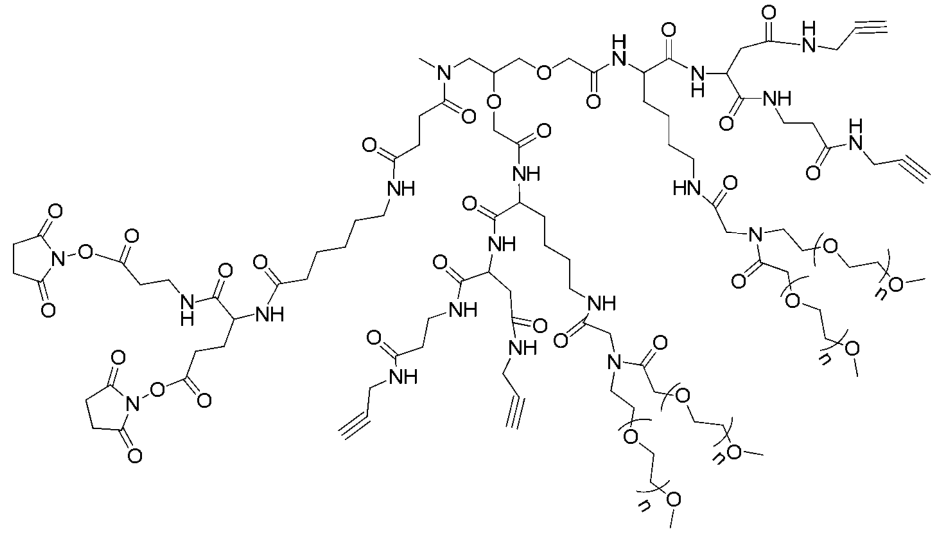

To a flask containing **76-245** (0.1444 mmol) was added DMF. After dissolution with the assistance of ultrasonication, TSTU (0.1 g, 0.32 mmol) was added, then DIEA (0.24 mL, 1.44 mmol) was added dropwise at room temperature, and the contents were allowed to react overnight under stirring. After the reaction was completed, methyl tert-butyl ether (60 mL) was added. A powdery solid precipitated from the reaction mixture and was filtered. The filter cake was washed with methyl tert-butyl ether (40 mL × 3) and then dried in a vacuum oven to provide the product.

19.19 Preparation of **87-26**

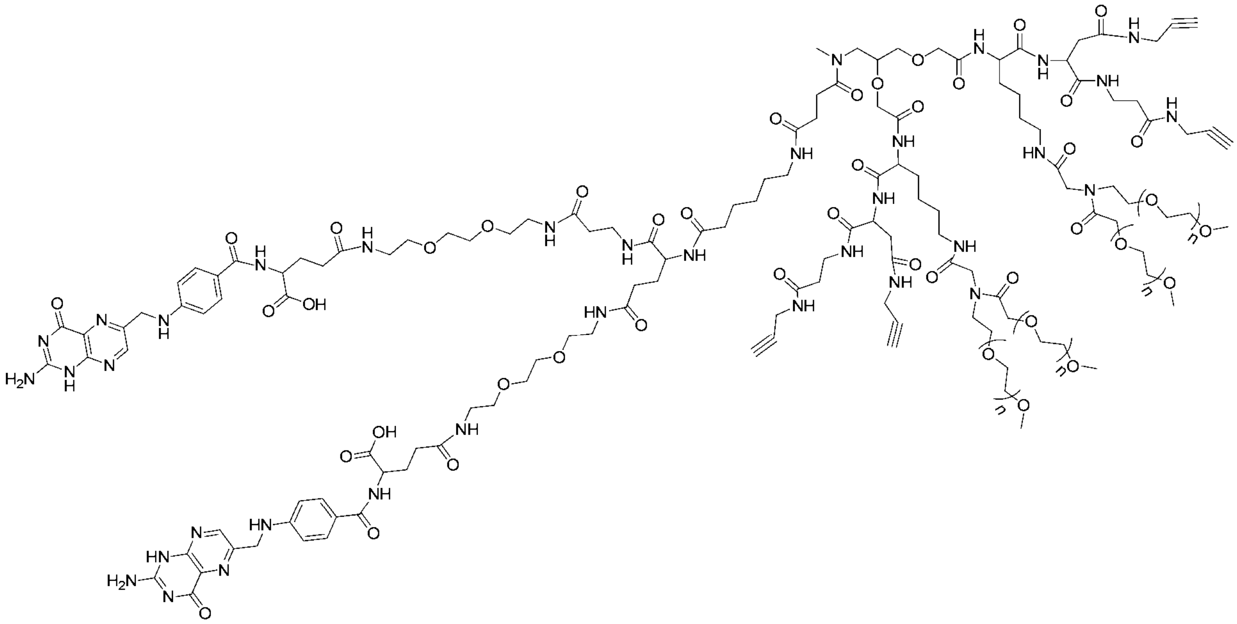

**76-253** (0.1444 mmol) and **74-185** (0.18 g, 0.3149 mmol) were added to a 100 mL round-bottom flask, and then DMF (10 mL) was added for dissolution with the assistance of ultrasonication. The mixture was stirred at 0°C for 1 minute, followed by the dropwise addition of DIEA (0.5 mL, 3.0251 mmol). The flask was taken out, and the reaction was carried out under stirring at room temperature. After the reaction was completed, methyl tert-butyl ether (200 mL) was added for precipitation. A solid precipitated and was filtered. The filter cake was collected, dissolved in an appropriate amount of DMF, then methyl tert-butyl ether was added in batches with small amounts until a solid precipitated, which was filtered by suction. The filter cake was dried to provide 3.26 g of the product.

19.20 Preparation of **87-43**

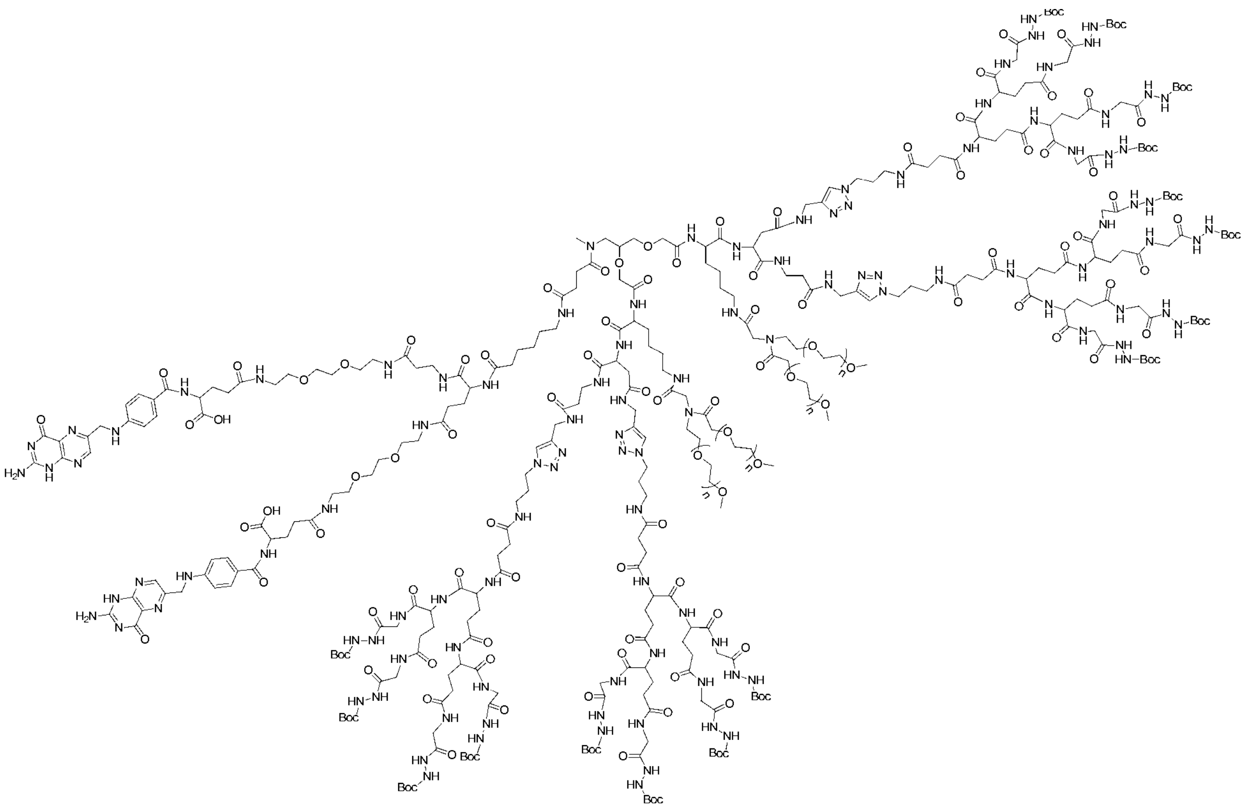

**87-26** (1.0 g, 0.044 mmol) and **87-33** (0.35 g, 0.27 mmol) were placed in a 250 mL reaction flask, and an appropriate amount of ultra-dry DMF was added for dissolution. The flask was filled with nitrogen. Then, anhydrous $CuSO_4$ (0.06 g, 0.35 mmol) and sodium ascorbate (0.14 g, 0.70 mmol) were weighed and added to the flask. The reaction was carried out under stirring at room temperature overnight under nitrogen protection. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, concentrated, and dried to provide 0.8 g of a solid product.

19.21 Preparation of **87-46**

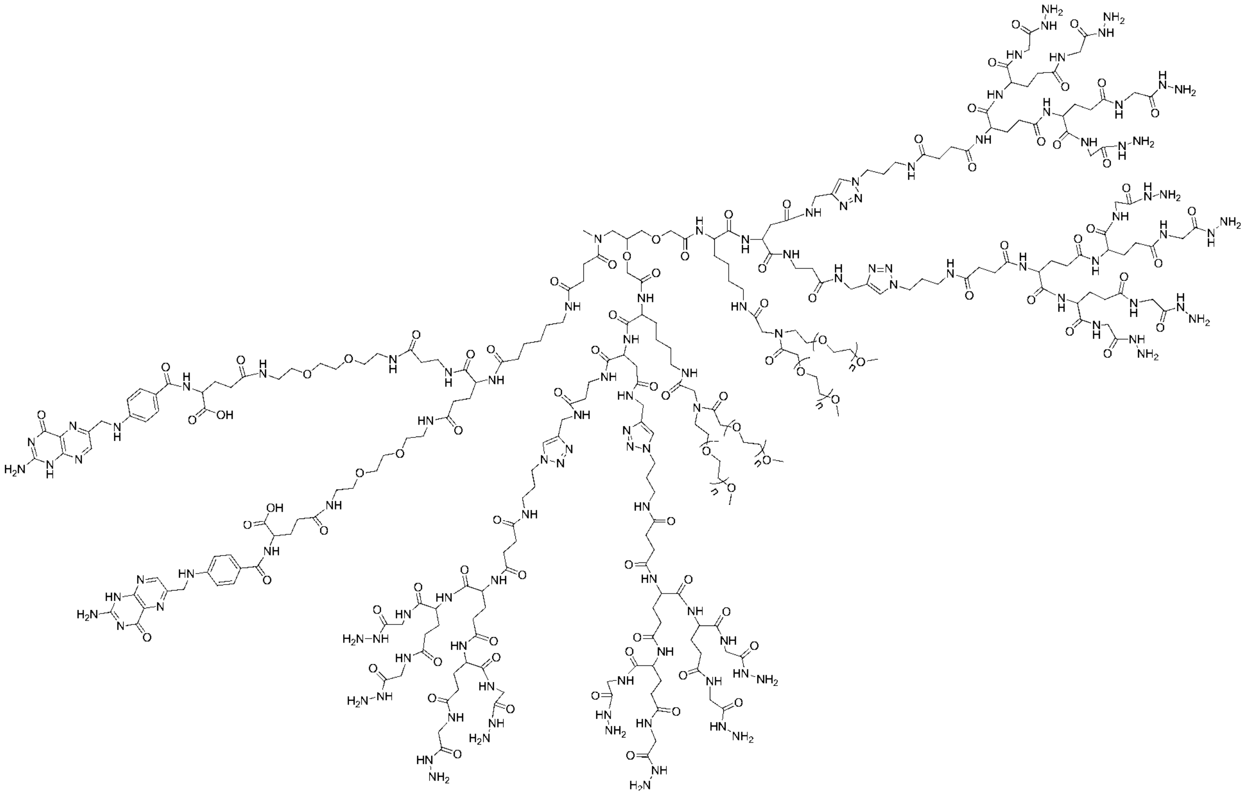

To a flask containing **87-43** (0.80 g, 0.022 mmol) was added dichloromethane. After dissolution with the assistance of ultrasonication, TFA (5 mL) was added, and the reaction mixture was stirred at room temperature overnight. After the reaction was completed, the reaction mixture was evaporated to provide an oil using a rotary evaporator. Methyl tert-butyl ether (60 mL) was then added, and a powdery solid precipitated from the reaction mixture and was filtered. The filter cake was washed with MTBE (40 mL × 3). The product had poor solubility. The filter cake was collected and dried in a vacuum oven to provide the product.

19.22 Preparation of **87-47**

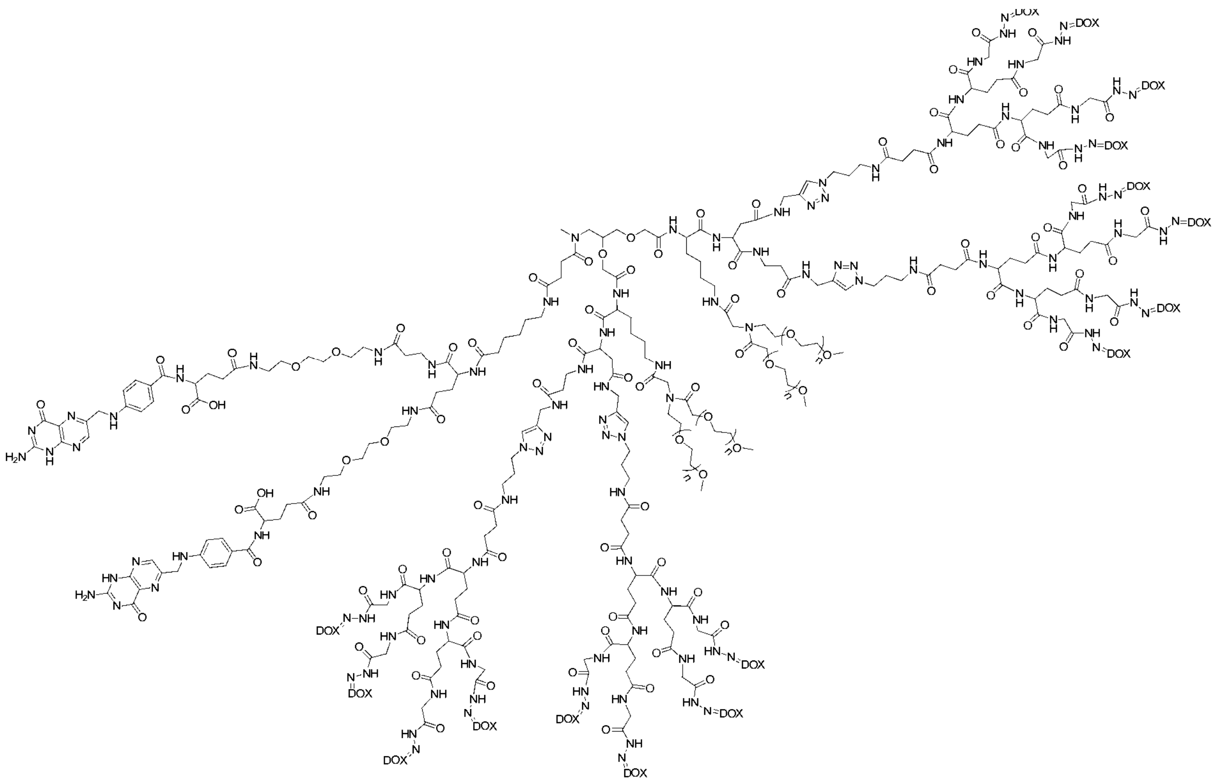

**87-46** (0.022 mmol), doxorubicin hydrochloride (0.26 g, 0.44 mmol) and methanol (10 mL) were added to a 100 mL round-bottom flask, and then TFA (0.3 mL) was added for dissolution with the assistance of ultrasonication. The reaction was carried out under stirring in the dark at room temperature. After the reaction was completed, the TFA and methanol were removed by rotary evaporation under reduced pressure. An appropriate amount of an anhydrous ethanol/dichloromethane (1/9) mixture was added for dissolution. Methyl tert-butyl ether was then added for precipitation. This process was repeated several times until the product became a fine solid powder, which was dried to provide 0.48 g of the product. $^1$H-NMR (600 MHz, DMSO-$d_6$) δ 14.19-13.97 (m, 16H), 13.41-13.21 (m, 16H), 8.46-8.11 (m, 32H), 8.08-7.61 (m, 62H), 7.35-6.98 (m, 40H), 5.58-5.41 (m, 30H), 5.37-5.24 (m, 24H), 5.03-4.86 (m, 31H), 4.61-4.38 (m, 43H), 4.34-4.11 (m, 50H), 4.06-3.80 (m, 82H), 3.76-3.60 (m, 61H), 3.57-3.46 (m, 1128H), 3.31-3.19 (m, 37H), 3.08-2.87 (m, 35H), 2.43-2.11 (m, 68H), 2.02-1.82 (m, 39H), 1.75-1.57 (m, 32H), 1.42-1.04 (m, 76H)

Example 20: Synthesis of Compound **72-248**

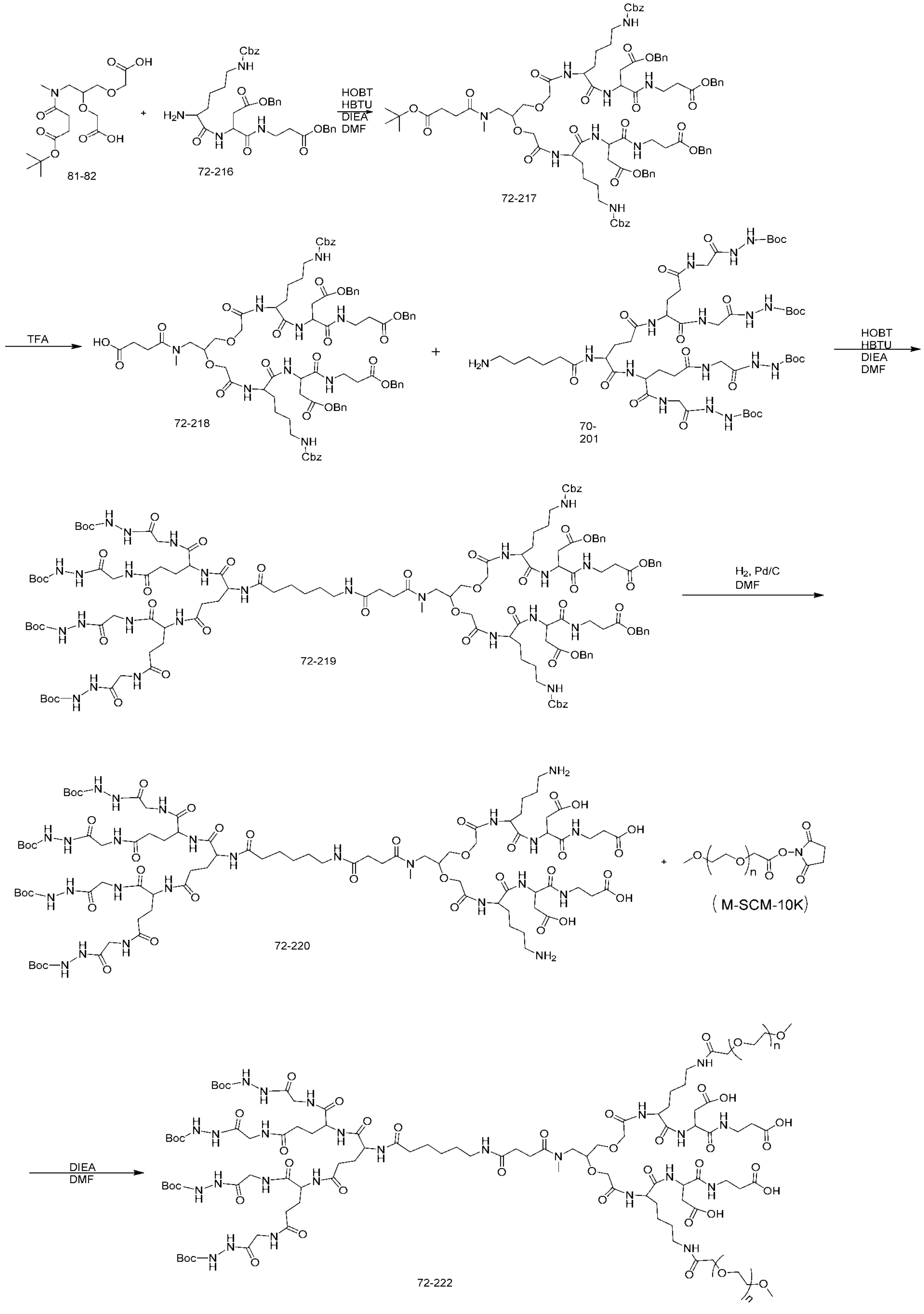


81-82 72-216 72-217 72-218 70-201 72-219 72-220 72-222

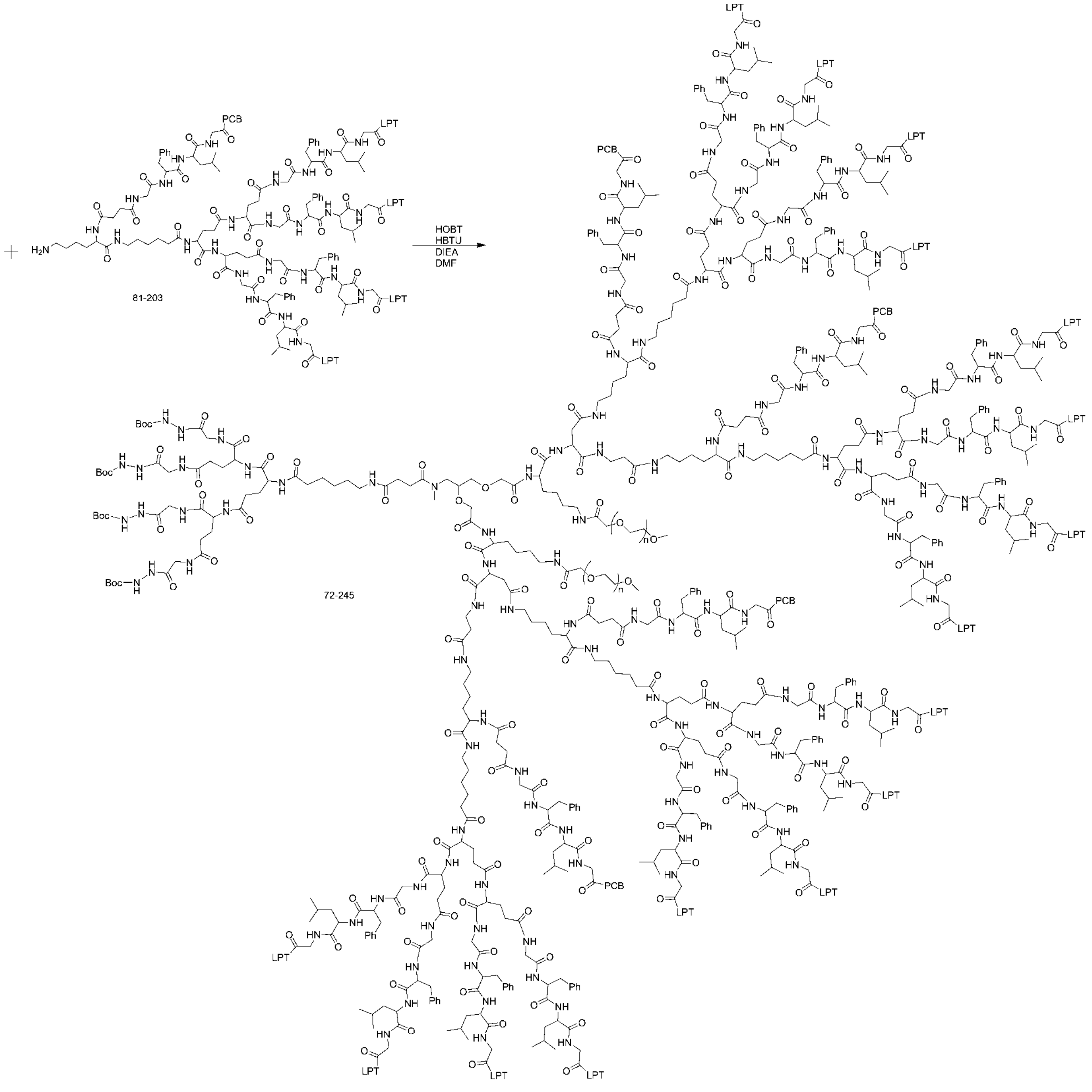


81-203

72-245

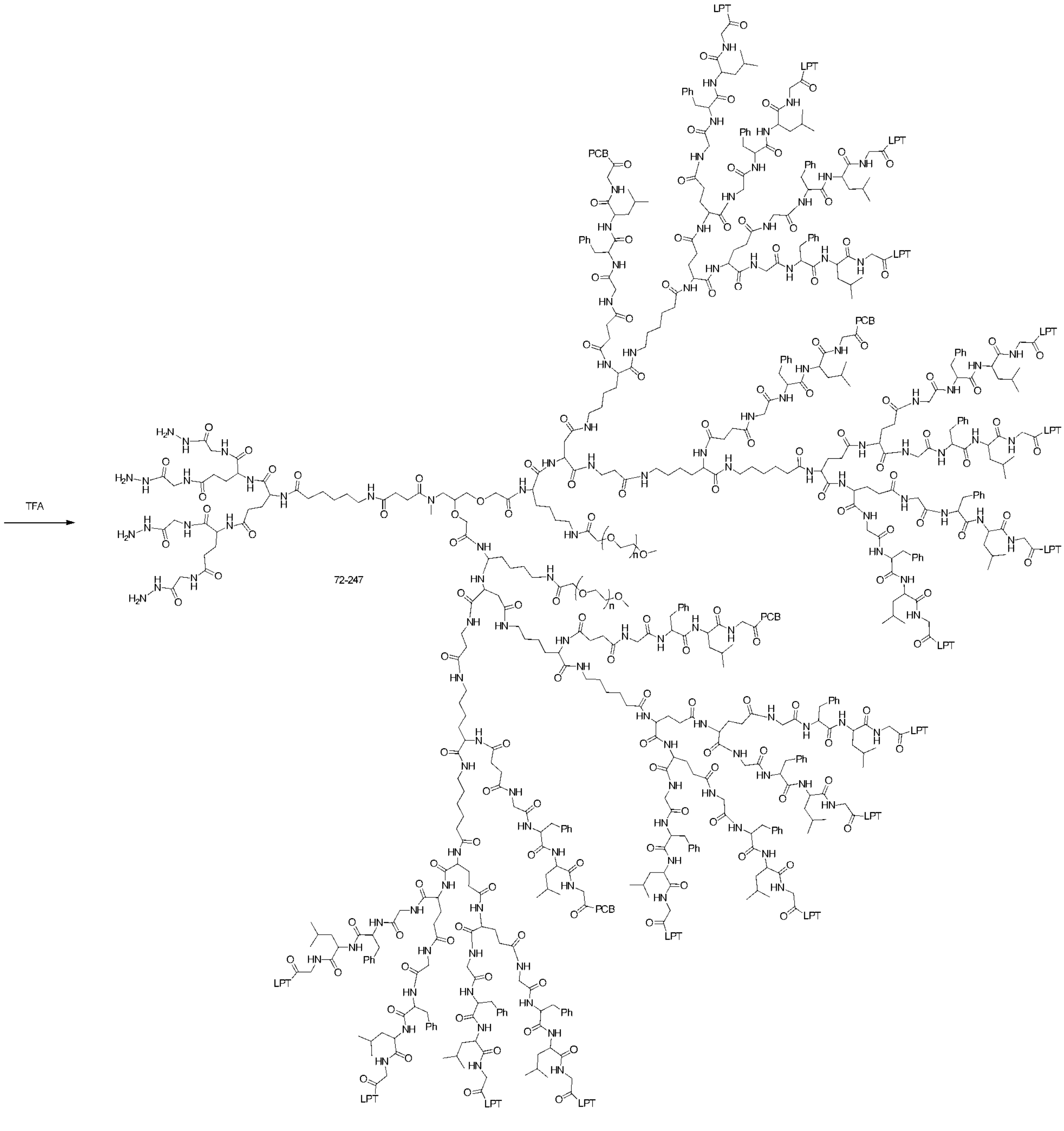


72-247

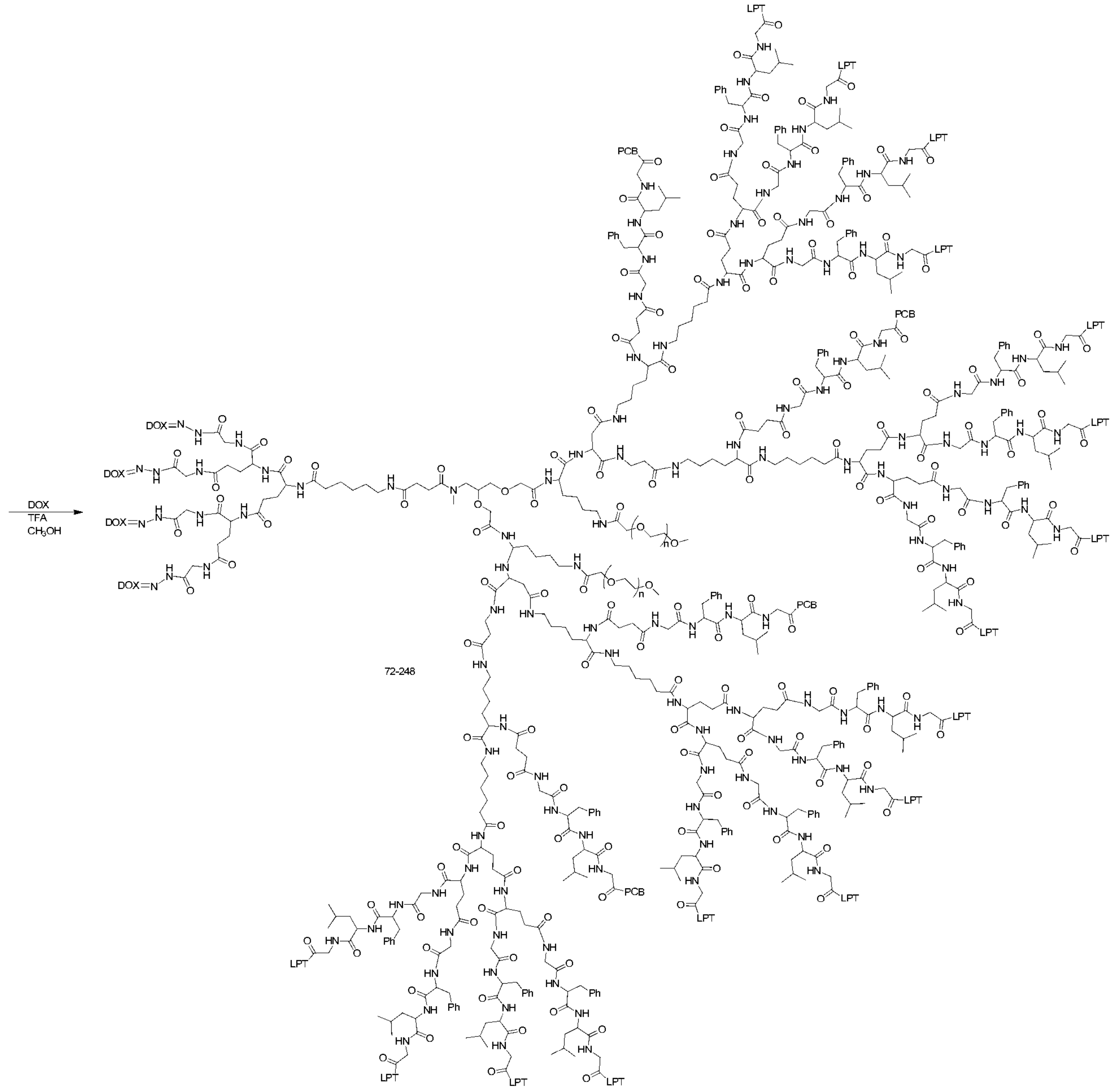


72-248

20.1 Preparation of **72-217**

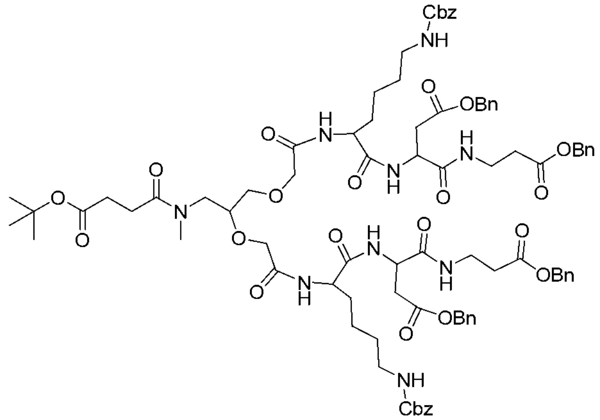

**81-82** (2.03 g, 5.38 mmol), **72-216** (8.7 g, 11.84 mmol), HBTU (6.2 g, 16.14 mmol) and HOBT (2.18 g, 16.14 mmol) were added to a 250 mL round-bottom flask, and DMF (40 mL) was added for dissolution. The reaction flask was allowed to stand at -5°C, and the mixture was stirred for approximately 20 minutes. DIEA (8 mL, 48.42 mmol) was then slowly added dropwise. After the dropwise addition was completed, the reaction flask was allowed to stand at -5°C, and the mixture was stirred for 1 hour. The flask was then brought to room temperature, and the mixture was stirred. After the reaction was completed, the reaction mixture was transferred to a 1 L separatory funnel, and ethyl acetate (200 mL) and purified water (200 mL) were added for extraction. The aqueous phase was further extracted with ethyl acetate (100 mL × 2). The organic phases were combined, concentrated, and washed three times with pure water. The organic phases were collected and dried to provide a solid product, which was dissolved in a mixed solvent of dichloromethane and methanol, loaded by dry method, and subjected to column chromatography using 1-4% methanol/dichloromethane as the eluent. The desired product was collected, concentrated, and dried in a vacuum oven to provide 6 g of the product with a yield of 92.3%. $^1$H-NMR (600 MHz, DMSO-$d_6$) δ 8.423-8.374 (m, 1H), 8.363-8.316 (m, 1H), 7.97-7.92 (m, 2H), 7.79-7.74 (m, 1H), 7.72-7.61 (m, 1H), 7.37-7.31 (m, 30H), 7.23-7.19 (m, 2H), 5.08-5.05 (m, 8H), 4.99-4.97 (m, 2H), 4.61-4.57 (m, 2H), 4.31-4.23 (m,2H), 4.08-3.98 (m, 3H), 3.96-3.89 (m, 2H), 3.75-3.40 (m, 7H), 3.35-3.17 (m, 6H), 3.00-2.98 (m, 3H), 2.96-2.93 (m, 4H), 2.83-2.82 (m, 1H), 2.80-2.78 (m, 1H), 2.77-2.75(m, 1H), 2.65-2.53 (m, 4H), 2.49-2.46 (m, 4H), 2.39-2.36 (m, 2H), 1.69-1.59 (m, 2H), 1.58-1.47 (m, 2H), 1.36-1.34 (m, 9H), 1.26-1.20 (m, 4H)

20.2 Preparation of **72-218**

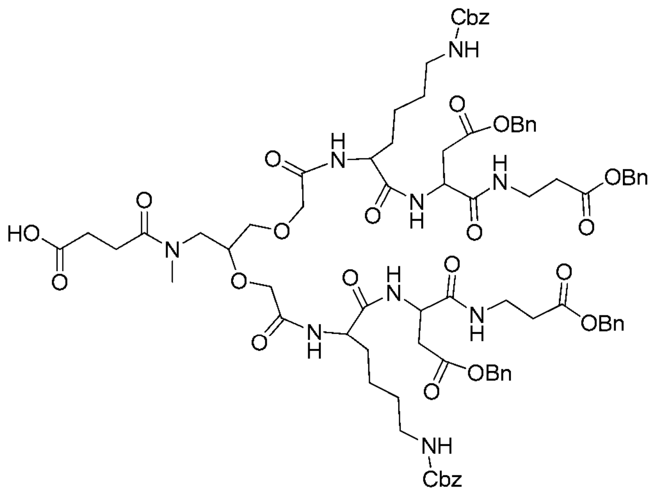

**72-217** (4 g, 2.448 mmol) was added to a 250 mL round-bottom flask, then TFA (3.6 mL, 48.96 mmol) was added, and the mixture was reacted under stirring at room temperature. After the reaction was completed, the reaction mixture was evaporated to provide an oil using a rotary evaporator. Methyl tert-butyl ether (60 mL) was then added, and a powdery solid precipitated from the reaction mixture and was filtered. The filter cake was washed with methyl tert-butyl ether (40 mL × 3). The filter cake was collected and dried in a vacuum oven to provide 3.2 g of the product with a yield of 82.9%.

20.3 Preparation of **72-219**

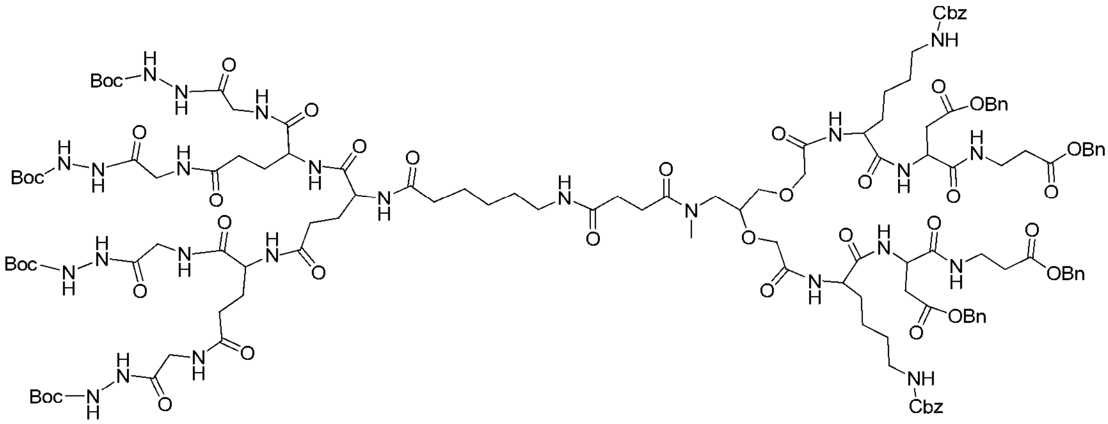

**72-218** (2 g, 1.2668 mmol), **70-201** (2.286 g, 1.900 mmol), HBTU (0.72 g, 1.900 mmol) and HOBT (0.256 g, 0.9923 mmol) were added to a 100 mL round-bottom flask, and DMF (50 mL) was added for dissolution. The reaction flask was allowed to stand at room temperature, and the mixture was stirred for approximately 20 minutes. DIEA (0.94 mL, 5.700 mmol) was then slowly added dropwise. After the dropwise addition was completed, the reaction was continuously carried out under stirring at room temperature. After the reaction was completed, n-hexane (30 mL) and methyl tert-butyl ether (200 mL) were added for precipitation, the supernatant was discarded, and n-hexane and methyl tert-butyl ether were added for precipitation. This process was repeated four times. The mixture was filtered to provide a solid product, which was dried to provide 3 g of the product.

20.4 Preparation of **72-220**

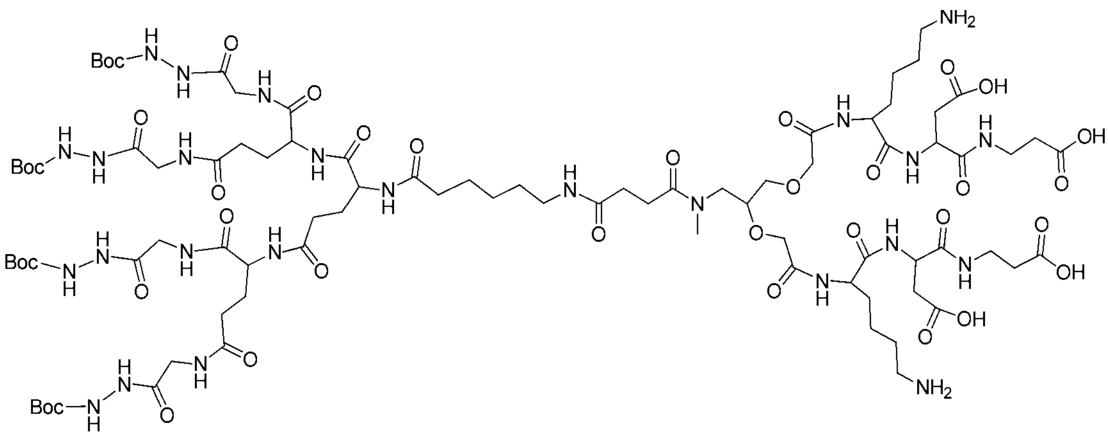

**72-219** (2 g, 0.723 mmol) was dissolved in DMF (30 mL) in a reaction kettle, then 10% Pd/C catalyst (600 mg) was added. The reaction kettle was evacuated using a water pump and hydrogen was introduced. This process was repeated three times. The hydrogen pressure was finally adjusted to 1.8 MPa. The reaction was carried out under stirring at high speed at 50°C. After the reaction was completed, the reaction mixture was transferred to a filter funnel filled with filter paper using a rubber-tipped pipette. After filtration, the filtrate was collected and repeatedly precipitated with large amounts of n-hexane and methyl tert-butyl ether until a clear solid was formed. Dichloromethane and toluene were added, and the mixture was concentrated using a rotary evaporator and dried to provide 1.5 g of the product.

20.5 Preparation of **72-222**

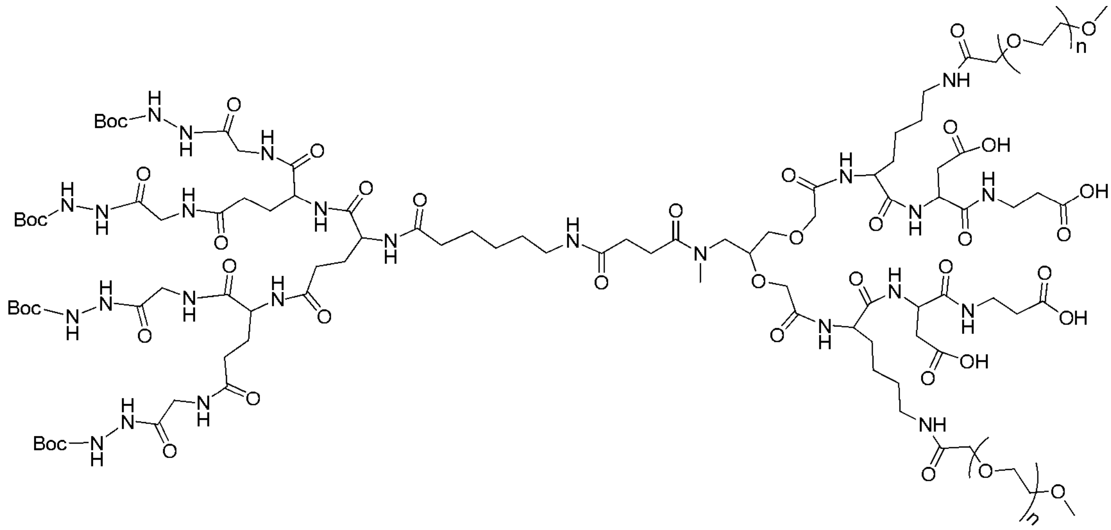

**72-220** (0.3 g, 0.0702 mmol) was placed in a 250 mL flask, dissolved in DMF (30 mL), and stirred at room temperature for 10 minutes. DIEA (0.96 mL, 5.602 mmol) was then slowly added dropwise. After stirring at -15°C for 30 minutes, M-SCM-10K (3 g, 0.2808 mmol, purchased from JenKem, Lot Number: ZZ390P163) was added, and the reaction was stirred very slowly in the dark at room temperature for one week. After the reaction was completed, methyl tert-butyl ether (200 mL) and n-hexane (70 mL) were added. A solid precipitated and was filtered. The filter cake was washed with methyl tert-butyl ether (40 mL × 3). The filter cake was collected and dried in a vacuum oven to provide 2.5 g of the product.

20.6 Preparation of **72-245**

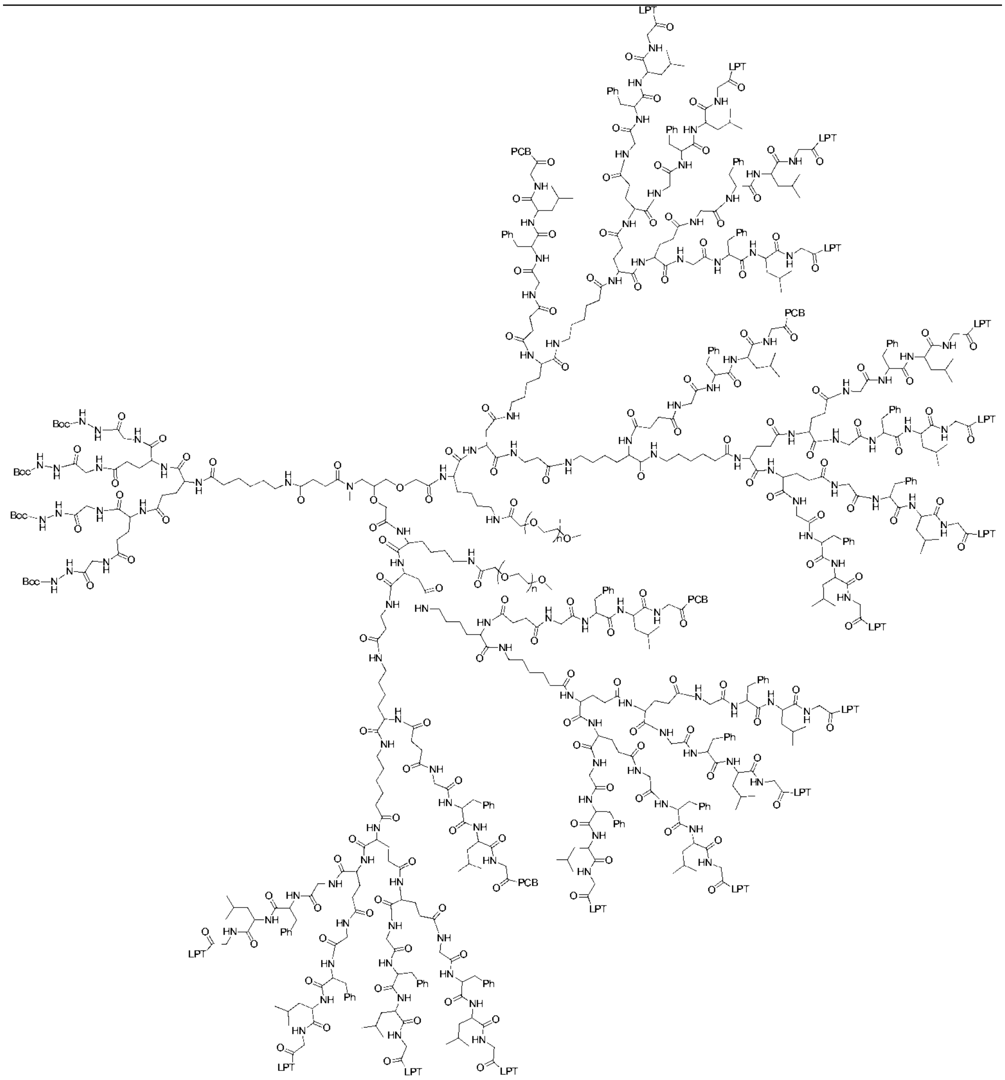

**72-222** (2.5 g, 0.1066 mmol), **81-203** (0.75 g, 0.64 mmol), HBTU (0.085 g, 0.64 mmol) and HOBT (0.335 g, 0.64 mmol) were added to a 100 mL round-bottom flask, and dissolved in DMF (50 mL). The reaction flask was allowed to stand at room temperature, and the mixture was stirred for approximately 20 minutes. DIEA (0.08 mL, 0.4797 mmol) was then slowly added dropwise. After the dropwise addition was completed, the reaction was continued under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to provide a solid product, which was dried to provide 2.1 g of the product.

20.7 Preparation of **72-247**

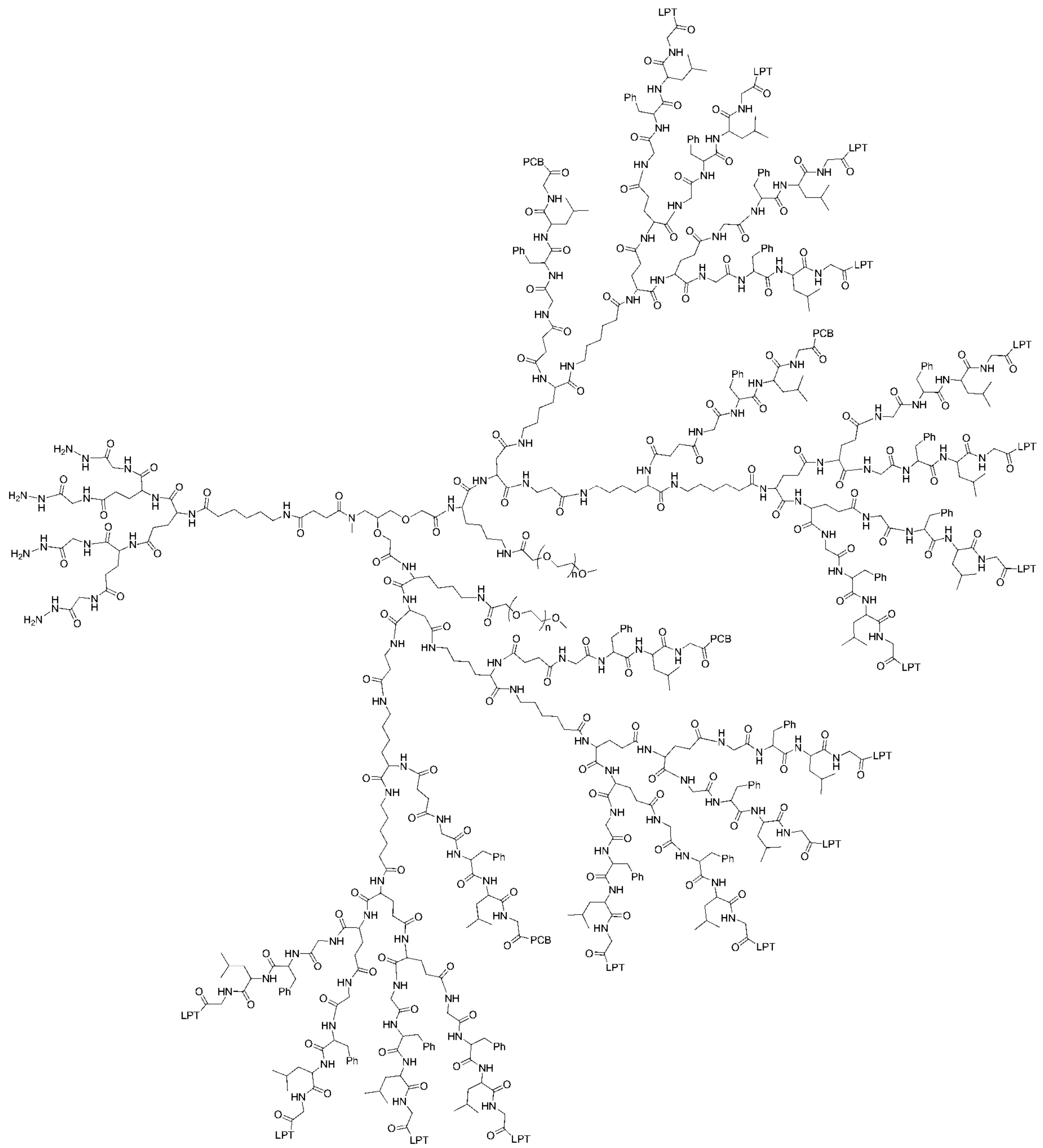

**72-245** (0.096 g, 0.0216 mmol) was added to a 100 mL round-bottom flask, then TFA (0.03 mL, 0.433 mmol) was added, and the mixture was reacted under stirring at room temperature. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3) and then dried in a vacuum oven to provide 0.087 g of the product. $^1$H-NMR (600 MHz, DMSO-$d_6$) δ 11.03-10.71 (m, 20H), 8.86-8.77 (m, 40H), 8.31-7.98 (m, 176H), 7.94-7.92 (m, 16H), 7.87-7.82 (m, 28H), 7.68-7.65 (m, 20H), 7.49-7.44 (m, 20H), 7.35-7.31 (m, 60H), 7.25-7.17 (m, 144H), 6.71-6.56 (m, 16H), 4.80-4.66 (m, 40H), 4.60-4.53 (m, 20H), 4.36-4.32 (m, 24H), 4.24-4.15 (m, 51H), 3.78-3.60 (m, 112H), 3.55-3.48 (m, 1910H), 3.22-3.16 (m, 22H), 2.90-2.82 (m, 47H), 2.78-2.68 (m, 39H), 2.62-2.61 (m, 2H), 2.45-2.27 (m, 48H), 2.18-2.07 (m, 58H), 1.85-1.67 (m, 48H), 1.62-1.43 (m, 120H), 1.37-1.33 (m, 26H), 0.89-0.79 (m, 120H)

20.8 Preparation of **72-248**

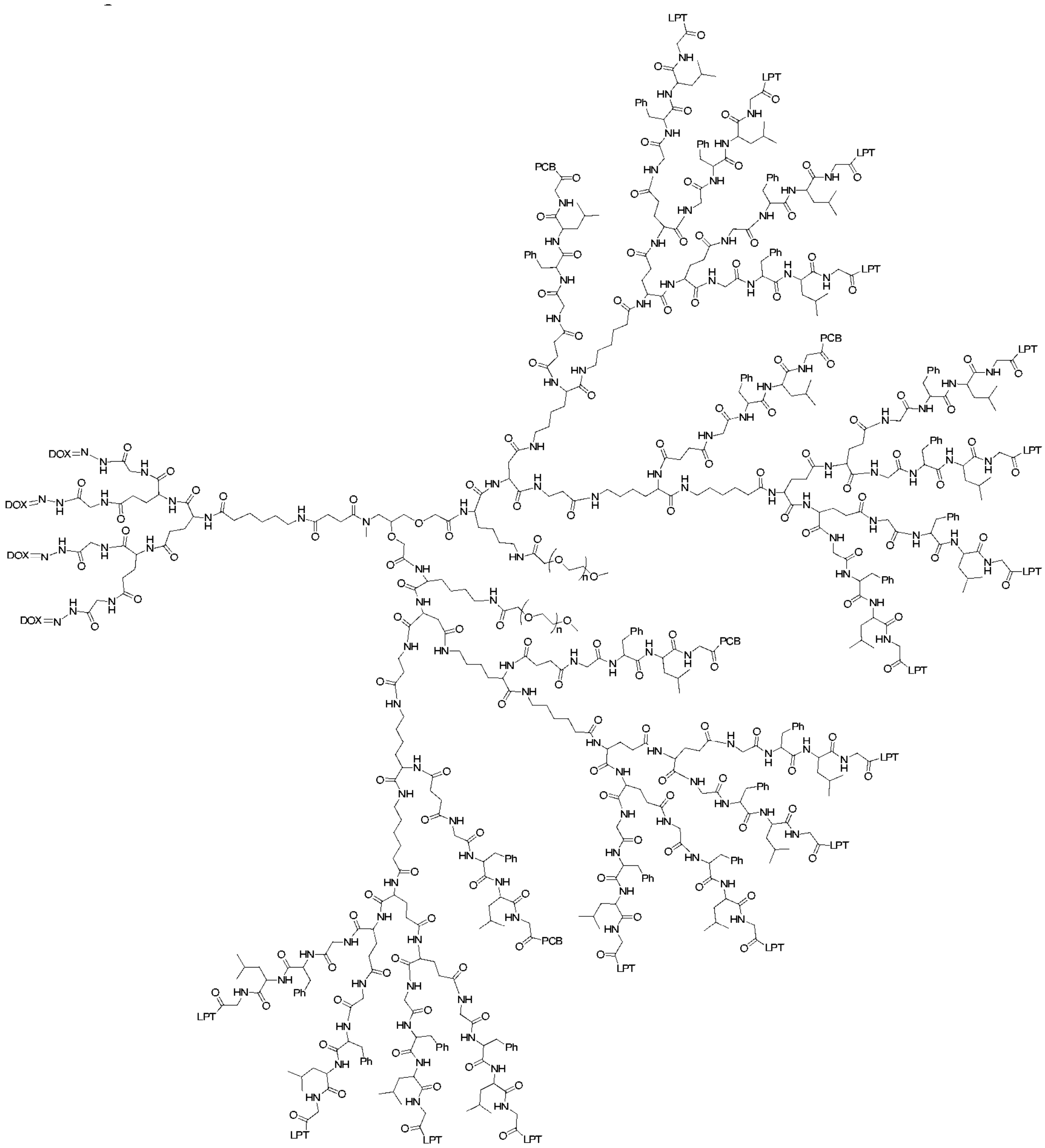

**72-247** (0.087 g, 0.00198 mmol) was added to a 250 mL round-bottom flask, methanol (3 mL) was added for dissolution with the assistance of ultrasonication, then doxorubicin hydrochloride (0.005 g, 0.008718 mmol, abbreviated as DOX) was added. After dissolution with the assistance of ultrasonication, the reaction was carried out under stirring at room temperature. After the reaction was completed, the mixture was evaporated to dryness, and n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to provide a solid product. The obtained solid product was dissolved in a mixture of dichloromethane and methanol, followed by the addition of methyl tert-butyl ether for precipitation, and the mixture was filtered. This process was repeated three times. The filter cake was collected and dried in a vacuum oven to provide 0.09 g of the product. $^1$H-NMR (600 MHz, DMSO-$d_6$) $\delta$ 10.08-9.89 (m, 20H), 8.78-8.73 (m, 20H), 8.61-8.55 (m, 20H), 8.21-7.98 (m, 176H), 7.88-7.76 (m, 56H), 7.73-7.68 (m, 20H), 7.49-7.44 (m, 20H), 7.25-7.07 (m, 204H), 6.71-6.56 (m, 8H), 5.52-5.41 (m, 8H), 4.82-4.67 (m, 48H), 4.64-4.51 (m, 28H), 4.43-4.31 (m, 32H), 4.3-4.05 (m, 63H), 3.83-3.81 (m, 144H), 3.55-3.48 (m, 1910H), 3.22-3.16 (m, 22H), 3.07-3.04 (m, 47H), 2.78-2.68 (m, 49H), 2.20-2.04 (m, 110H), 1.78-1.67 (m, 52H), 1.53-1.40 (m, 120H), 1.21-1.16 (m, 38H), 0.89-0.79 (m, 120H)

Example 21: Synthesis of Compound **72-279**

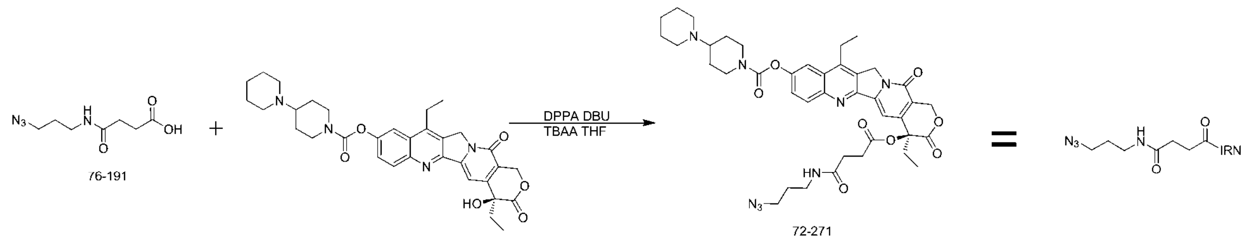


76-191

72-271

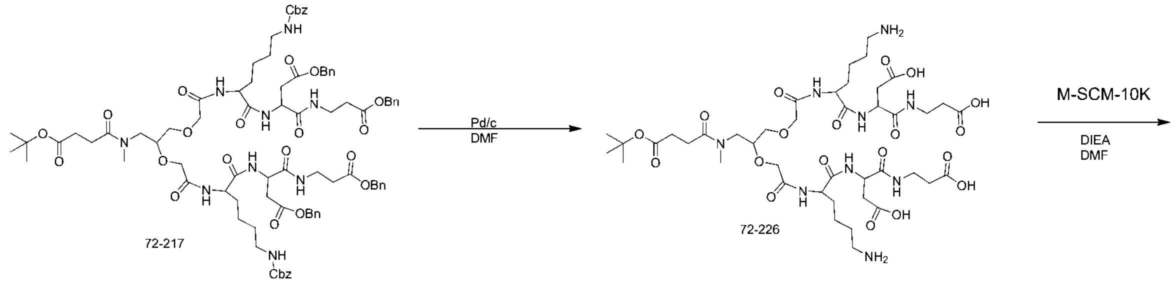


72-217

72-226

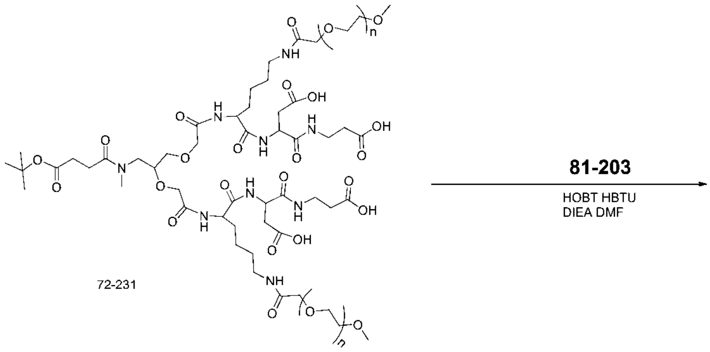


72-231

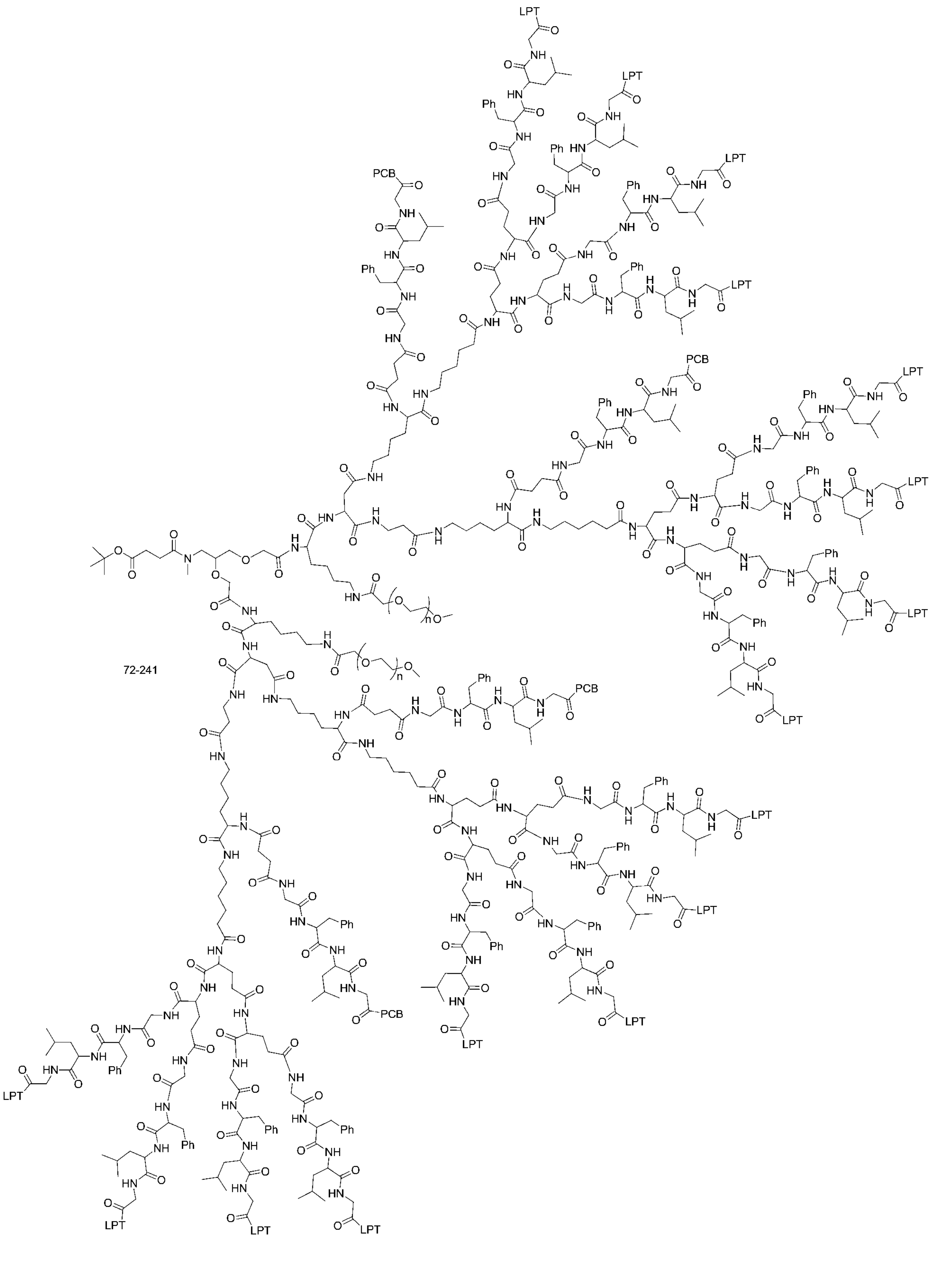

72-241

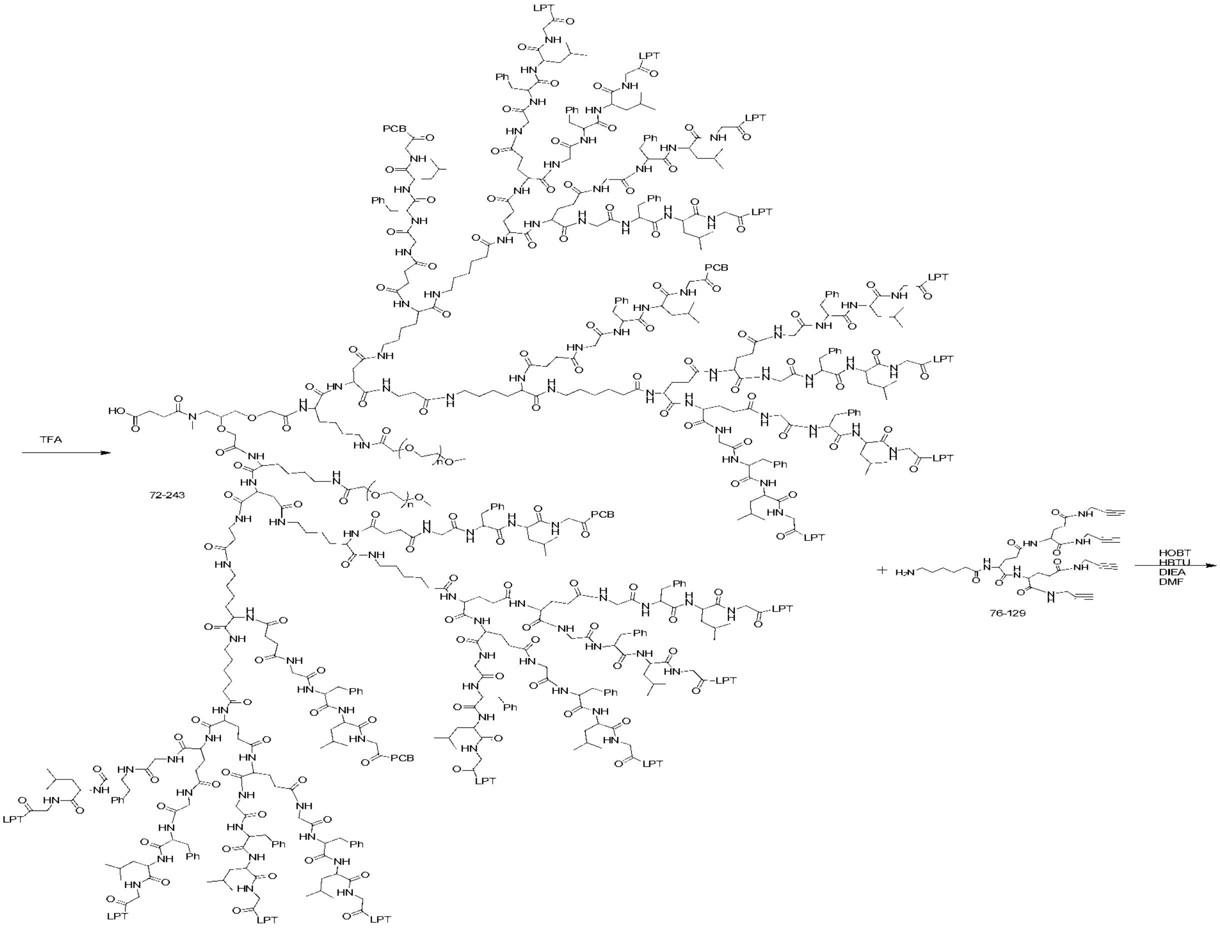


72-243

76-129

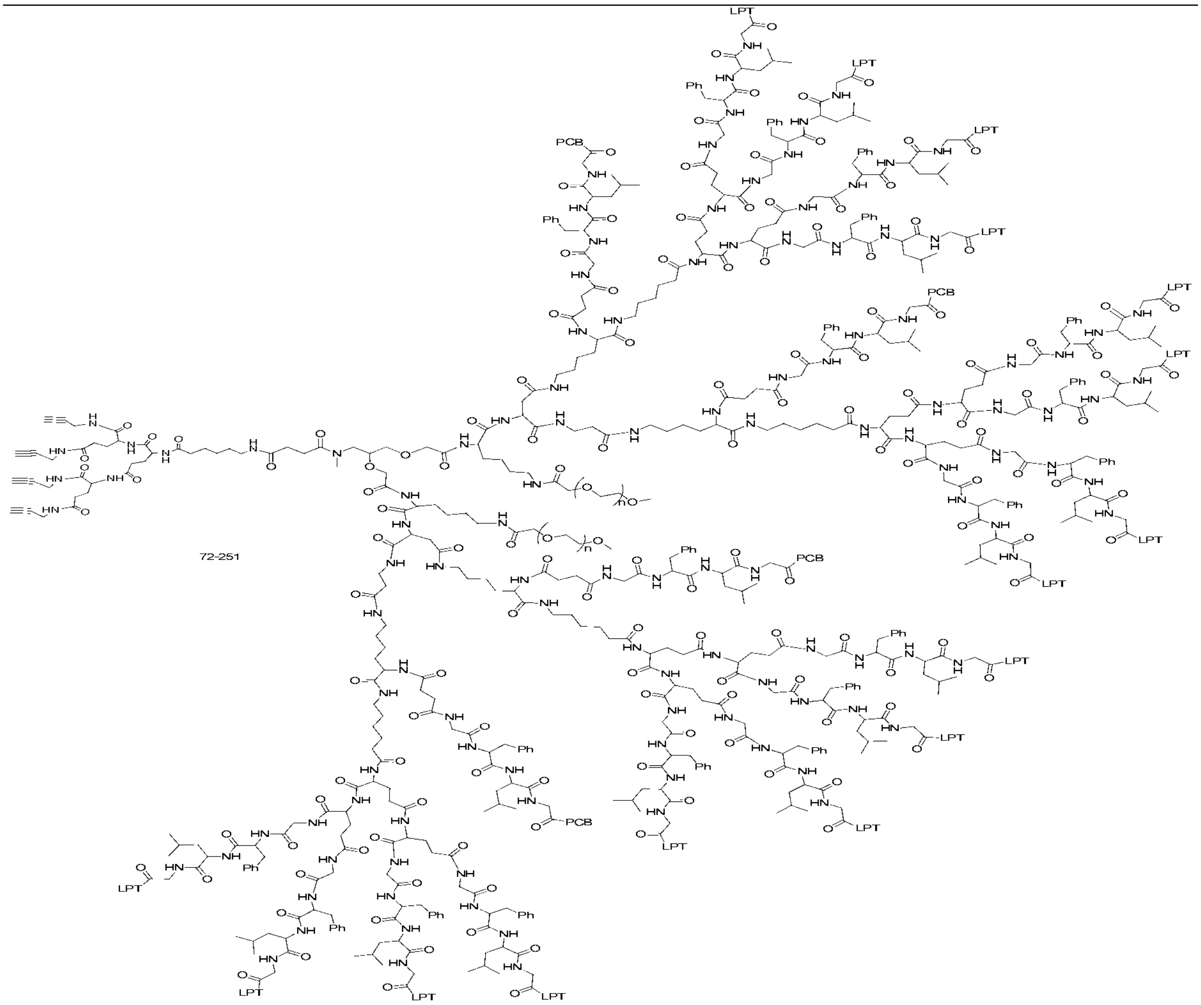

72-251

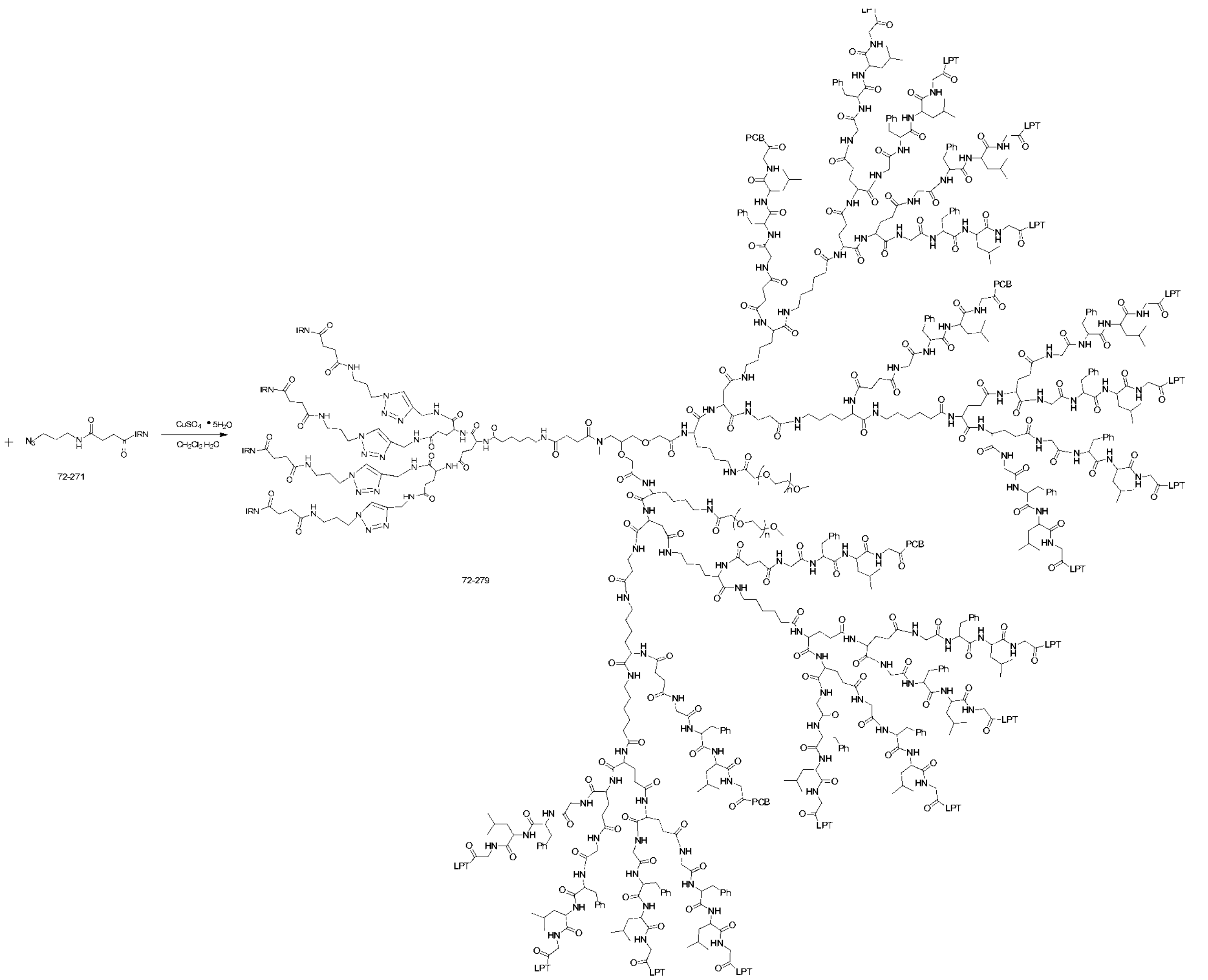


72-271

72-279

21.1 Preparation of **72-271**

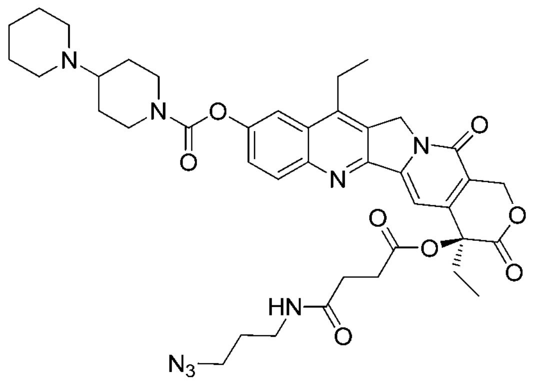

**76-191** (0.297 g, 1.79 mmol), irinotecan (0.807 g, 1.377 mmol, purchased from Innochem) and DMAP (0.04 g, 0.2754 mmol) were added to a 250 mL flask, dichloromethane (20 mL) was added to dissolve the mixture, and the mixture was stirred at 0°C for 20 minutes. DCC (1.42 g, 6.885 mmol) was then added and stirred for another 30 minutes. The flask was taken out, and the reaction was carried out under stirring at room temperature overnight. After the reaction was completed, the mixture was filtered and rotary evaporated to dryness. The resulting product was dissolved in a dichloromethane/methanol (4/1) mixture, loaded by dry method, and subjected to column chromatography using 3-6% methanol/dichloromethane as the eluent. The desired product was collected, concentrated, and dried in a vacuum

oven to provide 0.726 g of the product. ESI [M+H+] 769.36444

21.2 Preparation of **72-226**

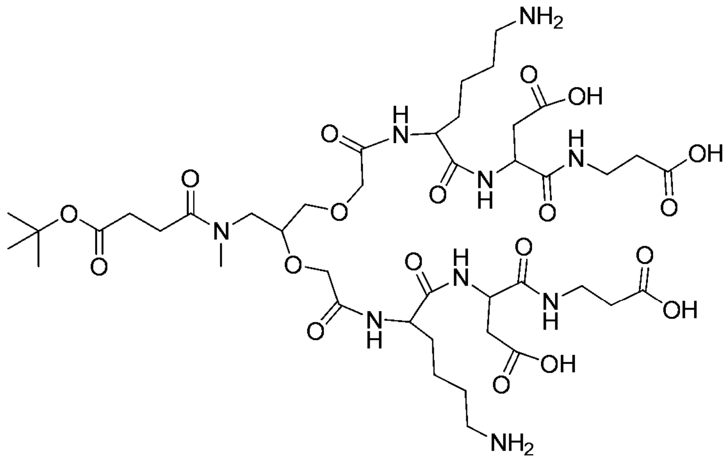

**72-217** (2 g, 1.2233 mmol) was dissolved in DMF (30 mL) and placed in a reaction kettle, and then 10% Pd/C catalyst (600 mg) was added. A water pump was connected to evacuate the reaction kettle, and hydrogen was introduced. This process was repeated three times. The hydrogen pressure was finally adjusted to 1.8 MPa. The reaction was carried out under stirring at high speed at 50°C. After the reaction was completed, the reaction mixture was transferred to a filtration funnel filled with filter paper using a rubber-tipped pipette. After filtration, the filtrate was collected and repeatedly precipitated with large amounts of n-hexane and methyl tert-butyl ether until a clear solid was formed, to which were added dichloromethane and toluene, and rotary evaporated and dried to provide 1.1 g of the product.

21.3 Preparation of **72-231**

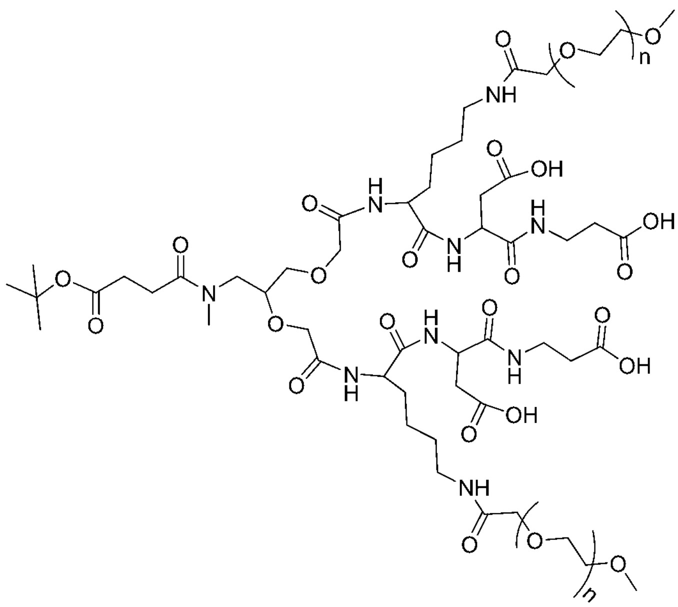

**72-226** (0.047 g, 0.0469 mmol) was placed in a 250 mL flask, DMF (30 mL) was added for dissolution, and the mixture was stirred at room temperature for 10 minutes. DIEA (0.155 mL, 0.9387 mmol) was then slowly added dropwise. After stirring at -15°C for 30 minutes, M-SCM-10K (1 g, 0.0939 mmol, purchased from JenKem, Lot Number: ZZ390P163) was added. The reaction was carried out under stirring in the dark at room temperature for one week. After the reaction was

completed, methyl tert-butyl ether (200 mL) and n-hexane (70 mL) were added. A solid precipitated and was filtered. The filter cake was washed with methyl tert-butyl ether (40 mL × 3). The filter cake was collected and dried in a vacuum oven to provide 0.98 g of the product with a yield of 94.69%.

21.4 Preparation of **72-241**

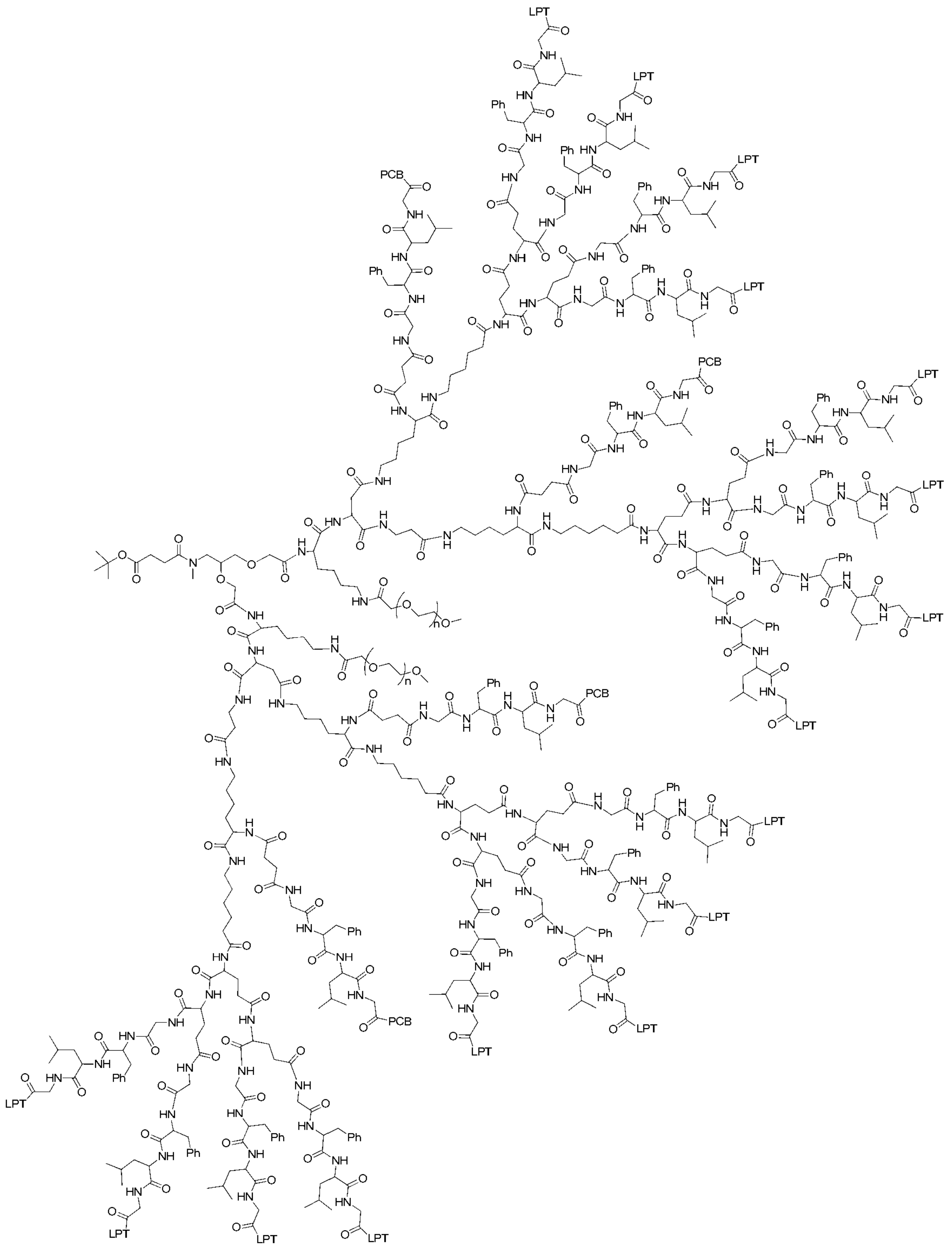

**72-231** (2.5 g, 0.112 mmol), **81-203** (3.56 g, 0.672 mmol), HBTU (0.25 g, 0.672 mmol) and HOBT (0.09 g, 0.672 mmol) were added to a 100 mL round-bottom flask, then DMF (50 mL) was added for dissolution, and the mixture was

stirred at room temperature for approximately 20 minutes. DIEA (0.3 mL, 2.016 mmol) was then slowly added dropwise. After the dropwise addition was completed, the reaction was continued under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to provide a solid product, which was dried to provide 2.1 g of the product.

21.5 Preparation of **72-243**

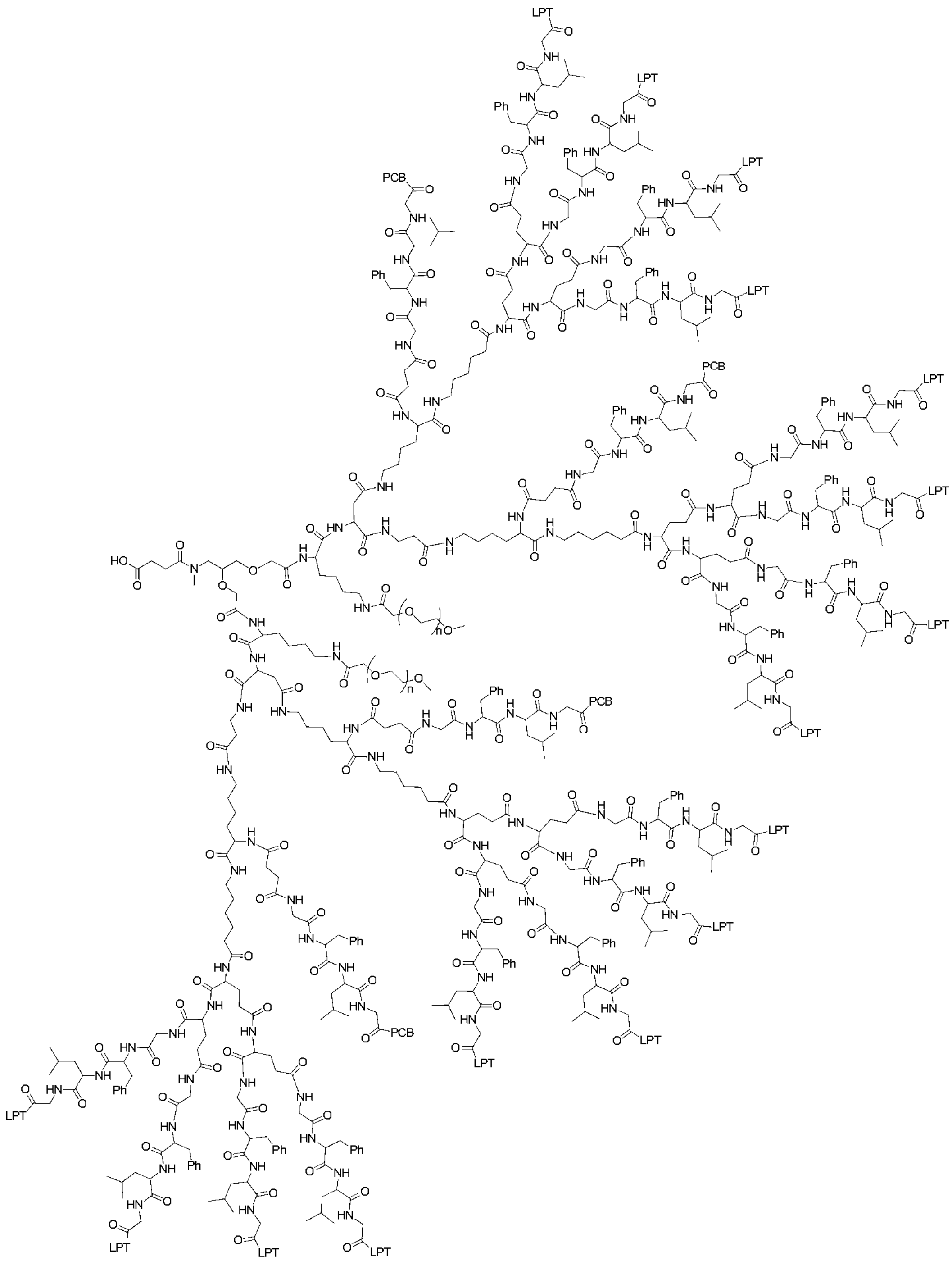

**72-241** (2.1 g, 0.094 mmol) was added into a 250 mL round-bottom flask, then TFA (0.14 mL, 1.8816 mmol) was added, and the mixture was reacted under stirring at room temperature. After the reaction was completed, the reaction

mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide 2 g of the product.

21.6 Preparation of **72-251**

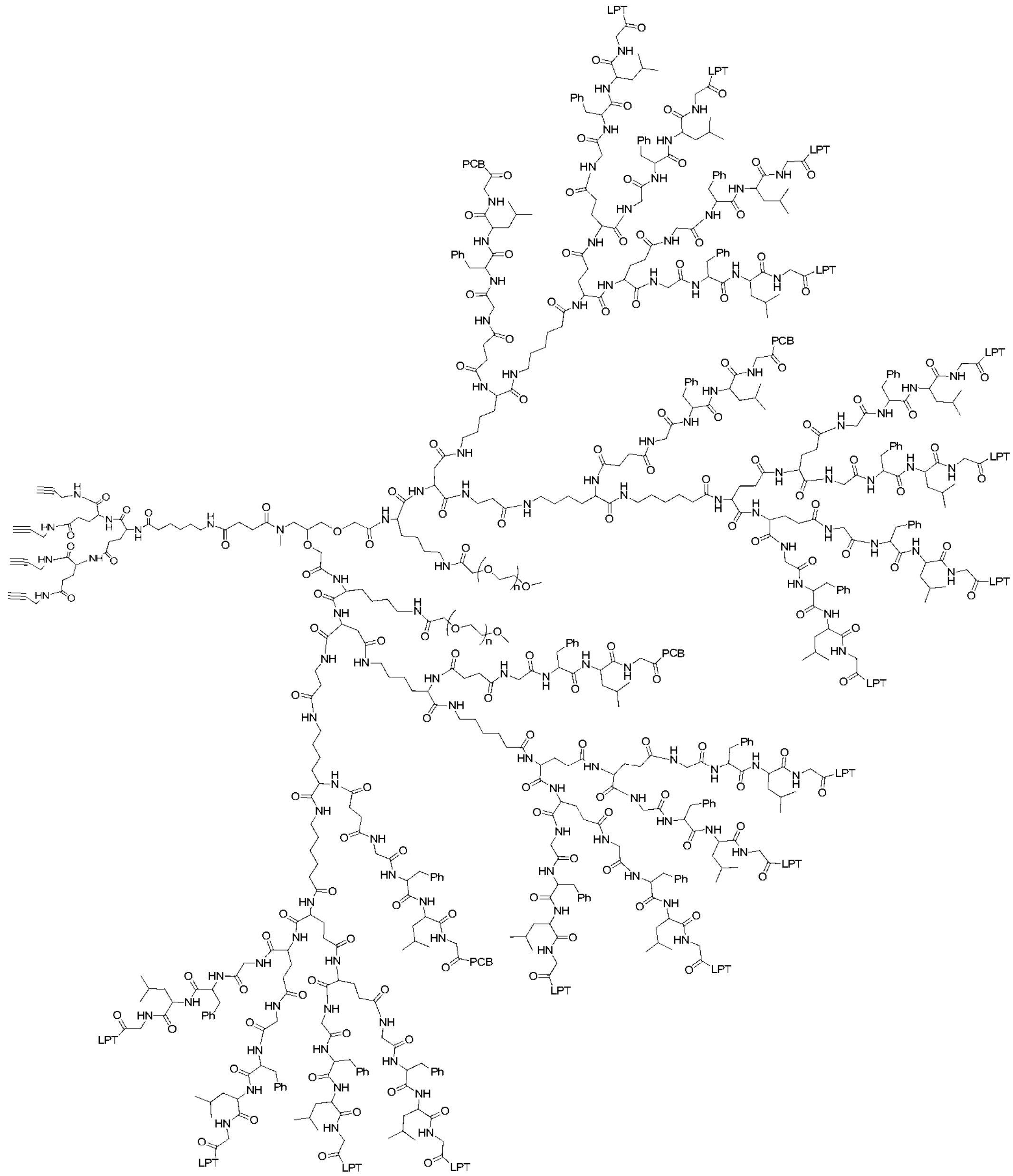

**72-243** (0.9 g, 0.0213 mmol), **76-129** (0.05 g, 0.04365 mmol), HBTU (0.009 g, 0.04365 mmol) and HOBT (0.003 g, 0.04367 mmol) were added to a 250 mL round-bottom flask, then DMF (40 mL) was added for dissolution, and the mixture was stirred at room temperature for approximately 20 minutes. DIEA (0.03 mL, 0.0261 mmol) was then slowly added dropwise. After the dropwise addition was completed, stirring was continued at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to provide a solid product. The obtained solid product was dissolved in a mixed solvent of dichloromethane and methanol, then methyl tert-butyl ether was added for precipitation, and the mixture was filtered. This process was repeated three times. The solid was finally dried in a vacuum oven to provide 0.8 g of the product. $^1$H-NMR

(600 MHz, DMSO-$d_6$) δ 9.92-9.81 (m, 20H), 8.77-8.71 (m, 20H), 8.58-8.53 (m, 20H), 8.21-8.00 (m, 176H), 7.86-7.67 (m, 56H), 7.50-7.45 (m, 22H), 7.35-7.18 (m, 180H), 7.15-7.07 (m, 24H), 6.72-6.54 (m, 6H), 4.77-4.69 (m, 40H), 4.60-4.55 (m, 20H), 4.28-4.33 (m, 24H), 4.25-4.17 (m, 64H), 4.03-3.97 (m, 14H), 3.86-3.76 (m, 113H), 3.57-3.47 (m, 1910H), 3.03-3.00 (m, 52H), 2.87-2.71 (m,36H), 2.47-2.04 (m, 94H), 1.92-1.66 (m, 52H), 1.63-1.42 (m, 124H), 1.27-1.13 (m, 32H), 0.90-0.81 (m, 120H)

21.7 Preparation of **72-279**

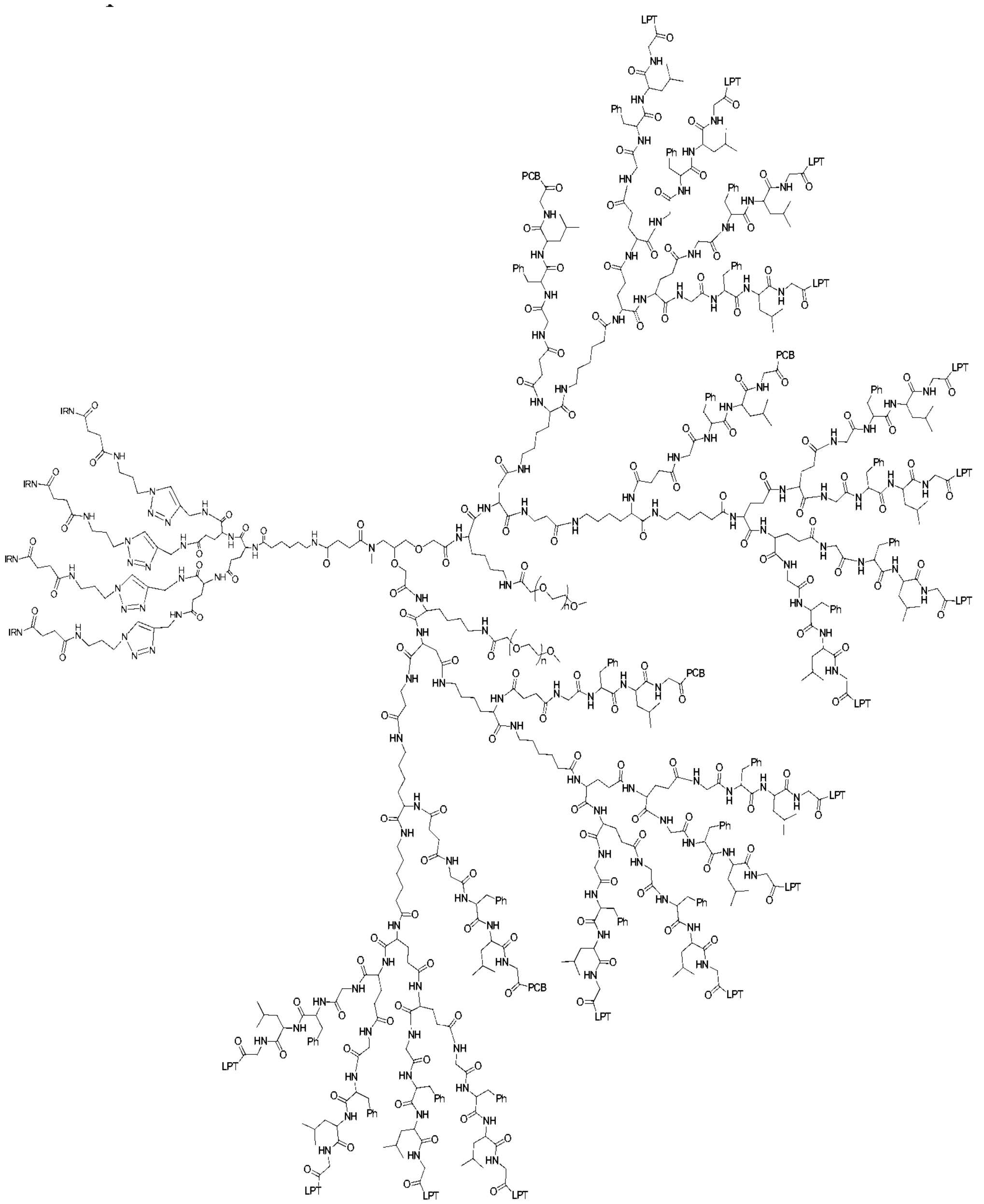

**72-251** (0.56 g, 0.0127 mmol) and **72-271** (0.07 g, 0.0127 mmol) were placed in a reaction flask, and anhydrous DMF (20 mL) was added for dissolution. The flask was then filled with nitrogen. $CuSO_4$ (0.03 g, 0.204 mmol) and sodium ascorbate (0.08 g, 0.409 mmol) were then added and stirred at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated five times. The mixture was filtered to provide a solid product, which was collected and dried to provide 0.3 g of the product. $^1$H-NMR (600 MHz, DMSO-$d_6$) δ 10.10-9.56 (m, 20H), 8.90-8.46 (m, 40H), 8.10-7.90 (m, 180H), 7.86-7.77 (m, 60H), 7.48-7.43

(m, 26H), 7.25-7.15 (m, 188H), 7.13-7.11 (m, 24H), 6.70-6.65 (m, 10H), 4.90-4.64 (m, 48H), 4.61-4.59 (m, 28H), 4.36-4.33 (m, 32H), 4.23-4.16 (m, 64H), 4.00-3.73 (m, 127H), 3.57-3.47 (m, 1910H), 3.03-3.00 (m, 106H), 2.85-2.80 (m, 36H), 2.74-2.72 (m, 20H), 2.41-2.32 (m, 50H), 2.15-2.05 (m, 94H), 1.89-1.80 (m, 60H), 1.55-1.45 (m, 112H), 1.28-1.20 (m, 44H), 0.87-0.80 (m, 132H)

Example 22: Synthesis of Compound **87-40**

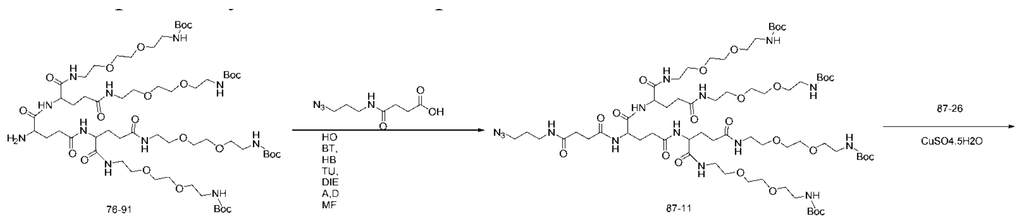


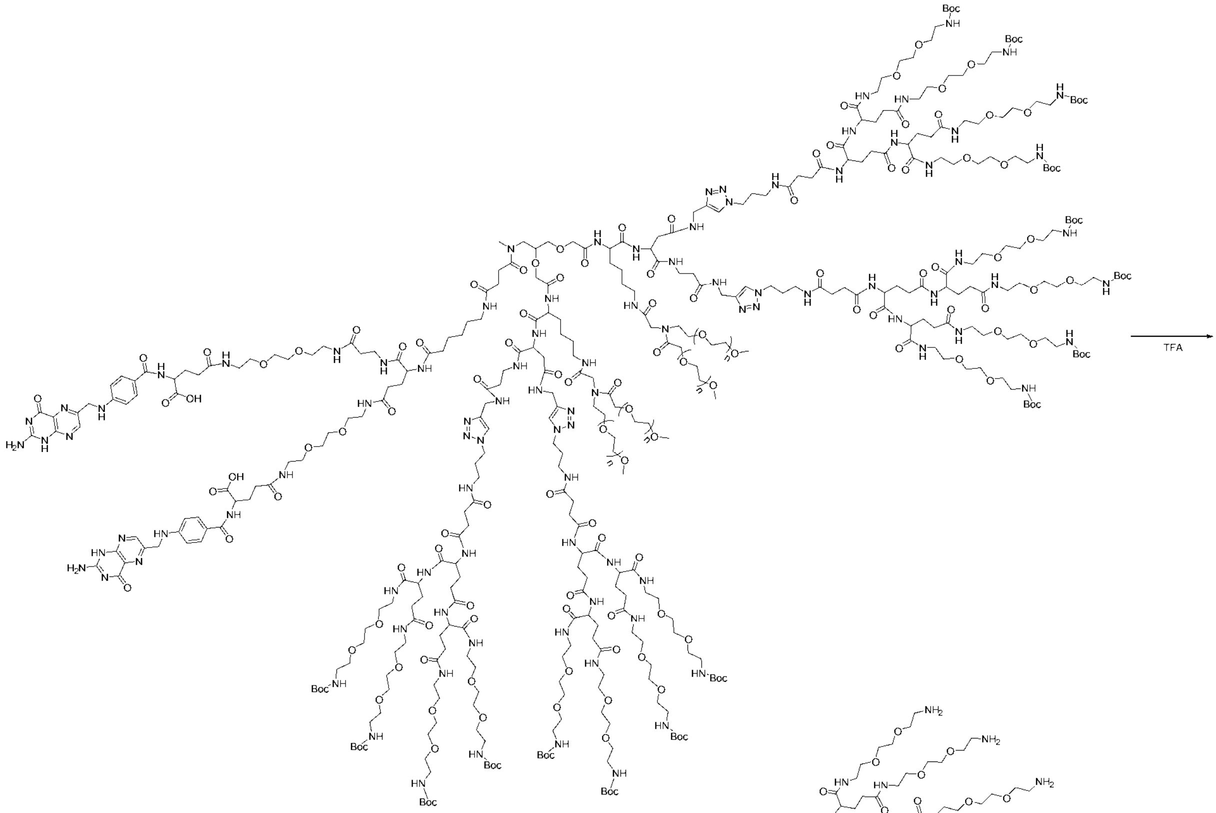


87-30

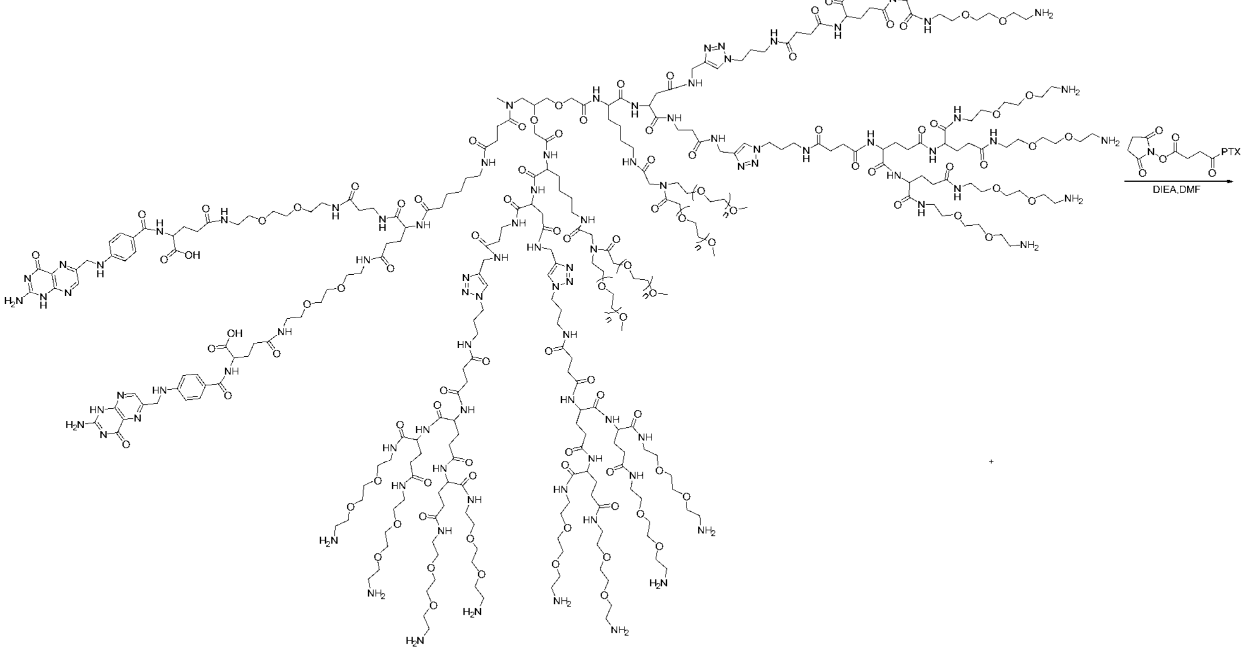


87-39

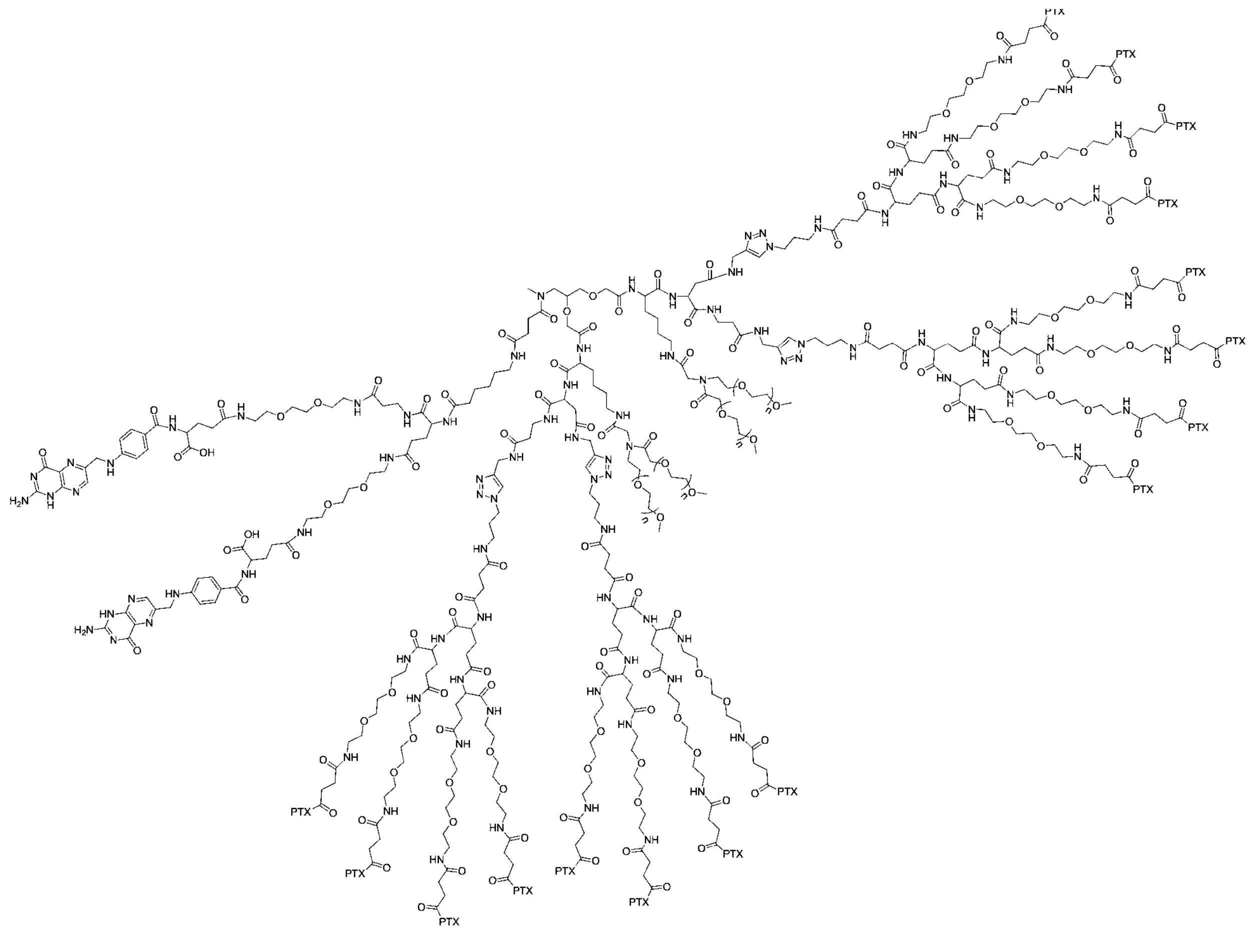

87-40

22.1 Preparation of **87-11**

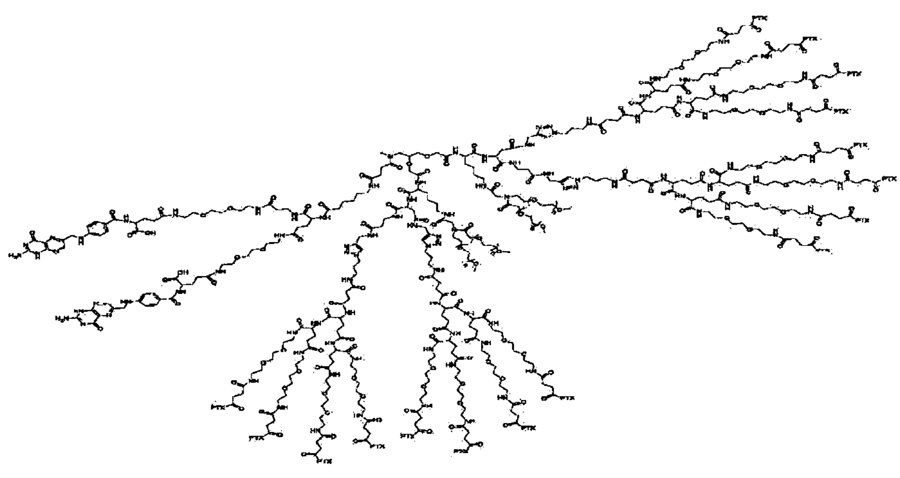

**76-191** (0.84 mmol), HBTU (0.38 g, 1.01 mmol) and HOBT (0.14 g, 1.01 mmol) were weighed and added into a flask containing **76-91** (0.84 mmol), then an appropriate amount of DMF was added to dissolve the mixture, and the mixture was allowed to stir at -5°C. DIEA (0.50 mL, 3.03 mmol) was then slowly added dropwise. After the dropwise addition was completed, the reaction was carried out for half an hour. The flask was taken out, and the reaction was carried out under stirring at room temperature overnight. After the reaction was completed, pure water (200 mL) and ethyl acetate (200 mL) were added to a 2-liter separatory funnel. The reaction mixture was then poured into the funnel for extraction. The organic

phase was separated, and the aqueous phase was further extracted with ethyl acetate (100 mL × 2). The organic phases were combined, then washed once with saturated sodium bicarbonate solution (200 mL). Finally, the organic phase was dehydrated over anhydrous magnesium sulfate and concentrated on a rotary evaporator, then an appropriate amount of mixed solvent and 200-300 mesh silica gel powder were added, and the mixture was evaporated to dryness. The resulting product was loaded by dry method, and subjected to column chromatography using 1-6% methanol/ dichloromethane as the eluent. The desired product was collected, concentrated, and evaporated to dryness to provide 1.0 g of the product. ESI [M+H$^+$] 1508.87, [M+Na$^+$] 1530.86

22.2 Preparation of **87-30**

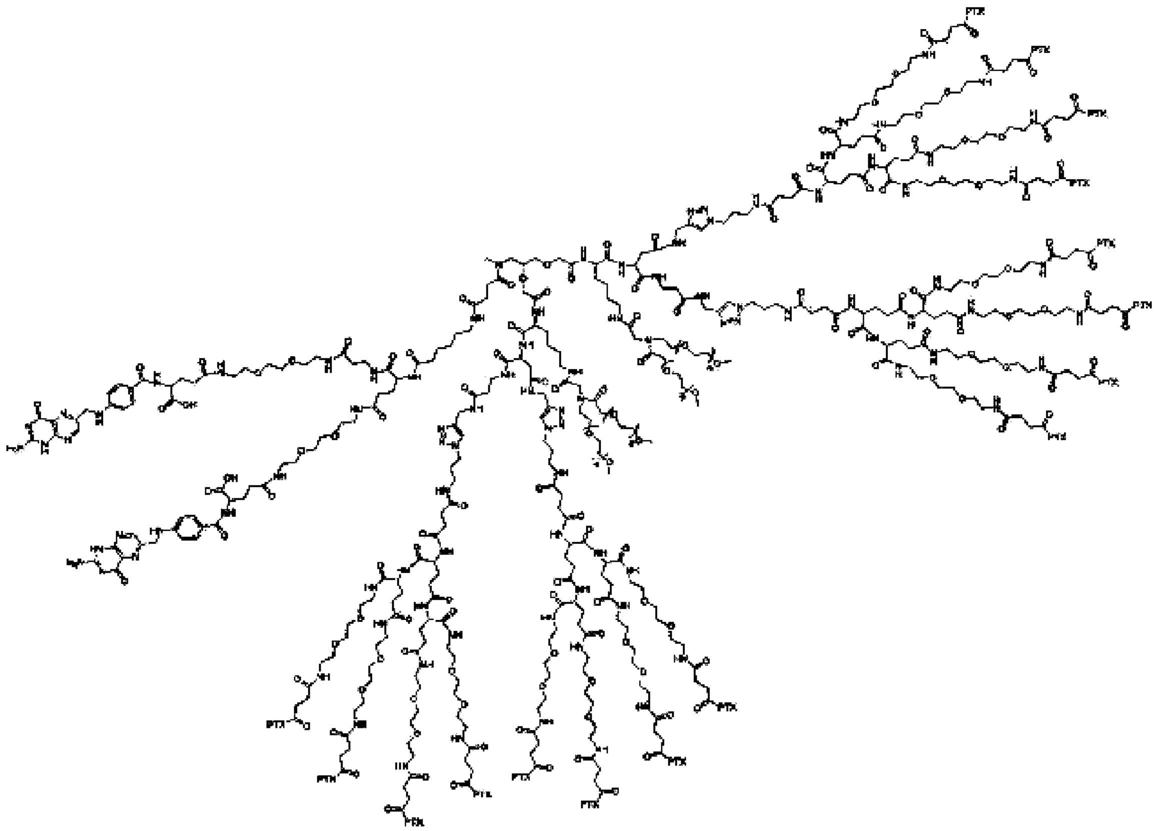

**87-26** (1.0 g, 0.044 mmol) and **87-11** (0.4 g, 0.26 mmol) were weighed and placed in a 250 mL reaction flask, and then an appropriate amount of ultra-dry DMF. was added for dissolution. The flask was filled with nitrogen. Anhydrous $CuSO_4$ (0.055 g, 0.35 mmol) and sodium ascorbate (0.14 g, 0.70 mmol) were then weighed and added to the flask. The reaction was carried out under stirring at room temperature overnight under nitrogen protection. After the reaction was completed, n-hexane (25 mL) and methyl tert-butyl ether (200 mL) were added for precipitation, the supernatant was then discarded, and n-hexane and methyl tert-butyl ether were added for precipitation. This process was repeated three times. The mixture was filtered to provide a solid product, which was collected and dried to provide a solid product.

22.3 Preparation of **87-39**

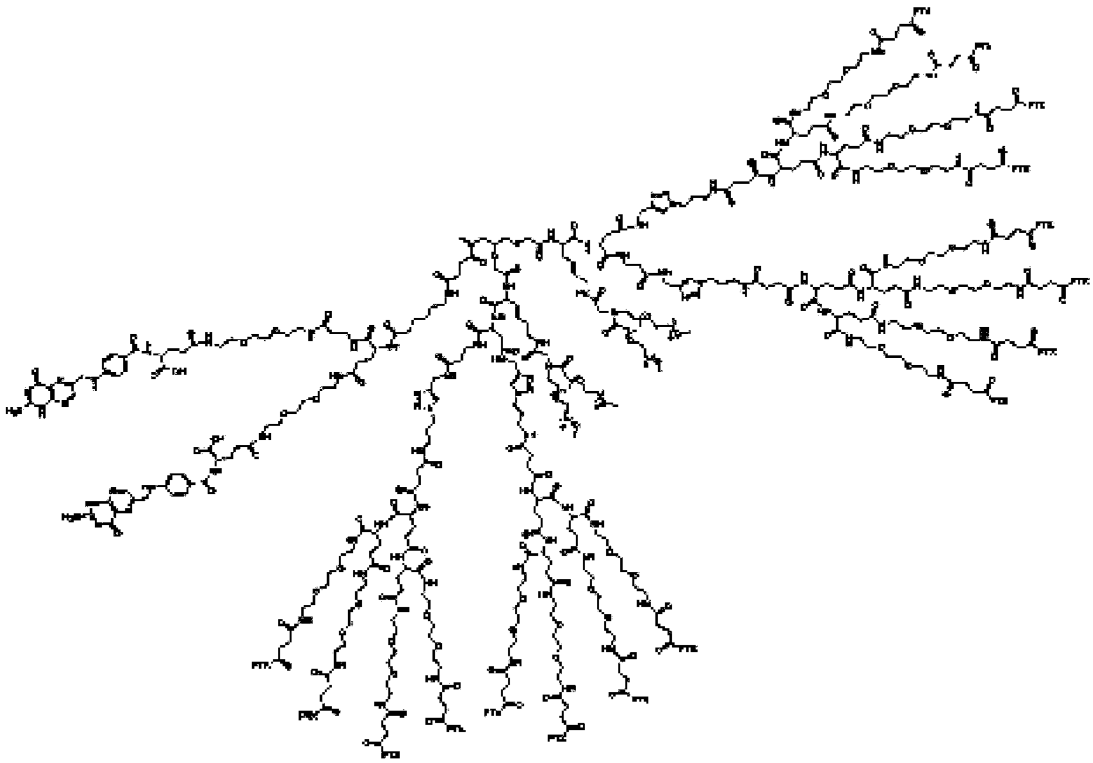

**87-30** (0.044 mmol) was placed in a 500 mL round-bottom flask, dichloromethane (15 mL) was added for dissolution with the assistance of ultrasonication, then TFA (10 mL) was added, and the mixture was reacted under stirring at room temperature. After the reaction was completed, the dichloromethane and some TFA were removed by rotary evaporation under reduced pressure. Methyl tert-butyl ether (200 mL) was then added for precipitation. A solid precipitated, was filtered and evaporated to dryness to provide a solid product.

22.4 Preparation of **87-40**

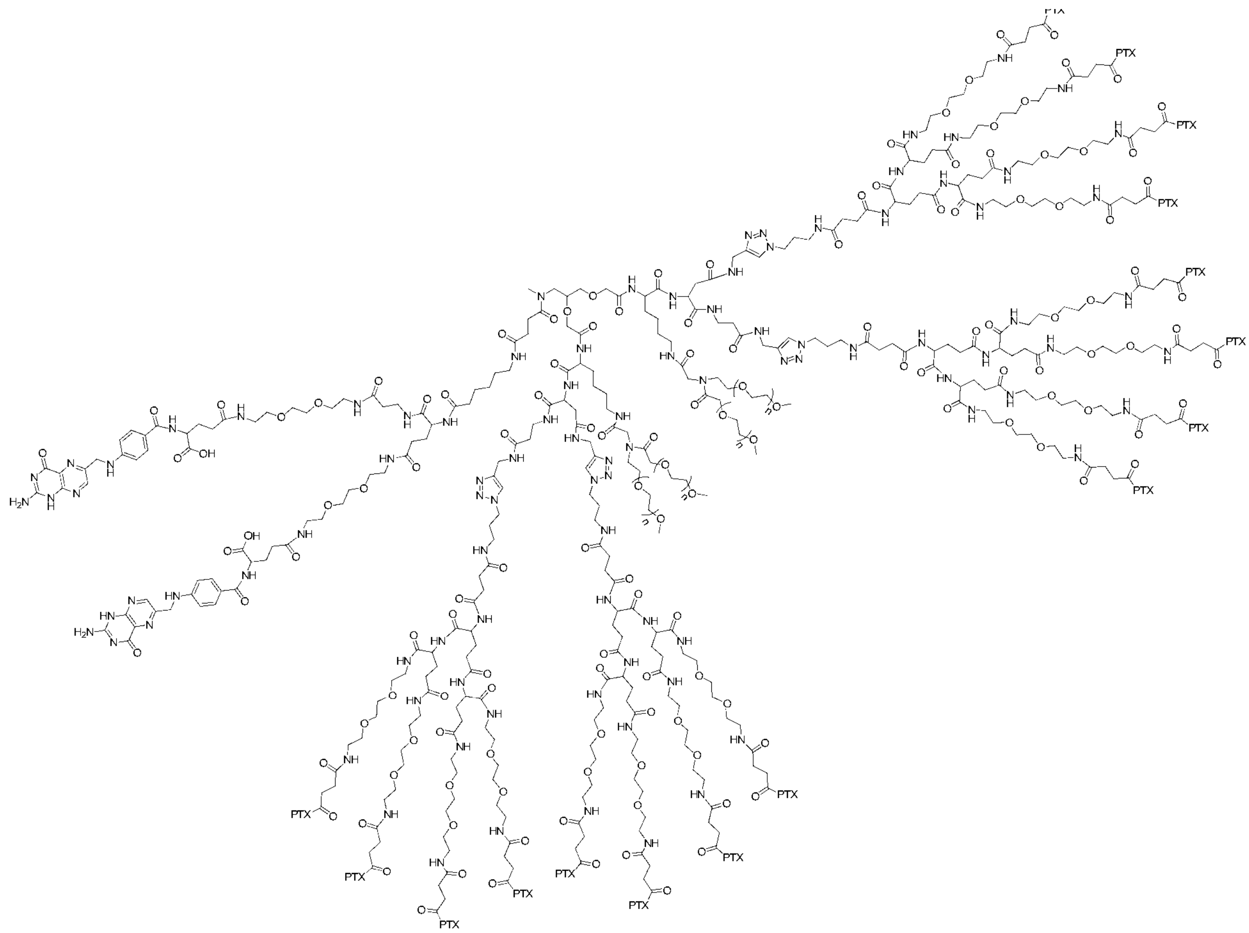

**87-39** (0.044 mmol) was placed in a 250 mL round-bottom flask, then ultra-dry DMF (20 mL) was added for dissolution with the assistance of ultrasonication, and the mixture was stirred at 0°C for 2 minutes. DIEA (1.16 mL, 7.04 mmol) was then added dropwise, followed by the addition of **77-164** (0.92 g, 0.88 mmol), and the reaction was stirred at room temperature. After the reaction was completed, methyl tert-butyl ether (200 mL) was added for precipitation. A solid precipitated and was filtered. The filter cake was dissolved in an appropriate amount of anhydrous ethanol/dichloromethane (1/9). Methyl tert-butyl ether was then added in batches with small amounts until a solid precipitated. The solid was filtered, and the obtained solid product was dissolved in dichloromethane and methanol, loaded by dry method, and subjected to column chromatography using 6%-12% methanol/dichloromethane as the eluent. The desired product was collected, concentrated, and dried to provide 0.93 g of the product with a yield of 50.00%. $^1$H-NMR (600 MHz, DMSO-$d_6$) δ 9.31-9.15 (m, 16H), 8.32-8.09 (m, 16H), 8.03-7.94 (m, 48H), 7.90-7.80 (m, 43H), 7.77-7.62 (m, 52H), 7.60-7.38 (m, 116H), 7.26-7.10 (m, 16H), 6.88-6.78 (m, 3H), 6.35-6.20 (m, 16H), 5.90-5.74 (m, 16H), 5.60-5.48 (m, 16H), 5.47-5.28 (m, 33H), 4.98-4.86 (m, 32H), 4.70-4.60 (m, 16H), 4.23-4.15 (m, 10H), 4.05-3.97 (m, 32H), 3.57-3.45 (m, 1867H), 3.21-3.09 (m, 70H), 2.67-2.57 (m, 47H), 2.44-2.33 (m, 61H), 2.29-2.19 (m, 58H), 2.16-2.03 (m, 72H), 1.95-1.87 (m, 17H), 1.84-1.72 (m, 76H), 1.69-1.58 (m, 30H), 1.57-1.42 (m, 71H), 1.05-1.01 (m, 41H), 1.01-0.96 (m, 45H)

Example 23: Synthesis of Compound **87-42**

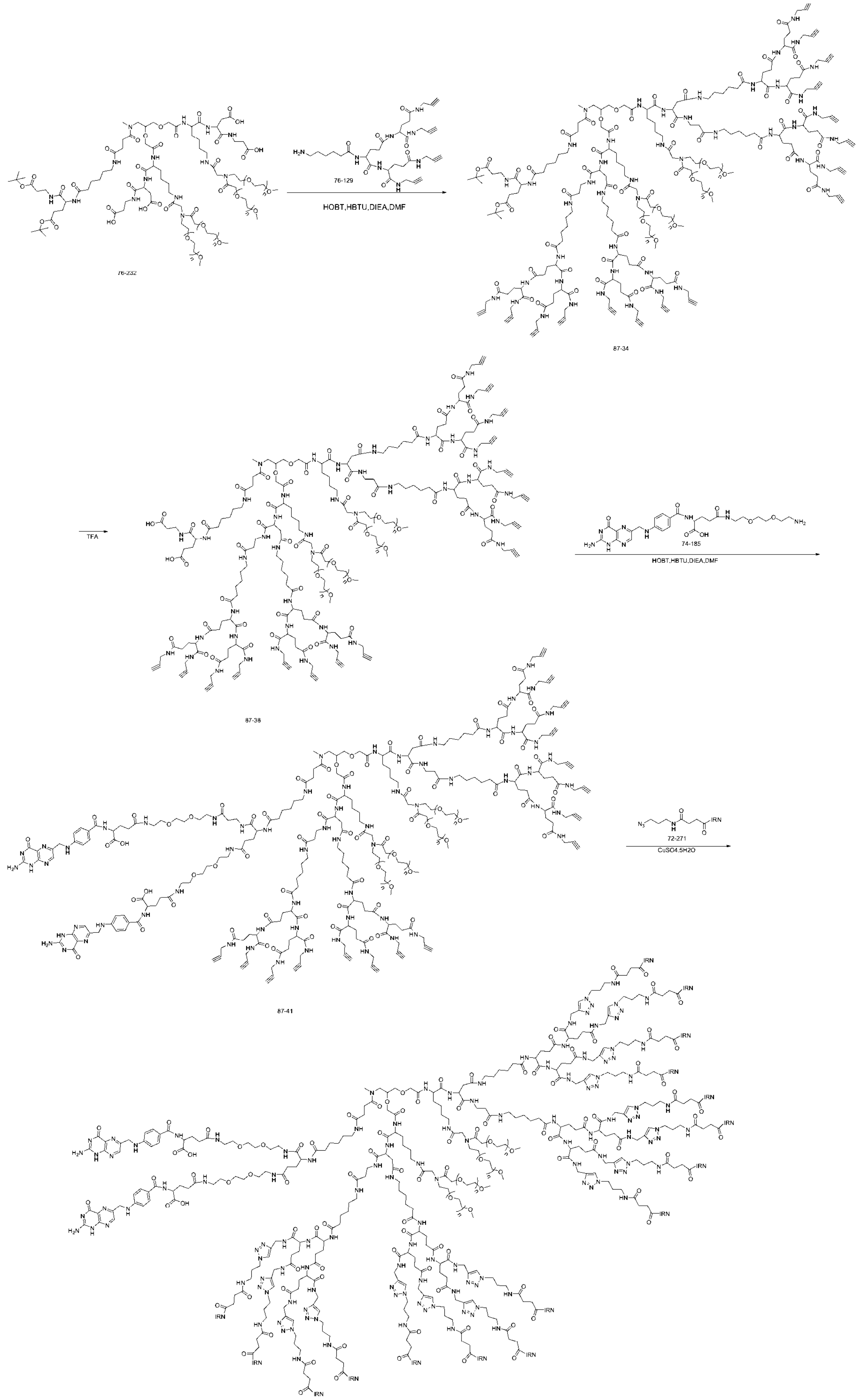


76-232

76-129

87-34

87-38

74-185

87-41

72-271

87-42

23.1 Preparation of **87-34**

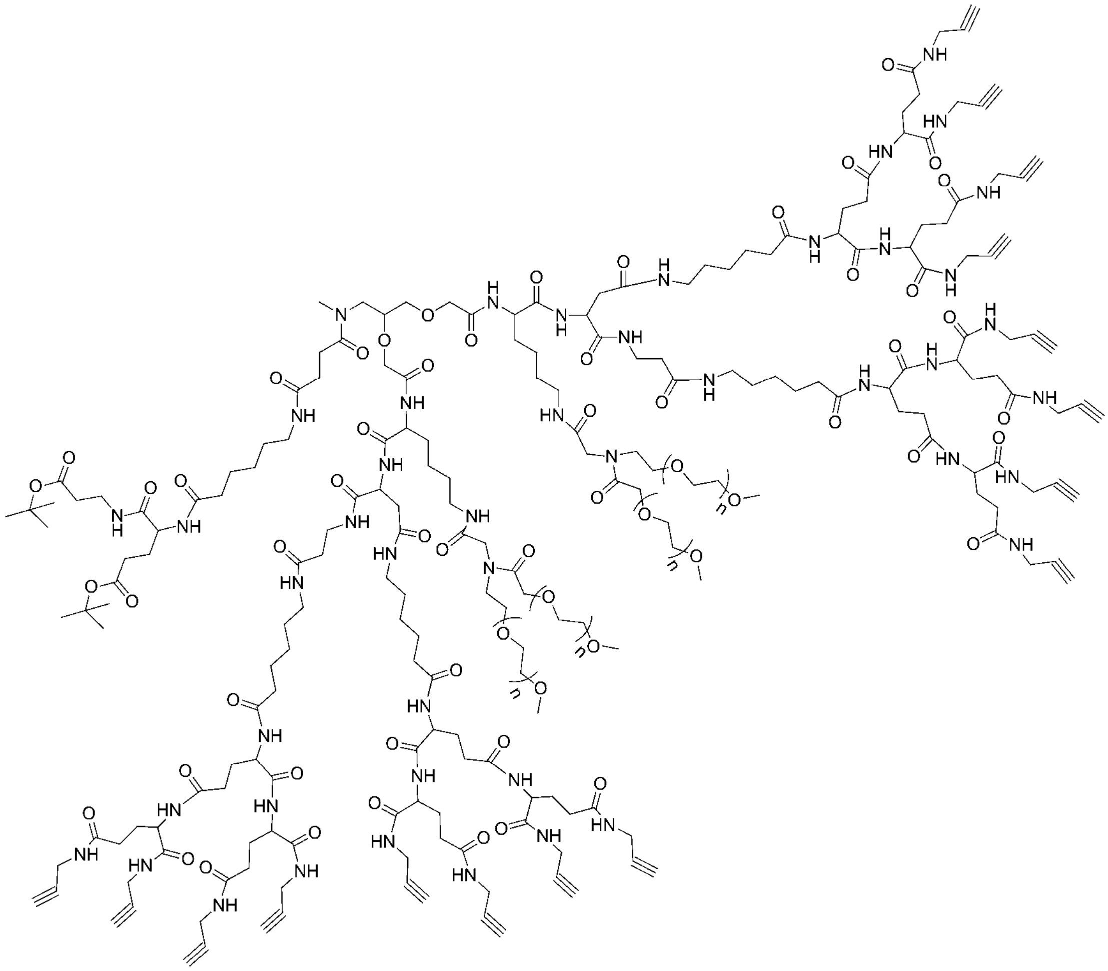

**76-232** (0.1443 mmol), **76-129** (0.43 g, 0.64 mmol), HBTU (0.3 g, 0.69 mmol) and HOBT (0.1 g, 0.69 mmol) were added to a 250 mL round-bottom flask, and then DMF (15 mL) was added for dissolution with the assistance of ultrasonication. The mixture was stirred at 0°C for 2 minutes, followed by the dropwise addition of DIEA (0.34 mL, 2.08 mmol). The flask was taken out, and the reaction was carried out under stirring at room temperature. After the reaction was completed, methyl tert-butyl ether (200 mL) was added for precipitation. A solid precipitated and was filtered. The crude product was dissolved in a methanol/dichloromethane mixture, then 100-200 mesh silica gel powder was added, and the mixture was evaporated to dryness. The resulting product was loaded by dry method, and subjected to column chromatography using 5-12% methanol/dichloromethane as the eluent. The desired product was collected and evaporated to dryness to provide a solid product.

23.2 Preparation of **87-38**

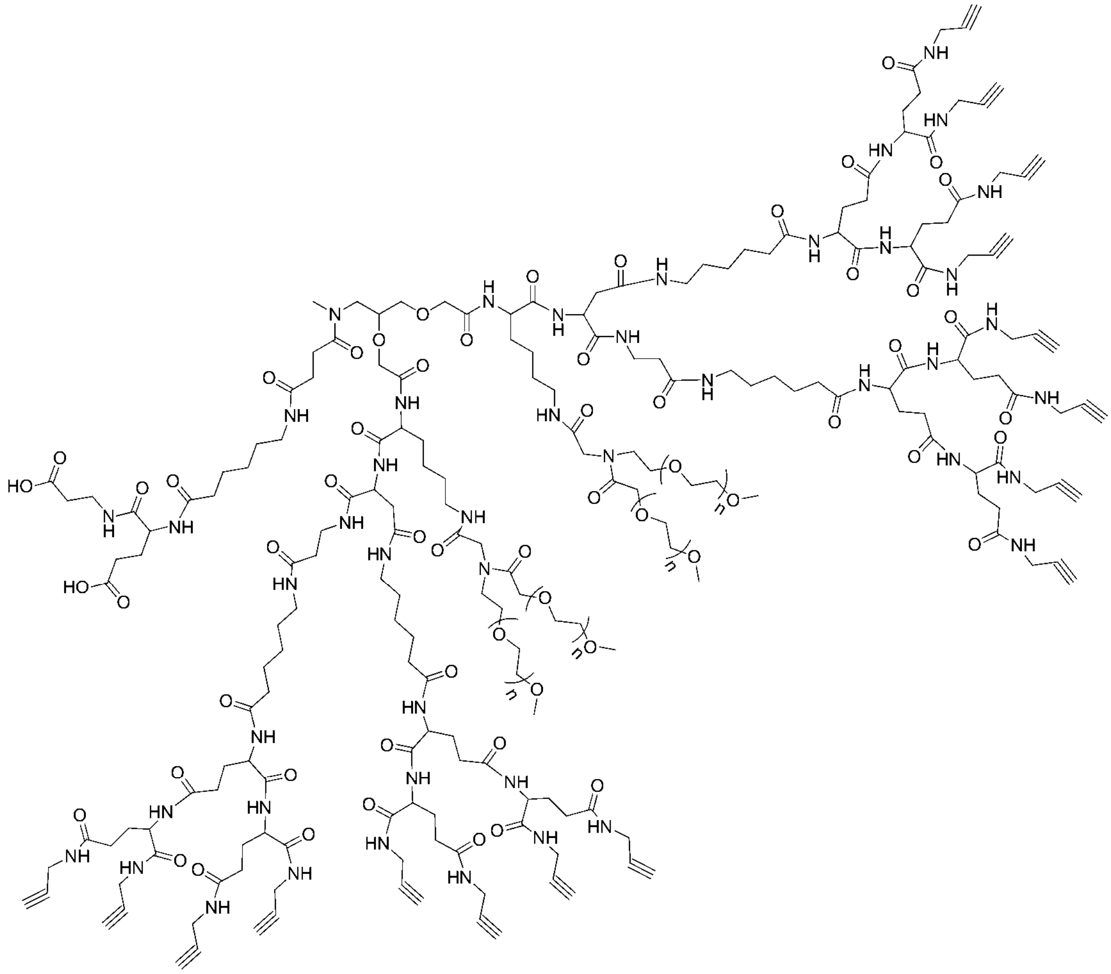

To a flask containing **87-34** (0.1443 mmol), dichloromethane was added for dissolution with the assistance of ultrasonication, then TFA (0.43 mL) was added, and the reaction was stirred at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide a solid product.

23.3 Preparation of **87-41**

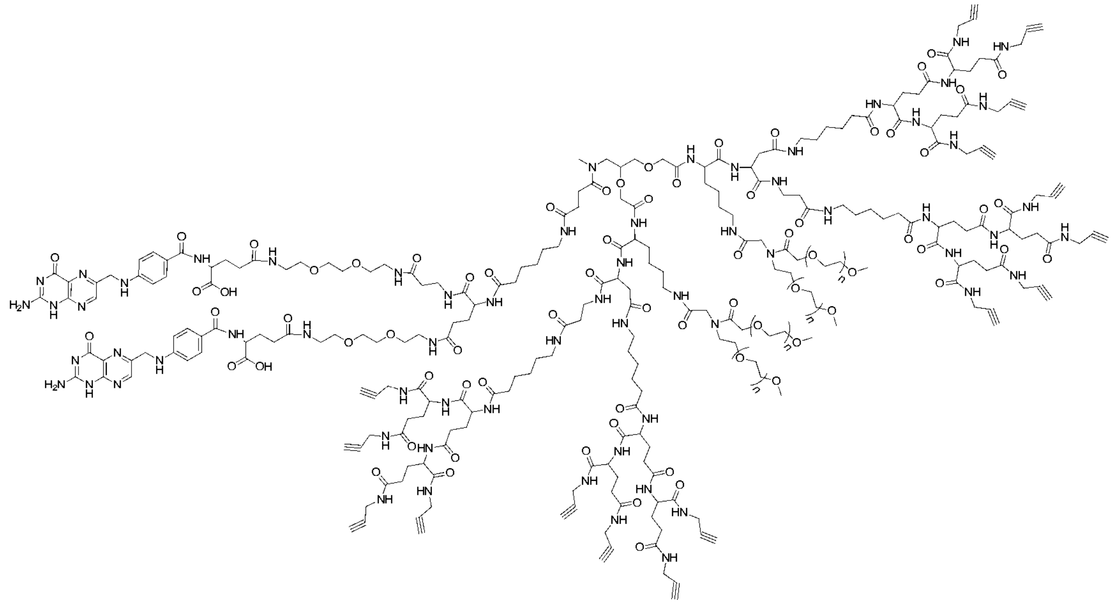

**87-38** (0.1443 mmol), **74-185** (0.18 g, 0.32 mmol), HBTU (0.3 g, 0.69 mmol) and HOBT (0.1 g, 0.69 mmol) were added to a 100 mL round-bottom flask, and then DMF (10 mL) was added for dissolution with the assistance of ultrasonication. The mixture was stirred at 0°C for 1 minute. DIEA (0.18 mL, 1.04 mmol) was then added dropwise. The flask was taken out, and the reaction was carried out under stirring at room temperature. After the reaction was completed, methyl tert-butyl ether (200 mL) was added for precipitation. A solid precipitated and was filtered. The filter cake was collected. The filter cake was dissolved in an appropriate amount of DMF. MTBE was then added in batches with small amounts until a solid precipitated, which was filtered and dried to provide 3.26 g of the product.

23.5 Preparation of **87-42**

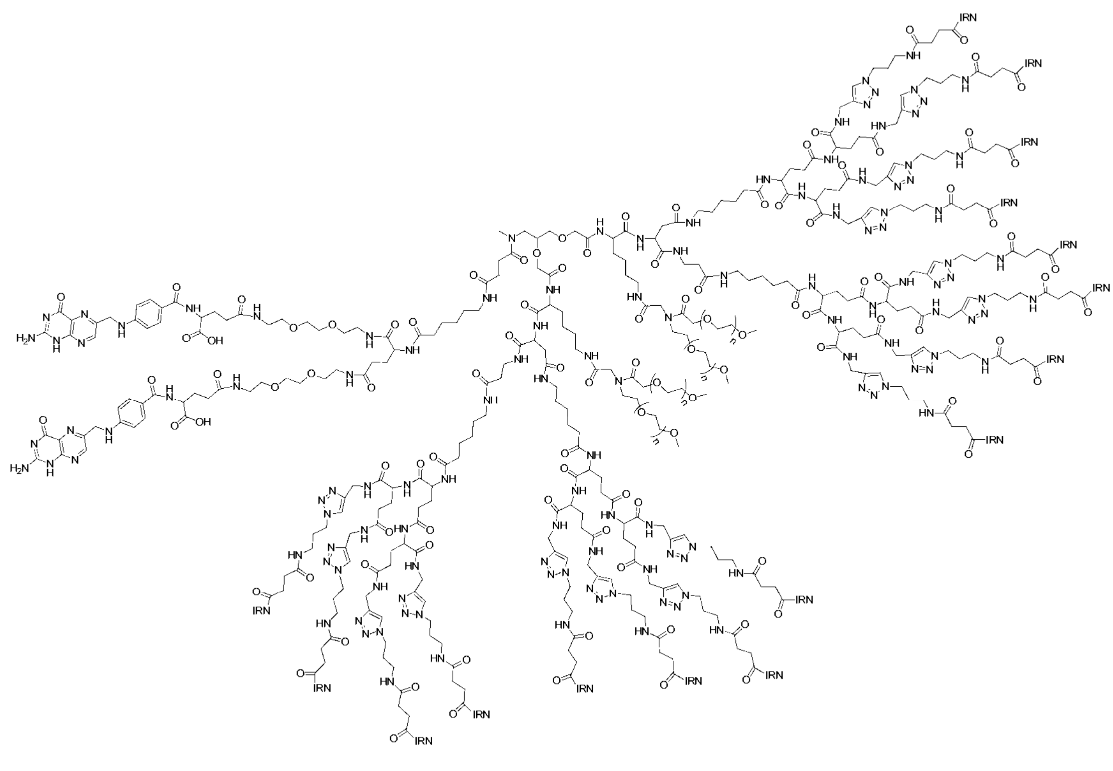

**87-41** (0.1443 mmol) and anhydrous $CuSO_4$ (0.73 g, 4.6 mmol) were weighed and added into a flask containing

anhydrous DMF (10 mL), and then **72-271** (2.3 mmol) and sodium ascorbate (1.83 g, 9.2 mmol) were added. After dissolution with the assistance of ultrasonication, the reaction was carried out under stirring at room temperature under nitrogen protection overnight. After the reaction was completed, n-hexane (25 mL) and methyl tert-butyl ether (200 mL) were added for precipitation. A solid precipitated and was filtered. The solid was dissolved in dichloromethane, and then n-hexane (25 mL) and methyl tert-butyl ether (200 mL) were added for precipitation. This process was repeated three times. The mixture was filtered to provide a solid product. The obtained solid product was dissolved in a methanol/dichloromethane (1/4) mixture, then silica gel powder was added, and the mixture was evaporated to dryness. The resulting product was loaded by dry method, and subjected to column chromatography using 1% ammonia water/5-7% methanol/dichloromethane as the eluent. The desired product was collected, evaporated to dryness, and dried in a vacuum oven to provide 0.27 g of the product with a yield of 5.04%. $^1$H-NMR (600 MHz, DMSO-$d_6$) $\delta$ 9.63-9.54 (m, 2H), 8.70-8.36 (m, 16H), 8.19-7.82 (m, 84H), 7.70-7.52 (m, 24H), 7.10-6.97 (m, 18H), 6.84-6.69 (m, 10H), 4.39-4.02 (m, 163H), 3.87-3.76 (m, 70H), 3.56-3.48 (m, 1128H), 3.12-3.01 (m, 138H), 2.67-2.58 (m, 102H), 2.14-2.06 (m, 74H), 1.92-1.79 (m, 86H), 1.76-1.59 (m, 106H), 1.45-1.33 (m, 72H), 1.27-1.16 (m, 72H), 0.95-0.85 (m, 48H)

Example 24: Synthesis of Compound **85-63**

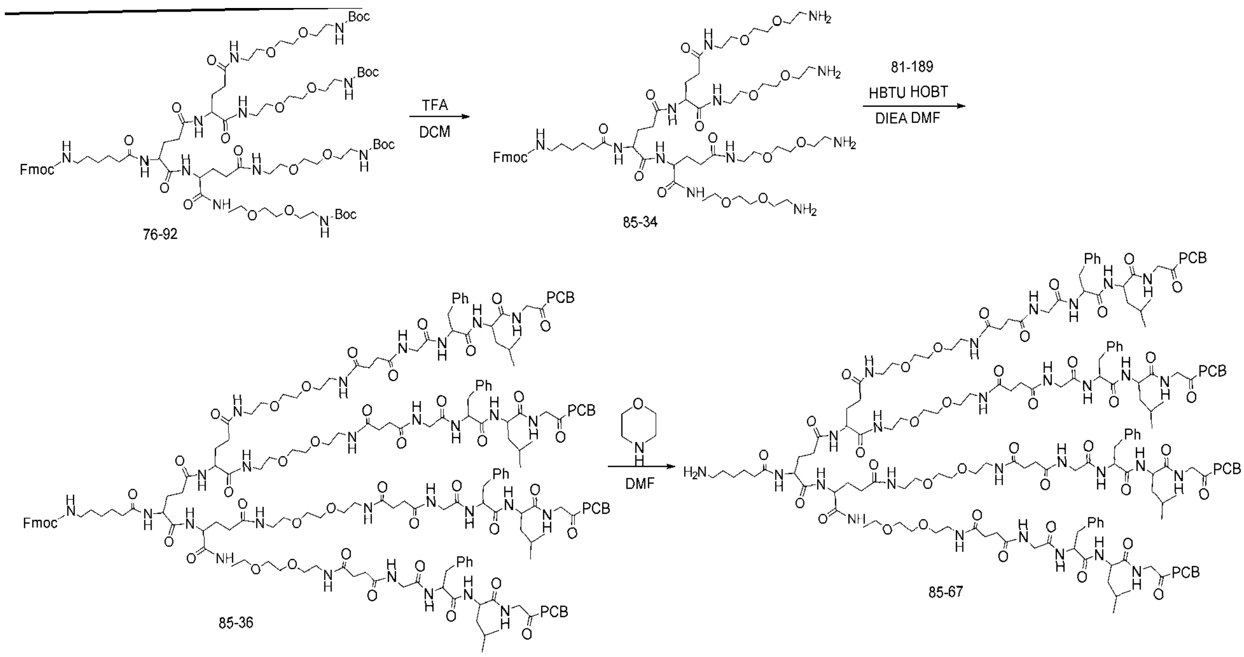


76-92

85-34

85-36

85-67

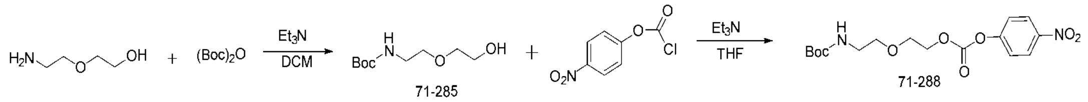

71-285

71-288

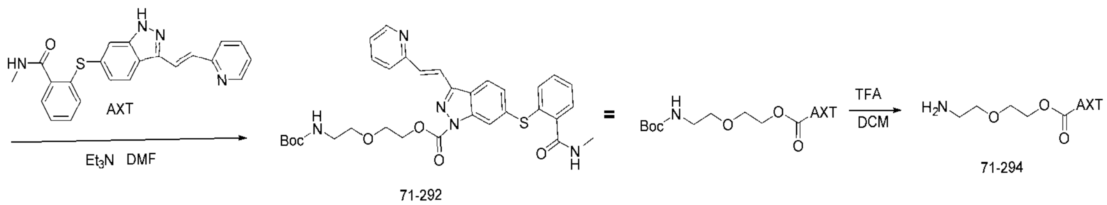

71-292

71-294

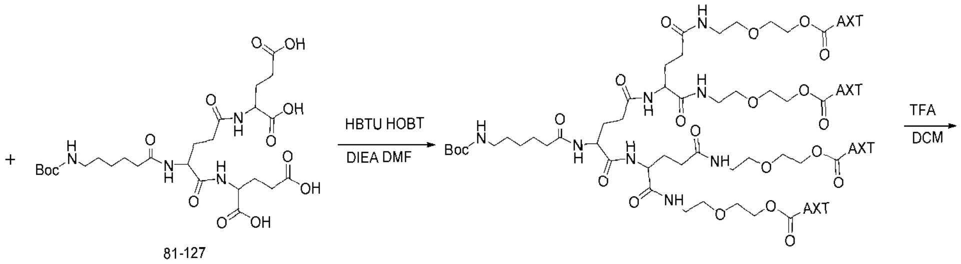

81-127

85-32

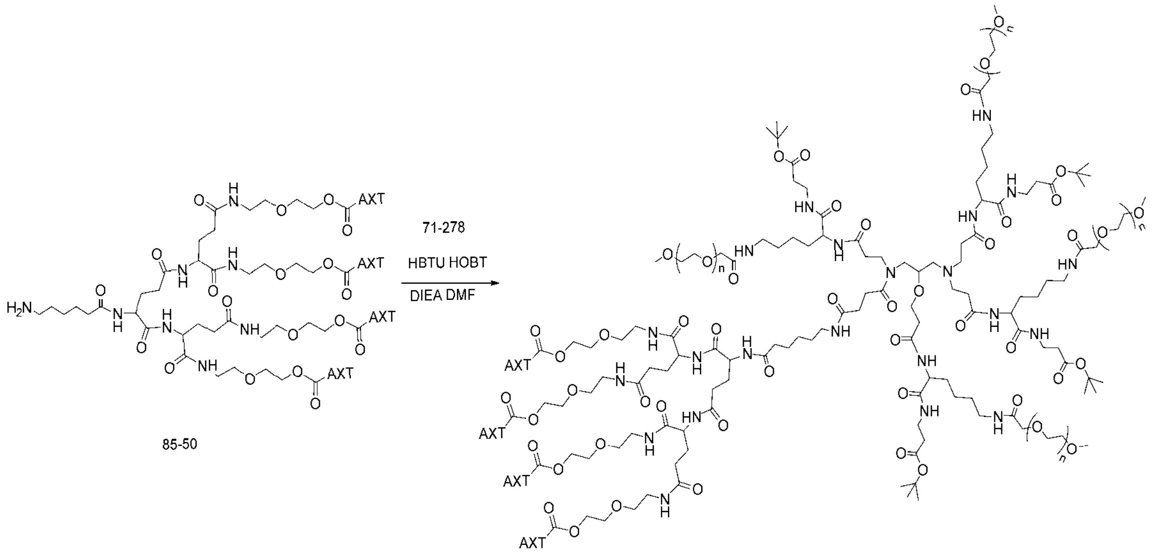

85-50

85-51

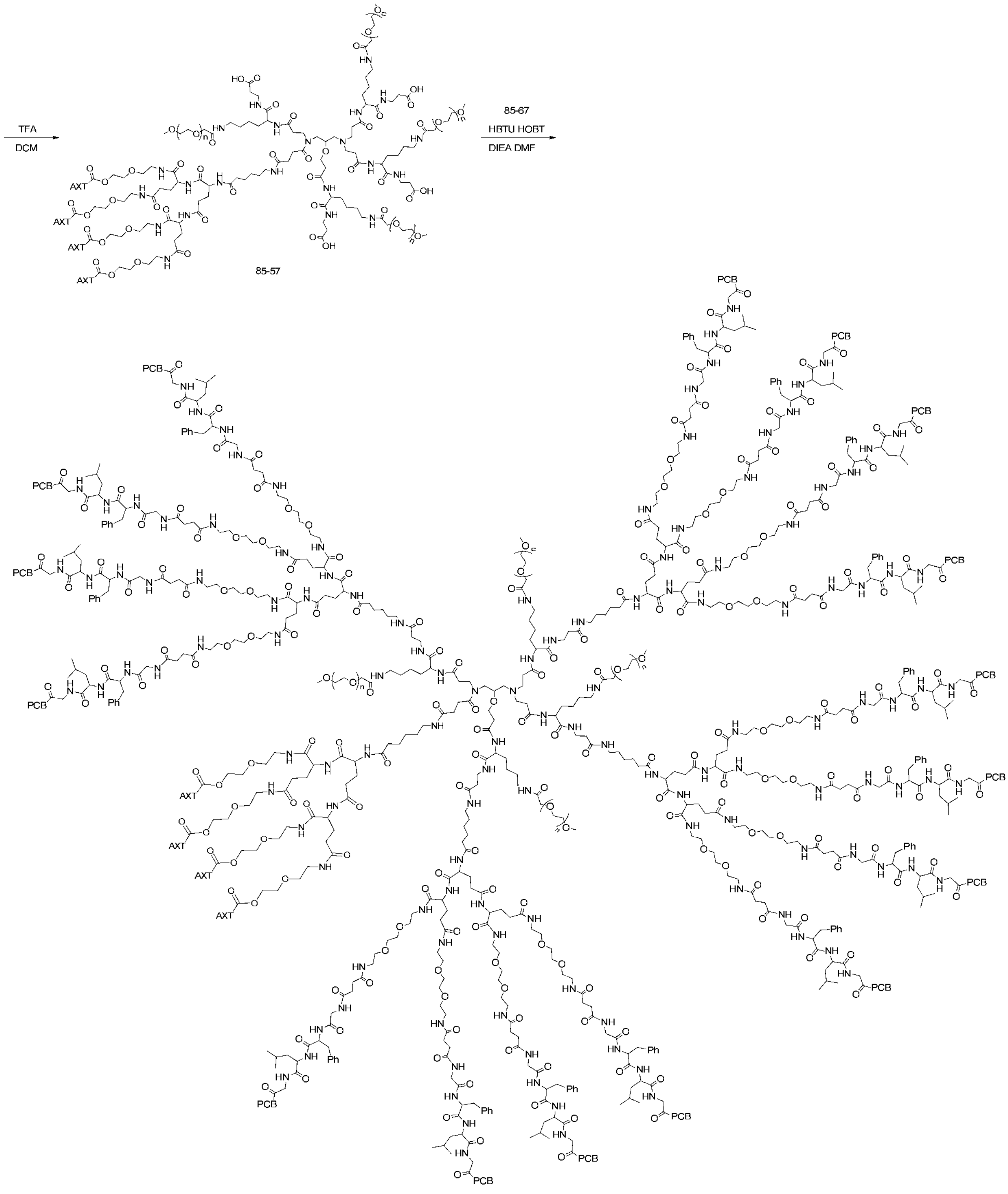


85-57

85-63

24.1 Preparation of **85-34**

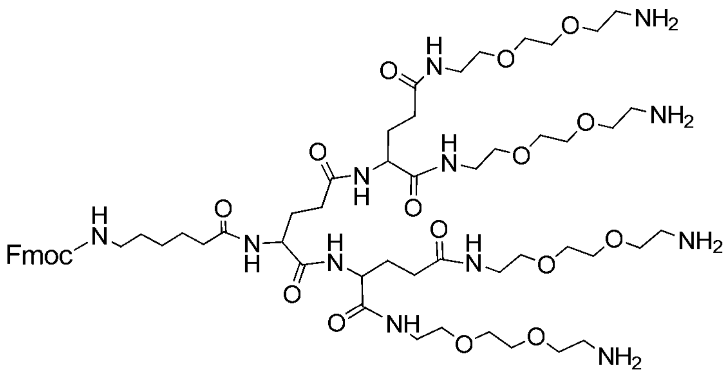

To a flask containing compound **76-92** (3.5 g, 2.1059 mmol), dichloromethane (6 mL) was added was added for dissolution with the assistance of ultrasonication. Then TFA (6.3 mL, 84.2374 mmol) was added, and the mixture was reacted under stirring at room temperature. After the reaction was completed, the reaction mixture was concentrated and evaporated to dryness to remove the dichloromethane, and methyl tert-butyl ether (300 mL) was added for precipitation. A solid product precipitated and was filtered. The filter cake was washed three times with methyl tert-butyl ether (40 mL). The filter cake was collected and dried under vacuum to provide 2.65 g of the product.

24.2 Preparation of **85-36**

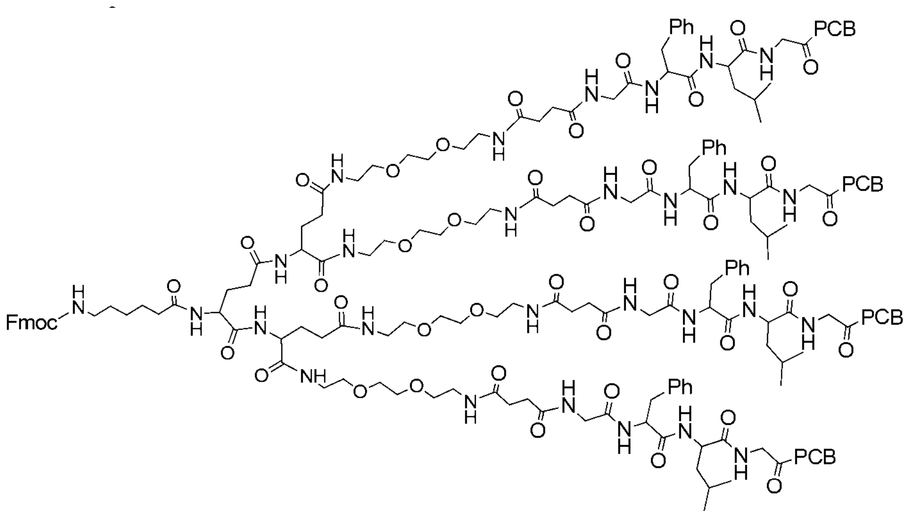

**85-34** (2.65 g, 2.1059 mmol), **81-189** (7.76 g, 8.4236 mmol), HBTU (4.79 g, 12.6354 mmol) and HOBT (1.70 g, 12.6354 mmol) were placed in a 500 mL reaction flask, DMF (100 mL) was added for dissolution, and the mixture was stirred at -5°C for approximately 20 minutes. DIEA (6.3 mL, 37.9062 mmol) was then slowly added dropwise, and the reaction was continued at 0°C for 30 minutes. The reaction was then brought to room temperature and carried out under stirring. After the reaction was completed, n-hexane (250 mL) and methyl tert-butyl ether (70 mL) were added for precipitation, the supernatant was discarded, and n-hexane and methyl tert-butyl ether were added again for precipitation. This process was repeated three times. The mixture was filtered to provide a solid product, which was collected and dissolved in a 4% methanol/ dichloromethane mixture with the assistance of ultrasonication, and a solid product was obtained by filtration. This operation was repeated three times. The solid product was collected and dried in a vacuum oven to provide 7.5 g of the product with a yield of 73.1%. $^1$H-NMR (600 MHz, DMSO-$d_6$) δ8.33-7.93 (m, 28H), 7.92-7.78 (m, 12H), 7.71-7.60 (m, 2H), 7.54-7.44 (m, 4H),7.41-7.35 (m,2H), 7.32-7.09 (m,24H), 4.55-4.43 (m, 4H), 4.39-4.15 (m, 10H), 4.12-3.90 (m, 10H), 3.69-3.53 (m, 26H), 3.51-3.42 (m, 20H), 3.25-3.00 (m, 44H), 3.00-2.71 (m, 10H), 2.45-2.18 (m, 54H), 2.11-2.07 (m, 4H), 1.92-1.70 (m, 22H), 1.63-1.42 (m, 24H), 1.40-1.32 (m, 2H), 1.22-1.16 (m, 2H), 0.92-0.78 (m, 24H)

24.3 Preparation of 85-67

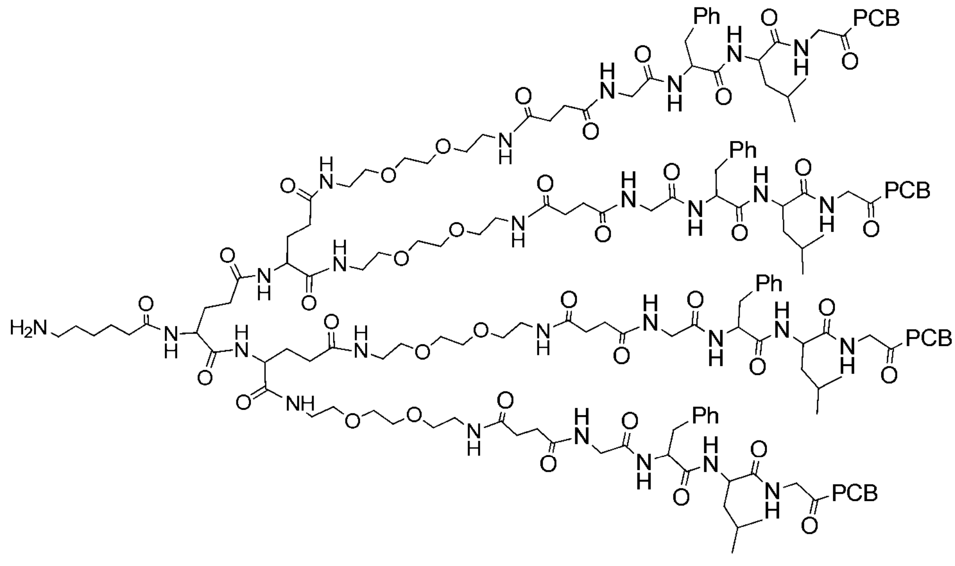

**85-36** (3.0 g, 0.6150 mmol) was placed in a 500 mL flask, DMF (15 mL) was added for dissolution, then morpholine (1.1 mL, 12.3009 mmol) was added, and the mixture was reacted under stirring in a 45°C water bath for 2 hours. After the reaction was completed, n-hexane (25 mL) and methyl tert-butyl ether (200 mL) were added for precipitation, the supernatant was discarded, and then n-hexane and methyl tert-butyl ether were added again for precipitation. This process was repeated four times. The mixture was filtered to provide a solid product, which was dried in a vacuum oven to provide 2.86 g of the product. ESI [M+Na$^+$] 4677.803

24.6 Preparation of 71-285

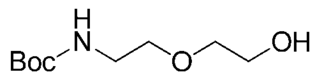

2-(2-Aminoethoxy)ethanol (2.4 g, 22.9063 mmol) was weighed and added to a reaction flask, dichloromethane (10 mL) was added for dissolution, and the mixture was stirred at room temperature. Triethylamine (6.4 mL, 45.8126 mmol) was added. $(Boc)_2O$ (5.0 g, 22.9063 mmol) was then weighed and dissolved in dichloromethane (10 mL), and slowly added dropwise to the reaction flask. After the dropwise addition was completed, the reaction was carried out under stirring at room temperature. After the reaction was completed, the reaction mixture was evaporated to dryness using a rotary evaporator. The resultant product was dissolved in a 20% methanol/dichloromethane mixture, and then silica gel powder was added. The mixture was mixed evenly and evaporated to dryness. The resultant product was loaded by dry method, and subjected to column chromatography using 1-3% methanol/dichloromethane as the eluent. The desired product was collected, concentrated, and evaporated to dryness to provide 4.5 g of the product with a yield of 95.74%. $^1$H-NMR (600 MHz, DMSO-$d_6$) δ 6.76 (t, *J* = 5.3 Hz, 1H), 3.48 (dd, *J* = 10.5, 5.3 Hz, 2H), 3.42-3.31 (m, 5H), 3.07 (dd, *J* = 11.8, 5.9 Hz, 2H), 1.37 (s, 9H); ESI [M+Na$^+$] 228.11987

24.7 Preparation of 71-288

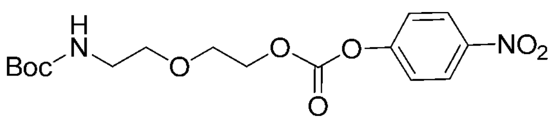

**71-285** (4.5 g, 21.9243 mmol) was weighed and added into a reaction flask, then tetrahydrofuran (19 mL) and triethylamine (9.1 mL) were added, and the contents were mixed for dissolution. The reaction flask was cooled to 0°C in an ice-water bath, followed by the addition of a solution of 4-nitrobenzene chloroformate (4.41 g, 21.9243 mmol) in tetrahydrofuran (20 mL). After the dropwise addition was completed, the temperature was raised to room temperature

and the mixture was stirred at room temperature for 4 hours. After the reaction was completed, the mixture was concentrated to remove most of the solvent, then ethyl acetate (100 mL) and deionized water (100 mL) were added for extraction. After standing for layer separation, the organic phase was collected and concentrated. The obtained product was dissolved in a 20% methanol/dichloromethane mixture, then silica gel powder was added, and the contents were mixed thoroughly and evaporated to dryness. The resulting product was loaded by dry method, and subjected to column chromatography using 20-30% ethyl acetate/petroleum ether as the eluent. The desired product was collected, concentrated, and evaporated to dryness to provide 4.49 g of the product with a yield of 55.42%. $^1$H-NMR (600 MHz, DMSO-$d_6$) δ 8.33-8.30 (m, 1H), 8.14-8.09 (m, 1H), 7.60-7.53 (m, 1H), 6.96-6.90 (m, 1H), 6.82 (s, 1H), 4.38-4.34; ESI [M+Na$^+$] 393.12424

24.8 Preparation of **71-292**

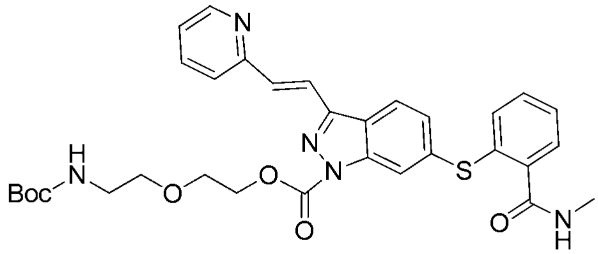

Axitinib (3.9 g, 10.1029 mmol) was weighed and added into a reaction flask, then DMF (20 mL) and triethylamine (4.22 mL, 30.3087 mmol) were added, and the contents were mixed for dissolution, and stirred at room temperature. **71-288** (4.49 g, 12.1235 mmol) was then added and stirred overnight at room temperature. After the reaction was completed, deionized water (100 mL) and ethyl acetate (120 mL) were added for extraction. After standing for layer separation, the organic phase was collected and washed with sodium chloride aqueous solution (70 mL × 3) and concentrated. The obtained product was dissolved in a 20% methanol/dichloromethane mixture, then silica gel powder was added, and the contents were mixed thoroughly and evaporated to dryness. The resulting product was loaded by dry method, and subjected to column chromatography using 0-3% methanol/dichloromethane as the eluent. The desired product was collected, concentrated, and evaporated to dryness to provide 3.869 g of the product with a yield of 61.87%. $^1$H-NMR (600 MHz, DMSO-$d_6$) δ 8.66 (d, $J$ = 3.9 Hz, 1H), 8.41 (q, $J$ = 4.5 Hz, 1H), 8.33 (d, $J$ = 8.4 Hz, 1H), 8.18 (s, 1H), 7.94 (d, $J$ = 16.4 Hz, 1H), 7.87 (td, $J$ = 7.7, 1.8 Hz, 1H), 7.79 (dd, $J$ = 17.2, 12.1 Hz, 2H), 7.54 -7.51 (m, 1H), 7.41 (dd, $J$ = 8.4, 1.5 Hz, 1H), 7.39-7.34 (m, 3H), 7.18 (dd, $J$ = 7.6, 1.4 Hz, 1H), 6.79 (t, $J$ = 5.6 Hz, 1H), 4.59-4.53 (m, 2H), 3.79-3.75 (m, 2H), 3.46 (t, $J$ = 6.0 Hz, 2H), 3.10 (dd, $J$ = 11.8, 5.9 Hz, 2H), 2.76 (d, $J$ = 4.6 Hz, 3H), 1.33 (s, 9H); ESI [M+H$^+$] 618.23706, [M+Na$^+$] 640.21857

24.9 Preparation of **71-294**

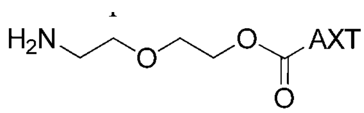

**71-292** (3.869 g, 6.2634 mmol) was weighed, then dichloromethane (5 mL) and TFA (4.6 mL, 62.634 mmol) were added, and the mixture was reacted under stirring at room temperature. After the reaction was completed, the reaction mixture was concentrated, and methyl tert-butyl ether (150 mL) and n-hexane (100 mL) were added for precipitation. A solid product precipitated, which was collected by filtration and dried in a vacuum oven to provide 3.24 g of product. $^1$H-NMR (600 MHz, DMSO-$d_6$) δ 8.67 (d, $J$ = 4.1 Hz, 1H), 8.44 (dd, $J$ = 9.1, 4.5 Hz, 1H), 8.34 (d, $J$ = 8.4 Hz, 1H), 8.18 (d, $J$ = 0.7 Hz, 1H), 7.95 (d, $J$ = 16.4 Hz, 1H), 7.90 (td, $J$ = 7.7, 1.7 Hz, 1H), 7.80 (d, $J$ = 5.6 Hz, 2H), 7.54 (dd, $J$ = 7.1, 2.0 Hz, 1H), 7.42 (dd, $J$ = 8.4, 1.4 Hz, 1H), 7.41-7.35 (m, 3H), 7.20-7.16 (m, 1H), 4.66-4.56 (m, 2H), 3.86-3.83 (m, 2H), 3.70-3.67 (m, 4H), 3.02 (dd, $J$ = 10.7, 5.5 Hz, 2H), 2.76 (d, $J$ = 4.6 Hz, 3H); ESI [M+H$^+$] 518.18384

24.10 Preparation of **85-32**

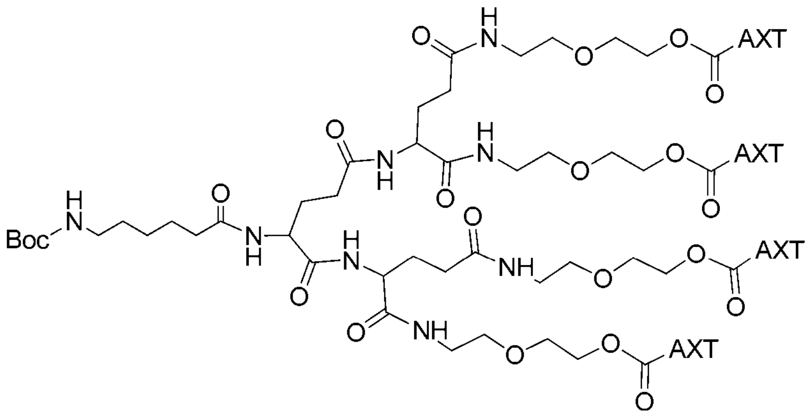

**71-294** (3.24 g, 6.2596 mmol), **81-127** (0.74 g, 1.2037 mmol), HBTU (2.73 g, 7.2222 mmol) and HOBT (0.97 g, 7.222 mmol) were added into a 500 mL reaction flask containing DMF (40 mL), and the mixture was stirred at -5°C for approximately 20 minutes. DIEA (3.5 mL, 21.6666 mmol) was slowly added dropwise, and the reaction was continued at 0°C for 1 hour. The reaction was then brought to room temperature and carried out under stirring. After the reaction was completed, n-hexane (250 mL) and methyl tert-butyl ether (70 mL) were added for precipitation, the supernatant was discarded, and n-hexane and methyl tert-butyl ether were added again for precipitation. This process was repeated four times. The mixture was filtered to obtain a solid product. The solid product was collected, washed with the assistance of ultrasonication with a mixture of 97% dichloromethane and 3% methanol, and filtered to provide a solid product. This process was repeated twice. The solid product was collected and dried in a vacuum oven to provide 2.55 g of the product with a yield of 81.25%. $^1$H-NMR (600 MHz, DMSO-$d_6$) δ 8.61 (s, 4H), 8.45-8.33 (m, 4H), 8.30 -8.16 (m, 4H), 8.16-8.03 (m, 6H), 8.02-7.93 (m, 2H), 7.92-7.78 (m, 10H), 7.77-7.66 (m, 8H), 7.54-7.48 (m, 4H), 7.40-7.23 (m, 16H), 7.20-7.09 (m, 4H), 4.59-4.44 (m, 8H), 4.23-4.07 (m, 3H), 3.73 (s, 8H), 3.50-3.40 (m, 8H), 3.28-3.23 (m, 2H), 3.22-3.14 (m, 6H), 3.06 (s, 2H), 2.87 -2.79 (m, 2H), 2.78-2.67 (m, 12H), 2.17-2.09 (m, 8H), 1.90-1.77 (m, 3H), 1.76-1.64 (m, 3H), 1.45-1.38 (m, 2H), 1.36-1.25 (m, 11H), 1.19-1.12 (m, 2H); ESI [M+Na$^+$] 2637.778

24.11 Preparation of **85-50**

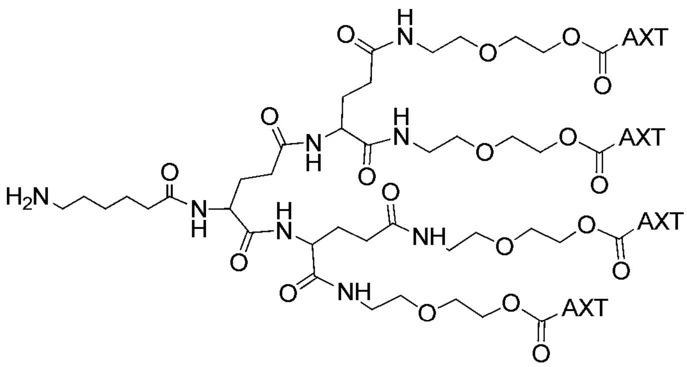

To a flask containing **85-32** (2.55 g, 0.9744 mmol) was added dichloromethane (5 mL). After complete dissolution with the assistance of ultrasonication, TFA (0.7 mL, 9.744 mmol) was added and the mixture was stirred at room temperature. After the reaction was completed, the reaction mixture was concentrated and evaporated to dryness to remove the dichloromethane. Methyl tert-butyl ether (300 mL) was then added for precipitation. A solid product precipitated and was filtered. The filter cake was washed three times with methyl tert-butyl ether (40 mL). The filter cake was collected and vacuum dried to provide 2.45 g of the product.

24.12 Preparation of**85-51**

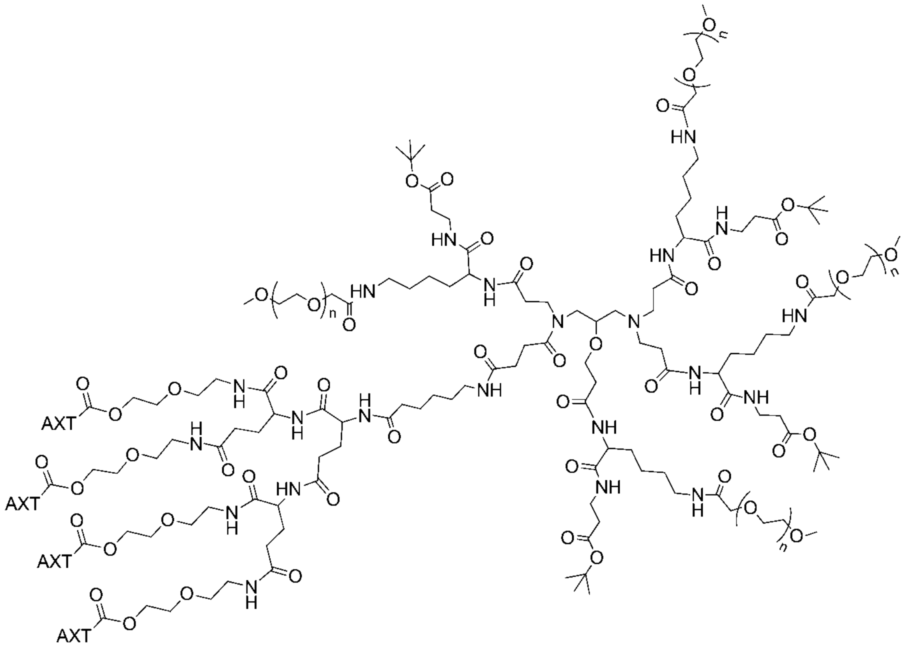

**71-278** (1.8 g, 0.0412 mmol), **85-50** (0.16 g, 0.0618 mmol), HBTU (0.023 g, 0.0618 mmol) and HOBT (0.008 g, 0.0618 mmol) were placed in a 250 mL flask containing DMF (25 mL), and the mixture was stirred at room temperature for approximately 20 minutes. DIEA (0.03 mL, 0.1855 mmol) was then slowly added dropwise, and the reaction was stirred continuously at room temperature. After the reaction was completed, n-hexane (250 mL) and methyl tert-butyl ether (70 mL) were added for precipitation, the supernatant was discarded, and n-hexane and methyl tert-butyl ether were added again for precipitation. This process was repeated five times. The mixture was filtered to provide a solid product, which was dried in a vacuum oven to provide 2.0 g of the product. $^1$H-NMR (600 MHz, DMSO-$d_6$) δ8.64-8.57 (m, 4H), 8.42-8.36 (m, 4H), 8.31-8.19 (m, 7H), 8.16-8.06 (m, 7H), 8.03-7.93 (m, 7H), 7.90-7.78 (m, 10H), 7.78-7.69 (m, 8H), 7.67-7.59 (m, 4H), 7.53-7.47 (m, 4H), 7.39-7.25 (m, 13H), 7.17-7.11 (m, 4H), 4.21-4.16 (m,15H), 3.86-3.83 (m,23H), 3.82-3.80 (m, 8H), 3.51-3.50 (m, 3828H), 3.22-3.17 (m, 14H), 2.77-2.72 (m, 36H), 2.37-2.30 (m, 24H), 1.88-1.83 (m, 8H), 1.59-1.53 (m, 10H), 1.38-1.37 (m, 36H), 1.27-1.25 (m, 12H)

24.13 Preparation of **85-57**

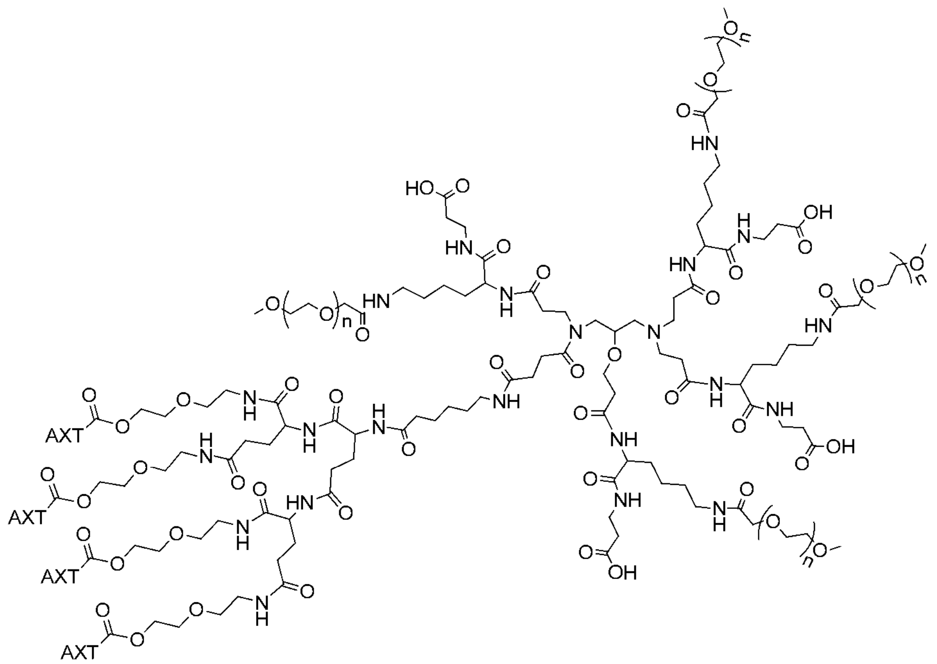

TFA (4 mL) was added into a flask containing **85-51** (1.0 g, 0.0216 mmol). The reaction mixture was dissolved with the assistance of ultrasonication, and stirred at room temperature overnight. After the reaction was completed, the reaction mixture was concentrated and evaporated to dryness to remove most of the TFA, and methyl tert-butyl ether (300 mL) was added for precipitation. A solid precipitated and was filtered. The filter cake was washed three times with methyl tert-butyl ether (40 mL). The filter cake was collected and dried under vacuum to provide 0.99 g of the product. $^1$H-NMR (600 MHz, DMSO-$d_6$) δ 9.78-9.74 (m, 4H), 8.64-8.55 (m, 4H), 8.41-8.36 (m, 4H), 8.25-8.21 (m, 6H), 8.16-8.09 (m, 8H), 7.89-7.79 (m, 14H), 7.74-7.69 (m, 10H), 7.52-7.43 (m, 8H), 7.34-7.31 (m, 12H), 7.15-7.12 (m, 6H), 4.55-4.52 (m, 8H), 4.26-4.16 (m, 7H), 3.88-3.80 (m,12H), 3.78-3.69 (m, 19H), 3.51-3.50 (m, 3828H), 3.19-3.17 (m, 14H), 3.10-3.02 (m, 20H), 2.99-2.94 (m, 10H), 2.86-2.82 (m, 6H), 2.41-2.33 (m, 10H), 2.19-2.01 (m, 16H), 1.62-1.52 (m, 6H), 1.49-1.41 (m, 10H), 1.23-1.16 (m, 12H)

24.14 Preparation of **85-63**

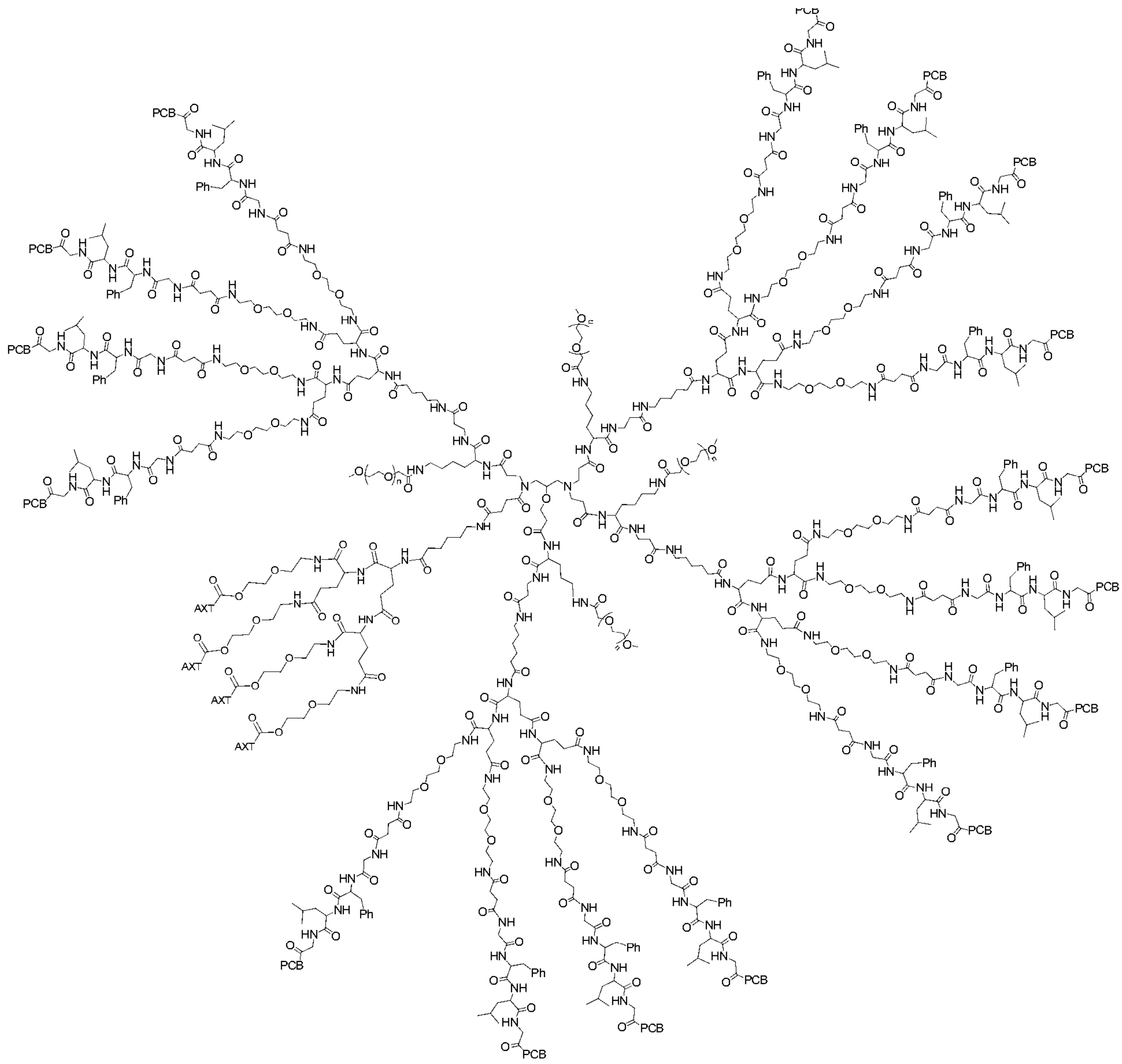

**85-57** (0.5 g, 0.0106 mmol), **85-67** (0.3 g, 0.064mmol), HBTU (0.024g, 0.0633mmol) and HOBT (0.0085g, 0.0633mmol) were added into a 250ml round-bottom flask, N-methylpyrrolidone (35ml) was added for dissolution, and the mixture was stirred at room temperature for about 20 minutes. DIEA (0.03ml, 0.1924mmol) was then slowly added dropwise. After the dropwise addition was completed, the reaction flask was allowed to stand at room temperature, and the mixture was stirred continuously. After the reaction was completed, n-hexane (100 mL) and methyl tert-butyl ether (100 mL) were added for precipitation, the supernatant was discarded, and n-hexane and methyl tert-butyl ether were added again for precipitation. This process was repeated three times. The mixture was filtered to provide a solid product. The obtained solid product was dissolved in a mixed solvent of dichloromethane/ methanol, loaded by dry method, and subjected to column chromatography using 1% triethylamine/6-12% methanol/dichloromethane as the eluent. The desired product was collected, concentrated, and dried in a vacuum oven to provide 0.58 g of the product. $^1$H-NMR (600 MHz, DMSO-$d_6$) δ 8.29-8.21 (m, 26H), 8.13-7.96 (m, 104H), 7.89-7.78 (m, 52H), 7.53-7.46 (m, 20H), 7.38-7.12 (m, 126H), 4.59-4.40 (m, 30H), 4.39-4.30 (m, 21H), 4.26-4.14 (m, 20H), 4.12-3.93 (m, 54H), 3.87-3.79 (m, 10H), 3.77-3.71 (m, 14H), 3.70-3.60 (m, 131H), 3.59-3.56 (m, 71H), 3.51-3.50 (m, 3828H), 3.25-3.23 (m, 19H), 3.22-3.13 (m, 164H), 3.09-3.04 (m, 45H), 2.95-2.80 (m,62H), 2.77-2.73 (m, 18H), 2.37-2.35 (m, 26H), 2.33-2.28 (m, 78H), 2.20-2.15 (m, 46H), 1.93-1.87 (m, 64H), 1.79-1.75 (m, 34H), 1.63-1.60 (m, 109H), 1.25-1.17 (m, 50H), 0.93-0.86 (m, 48H), 0.85-0.81 (m, 48H)

Example 25: Synthesis of Compound **88-233**

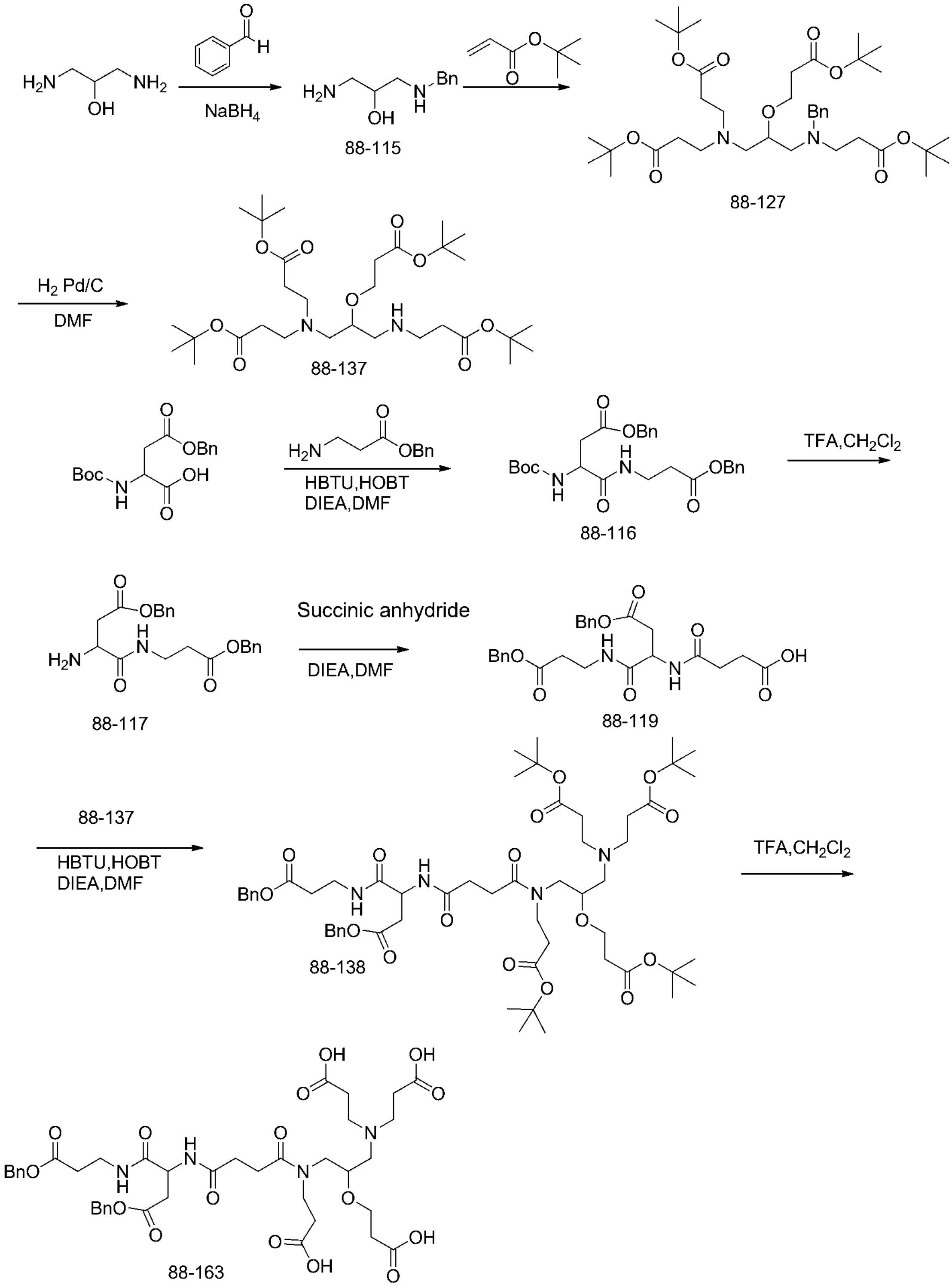
NaBH$_4$
88-115
88-127
H$_2$ Pd/C
DMF
88-137
HBTU,HOBT
DIEA,DMF
88-116
TFA,CH$_2$Cl$_2$
88-117
Succinic anhydride
DIEA,DMF
88-119
88-137
HBTU,HOBT
DIEA,DMF
88-138
TFA,CH$_2$Cl$_2$
88-163

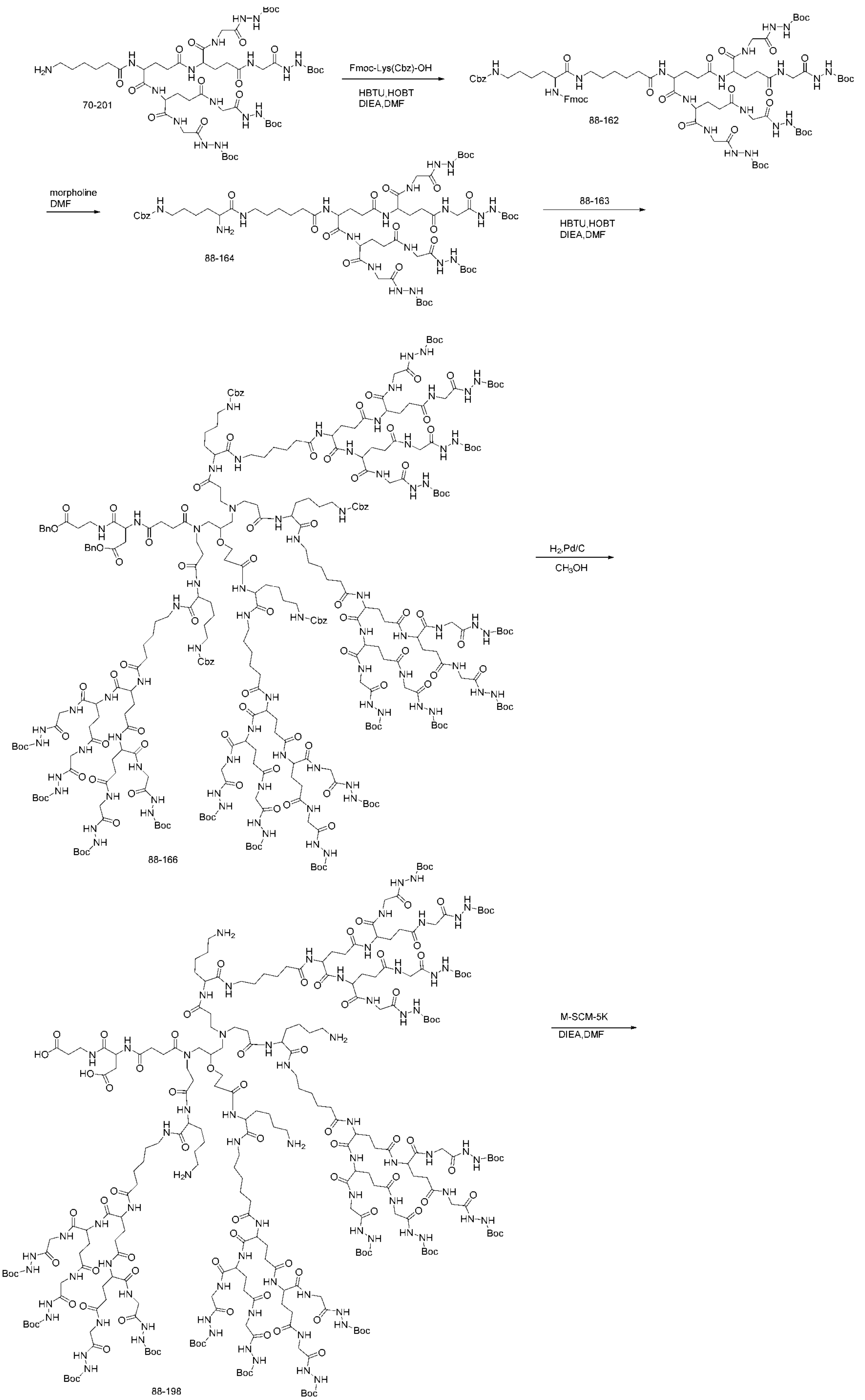


70-201

88-162

88-164

88-166

88-198

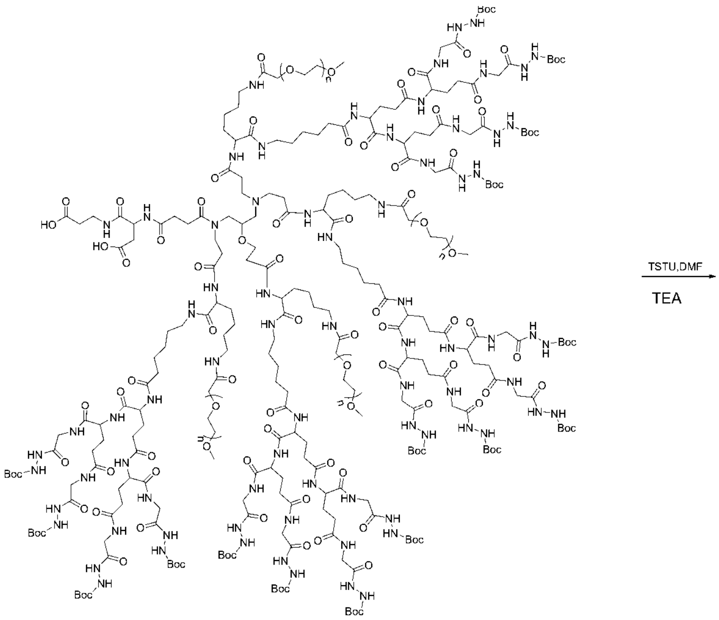


88-202

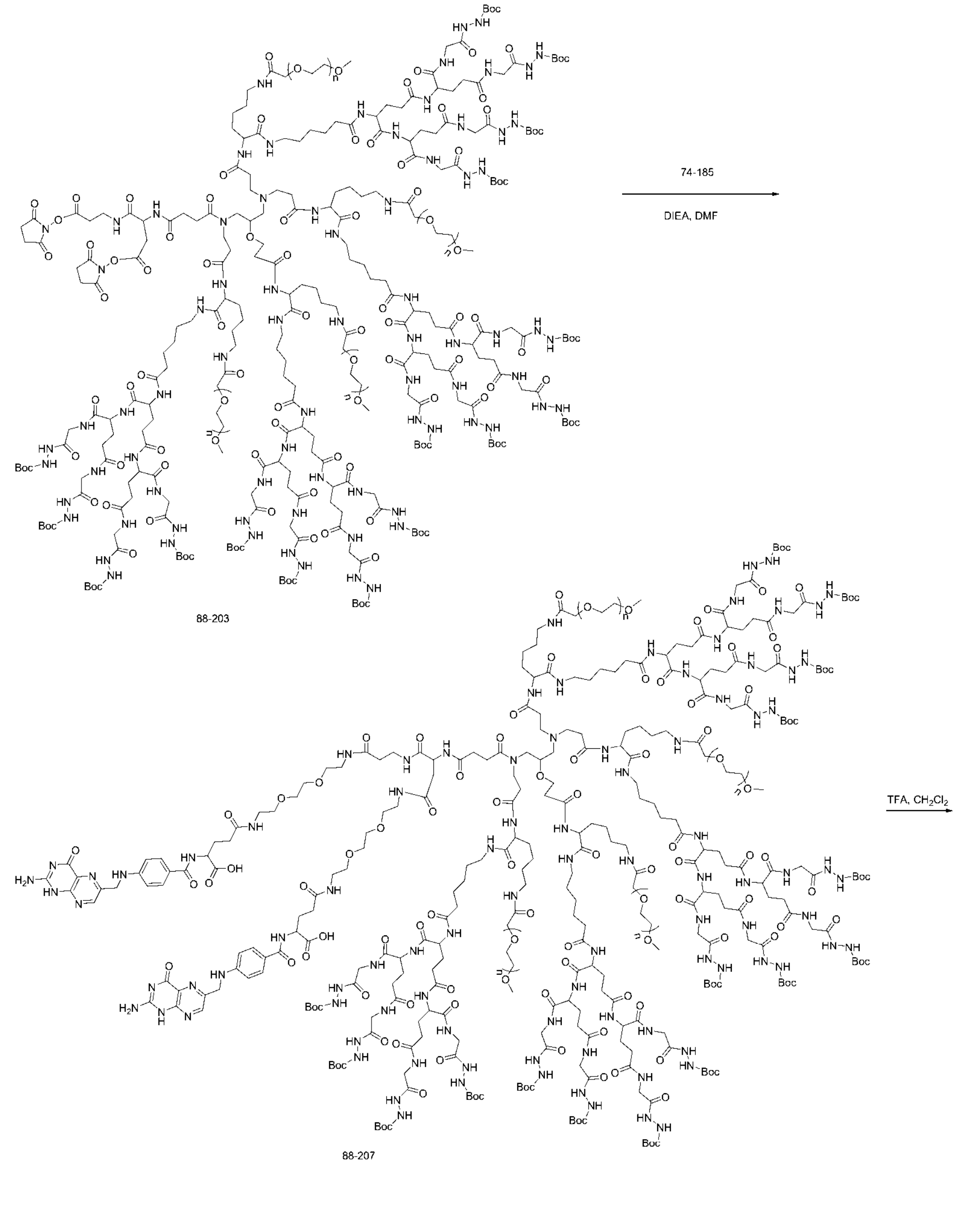


88-203

88-207

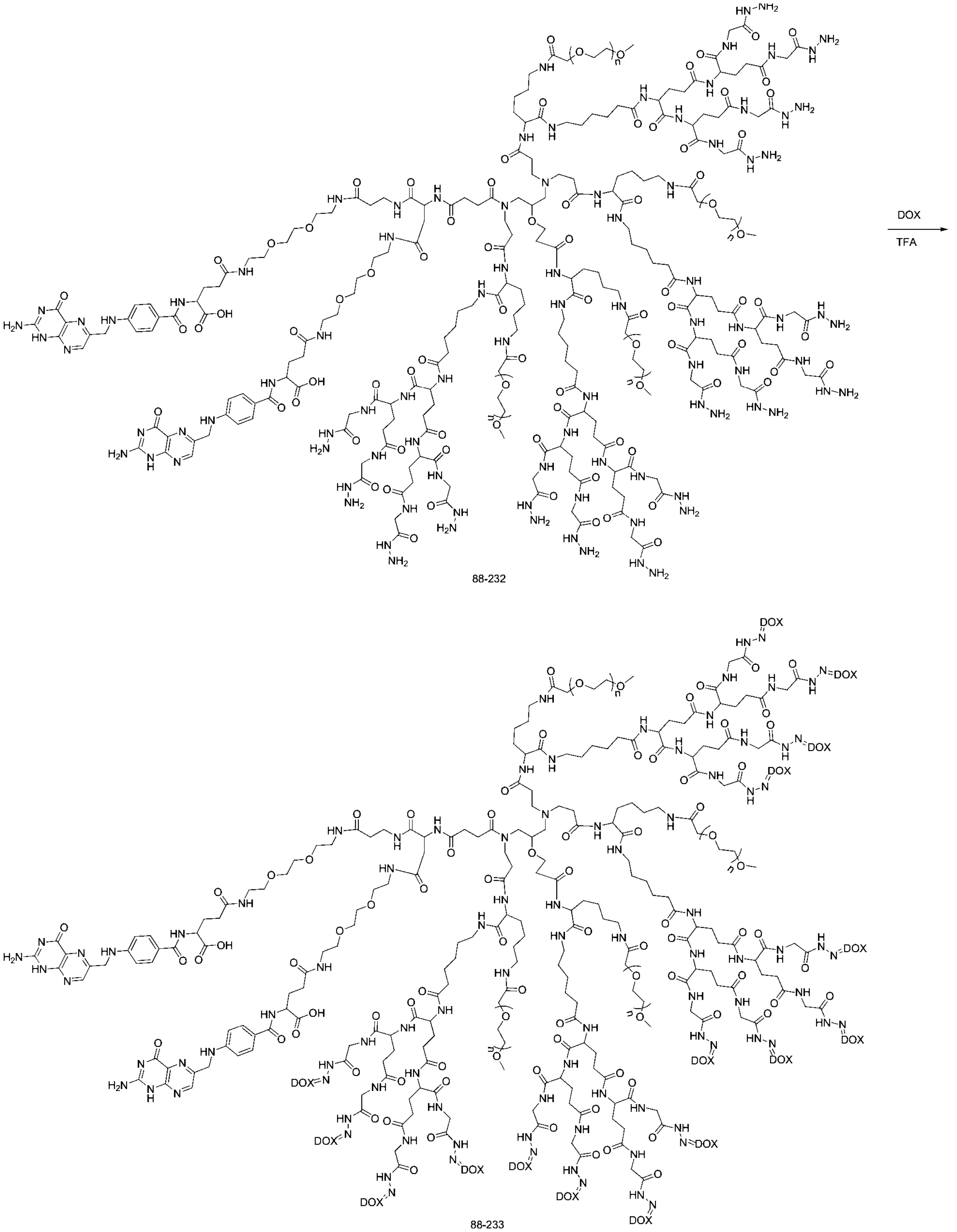


88-232

88-233

25.1 Preparation of **88-115**

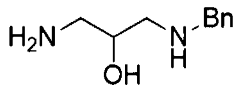

1,3-Diamino-2-propanol (10.0 g, 100.191 mmol) was dissolved in dichloromethane (30 mL) and stirred rapidly with a stirrer. Sodium sulfate (24 g, 124.88 mmol) and benzaldehyde (11.2 g, 89.383 mmol) were then added. After stirring at room temperature for 18 hours, the mixture was filtered, and the filtrate was concentrated. Anhydrous ethanol (20 mL) was added and cooled to 0°C. Solid $NaBH_4$ (10.95 g, 124.082 mmol) was then added in batches, each over ten minutes. After the addition was completed, the mixture was stirred for 1 hour. Water (10 mL) was added and the mixture was concentrated under reduced pressure. Dichloromethane (100 mL) was then added to dissolve the mixture. The mixture was washed with 1N HCl solution (20 mL × 2) and the pH was adjusted to alkaline with NaOH solution. The mixture was then extracted with dichloromethane (300 mL × 3). The dichloromethane phases were combined, dehydrated over anhydrous magnesium sulfate, and filtered. The filtrate was collected, and silica gel powder was added. The resulting product was loaded by dry method, and subjected to column chromatography using 1-4% methanol/dichloromethane as the eluent. The desired product was collected, concentrated, and dried to provide 5.4 g of the product with a yield of 54.00%. $^1H$-NMR (600 MHz, DMSO-$d_6$) δ 7.54-7.07 (m, 5H), 3.75-3.65 (m, 2H), 3.46 (s, 1H), 3.07-2.96 (m, 1H), 2.61-2.52 (m, 1H), 2.49-2.37 (m, 2H); ESI [M+H$^+$] 181.13

25.2 Preparation of **88-127**

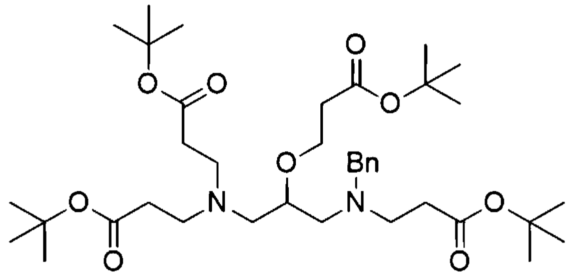

**88-115** (5.4 g, 29.95 mmol) was dissolved in DMSO (30 mL), and stirred in a water bath at 25°C, followed by a dropwise addition of 5 mol/L NaOH solution (2 mL). After stirring for 10 minutes, tert-butyl acrylate (34.8 mL, 239.66 mmol) was added dropwise, and the reaction was stirred overnight. The reaction was monitored by TLC. Ethyl acetate and saturated NaCl solution were added for extraction, followed by three extractions with ethyl acetate. The organic phases were combined, evaporated to dryness, and then dissolved in a methanol/dichloromethane mixture. Silica gel powder was added, and the mixture was evaporated to dryness. The resulting product was loaded by dry method, and subjected to column chromatography using ethyl acetate/petroleum ether as the eluent. The desired product was collected, concentrated, and dried to provide 3.5 g of the product. $^1H$-NMR (600 MHz, DMSO-$d_6$) δ 7.34-7.19 (m, 5H), 3.70-3.61 (m, 2H), 3.59-3.56 (m, 2H), 3.41-3.38 (m, 1H), 2.68-2.58 (m, 6H), 2.44-2.20 (m, 12H), 1.38 (s, 36H)

25.3 Preparation of **88-137**

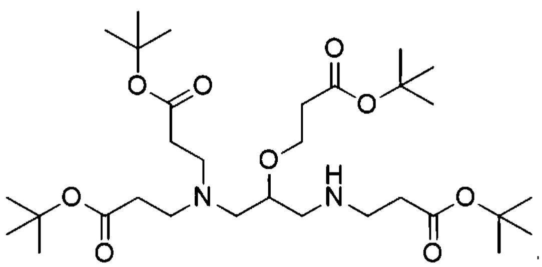

**88-127** (5.6 g, 8.551 mmol) was dissolved in DMF (20 mL). A stirrer bar was added, and 10% Pd/C catalyst (0.4 g) was added. Hydrogen was introduced at a pressure of 200 psi, and the reaction was carried out under stirring at room temperature overnight. After the reaction was completed, the reaction mixture was filtered using a Büchner funnel and filter

paper. The filter cake was washed with DMF (5 mL × 3). The filtrate was placed in a 250 mL round-bottom flask, and ethyl acetate/saturated NaCl solution were added for extraction. The aqueous phase was further extracted three times with ethyl acetate. The organic phases were combined and evaporated to dryness to provide 4.05 g of the product.

25.4 Preparation of **88-116**

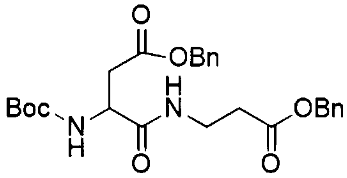

Boc-L-Asp-4-OBzl (6.0 g, 18.55 mmol), benzyl β-alanine hydrochloride (7.17 g, 20.41 mmol), HBTU (7.74 g, 20.41 mmol) and HOBT (2.76 g, 20.41 mmol) were added to a 500 mL flask, then DMF (30 mL) was added to dissolve the mixture, and the mixture was stirred at -5°C for approximately 20 minutes. DIEA (6.1 mL, 37.1 mmol) was then slowly added dropwise. After the dropwise addition was completed, stirring was continued at -5°C for 1 hour. The mixture was then brought to room temperature and allowed to react under stirring. After the reaction was completed, the reaction mixture was transferred to a 2 L separatory funnel, and saturated sodium bicarbonate solution (250 mL) and ethyl acetate (300 mL) were added for extraction. The organic phase was collected, and the aqueous phase was further extracted with ethyl acetate (200 mL × 3). The organic phases were combined and washed with saturated brine (200 mL × 2), concentrated, and evaporated to dryness to provide 8.98 g of the product.

25.5 Preparation of **88-117**

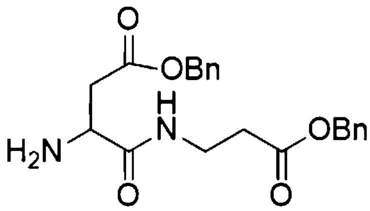

To a flask containing **88-116** (8.98 g, 18.55 mmol), dichloromethane (20 mL) was added for dissolution with the assistance of ultrasonication, then TFA (20.7 mL, 287.25 mmol) was added, and the mixture was reacted under stirring at room temperature overnight. After the reaction was completed, the reaction mixture was evaporated to provide an oil using a rotary evaporator. Methyl tert-butyl ether (40 mL × 3) was then added for washing. The supernatant was discarded, and the residue was evaporated to dryness, and dried in a vacuum oven to provide 7.12 g of the product.

25.6 Preparation of **88-119**

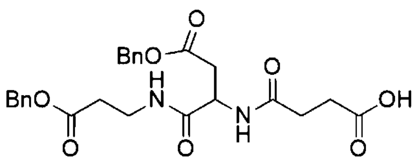

**88-117** (7.12 g, 18.55 mmol) was added to a 500 mL flask containing DMF (20 mL) and dissolved with the assistance of ultrasonication. DIEA (15.3 mL, 92.75 mmol) was slowly added dropwise. After the dropwise addition was completed, the mixture was stirred for 5 minutes. Succinic anhydride (5.568 g, 55.65 mmol, purchased from Innochem) was then added, and stirred for 3 hours. After the reaction was completed, saturated brine (200 mL) and ethyl acetate (200 mL) were added for extraction. After standing for layer separation, the organic phase was collected, and the aqueous

phase was further extracted with ethyl acetate (200 mL × 3). The organic phases were combined, evaporated to dryness to provide a solid, which was dissolved in dichloromethane and methanol, loaded by dry method, and subjected to column chromatography using 1% methanol/dichloromethane as the eluent. The desired product was collected, concentrated, and dried to provide 8.23 g of the product with a yield of 91.65%. ESI [M+H+] 485.18

25.7 Preparation of **88-138**

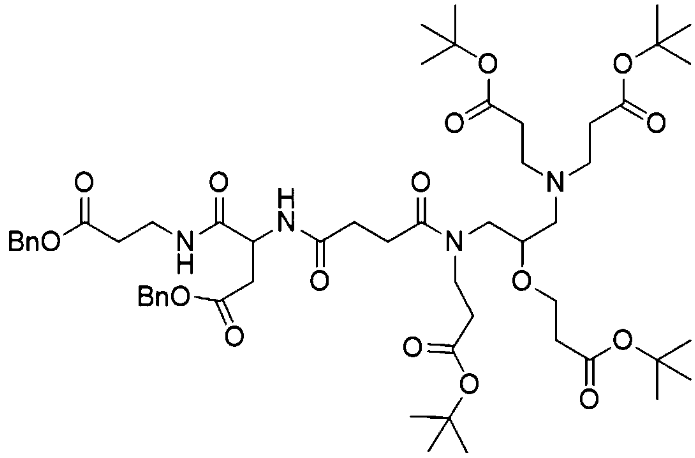

**88-119** (1.35 g, 2.78 mmol), **88-137** (1.6 g, 2.65 mmol), HBTU (1.05 g, 2.78 mmol) and HOBT (0.37 g, 2.78 mmol) were added to a 500 mL flask, DMF (50 mL) was added to dissolve the mixture, and the mixture was stirred at -5°C for approximately 20 minutes. DIEA (36.5 mL, 153.68 mmol) was then slowly added dropwise. After the dropwise addition was completed, stirring was continued at -5°C for 1 hour. The mixture was then brought to room temperature and reacted under stirring. After the reaction was completed, the reaction mixture was transferred to a 2 L separatory funnel, and saturated sodium bicarbonate solution (250 mL) and ethyl acetate (300 mL) were added for extraction. The organic phase was collected, and the aqueous phase was further extracted with ethyl acetate (200 mL × 3). The organic phases were combined and washed with saturated brine (200 mL × 2). The organic phases were collected, concentrated, and evaporated to dryness to provide 1.9 g of the product. $^1$H-NMR (600 MHz, DMSO-*d*$_6$) δ 8.32-8.24 (m , 1H), 7.98-7.92 (m, 1H), 7.49-7.19 (m, 10H), 5.15-5.00 (m, 4H), 4.67-4.53 (m, 1H), 3.76-3.61 (m, 2H), 3.60-3.46 (m, 5H), 2.92-2.87 (m, 1H), 2.84-2.78 (m, 1H), 2.75-2.71 (m, 1H), 2.70-2.53 (m, 11H), 2.40-2.12 (m, 12H), 1.46-1.33 (m, 36H); ESI [M+H+] 1069.59

25.8 Preparation of **88-163**

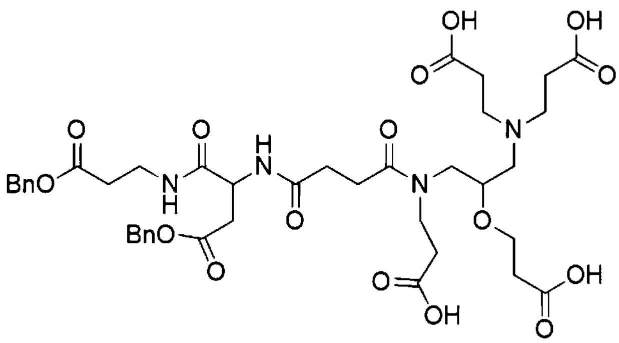

**88-138** (0.60 g, 0.56 mmol) was dissolved in dichloromethane (5 mL) in a flask with the assistance of ultrasonication. TFA (5 mL, 33.65 mmol) was then added, and the reaction was stirred at room temperature overnight. After the reaction was completed, the reaction mixture was evaporated to provide an oil using a rotary evaporator, methyl tert-butyl ether (40 mL) was added for precipitation, the supernatant was discarded, and methyl tert-butyl ether (40 mL) was added again for precipitation. This process was repeated three times. The resulting product was evaporated to

dryness and dried in a vacuum oven to provide 0.47 g of the product. $^1$H-NMR (600 MHz, DMSO-$d_6$) δ 13.95-11.31 (m, 4H), 8.36-8.20 (m, 1H), 8.01-7.87 (m, 1H), 7.38-7.29 (m, 10H), 5.17-5.02 (m, 4H), 3.89-3.81 (m, 2H), 3.69-3.51 (m, 18H), 3.18-3.15 (m, 1H), 2.83-2.72 (m, 5H), 2.64-2.55 (m, 4H), 2.43-2.35 (m, 2H); ESI [M+H$^+$] 845.34

25.9 Preparation of **88-162**

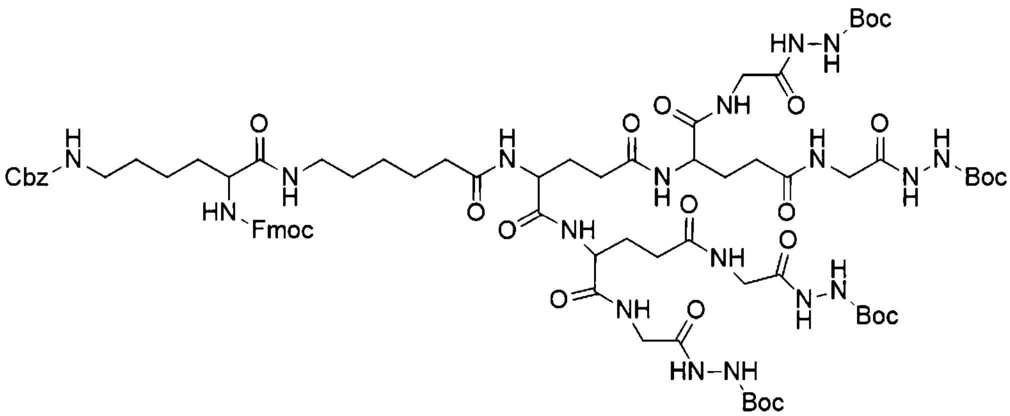

**70-201** (0.83 g, 0.69 mmol), N'-Fluorenylmethoxycarbonyl-N-benzyloxycarbonyl-L-lysine (0.38 g, 0.76 mmol), HBTU (0.28 g, 0.76 mmol) and HOBT (0.10 g, 0.76 mmol) were added to a 500 mL flask, then DMF (50 mL) was added to dissolve the mixture, and the mixture was stirred at -5°C for approximately 20 minutes. DIEA (0.3 mL, 1.52 mmol) was then slowly added dropwise. After the dropwise addition was completed, stirring was continued at -5°C for 1 hour. The mixture was then brought to room temperature and stirred for reaction. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried to provide 1.16 g of the product.

25.10 Preparation of **88-164**

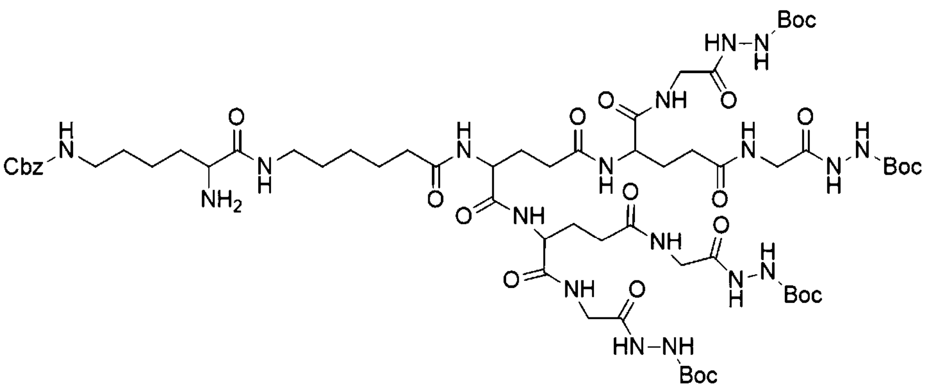

To a flask containing **88-162** (1.16 g, 0.69 mmol), DMF was added for dissolution with the assistance of ultrasonication, then morpholine (1 mL, 10.34 mmol) was added, and the mixture was reacted under stirring at room temperature for 2 h. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, and dried to provide 0.75 g of the product. $^1$H-NMR (600 MHz, DMSO-$d_6$) δ 8.36-8.08 (m, 8H), 7.96 (s, 9H), 5.00 (s, 2H), 4.26 (s, 3H), 4.19-4.08 (m, 8H), 3.61-3.57 (m, 8H), 3.10-3.04 (m, 4H), 2.24-2.08 (m, 14H), 1.51-1.46 (m, 4H), 1.39 (s, 36H), 1.30-1.19 (m, 8H); ESI [M+H$^+$] 1465.75

25.11 Preparation of **88-166**

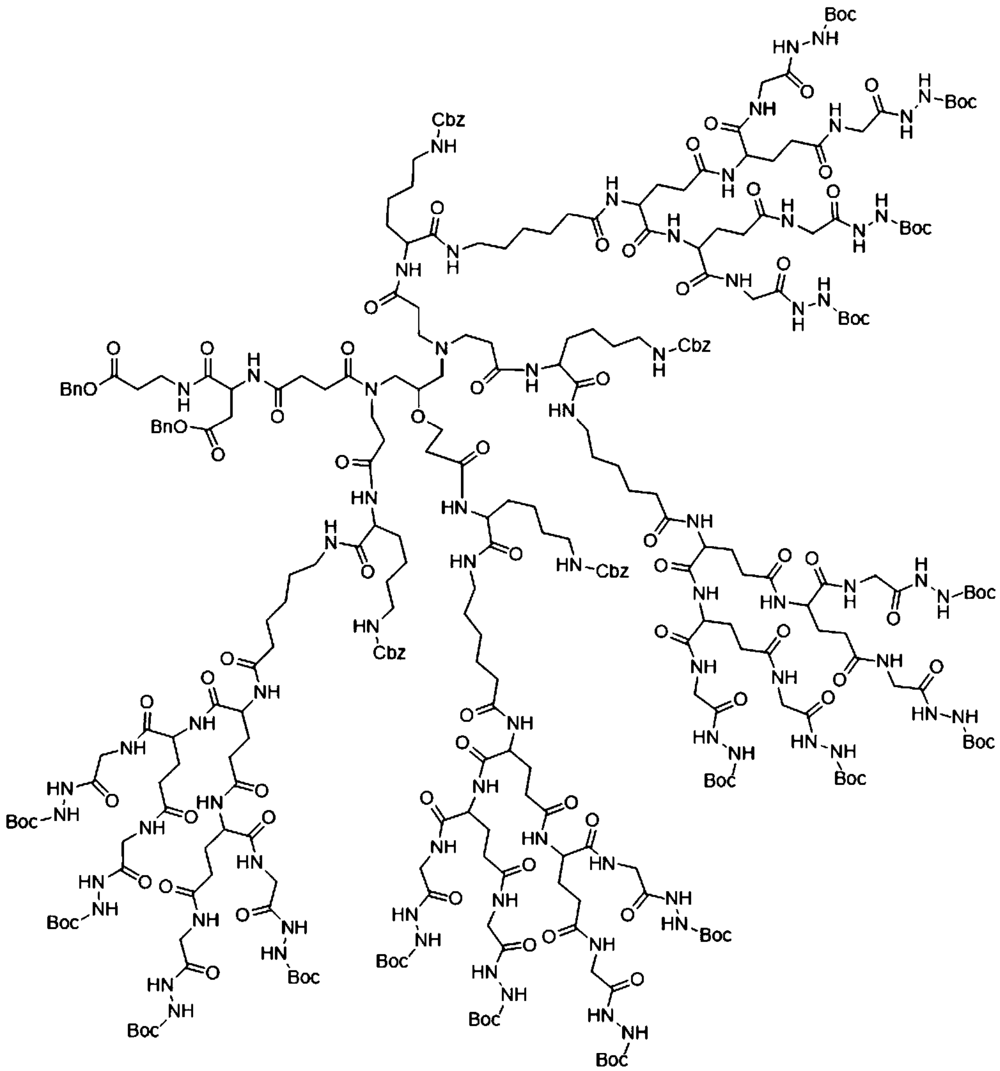

**88-164** (0.75 g, 0.51 mmol), HBTU (0.19 g, 0.51 mmol) and HOBT (0.07 g, 0.51 mmol) were weighed and added into a flask containing **88-163** (0.09 g, 0.12 mmol), then an appropriate amount of DMF was added to dissolve the mixture, and the mixture was allowed to stir at -5°C. DIEA (0.3 mL, 1.8 mmol) was then slowly added dropwise. After the dropwise addition was completed, the reaction was carried out for half an hour, the flask was then taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, and dried to provide the product.

25.12 Preparation of **88-198**

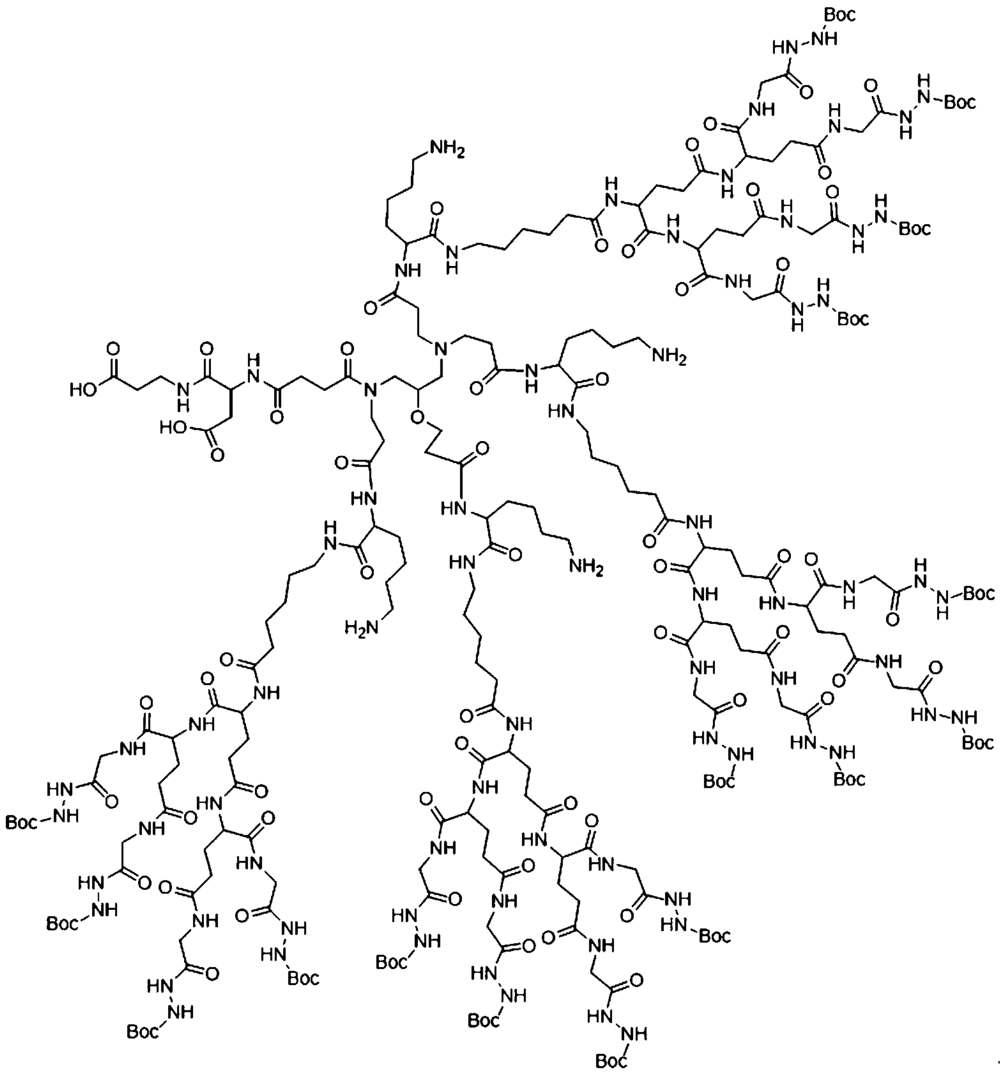

**88-166** (0.49 g, 0.0738 mmol) and 10% Pd/C catalyst (0.18 g) were placed in a hydrogenation reactor, and then methanol (30 mL) was added for dissolution. The hydrogenation reactor was sealed, evacuated with a water pump, and filled with hydrogen. This process was repeated three times. The pressure reading of the hydrogenation reactor was finally adjusted to 1.8 MPa. The reaction was allowed to proceed overnight at room temperature. After the reaction was completed, the reaction mixture was filtered through Celite, and the filter cake was washed with methanol (20 mL × 3). The filtrate was evaporated to dryness, then toluene (2 mL) and dichloromethane (10 mL) were added, and the mixture was evaporated to dryness. This process was repeated three times to remove the methanol and provide the product.

25.13 Preparation of **88-202**

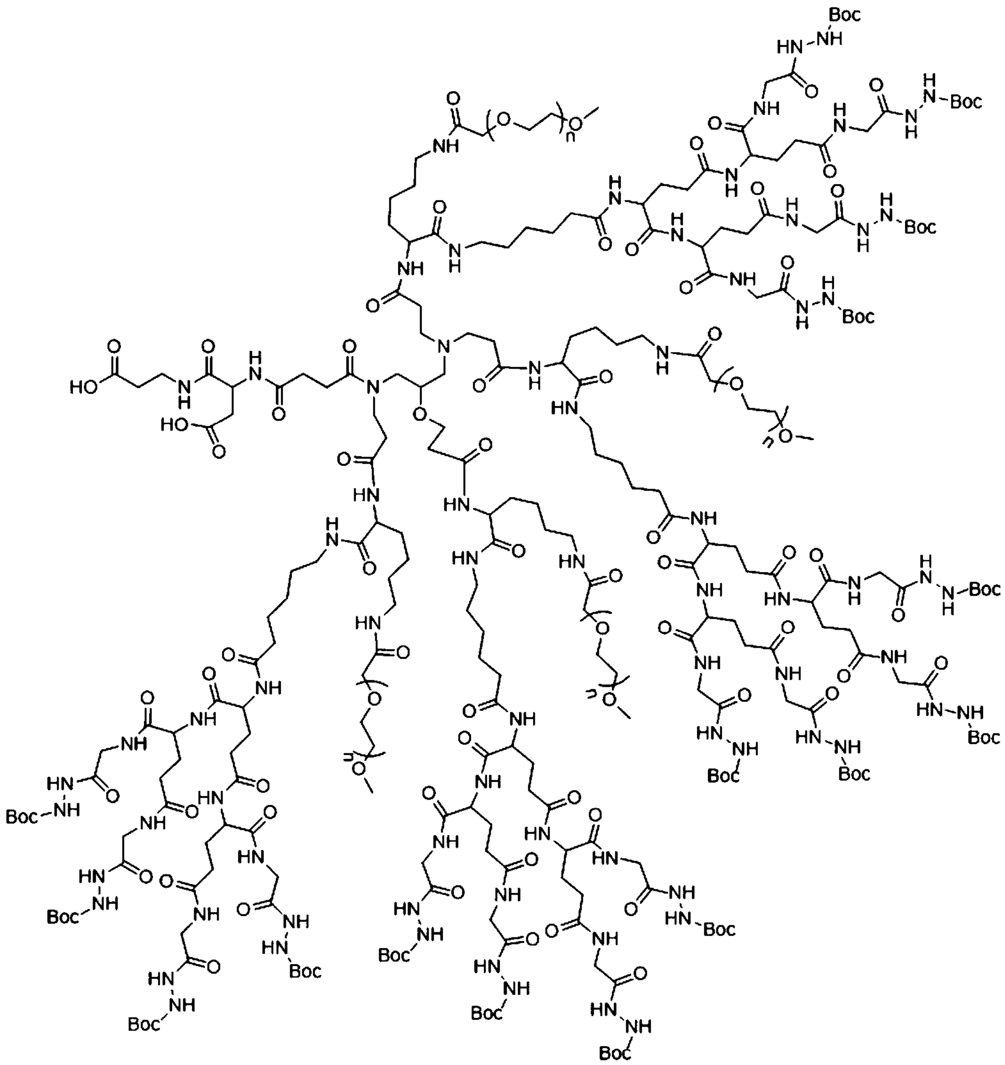

**88-198** (0.437 g, 0.0738 mmol) was placed in a 250 mL flask, and DMF (20 mL) was added for dissolution, followed by a slow and dropwise addition of a DMF solution containing DIEA (0.2 mL, 1.23 mmol) and M-SCM-5K (1.725 g, 0.3249 mmol, purchased from Jenkem), and reacted under stirring in the dark at low speed at room temperature for one week. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried in a vacuum oven to provide 2.13 g of the product.

25.14 Preparation of **88-203**

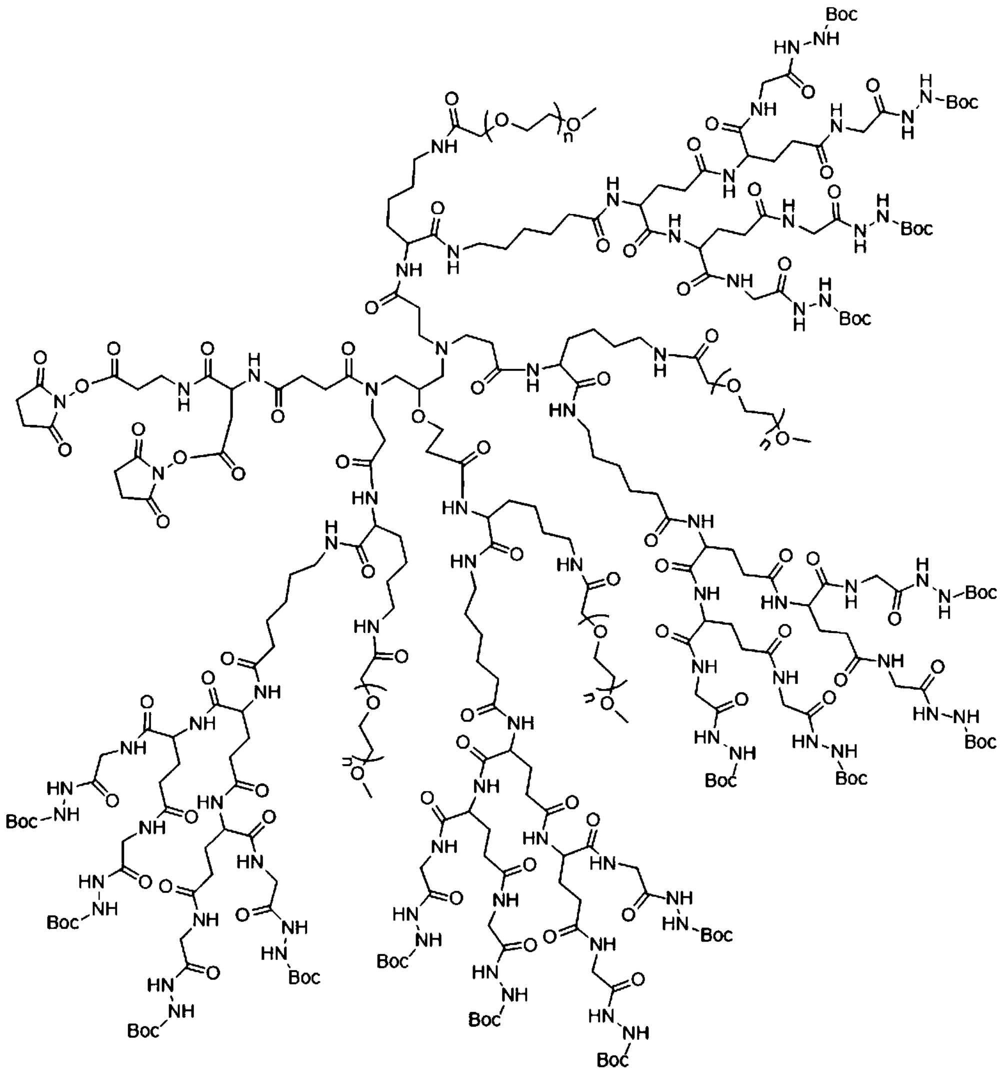

**88-202** (2.0 g, 0.073 mmol), TSTU (0.12 g, 0.16 mmol) and DMF (10 mL) were dissolved in a 50 mL flask, then triethylamine (0.12 mL, 0.95 mmol) was added, and the mixture was reacted under stirring at room temperature. After the reaction was completed, n-hexane (30 mL) and methyl tert-butyl ether (200 mL) were added for precipitation, the supernatant was discarded, and n-hexane and methyl tert-butyl ether were added again for precipitation. This process was repeated four times. The desired product was collected and dried in a vacuum oven to provide the product.

25.15 Preparation of **88-207**

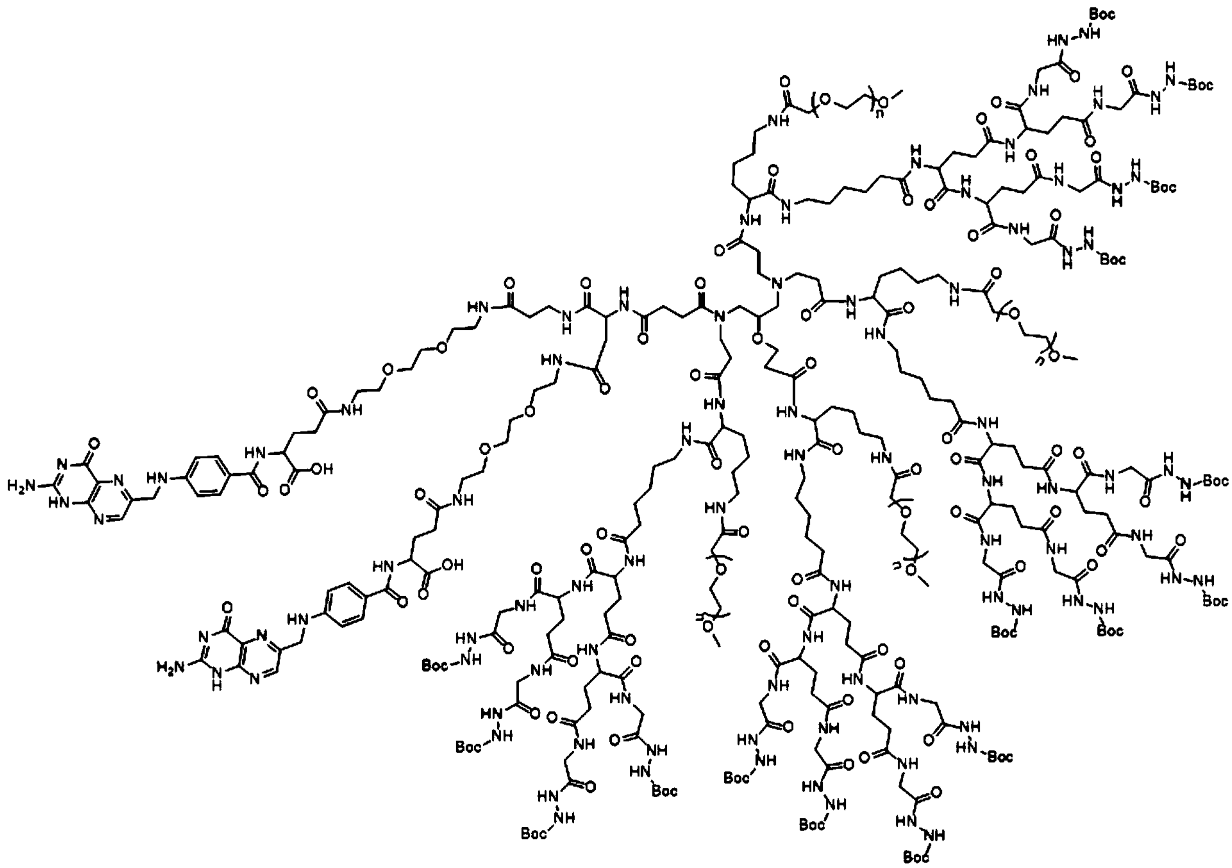

**88-203** (0.0738 mmol) and **74-185** (0.09 g, 0.1624 mmol) were added to a 250 mL round-bottom flask, then DMF (15 mL) was added for dissolution with the assistance of ultrasonication, and the contents were stirred at 0°C for 10 minutes. DIEA (0.12 mL, 0.7384 mmol) was then added dropwise. The flask was taken out, and reaction was carried out under stirring at room temperature. After the reaction was completed, methyl tert-butyl ether (200 mL) was added for precipitation. A solid precipitated and was filtered. The filter cake was dissolved in a methanol/dichloromethane mixture, then 200-300 mesh silica gel powder was added, and the mixture was evaporated to dryness. The resulting product was loaded by dry method, and subjected to column chromatography using 9% methanol/dichloromethane as the eluent. The desired product was collected, concentrated and evaporated to dryness to provide the product.

25.16 Preparation of **88-232**

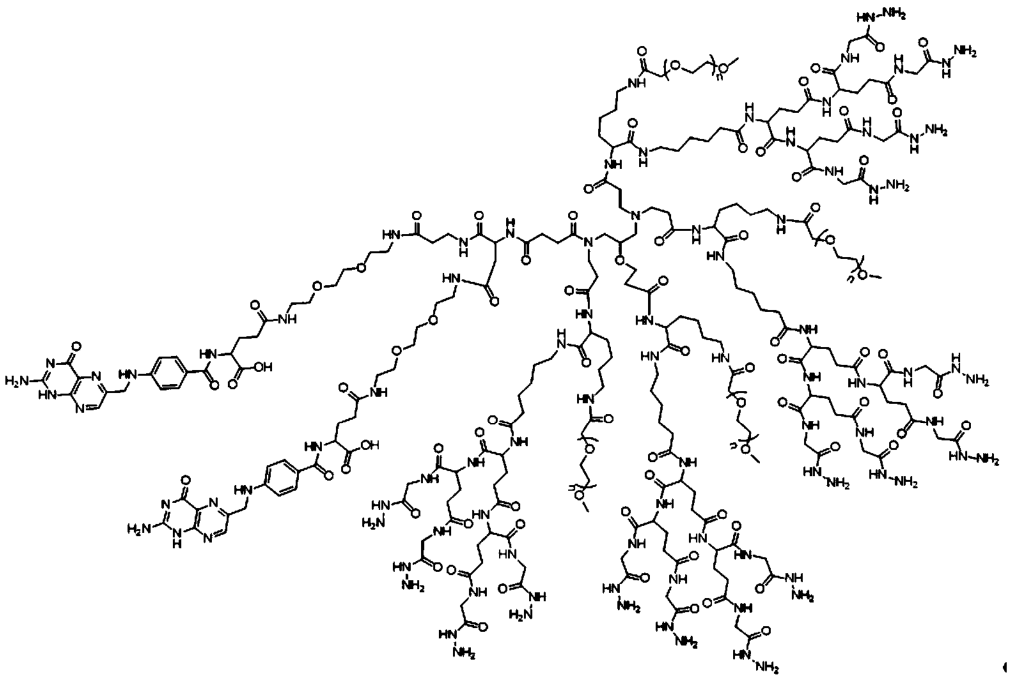

**88-207** (0.0607 mmol) was placed in a 500 mL round-bottom flask, then TFA (15 mL) was added, and the mixture was reacted under stirring at room temperature. After the reaction was completed, an appropriate amount of dichloromethane was added, and the dichloromethane and TFA were removed by rotary evaporation under reduced pressure. Dichloromethane was then added and rotary evaporated under reduced pressure. This process was repeated several times until the product became a solid powder. An appropriate amount of dichloromethane was added for dissolution, DIEA was added to adjust the pH to alkaline, and the mixture was concentrated under reduced pressure and evaporated to dryness to provide 1.5 g of the product.

25.17 Preparation of **88-233**

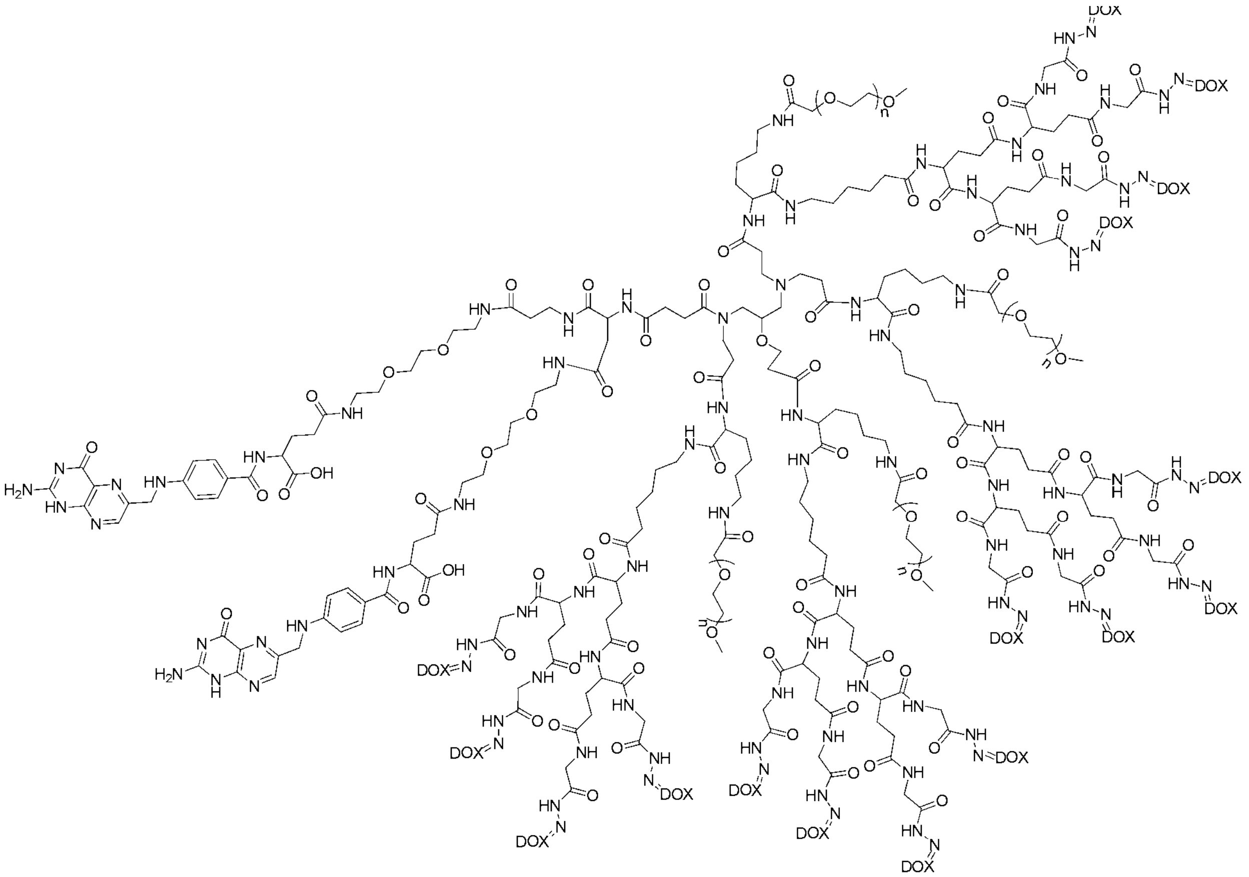

**88-232** (0.0607 mmol) was added to a 250 mL round-bottom flask, and then methanol (20 mL) was added for dissolution with the assistance of ultrasonication. DOX.HCl (1.13 g, 1.9424 mmol) was added and dissolved with the assistance of ultrasonication. Then TFA (1.386 mL, 12.16 mmol) was added, and the mixture was reacted under stirring at room temperature. After the reaction was completed, the mixture was evaporated to dryness, n-hexane (30 mL) and methyl tert-butyl ether (200 mL) were added for precipitation, the supernatant was discarded, and n-hexane and methyl tert-butyl ether were added again for precipitation. This process was repeated four times. The mixture was filtered to obtain a solid product. The obtained solid product was dissolved in a mixed solvent of dichloromethane and methanol, loaded by dry method, and subjected to column chromatography using 4-12% methanol/dichloromethane as the eluent. The desired product was collected, concentrated, and dried in a vacuum oven to provide 0.4 g of the product with a yield of 18.61%. $^1$H-NMR (600 MHz, DMSO-$d_6$) δ 14.24-13.93 (m, 16H), 13.51-13.15 (m, 16H), 9.27-8.98 (m, 13H), 8.75-7.48 (m, 56H), 7.25-6.45 (m, 49H), 5.57-5.44 (m, 5H), 5.36-5.24 (m, 5H), 5.05-4.82 (m, 10H), 4.64-4.38 (m, 16H), 4.27-4.09 (m, 17H), 4.04-3.82 (m, 32H), 3.70-3.58 (m, 54H), 3.54-3.46 (m, 2141H), 3.44-3.39 (m, 77H), 3.27-3.15 (m, 16H), 3.04-2.88 (m, 14H), 2.78-2.70 (m, 6H), 2.43-2.36 (m, 11H), 2.23-2.09 (m, 16H), 1.98-1.81 (m, 27H), 1.58-1.44 (m, 19H), 1.42-1.29 (m, 130H), 1.23-1.08 (m, 175H), 0.93-0.79 (m, 36H)

Example 26: Synthesis of Compound **82-220**

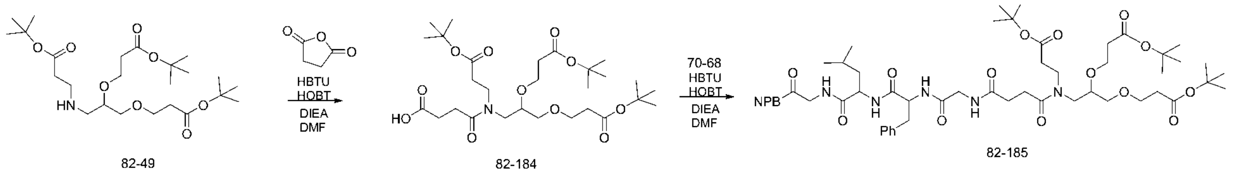


82-49 82-184 82-185

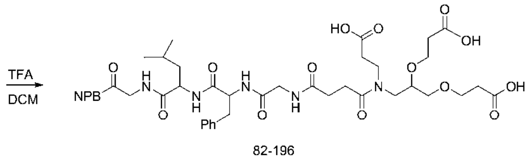


82-196

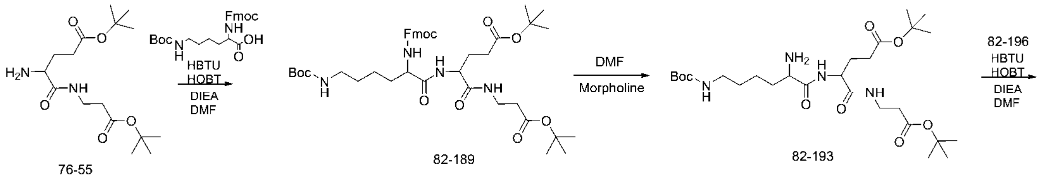


76-55 82-189 82-193

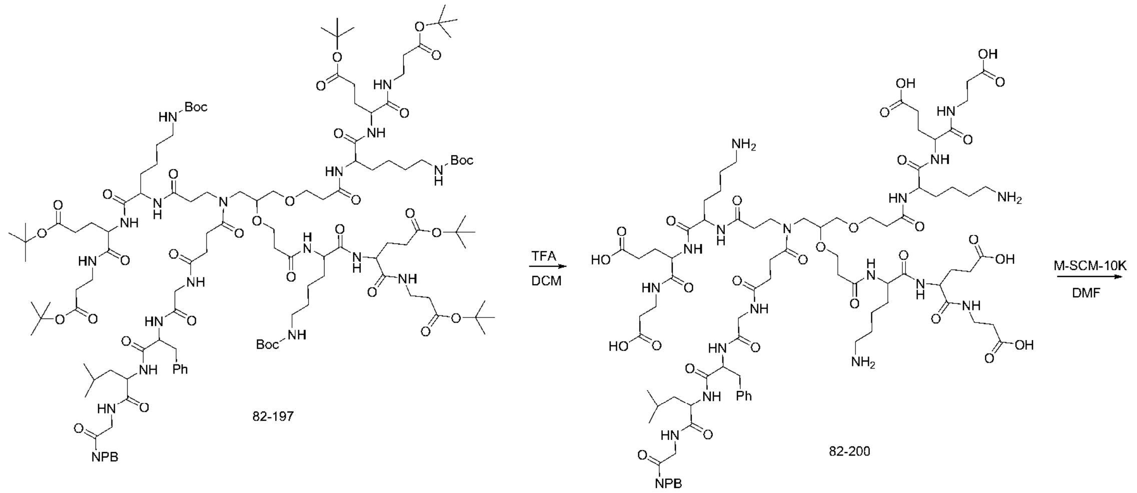


82-197 82-200

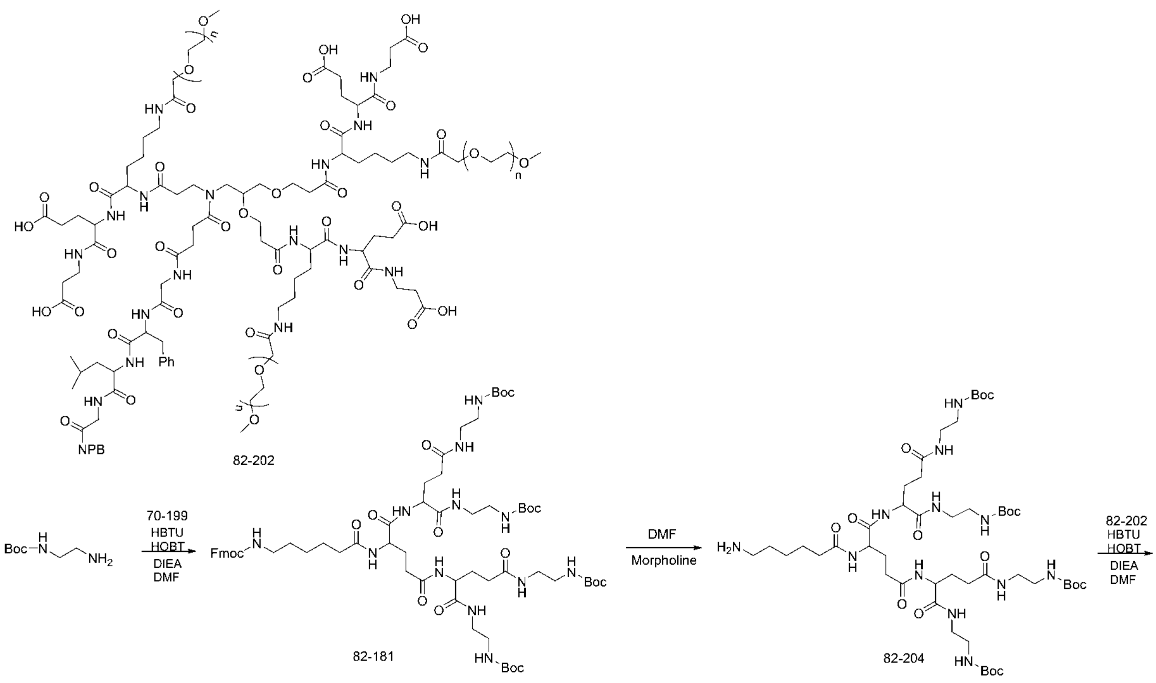


82-202

82-181 82-204

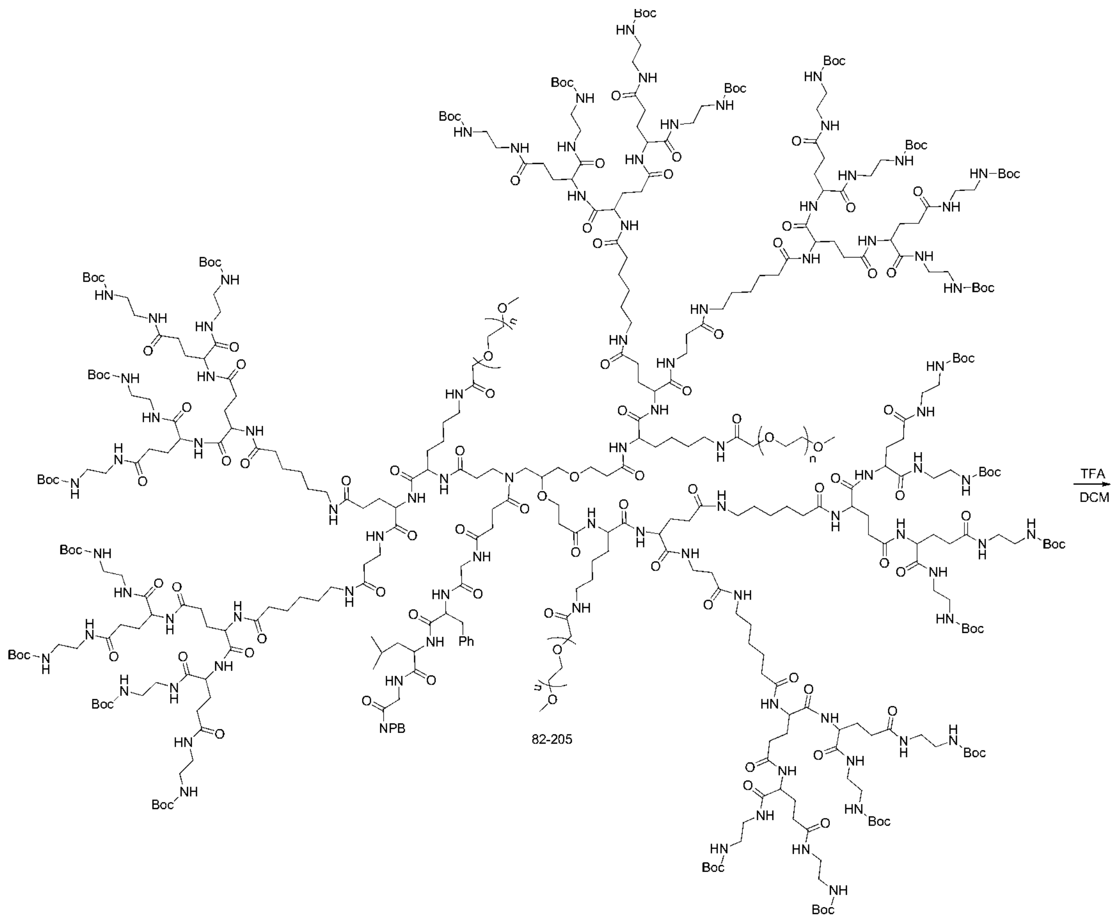


82-205

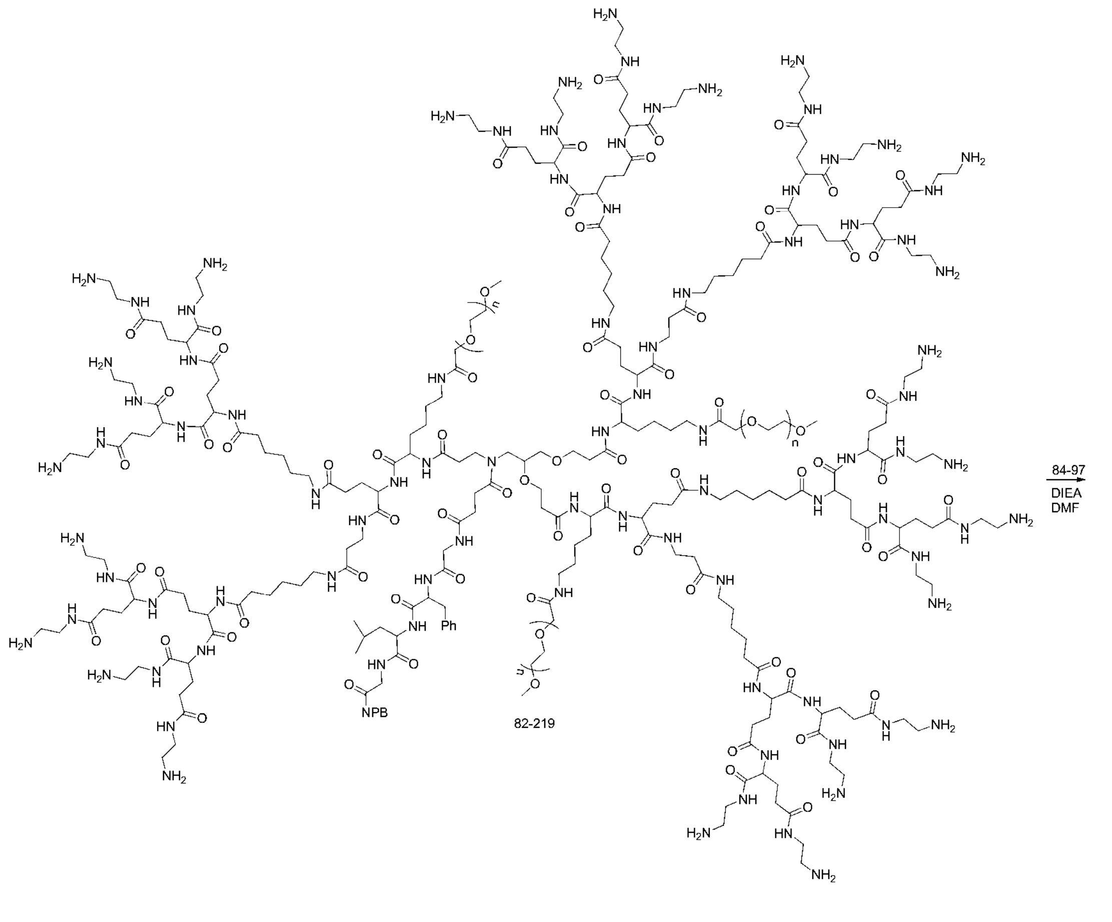


82-219

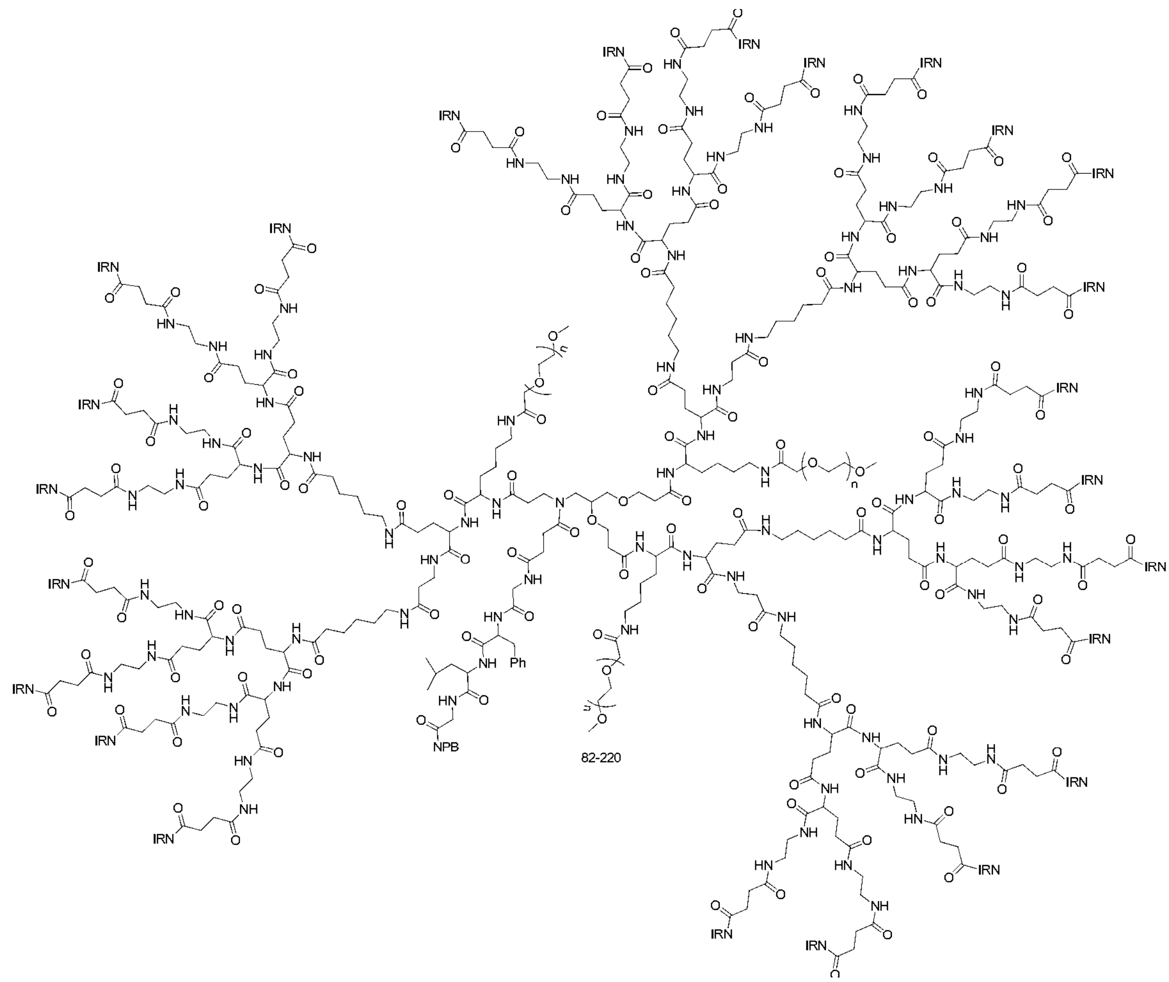

82-220

26.1 Preparation of **82-184**

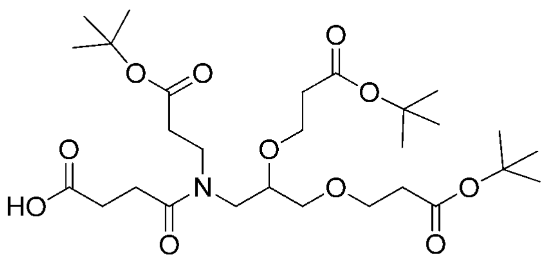

**82-49** (1.2 g, 2.5193 mmol) was weighed and added into a flask containing succinic anhydride (0.63 g, 6.2982 mmol), then an appropriate amount of DMF was added for dissolution, and the mixture was allowed to stir at -5°C. DIEA (1.73 mL, 10.4971 mmol) was slowly added dropwise. After the dropwise addition was completed, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product. The solid product was dissolved in a mixture of dichloromethane and methanol, loaded by dry method, and subjected to column chromatography using 3-6% methanol/dichloromethane as the eluent. The desired product was collected, concentrated, and dried in a vacuum oven to provide 1.45 g of product (the yield exceeded 100%).

26.2 Preparation of **82-185**

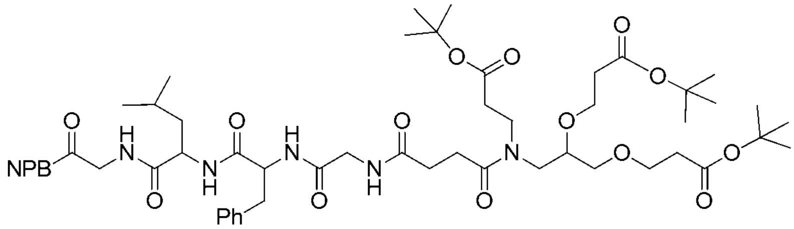

**82-184** (2.5193 mmol), HBTU (0.96 g, 2.5193 mmol) and HOBT (0.34 g, 2.5193 mmol) were weighed and added into a flask containing **70-68** (1.75 g, 2.5193 mmol), then an appropriate amount of DMF was added to dissolve the mixture, and allowed to stir at -5°C. DIEA (0.92 mL, 5.5425 mmol) was slowly added dropwise. After the dropwise addition was completed, the reaction was carried out for half an hour, the flask was then taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried to provide 1.64 g of the product with a yield of 52.06%. $^1$H-NMR (600 MHz, DMSO-*d*$_6$) δ 9.31 (s, 1H), 8.58 (s, 1H), 8.38-8.30 (m, 1H), 8.13-7.97 (m, 6H), 7.95-7.88 (m, 2H), 7.84-7.72 (m, 1H), 7.62-7.56 (m, 1H), 7.55-7.51 (m, 1H), 7.28-7.23 (m, 5H), 7.20-7.15 (m, 1H), 4.54-4.41 (m, 2H), 4.38-4.30 (m, 1H), 4.14-3.82 (m, 4H), 3.78-3.48 (m, 10H), 3.45-3.38 (m, 3H), 3.31-3.25 (m, 1H), 3.19-3.02 (m, 3H), 2.91-2.52 (m, 6H), 2.46-2.22 (m, 9H), 2.00-1.93 (m, 1H), 1.83-1.73 (m, 2H), 1.41-1.36 (m, 27H), 1.32 (s, 6H)

26.3 Preparation of **82-196**

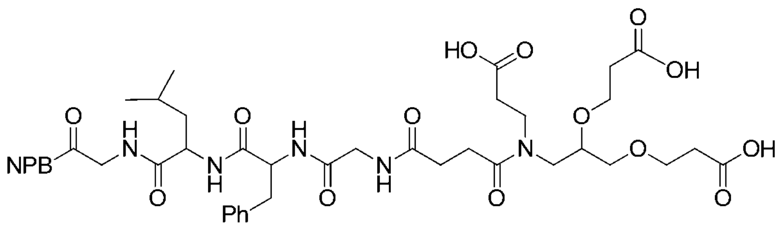

To a flask containing **82-195** (1.64 g, 1.3572 mmol), dichloromethane was added for dissolution with the assistance of ultrasonication, then TFA (3.02 mL, 40.7186 mmol) was added, and the mixture was reacted under stirring at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide the product.

26.4 Preparation of **82-189**

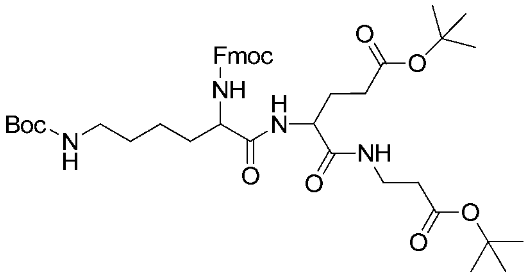

**76-55** (7.0510 mmol), HBTU (2.94 g, 7.7561 mmol) and HOBT (1.05 g, 7.7561 mmol) were weighed and added into a flask containing Fmoc-Lys(Boc)-OH (3.30 g, 7.0561 mmol, purchased from Innochem), then an appropriate amount of DMF was added to dissolve the mixture, and the contents were allowed to stir at -5°C. DIEA (2.56 mL, 15.5122 mmol) was slowly added dropwise. After the dropwise addition was completed, the reaction was carried out for half an hour, the flask was then taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, and dried in a vacuum oven to provide 6.5 g of the product.

26.5 Preparation of **82-193**

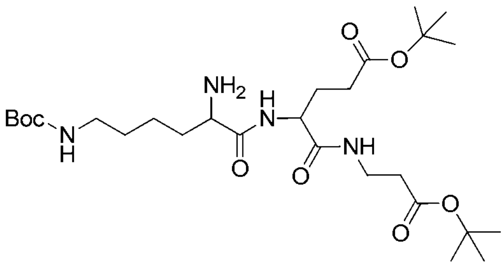

To a flask containing **82-189** (1.2886 mmol), DMF was added for dissolution with the assistance of ultrasonication, then morpholine (6.14 mL, 70.510 mmol) was added, and the mixture was reacted under stirring at room temperature for 2 h. After the reaction was completed, saturated NaCl solution (200 mL) and ethyl acetate (200 mL) were added for extraction. After standing for layer separation, the organic phase was collected, and the aqueous phase was further extracted with ethyl acetate (200 mL × 3). The organic phases were combined, evaporated to dryness, and then dissolved in a 1/4 methanol/dichloromethane mixture. Silica gel powder (100 mL) was added, and the mixture was evaporated to dryness, resulting in a powdery solid. The solid was loaded by dry method, and subjected to column chromatography using 6-10% methanol/dichloromethane as the eluent. The desired product was collected, concentrated, and dried in a vacuum oven to provide 2.5 g of the product with a yield of 63.45%.

26.6 Preparation of **82-197**

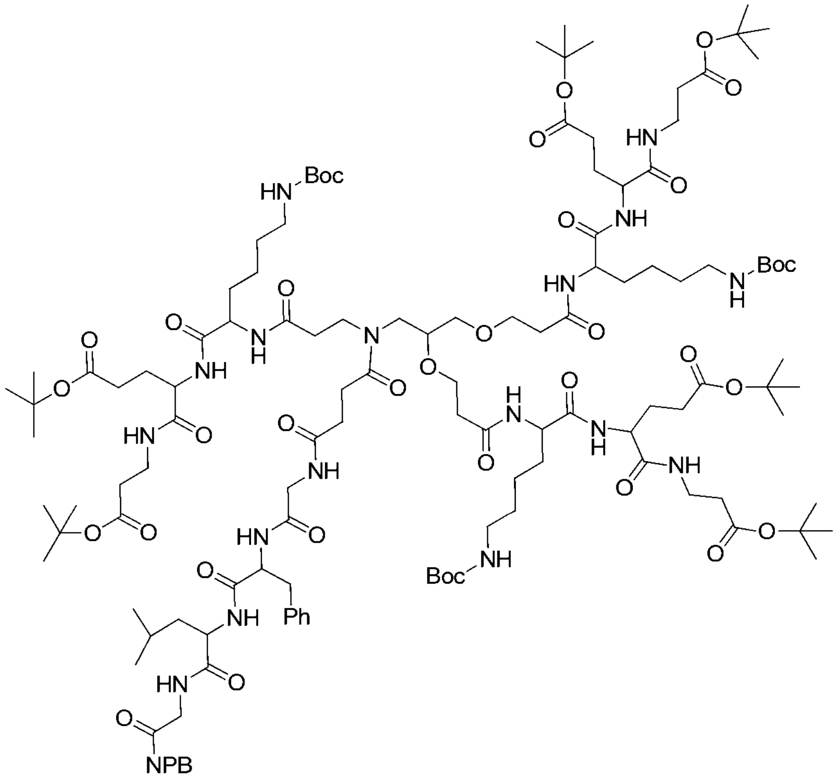

**82-196** (1.3572 mmol), HBTU (1.70 g, 4.4788 mmol) and HOBT (0.61 g, 4.4788 mmol) were weighed and added into a flask containing **82-193** (2.50 g, 4.4788 mmol), then an appropriate amount of DMF was added to dissolve the mixture, and the contents were allowed to stir at -5°C. DIEA (1.48 mL, 8.9575 mmol) was slowly added dropwise. After the dropwise addition was completed, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried to provide 2.31 g of the product. $^1$H-NMR (600 MHz, DMSO-$d_6$) δ 8.58 (s, 1H), 8.16-7.78 (m, 16H), 7.61-7.51 (m, 2H), 7.34-7.21 (m, 6H), 7.20-7.12 (m, 1H), 6.73 (s, 2H), 4.60-4.50 (m, 1H), 4.49-4.41 (m, 1H), 4.40-4.32 (m, 1H), 4.29-4.13 (m, 5H), 4.10-3.91 (m, 2H), 3.90-3.82 (m, 1H), 3.76-3.49 (m, 8H), 3.31-3.25 (m, 5H), 3.24-3.12 (m, 4H), 3.11-3.01 (m, 2H), 2.94-2.79 (m, 7H), 2.73-2.59 (m, 4H), 2.55-2.51 (m, 3H), 2.43-2.26 (m, 13H), 2.22-2.10 (m, 6H), 2.02-1.94 (m, 1H), 1.90-1.75 (m, 4H), 1.73-1.65 (m, 3H), 1.64-1.43 (m, 9H), 1.39-1.34 (m, 81H), 1.27-1.10 (m, 12H), 0.94-0.80 (m, 6H); ESI [M+Na$^+$] 2727.597

26.7 Preparation of **82-200**

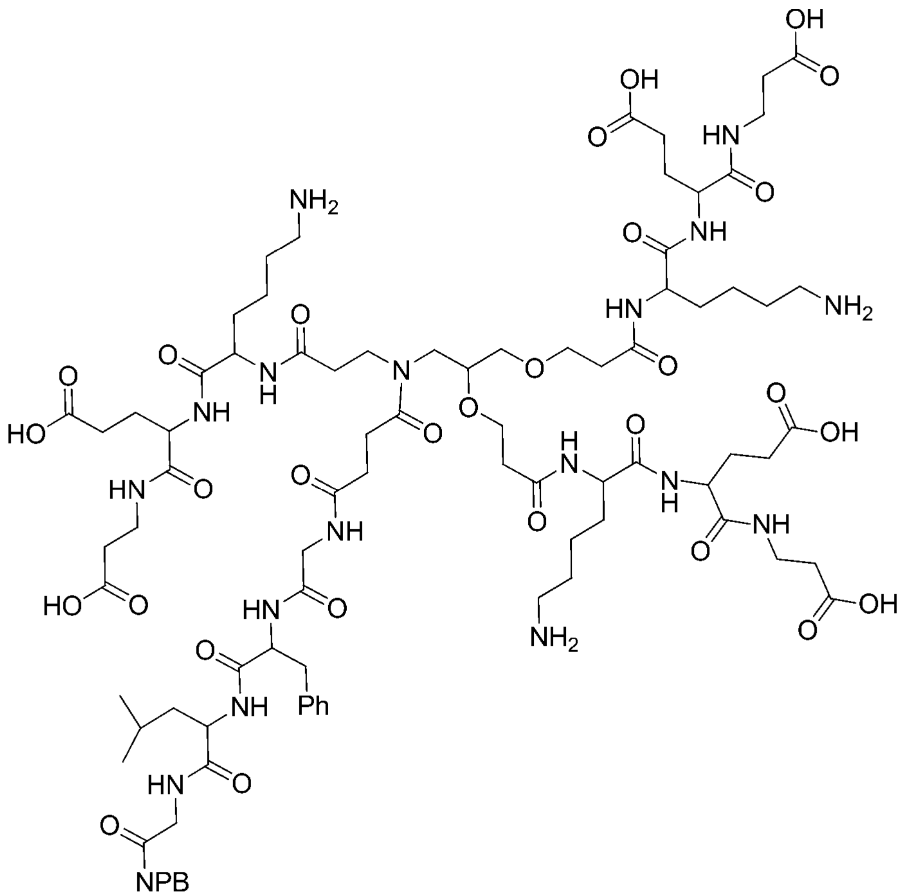

To a flask containing **82-197** (1 g, 0.3696 mmol), dichloromethane was added for dissolution with the assistance of ultrasonication, then TFA (2.19 mL, 29.5612 mmol) was added, and the mixture was reacted under stirring at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with methyl tert-butyl ether (40 mL × 3). The product had poor solubility. The filter cake was collected and dried in a vacuum oven to provide 0.96 g of the product.

26.8 Preparation of **82-202**

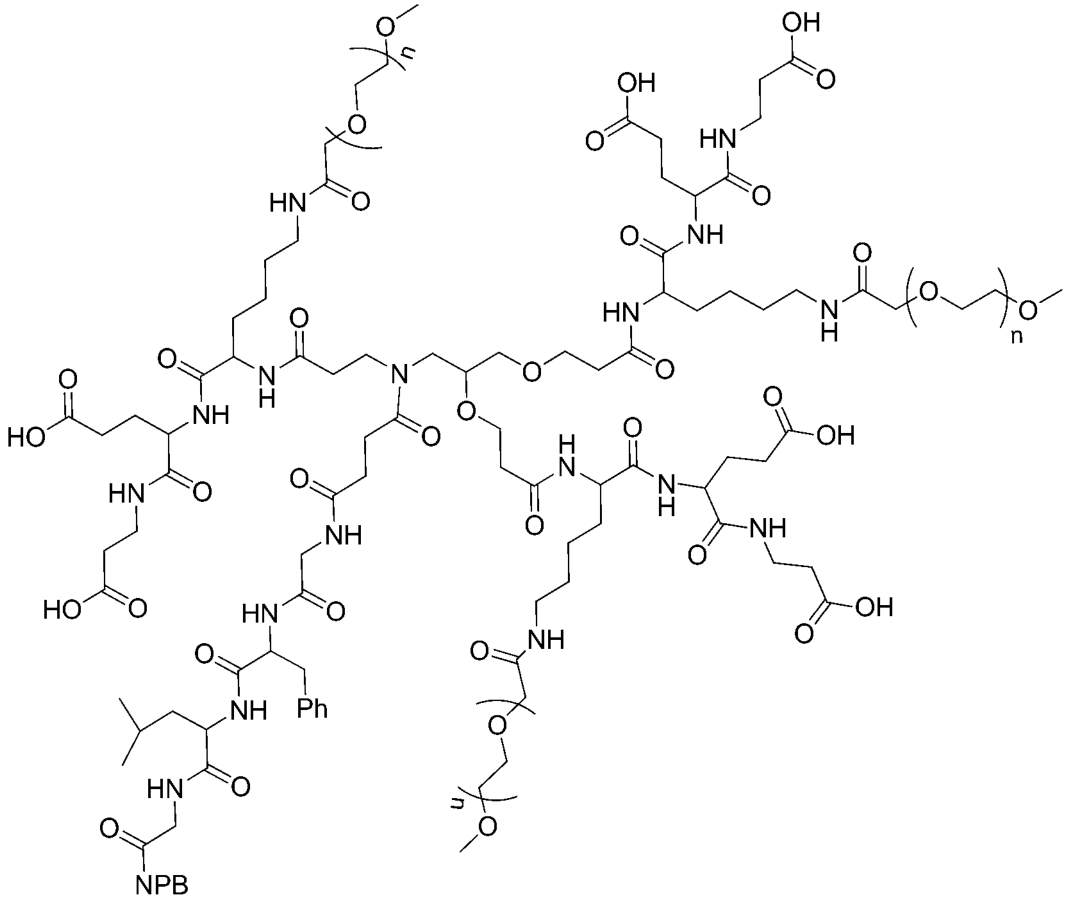

**82-200** (0.22 g, 0.1050 mmol) was placed in a 250 mL flask, DMF (20 mL) was added for dissolution, followed by a slow and dropwise addition of a DMF solution containing DIEA (1.24 mL, 7.524 mmol) and M-SCM-10K (3.46 g, 0.3254 mmol, purchased from Jenkem), and reacted under stirring in the dark at low speed at room temperature for one week. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dried in a vacuum oven to provide 3.5 g of the product.

26.9 Preparation of **82-181**

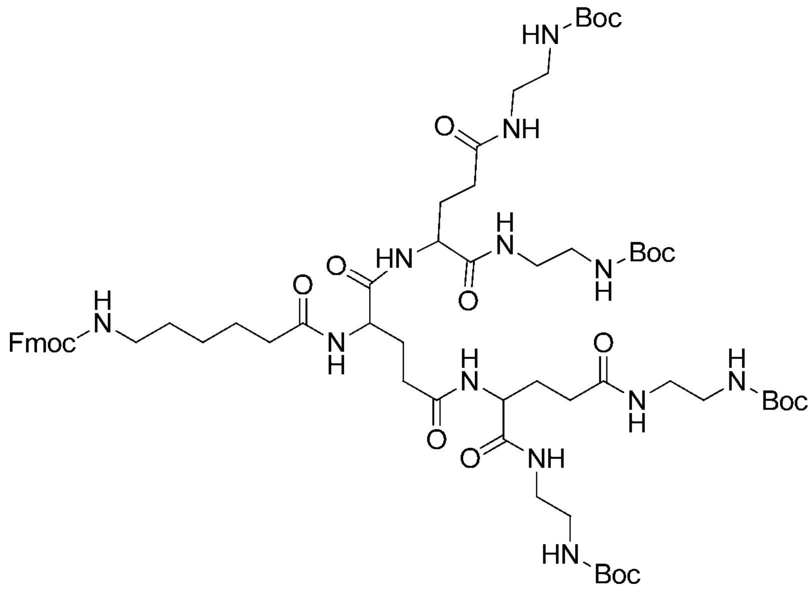

**70-199** (3.32 g, 9.4014 mmol), HBTU (3 g, 7.9277 mmol) and HOBT (1.07 g, 7.9277 mmol) were weighed and added into a flask containing Boc-ethylenediamine (9.4014 mmol), then an appropriate amount of DMF was added to dissolve the mixture, and allowed to stir at -5°C. DIEA (3.93 mL, 22.7830 mmol) was slowly added dropwise. After the dropwise addition was completed, the reaction was carried out for half an hour, the flask was then taken out, and the reaction was carried out under stirring at room temperature overnight. After the reaction was completed, saturated NaCl solution (200 mL) and ethyl acetate (200 mL) were added for extraction. After standing for layer separation, the organic phase was collected, and the aqueous phase was further extracted with ethyl acetate (3 × 200 mL). The organic phases were combined, evaporated to dryness, concentrated, and dried in a vacuum oven to provide 3.6 g of the product.

26.10 Preparation of **82-204**

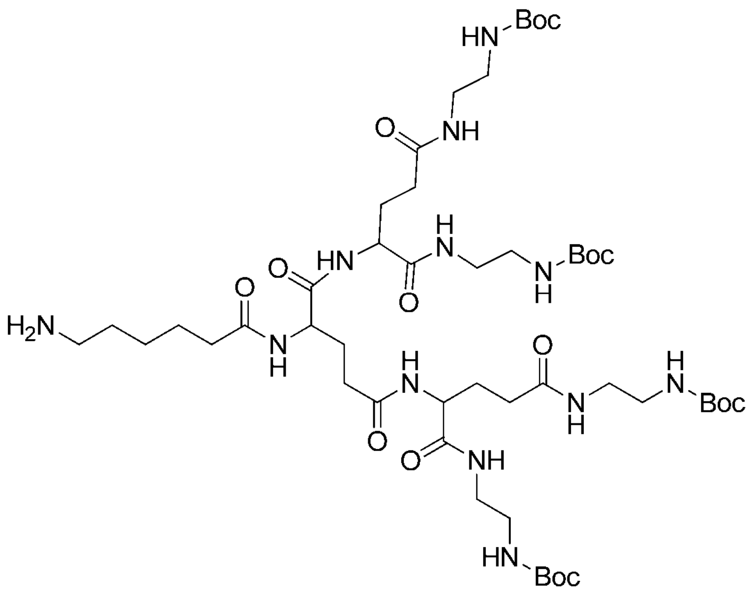

To a flask containing **82-181** (3.6 g), DMF (50 mL) was added for complete dissolution with the assistance of ultrasonication, then morpholine (10 mL) was added, and the mixture was reacted under stirring at room temperature for 2 h. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to obtain a solid product, which was then dried in a vacuum oven to

provide the product.

26.11 Preparation of **82-205**

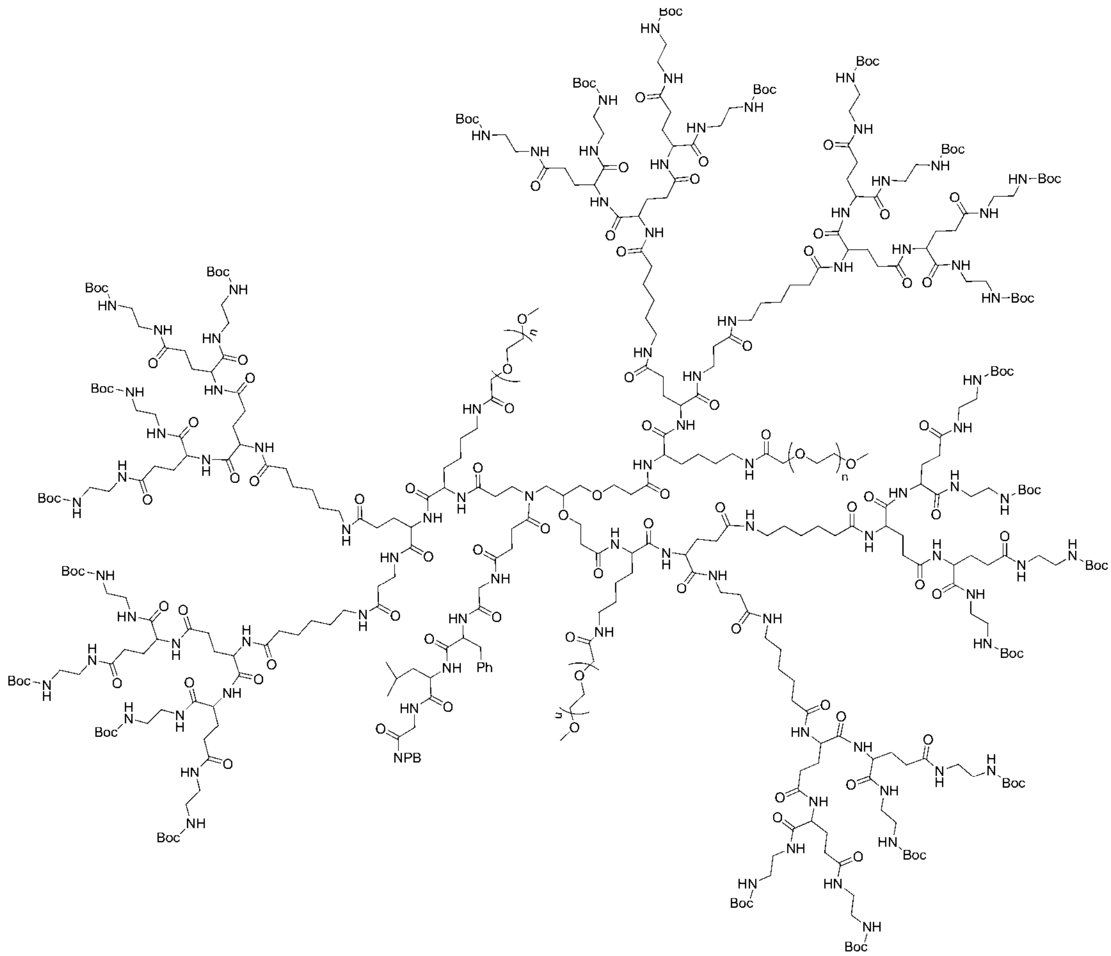

**82-204** (1.03 g, 0.945 mmol), HBTU (0.63 g, 1.6752 mmol) and HOBT (0.23 g, 1.6752 mmol) were weighed and added into a flask containing **82-202** (0.1050 mmol), then an appropriate amount of DMF was added to dissolve the mixture, and the contents were allowed to stir at -5°C. DIEA (0.30 mL, 1.8040 mmol) was slowly added dropwise. After the dropwise addition was completed, the reaction was carried out for half an hour, the flask was then taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected by suction, dissolved in a methanol/dichloromethane (1/4) mixture, then silica gel powder (100 mL) was added, and the mixture was evaporated to dryness, resulting in a powdery solid. The solid was loaded by dry method, and subjected to column chromatography using 6-10% methanol/dichloromethane as the eluent. The desired product was collected, concentrated, and dried in a vacuum oven to provide 1.53 g of the product.

26.12 Preparation of **82-219**

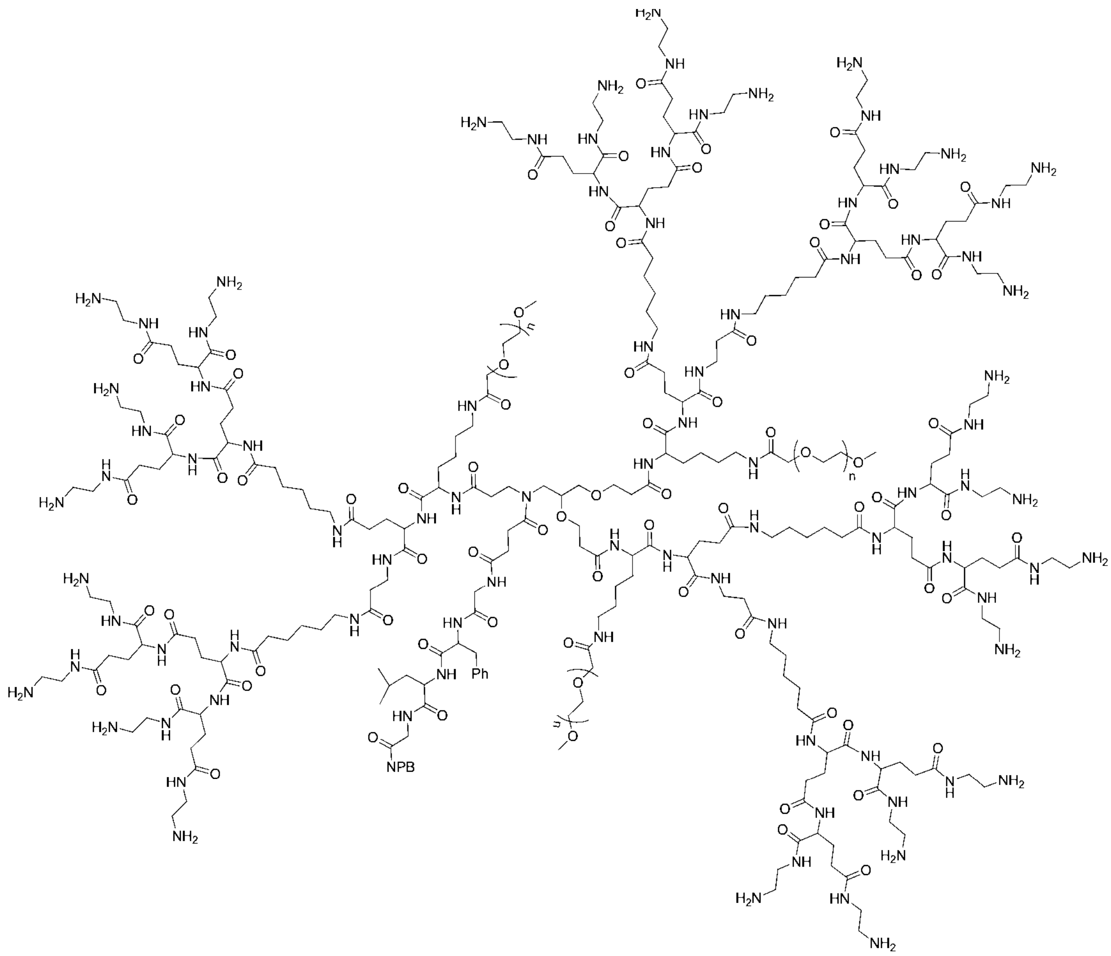

To a flask containing **82-205** (1.53 g, 0.0380 mmol), dichloromethane was added for complete dissolution with the assistance of ultrasonication, then TFA (10 mL) was added, and the mixture was reacted under stirring at room temperature overnight. After the reaction was completed, the reaction mixture was evaporated to provide an oil using a rotary evaporator. Methyl tert-butyl ether (60 mL) was then added. A powdery solid precipitated from the reaction mixture and was filtered. The filter cake was washed with MTBE (40 mL × 3). The product had poor solubility. The filter cake was collected and dried in a vacuum oven to provide the product.

26.13 Preparation of **82-220**

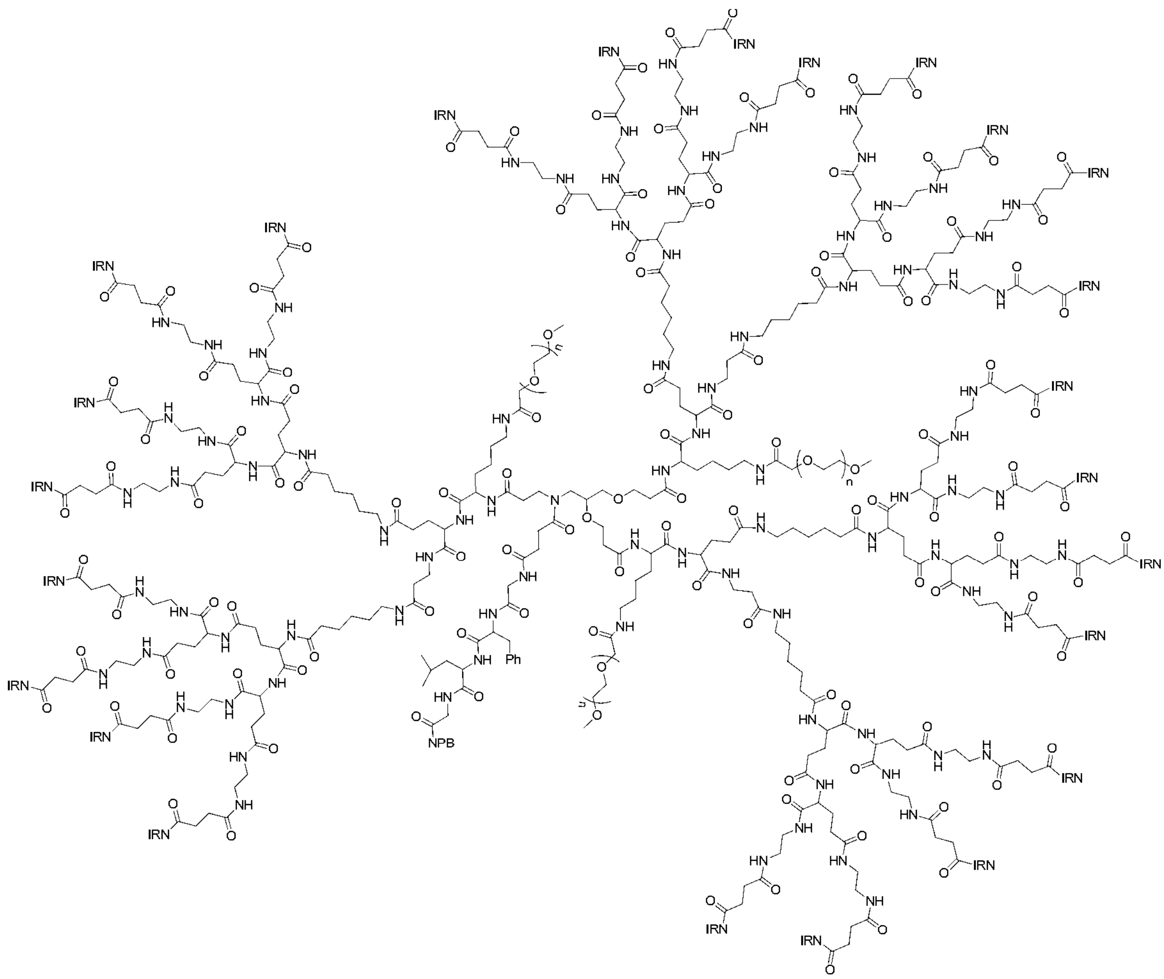

**82-219** (0.0269 mmol) was weighed and added into a flask containing **84-97** (1.0 g, 1.6752 mmol), then an appropriate amount of DMF was added to dissolve the mixture, and the contents were allowed to stand at -5°C. DIEA (0.30 mL, 1.8040 mmol) was slowly added dropwise. After the dropwise addition was completed, the reaction was carried out for half an hour, the flask was taken out, and the reaction was carried out under stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected and dissolved in methanol/dichloromethane (1/4) solution, then silica gel powder (100 mL) was added, and the mixture was evaporated to dryness to provide a powdery solid. The solid was loaded by dry method, and subjected to column chromatography using 6-10% methanol/dichloromethane as the eluent. The desired product was collected, concentrated, and dried in a vacuum oven to provide 0.9 g of the product. $^1$H-NMR (600 MHz, DMSO-$d_6$) $\delta$ 8.28-7.96 (m, 88H), 7.95-7.86 (m, 48H), 7.84-7.65 (m, 19H), 7.56-7.49 (m, 21H), 7.26-7.21 (m, 6H), 7.14-6.98 (m, 21H), 5.61-5.40 (m, 48H), 5.39-5.11 (m, 45H), 4.69-4.40 (m, 13H), 4.37-4.22 (m, 36H), 4.18-4.02 (m, 66H), 3.94-3.75 (m, 74H), 3.73-3.60 (m, 172H), 3.56-3.48 (m, 3594H), 3.19-3.00 (m, 142H), 2.95-2.84 (m, 23H), 2.81-2.69 (m, 64H), 2.46-2.33 (m, 43H), 2.17-2.04 (m, 74H), 1.93-1.79 (m, 44H), 1.66-1.52 (m, 103H), 1.47-1.36 (m, 59H), 1.25-1.17 (m, 72H), 0.97-0.76 (m, 76H)

Biological Testing

Experimental Example 1: Test of cytotoxicity of compounds

I. Materials:

1. Drugs: **70-261, 75-236, 77-162, 87-42, 82-220, 81-214, 72-248, 72-279, 84-102**
2. Cells: A549 cells, MDA-MB-231 cells, BT-474 cells

3. Equipment: PerkinElmer Multi-Mode Microplate Reader (PerkinElmer)

4. Reagents: CCK8 Cell Proliferation and Cytotoxicity Assay Kit (Dojindo); DMEM medium, fetal bovine serum, antibiotics (GIBCO)

II. Experimental method for testing growth inhibition of A549, MDA-MB-231, and BT474 cells at different drug concentrations

Cells were cultured and digested with trypsin. The cells were dissociated into single-cell suspensions using the corresponding culture media, seeded into a 96-well plate at 100 μL per well with a cell density of approximately 10-15%, and cultured overnight in a 37°C, 5% $CO_2$ incubator.

According to the drug concentration requirements, drug-containing culture media with different concentrations were prepared by serial dilution method.

The corresponding drugs for A549 cells were as follows:

| Plate layout | | | | | | | | | | | | |
|---|---|---|---|---|---|---|---|---|---|---|---|---|
| **70-261 [DOX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **75-236 [SN38] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **77-162 [PTX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **87-42 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

(continued)

| Plate layout | | | | | | | | | | | | |
|---|---|---|---|---|---|---|---|---|---|---|---|---|
| **82-220 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **84-102 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

The corresponding drugs for MDA-MB-231 cells were as follows:

| Plate layout | | | | | | | | | | | | |
|---|---|---|---|---|---|---|---|---|---|---|---|---|
| **70-261 [DOX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **75-236 [SN38] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **77-162 [PTX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

(continued)

| Plate layout | | | | | | | | | | | | |
|---|---|---|---|---|---|---|---|---|---|---|---|---|
| **87-42 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **82-220 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **84-102 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

The corresponding drugs for BT474 cells were as follows:

| Plate layout | | | | | | | | | | | | |
|---|---|---|---|---|---|---|---|---|---|---|---|---|
| **81-214 [LPT] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **72-248 [LPT] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

(continued)

| Plate layout | | | | | | | | | | | | |
|---|---|---|---|---|---|---|---|---|---|---|---|---|
| **72-279 [LPT] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

Drug gradient: 100 μL of fresh culture medium containing the corresponding drug prepared in step 2 were added respectively (the prepared drug concentration was 2 times that in the table above, the total volume after addition was 200 μL, and the final drug concentrations were the value in the table above), and the cells were cultured for 72 hours.
After 72 hours of drug treatment, the original culture medium was discarded.
100 μL of culture medium (containing 10% CCK8) was added to each well, and continuously incubated in the cell culture incubator for 1-2 hours.
The absorbance values at 450nm were measured.

III. Experimental results

(1) The inhibitory effects of treatment with different drug concentrations on the growth of A549 cells were tested. The experimental results are as follows.

Figure 1 shows the cell growth inhibitory effect of **70-261** at different concentrations after treatment for 72 hours on A549 cells, indicating that **70-261** was highly toxic, with an $IC_{50}$ value far below 0.02 μM.
Figure 2 and the table below show the cell growth inhibitory effect of **84-102** at different concentrations after treatment for 72 hours on A549 cells ($IC_{50}$ values are in μM).

| **84-102** | $IC_{50}$ |
|---|---|
| 2-fold dilution | 21.55 |
| 3-fold dilution | 17.61 |

Figure 3 shows the cell growth inhibitory effect of **77-162** at different concentrations after treatment for 72 hours on A549 cells, indicating that **77-162** was highly toxic, with an $IC_{50}$ value far below 0.02 μM.
Figure 4 and the table below show the cell growth inhibitory effect of **82-220** at different concentrations after treatment for 72 hours on A549 cells ($IC_{50}$ values are in μM).

| **82-220** | $IC_{50}$ |
|---|---|
| 2-fold dilution | 27.84 |
| 3-fold dilution | 25.87 |

Figure 5 and the table below show the cell growth inhibitory effect of **87-42** at different concentrations after treatment for 72 hours on A549 cells ($IC_{50}$ values are in μM).

| **87-42** | $IC_{50}$ |
|---|---|
| 2-fold dilution | 11.63 |
| 3-fold dilution | 12.09 |

Figure 6 and the table below show the cell growth inhibitory effect of **75-236** at different concentrations on A549 cells after treatment for 72 hours ($IC_{50}$ values are in μM).

| **75-236** | $IC_{50}$ |
|---|---|
| 2-fold dilution | 3.625 |
| 3-fold dilution | 4.064 |

(2) The inhibitory effects of treatment with different drug concentrations on the growth of MDA-MB-231 cells were tested, and the experimental results are as follows.

Figure 7 shows the cell growth inhibitory effect of **70-261** at different concentrations on MDA-MB-231 cells after treatment for 72 hours, indicating that **70-261** was highly toxic, with an $IC_{50}$ far below 0.02 μM.

Figure 8 and the table below show the cell growth inhibitory effect of **84-102** at different concentrations on MDA-MB-231 cells after treatment for 72 hours ($IC_{50}$ values are in μM).

| **84-102** | $IC_{50}$ |
|---|---|
| 2-fold dilution | 57.01 |
| 3-fold dilution | 55.23 |

Figure 9 shows the cell growth inhibitory effect of **77-162** at different concentrations on MDA-MB-231 cells after treatment for 72 hours, indicating that **77-162** was highly toxic, with an $IC_{50}$ far below 0.02 μM.

Figure 10 and the table below show the cell growth inhibitory effect of **82-220** at different concentrations on MDA-MB-231 cells after 72 hours of treatment ($IC_{50}$ values are in μM).

| **82-220** | $IC_{50}$ |
|---|---|
| 2-fold dilution | 45.57 |
| 3-fold dilution | 43.51 |

Figure 11 and the table below show the cell growth inhibitory effect of **87-42** at different concentrations on MDA-MB-231 cells after 72 hours of treatment ($IC_{50}$ values are in μM).

| **87-42** | $IC_{50}$ |
|---|---|
| 2-fold dilution | 24.95 |
| 3-fold dilution | 27.27 |

Figure 12 and the table below show the cell growth inhibitory effect of **75-236** at different concentrations on MDA-MB-231 cells after 72 hours of treatment ($IC_{50}$ values are in μM).

| **75-236** | $IC_{50}$ |
|---|---|
| 2-fold dilution | 14 |
| 3-fold dilution | 14.84 |

(3) The inhibitory effects of treatment with different drug concentrations on the growth of BT-474 cells was tested, and the experimental results are as follows.

Figure 13 and the table below show the cell growth inhibitory effect of **81-214** at different concentrations on BT-474 cells after 72 hours of treatment ($IC_{50}$ values are in μM).

| **81-214** | $IC_{50}$ |
|---|---|
| 2-fold dilution | 108.6 |

(continued)

| 81-214 | $IC_{50}$ |
|---|---|
| 3-fold dilution | 105.9 |

Figure 14 and the table below show the cell growth inhibitory effect of **72-248** at different concentrations on BT-474 cells after 72 hours of treatment ($IC_{50}$ values are in μM).

| 72-248 | $IC_{50}$ |
|---|---|
| 2-fold dilution | 3.959 |
| 3-fold dilution | 3.759 |

Figure 15 and the table below show the cell growth inhibitory effects of **72-279** at different concentrations on BT-474 cells after treatment for 72 hours ($IC_{50}$ values are in μM).

| 72-279 | $IC_{50}$ |
|---|---|
| 2-fold dilution | 158.2 |
| 3-fold dilution | 169.8 |

Experimental Example 2: Experiment on BT-474 animal tumor model

Purpose: To establish a subcutaneous xenograft tumor model of human breast cancer BT474 cells in NPG mice and investigate the antitumor effects of the example compounds on subcutaneous xenograft tumors.

Method: BT474 cells were resuscitated and subjected to cell passage and amplification. When sufficient cell number was reached, the cells in the logarithmic growth phase were harvested for cell inoculation. Before inoculation, a sustained-release estrogen tablet (17β-ESTRADIOL, lot number: SE-121, Innovative Research of America) was implanted subcutaneously in the nape of NPG mice. A BT474 cell suspension at a concentration of $8.4 \times 10^7$ cells/mL was mixed with matrigel (Matrigel Basement Membrance Matrix, CORNING) in a ratio of 1:1 to obtain a cell suspension with a concentration of $4.2 \times 10^7$ cells/mL. 0.2 mL of this suspension was inoculated into the right mammary pad of the mouse. Tumor growth was observed after inoculation, and 30 tumor-bearing animals with tumor volumes ranging from 132.04 to 233.06 $mm^3$ were selected for the experiment. Based on tumor volume and body weight, the animals were randomly divided into five groups: negative control group (sodium chloride injection), group **81-214** (95.4 mg/kg), group **81-231** (94.7 mg/kg), group **76-179** (139.9 mg/kg), and group **74-232** (60.4 mg/kg), with six animals in each group. The animals were administered intravenously at 10 mL/kg every three days for eight doses. The animals were euthanized on Day 29. During the experimental period, the animals were observed for general clinical symptoms twice daily, and their body weight and tumor diameter were measured twice weekly. Tumors were removed and weighed after euthanasia. Tumor volume, relative tumor volume (RTV), relative tumor growth rate (T/C%), and tumor weight inhibition rate (IRTW%) were calculated. Efficacy was defined as relative tumor growth rate (T/C%) ≤ 40% in the treatment group and $P \leq 0.05$ compared to the RTV of the negative control group. $IR_{TW} \geq 60\%$ was used as a secondary reference indicator for efficacy.

Results: During the entire experiment, one animal in the negative control group died on Day 15, weighing 19.3 g. The cause of death was presumed to be related to the excessive tumor burden. The body weight of animals in all groups showed a gradual increase, and no significant differences were observed between groups at any time point (P>0.05).

Tumors in the negative control group gradually grew throughout the experiment. By the end of the experiment (D29), the average tumor volume in group 1 was 2518.14±984.66 $mm^3$, and the average RTV was 13.78±5.44. The average tumor volumes in groups **81-214, 81-231, 76-179,** and **74-232** were 1306.83±613.48 $mm^3$, 1266.29±278.48 $mm^3$, 378.59±186.19 $mm^3$, and 2284.75±536.18 $mm^3$, respectively, and the average RTV values were 7.25±2.80, 7.12 ±1.14, 2.04±0.79, and 12.58±2.73, respectively. Both the average tumor volume and RTV decreased in all groups after drug administration. In group **76-179,** the mean tumor volume from Day 4 to Day 18 and RTV from Day 4 to Day 15 were significantly lower than those of the negative control group (P ≤ 0.05).

The tumor growth trends of each group are shown in Figure 16.

By the end of the experiment (D29), the T/C% values of the groups **81-214, 81-231, 76-179,** and **74-232** were 52.62%, 51.70%, 14.84%, and 91.33%, respectively, and their $IR_{TV}$% values were 47.38%, 48.30%, 85.16%, and 8.67%, respectively. The T/C% of the group **76-179** dropped below 40% from Day 8 to Day 29, and the RTV values from Day 8 to 15 were significantly lower than those of Group 1 (P ≤ 0.05).

At the end of the experiment, animals were euthanized and tumor weights were measured. The average tumor weights of the negative control, groups **81-214, 81-231, 76-179,** and **74-232** were 1.785±0.837 g, 1.061±0.506 g, 0.918 ±0.234 g, 0.247±0.101 g, and 1.616±0.494 g, respectively. The $IR_{TW}$% values were 40.57%, 48.54%, 86.19%, and 9.48%, respectively. The $IR_{TW}$% for group **76-179** was >60%.

Figure 17 shows a schematic diagram of the tumor weight inhibition rates for each group.

Conclusion: Under the conditions of this experiment, the test compounds **76-179** at a dose of 139.9 mg/kg, **81-214** at a dose of 95.4 mg/kg, and **81-231** at a dose of 94.7 mg/kg, administered once every three days via tail vein injection, significantly inhibited the growth of subcutaneous xenografts of human breast cancer BT474 cells in NPG mice. **76-179** exhibited the strongest inhibitory effect, while **74-232** exhibited the weakest.

Experimental Example 3: Experiments in A-549 human lung cancer and MDA-MB-231 human breast cancer animal tumor models

Purpose:

To investigate the effects of a series of polyethylene glycol small-molecule dual-drug (or triple-drug) compounds on subcutaneous mouse tumor models of lung cancer (A-549) and breast cancer (MDA-MB-231).

Methods:

Efficacy study on nude mouse xenograft model (subcutaneous tumor) of A-549 lung cancer cells: Quarantine-passed BALB/c-nude mice were inoculated subcutaneously on the right side of their backs with A-549 cells at a final concentration of $5\times10^7$ cells/ml in a volume of 0.1 mL per mouse. When the average tumor volume reached approximately 100 $mm^3$, the mice were randomly divided into the following groups: model control group, group **77-162,** group **82-220,** and group **74-232.** Drugs were administered via tail vein injection. The model control group received normal saline, while the groups **77-162, 82-220,** and **74-232** received doses of 60.1, 152.7, and 60.4 mg/kg, respectively, at an administration frequency of every three days for a total of four doses. The animals were observed daily, and body weight and tumor volume were measured every three days. Relative tumor volume and proliferation rate were calculated. The appearance of the animals and the tumors were photographed when sampling at the end of study. The tumor weights were measured, and tumor inhibition rates were calculated.

Efficacy study on nude mouse xenograft model (subcutaneous tumor) of MDA-Mb-231 breast cancer cells: Quarantine-passed BALB/c nude mice were inoculated subcutaneously on the right side of their backs with MDA-MB-231 cells at a final concentration of $5\times10^7$ cells/ml in a volume of 0.1 mL per mouse. When the average tumor volume reached approximately 100 $mm^3$, the mice were randomly divided into the following groups: model control group, group **77-162,** group **82-220,** group **75-209,** and group **87-47.** Drugs were administered via tail vein injection. The model control group received normal saline, while the groups **77-162, 82-220, 75-209,** and **87-47** received 60.1, 152.7, 62.2, and 39.7 mg/kg, respectively, at an administration frequency of every three days for a total of four doses. The animals were observed daily, and body weight and tumor volume were measured every three days. The relative tumor volume and proliferation rate were calculated. The appearance of the animals and the tumors were photographed when sampling at the end of study. The tumor weights were measured, and the tumor inhibition rate was calculated.

Results:

1. Efficacy study on nude mouse xenograft model (subcutaneous tumor) of lung cancer A-549 cells

(1) General observation: From inoculation to administration, all animals were in good mental and physical condition. The tumors grew normally, and the animals have normal excretion and diet. After administration, no obvious abnormalities were observed in the model control group, group **77-162,** group **82-220** and group **74-232.**

(2) Body weight (Figure 18): No abnormal changes were observed in the body weight of the animals of group **77-162, 82-220** and **74-232,** indicating that the test substances had no significant effect on the body weight of the animals.

(3) Tumor volume, relative tumor volume and relative tumor proliferation rate (Figure 19): Compared with the model control group, the final tumor volumes of group **77-162,** group **82-220** and group **74-232** after administration were significantly reduced. Compared with the model control group, the final relative tumor volume of group **77-162** was significantly reduced, and the relative tumor proliferation rates from Day 4 to Day 28 were 99.92%, 65.35%, 80.84%, 82.58%, 51.08%, 50.38%, 47.42%, 46.75% and 40.61% respectively; the final relative tumor volume of group **82-220** was significantly reduced, and the relative tumor proliferation rates from Day 4 to

Day 28 were 85.18%, 83. 81%, 90.40%, 35.54%, 22.91%, 22.07%, 16.02%, 14.03%, and 12.09%, respectively; the final relative tumor volume of group **74-232** was significantly reduced, and the relative tumor proliferation rates from Day 4 to Day 28 were 94.65%, 86.64%, 94.28%, 115.71%, 93.96%, 106.60%, 100.99%, 91.56%, and 71.37%, respectively.

(4) Tumor weight and tumor inhibition rate (Figure 20): Compared with the model control group, the tumor weights of mice in the groups **77-162, 82-220,** and **74-232** were significantly reduced, and the tumor inhibition rates were 60.03%, 88.41%, and 40.52%, respectively, all greater than 40%.

2. Efficacy study on nude mouse xenograft tumor model (subcutaneous tumor) of breast cancer MDA-MB-231 cells

(1) General observation: From inoculation to administration, all animals were in good mental and general condition, with normal tumor growth, animal excretion and diet. After administration, no obvious abnormalities were observed in the model control group, group **77-162,** group **82-220,** group **75-209** and group **87-47.**

(2) Body weight (Figure 21): After administration, no abnormal changes were observed in the body weights of group **77-162,** group **82-220,** group **75-209** and group **87-47,** indicating that the test substances had no significant effect on the body weight of the animals.

(3) Tumor volume, relative tumor volume and relative tumor growth rate (Figure 22): Compared with the model control group, the final tumor volumes of group **77-162,** group **82-220,** group **75-209** and group **87-47** after administration was significantly reduced. Compared with the model control group, the final relative tumor volume of group **77-162** was significantly reduced, and the relative tumor proliferation rates from Day 4 to Day 22 were 74.40%, 62.28%, 58.08%, 43.02%, 43.01%, 36.35%, and 34.52%, respectively; the final relative tumor volume of group **82-220** was significantly reduced, and the relative tumor proliferation rates from Day 4 to Day 22 were 73.96%, 74.33%, 56.68%, 32.23%, 27.08%, 28.05%, 28.70%, respectively; the final relative tumor volume of group **75-209** was significantly reduced, and the relative tumor proliferation rates from Day 4 to Day 22 were 66.81%, 76.80%, 76.94%, 43.97%, 29.78%, 28.03%, and 31.43%, respectively; the final relative tumor volume of group **87-47** was significantly reduced, and the relative tumor proliferation rates from Day 4 to Day 22 were 59.36%, 39.59%, 16.85%, 3.85%, 2.20%, 1.45%, and 0.73%, respectively.

(4) Tumor weight and tumor inhibition rate (Figure 23): Compared with the model control group, the tumor weight of mice in group **77-162,** group **82-220,** group **75-209,** and group **87-47** was significantly reduced, and the tumor inhibition rates were 56.60%, 72.82%, 68.17%, and 98.88%, respectively, all greater than 40%.

Conclusions:

1. Efficacy study on nude mouse xenograft tumor model (subcutaneous tumors) of A-549 lung cancer cells

Under the conditions of this experiment, a subcutaneous lung cancer (A-549) mouse model was established, and administered with **77-162, 82-220,** and **74-232** via tail vein injection once every three days for a total of four doses. The results showed that **77-162, 82-220,** and **74-232** significantly reduced the final tumor volume, relative tumor volume, relative tumor growth rate, and tumor weight after administration, with tumor inhibition rates of > 40%, suggesting that **77-162, 82-220,** and **74-232** are promising for the treatment of lung cancer.

2. Efficacy study on nude mouse xenograft tumor model (subcutaneous tumor) of MDA-MB-231 breast cancer cells

Under the condition of this experiment, a subcutaneous breast cancer (MDA-MB-231) mouse model was established, and administrated with **77-162, 82-220, 75-209,** and **87-47** via tail vein injection once every three days for a total of four doses. The results showed that **77-162, 82-220, 75-209,** and **87-47** significantly reduced tumor volume, relative tumor volume, relative tumor growth rate, and tumor weight, with tumor inhibition rates >40%, suggesting that **77-162, 82-220, 75-209,** and **87-47** are promising for the treatment of breast cancer.

Experimental Example 4: BNCT experiments on B16 melanoma model, SAS human oral squamous cell carcinoma model, and MG-63 human osteosarcoma model

① Preparation and treatment of mice of B16 melanoma model, SAS human oral squamous cell carcinoma model and MG-63 human osteosarcoma model

All C57BL6/j (female, 4-week-old) mice were acclimated to breeding conditions for 7 days (14-hour/10-hour light and dark cycle, with light of 200 Lx in daytime between 5:00 AM and 7:00 PM, temperature of 23.5±2.5°C, and humidity of 52.5±12.5%). Afterwards, the dorsal hair of the right hind leg of the mice was shaved. On the next day, under isoflurane

anesthesia (2-2.5% in ambient air), the mice were inoculated subcutaneously in the right thigh with B16-F10 cells, SAS human oral squamous cell carcinoma cells, and MG-63 human osteosarcoma cells ($10^7$ cells/mL; 50 μL/mouse). Tumors were allowed to grow for 7 days, and then randomly divided into control group and experimental groups. Tumor volume (V) was estimated using the following formula:

$$V = a \times b^2/2$$

where a and b are the lengths of long and short axes of tumor, respectively, as determined by a vernier caliper. On the day of irradiation (day 0), the average tumor volume for all mice was approximately 80 $mm^3$, which was considered as the initial tumor volume ($V_0$). All subcutaneous injections were performed on the dorsal side of the neck using a syringe with a 27-gauge needle. All animal experiments were conducted in accordance with the submitted animal experiment plan and were reviewed and approved by the Animal Ethics Committee.

② Boron neutron capture therapy (BNCT) studies in B16 melanoma model, SAS human oral squamous cell carcinoma model, and MG-63 human osteosarcoma model

The mice of B16 melanoma model, SAS human oral squamous cell carcinoma model, and MG-63 human osteosarcoma model were respectively divided into 6 groups, with 6 mice in each group. Subcutaneous injections were respectively performed with normal saline and normal saline solutions of compounds **76-258, 88-206, 72-188, 86-43,** and **87-35** (equivalent dose: 2 mg $^{10}B$/kg): normal saline (2 hours), compounds **76-258, 88-206, 72-188, 86-43, 87-35** (24 hours), where the hours in parentheses represent the time interval between injection and irradiation. The mice were placed in customized acrylic holders by securing their tails and thighs with autoclaved tape. These holders were attached around the holes of the acrylic plate with Velcro. The acrylic plate was then fixed to a 5 mm thick thermoplastic plate, in which the thermoplastic plate contained 40 wt% $^6LiF$ ($^6Li$ enrichment: 96%), and provided neutron shielding. Both the acrylic plate and the thermoplastic plate had a round hole in the center, so that the mouse's right thigh could be stretched and exposed to these holes. Through these holes, the tumors were irradiated with neutrons for 60 minutes at an average rate of $3.44 \pm 0.22 \times 10^{12}$ neutrons/$cm^2$ (n = 6). After neutron irradiation, the mice were housed for three weeks, with tumor volume measured twice weekly and tumor growth curves plotted. After the third week, the mice were sacrificed, the tumor masses were removed and weighed, and the tumor weight inhibition rate was calculated.

③ Statistical analysis

Statistical analysis was performed using the Student's t-test in GraphPad Prism 8 software. A p-value of less than 0.05 was considered statistically significant. Unless otherwise stated, all results are presented as mean ± standard deviation.

④ Experimental conclusions

Compared with the normal saline group, both the tumor volume and tumor mass weight of the groups of compounds **76-258, 88-206, 72-188, 86-43,** and **87-35** were significantly reduced, demonstrating that these compounds had significant antitumor activity.

While specific embodiments of the present invention have been described in detail, those skilled in the art will appreciate that, in light of the teachings disclosed herein, various modifications and substitutions may be made to those details, and such changes are within the scope of the present invention. The full scope of the present invention is given by the appended claims and any equivalents thereof.

## Claims

**1.** A compound represented by formula I or a pharmaceutically acceptable salt thereof,

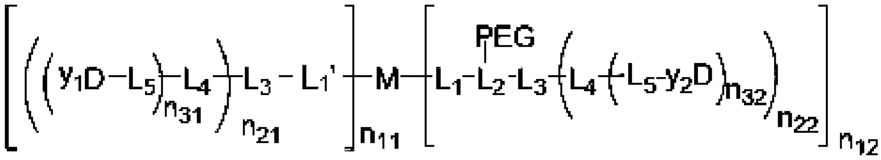

formula I

wherein,

M is a hydrocarbonyl containing two or more (e.g., 2, 3, 4, 5, or 6) identical or different heteroatoms (e.g., N, O, or S), and M is connected to $L_1$ and $L_1$' via the heteroatoms;

each $L_1$ is independently selected from the group consisting of

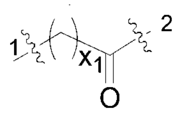

and

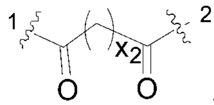,

wherein the terminus 1 is connected to M, and the terminus 2 is connected to $L_2$;

each $L_1$' is independently selected from the group consisting of

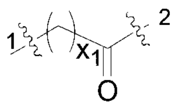

and

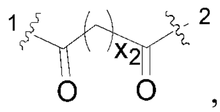,

wherein the terminus 1 is connected to M, and the terminus 2 is connected to $L_3$; and $L_1$ and $L_1$' are different;

$x_1$ and $x_2$, at each occurrence, are independently selected from the group consisting of 0, 1, 2, 3, 4, 5, 6, 7, 8, 9, and 10;

each $L_2$ is independently selected from the group consisting of residues of Lys, Cys, Thr, Ser, Asp, and Glu;

each PEG is independently selected from

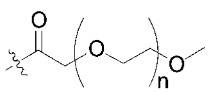, 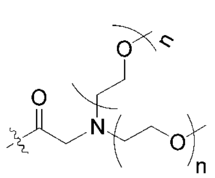

and

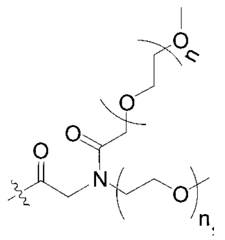

, each of which has a number-average molecular weight of 5k-10k, or 10k-40k, for example, 5k or 10k;

each $L_3$ is independently a bond or

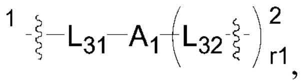

wherein the terminus 1 is connected to $L_2$ or $L_1$', and the terminus 2 is connected to $L_4$, wherein each $L_{31}$ and $L_{32}$ is independently selected from the group consisting of a bond and $-NH(CH_2)_{x3}C(O)-$, $x_3$ is selected from the group consisting of 0, 1, 2, 3, 4, 5, 6, 7, 8, 9, and 10, $A_1$ is selected from the group consisting of a bond, an amino acid residue or a derivative thereof, or a polypeptide fragment consisting of two or more amino acids or a derivative thereof, and r1 is selected from the group consisting of 1, 2, 3, 4, 5, and 6;

each $L_4$ is independently a bond or

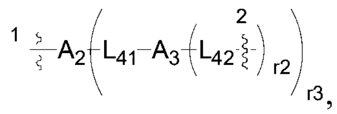

wherein the terminus 1 is connected to $L_3$ and the terminus 2 is connected to $L_5$, wherein each $L_{41}$ and $L_{42}$ is independently selected from the group consisting of a bond, $-NH(CH_2)_{x4}C(O)-$, $-NH(CH_2)_{x4}NH-$, $-NH((CH_2)_2O)_{x4}CH_2CH_2NH-$, and $-C(O)(CH_2)_{x4}C(O)-$; $A_2$ and $A_3$ are independently selected from the group consisting of a bond, an amino acid residue or a derivative thereof, or a polypeptide fragment consisting of two or more amino acids or a derivative thereof; r2 and r3 are each independently selected from the group consisting of 1, 2, 3, 4, 5, and 6;

each $L_5$ is independently a bond or selected from the group consisting of

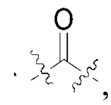

-NH-, hydrazino (i.e., -NH-N=), an amino acid residue or a derivative thereof, a polypeptide fragment consisting of two or more amino acids or a derivative thereof, $-NH(CH_2)_{x5}C(O)-$, $-NH(CH_2)_{x5}NH-$, $-NH((CH_2)_2O)_{x5}CH_2CH_2NH-$, $-NH((CH_2)_2O)_{x5}CO-$, $-C(O)(CH_2)_{x5}C(O)-$, and any combination thereof; or

$L_4$ and $L_5$ are connected to form

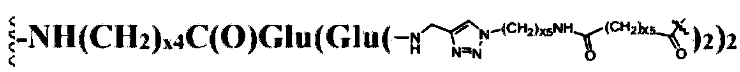

,

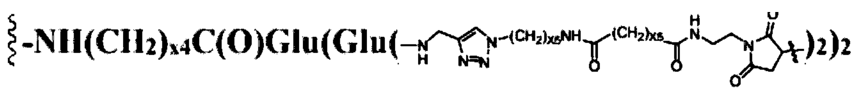

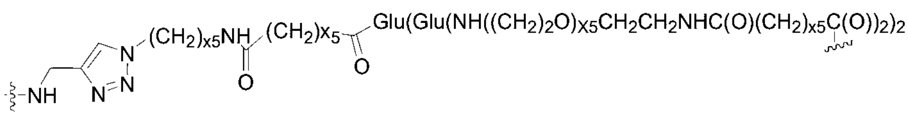

or

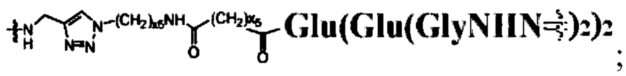

;

preferably, $L_4$ and $L_5$ are connected to form

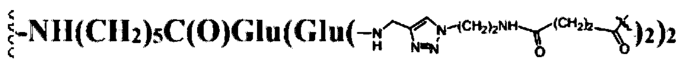

,

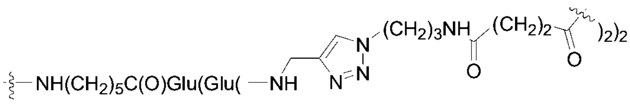

,

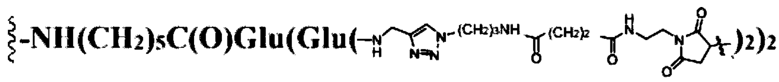

,

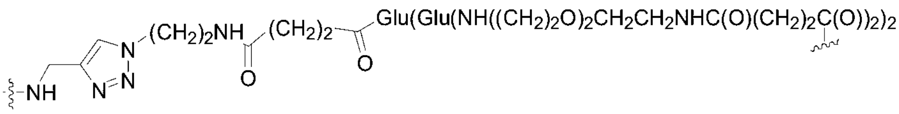

or

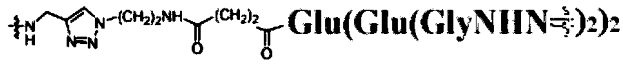

;

$x_4$ and $x_5$, at each occurrence, are independently selected from the group consisting of 0, 1, 2, 3, 4, 5, 6, 7, 8, 9, and 10;

each D is independently selected from the group consisting of a cytotoxic drug moiety, preferably, the cytotoxic drug is selected from the group consisting of tubulin inhibitors, DNA intercalators, DNA topoisomerase inhibitors, and RNA polymerase inhibitors; preferably, the cytotoxic drug is selected from the group consisting of PTX (paclitaxel), PCB (Palbociclib), SN38 (7-ethyl-10-hydroxy-camptothecin), NPB (Niraparib, MK-4827), AXT (Axitinib), LPT (lapatinib), DOX (doxorubicin), Ac-C-PLGLAG-iRGD, folic acid, SB7 (SB-743921), IRN (irinotecan), sodium dodecahydrododecaborate, PPT-iRGD, dodecaborate(2-), 1,2,3,4,5,6,7,8,9,10,11-undecahydro-12-mercapto-, sodium (1:2) (BSH, Sodium Mercaptododecaborate ($^{10}B$)),

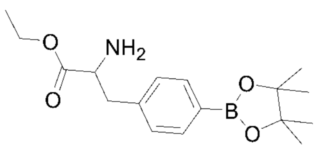

,

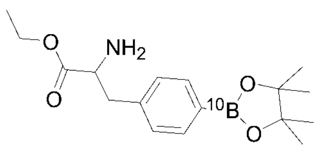

and

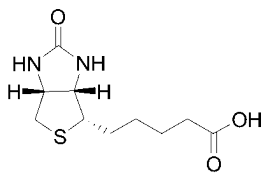

;

each $n_{11}$ and $n_{12}$ is independently selected from the group consisting of 1, 2, 3, 4, 5, 6, 7, 8, 9, and 10;
each $n_{21}$ and $n_{22}$ is independently selected from the group consisting of 1, 2, 3, 4, 5, 6, 7, 8, 9, and 10;
each $n_{31}$ and $n_{32}$ is independently selected from the group consisting of 1, 2, 3, 4, 5, 6, 7, 8, 9, and 10;
each $y_1$ and $y_2$ is independently selected from the group consisting of 1, 2, 3, 4, 5, 6, 7, 8, 9, and 10.

**2.** The compound or pharmaceutically acceptable salt thereof according to claim 1, wherein M is a $C_{2-10}$ hydrocarbonyl containing 2 to 4 identical or different heteroatoms;

preferably, M is a $C_{2-6}$ saturated hydrocarbonyl containing 2 to 4 heteroatoms independently selected from the group consisting of nitrogen and oxygen;
preferably, M is selected from the group consisting of the following structures:

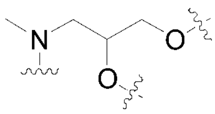 , 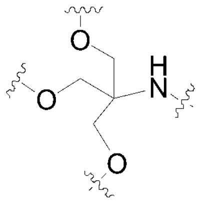 , 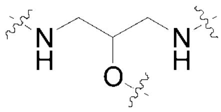 , 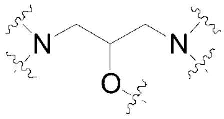 , 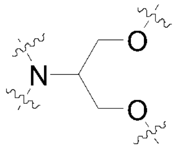 ,

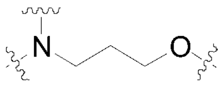 , 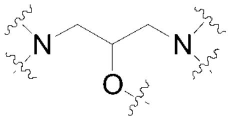 , 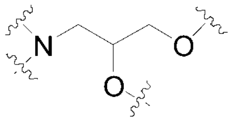 , 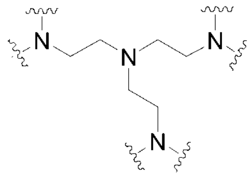

and

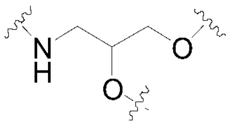 ;

preferably, M is connected to $L_1$' via a nitrogen atom, or M is connected to $L_1$' via an oxygen atom; further preferably, M is connected to $L_1$' via a nitrogen atom;
preferably, $n_{11}$ = 1, 2, or 3, and $n_{11} \leq n_{12}$; further preferably, $n_{11}$ = 1, $n_{12}$ = 2, 3, or 4; $n_{11}$ = 2, $n_{12}$ = 3; or $n_{11}$ = 3, $n_{12}$ = 3.

**3.** The compound or pharmaceutically acceptable salt thereof according to claim 1 or 2, wherein each $L_1$ and $L_1$' is independently selected from the group consisting of

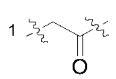 ,

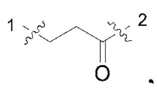 , and 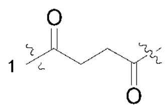 ;

preferably, $L_1$ is

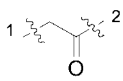 or 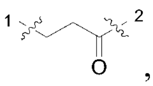 ,

and $L_1$' is

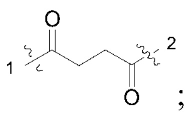 ;

preferably, $L_1$ is

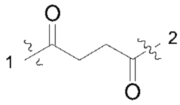
,

, and $L_1$' is

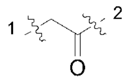
or
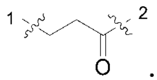
.

**4.** The compound or pharmaceutically acceptable salt thereof according to any one of claims 1 to 3, wherein each $L_2$ is independently a Lys residue.

**5.** The compound or pharmaceutically acceptable salt thereof according to any one of claims 1 to 4, wherein when $L_3$ is

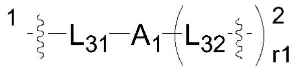
,

each $L_{31}$ and $L_{32}$ is independently selected from the group consisting of a bond and $-NH(CH_2)_{x3}C(O)-$, $x_3$ is selected from the group consisting of 0, 1, 2, 3, 4, 5, and 6, $A_1$ is selected from the group consisting of a bond, an amino acid residue or a derivative thereof, or a polypeptide fragment or a derivative thereof consisting of two or more amino acids, preferably $A_1$ is selected from the group consisting of Glu, Asp, and GluGlu, and r1 is selected from the group consisting of 1, 2, 3, 4, 5, and 6;
preferably, each $L_3$ is independently a bond or selected from the group consisting of the following structures: $-NH(CH_2)_2C(O)-$,

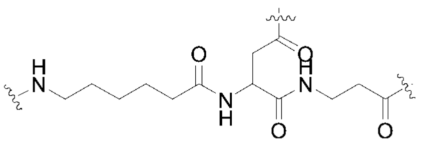
,
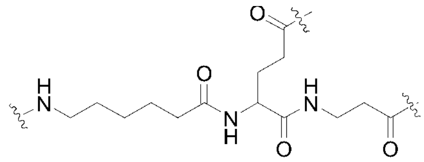
,

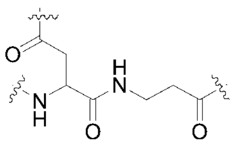
,
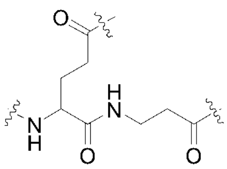
and
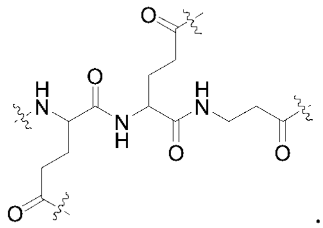
.

**6.** The compound or pharmaceutically acceptable salt thereof according to any one of claims 1 to 5, wherein when $L_4$ is

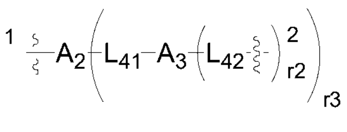
,

each $L_{41}$ and $L_{42}$ is independently selected from the group consisting of a bond, $-NH(CH_2)_{x4}C(O)-$, $-NH(CH_2)_{x4}NH-$, $-NH((CH_2)_2O)_{x4}CH_2CH_2NH-$ and $-C(O)(CH_2)_{x4}C(O)-$, each $x_4$ is independently selected from the group consisting of 0, 1, 2, 3, 4, 5 and 6, $A_2$ and $A_3$ are independently selected from the group consisting of a bond, an amino acid residue or a derivative thereof, or a polypeptide fragment or a derivative thereof consisting of two or more amino acids, preferably, each $A_2$ and $A_3$ is independently selected from the group consisting of Lys, Glu, Asp, GluGlu and $Glu(Glu)_2$, r1 is selected from the group consisting of 1, 2, 3, 4, 5, and 6;

preferably, each $L_4$ is independently a bond or selected from the group consisting of the following structures: $LysNH(CH_2)_5COGlu(Glu)_2$, $Lys(COC_2H_4CO)(NH(CH_2CH_2O)_2CH_2CH_2NH)$, $-NH(CH_2)_5COGlu$, $-NH(CH_2)_5CO-Glu(Glu)_2$, $-NH(CH_2)_5COGlu(Glu(NHCH_2CH_2NH)_2)_2$, $-NH(CH_2)_5COAsp$, and $-NH(CH_2)_5CO-Glu(Glu(NH(CH_2CH_2O)_2CH_2CH_2NH)_2)_2$.

**7.** The compound or pharmaceutically acceptable salt thereof according to any one of claims 1 to 6, wherein the amino acid in $L_5$ is selected from the group consisting of Glu, Gly, Phe, Leu, and Cys; preferably, the polypeptide composed of two or more amino acids is selected from the group consisting of GlyPheLeuGly and $Glu(Glu(Gly)_2)_2$; preferably, the derivative is selected from the group consisting of acylated (e.g., acetylated) or alkylated (e.g., methylated) derivatives;

preferably, $L_5$ is selected from the group consisting of a bond,

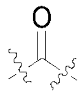,

-NH-N=, GlyPheLeuGly, $-NH(CH_2)_{x5}C(O)-$, $-NH((CH_2)_2O)_{x5}CH_2CH_2NH-$, $-NH((CH_2)_2O)_{x5}CH_2CH_2NHGlu$, $-NH(CH_2)_{x5}C(O)Glu(Glu(GlyNHN=)_2)_2$, $-NH((CH_2)_2O)_{x5}CO-$, $-C(O)(CH_2)_{x5}C(O)-$, $-C(O)(CH_2)_{x5}C(O)$-GlyPhe-LeuGly-, and

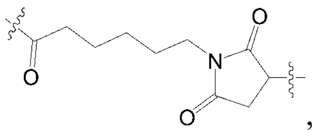,

each $x_5$ is independently selected from the group consisting of 1, 2, 3, 4, 5, and 6;
preferably, $L_5$ is selected from the group consisting of a bond,

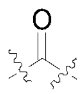,

GlyPheLeuGly, $-NH(CH_2)_5C(O)Glu(Glu(GlyNHN=)_2)_2$, $-NH(CH_2)_5C(O)-$, $-NH((CH_2)_2O)_2CH_2CH_2NH-$, $-NH((CH_2)_2O)_2CH_2CH_2NHGlu$, $-NH((CH_2)_2O)_2CO-$, $-C(O)(CH_2)_2C(O)-$, $-C(O)(CH_2)_2C(O)$-GlyPheLeuGly-, and

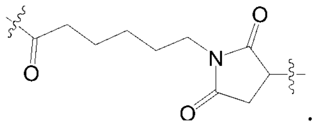.

**8.** The compound or pharmaceutically acceptable salt thereof according to any one of claims 1 to 7, wherein the compound is selected from the group consisting of:

| Comp d No. | Compound structure |
| --- | --- |
| **81-214** | 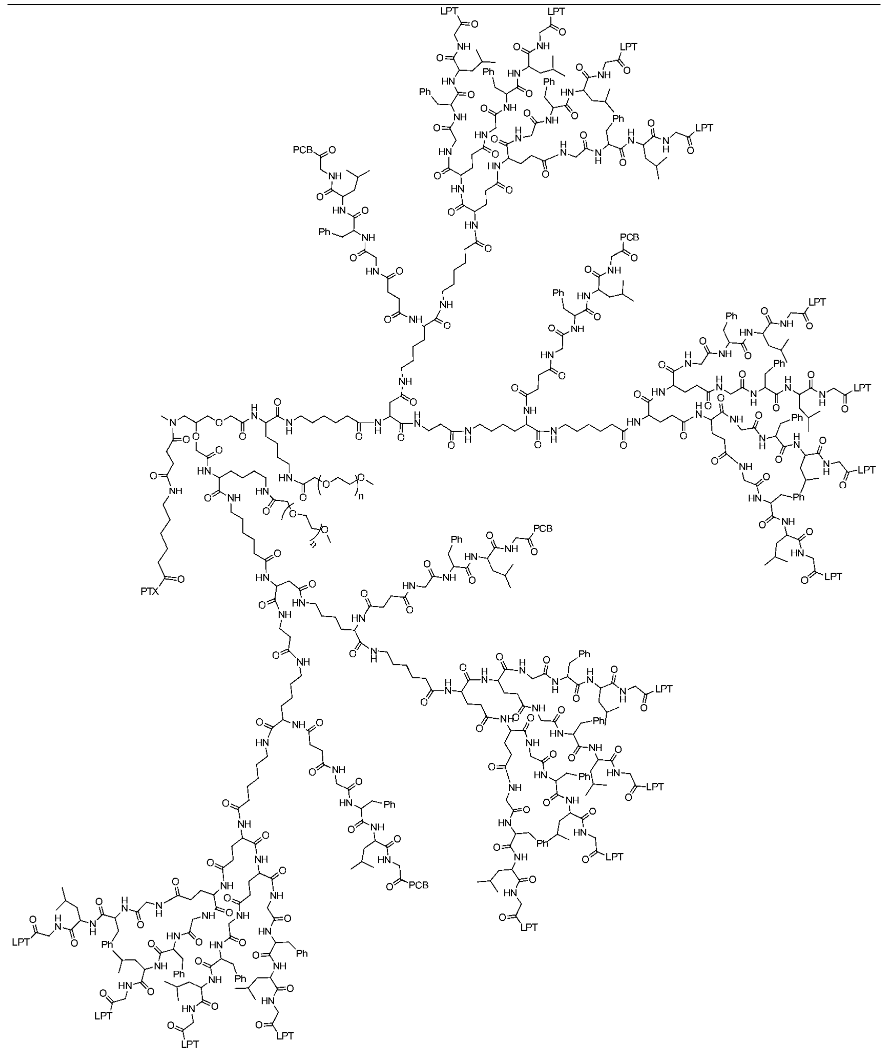 wherein, the number-average molecular weight of each polyethylene glycol fragment is 10k |

(continued)

| Compd No. | Compound structure |
| --- | --- |
| 76-258 | 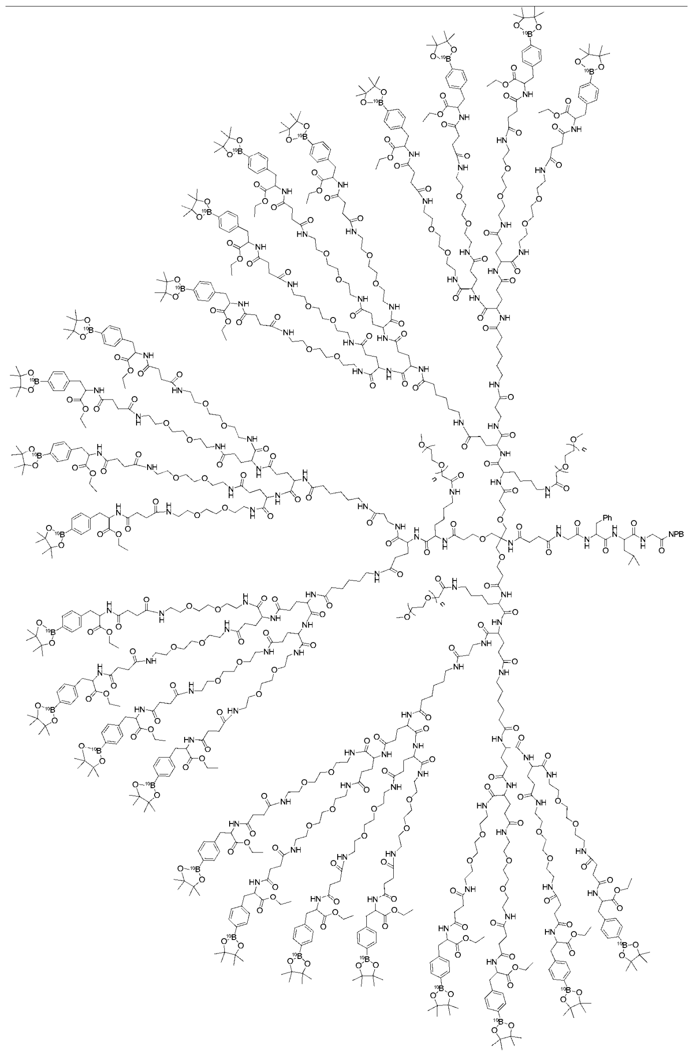 wherein, the number-average molecular weight of each polyethylene glycol fragment is 10k |

(continued)

| Comp d No. | Compound structure |
|---|---|
| **88-206** | 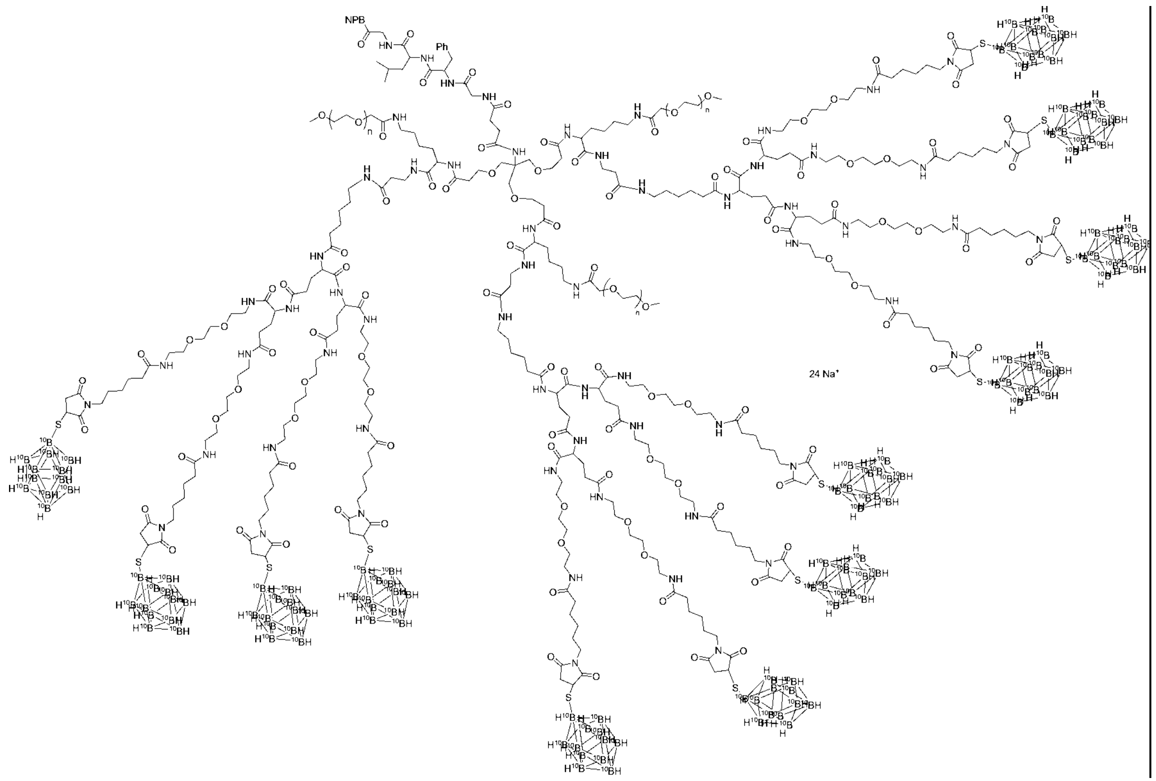<br>wherein, the number-average molecular weight of each polyethylene glycol fragment is 10k |

(continued)

| Comp d No. | Compound structure |
| --- | --- |
| **78-131** | 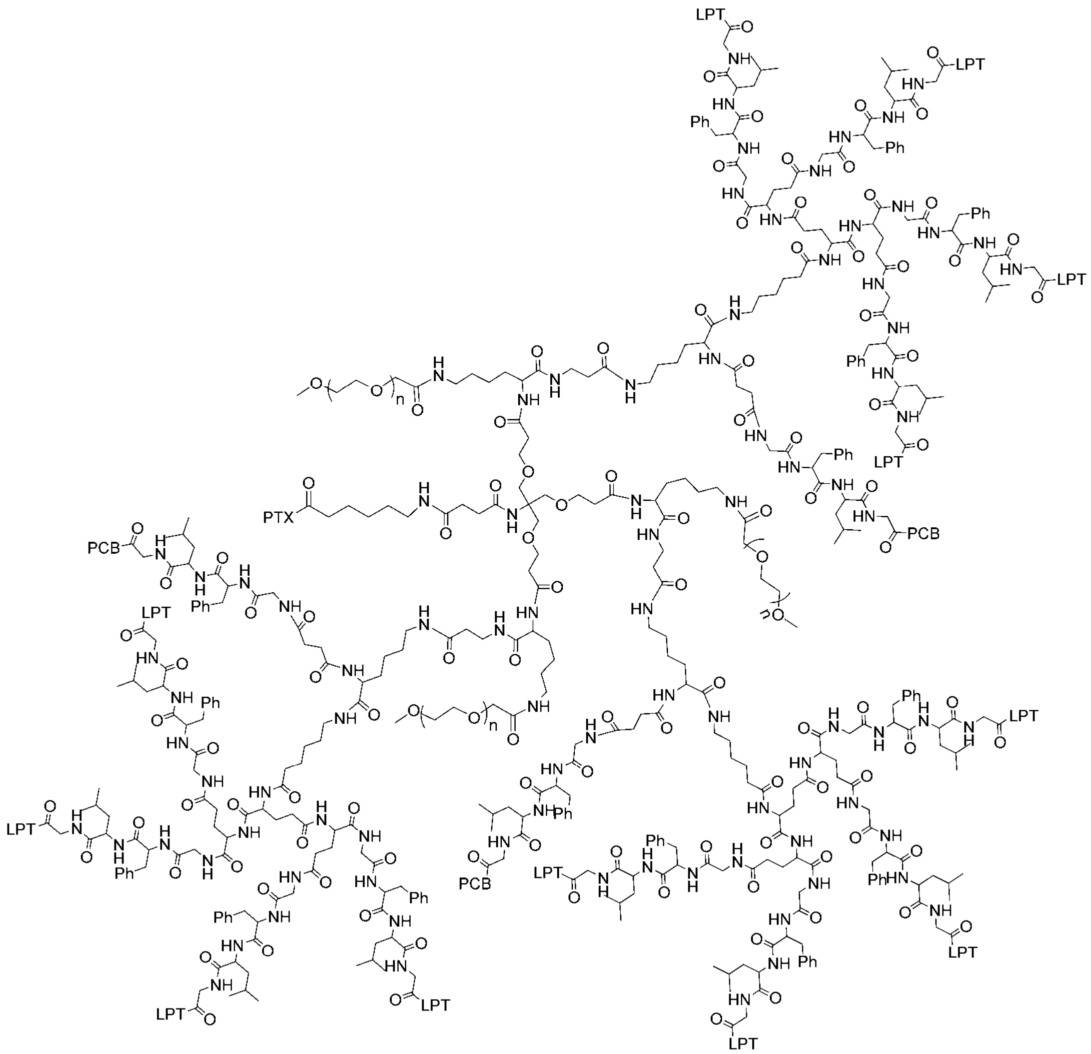 wherein, the number-average molecular weight of each polyethylene glycol fragment is 10k |

(continued)

| Comp d No. | Compound structure |
|---|---|
| **72-188** | 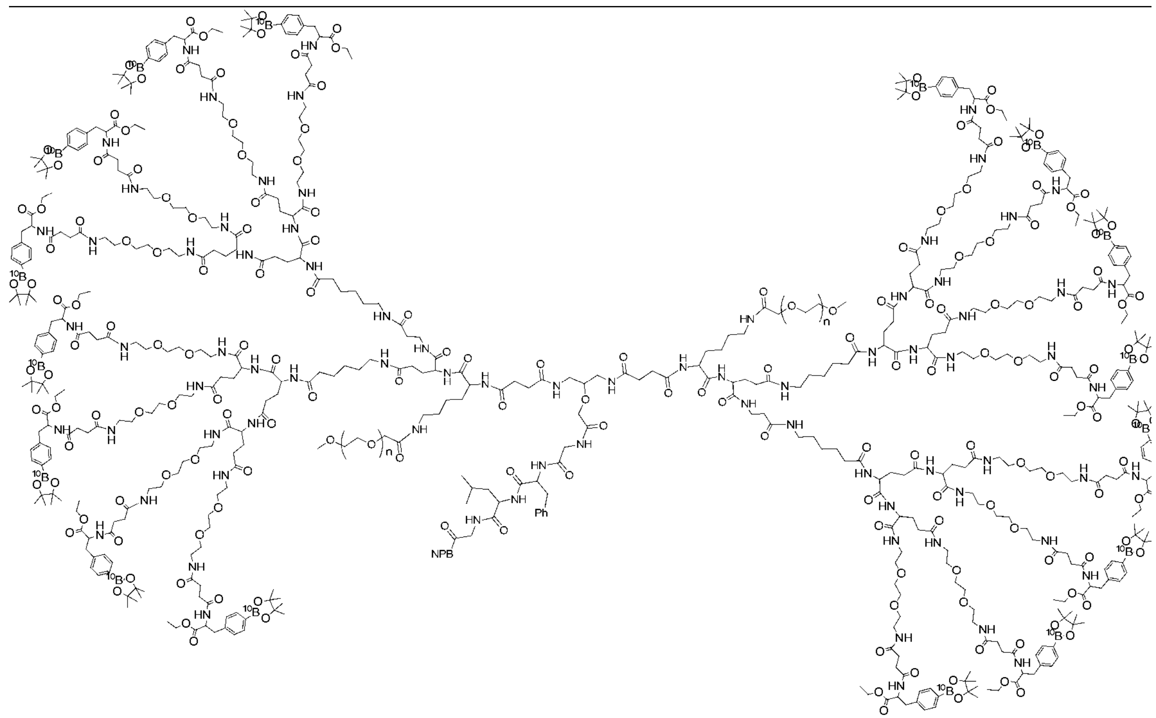 wherein, the number-average molecular weight of each polyethylene glycol fragment is 10k |

(continued)

| Comp d No. | Compound structure |
|---|---|
| **70-261** | 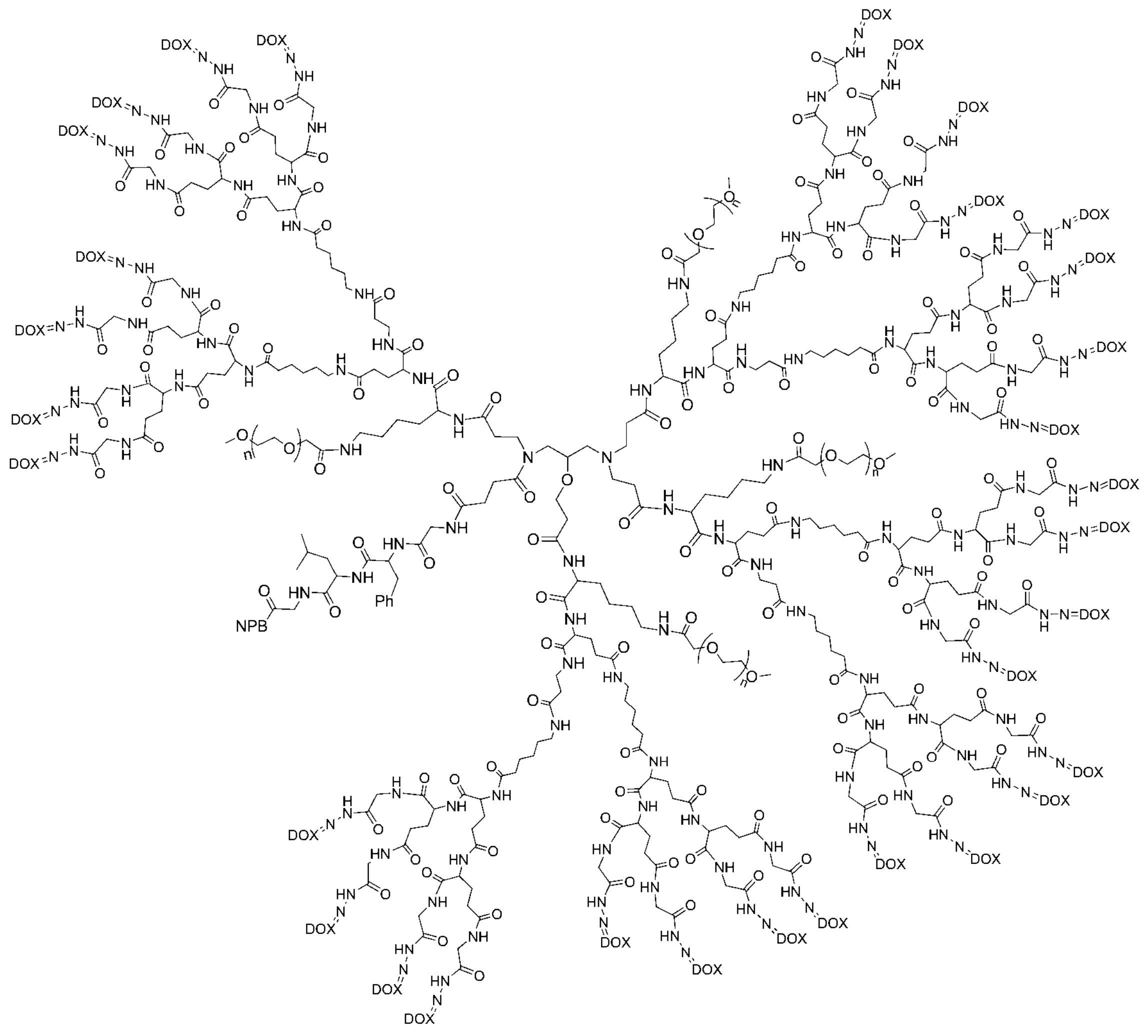<br>wherein, the number-average molecular weight of each polyethylene glycol fragment is 10k |

(continued)

| Comp d No. | Compound structure |
|---|---|
| **86-43** | 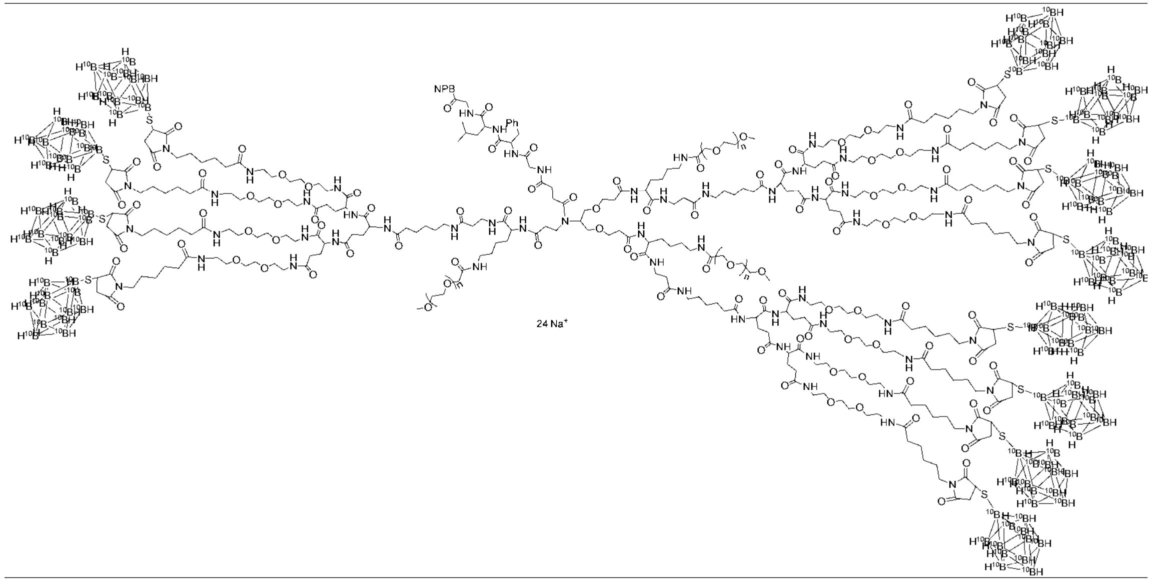<br>wherein, the number-average molecular weight of each polyethylene glycol fragment is 10k |

(continued)

| Comp d No. | Compound structure |
|---|---|
| **87-35** | 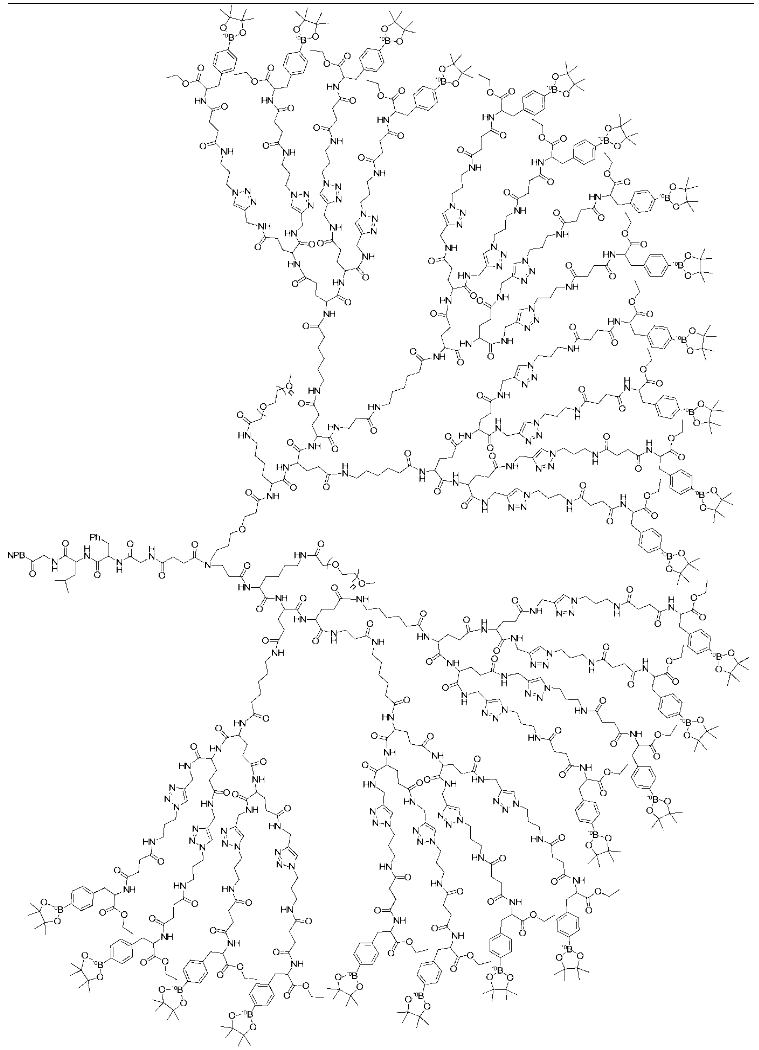 wherein, the number-average molecular weight of each polyethylene glycol fragment is 10k |

(continued)

| Comp d No. | Compound structure |
| --- | --- |
| 75-236 | 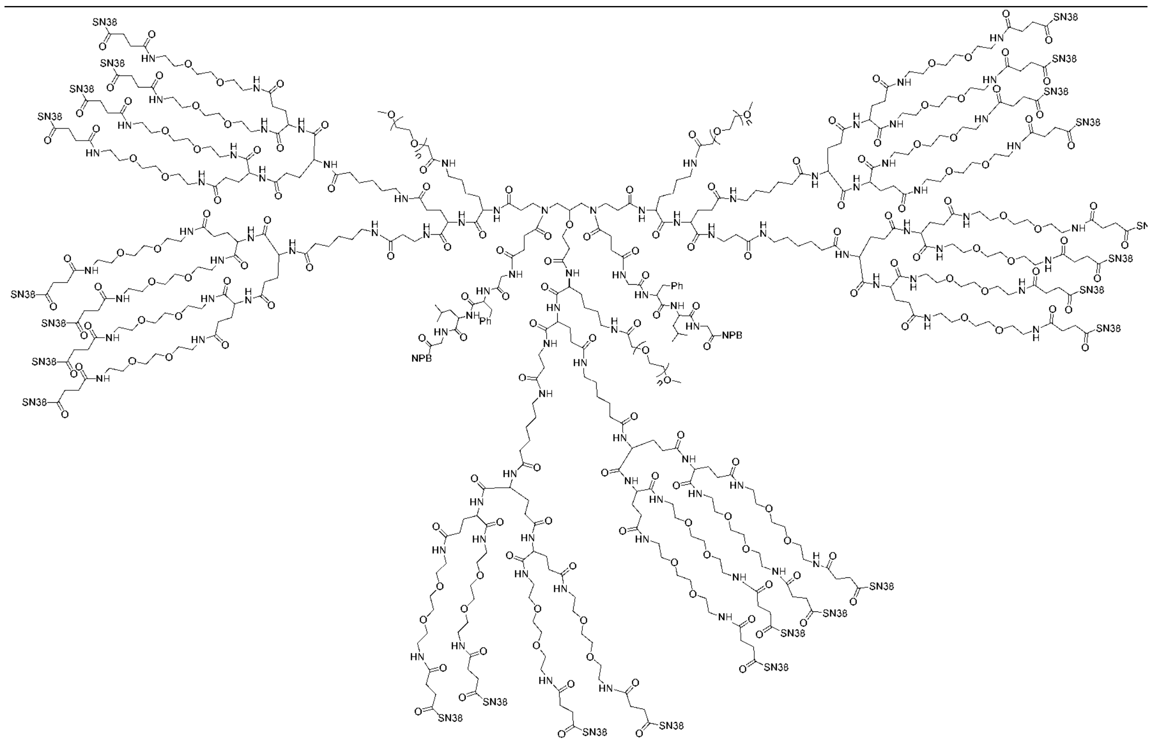 wherein, the number-average molecular weight of each polyethylene glycol fragment is 10k |

(continued)

| Comp d No. | Compound structure |
|---|---|
| **74-232** | 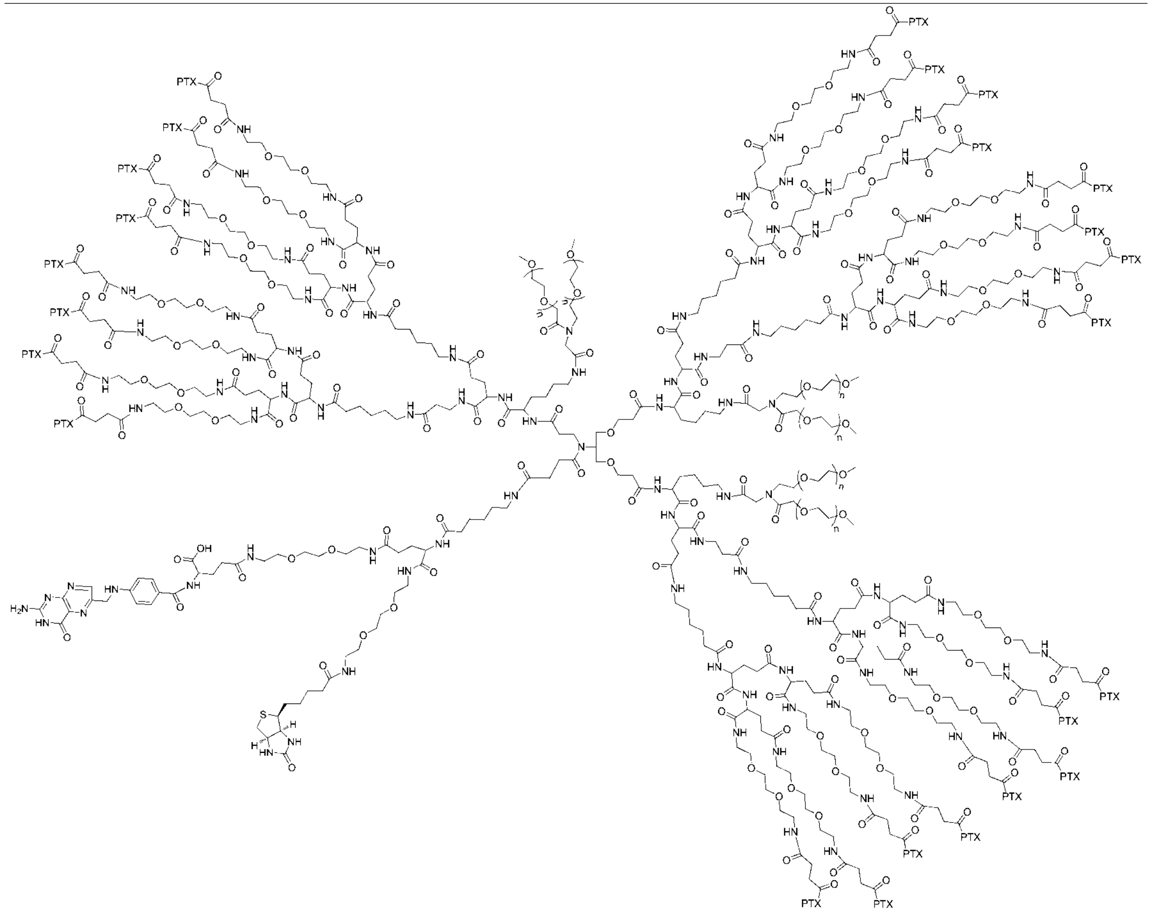<br>wherein, the number-average molecular weight of each polyethylene glycol fragment is 10k |

(continued)

| Comp d No. | Compound structure |
| --- | --- |
| **85-97** | 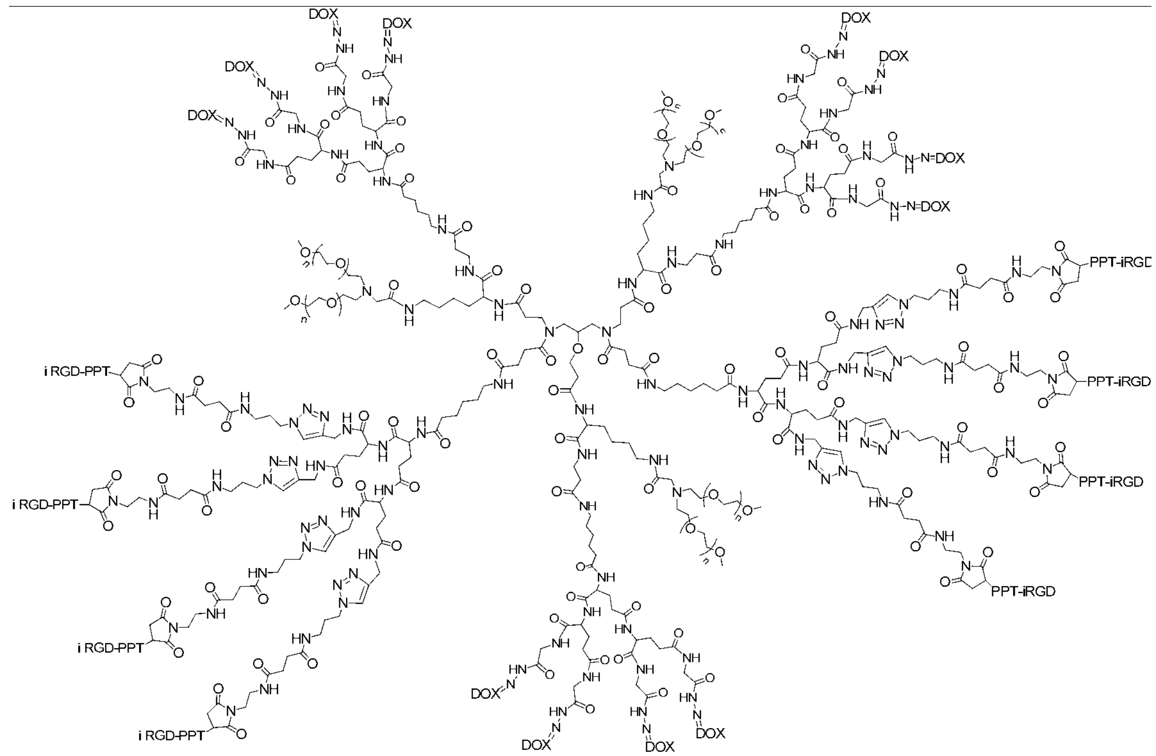 wherein, the number-average molecular weight of each polyethylene glycol fragment is 10k |

(continued)

| Comp d No. | Compound structure |
| --- | --- |
| **85-26** | 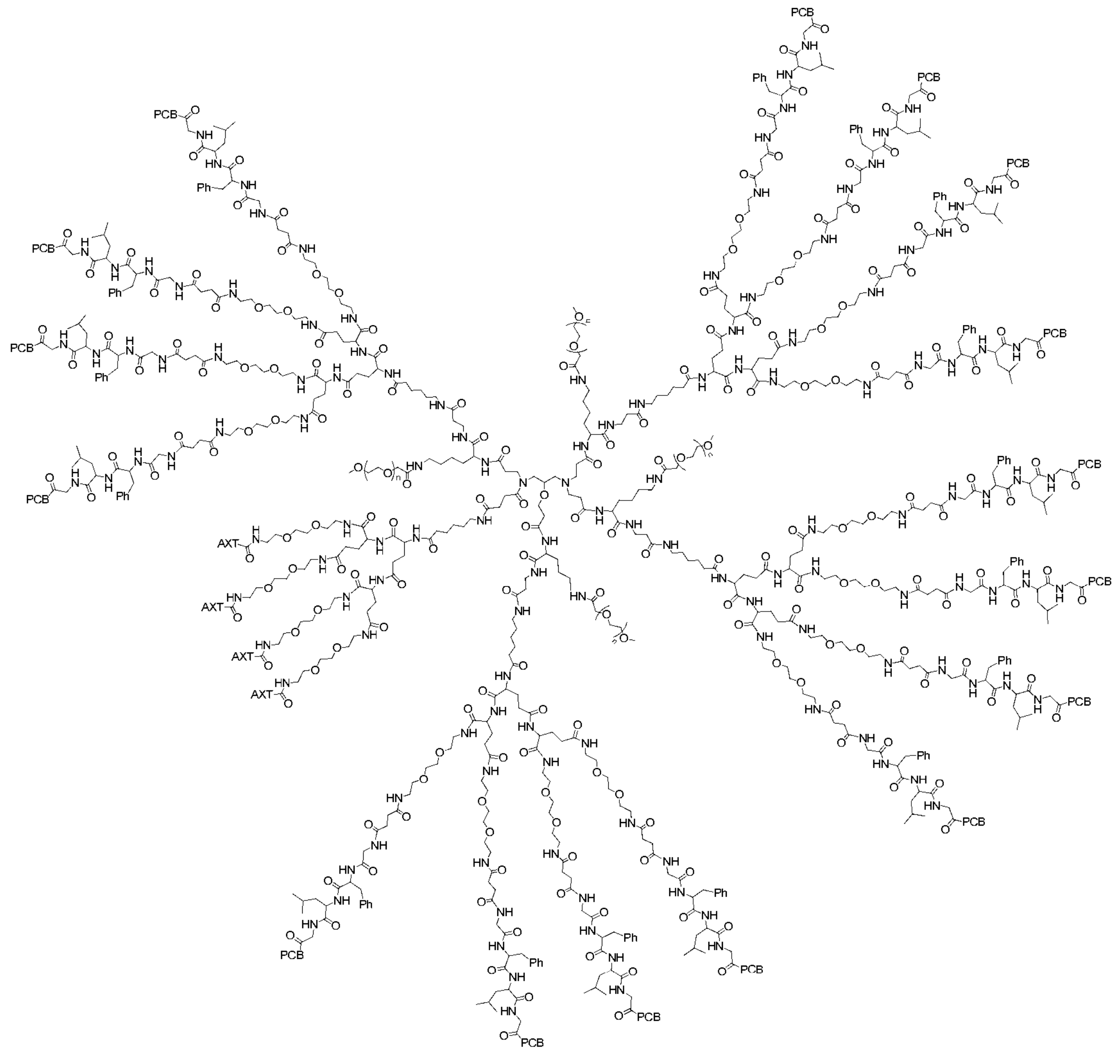 wherein, the number-average molecular weight of each polyethylene glycol fragment is 10k |

(continued)

| Comp d No. | Compound structure |
|---|---|
| **75-209** | 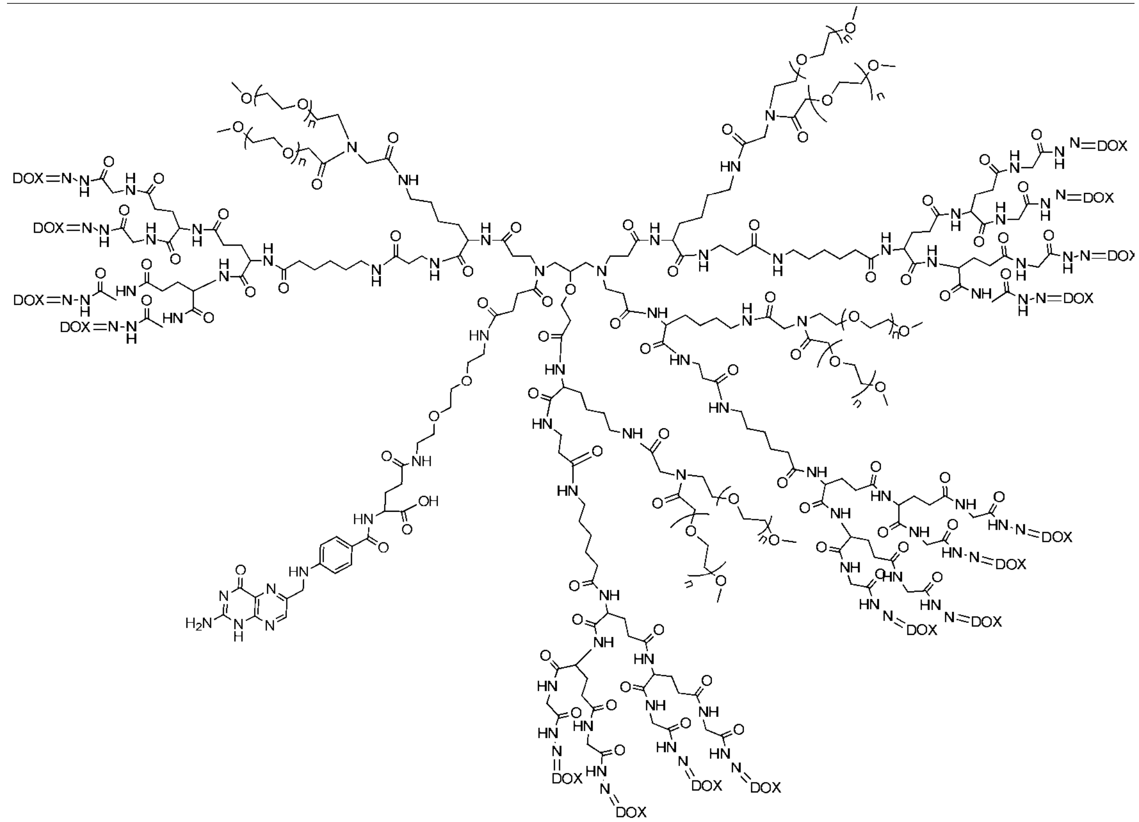 wherein, the number-average molecular weight of each polyethylene glycol fragment is 10k |

(continued)

| Comp d No. | Compound structure |
| --- | --- |
| **77-162** | 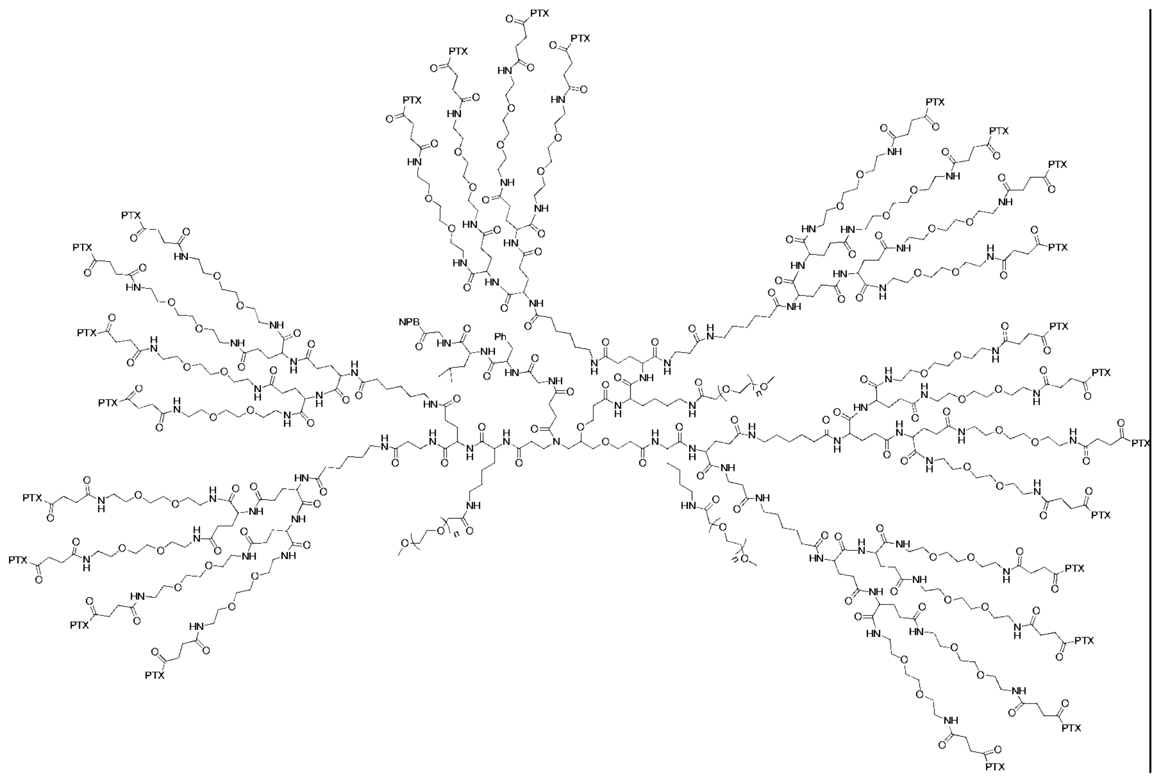 wherein, the number-average molecular weight of each polyethylene glycol fragment is 10k |

(continued)

| Comp d No. | Compound structure |
|---|---|
| **81-231** | 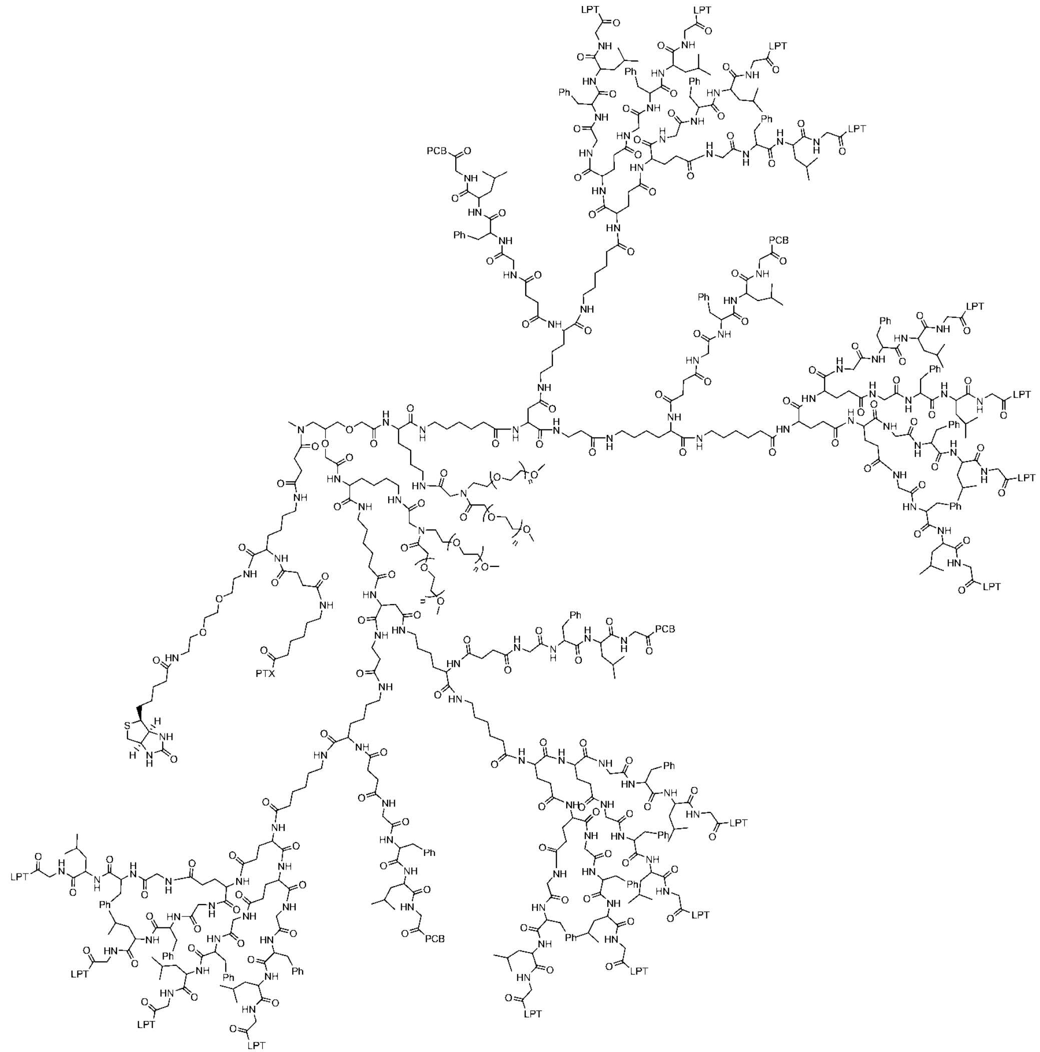<br>wherein, the number-average molecular weight of each polyethylene glycol fragment is 10k |

(continued)

| Comp d No. | Compound structure |
| --- | --- |
| **84-102** | 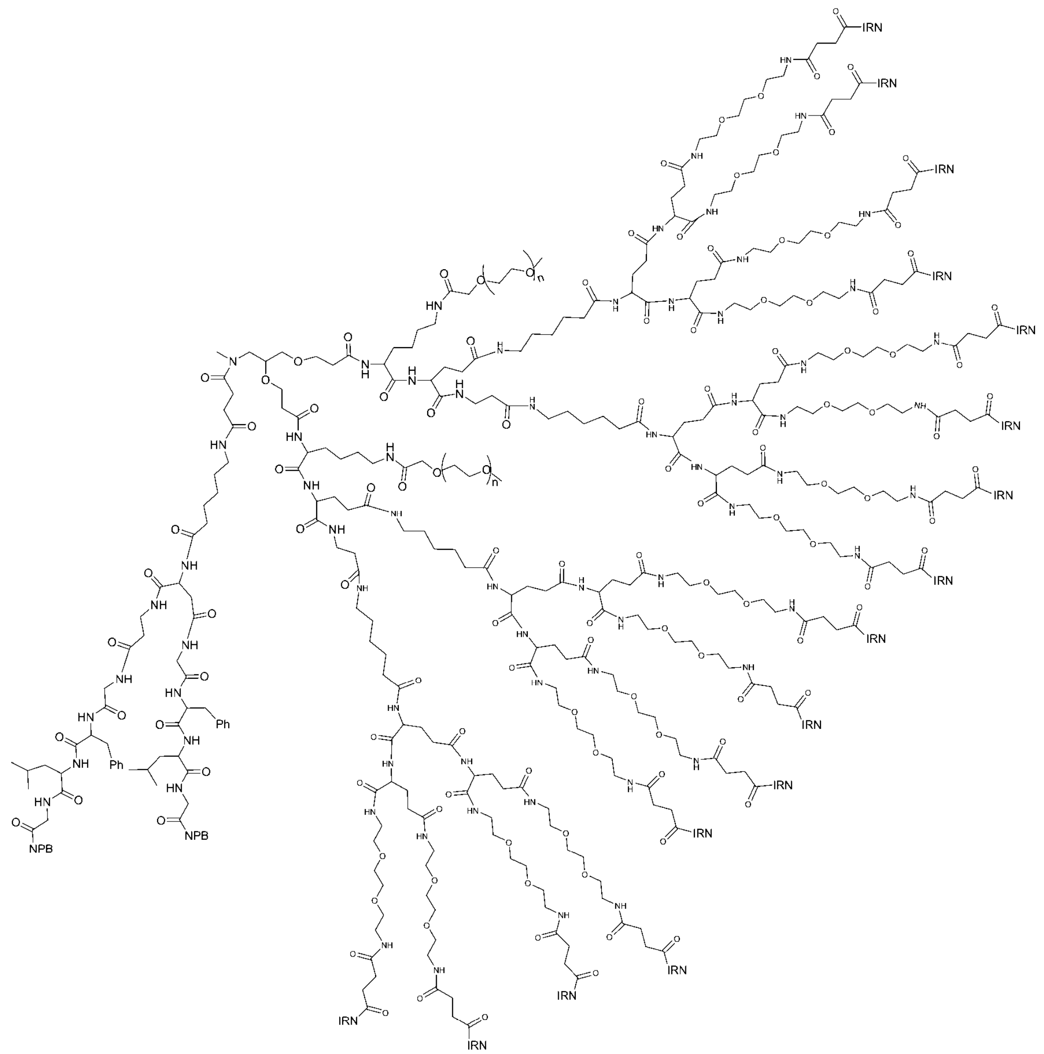<br>wherein, the number-average molecular weight of each polyethylene glycol fragment is 10k |

(continued)

| Comp d No. | Compound structure |
| --- | --- |
| **88-228** | 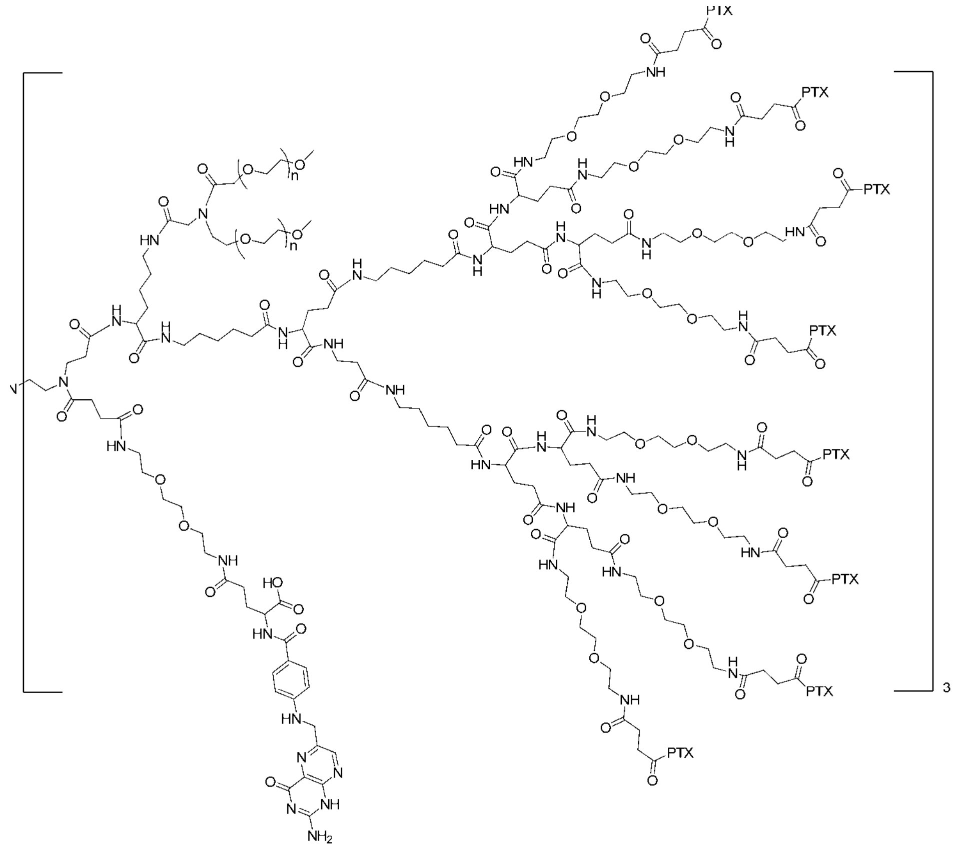<br>wherein, the number-average molecular weight of each polyethylene glycol fragment is 10k |

(continued)

| Comp d No. | Compound structure |
|---|---|
| **76-179** | 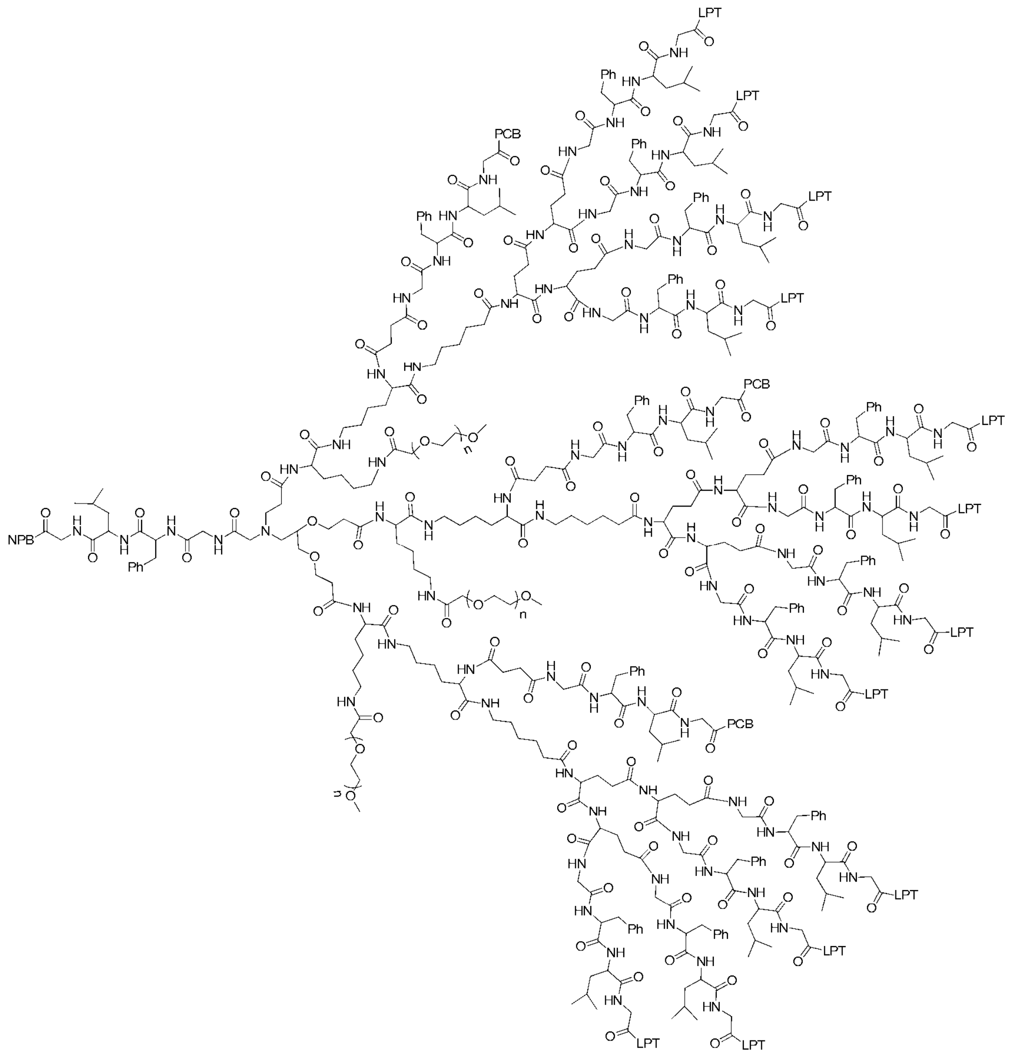<br>wherein, the number-average molecular weight of each polyethylene glycol fragment is 10k |

(continued)

| Comp d No. | Compound structure |
|---|---|
| **87-47** | 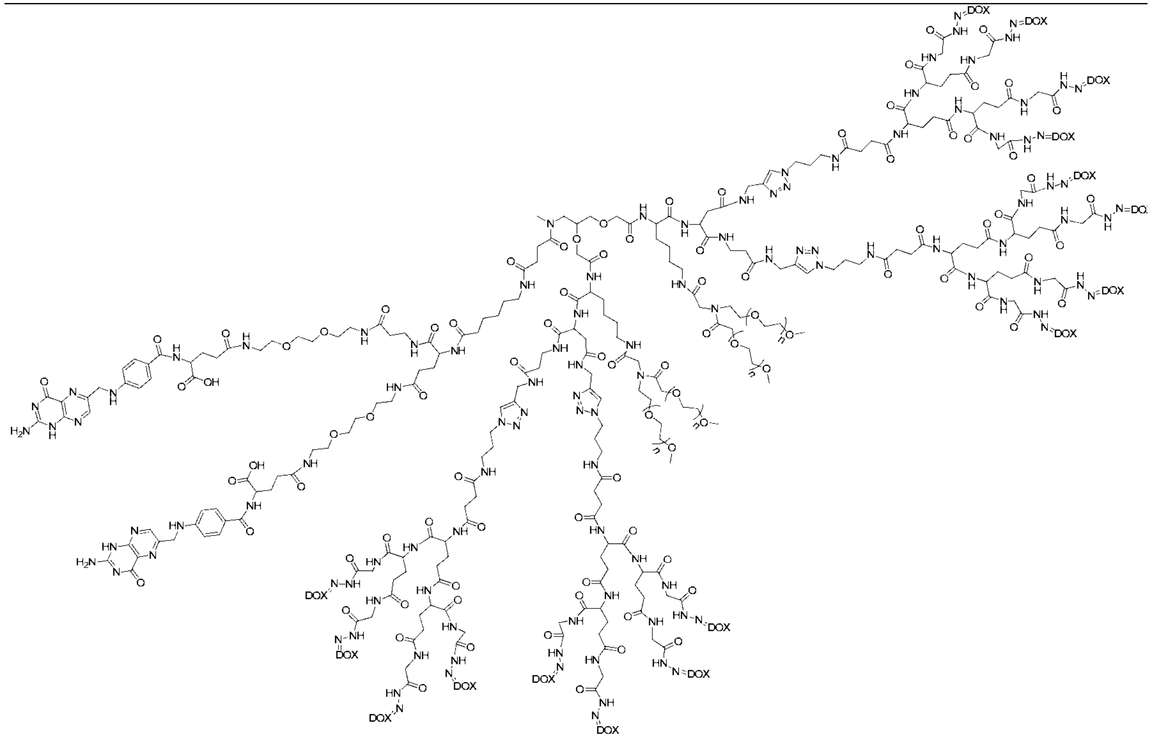 wherein, the number-average molecular weight of each polyethylene glycol fragment is 10k |

(continued)

| Comp d No. | Compound structure |
|---|---|
| **72-248** | 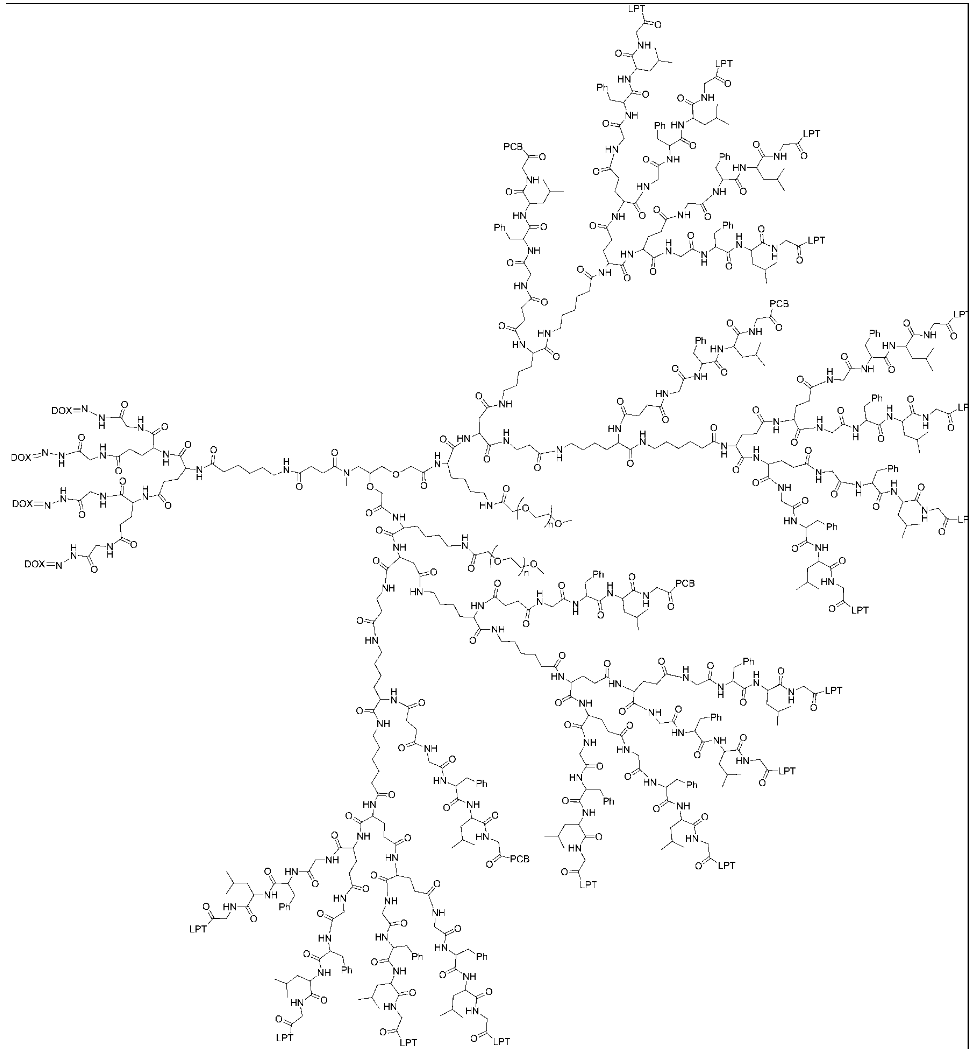<br>wherein, the number-average molecular weight of each polyethylene glycol fragment is 10k |

(continued)

| Comp d No. | Compound structure |
|---|---|
| **72-279** | 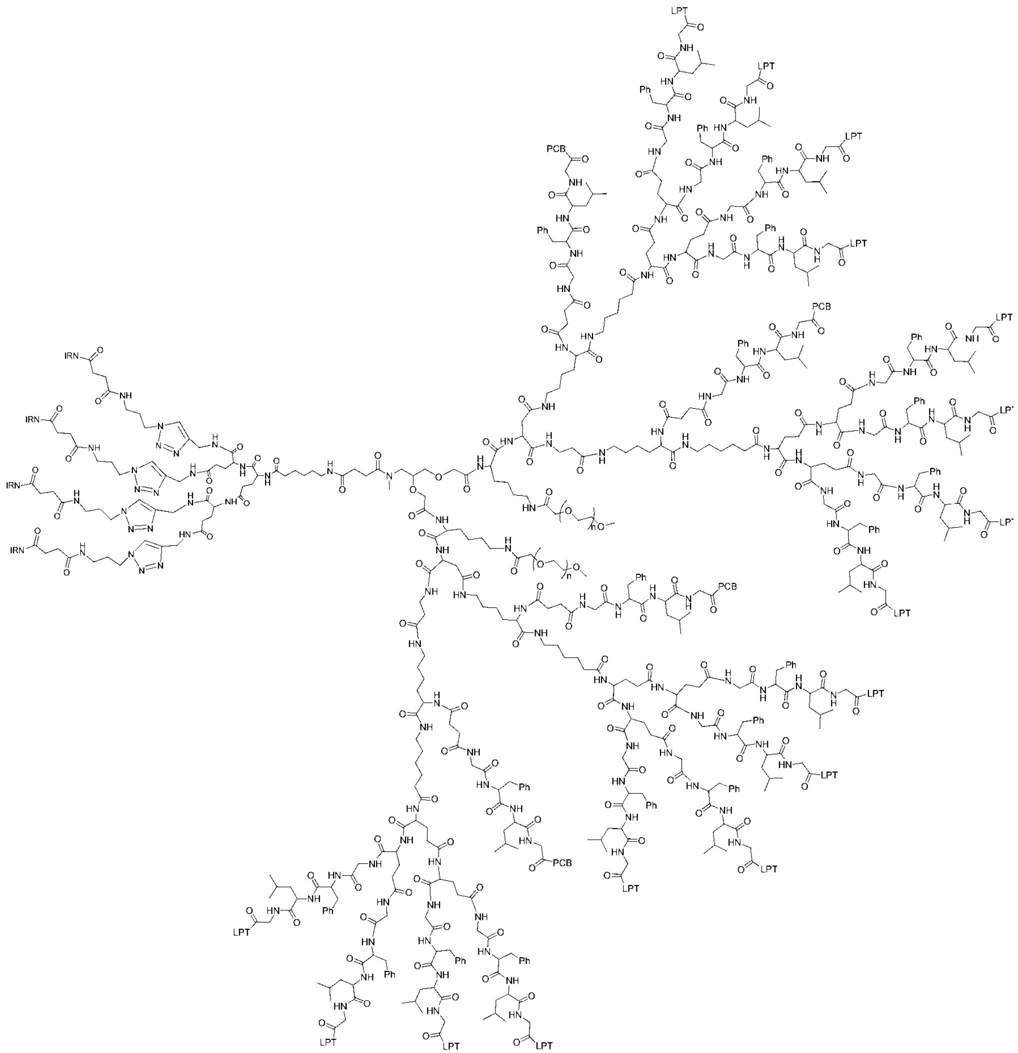 wherein, the number-average molecular weight of each polyethylene glycol fragment is 10k |

(continued)

| Comp d No. | Compound structure |
| --- | --- |
| **87-40** | 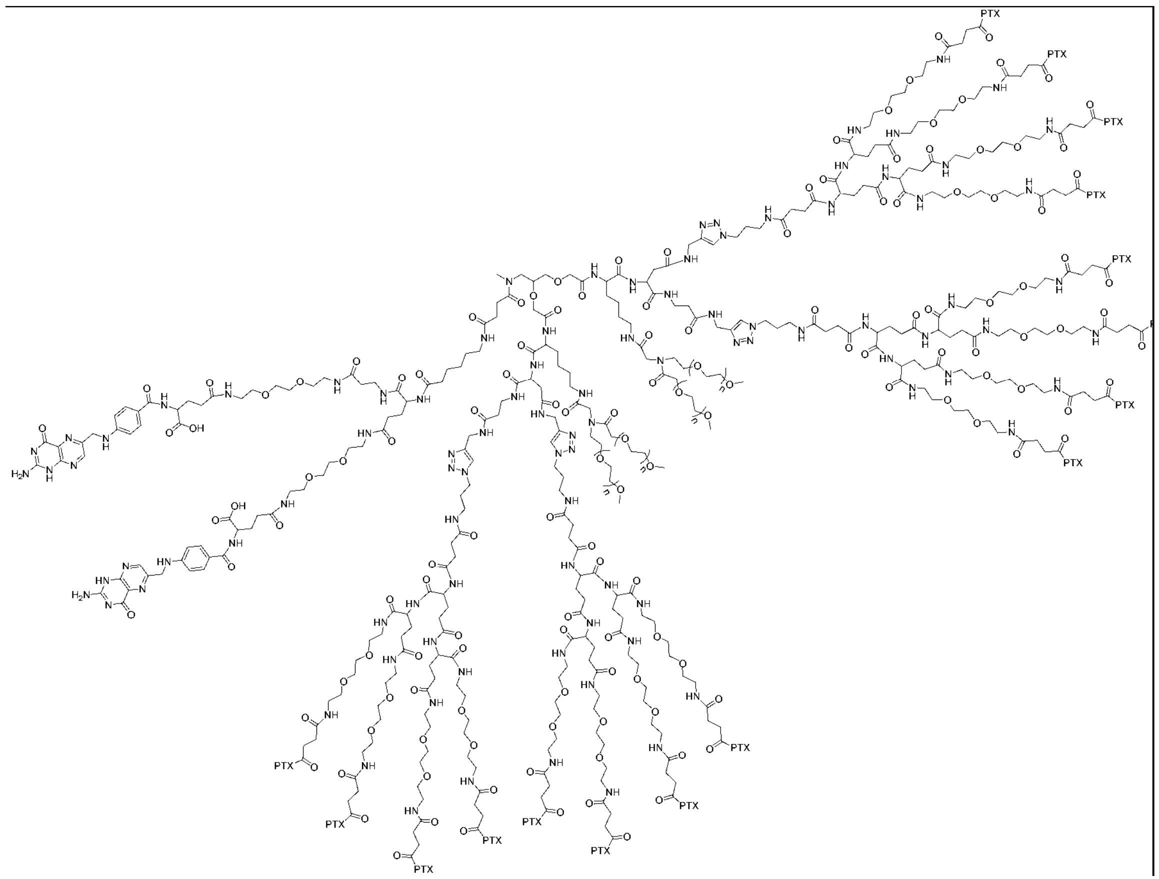 wherein, the number-average molecular weight of each polyethylene glycol fragment is 10k |

(continued)

| Comp d No. | Compound structure |
| --- | --- |
| **87-42** | 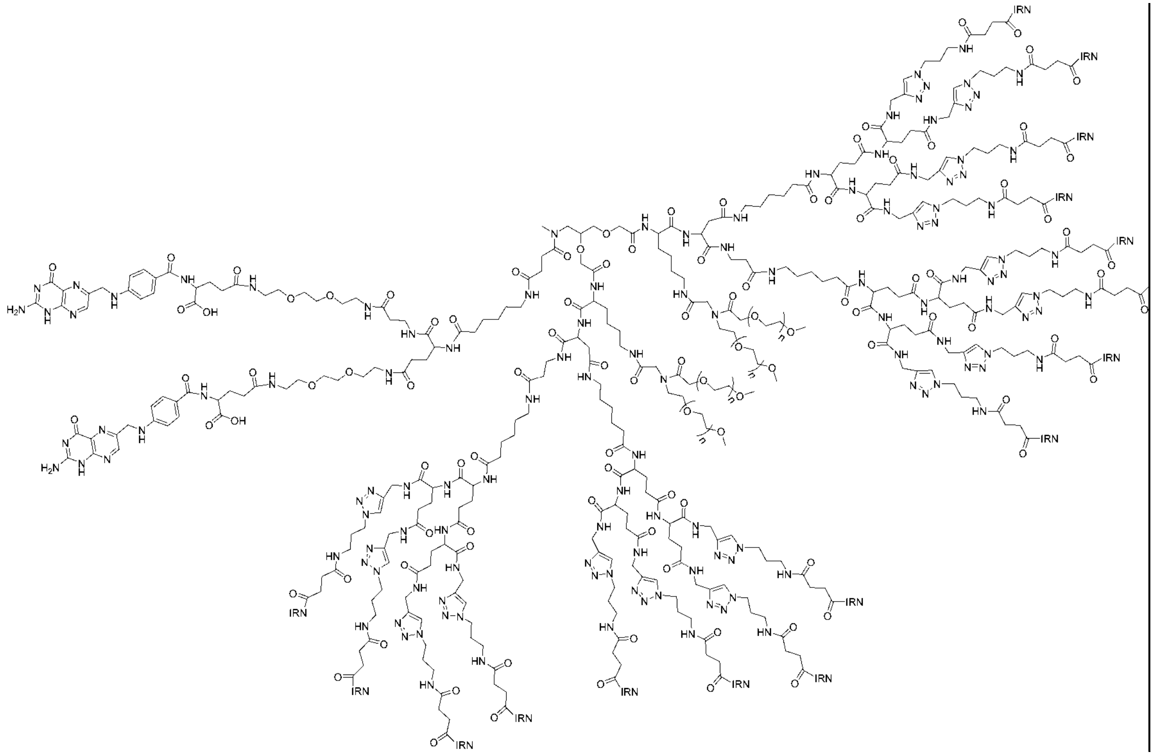 wherein, the number-average molecular weight of each polyethylene glycol fragment is 10k |

(continued)

| Comp d No. | Compound structure |
| --- | --- |
| **85-63** | 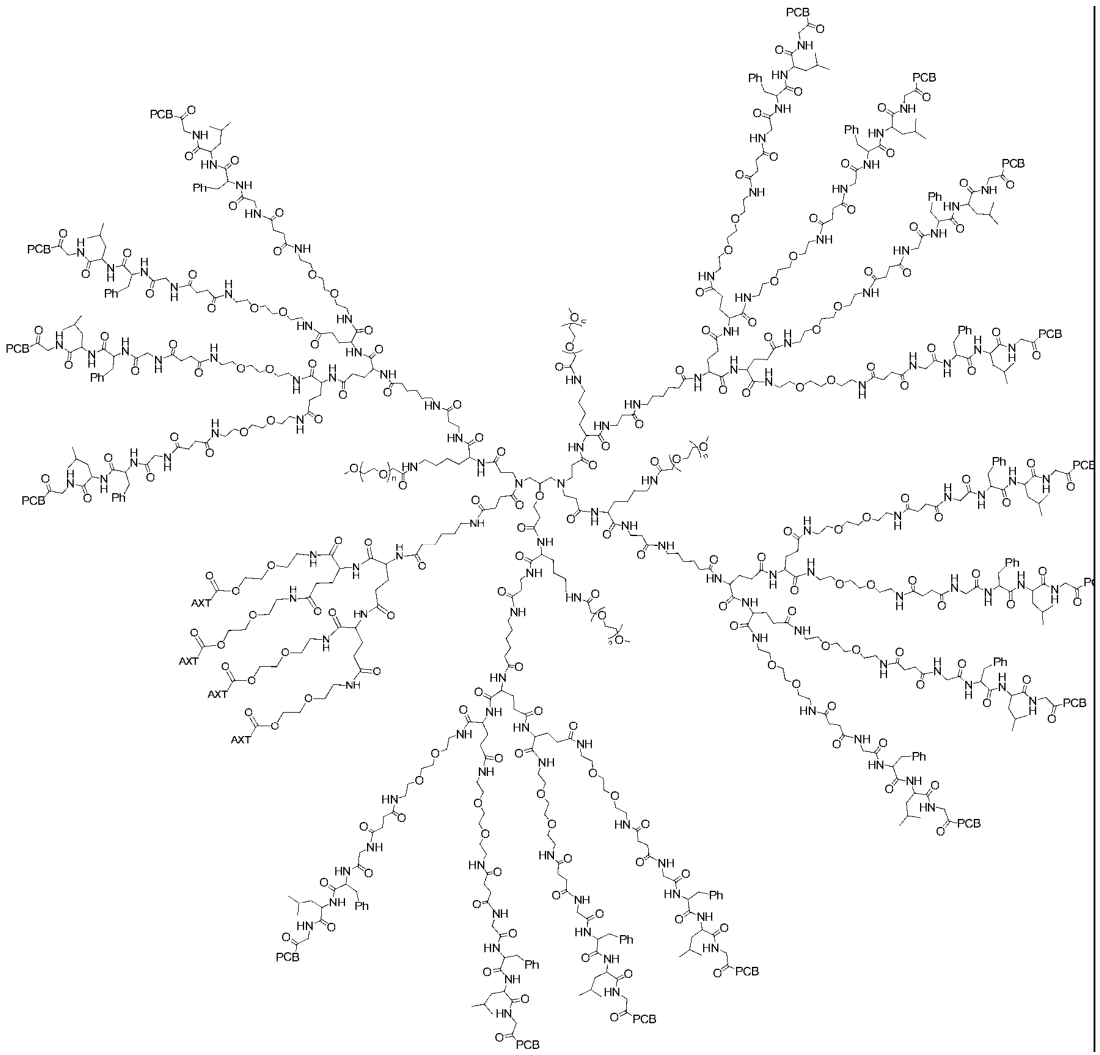 wherein, the number-average molecular weight of each polyethylene glycol fragment is 10k |

(continued)

| Comp d No. | Compound structure |
|---|---|
| **88-233** | 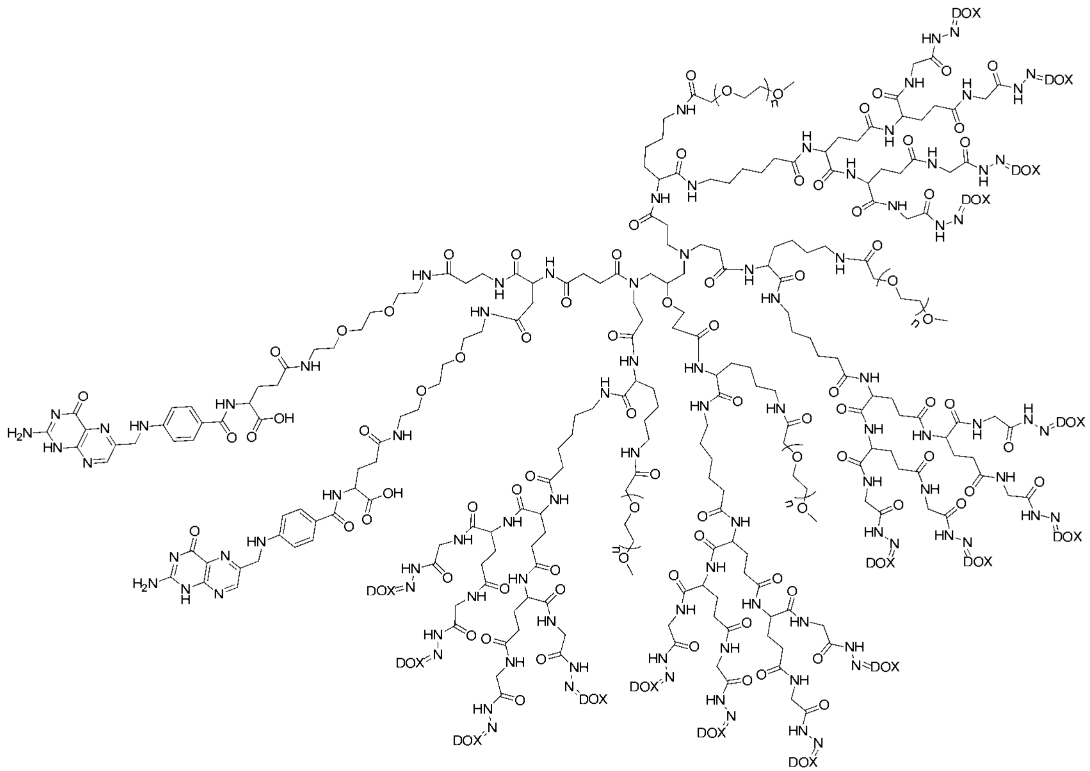 wherein, the number-average molecular weight of each polyethylene glycol fragment is 5k |

(continued)

| Comp d No. | Compound structure |
|---|---|
| **82-220** | 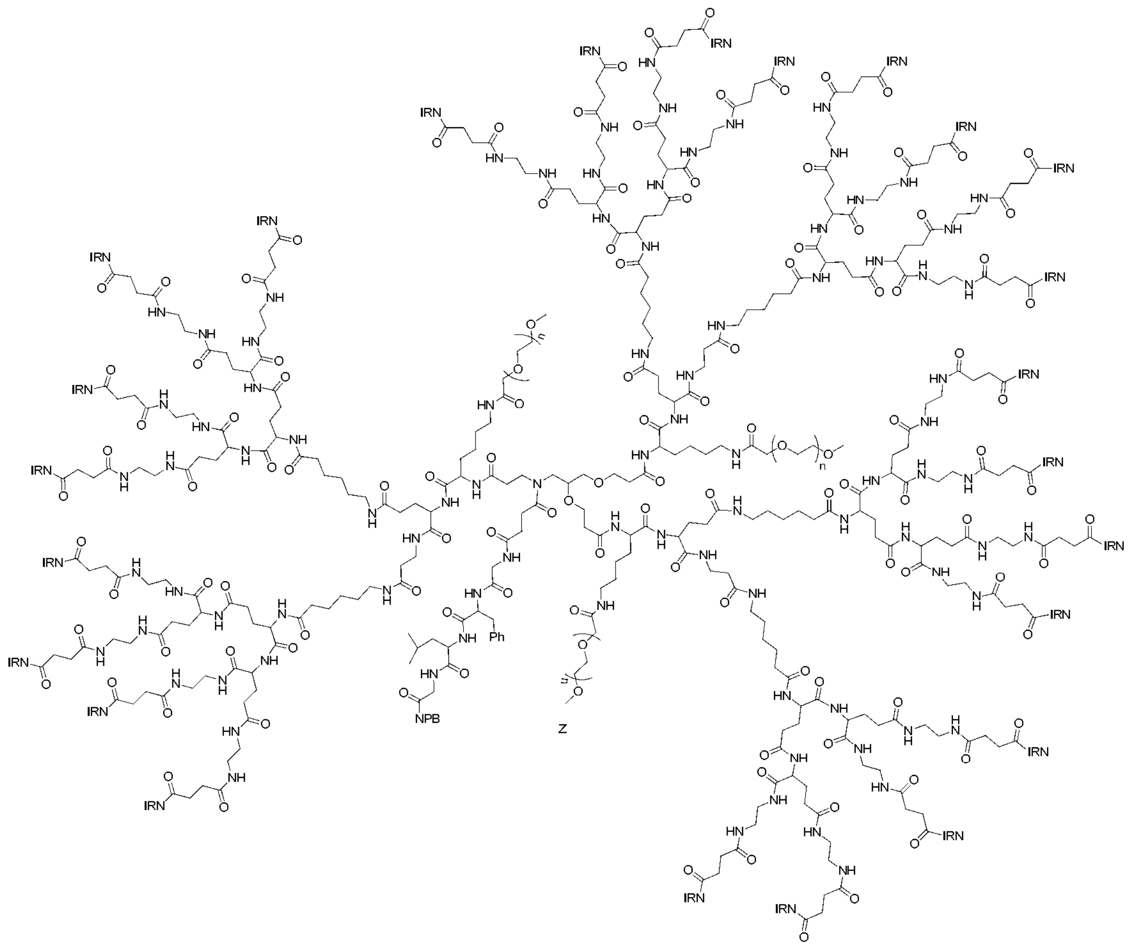<br>wherein, the number-average molecular weight of each polyethylene glycol fragment is 10k |

**9.** A compound represented by formula II or a pharmaceutically acceptable salt thereof,

$$(Pg_1')_{n_{11}}-M-(Pg_1)_{n_{12}}$$

formula II

wherein, each of $Pg_1$ and Pgi' is independently hydrogen or an amino protecting group, and $Pg_1$ and $Pg_1$' are different; preferably, the amino protecting group is selected from the group consisting of an alkyl-based protecting group (e.g., Bn, Trt, DMB, or PMB) and an alkoxycarbonyl-based protecting group (e.g., Boc, Fmoc, Cbz, or Teoc); preferably, $Pg_1$ is hydrogen and $Pg_1$' is an amino protecting group; alternatively, $Pg_1$ is an amino protecting group and $Pg_1$' is hydrogen;
the remaining groups are as defined in any one of claims 1 to 8.

**10.** The compound or pharmaceutically acceptable salt thereof according to claim 9, wherein the compound is selected from the group consisting of:

| | | |
|---|---|---|
| **59-223** 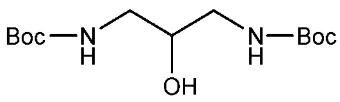 | **75-211** 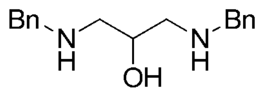 | **84-29** 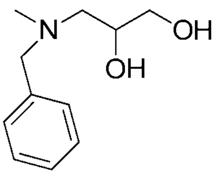 |
| **88-88** 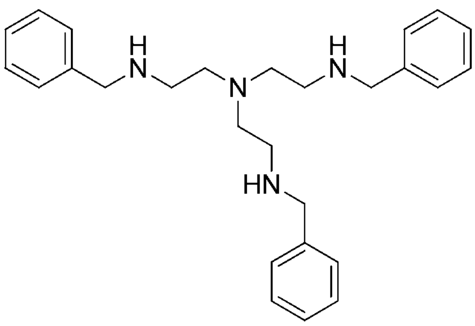 | **88-115** 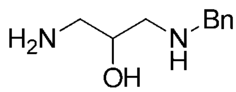 | |

**11.** A compound represented by formula III or a pharmaceutically acceptable salt thereof,

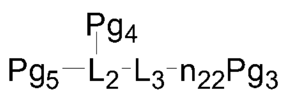

formula III

wherein, each $Pg_3$ is independently hydrogen or selected from the group consisting of an amino protecting group and a carboxyl protecting group, the amino protecting group is selected from the group consisting of an alkoxycarbonyl-based protecting group, such as Boc, Fmoc, Cbz, or Teoc; the carboxyl protecting group is selected from the group consisting of ester-based protecting group, such as methyl ester, ethyl ester, tert-butyl ester, allyl ester, or benzyl ester; preferably, each $Pg_3$ is independently hydrogen or a carboxyl protecting group, such as an ester-based protecting group, such as methyl ester, ethyl ester, tert-butyl ester, allyl ester, or benzyl ester, preferably tert-butyl ester or benzyl ester; preferably, each $Pg_3$ is the same, preferably all are tert-butyl ester or benzyl ester;
$Pg_4$ is hydrogen or a protecting group of the side chain of $L_2$, preferably, the protecting group is selected from the group consisting of an amino protecting group and a carboxyl protecting group, preferably, the amino protecting group is selected from the group consisting of an alkoxycarbonyl-based protecting group, such as Boc, Fmoc, Cbz, or Teoc, preferably, the carboxyl protecting group is selected from the group consisting of an ester-based

protecting group, such as methyl ester, ethyl ester, tert-butyl ester, allyl ester, or benzyl ester, preferably, $Pg_4$ is an amino protecting group, preferably, $Pg_4$ is Boc or Cbz;
$Pg_5$ is hydrogen or an amino protecting group, the amino protecting group is selected from the group consisting of an alkoxycarbonyl-based protecting group, such as Boc, Fmoc, Cbz, or Teoc; preferably, $Pg_5$ is hydrogen or Fmoc;
the remaining groups are as defined in any one of claims 1 to 8.

**12.** The compound or pharmaceutically acceptable salt thereof according to claim 11, wherein the compound is selected from the group consisting of:

| | |
|---|---|
| **74-102** 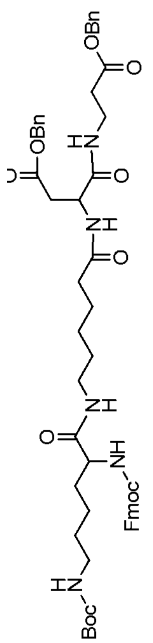 | **74-103** 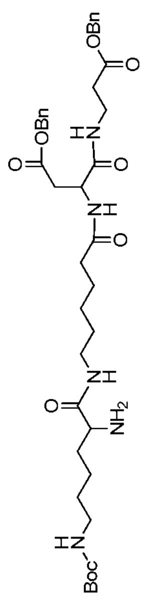 |
| **76-56** 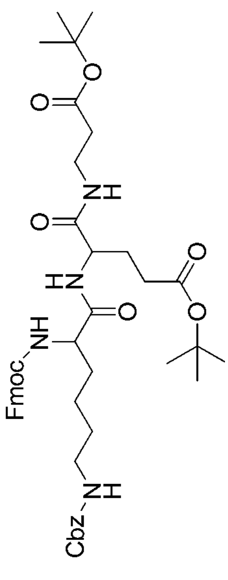 | **76-58** 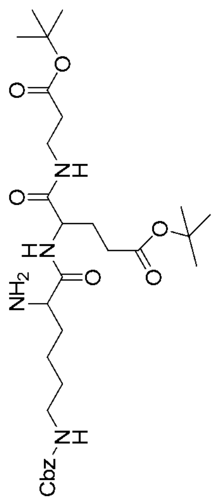 |
| **88-85** 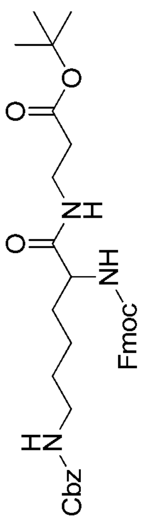 | **88-86** 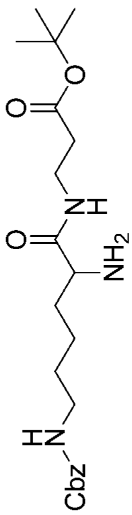 |
| **80-180** 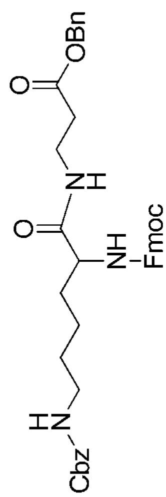 | **80-185** 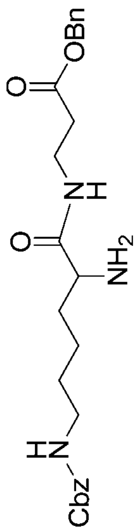 |
| **76-115** | **76-116** |

➽Too large Table

| | |
|---|---|
| 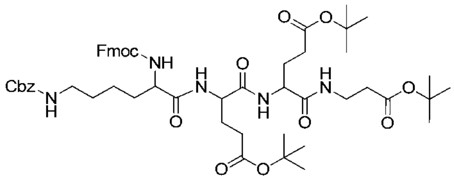 | 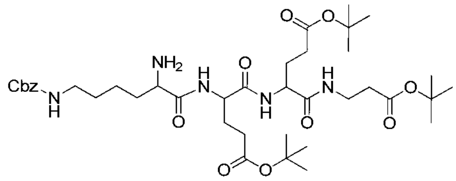 |
| 88-99<br>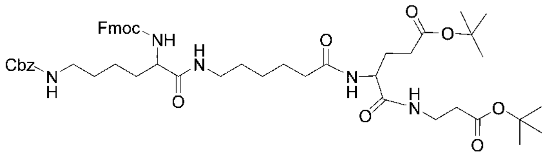 | 88-100<br>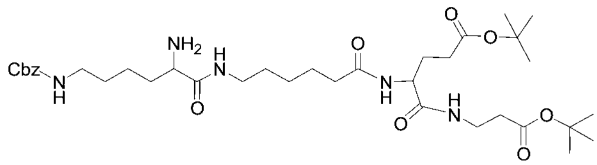 |
| 72-197<br>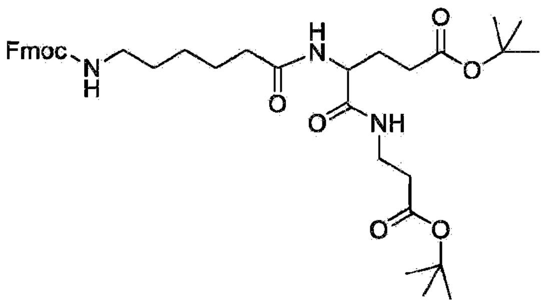 | 72-216<br>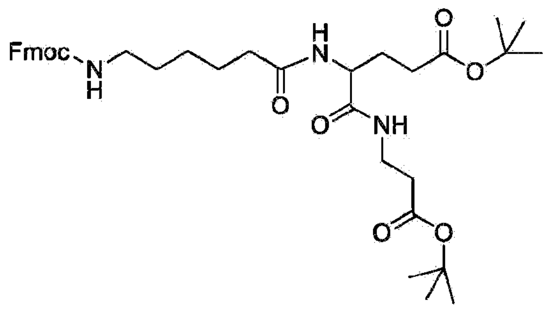 |
| 82-189<br>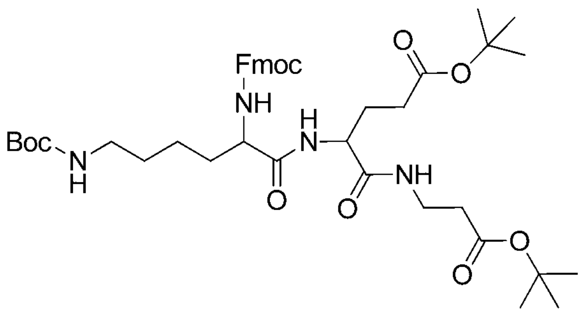 | 82-193<br>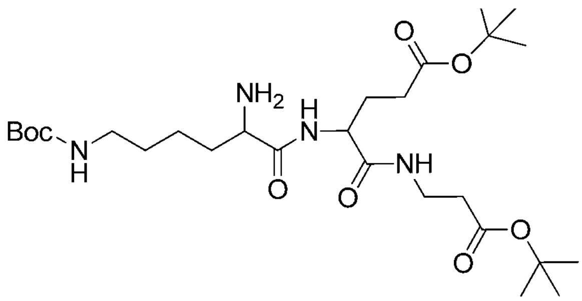 |

**13.** A compound represented by formula IV or a pharmaceutically acceptable salt thereof,

$$Pg_6-L_4\left(L_5-y_2Pg_7\right)_{n_{32}}$$

formula IV

wherein, each of $Pg_6$ and $Pg_7$ is independently hydrogen or selected from the group consisting of an amino protecting group, preferably, the amino protecting group is selected from the group consisting of an alkyl-based protecting group (e.g., Bn, Trt, DMB, or PMB) and an alkoxycarbonyl-based protecting group (e.g., Boc, Fmoc, Cbz, or Teoc);

the remaining groups are as defined in any one of claims 1 to 8.

**14.** A compound represented by formula V or a pharmaceutically acceptable salt thereof:

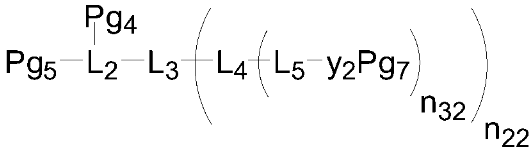

formula V

wherein, each group is as defined in any one of claims 1 to 13.

**15.** The compound or pharmaceutically acceptable salt thereof according to claim 14, wherein the compound is selected from the group consisting of:

➽Too large Table

| | |
|---|---|
| **88-162** | 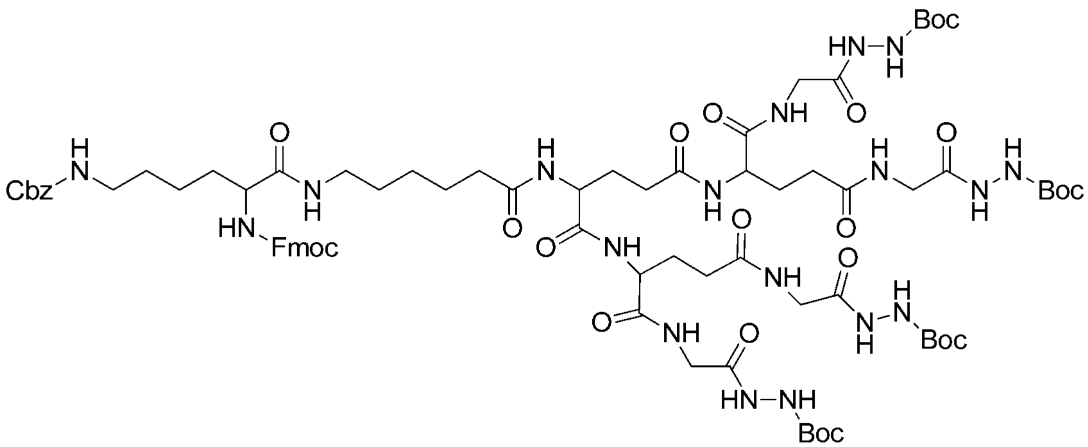 |
| **88-164** | 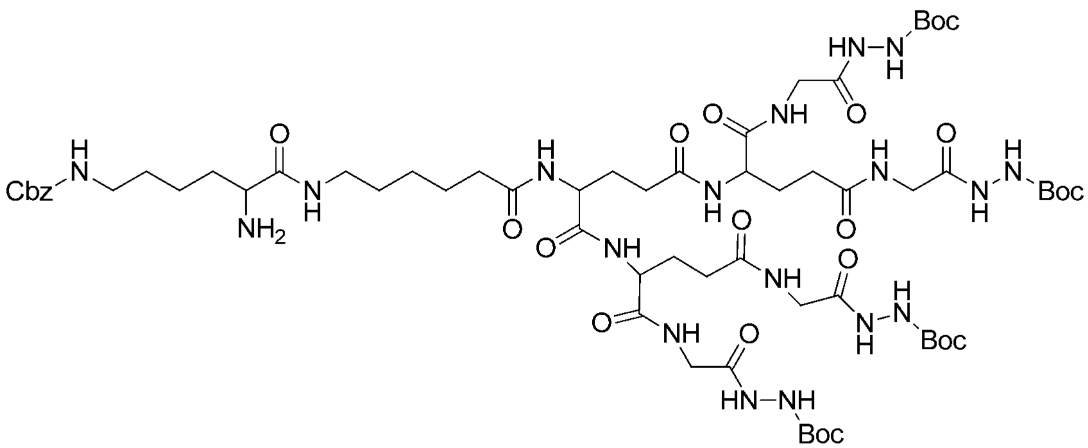 |

**16.** A compound represented by formula VI or a pharmaceutically acceptable salt thereof:

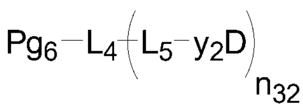

VI

wherein each group is as defined in any one of claims 1 to 15.

**17.** A compound represented by formula VII or a pharmaceutically acceptable salt thereof:

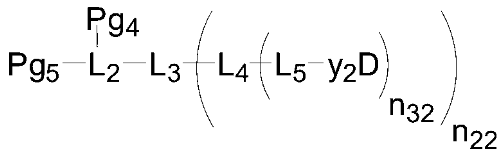

VII

wherein each group is as defined in any one of claims 1 to 16.

**18.** The compound or pharmaceutically acceptable salt thereof according to claim 17, wherein the compound is selected from the group consisting of:

| | |
|---|---|
| **76-168** | 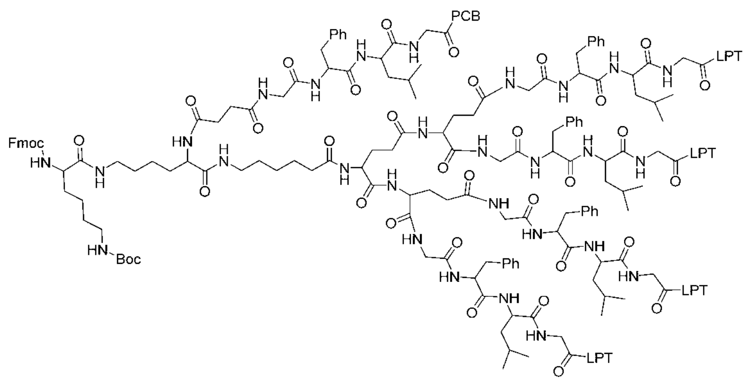 |
| **76-169** | 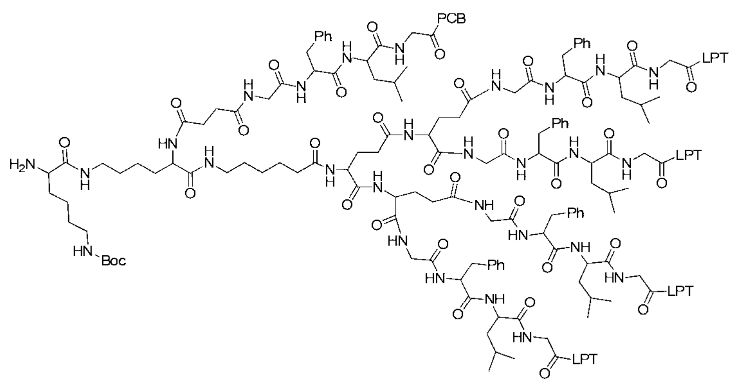 |

**19.** A compound represented by formula VIII or pharmaceutically acceptable salt thereof,

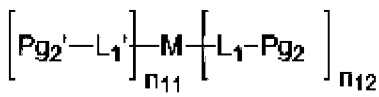

VIII

wherein, each of $Pg_2$ and $Pg_2$' is independently hydrogen or a carboxyl protecting group; preferably, the carboxyl protecting group is selected from the group consisting of an ester-based protecting group, such as methyl ester, ethyl ester, tert-butyl ester, allyl ester, or benzyl ester;

preferably, $Pg_2$ is hydrogen and $Pg_2$' is a carboxyl protecting group; $Pg_2$ is a carboxyl protecting group and $Pg_2$' is hydrogen; or, both $Pg_2$ and $Pg_2$' are carboxyl protecting groups (e.g., tert-butyl ester or benzyl ester), and $Pg_2$ and $Pg_2$' are different;

the remaining groups are as defined in any one of claims 1 to 18.

**20.** The compound or pharmaceutically acceptable salt thereof according to claim 19, wherein the compound is selected from the group consisting of:

➳Too large Table

| | |
|---|---|
| **81-65** 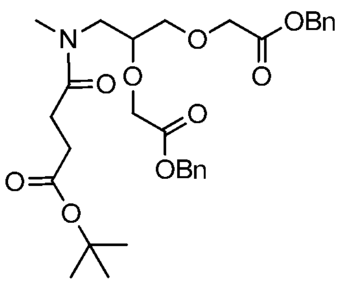 | **81-82** 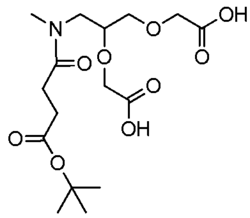 |
| **76-88** 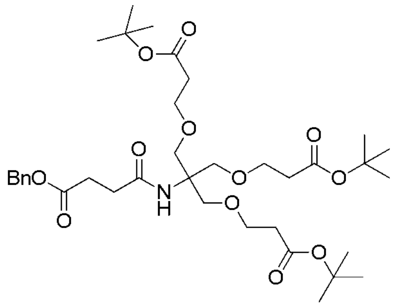 | **76-89** 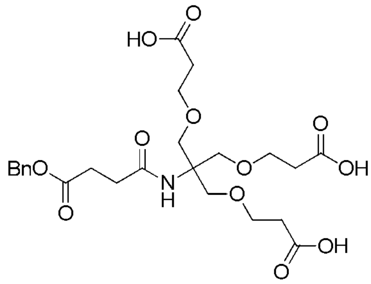 |
| **80-183** 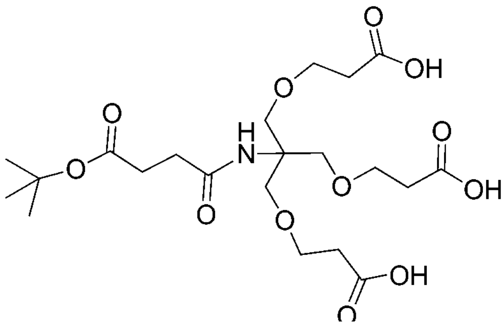 | **59-231** 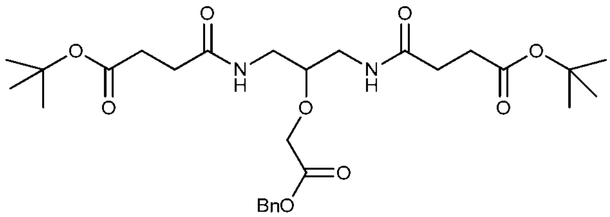 |
| **72-129** 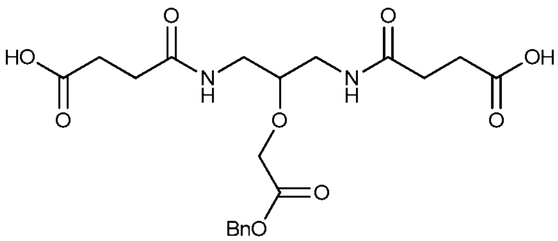 | **70-185** 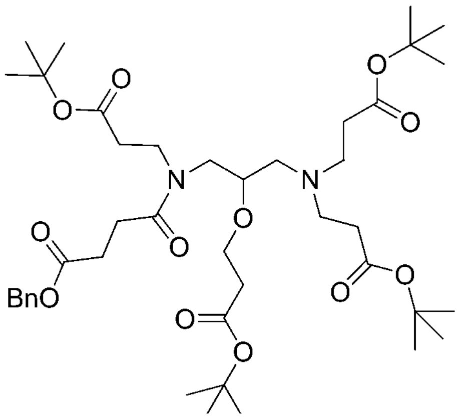 |
| **70-186** | **69-243** |

(continued)

| | |
|---|---|
| 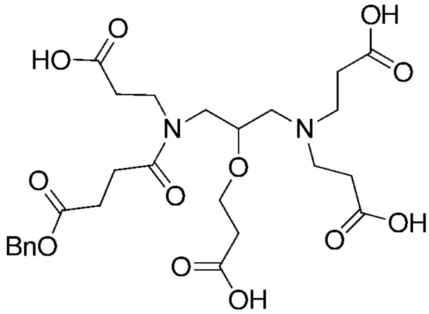 | 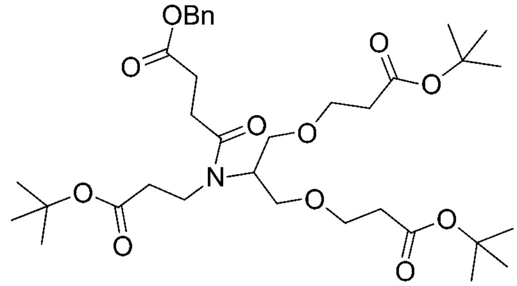 |
| **69-246** 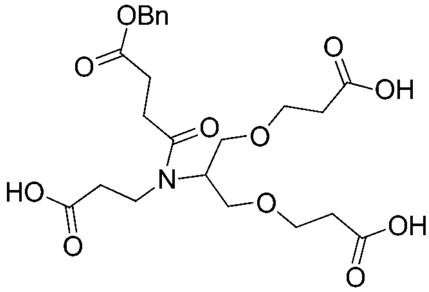 | **70-177** 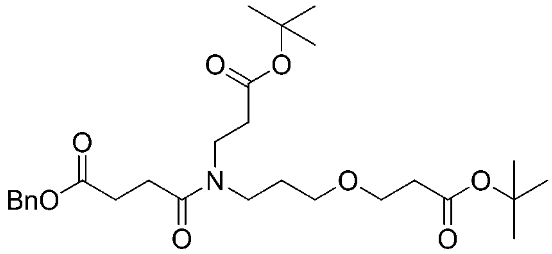 |
| **76-117** 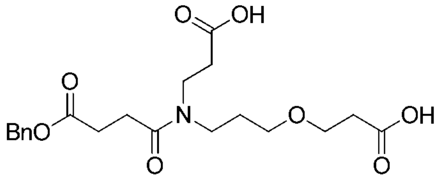 | **75-215** 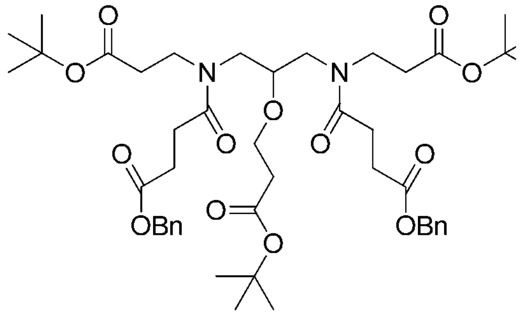 |
| **75-217** 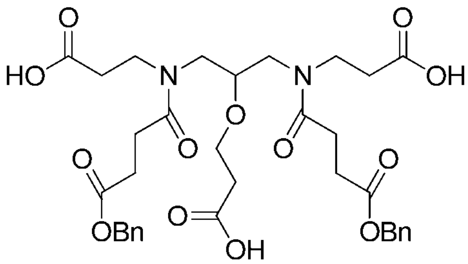 | **77-82** 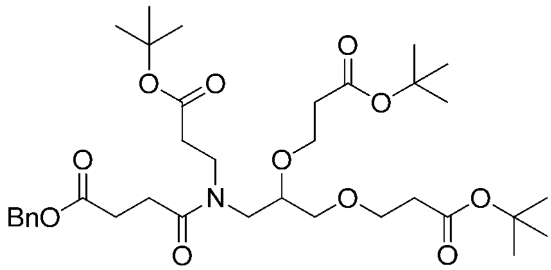 |
| **77-83** 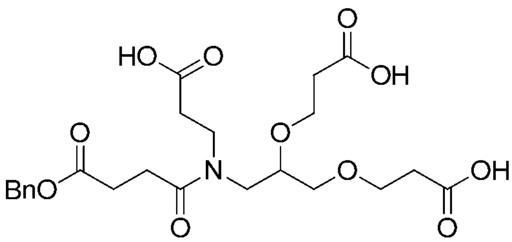 | **84-35** 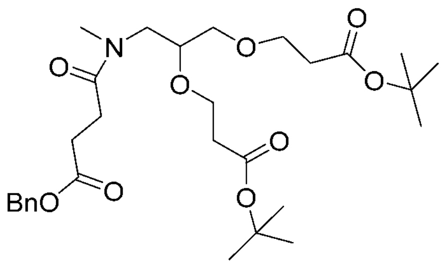 |
| **84-36** | **88-101** |

(continued)

| | |
|---|---|
| 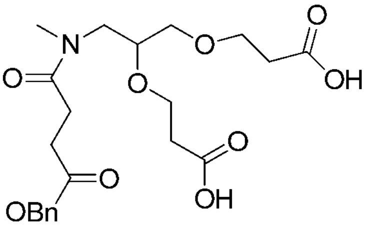 | 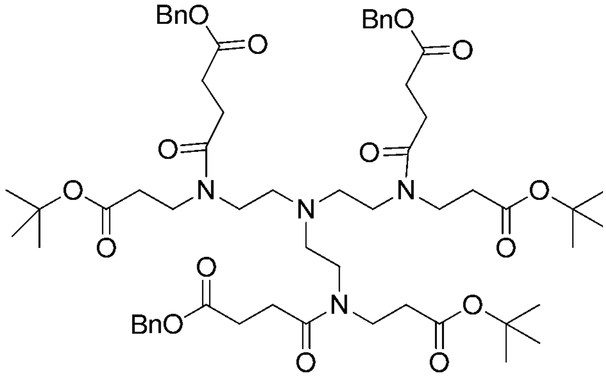 |
| **88-148** 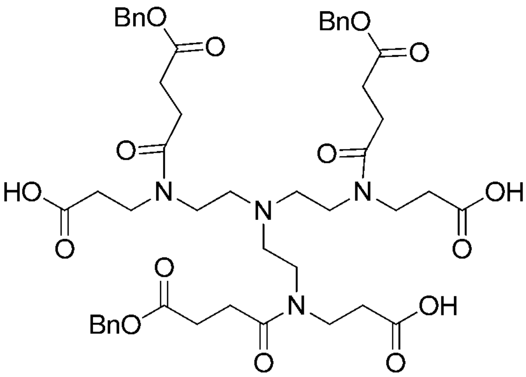 | **76-149** 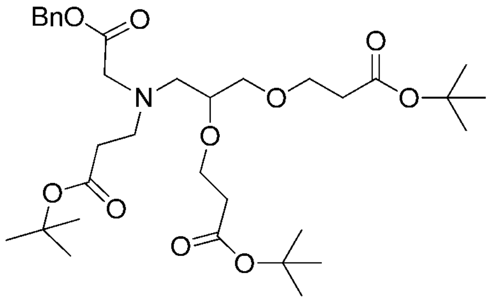 |
| **76-155** 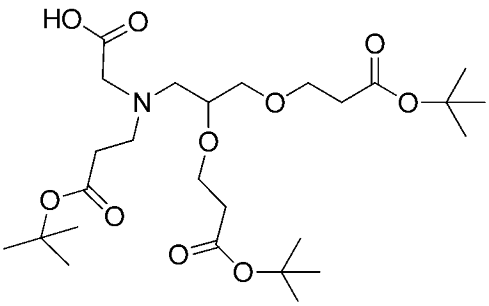 | **76-199** 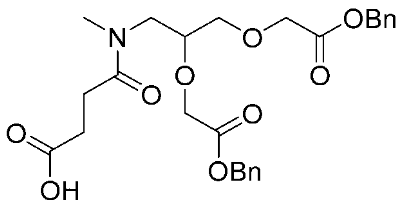 |
| **82-184** 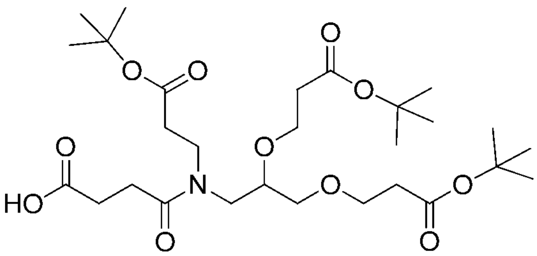 | |

**21.** A compound represented by formula IX or a pharmaceutically acceptable salt thereof,

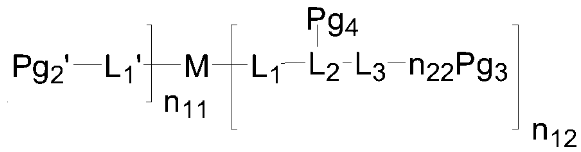

IX

wherein each group is as defined in any one of claims 1 to 20.

**22.** The compound or pharmaceutically acceptable salt thereof according to claim 21, wherein the compound is selected from the group consisting of:

| | |
|---|---|
| 76-103 | 81-197 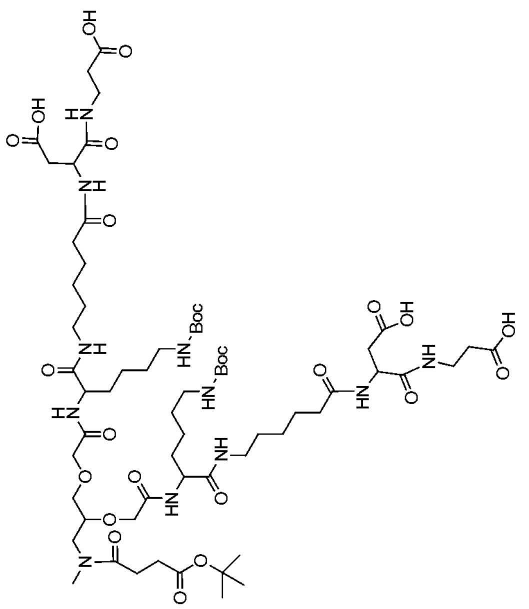 |
| 76-99 | 81-85 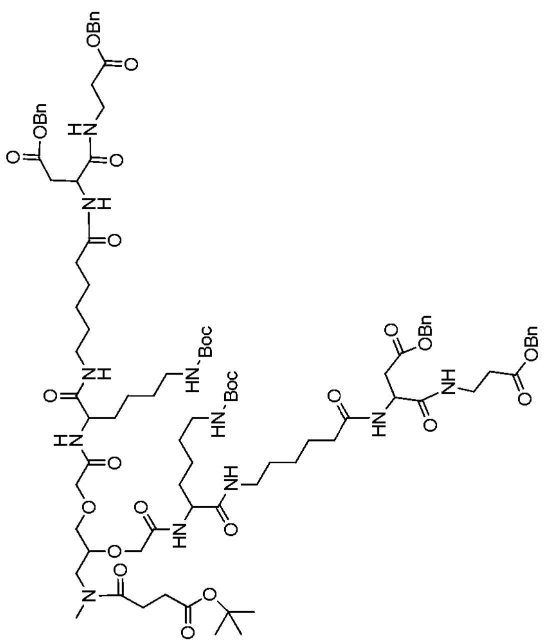 |

(continued)

| | |
|---|---|
| 88-89 | 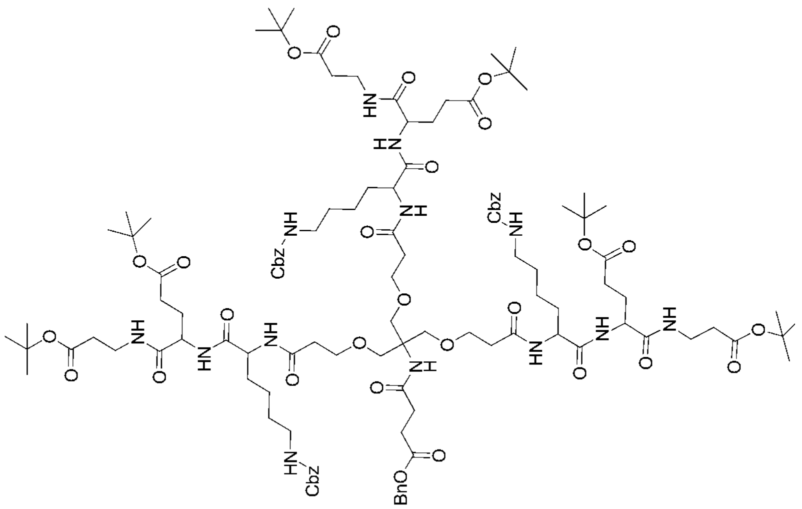 |
| 88-90 | 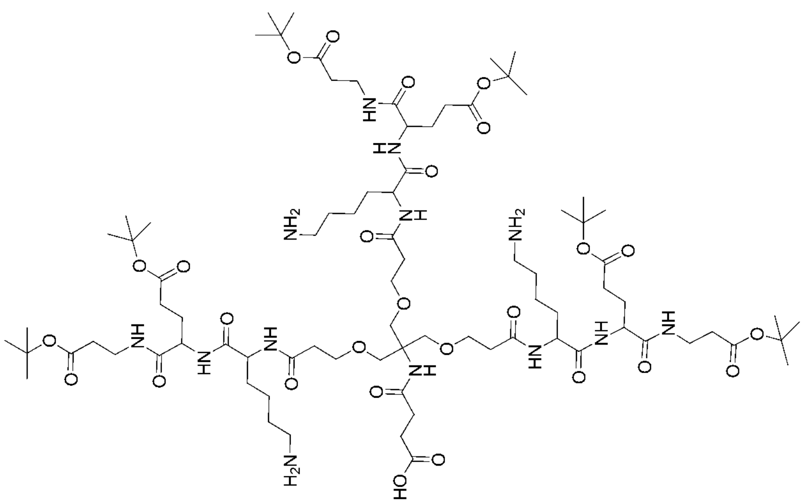 |

(continued)

| | |
|---|---|
| 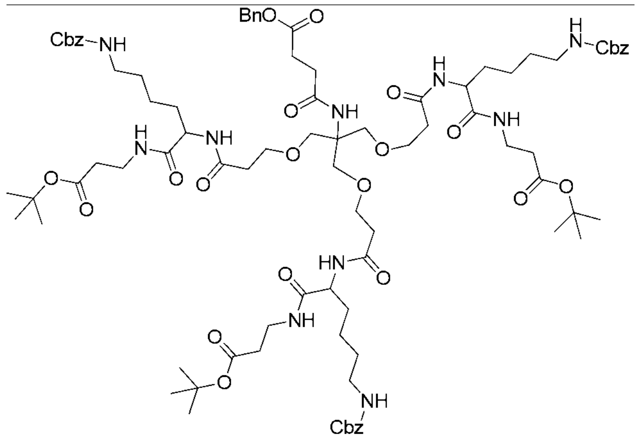 | 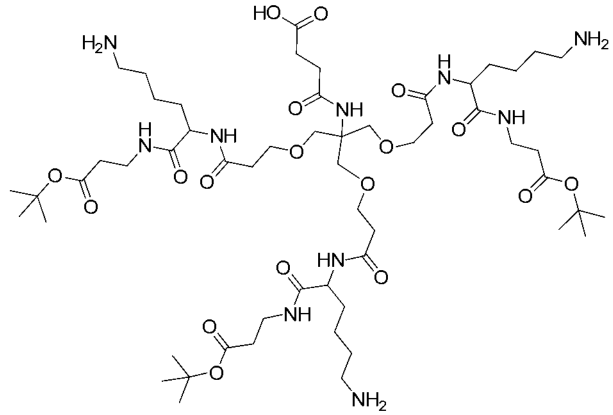 |
| **80-186** 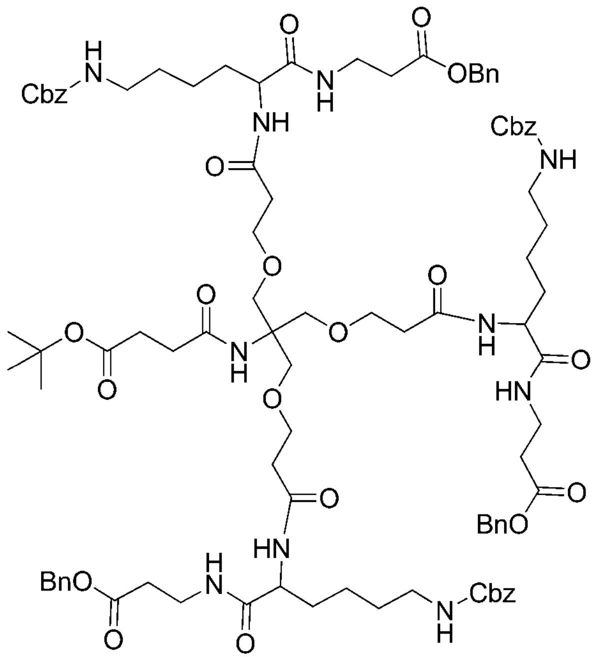 | **78-108** 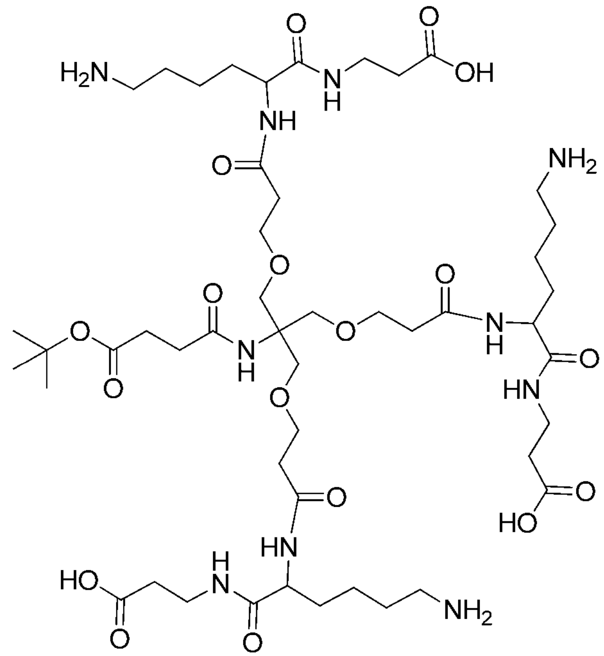 |

(continued)

| | |
|---|---|
| 72-139 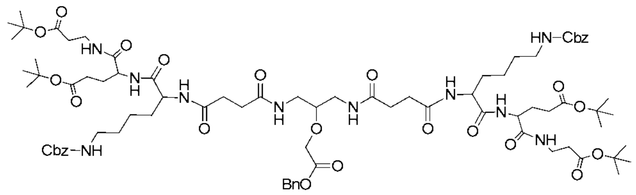 | 72-142 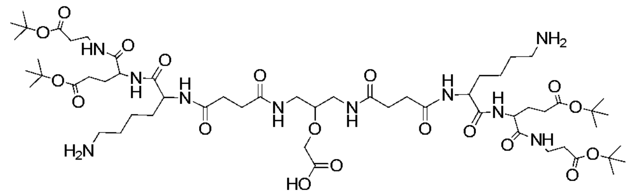 |
| 70-204 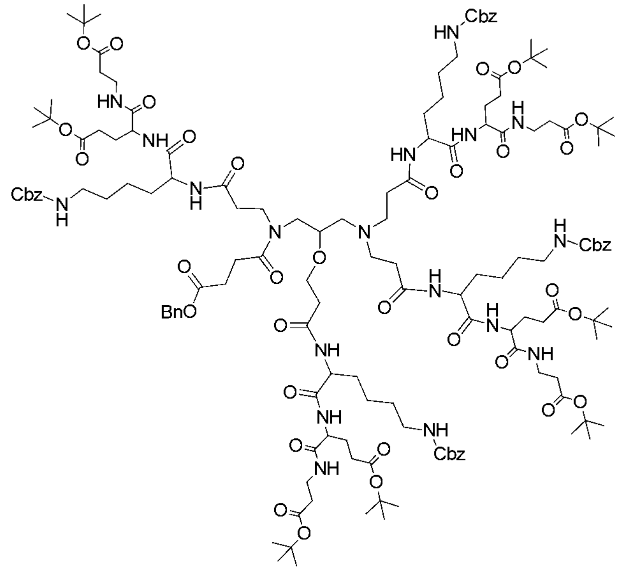 | 70-208 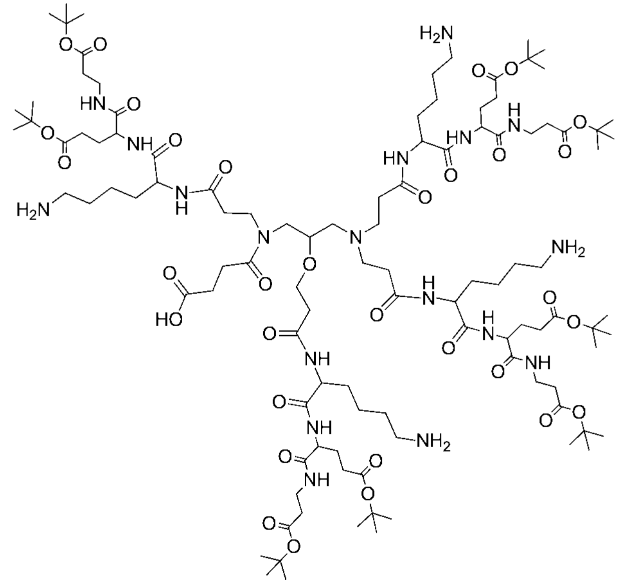 |
| 69-251 | 69-257 |

(continued)

| | | |
|---|---|---|
| 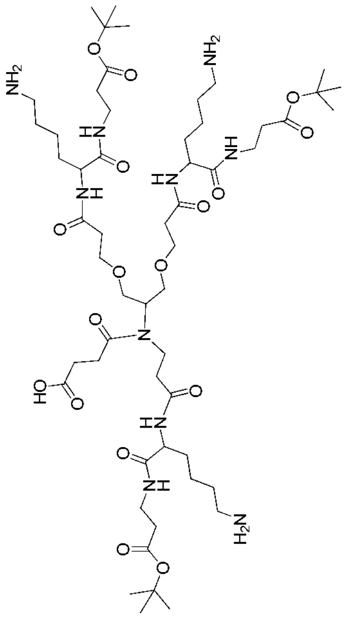 | 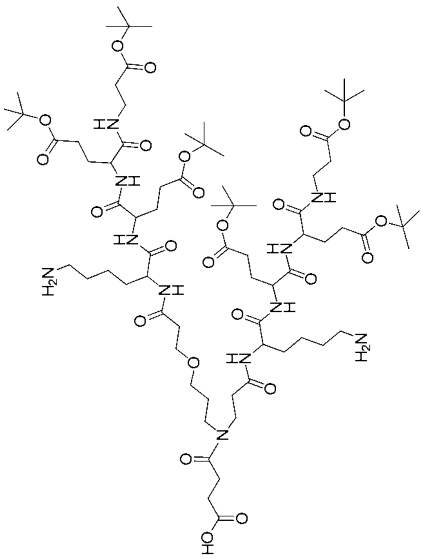 76-119 | 75-222 |
| 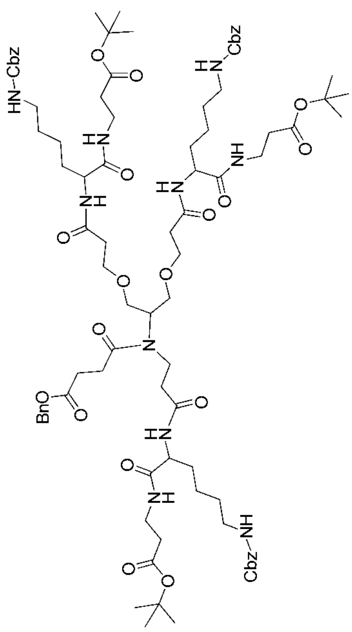 | 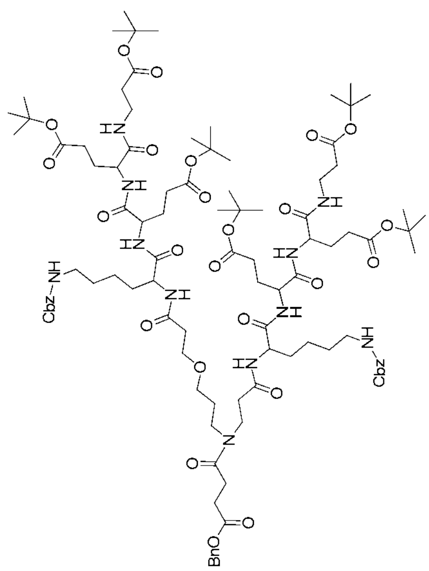 76-118 | 75-220 |

(continued)

| | |
|---|---|
| 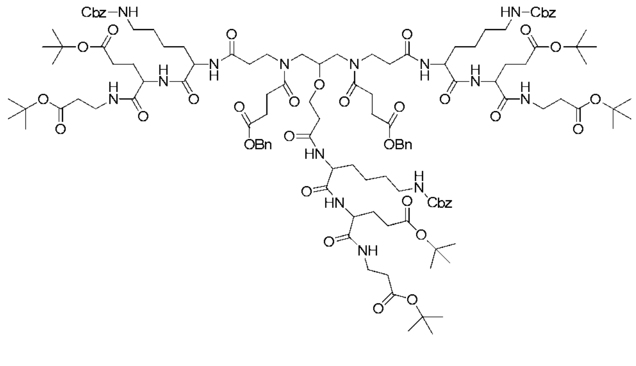 | 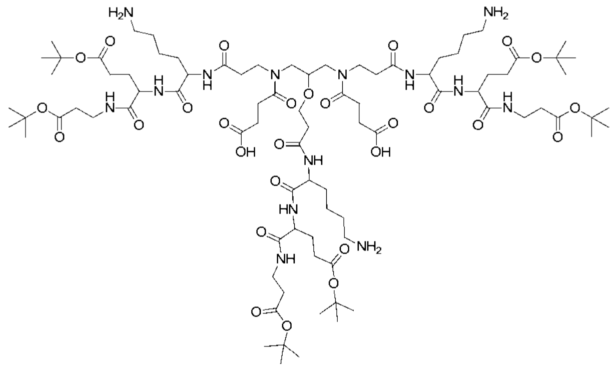 |
| **74-130** | **74-214** |
| 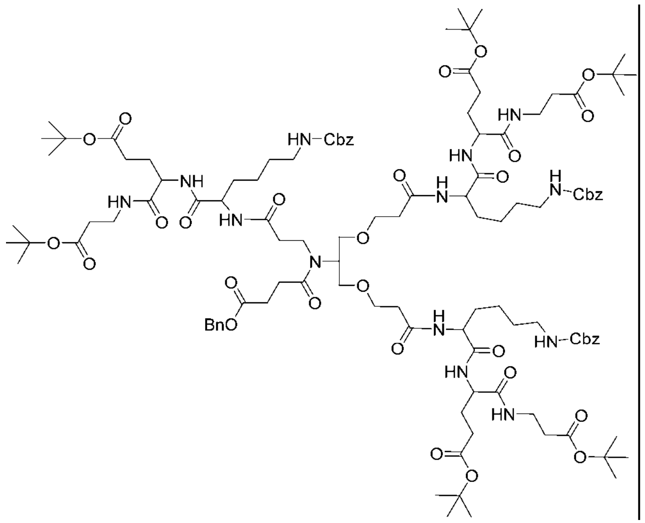 | 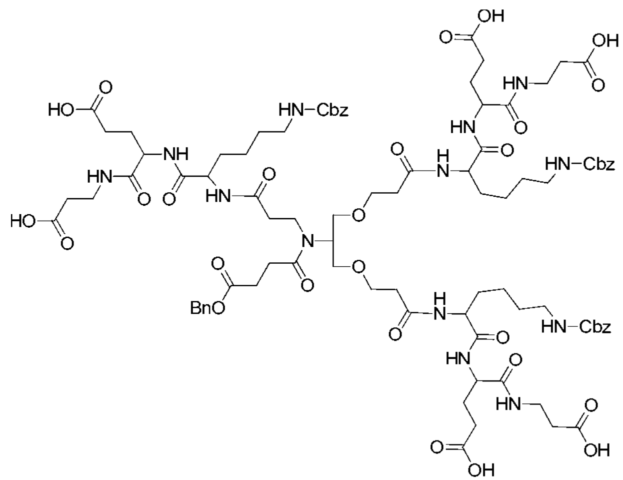 |
| **85-55** | **85-61** |

(continued)

| | |
|---|---|
| 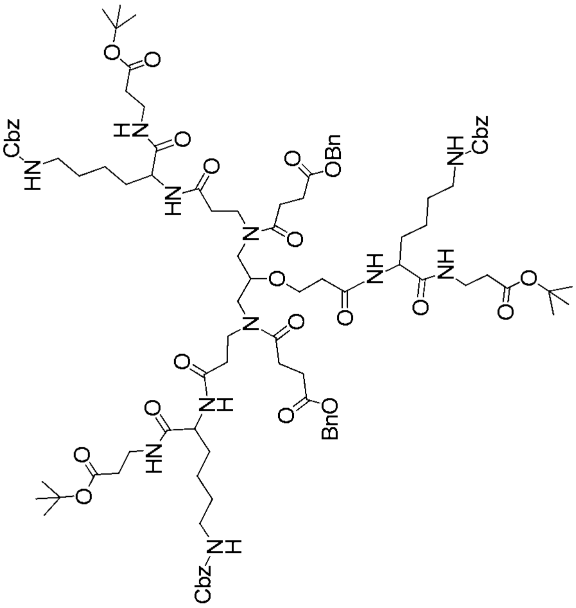 | 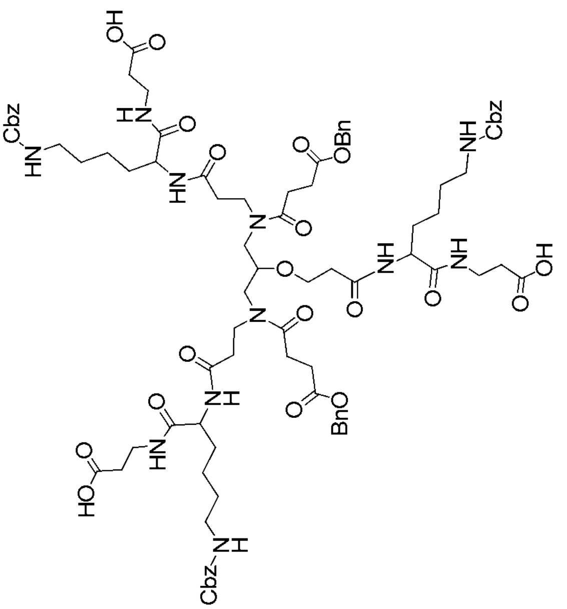 |
| **71-264** | **71-274** |

(continued)

| | |
|---|---|
| 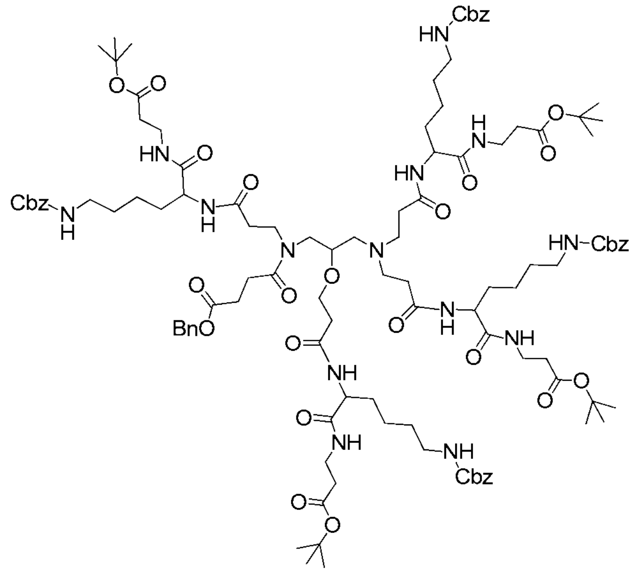 | 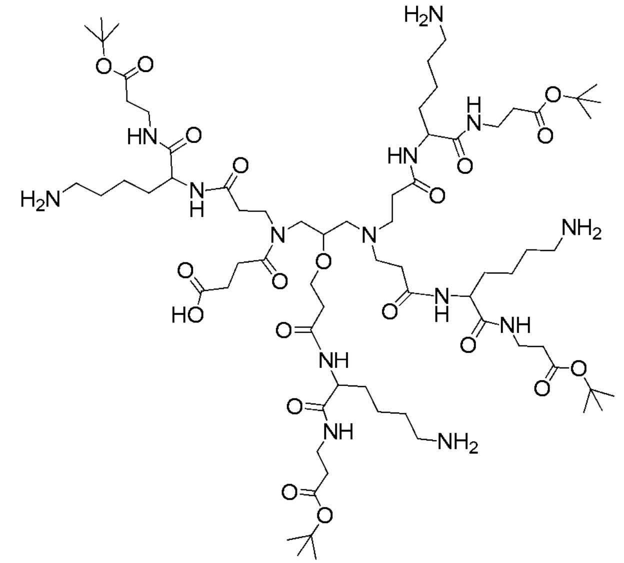 |
| **77-135** | **77-144** |
| 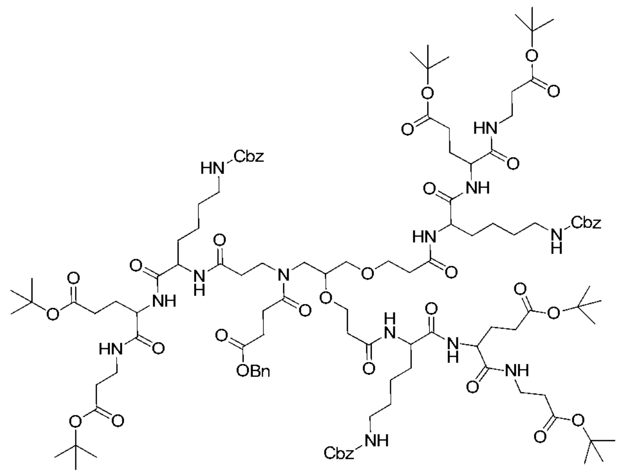 | 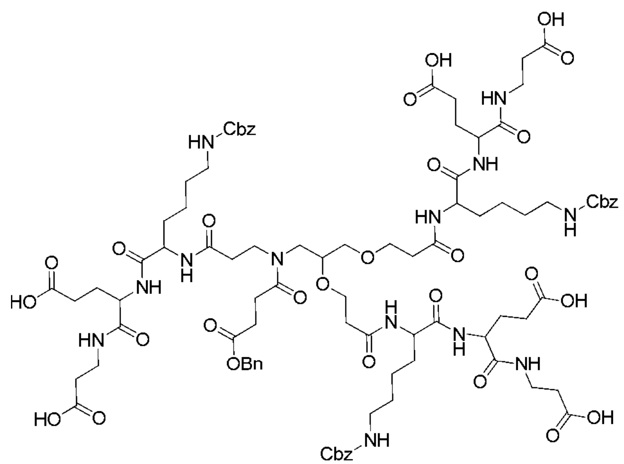 |
| **84-38** | **84-64** |

(continued)

| | |
|---|---|
| 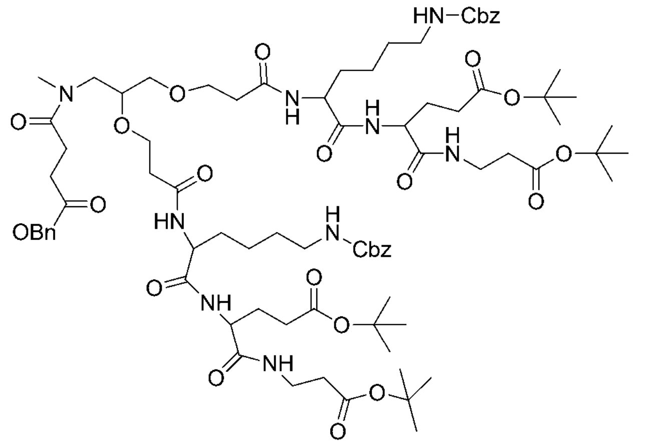 | 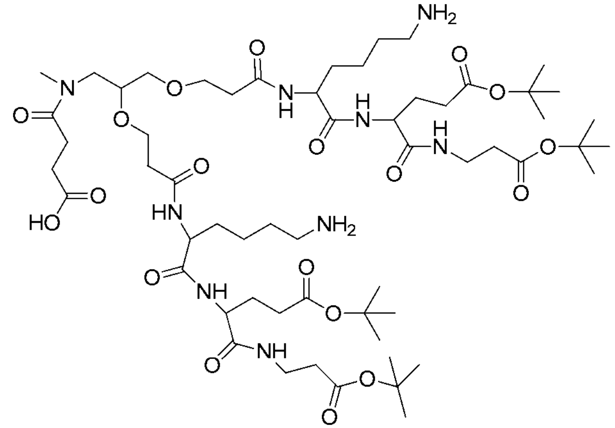 |
| **88-149** | **88-165** |
| 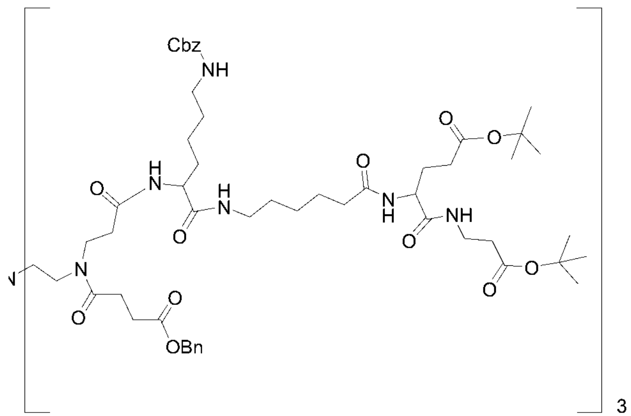 | 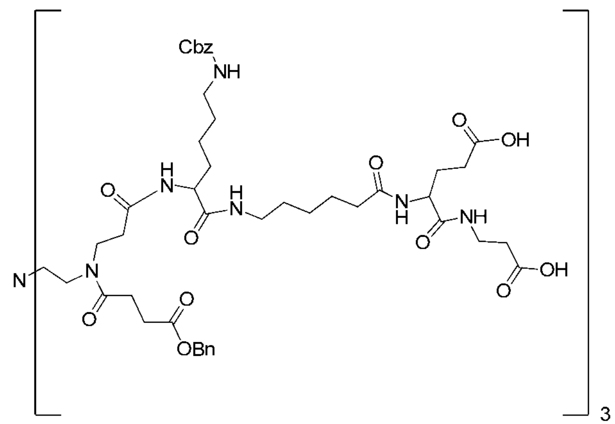 |
| **76-203** | **76-231** |

(continued)

| | |
|---|---|
| 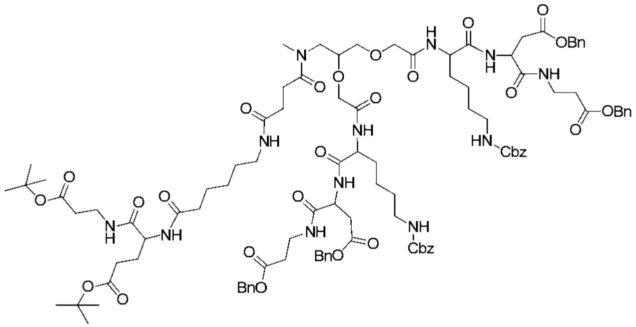 | 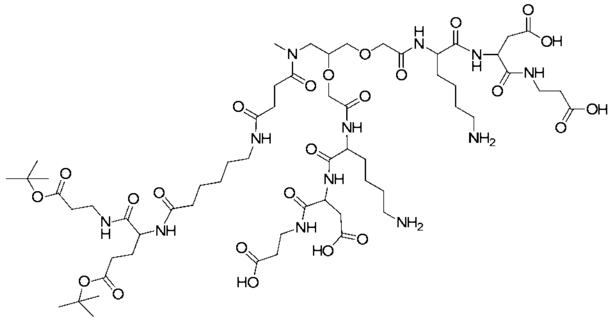 |
| **72-217** 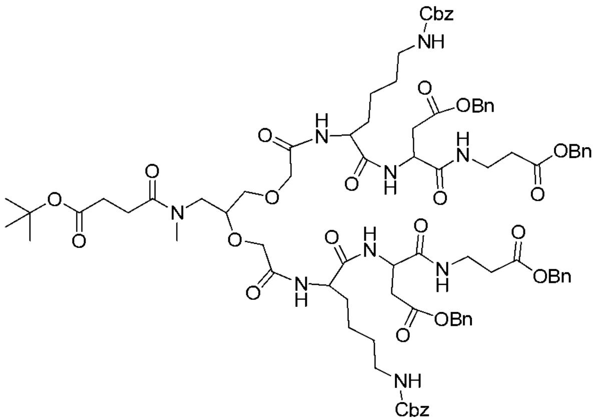 | **72-218** 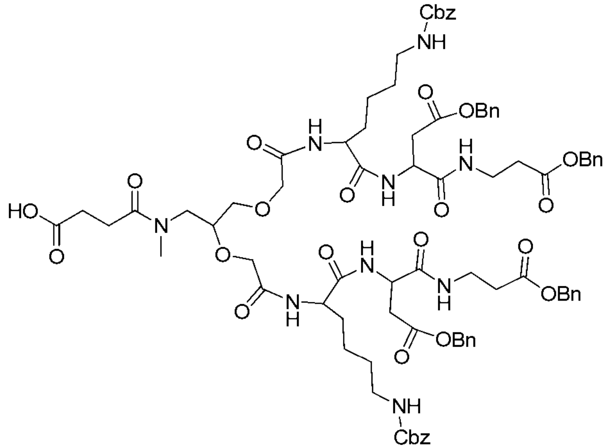 |
| **72-226** | |

(continued)

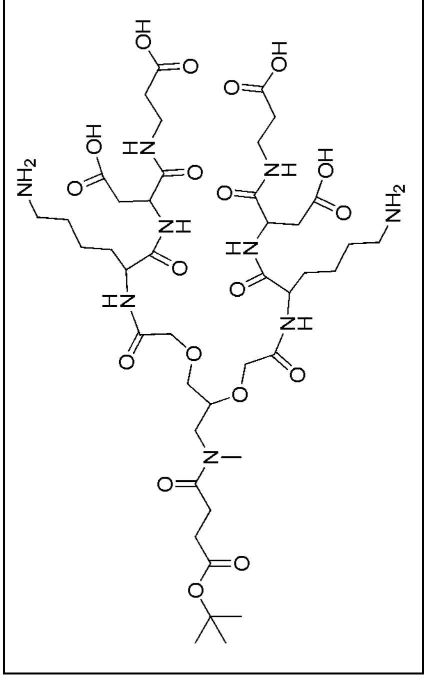

**23.** A compound represented by formula X or a pharmaceutically acceptable salt thereof,

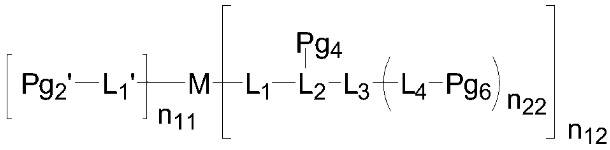

X

wherein each group is as defined in any one of claims 1 to 22.

**24.** The compound or pharmaceutically acceptable salt thereof according to claim 23, wherein the compound is selected from the group consisting of:

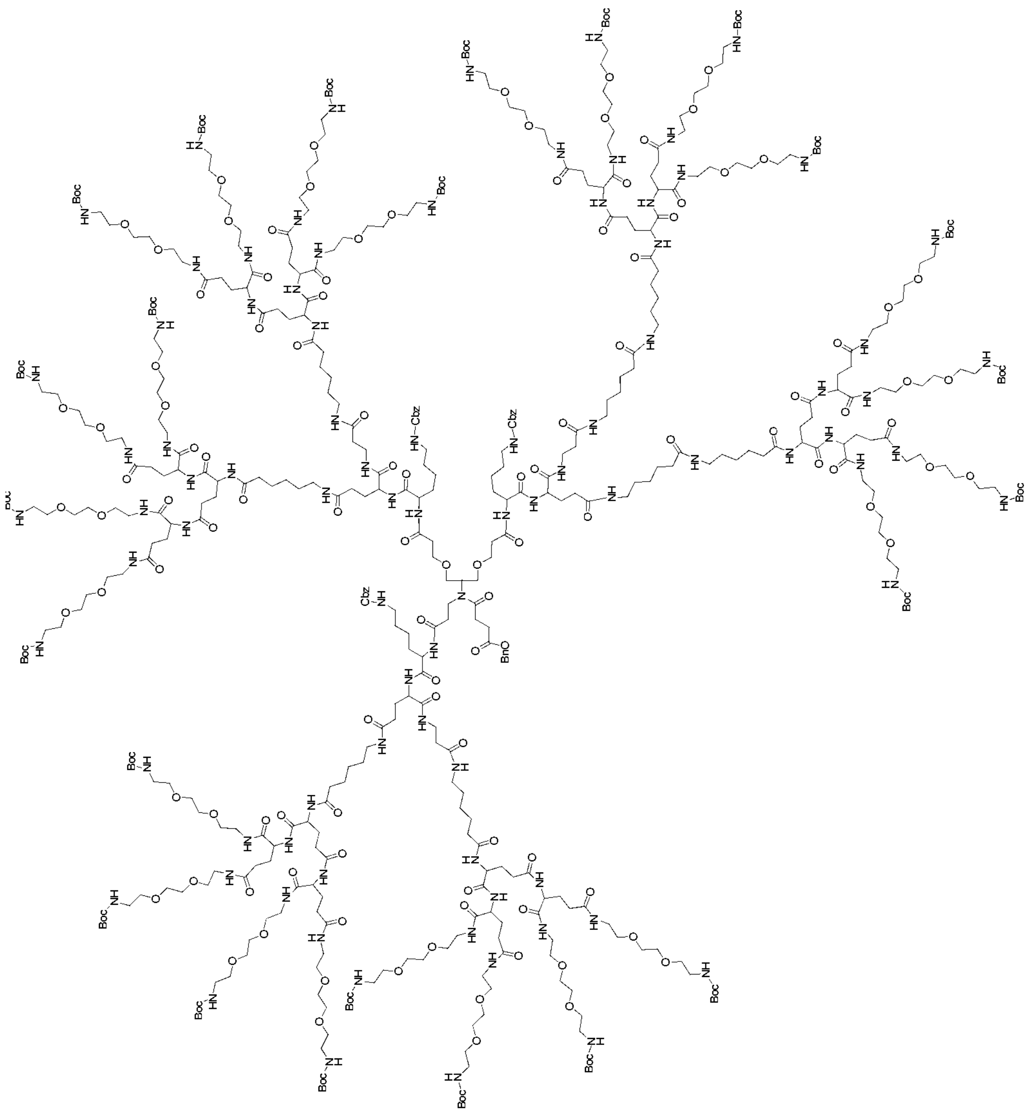

74-215

(continued)

| | |
|---|---|
| 74-216 | 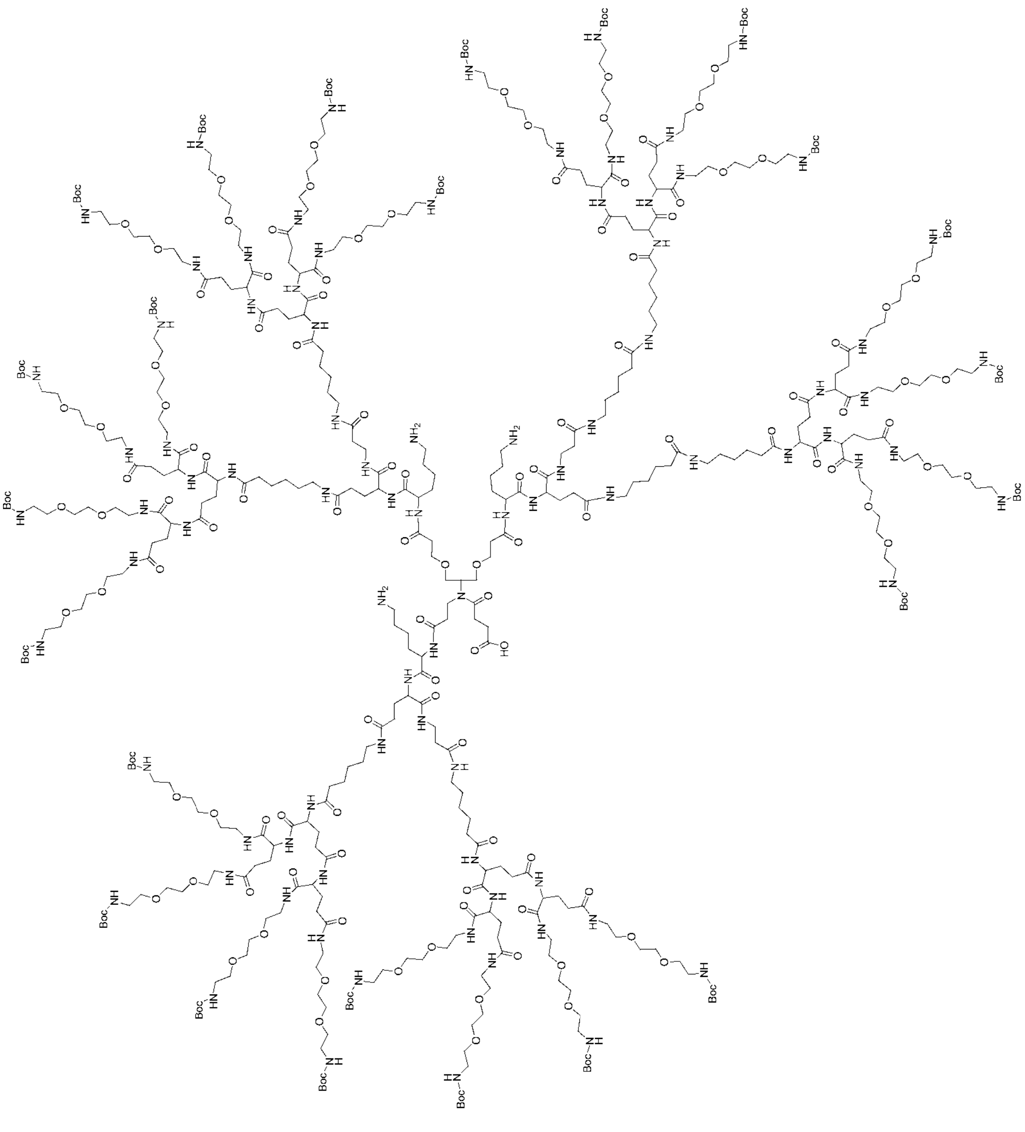 |

(continued)

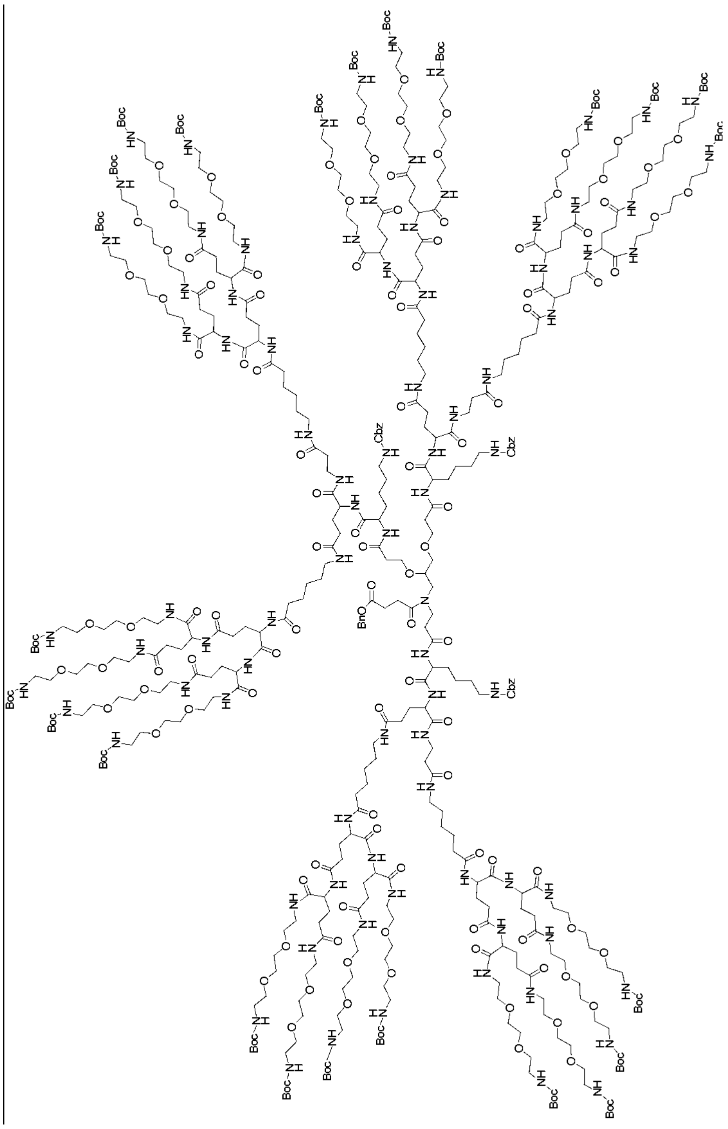

77-146

(continued)

| | |
|---|---|
| **77-149** | 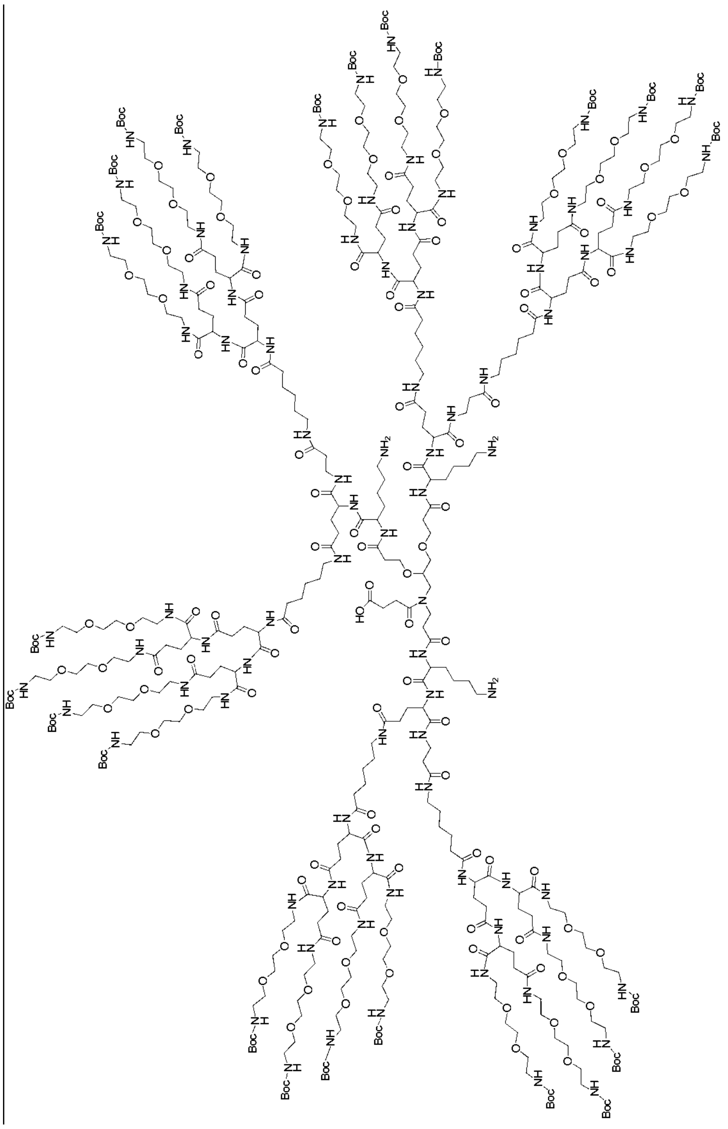 |

(continued)

| | |
|---|---|
| 88-168 | 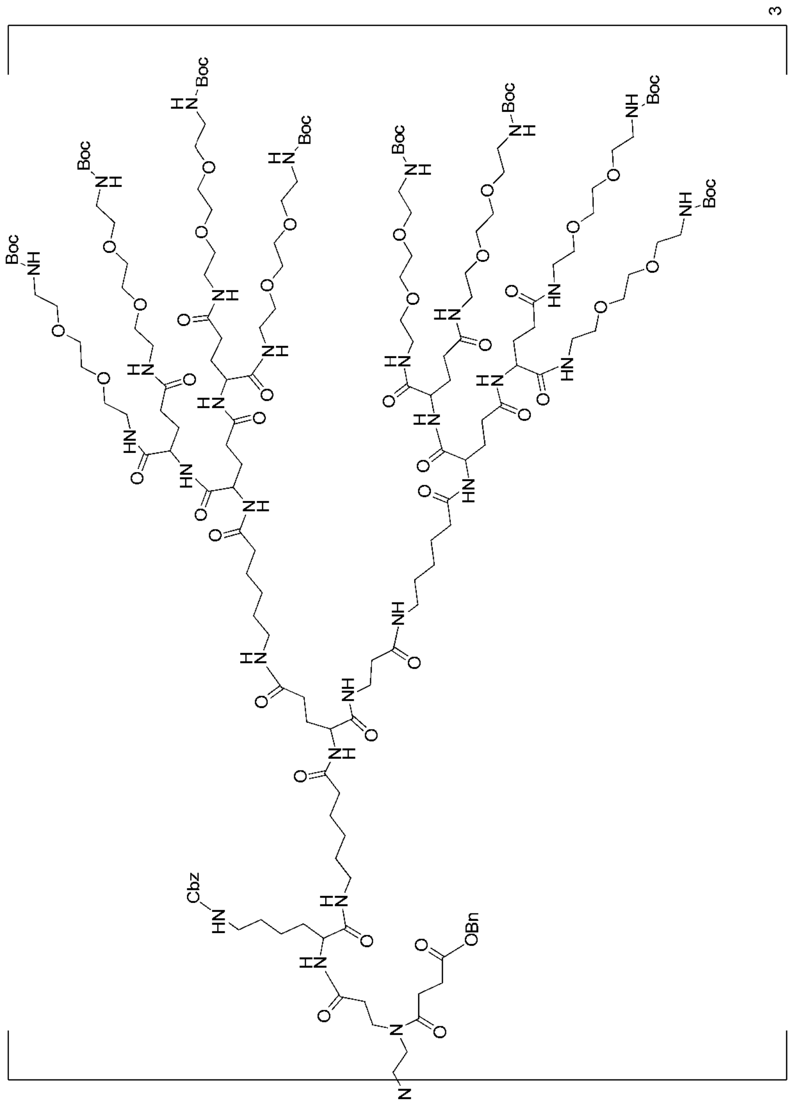 |

(continued)

| | |
|---|---|
| 88-169 | 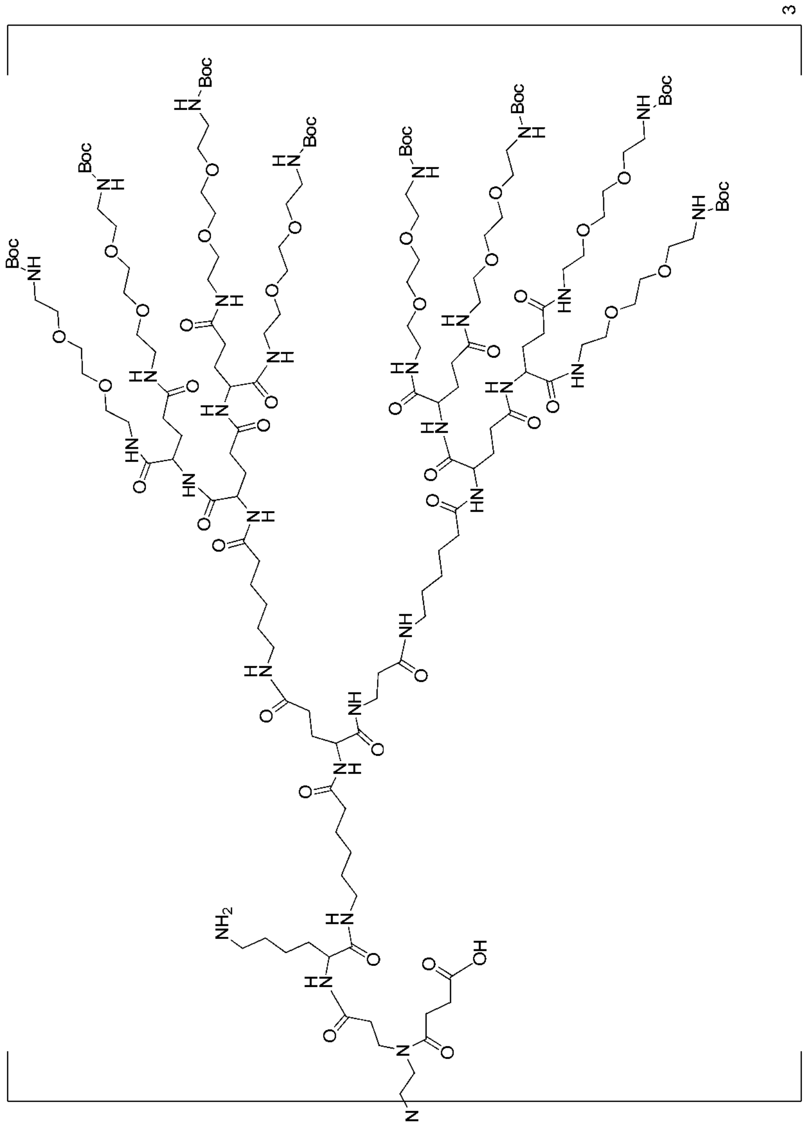 |

**25.** A compound represented by formula XI or a pharmaceutically acceptable salt thereof,

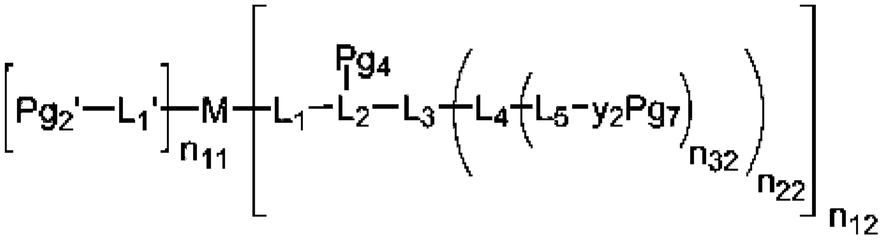

XI

wherein each group is as defined in any one of claims 1 to 24.

**26.** The compound or pharmaceutically acceptable salt thereof according to claim 25, wherein the compound is selected from the group consisting of:

| | |
|---|---|
| **85-80** | 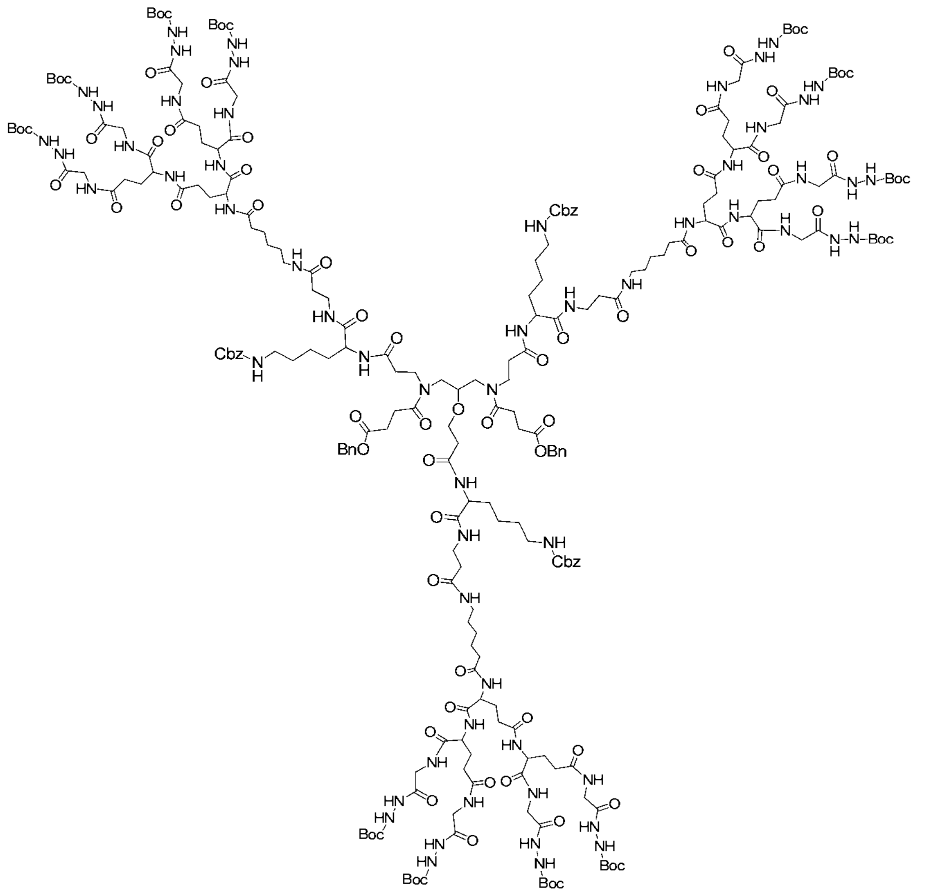 |

(continued)

| | |
|---|---|
| **85-82** | 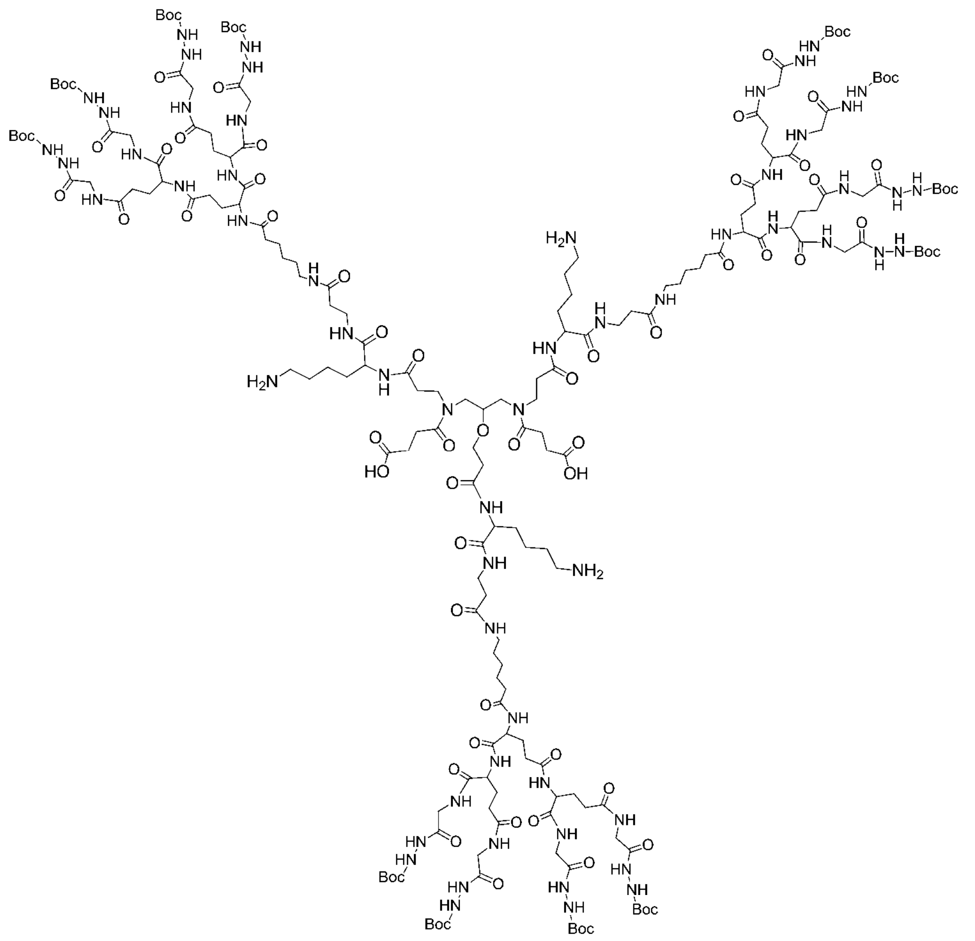 |

**27.** A compound represented by formula XII or a pharmaceutically acceptable salt thereof,

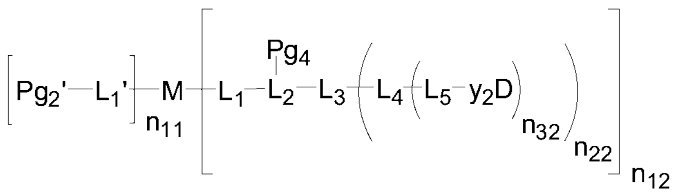

XII

wherein each group is as defined in any one of claims 1 to 26.

**28.** The compound or pharmaceutically acceptable salt thereof according to claim 27, wherein the compound is selected from the group consisting of:

➽Too large Table

| | |
|---|---|
| **81-204** | 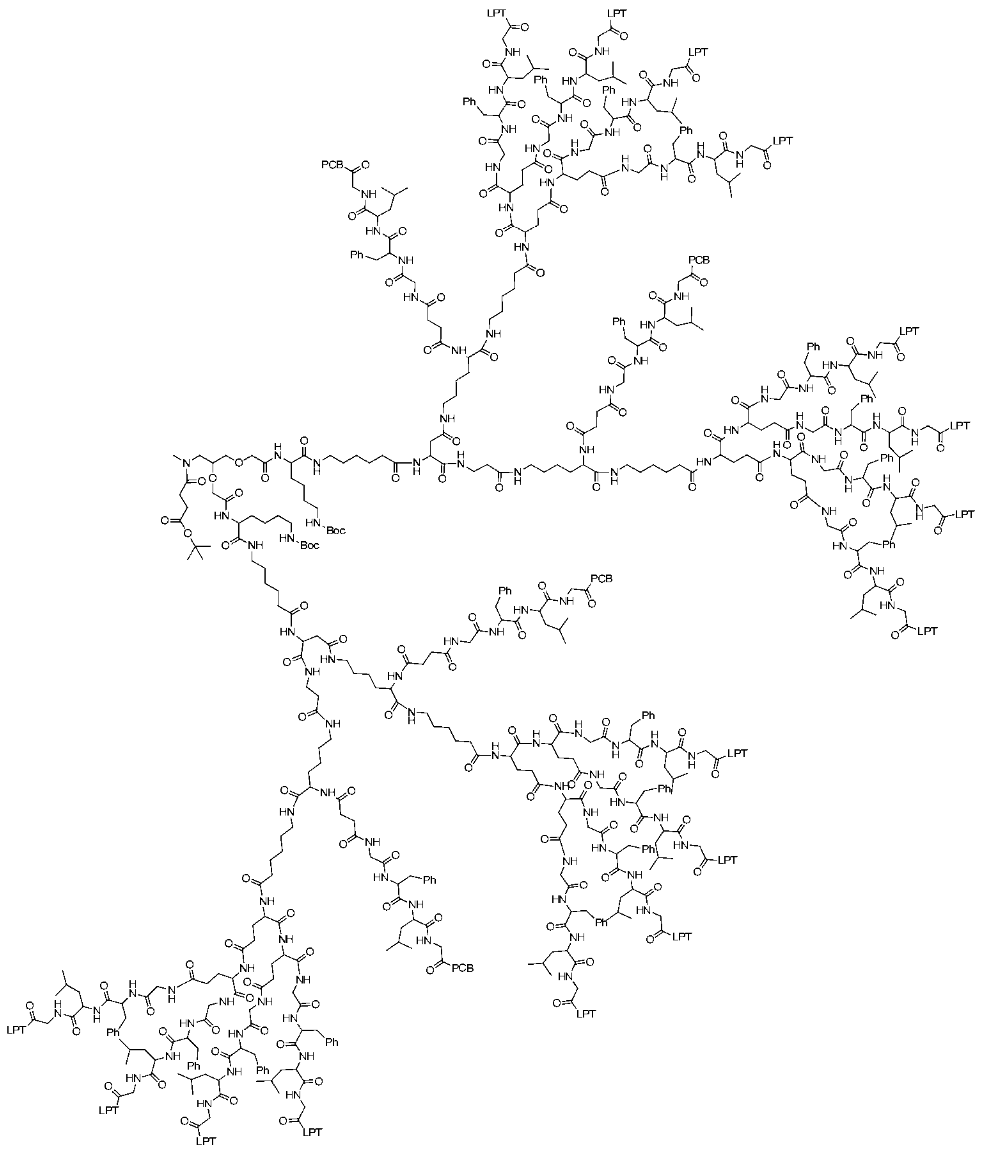 |

(continued)

| | |
|---|---|
| **81-205** | 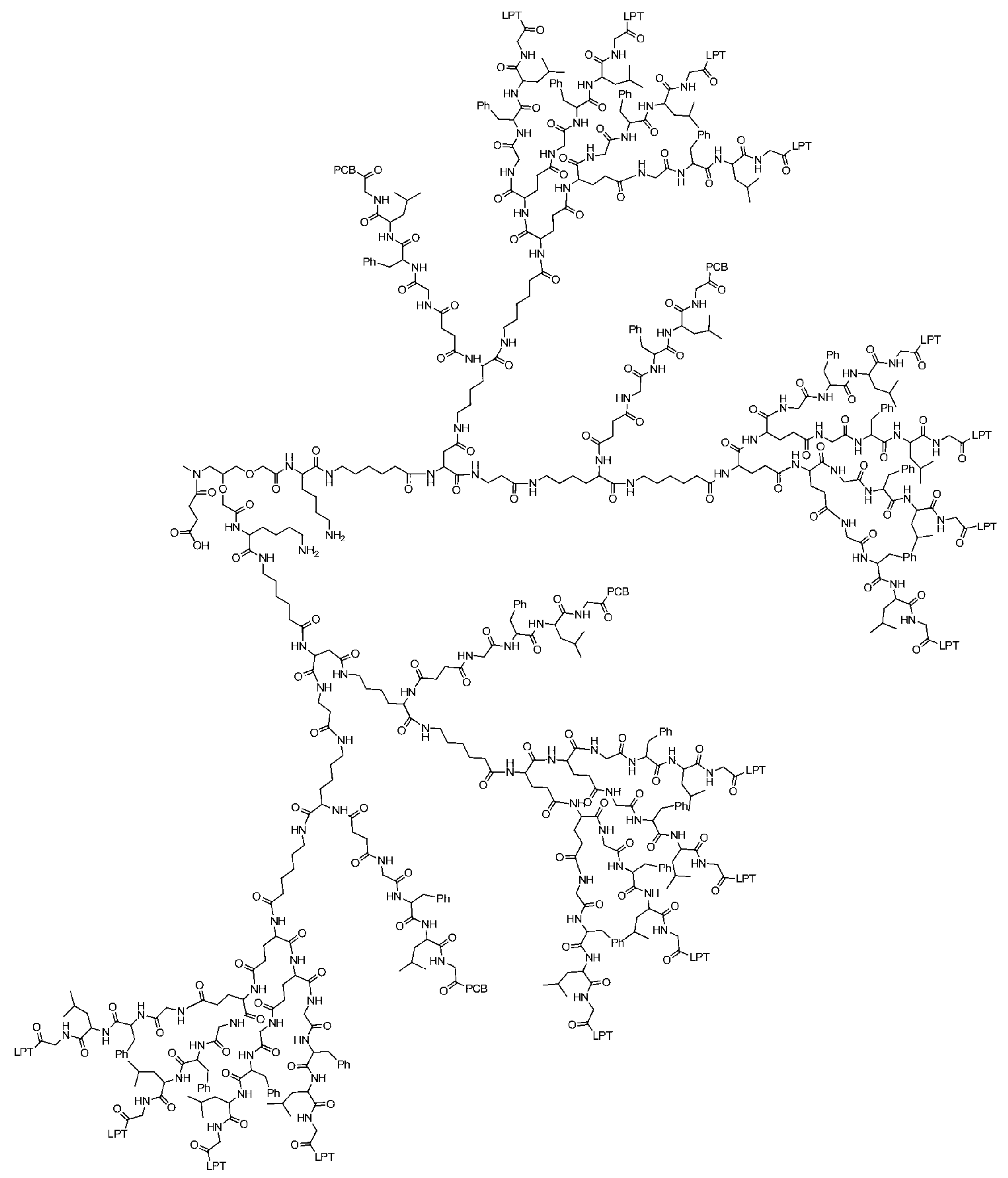 |

**29.** A compound represented by formula XIII or a pharmaceutically acceptable salt thereof,

$$\left[{}_{n_{21}}Pg_3'\text{-}L_3\text{-}L_1'\right]_{n_{11}}\!\!-M\!-\!\left[L_1\text{-}Pg_2\right]_{n_{12}}$$

XIII

wherein each group is as defined in any one of claims 1 to 28.

**30.** The compound or pharmaceutically acceptable salt thereof according to claim 29, wherein the compound is selected from the group consisting of:

| | |
|---|---|
| 76-201 | 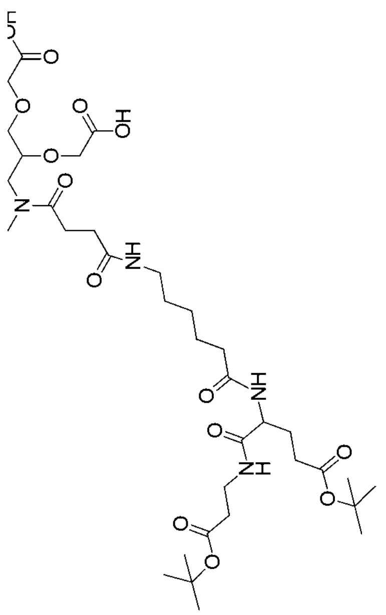 |
| 76-200 | 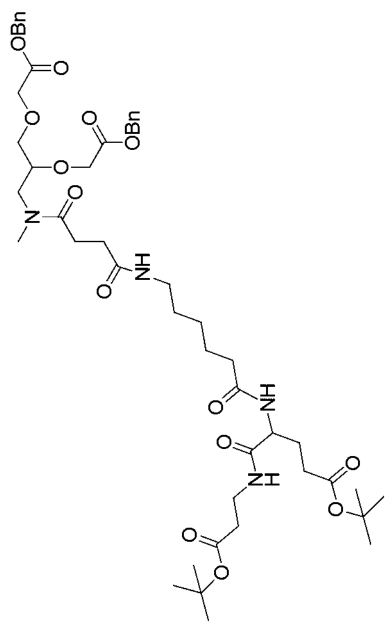 |
| 88-163 | 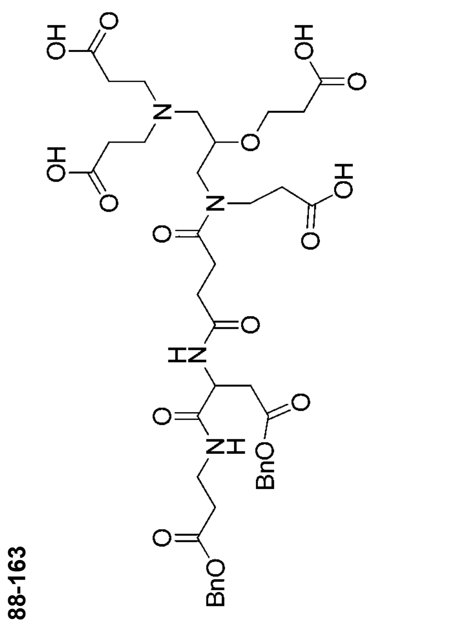 |
| 88-138 | 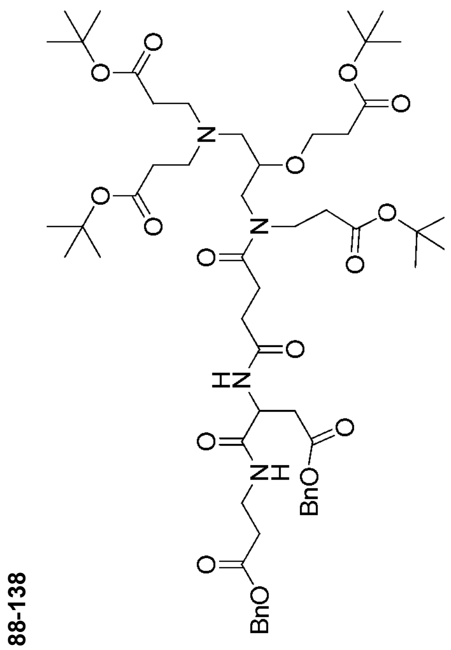 |

**31.** A compound represented by formula XIV or a pharmaceutically acceptable salt thereof,

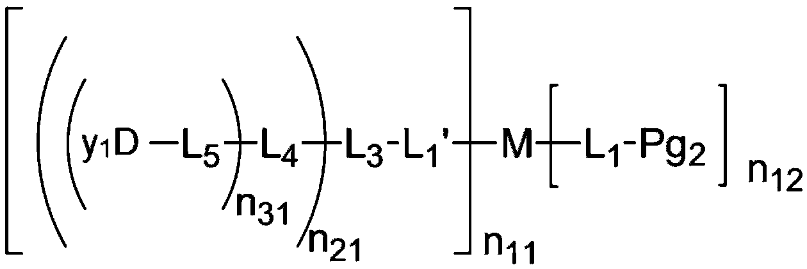

XIV

wherein each group is as defined in any one of claims 1 to 30.

**32.** The compound or pharmaceutically acceptable salt thereof according to claim 31, wherein the compound is selected from the group consisting of:

| | |
|---|---|
| **76-156** | 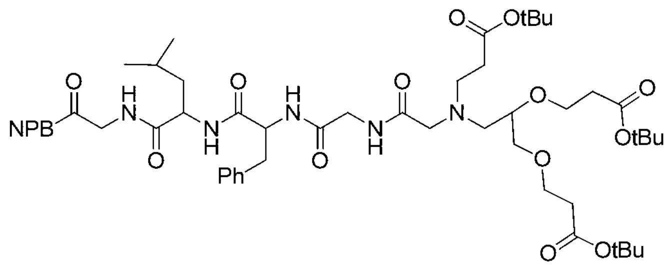 |
| **76-170** | 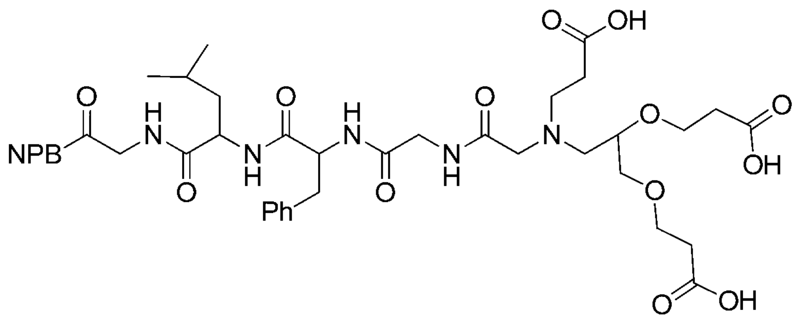 |
| **82-185** | 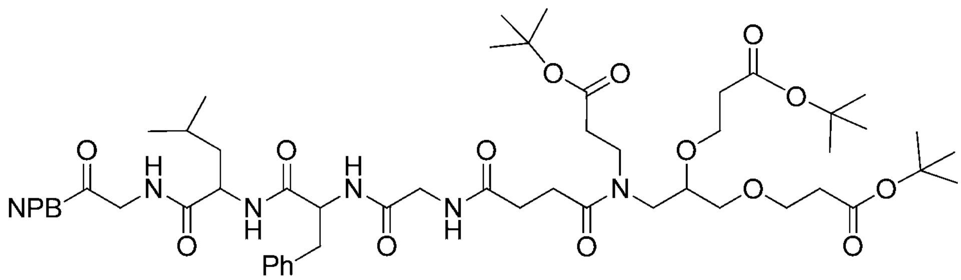 |
| **82-196** | 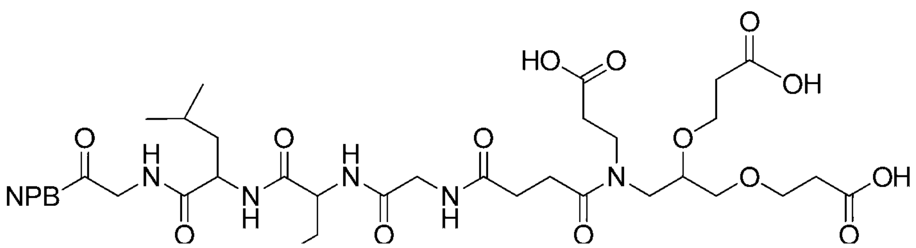 |

**33.** A compound represented by formula XV or a pharmaceutically acceptable salt thereof,

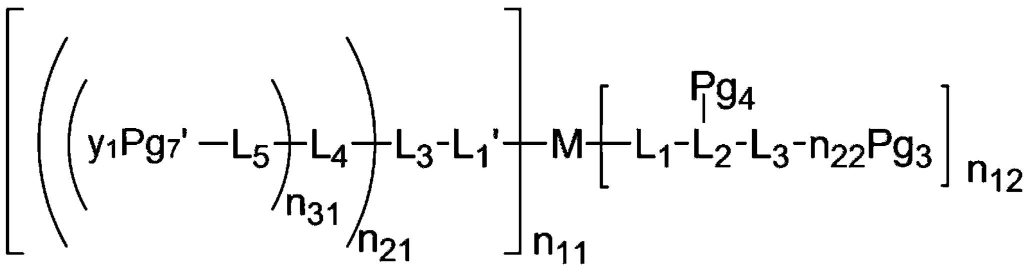

XV

wherein, $Pg_7$' is hydrogen or selected from the group consisting of an amino protecting group and a carboxyl protecting group; preferably, the amino protecting group is selected from the group consisting of an alkyl-based protecting group (e.g., Bn, Trt, DMB, or PMB) and an alkoxycarbonyl-based protecting group (e.g., Boc, Fmoc, Cbz, or Teoc), and the carboxyl protecting group is selected from the group consisting of an ester-based protecting group (e.g., methyl ester, ethyl ester, tert-butyl ester, allyl ester, or benzyl ester);
the remaining groups are as defined in any one of claims 1 to 32.

**34.** The compound or pharmaceutically acceptable salt thereof according to claim 33, wherein the compound is selected from the group consisting of:

➽Too large Table

| | |
|---|---|
| **72-219** | 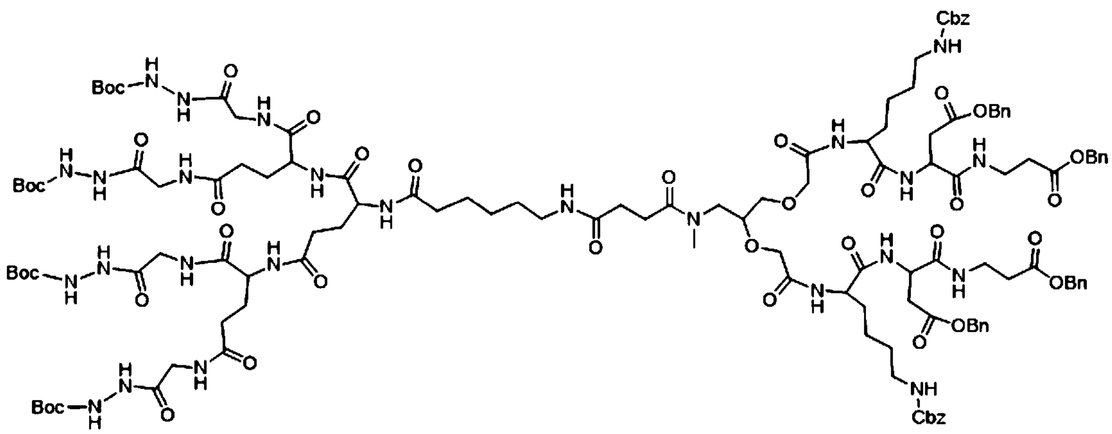 |
| **72-220** | 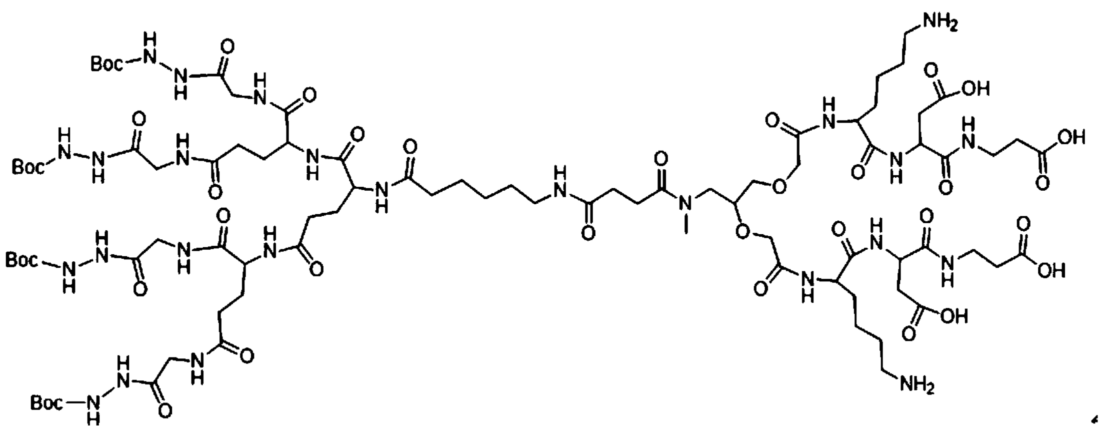 |

**35.** A compound represented by formula XVI or a pharmaceutically acceptable salt thereof,

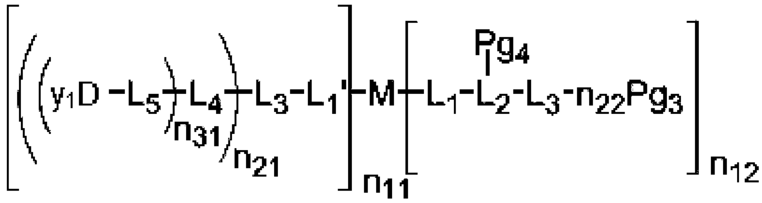

XVI

wherein each group is as defined in any one of claims 1 to 34.

**36.** The compound or pharmaceutically acceptable salt thereof according to claim 35, wherein the compound is selected from the group consisting of:

| | |
|---|---|
| **82-197** | 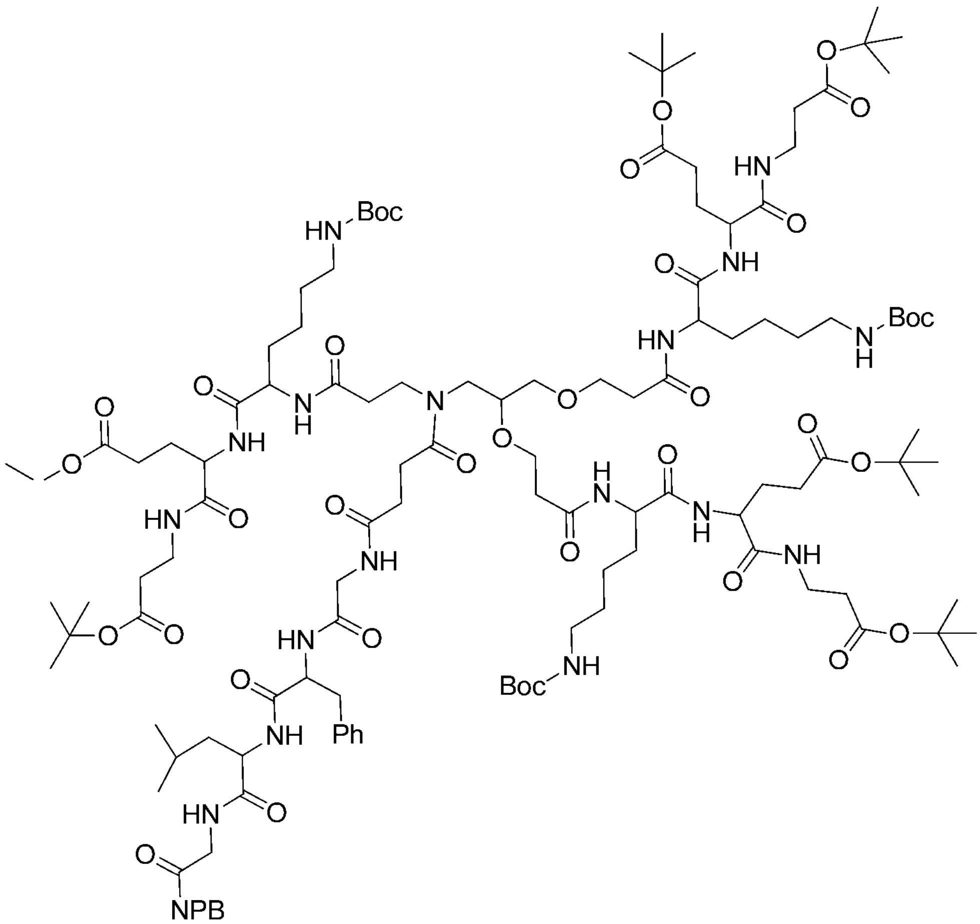 |

(continued)

| | |
|---|---|
| **82-200** | 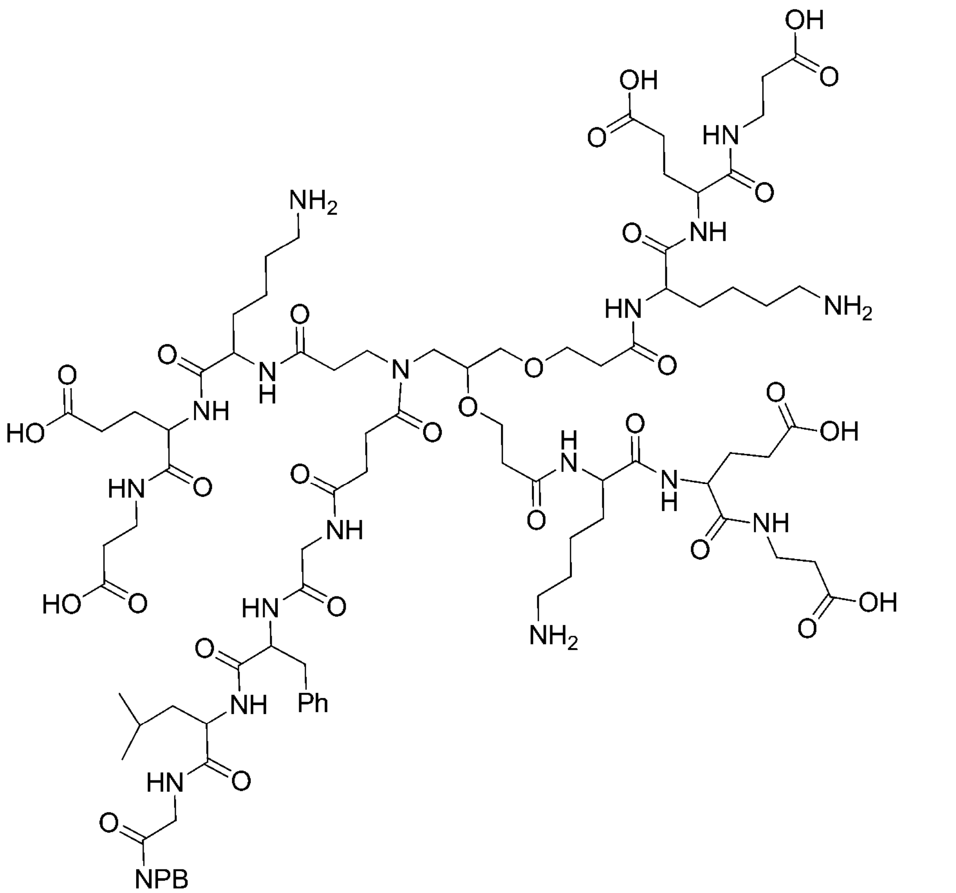 |

**37.** A compound represented by formula XVII or a pharmaceutically acceptable salt thereof,

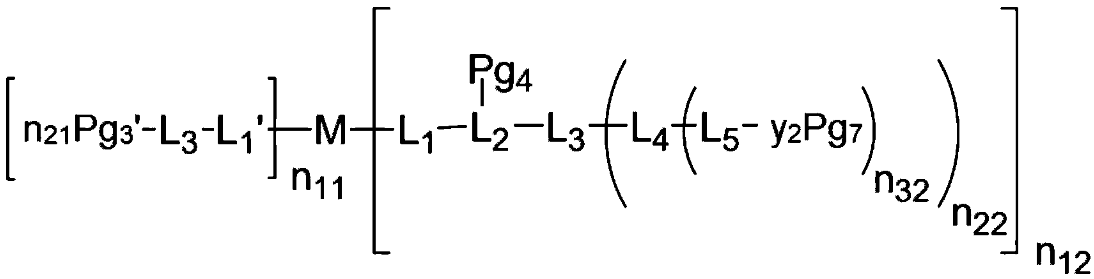

XVII

wherein each $Pg_3$' is independently hydrogen or selected from the group consisting of an amino protecting group and a carboxyl protecting group, the amino protecting group is selected from the group consisting of an alkoxycarbonyl-based protecting group, such as Boc, Fmoc, Cbz, or Teoc; the carboxyl protecting group is selected from the group consisting of an ester-based protecting group, such as methyl ester, ethyl ester, tert-butyl ester, allyl ester, or benzyl ester; preferably, each $Pg_3$ is independently hydrogen or a carboxyl protecting group, such as an ester-based protecting group, such as methyl ester, ethyl ester, tert-butyl ester, allyl ester, or benzyl ester, preferably tert-butyl ester or benzyl ester; preferably, each $Pg_3$ is the same, preferably all are tert-butyl ester or benzyl ester;

the remaining groups are as defined in any one of claims 1 to 36.

**38.** The compound or pharmaceutically acceptable salt thereof according to claims 37, wherein the compound is selected from the group consisting of:

➽Too large Table

| | |
|---|---|
| 88-166 | 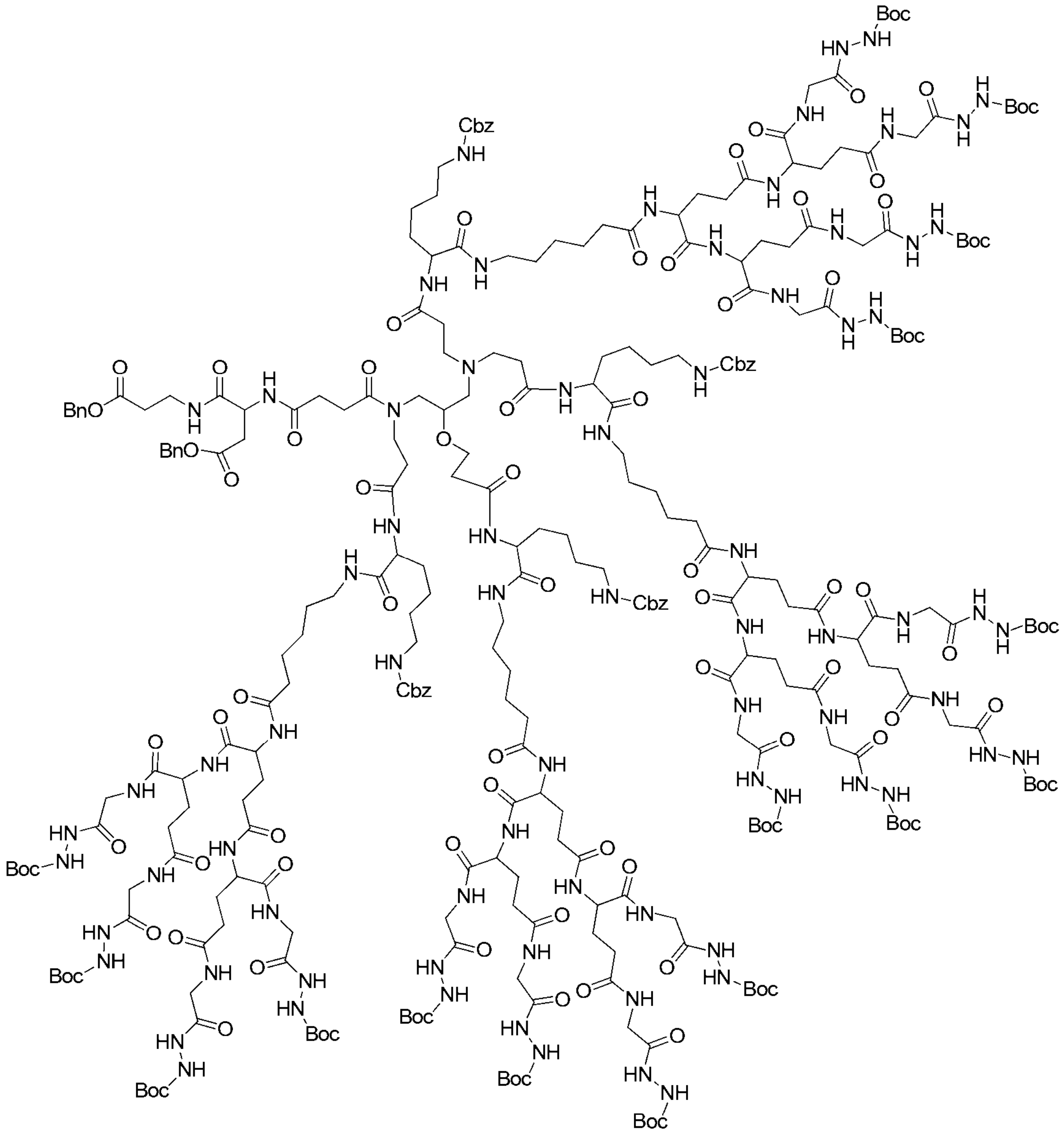 |

(continued)

| | |
|---|---|
| **88-211** | 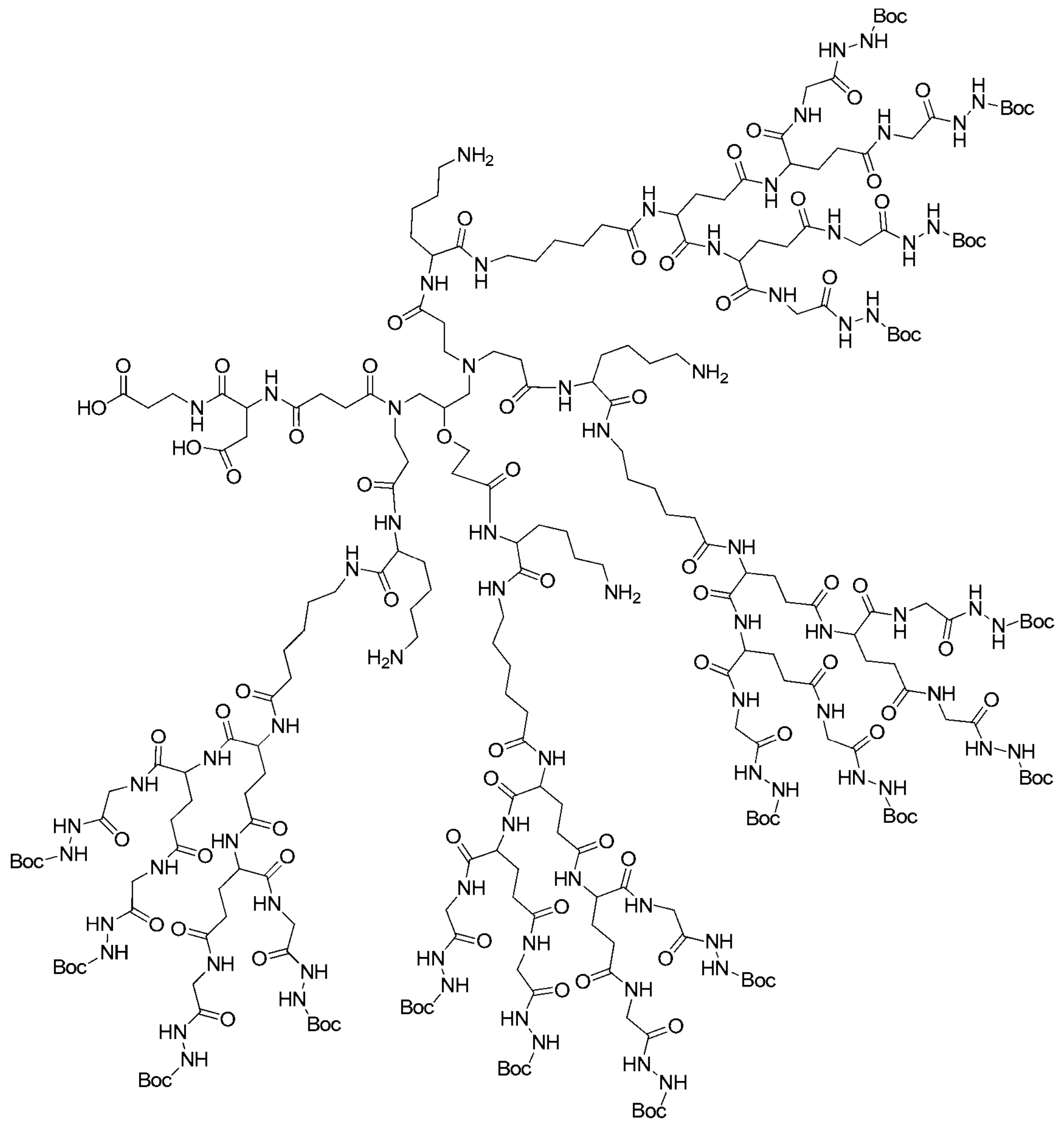 |

**39.** A compound represented by formula XVIII or a pharmaceutically acceptable salt thereof,

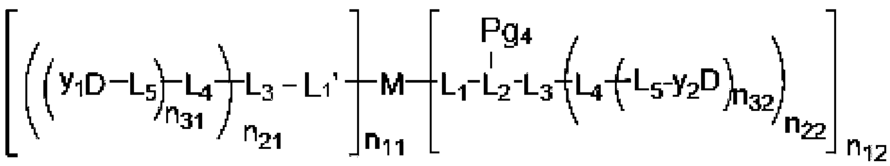

XVIII

wherein each group is as defined in any one of claims 1 to 38.

**40.** The compound or pharmaceutically acceptable salt thereof according to claim 39, wherein the compound is selected from the group consisting of:

| | |
|---|---|
| 76-171 | 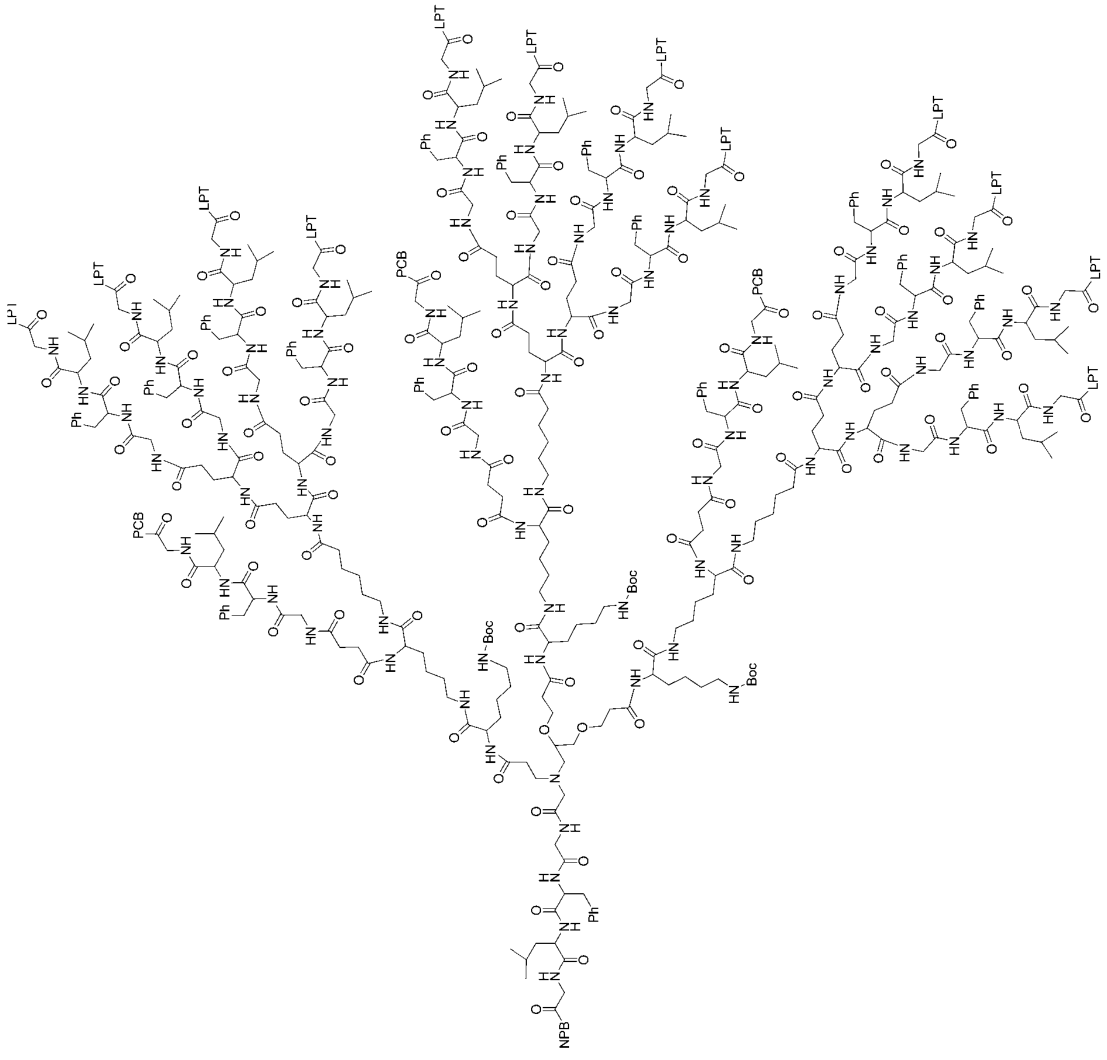 |

(continued)

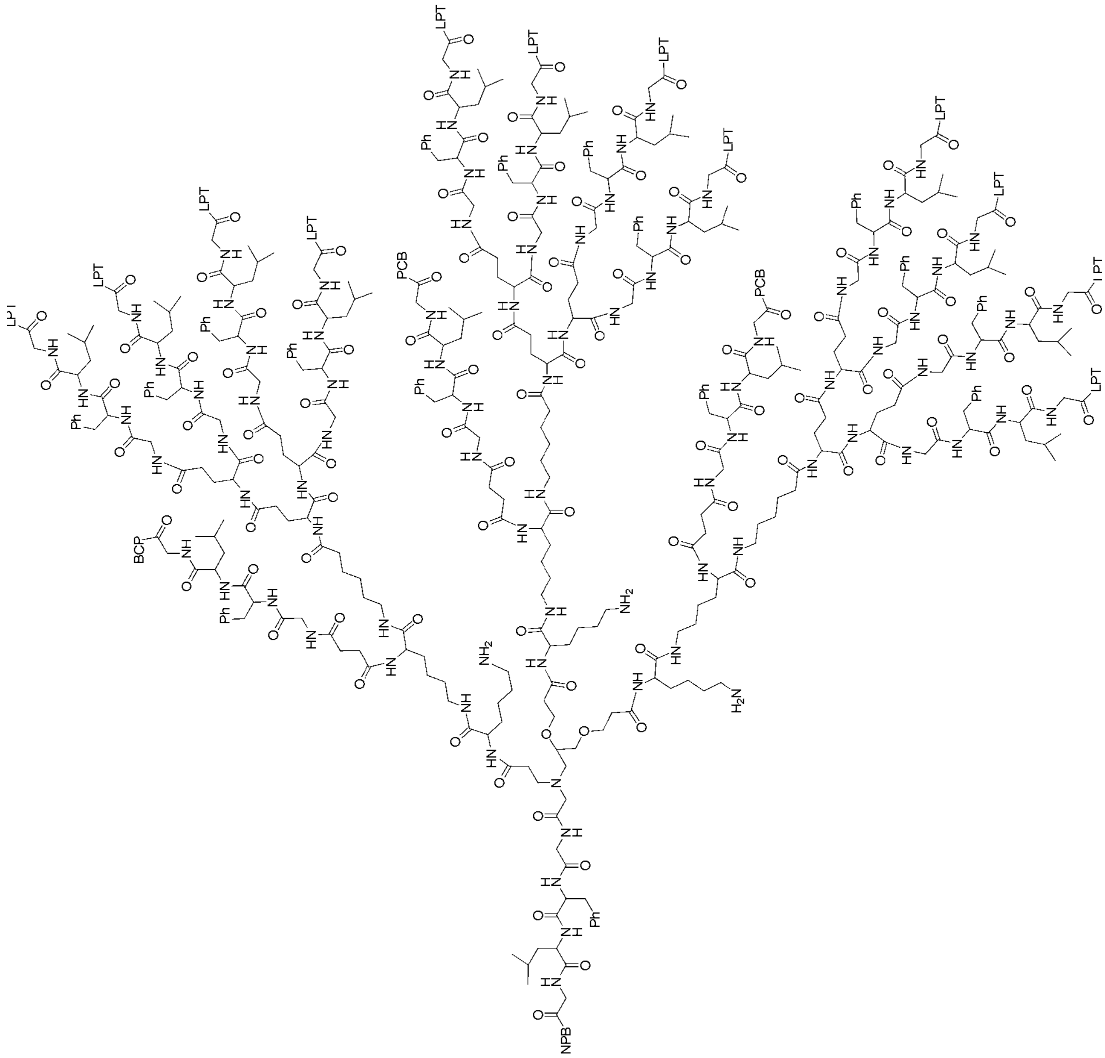

76-177

**41.** A compound represented by formula XIX or a pharmaceutically acceptable salt thereof,

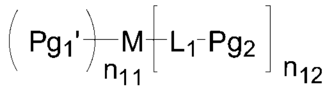

XIX

wherein each group is as defined in any one of claims 1 to 40.

**42.** The compound or pharmaceutically acceptable salt thereof according to claim 41, wherein the compound is selected from the group consisting of:

| **76-84** | **78-50** |
|---|---|
| 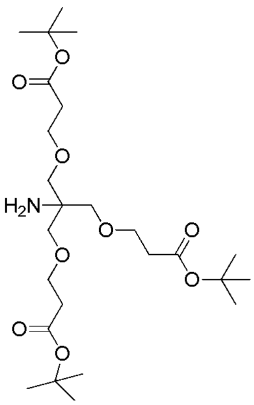 | 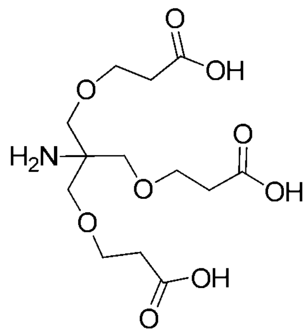 |
| **71-222** | **69-237** |
| 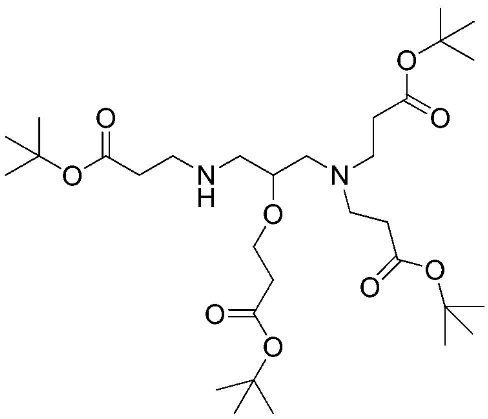 | 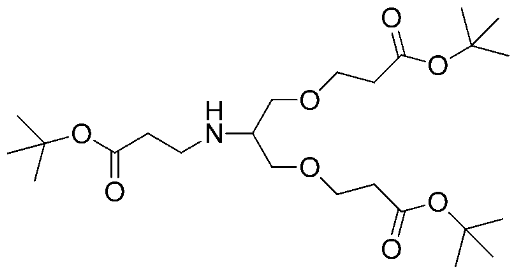 |
| **70-173** | **75-213** |
| 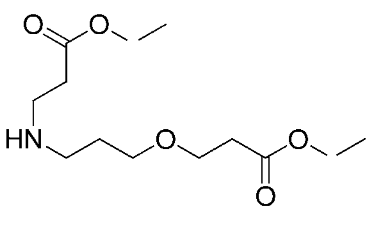 | 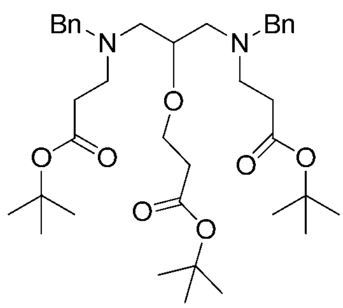 |
| **75-214** | **82-49** |

(continued)

| | |
|---|---|
| 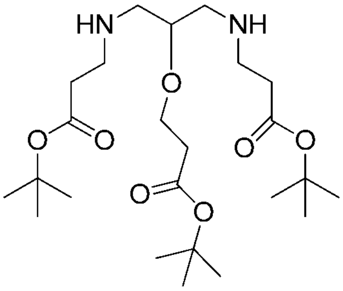 | 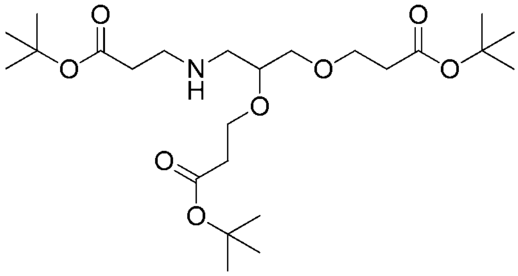 |
| **84-30**<br>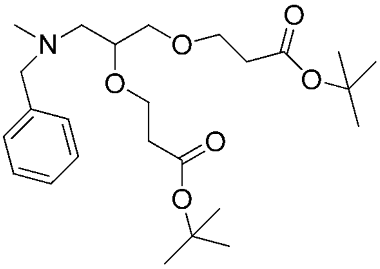 | **84-31** |
| | 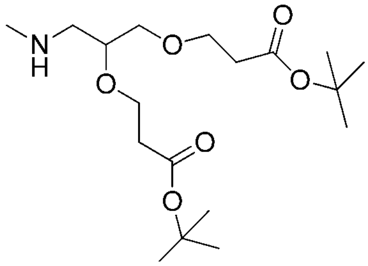 |
| **88-91**<br>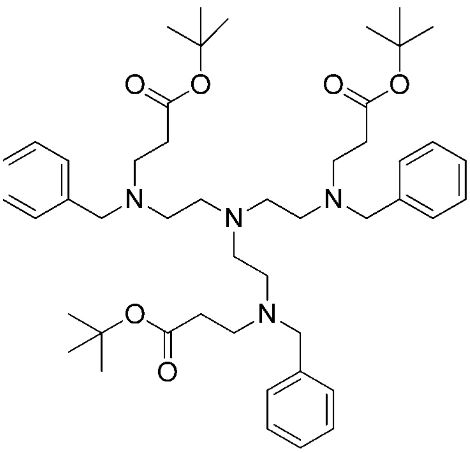 | **88-92**<br>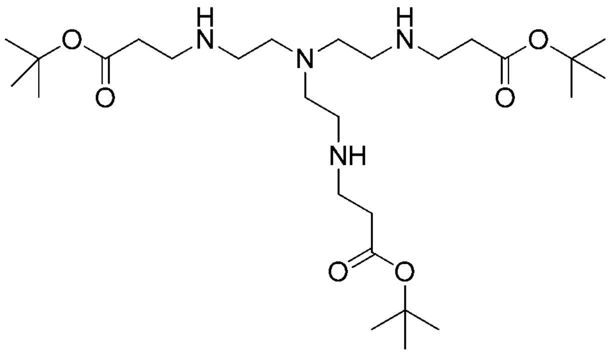 |
| **82-50**<br>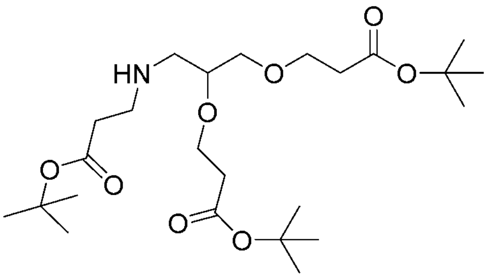 | **88-127**<br>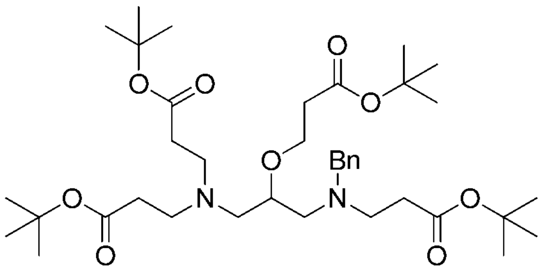 |

(continued)

| | |
|---|---|
| **88-137** 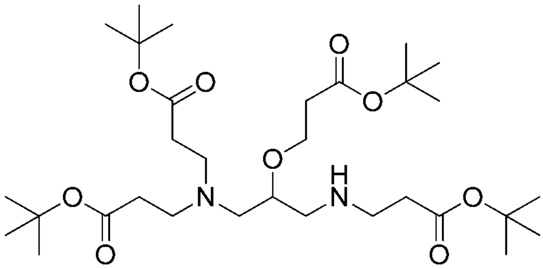 | |

**43.** A compound represented by formula XX or a pharmaceutically acceptable salt thereof,

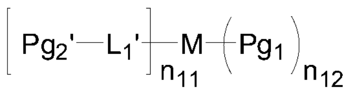

XX

wherein each group is as defined in any one of claims 1 to 42.

**44.** The compound or pharmaceutically acceptable salt thereof according to claim 43, wherein the compound is selected from the group consisting of:

| | |
|---|---|
| **81-60** 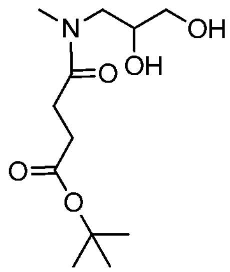 | **59-224** 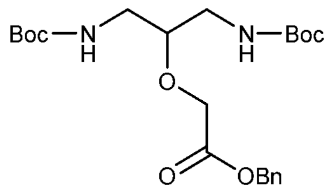 |
| **59-230** 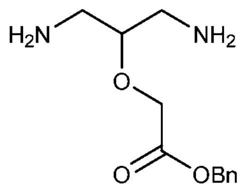 | |

**45.** A pharmaceutical composition, which comprises the compound or pharmaceutically acceptable salt thereof according to any one of claims 1 to 8 in an amount effective for treating and/or preventing a disease;

preferably, the composition further comprises one or more pharmaceutically acceptable excipients;
preferably, the pharmaceutical composition is formulated as an injectable formulation.

**46.** An injectable solution, which comprises the compound or pharmaceutically acceptable salt thereof according to any one of claims 1 to 8, or the pharmaceutical composition according to claim 45;
preferably, the injectable solution uses physiological saline as a carrier.

**47.** Use of the compound or pharmaceutically acceptable salt thereof according to any one of claims 1 to 8 in the manufacture of a medicament for treating and/or preventing a disease (e.g., a cancer);

preferably, the cancer is selected from the group consisting of colon cancer, leukemia, lymphoma, bladder cancer, bone cancer, brain tumor, medulloblastoma, glioma, breast cancer, adenoma/carcinoid, adrenocortical carcinoma, islet cell carcinoma, cervical cancer, endometrial cancer, ovarian cancer, colorectal cancer, skin cancer, esophageal cancer, eye cancer, gallbladder cancer, gastric cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid gland cancer, penile cancer, prostate cancer, urethral cancer, vaginal cancer, vulvar cancer, anal cancer, sarcoma, and metastases of the cancer.

**48.** Use of the compound or pharmaceutically acceptable salt thereof according to any one of claims 9 to 44 in the manufacture of a medicament, preferably, the medicament is selected from the group consisting of the compound or pharmaceutically acceptable salt thereof according to any one of claims 1 to 8.

**49.** A method for preparing the compound represented by formula I according to any one of claims 1 to 8, which is selected from the group consisting of the following routes:

(1) reacting the compound represented by formula XII with PEG connected with an activating group to obtain Intermediate 1-1;
(2) reacting Intermediate 1-1 obtained in step (1) with

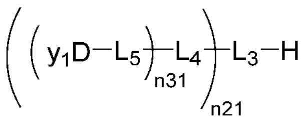

to obtain the compound represented by formula I;
Route 2:

(1) reacting the compound represented by formula IX with PEG connected with an activating group to obtain Intermediate 2-1;
(2) reacting Intermediate 2-1 obtained in step (1) directly with

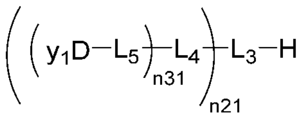

,

or connecting step-by-step the fragments of

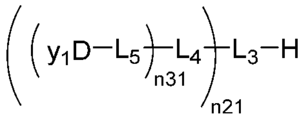

(e.g., connecting step-by-step $L_3$, $L_4$, $L_5$ and D, or $L_3$-$(L_4)_{n21}$, $L_5$ and D, or $L_3$-$(L_4)_{n21}$, $L_5$-$y_1$D, or $L_3$-$(L_4$-$(L_5)_{n31})_{n21}$ and D) to obtain Intermediate 2-2;
(3) reacting Intermediate 2-2 obtained in step (2) directly with the compound represented by formula VI, or reacting step-by-step with the compound represented by formula IV and the cytotoxic drug, or connecting step-by-step $L_4$, $L_5$-$y_2$D, or connecting step-by-step $L_4$, $L_5$ and D, to obtain the compound represented by formula I;

or, the order of steps (2) and (3) is exchanged;
Route 3:

(1) reacting Intermediate 2-1 obtained in step (1) of Route 2 with the compound represented by formula VI to obtain Intermediate 3-1;
(2) reacting Intermediate 3-1 obtained in step (1) with

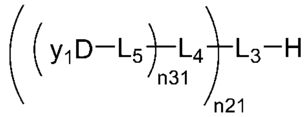

to obtain the compound represented by formula I;

Route 4:

(1) reacting the compound represented by formula X with PEG connected with an activating group to obtain Intermediate 4-1;

(2) reacting Intermediate 4-1 obtained in step (1) with

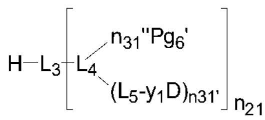

to obtain Intermediate 4-2, wherein $n_{31}'+n_{31}''=n_{31}$;

(3) reacting Intermediate 4-2 obtained in step (2) with $H\text{-}L_5\text{-}y_2D$ to obtain Intermediate 4-3;

(4) reacting Intermediate 4-3 obtained in step (3) with $H\text{-}L_5\text{-}y_1D$ to obtain the compound represented by formula I;

or, replacing

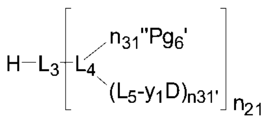

in step (2) with

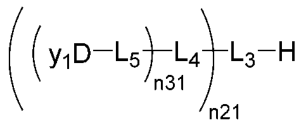,

in which case step (4) is not performed;

or, using the compound represented by formula XII as a raw material, performing steps (1), (2) and (4) to obtain the compound represented by formula I;

Route 5:

(1) reacting the compound represented by formula XI with PEG connected with an activated group to obtain Intermediate 5-1;

(2) reacting Intermediate 5-1 obtained in step (1) directly with

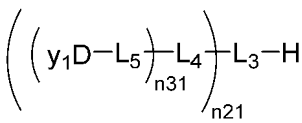,

or connecting step-by-step the fragments of

$$\left(\left(y_1D-L_5\right)_{n31}-L_4\right)_{n21}-L_3-H$$

(e.g., connecting step-by-step $L_3$, $L_4$, $L_5$ and D, or $L_3$-$(L_4)_{n21}$, $L_5$ and D, or $L_3$-$(L_4)_{n21}$ and $L_5$-$y_1$D, or $L_3$-$(L_4$-$(L_5)_{n31})_{n21}$ and D), to obtain Intermediate 5-2;

(3) reacting Intermediate 5-2 obtained in step (2) with the cytotoxic drug to obtain the compound represented by formula I;

Route 6:

(1) reacting the compound represented by formula XIV with the compound represented by formula VII to obtain Intermediate 6-1;

(2) reacting Intermediate 6-1 obtained in step (1) with PEG connected with an activating group to obtain the compound represented by formula I;

Route 7:

(1) reacting the compound represented by formula XIII with PEG connected with an activating group to obtain Intermediate 7-1;

(2) reacting Intermediate 7-1 obtained in step (1) with H-$L_4$ to obtain Intermediate 7-2;

(3) reacting Intermediate 7-2 obtained in step (2) with

$$\left(y_1D-L_5\right)_{n_{31}}-L_4-H$$

to obtain Intermediate 7-3;

(4) reacting Intermediate 7-3 obtained in step (3) with $L_5$-$y_2Pg_7$ or $L_5$'-$y_2Pg_7$ to obtain Intermediate 7-4;

(5) reacting Intermediate 7-4 obtained in step (4) with the cytotoxic drug or $L_5$"-$y_2$D to obtain the compound represented by formula I; wherein $L_5$' and $L_5$" are connected to form the $L_5$;

Route 8:

(1) reacting the compound represented by formula XV with PEG connected with an activating group to obtain Intermediate 8-1;

(2) reacting Intermediate 8-1 obtained in step (1) with the compound represented by formula VI to obtain Intermediate 8-2;

(3) reacting Intermediate 8-2 obtained in step (3) with the cytotoxic drug to obtain the compound represented by formula I;

Route 9:

(1) reacting the compound represented by formula XVII with PEG connected with an activating group to obtain Intermediate 9-1;

(2) reacting Intermediate 9-1 obtained in step (1) with H-$L_5$-$y_1$D to obtain Intermediate 9-2;

(3) reacting Intermediate 9-2 obtained in step (2) with the cytotoxic drug to obtain the compound represented by formula I;

Route 10:

(1) reacting the compound represented by formula XIV with the compound represented by formula III to obtain Intermediate 10-1;

(2) reacting Intermediate 10-1 obtained in step (1) with PEG connected with an activating group to obtain Intermediate 10-2;

(3) reacting Intermediate 10-2 obtained in step (2) with H-$L_4$-$n_{32}Pg_6$' to obtain Intermediate 10-3;

(4) reacting Intermediate 10-3 obtained in step (3) with H-$L_5$-$y_2$D to obtain the compound represented by formula I;

optionally, before or after the reaction of any step of Routes 1 to 10, a step of removal of the protecting group and/or activation (e.g., carbonyl activation) is further included;
preferably, the PEG activating group is preferably

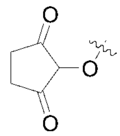
;

the remaining compounds and groups are as defined in any one of claims 1 to 44.

[Figure 1]

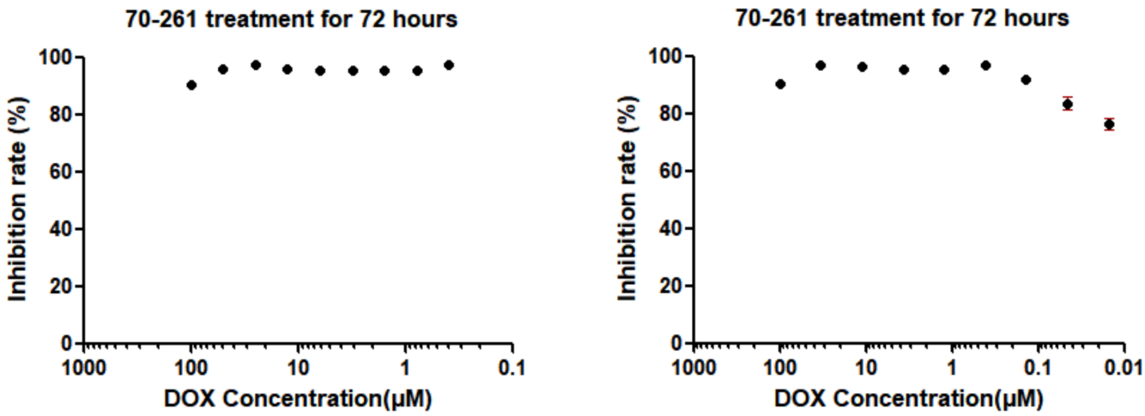


2-Fold dilution 3-Fold dilution

[Figure 2]

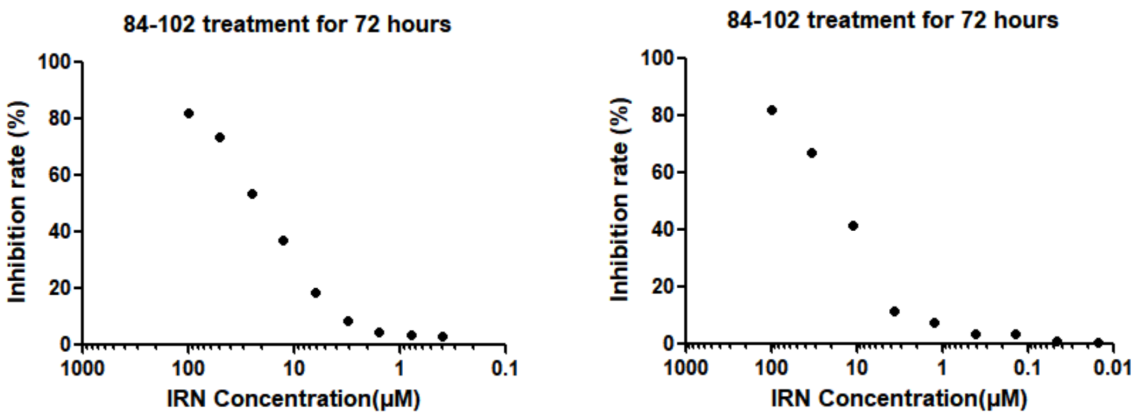


2-Fold dilution 3-Fold dilution

[Figure 3]

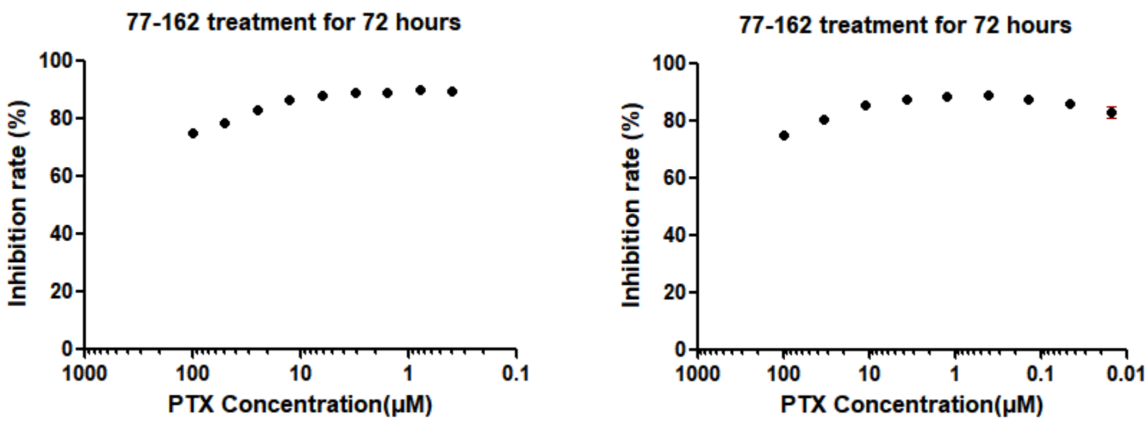


2-Fold dilution 3-Fold dilution

[Figure 4]

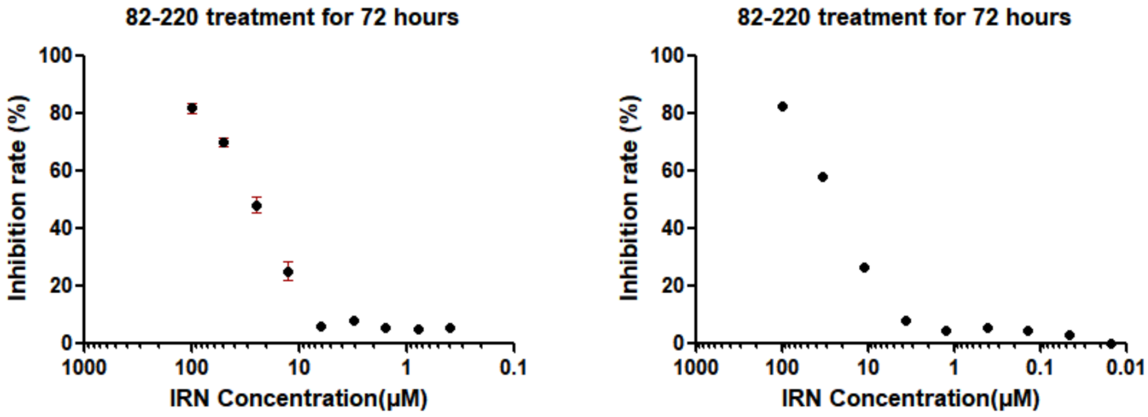


2-Fold dilution 3-Fold dilution

[Figure 5]

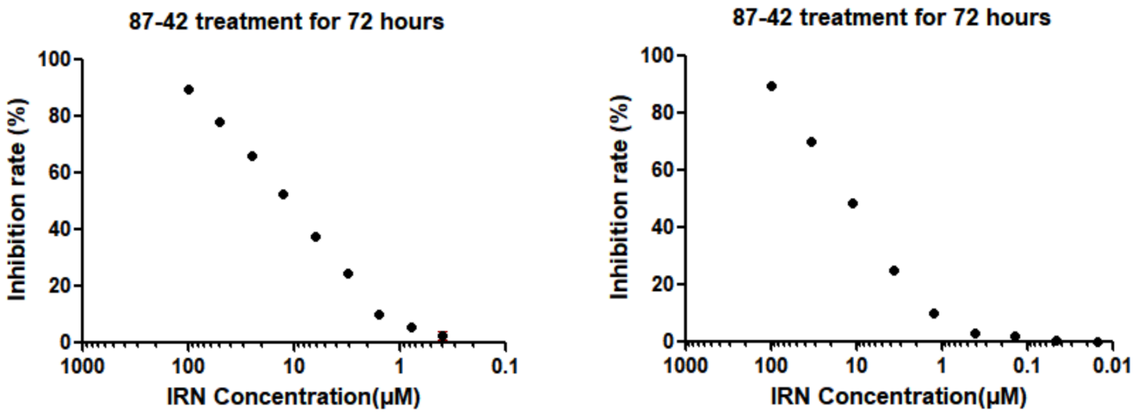


2-Fold dilution 3-Fold dilution

[Figure 6]

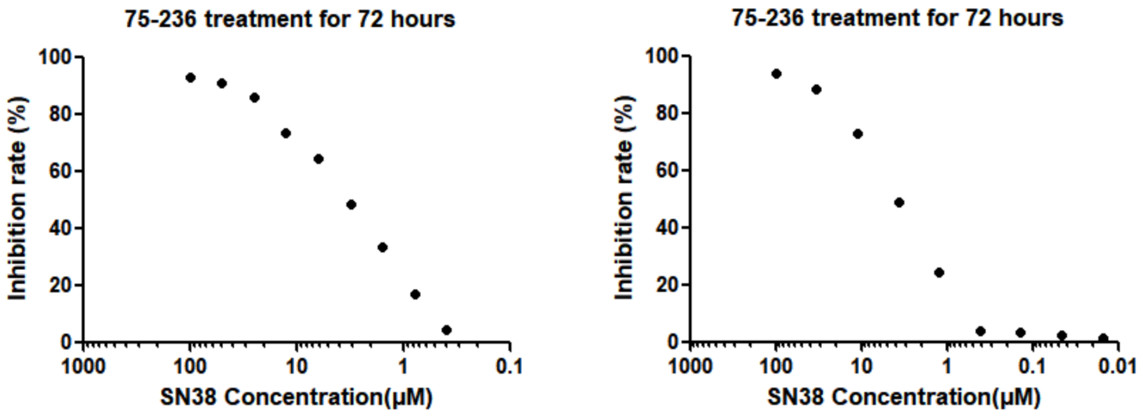


2-Fold dilution 3-Fold dilution

[Figure 7]

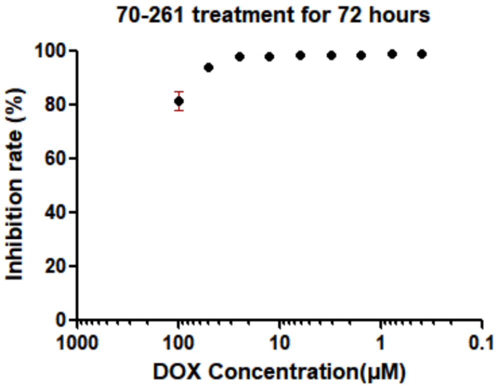


2-Fold dilution

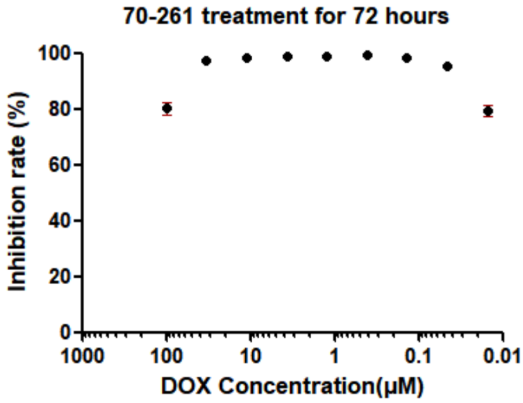


3-Fold dilution

[Figure 8]

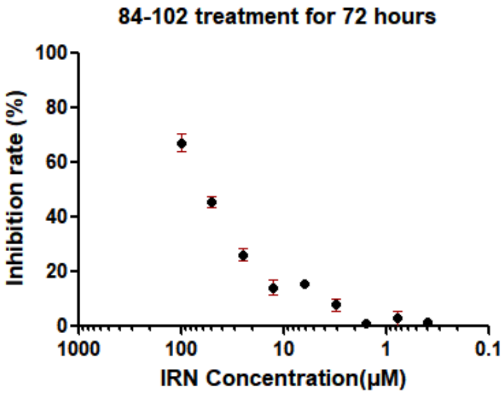


2-Fold dilution

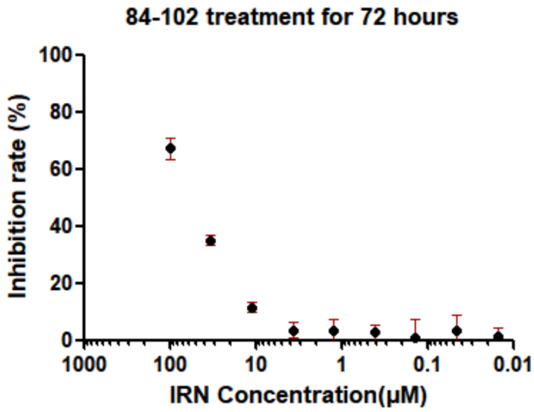


3-Fold dilution

2

[Figure 9]

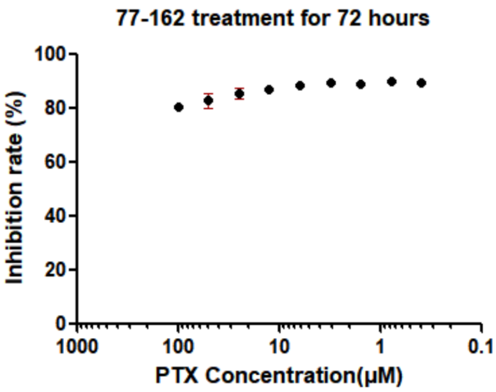


2-Fold dilution

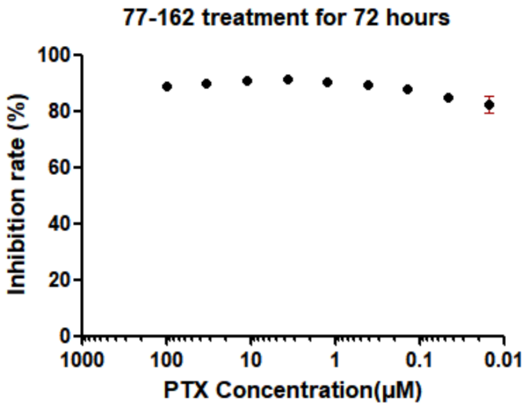


3-Fold dilution

[Figure 10]

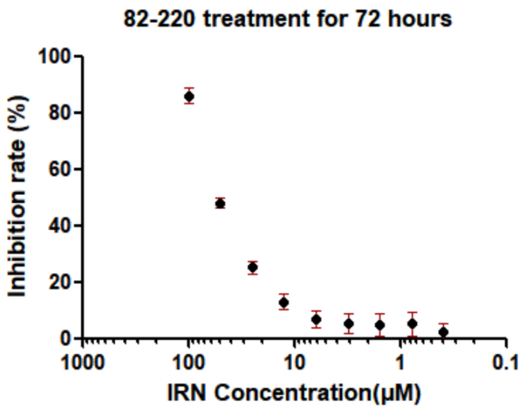


2-Fold dilution

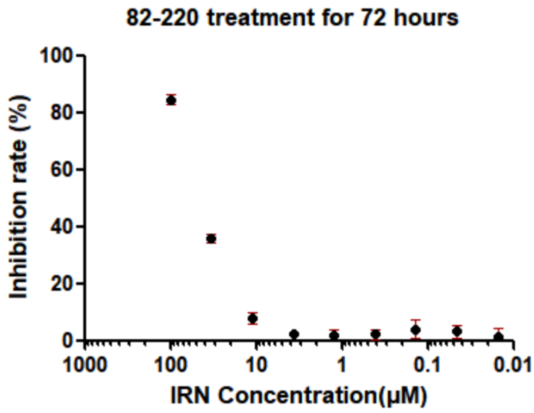


3-Fold dilution

[Figure 11]

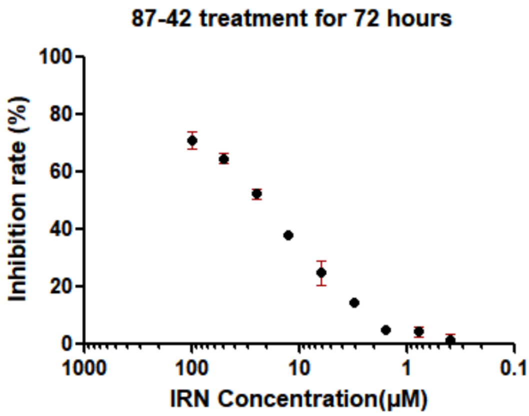


2-Fold dilution

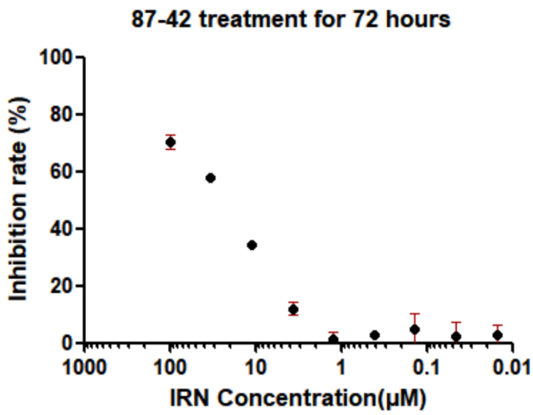


3-Fold dilution

[Figure 12]

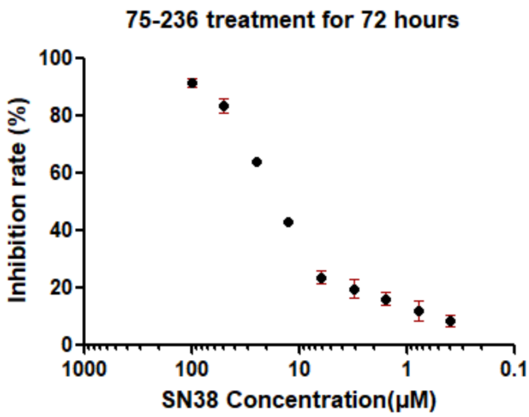


2-Fold dilution

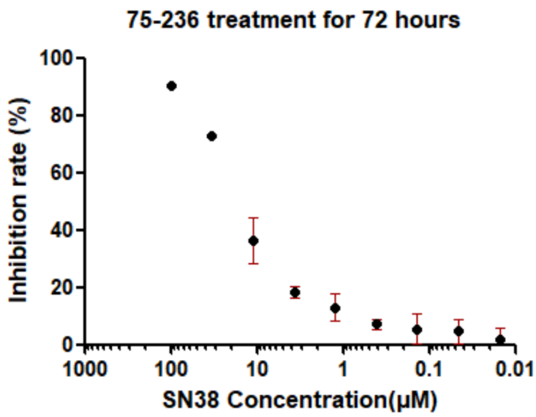


3-Fold dilution

[Figure 13]

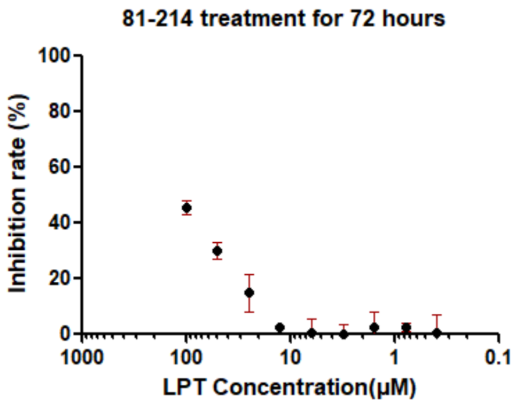


2-Fold dilution

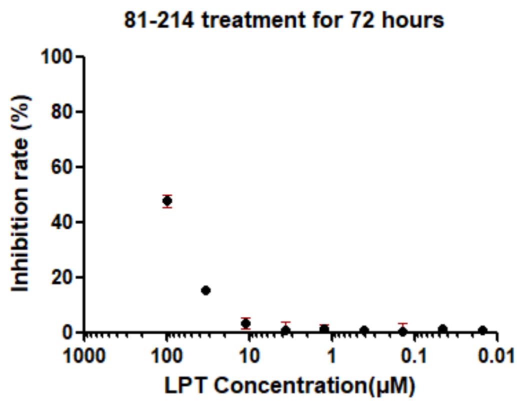


3-Fold dilution

[Figure 14]

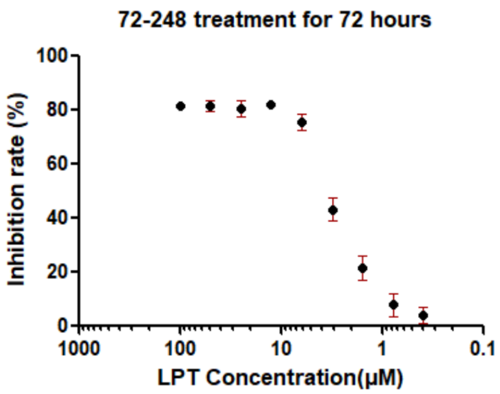


2-Fold dilution

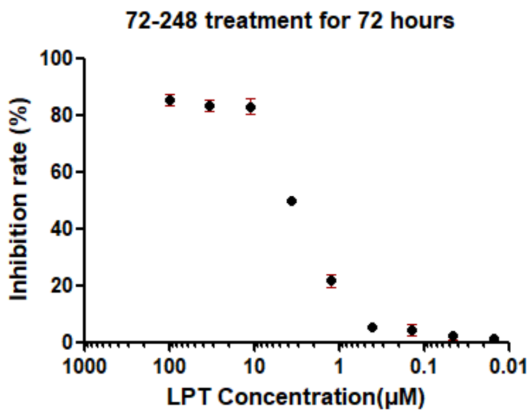


3-Fold dilution

[Figure 15]

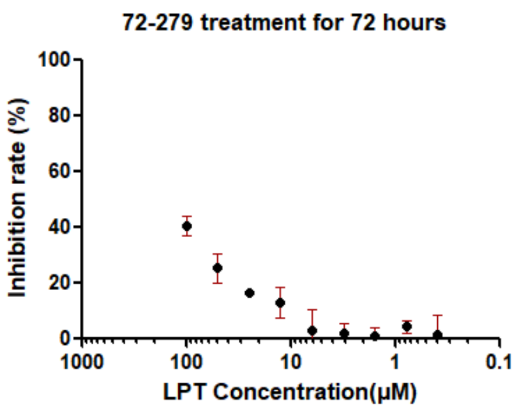


2-Fold dilution

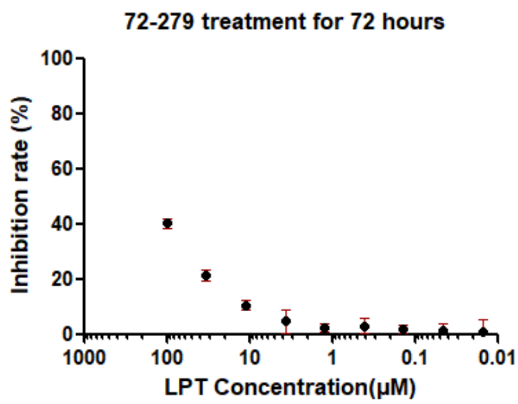


3-Fold dilution

[Figure 16]

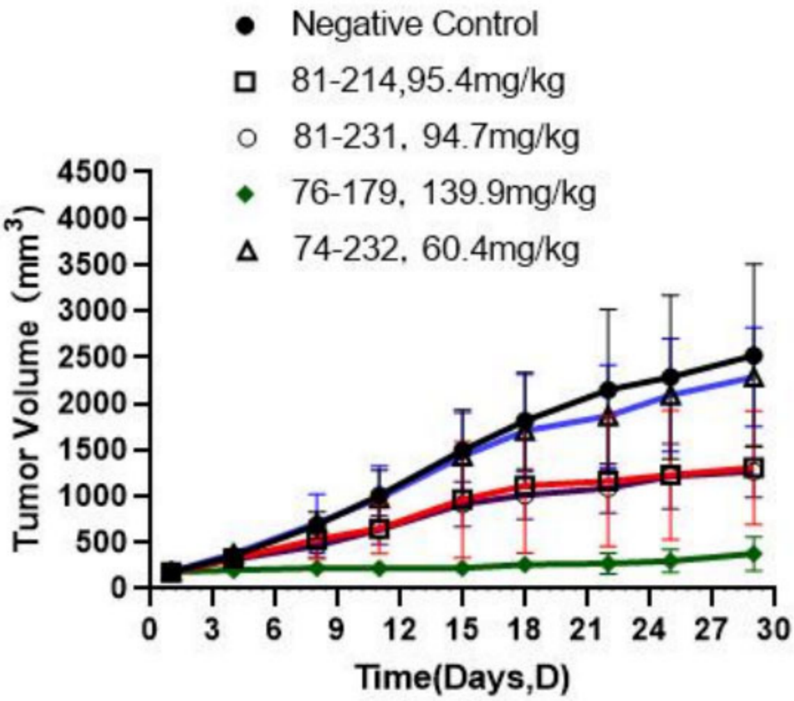


[Figure 17]

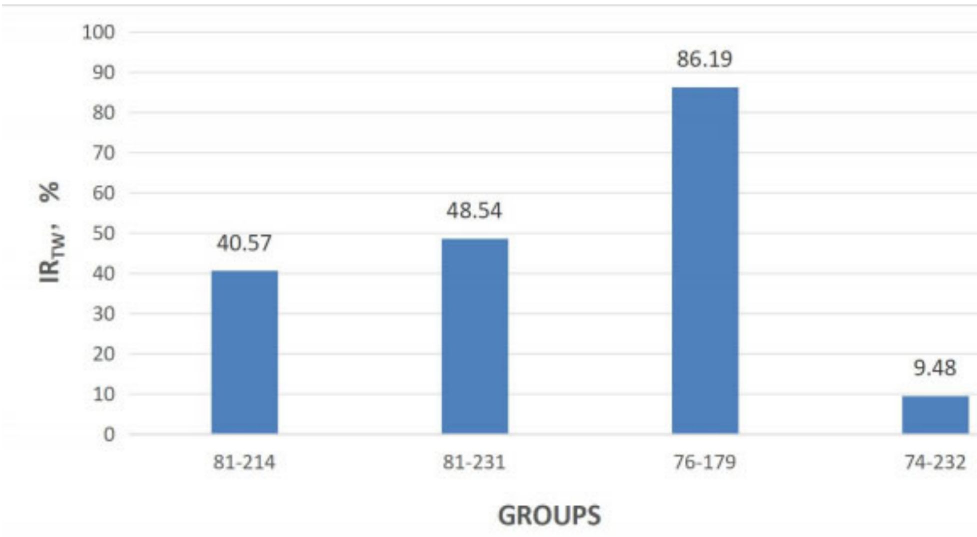


[Figure 18]

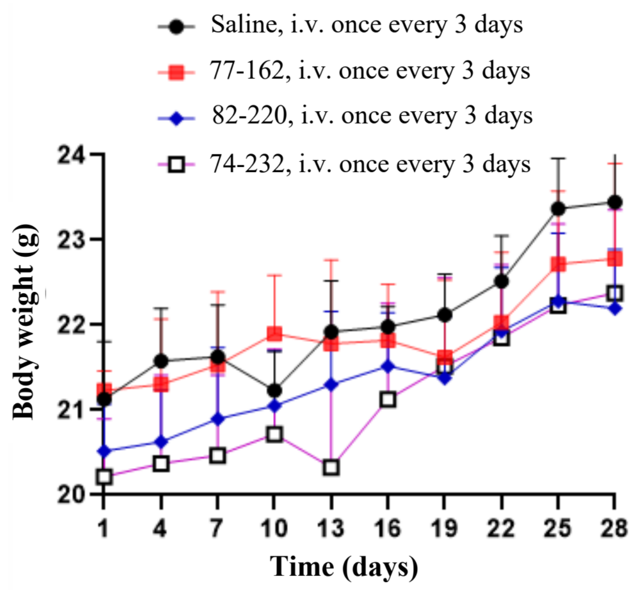


[Figure 19]

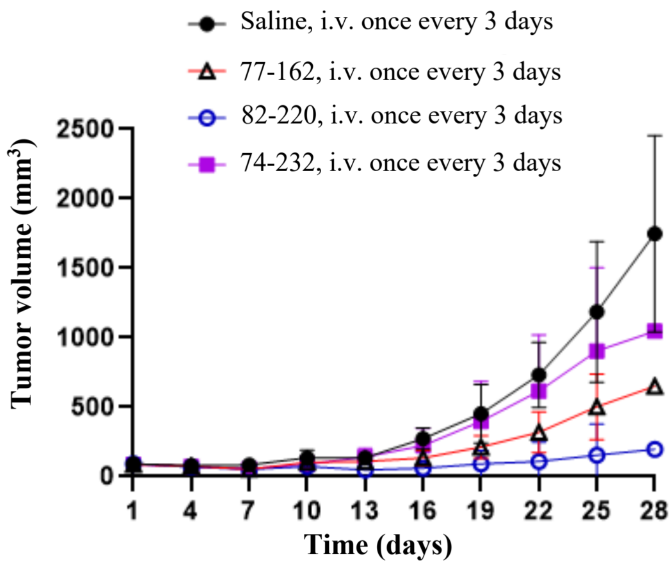


[Figure 20]

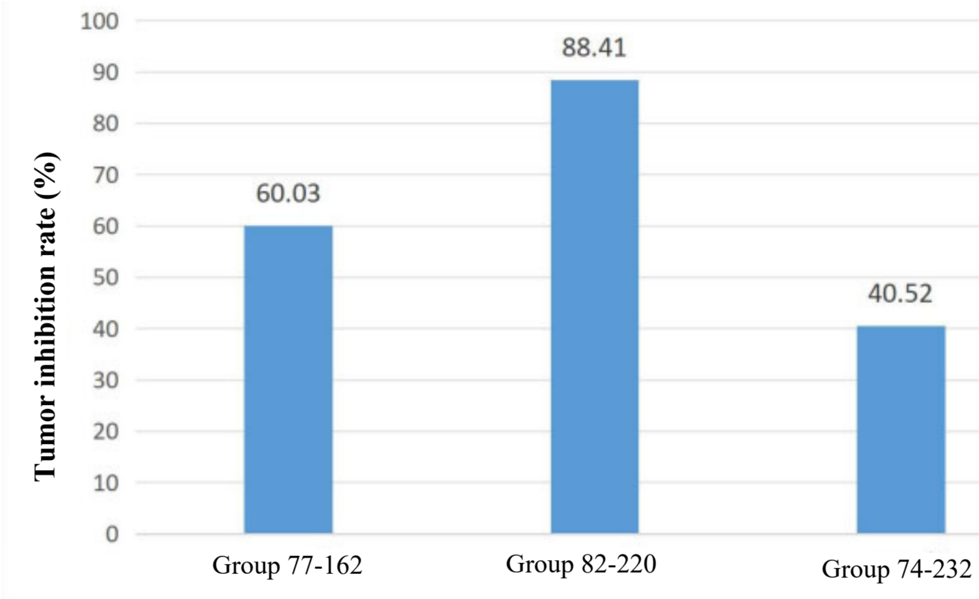


[Figure 21]

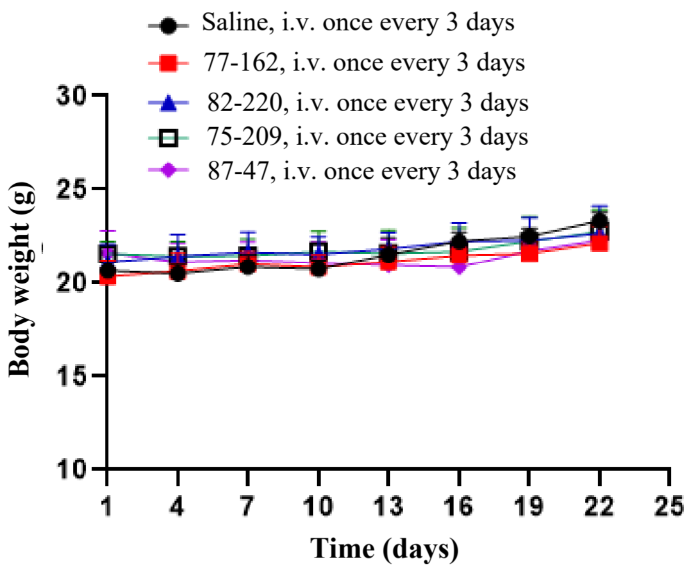


[Figure 22]

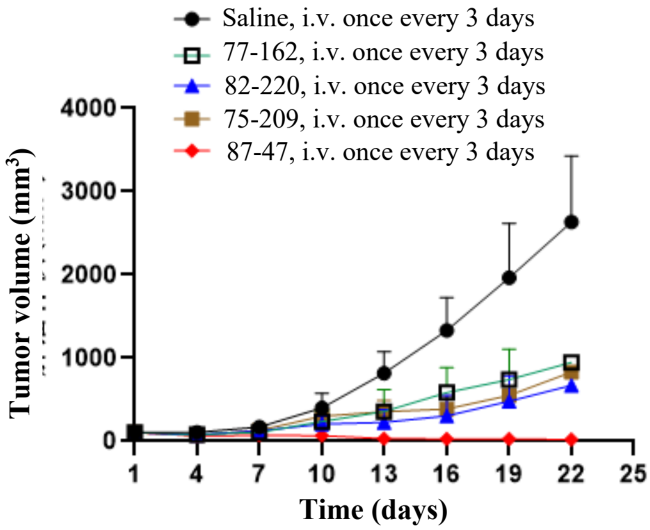
Saline, i.v. once every 3 days
77-162, i.v. once every 3 days
82-220, i.v. once every 3 days
75-209, i.v. once every 3 days
87-47, i.v. once every 3 days
Tumor volume (mm3)
4000
3000
2000
1000
0
1
4
7
10
13
16
19
22
25
Time (days)

[Figure 23]

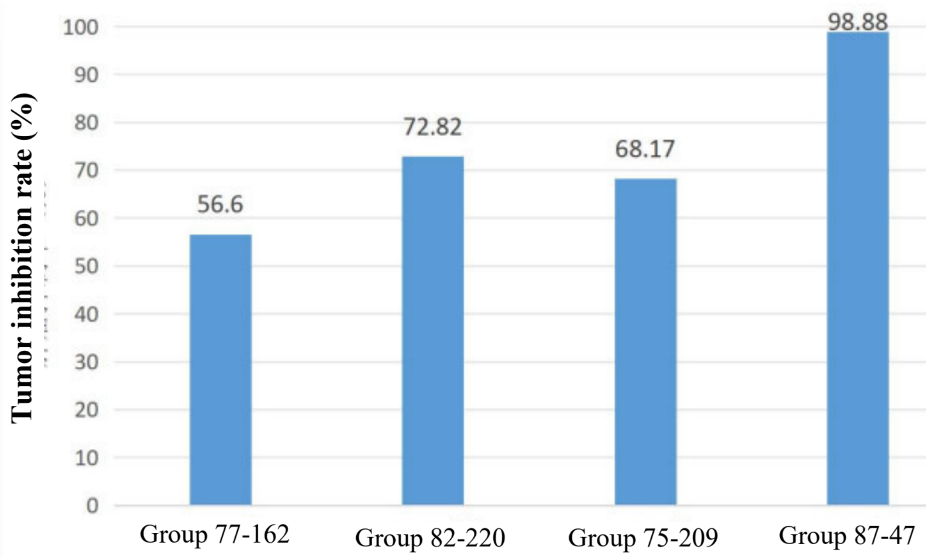
Tumor inhibition rate (%)
100
90
80
70
60
50
40
30
20
10
0
56.6
72.82
68.17
98.88
Group 77-162
Group 82-220
Group 75-209
Group 87-47

**INTERNATIONAL SEARCH REPORT**

International application No. **PCT/CN2024/099265**

**A. CLASSIFICATION OF SUBJECT MATTER**

C08G69/40(2006.01)i; C08G69/08(2006.01)i; C08G65/333(2006.01)i; A61K47/60(2017.01)i; A61K47/64 (2017.01)i; A61P35/00(2006.01)i

According to International Patent Classification (IPC) or to both national classification and IPC

**B. FIELDS SEARCHED**

Minimum documentation searched (classification system followed by classification symbols)

IPC: C08G69, C08G65, A61K47, A61P35

Documentation searched other than minimum documentation to the extent that such documents are included in the fields searched

Electronic data base consulted during the international search (name of data base and, where practicable, search terms used)

DWPI, WPABS, CNABS, CNTXT, ENTXT, CNKI, ISI web of knowledge: 多胺, 多元醇, 赖氨酸, 半胱氨酸, 苏氨酸, 色氨酸, 丝氨酸, 天冬氨酸, 谷氨酸, 聚乙二醇, 聚氧化烯, 聚多肽, 肿瘤, 癌症, polyamine, diamine, polyol, amino acid, polylysine, lysine, cysteine, threonine, serine, aspartic acid, glutamic acid, PEG, polyethylene glycol, polyethyleneglycol, polyethylene oxide, polyoxyethylene, polypeptide, peptide, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| A | CN 113995846 A (CHONGQING APU GELEI BIOTECHNOLOGY CO., LTD.) 01 February 2022 (2022-02-01)<br>claims 1-10 | 1-49 |
| A | CN 112851928 A (CHONGQING APU GELEI BIOTECHNOLOGY CO., LTD.) 28 May 2021 (2021-05-28)<br>entire document | 1-49 |
| A | CN 112843242 A (CHONGQING APU GELEI BIOTECHNOLOGY CO., LTD.) 28 May 2021 (2021-05-28)<br>entire document | 1-49 |
| A | CN 112915210 A (CHONGQING APU GELEI BIOTECHNOLOGY CO., LTD.) 08 June 2021 (2021-06-08)<br>entire document | 1-49 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **24 September 2024** | **27 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/099265

C. DOCUMENTS CONSIDERED TO BE RELEVANT

| Category* | Citation of document, with indication, where appropriate, of the relevant passages | Relevant to claim No. |
|---|---|---|
| A | CN 104987504 A (NANJING MINGZHEN PHARMACEUTICAL TECHNOLOGY CO., LTD.) 21 October 2015 (2015-10-21) entire document | 1-49 |
| A | CN 113995847 A (CHONGQING APU GELEI BIOTECHNOLOGY CO., LTD.) 01 February 2022 (2022-02-01) entire document | 1-49 |

Form PCT/ISA/210 (second sheet) (July 2022)

**INTERNATIONAL SEARCH REPORT**
**Information on patent family members**

International application No.
**PCT/CN2024/099265**

| Patent document cited in search report | | | Publication date (day/month/year) | Patent family member(s) | | | Publication date (day/month/year) |
|---|---|---|---|---|---|---|---|
| CN | 113995846 | A | 01 February 2022 | JP | 2023537248 | A | 31 August 2023 |
| | | | | EP | 4190360 | A1 | 07 June 2023 |
| | | | | WO | 2022022354 | A1 | 03 February 2022 |
| | | | | US | 2023348671 | A1 | 02 November 2023 |
| | | | | CN | 113995846 | B | 22 March 2024 |
| CN | 112851928 | A | 28 May 2021 | JP | 2022554026 | A | 27 December 2022 |
| | | | | AU | 2020389657 | A1 | 23 June 2022 |
| | | | | AU | 2020389657 | B2 | 01 August 2024 |
| | | | | BR | 112022010360 | A2 | 16 August 2022 |
| | | | | WO | 2021104120 | A1 | 03 June 2021 |
| | | | | US | 2023088403 | A1 | 23 March 2023 |
| | | | | US | 11793881 | B2 | 24 October 2023 |
| | | | | KR | 20220102659 | A | 20 July 2022 |
| | | | | KR | 102542715 | B1 | 14 June 2023 |
| | | | | MX | 2022006519 | A | 19 August 2022 |
| | | | | EP | 4066861 | A1 | 05 October 2022 |
| | | | | EP | 4066861 | A4 | 27 December 2023 |
| | | | | CA | 3159842 | A1 | 03 June 2021 |
| | | | | CN | 112851928 | B | 25 April 2023 |
| CN | 112843242 | A | 28 May 2021 | CN | 112843242 | B | 01 March 2024 |
| CN | 112915210 | A | 08 June 2021 | CN | 112915210 | B | 20 August 2024 |
| CN | 104987504 | A | 21 October 2015 | CN | 104987504 | B | 01 May 2018 |
| CN | 113995847 | A | 01 February 2022 | AU | 2021319279 | A1 | 23 February 2023 |
| | | | | EP | 4190361 | A1 | 07 June 2023 |
| | | | | US | 2024115713 | A1 | 11 April 2024 |
| | | | | JP | 2023537247 | A | 31 August 2023 |
| | | | | WO | 2022022360 | A1 | 03 February 2022 |
| | | | | CA | 3189933 | A1 | 03 February 2022 |
| | | | | CN | 113995847 | B | 25 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310714197


### Non-patent literature cited in the description


- **GENNARO AR**. Remington's Pharmaceutical Sciences. Mack Publishing Company, 1995